(19) Europäisches Patentamt
European Patent Office
Office européen des brevets

(11) EP 1 464 335 A2

(12) **EUROPEAN PATENT APPLICATION**

(43) Date of publication:
**06.10.2004 Bulletin 2004/41**

(21) Application number: **04007651.5**

(22) Date of filing: **30.03.2004**

(51) Int Cl.⁷: **A61K 31/4709**, C07D 401/12, C07D 403/12, C07D 405/12, C07D 409/12, C07D 413/12, C07D 417/12, C07D 417/14, C07D 215/38, A61K 31/506, A61P 3/04

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **31.03.2003 US 458530 P**
**19.08.2003 US 495911 P**
**09.10.2003 US 510186 P**
**16.12.2003 US 530360 P**

(71) Applicants:
- **Taisho Pharmaceutical Co. Ltd.**
  **Tokyo (JP)**
- **Arena Pharmaceuticals, Inc.**
  **San Diego, CA 92121 (US)**

(72) Inventors:
- **Sekiguchi, Yoshinori**
  **Taisho Pharmaceutical Co. Ltd**
  **Tokyo (JP)**
- **Kanuma, Kosuke Taisho Pharmaceutical Co. Ltd**
  **Tokyo (JP)**
- **Omodera, Katsunori**
  **Taisho Pharmaceutical Co. Ltd**
  **Tokyo (JP)**
- **Busujima, Tsuyoshi**
  **Taisho Pharmaceutical Co. Ltd**
  **Tokyo (JP)**
- **Tran, Thuy-Anh**
  **San Diego CA 92130 (US)**
- **Han, Sangdon**
  **San Diego CA 92127 (US)**
- **Casper, Martin**
  **San Diego CA 92130 (US)**
- **Kramer, Bryan A.**
  **San Diego Ca 92126 (US)**
- **Semple, Graeme**
  **San Diego CA 92127 (US)**
- **Zou, Ning**
  **San Diego CA 92127 (US)**

(74) Representative: **HOFFMANN - EITLE**
**Patent- und Rechtsanwälte**
**Arabellastrasse 4**
**81925 München (DE)**

(54) **QUINOLINE, TETRAHYDROQUINOLINE AND PYRIMIDINE DERIVATIVES AS MCH ANTAGONIST**

(57) The present invention relates to compounds of the Formula (I)

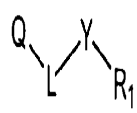

(I)

wherein Q is:

EP 1 464 335 A2

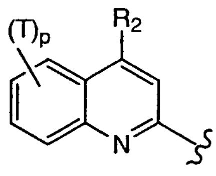

**(II)**

,

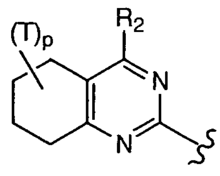

**(III)**

or

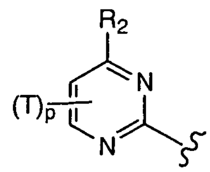

**(IV)**

which act as MCH receptor antagonists. These compositions are useful in pharmaceutical compositions whose use includes prophylaxis or treatment of improving memory function, sleeping and arousal, anxiety, depression, mood disorders, seizure, obesity, diabetes, appetite and eating disorders, cardiovascular disease, hypertension, dyslipidemia, myocardial infarction, binge eating disorders including bulimia, anorexia, mental disorders including manic depression, schizophrenia, delirium, dementia, stress, cognitive disorders, attention deficit disorder, substance abuse disorders and dyskinesias including Parkinson's disease, epilepsy, and addiction.

## Description

### Field of the Invention

The present invention relates to compounds which act as antagonists for MCH receptors and to the use of these compounds in pharmaceutical compositions.

### Background of the Invention

Melanin Concentrating Hormone (MCH), a cyclic peptide, has been identified as the endogenous ligand of the orphan G-protein coupled receptor SLC-1. See, for example, Shimomura et al., *Biochem. Biophys. Res. Commun.* 261, 622-26 (1999). Studies have indicated that MCH acts as a neurotransmitter/neuromodulator to alter a number of behavioral responses such as feeding habits. For example, injection of MCH into rats has been reported to increase their consumption of food. Reports indicate that genetically engineered mice which lack MCH show lower body weight and increased metabolism. See Saito et al., *TEM,* vol. 11,299 (2000). As such, the literature suggests that discovery of MCH antagonists that interact with SCL-1 expressing cells will be useful in developing obesity treatments. See Shimomura et al., *Biochem. Biophys. Res. Commun.* 261, 622-26 (1999).

G protein-coupled receptors (GPCRs) share a common structural motif. All these receptors have seven sequences of between 22 to 24 hydrophobic amino acids that form seven alpha helices, each of which spans the membrane. The fourth and fifth transmembrane helices are joined on the extracellular side of the membrane by a strand of amino acids that forms a relatively large loop. Another larger loop, composed primarily of hydrophilic amino acids, joins transmembrane helices five and six on the intracellular side of the membrane. The carboxy terminus of the receptor lies intracellularly, and the amino terminus lies in the extracellular space. It is thought that the loop joining helices five and six, as well as the carboxy terminus, interact with the G protein. Currently, Gq, Gs, Gi, and Go are G proteins that have been identified as possible proteins that interact with the receptor.

Under physiological conditions, GPCRs exist in the cell membrane in equilibrium between two different states or conformations: an "inactive" state and an "active" state. A receptor in an inactive state is unable to link to the intracellular transduction pathway to produce a biological response. Changing the receptor conformation to the active state allows linkage to the transduction pathway and produces a biological response.

A receptor may be stabilized in an active state by an endogenous ligand or an exogenous agonist ligand. Recent discoveries, including but not exclusively limited to, modifications to the amino acid sequence of the receptor, provide alternative mechanisms other than ligands to stabilize the active state conformation. These approaches effectively stabilize the receptor in an active state by simulating the effect of a ligand binding to the receptor. Stabilization by such ligand-independent approaches is termed "constitutive receptor activation." In contrast, antagonists can competitively bind to the receptor at the same site as agonists, but do not activate.the intracellular response initiated by the active form of the receptor, and therefore inhibit the intracellular responses by agonists.

Certain 2-aminoquinazoline derivatives have been reported to be NPY antagonists which are said to be effective in the treatment of disorders and diseases associated with the NPY receptor subtype Y5. See PCT Patent Application 97/20823. Quinazoline derivatives have also been found to be useful by enhancing antitumor activity. See PCT Patent Application 92/07844. And also the quinoline derivatives which have an antagonist activity for MCH receptor are known in these patents, WO03/070244, WO03/105850, WO03/45313, WO03/045920, and WO04/04726.

Recently, our current knowledge of human obesity has advanced dramatically. Previously, obesity was viewed as an oppugnant behavior of inappropriate eating in the setting of appealing foods. Studies of animal models of obesity, biochemical alterations in both humans and animals, and the complex interactions of psychosocial and cultural factors that create receptiveness to human obesity indicate that this disease in humans is multifaceted and deeply entrenched in biologic systems. Thus, it is almost certain that obesity has multiple causes and that there are different types of obesity. Not only does MCHR1 antagonist have potent and durable anti-obesity effects in rodents, it has surprising antidepressant and anxiolytic properties as well (Borowsky et al., *Nature Medicine,* 8, 825-830, 2002). MCHR1 antagonists have been reported to show antidepressant and anxiolytic activities in rodent models such as social interaction, forced swimming test and ultrasonic vocalization. These findings indicate that MCHR1 antagonists could be useful for treatment of obesity patients with multiple causes. Moreover, MCHR1 antagonists could be used to treat subjects not only with obesity, but also those with depression and anxiety. These advantages make it different from NPY receptor antagonists, with which anxiogenic-like activity can be expected, as NPY itself has anxiolytic-like effect.

Obesity is also regarded as a chronic disease and the possibly of long-term treatment is a concept that is receiving more attention. In this context, it is noteworthy that the depletion of MCH leads to hypophagia as well as leanness (Shimada et al., *Nature,* 396, 670-674, 1998). By contrast, NPY (Erickson et al., *Nature,* 381, 415-418, 1996), as well as the Y1 (Pedrazzini et al., *Nature Medicine,* 4, 722-726, 1998) and Y5 receptors (Marsh et al., *Nature Medicine,* 4, 718-721, 1998), disrupted mice maintained a stable body weight or rather became obese. Considering the above

reports, MCHR1 antagonists can be more attractive than Y1 or Y5 receptor antagonists in terms of long-term treatment of obese patients.

Obesity, which is the result of an imbalance between caloric intake and energy expenditure, is highly correlated with insulin resistance and diabetes in experimental animals and human. However, the molecular mechanisms that are involved in obesity-diabetes syndromes are not clear. During early development of obesity, increase insulin secretion balances insulin resistance and protects patients from hyperglycemia (Le Stunff, et al. *Diabetes* 43, 696-702 (1989)). However, after several decades, β cell function deteriorates and non-insulin-dependent diabetes develops in about 20% of the obese population (Pederson, P. *Diab. Metab. Rev.* 5, 505-509 (1989)) and (Brancati, F. L., et al., *Arch. Intern. Med.* 159, 957-963 (1999)). Given its high prevalence in modem societies, obesity has thus become the leading risk factor for NIDDM (Hill, J. O., et al., *Science* 280, 1371-1374 (1998)). However, the factors which predispose a fraction of patients to alteration of insulin secretion in response to fat accumulation remain unknown.

Whether someone is classified as overweight or obese is generally determined on the basis of their body mass index (BMI) which is calculated by dividing body weight (kg) by height squared ($m^2$). Thus, the units of BMI are $kg/m^2$ and it is possible to calculate the BMI range associated with minimum mortality in each decade of life. Overweight is defined as a BMI in the range 25-30 $kg/m^2$, and obesity as a BMI greater than 30 $kg/m^2$ (see TABLE below). There are problems with this definition in that it does not take into account the proportion of body mass that is muscle in relation to fat (adipose tissue). To account for this, obesity can also be defined on the basis of body fat content: greater than 25% and 30% in males and females, respectively.

| CLASSIFICATION OF WEIGHT BY BODY MASS INDEX (BMI) | |
|---|---|
| BMI | CLASSIFICATION |
| < 18.5 | Underweight |
| 18.5-24.9 | Normal |
| 25.0-29.9 | Overweight |
| 30.0-34.9 | Obesity (Class I) |
| 35.0-39.9 | Obesity (Class II) |
| >40 | Extreme Obesity (Class III) |

As the BMI increases there is an increased risk of death from a variety of causes that is independent of other risk factors. The most common diseases with obesity are cardiovascular disease (particularly hypertension), diabetes (obesity aggravates the development of diabetes), gall bladder disease (particularly cancer) and diseases of reproduction. Research has shown that even a modest reduction in body weight can correspond to a significant reduction in the risk of developing coronary heart disease.

Compounds marketed as anti-obesity agents include Orlistat (XENICAL™) and Sibutramine. Orlistat (a lipase inhibitor) inhibits fat absorption directly and tends to produce a high incidence of unpleasant (though relatively harmless) side-effects such as diarrhea. Sibutramine (a mixed 5-HT/noradrenaline reuptake inhibitor) can increase blood pressure and heart rate in some patients. The serotonin releaser/reuptake inhibitors fenfluramine (Pondimin™) and dexfenfluramine (Redux™) have been reported to decrease food intake and body weight over a prolonged period (greater than 6 months). However, both products were withdrawn after reports of preliminary evidence of heart valve abnormalities associated with their use. Accordingly, there is a need for the development of a safer anti-obesity agent.

Obesity considerably increases the risk of developing cardiovascular diseases as well. Coronary insufficiency, atheromatous disease, and cardiac insufficiency are at the forefront of the cardiovascular complication induced by obesity. It is estimated that if the entire population had an ideal weight, the risk of coronary insufficiency would decrease by 25 % and the risk of cardiac insufficiency and of cerebral vascular accidents by 35%. The incidence of coronary diseases is doubled in subjects less than 50 years of age who are 30% overweight. The diabetes patient faces a 30% reduced lifespan. After age 45, people with diabetes are about three times more likely than people without diabetes to have significant heart disease and up to five times more likely to have a stroke. These findings emphasize the inter-relations between risks factors for NIDDM and coronary heart disease and the potential value of an integrated approach to the prevention of these conditions based on the prevention of these conditions based on the prevention of obesity (Perry, I. J., et al., *BMJ* 310,560-564 (1995)).

An increasing number of children and adolescents are overweight. Although not all overweight children will necessarily become overweight adults, the growing occurrence of obesity in childhood is likely to be reflected in increasing obesity in adult years. The high prevalence of obesity in our adult population and the likelihood that the nation of the future will be even more obese demands a re-examination of the health implications of this disease. See, Health

Implications of Obesity. NIH Consens. Statement Online 1985 Feb 11-13; 5(9):1-7.

"Clinical obesity" is a measurement of the excess body fat relative to lean body mass and is defined as a body weight more than 20% above the ideal body weight. Recent estimates suggest that 1 in 2 adults in the United States is clinically obese, an increase of more than 25% over the past decades. Flegal M.D. et al., 22 Int. *J. Obes. Relat. Metab. Disor.* 39 (1998). Both overweight conditions and clinical obesity are a major health concerns worldwide, in particular because clinical obesity is often accompanied by numerous complications, *i.e.*, hypertension and Type II diabetes, which in turn can cause coronary artery disease, stroke, late-stage complications of diabetes and premature death. *(See,* e.g., Nishina P.M. et al., 43 *Metab.* 554 (1994)).

Although the etiologic mechanisms underlying obesity require further clarification, the net effect of such mechanisms leads to an imbalance between energy intake and expenditure. Both genetic and environmental factors are likely to be involved in the pathogenesis of obesity. These include excess caloric intake, decreased physical activity, and metabolic and endocrine abnormalities.

Treatment of overweight conditions and clinical obesity via pharmaceutical agents are not only of importance with respect to the conditions themselves, but also with respect to the possibility of preventing other diseases that are associated with, *e.g.*, clinical obesity, as well as enhancement of the positive feeling of "self" that often accompanies those who are overweight or clinically obese and who encounter a significant reduction in body weight. Given the foregoing discussion, it is apparent that compounds which help in the treatment of such disorders would be useful and would provide an advance in both research and clinical medicine. The present invention is directed to these, as well as other, important ends.

## SUMMARY OF THE INVENTION

The present invention is drawn to compounds, which bind to and modulate the activity of a GPCR referred to herein as **MCH**, and uses thereof. The term **MCH**, as used herein, includes the human sequences found in GeneBank accession number NM_005297, naturally-occurring allelic variants, mammalian orthologs, biologically active fragments and recombinant mutants thereof.

One aspect of the present invention relates to certain substituted heterocyclic compounds represented by Formula (I):

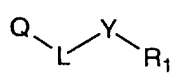

**(I)**

wherein Q is:

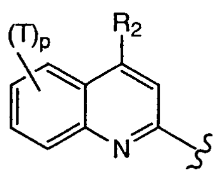

,

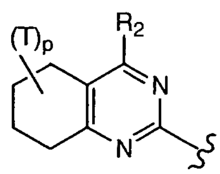

or

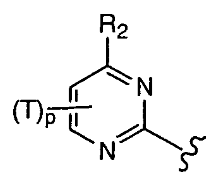

**(II)** **(III)** **(IV)**

$R_1$ is selected from the group consisting of:

(i) $C_{1-16}$ alkyl, and
$C_{1-16}$ alkyl substituted by substituent(s) independently selected from the group consisting of:

- halogen,
- hydroxy,
- oxo,
- $C_{1-5}$ alkoxy,
- $C_{1-5}$ alkoxy substituted by substituent(s) independently selected from the group consisting of:

  •• carbocyclic aryl,

- •• heterocyclyl, and
- •• heterocyclyl substituted by $C_{1-5}$ alkyl,

- $C_{1-5}$ alkylcarbonyloxy,
- carbocyclyloxy,
- carbocyclic aryloxy,
- carbocyclic aryloxy substituted by substituent(s) independently selected from the group consisting of:

  - •• halogen,
  - •• hydroxy,
  - •• carboxy,
  - •• carbamoyl,
  - •• nitro,
  - •• cyano,
  - •• amino,
  - •• carbocyclic aryl,
  - •• carbocyclic aryl substituted by $C_{1-5}$ alkoxy,
  - •• $C_{1-5}$ alkoxy,
  - •• $C_{1-5}$ alkoxy substituted by halogen,
  - •• $C_{1-5}$ alkyl, and
  - •• $C_{1-5}$ alkyl substituted by substituent(s) independently selected from the group consisting of:

    - ••• halogen,
    - ••• hydroxy,
    - ••• carboxy,
    - ••• oxo,
    - ••• mono-$C_{1-5}$ alkylamino,
    - ••• di-$C_{1-5}$ alkylamino,
    - ••• mono-$C_{1-5}$ alkylamino substituted by carbocyclic aryl,
    - ••• di-$C_{1-5}$ alkylamino substituted by carbocyclic aryl,
    - ••• mono-$C_{1-5}$ alkylamino substituted by halogenated carbocyclic aryl,
    - ••• di-$C_{1-5}$ alkylamino substituted by halogenated carbocyclic aryl,
    - ••• carbocyclic arylcarbonylamino, and
    - ••• carbocyclic arylcarbonylamino substituted by halogen,

- heterocyclyloxy,
- heterocyclyloxy substituted by substituent(s) independently selected from the group consisting of:

  - •• halogen,
  - •• hydroxy,
  - •• carboxy,
  - •• carbamoyl,
  - •• nitro,
  - •• cyano,
  - •• amino,
  - •• carbocyclic aryl,
  - •• carbocyclic aryl substituted by $C_{1-5}$ alkoxy,
  - •• $C_{1-5}$ alkoxy,
  - •• $C_{1-5}$ alkoxy substituted by substituent(s) independently selected from the group consisting of:

    - ••• halogen,
    - ••• hydroxy, and
    - ••• carboxy,

  - •• $C_{1-5}$ alkyl, and
  - •• $C_{1-5}$ alkyl substituted by substituent(s) independently selected from the group consisting of:

    - ••• halogen,

      - hydroxy, and
      - carboxy,

- substituted heterocyclyl-ethylideneaminooxy,
- $C_{1-5}$ alkoxycarbonyl,
- $C_{1-5}$ alkoxycarbonyl substituted by carbocyclic aryl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by substituent(s) independently selected from the group consisting of:

  - cyano,
  - carbocyclic aryl, and
  - heterocyclyl,

- di-$C_{1-5}$ alkylamino,
- di-$C_{1-5}$ alkylamino substituted by substituent(s) independently selected from the group consisting of:

  - cyano,
  - carbocyclic aryl, and
  - heterocyclyl,

- mono-carbocyclic arylamino,
- mono-carbocyclic arylamino substituted by substituent(s) independently selected from the group consisting of:

  - halogen,
  - hydroxy,
  - carboxy,
  - carbamoyl,
  - nitro,
  - cyano,
  - amino,
  - carbocyclic aryl,
  - carbocyclic aryl substituted by $C_{1-5}$ alkoxy,
  - $C_{1-5}$ alkoxy,
  - $C_{1-5}$ alkoxy substituted by substituent(s) independently selected from the group consisting of:

    - halogen,
    - hydroxy, and
    - carboxy,

  - $C_{1-5}$ alkyl, and
  - $C_{1-5}$ alkyl substituted by substituent(s) independently selected from the group consisting of:

    - halogen,
    - hydroxy, and
    - carboxy,

- di-carbocyclic arylamino,
- di-carbocyclic arylamino substituted by substituent(s) independently selected from the group consisting of:

  - halogen,
  - hydroxy,
  - carboxy,
  - carbamoyl,
  - nitro,
  - cyano,

- •• amino,
- •• carbocyclic aryl,
- •• carbocyclic aryl substituted by $C_{1-5}$ alkoxy,
- •• $C_{1-5}$ alkoxy,
- •• $C_{1-5}$ alkoxy substituted by substituent(s) independently selected from the group consisting of:
  - ••• halogen,
  - ••• hydroxy, and
  - ••• carboxy,
- •• $C_{1-5}$ alkyl, and
- •• $C_{1-5}$ alkyl substituted by substituent(s) independently selected from the group consisting of:
  - ••• halogen,
  - ••• hydroxy, and
  - ••• carboxy,

- • mono-heterocyclylamino,
- • mono-heterocyclylamino substituted by substituent(s) independently selected from the group consisting of:
  - •• halogen,
  - •• hydroxy,
  - •• carboxy,
  - •• carbamoyl,
  - •• nitro,
  - •• cyano,
  - •• amino,
  - •• carbocyclic aryl,
  - •• carbocyclic aryl substituted by $C_{1-5}$ alkoxy,
  - •• $C_{1-5}$ alkoxy,
  - •• $C_{1-5}$ alkoxy substituted by substituent(s) independently selected from the group consisting of:
    - ••• halogen,
    - ••• hydroxy, and
    - ••• carboxy,
  - •• $C_{1-5}$ alkyl, and
  - •• $C_{1-5}$ alkyl substituted by substituent(s) independently selected from the group consisting of:
    - ••• halogen,
    - ••• hydroxy, and
    - ••• carboxy,

- • di-heterocyclylamino,
- • di-heterocyclylamino substituted by substituent(s) independently selected from the group consisting of:
  - •• halogen,
  - •• hydroxy,
  - •• carboxy,
  - •• carbamoyl,
  - •• nitro,
  - •• cyano,
  - •• amino,
  - •• carbocyclic aryl,
  - •• carbocyclic aryl substituted by $C_{1-5}$ alkoxy,
  - •• $C_{1-5}$ alkoxy,
  - •• $C_{1-5}$ alkoxy substituted by substituent(s) independently selected from the group consisting of:

            - halogen,
            - hydroxy, and
            - carboxy,

        - $C_{1-5}$ alkyl, and
        - $C_{1-5}$ alkyl substituted by substituent(s) independently selected from the group consisting of:

            - halogen,
            - hydroxy, and
            - carboxy,

- $C_{1-5}$ alkylcarbonylamino,
- $C_{1-5}$ alkylcarbonylamino substituted by substituent(s) independently selected from the group consisting of:

    - $C_{1-5}$ alkylcarbonylamino,
    - carbocyclic arylcarbonylamino, and
    - heterocyclyl,

- $C_{1-5}$ alkoxycarbonylamino,
- carbocyclic arylcarbonylamino,
- heterocyclyl carbonylamino,
- carbocyclic arylsulfonylamino,
- carbocyclic arylsulfonylamino substituted by substituent(s) independently selected from the group consisting of:

    - nitro,
    - $C_{1-5}$ alkyl,
    - mono-$C_{1-5}$ alkylamino, and
    - di-$C_{1-5}$ alkylamino,

- $C_{1-5}$ alkylthio,
- $C_{1-5}$ alkylthio substituted by substituent(s) independently selected from the group consisting of:

    - mono-carbocyclic arylaminocarbonyl,
    - mono-carbocyclic arylaminocarbonyl substituted by halogen,
    - di-carbocyclic arylaminocarbonyl,
    - di-carbocyclic arylaminocarbonyl substituted by halogen,
    - mono-carbocyclic arylamino,
    - mono-carbocyclic arylamino substituted by halogen,
    - di-carbocyclic arylamino,
    - di-carbocyclic arylamino substituted by halogen,
    - carbocyclic aryl, and
    - carbocyclic aryl substituted by substituent(s) independently selected from the group consisting of:

        - halogen, and
        - $C_{1-5}$ alkoxy,

- carbocyclic arylthio,
- carbocyclic arylthio substituted by substituent(s) independently selected from the group consisting of:

    - halogen,
    - $C_{1-5}$ alkyl, and
    - $C_{1-5}$ alkyl substituted by halogen,

- carbocyclic arylsulfinyl,
- carbocyclic arylsulfinyl substituted by substituent(s) independently selected from the group consisting of:

    - halogen,

        - $C_{1-5}$ alkyl, and
        - $C_{1-5}$ alkyl substituted by halogen,

    - carbocyclic arylsulfonyl,
    - carbocyclic arylsulfonyl substituted by substituent(s) independently selected from the group consisting of:

        - halogen,
        - $C_{1-5}$ alkyl, and
        - $C_{1-5}$ alkyl substituted by halogen,

    - heterocyclylthio,
    - heterocyclylthio substituted by substituent(s) independently selected from the group consisting of:

        - nitro, and
        - $C_{1-5}$ alkyl,

    - $C_{3-6}$ cycloalkyl,
    - $C_{3-6}$ cycloalkyl substituted by $C_{1-5}$ alkyl,
    - $C_{3-6}$ cycloalkyl substituted by carbocyclic aryl,
    - $C_{3-6}$ cycloalkenyl,
    - carbocyclyl,
    - carbocyclyl substituted by substituent(s) independently selected from the group consisting of:

        - halogen,
        - $C_{1-5}$ alkyl,
        - $C_{1-5-}$ alkoxy,
        - $C_{2-5}$ alkenyl, and
        - $C_{2-5}$ alkenyl substituted by substituent(s) independently selected from the group consisting of:

            - carbocyclic aryl, and
            - carbocyclic aryl substituted by $C_{1-5}$ alkylsulfinyl,

    - carbocyclic aryl,
    - carbocyclic aryl substituted by substituent(s) independently selected from the group consisting of:

        - halogen,
        - hydroxy,
        - carboxy,
        - carbamoyl,
        - cyano,
        - nitro,
        - amino,
        - $C_{1-5}$ alkylcarbonylamino,
        - $C_{3-6}$ cycloalkylcarbonylamino,
        - $C_{1-5}$ alkyl,
        - $C_{1-5}$ alkyl substituted by substituent(s) independently selected from the group consisting of:

            - halogen,
            - hydroxy,
            - carboxy,
            - carbamoyl,
            - oxo,
            - carbocyclic aryl,
            - heterocyclyl,
            - mono-carbocyclic arylamino,
            - di-carbocyclic arylamino,
            - mono-carbocyclic arylamino substituted by substituent(s) independently selected from the group consisting of:

- •••• halogen,
- •••• nitro,
- •••• $C_{1-5}$ alkyl,
- •••• $C_{1-5}$ alkoxy, and
- •••• $C_{1-5}$ alkoxy substituted by halogen,

- ••• di-carbocyclic arylamino substituted by substituent(s) independently selected from the group consisting of:

- •••• halogen,
- •••• nitro,
- •••• $C_{1-5}$ alkyl,
- •••• $C_{1-5}$ alkoxy, and
- •••• $C_{1-5}$ alkoxy substituted by halogen,

- •• $C_{2-5}$ alkenyl,
- •• $C_{1-5}$ alkoxy,
- •• $C_{1-5}$ alkoxy substituted by substituent(s) independently selected from the group consisting of:

- ••• halogen, and
- ••• carbocyclic aryl,

- •• carbocyclic aryloxy,
- •• $C_{1-5}$ alkoxycarbonyl,
- •• $C_{1-5}$ alkylcarbonyloxy,
- •• mono-$C_{1-5}$ alkylamino,
- •• di-$C_{1-5}$ alkylamino,
- •• mono-carbocyclic arylamino,
- •• mono-carbocyclic arylamino substituted by halogen,
- •• di-carbocyclic arylamino,
- •• di-carbocyclic arylamino substituted by halogen,
- •• mono-carbocyclic arylaminocarbonyl,
- •• mono-carbocyclic arylaminocarbonyl substituted by substituent(s) selected from the group consisting of:

- ••• halogen,
- ••• nitro,
- ••• $C_{1-5}$ alkyl,
- ••• $C_{1-5}$ alkoxy, and
- ••• $C_{1-5}$ alkoxy substituted by halogen,

- •• di-carbocyclic arylaminocarbonyl,
- •• di-carbocyclic arylaminocarbonyl substituted by substituent(s) selected from the group consisting of:

- ••• halogen,
- ••• nitro,
- ••• $C_{1-5}$ alkyl,
- ••• $C_{1-5}$ alkoxy, and
- ••• $C_{1-5}$ alkoxy substituted by halogen,

- •• mercapto,
- •• $C_{1-5}$ alkylthio,
- •• $C_{1-5}$ alkylthio substituted by halogen,
- •• $C_{1-5}$ alkylsulfonyl,
- •• $C_{3-6}$ cycloalkyl,
- •• carbocyclic aryl, and
- •• heterocyclyl,

- heterocyclyl, and
- heterocyclyl substituted by substituent(s) independently selected from the group consisting of:
  - •• halogen,
  - •• hydroxy,
  - •• carboxy,
  - •• carbamoyl,
  - •• cyano,
  - •• nitro,
  - •• amino,
  - •• $C_{1-5}$ alkyl,
  - •• $C_{1-5}$ alkyl substituted by substituent(s) independently selected from the group consisting of:
    - ••• halogen,
    - ••• hydroxy,
    - ••• carboxy, and
    - ••• carbamoyl,
  - •• $C_{1-5}$ alkyl substituted by carbocyclic aryl,
  - •• $C_{1-5}$ alkoxy,
  - •• $C_{1-5}$ alkoxy substituted by halogen,
  - •• $C_{1-5}$ alkoxy substituted by carbocyclic aryl,
  - •• carbocyclic aryl, and
  - •• carbocyclic aryl substituted by halogen,

(ii) $C_{2-8}$ alkenyl, and
$C_{2-8}$ alkenyl substituted by substituent(s) independently selected from the group consisting of:

- halogen,
- oxo,
- $C_{1-5}$ alkoxy,
- $C_{1-5}$ alkoxy substituted by carbocyclic aryl,
- carbocyclic aryl,
- carbocyclic aryl substituted by substituent(s) independently selected from the group consisting of:
  - •• halogen,
  - •• hydroxy,
  - •• nitro,
  - •• $C_{1-5}$ alkyl,
  - •• $C_{1-5}$ alkyl substituted by halogen,
  - •• $C_{1-5}$ alkoxy, and
  - •• $C_{1-5}$ alkoxy substituted by halogen,
- heterocyclyl, and
- heterocyclyl substituted by substituent(s) independently selected from the group consisting of:
  - •• hydroxy,
  - •• nitro,
  - •• $C_{1-5}$ alkyl, and
  - •• $C_{1-5}$ alkoxy,

(iii) $C_{2-5}$ alkynyl, and
$C_{2-5}$ alkynyl substituted by carbocyclic aryl,
(iv) $C_{3-12}$ cycloalkyl, and
$C_{3-12}$ cycloalkyl substituted by substituent(s) independently selected from the group consisting of:

- $C_{1-5}$ alkyl,
- $C_{1-5}$ alkyl substituted by substituent(s) independently selected from the group consisting of:
  - •• hydroxy,

  - •• oxo, and
  - •• carbocyclic aryl,

- mono-$C_{1-5}$ alkylamino,
- mono-$C_{1-5}$ alkylamino substituted by carbocyclic aryl,
- di-$C_{1-5}$ alkylamino,
- di-$C_{1-5}$ alkylamino substituted by carbocyclic aryl,
- carbocyclic arylcarbonylamino,
- carbocyclic aryl, and
- carbocyclic aryl substituted by substituent(s) independently selected from the group consisting of:

  - •• halogen,
  - •• $C_{1-5}$ alkoxy,
  - •• $C_{1-5}$ alkyl, and
  - •• $C_{1-5}$ alkyl substituted by halogen,

(v) $C_{3-6}$ cycloalkenyl, and
$C_{3-6}$ cycloalkenyl substituted by $C_{1-5}$ alkyl,
(vi) carbocyclyl, and
carbocyclyl substituted by substitutent(s) independently selected from the group consisting of:

- hydroxy, and
- nitro,

(vii) carbocyclic aryl, and
carbocyclic aryl substituted by substituent(s) independently selected from the group consisting of:

- halogen,
- hydroxy,
- cyano,
- nitro,
- $C_{1-10}$ alkyl,
- $C_{1-10}$ alkyl substituted by substituent(s) independently selected from the group consisting of:

  - •• halogen,
  - •• hydroxy,
  - •• carboxy,
  - •• carbamoyl,
  - •• oxo,
  - •• $C_{1-5}$ alkoxy,
  - •• carbocyclic aryloxy,
  - •• mono-$C_{1-5}$ alkylamino-N-oxy,
  - •• di-$C_{1-5}$ alkylamino-N-oxy,
  - •• mono-$C_{1-5}$ alkylamino,
  - •• di-$C_{1-5}$ alkylamino,
  - •• mono-$C_{1-5}$ alkylamino substituted by carbocyclic aryl,
  - •• di-$C_{1-5}$ alkylamino substituted by carbocyclic aryl,
  - •• mono-carbocyclic arylamino,
  - •• di-carbocyclic arylamino,
  - •• carbocyclylimino,
  - •• carbocyclylimino substituted by carbocyclic aryl,
  - •• mono-carbocyclic arylamino,
  - •• di-carbocyclic arylamino,
  - •• mono-carbocyclic arylamino substituted by $C_{1-5}$ alkoxy,
  - •• di-carbocyclic arylamino substituted by $C_{1-5}$ alkoxy,
  - •• mono-carbocyclic arylaminocarbonyl,
  - •• di-carbocyclic arylaminocarbonyl,
  - •• mono-carbocyclic arylaminocarbonyl substituted by $C_{1-5}$ alkoxy,

- •• di-carbocyclic arylaminocarbonyl substituted by $C_{1-5}$ alkoxy,
- •• carbocyclic aryl,
- •• carbocyclic aryl substituted by substituent(s) independently selected from the group consisting of:
  - ••• halogen,
  - ••• $C_{1-5}$ alkyl, and
  - ••• $C_{1-5}$ alkyl substituted by halogen,
- •• heterocyclyl, and
- •• heterocyclyl substituted by $C_{1-5}$ alkyl,

- $C_{2-5}$ alkenyl,
- $C_{2-5}$ alkenyl substituted by carbocyclic aryl,
- $C_{1-9}$ alkoxy,
- $C_{1-9}$ alkoxy substituted by substituent(s) independently selected from the group consisting of:
  - •• hydroxy,
  - •• halogen,
  - •• carboxy,
  - •• mono-$C_{1-5}$ alkylamino,
  - •• di-$C_{1-5}$ alkylamino,
  - •• carbocyclic aryl,
  - •• halogenated carbocyclic aryl,
  - •• heterocyclyl,
  - •• heterocyclyl substituted by substituent(s) independently selected from the group consisting of:
    - ••• halogen,
    - ••• heterocyclyl, and
    - ••• heterocyclyl substituted by substituent(s) independently selected from the group consisting of:
      - •••• halogen,
      - •••• $C_{1-5}$ alkyl, and
      - •••• $C_{1-5}$ alkyl substituted by halogen,
- $C_{2-5}$ alkenyloxy,
- $C_{3-6}$ cycloalkoxy,
- $C_{1-5}$ alkylcarbonyloxy,
- carbocyclic aryloxy,
- carbocyclic aryloxy substituted by substituent(s) independently selected from the group consisting of:
  - •• halogen,
  - •• hydroxy,
  - •• carboxy,
  - •• carbamoyl,
  - •• cyano,
  - •• nitro,
  - •• amino,
  - •• $C_{1-5}$ alkyl,
  - •• $C_{1-5}$ alkyl substituted by substituent(s) independently selected from the group consisting of:
    - ••• halogen,
    - ••• hydroxy,
    - ••• carboxy, and
    - ••• carbamoyl,
  - •• $C_{1-5}$ alkoxy, and
  - •• $C_{1-5}$ alkoxy substituted by halogen,

- heterocyclyloxy,
- heterocyclyloxy substituted by substituent(s) independently selected from the group consisting of:
  - halogen,
  - hydroxy,
  - carboxy,
  - carbamoyl,
  - cyano,
  - nitro,
  - amino,
  - $C_{1-5}$ alkyl,
  - $C_{1-5}$ alkyl substituted by substituent(s) independently selected from the group consisting of:
    - halogen,
    - hydroxy,
    - carboxy, and
    - carbamoyl,
  - $C_{1-5}$ alkoxy, and
  - $C_{1-5}$ alkoxy substituted by halogen,
- (carbocyclic aryl)$S(O)_2O$,
- carboxy,
- carbamoyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- mono-carbocyclic arylaminocarbonyl,
- di-carbocyclic arylaminocarbonyl,
- mono-carbocyclic arylaminocarbonyl substituted by $C_{1-5}$ alkyl,
- di-carbocyclic arylaminocarbonyl substituted by $C_{1-5}$ alkyl,
- amino,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- mono-carbocyclic arylamino,
- di-carbocyclic arylamino,
- $C_{1-5}$ alkylcarbonylamino,
- $C_{3-6}$ cycloalkylcarbonylamino,
- $C_{2-5}$ alkynylcarbonylamino,
- $C_{2-5}$ alkynylcarbonylamino substituted by carbocyclic aryl,
- $C_{1-5}$ alkoxycarbonylamino,
- carbocyclic arylsulfonylamino,
- carbocyclic arylsulfonylamino substituted by $C_{1-5}$ alkyl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- carbocyclic aryl azo,
- carbocyclic aryl azo substituted by mono-$C_{1-5}$ alkylamino,
- carbocyclic aryl azo substituted by di-$C_{1-5}$ alkylamino,
- $C_{1-5}$ alkylthio,
- $C_{1-5}$ alkylthio substituted by halogen,
- carbocyclic arylthio,
- carbocyclic arylthio substituted by substituent(s) independently selected from the group consisting of:

  - •• halogen,
  - •• nitro,
  - •• cyano, and
  - •• $C_{1-5}$ alkyl,

- • aminosulfonyl,
- • heterocyclylthio,
- • $C_{1-5}$ alkylsulfonyl,
- • mono-$C_{1-5}$ alkylaminosulfonyl,
- • di-$C_{1-5}$ alkylaminosulfonyl,
- • heterocyclylsulfonyl,
- • $C_{3-6}$ cycloalkyl,
- • $C_{3-6}$ cycloalkyl substituted by $C_{1-5}$ alkyl,
- • carbocyclic aryl,
- • carbocyclic aryl substituted by substituent(s) independently selected from the group consisting of:

  - •• $C_{1-7}$ alkyl, and
  - •• $C_{1-7}$ alkyl substituted by halogen,

- • heterocyclyl, and
- • heterocyclyl substituted by substituent(s) independently selected from the group consisting of:

  - •• $C_{1-5}$ alkyl,
  - •• carbocyclic aryl, and
  - •• halogenated carbocyclic aryl,

- • $C_{1-5}$ alkoxycarbonyl substituted by carbocyclic aryl, and

(viii) heterocyclyl, and
heterocyclyl substituted by substituent(s) independently selected from the group consisting of:

- • halogen,
- • hydroxy,
- • carboxy,
- • carbamoyl,
- • cyano,
- • nitro,
- • amino,
- • $C_{1-5}$ alkyl,
- • $C_{1-5}$ alkyl substituted by substituent(s) independently selected from the group consisting of:

  - •• halogen,
  - •• hydroxy,
  - •• carboxy,
  - •• carbamoyl,
  - •• oxo,
  - •• $C_{1-5}$ alkylcarbonyloxy,
  - •• carbocyclic arylcarbonylamino,
  - •• carbocyclic arylcarbonylamino substituted by halogen,
  - •• $C_{1-5}$ alkoxycarbonyl,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by substituent(s) independently selected from the group consisting of:

    - ••• halogen, and
    - ••• nitro,

- •• heterocyclyl, and
- •• heterocyclyl substituted by substituent(s) independently selected from the group consisting of:

  - ••• halogen,
  - ••• $C_{1-5}$ alkyl, and
  - ••• $C_{1-5}$ alkyl substituted by halogen,

- • $C_{1-5}$ alkoxy,
- • $C_{1-5}$ alkoxy substituted by halogen,
- • $C_{1-5}$ alkoxy substituted by carbocyclic aryl,
- • carbocyclic aryloxy,
- • carbocyclic aryloxy substituted by substituent(s) independently selected from the group consisting of:

  - •• halogen,
  - •• nitro,
  - •• cyano,
  - •• hydroxy,
  - •• carboxy,
  - •• carbamoyl,
  - •• amino,
  - •• $C_{1-5}$ alkyl,
  - •• $C_{1-5}$ alkyl substituted by substituent(s) independently selected from the group consisting of:

    - ••• halogen,
    - ••• hydroxy,
    - ••• carboxy, and
    - ••• carbamoyl,

  - •• mono-$C_{1-5}$ alkylamino,
  - •• di-$C_{1-5}$ alkylamino,
  - •• $C_{1-5}$ alkylcarbonylamino,
  - •• $C_{3-6}$ cycloalkycarbonylamino,
  - •• $C_{1-5}$ alkoxy,
  - •• $C_{1-5}$ alkoxy substituted by halogen,
  - •• $C_{3-6}$ cycloalkyl,
  - •• $C_{2-5}$ alkenyl,
  - •• $C_{2-5}$alkynyl,
  - •• carboxy,
  - •• $C_{1-5}$ alkoxycarbonyl,
  - •• mono-$C_{1-5}$ alkylaminocarbonyl,
  - •• di-$C_{1-5}$ alkylaminocarbonyl,
  - •• mono-$C_{3-6}$ cycloalkylaminocarbonyl,
  - •• di-$C_{3-6}$ cycloalkylaminocarbonyl,
  - •• mono-$C_{1-5}$ alkylaminocarbonylamino,
  - •• di-$C_{1-5}$ alkylaminocarbonylamino,
  - •• mono-$C_{3-6}$ cycloalkylaminocarbonylamino,
  - •• di-$C_{3-6}$ cycloalkylaminocarbonylamino,
  - •• $C_{1-5}$ alkylthio,
  - •• $C_{1-5}$ alkylthio substituted by halogen,
  - •• $C_{1-5}$ alkylsulfinyl,
  - •• $C_{1-5}$ alkylsulfinyl substituted by halogen,
  - •• $C_{1-5}$ alkylsulfonyl, and
  - •• $C_{1-5}$ alkylsulfonyl substituted by halogen,

- • heterocyclyloxy,
- • heterocyclyloxy substituted by substituent(s) independently selected from the group consisting of:

  - •• halogen,

- •• nitro,
- •• hydroxy,
- •• carboxy,
- •• carbamoyl,
- •• cyano,
- •• amino,
- •• $C_{1-5}$ alkyl,
- •• $C_{1-5}$ alkyl substituted by substituent(s) independently selected from the group consisting of:

    - ••• halogen,
    - ••• hydroxy,
    - ••• carboxy, and
    - ••• carbamoyl,

- •• $C_{1-5}$ alkoxy, and
- •• $C_{1-5}$ alkoxy substituted by halogen,

- • mono-$C_{1-5}$ alkylamino,
- • di-$C_{1-5}$ alkylamino,
- • mono-carbocyclic arylamino,
- • mono-carbocyclic arylamino substituted by halogen,
- • $C_{1-5}$ alkylcarbonylamino,
- • $C_{1-5}$ alkylthio,
- • $C_{2-5}$ alkenylthio,
- • carbocyclic arylthio,
- • carbocyclic arylthio substituted by halogen,
- • carbocyclic arylthio substituted by $C_{1-5}$ alkoxycarbonyl,
- • heterocyclylthio,
- • heterocyclylthio substituted by $C_{1-5}$ alkyl,
- • $C_{1-5}$ alkylsulfinyl,
- • $C_{1-5}$ alkylsulfonyl,
- • carbocyclic arylsulfinyl,
- • carbocyclic arylsulfinyl substituted by halogen,
- • carbocyclic arylsulfonyl,
- • carbocyclic arylsulfonyl substituted by substituent(s) independently selected from the group consisting of:

    - •• halogen,
    - •• $C_{1-5}$ alkoxy,
    - •• $C_{1-5}$ alkyl, and
    - •• $C_{1-5}$ alkyl substituted by halogen,

- • $C_{1-5}$ alkoxycarbonyl,
- • $C_{1-5}$ alkoxycarbonyl substituted by carbocyclic aryl,
- • carbocyclic aryl,
- • carbocyclic aryl substituted by substituent(s) independently selected from the group consisting of:

    - •• halogen,
    - •• nitro,
    - •• $C_{1-5}$ alkyl,
    - •• $C_{1-5}$ alkyl substituted by halogen,
    - •• $C_{1-5}$ alkoxy, and
    - •• $C_{1-5}$ alkoxy substituted by halogen,

- • heterocyclyl, and
- • heterocyclyl substituted by substituent(s) independently selected from the group consisting of:

    - •• halogen,
    - •• $C_{1-5}$ alkyl,

- •• $C_{1-5}$ alkyl substituted by halogen,
- •• $C_{1-5}$ alkoxy, and
- •• $C_{1-5}$ alkoxycarbonyl;

$R_2$ is selected from the group consisting of:

hydrogen, halogen, hydroxy, carboxy, carbamoyl, amino, $C_{1-5}$ alkyl, $C_{1-5}$ alkyl substituted by halogen, $C_{1-5}$ alkyl substituted by hydroxy, $C_{1-5}$ alkyl substituted by carboxy, $C_{1-5}$ alkyl substituted by carbamoyl, $C_{1-5}$ alkoxy, $C_{1-5}$ alkoxy substituted by halogen, $-NHNH_2$, -NHNHBoc, $-N(R_{2a})(R_{2b})$, morpholino, 4-acetyl-piperazyl, or 4-phenyl-piperazyl,

wherein $R_{2a}$ is hydrogen or $C_{1-5}$ alkyl and $R_{2b}$ is $C_{1-5}$ alkyl, $C_{3-6}$ cycloalkyl, or $C_{1-5}$ alkyl substituted by substituent (s) independently selected from the group consisting of:

- halogen,
- hydroxy,
- carboxy,
- carbamoyl,
- $C_{1-5}$ alkoxy,
- amino,
- -NHBoc,
- $C_{3-6}$ cycloalkyl,
- carbocyclic aryl,
- carbocyclic aryl substituted by substituent(s) independently selected from the group consisting of:
  - •• halogen,
  - •• $C_{1-5}$ alkyl,
  - •• $C_{1-5}$ alkoxy, and
  - •• $-SO_2NH_2$,
- heterocyclyl, and

$C_{3-6}$ cycloalkyl, carbocyclic aryl, carbocyclic aryl substituted by substituent(s) independently selected from the group consisting of:

- halogen,
- $C_{1-5}$ alkyl,
- $C_{1-5}$ alkoxy, and
- a group of Formula (V):

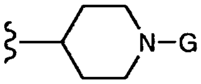

**(V)**

wherein Boc is carbamic acid *tert*-butyl ester and G is $C_{1-5}$ alkyl or $C_{1-5}$ alkyl substituted by substituent(s) independently selected from the group consisting of:

- carbocyclic aryl,
- halogenated carbocyclic aryl, and
- carbocyclic aryl substituted by $C_{1-5}$ alkoxy;

or $R_2$ is methylamino or dimethylamino when Q is Formula (II) and Y is a single bond or $-CH_2-$;
Each T is independently selected from the group consisting of halogen, hydroxy, carboxy, carbamoyl, amino, cyano, nitro, $C_{1-5}$ alkyl, $C_{1-5}$ alkyl substituted by halogen, $C_{1-5}$ alkyl substituted by hydroxy, $C_{1-5}$ alkyl substituted by carboxy, $C_{1-5}$ alkyl substituted by carbamoyl, $C_{2-5}$ alkenyl, $C_{2-5}$ alkynyl, $C_{3-6}$ cycloalkyl, $C_{1-5}$ alkoxy, $C_{1-5}$ alkoxy sub-

stituted by halogen, carbocyclic aryl, heterocyclyl, and $-N(R_{2a})(R_{2b})$;
p is 0,1, 2, 3, 4 or 5;
L is selected from the group consisting of Formulae (VI) to (XXI):

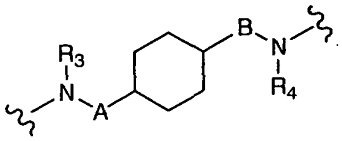
(VI)
,
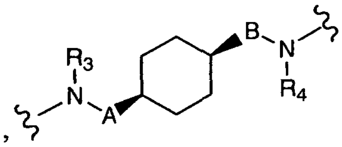
(VII)
,
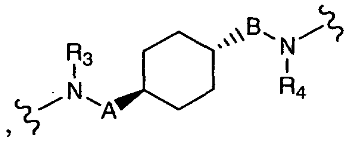
(VIII)
,

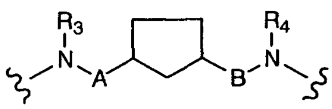
(IX)
,
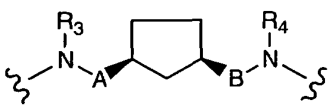
(X)
,
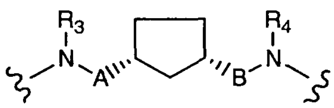
(XI)
,

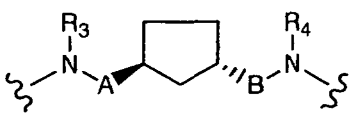
(XII)
,
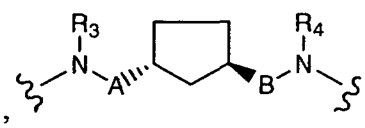
(XIII)

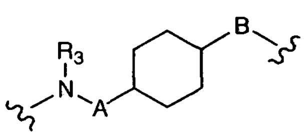
(XIV)
,
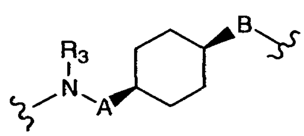
(XV)
,
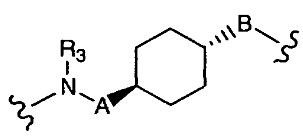
(XVI)
,

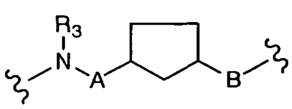
(XVII)
,
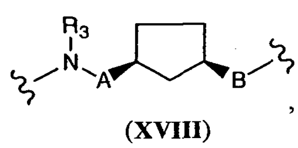
(XVIII)
,
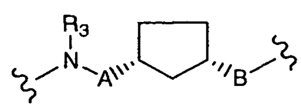
(XIX)
,

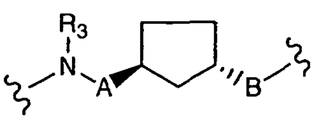
(XX)
,
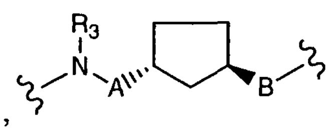
(XXI)
;

wherein $R_3$ and $R_4$ are independently hydrogen or $C_{1-5}$ alkyl; and A and B are independently a single bond, $-CH_2-$, or $-(CH_2)_2-$;
and
Y represents:

(i) $-C(O)NR_5-$, $-C(S)NR_5-$, $-C(O)O-$, $-S(O)_2-$, $-C(O)-$, $-C(S)-$, a single bond, or $-CH_2-$ when L is selected from the group consisting of Formulae (VI) to (XIIII); or

(ii) $-C(O)NR_5-$, $-C(S)NR_5-$, $-C(O)O-$ or $-OC(O)-$ when L is selected from the group consisting of Formulae (XIV) to (XXI);

wherein $R_5$ is hydrogen or $C_{1-5}$ alkyl, or when Y is $-C(O)NR_5-$ then $R_5$ and $R_1$ together with the nitrogen they are bonded form a heterocyclyl group;

wherein carbocyclic aryl is phenyl, naphthyl, anthranyl, phenanthryl, or biphenyl;

carbocyclyl is 10,11-dihydro-5-oxo-dibenzo[a,d]cycloheptyl, 1-oxo-indanyl, 7,7-dimethyl-2-oxo-bicyclo[2.2.1]heptyl, 9*H*-fluorenyl, 9-oxo-fluorenyl, acenaphthyl, anthraquinonyl, *C*-fluoren-9-ylidene, indanyl, indenyl, 1,2,3,4-tetrahydro-naphthyl, or bicyclo[2.2.1]heptenyl;

heterocyclyl is 1,2,3,4-tetrahydro-isoquinolyl, 1,2,3-thiadiazolyl, 1,2,3-triazolyl, 1,2-dihydro-3-oxo-pyrazolyl, 1,3,4-thiadiazolyl, 1,3-dioxo-isoindolyl, 1,3-dioxolanyl, 1*H*-indolyl, 1*H*-pyrrolo[2,3-c]pyridyl, 1*H*-pyrrolyl, 1-oxo-3*H*-isobenzofuranyl, 2,2',5',2"-terthiophenyl, 2,2'-bithiophenyl, 2,3-dihydro-1-oxo-isoindolyl, 2,3-dihydro-benzo[1,4]dioxinyl, 2,3-dihydro-benzofuryl, 2,4-dihydro-3-oxo-pyrazolyl, 2*H*-benzopyranyl, 2-oxo-benzopyranyl, 2-oxo-pyrrolidinyl, 3,4-dihydro-2*H*-benzo[1,4]oxazinyl, 3,4-dihydro-2*H*-benzo[b][1,4]dioxepinyl, 4*H*-benzo[1,3]dioxinyl, 4*H*-benzopyranyl, 4-oxo-1,5,6,7-tetrahydro-indolyl, 4-oxo-3,4-dihydro-phthalazinyl, 4-oxo-benzopyranyl, 9,10,10-trioxo-thioxanthenyl, 9*H*-carbazolyl, 9H-xanthenyl, azetidinyl, benzimidazolyl, benzo[1,3]dioxolyl, benzo[2,1,3]oxadiazolyl, benzo[1,2,5]oxadiazolyl, benzo[b]thienyl, benzofuryl, benzothiazolyl, cinnolyl, furyl, imidazo[2,1-b]thiazolyl, imidazolyl, isoxazolyl, morpholino, morpholinyl, oxazolyl, oxolanyl, piperazyl, piperidyl, piridyl, pyrazolo[5,1-b]thiazolyl, pyrazolyl, pyrazinyl, pyridyl, pyrimidyl, pyrrolidyl, quinolyl, quinoxalyl, thiazolidyl, thiazolyl, thienyl, thiolanyl, 2,3-dihydro-benzofuryl, tetrahydro-thienyl, or benzofuranyl; and

halogen is fluoro, chloro, bromo, or iodo;

or a pharmaceutically acceptable salt, hydrate, or solvate thereof.

One aspect of the present invention pertains to pharmaceutical compositions comprising at least one compound, as described herein, in combination with a pharmaceutically acceptable carrier.

One aspect of the present invention pertains to methods for the prophylaxis or treatment of improving memory function, sleeping and arousal, anxiety, depression, mood disorders, seizure, obesity, diabetes, appetite and eating disorders, cardiovascular disease, hypertension, dyslipidemia, myocardial infarction, binge eating disorders including bulimia, anorexia, mental disorders including manic depression, schizophrenia, delirium, dementia, stress, cognitive disorders, attention deficit disorder, substance abuse disorders and dyskinesias including Parkinson's disease, epilepsy, and addiction comprising administering to an individual suffering from said condition a therapeutically effective amount of a compound, as described herein, or a pharmaceutical composition thereof.

One aspect of the present invention pertains to methods for the prophylaxis or treatment of an eating disorder, obesity or an obesity related disorder comprising administering to an individual suffering from the condition a therapeutically effective amount of a compound, as described herein, or a pharmaceutical composition thereof.

One aspect of the present invention pertains to methods for the prophylaxis or treatment of anxiety, depression, schizophrenia, addiction, or epilepsy comprising administering to an individual suffering from the condition a therapeutically effective amount of a compound, as described herein, or a pharmaceutical composition.

One aspect of the present invention pertains to compounds of the present invention, as described herein, or a pharmaceutical composition thereof, for use in a method of treatment of the human or animal body by therapy.

One aspect of the present invention pertains to compounds of the present invention, as described herein, or a pharmaceutical composition thereof, for use in a method of prophylaxis or treatment of an eating disorder, obesity or an obesity related disorder of the human or animal body by therapy.

One aspect of the present invention pertains to compounds of the present invention, as described herein, or a pharmaceutical composition thereof, for use in a method of prophylaxis or treatment of anxiety, depression, schizophrenia, addiction, or epilepsy of the human or animal body by therapy.

One aspect of the present invention pertains to compounds of the present invention, as described herein, for the manufacture of a medicament for use in the prophylaxis or treatment of an eating disorder, obesity or obesity related disorders.

One aspect of the present invention pertains to compounds of the present invention, as described herein, for the manufacture of a medicament for use in the prophylaxis or treatment of anxiety, depression, schizophrenia, addiction, or epilepsy.

One aspect of the present invention pertains to methods of decreasing food intake of an individual comprising administering to the individual a therapeutically effective amount of a compound, as described herein, or a pharmaceutical composition thereof.

One aspect of the present invention pertains to methods of inducing satiety in an individual comprising administering to said individual a therapeutically effective amount of a compound, as described herein, or a pharmaceutical composition thereof.

One aspect of the present invention pertains to methods of controlling or reducing weight gain in an individual comprising administering to said individual a therapeutically effective amount of a compound, as described herein, or a pharmaceutical composition thereof.

One aspect of the present invention pertains to methods of modulating a MCH receptor in an individual comprising contacting the receptor with a compound, as described herein. In some embodiments, the compound is an antagonist. In some embodiments, the modulation of the MCH receptor is for the prophylaxis or treatment of an eating disorder, obesity or obesity related disorder. In some embodiments, the modulation of the MCH receptor reduces food intake of the individual. In some embodiments, the modulation of the MCH receptor induces satiety in the individual. In some embodiments, the modulation of the MCH receptor controls or reduces weight gain of the individual. In some embodiments, the modulation of the MCH receptor is for prophylaxis or treatment of anxiety, depression, schizophrenia, addiction, or epilepsy.

In some embodiments, the individual is a mammal.

In some embodiments, the mammal is a human.

In some embodiments, the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

One aspect of the present invention pertains to methods of producing a pharmaceutical composition comprising admixing a compound, as described herein, and a pharmaceutically acceptable carrier.

This application claims priority to US Provisional Patent Applications, Serial No. 60/458,530, filed March 31, 2003; Serial No. 60/495,911, filed August 19, 2003; Serial 60/510,186, filed October 9, 2003; and Serial No. 60/530,360, filed December 16, 2003; all of which are incorporated herein by reference in their entirety.

**Detailed Description of the Invention**

One aspect of the present invention relates to certain substituted heterocyclic compounds represented by Formula (I):

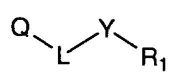

**(I)**

or a pharmaceutically acceptable salt, hydrate or solvate thereof, wherein Q, L, Y, and $R_1$ are as described herein, *supra* and *infra.*

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable subcombination.

In some embodiments of the present invention, $R_2$ is selected from the group consisting of:

hydrogen, halogen, hydroxy, carboxy, carbamoyl, amino, $C_{1-5}$ alkyl substituted by hydroxy, $C_{1-5}$ alkyl substituted by carboxy, $C_{1-5}$ alkyl substituted by carbamoyl, $C_{1-5}$ alkoxy, $C_{1-5}$ alkoxy substituted by halogen, $-NHNH_2$, -NHN-HBoc, $-N(R_{2a})(R_{2b})$, morpholino, 4-acetyl-piperazyl, or 4-phenyl-piperazyl,

wherein $R_{2a}$ is hydrogen or $C_{1-5}$ alkyl and $R_{2b}$ is $C_{1-5}$ alkyl, $C_{3-6}$ cycloalkyl, or $C_{1-5}$ alkyl substituted by substituent (s) independently selected from the group consisting of:

- halogen,
- hydroxy,
- carboxy,
- carbamoyl,
- $C_{1-5}$ alkoxy,
- amino,
- -NHBoc,
- $C_{3-6}$ cycloalkyl,

- carbocyclic aryl,
- carbocyclic aryl substituted by substituent(s) independently selected from the group consisting of:
  - halogen,
  - $C_{1-5}$ alkyl,
  - $C_{1-5}$ alkoxy, and
  - $-SO_2NH_2$,
- heterocyclyl, and

$C_{3-6}$ cycloalkyl, carbocyclic aryl, carbocyclic aryl substituted by substituent(s) independently selected from the group consisting of:

- halogen,
- $C_{1-5}$ alkyl,
- $C_{1-5}$ alkoxy, and
- a group of Formula (V):

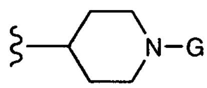

**(V)**

wherein Boc is carbamic acid *tert*-butyl ester and G is $C_{1-5}$ alkyl or $C_{1-5}$ alkyl substituted by substituent(s) independently selected from the group consisting of:

- carbocyclic aryl,
- halogenated carbocyclic aryl, and
- carbocyclic aryl substituted by $C_{1-5}$ alkoxy.

In some embodiments of the present invention, $R_2$ is $-N(R_{2a})(R_{2b})$, wherein $R_{2a}$ is hydrogen or $C_{1-5}$ alkyl and $R_{2b}$ is $C_{1-5}$ alkyl, $C_{3-6}$ cycloalkyl, or $C_{1-5}$ alkyl substituted by substituent(s) independently selected from the group consisting of:

- halogen,
- hydroxy,
- carboxy,
- carbamoyl,
- $C_{1-5}$ alkoxy,
- amino,
- -NHBoc,
- $C_{3-6}$ cycloalkyl,
- carbocyclic aryl,
- carbocyclic aryl substituted by substituent(s) independently selected from the group consisting of:
  - halogen,
  - $C_{1-5}$ alkyl,
  - $C_{1-5}$ alkoxy, and
  - $-SO_2NH_2$,
- heterocyclyl, and

$C_{3-6}$ cycloalkyl, carbocyclic aryl, carbocyclic aryl substituted by substituent(s) independently selected from the group consisting of:

- halogen,

- $C_{1-5}$ alkyl,
- $C_{1-5}$ alkoxy, and
- a group of Formula (V):

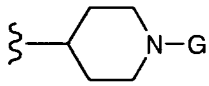

**(V)**

wherein Boc is carbamic acid *tert-butyl* ester and G is $C_{1-5}$ alkyl or $C_{1-5}$ alkyl substituted by substituent(s) independently selected from the group consisting of:

- carbocyclic aryl,
- halogenated carbocyclic aryl, and
- carbocyclic aryl substituted by $C_{1-5}$ alkoxy.

In some embodiments of the present invention, $R_2$ is $-N(R_{2a})(R_{2b})$, wherein $R_{2a}$ is hydrogen or $C_{1-5}$ alkyl and $R_{2b}$ is $C_{1-5}$ alkyl or $C_{3-6}$ cycloalkyl.

In some embodiments of the present invention, $R_1$ is selected from the group consisting of:

(i) $C_{1-8}$ alkyl, and
$C_{1-8}$ alkyl substituted by substituent(s) independently selected from the group consisting of:

- halogen,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by substituent(s) independently selected from the group consisting of:
    - •• halogen,
    - •• nitro,
    - •• $C_{1-5}$ alkyl, and
    - •• $C_{1-5}$ alkoxy,
- heterocyclyloxy,
- heterocyclyloxy substituted by $C_{1-5}$ alkyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by cyano,
- mono-$C_{1-5}$ alkylamino substituted by carbocyclic aryl,
- di-$C_{1-5}$ alkylamino,
- di-$C_{1-5}$ alkylamino substituted by cyano,
- di-$C_{1-5}$ alkylamino substituted by carbocyclic aryl,
- mono-carbocyclic arylamino,
- mono-carbocyclic arylamino substituted by halogen,
- mono-carbocyclic arylamino substituted by $C_{1-5}$ alkyl,
- di-carbocyclic arylamino,
- di-carbocyclic arylamino substituted by halogen,
- di-carbocyclic arylamino substituted by $C_{1-5}$ alkyl,
- $C_{1-5}$ alkoxycarbonylamino,
- carbocyclic arylcarbonylamino,
- carbocyclic arylsulfonylamino,

- carbocyclic arylsulfonylamino substituted $C_{1-5}$ alkyl,
- $C_{1-5}$ alkylthio,
- $C_{1-5}$ alkylthio substituted by substituent(s) independently selected from the group consisting of:

  - •• carbocyclic aryl,
  - •• carbocyclic aryl substituted by halogen, and
  - •• carbocyclic aryl substituted by $C_{1-5}$ alkoxy,

- carbocyclic arylthio,
- heterocyclylthio,
- heterocyclylthio substituted by nitro,
- heterocyclylthio substituted by $C_{1-5}$ alkyl,
- $C_{3-6}$ cycloalkyl,
- $C_{3-6}$ cycloalkenyl,
- carbocydyl,
- carbocyclyl substituted by substituent(s) independently selected from the group consisting of:

  - •• halogen,
  - •• $C_{1-5}$ alkyl,
  - •• $C_{1-5}$ alkoxy,
  - •• $C_{2-5}$ alkenyl, and
  - •• $C_{2-5}$ alkenyl substituted by substituent(s) independently selected from the group consisting of:

    - ••• carbocyclic aryl, and
    - ••• carbocyclic aryl substituted by $C_{1-5}$ alkylsulfinyl,

- carbocyclic aryl,
- carbocyclic aryl substituted by substituent(s) independently selected from the group consisting of:

  - •• halogen,
  - •• hydroxy,
  - •• nitro,
  - •• $C_{1-5}$ alkyl,
  - •• $C_{1-5}$ alkyl substituted by substituent(s) independently selected from the group consisting of:

    - ••• oxo,
    - ••• carbocyclic aryl, and
    - ••• heterocyclyl,

  - •• $C_{2-5}$ alkenyl,
  - •• $C_{1-5}$ alkoxy,
  - •• $C_{1-5}$ alkoxy substituted by halogen,
  - •• $C_{1-5}$ alkoxy substituted by carbocyclic aryl,
  - •• carbocyclic aryloxy,
  - •• mono-carbocyclic arylaminocarbonyl,
  - •• mono-carbocyclic arylaminocarbonyl substituted by halogen,
  - •• di-carbocyclic arylaminocarbonyl,
  - •• di-carbocyclic arylaminocarbonyl substituted by halogen,
  - •• carbocyclic aryl, and
  - •• heterocyclyl,

- heterocyclyl, and
- heterocyclyl substituted by substituent(s) independently selected from the group consisting of:

  - •• $C_{1-5}$ alkyl,
  - •• $C_{1-5}$ alkyl substituted by carbocyclic aryl,
  - •• $C_{1-5}$ alkoxy,
  - •• C1-5 alkoxy substituted by carbocyclic aryl,

- •• carbocyclic aryl, and
- •• carbocyclic aryl substituted by halogen,

(ii) $C_{2-7}$ alkenyl, and
$C_{2-7}$ alkenyl substituted by substituent(s) independently selected from the group consisting of:

- carbocyclic aryl,
- carbocyclic aryl substituted by substituent(s) independently selected from the group consisting of:
    - •• halogen,
    - •• nitro, and
    - •• $C_{1-5}$ alkoxy,

(iii) $C_{2-5}$ alkynyl, and
$C_{2-5}$ alkynyl substituted by carbocyclic aryl,
(iv) $C_{3-12}$ cycloalkyl, and
$C_{3-12}$ cycloalkyl substituted by substituent(s) independently selected from the group consisting of:

- $C_{1-5}$ alkyl,
- $C_{1-5}$ alkyl substituted by oxo,
- $C_{1-5}$ alkyl substituted by carbocyclic aryl, and
- carbocyclic aryl,

(v) carbocyclyl,
(vi) carbocyclic aryl, and
carbocyclic aryl substituted by substituent(s) independently selected from the group consisting of:

- halogen,
- hydroxy,
- cyano,
- nitro,
- carboxy,
- carbamoyl,
- $C_{1-10}$ alkyl,
- $C_{1-10}$ alkyl substituted by substituent(s) independently selected from the group consisting of:
    - •• halogen,
    - •• hydroxy,
    - •• oxo,
    - •• carbocyclic aryloxy,
    - •• carbocyclic aryl, and
    - •• carbocyclic aryl substituted by $C_{1-5}$ alkyl,
- $C_{1-7}$ alkoxy,
- $C_{1-7}$ alkoxy substituted by substituent(s) independently selected from the group consisting of:
    - •• halogen,
    - •• carbocyclic aryl, and
    - •• halogenated carbocyclic aryl,
- $C_{2-5}$ alkenyloxy,
- $C_{3-6}$ cycloalkoxy,
- carbocyclic aryloxy,
- carbocyclic aryloxy substituted by nitro,
- carbocyclic aryloxy substituted by $C_{1-5}$ alkoxy,
- $C_{1-5}$ alkoxycarbonyl,
- mono-$C_{1-5}$ alkylaminocarbonyl,
- di-$C_{1-5}$ alkylaminocarbonyl,

- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{2-5}$ alkynylcarbonylamino,
- $C_{2-5}$ alkynylcarbonylamino substituted by carbocyclic aryl,
- $C_{1-5}$ alkoxycarbonylamino,
- (carbocyclic aryl)NHC(O)NH,
- (carbocyclic aryl)NHC(O)NH substituted by $C_{1-5}$ alkoxy,
- (carbocyclic aryl)N-HC(O)NH substituted by haloganated $C_{1-5}$ alkoxy,
- carbocyclic aryl azo,
- carbocyclic aryl azo substituted by mono-$C_{1-5}$ alkylamino,
- carbocyclic aryl azo substituted by di-$C_{1-5}$ alkylamino,
- $C_{1-5}$ alkylthio,
- $C_{1-5}$ alkylthio substituted by halogen,
- carbocyclic arylthio,
- carbocyclic arylthio substituted by nitro,
- carbocyclic arylthio substituted by cyano,
- aminosulfonyl,
- mono-$C_{1-5}$ alkylaminosulfonyl,
- di-$C_{1-5}$ alkylaminosulfonyl,
- heterocyclylsulfonyl,
- $C_{3-6}$ cycloalkyl,
- $C_{3-6}$ cycloalkyl substituted by $C_{1-5}$ alkyl,
- carbocyclic aryl,
- heterocyclyl, and
- heterocyclyl substituted by substituent(s) independently selected from the group consisting of:

  - •• $C_{1-5}$ alkyl,
  - •• carbocyclic aryl, and
  - •• halogenated carbocyclic aryl,

(vii) heterocyclyl, and
heterocyclyl substituted by substituent(s) independently selected from the group consisting of:

- halogen,
- nitro,
- amino,
- hydroxy,
- $C_{1-5}$ alkyl,
- $C_{1-5}$ alkyl substituted by substituent(s) independently selected from the group consisting of:

  - •• halogen,
  - •• hydroxy,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by halogen, and
  - •• heterocyclyl,

- $C_{1-5}$ alkoxy,
- carbocyclic aryloxy,
- carbocyclic aryloxy substituted by halogen,
- carbocyclic aryloxy substituted by $C_{1-5}$ alkyl,

- carbocyclic aryloxy substituted by $C_{1-5}$ alkoxy,
- mono-$C_{1-5}$ alkylamino,
- di-$C_{1-5}$ alkylamino,
- $C_{1-5}$ alkylthio,
- $C_{2-5}$ alkenylthio,
- carbocyclic arylthio,
- carbocyclic arylthio substituted by $C_{1-5}$ alkoxycarbonyl,
- $C_{1-5}$ alkylsulfonyl,
- carbocyclic arylsulfonyl,
- carbocyclic arylsulfonyl substituted by $C_{1-5}$ alkyl,
- $C_{1-5}$ alkoxycarbonyl,
- $C_{1-5}$ alkoxycarbonyl substituted by carbocyclic aryl,
- carbocyclic aryl,
- carbocyclic aryl substituted by substituent(s) independently selected from the group consisting of:

  - •• halogen,
  - •• nitro,
  - •• $C_{1-5}$ alkyl, and
  - •• $C_{1-5}$ alkyl substituted by halogen,

- heterocyclyl;

wherein carbocyclic aryl is phenyl, naphthyl, or anthranyl;

carbocyclyl is 1,2,3,4-tetrahydronaphthyl, 1-oxo-indanyl, 9-fluorenyl, 9*H*-fluorenyl, 9-oxo-9*H*-fluorenyl, adamantly, bicyclo[2.2.1]heptenyl, bicyclo[2.2.1]heptyl, indanyl, indenyl, or menthyl;

heterocyclyl is 1,2,3-triazolyl, 1*H*-indolyl, 1*H*-pyrrolyl, 2,3-dihydro-1-oxo-isoindolyl, 2,3-dihydro-benzo[1,4]dioxinyl, 2,3-dihydro-benzofuryl, 2,4-dihydro-3-oxo-pyrazolyl, 2*H*-benzopyranyl, 2-oxo-benzopyranyl, 3,4-dihydro-2*H*-benzo[b][1,4]dioxepinyl, 4,5,6,7-tetrahydro-benzo[b]thienyl, 4*H*-benzo[1,3]dioxinyl, 4-oxo-1,5,6,7-tetrahydro-indolyl, 4-oxo-benzopyranyl, 9*H*-carbazolyl, 9*H*-xanthenyl, azetidinyl, benzo[1,3]dioxolyl, benzo[2,1,3]oxadiazolyl, benzo [1,2,5]oxadiazolyl, benzo[2,1,3]thiadiazolyl, benzo[b]thienyl, benzofuryl, benzothiazolyl, furyl, imidazo[2,1-b]thiazolyl, imidazolyl, isoxazolyl, morpholino, morpholinyl, oxazolyl, phenanthro[9,10-d]oxazolyl, piperidyl, pyrazolyl, pyridyl, pyrimidyl, quinolyl, quinoxalyl, tetrahydrofuryl, thiazolyl, or thienyl; and

halogen is fluoro, chloro, bromo, or iodo;

or a pharmaceutically acceptable salt, hydrate, or solvate thereof.

In some embodiments of the present invention, Q is Formula (II);

$R_1$ is selected from the group consisting of:

(i) $C_{1-8}$ alkyl, and
$C_{1-8}$ alkyl substituted by substituent(s) independently selected from the group consisting of:

- halogen,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- heterocyclyloxy,
- heterocyclyloxy substituted by $C_{1-5}$ alkyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by cyano,
- mono-$C_{1-5}$ alkylamino substituted by carbocyclic aryl,
- di-$C_{1-5}$ alkylamino,

- di-$C_{1-5}$ alkylamino substituted by cyano,
- di-$C_{1-5}$ alkylamino substituted by carbocyclic aryl,
- mono-carbocyclic arylamino,
- di-carbocyclic arylamino,
- $C_{1-5}$ alkoxycarbonylamino,
- carbocyclic arylcarbonylamino,
- carbocyclic arylsulfonylamino,
- carbocyclic arylsulfonylamino substituted $C_{1-5}$ alkyl,
- $C_{1-5}$ alkylthio,
- $C_{1-5}$ alkylthio substituted by substituent(s) independently selected from the group consisting of:
  - carbocyclic aryl,
  - carbocyclic aryl substituted by halogen, and
  - carbocyclic aryl substituted by $C_{1-5}$ alkoxy,
- carbocyclic arylthio,
- heterocyclylthio,
- heterocyclylthio substituted by $C_{1-5}$ alkyl,
- $C_{3-6}$ cycloalkyl,
- $C_{3-6}$ cycloalkenyl,
- carbocyclyl,
- carbocyclyl substituted by substituent(s) independently selected from the group consisting of:
  - halogen,
  - $C_{1-5}$ alkyl,
  - $C_{1-5}$ alkoxy,
  - $C_{2-5}$ alkenyl, and
  - $C_{2-5}$ alkenyl substituted by substituent(s) independently selected from the group consisting of:
    - carbocyclic aryl, and
    - carbocyclic aryl substituted by $C_{1-5}$ alkylsulfinyl,
- carbocyclic aryl,
- carbocyclic aryl substituted by substituent(s) independently selected from the group consisting of:
  - halogen,
  - hydroxy,
  - nitro,
  - $C_{1-5}$ alkyl,
  - $C_{1-5}$ alkyl substituted by substituent(s) independently selected from the group consisting of:
    - oxo,
    - carbocyclic aryl, and
    - heterocyclyl,
  - $C_{2-5}$ alkenyl,
  - $C_{1-5}$ alkoxy,
  - $C_{1-5}$ alkoxy substituted by halogen,
  - $C_{1-5}$ alkoxy substituted by carbocyclic aryl,
  - carbocyclic aryloxy,
  - mono-carbocyclic arylaminocarbonyl,
  - mono-carbocyclic arylaminocarbonyl substituted by halogen,
  - di-carbocyclic arylaminocarbonyl,
  - di-carbocyclic arylaminocarbonyl substituted by halogen,
  - carbocyclic aryl, and
  - heterocyclyl,
- heterocyclyl, and

- heterocyclyl substituted by substituent(s) independently selected from the group consisting of:

  - •• $C_{1-5}$ alkyl,
  - •• $C_{1-5}$ alkyl substituted by carbocyclic aryl,
  - •• $C_{1-5}$ alkoxy,
  - •• $C_{1-5}$ alkoxy substituted by carbocyclic aryl,
  - •• carbocyclic aryl, and
  - •• carbocyclic aryl substituted by halogen,

(ii) $C_{2-7}$ alkenyl, and
$C_{2-7}$ alkenyl substituted by substituent(s) independently selected from the group consisting of:

- carbocyclic aryl,
- carbocyclic aryl substituted by substituent(s) independently selected from the group consisting of:

  - •• halogen,
  - •• nitro, and
  - •• $C_{1-5}$ alkoxy,

(iii) $C_{2-5}$ alkynyl, and
$C_{2-5}$ alkynyl substituted by carbocyclic aryl,
(iv) $C_{3-6}$ cycloalkyl, and
$C_{3-6}$ cycloalkyl substituted by substituent(s) independently selected from the group consisting of:

- $C_{1-5}$ alkyl,
- $C_{1-5}$ alkyl substituted by oxo,
- $C_{1-5}$ alkyl substituted by carbocyclic aryl, and
- carbocyclic aryl,

(v) carbocyclyl,
(vi) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:

  - •• halogen,
  - •• oxo,
  - •• carbocyclic aryloxy,
  - •• carbocyclic aryl, and
  - •• carbocyclic aryl substituted by $C_{1-5}$ alkyl,

- $C_{1-5}$ alkoxy,
- $C_{1-5}$ alkoxy substituted by substituent(s) independently selected from the group consisting of:

  - •• halogen,
  - •• carbocyclic aryl, and
  - •• halogenated carbocyclic aryl,

- $C_{2-5}$ alkenyloxy,
- $C_{3-6}$ cycloalkoxy,
- carbocyclic aryloxy,
- carbocyclic aryloxy substituted by $C_{1-5}$ alkoxy,
- $C_{1-5}$ alkoxycarbonyl,

- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,
- mono-$C_{1-5}$ alkylaminosulfonyl,
- di-$C_{1-5}$ alkylaminosulfonyl, and
- carbocyclic aryl,

(vii) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:

  - •• halogen,
  - •• hydroxy,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by halogen, and
  - •• heterocyclyl,

- $C_{1-5}$ alkoxy,
- carbocyclic aryloxy,
- carbocyclic aryloxy substituted by $C_{1-5}$ alkyl,
- $C_{1-5}$ alkylthio,
- $C_{2-5}$ alkenylthio,
- carbocyclic arylthio,
- carbocyclic arylthio substituted by $C_{1-5}$ alkoxycarbonyl,
- $C_{1-5}$ alkylsulfonyl,
- carbocyclic arylsulfonyl,
- carbocyclic arylsulfonyl substituted by $C_{1-5}$ alkyl,
- $C_{1-5}$ alkoxycarbonyl,
- carbocyclic aryl,
- carbocyclic aryl substituted by substituent(s) independently selected from the group consisting of:

  - •• halogen,
  - •• nitro,
  - •• $C_{1-5}$ alkyl, and
  - •• $C_{1-5}$ alkyl substituted by halogen,

- heterocyclyl;

$R_2$ is methylamino or dimethylamino when Y is a single bond or $-CH_2-$;

wherein carbocyclic aryl is phenyl, naphthyl, or anthranyl;

carbocyclyl is 1,2,3,4-tetrahydronaphthyl, 1-oxo-indanyl, 9-fluorenyl, 9-oxo-9*H*-fluorenyl, bicyclo[2.2.1]heptyl, indenyl, or menthyl;

heterocyclyl is 1,2,3-triazolyl, 1*H*-indolyl, 1*H*-pyrrolyl, 2,3-dihydro-1-oxo-isoindolyl, 2,3-dihydro-benzo[1,4]dioxinyl, 2,3-dihydro-benzofuryl, 2,4-dihydro-3-oxo-pyrazolyl, 2*H*-benzopyranyl, 2-oxo-benzopyranyl, 3,4-dihydro-2*H*-benzo[b][1,4]dioxepinyl, 4-oxo-benzopyranyl, 9*H*-carbazolyl, 9*H*-xanthenyl, azetidinyl, benzo[1,3]dioxolyl, benzo[2,1,3]oxadiazolyl, benzo[1,2,5]oxadiazolyl, benzo[b]thienyl, benzofuryl, benzothiazolyl, furyl, imidazo[2,1-b]thiazolyl, imidazolyl, isoxazolyl, morpholino, pyrazolyl, pyridyl, pyrimidyl, quinolyl, quinoxalyl, thiazolyl, or thienyl; and

halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-7}$ alkyl, and

$C_{1-7}$ alkyl substituted by substituent(s) independently selected from the group consisting of:

- $C_{1-5}$ alkoxy,
- $C_{1-5}$ alkoxy substituted by carbocyclic aryl,
- carbocyclic aryloxy,
- carbocyclic aryloxy substituted by halogen,
- mono-$C_{1-5}$ alkylamino,
- mono-$C_{1-5}$ alkylamino substituted by substituent(s) independently selected from the group consisting of:
  - cyano, and
  - carbocyclic aryl,
- di-$C_{1-5}$ alkylamino,
- di-$C_{1-5}$ alkylamino substituted by substituent(s) independently selected from the group consisting of:
  - cyano, and
  - carbocyclic aryl,
- mono-carbocyclic arylamino,
- di-carbocyclic arylamino,
- carbocyclic arylsulfonylamino,
- carbocyclic arylsulfonylamino substituted by $C_{1-5}$ alkyl,
- carbocyclic aryl,
- carbocyclic aryl substituted by substituent(s) independently selected from the group consisting of:
  - halogen,
  - nitro,
  - $C_{1-5}$ alkyl,
  - $C_{1-5}$ alkyl substituted by substituent(s) independently selected from the group consisting of:
    - oxo, and
    - carbocyclic aryl,
  - $C_{1-5}$ alkoxy,
  - $C_{1-5}$ alkoxy substituted by halogen,
- heterocyclyl,
- heterocyelyl substituted by carbocyclic aryl, and
- heterocyclyl substituted by halogen,

(ii) $C_{2-7}$ alkenyl, and

$C_{2-7}$ alkenyl substituted by substituent(s) independently selected from the group consisting of:

- carbocyclic aryl, and
- carbocyclic aryl substituted by $C_{1-5}$ alkoxy,

(iii) $C_{2-5}$ alkynyl, and
$C_{2-5}$ alkynyl substituted by carbocyclic aryl,
(iv) $C_{3-6}$ cycloalkyl, and
$C_{3-6}$ cycloalkyl substituted by substituent(s) independently selected from the group consisting of:

- $C_{1-5}$ alkyl, and
- $C_{1-5}$ alkyl substituted by carbocyclic aryl,

(v) carbocyclic aryl, and
carbocyclic aryl substituted by substituent(s) independently selected from the group consisting of:

- halogen,
- hydroxy,
- cyano,
- $C_{1-5}$ alkyl,
- $C_{1-5}$ alkyl substituted by halogen,
- $C_{1-5}$ alkoxy,
- $C_{1-5}$ alkoxy substituted by substituent(s) independently selected from the group consisting of:

  - •• halogen, and
  - •• carbocyclic aryl,
  - •• carbocyclic aryl substituted by halogen,

- $C_{2-5}$ alkenyloxy,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{1-5}$ alkylthio, and
- $C_{1-5}$ alkylthio substituted by halogen,

(vi) heterocyclyl, and
heterocyclyl substituted by substituent(s) independently selected from the group consisting of:

- halogen,
- $C_{1-5}$ alkyl,
- $C_{1-5}$ alkyl substituted by substituent(s) independently selected from the group consisting of:

  - •• hydroxy, and
  - •• carbocyclic aryl,

- $C_{1-5}$ alkoxy,
- carbocyclic arylthio,
- carbocyclic arylthio substituted by $C_{1-5}$ alkoxycarbonyl,
- $C_{1-5}$ alkoxycarbonyl,
- carbocyclic aryl,
- carbocyclic aryl substituted by substituent(s) independently selected from the group consisting of:

  - •• halogen,
  - •• $C_{1-5}$ alkyl, and
  - •• $C_{1-5}$ alkyl substituted by halogen,

L is Formula (VII);

Y is a single bond or $-CH_2-$;
$R_2$ is methylamino or dimethylamino;
wherein carbocyclic aryl is phenyl or naphthyl;
heterocyclyl is 1*H*-indolyl, 1*H*-pyrrolyl, 2,3-dihydro-benzo[1,4]dioxinyl, 4-oxo-benzopyranyl, 9*H*-carbazolyl, azetidinyl, benzo[1,3]dioxolyl, benzo[b]thienyl, furyl, imidazo[2,1-b]thiazolyl, pyrazolyl, pyridyl, or thienyl; and
halogen is fluoro, chloro, bromo, or iodo;

or a pharmaceutically acceptable salt, hydrate, or solvate thereof.

In some embodiments of the present invention, p is 0; $R_3$ and $R_4$ are hydrogen; A is a single bond or $-CH_2-$; and B is a single bond or $-CH_2-$; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- mono-$C_{1-5}$ alkylamino,
- mono-$C_{1-5}$ alkylamino substituted by cyano,
- di-$C_{1-5}$ alkylamino,
- di-$C_{1-5}$ alkylamino substituted by cyano,
- mono-carbocyclic arylamino,
- di-carbocyclic arylamino,
- carbocyclic aryl,
- carbocyclic aryl substituted by substituent(s) independently selected from the group consisting of:

    - • halogen, and
    - •$C_{1-5}$ alkoxy,

- heterocyclyl, and
- heterocyclyl substituted by carbocyclic aryl,

(ii) $C_{2-5}$ alkenyl, and
$C_{2-5}$ alkenyl substituted by carbocyclic aryl,
(iii) carbocyclic aryl, and
carbocyclic aryl substituted by substituent(s) independently selected from the group consisting of:

- halogen,
- hydroxy,
- $C_{1-5}$ alkoxy,
- $C_{1-5}$ alkoxy substituted by halogen, and
- $C_{2-5}$ alkenyloxy,

(iv) heterocyclyl, and
heterocyclyl substituted by substituent(s) independently selected from the group consisting of:

- halogen,
- $C_{1-5}$ alkyl,
- $C_{1-5}$ alkyl substituted by carbocyclic aryl,
- $C_{1-5}$ alkoxy, and
- $C_{1-5}$ alkoxycarbonyl;

wherein carbocyclic aryl is phenyl or naphthyl;
heterocyclyl is 1*H*-indolyl, azetidinyl, or benzo[1,3]dioxolyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

ethyl 4,6-dichloro-3-{[(cis-4-{[4-(dimethylamino)quinolin-2-yl]amino}-cyclohexyl)amino]-methyl}-1H-indole-2-carboxylate;
3-[{2-[(cis-4-{[4-(dimethylamino)quinolin-2-yl]amino}cyclohexyl)-amino]ethyl}(phenyl)-amino]propanenitrile;
$N^4$,$N^4$-dimethyl-$N^2$-(cis-4-{[2-(2-phenyl-1H-indol-3-yl)ethyl]amino}-cyclohexyl)quinoline-2,4-diamine;
$N^2$-[cis-4-({[1-(diphenylmethyl)azetidin-3-yl]methyl}amino)cyclohexyl]-$N^4$,$N^4$-dimethylquinoline-2,4-diamine;
$N^2$-(cis-4-{[(2,6-dimethoxybenzyl)amino]methyl}cyclohexyl)-$N^4$,$N^4$-dimethylquinoline-2,4-diamine;
$N^2$-(cis-4-{[(2-ethoxybenzyl)amino]methyl}cyclohexyl)-$N^4$,$N^4$-dimethylquinoline-2,4-diamine;
$N^2$-[cis-4-({[(4-methoxy-1-naphthyl)methyl]amino}methyl)cyclohexyl]-$N^4$,$N^4$-dimethylquinoline-2,4-diamine;
4-bromo-2-({[(cis-4-{[4-(dimethylamino)quinolin-2-yl]amino}cyclohexyl)-methyl]amino}-methyl)-6-methoxyphenol;
$N^2$-[cis-4-({[(5-bromo-1H-indol-3-yl)methyl]aminomethyl)cyclohexyl]-$N^4$,$N^4$-dimethylquinoline-2,4-diamine;
$N^2$-(cis-4-{[(5-bromo-2,4-dimethoxybenzyl)amino]methyl}cyclohexyl)-$N^4$,$N^4$-dimethylquinoline-2,4-diamine;
$N^2$-(cis-4-{[(3,3-diphenylprop-2-en-1-yl)amino]methyl}cyclohexyl)-$N^4$,$N^4$-dimethylquinoline-2,4-diamine;
$N^4$,$N^4$-dimethyl-$N^2$-(cis-4-{[(2,4,6-trimethoxybenzyl)amino]methyl}-cyclohexyl)quinoline-2,4-diamine;
$N^2$-(cis-4-{[(2,5-diethoxybenzyl)amino]methyl}cyclohexyl)-$N^4$,$N^4$-dimethylquinoline-2,4-diamine;
$N^2$-(cis-4-{[(2,4-diethoxybenzyl)amino]methyl}cyclohexyl)-$N^4$,$N^4$-dimethylquinoline-2,4-diamine;
$N^2$-(cis-4-{[(3,5-dibromo-2-methoxybenzyl)amino]methyl}cyclohexyl)-$N^4$,$N^4$-dimethylquinoline-2,4-diamine;
$N^4$,$N^4$-dimethyl-$N^2$-(cis-4-{[(2,4,5-triethoxybenzyl)amino]methyl}-cyclohexyl)quinotine-2,4-diamine;
$N^4$,$N^4$-dimethyl-$N^2$-(cis-4-{[(2,4,5-trimethoxybenzyl)amino]methyl}-cyclohexyl)quinoline-2,4-diamine;
$N^2$-[cis-4-({[2-(allyloxy)benzyl]amino]methyl)cyclohexyl]-$N^4$,$N^4$-dimethylquinoline-2,4-diamine;
$N^2$-[cis-4-({[(7-methoxy-1,3-benzodioxol-5-yl)methyl]amino}methyl)-cyclohexyl]-$N^4$,$N^4$-dimethylquinoline-2,4-diamine;
$N^2$-{cis-4-[2-(4-bromo-2-trifluoromethoxy-phenyl)-ethylamino]-cyclohexyl}-$N^4$,$N^4$-dimethyl-quinoline-2,4-diamine;
$N^2$-[cis-4-(4-bromo-2-trifluoromethoxy-benzyl)amino-cyclohexyl]-$N^4$,$N^4$-dimethyl-quinoline - 2,4-diamine;
$N^2$-[cis-4-(4-bromo-2-trifluoromethoxy-benzyl)amino-cyclohexyl]-$N^4$-methyl-quinoline-2,4-diamine;
$N^2$-{4-[2-(4-bromo-2-trifluoromethoxy-phenyl)-ethylamino]-cyclohexyl}-$N^4$-methyl-quinoline-2,4-diamine;
$N^4$-methyl-$N^2$-{cis-4-[(2-trifluoromethoxy-benzyl)amino-methyl]-cyclohexyl}-quinoline-2,4-diamine;
$N^2$-{cis-4-[(4-bromo-2-trifluoromethoxy-benzyl)amino-methyl]-cyclohexyl}-$N^4$-methyl-quinoline-2,4-diamine;
$N^2$-{cis-4-[(4-bromo-2-trifluoromethoxy-benzyl)amino-methyl]-cyclohexyl}-$N^4$,$N^4$-dimethyl-quinoline-2,4-diamine;
$N^4$,$N^4$-dimethyl-$N^2$-{cis-4-[(2-trifluoromethoxy-benzyl)amino-methyl]-cyclohexyl}-quinoline-2,4-diamine;
cis-N-(3,5-dimethoxybenzyl)-N'-(4-methylquinolin-2-yl)cyclohexane-1,4-diamine; and
cis-N-(3,5-dichlorobenzyl)-N'-(4-methylquinolin-2-yl)cyclohexane-1,4-diamine;

or a pharmaceutically acceptable salt, hydrate, or solvate thereof.

In some embodiments of the present invention $R_1$ is selected from the group consisting of:

(i) $C_{1-5}$ alkyl, and
$C_{1-5}$ alkyl substituted by substituent(s) independently selected from the group consisting of:

- hydroxy,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by substituent(s) independently selected from the group consisting of:
  - halogen,
  - nitro,
  - $C_{1-5}$ alkyl,
  - $C_{1-5}$ alkoxy, and
  - $C_{1-5}$ alkoxy substituted by halogen,
- heterocyclyloxy,
- heterocyclyloxy substituted by $C_{1-5}$ alkyl,
- mono-$C_{1-5}$ alkylaminocarbonyl,
- di-$C_{1-5}$ alkylaminocarbonyl,
- mono-$C_{1-5}$ alkylamino,
- di-$C_{1-5}$ alkylamino,

- mono-carbocyclic arylamino,
- di-carbocyclic arylamino,
- mono-carbocyclic arylamino substituted by halogen,
- di-carbocyclic arylamino substituted by halogen,
- carbocyclic arylcarbonylamino,
- $C_{1-5}$ alkoxycarbonylamino,
- $C_{1-5}$ alkylthio,
- $C_{1-5}$ alkylthio substituted by substituent(s) independently selected from the group consisting of:

  - •• carbocyclic aryl, and
  - •• Carbocyclic aryl substituted by substituent(s) independently selected from the group consisting of:

    - ••• halogen, and
    - ••• $C_{1-5}$ alkoxy,

- carbocyclic arylthio,
- heterocyclylthio,
- heterocyclylthio substituted by $C_{1-5}$ alkyl,
- $C_{3-6}$ cycloalkyl,
- $C_{3-6}$ cycloalkenyl,
- carbocyclyl,
- carbocyclyl substituted by substituent(s) independently selected from the group consisting of:

  - •• halogen,
  - •• $C_{1-5}$ alkyl,
  - •• $C_{1-5}$ alkoxy,
  - •• $C_{2-5}$ alkenyl, and
  - •• $C_{2-5}$ alkenyl substituted by substituent(s) independently selected from the group consisting of:

    - ••• carbocyclic aryl, and
    - ••• carbocyclic aryl substituted by $C_{1-5}$ alkylsulfinyl,

- carbocyclic aryl,
- carbocyclic aryl substituted by substituent(s) independently selected from the group consisting of:

  - •• halogen,
  - •• hydroxy,
  - •• nitro,
  - •• $C_{1-5}$ alkyl,
  - •• $C_{1-5}$ alkyl substituted by substituent(s) independently selected from the group consisting of:

    - ••• oxo,
    - ••• carbocyclic aryl, and
    - ••• heterocyclyl,

  - •• $C_{1-5}$ alkoxy,
  - •• $C_{1-5}$ alkoxy substituted by halogen,
  - •• $C_{1-5}$ alkoxy substituted by carbocyclic aryl,
  - •• carbocyclic aryloxy,
  - •• mono-carbocyclic arylaminocarbonyl,
  - •• mono-carbocyclic arylaminocarbonyl substituted by halogen,
  - •• di-carbocyclic arylaminocarbonyl,
  - •• di-carbocyclic arylaminocarbonyl substituted by halogen,
  - •• carbocyclic aryl, and
  - •• heterocyclyl,

- heterocyclyl, and
- heterocyclyl substituted by substituent(s) independently selected from the group consisting of:

- •• $C_{1-5}$ alkyl,
- •• $C_{1-5}$ alkyl substituted by carbocyclic aryl,
- •• $C_{1-5}$ alkoxy,
- •• $C_{1-5}$ alkoxy substituted by carbocyclic aryl,
- •• carbocyclic aryl, and
- •• carbocyclic aryl substituted by halogen,

(ii) $C_{2-5}$ alkenyl, and
$C_{2-5}$ alkenyl substituted by substituent(s) independently selected from the group consisting of:

- • carbocyclic aryl,
- • carbocyclic aryl substituted by substituent(s) independently selected from the group consisting of:
  - •• halogen, and
  - •• nitro,

(iii) $C_{3-6}$ cycloalkyl, and
$C_{3-6}$ cycloalkyl substituted by substituent(s) independently selected from the group consisting of:

- • $C_{1-5}$ alkyl,
- • $C_{1-5}$ alkyl substituted by substituent(s) independently selected from the group consisting of:
  - •• oxo, and
  - •• carbocyclic aryl, and
- • carbocyclic aryl,

(iv) carbocyclyl,
(v) carbocyclic aryl, and
carbocyclic aryl substituted by substituent(s) independently selected from the group consisting of:

- • halogen,
- • hydroxy,
- • cyano,
- • nitro,
- • carboxy,
- • carbamoyl,
- • $C_{1-5}$ alkyl,
- • $C_{1-5}$ alkyl substituted by substituent(s) independently selected from the group consisting of:
  - •• halogen,
  - •• hydroxy,
  - •• oxo,
  - •• carbocyclic aryloxy,
  - •• carbocyclic aryl, and
  - •• carbocyclic aryl substituted by $C_{1-5}$ alkyl,
- • $C_{1-5}$ alkoxy,
- • $C_{1-5}$ alkoxy substituted by substituent(s) independently selected from the group consisting of:
  - •• halogen, and
  - •• carbocyclic aryl,
- • carbocyclic aryloxy,
- • carbocyclic aryloxy substituted by $C_{1-5}$ alkoxy,
- • $C_{1-5}$ alkoxycarbonyl,
- • mono-$C_{1-5}$ alkylaminocarbonyl,
- • di-$C_{1-5}$ alkylaminocarbonyl,

- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,
- mono-$C_{1-5}$ alkylaminosulfonyl,
- di-$C_{1-5}$ alkylaminosulfonyl, and
- carbocyclic aryl,

(vi) heterocyclyl, and
heterocyclyl substituted by substituent(s) independently selected from the group consisting of:

- halogen,
- nitro,
- hydroxy,
- amino,
- $C_{1-5}$ alkyl,
- $C_{1-5}$ alkyl substituted by substituent(s) independently selected from the group consisting of:

  - •• halogen,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by halogen, and
  - •• heterocyclyl,

- $C_{1-5}$ alkoxy,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,
- di-$C_{1-5}$ alkylamino,
- $C_{1-5}$ alkylthio,
- $C_{1-5}$ alkenylthio,
- carbocyclic arylthio,
- $C_{1-5}$ alkylsulfonyl,
- carbocyclic arylsulfonyl,
- carbocyclic arylsulfonyl substituted by $C_{1-5}$ alkyl,
- carbocyclic aryl,
- carbocyclic aryl substituted by substituent(s) independently selected from the group consisting of:

  - •• halogen,
  - •• nitro, and
  - •• $C_{1-5}$ alkyl,

- heterocyclyl;

L is Formula (VII);
Y is -C(O)-;
wherein carbocyclic aryl is phenyl, naphthyl, or anthranyl;
carbocyclyl is 1,2,3,4-tetrahydronaphthyl, 1-oxo-indanyl, 9-oxo-9*H*-fluorenyl, or indeny;
heterocyclyl is 1,2,3-triazolyl, 1*H*-indolyl, 1*H*-pyrrolyl, 2,3-dihydro-1-oxo-isoindolyl, 2,3-dihydro-benzofuryl, 2,4-dihydro-3-oxo-pyrazolyl, 2*H*-benzopyranyl, 2-oxo-benzopyranyl, 9*H*-xanthenyl, benzo[1,3]dioxolyl, benzo [2,1,3]oxadiazolyl, benzo[1,2,5]oxadiazolyl, benzo[b]thienyl, benzofuryl, benzothiazolyl, furyl, imidazolyl, isoxazolyl, morpholino, pyrazolyl, pyridyl, pyrimidyl, quinolyl, quinoxalyl, thiazolyl, or thienyl; and
halogen is fluoro, chloro, bromo, or iodo;

or a pharmaceutically acceptable salt, hydrate, or solvate thereof.

In some embodiments of the present invention, $R_2$ is hydrogen, halogen, methyl, trifluoromethyl, methoxy, carbamoyl, amino, methylamino, or dimethylamino; p is 0; $R_3$ and $R_4$ are hydrogen; A is a single bond; B is a single bond or $-CH_2-$; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- oxo,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,
- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- $C_{3-6}$ cycloalkyl,
- carbocyclic aryl,
- carbocyclic aryl by substituent(s) independently selected from the group consisting of:
  - •• halogen,
  - •• $C_{1-5}$ alkyl, and
  - •• $C_{1-5}$ alkoxy,
- heterocyclyl, and
- heterocyclyl substituted by substituent(s) independently selected from the group consisting of:
  - •• $C_{1-5}$ alkyl,
  - •• $C_{1-5}$ alkoxy, and
  - •• carbocyclic aryl,

(ii) $C_{2-5}$ alkenyl, and
$C_{2-5}$ alkenyl substituted by substituent(s) independently selected from the group consisting of:

- carbocyclic aryl,
- carbocyclic aryl substituted by substituent(s) independently selected from the group consisting of:
  - •• halogen, and
  - •• nitro,

(iii) carbocyclic aryl, and
carbocyclic aryl substituted by substituent(s) independently selected from the group consisting of:

- halogen,

- hydroxy,
- cyano,
- nitro,
- carbamoyl,
- $C_{1-5}$ alkyl,
- $C_{1-5}$ alkyl substituted by halogen,
- $C_{1-5}$ alkyl substituted by hydroxy,
- $C_{1-5}$ alkoxycarbonyl,
- $C_{1-5}$ alkoxy,
- $C_{1-5}$ alkoxy substituted by substituent(s) independently selected from the group consisting of:

  - •• halogen, and
  - •• carbocyclic aryl,

- carbocyclic aryloxy, and
- carbocyclic aryloxy substituted by $C_{1-5}$ alkoxy,

(iv) heterocyclyl, and
heterocyclyl substituted by substituent(s) independently selected from the group consisting of:

- halogen,
- nitro,
- amino,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,
- di-$C_{1-5}$ alkylamino,
- carbocyclic aryl,
- carbocyclic aryl substituted by halogen,
- carbocyclic aryl substituted by nitro, and
- heterocyclyl;

wherein carbocyclic aryl is phenyl;
heterocyclyl is 1,2,3-triazolyl, 1*H*-indolyl, 1*H*-pyrrolyl, 9*H*-xanthenyl, benzo[2,1,3]oxadiazolyl, benzo[1,2,5]oxadiazolyl, furyl, isoxazolyl, pyridyl, quinolyl, quinoxalyl, thiazolyl, or thienyl; and
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,
- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,

- carbocyclic aryl,
- carbocyclic aryl by substituent(s) independently selected from the group consisting of:

  - halogen,
  - $C_{1-5}$ alkyl, and
  - $C_{1-5}$ alkoxy,

  and
- heterocyclyl,

(ii) carbocyclic aryl, and
carbocyclic aryl substituted by substituent(s) independently selected from the group consisting of:

- halogen,
- nitro,
- hydroxy,
- cyano,
- $C_{1-5}$ alkyl,
- $C_{1-5}$ alkyl substituted by halogen,
- $C_{1-5}$ alkoxycarbonyl,
- $C_{1-5}$ alkoxy,
- $C_{1-5}$ alkoxy substituted by halogen,
- carbocyclic aryloxy, and
- carbocyclic aryloxy substituted by $C_{1-5}$ alkoxy,

(iii) heterocyclyl, and
heterocyclyl substituted by substituent(s) independently selected from the group consisting of:

- halogen,
- nitro,
- $C_{1-5}$ alkyl,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- carbocyclic aryl,
- carbocyclic aryl substituted by halogen,
- carbocyclic aryl substituted by nitro, and
- heterocyclyl;

wherein carbocyclic aryl is phenyl;
heterocyclyl is 1*H*-indolyl, 1*H*-pyrrolyl, 9*H*-xanthenyl, benzo[2,1,3]oxadiazolyl, benzo[1,2,5]oxadiazolyl, furyl, isoxazolyl, pyridyl, thiazolyl, or thienyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(cis-4-{[4-(dimethylamino)quinolin-2-yl]amino}cyclohexyl)-3-methoxybenzamide;
3-bromo-N-(cis-4-{[4-(dimethylamino)quinolin-2-yl]amino}cyclohexyl)-benzamide;
4-bromo-N-(cis-4-{[4-(dimethylamino)quinolin-2-yl]amino}cyclohexyl)-benzamide;
N-(cis-4-{[4-(dimethylamino)quinolin-2-yl]amino}cyclohexyl)-2,1,3-benzoxadiazole-5-carboxamide;
3-chloro-N-(cis-4-{[4-(dimethylamino)quinolin-2-yl]amino}cyclohexyl)-benzamide;
4-chloro-N-(cis-4-{[4-(dimethylamino)quinolin-2-yl]amino}cyclohexyl)-benzamide;
4-chloro-N-(cis-4-{[4-(dimethylamino)quinolin-2-yl]amino}cyclohexyl)-3-nitrobenzamide;
3-cyano-N-(cis-4-{[4-(dimethylamino)quinolin-2-yl]amino}cyclohexyl)-benzamide;
3,5-dichloro-N-(cis-4-{[4-(dimethylamino)quinolin-2-yl]amino}-cyclohexyl)benzamide;

3,4-dichloro-N-(cis-4-{[4-(dimethylamino)quinolin-2-yl]amino}-cyclohexyl)benzamide;
N-(cis-4-{[4-(dimethylamino)quinolin-2-yl]amino}cyclohexyl)-2,2-diphenylacetamide;
N-(cis-4-{[4-(dimethylamino)quinolin-2-yl]amino}cyclohexyl)-3,4-difluorobenzamide;
N-(cis-4-{[4-(dimethylamino)quinolin-2-yl]amino}cyclohexyl)-3,5-difluorobenzamide;
N-(cis-4-{[4-(dimethylamino)quinolin-2-yl]amino}cyclohexyl)-4-fluorobenzamide;
N-(cis-4-{[4-(dimethylamino)quinolin-2-yl]amino}cyclohexyl)-3-fluoro-5-(trifluoromethyl)benzamide;
N-(cis-4-{[4-(dimethylamino)quinolin-2-yl]amino}cyclohexyl)-4-methyl-3-nitrobenzamide;
N-(cis-4-{[4-(dimethylamino)quinolin-2-yl]amino}cyclohexyl)-3-nitrobenzamide;
N-(cis-4-{[4-(dimethylamino)quinolin-2-yl]amino}cyclohexyl)-2-phenoxybutanamide;
N-(cis-4-{[4-(dimethylamino)quinolin-2-yl]amino}cyclohexyl)-2-phenoxypropanamide;
N-(cis-4-{[4-(dimethylamino)quinolin-2-yl]amino}cyclohexyl)-3-methylbenzamide;
N-(cis-4-{[4-(dimethylamino)quinolin-2-yl]amino}cyclohexyl)-3-(trifluoromethoxy)-benzamide;
4-bromo-N-(cis-4-{[4-(dimethylamino)quinolin-2-yl]amino}cyclohexyl)-3-methylbenzamide;
N-(cis-4-{[4-(dimethylamino)quinolin-2-yl]amino}cyclohexyl)-3-iodobenzamide;
3-chloro-N-(cis-4-{[4-(dimethylamino)quinolin-2-yl]amino}cyclohexyl)-2,4-difluorobenzamide;
N-(cis-4-{[4-(dimethylamino)quinolin-2-yl]amino}cyclohexyl)-2,5-dimethyl-3-furamide;
3-chloro-N-(cis-4-{[4-(dimethylamino)quinolin-2-yl]amino}cyclohexyl)-4-fluorobenzamide;
N-(cis-4-{[4-(dimethylamino)quinolin-2-yl]amino}cyclohexyl)-3-fluoro-4-methylbenzamide;
N-(cis-4-{[4-(dimethylamino)quinolin-2-yl]amino}cyclohexyl)-3,5-dimethoxybenzamide;
N-(cis-4-{[4-(dimethylamino)quinolin-2-yl]amino}cyclohexyl)-3,5-bis(trifluoromethyl)-benzamide;
(2E)-N-(cis-4-{[4-(dimethylamino)quinolin-2-yl]amino}cyclohexyl)-3-(4-nitrophenyl)-acrylamide;
N-(cis-4-{[4-(dimethylamino)quinolin-2-yl]amino)cyclohexyl)-4-fluoro-3-methylbenzamide;
2,5-dichloro-N-(cis-4-{[4-(dimethylamino)quinolin-2-yl]amino}-cyclohexyl)thiophene-3-carboxamide;
2-(4-chlorophenoxy)-N-(cis-4-{[4-(dimethylamino)quinolin-2-yl]amino}-cyclohexyl)-acetamide;
3-(2-chlorophenyl)-N-(cis-4-{[4-(dimethylamino)quinolin-2-yl]amino}-cyclohexyl)-5-methylisoxazole-4-carboxamide;
1-(4-chlorophenyl)-N-(cis-4-{[4-(dimethylamino)quinolin-2-yl]amino}-cyclohexyl)-cyclopentanecarboxamide;
3-(2-chloro-6-fluorophenyl)-N-(cis-4-{[4-(dimethylamino)quinolin-2-yl]amino}cyclohexyl)-5-methylisoxazole-4-carboxamide;
N-(cis-4-{[4-(dimethylamino)quinolin-2-yl]amino}cyclohexyl)-3-fluorobenzamide;
N-(cis-4-{[4-(dimethylamino)quinolin-2-yl]amino}cyclohexyl)-4-fluoro-3-(trifluoromethyl)benzamide;
N-(cis-4-{[4-(dimethylamino)quinolin-2-yl]amino}cyclohexyl)-5-methyl-2-phenyl-2H-1,2,3-triazole-4-carboxamide;
N-(cis-4-{[4-(dimethylamino)quinolin-2-yl]amino}cyclohexyl)-2-(4-methoxyphenoxy)-5-nitrobenzamide;
N-(cis-4-{[4-(dimethylamino)quinolin-2-yl]amino}cyclohexyl)-5-nitro-2-furamide;
N-(cis-4-{[4-(dimethylamino)quinolin-2-yl]amino}cyclohexyl)-2-phenoxyacetamide;
N-(cis-4-{[4-(dimethylamino)quinolin-2-yl]amino}cyclohexyl)quinoxaline-2-carboxamide;
2-(3-chlorophenoxy)-N-(cis-4-{[4-(dimethylamino)quinolin-2-yl]amino}-cyclohexyl)-acetamide;
3-(2,6-dichlorophenyl)-N-(cis-4-{[4-(dimethylamino)quinolin-2-yl]amino}-cyclohexyl)-5-methylisoxazole-4-carboxamide;
N-(cis-4-{[4-(dimethylamino)quinolin-2-yl]amino}cyclohexyl)-2-phenoxynicotinamide;
N-(cis-4-{[4-(dimethylamino)quinolin-2-yl]amino}cyclohexyl)-2-(4-methylphenoxy)-nicotinamide;
N-(cis-4-{[4-(dimethylamino)quinolin-2-yl]amino}cyclohexyl)-2-(2-thienyl)-1,3-thiazole-4-carboxamide;
5-bromo-N-(cis-4-{[4-(dimethylamino)quinolin-2-yl]amino}cyclohexyl)-thiophene-2-carboxamide;
N-(cis-4-{[4-(dimethylamino)quinolin-2-yl]amino}cyclohexyl)-2-(2,3,6-trichlorophenyl)-acetamide;
5-(4-chloro-2-nitrophenyl)-N-(cis-4-{[4-(dimethylamino)quinolin-2-yl]amino}cyclohexyl)-2-furamide;
5-chloro-N-(cis-4-{[4-(dimethylamino)quinolin-2-yl]amino}cyclohexyl)-thiophene-2-carboxamide;
N-(cis-4-{[4-(dimethylamino)quinolin-2-yl]amino}cyclohexyl)-5-iodo-2-furamide;
N-(cis-4-{[4-(dimethylamino)quinolin-2-yl]amino}cyclohexyl)-2-(2-iodophenyl)acetamide;
N-(cis-4-{[4-(dimethylamino)quinolin-2-yl]amino}cyclohexyl)-2-(5-methoxy-2-methyl-1H-indol-3-yl)acetamide;
(2E)-N-(cis-4-{[4-(dimethylamino)quinolin-2-yl]amino}cyclohexyl)-3-(3-nitrophenyl)-acrylamide;
2,2-bis(4-chlorophenyl)-N-(cis-4-{[4-(dimethylamino)quinolin-2-yl]-amino}cyclohexyl)-acetamide;
N-(cis-4-{[4-(dimethylamino)quinolin-2-yl]amino}cyclohexyl)-5-nitrothiophene-2-carboxamide;
N-(cis-4-{[4-(dimethylamino)quinolin-2-yl]amino}cyclohexyl)-3-methyl-4-nitrobenzamide;
N-(cis-4-{[4-(dimethylamino)quinolin-2-yl]amino}cyclohexyl)-3-methoxy-4-nitrobenzamide;
5-bromo-N-(cis-4-{[4-(dimethylamino)quinolin-2-yl]amino}cyclohexyl)-2-furamide;
4,5-dibromo-N-(cis-4-{[4-(dimethylamino)quinolin-2-yl]amino}-cyclohexyl)thiophene-2-carboxamide;
4,5-dibromo-N-(cis-4-{[4-(dimethylamino)quinolin-2-yl]amino}-cyclohexyl)-2-furamide;
N-(cis-4-{[4-(dimethylamino)quinolin-2-yl]amino}cyclohexyl)-2-(1H-indol-3-yl)acetamide;

N-(cis-4-{[4-(dimethylamino)quinolin-2-yl]amino}cyclohexyl)-2-(1H-indol-3-yl)-4-oxo-4-phenylbutanamide;
N-(cis-4-{[4-(dimethylamino)quinolin-2-yl]amino}cyclohexyl)-2-(2-phenyl-1H-indol-3-yl)acetamide;
N-(cis-4-{[4-(dimethylamino)quinolin-2-yl]amino}cyclohexyl)-2-(2,4,6-trichlorophenoxy)-acetamide;
3-(benzyloxy)-N-(cis-4-{[4-(dimethylamino)quinolin-2-yl]amino}-cyclohexyl)-4-methoxybenzamide;
N-(cis-4-{[4-(dimethylamino)quinolin-2-yl]amino}cyclohexyl)-2-phenoxybenzamide;
N-(cis-4-{[4-(dimethylamino)quinolin-2-yl]amino}cyclohexyl)-2-phenylquinoline-4-carboxamide;
N-(cis-4-{[4-(dimethylamino)quinolin-2-yl]amino}cyclohexyl)-5-(3-nitrophenyl)-2-furamide;
N-(cis-4-{[4-(dimethylamino)quinolin-2-yl]amino}cyclohexyl)-5-nitrothiophene-3-carboxamide;
N-(cis-4-{[4-(dimethylamino)quinolin-2-yl]amino}cyclohexyl)-1-methyl-4-nitro-1H-pyrrole-2-carboxamide;
N-(cis-4-{[4-(dimethylamino)quinolin-2-yl]amino}cyclohexyl)-4-nitrobenzamide;
N-(cis-4-{[4-(dimethylamino)quinolin-2-yl]amino}cyclohexyl)-2-methoxy-4-nitrobenzamide;
N-(cis-4-{[4-(dimethylamino)quinolin-2-yl]amino}cyclohexyl)-3-fluoro-4-(trifluoromethyl)benzamide;
N-(cis-4-{[4-(dimethylamino)quinolin-2-yl]amino}cyclohexyl)-3,5-dimethyl-4-nitrobenzamide;
N-(cis-4-{[4-(dimethylamino)quinolin-2-yl]amino}cyclohexyl)-2-mesityl-2-oxoacetamide;
5-chloro-N-(cis-4-{[4-(dimethylamino)quinolin-2-yl]amino}cyclohexyl)-2-hydroxybenzamide;
N-[(cis-4-{[4-(dimethylamino)quinolin-2-yl]amino}cyclohexyl)methyl]-3-methoxybenzamide;
3-bromo-N-[(cis-4-{[4-(dimethylamino)quinolin-2-yl]amino}cyclohexyl)-methyl]-benzamide;
4-bromo-N-[(cis-4-{[4-(dimethylamino)quinolin-2-yl]amino}cyclohexyl)-methyl]-benzamide;
N-[(cis-4-{[4-(dimethylamino)quinolin-2-yl]amino}cyclohexyl)methyl]-2,1,3-benzoxadiazole-5-carboxamide;
3-chloro-N-[(cis-4-{[4-(dimethylamino)quinolin-2-yl]amino}cyclohexyl)-methyl]-benzamide;
4-chloro-N-[(cis-4-{[4-(dimethylamino)quinolin-2-yl]amino}cyclohexyl)-methyl]-benzamide;
4-chloro-N-[(cis-4-{[4-(dimethylamino)quinolin-2-yl]amino}cyclohexyl)-methyl]-3-nitrobenzamide;
3-cyano-N-[(cis-4-{[4-(dimethylamino)quinolin-2-yl]amino}cyclohexyl)-methyl]benzamide;
3,5-dichloro-N-[(cis-4-{[4-(dimethylamino)quinolin-2-yl]amino}-cyclohexyl)methyl]-benzamide;
3,4-dichloro-N-[(cis-4-{[4-(dimethylamino)quinolin-2-yl]amino}-cyclohexyl)methyl]-benzamide;
N-[(cis-4-{[4-(dimethylamino)quinolin-2-yl]amino}cyclohexyl)methyl]-2,2-diphenylacetamide;
N-[(cis-4-{[4-(dimethylamino)quinolin-2-yl]amino}cyclohexyl)methyl]-3,4-difluorobenzamide;
N-[(cis-4-{[4-(dimethylamino)quinolin-2-yl]amino}cyclohexyl)methyl]-3,5-difluorobenzamide;
N-[(cis-4-{[4-(dimethylamino)quinolin-2-yl]amino}cyclohexyl)methyl]-4-fluorobenzamide;
N-[(cis-4-{[4-(dimethylamino)quinolin-2-yl]amino}cyclohexyl)methyl]-3-fluoro-5-(trifluoromethyl)benzamide;
N-[(cis-4-{[4-(dimethylamino)quinolin-2-yl]amino}cyclohexyl)methyl]-4-methyl-3-nitrobenzamide;
N-[(cis-4-{[4-(dimethylamino)quinolin-2-yl]amino}cyclohexyl)methyl]-3-nitrobenzamide;
N-[(cis-4-{[4-(dimethylamino)quinolin-2-yl]amino}cyclohexyl)methyl]-2-phenoxybutanamide;
N-[(cis-4-{[4-(dimethylamino)quinolin-2-yl]amino}cyclohexyl)methyl]-2-phenoxypropanamide;
N-[(cis-4-{[4-(dimethylamino)quinolin-2-yl]amino}cyclohexyl)methyl]-3-methylbenzamide;
N-[(cis-4-{[4-(dimethylamino)quinolin-2-yl]amino}cyclohexyl)methyl]-3-(trifluoromethoxy)benzamide;
4-bromo-N-[(cis-4-{[4-(dimethylamino)quinolin-2-yl]amino}cyclohexyl)-methyl]-3-methylbenzamide;
N-[(cis-4-{[4-(dimethylamino)quinolin-2-yl]amino}cyclohexyl)methyl]-3-iodobenzamide;
3-chloro-N-[(cis-4-{[4-(dimethylamino)quinolin-2-yl]amino}cyclohexyl)-methyl]-2,4-difluorobenzamide;
N-[(cis-4-{[4-(dimethylamino)quinolin-2-yl]amino}cyclohexyl)methyl]-2,5-dimethyl-3-furamide;
3-chloro-N-[(cis-4-{[4-(dimethylamino)quinolin-2-yl]amino}cyclohexyl)-methyl]-4-fluorobenzamide;
N-[(cis-4-{[4-(dimethylamino)quinolin-2-yl]amino}cyclohexyl)methyl]-3-fluoro-4-methylbenzamide;
N-[(cis-4-{[4-(dimethylamino)quinolin-2-yl]amino}cyclohexyl)methyl]-3,5-dimethoxybenzamide;
N-[(cis-4-{[4-(dimethylamino)quinolin-2-yl]amino}cyclohexyl)methyl]-3,5-bis(trifluoromethyl)benzamide;
(2E)-N-[(cis-4-{[4-(dimethylamino)quinolin-2-yl]amino}cyclohexyl)-methyl]-3-(4-nitrophenyl)acrylamide;
N-[(cis-4-{[4-(dimethylamino)quinolin-2-yl]amino}cyclohexyl)methyl]-4-fluoro-3-methylbenzamide;
2,5-dichloro-N-[(cis-4-{[4-(dimethylamino)quinolin-2-yl]amino}-cyclohexyl)methyl]-thiophene-3-carboxamide;
2,6-dichloro-N-[(cis-4-{[4-(dimethylamino)quinolin-2-yl]amino}-cyclohexyl)methyl]-benzamide;
N-[(cis-4-{[4-(dimethylamino)quinolin-2-yl]amino}cyclohexyl)methyl]-2,4,6-trimethylbenzamide;
2,4,6-trichloro-N-[(cis-4-{[4-(dimethylamino)quinolin-2-yl]amino}-cyclohexyl)methyl]-benzamide;
(2E)-3-(2-chlorophenyl)-N-[(cis-4-{[4-(dimethylamino)quinolin-2-yl]amino}cyclohexyl)-methyl]acrylamide;
5-bromo-N-[(cis-4-{[4-(dimethylamino)quinolin-2-yl]amino}cyclohexyl)-methyl]thiophene-2-carboxamide;
N-[(cis-4-{[4-(dimethylamino)quinolin-2-yl]amino}cyclohexyl)methyl]-2-(2,3,6-trichlorophenyl)acetamide;
5-(4-chloro-2-nitrophenyl)-N-[(cis-4-{[4-(dimethylamino)quinolin-2-yl]amino}-cyclohexyl)methyl]-2-furamide;
5-chloro-N-[(cis-4-{[4-(dimethylamino)quinolin-2-yl]amino}cyclohexyl)-methyl]thiophene-2-carboxamide;
N-[(cis-4-{[4-(dimethylamino)quinolin-2-yl]amino}cyclohexyl)methyl]-5-iodo-2-furamide;
N-[(cis-4-{[4-(dimethylamino)quinolin-2-yl]amino}cyclohexyl)methyl]-2-(2-iodophenyl)-acetamide;
(2E)-N-[(cis-4-{[4-(dimethylamino)quinolin-2-yl]amino}cyclohexyl)-methyl]-3-(3-nitrophenyl)acrylamide;

2,2-bis(4-chlorophenyl)-N-[(cis-4-{[4-(dimethylamino)quinolin-2-yl]amino}cyclohexyl)-methyl]acetamide;
N-[(cis-4-{[4-(dimethylamino)quinolin-2-yl]amino}cyclohexyl)methyl]-5-nitrothiophene-2-carboxamide;
N-[(cis-4-{[4-(dimethylamino)quinolin-2-yl]amino}cyclohexyl)methyl]-3-methyl-4-nitrobenzamide;
N-[(cis-4-{[4-(dimethylamino)quinolin-2-yl]amino}cyclohexyl)methyl]-3-methoxy-4-nitrobenzamide;
N-[cis-4-(4-methyl-quinolin-2-ylamino)-cyclohexyl]-2-phenoxy-nicotinamide;
3,4-difluoro-N-[cis-4-(4-methyl-quinolin-2-ylamino)-cyclohexyl]-benzamide;
3,4-difluoro-N-[cis-4-(quinolin-2-ylamino)-cyclohexyl]-benzamide;
2-phenoxy-N-[cis-4-(quinolin-2-ylamino)-cyclohexyl]-nicotinamide;
3-chloro-N-[cis-4-(4-methyl-quinolin-2-ylamino)-cyclohexyl]-benzamide;
N-[cis-4-(4-chloro-quinolin-2-ylamino)-cyclohexyl]-2-phenoxy-nicotinamide;
3-methyl-N-[cis-4-(quinolin-2-ylamino)-cyclohexyl]-benzamide;
3-methoxy-N-[cis-4-(quinolin-2-ylamino)-cyclohexyl]-benzamide;
3-chloro-N-[cis-4-(quinolin-2-ylamino)-cyclohexyl]-benzamide;
5-nitro-thiophene-3-carboxylic acid [cis-4-(quinolin-2-ylamino)-cyclohexyl]-amide;
5-nitro-thiophene-3-carboxylic acid [cis-4-(4-methyl-quinolin-2-ylamino)-cyclohexyl]-amide;
3-chloro-4-fluoro-N-[cis-4-(quinolin-2-ylamino)-cyclohexyl]-benzamide;
3,5-dimethoxy-N-[cis-4-(quinolin-2-ylamino)-cyclohexyl]-benzamide;
3,4-dichloro-N-[cis-4-(quinolin-2-ylamino)-cyclohexyl]-benzamide;
benzo[2,3,1]oxadiazole-5-carboxylic acid [cis-4-(quinolin-2-ylamino)-cyclohexyl]-amide;
3-methyl-N-[cis-4-(4-methyl-quinolin-2-ylamino)-cyclohexyl]-benzamide;
3-methoxy-N-[cis-4-(4-methyl-quinolin-2-ylamino)-cyclohexyl]-benzamide;
4-cyano-N-[cis-4-(4-methyl-quinolin-2-ylamino)-cyclohexyl]-benzamide;
1-methyl-4-nitro-1H-pyrrole-2-carboxylic acid [cis-4-(quinolin-2-ylamino)-cyclohexyl]-amide;
9H-xanthene-9-carboxylic acid [cis-4-(quinolin-2-ylamino)-cyclohexyl]-amide;
5-(4-chloro-phenyl)-furan-2-carboxylic acid [cis-4-(quinolin-2-ylamino)-cyclohexyl]-arnide;
3-nitro-N-[cis-4-(quinolin-2-ylamino)-cyclohexyl]-benzamide;
4-fluoro-3-methyl-N-[cis-4-(quinolin-2-ylamino)-cyclohexyl]-benzamide;
3-bromo-N-[cis-4-(quinolin-2-ylamino)-cyclohexyl]-benzamide;
2-(2-bromo-phenoxy)-N-[cis-4-(quinolin-2-ylamino)-cyclohexyl]-nicotinamide;
3-cyano-N-[cis-4-(quinolin-2-ylamino)-cyclohexyl]-benzamide;
N-[cis-4-(quinolin-2-ylamino)-cyclohexyl]-trifluoromethyl-benzamide;
N-[cis-4-(4-chloro-quinolin-2-ylamino)-cyclohexyl]-3,4-difluoro-benzamide;
3,4-dichloro-N-[cis-4-(4-methyl-quinolin-2-ylamino)-cyclohexyl]-benzamide;
3-chloro-4-fluoro-N-[cis-4-(4-methyl-quinolin-2-ylamino)-cyclohexyl]-benzamide;
4-fluoro-3-methyl-N-[cis-4-(4-methyl-quinolin-2-ylamino)-cyclohexyl]-benzamide;
1-methyl-4-nitro-1H-pyrrole-2-carboxylic acid [cis-4-(4-methyl-quinolin-2-ylamino)-cyclohexyl]-amide;
9H-xanthene-9-carboxylic acid [cis-4-(4-methyl-quinolin-2-ylamino)-cyclohexyl]-amide;
5-bromo-furan-2-carboxylic acid [cis-4-(4-methyl-quinolin-2-ylamino)-cyclohexyl]-amide;
N-[cis-4-(4-methyl-quinolin-2-ylamino)-cyclohexyl]-2-*m*-tolyloxy-acetamide;
N-[cis-4-(quinolin-2-ylamino)-cyclohexyl]-2-*m*-tolyloxy-acetamide;
2,2-diphenyl-N-[cis-4-(quinolin-2-ylamino)-cyclohexyl]-acetamide;
5-bromo-furan-2-carboxylic acid [cis-4-(quinolin-2-ylamino)-cyclohexyl]-amide;
benzo[2,3,1]oxadiazole-5-carboxylic acid [cis-4-(4-methyl-quinolin-2-ylamino)-cyclohexyl]-amide;
3-bromo-N-[cis-4-(4-methyl-quinolin-2-ylamino)-cyclohexyl]-benzamide;
3-cyano-N-[cis-4-(4-methyl-quinolin-2-ylamino)-cyclohexyl]-benzamide;
N-[cis-4-(4-methyl-quinolin-2-ylamino)-cyclohexyl]-3-trifluoromethyl-benzamide;
N-[cis-4-(4-methyl-quinolin-2-ylamino)-cyclohexyl]-2,2-diphenyl-acetamide;
2-(4-fluoro-phenoxy)-N-[cis-4-(quinolin-2-ylamino)-cyclohexyl]-nicotinamide;
2-(4-fluoro-phenoxy)-N-(cis-4-(4-methyl-quinolin-2-ylamino)-cyclohexyl]-nicotinamide;
2-(3,4-difluoro-phenoxy)-N-[cis-4-(quinolin-2-ylamino)-cyclohexyl]-nicotinamide;
2-(3,4-difluoro-phenoxy)-N-[cis-4-(4-methyl-quinolin-2-ylamino)-cyclohexyl]-nicotinamide;
N-[cis-4-(quinolin-2-ylamino)-cyclohexyl]-2 *p*-tolyloxy-nicotinamide;
N-[cis-4-(4-methyl-quinolin-2-ylamino)-cyclohexyl]-2-*p*-tolyloxy-nicotinamide;
2-(4-chloro-phenoxy)-N-[cis-4-(quinolin-2-ylamino)-cyclohexyl]-nicotinamide;
2-(4-chloro-phenoxy)-N-[cis-4-(4-methyl-quinolin-2-ylamino)-cyclohexyl]-nicotinamide;
2-(4-bromo-phenoxy)-N-[cis-4-(quinolin-2-ylamino)-cyclohexyl]-nicotinamide;
2-(4-bromo-phenoxy)-N-[cis-4-(4-methyl-quinolin-2-ylamino)-cyclohexyl]-nicotinamide;
2-(4-methoxy-phenoxy)-N-[cis-4-(quinolin-2-ylamino)-cyclohexyl]-nicotinamide;

2-(4-methoxy-phenoxy)-N-[cis-4-(4-methyl-quinolin-2-ylamino)-cyclohexyl]-nicotinamide;
2-(3-chloro-4-fluoro-phenoxy)-N-[cis-4-(quinolin-2-ylamino)-cyclohexyl]-nicotinamide;
2-(3-chloro-4-fluoro-phenoxy)-N-[cis-4-(4-methyl-quinolin-2-ylamino)-cyclohexyl]-nicotinamide;
N-[cis-4-(quinolin-2-ylamino)-cyclohexyl]-2-*m*-tolyloxy-nicotinamide;
N-[cis-4-(4-methyl-quinolin-2-ylamino)-cyclohexyl]-2-*m*-tolyloxy-nicotinamide;
2-(3-methoxy-phenoxy)-N-[cis-4-(4-methyl-quinolin-2-ylamino)-cyclohexyl]-acetamide;
2-(3-chloro-phenoxy)-N-[cis-4-(4-methyl-quinolin-2-ylamino)-cyclohexyl]-acetamide;
2-(3-chloro-4-fluoro-phenoxy)-N-[cis-4-(4-methyl-quinolin-2-ylamino)-cyclohexyl]-acetamide;
2-(3,4-dichloro-phenoxy)-N-[cis-4-(4-methyl-quinolin-2-ylamino)-cyclohexyl]-acetamide;
C-(methyl-phenyl-amino)-N-[cis-4-(4-methyl-quinolin-2-ylamino)-cyclohexyl]-acetamide;
2-(3,4-dichloro-phenylamino)-N-[cis-4-(4-methyl-quinolin-2-ylamino)-cyclohexyl]-acetamide;
2-(3-methoxy-phenoxy)-N-[cis-4-(quinolin-2-ylamino)-cyclohexyl]-acetamide;
2-(3-chloro-phenoxy)-N-[cis-4-(quinolin-2-ylamino)-cyclohexyl]-acetamide;
2-(3-chloro-4-fluoro-phenoxy)-N-[cis-4-(quinolin-2-ylamino)-cyclohexyl]-acetamide;
2-(3,4-dichloro-phenoxy)-N-[cis-4-(quinolin-2-ylamino)-cyclohexyl]-acetamide;
C-(methyl-phenyl-amino)-N-[cis-4-(quinolin-2-ylamino)-cyclohexyl]-acetamide;
2-(3,4-dichloro-phenylamino)-N-[cis-4-(quinolin-2-ylamino)-cyclohexyl]-acetamide;
3-hydroxy-N-[cis-4-(quinolin-2-ylamino)-cyclohexyl]-benzamide;
N-[cis-4-(quinolin-2-ylamino)-cyclohexyl]-isophthalamic acid methyl ester;
N-[cis-4-(quinolin-2-ylamino)-cyclohexyl]-3-trifluoromethoxy-benzamide;
N-[cis-4-(4-methyl-quinolin-2-ylamino)-cyclohexyl]-bis-trifluoromethyl-benzamide;
N-[cis-4-(4-methyl-quinolin-2-ylamino)-cyclohexyl]-3-trifluoromethoxy-benzamide;
N-[cis-4-(4-amino-quinolin-2-ylamino)-cyclohexyl]-3,4-difluoro-benzamide;
C-(ethyl-phenyl-amino)-N-[cis-4-(quinolin-2-ylamino)-cyclohexyl]-acetamide;
C-(ethyl-phenyl-amino)-N-[cis-4-(4-methyl-quinolin-2-ylamino)-cyclohexyl]-acetamide;
3-hydroxy-N-[cis-4-(4-methyl-quinolin-2-ylamino)-cyclohexyl]-benzamide;
2-amino-N-[cis-4-(4-methyl-quinolin-2-ylamino)-cyclohexyl]-nicotinamide;
2,3-difluoro-N-[cis-4-(4-methyl-quinolin-2-ylamino)-cyclohexyl]-benzamide;
2,4-difluoro-N-[cis-4-(4-methyl-quinolin-2-ylamino)-cyclohexyl]-benzamide;
2,5-difluoro-N-[cis-4-(4-methyl-quinolin-2-ylamino)-cyclohexyl]-benzamide;
2,6-difluoro-N-[cis-4-(4-methyl-quinolin-2-ylamino)-cyclohexyl]-benzamide;
3,5-difluoro-N-[cis-4-(4-methyl-quinolin-2-ylamino)-cyclohexyl]-benzamide;
C-[(4-chloro-phenyl)-ethyl-amino]-N-[cis-4-(4-methyl-quinolin-2-ylamino)-cyclohexyl]-acetamide;
4-chloro-3-fluoro-N-[cis-4-(4-methyl-quinolin-2-ylamino)-cyclohexyl]-benzamide;
4-fluoro-N-[cis-4-(4-methyl-quinolin-2-ylamino)-cyclohexyl]-benzamide;
3-fluoro-N-[cis-4-(4-methyl-quinolin-2-ylamino)-cyclohexyl]-benzamide;
2-fluoro-N-[cis-4-(4-methyl-quinolin-2-ylamino)-cyclohexyl]-benzamide;
4-chloro-N-[cis-4-(4-methyl-quinolin-2-ylamino)-cyclohexyl]-benzamide;
N-[cis-4-(4-methyl-quinolin-2-ylamino)-cyclohexyl]-isophthalamic acid methyl ester;
3,5-difluoro-N-[cis-4-(quinolin-2-ylamino)-cyclohexyl]-benzamide;
4-chloro-3-fluoro-N-[cis-4-(quinolin-2-ylamino)-cyclohexyl]-benzamide;
C-[(4-chloro-phenyl)-ethyl-amino]-N-[cis-4-(quinolin-2-ylamino)-cyclohexyl]-acetamide;
6-chloro-N-[cis-4-(4-methyl-quinolin-2-ylamino)-cyclohexyl]-nicotinamide;
6-dimethylamino-N-[cis-4-(4-methyl-quinolin-2-ylamino)-cyclohexyl]-nicotinamide;
3-hydroxymethyl-N-[cis-4-(4-methyl-quinolin-2-ylamino)-cyclohexyl]-benzamide;
N-[cis-4-(4-methyl-quinolin-2-ylamino)-cyclohexyl]-isophthalamide;
3-chloro-5-fluoro-N-[cis-4-(4-methyl-quinolin-2-ylamino)-cyclohexyl]-benzamide;
3,4,5-trifluoro-N-[cis-4-(4-methyl-quinolin-2-ylamino)-cyclohexyl]-benzamide;
pyridine-2-carboxylic acid [cis-4-(4-methyl-quinolin-2-ylamino)-cyclohexyl]-amide;
4-chloro-pyridine-2-carboxylic acid [cis-4-(4-methyl-quinolin-2-ylamino)-cyclohexyl]-amide;
5-bromo-N-[cis-4-(4-methyl-quinolin-2-ylamino)-cyclohexyl]-nicotinamide;
N-[cis-4-(4-methyl-quinolin-2-ylamino)-cyclohexyl]-6-trifluoromethyl-nicotinamide;
3,4-difluoro-N-[cis-4-(4-methyl-quinolin-2-ylamino)-cyclohexylmethyl]-benzamide;
N-[cis-4-(4-methyl-quinolin-2-ylamino)-cyclohexylmethyl]-2-phenoxy-nicotinamide;
N-[cis-4-(4-dimethylamino-quinolin-2-ylamino)-cyclohexylmethyl]-3,4-difluoro-benzamide;
3,4-difluoro-N-[cis-4-(quinolin-2-ylamino)-cyclohexylmethyl]-benzamide;
2-phenoxy-N-[cis-4-(quinolin-2-ylamino)-cyclohexylmethyl]-nicotinamide;
4-methyl-N-{cis-4-[(4-methylquinolin-2-yl)amino]cyclohexyl}benzamide;

2-(4-chlorophenoxy)-N-{cis-4-[(4-methylquinolin-2-yl)amino]cyclohexyl}acetamide;
3,4,5-trimethoxy-N-{cis-4-[(4-methylquinolin-2-yl)amino]cyclohexyl}benzamide;
2-(3,4-difluorophenyl)-N-cis-4-[(4-methylquinolin-2-yl)amino]cyclohexyl}acetamide;
2-(2-bromo-4,5-dimethoxyphenyl)-N-{cis-4-[(4-methylquinolin-2-yl)amino]cyclohexyl}-acetamide;
2,6-dimethoxy-N-{cis-4-[(4-methylquinolin-2-yl)amino]cyclohexyl}nicotinamide;
N-{cis-4-[(4-methylquinolin-2-yl)amino]cyclohexyl}-4-(trifluoromethoxy)benzamide;
5-chloro-N-[cis-4-(4-methyl-quinolin-2-ylamino)-cyclohexyl]-nicotinamide; and
5-fluoro-N-[cis-4-(4-methyl-quinolin-2-ylamino)-cyclohexyl]-nicotinamide;

or a pharmaceutically acceptable salt, hydrate, or solvate thereof.

In some embodiments, compounds of the present invention are of Formula (I) wherein the compound is selected from the group consisting of:

3-bromo-N-(cis-4-{[4-(dimethylamino)quinolin-2-yl]amino}cyclohexyl)-benzamide;
N-(cis-4-{[4-(dimethylamino)quinolin-2-yl]amino}cyclohexyl)-2,1,3-benzoxadiazole-5-carboxamide;
3-chloro-N-(cis-4-{[4-(dimethylamino)quinolin-2-yl]amino}cyclohexyl)-benzamide;
4-chloro-N-(cis-4-{[4-(dimethylamino)quinolin-2-yl]amino}cyclohexyl)-benzamide;
4-chloro-N-(cis-4-{[4-(dimethylamino)quinolin-2-yl]amino}cyclohexyl)-3-nitrobenzamide;
3,4-dichloro-N-(cis-4-{[4-(dimethylamino)quinolin-2-yl]amino}-cyclohexyl)benzamide;
N-(cis-4-{[4-(dimethylamino)quinolin-2-yl]amino}cyclohexyl)-3,4-difluorobenzamide;
N-(cis-4-{[4-(dimethylamino)quinolin-2-yl]amino}cyclohexyl)-4-fluorobenzamide;
N-(cis-4-{[4-(dimethylamino)quinolin-2-yl]amino}cyclohexyl)-3-nitrobenzamide;
N-(cis-4-{[4-(dimethylamino)quinolin-2-yl]amino}cyclohexyl)-2-phenoxybutanamide;
N-(cis-4-{[4-(dimethylamino)quinolin-2-yl]amino}cyclohexyl)-2-phenoxypropanamide;
N-(cis-4-{[4-(dimethylamino)quinolin-2-yl]amino}cyclohexyl)-3-methylbenzamide;
4-bromo-N-(cis-4-{[4-(dimethylamino)quinolin-2-yl]amino}cyclohexyl)-3-methylbenzamide;
N-(cis-4-{[4-(dimethylamino)quinolin-2-yl]amino}cyclohexyl)-2,5-dimethyl-3-furamide;
3-chloro-N-(cis-4-{[4-(dimethylamino)quinolin-2-yl]amino}cyclohexyl)-4-fluorobenzamide;
N-(cis-4-{[4-(dimethylamino)quinolin-2-yl]amino}cyclohexyl)-3,5-dimethoxybenzamide;
N-(cis-4-{[4-(dimethylamino)quinolin-2-yl]amino}cyclohexyl)-4-fluoro-3-methylbenzamide;
2-(4-chlorophenoxy)-N-(cis-4-{[4-(dimethylamino)quinolin-2-yl]amino}-cyclohexyl)-acetamide;
3-(2-chlorophenyl)-N-(cis-4-{[4-(dimethylamino)quinolin-2-yl]amino}-cyclohexyl)-5-methylisoxazole-4-carboxamide;
3-(2-chloro-6-fluorophenyl)-N-(cis-4-{[4-(dimethylamino)quinolin-2-yl]amino}cyclohexyl)-5-methylisoxazole-4-carboxamide;
N-(cis-4-{[4-(dimethylamino)quinolin-2-yl]amino}cyclohexyl)-4-fluoro-3-(trifluoromethyl)benzamide;
N-(cis-4-{[4-(dimethylamino)quinolin-2-yl]amino}cyclohexyl)-2-(4-methoxyphenoxy)-5-nitrobenzamide;
N-(cis-4-{[4-(dimethylamino)quinolin-2-yl]amino}cyclohexyl)-5-nitro-2-furamide;
N-(cis-4-{[4-(dimethylamino)quinolin-2-yl]amino}cyclohexyl)-2-phenoxyacetamide;
2-(3-chlorophenoxy)-N-(cis-4-{[4-(dimethylamino)quinolin-2-yl]amino}-cyclohexyl)-acetamide;
3-(2,6-dichlorophenyl)-N-(cis-4-{[4-(dimethylamino)quinolin-2-yl]amino}-cyclohexyl)-5-methylisoxazole-4-carboxamide;
N-(cis-4-{[4-(dimethylamino)quinolin-2-yl]amino}cyclohexyl)-2-phenoxynicotinamide;
N-(cis-4-{[4-(dimethylamino)quinolin-2-yl]amino}cyclohexyl)-2-(4-methylphenoxy)-nicotinamide;
N-(cis-4-{[4-(dimethylamino)quinolin-2-yl]amino}cyclohexyl)-2-(2-thienyl)-1,3-thiazole-4-carboxamide;
N-(cis-4-{[4-(dimethylamino)quinolin-2-yl]amino}cyclohexyl)-2-(2,3,6-trichlorophenyl)-acetamide;
N-(cis-4-{[4-(dimethylamino)quinolin-2-yl]amino}cyclohexyl)-5-iodo-2-furamide;
N-(cis-4-{[4-(dimethylamino)quinolin-2-yl]amino}cyclohexyl)-5-nitrothiophene-2-carboxamide;
N-(cis-4-{[4-(dimethylamino)quinolin-2-yl]amino}cyclohexyl)-3-methyl-4-nitrobenzamide;
N-(cis-4-{[4-(dimethylamino)quinolin-2-yl]amino}cyclohexyl)-3-methoxy-4-nitrobenzamide;
5-bromo-N-(cis-4-{[4-(dimethylamino)quinolin-2-yl]amino}cyclohexyl)-2-furamide;
4,5-dibromo-N-(cis-4-{[4-(dimethylamino)quinolin-2-yl]amino}-cyclohexyl)-2-furamide;
N-(cis-4-{[4-(dimethylamino)quinolin-2-yl]amino}cyclohexyl)-2-(1H-indol-3-yl)acetamide;
N-(cis-4-{[4-(dimethylamino)quinolin-2-yl]amino}cyclohexyl)-5-(3-nitrophenyl)-2-furamide;
N-(cis-4-{[4-(dimethylamino)quinolin-2-yl]amino}cyclohexyl)-5-nitrothiophene-3-carboxamide;
N-(cis-4-{[4-(dimethylamino)quinolin-2-yl]amino}cyclohexyl)-1-methyl-4-nitro-1H-pyrrole-2-carboxamide;
N-(cis-4-{[4-(dimethylamino)quinolin-2-yl]amino}cyclohexyl)-4-nitrobenzamide;
N-(cis-4-{[4-(dimethylamino)quinolin-2-yl]amino}cyclohexyl)-3-fluoro-4-(trifluoromethyl)benzamide;

3-bromo-N-[(cis-4-{[4-(dimethylamino)quinolin-2-yl]amino}cyclohexyl)-methyl]-benzamide;
N-[(cis-4-{[4-(dimethylamino)quinolin-2-yl]amino}cyclohexyl)methyl]-2,1,3-benzoxadiazole-5-carboxamide;
3-chloro-N-[(cis-4-{[4-(dimethylamino)quinolin-2-yl]amino}cyclohexyl)-methyl]-benzamide;
4-chloro-N-[(cis-4-{[4-(dimethylamino)quinolin-2-yl]amino}cyclohexyl)-methyl]-benzamide;
4-chloro-N-[(cis-4-{[4-(dimethylamino)quinolin-2-yl]amino}cyclohexyl)-methyl]-3-nitrobenzamide;
3,4-dichloro-N-[(cis-4-{[4-(dimethylamino)quinolin-2-yl]amino}-cyclohexyl)methyl]-benzamide;
N-[(cis-4-{[4-(dimethylamino)quinolin-2-yl]amino}cyclohexyl)methyl]-3,4-difluorobenzamide;
N-[(cis-4-{[4-(dimethylamino)quinolin-2-yl]amino}cyclohexyl)methyl]-4-fluorobenzamide;
N-[(cis-4-{[4-(dimethylamino)quinolin-2-yl]amino}cyclohexyl)methyl]-3-nitrobenzamide;
N-[(cis-4-{(4-(dimethylamino)quinolin-2-yl]amino}cyclohexyl)methyl]-2-phenoxybutanamide;
N-[(cis-4-{[4-(dimethylamino)quinolin-2-yl]amino}cyclohexyl)methyl]-2-phenoxypropanamide;
N-[(cis-4-{[4-(dimethylamino)quinolin-2-yl]amino}cyclohexyl)methyl]-3-methylbenzamide;
4-bromo-N-[(cis-4-{[4-(dimethylamino)quinolin-2-yl]amino}cyclohexyl)-methyl]-3-methylbenzamide;
N-[(cis-4-{[4-(dimethylamino)quinolin-2-yl]amino}cyclohexyl)methyl]-2,5-dimethyl-3-furamide;
3-chloro-N-[(cis-4-{[4-(dimethylamino)quinolin-2-yl]amino}cyclohexyl)-methyl]-4-fluorobenzamide;
N-[(cis-4-{[4-(dimethylamino)quinolin-2-yl]amino}cyclohexyl)methyl]-3,5-dimethoxybenzamide;
N-[(cis-4-{[4-(dimethylamino)quinolin-2-yl]amino}cyclohexyl)methyl]-4-fluoro-3-methylbenzamide;
N-[(cis-4-{[4-(dimethylamino)quinolin-2-yl]amino}cyclohexyl)methyl]-2,4,6-trimethylbenzamide;
2,4,6-trichloro-N-[(cis-4-{[4-(dimethylamino)quinolin-2-yl]amino}-cyclohexyl)methyl]-benzamide;
N-[(cis-4-{[4-(dimethylamino)quinolin-2-yl]amino}cyclohexyl)methyl]-2-(2,3,6-trichlorophenyl)acetamide;
N-[(cis-4-{[4-(dimethylamino)quinolin-2-yl]amino}cyclohexyl)methyl]-5-iodo-2-furamide;
N-[(cis-4-{[4-(dimethylamino)quinolin-2-yl]amino}cyclohexyl)methyl]-5-nitrothiophene-2-carboxamide;
N-[(cis-4-{[4-(dimethylamino)quinolin-2-yl]amino}cyclohexyl)methyl]-3-methyl-4-nitrobenzamide;
N-[(cis-4-{[4-(dimethylamino)quinolin-2-yl]amino}cyclohexyl)methyl]-3-methoxy-4-nitrobenzamide;
N-[cis-4-(4-methyl-quinolin-2-ylamino)-cyclohexyl]-2-phenoxy-nicotinamide;
3,4-difluoro-N-[cis-4-(4-methyl-quinolin-2-ylamino)-cyclohexyl]-benzamide;
3,4-difluoro-N-[cis-4-(quinolin-2-ylamino)-cyclohexyl]-benzamide;
2-phenoxy-N-[cis-4-(quinolin-2-ylamino)-cyclohexyl]-nicotinamide;
3-chloro-N-[cis-4-(4-methyl-quinolin-2-ylamino)-cyclohexyl]-benzamide;
3-methyl-N-(cis-4-(quinolin-2-ylamino)-cyclohexyl]-benzamide;
3-methoxy-N-[cis-4-(quinolin-2-ylamino)-cyclohexyl]-benzamide;
3-chloro-N-[cis-4-(quinolin-2-ylamino)-cyclohexyl]-benzamide;
5-nitro-thiophene-3-carboxylic acid [cis-4-(quinolin-2-ylamino)-cyclohexyl]-amide;
5-nitro-thiophene-3-carboxylic acid [cis-4-(4-methyl-quinolin-2-ylamino)-cyclohexyl]-amide;
3-chloro-4-fluoro-N-[cis-4-(quinolin-2-ylamino)-cyclohexyl]-benzamide;
3,5-dimethoxy-N-[cis-4-(quinolin-2-ylamino)-cyclohexyl]-benzamide;
3,4-dichloro-N-[cis-4-(quinolin-2-ylamino)-cyclohexyl]-benzamide;
benzo[2,3,1]oxadiazole-5-carboxylic acid [cis-4-(quinolin-2-ylamino)-cyclohexyl]-amide;
3-methyl-N-[cis-4-(4-methyl-quinolin-2-ylamino)-cyclohexyl]-benzamide;
3-methoxy-N-[cis-4-(4-methyl-quinolin-2-ylamino)-cyclohexyl]-benzamide;
4-cyano-N-[cis-4-(4-methyl-quinolin-2-ylamino)-cyclohexyl]-benzamide;
1-methyl-4-nitro-1H-pyrrole-2-carboxylic acid [cis-4-(quinolin-2-ylamino)-cyclohexyl]-amide;
9H-xanthene-9-carboxylic acid [cis-4-(quinolin-2-ylamino)-cyclohexyl]-amide;
3-nitro-N-[cis-4-(quinolin-2-ylamino)-cyclohexyl]-benzamide;
4-fluoro-3-methyl-N-[cis-4-(quinolin-2-ylamino)-cyclohexyl]-benzamide;
3-bromo-N-[cis-4-(quinolin-2-ylamino)-cyclohexyl]-benzamide;
2-(2-bromo-phenoxy)-N-[cis-4-(quinolin-2-ylamino)-cyclohexyl]-nicotinamide;
3-cyano-N-[cis-4-(quinolin-2-ylamino)-cyclohexyl]-benzamide;
N-[cis-4-(quinolin-2-ylamino)-cyclohexyl]-trifluoromethyl-benzamide;
N-[cis-4-(4-chloro-quinolin-2-ylamino)-cyclohexyl]-3,4-difluoro-benzamide;
3,4-dichloro-N-[cis-4-(4-methyl-quinolin-2-ylamino)-cyclohexyl]-benzamide;
3-chloro-4-fluoro-N-[cis-4-(4-methyl-quinolin-2_ylamino)-cyclohexyl]-benzamide;
4-fluoro-3-methyl-N-[cis-4-(4-methyl-quinolin-2-ylamino)-cyclohexyl]-benzamide;
1-methyl-4-nitro-1H-pyrrole-2-carboxylic acid [cis-4-(4-methyl-quinolin-2-ylamino)-cyclohexyl]-amide;
9H-xanthene-9-carboxylic acid [cis-4-(4-methyl-quinolin-2-ylamino)-cyclohexyl]-amide;
5-bromo-furan-2-carboxylic acid [cis-4-(4-methyl-quinolin-2-ylaniino)-cyclohexyl]-amide;
N-[cis-4-(4-methyl-quinolin-2-ylamino)-cyclohexyl]-2-*m*-tolyloxy-acetamide;
N-[cis-4-(quinolin-2-ylamino)-cyclohexyl]-2-*m*-tolyloxy-acetamide;

2,2-diphenyl-N-[cis-4-(quinolin-2-ylamino)-cyclohexyl]-acetamide;
5-bromo-furan-2-carboxylic acid [cis-4-(quinolin-2-ylamino)-cyclohexyl]-amide;
benzo[2,3,1]oxadiazole-5-carboxylic acid [cis-4-(4-methyl-quinolin-2-ylamino)-cyclohexyl]-amide;
3-bromo-N-[cis-4-(4-methyl-quinolin-2-ylamino)-cyclohexyl)-benzamide;
3-cyano-N-[cis-4-(4-methyl-quinolin-2-ylamino)-cyclohexyl]-benzamide;
N-[cis-4-(4-methyl-quinolin-2-ylamino)-cyclohexyl]-3-trifluoromethyl-benzamide;
N-[cis-4-(4-methyl-quinolin-2-ylamino)-cyclohexyl]-2,2-diphenyl-acetamide;
2-(4-fluoro-phenoxy)-N-[cis-4-(quinolin-2-ylamino)-cyclohexyl]-nicotinamide;
2-(4-fluoro-phenoxy)-N-[cis-4-(4-methyl-quinolin-2-ylamino)-cyclohexyl]-nicotinamide;
2-(3,4-difluoro-phenoxy)-N-[cis-4-(quinolin-2-ylamino)-cyclohexyl]-nicotinamide;
2-(3,4-difluoro-phenoxy)-N-[cis-4-(4-methyl-quinolin-2-ylamino)-cyclohexyl]-nicotinamide;
N-[cis-4-(quinolin-2-ylamino)-cyclohexyl)-2-*p*-tolyloxy-nicotinamide;
N-[cis-4-(4-methyl-quinolin-2-ylamino)-cyclohexyl]-2-*p*-tolyloxy-nicotinamide;
2-(4-chloro-phenoxy)-N-[cis-4-(quinolin-2-ylamino)-cyclohexyl]-nicotinamide;
2-(4-chloro-phenoxy)-N-[cis-4-(4-methyl-quinolin-2-ylamino)-cyclohexyl]-nicotinamide;
2-(4-bromo-phenoxy)-N-[cis-4-(quinolin-2-ylamino)-cyclohexyl]-nicotinamide;
2-(4-bromo-phenoxy)-N-[cis-4-(4-methyl-quinolin-2-ylamino)-cyclohexyl]-nicotinamide;
2-(4-methoxy-phenoxy)-N-[cis-4-(quinolin-2-ylamino)-cyclohexyl]-nicotinamide;
2-(4-methoxy-phenoxy)-N-[cis-4-(4-methyl-quinolin-2-ylamino)-cyclohexyl]-nicotinamide;
2-(3-chloro-4-fluoro-phenoxy)-N-[cis-4-(4-methyl-quinolin-2-ylamino)-cyclohexyl]-nicotinamide;
N-[cis-4-(quinolin-2-ylamino)-cyclohexyl]-2-*m*-tolyloxy-nicotinamide;
N-[cis-4-(4-methyl-quinolin-2-ylamino)-cyclohexyl]-2-*m*-tolyloxy-nicotinamide;
2-(3-methoxy-phenoxy)-N-[cis-4-(4-methyl-quinolin-2-ylamino)-cyclohexyl]-acetamide;
2-(3-chloro-phenoxy)-N-[cis-4-(4-methyl-quinolin-2-ylamino)-cyclohexyl]-acetamide;
2-(3-chloro-4-fluoro-phenoxy)-N-[cis-4-(4-methyl-quinolin-2-ylamino)-cyclohexyl]-acetamide;
2-(3,4-dichloro-phenoxy)-N-[cis-4-(4-methyl-quinolin-2-ylamino)-cyclohexyl]-acetamide;
C-(methyl-phenyl-amino)-N-[cis-4-(4-methyl-quinolin-2-ylamino)-cyclohexyl]-acetamide;
2-(3-methoxy-phenoxy)-N-[cis-4-(quinolin-2-ylamino)-cyclohexyl]-acetamide;
2-(3-chloro-phenoxy)-N-[cis-4-(quinolin-2-ylamino)-cyclohexyl]-acetamide;
2-(3-chloro-4-fluoro-phenoxy)-N-[cis-4-(quinolin-2-ylamino)-cyclohexyl]-acetamide;
2-(3,4-dichloro-phenoxy)-N-[cis-4-(quinolin-2-ylamino)-cyclohexyl]-acetamide;
C-(methyl-phenyl-amino)-N-[cis-4-(quinolin-2-ylamino)-cyclohexyl]-acetamide;
N-[cis-4-(4-methyl-quinolin-2-ylamino)-cyclohexyl]-3-trifluoromethoxy-benzamide;
N-[cis-4-(4-amino-quinolin-2-ylamino)-cyclohexyl]-3,4-difluoro-benzamide;
C-(ethyl-phenyl-amino)-N-[cis-4-(quinolin-2-ylamino)-cyclohexyl]-acetamide;
C-(ethyl-phenyl-amino)-N-[cis-4-(4-methyl-quinolin-2-ylamino)-cyclohexyl]-acetamide;
3-hydroxy-N-[cis-4-(4-methyl-quinolin-2-ylamino)-cyclohexyl]-benzamide;
2,4-difluoro-N-[cis-4-(4-methyl-quinolin-2-ylamino)-cyclohexyl]-benzamide;
3,5-difluoro-N-[cis-4-(4-methyl-quinolin-2-ylamino)-cyclohexyl]-benzamide;
C-[(4-chloro-phenyl)-ethyl-amino]-N-[cis-4-(4-methyl-quinolin-2-ylamino)-cyclohexyl]-acetamide;
4-chloro-3-fluoro-N-[cis-4-(4-methyl-quinolin-2-ylamino)-cyclohexyl]-benzamide;
4-fluoro-N-[cis-4-(4-methyl-quinolin-2-ylamino)-cyclohexyl]-benzamide;
3-fluoro-N-[cis-4-(4-methyl-quinolin-2-ylamino)-cyclohexyl]-benzamide;
4-chloro-N-[cis-4-(4-methyl-quinolin-2-ylamino)-cyclohexyl]-benzamide;
N-[cis-4-(4-methyl-quinolin-2-ylamino)-cyclohexyl]-isophthalamic acid methyl ester;
3,5-difluoro-N-[cis-4-(quinolin-2-ylamino)-cyclohexyl]-benzamide;
4-chloro-3-fluoro-N-[cis-4-(quinolin-2-ylamino)-cyclohexyl]-benzamide;
C-[(4-chloro-phenyl)-ethyl-amino]-N-[cis-4-(quinolin-2-ylamino)-cyclohexyl]-acetamide;
6-chloro-N-[cis-4-(4-methyl-quinolin-2-ylamino)-cyclohexyl]-nicotinamide;
3-chloro-5-fluoro-N-[cis-4-(4-methyl-quinolin-2-ylamino)-cyclohexyl]-benzamide;
3,4,5-trifluoro-N-[cis-4-(4-methyl-quinolin-2-ylamino)-cyclohexyl]-benzamide;
5-bromo-N-[cis-4-(4-methyl-quinolin-2-ylamino)-cyclohexyl]-nicotinamide;
4-methyl-N-{cis-4-[(4-methylquinolin-2-yl)amino]cyclohexyl}benzamide;
2-(4-chlorophenoxy)-N-{cis-4-[(4-methylquinolin-2-yl)amino]cyclohexyl}-acetamide;
3,4,5-trimethoxy-N-{cis-4-[(4-methylquinolin-2-yl)amino]cyclohexyl}benzamide;
2-(3,4-difluorophenyl)-N-{cis-4-[(4-methylquinolin-2-yl)amino]cyclohexyl}-acetamide;
2-(2-bromo-4,5-dimethoxyphenyl)-N-{cis-4-[(4-methylquinolin-2-yl)amino]-cyclohexyl}-acetamide;
2,6-dimethoxy-N-{cis-4-[(4-methylquinolin-2-yl)amino]cyclohexyl}nicotinamide;

N-{cis-4-[(4-methylquinolin-2-yl)amino]cyclohexyl}-4-(trifluoromethoxy)-benzamide;
5-chloro-N-[cis-4-(4-methyl-quinolin-2-ylamino)-cyclohexyl]-nicotinamide; and
5-fluoro-N-[cis-4-(4-methyl-quinolin-2-ylamino)-cyclohexyl]-nicotinamide;

or a pharmaceutically acceptable salt, hydrate, or solvate thereof.

In some embodiments of the present invention, $R_1$ is selected from the group consisting of:

$C_{1-6}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:
  - •• halogen,
  - •• $C_{1-5}$ alkyl,
  - •• $C_{1-5}$ alkyl substituted by halogen,
  - •• $C_{1-5}$ alkoxy, and
  - •• $C_{1-5}$ alkoxy substituted by halogen,

L is Formula (XV);
Y is $-C(O)NR_5-$;
wherein carbocyclic aryl is phenyl; and
halogen is fluoro, chloro, or bromo;

or a pharmaceutically acceptable salt, hydrate, or solvate thereof.

In some embodiments of the present invention, $R_1$ is selected from the group consisting of:

$C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:
  - •• halogen,
  - •• $C_{1-5}$ alkyl, and
  - •• $C_{1-5}$ alkyl substituted by halogen,

wherein carbocyclic aryl is phenyl; and
halogen is fluoro, chloro, or bromo;

or a pharmaceutically acceptable salt, hydrate, or solvate thereof.

In some embodiments of the present invention, $R_2$ is methyl; p is 0; $R_3$ and $R_4$ are both hydrogen; A and B are both single bonds; and $R_5$ is hydrogen: or a pharmaceutically acceptable salt, hydrate, or solvate thereof.

In some embodiments, compounds of the present invention are of Formula (I) wherein the compound is selected from the group consisting of:

cis-N-[(1R)-1-(4-bromophenyl)ethyl]-4-[(4-methylquinolin-2-yl)amino]-cyclohexanecarboxamide;
cis-N-{(1S)-1-[3,5-bis(trifluoromethyl)phenyl]ethyl}-4-[(4-methylquinolin-2-yl)amino]-cyclohexanecarboxamide;
cis-N-[(1R)-1-(2-fluorophenyl)ethyl]-4-[(4-methylquinolin-2-yl)amino]-cyclohexanecarboxamide;
cis-N-[(1S)-1-(2-fluorophenyl)ethyl]-4-[(4-methylquinolin-2-yl)amino]-cyclohexanecarboxamide;
cis-4-[(4-methylquinolin-2-yl)aznino]-N-{(1S)-1-[2-(trifluoromethyl)phenyl]ethyl}-cyclohexanecarboxamide;
cis-4-[(4-methylquinolin-2-yl)amino]-N-{(1S)-1-[3-(trifluoromethyl)phenyl]ethyl}-cyclohexanecarboxamide;
cis-N-[(1R)-1-(4-chlorophenyl)ethyl]-4-[(4-methylquinolin-2-yl)amino]-cyclohexanecarboxamide; and
cis-N-[(1S)-1-(4-chlorophenyl)ethyl]-4-[(4-methylquinolin-2-yl)amino]-cyclohexanecarboxamide;

or a pharmaceutically acceptable salt, hydrate, or solvate thereof.

In some embodiments, compounds of the present invention are of Formula (I) wherein the compound is selected from the group consisting of:

cis-N-[(1R)-1-(4-bromophenyl)ethyl]-4-[(4-methylquinolin-2-yl)amino]-cyclohexanecarboxamide;
cis-N-[(1S)-1-(2-auorophenyl)ethyl]-4-[(4-methylquinolin-2-yl)amino]-cyclohexanecarboxamide;
cis-4-[(4-methylquinolin-2-yl)amino]-N-{(1S)-1-[2-(trifluoromethyl)phenyl]ethyl}-cyclohexanecarboxamide; and
cis-4-[(4-methylquinolin-2-yl)amino]-N-{(1S)-1-[3-(trifluoromethyl)phenyl]ethyl}-cyclohexanecarboxamide;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- $C_{1-5}$ alkoxycarbonyl,
- $C_{1-5}$ alkylthio,
- carbocyclic aryl,
- carbocyclic aryl substituted by substituent(s) independently selected from the group consisting of:
  - •• halogen,
  - •• $C_{1-5}$ alkyl, and
  - •• $C_{2-5}$ alkenyl,

(ii) $C_{3-6}$cycloalkyl, and
$C_{3-6}$ cycloalkyl substituted by carbocyclic aryl,
(iii) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by halogen,
- $C_{1-5}$ alkoxycarbonyl,
- $C_{1-5}$ alkoxy,
- $C_{1-5}$ alkoxy substituted by carbocyclic aryl,
- $C_{3-6}$ cycloalkoxy,
- carbocyclic aryloxy,
- $C_{1-5}$ alkylthio, and
- carbocyclic aryl,

(iv) heterocyclyl, and
heterocyclyl substituted by substituent(s) independently selected from the group consisting of:

- $C_{1-5}$ alkyl,
- $C_{1-5}$ alkyl substituted by halogen, and
- carbocyclic aryl;

L is Formula (VII);
Y is $-C(O)NR_5-$;
wherein carbocyclic aryl is phenyl or naphthyl;
heterocyclyl is 2,3-dihydro-benzo[1,4]dioxinyl,
3,4-dihydro-2H-benzo[b][1,4]-dioxepinyl, benzo[1,3]dioxolyl, furyl, or isoxazolyl; and
halogen is fluoro, chloro, bromo, or iodo;

or a pharmaceutically acceptable salt, hydrate, or solvate thereof.

In some embodiments of the present invention, $R_2$ is hydrogen, methyl, methylamino, or dimethylamino; p is 0; $R_3$ and $R_4$ are hydrogen; A is a single bond; B is a single bond or $-CH_2-$; $R_5$ is hydrogen; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- $C_{1-5}$ alkoxycarbonyl,
- carbocyclic aryl, and
- carbocyclic aryl substituted by halogen,

(ii) carbocyclic aryl, and
carbocyclic aryl substituted by substituent(s) independently selected from the group consisting of:

- halogen,
- nitro,
- $C_{1-5}$ alkyl,
- $C_{1-5}$ alkyl substituted by halogen, and
- $C_{1-5}$ alkoxy,

(iii) heterocyclyl,
heterocyclyl substituted by $C_{1-5}$ alkyl, and
heterocyclyl substituted by carbocyclic aryl;
wherein carbocyclic aryl is phenyl or naphthyl;
heterocyclyl is isoxazolyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(2-chlorophenyl)-N'-(cis-4-[4-(dimethylamino)quinolin-2-yl]amino}-cyclohexyl)urea;
N-(cis-4-{[4-(dimethylamino)quinolin-2-yl]amino}cyclohexyl)-N'-(2-ethyl-6-methylphenyl)urea;
N-(cis-4-{[4-(dimethylamino)quinolin-2-yl]amino}cyclohexyl)-N'-mesitylurea;
N-(cis-4-{[4-(dimethylamino)quinolin-2-yl]amino}cyclohexyl)-N'-(2,4,6-trichlorophenyl)-urea;
N-(cis-4-{[4-(dimethylamino)quinolin-2-yl]amino}cyclohexyl)-N'-(2,4,6-tribromophenyl)-urea;
N-(2,4-dibromo-6-fluorophenyl)-N'-(cis-4-{[4-(dimethylamino)quinolin-2-yl]amino}-cyclohexyl)urea;
N-(2,6-diethylphenyl)-N'-(cis-4-{[4-(dimethylamino)quinolin-2-yl]amino}-cyclohexyl)urea;
N-(2-chlorobenzyl)-N'-(cis-4-{[4-(dimethylamino)quinolin-2-yl]amino}-cyclohexyl)urea;
N-(cis-4-{[4-(dimethylamino)quinolin-2-yl]amino}cyclohexyl)-N'-(2-ethyl-6-isopropylphenyl)urea;
N-(cis-4-{[4-(dimethylamino)quinolin-2-yl]amino}cyclohexyl)-N'-(2-isopropyl-6-methylphenyl)urea;
N-(2-tert-butyl-6-methylphenyl)-N'-(cis-4-{[4-(dimethylamino)quinolin-2-yl]amino}-cyclohexyl)urea;
N-(cis-4-{[4-(dimethylamino)quinolin-2-yl]amino}cyclohexyl)-N'-(diphenylmethyl)urea;
N-(4-bromo-2,6-dimethylphenyl)-N'-(cis-4-{[4-(dimethylamino)quinolin-2-yl]amino}-cyclohexyl)urea;
N-(cis-4-{[4-(dimethylamino)quinolin-2-yl]amino}cyclohexyl)-N'-(3-methyl-5-phenylisoxazol-4-yl)urea;
N-(cis-4-{[4-(dimethylamino)quinolin-2-yl]amino}cyclohexyl)-N'-1-naphthylurea;
N-(cis-4-{[4-(dimethylamino)quinolin-2-yl]amino}cyclohexyl)-N'-[1-(1-naphthyl)ethyl]-urea;
methyl N-{[(cis-4-{[4-(dimethylamino)quinolin-2-yl]amino}cyclohexyl)-amino]carbonyl}-phenylalaninate;
N-(cis-4-{[4-(dimethylamino)quinolin-2-yl]amino}cyclohexyl)-N'-(3,4,5-trimethoxyphenyl)urea;
N-(5-chloro-2,4-dimethoxyphenyl)-N'-(cis-4-{[4-(dimethylamino)quinolin-2-yl]amino}-cyclohexyl)urea;
N-(4-bromo-2-methylphenyl)-N'-(cis-4-{[4-(dimethylamino)quinolin-2-yl]amino}-cyclohexyl)urea;
N-[(cis-4-{[4-(dimethylamino)quinolin-2-yl]amino}cyclohexyl)methyl]-N'-(2-ethyl-6-methylphenyl)urea;
N-[(cis-4-{[4-(dimethylamino)quinolin-2-yl]amino}cyclohexyl)methyl]-N'-mesitylurea;
N-[(cis-4-{[4-(dimethylamino)quinolin-2-yl]amino}cyclohexyl)methyl]-N'-(2,4,6-trichlorophenyl)urea;
N-[(cis-4-{[4-(dimethylamino)quinolin-2-yl]amino}cyclohexyl)methyl]-N'-(2,4,6-tribromophenyl)urea;
N-(2,4-dibromo-6-fluorophenyl)-N'-[(cis-4-{[4-(dimethylamino)quinolin-2-yl]amino}-cyclohexyl)methyl]urea;
N-(2,6-diethylphenyl)-N'-[(cis-4-{[4-(dimethylamino)quinolin-2-yl]amino}-cyclohexyl)-methyl]urea;
N-[2-chloro-6-(trifluoromethyl)phenyl]-N'-[(cis-4-{[4-(dimethylamino)-quinolin-2-yl]-amino}cyclohexyl)methyl]urea;
N-[(cis-4-{[4-(dimethylamino)quinolin-2-yl]amino}cyclohexyl)methyl]-N'-(2-ethyl-6-isopropylphenyl)urea;
N-[(cis-4-{[4-(dimethylamino)quinolin-2-yl]amino}cyclohexyl)methyl]-N'-(2-isopropyl-6-methylphenyl)urea;
N-[(cis-4-{[4-(dimethylamino)quinolin-2-yl]amino}cyclohexyl)methyl]-N'-(2-methyl-3-nitrophenyl)urea;
N-(2-tert-butyl-6-methylphenyl)-N'-[(cis-4-{[4-(dimethylamino)quinolin-2-yl]amino}-cyclohexyl)methyl]urea;

N-(2-tert-butylphenyl)-N'-[(cis-4-{[4-(dimethylamino)quinolin-2-yl]-amino}cyclohexyl)-methyl]urea;
N-[(cis-4-{[4-(dimethylamano)quinolin-2-yl]amino}cyclohexyl)methyl]-N'-(diphenylmethyl)urea;
N-(4-bromo-2,6-dimethylphenyl)-N'-[(cis-4-([4-(dimethylamino)quinolin-2-yl] amino)-cyclohexyl)methyl]urea;
N-(2,3-dichlorophenyl)-N'-[(cis-4-{[4-(dimethylamino)quinolin-2-yl]-amino}cyclohexyl)-methyl]urea;
N-(2,6-diisopropylphenyl)-N'-[(cis-4-{[4-(dimethylamino)quinolin-2-yl]-amino}-cyclohexyl)methyl]urea;
1-(2,3-dichloro-phenyl)-3-[cis-4-(4-methyl-quinolin-2-ylamino)-cyclohexyl]-urea; and
1-(2,3-dichloro-phenyl)-3-[cis-4-(4-methyl-quinolin-2-ylamino)-cyclohexylmethyl]-urea;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- carbocyclic aryl,
- carbocyclic aryl substituted by substituent(s) independently selected from the group consisting of:
  - •• halogen, and
  - •• $C_{1-5}$ alkoxy,

(ii) carbocyclyl,
(iii) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by halogen,
- $C_{1-5}$ alkoxy carbonyl,
- $C_{1-5}$ alkoxy,
- $C_{1-5}$ alkoxy substituted by halogen,
- mono-$C_{1-5}$ alkylamino,
- di-$C_{1-5}$ alkylamino, and
- carbocyclic aryl,

(iv) heterocyclyl, and
heterocyclyl substituted by substituent(s) independently selected from the group consisting of:

- $C_{1-5}$ alkyl,
- $C_{1-5}$ alkoxy carbonyl, and
- carbocyclic aryl;

L is Formula (VII);
Y is -C(S)$NR_5$-;
wherein carbocyclic aryl is phenyl or naphthyl;
carbocyclyl is bicyclo[2.2.1]heptyl;
heterocyclyl is 2,3-dihydro-benzo[1,4]dioxinyl, benzo[1,3]dioxolyl, isoxazolyl, or thienyl; and
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are hydrogen; A is a single bond; B is a single bond or -$CH_2$-; $R_5$ is hydrogen; or a pharmaceutically acceptable salt, hydrate, or solvate thereof.

In some embodiments of the present invention, $R_1$ is selected from the group consisting of:

(i) carbocyclic aryl, and

carbocyclic aryl substituted by substituent(s) independently selected from the group consisting of:

- halogen,
- $C_{1-5}$ alkyl,
- $C_{1-5}$ alkyl substituted by halogen,
- $C_{1-5}$ alkoxy,
- mono-$C_{1-5}$ alkylamino, and
- di-$C_{1-5}$ alkylamino,

(ii) heterocyclyl, and
heterocyclyl substituted by $C_{1-5}$ alkyl, and
heterocyclyl substituted by $C_{1-5}$ alkoxy carbonyl;
wherein carbocyclic aryl is phenyl or naphthyl;
heterocyclyl is thienyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(2,4-dimethoxyphenyl)-N'-(cis-4-{[4-(dimethylamino)quinolin-2-yl]-amino}cyclohexyl)-thiourea;
N-(cis-4-{[4-(dimethylamino)quinolin-2-yl]amino}cyclohexyl)-N'-(3,4,5-trimethoxyphenyl)thiourea;
N-[4-(dimethylamino)-1-naphthyl]-N'-(cis-4-{(4-(dimethylamino)quinolin-2-yl]amino}-cyclohexyl)thiourea;
N-(cis-4-[4-(dimethylamino)quinolin-2-yl]amino}cyclohexyl)-N'-(2,4,6-tribromophenyl)-thiourea;
N-(cis-4-{[4-(dimethylamino)quinolin-2-yl]amino}cyclohexyl)-N'-(2,4,6-trichlorophenyl)-thiourea;
N-(cis-4-{[4-(dimethylamino)quinolin-2-yl]amino}cyclohexyl)-N'-mesitylthiourea;
N-(2,6-diethylphenyl)-N'-(cis-4-{[4-(dimethylamino)quinolin-2-yl]amino}-cyclohexyl)-thiourea;
N-(4-bromo-2,6-dimethylphenyl)-N'-(cis-4-{[4-(dimethylamino)quinolin-2-yl]amino}-cyclohexyl)thiourea;
N-(4-bromo-2-methylphenyl)-N'-(cis-4-{[4-(dimethylamino)quinolin-2-yl]amino}-cyclohexyl)thiourea;
N-[4-bromo-2-(trifluoromethyl)phenyl]-N'-(cis-4-{[4-(dimethylamino)-quinolin-2-yl]-amino}cyclohexyl)thiourea;
N-(5-chloro-2,4-dimethoxyphenyl)-N'-(cis-4-{[4-(dimethylamino)quinolin-2-yl]amino}-cyclohexyl)thiourea;
N-(2,4-dibromo-6-fluorophenyl)-N'-(cis-4-{[4-(dimethylamino)quinolin-2-yl]amino}-cyclohexyl)thiourea;
N-(2,4-dichloro-6-methylphenyl)-N'-(cis-4-{[4-(dimethylamino)quinolin-2-yl]amino}-cyclohexyl)thiourea; and
methyl 3-{[(cis-4-{[4-(dimethylamino)quinolin-2-yl]amino)cyclohexyl)-amino]-carbonothioyl}amino)-4-methylthiophene-2-carboxylate;

or a pharmaceutically acceptable salt, hydrate, or solvate thereof.

In some embodiments of the present invention, $R_1$ is selected from the group consisting of:

(i) $C_{1-8}$ alkyl, and
$C_{1-8}$ alkyl substituted by substituent(s) independently selected from the group consisting of:

- halogen,
- $C_{1-5}$ alkoxy,
- $C_{1-5}$ alkoxy substituted by carbocyclic aryl,
- carbocyclyl,
- carbocyclic aryl,
- carbocyclic aryl substituted by substituent(s) independently selected from the group consisting of:
  - •• halogen,
  - •• nitro, and
  - •• $C_{1-5}$ alkoxy,

(ii) $C_{2-5}$ alkenyl,
(iii) carbocyclyl,
(iv) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen, and
- $C_{1-5}$ alkoxy;

L is Formula (VII);
Y is -C(O)O-;
wherein carbocyclic aryl is phenyl or naphthyl;
carbocyclyl is 9H-fluorenyl or menthyl; and
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are hydrogen; A is a single bond; B is a single bond or $-CH_2-$; or a pharmaceutically acceptable salt, hydrate, or solvate thereof.

In some embodiments of the present invention, Q is Formula (III);

(i) $C_{1-8}$ alkyl, and
$C_{1-8}$ alkyl substituted by substituent(s) independently selected from the group consisting of:

- halogen,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- heterocyclyloxy,
- heterocyclyloxy substituted by $C_{1-5}$ alkyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by cyano,
- mono-$C_{1-5}$ alkylamino substituted by carbocyclic aryl,
- di-$C_{1-5}$ alkylamino,
- di-$C_{1-5}$ alkylamino substituted by cyano,
- di-$C_{1-5}$ alkylamino substituted by carbocyclic aryl,
- mono-carbocyclic arylamino,
- mono-carbocyclic arylamino substituted by $C_{1-5}$ alkyl,
- di-carbocyclic arylamino,
- di-carbocyclic arylamino substituted by $C_{1-5}$ alkyl,
- carbocyclic arylsulfonylamino,
- carbocyclic arylsulfonylamino substituted $C_{1-5}$ alkyl,
- $C_{1-5}$ alkylthio,
- $C_{1-5}$ alkylthio substituted by substituent(s) independently selected from the group consisting of:
  - •• carbocyclic aryl,
  - •• carbocyclic aryl substituted by halogen, and
  - •• carbocyclic aryl substituted by $C_{1-5}$ alkoxy,
- carbocyclic arylthio,
- heterocyclylthio,
- heterocyclylthio substituted by $C_{1-5}$ alkyl,
- $C_{3-6}$ cycloalkyl,
- $C_{3-6}$ cycloalkenyl,
- carbocyclyl,

- carbocyclyl substituted by substituent(s) independently selected from the group consisting of:
    - •• halogen,
    - •• $C_{1-5}$ alkyl,
    - •• $C_{1-5}$ alkoxy,
    - •• $C_{2-5}$ alkenyl, and
    - •• $C_{2-5}$ alkenyl substituted by substituent(s) independently selected from the group consisting of:
        - ••• carbocyclic aryl, and
        - ••• carbocyclic aryl substituted by $C_{1-5}$ alkylsulfinyl,
- carbocyclic aryl,
- carbocyclic aryl substituted by substituent(s) independently selected from the group consisting of:
    - •• halogen,
    - •• hydroxy,
    - •• nitro,
    - •• $C_{1-5}$ alkyl,
    - •• $C_{1-5}$ alkyl substituted by substituent(s) independently selected from the group consisting of:
        - ••• oxo,
        - ••• carbocyclic aryl, and
        - ••• heterocyclyl,
    - •• $C_{2-5}$ alkenyl,
    - •• $C_{1-5}$ alkoxy,
    - •• $C_{1-5}$ alkoxy substituted by halogen,
    - •• $C_{1-5}$ alkoxy substituted by carbocyclic aryl,
    - •• carbocyclic aryloxy,
    - •• mono-carbocyclic arylaminocarbonyl,
    - •• mono-carbocyclic arylaminocarbonyl substituted by halogen,
    - •• di-carbvcyclic arylaminocarbonyl,
    - •• di-carbocyclic arylaminocarbonyl substituted by halogen,
    - •• carbocyclic aryl, and
    - •• heterocyclyl,
- heterocyclyl, and
- heterocyclyl substituted by substituent(s) independently selected from the group consisting of:
    - •• $C_{1-5}$ alkyl,
    - •• $C_{1-5}$ alkoxy,
    - •• $C_{1-5}$ alkoxy substituted by carbocyclic aryl,
    - •• carbocyclic aryl, and
    - •• carbocyclic aryl substituted by halogen,

(ii) $C_{2-7}$ alkenyl, and
$C_{2-7}$ alkenyl substituted by substituent(s) independently selected from the group consisting of:

- carbocyclic aryl,
- carbocyclic aryl substituted by substituent(s) independently selected from the group consisting of:
    - •• halogen,
    - •• nitro, and
    - •• $C_{1-5}$ alkoxy,

(iii) $C_{2-5}$ alkynyl,
(iv) $C_{3-12}$ cycloalkyl, and
$C_{3-12}$ cycloalkyl substituted by substituent(s) independently selected from the group consisting of:

- $C_{1-5}$ alkyl,
- $C_{1-5}$ alkyl substituted by oxo,
- $C_{1-5}$ alkyl substituted by carbocyclic aryl, and
- carbocyclic aryl,

(v) carbocyclyl,
(vi) carbocyclic aryl, and
carbocyclic aryl substituted by substituent(s) independently selected from the group consisting of:

- halogen,
- hydroxy,
- cyano,
- nitro,
- $C_{1-10}$ alkyl,
- $C_{1-10}$ alkyl substituted by substituent(s) independently selected from the group consisting of:

  - •• halogen,
  - •• oxo,
  - •• carbocyclic aryloxy,
  - •• carbocyclic aryl, and
  - •• carbocyclic aryl substituted by $C_{1-5}$ alkyl,

- $C_{1-7}$ alkoxy,
- $C_{1-7}$ alkoxy substituted by substituent(s) independently selected from the group consisting of:

  - •• halogen,
  - •• carbocyclic aryl, and
  - •• halogenated carbocyclic aryl,

- $C_{2-5}$ alkenyloxy,
- $C_{3-6}$ cycloalkoxy,
- carbocyclic aryloxy,
- carbocyclic aryloxy substituted by nitro,
- carbocyclic aryloxy substituted by $C_{1-5}$ alkoxy,
- carboxy,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{2-5}$ alkynylcarbonylamino,
- $C_{2-5}$ alkynylcarbonylamino substituted by carbocyclic aryl,
- $C_{1-5}$ alkoxycarbonylamino,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- carbocyclic aryl azo,
- carbocyclic aryl azo substituted by mono-$C_{1-5}$ alkylamino,
- carbocyclic aryl azo substituted by di-$C_{1-5}$ alkylamino,
- $C_{1-5}$ alkylthio,
- $C_{1-5}$ alkylthio substituted by halogen,
- carbocyclic arylthio,
- carbocyclic arylthio substituted by nitro,

- carbocyclic arylthio substituted by cyano,
- aminosulfonyl,
- mono-$C_{1-5}$ alkylaminosulfonyl,
- di-$C_{1-5}$ alkylaminosulfonyl,
- heterocyclylsulfonyl,
- $C_{3-6}$ cycloalkyl,
- $C_{3-6}$ cycloalkyl substituted by $C_{1-5}$ alkyl,
- carbocyclic aryl,
- heterocyclyl, and
- heterocyclyl substituted by substituent(s) independently selected from the group consisting of:

  - •• $C_{1-5}$ alkyl,
  - •• carbocyclic aryl, and
  - •• halogenated carbocyclic aryl,

(vii) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:

  - •• halogen,
  - •• hydroxy,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by halogen, and
  - •• heterocyclyl,

- $C_{1-5}$ alkoxy,
- carbocyclic aryloxy,
- carbocyclic aryloxy substituted by $C_{1-5}$ alkyl,
- $C_{1-5}$ alkylthio,
- $C_{1-5}$ alkenylthio,
- carbocyclic arylthio,
- carbocyclic arylthio substituted by $C_{1-5}$ alkoxycarbonyl,
- $C_{1-5}$ alkylsulfonyl,
- carbocyclic arylsulfonyl,
- carbocyclic arylsulfonyl substituted by $C_{1-5}$ alkyl,
- $C_{1-5}$ alkoxycarbonyl,
- $C_{1-5}$ alkoxycarbonyl substituted by carbocyclic aryl,
- carbocyclic aryl,
- carbocyclic aryl substituted by substituent(s) independently selected from the group consisting of:

  - •• halogen,
  - •• nitro,
  - •• $C_{1-5}$ alkyl, and
  - •• $C_{1-5}$ alkyl substituted by halogen,

- heterocyclyl;

wherein carbocyclic aryl is phenyl, naphthyl, or anthranyl;

carbocyclyl is 1,2,3,4-tetrahydronaphthyl, 1-oxo-indanyl, 9-fluorenyl, 9*H*-fluorenyl, 9-oxo-9*H*-fluorenyl, adamantly, bicyclo[2.2.1]heptenyl, bicyclo[2.2.1]heptyl, indanyl, indenyl, or menthyl;

heterocyclyl is 1,2,3-triazolyl, 1*H*-indolyl, 1*H*-pyrrolyl, 2,3-dihydro-1-oxo-isoindolyl, 2,3-dihydro-benzo[1,4]dioxinyl, 2,4-dihydro-3-oxo-pyrazolyl, 2*H*-benzopyranyl, 2-oxo-benzopyranyl, 3,4-dihydro-2*H*-benzo[b][1,4]dioxepinyl, 4,5,6,7-tetrahydro-benzo[b]thienyl, 4*H*-benzo[1,3]dioxinyl, 4-oxo-1,5,6,7-tetrahydro-indolyl, 4-oxo-benzopyranyl, 9*H*-carbazolyl, 9*H*-xanthenyl, azetidinyl, benzo[1,3]dioxolyl, benzo[2,1,3]oxadiazolyl, benzo[1,2,5]oxadiazolyl, benzo[2,1,3]thiadiazolyl, benzo[b]thienyl, benzofuryl, benzothiazolyl, furyl, imidazo[2,1-b]thiazolyl, isoxazolyl, morpholino, morpholinyl, oxazolyl, phenanthro[9,10-d]oxazolyl, piperidyl, pyrazolyl, pyridyl, pyrimidyl, quinolyl, quinoxalyl, tetrahydrofuryl, thiazolyl, or thienyl; and

halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-7}$ alkyl, and

$C_{1-7}$ alkyl substituted by substituent(s) independently selected from the group consisting of:

- $C_{1-5}$ alkoxy,
- $C_{1-5}$ alkoxy substituted by carbocyclic aryl,
- carbocyclic aryloxy,
- mono-$C_{1-5}$ alkylamino,
- mono-$C_{1-5}$ alkylamino substituted by substituent(s) independently selected from the group consisting of:
    - •• cyano, and
    - •• carbocyclic aryl,
- di-$C_{1-5}$ alkylamino,
- di-$C_{1-5}$ alkylamino substituted by substituent(s) independently selected from the group consisting of:
    - •• cyano, and
    - •• carbocyclic aryl,
- mono-carbocyclic arylamino,
- di-carbocyclic arylamino,
- mono-carbocyclic arylamino substituted by $C_{1-5}$ alkyl,
- di-carbocyclic arylamino substituted by $C_{1-5}$ alkyl,
- carbocyclic arylsulfonylamino,
- carbocyclic arylsulfonylamino substituted by $C_{1-5}$ alkyl,
- carbocyclic aryl,
- carbocyclic aryl substituted by substituent(s) independently selected from the group consisting of:
    - •• halogen,
    - •• nitro,
    - •• $C_{1-5}$ alkyl,
    - •• $C_{1-5}$ alkyl substituted by substituent(s) independently selected from the group consisting of:
        - ••• oxo, and
        - ••• carbocyclic aryl,
    - •• $C_{1-5}$ alkoxy,
- heterocyclyl, and
- heterocyclyl substituted by carbocyclic aryl,

(ii) $C_{2-7}$ alkenyl, and

$C_{2-7}$ alkenyl substituted by substituent(s) independently selected from the group consisting of:

- carbocyclic aryl, and
- carbocyclic aryl substituted by $C_{1-5}$ alkoxy,

(iii) $C_{3-6}$ cycloalkyl, and

$C_{3-6}$ cycloalkyl substituted by substituent(s) independently selected from the group consisting of:

- $C_{1-5}$ alkyl, and
- $C_{1-5}$ alkyl substituted by carbocyclic aryl,

(iv) carbocyclic aryl, and
carbocyclic aryl substituted by substituent(s) independently selected from the group consisting of:

- halogen,
- hydroxy,
- cyano,
- $C_{1-5}$ alkyl,
- $C_{1-5}$ alkyl substituted by halogen,
- $C_{1-5}$ alkoxy,
- $C_{1-5}$ alkoxy substituted by substituent(s) independently selected from the group consisting of:
  - •• halogen, and
  - •• carbocyclic aryl,
  - •• carbocyclic aryl substituted by halogen,
- $C_{2-5}$ alkenyloxy,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{1-5}$ alkylthio, and
- $C_{1-5}$ alkylthio substituted by halogen,

(v) heterocyclyl, and
heterocyclyl substituted by substituent(s) independently selected from the group consisting of:

- halogen,
- $C_{1-5}$ alkyl,
- $C_{1-5}$ alkyl substituted by substituent(s) independently selected from the group consisting of:
  - •• hydroxy, and
  - •• carbocyclic aryl,
- $C_{1-5}$ alkoxy,
- carbocyclic arylthio,
- carbocyclic arylthio substituted by $C_{1-5}$ alkoxycarbonyl,
- $C_{1-5}$ alkoxycarbonyl,
- carbocyclic aryl,
- carbocyclic aryl substituted by substituent(s) independently selected from the group consisting of:
  - •• halogen,
  - •• $C_{1-5}$ alkyl, and
  - •• $C_{1-5}$ alkyl substituted by halogen;

L is Formula (VII);
Y is a single bond or $-CH_2-$;
wherein carbocyclic aryl is phenyl or naphthyl;
heterocyclyl is 1*H*-indolyl, 1*H*-pyrrolyl, 2,3-dihydro-benzo[1,4]dioxinyl, 4-oxo-benzopyranyl, 9*H*-carbazolyl, azetidinyl, benzo[1,3]dioxolyl, benzo[b]thienyl, furyl, imidazo[2,1-b]thiazolyl, pyrazolyl, pyridyl, or thienyl; and
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are

hydrogen; A is a single bond; B is a single bond or $-CH_2-$; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- mono-$C_{1-5}$ alkylamino,
- mono-$C_{1-5}$ alkylamino substituted by cyano,
- di-$C_{1-5}$ alkylamino,
- di-$C_{1-5}$ alkylamino substituted by cyano,
- mono-carbocyclic arylamino,
- di-carbocyclic arylamino,
- mono-carbocyclic arylamino substituted by $C_{1-5}$ alkyl,
- di-carbocyclic arylamino substituted by $C_{1-5}$ alkyl,
- carbocyclic arylsulfonylamino,
- carbocyclic arylsulfonylamino substituted by $C_{1-5}$ alkyl,
- carbocyclic aryl, and
- carbocyclic aryl substituted by $C_{1-5}$ alkoxy,

(ii) $C_{2-5}$ alkenyl, and
$C_{2-5}$ alkenyl substituted by carbocyclic aryl,
(iii) carbocyclic aryl, and
carbocyclic aryl substituted by substituent(s) independently selected from the group consisting of:

- halogen,
- hydroxy,
- $C_{1-5}$ alkyl,
- $C_{1-5}$ alkoxy,
- $C_{1-5}$ alkoxy substituted by halogen,
- mono-$C_{1-5}$ alkylamino, and
- di-$C_{1-5}$ alkylamino,

(iv) heterocyclyl, and
heterocyclyl substituted by substituent(s) independently selected from the group consisting of:

- halogen,
- $C_{1-5}$ alkyl,
- $C_{1-5}$ alkyl substituted by carbocyclic aryl,
- $C_{1-5}$ alkoxy,
- $C_{1-5}$ alkoxycarbonyl,
- carbocyclic aryl,
- carbocyclic aryl substituted by substituent(s) independently selected from the group consisting of:

    •• halogen,
    •• $C_{1-5}$ alkyl, and
    •• $C_{1-5}$ alkyl substituted by halogen;

wherein carbocyclic aryl is phenyl or naphthyl;
heterocyclyl is 1*H*-indolyl, 4-oxo-benzopyranyl, azetidinyl, benzo[1,3]dioxolyl, or pyrazolyl; and
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- mono-$C_{1-5}$ alkylamino,
- mono-$C_{1-5}$ alkylamino substituted by cyano,
- di-$C_{1-5}$ alkylamino,
- di-$C_{1-5}$ alkylamino substituted by cyano,
- mono-carbocyclic arylamino,
- di-carbocyclic arylamino,
- carbocyclic arylsulfonylamino,
- carbocyclic arylsulfonylamino substituted by $C_{1-5}$ alkyl, and
- carbocyclic aryl,

(ii) $C_{2-5}$ alkenyl, and
$C_{2-5}$ alkenyl substituted by carbocyclic aryl,
(iii) carbocyclic aryl, and
carbocyclic aryl substituted by substituent(s) independently selected from the group consisting of:

- halogen,
- hydroxy,
- $C_{1-5}$ alkoxy, and
- $C_{1-5}$ alkoxy substituted by halogen,

(iv) heterocyclyl, and
heterocyclyl substituted by substituent(s) independently selected from the group consisting of:

- halogen,
- $C_{1-5}$ alkyl,
- $C_{1-5}$ alkyl substituted by carbocyclic aryl,
- $C_{1-5}$ alkoxy,
- $C_{1-5}$ alkoxycarbonyl,
- carbocyclic aryl, and
- carbocyclic aryl substituted by halogen;

wherein carbocyclic aryl is phenyl;
heterocyclyl is 1*H*-indolyl, azetidinyl, or pyrazolyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

$N^2$-{cis-4-[(2,6-dimethoxybenzyl)amino]cyclohexyl}-$N^4$,$N^4$-dimethyl-5,6,7,8-tetrahydroquinazoline-2,4-diamine;
$N^2$-{cis-4[(2-ethoxybenzyl)amino]cyclohexyl}-$N^4$,$N^4$-dimethyl-5,6,7,8-tetrahydroquinazoline-2,4-diamine;
$N^2$-{cis-4-[(1H-indol-3-ylmethyl)amino]cyclohexyl}-$N^4$,$N^4$-dimethyl-5,6,7,8-tetrahydroquinazoline-2,4-diamine;
$N^2$-{cis-4-[(2,5-dimethoxybenzyl)amino]cyclohexyl}-$N^4$,$N^4$-dimethyl-5,6,7,8-tetrahydroquinazoline-2,4-diamine;
$N^2$-(cis-4-{[(4-methoxy-1-naphthyl)methyl]amino}cyclohexyl)-$N^4$,$N^4$-dimethyl-5,6,7,8-tetrahydroquinazoline-2,4-diamine;
$N^2$-(cis-4-{[(5-methoxy-1H-indol-3-yl)methyl]amino}cyclohexyl)-$N^4$,$N^4$-dimethyl-5,6,7,8-tetrahydroquinazoline-2,4-diamine;
4-bromo-2-{[(cis-4-{[4-(dimethylamino)-5,6,7,8-tetrahydroquinazolin-2-yl]amino}-cyclohexyl)amino]methyl}-6-methoxyphenol;
$N^2$-(cis-4-{[(5-bromo-1H-indol-3-yl)methyl]amino}cyclohexyl)-$N^4$,$N^4$-dimethyl-5,6,7,8-tetrahydroquinazoline-2,4-diamine;
4-{[(cis-4-{[4-(dimethylamino)-5,6,7,8-tetrahydroquinazolin-2-yl]amino}-cyclohexyl)-amino]methyl}-2,6-dimethoxyphenol;
$N^2$-{cis-4-[(3-ethoxy-4-methoxybenzyl)amino]cyclohexyl}-$N^4$,$N^4$-dimethyl-5,6,7,8-tetrahydroquinazoline-2,4-diamine;
$N^4$,$N^4$-dimethyl-$N^2$-{cis-4-[({3-[4-(trifluoromethyl)phenyl]-1H-pyrazol-4-yl}methyl)-amino]cyclohexyl}-5,6,7,8-tetrahydroquinazoline-2,4-diamine;
$N^4$,$N^4$-dimethyl-$N^2$-{cis-4-[(3,4,5-trimethoxybenzyl)amino]cyclohexyl}-5,6,7,8-tetrahydroquinazoline-2,4-diamine;

$N^4$,$N^4$-dimethyl-$N^2$-{cis-4-[(pentamethylbenzyl)amino]cyclohexyl}-5,6,7,8-tetrahydroquinazoline-2,4-diamine;
$N^2$-{cis-4-[(3,5-dimethoxybenzyl)amino]cyclohexyl}-$N^4$,$N^4$-dimethyl-5,6,7,8-tetrahydroquinazoline-2,4-diamine;
4-{[(cis-4-{[4-(dimethylamino)-5,6,7,8-tetrahydroquinazolin-2-yl]amino}-cyclohexyl)-amino]methyl}-2-iodo-6-methoxyphenol;
4-{[(cis-4-{[4-(dimethylamino)-5,6,7,8-tetrahydroquinazolin-2-yl]amino}-cyclohexyl)-amino]methyl}-2,6-dimethylphenol;
3-{[(cis-4-{[4-(dimethylamino)-5,6,7,8-tetrahydroquinazolin-2-yl]amino}-cyclohexyl)-amino]methyl}-6,8-dimethyl-4H-chromen-4-one;
ethyl 4,6-dichloro-3-{[(cis-4-{[4-(dimethylamino)-5,6,7,8-tetrahydroquinazolin-2-yl]-amino}cyclohexyl)amino]methyl}-1H-indole-2-carboxylate;
$N^2$-[cis-4-({[3-(4-fluorophenyl)-1H-pyrazol-4-yl]methyl}amino)cyclohexyl]-$N^4$,$N^4$-dimethyl-5,6,7,8-tetrahydroquinazoline-2,4-diamine;
$N^4$,$N^4$-dimethyl-$N^2$-[4-(pentamethylphenylmethyl-amino)-cyclohexyl]-5,6,7,8-tetrahydro-quinazoline-2,4-diamine;
3-[{2-[(cis-4-{[4-(dimethylamino)-5,6,7,8-tetrahydroquinazolin-2-yl]amino}cyclohexyl)-amino]ethyl}(3-methylphenyl)amino]propanenitrile;
3-[{2-[(cis-4-{[4-(dimethylamino)-5,6,7,8-tetrahydroquinazolin-2-yl]amino}cyclohexyl)-amino]ethyl}(phenyl)amino] propanenitrile;
N-{(1S)-1-benzyl-2-[(cis-4-{[4-(dimethylamino)-5,6,7,8-tetrahydroquinazolin-2-yl]amino}-cyclohexyl)amino]ethyl}-4-methylbenzenesulfonamide;
$N^2$-(cis-4-{[2-(3,5-dimethoxyphenyl)ethyl]amino}cyclohexyl)-$N^4$,$N^4$-dimethyl-5,6,7,8-tetrahydroquinazoline-2,4-diamine;
$N^2$-[cis-4-({[1-(diphenylmethyl)azetidin-3-yl]methyl}amino)cyclohexyl]-$N^4$,$N^4$-dimethyl-5,6,7,8-tetrahydroquinazoline-2,4-diamine;
$N^2$-(cis-4-{[(2,6-dimethoxybenzyl)amino]methyl}cyclohexyl)-$N^4$,$N^4$-dimethyl-5,6,7,8-tetrahydroquinazoline-2,4-diamine;
$N^2$-(cis-4-{[(2-ethoxybenzyl)amino]methyl}cyclohexyl)-$N^4$,$N^4$-dimethyl-5,6,7,8-tetrahydroquinazoline-2,4-diamine;
$N^2$-(cis-4-{[(1H-indol-3-ylmethyl)amino]methyl}cyclohexyl)-$N^4$,$N^4$-dimethyl-5,6,7,8-tetrahydroquinazoline-2,4-diamine;
$N^2$-(cis-4-{[(2,5-dimethoxybenzyl)amino]methyl}cyclohexyl)-$N^4$,$N^4$-dimethyl-5,6,7,8-tetrahydroquinazoline-2,4-diamine;
$N^2$-[cis-4-({[(4-methoxy-1-naphthyl)methyl]amino}methyl)cyclohexyl]-$N^4$,$N^4$-dimethyl-5,6,7,8-tetrahydroquinazoline-2,4-diamine;
$N^2$-[cis-4-({[(5-methoxy-1H-indol-3-yl)methyl]amino}methyl)cyclohexyl]-$N^4$,$N^4$-dimethyl-5,6,7,8-tetrahydroquinazoline-2,4-diamine;
4-bromo-2-({[(cis-4-{[4-(dimethylamino)-5,6,7,8-tetrahydroquinazolin-2-yl]amino}-cyclohexyl)methyl]amino}methyl)-6-methoxyphenol;
$N^2$-[cis-4-({[(5-bromo-1H-indol-3-yl)methyl]amino}methyl)cyclohexyl]-$N^4$,$N^4$-dimethyl-5,6,7,8-tetrahydroquinazoline-2,4-diamine;
$N^2$-(cis-4-{[(3-ethoxy-4-methoxybenzyl)amino]methyl}cyclohexyl)-$N^4$,$N^4$-dimethyl-5,6,7,8-tetrahydroquinazoline-2,4-diamine;
$N^4$,$N^4$-dimethyl-$N^2$-(cis-4-{[({3-[4-(trifluoromethyl)phenyl]-1H-pyrazol-4-yl}methyl)-amino]methyl}cyclohexyl)-5,6,7,8-tetrahydroquinazoline-2,4-diamine;
$N^4$,$N^4$-dimethyl-$N^2$-(cis-4-{[(3,4,5-trimethoxybenzyl)amino]-methyl}cyclohexyl)-5,6,7,8-tetrahydroquinazoline-2,4-diamine;
$N^2$-(cis-4-{[(3,5-dimethoxybenzyl)amino]methyl}cyclohexyl)-$N^4$,$N^4$-dimethyl-5,6,7,8-tetrahydroquinazoline-2,4-diamine;
4-({[(cis-4{[4-(dimethylamino)-5,6,7,8-tetrahydroquinazolin-2-yl]amino}cyclohexyl)-methyl]amino}methyl)-2-iodo-6-methoxyphenol;
4-({[(cis-4-{[4-(dimethylamino)-5,6,7,8-tetrahydroquinazolin-2-yl]amino}cyclohexyl)-methyl]amino}methyl)-2,6-dimethylphenol;
3-chloro-4-({[(cis-4-{[4-(dimethylamino)-5,6,7,8-tetrahydroquinazolin-2-yl]amino}-cyclohexyl)methyl]amino}methyl)phenol;
$N^2$-[cis-4-({[4-(diethylamino)benzyl]amino}methyl)cyclohexyl]-$N^4$,$N^4$-dimethyl-5,6,7,8-tetrahydroquinazoline-2,4-diamine;
$N^2$-(cis-4-{[(3,3-diphenylprop-2-en-1-yl)amino]methyl}cyclohexyl)-$N^4$,$N^4$-dimethyl-5,6,7,8-tetrahydroquinazoline-2,4-diamine;
4-{[(cis-4-{[4-(dimethylamino)-5,6,7,8-tetrahydroquinazolin-2-yl]amino}cyclohexyl)-methyl]amino}methyl)-

2-ethoxyphenol;
$N^2$-{cis-4-[({[4-(dimethylamino)-1-naphthyl]methyl}amino)methyl]-cyclohexyl}-$N^4$,$N^4$-dimethyl-5,6,7,8-tetrahydroquinazoline-2,4-diamine;
$N^4$,$N^4$-dimethyl-$N^2$-(cis-4-{[(2,4,6-trimethoxybenzyl)amino]methyl}-cyclohexyl)-5,6,7,8-tetrahydroquinazoline-2,4-diamine;
2-bromo-4-chloro-6-({[(cis-4-{[4-(dimethylamino)-5,6,7,8-tetrahydroquinazolin-2-yl]-amino}cyclohexyl)methyl]amino}methyl)phenol;
$N^2$-{cis-4-([(2,5-diethoxybenzyl)amino]methyl}cyclohexyl)-$N^4$,$N^4$-dimethyl-5,6,7, 8-tetrahydroquinazoline-2,4-diamine;
$N^2$-(cis-4-{[(2,4-diethoxybenzyl)amino]methyl}cyclohexyl)-$N^4$,$N^4$-dimethyl-5,6,7,8-tetrahydroquinazoline-2,4-diamine;
$N^2$-(cis-4-{[(3,5-dibromo-2-methoxybenzyl)amino]methyl}cyclohexyl)-$N^4$,$N^4$-dimethyl-5,6,7,8-tetrahydroquinazoline-2,4-diamine;
$N^4$,$N^4$-dimethyl-$N^2$-(cis-4-{[(2,4,5-triethoxybenzyl)amino]methyl}-cyclohexyl)-5,6,7,8-tetrahydroquinazoline-2,4-diamine;
$N^4$,$N^4$-dimethyl-$N^2$-(cis-4-{[(2,4,5-trimethoxybenzyl)amino]methyl}-cyclohexyl)-5,6,7,8-tetrahydroquinazoline-2,4-diamine;
$N^2$-[cis-4-({[(7-methoxy-1,3-benzodioxol-5-yl)methyl]amino}methyl)-cyclohexyl]-$N^4$,$N^4$-dimethyl-5,6,7,8-tetrahydroquinazoline-2,4-diamine;
4-({[(cis-4-{[4-(dimethylamino)-5,6,7,8-tetrahydroquinazolin-2-yl]amino}cyclohexyl)-methyl]amino}methyl)-2-methylphenol;
$N^2$-(cis-4-{[(4-methoxy-2,5-dimethylbenzyl)amino]methyl}cyclohexyl)-$N^4$,$N^4$-dimethyl-5,6,7,8-tetrahydroquinazoline-2,4-diamine;
4-({[(cis-4-{[4-(dimethylaniino)-5,6,7,8-tetrahydroquinazolin-2-yl]amino}cyclohexyl)-methyl]amino}methyl)-2-fluoro-6-methoxyphenol;
$N^4$,$N^4$-dimethyl-$N^2$-[cis-4-({([(1-phenyl-5-propyl-1H-pyrazol-4-yl)methyl]amino)methyl)-cyclohexyl]-5,6,7,8-tetrahydroquinazoline-2,4-diamine;
$N^2$-{cis-4-[({[1-(4-chlorophenyl)-5-propyl-1H-pyrazol-4-yl]methyl}-amino)methyl]-cyclohexyl}-$N^4$$N^4$-dimethyl-5,6,7,8-tetrahydroquinazoline-2,4-diamine;
$N^2$-{cis-4-[2-(4-bromo-2-trifluoromethoxy-phenyl)-ethylamino]-cyclohexyl}-$N^4$,$N^4$-dimethyl-5,6,7,8-tetrahydroquinazoline-2,4-diamine;
$N^2$-{cis-4-[2-(4-bromo-2-trifluoromethoxy-phenyl)-ethylamino]-cyclohexyl}-$N^4$-methyl-5,6,7,8-tetrahydro-quinazoline-2,4-diamine;
$N^2$-{cis-4-[(4-bromo-2-trifluoromethoxy-benzyl)amino-methyl]-cyclohexyl}-$N^4$-methyl-5,6,7,8-tetrahydro-quinazoline-2,4-diamine;
$N^2$-{cis-4-[(4-bromo-2-trifluoromethoxy-benzyl)amino-methyl]-cyclohexyl}-$N^4$,$N^4$-dimethyl-5,6,7,8-tetrahydroquinazoline-2,4-diamine;
$N^4$,$N^4$-dimethyl-$N^2$-{cis-4-[(2-trifluoromethoxy-benzyl)amino-methyl]-cyclohexyl}-5,6,7,8-tetrahydro-quinazoline-2,4-diamine; and
$N^4$-methyl-$N^2$-{cis-4-[(2-trifluoromethoxy-benzyl)amino-methyl]-cyclohexyl}-5,6,7,8-tetrahydro-quinazoline-2,4-diamine;

or a pharmaceutically acceptable salt, hydrate, or solvate thereof.

In some embodiments, compounds of the present invention are of Formula (I) wherein the compound is selected from the group consisting of:

$N^2$-(cis-4-{[(5-methoxy-1H-indol-3-yl)methyl]amino}cyclohexyl)-$N^4$,$N^4$-dimethyl-5,6,7,8-tetrahydroquinazoline-2,4-diamine;
ethyl 4,6-dichloro-3-{[(cis-4-{[4-(dimethylamino)-5,6,7,8-tetrahydroquinazolin-2-yl]-amino}cyclohexyl)amino]methyl}-1H-indole-2-carboxylate;
$N^2$-[cis-4-({[3-(4-fluorophenyl)-1H-pyrazol-4-yl]methyl}amino)cyclohexyl]-$N^4$,$N^4$-dimethyl-5,6,7,8-tetrahydroquinazoline-2,4-diamine;
3-[{2-[(cis-4-{[4-(dimethylamino)-5,6,7,8-tetrahydroquinazolin-2-yl]amino}cyclohexyl)-amino]ethyl}(phenyl)amino]propanenitrile;
N-{(1S)-1-benzyl-2-[(cis-4-{[4-(dimethylamino)-5,6,7,8-tetrahydroquinazolin-2-yl]amino}-cyclohexyl)amino]ethyl}-4-methylbenzenesulfonamide;
$N^2$-[cis-4-({[1-(diphenylmethyl)azetidin-3-yl]methyl}amino)cyclohexyl]-$N^4$,$N^4$-dimethyl-5,6,7,8-tetrahydroquinazoline-2,4-diamine;

$N^2$-(cis-4-{[(2,6-dimethoxybenzyl)amino]methyl}cyclohexyl)-$N^4$,$N^4$-dimethyl-5,6,7,8-tetrahydroquinazoline-2,4-diamine;
$N^2$-[cis-4-({[(5-methoxy-1H-indol-3-yl)methyl]amino}methyl)cyclohexyl]-$N^4$,$N^4$-dimethyl-5,6,7,8-tetrahydroquinazoline-2,4-diamine;
$N^2$-[cis-4-({[(5-bromo-1H-indol-3-yl)methyl]amino}methyl)cyclohexyl]-$N^4$,$N^4$-dimethyl-5,6,7,8-tetrahydroquinazoline-2,4-diamine;
$N^2$-(cis-4-{[(3-ethoxy-4-methoxybenzyl)amino]methyl}cyclohexyl)-$N^4$,$N^4$-dimethyl-5,6,7,8-tetrahydroquinazoline-2,4-diamine;
4-({[(cis-4-{[4-(dimethylamino)-5,6,7,8-tetrahydroquinazolin-2-yl]amino}cyclohexyl)-methyl]amino}methyl)-2-iodo-6-methoxyphenol;
$N^2$-(cis-4-{[(3,3-diphenylprop-2-en-1-yl)amino]methyl}cyclohexyl)-$N^4$,$N^4$-dimethyl-5,6,7,8-tetrahydroquinazoline-2,4-diamine;
$N^4$,$N^4$-dimethyl-$N^2$-(cis-4-{[(2,4,6-trimethoxybenzyl)amino]methyl}-cyclohexyl)-5,6,7,8-tetrahydroquinazoline-2,4-diamine;
$N^2$-(cis-4-{[(2,5-diethoxybenzyl)amino]methyl}cyclohexyl)-$N^4$,$N^4$-dimethyl-5,6,7,8-tetrahydroquinazoline-2,4-diamine;
$N^2$-(cis-4-{[(2,4-diethoxybenzyl)amino]methyl}cyclohexyl)-$N^4$,$N^4$-dimethyl-5,6,7,8-tetrahydroquinazoline-2,4-diamine;
$N^2$-(cis-4-{[(3,5-dibromo-2-methoxybenzyl)amino]methyl}cyclohexyl)-$N^4$,$N^4$-dimethyl-5,6,7,8-tetrahydroquinazoline-2,4-diamine;
$N^4$,$N^4$-dimethyl-$N^2$-(cis-4-{[(2,4,5-triethoxybenzyl)amino]methyl}-cyclohexyl)-5,6,7,8-tetrahydroquinazoline-2,4-diamine;
$N^4$,$N^4$-dimethyl-$N^2$-(cis-4-{[(2,4,5-trimethoxybenzyl)amino]methyl}-cyclohexyl)-5,6,7,8-tetrahydroquinazoline-2,4-diamine;
$N^4$,$N^4$-dimethyl-$N^2$-[cis-4-({[(1-phenyl-5-propyl-1H-pyrazol-4-yl)methyl]amino}methyl)-cyclohexyl]-5,6,7,8-tetrahydroquinazoline-2,4-diamine;
$N^2$-{cis-4-[({[1-(4-chlorophenyl)-5-propyl-1H-pyrazol-4-yl]methyl}-amino)methyl]-cyclohexyl)-$N^4$,$N^4$-dimethyl-5,6,7,8-tetrahydroquinazoline-2,4-diamine;
$N^2$-{cis-4-[2-(4-bromo-2-trifluoromethoxy-phenyl)-ethylamino]-cyclohexyl}-$N^4$,$N^4$-dimethyl-5,6,7,8-tetrahydroquinazoline-2,4-diamine;
$N^2$-{cis-4-[(4-bromo-2-trifluoromethoxy-benzyl)amino-methyl]-cyclohexyl}-$N^4$-methyl-5,6,7,8-tetrahydro-quinazoline-2,4-diamine;
$N^2$-{cis-4-[(4-bromo-2-trifluoromethoxy-benzyl)amino-methyl]-cyclohexyl)-$N^4$,$N^4$-dimethyl-5,6,7,8-tetrahydroquinazoline-2,4-diamine; and
$N^4$,$N^4$-dimethyl-$N^2$-{cis-4-[(2-trifluoromethoxy-benzyl)amino-methyl]-cyclohexyl}-5,6,7,8-tetrahydro-quinazoline-2,4-diamine;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- heterocyclyloxy,
- heterocyclyloxy substituted by $C_{1-5}$ alkyl,
- mono-$C_{1-5}$ alkylaminocarbonyl,
- di-$C_{1-5}$ alkylaminocarbonyl,
- carbocyclic arylcarbonylamino,
- $C_{1-5}$ alkylthio,
- $C_{1-5}$ alkylthio substituted by substituent(s) independently selected from the group consisting of:

- •• carbocyclic aryl, and
- •• carbocyclic aryl substituted by substituent(s) independently selected from the group consisting of:
    - ••• halogen, and
    - ••• $C_{1-5}$ alkoxy,

- • carbocyclic arylthio,
- • heterocyclylthio,
- • heterocyclylthic substituted by $C_{1-5}$ alkyl,
- • $C_{3-6}$ cycloalkyl,
- • $C_{3-6}$ cycloalkenyl,
- • carbocyclyl,
- • carbocyclyl substituted by substituent(s) independently selected from the group consisting of:
    - •• halogen,
    - •• $C_{1-5}$ alkyl,
    - •• $C_{1-5}$ alkoxy,
    - •• $C_{2-5}$ alkenyl, and
    - •• $C_{2-5}$ alkenyl substituted by substituent(s) independently selected from the group consisting of:
        - ••• carbocyclic aryl, and
        - ••• carbocyclic aryl substituted by $C_{1-5}$ alkylsulfinyl,

- • carbocyclic aryl,
- • carbocyclic aryl substituted by substituent(s) independently selected from the group consisting of:
    - •• halogen,
    - •• hydroxy,
    - •• nitro,
    - •• $C_{1-5}$ alkyl,
    - •• $C_{1-5}$ alkyl substituted by substituent(s) independently selected from the group consisting of:
        - ••• oxo,
        - ••• carbocyclic aryl, and
        - ••• heterocyclyl,

    - •• $C_{1-5}$ alkoxy,
    - •• $C_{1-5}$ alkoxy substituted by halogen,
    - •• $C_{1-5}$ alkoxy substituted by carbocyclic aryl,
    - •• carbocyclic aryloxy,
    - •• mono-carbocyclic arylaminocarbonyl,
    - •• mono-carbocyclic arylaminocarbonyl substituted by halogen,
    - •• di-carbocyclic arylaminocarbonyl,
    - •• di-carbocyclic arylaminocarbonyl substituted by halogen,
    - •• carbocyclic aryl, and
    - •• heterocyclyl,

- • heterocyclyl, and
- • heterocyclyl substituted by substituent(s) independently selected from the group consisting of:
    - •• $C_{1-5}$ alkyl,
    - •• $C_{1-5}$ alkoxy,
    - •• $C_{1-5}$ alkoxy substituted by carbocyclic aryl,
    - •• carbocyclic aryl, and
    - •• carbocyclic aryl substituted by halogen,

(ii) $C_{2-5}$ alkenyl, and
$C_{2-5}$ alkenyl substituted by substituent(s) independently selected from the group consisting of:

- carbocyclic aryl,
- carbocyclic aryl substituted by substituent(s) independently selected from the group consisting of:

  - •• halogen, and
  - •• nitro,

(iii) $C_{3-6}$ cycloalkyl, and
$C_{3-6}$ cycloalkyl substituted by substituent(s) independently selected from the group consisting of:

- $C_{1-5}$ alkyl,
- $C_{1-5}$ alkyl substituted by substituent(s) independently selected from the group consisting of:

  - •• oxo, and
  - •• carbocyclic aryl,

- carbocyclie aryl,

(iv) carbocyclyl,
(v) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:

  - •• halogen,
  - •• oxo,
  - •• carbocyclic aryloxy,
  - •• carbocyclic aryl, and
  - •• carbocyclic aryl substituted by $C_{1-5}$ alkyl,

- $C_{1-5}$ alkoxy,
- $C_{1-5}$ alkoxy substituted by substituent(s) independently selected from the group consisting of:

  - •• halogen, and
  - •• carbocyclic aryl,

- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,

- mono-$C_{1-5}$ alkylaminosulfonyl,
- di-$C_{1-5}$ alkylaminosulfonyl,
- carbocyclic aryl,
- heterocyclyl,
- heterocyclyl substituted by substituent(s) independently selected from the group consisting of:
  - •• $C_{1-5}$ alkyl,
  - •• carbocyclic aryl, and
  - •• halogenated carbocyclic aryl,

(vi) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:
  - •• halogen,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by halogen, and
  - •• heterocyclyl,
- $C_{1-5}$ alkoxy,
- carbocyclic aryloxy,
- carbocyclic aryloxy substituted by $C_{1-5}$ alkyl,
- $C_{1-5}$ alkylthio,
- $C_{2-5}$ alkenylthio,
- carbocyclic arylthio,
- $C_{1-5}$ alkylsulfonyl,
- carbocyclic arylsulfonyl,
- carbocyclic arylsulfonyl substituted by $C_{1-5}$ alkyl,
- carbocyclic aryl,
- carbocyclic aryl substituted by substituent(s) independently selected from the group consisting of:
  - •• halogen,
  - •• nitro, and
  - •• $C_{1-5}$ alkyl,
- heterocyclyl;

L is Formula (VII);
Y is -C(O)-; wherein carbocyclic aryl is phenyl, naphthyl, or anthranyl;
carbocyclyl is 1,2,3,4-tetrahydronaphthyl, 1-oxo-indanyl, 9-oxo-9*H*-fluorenyl, or indenyl;
heterocyclyl is 1,2,3-triazolyl, 1*H*-indolyl, 1*H*-pyrrolyl, 2,3-dihydro-1-oxo-isoindolyl, 2,4-dihydro-3-oxo-pyrazolyl, 2*H*-benzopyranyl, 2-oxo-benzopyranyl, 4-oxo-1,5,6,7-tetrahydro-indolyl, 9*H*-xanthenyl, benzo[1,3]dioxolyl, benzo[2,2,3]oxadiazolyl, benzo[1,2,5]oxadiazolyl, benzo[b]thienyl, benzofuryl, benzothiazolyl, furyl, isoxazolyl, morpholino, pyrazolyl, pyridyl, pyrimidyl, quinolyl, quinoxalyl, thiazolyl, or thienyl; and
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are hydrogen; A is a single bond; B is a single bond or $-CH_2-$; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- mono-$C_{1-5}$ alkylaminocarbonyl,
- di-$C_{1-5}$ alkylaminocarbonyl,
- carbocyclic arylcarbonylamino,
- $C_{1-5}$ alkylthio,
- $C_{1-5}$ alkylthio substituted by substituent(s) independently selected from the group consisting of:
    - carbocyclic aryl, and
    - carbocyclic aryl substituted by halogen,
- heterocyclylthio,
- heterocyclylthio substituted by $C_{1-5}$ alkyl,
- $C_{3-6}$ cycloalkyl,
- carbocyclyl,
- carbocyclyl substituted by substituent(s) independently selected from the group consisting of:
    - halogen,
    - $C_{1-5}$ alkyl,
    - $C_{2-5}$ alkenyl, and
    - $C_{2-5}$ alkenyl substituted by substituent(s) independently selected from the group consisting of:
        - carbocyclic aryl, and
        - carbocyclic aryl substituted by $C_{1-5}$ alkylsulfinyl,
- carbocyclic aryl,
- carbocyclic aryl substituted by substituent(s) independently selected from the group consisting of:
    - halogen,
    - hydroxy,
    - nitro,
    - $C_{1-5}$ alkyl,
    - $C_{1-5}$ alkyl substituted by substituent(s) independently selected from the group consisting of:
        - oxo, and
        - heterocyclyl,
    - $C_{1-5}$ alkoxy,
    - carbocyclic aryloxy,
    - carbocyclic aryl, and
    - heterocyclyl,
- heterocyclyl, and
- heterocyclyl substituted by substituent(s) independently selected from the group consisting of:
    - $C_{1-5}$ alkyl,
    - $C_{1-5}$ alkoxy, and
    - carbocyclic aryl,

(ii) $C_{2-5}$ alkenyl, and
$C_{2-5}$ alkenyl substituted by substituent(s) independently selected from the group consisting of:

- carbocyclic aryl, and
- carbocyclic aryl substituted by nitro,

(iii) $C_{3-6}$ cycloalkyl, and
$C_{3-6}$ cycloalkyl substituted by carbocyclic aryl,
(iv) carbocyclyl,
(v) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:

  - •• halogen,
  - •• oxo, and
  - •• carbocyclic aryl,

- $C_{1-5}$ alkoxy,
- $C_{1-5}$ alkoxy substituted by substituent(s) independently selected from the group consisting of:

  - •• halogen, and
  - •• carbocyclic aryl,

- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy, and
- (carbocyclic aryl)NHC(O)NH substituted by haloganated $C_{1-5}$ alkoxy,

(vi) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:

  - •• halogen,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl, and
  - •• heterocyclyl,

- carbocyclic aryloxy,
- carbocyclic aryloxy substituted by $C_{1-5}$ alkyl,
- $C_{1-5}$ alkylthio,

- carbocyclic aryl,
- carbocyclic aryl substituted by substituent(s) independently selected from the group consisting of:
  - halogen,
  - nitro, and
  - $C_{1-5}$ alkyl,
- heterocyclyl;

wherein carbocyclic aryl is phenyl;
carbocyclyl is 1-oxo-indanyl or indenyl;
heterocyclyl is 1,2,3-triazolyl, 1*H*-indolyl, 1*H*-pyrrolyl, 2,3-dihydro-1-oxo-isoindolyl, 2-oxo-benzopyranyl, benzo[2,1,3]oxadiazolyl, benzo[1,2,5]oxadiazolyl, furyl, isoxazolyl, morpholino, pyrazolyl, pyridyl, pyrimidyl, quinolyl, quinoxalyl, thiazolyl, or thienyl;
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkylcarbonyloxy,
- carbocyclic aryloxy,
- carbocyclic aryloxy substituted by halogen,
- mono-$C_{1-5}$ alkylaminocarbonyl,
- di-$C_{1-5}$ alkylaminocarbonyl,
- carbocyclic arylcarbonylamino,
- carbocyclyl,
- carbocyclyl substituted by substituent(s) independently selected from the group consisting of:
  - halogen,
  - $C_{,-5}$ alkyl,
  - $C_{1-5}$ alkenyl, and
  - $C_{2-5}$ alkenyl substituted by substituent(s) independently selected from the group consisting of:
    - carbocyclic aryl, and
    - carbocyclic aryl substituted by $C_{1-5}$ alkylsulfinyl,
- carbocyclic aryl,
- carbocyclic aryl substituted by substituent(s) independently selected from the group consisting of:
  - halogen,
  - hydroxy,
  - nitro,
  - $C_{1-5}$ alkyl, and
  - $C_{1-5}$ alkoxy,
- heterocyclyl, and
- heterocyclyl substituted by substituent(s) independently selected from the group consisting of:
  - $C_{1-5}$ alkyl,
  - $C_{1-5}$ alkoxy, and
  - carbocyclic aryl,

(ii) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:
  - •• halogen, and
  - •• oxo,
- $C_{1-5}$ alkoxy,
- $C_{1-5}$ alkoxy substituted by carbocyclic aryl,
- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy, and
- (carbocyclic aryl)NHC(O)NH substituted by haloganated $C_{1-5}$ alkoxy,

(iii) heterocyclyl, and
heterocyclyl substituted by substituent(s) independently selected from the group consisting of:

- halogen,
- nitro,
- $C_{1-5}$ alkyl,
- $C_{1-5}$ alkyl substituted by halogen,
- $C_{1-5}$ alkyl substituted by heterocyclyl,
- carbocyclic aryloxy,
- carbocyclic aryloxy substituted by $C_{1-5}$ alkyl,
- $C_{1-5}$ alkylthio,
- carbocyclic aryl,
- carbocyclic aryl substituted by halogen, and
- carbocyclic aryl substituted by nitro;

wherein carbocyclic aryl is phenyl;
carbocyclyl is indenyl;
heterocyclyl is 1*H*-indolyl, 1*H*-pyrrolyl, 2-oxo-benzopyranyl, benzo[2,1,3]oxadiazolyl, benzo[1,2,5]oxadiazolyl, furyl, isoxazolyl, morpholino, pyridyl, quinoxalyl, or thienyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(cis-4-{[4-(dimethylamino)-5,6,7,8-tetrahydroquinazolin-2-yl]amino}-cyclohexyl)-3-methoxybenzamide;
3-bromo-N-(cis-4-{[4-(dimethylamino)-5,6,7,8-tetrahydroquinazolin-2-yl]amino}-cyclohexyl)benzamide;
4-bromo-N-(cis-4-{[4-(dimethylamino)-5,6,7,8 -tetrahydroquinazolin-2-yl]amino}-cyclohexyl)benzamide;
N-(cis-4-{[4-(dimethylamino)-5,6,7,8-tetrahydroquinazolin-2-yl]amino}-cyclohexyl)-2,1,3-benzoxadiazole-5-carboxamide;
3-chloro-N-(cis-4-{[4-(dimethylamino)-5,6,7,8-tetrahydroquinazolin-2-yl]amino}-cyclohexyl)benzamide;
4-chloro-N-(cis-4-{[4-(dimethylamino)-5,6,7,8-tetrahydroquinazolin-2-yl]amino}-cyclohexyl)benzamide;
4-chloro-N-(cis-4-{[4-(dimethylamino)-5,6,7,8-tetrahydroquinazolin-2-yl]amino}-cyclohexyl)-3-nitrobenzamide;
2-(4-chlorophenyl)-N-(cis-4-{[4-(dimethylamino)-5,6,7,8-tetrahydroquinazolin-2-yl]-amino}cyclohexyl)acetamide;

3-cyano-N-(cis-4-{[4-(dimethylamino)-5,6,7,8-tetrahydroquinazolin-2-yl]amino}-cyclohexyl)benzamide;
3,5-dichloro-N-(cis-4-{[4-(dimethylamino)-5,6,7,8-tetrahydroquinazolin-2-yl]amino}-cyclohexyl)benzamide;
3,4-dichloro-N-(cis-4-{[4-(dimethylamino)-5,6,7,8-tetrahydroquinazolin-2-yl]amino}-cyclohexyl)benzamide;
N-(cis-4-{[4-(dimethylamino)-5,6,7,8-tetrahydroquinazolin-2-yl]amino}-cyclohexyl)-2,2-diphenylacetamide;
N-(cis-4-{[4-(dimethylamino)-5,6,7,8-tetrahydroquinazolin-2-yl]amino}-cyclohexyl)-3,4-difluorobenzamide;
N-(cis-4-{[4-(dimethylamino)-5,6,7,8-tetrahydroquinazolin-2-yl]amino}-cyclohexyl)-3,5-difluorobenzamide;
N-(cis-4-{[4-(dimethylamino)-5,6,7,8-tetrahydroquinazolin-2-yl]amino}-cyclohexyl)-4-fluorobenzamide;
N-(cis-4-{[4-(dimethylamino)-5,6,7,8-tetrahydroquinazolin-2-yl]amino}-cyclohexyl)-3-fluoro-5-(trifluoromethyl) benzamide;
N-(cis-4-{[4-(dimethylamino)-5,6,7,8-tetrahydroquinazolin-2-yl]amino}-cyclohexyl)-hexanamide;
N-(cis-4-{[4-(dimethylamino)-5,6,7,8-tetrahydroquinazolin-2-yl]amino}-cyclohexyl)-4-methyl-3-nitrobenzamide;
N-(cis-4-{[4-(dimethylamino)-5,6,7,8-tetrahydroquinazolin-2-yl]amino}-cyclohexyl)-3-nitrobenzamide;
(2R)-N-(cis-4-{[4-(dimethylamino)-5,6,7,8-tetrahydroquinazolin-2-yl]amino}cyclohexyl)-2-phenylcyclopropane-carboxamide;
N-(cis-4-{[4-(dimethylamino)-5,6,7,8-tetrahydroquinazolin-2-yl]amino}-cyclohexyl)-2-phenoxybutanamide;
N-(cis-4-{[4-(dimethylamino)-5,6,7,8-tetrahydroquinazolin-2-yl]amino}-cyclohexyl)-2-phenoxypropanamide;
N-(cis-4-{[4-(dimethylamino)-5,6,7,8-tetrahydroquinazolin-2-yl]amino}-cyclohexyl)-3-methylbenzamide;
N-(cis-4-{[4-(dimethylamino)-5,6,7,8-tetrahydroquinazolin-2-yl]amino}-cyclohexyl)-4-methylbenzamide;
N-(cis-4-{[4-(dimethylamino)-5,6,7,8-tetrahydroquinazolin-2-yl]amino}-cyclohexyl)-3-(trifluoromethoxy)benzamide;
4-bromo-N-(cis-4-{[4-(dimethylamino)-5,6,7,8-tetrahydroquinazolin-2-yl]amino}-cyclohexyl)-3-methylbenzamide;
N-(cis-4-{[4-(dimethylamino)-5,6,7,8-tetrahydroquinazotin-2-yl]amino}-cyclohexyl)-3-iodobenzamide;
2-chloro-N-(cis-4-{[4-(dimethylamino)-5,6,7,8-tetrahydroquinazolin-2-yl]amino}-cyclohexyl)-4-fluorobenzamide;
N-(cis-4-{[4-(dimethylamino)-5,6,7,8-tetrahydroquinazolin-2-yl]amino}-cyclohexyl)-2-(3-methoxyphenyl)acetamide;
N-(cis-4-{[4-(dimethylamino)-5,6,7,8-tetrahydroquinazoiin-2-yl]amino}-cyclohexyl)-2-(4-fluorophenyl)acetamide;
N-(cis-4-{[4-(dimethylamino)-5,6,7,8-tetrahydroquinazolin-2-yl]amino}-cyclohexyl)-2-(4-methoxyphenyl)acetamide;
N-(cis-4-{[4-(dimethylamino)-5,6,7,8-tetrahydroquinazolin-2-yl]amino}-cyclohexyl)-5-methyl-2-(trifluoromethyl)-3-furamide;
N-(cis-4-{[4-(dimethylamino)-5,6,7,8-tetrahydroquinazolin-2-yl]amino}-cyclohexyl)-2,5-dimethyl-3-furamide;
3-chloro-N-(cis-4-{[4-(dimethylamino)-5,6,7,8-tetrahydroquinazolin-2-yl]amino}-cyclohexyl)-4-fluorobenzamide;
N-(cis-4-{[4-(dimethylamino)-5,6,7,8-tetrahydroquinazolin-2-yl]amino}-cyclohexyl)-3-fluoro-4-methylbenzamide;
N-(cis-4-{[4-(dimethylamino)-5,6,7,8-tetrahydroquinazolin-2-yl]amino}-cyclohexyl)-3,5-dimethoxybenzamide;
N-(cis-4-{[4-(dimethylamino)-5,6,7,8-tetrahydroquinazolin-2-yl]amino}-cyclohexyl)-3,5-bis(trifluoromethyl)benzamide;
N-(cis-4-{[4-(dimethylamino)-5,6,7,8-tetrahydroquinazolin-2-yl]amino}-cyclohexyl)-4-fluoro-3-methylbenzamide;
2,5-dichloro-N-(cis-4-{[4-(dimethylamino)-5,6,7,8-tetrahydroquinazolin-2-yl]amino}-cyclohexyl)thiophene-3-carboxamide;
N-(cis-4-{[4-(dimethylamino)-5,6,7,8-tetrahydroquinazolin-2-yl]amino}-cyclohexyl)-2-(propylthio)nicotinamide;
1-benzyl-3-tert-butyl-N-(cis-4-{[4-(dimethylamino)-5,6,7,8-tetrahydroquinazolin-2-yl]-amino}cyclohexyl)-1H-pyrazole-5-carboxamide;
5-bromo-N-(cis-4-{[4-(dimethylamino)-5,6,7,8-tetrahydroquinazolin-2-yl]amino}-cyclohexyl)nicotinamide;
2-[(cis-4-{[4-(dimethylamino)-5,6,7,8-tetrahydroquinazolin-2-yl]amino}-cyclohexyl)-amino]-2-oxo-1-phenylethyl acetate;
N-(cis-4-{[4-(dimethylamino)-5,6,7,8-tetrahydroquinazolin-2-yl]amino}-cyclohexyl)-benzamide;
2-(benzyloxy)-N-(cis-4-{[4-(dimethylamino)-5,6,7,8-tetrahydroquinazolin-2-yl]amino}-cyclohexyl)acetamide;
2-(4-chlorophenoxy)-N-(cis-4-{[4-(dimethylamino)-5,6,7,8-tetrahydroquinazolin-2-yl]-amino}cyclohexyl)acetamide;
3-(2-chlorophenyl)-N-(cis-4-{[4-(dimethylamino)-5,6,7,8-tetrahydroquinazolin-2-yl]-amino}cyclohexyl)-5-methylisoxazole-4-carboxamide;
1-(4-chlorophenyl)-N-(cis-4-{[4-(dimethylamino)-5,6,7,8-tetrahydroquinazolin-2-yl]-amino}cyclohexyl)cyclopentanecarboxamide;
3-(2-chloro-6-fluorophenyl)-N-(cis-4-{[4-(dimethylamino)-5,6,7,8-tetrahydroquinazolin-2-yl]amino}cyclohexyl)-5-methylisoxazole-4-carboxamide;
N-(cis-4-{[4-(dimethylamino)-5,6,7,8-tetrahydroquinazolin-2-yl]amino}-cyclohexyl)-1,3-dimethyl-1H-pyrazole-5-carboxamide;
N-(cis-4-{[4-(dimethylamino)-5,6,7,8-tetrahydroquinazolin-2-yl]amino}-cyclohexyl)-3-fluorobenzamide;

N-(cis-4-{[4-(dimethylamino)-5,6,7,8-tetrahydroquinazolin-2-yl]amino}-cyclohexyl)-4-fluoro-3-(trifluoromethyl) benzamide;
N-(cis-4-{[4-(dimethylamino)-5,6,7,8-tetrahydroquinazolin-2-yl)amino}-cyclohexyl)-5-methyl-2-phenyl-2H-1,2,3-triazole-4-carboxamide;
N-(cis-4-{[4-(dimethylamino)-5,6,7,8-tetrahydroquinazolin-2-yl)amino}-cyclohexyl)-2-(4-methoxyphenoxy)-5-nitrobenzamide;
N-(cis-4-{[4-(dimethylamino)-5,6,7,8-tetrahydroquinazolin-2-yl]amino}-cyclohexyl)-5-nitro-2-furamide;
N-(cis-4-{[4-(dimethylamino)-5,6,7,8-tetrahydroquinazolin-2-yl]amino}-cyclohexyl)-2-phenoxyacetamide;
N-(cis-4-{[4-(dimethylamino)-5,6,7,8-tetrahydroquinazolin-2-yl]amino}-cyclohexyl)-quinoxaline-2-carboxamide;
N-(cis-4-{[4-(dimethylamino)-5,6,7,8-tetrahydroquinazolin-2-yl]amino}-cyclohexyl)-3-(trifluoromethyl)benzamide;
2-(3-chlorophenoxy)-N-(cis-4-{[4-(dimethylamino)-5,6,7,8-tetrahydroquinazolin-2-yl]-amino}cyclohexyl)acetamide;
3-(2,6-dichlorophenyl)-N-(cis-4-{[4-(dimethylamino)-5,6,7,8-tetrahydroquinazolin-2-yl]-amino}cyclohexyl)-5-methylisoxazole-4-carboxamide;
N-(cis-4-{[4-(dimethylamino)-5,6,7,8-tetrahydroquinazolin-2-yl]amino}-cyclohexyl)-2-phenoxynicotinamide;
N-(cis-4-{[4-(dimethylamino)-5,6,7,8-tetrahydroquinazolin-2-yl]amino}-cyclohexyl)-2-(4-methylphenoxy)nicotinamide;
N-(cis-4-{[4-(dimethylamino)-5,6,7,8-tetrahydroquinazolin-2-yl]amino}-cyclohexyl)-2-(2-thienyl)-1,3-thiazole-4-carboxamide;
5-bromo-N-(cis-4-{[4-(dimethylamino)-5,6,7,8-tetrahydroquinazolin-2-yl]amino}-cyclohexyl)thiophene-2-carboxamide;
N-(cis-4-{[4-(dimethylamino)-5,6,7,8-tetrahydroquinazolin-2-yl]amino}-cyclohexyl)-2-(2,3,6-trichlorophenyl)acetamide;
2-(2-chloro-4-fluorophenyl)-N-(cis-4-{[4-(dimethylamino)-5,6,7,8-tetrahydroquinazolin-2-yl]amino}cyclohexyl) acetamide;
5-(4-chloro-2-nitrophenyl)-N-(cis-4-{[4-(dimethylamino)-5,6,7,8-tetrahydroquinazolin-2-yl]amino}cyclohexyl)-2-furamide;
5-chloro-N-(cis-4-{[4-(dimethylamino)-5,6,7,8-tetrahydroquinazolin-2-yl]amino}-cyclohexyl)thiophene-2-carboxamide;
N-(cis-4-{[4-(dimethylamino)-5,6,7,8-tetrahydroquinazolin-2-yl]amino}-cyclohexyl)-2,3-diphenylpropanamide;
N-(cis-4-{[4-(dimethylamino)-5,6,7,8-tetrahydroquinazolin-2-yl]amino}-cyctohexyl)-3-(2-hydroxyphenyl)propanamide;
N-(cis-4-{[4-(dimethylamino)-5,6,7,8-tetrahydroquinazolin-2-yl]amino}-cyclohexyl)-5-iodo-2-furamide;
N-(cis-4-{[4-(dimethylamino)-5,6,7,8-tetrahydroquinazolin-2-yl]amino}-cyclohexyl)-2-(2-iodophenyl)acetamide;
N-(cis-4-{[4-(dimethylamino)-5,6,7,8-tetrahydroquinazolin-2-yl]amino}-cyclohexyl)-2-(5-methoxy-2-methyl-1H-indol-3-yl)acetamide;
N-(cis-4-{[4-(dimethylamino)-5,6,7,8-tetrahydroquinazolin-2-yl]amino}-cyclohexyl)-3-oxoindane-1-carboxamide;
2-benzyl-N-(cis-4-{[4-(dimethylamino)-5,6,7,8-tetrahydroquinazolin-2-yl]amino}-cyclohexyl)benzamide;
2,2-bis(4-chlorophenyl)-N-(cis-4-{[4-(dimethylarnino)-5,6,7,8-tetrahydroquinazolin-2-yl]-amino}cyclohexyl)acetamide;
N-(cis-4-{[4-(dimethylamino)-5,6,7,8-tetrahydroquinazolin-2-yl]amino}-cyclohexyl)-5-(4-methyl-2-nitrophenyl)-2-furamide;
N-(cis-4-{[4-(dimethylamino)-5,6,7,8-tetrahydroquinazolin-2-yl]amino}-cyclohexyl)-5-nitrothiophene-2-carboxamide;
N-(cis-4-{[4-(dimethylamino)-5,6,7,8-tetrahydroquinazolin-2-yl]amino}-cyclohexyl)-3-methyl-4-nitrobenzamide;
N-(cis-4-{[4-(dimethylamino)-5,6,7,8-tetrahydroquinazolin-2-yl]amino}-cyclohexyl)-3-methoxy-4-nitrobenzamide;
3-acetyl-N-(cis-4-{[4-(dimethylamino)-5,6,7,8-tetrahydroquinazolin-2-yl]amino}-cyclohexyl)benzamide;
5-bromo-N-(cis-4-{[4-(dimethylamino)-5,6,7,8-tetrahydroquinazolin-2-yl]amino}-cyclohexyl)-2-furamide;
N-(cis-4-{[4-(dimethylamino)-5,6,7,8-tetrahydroquinazolin-2-yl]amino}-cyclohexyl)-2-[(4-methylpyrimidin-2-yl) thio]acetamide;
5-(4-chlorophenyl)-N-(cis-4-{[4-(dimethylamino)-5,6,7,8-tetrahydroquinazolin-2-yl]-amino}cyclohexyl)-2-furamide;
2-(3,4-dichlorophenyl)-N-(cis-4-{[4-(dimethylamino)-5,6,7,8-tetrahydroquinazolin-2-yl]-amino}cyclohexyl)acetamide;
N-(cis-4-{[4-(dimethylamino)-5,6,7,8-tetrahydroquinazolin-2-yl]amino}-cyclohexyl)-2-(4-hydroxy-3,5-dimethoxyphenyl)acetamide;
4,5-dibromo-N-(cis-4-{[4-(dimethylamino)-5,6,7,8-tetrahydroquinazolin-2-yl]amino}-cyclohexyl)thiophene-2-carboxamide;

$N^2$,$N^6$-dibenzoyl-N-(cis-4-{[4-(dimethylamino)-5,6,7,8-tetrahydroquinazolin-2-yl]amino}-cyclohexyl)lysinamide;
3-(dimethylamino)-N-(cis-4-{[4-(dimethylamino)-5,6,7,8-tetrahydroquinazolin-2-yl]-amino}cyclohexyl)benzamide;
4,5-dibromo-N-(cis-4-{[4-(dimethylamino)-5,6,7,8-tetrahydroquinazolin-2-yl]amino}-cyclohexyl)-2-furamide;
N-(cis-4-{[4-(dimethylamino)-5,6,7,8-tetrahydroquinazolin-2-yl]amino}-cyclohexyl)-4-(4-fluorophenyl)-4-oxobutanamide;
N-(cis-4-{[4-(dimethylamino)-5,6,7,8-tetrahydroquinazolin-2-yl]amino}-cyclohexyl)-2-(2-fluorobiphenyl-4-yl)propanamide;
N-(cis-4-{[4-(dimethylamino)-5,6,7,8-tetrahydroquinazolin-2-yl]amino}-cyclohexyl)-2-[4-(1-oxo-1,3-dihydro-2H-isoindol-2-yl)phenyl]propanamide;
N-(cis-4-{[4-(dimethylamino)-5,6,7,8-tetrahydroquinazolin-2-yl]amino}-cyclohexyl)-2-(1H-indol-3-yl)acetamide;
N-(cis-4-{[4-(dimethylamino)-5,6,7,8-tetrahydroquinazolin-2-yl]amino}-cyclohexyl)-2-(7-methoxy-2-oxo-2H-chromen-4-yl)acetamide;
N-(cis-4-{[4-(dimethylamino)-5,6,7,8-tetrahydroquinazolin-2-yl]amino}-cyclohexyl)-2-(1H-indol-3-yl)-4-oxo-4-phenylbutanamide;
N-(cis-4-{[4-(dimethylamino)-5,6,7,8-tetrahydroquinazolin-2-yl]amino}-cyclohexyl)-3,5-dimethyl-2-[({[4-(trifluoromethoxy)phenyl]amino}carbonyl)amino]-benzamide;
3,5-dichloro-N-(cis-4-{[4-(dimethylamino)-5,6,7,8-tetrahydroquinazolin-2-yl]amino)-cyclohexyl)-2-[(3-phenylprop-2-ynoyl)amino]benzamide;
4-(4-tert-butylphenyl)-N-(cis-4-{[4-(dimethylamino)-5,6,7,8-tetrahydroquinazolin-2-yl]-amino}cyclohexyl)-2-(7-ethyl-1H-indol-3-yl)-4-oxobutanamide;
N-(cis-4-{[4-(dimethylamino)-5,6,7,8-tetrahydroquinazolin-2-yl]amino}-cyclohexyl)-2-(1-methyl-1H-indol-3-yl)-4-(4-methylphenyl)-4-oxobutanamide;
N-(cis-4-{[4-(dimethylamino)-5,6,7,8-tetrahydroquinazolin-2-yl]amino}-cyclohexyl)-2-methyl-1-(3-morpholin-4-ylpropyl)-5-phenyl-1H-pyrrole-3-carboxamide;
N-(cis-4-{[4-(dimethylamino)-5,6,7,8-tetrahydroquinazolin-2-yl]amino}-cyclohexyl)-4-(4-nitrophenyl)butanamide;
N-(cis-4-{[4-(dimethylamino)-5,6,7,8-tetrahydroquinazolin-2-yl]amino}-cyclohexyl)-2-(3-phenoxyphenyl)acetamide;
N-(cis-4-{[4-(dimethylamino)-5,6,7,8-tetrahydroquinazolin-2-yl]amino}-cyclohexyl)-2-(4-phenoxyphenyl)acetamide;
N-(cis-4-{[4-(dimethylamino)-5,6,7,8-tetrahydroquinazolin-2-yl]amino}-cyclohexyl)-2-(2-phenyl-1H-indol-3-yl) acetamide;
$N^2$-benzoyl-$N^5$-(cis-4-{[4-(dimethylamino)-5,6,7,8-tetrahydroquinazolin-2-yl]amino}-cyclohexyl)-$N^1$,$N^1$-dipropylglutamamide;
N-(cis-4-{[4-(dimethylamino)-5,6,7,8-tetrahydroquinazolin-2-yl]amino}-cyclohexyl)-3-phenoxybenzamide;
N-(cis-4-{[4-(dimethylamino)-5,6,7,8-tetrahydroquinazolin-2-yl]amino}-cyclohexyl)-2-(ethylthio)-2,2-diphenylacetamide;
N'-(cis-4-{[4-(dimethylamino)-5,6,7,8-tetrahydroquinazolin-2-yl]amino}-cyclohexyl)-N,N-bis[(1S)-1-phenylethyl] phthalamide;
(2S)-N-(cis-4-{[4-(dimethylamino)-5,6,7,8-tetrahydroquinazolin-2-yl]amino}cyclohexyl)-2-(2-fluorobiphenyl-4-yl) propanamide;
2-[(4-chlombenzyl)thio]-N-(cis-4-{[4-(dimethylamino)-5,6,7,8-tetrahydroquinazolin-2-yl]-amino}cyclohexyl)-4-(4-methylphenyl)-4-oxobutanamide;
N-(cis-4-{[4-(dimethylamino)-5,6,7,8-tetrahydroquinazolin-2-yl]amino}-cyclohexyl)-2-{(1E)-5-fluoro-2-methyl-1-[4-(methylsulfonyl)benzylidene]-1H-inden-3-yl]acetamide;
N-(cis-4-{[4-(dimethylamino)-5,6,7,8-tetrahydroquinazolin-2-yl]amino}-cyclohexyl)-2-[4-(2-thienylcarbonyl)phenyl]propanamide;
3-(benzyloxy)-N-(cis-4-{[4-(dimethylamino)-5,6,7,8-tetrahydroquinazolin-2-yl]amino}-cyclohexyl)-4-methoxybenzamide;
N-(cis-4-{[4-(dimethylamino)-5,6,7,8-tetrahydroquinazolin-2-yl]amino}-cyclohexyl)-2-methyl-1,5-diphenyl-1H-pyrrole-3-carboxamide;
1-{2-[(2-chloro-6-fluorobenzyl)thio]ethyl}-N-(cis-4-{[4-(dimethylamino)-5,6,7,8-tetrahydroquinazolin-2-yl]amino} cyclohexyl)-2-methyl-5-phenyl-1H-pyrrole-3-carboxamide;
N-(cis-4-{[4-(dimethylamino)-5,6,7,8-tetrahydroquinazolin-2-yl]amino}-cyclohexyl)-2-phenoxybenzamide;
N-(cis-4-{[4-(dimethylamino)-5,6,7,8-tetrahydroquinazolin-2-yl]amino}-cyclohexyl)-2-phenylquinoline-4-carboxamide;
N-(cis-4-{[4-(dimethylamino)-5,6,7,8-tetrahydroquinazolin-2-yl]amino}-cyclohexyl)-5-(3-nitrophenyl)-2-furamide;
N-(cis-4-{[4-(dimethylamino)-5,6,7,8-tetrahydroquinazolin-2-yl]amino}-cyclohexyl)-5-nitrothiophene-3-carboxamide;

N-(cis-4-{[4-(dimethylamino)-5,6,7,8-tetrahydroquinazolin-2-yl]amino}-cyclohexyl)-1-methyl-4-nitro-1H-pyrrole-2-carboxamide;
N-(cis-4-{[4-(dimethylamino)-5,6,7,8-tetrahydroquinazolin-2-yl]amino}-cyclohexyl)-2-methoxy-4-nitrobenzamide;
N-(cis-4-{[4-(dimethylamino)-5,6,7,8-tetrahydroquinazolin-2-yl]amino}-cyclohexyl)-2-methoxy-2-phenylacetamide;
5-chloro-N-(cis-4-{[4-(dimethylamino)-5,6,7,8-tetrahydroquinazolin-2-yl]amino}-cyclohexyl)-2-hydroxybenzamide;
3-bromo-N-[(cis-4-{[4-(dimethylamino)-5,6,7,8-tetrahydroquinazolin-2-yl]amino}-cyclohexyl)methyl]benzamide;
N-[(cis-4-{[4-(dimethylamino)-5,6,7,8-tetrahydroquinazolin-2-yl]amino}-cyclohexyl)-methyl]-2-(ethylthio)nicotinamide;
N-[(cis-4-{[4-(dimethylamino)-5,6,7,8-tetrahydroquinazolin-2-yl]amino}-cyclohexyl)-methyl]-2-(4-methoxyphenyl) acetamide;
N-[(cis-4-{[4-(dimethylamino)-5,6,7,8-tetrahydroquinazolin-2-yl]amino}-cyclohexyl)-methyl]-5-methyl-2-(trifluoromethyl)-3-furamide;
(2E)-N-[(cis-4-{[4-(dimethylamino)-5,6,7,8-tetrahydroquinazolin-2-yl]amino}cyclohexyl)-methyl]-3-(4-nitrophenyl) acrylamide;
N-[(cis-4-{[4-(dimethylamino)-5,6,7,8-tetrahydroquinazolin-2-yl]amino}-cyclohexyl)-methyl]-4-fluoro-3-methylbenzamide;
N-[(cis-4-{[4-(dimethylamino)-5,6,7,8-tetrahydroquinazolin-2-yl]amino}-cyclohexyl)-methyl]-2-(propylthio)nicotinamide;
2,6-dichloro-N-[(cis-4-{[4-(dimethylamino)-5,6,7,8-tetrahydroquinazolin-2-yl]amino}-cyclohexyl)methyl]benzamide;
N-[(cis-4-{[4-(dimethylamino)-5,6,7,8-tetrahydroquinazolin-2-yl]amino}-cyclohexyl)-methyl]-2,4,6-trimethylbenzamide;
2-chloro-N-[(cis-4-{[4-(dimethylamino)-5,6,7,8-tetrahydroquinazolin-2-yl]amino}-cyclohexyl)methyl]-6-fluorobenzamide;
2,4,6-trichloro-N-[(cis-4-{[4-(dimethylamino)-5,6,7,8-tetrahydroquinazolin-2-yl]amino}-cyclohexyl)methyl]benzamide;
N-[(cis-4-{[4-(dimethylamino)-5,6,7,8-tetrahydroquinazolin-2-yl]amino}-cyclohexyl)-methyl]-2-(2,3,6-trichlorophenyl)acetamide;
(2E)-N-[(cis-4-{[4-(dimethylamino)-5,6,7,8-tetrahydroquinazolin-2-yl]amino}cyclohexyl)-methyl]-3-(3-nitrophenyl) acrylamide; and
N-[cis-4-(4-dimethylamino-5,6,7,8-tetrahydro-quinazolin-2-ylamino)-cyclohexylmethyl]-3,4-difluoro-benzamide;

or a pharmaceutically acceptable salt, hydrate, or solvate thereof.

In some embodiments, compounds of the present invention are of Formula (I) wherein the compound is selected from the group consisting of:

N-(cis-4-{[4-(dimethylaminono)-5,6,7,8-tetrahydroquinazolin-2-yl]amino}-cyclohexyl)-3-methoxybenzamide;
3-bromo-N-(cis-4-{[4-(dimethylamino)-5,6,7,8-tetrahydroquinazolin-2-yl]amino}-cyclohexyl)benzamide;
N-(cis-4-{[4-(dimethylamino)-5,6,7,8-tetrahydroquinazolin-2-yl]amino}-cyclohexyl)-2,1,3-benzoxadiazole-5-carboxamide;
3-chloro-N-(cis-4-{[4-(dimethylamino)-5,6,7,8-tetrahydroquinazolin-2-yl]amino}-cyclohexyl)benzamide;
4-chloro-N-(cis-4-{[4-(dimethylamino)-5,6,7,8-tetrahydroquinazolin-2-yl]amino}-cyclohexyl)benzamide;
4-chloro-N-(cis-4-{[4-(dimethylamino)-5,6,7,8-tetrahydroquinazolin-2-yl]amino}-cyclohexyl)-3-nitrobenzamide;
2-(4-chlorophenyl)-N-(cis-4-{[4-(dimethylamino)-5,6,7,8-tetrahydroquinazolin-2-yl]-amino}cyclohexyl)acetamide;
3-cyano-N-(cis-4-{[4-(dimethylamino)-5,6,7,8-tetrahydroquinazolin-2-yl]amino}-cyclohexyl)benzamide;
3,5-dichloro-N-(cis-4-{[4-(dimethylamino)-5,6,7,8-tetrahydroquinazolin-2-yl]amino}-cyclohexyl)benzamide;
3,4-dichloro-N-(cis-4-{[4-(dimethylamino)-5,6,7,8-tetrahydroquinazolin-2-yl]amino}-cyclohexyl)benzamide;
N-(cis-4-{[4-(dimethylamino)-5,6,7,8-tetrahydroquinazolin-2-yl]amino}-cyclohexyl)-2,2-diphenylacetamide;
N-(cis-4-{[4-(dimethylamino)-5,6,7,8-tetrahydroquinazolin-2-yl]amino}-cyclohexyl)-3,4-difluorobenzamide;
N-(cis-4-{[4-(dimethylamino)-5,6,7,8-tetrahydroquinazolin-2-yl]amino}-cyclohexyl)-3,5-difluorobenzamide;
N-(cis-4-{[4-(dimethylamino)-5,6,7,8-tetrahydroquinazolin-2-yl]amino}-cyclohexyl)-4-fluorobenzamide;
N-(cis-4-{[4-(dimethylamino)-5,6,7,8-tetrahydroquinazolin-2-yl]amino}-cyclohexyl)-3-ftuoro-5-(trifluoromethyl) benzamide;
N-(cis-4-{[4-(dimethylamino)-5,6,7,8-tetrahydroquinazolin-2-yl]amino}-cyclohexyl)-4-methyl-3-nitrobenzamide;
N-(cis-4-{[4-(dimethylamino)-5,6,7,8-tetrahydroquinazolin-2-yl]amino}-cyclohexyl)-3-nitrobenzamide;
N-(cis-4-{[4-(dimethylamino)-5,6,7,8-tetrahydroquinazolin-2-yl]amino}-cyclohexyl)-2-phenoxybutanamide;

N-(cis-4-{[4-(dimethylamino)-5,6,7,8-tetrahydroquinazolin-2-yl]amino}-cyclohexyl)-2-phenoxypropanamide;
N-(cis-4-{[4-(dimethylamino)-5,6,7,8-tetrahydroquinazolin-2-yl]amino}-cyclohexyl)-3-methylbenzamide;
N-(cis-4-[4-(dimethylamino)-5,6,7,8-tetrahydroquinazolin-2-yl]amino}-cyclohexyl)-3-iodobenzamide;
N-(cis-4-){[4-(dimethylamino)-5,6,7,8-tetrahydroquinazolin-2-yl]amino}-cyclohexyl)-2-(4-fluorophenyl)acetamide;
N-(cis-4-{[4-(dimethylamino)-5,6,7,8-tetrahydroquinazolin-2-yl]amino}-cyclohexyl)-2,5-dimethyl-3-furamide;
3-chloro-N-(cis-4-{[4-(dimethylamino)-5,6,7,8-tetrahydroquinazolin-2-yl]amino}-cyclohexyl)-4-fluorobenzamide;
N-(cis-4-{[4-(dimethylamino)-5,6,7,8-tetrahydroquinazolin-2-yl]amino}-cyclohexyl)-3,5-dimethoxybenzamide;
N-(cis-4-{[4-(dimethylamino)-5,6,7,8-tetrahydroquinazolin-2-yl)amino}-cyclohexyl)-3,5-bis(trifluoromethyl)benzamide;
N-(cis-4-{[4-(dimethylamino)-5,6,7,8-tetrahydroquinazolin-2-yl]amino}-cyclohexyl)-4-fluoro-3-methylbenzamide;
2,5-dichloro-N-(cis-4-{[4-(dimethylamino)-5,6,7,8-tetrahydroquinazolin-2-yl]amino}-cyclohexyl)thiophene-3-carboxamide;
5-bromo-N-(cis-4-{[4-(dimethylamino)-5,6,7,8-tetrahydroquinazolin-2-yl]amino}-cyclohexyl)nicotinamide;
2-[(cis-4-{[4-(dimethylamino)-5,6,7,8-tetrahydroquinazolin-2-yl]amino}-cyclohexyl)-amino]-2-oxo-1-phenylethyl acetate;
3-(2-chloro-6-fluorophenyl)-N-(cis-4-{[4-(dimethylamino)-5,6,7,8-tetrahydroquinazolin-2-yl]amino}cyclohexyl)-5-methylisoxazole-4-carboxamide;
N-(cis-4-{[4-(dimethylamino)-5,6,7,8-tetrahydroquinazolin-2-yl]amino}-cyclohexyl)-3-fluorobenzamide;
N-(cis-4-{[4-(dimethylamino)-5,6,7,8-tetrahydroquinazolin-2-yl]amino}-cyclohexyl)-4-fluoro-3-(trifluoromethyl) benzamide;
N-(cis-4-{[4-(dimethylamino)-5,6,7,8-tetrahydroquinazolin-2-yl]amino}-cyclohexyl)-2-(4-methoxyphenoxy)-5-nitrobenzamide;
N-(cis-4-{[4-(dimethylamino)-5,6,7,8-tetrahydroquinazolin-2-yl]amino}-cyclohexyl)-5-nitro-2-furamide;
N-(cis-4-{[4-(dimethylamino)-5,6,7,8-tetrahydroquinazolin-2-yl]amino}-cyclohexyl)-2-phenoxyacetamide;
N-(cis-4-{[4-(dimethylamino)-5,6,7,8-tetrahydroquinazolin-2-yl]amino}-cyclohexyl)-quinoxaline-2-carboxamide;
N-(cis-4-{[4-(dimethylamino)-5,6,7,8-tetrahydroquinazolin-2-yl]amino}-cyclohexyl)-3-(trifluoromethyl)benzamide;
2-(3-chlorophenoxy)-N-(cis-4-{[4-(dimethylamino)-5,6,7,8-tetrahydroquinazolin-2-yl]-amino}cyclohexyl)acetamide;
3-(2,6-dichlorophenyl}-N-(cis-4-{[4-(dimethylamino)-5,6,7,8-tetrahydroquinazolin-2-yl]-amino}cyclohexyl)-5-methylisoxazole-4-carboxamide;
N-(cis-4-{[4-(dimethylamino)-5,6,7,8-tetrahydroquinazolin-2-yl]amino}-cyclohexyl)-2-(4-methylphenoxy)nicotinamide;
2-(2-chloro-4-fluorophenyl)-N-(cis-4-{[4-(dimethylamino)-5,6,7,8-tetrahydroquinazolin-2-yl]amino}cyclohexyl) acetamide;
5-(4-chloro-2-nitrophenyl)-N-(cis-4-{[4-(dimethylamino)-5,6,7,8-tetrahydroquinazolin-2-yl]amino}cyclohexyl)-2-furamide;
5-chloro-N-(cis-4-{[4-(dimethylamino)-5,6,7,8-tetrahydroquinazolin-2-yl]amino}-cyclohexyl)thiophene-2-carboxamide;
N-(cis-4-{[4-(dimethylamino)-5,6,7,8-tetrahydroquinazolin-2-yl]amino}-cyclohexyl)-5-iodo-2-furamide;
2,2-bis(4-chlorophenyl)-N-(cis-4-{[4-(dimethylamino)-5,6,7,8-tetrahydroquinazolin-2-yl]-amino}cyclohexyl)acetamide;
N-(cis-4-{[4-(dimethylamino)-5,6,7,8-tetrahydroquinazolin-2-yl]amino}-cyclohexyl)-5-nitrothiophene-2-carboxamide;
N-(cis-4-{[4-(dimethylamino)-5,6,7,8-tetrahydroquinazolin-2-yl]amino}-cyclohexyl)-3-methyl-4-nitrobenzamide;
N-(cis-4-{[4-(dimethylamino)-5,6,7,8-tetrahydroquinazolin-2-yl]amino}-cyclohexyl)-3-methoxy-4-nitrobenzamide;
3-acetyl-N-(cis-4-{[4-(dimethylamino)-5,6,7,8-tetrahydroquinazolin-2-yl]amino}-cyclohexyl)benzamide;
5-bromo-N-(cis-4-{[4-(dimethylamino)-5,6,7,8-tetrahydroquinazolin-2-yl]amino}-cyclohexyl)-2-furamide;
5-(4-chlorophenyl)-N-(cis-4-{[4-(dimethylamino)-5,6,7,8-tetrahydroquinazolin-2-yl]-amino}cyclohexyl)-2-furamide;
2-(3,4-dichlorophenyl)-N-(cis-4-{[4-(dimethylamino)-5,6,7,8-tetrahydroquinazolin-2-yl]-amino}cyclohexyl)acetamide;
N-(cis-4-{[4-(dimethylamino)-5,6,7,8-tetrahydroquinazolin-2-yl]amino}-cyclohexyl)-2-(4-hydroxy-3,5-dimethoxyphenyl)acetamide;
4,5-dibromo-N-(cis-4-{[4-(dimethylamino)-5,6,7,8-tetrahydroquinazolin-2-yl]amino}-cyclohexyl)-2-furamide;
N-(cis-4-{[4-(dimethylamino)-5,6,7,8-tetrahydroquinazolin-2-yl]amino}-cyclohexyl)-2-(1H-indol-3-yl)acetamide;
N-(cis-4-{[4-(dimethylamino)-5,6,7,8-tetrahydroquinazolin-2-yl]amino}-cyclohexyl)-2-(7-methoxy-2-oxo-2H-chromen-4-yl)acetamide;
N-(cis-4-{[4-(dimethylanuno)-5,6,7,8-tetrahydroquinazolin-2-yl]amino}-cyclohexyl)-3,5-dimethyl-2-[({[4-(trifluoromethoxy)phenyl] amino}carbonyl)amino]-benzamide;
3,5-dichloro-N-(cis-4-{[4-(dimethylamino)-5,6,7,8-tetrahydroquinazolin-2-yl]amino}-cyclohexyl)-2-[(3-phenylprop-2-ynoyl)amino]benzamide;
4-(4-tert-butylphenyl)-N-(cis-4-{[4-(dimethylamino)-5,6,7,8-tetrahydroquinazolin-2-yl]-amino}cyclohexyl)-2-(7-ethyl-1H-indol-3-yl)-4-oxobutanamide;
N-(cis-4-{[4-(dimethylamino)-5,6,7,8-tetrahydroquinazolin-2-yl]amino}-cyclohexyl)-2-methyl-1-(3-morpholin-4-ylpropyl)-5-phenyl-1H-pyrrole-3-carboxamide;
N-(cis-4-{[4-(dimethylamino)-5,6,7,8-tetrahydroquinazolin-2-yl]amino}-cyclohexyl)-4-(4-nitrophenyl)butanamide;
N-(cis-4-{[4-(dimethylamino)-5,6,7,8-tetrahydroquinazolin-2-yl]amino}-cyclohexyl)-2-(2-phenyl-1H-indol-3-yl) acetamide;
$N^2$-benzoyl-$N^5$-(cis-4-{[4-(dimethylamino)-5,6,7,8-tetrahydroquinazolin-2-yl)amino}-cyclohexyl)-$N^1$,$N^1$-dipropylglutamamide;
N-(cis-4-{[4-(dimethylamino)-5,6,7,8-tetrahydroquinazolin-2-yl]amino}-cyclohexyl)-3-phenoxybenzamide;
N'-(cis-4-{[4-(dimethylamino)-5,6,7,8-tetrahydroquinazolin-2-yl]amino}-cyclohexyl)-N,N-bis[(1S)-1-phenylethyl] phthalamide;
N-(cis-4-{[4-(dimethylamino)-5,6,7,8-tetrahydroquinazolin-2-yl]amino}-cyclohexyl)-2-{(1E)-5-fluoro-2-methyl-1-[4-(methylsulfinyl)benzylidene]-1H-inden-3-yl}acetamide;
3-(benzyloxy)-N-(cis-4-{[4-(dimethylamino)-5,6,7,8-tetrahydroquinazolin-2-yl]amino}-cyclohexyl)-4-methoxybenzamide;
N-(cis-4-{[4-(dimethylamino)-5,6,7,8-tetrahydroquinazolin-2-yl]amino}-cyclohexyl)-2-phenoxybenzamide;
N-(cis-4-{[4-(dimethylamino)-5,6,7,8-tetrahydroquinazolin-2-yl]amino}-cyclohexyl)-5-nitrothiophene-3-carboxamide;
N-(cis-4-{[4-(dimethylamino)-5,6,7,8-tetrahydroquinazolin-2-yl]amino}-cyclohexyl)-1-methyl-4-nitro-1H-pyrrole-2-carboxamide;
5-chloro-N-(cis-4-{[4-(dimethylamino)-5,6,7,8-tetrahydroquinazolin-2-yl]amino}-cyclohexyl)-2-hydroxybenzamide;
N-[(cis-4-{[4-(dimethylamino)-5,6,7,8-tetrahydroquinazolin-2-yl]amino}-cyclohexyl)-methyl]-2-(ethylthio)nicotinamide;
N-[(cis-4-{[4-(dimethylamino)-5,6,7,8-tetrahydroquinazolin-2-yl]amino}-cyclohexyl)-methyl]-2-(4-methoxyphenyl) acetamide;
N-[(cis-4-{[4-(dimethylamino)-5,6,7,8-tetrahydroquinazolin-2-yl]amino}-cyclohexyl)-methyl]-5-methyl-2-(trifluoromethyl)-3-furamide;
N-[(cis-4-{[4-(dimethylamino)-5,6,7,8-tetrahydroquinazolin-2-yl]amino}-cyclohexyl)-methyl]-2-(propylthio)nicotinamide; and
2,4,6-trichloro-N-[(cis-4-{[4-(dimethylamino)-5,6,7,8-tetrahydroquinazolin-2-yl]amino}-cyclohexyl)methyl]benzamide;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkoxy carbonyl,
- carbocyclic aryl,
- carbocyclic aryl substituted by substituent(s) independently selected from the group consisting of:
  - halogen,
  - $C_{1-5}$ alkyl,
  - $C_{2-5}$ alkenyl, and
  - $C_{1-5}$ alkoxy,
- $C_{1-5}$ alkylthio, and
- heterocyclyl,

(ii) $C_{3-6}$ cycloalkyl, and
$C_{3-6}$ cycloalkyl substituted by carbocyclic aryl,

(iii) carbocyclyl,
(iv) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by substituent(s) independently selected from the group consisting of:
  - •• halogen,
  - •• oxo, and
  - •• carbocyclic aryl,
- $C_{1-5}$ alkoxy carbonyl,
- $C_{1-7}$ alkoxy,
- $C_{1-7}$ alkoxy substituted by substituent(s) independently selected from the group consisting of:
  - •• halogen, and
  - •• carbocyclic aryl,
- $C_{3-6}$ cycloalkoxy,
- carbocyclic aryloxy,
- mono-$C_{1-5}$ alkylamino,
- di-$C_{1-5}$ alkylamino,
- $C_{1-5}$ alkylthio,
- $C_{1-5}$ alkylthio substituted by halogen, and
- carbocyclic aryl,

(v) heterocyclyl, and
heterocyclyl substituted by substituent(s) independently selected from the group consisting of:

- halogen,
- $C_{1-5}$ alkyl,
- $C_{1-5}$ alkyl substituted by halogen,
- $C_{1-5}$ alkoxy carbonyl
- $C_{1-5}$ alkoxy carbonyl substituted by carbocyclic aryl, and
- carbocyclic aryl;

L is Formula (VII);
Y is -C(O)$NR_5$-;
wherein carbocyclic aryl is phenyl or naphthyl;
carbocyclyl is indanyl, adamantly, or 9*H*-fluorenyl;
heterocyclyl is 2,3-dihydro-benzo[1,4]dioxinyl, 3,4-dihydro-2*H*-benzo[b][1,4]dioxepinyl, 4*H*-benzo[1,3]dioxinyl, benzo[1,3]dioxolyl, furyl, isoxazolyl, piperidyl, pyridyl, or thienyl;
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are hydrogen; A is a single bond; B is a single bond or -$CH_2$- $R_5$ is hydrogen; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- $C_{1-5}$ alkoxy carbonyl,
- carbocyclic aryl, and

- carbocyclic aryl substituted by halogen,

(ii) carbocyclic aryl, and
carbocyclic aryl substituted by substituent(s) independently selected from the group consisting of:

- halogen,
- nitro,
- $C_{1-5}$ alkyl,
- $C_{1-5}$ alkyl substituted by halogen,
- $C_{1-5}$ alkoxy, and
- $C_{1-5}$ alkoxy substituted by halogen,

(iii) heterocyclyl, and
heterocyclyl substituted by $C_{1-5}$ alkyl, and
heterocyclyl substituted by carbocyclic aryl;
wherein carbocyclic aryl is phenyl or naphthyl;
heterocyclyl is isoxazolyl;
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(cis-4-{[4-(dimethylamino)-5,6,7,8-tetrahydroquinazolin-2-yl]amino}-cyclohexyl)-N'-(2-ethyl-6-methylphenyl) urea;
N-(cis-4-{[4-(dimethylamino)-5,6,7,8-tetrahydroquinazolin-2-yl]amino}-cyclohexyl)-N'-(4-fluorophenyl)urea;
N-(cis-4-{[4-(dimethylamino)-5,6,7,8-tetrahydroquinazolin-2-yl]amino}-cyclohexyl)-N'-mesitylurea;
N-(cis-4-{[4-(dimethylamino)-5,6,7,8-tetrahydroquinazolin-2-yl]amino}-cyclohexyl)-N'-(2,4,6-trichlorophenyl)urea;
N-(cis-4-{[4-(dimethylamino)-5,6,7,8-tetrahydroquinazolin-2-yl]amino}-cyclohexyl)-N'-(2,4,6-tribromophenyl)urea;
N-(2,4-dibromo-6-fluorophenyl)-N'-(cis-4-{[4-(dimethylamino)-5,6,7,8-tetrahydroquinazolin-2-yl]amino}cyclohexyl)urea;
N-(2,6-diethylphenyl)-N'-(cis-4-{[4-(dimethylamino)-5,6,7,8-tetrahydroquinazolin-2-yl]-amino}cyclohexyl)urea;
N-(2-chlorobenzyl)-N'-(cis-4-{[4-(dimethylamino)-5,6,7,8-tetrahydroquinazolin-2-yl]-amino}cyclohexyl)urea;
N-(cis-4-{[4-(dimethylamino)-5,6,7,8-tetrahydroquinazolin-2-yl]amino}-cyclohexyl)-N'-(2-ethyl-6-isopropylphenyl) urea;
N-(cis-4-{[4-(dimethylamino)-5,6,7,8-tetrahydroquinazolin-2-yl]amino}-cyclohexyl)-N'-(2-ethylphenyl)urea;
N-(cis-4-{[4-(dimethylamino)-5,6,7,8-tetrahydroquinazolin-2-yl]amino}-cyclohexyl)-N'-(2-isopropyl-6-methylphenyl)urea;
N-(2-tert-butyl-6-methylphenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(diphenylmethyl)urea;
N-(4-bromo-2,6-dimethylphenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(3-methyl-5-phenylisoxazol-4-yl)urea;
N-(cis-4-{[4-(dimethylamino)-5,6,7,8-tetrahydroquinazolin-2-yl]amino}-cyclohexyl)-N'-1-naphthylurea;
N-(cis-4-{[4-(dimethylamino)-5,6,7,8-tetrahydroquinazolin-2-yl]amino}-cyclohexyl)-N'-[1-(1-naphthyl)ethyl]urea;
N-(2,4-dibromophenyl)-N'-(cis-4-{[4-(dimethylamino)-5,6,7,8-tetrahydroquinazolin-2-yl]-amino}cyclohexyl)urea;
N-(2,4-dichlorobenzyl)-N'-(cis-4-{[4-(dimethylamino)-5,6,7,8-tetrahydroquinazolin-2-yl]-amino}cyclohexyl)urea;
N-(2,4-dimethoxyphenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(2-ethoxyphenyl)urea;
N-(cis-4-{[4-(dimethylamino)-5,6,7,8-tetrahydroquinazolin-2-yl]amino}-cyclohexyl)-N'-(2-fluorobenzyl)urea;
N-(cis-4-{[4-(dimethylamino)-5,6,7,8-tetrahydroquinazolin-2-yl]amino}-cyclohexyl)-N'-(3,4,5-trimethoxyphenyl) urea;
N-(3,4-dimethoxyphenyl)-N'-(cis-4-{[4-(dimethylamino)-5,6,7,8-tetrahydroquinazolin-2-yl]amino}cyclohexyl)urea;
N-(4-chloro-2-methylphenyl)-N'-(cis-4-{[4-(dimethylamino)-5,6,7,8-tetrahydroquinazolin-2-yl]amino}cyclohexyl) urea;
N-(cis-4-{[4-(dimethylamino)-5,6,7,8-tetrahydroquinazolin-2-yl]amino}-cyclohexyl)-N'-(4-fluorobenzyl)urea;

N-(cis-4-{[4-(dimethylamino)-5,6,7,8-tetrahydroquinazolin-2-yl]amino}-cyclohexyl)-N'-(4-methoxy-2-methylphenyl)urea;
N-(5-chloro-2,4-dimethoxyphenyl)-N'-(cis-4-{[4-(dimethylamino)-5,6,7,8-tetrahydroquinazolin-2-yl]amino}cyclohexyl)urea;
N-[1-(4-bromophenyl)ethyl]-N'-(cis-4-{[4-(dimethylamino)-5,6,7,8-tetrahydroquinazolin-2-yl]amino}cyclohexyl) urea;
N-(4-bromo-2-methylphenyl}-N'-(cis-4-{[4-(dimethylamino)-5,6,7,8-tetrahydroquinazolin-2-yl]amino}cyclohexyl) urea;
N-(cis-4-{[4-(dimethylamino)-5,6,7,8-tetrahydroquinazolin-2-yl]amino}-cyclohexyl)-N'-(5-methyl-3-phenylisoxazol-4-yl)urea;
N-(2,3-dichlorophenyl)N'-(cis-4-{[4-(dimethylamino)-5,6,7,8-tetrahydroquinazolin-2-yl]-amino}cyclohexyl)urea;
N-(cis-4-{[4-(dimethylamino)-5,6,7,8-tetrahydroquinazolin-2-yl]amino}-cyclohexyl)-N'-(4-methylphenyl)urea;
N-(2,6-diisopropylphenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(2,4,5-trichlorophenyl)urea;
N-(2,5-dimethoxyphenyl)-N'-(cis-4-{[4-(dimethylamino)-5,6,7,8-tetrahydroquinazolin-2-yl]amino}cyclohexyl)urea;
N-(4-bromo-2-chlorophenyl)-N'-(cis-4-{[4-(dimethylamino)-5,6,7,8-tetrahydroquinazolin-2-yl]amino}cyclohexyl) urea;
N-(cis-4-{[4-(dimethylamino)-5,6,7,8-tetrahydroquinazolin-2-yl]amino}cyclohexyl)-N'-[2-(trifluoromethoxy)phenyl] urea;
N-[(cis-4-{[4-(dimethylanuno)-5,6,7,8-tetrahydroquinazolin-2-yl]amino}-cyclohexyl)-methyl]-N'-(2,6-dimethylphenyl)urea;
N-(2,4-difluorophenyl)-N'-[(cis-4-{[4-(dimethylamino)-5,6,7,8-tetrahydroquinazolin-2-yl]-amino}cyclohexyl)methyl] urea;
N-[(cis-4-{[4-(dimethylamino)-5,6,7,8-tetrahydroquinazolin-2-yl]amino}-cyclohexyl)-methyl]-N'-(2-ethyl-6-methylphenyl)urea;
ethyl N-({[(cis-4-{[4-(dimethylamino)-5,6,7,8-tetrahydroquinazolin-2-yl]-amino}-cyclohexyl)methyl]amino}carbonyl)leucinate;
N-[(cis-4-{[4-(dimethylamino)-5,6,7,8-tetrahydroquinazolin-2-yl]amino}-cyclohexyl)-methyl]-N'-(4-fluorophenyl) urea;
N-[(cis-4-{[4-(dimethylamino)-5,6,7,8-tetrahydroquinazolin-2-yl]amino}-cyclohexyl)-methyl]-N'-mesitylurea;
N-[(cis-4-{[4-(dimethylamino)-5,6,7,8-tetrahydroquinazolin-2-yl]amino}-cyclohexyl)-methyl]-N'-(2,4,6-trichlorophenyl)urea;
N-[(cis-4-{[4-(dimethylamino)-5,6,7,8-tetrahydroquinazolin-2-yl]amino}-cyclohexyl)-methyl]-N'-(2,4,6-tribromophenyl)urea;
N-(2,6-diethylphenyl)-N'-[(cis-4-{[4-(dimethylamino)-5,6,7,8-tetrahydroquinazolin-2-yl]-amino}cyclohexyl)methyl] urea;
N-[2-chloro-6-(trifluoromethyl)phenyl]-N'-[(cis-4-{[4-(dimethylamino)-5,6,7,8-tetrahydroquinazolin-2-yl]amino}cyclohexyl)methyl]urea;
N-(2-chloro-6-methylphenyl)-N'-[(cis-4-{[4-(dimethylamino)-5,6,7,8-tetrahydroquinazolin-2-yl]amino}cyclohexyl) methyl]urea;
N-[(cis-4-{[4-(dimethylamino)-5,6,7,8-tetrahydroquinazolin-2-yl]amino}-cyclohexyl)-methyl]-N'-(2-ethyl-6-isopropylphenyl)urea;
N-[(cis-4-{[4-(dimethylamino)-5,6,7,8-tetrahydroquinazolin-2-yl]amino}-cyclohexyl)-methyl]-N'-(2-isopropyl-6-methylphenyl)urea;
N-(2-tert-butyl-6-methylphenyl)-N'-[(cis-4-{[4-(dimethylamino)-5,6,7,8-tetrahydroquinazolin-2-yl]amino}cyclohexyl)methyl]urea;
N-(2-tert-butylphenyl)-N'-[(cis-4-{[4-(dimethylamino)-5,6,7,8-tetrahydroquinazolin-2-yl]-amino}cyclohexyl)methyl] urea;
N-(3-chloro-2-methylphenyl)-N'-[(cis-4-{[4-(dimethylamino)-5,6,7,8-tetrahydroquinazolin-2-yl]amino}cyclohexyl) methyl]urea;
N-(4-bromo-2,6-dimethylphenyl)-N'-[(cis-4-{[4-(dimethylamino)-5,6,7,8-tetrahydroquinazolin-2-yl]amino}cyclohexyl)methyl]urea;
N-(2,6-diisopropylphenyl)-N'-[(cis-4-{[4-(dimethylamino)-5,6,7,8-tetrahydroquinazolin-2-yl]amino}cyclohexyl)methyl]urea;
N-[(cis-4-{[4-(dimethylamino)-5,6,7,8-tetrahydroquinazolin-2-yl]amino}-cyclohexyl)-methyl]-N'-(2,3-dimethyl-6-nitrophenyl)urea;
N-(2,6-dibromo-4-fluorophenyl)-N'-[(cis-4-{[4-(dimethylamino)-5,6,7,8-tetrahydroquinazolin-2-yl]amino}cyclohexyl)methyl]urea;

N-(2,6-dichlorophenyl)-N'-[(cis-4-{[4-(dimethylamino)-5,6,7,8-tetrahydroquinazolin-2-yl]-amino}cyclohexyl)methyl]urea; and
1-(2,3-dichloro-phenyl)-3-[cis-4-(4-dimethylamino-5,6,7,8-tetrahydro-quinazolin-2-ylamino)-cyclohexylmethyl]-urea;

or a pharmaceutically acceptable salt, hydrate, or solvate thereof.

In some embodiments of the present invention, $R_1$ is selected from the group consisting of:

(i) $C_{1-8}$ alkyl, and
$C_{1-8}$ alkyl substituted by substituent(s) independently selected from the group consisting of:

- mono-$C_{1-5}$ alkylamino,
- di-$C_{1-5}$ alkylamino,
- $C_{3-6}$ cycloalkyl,
- $C_{3-6}$ cycloalkenyl,
- carbocyclic aryl,
- carbocyclic aryl substituted by substituent(s) independently selected from the group consisting of:
  - halogen,
  - $C_{1-5}$ alkyl, and
  - $C_{1-5}$ alkoxy,
- heterocyclyl,

(ii) $C_{2-5}$ alkynyl,
(iii) $C_{2-5}$ alkenyl,
(iv) $C_{3-12}$ cycloalkyl,
(v) carbocyclyl,
(vi) carbocyclic aryl, and
carbocyclic aryl substituted by substituent(s) independently selected from the group consisting of:

- halogen,
- cyano,
- nitro,
- $C_{1-10}$. alkyl,
- $C_{1-10}$ alkyl substituted by substituent(s) independently selected from the group consisting of:
  - halogen, and
  - oxo,
- carboxy,
- $C_{1-5}$ alkoxy carbonyl,
- $C_{1-5}$ alkoxy,
- $C_{1-5}$ alkoxy substituted by substituent(s) independently selected from the group consisting of:
  - halogen, and
  - carbocyclic aryl,
- carbocyclic aryloxy,
- carbocyclic aryloxy substituted by nitro,
- mono-$C_{1-5}$ alkylamino,
- di-$C_{1-5}$ alkylamino,
- $C_{1-5}$ alkoxy carbonylamino,
- carbocyclic aryl azo,
- carbocyclic aryl azo substituted by substituent(s) independently selected from the group consisting of:
  - mono-$C_{1-5}$ alkylamino, and
  - di-$C_{1-5}$ alkylamino,

- $C_{1-5}$ alkylthio,
- $C_{1-5}$ alkylthio substituted by halogen,
- carbocyclic arylthio,
- carbocyclic arylthio substituted by nitro,
- amino sulfonyl,
- heterocyclyl sulfonyl,
- $C_{3-6}$ cycloalkyl,
- $C_{3-6}$ cycloalkyl substituted by $C_{1-5}$ alkyl,
- carbocyclic aryl, and
- heterocyclyl,

(vii) heterocyclyl, and
heterocyclyl substituted by substituent(s) independently selected from the group consisting of:

- $C_{1-5}$ alkyl,
- $C_{1-5}$ alkoxy carbonyl,
- carbocyclic aryloxy,
- carbocyclic aryl, and
- heterocyclyl;

L is Formula (VII);
Y is -C(S)$NR_5$-;
wherein carbocyclic aryl is phenyl or naphthyl;
carbocyclyl is indanyl, bicyclo[2.2.1]heptyl, bicyclo[2.2.1]heptenyl, or adamantly;
heterocyclyl is 2,3-dihydro-benzo[1,4]dioxinyl, 4,5,6,7-tetrahydro-benzo[b]thienyl, benzo[1,3]dioxolyl, benzo[2,1,3]thiadiazolyl, furyl, isoxazolyl, morpholinyl, oxazolyl, phenanthro[9,10-d]oxazolyl, piperidyl, pyrazolyl, pyridyl, tetrahydrofuryl, or thienyl; and
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are hydrogen; A is a single bond; B is a single bond or -$CH_2$-: $R_5$ is hydrogen; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by carbocyclic aryl,
(ii) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen,
- $C_{1-5}$ alkoxy,
- $C_{1-5}$ alkoxy substituted by halogen,
- mono-$C_{1-5}$ alkylamino, and
- di-$C_{1-5}$ alkylamino;

wherein carbocyclic aryl is phenyl or naphthyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(2,4-dimethoxyphenyl)-N'-(cis-4-{[4-(dimethylamino)-5,6,7,8-tetrahydroquinazolin-2-yl]amino}cyclohexyl)thiourea;
N-(cis-4-{[4-(dimethylamino)-5,6,7,8-tetrahydroquinazolin-2-yl]amino}-cyclohexyl)-N'-(3,4,5-trimethoxyphenyl)thiourea;
N-(3,4-dimethoxyphenyl)-N'-(cis-4-{[4-(dimethylamino)-5,6,7,8-tetrahydroquinazolin-2-yl]amino}cyclohexyl)thiourea;
N-[4-(dimethylamino)-1-naphthyl]-N'-(cis-4-{[4-(dimethylamino)-5,6,7,8-tetrahydroquinazolin-2-yl]amino}cyclohexyl)thiourea;
N-(cis-4-{[4-(dimethylamino)-5,6,7,8-tetrahydroquinazolin-2-yl]amino}-cyclohexyl)-N'-(2-methoxy-5-methylphenyl)thiourea;
N-(4-bromo-2-chlorophenyl)-N'-(cis-4-{[4-(dimethylamino)-5,6,7,8-tetrahydroquinazolin-2-yl]amino}cyclohexyl) thiourea;
N-(cis-4-{[4-(dimethylamino)-5,6,7,8-tetrahydroquinazolin-2-yl]amino}-cyclohexyl)-N'-(4-iodophenyl)thiourea;
N-(cis-4-{[4-(dimethylamino)-5,6,7,8-tetrahydroquinazolin-2-yl]amino}-cyclohexyl)-N'-(2,4,6-tribromophenyl) thiourea;
N-(cis-4-{[4-(dimethylamino)-5,6,7,8-tetrahydroquinazolin-2-yl]amino}-cyclohexyl)-N'-(2,4,6-trichlorophenyl) thiourea;
N-(cis-4-{[4-(dimethylamino)-5,6,7,8-tetrahydroquinazolin-2-yl]amino}-cyclohexyl)-N'-mesitylthiourea;
N-(cis-4-{[4-(dimethylamino)-5,6,7,8-tetrahydroquinazolin-2-yl]amino}-cyclohexyl)-N'-(2,4-dimethylphenyl)thiourea;
N-(2,6-diethylphenyl)-N'-(cis-4-{[4-(dimethylamino)-5,6,7,8-tetrahydroquinazolin-2-yl]-amino}cyclohexyl)thiourea;
N-(4-bromo-2,6-dimethylphenyl)-N'-(cis-4-{[4-(dimethylamino)-5,6,7,8-tetrahydroquinazolin-2-yl]amino}cyclohexyl)thiourea;
N-(4-bromo-2-methylphenyl)-N'-(cis-4-{[4-(dimethylamino)-5,6,7,8-tetrahydroquinazolin-2-yl]amino}cyclohexyl) thiourea;
N-[4-bromo-2-(trifluoromethyl)phenyl]-N'-(cis-4-{[4-(dimethylamino)-5,6,7,8-tetrahydroquinazolin-2-yl]amino}cyclohexyl)thiourea;
N-(4-chloro-2-methylphenyl)-N'-(cis-4-{[4-(dimethylamino)-5,6,7,8-tetrahydroquinazolin-2-yl]amino}cyclohexyl) thiourea;
N-[4-chloro-2-(trifluoromethyl)phenyl]-N'-(cis-4-{[4-(dimethylamino)-5,6,7,8-tetrahydroquinazolin-2-yl]amino}cyclohexyl)thiourea;
N-(cis-4-{[4-(dimethylamino)-5,6,7,8-tetrahydroquinazolin-2-yl]amino}-cyclohexyl)-N'-(4-fluoro-2-methylphenyl) thiourea;
N-(cis-4-{[4-(dimethylamino)-5,6,7,8-tetrahydroquinazolin-2-yl]amino}-cyclohexyl)-N'-(4-methoxy-2-methylphenyl)thiourea;
N-(5-chloro-2,4-dimethoxyphenyl)-N'-(cis-4-{[4-(dimethylamino)-5,6,7,8-tetrahydroquinazolin-2-yl]amino}cyclohexyl)thiourea;
N-(2,4-dibromo-6-fluorophenyl)-N'-(cis-4-{[4-(dimethylamino)-5,6,7,8-tetrahydroquinazolin-2-yl]amino}cyclohexyl)thiourea;
N-(2,4-dichloro-6-methylphenyl)-N'-(cis-4-{[4-(dimethylamino)-5,6,7,8-tetrahydroquinazolin-2-yl]amino}cyclohexyl)thiourea;
N-(cis-4-{[4-(dimethylamino)-5,6,7,8-tetrahydroquinazolin-2-yl]amino}-cyclohexyl)-N'-(2-ethoxyphenyl)thiourea;
N-[4-bromo-2-(trifluoromethoxy)phenyl]-N'-(cis-4-{[(4-(dimethylamino)-5,6,7,8-tetrahydroquinazolin-2-yl]amino} cyclohexyl)thiourea;
N-(4-chloro-2,5-dimethoxyphenyl)-N'-(cis-4-{[4-(dimethylamino)-5,6,7,8-tetrahydroquinazolin-2-yl]amino}cyclohexyl)thiourea; and
N-(cis-4-{[4-(dimethylamino)-5,6,7,8-tetrahydroquinazolin-2-yl]amino}-cyclohexyl)-N'-(2,2-diphenylethyl)thiourea;

or a pharmaceutically acceptable salt, hydrate, or solvate thereof.

In some embodiments of the present invention, $R_1$ is selected from the group consisting of:

(i) $C_{1-8}$ alkyl, and
$C_{1-8}$ alkyl substituted by substituent(s) independently selected from the group consisting of:

- halogen,
- $C_{1-5}$ alkoxy,
- $C_{1-5}$ alkoxy substituted by carbocyclic aryl,
- carbocyclyl,
- carbocyclic aryl,
- carbocyclic aryl substituted by substituent(s) independently selected from the group consisting of:

- •• halogen,
- •• nitro, and
- •• $C_{1-5}$ alkoxy,

(ii) $C_{2-5}$ alkenyl,
(iii) carbocyclyl,
(iv) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen, and
- $C_{1-5}$ alkoxy;

L is Formula (VII);
Y is -C(O)O-;
wherein carbocyclic aryl is phenyl or naphthyl;
carbocyclyl is 9*H*-fluorenyl or menthyl; and
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are hydrogen; A is a single bond; B is a single bond or -$CH_2$-; or a pharmaceutically acceptable salt, hydrate, or solvate thereof.

In some embodiments of the present invention, Q is Formula (IV); p is 0;
$R_1$ is selected from the group consisting of:

(i) $C_{1-8}$ alkyl, and
$C_{1-8}$ alkyl substituted by substituent(s) independently selected from the group consisting of:

- halogen,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- carbocyclic aryloxy substituted by $C_{1-5}$ alkoxy,
- heterocyclyloxy,
- heterocyelyloxy substituted by $C_{1-5}$ alkyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by cyano,
- mono-$C_{1-5}$ alkylamino substituted by carbocyclic aryl,
- di-$C_{1-5}$ alkylamino,
- di-$C_{1-5}$ alkylamino substituted by cyano,
- di-$C_{1-5}$ alkylamino substituted by carbocyclic aryl,
- mono-carbocyclic arylamino,
- mono-carbocyclic arylamino substituted by $C_{1-5}$ alkyl,
- di-carbocyclic arylamino,
- di-carbocyclic arylamino substituted by $C_{1-5}$ alkyl,
- $C_{1-5}$ alkoxycarbonylamino,
- carbocyclic arylcarbonylamino,
- $C_{1-5}$ alkylthio,
- $C_{1-5}$ alkylthio substituted by substituent(s) independently selected from the group consisting of:

    - •• carbocyclic aryl,
    - •• carbocyclic aryl substituted by halogen, and
    - •• carbocyclic aryl substituted by $C_{1-5}$ alkoxy,

- • carbocyclic arylthio,
- • heterocyclylthio,
- • heterocyclylthio substituted by nitro,
- • heterocyclylthio substituted by $C_{1-5}$ alkyl,
- • $C_{3-6}$ cycloalkyl,
- • $C_{3-6}$ cycloalkenyl,
- • carbocyclyl,
- • carbocyclyl substituted by substituent(s) independently selected from the group consisting of:

    - •• halogen,
    - •• $C_{1-5}$ alkyl,
    - •• $C_{1-5}$ alkoxy,
    - •• $C_{2-5}$ alkenyl, and
    - •• $C_{2-5}$ alkenyl substituted by substituent(s) independently selected from the group consisting of:

        - ••• carbocyclic aryl, and
        - ••• carbocyclic aryl substituted by $C_{1-5}$ alkylsulfinyl,

- • carbocyclic aryl,
- • carbocyclic aryl substituted by substituent(s) independently selected from the group consisting of:

    - •• halogen,
    - •• hydroxy,
    - •• nitro,
    - •• $C_{1-5}$ alkyl,
    - •• $C_{1-5}$ alkyl substituted by substituent(s) independently selected from the group consisting of:

        - ••• oxo,
        - ••• carbocyclic aryl, and
        - ••• heterocyclyl,

    - •• $C_{2-5}$ alkenyl,
    - •• $C_{2-5}$ alkoxy,
    - •• $C_{1-5}$ alkoxy substituted by halogen,
    - •• $C_{1-5}$ alkoxy substituted by carbocyclic aryl,
    - •• carbocyclic aryloxy,
    - •• carbocyclic aryl, and
    - •• heterocyclyl,

- • heterocyclyl, and
- • heterocyclyl substituted by substituent(s) independently selected from the group consisting of:

    - •• $C_{1-5}$ alkyl,
    - •• $C_{1-5}$ alkyl substituted by carbocyclic aryl,
    - •• $C_{1-5}$ alkoxy,
    - •• $C_{1-5}$ alkoxy substituted by carbocyclic aryl,
    - •• carbocyclic aryl, and
    - •• carbocyclic aryl substituted by halogen,

(ii) $C_{2-7}$ alkenyl, and
$C_{2-7}$ alkenyl substituted by substituent(s) independently selected from the group consisting of:

- • carbocyclic aryl,
- • carbocyclic aryl substituted by substituent(s) independently selected from the group consisting of:

- - halogen,
  - nitro, and
  - $C_{1-5}$ alkoxy,

(iii) $C_{2-5}$ alkynyl, and
$C_{2-5}$ alkynyl substituted by carbocyclic aryl,
(iv) $C_{3-6}$ cycloalkyl, and
$C_{3-6}$ cycloalkyl substituted by substituent(s) independently selected from the group consisting of:

- $C_{1-5}$ alkyl,
- $C_{1-5}$ alkyl substituted by oxo,
- $C_{1-5}$ alkyl substituted by carbocyclic aryl, and
- carbocyclic aryl,

(v) carbocyclyl,
(vi) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:
  - halogen,
  - oxo,
  - carbocyclic aryloxy,
  - carbocyclic aryl, and
  - carbocyclic aryl substituted by $C_{1-5}$ alkyl,
- $C_{1-5}$ alkoxy,
- $C_{1-5}$ alkoxy substituted by substituent(s) independently selected from the group consisting of:
  - halogen,
  - carbocyclic aryl, and
  - halogenated carbocyclic aryl,
- $C_{2-5}$ alkenyloxy,
- $C_{3-6}$ cycloalkoxy,
- carbocyclic aryloxy,
- carbocyclic aryloxy substituted by $C_{1-5}$ alkoxy,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,

- $C_{1-5}$ alkylthio substituted by halogen,
- carbocyclic arylthio,
- carbocyclic arylthio substituted by cyano,
- mono-$C_{1-5}$ alkylaminosulfonyl,
- di-$C_{1-5}$ alkylaminosulfonyl,
- carbocyclic aryl,
- heterocyclyl, and
- heterocyclyl substituted by substituent(s) independently selected from the group consisting of:

    - •• $C_{1-5}$ alkyl,
    - •• carbocyclic aryl, and
    - •• halogenated carbocyclic aryl,

(vii) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:

    - •• halogen,
    - •• hydroxy,
    - •• $C_{1-5}$ alkylthio,
    - •• $C_{1-5}$ alkylthio substituted by carbocyclic aryl,
    - •• $C_{1-5}$ alkylthio substituted by halogenated carbocyclic aryl,
    - •• carbocyclic aryl,
    - •• carbocyclic aryl substituted by halogen, and
    - •• heterocyclyl,

- $C_{1-5}$ alkoxy,
- carbocyclic aryloxy,
- carbocyclic aryloxy substituted by $C_{1-5}$ alkyl,
- $C_{1-5}$ alkylthio,
- $C_{2-5}$ alkenylthio,
- carbocyclic arylthio,
- carbocyclic arylthio substituted by $C_{1-5}$ alkoxycarbonyl,
- $C_{1-5}$ alkylsulfonyl,
- carbocyclic arylsulfonyl,
- carbocyclic arylsulfonyl substituted by $C_{1-5}$ alkyl,
- $C_{1-5}$ alkoxycarbonyl,
- carbocyclic aryl,
- carbocyclic aryl substituted by substituent(s) independently selected from the group consisting of:

    - •• halogen,
    - •• nitro,
    - •• $C_{1-5}$ alkyl, and
    - •• $C_{1-5}$ alkyl substituted by halogen,

- heterocyclyl;

wherein carbocyclic aryl is phenyl, naphthyl, or anthranyl;

carbocyclyl is 1,2,3,4-tetrahydronaphthyl, 1-oxo-indanyl, 9-fluorenyl, 9-oxo-9*H*-fluorenyl, bicyclo[2.2.1]heptyl, indenyl, or menthyl;

heterocyclyl is 1,2,3-triazolyl, 1*H*-indolyl, 1*H*-pyrrolyl, 2,3-dihydro-1-oxo-isoindolyl, 2,3-dihydro-benzo[1,4]dioxinyl, 2,3-dihydro-benzofuryl, 2,4-dihydro-3-oxo-pyrazolyl, 2*H*-benzopyranyl, 2-oxo-benzopyranyl, 3,4-dihydro-2*H*-benzo[b][1,4]dioxepinyl, 4-oxo-1,5,6,7-tetrahydro-indolyl, 4-oxo-benzopyranyl, 9*H*-carbazolyl, 9*H*-xanthenyl, benzo[1,3]dioxolyl, benzo[2,1,3]oxadiazolyl, benzo[1,2,5]oxadiazolyl, benzo[b]thienyl, benzofuryl, benzothiazolyl,

furyl, imidazo[2,1-b]thiazolyl, imidazolyl, isoxazolyl, morpholino, pyrazolyl, pyridyl, pyrimidyl, quinolyl, quinoxalyl, thiazolyl, or thienyl; and
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-7}$ alkyl, and
$C_{1-7}$ alkyl substituted by substituent(s) independently selected from the group consisting of:

- $C_{1-5}$ alkoxy,
- $C_{1-5}$ alkoxy substituted by carbocyclic aryl,
- carbocyclic aryloxy,
- carbocyclic aryloxy substituted by halogen,
- mono-$C_{1-5}$ alkylamino,
- mono-$C_{1-5}$ alkylamino substituted by substituent(s) independently selected from the group consisting of:
    - •• cyano, and
    - •• carbocyclic aryl,
- di-$C_{1-5}$ alkylamino,
- di-$C_{1-5}$ alkylamino substituted by substituent(s) independently selected from the group consisting of:
    - •• cyano, and
    - •• carbocyclic aryl,
- mono-carbocyclic arylamino,
- di-carbocyclic arylamino,
- mono-carbocyclic arylamino substituted by $C_{1-5}$ alkyl,
- di-carbocyclic arylamino substituted by $C_{1-5}$ alkyl,
- carbocyclic aryl,
- carbocyclic aryl substituted by substituent(s) independently selected from the group consisting of:
    - •• halogen,
    - •• $C_{1-5}$ alkyl, and
    - •• $C_{1-5}$ alkoxy,

(ii) $C_{2-7}$ alkenyl, and
$C_{2-7}$ alkenyl substituted by substituent(s) independently selected from the group consisting of:

- carbocyclic aryl, and
- carbocyclic aryl substituted by $C_{1-5}$ alkoxy,

(iii) $C_{2-5}$ alkynyl, and
$C_{2-5}$ alkynyl substituted by carbocyclic aryl,
(iv) carbocyclic aryl, and
carbocyclic aryl substituted by substituent(s) independently selected from the group consisting of:

- halogen,
- hydroxy,
- cyano,
- $C_{1-5}$ alkyl,
- $C_{1-5}$ alkyl substituted by halogen,
- $C_{1-5}$ alkoxy,
- $C_{1-5}$ alkoxy substituted by substituent(s) independently selected from the group consisting of:
    - •• halogen,
    - •• carbocyclic aryl, and

  - •• carbocyclic aryl substituted by halogen,

- $C_{2-5}$ alkenyloxy,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{1-5}$ alkylthio, and
- $C_{1-5}$ alkylthio substituted by halogen,

(v) heterocyclyl, and
heterocyclyl substituted by substituent(s) independently selected from the group consisting of:

- halogen,
- $C_{1-5}$ alkyl,
- $C_{1-5}$ alkyl substituted by hydroxy,
- $C_{1-5}$ alkoxy,
- carbocyclic arylthio,
- carbocyclic arylthio substituted by $C_{1-5}$ alkoxycarbonyl,
- $C_{1-5}$ alkoxycarbonyl,
- carbocyclic aryl,
- carbocyclic aryl substituted by substituent(s) independently selected from the group consisting of:

  - •• halogen,
  - •• $C_{1-5}$ alkyl, and
  - •• $C_{1-5}$ alkyl substituted by halogen;

L is Formula (VII);
Y is a single bond or $-CH_2-$;
wherein carbocyclic aryl is phenyl or naphthyl;
heterocyclyl is 1*H*-indolyl, 1*H*-pyrrolyl, 2,3-dihydro-benzo[1,4]dioxinyl, 4-oxo-benzopyranyl, 9*H*-carbazolyl, benzo[1,3]dioxolyl, benzo[b]thienyl, furyl, imidazo[2,1-b]thiazolyl, pyrazolyl, pyridyl, or thienyl; and
halogen is fluoro, chloro, bromo, or iodo;

or a pharmaceutically acceptable salt, hydrate, or solvate thereof.

In some embodiments of the present invention, $R_2$ is methylamino, or dimethylamino; p is 0; $R_3$ and $R_4$ are hydrogen; A is a single bond; B is a single bond or $-CH_2-$; or a pharmaceutically acceptable salt, hydrate, or solvate thereof.

In some embodiments of the present invention, $R_1$ is selected from the group consisting of:

(i) $C_{2-5}$ alkenyl, and
$C_{2-5}$ alkenyl substituted by carbocyclic aryl,
(ii) carbocyclic aryl, and
carbocyclic aryl substituted by substituent(s) independently selected from the group consisting of:

- halogen,
- hydroxy,
- $C_{1-5}$ alkyl,
- $C_{1-5}$ alkoxy,
- $C_{1-5}$ alkoxy substituted by substituent(s) independently selected from the group consisting of:

  - •• halogen,
  - •• carbocyclic aryl, and
  - •• carbocyclic aryl substituted by halogen,

- $C_{2-5}$ alkenyloxy,
- mono-$C_{1-5}$ alkylamino,
- di-$C_{1-5}$ alkylamino,

- mono-$C_{1-5}$ alkylamino substituted by cyano, and
- di-$C_{1-5}$ alkylamino substituted by cyano,

(iii) heterocyclyl, and
heterocyclyl substituted by substituent(s) independently selected from the group consisting of:

- halogen,
- $C_{1-5}$ alkyl,
- $C_{1-5}$ alkoxy,
- $C_{1-5}$ alkoxycarbonyl,
- carbocyclic aryl,
- carbocyclic aryl substituted by substituent(s) independently selected from the group consisting of:
  - •• halogen,
  - •• $C_{1-5}$ alkyl, and
  - •• $C_{1-5}$ alkyl substituted by halogen;

wherein carbocyclic aryl is phenyl or naphthyl;
heterocyclyl is 1*H*-indolyl, 9*H*-carbazolyl, benzo[1,3]dioxolyl, pyrazolyl, or pyridyl; and
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{2-5}$ alkenyl, and
$C_{2-5}$ alkenyl substituted by carbocyclic aryl,
(ii) carbocyclic aryl, and
carbocyclic aryl substituted by substituent(s) independently selected from the group consisting of:

- halogen,
- hydroxy,
- $C_{1-5}$ alkyl,
- $C_{1-5}$ alkoxy,
- $C_{1-5}$ alkoxy substituted by halogen,
- $C_{2-5}$ alkenyloxy,

(iii) heterocyclyl, and
heterocyclyl substituted by substituent(s) independently selected from the group consisting of:

- halogen,
- $C_{1-5}$ alkyl,
- $C_{1-5}$ alkoxy,
- $C_{1-5}$ alkoxycarbonyl,
- carbocyclic aryl,
- carbocyclic aryl substituted by $C_{1-5}$ alkyl, and
- carbocyclic aryl substituted by halogenated $C_{1-5}$ alkyl;

wherein carbocyclic aryl is phenyl or naphthyl;
heterocyclyl is 1*H*-indolyl, 9*H*-carbazolyl, benzo[1,3]dioxolyl, or pyrazolyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

$N^2$-(cis-4-{[(5-bromo-1H-indol-3-yl)methyl]amino}cyclohexyl)-$N^4$,$N^4$-dimethylpyrimidine-2,4-diamine;
$N^2$-[cis-4-({[5-(4-fluorophenyl)pyridin-3-yl]methyl}amino)cyclohexyl]-$N^4$,$N^4$-dimethylpyrimidine-2,4-diamine;
ethyl 4,6-dichloro-3-{[(cis-4-{[4-(dimethylamino)pyrimidin-2-yl]amino}-cyclohexyl)-amino]methyl}-1H-indole-

2-carboxylate;
$N^2$-(cis-4-{[(2,6-dimethoxybenzyl)amino]methyl}cyclohexyl)-$N^4$,$N^4$-dimethylpyrimidine-2,4-diamine;
$N^2$-(cis-4-{[(2-ethoxybenzyl)amino]methyl}cyclohexyl)-$N^4$,$N^4$-dimethylpyrimidine-2,4-diamine;
$N^2$-cis-4-({[(4-methoxy-1-naphthyl)methyl]amino}methyl)cyclohexyl]-$N^4$,$N^4$-dimethylpyrimidine-2,4-diamine;
$N^2$-[cis-4-({[(5-methoxy-1H-indol-3-yl)methyl]amino}methyl)cyclohexyl]-$N^4$,$N^4$-dimethylpyrimidine-2,4-diamine;
$N^2$-[cis-4-({[(2-methoxy-1-naphthyl)methyl]amino}methyl)cyclohexyl]-$N^4$,$N^4$-dimethylpyrimidine-2,4-diamine;
4-bromo-2-({[(cis-4-{[4-(dimethy!amino)pyrimidin-2-yl]amino)}-cyclohexyl)methyl]-amino}methyl)-6-methoxyphenol;
$N^2$-[cis-4-({[(5-bromo-1H-indol-3-yl)methyl]amino}methyl)cyclohexyl]-$N^4$,$N^4$-dimethylpyrimidine-2,4-diamine;
$N^2$-(cis-4-{[(2,4-dimethoxybenzyl)amino]methyl}cyclohexyl)-$N^4$,$N^4$-dimethylpyrimidine-2,4-diamine;
$N^4$,$N^4$-dimethyl-$N^2$-(cis-4-{[(2,3,4-trimethoxybenzyl)amino]methyl}-cyclohexyl)pyrimidine-2,4-diamine;
$N^2$-(cis-4-{[(3-ethoxy-4-methoxybenzyl)amino]methyl}cyclohexyl)-$N^4$,$N^4$-dimethylpyrimidine-2,4-diamine;
$N^4$,$N^4$-dimethyl-$N^2$-(cis-4-{[({3-[4-(trifluoromethyl)phenyl]-1H-pyrazol-4-yl)methyl)-amino]methyl}cyclohexyl)pyrimidine-2,4-diamine;
$N^4$,$N^4$-dimethyl-$N^2$-(cis-4-{[(3,4,5-trimethoxybenzyl)amino]methyl}-cyclohexyl)pyrimidine-2,4-diamine;
4-({[(cis-4-{[4-(dimethylamino)pyrimidin-2-yl]amino}cyclohexyl)-methyl]amino}methyl)-2-iodo-6-methoxyphenol;
4-({[(cis-4-{[4-(dimethylamino)pyrimidin-2-yl]amino}cyclohexyl)methyl}-amino}methyl)-2,6-dimethylphenol;
$N^2$-(cis-4-{[(5-bromo-2,4-dimethoxybenzyl)amino]methyl}cyclohexyl)-$N^4$,$N^4$-dimethylpyrimidine-2,4-diamine;
$N^2$-(cis-4-{[(5-bromo-2-methoxybenzyl)amino]methyl}cyclohexyl)-$N^4$,$N^4$-dimethylpyrimidine-2,4-diamine;
$N^2$-[cis-4-({[4-(diethylamino)benzyl]amino}methyl)cyclohexyl]-$N^4$,$N^4$-dimethylpyrimidine-2,4-diamine;
$N^2$-[cis-4-({[(9-ethyl-9H-carbazol-3-yl)methyl]amino}methyl)cyclohexyl]-$N^4$,$N^4$-dimethylpyrimidine-2,4-diamine;
$N^2$-(cis-4-{[(4-isopropoxybenzyl)amino]methyl}cyclohexyl)-$N^4$,$N^4$-dimethylpyrimidine-2,4-diamine;
$N^2$-(cis-4-{[(3,3-diphenylprop-2-en-1-yl)amino]methyl}cyclohexyl)-$N^4$,$N^4$-dimethylpyrimidine-2,4-diamine;
4-({[(cis-4-{[4-(dimethylamino)pyrimidin-2-yl]amino}cyclohexyl)methyl]-amino}methyl)-2-ethoxyphenol;
$N^2$-{cis-4-[({[4-(dimethylamino)-1-naphthyl]methyl}amino)methyl]-cyclohexyl}-$N^4$,$N^4$-dimethylpyrimidine-2,4-diamine;
$N^4$,$N^4$-dimethyl-$N^2$-(cis-4-{[(2,4,6-trimethoxybenzyl)amino]methyl}-cyclohexyl)pyrimidine-2,4-diamine;
$N^2$-(cis-4-{[(5-bromo-2-ethoxybenzyl)amino]methyl}cyclohexyl)-$N^4$,$N^4$-dimethylpyrimidine-2,4-diamine;
$N^2$-(cis-4-{[(2,4-dimethoxy-3-methylbenzyl)amino]methyl}cyclohexyl)-$N^4$,$N^4$-dimethylpyrimidine-2,4-diamine;
$N^2$-(cis-4-{[(2,5-diethoxybenzyl)amino]methyl}cyclohexyl)-$N^4$,$N^4$-dimethylpyrimidine-2,4-diamine;
$N^2$-(cis-4-{[(2,4-diethoxybenzyl)amino]methyl}cyclohexyl)-$N^4$,$N^4$-dimethylpyrimidine-2,4-diamine;
$N^2$-(cis-4-{[(3,5-dibromo-2-methoxybenzyl)amino]methyl}cyclohexyl)-$N^4$,$N^4$-dimethylpyrimidine-2,4-diamine;
$N^4$,$N^4$-dimethyl-$N^2$-(cis-4-{[(2,4,5-triethoxybenzyl)amino]methyl}-cyclohexyl)pyrimidine-2,4-diamine;
$N^4$,$N^4$-dimethyl-$N^2$-(cis-4-{[(2,4,5-trimethoxybenzyl)amino]methyl}-cyclohexyl)pyrimidine-2,4-diamine;
$N^2$-[cis-4-({[2-(allyloxy)benzyl]amino}methyl)cyclohexyl]-$N^4$,$N^4$-dimethylpyrimidine-2,4-diamine;
$N^4$,$N^4$-dimethyl-$N^2$-[cis-4-({[(1-methyl-1H-indol-3-yl)methyl]amino}-methyl)cyclohexyl]-pyrimidine-2,4-diamine;
$N^2$-[cis-4-({[(7-methoxy-1,3-benzodioxol-5-yl)methyl]amino}methyl)-cyclohexyl]-$N^4$,$N^4$-dimethylpyrimidine-2,4-diamine;
$N^2$-(cis-4-{[(3-bromo-4,5-dimethoxybenzyl)amino]methyl}cyclohexyl)-$N^4$,$N^4$-dimethylpyrimidine-2,4-diamine;
$N^2$-(cis-4-{[(4-methoxy-3-methylbenzyl)amino]methyl}cyclohexyl)-$N^4$,$N^4$-dimethylpyrimidine-2,4-diamine;
$N^2$-(cis-4-{[(2-bromo-4,5-dimethoxybenzyl)amino]methyl}cyclohexyl)-$N^4$,$N^4$-dimethylpyrimidine-2,4-diamine;
$N^2$-(cis-4-{[(3,4-dimethoxybenzyl)amino]methyl}cyclohexyl)-$N^4$,$N^4$-dimethylpyrimidine-2,4-diamine;
$N^2$-(cis-4-{[(4-methoxy-2,5-dimethylbenzyl)amino]methyl}cyclohexyl)-$N^4$,$N^4$-dimethylpyrimidine-2,4-diamine;
3-[[4-({[(cis-4-{[4-(dimethylamino)pyrimidin-2-yl]amino}cyclohexyl)-methyl]amino}-methyl)phenyl](methyl)amino]propanenitrile;
$N^2$-{cis-4-[({4-[(4-bromobenzyl)oxy]benzyl}amino)methyl]cyclohexyl}-$N^4$,$N^4$-dimethylpyrimidine-2,4-diamine;
$N^2$-(cis-4-{[(3,5-dibromo-2-ethoxybenzyl)amino]methyl}cyclohexyl)-$N^4$,$N^4$-dimethylpyrimidine-2,4-diamine;
$N^2$-[4-(4-bromo-2-trifluoromethoxy-benzyl)amino-cyclohexyl]-$N^4$,$N^4$-dimethyl-pyrimidine-2,4-diamine;
$N^2$-{cis-4-[2-(4-bromo-2-trifluoromethoxy-phenyl)-ethylamino]-cyclohexyl}-$N^4$,$N^4$-dimethyl-pyrimidine-2,4-diamine; and
$N^2$-{cis-4-[(4-bromo-2-trifluoromethoxy-benzyl)amino-methyl]-cyclohexyl}-$N^4$,$N^4$-dimethyl-pyrimidine-2,4-diamine;

or a pharmaceutically acceptable salt, hydrate, or solvate thereof.

In some embodiments, compounds of the present invention are of Formula (I) wherein the compound is selected from the group consisting of:

ethyl 4,5-dichloro-3-{[(cis-4-{[4-(dimethylamino)pyrimidin-2-yl]amino}-cyclohexyl)-amino]methyl}-1H-indole-

2-carboxylate;
$N^2$-[cis-4-({[(4-methoxy-1-naphthyl)methyl]amino}methyl)cyclohexyl]-$N^4$,$N^4$-dimethylpyrimidine-2,4-diamine;
$N^2$-[cis-4-({[(2-methoxy-1-naphthyl)methyl]amino}methyl)cyclohexyl]-$N^4$,$N^4$-dimethylpyrimidine-2,4-diamine;
4-bromo-2-({[(cis-4-{[4-(dimethylamino)pyrimidin-2-yl]amino}-cyclohexyl)methyl]-amino}methyl)-6-methoxyphenol;
$N^2$-[cis-4-({[(5-bromo-1H-indol-3-yl)methyl]amino}methyl)cyclohexyl]-$N^4$,$N^4$-dimethylpyrimidine-2,4-diamine;
$N^4$,$N^4$-dimethyl-$N^2$-(cis-4-{[(2,3,4-trimethoxybenzyl)amino]methyl}-cyclohexyl)pyrimidine-2,4-diamine;
$N^2$-(cis-4-{[(3-ethoxy-4-methoxybenzyl)amino]methyl}cyclohexyl)-$N^4$,$N^4$-dimethylpyrimidine-2,4-diamine;
$N^4$,$N^4$-dimethyl-$N^2$-(cis-4-{[({3-[4-(trifluoromethyl)phenyl]-1H-pyrazol-4-yl}methyl)-amino]methyl}cyclohexyl)pyrimidine-2,4-diamine;
4-({[(cis-4-{[4-(dimethylamino)pyrimidin-2-yl]amino}cyclohexyl)methyl]-amino}methyl)-2-iodo-6-methoxyphenol;
4-({[(cis-4-{[4-(dimethylamino)pyrimidin-2-yl]amino}cyclohexyl)methyl]-amino}methyl)-2,6-dimethylphenol;
$N^2$-(cis-4-{[(5-bromo-2,4-dimethoxybenzyl)amino]methyl}cyclohexyl)-$N^4$,$N^4$-dimethylpyrimidine-2,4-diamine;
$N^2$-(cis-4-{[(5-bromo-2-methoxybenzyl)amino]methyl}cyclohexyl)-$N^4$,$N^4$-dimethylpyrimidine-2,4-diamine;
$N^2$-[cis-4-({[(9-ethyl-9H-carbazol-3-yl)methyl]amino}methyl)cyclohexyl]-$N^4$,$N^4$-dimethylpyrimidine-2,4-diamine;
$N^2$-(cis-4-{[(3,3-diphenylprop-2-en-1-yl)amino]methyl}cyclohexyl)-$N^4$,$N^4$-dimethylpyrimidine-2,4-diamine;
$N^4$,$N^4$-dimethyl-$N^2$-(cis-4-{[(2,4,6-trimethoxybenzyl)amino]methyl}-cyclohexyl)pyrimidine-2,4-diamine;
$N^2$-(cis-4-{[(5-bromo-2-ethoxybenzyl)amino]methyl}cyclohexyl)-$N^4$,$N^4$-dimethylpyrimidine-2,4-diamine;
$N^2$-(cis-4-{[(2,4-dimethoxy-3-methylbenzyl)amino]methyl}cyclohexyl)-$N^4$,$N^4$-dimethylpyrimidine-2,4-diamine;
$N^2$-(cis-4-{[(2,5-diethoxybenzyl)amino]methyl}cyclohexyl)-$N^4$,$N^4$-dimethylpyrimidine-2,4-diamine;
$N^2$-(cis-4-{[(3,5-dibromo-2-methoxybenzyl)amino]methyl}cyclohexyl)-$N^4$,$N^4$-dimethylpyrimidine-2,4-diamine;
$N^4$,$N^4$-dimethyl-$N^2$-(cis-4-{[(2,4,5-triethoxybenzyl)amino]methyl}-cyclohexyl)pyrimidine-2,4-diamine;
$N^2$-[cis-4-({[2-(allyloxy)benzyl]amino}methyl)cyclohexyl]-$N^4$,$N^4$-dimethylpyrimidine-2,4-diamine;
$N^2$-[cis-4-({[(7-methoxy-1,3-benzodioxol-5-yl)methyl]amino}methyl)-cyclohexyl]-$N^4$,$N^4$-dimethylpyrimidine-2,4-diamine;
$N^2$-(cis-4-{[(3-bromo-4,5-dimethoxybenzyl)amino]methyl}cyclohexyl)-$N^4$,$N^4$-dimethylpyrimidine-2,4-diamine;
$N^2$-{cis-4-[2-(4-bromo-2-trifluoromethoxy-phenyl)-ethylamino]-cyclohexyl}-$N^4$,$N^4$-dimethyl-pyrimidine-2,4-diamine; and
$N^2$-{cis-4-[(4-bromo-2-thifluoromethoxy-benzyl)amino-methyl]-cyclohexyl}-$N^4$,$N^4$-dimethyl-pyrimidine-2,4-diamine;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- carbocyclic aryloxy substituted by $C_{1-5}$ alkoxy,
- heterocyclyloxy,
- heterocyclyloxy substituted by $C_{1-5}$ alkyl,
- mono-$C_{1-5}$ alkylaminocarbonyl,
- di-$C_{1-5}$ alkylaminocarbonyl,
- mono-$C_{1-5}$ alkylamino,
- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- carbocyclic arylcarbonylamino,
- $C_{1-5}$ alkoxycarbonylamino,
- $C_{1-5}$ alkylthio,

- $C_{1-5}$ alkylthio substituted by substituent(s) independently selected from the group consisting of:
    - •• carbocyclic aryl, and
    - •• carbocyclic aryl substituted by substituent(s) independently selected from the group consisting of:
        - ••• halogen, and
        - ••• $C_{1-5}$ alkoxy,
- carbocyclic arylthio,
- heterocyclylthio,
- heterocyclylthio substituted by $C_{1-5}$ alkyl,
- heterocyclylthio substituted by nitro,
- $C_{3-6}$ cycloalkyl,
- $C_{3-6}$ cycloalkenyl,
- carbocyclyl,
- carbocyclyl substituted by substituent(s) independently selected from the group consisting of:
    - •• halogen,
    - •• $C_{1-5}$ alkyl,
    - •• $C_{1-5}$ alkoxy,
    - •• $C_{2-5}$ alkenyl, and
    - •• $C_{2-5}$ alkenyl substituted by substituent(s) independently selected from the group consisting of:
        - ••• carbocyclic aryl, and
        - ••• carbocyclic aryl substituted by $C_{1-5}$ alkylsulfinyl,
- carbocyclic aryl,
- carbocyclic aryl substituted by substituent(s) independently selected from the group consisting of:
    - •• halogen,
    - •• hydroxy,
    - •• nitro,
    - •• $C_{1-5}$ alkyl,
    - •• $C_{1-5}$ alkyl substituted by substituent(s) independently selected from the group consisting of:
        - ••• oxo,
        - ••• carbocyclic aryl, and
        - ••• heterocyclyl,
    - •• $C_{1-5}$ alkoxy,
    - •• $C_{1-5}$ alkoxy substituted by halogen,
    - •• $C_{1-5}$ alkoxy substituted by carbocyclic aryl,
    - •• carbocyclic aryloxy,
    - •• carbocyclic aryl, and
    - •• heterocyclyl,
- heterocyclyl, and
- heterocyclyl substituted by substituent(s) independently selected from the group consisting of:
    - •• $C_{1-5}$ alkyl,
    - •• $C_{1-5}$ alkyl substituted by carbocyclic aryl,
    - •• $C_{1-5}$ alkoxy,
    - •• $C_{1-5}$ alkoxy substituted by carbocyclic aryl,
    - •• carbocyclic aryl, and
    - •• carbocyclic aryl substituted by halogen,

(ii) $C_{2-5}$ alkenyl, and
$C_{2-5}$ alkenyl substituted by substituent(s) independently selected from the group consisting of:

- carbocyclic aryl,
- carbocyclic aryl substituted by substituent(s) independently selected from the group consisting of:

  - halogen, and
  - nitro,

(iii) $C_{3-6}$ cycloalkyl, and
$C_{3-6}$ cycloalkyl substituted by substituent(s) independently selected from the group consisting of:

- $C_{1-5}$ alkyl,
- $C_{1-5}$ alkyl substituted by substituent(s) independently selected from the group consisting of:

  - oxo, and
  - carbocyclic aryl, and

- carbocyclic aryl,

(iv) carbocyclyl,
(v) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:

  - halogen,
  - oxo,
  - carbocyclic aryloxy,
  - carbocyclic aryl, and
  - carbocyclic aryl substituted by $C_{1-5}$ alkyl,

- $C_{1-5}$ alkoxy,
- $C_{1-5}$ alkoxy substituted by substituent(s) independently selected from the group consisting of:

  - halogen, and
  - carbocyclic aryl,

- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,

- mono-$C_{1-5}$ alkylaminosulfonyl,
- di-$C_{1-5}$ alkylaminosulfonyl, and
- carbocyclic aryl,
- carbocyclic aryl substituted by halogen,
- heterocyclyl, and
- heterocyclyl substituted by substituent(s) independently selected from the group consisting of:
    - •• $C_{1-5}$ alkyl,
    - •• carbocyclic aryl, and
    - •• halogenated carbocyclic aryl,

(vi) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:
    - •• halogen,
    - •• $C_{1-5}$ alkylthio,
    - •• $C_{1-5}$ alkylthio substituted by carbocyclic aryl,
    - •• $C_{1-5}$ alkylthio substituted by halogenated carbocyclic aryl,
    - •• carbocyclic aryl,
    - •• carbocyclic aryl substituted by halogen, and
    - •• heterocyclyl,
- $C_{1-5}$ alkoxy,
- carbocyclic aryloxy,
- carbocyclic aryloxy substituted by halogen,
- carbocyclic aryloxy substituted by $C_{1-5}$ alkyl,
- $C_{1-5}$ alkylthio,
- $C_{2-5}$ alkenylthio,
- carbocyclic arylthio,
- $C_{1-5}$ alkylsulfonyl,
- carbocyclic arylsulfonyl,
- carbocyclic arylsulfonyl substituted by $C_{1-5}$ alkyl,
- carbocyclic aryl,
- carbocyclic aryl substituted by substituent(s) independently selected from the group consisting of:
    - •• halogen,
    - •• nitro, and
    - •• $C_{1-5}$ alkyl,
- heterocyclyl;

L is Formula (VII);
Y is -C(O)-;
wherein carbocyclic aryl is phenyl, naphthyl, or anthranyl;
carbocyclyl is 1,2,3,4-tetrahydronaphthyl, 1-oxo-indanyl, 9-oxo-9*H*-fluorenyl, or indenyl;
heterocyclyl is 1,2,3-triazolyl, 1*H*-indolyl, 1*H*-pyrrolyl, 2,3-dihydro-1-oxo-isoindolyl, 2,3-dihydro-benzofuryl, 2,4-dihydro-3-oxo-pyrazolyl, 2*H*-benzopyranyl, 2-oxo-benzopyranyl, 4-oxo-1,5,6,7-tetrahydro-indolyl, 9*H*-xanthenyl, benzo[1,3]dioxolyl, benzo[2,1,3]oxadiazolyl, benzo[1,2,5]oxadiazolyl, benzo[b]thienyl, benzofuryl, benzothiazolyl, furyl, imidazolyl, isoxazolyl, morpholino, pyrazolyl, pyridyl, pyrimidyl, quinolyl, quinoxalyl, thiazolyl, or thienyl; and
halogen is fluoro, chloro, bromo, or iodo;

or a pharmaceutically acceptable salt, hydrate, or solvate thereof.

In some embodiments of the present invention, $R_2$ is hydrogen, trifluoromethyl, methoxy, methylamino, dimethylamino, ethyl amino, ethylmethylamino, or hydroxylethylmethylamino; p is 0; $R_3$ and $R_4$ are hydrogen; A is a single bond; B is a single bond or $-CH_2-$; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- oxo,
- carbocyclic aryloxy,
- carbocyclic aryloxy substituted by halogen,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylamino,
- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- carbocyclic arylcarbonylamino,
- $C_{1-5}$ alkylthio,
- $C_{1-6}$ cycloalkyl,
- carbocyclyl,
- carbocyclyl substituted by substituent(s) independently selected from the group consisting of:
  - •• halogen,
  - •• $C_{1-5}$ alkyl,
  - •• $C_{2-5}$ alkenyl, and
  - •• $C_{2-5}$ alkenyl substituted by substituent(s) independently selected from the group consisting of:
    - ••• carbocyclic aryl, and
    - ••• carbocyclic aryl substituted by $C_{1-5}$ alkylsulfinyl,
- carbocyclic aryl,
- carbocyclic aryl substituted by substituent(s) independently selected from the group consisting of:
  - •• halogen,
  - •• hydroxy,
  - •• nitro,
  - •• $C_{1-5}$ alkyl,
  - •• $C_{1-5}$ alkyl substituted by substituent(s) independently selected from the group consisting of:
    - ••• oxo,
    - ••• carbocyclic aryl, and
    - ••• heterocyclyl,
  - •• $C_{1-5}$ alkoxy,
  - •• $C_{1-5}$ alkoxy substituted by halogen,
- heterocyclyl, and
- heterocyclyl substituted by substituent(s) independently selected from the group consisting of:
  - •• $C_{1-5}$ alkyl,
  - •• carbocyclic aryl, and
  - •• carbocyclic aryl substituted by halogen,

(ii) $C_{2-5}$ alkenyl, and

$C_{2-5}$ alkenyl substituted by substituent(s) independently selected from the group consisting of:

- carbocyclic aryl,
- carbocyclic aryl substituted by substituent(s) independently selected from the group consisting of:
  - halogen, and
  - nitro,

(iii) carbocyclyl,
(iv) carbocyclic aryl, and
carbocyclic aryl substituted by substituent(s) independently selected from the group consisting of:

- halogen,
- hydroxy,
- nitro,
- $C_{1-5}$ alkyl,
- $C_{1-5}$ alkyl substituted by substituent(s) independently selected from the group consisting of:
  - halogen,
  - oxo, and
  - carbocyclic aryl,
- $C_{1-5}$ alkoxy,
- $C_{1-5}$ alkoxy substituted by substituent(s) independently selected from the group consisting of:
  - halogen, and
  - carbocyclic aryl,
- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- mono-$C_{1-5}$ alkylaminosulfonyl, and
- di-$C_{1-5}$ alkylaminosulfonyl,

(v) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:
  - $C_{1-5}$ alkylthio,
  - $C_{1-5}$ alkylthio substituted by carbocyclic aryl,
  - $C_{1-5}$ alkylthio substituted by halogenated carbocyclic aryl,
  - carbocyclic aryl,
  - carbocyclic aryl substituted by halogen, and
  - heterocyclyl,
- carbocyclic aryloxy,
- carbocyclic aryloxy substituted by halogen,

- carbocyclic aryloxy substituted by $C_{1-5}$ alkyl,
- $C_{1-5}$ alkylthio,
- $C_{1-5}$ alkylsulfonyl,
- carbocyclic arylsulfonyl,
- carbocyclic arylsulfonyl substituted by $C_{1-5}$ alkyl,
- carbocyclic aryl,
- carbocyclic aryl substituted by substituent(s) independently selected from the group consisting of:

  - •• halogen,
  - •• nitro, and
  - •• $C_{1-5}$ alkyl,

- heterocyclyl;

wherein carbocyclic aryl is phenyl or naphthyl;
carbocyclyl is 1-oxo-indanyl, 9-oxo-9*H*-fluorenyl, or indenyl;
heterocyclyl is 1,2,3-triazolyl, 1*H*-indolyl, 1*H*-pyrrolyl, 2,3-dihydro-benzofuryl, 2*H*-benzopyranyl, 9*H*-xanthenyl, benzo[2,1,3]oxadiazolyl, benzo[1,2,5]oxadiazolyl, benzo[b]thienyl, furyl, isoxazolyl, morpholino, pyrazolyl, pyridyl, quinolyl, quinoxalyl, thiazolyl, or thienyl; and
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- oxo,
- carbocyclic aryloxy,
- carbocyclic aryloxy substituted by halogen,
- carbocyclic aryloxy substituted by $C_{1-5}$ alkoxy,
- mono-$C_{1-5}$ alkylamino,
- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- $C_{1-5}$ alkylthio,
- carbocyclic aryl,
- carbocyclic aryl substituted by substituent(s) independently selected from the group consisting of:

  - •• halogen,
  - •• hydroxy,
  - •• $C_{1-5}$ alkyl,
  - •• $C_{1-5}$ alkoxy, and
  - •• $C_{1-5}$ alkoxy substituted by halogen,

- heterocyclyl, and
- heterocyclyl substituted by substituent(s) independently selected from the group consisting of:

  - •• $C_{1-5}$ alkyl,
  - •• carbocyclic aryl, and
  - •• carbocyclic aryl substituted by halogen,

(ii) $C_{2-5}$ alkenyl, and
$C_{2-5}$ alkenyl substituted by substituent(s) independently selected from the group consisting of:

- carbocyclic aryl,

- carbocyclic aryl substituted by nitro,

(iii) carbocyclyl,
(iv) carbocyclic aryl, and
carbocyclic aryl substituted by substituent(s) independently selected from the group consisting of:

- halogen,
- hydroxy,
- nitro,
- $C_{1-5}$ alkyl,
- $C_{1-5}$ alkyl substituted by halogen,
- $C_{1-5}$ alkoxy,
- $C_{1-5}$ alkoxy substituted by halogen,
- $C_{1-5}$ alkoxy substituted by carbocyclic aryl,
- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- mono-$C_{1-5}$ alkylaminosulfonyl, and
- di-$C_{1-5}$ alkylaminosulfonyl,

(v) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:
    - •• $C_{1-5}$ alkylthio,
    - •• $C_{1-5}$ alkylthio substituted by carbocyclic aryl, and
    - •• $C_{1-5}$ alkylthio substituted by halogenated carbocyclic aryl,
- carbocyclic aryloxy,
- carbocyclic aryloxy substituted by halogen,
- carbocyclic aryloxy substituted by $C_{1-5}$ alkyl,
- carbocyclic aryl,
- carbocyclic aryl substituted by halogen,
- carbocyclic aryl substituted by nitro, and
- heterocyclyl;

wherein carbocyclic aryl is phenyl or naphthyl;
carbocyclyl is 1-oxo-indanyl;
heterocyclyl is 1,2,3-triazolyl, 1*H*-indolyl, 1*H*-pyrrolyl, 2,3-dihydro-benzofuryl, 9*H*-xanthenyl, benzo[2,1,3]oxadiazolyl, benzo[1,2,5]oxadiazolyl, benzo[b]thienyl, furyl, isoxazolyl, pyridyl, quinoxalyl, thiazolyl, or thienyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(cis-4-{[4-(dimethylamino)pyrimidin-2-yl]amino}cyclohexyl)-3-methoxybenzamide;
3-bromo-N-(cis-4-{[4-(dimethylamino)pyrimidin-2-yl]amino}cyclohexyl)-benzamide;
N-(cis-4-{[4-(dimethylamino)pyrimidin-2-yl]amino}cyclohexyl)-2,1,3-benzoxadiazole-5-carboxamide;
3-chloro-N-(cis-4-{[4-(dimethylamino)pyrimidin-2-yl]amino}cyclohexyl)-benzamide;
4-chloro-N-(cis-4-{[4-(dimethylamino)pyrimidin-2-yl]amino}cyclohexyl)-benzamide;

4-chloro-N-(cis-4-{[4-(dimethylamino)pyrimidin-2-yl]amino}cyclohexyl)-3-nitrobenzamide;
3,5-dichloro-N-(cis-4-{[4-(dimethylamino)pyrimidin-2-yl]amino}-cyclohexyl)benzamide;
3,4-dichloro-N-(cis-4-{[4-(dimethylamino)pyrimidin-2-yl]amino}-cyclohexyl)benzamide;
N-(cis-4-{[4-(dimethylamino)pyrimidin-2-yl]amino}cyclohexyl)-2,2-diphenylacetamide;
N-(cis-4-{[4-(dimethylamino)pyrimidin-2-yl]amino}cyclohexyl)-3,4-difluorobenzamide;
N-(cis-4-{[4-(dimethylamino)pyrimidin-2-yl]amino}cyclohexyl)-3,5-difluorobenzamide;
N-(cis-4-{[4-(dimethylamino)pyrimidin-2-yl]amino}cyclohexyl)-3-fluoro-5-(trifluoromethyl)benzamide;
N-(cis-4-{[4-(dimethylamino)pyrimidin-2-yl]amino}cyclohexyl)-4-methyl-3-nitrobenzamide;
N-(cis-4-{[4-(dimethylamino)pyrimidin-2-yl]amino}cyclohexyl)-3-nitrobenzamide;
N-(cis-4-{[4-(dimethylamino)pyrimidin-2-yl]amino}cyclohexyl)-2-phenoxybutanamide;
N-(cis-4-{[4-(dimethylamino)pyrimidin-2-yl]amino}cyclohexyl)-3-methylbenzamide;
N-(cis-4-{[4-(dimethylamino)pyrimidin-2-yl]amino}cyclohexyl)-3-(trifluoromethoxy)-benzamide;
4-bromo-N-(cis-4-{[4-(dimethylamino)pyrimidin-2-yl]amino}cyclohexyl)-3-methylbenzamide;
N-(cis-4-{[4-(dimethylamino)pyrimidin-2-yl]amino}cyclohexyl)-3-iodobenzamide;
N-(cis-4-{[4-(dimethylamino)pyrimidin-2-yl]amino}cyclohexyl)-2,5-dimethyl-3-furamide;
3-chloro-N-(cis-4-{[4-(dimethylamino)pyrimidin-2-yl]amino}cyclohexyl)-4-fluorobenzamide;
N-(cis-4-{[4-(dimethylamino)pyrimidin-2-yl]amino}cyclohexyl)-3,5-dimethoxybenzamide;
N-(cis-4-{[4-(dimethylamino)pyrimidin-2-yl]amino}cyclohexyl)-3,5-bis(trifluoromethyl)-benzamide;
N-(cis-4-{[4-(dimethylamino)pyrimidin-2-yl]amino}cyclohexyl)-4-fluoro-3-methylbenzamide;
2,5-dichloro-N-(cis-4-{[4-(dimethylamino)pyrimidin-2-yl]amino}-cyclohexyl)thiophene-3-carboxamide;
1-benzyl-3-tert-butyl-N-(cis-4-{[4-(dimethylamino)pyrimidin-2-yl]amino}-cyclohexyl)-1H-pyrazole-5-carboxamide;
N-(cis-4-{[4-(dimethylamino)pyrimidin-2-yl]amino}cyclohexyl)-2-(1-naphthyl)acetamide;
2-(4-chlorophenoxy)-N-(cis-4-{[4-(dimethylamino)pyrimidin-2-yl]amino}-cyclohexyl)-acetamide;
1-(4-chlorophenyl)-N-(cis-4-{[4-(dimethylamino)pyrimidin-2-yl]amino}-cyclohexyl)-cyclopentanecarboxamide;
3-(2-chloro-6-fluorophenyl)-N-(cis-4-{[4-(dimethylamino)pyrimidin-2-yl]amino}-cyclohexyl)-5-methylisoxazole-4-carboxamide;
N-(cis-4-{[4-(dimethylamino)pyrimidin-2-yl] amino}cyclohexyl)-4-fluoro-3-(trifluoromethyl)benzamide;
N-(cis-4-{[4-(dimethylamino)pyrimidin-2-yl]amino}cyclohexyl)-5-methyl-2-phenyl-2H-1,2,3-triazole-4-carboxamide;
N-(cis-4-{[4-(dimethylamino)pyrimidin-2-yl]amino}cyclohexyl)-2-(4-methoxyphenoxy)-5-nitrobenzamide;
N-(cis-4-{[4-(dimethylamino)pyrimidin-2-yl]amino}cyclohexyl)-2-phenoxyacetamide;
N-(cis-4-{[4-(dimethylamino)pyrimidin-2-yl]amino}cyclohexyl)-quinoxaline-2-carboxamide;
N-(cis-4-{[4-(dimethylamino)pyrimidin-2-yl]amino}cyclohexyl)-3-(trifluoromethyl)-benzamide;
N-(cis-4-{[4-(dimethylamino)pyrimidin-2-yl]amino}cyclohexyl)-2-(pentafluorophenoxy)-acetamide;
2-(3-chlorophenoxy)-N-(cis-4-{[4-(dimethylamino)pyrimidin-2-yl]amino}cyclohexyl)-acetamide;
3-(2,6-dichlorophenyl)-N-(cis-4-{[4-(dimethylamino)pyrimidin-2-yl]amino )cyclohexyl)-5-methylisoxazole-4-carboxamide;
N-(cis-4-{[4-(dimethylamino)pyrimidin-2-yl]amino}cyclohexyl)-2-phenoxynicotinamide;
N-(cis-4-{[4-(dimethylamino)pyrimidin-2-yl]amino}cyclohexyl)-2-(4-methylphenoxy)-nicotinamide;
N-(cis-4-{[4-(dimethylamino)pyrimidin-2-yl]amino}cyclohexyl)-4-[(dipropylamino)-sulfonyl]benzamide;
2-(4-chlorophenoxy)-N-(cis-4-{[4-(dimethylamino)pyrimidin-2-yl]amino}cyclohexyl)-2-methylpropanamide;
2-(2,3-dihydro-1-benzofuran-5-yl)-N-(cis-4-{[4-(dimethylamino)pyrimidin-2-yl]amino}-cyclohexyl)-1,3-thiazole-4-carboxamide;
3-tert-butyl-1-(2,4-dichlorobenzyl)-N-(cis-4-{[4-(dimethylamino)pyrimidin-2-yl]amino}-cyclohexyl)-1H-pyrazole-5-carboxamide;
6-chloro-N-(cis-4-{[4-(dimethylamino)pyrimidin-2-yl]amino}cyclohexyl)-2H-chromene-3-carboxamide;
N-(cis-4-{[4-(dimethylamino)pyrimidin-2-yl]amino}cyclohexyl)-2-(2-thienyl)-1,3-thiazole-4-carboxamide;
5-(4-chloro-2-nitrophenyl)-N-(cis-4-{[4-(dimethylamino)pyrimidin-2-yl]amino}cyclohexyl)-2-furamide;
N-(cis-4-{[4-(dimethylamino)pyrimidin-2-yl]amino}cyclohexyl)-5-iodo-2-furamide;
N-(cis-4-{[4-(dimethylamino)pyrimidin-2-yl]amino}cyclohexyl)-5-(4-methyl-2-nitrophenyl)-2-furamide;
N-(cis-4-{[4-(dimethylamino)pyrimidin-2-yl]amino}cyclohexyl)-5-nitrothiophene-2-carboxamide;
N-(cis-4-{[4-(dimethylamino)pyrimidin-2-yl]amino}cyclohexyl)-3-methyl-4-nitrobenzamide;
N-(cis-4-{[4-(dimethylamino)pyrimidin-2-yl]amino}cyclohexyl)-3-methoxy-4-nitrobenzamide;
1-benzyl-N-(cis-4-{[4-(dimethylamino)pyrimidin-2-yl]amino}cyclohexyl)-1H-indole-3-carboxamide;
3-acetyl-N-(cis-4-{[4-(dimethylamino)pyrimidin-2-yl]amino}cyclohexyl)-benzamide;
5-bromo-N-(cis-4-{[4-(dimethylamino)pyrimidin-2-yl]amino}cyclohexyl)-2-furamide;
5-(4-chlorophenyl)-N-(cis-4-{[4-(dimethylamino)pyrimidin-2-yl]amino}-cyclohexyl)-2-furamide;
4,5-dibromo-N-(cis-4-{[4-(dimethylamino)pyrimidin-2-yl]amino}-cyclohexyl)thiophene-2-carboxamide;

2-(3,5-di-tert-butyl-4-hydroxyphenyl)-N-(cis-4-[4-(dimethylamino)-pyrimidin-2-yl]amino}cyclohexyl)acetamide;
$N^2$,$N^6$-dibenzoyl-N-(cis-4-{[4-(dimethylamino)pyrimidin-2-yl]amino}-cyclohexyl)-lysinamide;
3-(dimethytamino)-N-(cis-4-{[4-(dimethylamino)pyrimidin-2-yl]amino}-cyclohexyl)-benzamide;
4,5-dibromo-N-(cis-4-{[4-(dimethylamino)pyrimidin-2-yl]amino}-cyclohexyl)-2-furamide;
N-(cis-4-{[4-(dimethylamino)pyrimidin-2-yl]amino}cyclohexyl)-2-(1H-indol-3-yl)-acetamide;
N-(cis-4-{[4-(dimethylamino)pyrimidin-2-yl]amino}cyclohexyl)-2-(5-methyl-2-phenyl-1,3-thiazol-4-yl)acetamide;
N-(cis-4-{[4-(dimethylamino)pyrimidin-2-yl]amino}cyclohexyl)-2-(1H-indol-3-yl)-4-oxo-4-phenylbutanamide;
4-(4-bromophenyl)-N-(cis-4-{[4-(dimethylamino)pyrimidin-2-yl]amino}-cyclohexyl)-2-(1H-indol-3-yl)-4-oxobutanamide;
3,5-dichloro-N-(cis-4-{[4-(dimethylamino)pyrimidin-2-yl]amino}-cyclohexyl)-2-[(3-phenylprop-2-ynoyl)amino]benzamide;
N-(cis-4-{[4-(dimethylamino)pyrimidin-2-yl]amino}cyclohexyl)-2-(1-methyl-1H-indol-3-yl)-4-(4-methylphenyl)-4-oxobutanamide;
N-(cis-4-{[4-(dimethylamino)pyrimidin-2-yl]amino}cyclohexyl)-2-methyl-1-(3-morpholin-4-ylpropyl)-5-phenyl-1H-pyrrole-3-carboxamide;
N-(cis-4-{[4-(dimethylamino)pyrimidin-2-yl]amino}cyclohexyl)-4-(4-nitrophenyl)-butanamide;
N-(cis-4-{[4-(dimethylamino)pyrimidin-2-yl]amino}cyclohexyl)-2-(2-phenyl-1H-indol-3-yl)acetamide;
$N^2$-benzoyl-$N^5$-(cis-4-{[4-(dimethylamino)pyrimidin-2-yl]amino}-cyclohexyl)-$N^1$,$N^1$-dipropylglutamamide;
N-(cis-4-{[4-(dimethylamino)pyrimidin-2-yl]amino}cyclohexyl)-3-phenoxybenzamide;
3-benzoyl-N-(cis-4-{[4-(dimethylamino)pyrimidin-2-yl]amino}-cyclohexyl)benzamide;
N-(cis-4-{[4-(dimethylamino)pyrimidin-2-yl]amino}cyclohexyl)-2-(ethylthio)-2,2-diphenylacetamide;
N-(cis-4-{[4-(dimethylamino)pyrimidin-2-yl]amino}cyclohexyl)-N'-[(1R)-1-(1-naphthyl)ethyl]phthalamide;
(2S)-2-(3-benzoylphenyl)-N-(cis-4-{[4-(dimethylamino)pyrimidin-2-yl]amino}cyclohexyl)-propanamide;
N'-(cis-4-{[4-(dimethylamino)pyrimidin-2-yl]amino}cyclohexyl)-N,N-bis [(1S)-1-phenylethyl]phthalamide;
N-(cis-4-{[4-(dimethylamino)pyrimidin-2-yl]amino}cyclohexyl)-2-{(1E)-5-fluoro-2-methyl-1-[4-(methylsulfinyl)benzylidene]-1H-inden-3-yl acetamide;
N-(cis-4-{[4-(dimethylamino)pyrimidin-2-yl]amino cyclohexyl)-2-[4-(2-thienylcarbonyl)-phenyl]propanamide;
3-(benzyloxy)-N-(cis-4-{[4-(dimethylamino)pyrimidin-2-yl]amino}-cyclohexyl)-4-methoxybenzamide;
N-(cis-4-{[4-(dimethylamino)pyrimidin-2-yl]amino}cyclohexyl)-2-methyl-1,5-diphenyl-1H-pyrrole-3-carboxamide;
1-{2-[(2-chloro-6-fluorobenzyl)thio]ethyl}-N-(cis-4-{[4-(dimethylamino)-pyrimidin-2-yl]-amino}cyclohexyl)-2-methyl-5-phenyl-1H-pyrrole-3-carboxamide;
N-(cis-4-{[4-(dimethylamino)pyrimidin-2-yl]amino}cyclohexyl)-2-phenoxybenzamide;
N-(cis-4-{[4-(dimethylamino)pyrimidin-2-yl]amino}cyclohexyl)-2-phenylquinoline-4-carboxamide;
2-[4-(4-chlorophenyl)-2-phenyl-1,3-thiazol-5-yl]-N-(cis-4-{[4-(dimethylamino)pyrimidin-2-yl]amino}cyclohexyl) acetamide;
N-(cis-4-{[4-(dimethylamino)pyrimidin-2-yl]amino}cyclohexyl)-1-[(4-methylphenyl)-sulfonyl]-1H-pyrrole-3-carboxamide;
N-(cis-4-{[4-(dimethylamino)pyrimidin-2-yl]amino}cyclohexyl)-5-(3-nitrophenyl)-2-furamide;
3-chloro-N-(cis-4-{[4-(dimethylamino)pyrimidin-2-yl]amino}cyclohexyl)-4-(isopropylsulfonyl)-5-(methylthio)thiophene-2-carboxamide;
N-(cis-4-{[4-(dimethylamino)pyrimidin-2-yl]amino}cyclohexyl)-3-iodo-4-(isopropylsulfonyl)-5-(methylthio)thiophene-2-carboxamide;
N-(cis-4-{[4-(dimethylamino)pyrimidin-2-yl]amino}cyclohexyl)-5-nitrothiophene-3-carboxamide;
N-(cis-4-{[4-(dimethylamino)pyrimidin-2-yl]amino}cyclohexyl)-1-methyl-4-nitro-1H-pyrrole-2-carboxamide;
N-(cis-4-{[4-(dimethylamino)pyrimidin-2-yl]amino}cyclohexyl)-4-nitrobenzamide;
N-(cis-4-{[4-(dimethylamino)pyrimidin-2-yl]amino)}cyclohexyl)-3,5-dimethyl-4-nitrobenzamide;
N-(cis-4-{[4-(dimethylamino)pyrimidin-2-yl] amino}cyclohexyl)-2-mesityl-2-oxoacetamide;
3,5-di-tert-butyl-N-(cis-4-1[4-(dimethylamino)pyrimidin-2-yl]amino}-cyclohexyl)-4-hydroxybenzamide;
4-chloro-N-[(cis-4-{ [4-(dimethylamino)pyrimidin-2-yl]amino}cyclohexyl)-methyl]-benzamide;
(2E)-N-[(cis-4-{[4-(dimethylamino)pyrimidin-2-yl]amino}cyclohexyl)-methyl]-3-phenylacrylamide;
4-chloro-N-[(cis-4-{[4-(dimethylamino)pyrimidin-2-yl]amino}cyclohexyl)-methyl]-3-nitrobenzamide;
2-(4-chlorophenyl)-N-[(cis-4-{[4-(dimethylamino)pyrimidin-2-yl]amino}-cyclohexyl)-methyl]acetamide;
3,5-dichloro-N-[(cis-4-{[4-(dimethylamino)pyrimidin-2-yl]amino} -cyclohexyl)methyl]-benzamide;
3,4-dichloro-N-[(cis-4-{[4-(dimethylamino)pyrimidin-2-yl]amino)-cyclohexyl)methyl]-benzamide;
N-[(cis-4-{[4-(dimethylamino)pyrimidin-2-yl]amino}cyclohexyl)methyl]-2,2-diphenylacetamide;
2,4-dichloro-N-[(cis-4-{[4-(dimethylamino)pyrimidin-2-yl]amino}cyclohexyl)methyl]-5-fluorobenzamide;
N-[(cis-4-{[4-(dimethylamino)pyrimidin-2-yl]amino}cyclohexyl)methyl]-2-phenoxybutanamide;
N-[(cis-4-{[4-(dimethylamino)pyrimidin-2-yl]amino}cyclohexyl)methyl]-2-phenylbutanamide;

N-[(cis-4-{[4-(dimethylamino)pyrimidin-2-yl]amino}cyclohexyl)methyl]-2-(3-methoxyphenyl)acetamide;
N-[(cis-4-{[4-(dimethylamino)pyrimidin-2-yl]amino}cyclohexyl)methyl]-2-(4-methoxyphenyl)acetamide;
N-[(cis-4-{[4-(dimethylamino)pyrimidin-2-yl]amino}cyclohexyl)methyl]-3,5-bis(trifluoromethyl)benzamide;
(2E)-N-[(cis-4-{[4-(dimethylamino)pyrimidin-2-yl]amino}cyclohexyl)-methyl]-3-(4-nitrophenyl)acrylamide;
2-(2-bromophenyl)-N-[(cis-4-{[4-(dimethylamino)pyrimidin-2-yl]amino}-cyclohexyl)-methyl]acetamide;
N-[(cis-4-{[4-(dimethylamino)pyrimidin-2-yl]amino}cyclohexyl)methyl]-2-(propylthio)-nicotinamide;
N-[(cis-4-{[4-(dimethylamino)pyrimidin-2-yl]amino}cyclohexyl)methyl]-2-(1-naphthyl)-acetamide;
N-[(cis-4-{[4-(dimethylamino)pyrimidin-2-yl]amino}cyclohexyl)methyl]-9-oxo-9H-fluorene-4-carboxamide;
N-[(cis-4-{[4-(dimethylamino)pyrimidin-2-yl]amino}cyclohexyl)methyl]-2,4,6-trimethylbenzamide;
2,4,6-trichloro-N-[(cis-4-{[4-(dimethylamino)pyrimidin-2-yl]amino}-cyclohexyl)methyl]-benzamide;
(2E)-3-(2-chlorophenyl)-N-[(cis-4-{[4-(dimethylamino)pyrimidin-2-yl]amino}cyclohexyl)-methyl]acrylamide;
N-[(cis-4-{[4-(dimethylamino)pyrimidin-2-yl]amino}cyclohexyl)methyl]-2-(2,3,6-trichlorophenyl)acetamide;
N-[(cis-4-{[4-(dimethylamino)pyrimidin-2-yl]amino}cyclohexyl)methyl]-2,3-diphenylpropanamide;
N-[(cis-4-{[4-(dimethylamino}pyrimidin-2-yl]amino}cyclohexyl)methyl]-5-iodo-2-furamide;
(2E)-N-[(cis-4-{[4-(dimethylamino)pyrimidin-2-yl]amino}cyclohexyl)-methyl]-3-(3-nitrophenyl)acrylamide;
N-[(cis-4-{[4-(dimethylamino)pyrimidin-2-yl]amino}cyclohexyl)methyl]-3-oxoindane-1-carboxamide;
2-benzyl-N-[(cis-4-{[4-(dimethylamino)pyrimidin-2-yl]amino}cyclohexyl)-methyl]-benzamide;
2,2-bis(4-chlorophenyl)-N-[(cis-4-{[4-(dimethylamino)pyrimidin-2-yl]amino}cyclohexyl)-methyl]acetamide;
N-[(cis-4-[[4-(dimethylamino)pyrimidin-2-yl]amino}cyclohexyl)methyl]-3-methyl-4-nitrobenzamide;
N-[(cis-4-{[4-(dimethylamino)pyrimidin-2-yl]amino}cyclohexyl)methyl]-3-methoxy-4-nitrobenzamide;
N-[(cis-4-{[4-(dimethylamino)pyrimidin-2-yl]amino}cyclohexyl)methyl]-2-[2-(trifluoromethoxy)phenyl]acetamide;
N-[(cis-4-{[4-(dimethylamino)pyrimidin-2-yl]amino}cyclohexyl)methyl]-9H-xanthene-9-carboxamide;
2-(1-benzothien-3-yl)-N-[(cis-4-{[4-(dimethylamino)pyrimidin-2-yl]amino}cyclohexyl)-methyl]acetamide;
N-[cis-4-(4-dimethylamino-pyrimidin-2-ylamino)-cyclohexyl]-2-(4-fluoro-phenoxy)-nicotinamide;
N-[cis-4-(4-dimethylamino-pyrimidin-2-ylamino)-cyclohexyl]-C-(ethyl-phenyl-amino)-acetamide;
C-[cis-(4-chloro-phenyl)-ethyl-amino]-N-[4-(4-dimethylamino-pyrimidin-2-ylamino)-cyclohexyl]-acetamide;
2-(3,4-difluoro-phenyl)-N-[cis-4-(4-dimethylamino-pyrimidin-2-ylamino)-cyclohexyl]-acetamide;
4-chloro-N-[cis-4-(4-dimethylamino-pyrimidin-2-ylamino)-cyclohexyl]-3-fluoro-benzamide;
5-bromo-N-[cis-4-(4-dimethylamino-pyrimidin-2-ylamino)-cyclohexyl]-nicotinamide;
3-chloro-4-fluoro-N-[cis-4-(4-methylamino-pyrimidin-2-ylamino)-cyclohexyl]-benzamide;
N-[cis-4-(4-dimethylamino-pyrimidin-2-ylamino)-cyclohexyl]-4-fluoro-benzamide;
3-chloro-N-[cis-4-(4-dimethylamino-pyrimidin-2-ylamino)-cyclohexyl]-5-fluoro-benzamide;
N-[cis-4-(4-dimethylamino-pyrimidin-2-ylamino)-cyclohexyl]-3,4,5-trifluoro-benzamide;
N-[cis-4-(4-dimethylamino-pyrimidin-2-ylamino)-cyclohexylmethyl]-3,4-difluorobenzamide;
2-(3,4-dichloro-phenoxy)-N-[cis-4-(4-dimethylamino-pyrimidin-2-ylamino)-cyclohexyl]-acetamide;
N-[cis-4-(4-dimethylamino-pyrimidin-2-ylamino)-cyclohexyl]-2-(3-methoxy-phenoxy)-acetamide; and
N-[cis-4-(4-dimethymamino-pyrimidin-2-ylamino)-cyclohexyl]-C-(ethyl-phenyl-amino)-acetamide;

or a pharmaceutically acceptable salt, hydrate, or solvate thereof.

In some embodiments, compounds of the present invention are of Formula (I) wherein the compound is selected from the group consisting of:

3-bromo-N-(cis-4-{[4-(dimethyiamino)pyrimidin-2-yl]amino}cyclohexyl)-benzamide;
N-(cis-4-{[4-(dimethylamino)pyrimidin-2-yl]amino}cyclohexyl)-2,1,3-benzoxadiazole-5-carboxamide;
3-chloro-N-(cis-4-{[4-(dimethylamino)pyrimidin-2-yl]amino}cyclohexyl)-benzamide;
4-chloro-N-(cis-4-{[4-(dimethylamino)pyrimidin-2-yl]amino}cyclohexyl)-3-nitrobenzamide;
3,5-dichloro-N-(cis-4-{[4-(dimethylamino)pyrimidin-2-yl]amino}-cyclohexyl)benzamide;
3,4-dichloro-N-(cis-4-{[4-(dimethylamino)pyrimidin-2-yl]amino}-cyclohexyl)benzamide;
N-(cis-4-{[4-(dimethylamino)pyrimidin-2-yl]amino}cyclohexyl)-3,4-difluorobenzamide;
N-(cis-4-{[4-(dimethylamino)pyrimidin-2-yl]amino}cyclohexyl)-3-nitrobenzamide;
N-(cis-4-[4-(dimethylamino)pyrimidin-2-yl]amino}cyclohexyl)-3-(trifluoromethoxy)-benzamide;
4-bromo-N-(cis-4-{[4-(dimethylamino)pyrimidin-2-yl]amino}cyclohexyl)-3-methylbenzamide;
N-(cis-4-{[4-(dimethylamino)pyrimidin-2-yl]amino}cyclohexyl)-3-iodobenzamide;
3-chloro-N-(cis-4-{[4-(dimethylamino)pyrimidin-2-yl]amino}cyclohexyl)-4-fluorobenzamide;
N-(cis-4-{[4-(dimethylamino)pyrimidin-2-yl]amino}cyclohexyl)-3,5-dimethoxybenzamide;
N-(cis-4-[4-(dimethylamino)pyrimidin-2-yl]amino}cyclohexyl)-3,5-bis(trifluoromethyl)-benzamide;
N-(cis-4-{[4-(dimethytamino)pynmidin-2-yl]amino}cyclohexyl)-4-fluoro-3-methylbenzamide;
2-(4-chlorophenoxy)-N-(cis-4-{[(4-(dimethylamino)pyrimidin-2-yl]amino}-cyclohexyl)-acetamide;

N-(cis-4-{[4-(dimethylamino)pyrimidin-2-yl]amino}cyclohexyl)-4-fluoro-3-(trifluoromethyl)benzamide;
N-(cis-4-{[4-(dimethylamino)pyrimidin-2-yl]amino}cyclohexyl)-5-methyl-2-phenyl-2H-1,2,3-triazole-4-carboxamide;
N-(cis-4-{[4-(dimethylamino)pyrinudin-2-yl]amino}cyclohexyl)-2-(4-methoxyphenoxy)-5-nitrobenzamide;
N-(cis-4-{[4-(dimethylamino)pyrimidin-2-yl]amino{cyclohexyl)-quinoxaline-2-carboxamide;
2-(3-chlorophenoxy)-N-(cis-4-{[4-(dimethylamino)pyrimidin-2-yl]amino}-cyclohexyl)-acetamide;
3-(2,6-dichlorophenyl)-N-(cis-4-{[4-(dimethylamino)pyrimidin-2-yl]amino}cyclohexyl)-5-methylisoxazole-4-carboxamide;
N-(cis-4-{[4-(dimethylamino)pyrimidin-2-yl]amino}cyclohexyl)-2-(4-methylphenoxy)-nicotinamide;
N-(cis-4-{[4-(dimethylamino)pyrimidin-2-yl]amino}cyclohexyl)-4-[(dipropylamino)-sulfonyl]benzamide;
2-(2,3-dihydro-1-benzofuran-5-yl)-N-(cis-4-{[4-(dimethylamino)pyrimidin-2-yl]amino}-cyclohexyl)-1,3-thiazole-4-carboxamide;
N-(cis-4-{[4-(dimethylamino)pyrimidin-2-yl]amino}cyclohexyl)-2-(2-thienyl)-1,3-thiazole-4-carboxamide;
5-(4-chloro-2-nitrophenyl)-N-(cis-4-{[4-(dimethylamino)pyrimidin-2-yl]amino}cyclohexyl)-2-furamide;
N-(cis-4-{[4-(dimethylamino)pyrimidin-2-yl]amino}cyclohexyl)-3-methoxy-4-nitrobenzamide;
5-bromo-N-(cis-4-{[4-(dimethylamino)pyrimidin-2-yl]amino}cyclohexyl)-2-furamide;
5-(4-chlorophenyl)-N-(cis-4-{[4-(dimethylamino)pyrimidin-2-yl]amino}-cyclohexyl)-2-furamide;
2-(3,5-di-tert-butyl-4-hydroxyphenyl)-N-(cis-4-{[4-(dimethylamino)-pyrimidin-2-yl]-amino}cyclohexyl)acetamide;
4,5-dibromo-N-(cis-4-{[4-(dimethylamino)pyrimidin-2-yl]amino} -cyclohexyl)-2-furamide;
N-(cis-4-{[4-(dimethylamino)pyrimidin-2-yl]amino}cyclohexyl)-2-(1H-indol-3-yl)-4-oxo-4-phenylbutanamide;
N-(cis-4-{[4-(dimethylamino)pyrimidin-2-yl]amino)}cyclohexyl)-2-(1-methyl-1H-indol-3-yl)-4-(4-methylphenyl)-4-oxobutanamide;
N-(cis-4-{[4-(dimethylamino)pyrimidin-2-yl]amino}cyclohexyl)-2-(2-phenyl-1H-indol-3-yl)acetamide;
N-(cis-4-{[4-(dimethylamino)pyrimidin-2-yl]amino}cyclohexyl)-2-(ethylthio)-2,2-diphenylacetamide;
N'-(cis-4-{[4-(dimethylamino)pyrimidin-2-yl]amino}cyclohexyl)-N,N-bis[(1S)-1-phenylethyl]phthalamide;
3-(benzyloxy)-N-(cis-4-{[4-(dimethylamino)pyrimidin-2-yl]amino}-cyclohexyl)-4-methoxybenzamide;
N-(cis-4-[4-(dimethylamino)pyrimidin-2-yl]amino}cyclohexyl)-2-methyl-1,5-diphenyl-1H-pyrrole-3-carboxamide;
1-{2-[(2-chloro-6-fluorobenzyl)thio]ethyl}-N-(cis-4-{[4-(dimethylamino)-pyrimidin-2-yl]-amino}cyclohexyl)-2-methyl-5-phenyl-1H-pyrrole-3-carboxamide ;
2-[4-(4-ehlorophenyl)-2-phenyl-1,3-thiazol-5-yl]-N-(cis-4-{[4-(dimethylamino)pyrimidin-2-yl]amino}cyclohexyl) acetamide;
N-(cis-4-{[4-(dimethylamino)pyrimidin-2-yl]amino}cyclohexyl)-5-nitrothiophene-3-carboxamide;
N-(cis-4-{[4-(dimethylamino)pyrimidin-2-yl]amino}cyclohexyl)-1-methyl-4-nitro-1H-pyrrole-2-carboxamide;
3,5-di-tert-butyl-N-(cis-4-{[4-(dimethylamino)pyrimidin-2-yl]amino}-cyclohexyl)-4-hydroxybenzamide;
N-[(cis-4-{[4-(dimethylamino)pyrimidin-2-yl]amino}cyclohexyl)methyl]-2,2-diphenylacetamide;
N-[(cis-4-{[4-(dimethylamino)pyrimidin-2-yl]amino}cyclohexyl)methyl]-2-phenylbutanamide;
(2E)-N-[(cis-4-{[4-(dimethylamino)pyrimidin-2-yl] amino}cyclohexyl)-methyl]-3-(4-nitrophenyl)acrylamide;
N-[(cis-4-{[4-(dimethylamino)pyrimidin-2-yl]amino}cyclohexyl)methyl]-2-(1-naphthyl)-acetamide;
N-[(cis-4-{[4-(dimethylamino)pyrimidin-2-yl]amino}cyclohexyl)methyl]-2-(2,3,6-trichlorophenyl)acetamide;
(2E)-N-[(cis-4-{[4-(dimethylamino)pyrimidin-2-yl]amino}cyclohexyl)-methyl]-3-(3-nitrophenyl)acrylamide;
N-[(cis-4-{[4-(dimethylamino)pyrimidin-2-yl]amino}cyclohexyl)methyl]-3-oxoindane-1-carboxamide;
2,2-bis(4-chlorophenyl)-N-[(cis-4-{[4-(dimethylamino)pyrimidin-2-yl]amino}cyclohexyl)-methyl]acetamide;
N-[(cis-4-{[4-(dimethylamino)pyrimidin-2-yl]amino}cyclohexyl)methyl]-3-methyl-4-nitrobenzamide;
N-[(cis-4-{[4-(dimethylamino)pyrimidin-2-yl]amino}cyclohexyl)methyl]-3-methoxy-4-nitrobenzamide;
N-[(cis-4-{[4-(dimethylamino)pyrimidin-2-yl]amino}cyclohexyl)methyl]-2-[2-(trifluoromethoxy)phenyl]acetamide;
N-[(cis-4-{[4-(dimethylamino)pyrimidin-2-yl]amino}cyclohexyl)methyl]-9H-xanthene-9-carboxamide;
2-(1-benzothien-3-yl)-N-[(cis-4-{[4-(dimethylamino)pyrimidin-2-yl]amino}cyclohexyl)-methyl]acetamide;
N-[cis-4-(4-dimethylamino-pyrimidin-2-ylamino)-cyclohexyl]-2-(4-fluoro-phenoxy)-nicotinamide;
N-[cis-4-(4-dimethylamino-pyrimidin-2-ylamino)-cyclohexyl]-C-(ethyl-phenyl-amino)-acetamide;
C-[cis-(4-chloro-phenyl)-ethyl-amino]-N-[4-(4-dimethylamino-pyrimidin-2-ylamino)-cyclohexyl]-acetamide;
4-chloro-N-[cis-4-(4-dimethylamino-pyrimidin-2-ylamino)-cyclohexyl]-3-fluoro-benzamide;
N-[cis-4-(4-dimethylamino-pyrimidin-2-ylamino)-cyclohexyl]-3,4,5-trifluoro-benzamide;
2-(3,4-dichloro-phenoxy)-N-(cis-4-(4-dimethylamino-pyrimidin-2-ylamino)-cyclohexyl]-acetamide;
N-[cis-4-(4-dimethylamino-pyrimidin-2-ylamino)-cyclohexyl]-2-(3-methoxy-phenoxy)-acetamide; and
N-[cis-4-(4-dimethylamino-pyrimidin-2-ylamino)-cyclohexyl]-C-(ethyl-phenyl-amino)-acetamide;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- $C_{1-5}$ alkoxy carbonyl,
- $C_{1-5}$ alkylthio,
- carbocyclic aryl,
- carbocyclic aryl substituted by substituent(s) independently selected from the group consisting of:
    - •• halogen,
    - •• $C_{1-5}$ alkyl, and
    - •• $C_{2-5}$ alkenyl,

(ii) $C_{3-6}$ cycloalkyl,
$C_{3-6}$ cycloalkyl substituted by carbocyclic aryl,
(iii) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by halogen,
- $C_{1-5}$ alkoxy carbonyl,
- $C_{1-5}$ alkoxy,
- $C_{3-6}$ cycloalkoxy,
- carbocyclic aryloxy,
- $C_{1-5}$ alkylthio, and
- carbocyclic aryl,

(iv) heterocyclyl, and
heterocyclyl substituted by substituent(s) independently selected from the group consisting of:

- $C_{1-5}$ alkyl,
- $C_{1-5}$ alkyl substituted by halogen, and
- carbocyclic aryl;

L is Formula (VII);
Y is -C(O)$NR_5$-;
wherein carbocyclic aryl is phenyl or naphthyl;
heterocyclyl is 2,3-dihydro-benzo[1,4]dioxinyl, 3,4-dihydro-2*H*-benzo[b][1,4]dioxepinyl, benzo[1,3]dioxolyl, furyl, or isoxazolyl; and
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are hydrogen; A is a single bond; B is a single bond or -$CH_2$-: $R_5$ is hydrogen; or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by carbocyclic aryl,
(ii) carbocyclic aryl, and
carbocyclic aryl substituted by substituent(s) independently selected from the group consisting of:

- halogen,
- nitro,
- $C_{1-5}$ alkyl,
- $C_{1-5}$ alkyl substituted by halogen,

- $C_{1-5}$ alkoxy, and
- $C_{3-6}$ cycloalkoxy,

(iii) heterocyclyl, and
heterocyclyl substituted by $C_{1-5}$ alkyl, and
heterocyclyl substituted by carbocyclic aryl;
wherein carbocyclic aryl is phenyl or naphthyl;
heterocyclyl is isoxazolyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(cis-4-{[4-(dimethylamino)pyrimidin-2-yl]amino}cyclohexyl)-N'-mesitylurea;
N-(cis-4-{[4-(dimethylamino)pyrimidin-2-yl]amino}cyclohexyl)-N'-(2,4,6-trichlorophenyl)-urea;
N-(cis-4-{[4-(dimethylamino)pyrimidin-2-yl]amino}cyclohexyl)-N'-(2,4,6-tribromophenyl)-urea;
N-(2,4-dibromo-6-fluorophenyl)-N'-(cis-4-{[4-(dimethylamino)pyrimidin-2-yl]amino}-cyclohexyl)urea;
N-(cis-4-{[4-(dimethylamino)pyrimidin-2-yl]amino}cyclohexyl)-N'-(diphenylmethyl)urea;
N-(4-bromo-2,6-dimethylphenyl)-N'-(cis-4-{[4-(dimethylamino)pyrimidin-2-yl]amino}-cyclohexyl)urea;
N-(cis-4-{[4-(dimethylamino)pyrimidin-2-yl]amino}cyclohexyl)-N'-[1-(1-naphthyl)ethyl]-urea;
N-(cis-4-{[4-(dimethylamino)pyrimidin-2-yl]amino}cyclohexyl)-N'-(3,4,5-trimethoxyphenyl)urea;
N-(4-chloro-2-methylphenyl)-N'-(cis-4-{[4-(dimethylamino)pyrimidin-2-yl]amino}-cyclohexyl)urea;
N-(5-chloro-2,4-dimethoxyphenyl)-N'-(cis-4-{[4-(dimethylamino)-pyrimidin-2-yl]amino}-cyclohexyl)urea;
N-(4-bromo-2-methylphenyl)-N'-(cis-4-{[4-(dimethylamino)pyrimidin-2-yl]amino}-cyclohexyl)urea;
N-(2,6-dibromo-4-isopropylphenyl)-N'-(cis-4-{[4-(dimethylamino)-pyrimidin-2-yl]amino}-cyclohexyl)urea;
N-[3-(cyclopentyloxy)-4-methoxyphenyl]-N'-(cis-4-{[4-(dimethylamino)-pyrimidin-2-yl]-amino}cyclohexyl)urea;
N-[(cis-4-{[4-(dimethylamino)pyrimidin-2-yl]amino}cyclohexyl)methyl]-N'-(2,6-dimethylphenyl)urea;
N-(2,4-difluorophenyl)-N'-[(cis-4-{[4-(dimethylamino)pyrimidin-2-yl]amino}cyclohexyl)-methyl]urea;
N-[(cis-4-{[4-(dimethylamino)pyrimidin-2-yl]amino}cyclohexyl)methyl]-N'-(2-ethyl-6-methylphenyl)urea;
N-[(cis-4-{[4-(dimethylamino)pyrimidin-2-yl]amino}cyclohexyl)methyl]-N'-(4-fluorophenyl)urea;
N-[(cis-4-{[4-(dimethylamino)pyrimidin-2-yl]amino}cyclohexyl)methyl]-N'-mesitylurea;
N-[(cis-4-{[4-(dimethylamino)pyrimidin-2-yl]amino}cyclohexyl)methyl]-N'-(2,4,6-trichlorophenyl)urea;
N-[(cis-4-{[4-(dimethylamino)pyrimidin-2-yl]amino}cyclohexyl)methyl]-N'-(2,4,6-tribromophenyl)urea;
N-(2,4-dibromo-6-fluorophenyl)-N'-[(cis-4-{[4-(dimethylamino)pyrimidin-2-yl]amino}-cyclohexyl)methyl]urea;
N-(2,6-diethylphenyl)-N'-[(cis-4-{[4-(dimethylamino)pyrimidin-2-yl]amino}cyclohexyl)-methyl]urea;
N-[2-chloro-6-(trifluoromethyl)phenyl]-N'-[(cis-4-{[4-(dimethylamino)-pyrimidin-2-yl]-amino}cyclohexyl)methyl] urea;
N-(2-chloro-6-methylphenyl)-N'-[(cis-4-{[4-(dimethylamino)pyrimidin-2-yl]amino}-cyclohexyl)methyl]urea;
N-[(cis-4-{[4-(dimethylamino)pyrimidin-2-yl]amino}cyclohexyl)methyl]-N'-(2-ethyl-6-isopropylphenyl)urea;
N-[(cis-4-{[4-(dimethylamino)pyrimidin-2-yl]amino}cyclohexyl)methyl]-N'-(2-isopropyl-6-methylphenyl)urea;
N-[(cis-4-{[4-(dimethylamino)pyrimidin-2-yl]amino}cyclohexyl)methyl]-N'-(2-methyl-3-nitrophenyl)urea;
N-[(cis-4-{[4-(dimethylamino)pyrimidin-2-yl]amino}cyclohexyl)methyl]-N'-(2-propylphenyl)urea;
N-(2-tert-butyl-6-methylphenyl)-N'-[(cis-4-{[4-(dimethylamino)pyrimidin-2-yl]amino}-cyclohexyl)methyl]urea;
N-(2-tert-butylphenyl)-N'-[(cis-4-{[4-(dimethylamino)pyrimidin-2-yl]amino}cyclohexyl)-methyl]urea;
N-(3-chloro-2-methylphenyl)-N'-[(cis-4-{[4-(dimethylamino)pyrimidin-2-yl]amino}-cyclohexyl)methyl]urea;
N-(4-bromo-2,6-difluorophenyl)-N'-[(cis-4-{[4-(dimethylamino)pyrimidin-2-yl]amino}-cyclohexyl)methyl]urea;
N-[4-chloro-2-(triftuoromethyl)phenyl]-N'-[(cis-4-{[4-(dimethylamino)-pyrimidin-2-yl]-amino}cyclohexyl)methyl] urea;
N-[(cis-4-{[4-(dimethylamino)pyrimidin-2-yl]amino}cyclohexyl)methyl]-N'-(diphenylmethyl)urea;
N-(4-bromo-2,6-dimethylphenyl)-N'-[(cis-4-{[4-(dimethylamino)pyrimidin-2-yl]amino}-cyclohexyl)methyl]urea;
N-[(cis-4-{[4-(dimethylamino)pyrimidin-2-yl]amino}cyclohexyl)methyl]-N'-(3-methyl-5-phenylisoxazol-4-yl)urea;
N-(3,5-dichlorophenyl)-N'-[(cis-4-{[4-(dimethylamino)pyrimidin-2-yl]-amino}cyclohexyl)-methyl]urea;
N-(2,3-dichlorophenyl)-N'-[(cis-4-{[4-(dimethylamino)pyrimidin-2-yl]-amino}cyclohexyl)-methyl]urea;
N-(2,6-diisopropylphenyl)-N'-[(cis-4-{[4-(dimethylamino)pyrimidin-2-yl]-amino}-cyclohexyl)methyl]urea;
N-[(cis-4-{[4-(dimethylamino)pyrimidin-2-yl]amino}cyclohexyl)methyl]-N'-(2,3-dimethyl-6-nitrophenyl)urea;
N-(2,6-dibromo-4-fluorophenyl)-N'-[(cis-4-{[4-(dimethylamino)pyrimidin-2-yl]amino}-cyclohexyl)methyl]urea;
N-(2,6-dichlorophenyl)-N'-[(cis-4-{[4-(dimethylamino)pyrimidin-2-yl]-amino}cyclohexyl)-methyl]urea;

N-[(cis-4-{[4-(dimethylamino)pyrimidin-2-yl]amino}cyclohexyl)methyl]-N'-(2-methoxy-5-methylphenyl)urea;
N-[(cis-4-{[4-(dimethylamino)pyrimidin-2-yl]amino}cyclohexyl)methyl]-N'-(2-methyl-6-nitrophenyl)urea;
N-(3,4-difluorophenyl)-N'-[(cis-4-{[4-(dimethylamino)pyrimidin-2-yl]-amino}cyclohexyl)-methyl]urea;
N-(3,5-difluorophenyl)-N'-[(cis-4-{[4-(dimethylamino)pyrimidin-2-yl]-amino}cyclohexyl)-methyl]urea; and
N-(3-chloro-4-fluorophenyl)-N'-[(cis-4-{[4-(dimethylamino)pyrimidin-2-yl]amino}-cyclohexyl)methyl]urea;

or a pharmaceutically acceptable salt, hydrate, or solvate thereof.

In some embodiments of the present invention, $R_1$ is selected from the group consisting of:

(i) $C_{1-5}$ alkyl, and
$C_{1-5}$ alkyl substituted by substituent(s) independently selected from the group consisting of:

- arbocyclic aryl,
- carbocyclic aryl substituted by substituent(s) independently selected from the group consisting of:
  - •• halogen, and
  - •• $C_{1-5}$ alkoxy,

(ii) carbocyclyl,
(iii) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by halogen,
- $C_{1-5}$ alkoxy carbonyl,
- $C_{1-5}$ alkoxy,
- $C_{1-5}$ alkoxy substituted by halogen,
- mono-$C_{1-5}$ alkylamino,
- di-$C_{1-5}$ alkylamino, and
- carbocyclic aryl,

(iv) heterocyclyl, and
heterocyclyl substituted by substituent(s) independently selected from the group consisting of:

- $C_{1-5}$ alkyl,
- $C_{1-5}$ alkoxy carbonyl, and
- carbocyclic aryl;

L is Formula (VIII);
Y is $-C(S)NR_5-$;
wherein carbocyclic aryl is phenyl or naphthyl;
carbocyclyl is bicyclo[2.2.1]heptyl;
heterocyclyl is 2,3-dihydro-benzo[1,4]dioxinyl, benzo[1,3]dioxolyl, isoxazolyl, or thienyl; and
halogen is fluoro, chloro, bromo, or iodo;

or a pharmaceutically acceptable salt, hydrate, or solvate thereof.

In some embodiments of the present invention, $R_2$ is methylamino, or dimethylamino; p is 0; $R_3$ and $R_4$ are hydrogen; A is a single bond; B is a single bond or $-CH_2-$; $R_5$ is hydrogen; or a pharmaceutically acceptable salt, hydrate, or solvate thereof.

In some embodiments of the present invention, $R_1$ is selected from the group consisting of:

carbocyclic aryl, and
carbocyclic aryl substituted by substituent(s) independently selected from the group consisting of:

- halogen,

- cyano,
- $C_{1-5}$ alkyl,
- $C_{1-5}$ alkoxy,
- mono-$C_{1-5}$ alkylamino, and
- di-$C_{1-5}$ alkylamino;

wherein carbocyclic aryl is phenyl or naphthyl; and
halogen is fluoro, chloro, bromo, or iodo;

or a pharmaceutically acceptable salt, hydrate, or solvate thereof.

In some embodiments, compounds of the present invention are of Formula (I) wherein the compound is selected from the group consisting of:

N-(4-cyanophenyl)-N'-(cis-4-{[4-(dimethylamino)pyrimidin-2-yl]amino}-cyclohexyl)-thiourea;
N-(2,4-dimethoxyphenyl)-N'-(cis-4-{[4-(dimethylamino)pyrimidin-2-yl]-amino}-cyclohexyl)thiourea;
N-(cis-4-{[4-(dimethylamino)pyrimidin-2-yl]amino}cyclohexyl)-N'-(3,4,5-trimethoxyphenyl)thiourea;
N-(3,4-dimethoxyphenyl)-N'-(cis-4-{[4-(dimethylamino)pyrimidin-2-yl]-amino}-cyclohexyl)thiourea;
N-[4-(dimethylamino)-1-naphthyl]-N'-(cis-4-{[4-(dimethylamino)-pyrimidin-2-yl]amino}-cyclohexyl)thiourea;
N-(cis-4-[4-(dimethylamino)pyrimidin-2-yl]amino}cyclohexyl)-N'-(2,4,6-tribromophenyl)-thiourea;
N-(cis-4-{[4-(dimethylamino)pyrimidin-2-yl]amino}cyclohexyl)-N'-mesitylthiourea;
N-(4-bromo-2,6-dimethylphenyl)-N'-(cis-4-[4-(dimethylamino)pyrimidin-2-yl]amino}-cyclohexyl)thiourea;
N-(5-chloro-2,4-dimethoxyphenyl)-N'-(cis-4-{[4-(dimethylamino)-pyrimidin-2-yl]amino}-cyclohexyl)thiourea;
N-(2,4-dibromo-6-fluorophenyl)-N'-(cis-4-{[4-(dimethylamino)pyrimidin-2-yl]amino}-cyclohexyl)thiourea; and
N-(2,4-dichloro-6-methylphenyl)-N'-(cis-4-{[4-(dimethylamino)pyrimidin-2-yl}amino}-cyclohexyl)thiourea;

or a pharmaceutically acceptable salt, hydrate, or solvate thereof.

In some embodiments of the present invention, $R_1$ is selected from the group consisting of:

(i) $C_{1-8}$ alkyl, and
$C_{1-8}$ alkyl substituted by substituent(s) independently selected from the group consisting of:

- halogen,
- $C_{1-5}$ alkoxy,
- $C_{1-5}$ alkoxy substituted by carbocyclic aryl,
- carbocyclyl,
- carbocyclic aryl,
- carbocyclic aryl substituted by substituent(s) independently selected from the group consisting of:
    - •• halogen,
    - •• nitro, and
    - •• $C_{1-5}$ alkoxy,

(ii) $C_{2-5}$ alkenyl,
(iii) carbocyclyl,
(iv) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen, and
- $C_{1-5}$ alkoxy;

L is Formula (VII);
Y is -C(O)O-;
wherein carbocyclic aryl is phenyl or naphthyl;
carbocyclyl is 9*H*-fluorenyl or menthyl; and
halogen is fluoro, chloro, bromo, or iodo;

or a pharmaceutically acceptable salt, hydrate, or solvate thereof.

In some embodiments of the present invention, $R_2$ is methylamino or dimethylamino; p is 0; $R_3$ and $R_4$ are hydrogen; A is a single bond; B is a single bond or $-CH_2-$; or a pharmaceutically acceptable salt, hydrate, or solvate thereof.

In some embodiments of the present invention, Q is Formula (IV); p is 1 or 2;

$R_1$ is selected from the group consisting of:

(i) $C_{1-6}$ alkyl, and
$C_{1-16}$ alkyl substituted by substituent(s) independently selected from the group consisting of:

- hydroxy,
- oxo,
- carbocyclic aryloxy,
- carbocyclic aryloxy substituted by substituent(s) independently selected from the group consisting of:
  - halogen,
  - $C_{1-5}$ alkyl,
  - $C_{1-5}$ alkyl substituted by halogen, and
  - $C_{1-5}$ alkoxy,
- heterocyclyloxy,
- heterocyclyloxy substituted by substituent(s) independently selected from the group consisting of:
  - halogen,
  - $C_{1-5}$ alkyl, and
  - $C_{1-5}$ alkyl substituted by halogen,
- mono-carbocyclic arylamino,
- mono-carbocyclic arylamino substituted by substituent(s) independently selected from the group consisting of:
  - halogen,
  - $C_{1-5}$ alkoxy, and
  - $C_{1-5}$ alkyl,
- carbocyclic arylsulfinyl,
- carbocyclic arylsulfinyl substituted by substituent(s) independently selected from the group consisting of:
  - halogen,
  - $C_{1-5}$ alkyl, and
  - $C_{1-5}$ alkyl substituted by halogen,
- carbocyclic arylsulfonyl,
- carbocyclic arylsulfonyl substituted by substituent(s) independently selected from the group consisting of:
  - halogen,
  - $C_{1-5}$ alkyl, and
  - $C_{1-5}$ alkyl substituted by halogen,
- carbocyclic aryl,
- carbocyclic aryl substituted by substituent(s) independently selected from the group consisting of:
  - halogen,
  - nitro,
  - $C_{1-5}$ alkylcarbonylamino,
  - $C_{3-6}$ cycloalkylcarbonylamino,
  - $C_{1-5}$ alkyl,
  - $C_{1-5}$ alkyl substituted by halogen,
  - $C_{1-5}$ alkoxy, and

- • $C_{1-5}$ alkoxy substituted by halogen, and

- heterocyclyl,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by substitutent(s) independently selected from the group consisting of:

  - • C1-5 alkoxy,
  - • halogen,
  - • $C_{1-5}$ alkyl, and
  - • $C_{1-5}$ alkyl substituted by halogen,

(iii) carbocyclic aryl, and
carbocyclic aryl substituted by substituent(s) independently selected from the group consisting of:

- halogen,
- cyano,
- nitro,
- $C_{1-10}$ alkyl,
- $C_{1-10}$ alkyl substituted by substituent(s) independently selected from the group consisting of:

  - • halogen, and
  - • hydroxy,

- $C_{1-9}$ alkoxy,
- $C_{1-9}$ alkoxy substituted by substituent(s) independently selected from the group consisting of:

  - • halogen, and
  - • carbocyclic aryl,

- carboxy,
- $C_{1-5}$ alkoxycarbonyl,
- di-$C_{1-5}$ alkylamino,
- $C_{1-5}$ alkylcarbonylamino,
- $C_{3-6}$ cycloalkylcarbonylamino,
- $C_{1-5}$ alkylthio,
- $C_{1-5}$ alkylsulfinyl,
- $C_{1-5}$ alkylsulfonyl,
- carbocyclic aryl,

(iv) heterocyclyl, and
heterocyclyl substituted by substituent(s) independently selected from the group consisting of:

- halogen,
- hydroxy,
- amino,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by substituent(s) independently selected from the group consisting of:

  - • halogen,
  - • $C_{1-5}$ alkyl,
  - • $C_{1-5}$ alkyl substituted by halogen, and

  - •• $C_{1-5}$ alkoxy,

- heterocyclyloxy,
- heterocyclyloxy substituted by halogen,
- heterocyclyl sulfonyl,
- heterocyclyl sulfonyl substituted by $C_{1-5}$ alkyl,
- mono-carbocyclic arylamino,
- mono-carbocyclic arylamino substituted by halogen,
- $C_{1-5}$ alkylthio,
- $C_{1-5}$ alkylsulfinyl,
- carbocyclic arylsulfinyl,
- carbocyclic arylsulfinyl substituted by halogen,
- carbocyclic arylsulfonyl,
- carbocyclic arylsulfonyl substituted by substituents(s) independently selected from the group consisting of:

  - •• halogen,
  - •• $C_{1-5}$ alkoxy,
  - •• $C_{1-5}$ alkyl, and
  - •• $C_{1-5}$ alkyl substituted by halogen,

$R_2$ is selected from the group consisting of:

amino, $C_{1-5}$ alkyl, $C_{1-5}$ alkoxy, $-N(R_{2a})(R_{2b})$, wherein $R_{2a}$ is hydrogen or $C_{1-5}$ alkyl and $R_{2b}$ is $C_{1-5}$ alkyl or $C_{3-6}$ cycloalkyl;
wherein carbocyclic aryl is phenyl or naphthyl;
heterocyclyl is 3,4-dihydro-1*H*-isoquinolinyl, benzo[1,3]dioxolyl, furyl, isoxazolyl, oxazolyl, pyrazolyl, pyrazinyl, pyridyl, pyrimidyl, or thienyl; and
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-16}$ alkyl, and
$C_{1-16}$ alkyl substituted by substituent(s) independently selected from the group consisting of:

- oxo,
- carbocyclic aryl,
- carbocyclic aryl substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy, and
  - •• $C_{1-5}$ alkoxy substituted by halogen,

(ii) heterocyclyl, and
heterocyclyl substituted by substituent(s) independently selected from the group consisting of:

- carbocyclic arylsulfinyl, and
- carbocyclic arylsulfinyl substituted by halogen,

L is Formula (VII);
Y is a single bond or $-CH_2-$;
$R_2$ is $-N(R_{2a})(R_{2b})$, wherein $R_{2a}$ is $C_{1-5}$ alkyl and $R_{2b}$ is $C_{1-5}$ alkyl;
carbocyclic aryl is phenyl;
heterocyclyl is pyrazinyl; and
halogen is fluoro, chloro, or bromo;

or a pharmaceutically acceptable salt, hydrate, or solvate thereof.

In some embodiments of the present invention, $R_1$ is selected from the group consisting of:

(i) $C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:

  - •• halogen, and
  - •• $C_{1-5}$ alkoxy,

(ii) heterocyclyl, and
heterocyclyl substituted by substituent(s) independently selected from the group consisting of:

- carbocyclic arylsulfinyl, and
- carbocyclic arylsulfinyl substituted by halogen,

$R_2$ is -N($R_{2a}$)($R_{2b}$), wherein $R_{2a}$ is $C_{1-5}$ alkyl and $R_{2b}$ is $C_{1-5}$ alkyl;
carbocyclic aryl is phenyl;
heterocyclyl is pyrazinyl; and
halogen is fluoro or bromo;

or a pharmaceutically acceptable salt, hydrate, or solvate thereof.

In some embodiments of the present invention, $R_1$ is selected from the group consisting of:

heterocyclyl, and
heterocyclyl substituted by substituent(s) independently selected from the group consisting of:

- carbocyclic arylsulfinyl, and
- carbocyclic arylsulfinyl substituted by halogen,

$R_2$ is -N($R_{2a}$)($R_{2b}$), wherein $R_{2a}$ is $C_{1-5}$ alkyl and $R_{2b}$ is $C_{1-5}$ alkyl;
carbocyclic aryl is phenyl;
heterocyclyl is pyrazinyl; and
halogen is fluoro;
or a pharmaceutically acceptable salt, hydrate, or solvate thereof.

In some embodiments of the present invention, p is 1 and T is $C_{1-5}$ alkyl; $R_3$ and $R_4$ are both hydrogen; A and B are both single bonds: or a pharmaceutically acceptable salt, hydrate, or solvate thereof.

In some embodiments, compounds of the present invention are of Formula (I) wherein the compound is selected from the group consisting of:

$N^2$-{cis-4-[(3,5-dimethoxybenzyl)amino]cyclohexyl}-$N^4$,$N^4$,5-trimethylpyrimidine-2,4-diamine;
$N^2$-{cis-4-[(3-bromobenzyl)amino]cyclohexyl}-$N^4$,$N^4$,5,6-tetramethylpyrimidine-2, 4-diamine;
$N^2$-{cis-4-[(3,4-difluorobenzyl)amino]cyclohexyl}-$N^4$,$N^4$,5,6-tetramethylpyrimidine-2,4-diamine; and
$N^2$-[cis-4-({6-[(3,4-difluorophenyl)sulfinyl]pyrazin-2-yl}amino)cyclohexyl]-$N^4$,$N^4$,5-trimethylpyrimidine-2,4-diamine;

or a pharmaceutically acceptable salt, hydrate, or solvate thereof.

In some embodiments, compounds of the present invention are of Formula (I) wherein the compound is:

$N^2$-[cis-4-({6-[(3,4-difluorophenyl)sulfinyl]pyrazin-2-yl}amino)cyclohexyl]-$N^4$,$N^4$,5-trimethylpyrimidine-2,4-diamine;

or a pharmaceutically acceptable salt, hydrate, or solvate thereof.

In some embodiments of the present invention, $R_1$ is selected from the group consisting of:

(i) $C_{1-16}$ alkyl, and

$C_{1-16}$ alkyl substituted by substituent(s) independently selected from the group consisting of:

- hydroxy,
- carbocyclic aryloxy,
- carbocyclic aryloxy substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy,

- heterocyclyloxy,
- heterocyclyloxy substituted by substituent(s) independently selected from the group consisting of:

  - •• halogen,
  - •• $C_{1-5}$ alkyl, and
  - •• $C_{1-5}$ alkyl substituted by halogen,

- mono-carbocyclic arylamino,
- mono-carbocyclic arylamino substituted by substituent(s) independently selected from the group consisting of:

  - •• halogen,
  - •• $C_{1-5}$ alkoxy, and
  - •• $C_{1-5}$ alkyl,

- carbocyclic arylsulfinyl,
- carbocyclic arylsulfinyl substituted by substituent(s) independently selected from the group consisting of:

  - •• halogen,
  - •• $C_{1-5}$ alkyl, and
  - •• $C_{1-5}$ alkyl substituted by halogen,

- carbocyclic arylsulfonyl,
- carbocyclic arylsulfonyl substituted by substituent(s) independently selected from the group consisting of:

  - •• halogen,
  - •• $C_{1-5}$ alkyl, and
  - •• $C_{1-5}$ alkyl substituted by halogen,

- carbocyclic aryl,
- carbocyclic aryl substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by substitutent(s) independently selected from the group consisting of:

  - •• $C_{1-5}$ alkoxy,
  - •• halogen,
  - •• $C_{1-5}$ alkyl, and
  - •• $C_{1-5}$ alkyl substituted by halogen,

(iii) carbocyclic aryl, and
carbocyclic aryl substituted by substituent(s) independently selected from the group consisting of:

- halogen,
- cyano,
- nitro,
- $C_{1-10}$ alkyl,
- $C_{1-10}$ alkyl substituted by substituent(s) independently selected from the group consisting of:
    - •• halogen, and
    - •• hydroxy,
- $C_{1-9}$ alkoxy,
- $C_{1-9}$ alkoxy substituted by halogen,
- carboxy,
- $C_{1-5}$ alkoxycarbonyl,
- di-$C_{1-5}$ alkylamino,
- $C_{1-5}$ alkylcarbonylamino,
- $C_{3-6}$ cycloalkylcarbonylamino,
- $C_{1-5}$ alkylsulfonyl, and
- carbocyclic aryl,

(iv) heterocyclyl, and
heterocyclyl substituted by substituent(s) independently selected from the group consisting of:

- halogen,
- hydroxy,
- amino,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by substituent(s) independently selected from the group consisting of:
    - •• halogen,
    - •• $C_{1-5}$ alkyl,
    - •• $C_{1-5}$ alkyl substituted by halogen, and
    - •• $C_{1-5}$ alkoxy,
- heterocyclyloxy,
- heterocyclyloxy substituted by halogen,
- heterocyclyl sulfonyl,
- heterocyclyl sulfonyl substituted by $C_{1-5}$ alkyl,
- mono-carbocyclic arylamino,
- mono-carbocyclic arylamino substituted by halogen,
- $C_{1-5}$ alkylthio,
- $C_{1-5}$ alkylsulfinyl,
- carbocyclic arylsulfonyl,
- carbocyclic arylsulfonyl substituted by substituents(s) independently selected from the group consisting of:
    - •• halogen,
    - •• $C_{1-5}$ alkoxy,
    - •• $C_{1-5}$ alkyl, and
    - •• $C_{1-5}$ alkyl substituted by halogen,

L is Formula (VIII);
Y is -C(O)-;
$R_2$ is selected from the group consisting of:

amino, $C_{1-5}$ alkyl, $C_{1-5}$ alkoxy, $-N(R_{2a})(R_{2b})$, wherein $R_{2a}$ is hydrogen or $C_{1-5}$ alkyl and $R_{2b}$ is $C_{1-5}$ alkyl or $C_{3-6}$ cycloalkyl;

wherein carbocyclic aryl is phenyl;
heterocyclyl is benzo[1,3]dioxolyl, furyl, isoxazolyl, oxazolyl, pyrazolyl, pyrazinyl, pyridyl, pyrimidyl, or thienyl; and
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-16}$ alkyl, and
$C_{1-16}$ alkyl substituted by substituent(s) independently selected from the group consisting of:

- hydroxy,
- carbocyclic aryloxy,
- carbocyclic aryloxy substituted by substituent(s) independently selected from the group consisting of:
  - halogen,
  - $C_{1-5}$ alkyl,
  - $C_{1-5}$ alkyl substituted by halogen, and
  - $C_{1-5}$ alkoxy,
- heterocyclyloxy,
- heterocyclyloxy substituted by halogen,
- mono-carbocyclic arylamino,
- mono-carbocyclic arylamino substituted by substituent(s) independently selected from the group consisting of:
  - halogen,
  - $C_{1-5}$ alkoxy, and
  - $C_{1-5}$ alkyl,
- carbocyclic arylsulfinyl,
- carbocyclic arylsulfinyl substituted by substituent(s) independently selected from the group consisting of:
  - halogen,
  - $C_{1-5}$ alkyl, and
  - $C_{1-5}$ alkyl substituted by halogen,
- carbocyclic arylsulfonyl,
- carbocyclic arylsulfonyl substituted by substituent(s) independently selected from the group consisting of:
  - $C_{1-5}$ alkyl, and
  - $C_{1-5}$ alkyl substituted by halogen,
- carbocyclic aryl,
- carbocyclic aryl substituted by substituent(s) independently selected from the group consisting of:
  - halogen,
  - $C_{1-5}$ alkyl, and
  - $C_{1-5}$ alkyl substituted by halogen,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by substitutent(s) independently selected from the group consisting of:

- $C_{1-5}$ alkoxy,
- halogen,
- $C_{1-5}$ alkyl, and
- $C_{1-5}$ alkyl substituted by halogen,

(iii) carbocyclic aryl, and
carbocyclic aryl substituted by substituent(s) independently selected from the group consisting of:

- halogen,
- cyano,
- nitro,
- $C_{1-10}$ alkyl,
- $C_{1-10}$ alkyl substituted by substituent(s) independently selected from the group consisting of:
  - halogen, and
  - hydroxy,
- $C_{1-9}$ alkoxy,
- $C_{1-9}$ alkoxy substituted by halogen,
- carboxy,
- $C_{1-5}$ alkoxycarbonyl, and
- $C_{1-5}$ alkylsulfonyl,

(iv) heterocyclyl, and
heterocyclyl substituted by substituent(s) independently selected from the group consisting of:

- halogen,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by substituent(s) independently selected from the group consisting of:
  - halogen,
  - $C_{1-5}$ alkyl,
  - $C_{1-5}$ alkyl substituted by halogen, and
  - $C_{1-5}$ alkoxy,
- heterocyclyloxy,
- heterocyclyloxy substituted by halogen,
- heterocyclyl sulfonyl,
- heterocyclyl sulfonyl substituted by $C_{1-5}$ alkyl,
- mono-carbocyclic arylamino,
- mono-carbocyclic arylamino substituted by halogen,
- $C_{1-5}$ alkylthio,
- carbocyclic arylsulfonyl,
- carbocyclic arylsulfonyl substituted by substituents(s) independently selected from the group consisting of:
  - halogen,
  - $C_{1-5}$ alkyl, and
  - $C_{1-5}$ alkyl substituted by halogen,

$R_2$ is selected from the group consisting of:

$C_{1-5}$ alkoxy, -N($R_{2a}$)($R_{2b}$), wherein $R_{2a}$ is hydrogen or $C_{1-5}$ alkyl and $R_{2b}$ is $C_{1-5}$ alkyl;

wherein carbocyclic aryl is phenyl;
heterocyclyl is benzo[1,3]dioxolyl, furyl, isoxazolyl, oxazolyl, pyrazolyl, pyridyl, or thienyl; and

halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-16}$ alkyl, and
$C_{1-16}$ alkyl substituted by substituent(s) independently selected from the group consisting of:

- hydroxy,
- carbocyclic aryloxy,
- carbocyclic aryloxy substituted by substituent(s) independently selected from the group consisting of:
  - •• halogen,
  - •• $C_{1-5}$ alkyl,
  - •• $C_{1-5}$ alkyl substituted by halogen, and
  - •• $C_{1-5}$ alkoxy,
- heterocyclyloxy,
- heterocyclyloxy substituted by substituent(s) independently selected from the group consisting of:
  - •• halogen,
  - •• $C_{1-5}$ alkyl, and
  - •• $C_{1-5}$ alkyl substituted by halogen,
- mono-carbocyclic arylamino,
- mono-carbocyclic arylamino substituted by substituent(s) independently selected from the group consisting of:
  - •• halogen,
  - •• $C_{1-5}$ alkoxy, and
  - •• $C_{1-5}$ alkyl,
- carbocyclic arylsulfinyl,
- carbocyclic arylsulfinyl substituted by substituent(s) independently selected from the group consisting of:
  - •• halogen,
  - •• $C_{1-5}$ alkyl, and
  - •• $C_{1-5}$ alkyl substituted by halogen,
- carbocyclic aryl,
- carbocyclic aryl substituted by substituent(s) independently selected from the group consisting of:
  - •• halogen,
  - •• $C_{1-5}$ alkyl, and
  - •• $C_{1-5}$ alkyl substituted by halogen,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by substitutent(s) independently selected from the group consisting of:
  - •• $C_{1-5}$ alkoxy,
  - •• halogen,
  - •• $C_{1-5}$ alkyl, and
  - •• $C_{1-5}$ alkyl substituted by halogen,

(iii) carbocyclic aryl, and
carbocyclic aryl substituted by substituent(s) independently selected from the group consisting of:

- halogen,
- cyano,
- nitro,
- $C_{1-10}$ alkyl,
- $C_{1-10}$ alkyl substituted by halogen,
- $C_{1-9}$ alkoxy, and
- $C_{1-9}$ alkoxy substituted by halogen,

(iv) heterocyclyl, and
heterocyclyl substituted by substituent(s) independently selected from the group consisting of:

- halogen,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy,

- $C_{1-5}$ alkylthio,
- carbocyclic arylsulfonyl,
- carbocyclic arylsulfonyl substituted by halogen,

$R_2$ is selected from the group consisting of:

-N($R_{2a}$)($R_{2b}$). wherein $R_{2a}$ is hydrogen or $C_{1-5}$ alkyl and $R_{2b}$ is $C_{1-5}$ alkyl;

wherein carbocyclic aryl is phenyl;
heterocyclyl is benzo[1,3]dioxolyl, furyl, pyrazolyl, pyridyl, or thienyl; and
halogen is fluoro, chloro, bromo, or iodo;

or a pharmaceutically acceptable salt, hydrate, or solvate thereof.

In some embodiments of the present invention, p is 1 and T is $C_{1-5}$ alkyl; $R_3$ and $R_4$ are both hydrogen; A is a single bond and B is a single bond or -$CH_2$-; or a pharmaceutically acceptable salt, hydrate, or solvate thereof.

In some embodiments, compounds of the present invention are of Formula (I) wherein the compound is selected from the group consisting of:

N-[(cis-4-{[4-(dimethylamino)-5-methylpyrimidin-2-yl]amino}cyclohexyl)methyl]-3,5-bis(trifluoromethyl)benzamide;
N-[(cis-4-{[4-(dimethylamino)-6-methylpyrimidin-2-yl]amino}cyclohexyl)methyl]-3,5-bis(trifluoromethyl)benzamide;
N-[(cis-4-{[4-(dimethylamino)-6-methylpyrimidin-2-yl]amino}cyclohexyl)methyl]-3,4-difluorobenzamide;
3,5-dichloro-N-[(cis-4-{[4-(dimethylamino)-6-methylpyrimidin-2-yl]amino}cyclohexyl)-methyl]benzamide;
N-[(cis-4-{[4-(dimethylamino)-5-methylpyrimidin-2-yl]amino}cyclohexyl)methyl]-3,4-difluorobenzamide;
N-[(cis-4-{[4-(dimethylamino)-5-methylpyrimidin-2-yl]amino}cyclohexyl)methyl]-3,5-dimethoxybenzamide;
N-[(cis-4-{[4-(dimethylamino)-5-methylpyrimidin-2-yl]amino}cyclohexyl)methyl]-3-fluoro-4-methylbenzamide;
N-[(cis-4-([4-(dimethylamino)-5-methylpyrimidin-2-yl]amino}cyclohexyl)methyl]-3-(trifluoromethyl)benzamide;
N-[(cis-4-{[4-(dimethylamino)-5-methylpyrimidin-2-yl]amino}cyclohexyl)methyl]-3-(trifluoromethoxy)benzamide;
4-bromo-N-[(cis-4-{[4-(dimethylamino)-5-methylpyrimidin-2-yl]amino}cyclohexyl)-methyl]-3-methylbenzamide;
N-[(cis-4-{[4-(dimethylamino)-5-methylpyrimidin-2-yl] amino}cyclohexyl)methyl]-3-fluoro-4-(trifluoromethyl)benzamide;
3,5-dichloro-N-[(cis-4-{[4-(dimethylamino)-5-methylpyrimidin-2-yl]amino}cyclohexyl)-methyl]benzamide;
3,4-dichloro-N-[(cis-4-{[4-(dimethylamino)-5-methylpyrimidin-2-yl]amino}cyclohexyl)-methyl]benzamide;
4-chloro-N-[(cis-4-{[4-(dimethylamino)-5-methylpyrimidin-2-yl]amino}cyclohexyl)-methyl]benzamide;

4-chloro-N-[(cis-4-{[4-(dimethylamino)-6-methylpyrimidin-2-yl]amino}cyclohexyl)-methyl]benzamide;
N-[cis-4-({[4-(dimethylamino)-5-methylpyrimidin-2-yl]amino}methyl)cyclohexyl]-3,5-dimethoxybenzamide;
4-bromo-N-[cis-4-({[4-(dimethylamino)-5-methylpyrimidin-2-yl]amino}methyl)-cyclohexyl]benzamide;
4-bromo-N-[cis-4-({[4-(dimethylamino)-5-methylpyrimidin-2-yl]amino}methyl)-cyclohexyl]-3-methylbenzamide;
3,5-dichloro-N-[cis-4-({[4-(dimethylamino)-5-methylpyrimidin-2-yl]amino}methyl)-cyclohexyl]benzamide;
3,4-dichloro-N-[cis-4-({[4-(dimethylamino)-5-methylpyrimidin-2-yl]amino}methyl)-cyclohexyl]benzamide;
N-[cis-4-({[4-(dimethylamino)-5-methylpyrimidin-2-yl]amino}methyl)cyclohexyl]-3,5-bis(trifluoromethyl)benzamide;
N-[cis-4-({[4-(dimethylamino)-6-methylpyrimidin-2-yl]amino}methyl)cyclohexyl]-3,4-difluorobenzamide;
4-bromo-N-[cis-4-({[4-(dimethylamino)-6-methylpyrimidin-2-yl]amino}methyl)-cyclohexyl]benzamide;
4-bromo-N-[cis-4-({[4-(dimethylamino)-6-methylpyrimidin-2-yl]amino}methyl)-cyclohexyl]-3-methylbenzamide;
N-(cis-4-[4-{(dimethylamino)-5-methylpyrimidin-2-yl]amino}cyclohexyl)-2-(2-fluorophenoxy)nicotinamide;
N-(cis-4-{[4-(dimethylamino)-6-methylpyrimidin-2-yl]amino}cyclohexyl)-3,4,5-trimethoxybenzamide;
N-(4-{[4-(dimethylamino)-6-methylpyrimidin-2-yl]amino}cyclohexyl)-3-nitrobenzamide;
N-(cis-4-{[4-(dimethylamino)-6-methylpyrimidin-2-yl]amino}cyclohexyl)-2,2-diphenylacetamide;
N-(cis-4-{[4-(dimethylamino)-6-methylpyrimidin-2-yl]amino}cyclohexyl)-4-methylbenzamide;
4-chloro-N-(cis-4-{[4-(dimethylamino)-6-methylpyrimidin-2-yl]amino}cyclohexyl)-benzamide;
3-chloro-N-(cis-4-{[4-(dimethylamino)-6-methylpyrimidin-2-yl]amino}cyclohexyl)-benzamide;
N-(cis-4-{[4-(dimethylamino)-6-methylpyrimidin-2-yl]amino}cyclohexyl)-3,4-difluorobenzamide;
N-(cis-4-{[4-(dimethylamino)-6-methylpyrimidin-2-yl]amino}cyclohexyl)-3-methylbenzamide;
N-(cis-4-{[4-(dimethylamino)-6-methylpyrimidin-2-yl]amino}cyclohexyl)-3-methoxybenzamide;
N-(cis-4-{[4-{dimethylamino)-6-methylpyrimidin-2-yl]amino}cyclohexyl)-4-fluorobenzamide;
N-(cis-4-{[4-(dimethylamino)-5-methylpyrimidin-2-yl]amino}cyclohexyl)-3-methylbenzamide;
N-(cis-4-{[4-(dimethylamino)-5-methylpyrimidin-2-yl]amino}cyclohexyl)-3-methoxybenzamide;
N-(cis-4-{[4-(dimethylamino)-5-methylpyrimidin-2-yl]amino}cyclohexyl)-4-methylbenzamide;
N-(cis-4-{([4-(dimethylamino)-5-methylpyrimidin-2-yl]amino}cyclohexyl)-3,4-difluorobenzamide;
3-chloro-N-(cis-4-{[4-(dimethylamino-5-methylpyrimidin-2-yl]amino}cyclohexyl)-benzamide;
N-(cis-4-{[4-(dimethylamino)-5-methylpyrimidin-2-yl]amino}cyclohexyl)-2-(3-methylphenoxy)nicotinamide;
2-(4-bromophenoxy)-N-(cis-4-{[4-(dimethylamino)-5-methylpyrimidin-2-yl]amino}-cyclohexyl)nicotinamide;
2-(4-chlorophenoxy)-N-(cis-4-{[4-(dimethylamino)-5-methylpyrimidin-2-yl]amino}-cyclohexyl)nicotinamide;
N-(cis-4-{[4-(dimethylamino)-5-methylpyrimidin-2-yl]amino}cyclohexyl)-2-(4-fluorophenoxy)nicotinamide;
N-(cis-4-{[4-(dimethylamino)-5-methylpyrimidin-2-yl]amino}cyclohexyl)-2-(3-fluorophenoxy)nicotinamide;
2-(2-bromophenoxy)-N-(cis-4-{[4-(dimethylamino)-5-methylpyrimidin-2-yl]amino}-cyclohexyl)nicotinamide;
N-(cis-4-{[4-(dimethylamino)-5-methylpyrimidin-2-yl]amino}cyclohexyl)-2-(3-methoxyphenoxy)nicotinamide;
N-(cis-4-{[4-(dimethylamino)-5-methylpyrimidin-2-yl]amino}cyclohexyl)-2-(4-methoxyphenoxy)nicotinamide;
N-(cis-4-{[4-(dimethylamino)-5-methylpyrimidin-2-yl]amino}cyclohexyl)-2-(4-iodophenoxy)nicotinamide;
2-(3,4-dichlorophenoxy)-N-(cis-4-{[5-methyl-4-(methylamino)pyrimidin-2-yl]amino}-cyclohexyl)acetamide;
2-(2,3-dichlorophenoxy)-N-(cis-4-{[5-methyl-4-(methylamino)pyrimidin-2-yl]amino}-cyclohexyl)acetamide;
2-[(3,4-difluorophenyl)sulfonyl]-N-(cis-4-{[4-(dimethylamino)-5-methylpyrimidin-2-yl]-amino}cyclohexyl)nicotinamide;
N-(cis-4-{[4-(dimethylamino)-5-ethylpyrimidin-2-yl]amino}cyclohexyl)-3,4-difluorobenzamide;
N-[cis-4-({4-[ethyl(methyl)amino]-5-methylpyrimidin-2-yl amino}cyclohexyl]-3,4-difluorobenzamide;
N-(cis-4-{[4-(dimethylamino)-6-methylpyrimidin-2-yl]amino}cyclohexyl)-3,5-dimethoxybenzamide;
N-(cis-4-{[4-(dimethylamino)-5-methylpyrimidin-2-yl]amino}cyclohexyl]-2-(2-methoxyphenoxy)nicotinamide;
2-(2-chlorophenoxy)-N-(cis-4-{[4-(dimethylamino)-5-methylpyrimidin-2-yl]amino}-cyclohexyl)nicotinamide;
2-(3-chlorophenoxy)-N-(cis-4-{[4-(dimethylamino)-5-methylpyrimidin-2-yl]amino}-cyclohexyl)nicotinamide;
2-(3-bromophenoxy)-N-(cis-4-{[4-(dimethylamino)-5-methylpyrimidin-2-yl]amino}-cyclohexyl)nicotinamide;
N-(cis-4-{[4-(dimethylamino)-5-methylpyrimidin-2-yl]amino}cyclohexyl)-2-[3-(trifluoromethyl)phenoxy]nicotinamide;
N-(cis-4-{[4-(dimethylamino)-5-methylpyrimidin-2-yl]amino}cyclohexyl)-2-(3-fluorophenoxy)acetamide;
N-(cis-4-{[4-(dimethylamino)-5-methylpyrimidin-2-yl]amino}cyclohexyl)-2-(3-methoxyphenoxy)acetamide;
N-(ds-4-{[4-(dmiethylamino)-5-methylpyrimidin-2-yl]amino}cyclohexyl)-2-[3-(trifluoromethyl)phenoxy]acetamide;
2-(3-chlorophenoxy)-N-(cis-4-{[4-(dimethylamino)-5-methylpyrimidin-2-yl]amino}-cyclohexyl)acetamide;
2-[(5-chloropyridin-3-yl)oxy]-N-(cis-4-{[4-(dimethylamino)-5-methylpyrimidin-2-yl]-amino}cyclohexyl)acetamide;
N-(cis-4-{(4-(dimethylamino)-5,6-dimethylpyrimidin-2-yl]amino}cyclohexyl)-3,4-difluorobenzamide;
2-(3,4-difluorophenyl)-N-(cis-4-{[4-(dimethylamino)-5-methylpyrimidin-2-yl]amino}-cyclohexyl)-2-hydroxyacetamide;
N-(cis-4-{[4-(dimethylamino)-5-methylpyrimidin-2-yl]amino}cyclohexyl)-2-hydroxy-2-(4-methoxyphenyl)acetamide;
2-(2,3-difluorophenyl)-N-(cis-4-{[4-(dimethylamino)-5-methylpyrimidin-2-yl]amino}-cyclohexyl)-2-hydroxyacetamide;
N-(cis-4-{[4-(dimethylamino)-5-methylpyrimidin-2-yl]amino}cyclohexyl)-2-hydroxy-2-[3-(trifluoromethyl)phenyl] acetamide;
N-(cis-4-{[4-(dimethylamino)-5-methylpyrimidin-2-yl]amino}cyclohexyl)-2-{[2-(trifluoromethyl)phenyl]sulfinyl} acetamide;
2-[(2-chlorophenyl)sulfinyl]-N-(cis-4-{[4-(dimethylamino)-5-methylpyrimidin-2-yl]amino}-cyclohexyl)acetamide;
2-[(3-bromophenyl)sulfinyl]-N-(cis-4-{[4-(dimethylamino)-5-methylpyrimidin-2-yl]amino}-cyclohexyl)acetamide;
2-[(3,4-difluorophenyl)sulfinyl]-N-(cis-4-{[4-(dimethylamino)-5-methylpyrimidin-2-yl]-amino}cyclohexyl)acetamide;
N-(cis-4-{(4-(dimethylamino)-5-methylpyrimidin-2-yl]amino}cyclohexyl)-3-(trifluoromethyl)benzamide;
N-(cis-4-{[4-(dimethylamino)-5-methylpyrimidin-2-yl]amino}cyclohexyl)-3-fluorobenzamide;
1-bromo-N-(cis-4-{[4-(dimethylamino)-5-methylpyrimidin-2-yl]amino}cyclohexyl) benzamide;
N-(cis-4-{[4-(dimethylamino)-5-methylpyrimidin-2-yl]amino}cyclohexyl)-4-(trifluoromethoxy)benzamide;
N-(cis-4-{[4-(dimethylamino)-5-methylpyrimidin-2-yl]amino}cyclohexyl)-4-fluorobenzamide;
3,4-dichloro-N-(cis-4-{[4-(dimethylamino)-5-methylpyrimidin-2-yl]amino}cyclohexyl)-benzamide;
N-(cis-4-{[4-(dimethylamino)-5-methylpyrimidin-2-yl]amino}cyclohexyl)-3,5-bis(trifluoromethyl)benzamide;
N-(cis-4-{[4-(dimethylamino)-5-methylpyrimidin-2-yl]amino}cyclohexyl)-3,5-dimethoxybenzamide;
N-(cis-4-{(4-(dimethylamino)-5-methylpyrimidin-2-yl]amino}cyclohexyl)-2,4-difluorobenzamide;
N-(cis-4-{[4-(dimethylamino)-5-methylpyrimidin-2-yl]amino}cyclohexyl)-2,5-difluorobenzamide;
N-(cis-4-{[4-(dimethylamino)-5-methylpyrimidin-2-yl]amino}cyclohexyl)-2,3,4-trifluorobenzamide;
4-chloro-N-(cis-4-{[4-(dimethylamino)-5-methylpyrimidin-2-yl]amino}cyclohexyl)-benzamide;
3-cyano-N-(cis-4-{[4-(dimethylamino)-5-methylpyrimidin-2-yl]amino}cyclohexyl)-benzamide;
4-cyano-N-(cis-4-{[4-(dimethylamino)-5-methylpyrimidin-2-yl]amino}cyclohexyl)-benzamide;
2-[(3,4-dichlorophenyl)sulfinyl]-N-(cis-4-{(4-(dimethylamino)-5-methylpyrimidin-2-yl]-amino}cyclohexyl)acetamide;
N-(cis-4-{[4-(dimethylamino)-5-methylpyrimidin-2-yl]amino}cyclohexyl)-2-{[3-(trifluoromethyl)phenyl]sulfinyl} acetamide;
N-(cis-4-{[4-(dimethylamino)-5-methylpyrimidin-2-yl]amino}cyclohexyl)-2-{[3-(trifluoromethyl)phenyl}sulfonyl acetamide;
N-(cis-4-{[4-(dimethylamino)-5-methylpyrimidin-2-yl]amino}cyclohexyl)-2-(isopropylthio)nicotinamide;
2-(tert-butylthio)-N-(cis-4-{[4-(dimethylamino)-5-methylpyrimidin-2-yl]amino}-cyclohexyl)nicotinamide;
N-(cis-4-{[4-(dimethylamino)-5-methylpyrimidin-2-yl]amino}cyclohexyl)-2-(propylthio)-nicotinamide;
N-(cis-4-{[4-(dimethylamino)-5-methylpyrimidin-2-yl] amino}cyclohexyl)-3-(methylsulfonyl)benzamide;
N-(cis-4-{[4-(dimethylamino)-6-methylpyrimidin-2-yl]amino}cyclohexyl)-3-fluorobenzamide;
N-(cis-4-{[4-(dimethylamino)-6-methylpyrimidin-2-yl]amino}cyclohexyl)-3-(trifluoromethyl)benzamide;
3-cyano-N-(cis-4-{[4-(dimethylamino)-6-methylpyrimidin-2-yl]amino}cyclohexyl)-benzamide;
4-cyano-N-(cis-4-{[4-(dimethylamino)-6-methylpyrimidin-2-yl]amino}cyclohexyl)-benzamide;
N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-3-(trifluoromethyl)benzamide;
3-cyano-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-3-methylbenzamide;
3-chloro-N-(cis-4-{[4-(dimethylamino)-5,6-dimethylpyrimidin-2-yl]amino}cyclohexyl)-benzamide;
3-bromo-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-3,5-dimethoxybenzamide;
N-(cis-4-{[4-(dimethylamino)-5,6-dimethylpyrimidin-2-yl]amino}cyclohexyl)-3,5-bis(trifluoromethyl)benzamide;
3,4-dichloro-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-4-(trifluoromethoxy)benzamide;
4-cyano-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-4-methylbenzamide;
N-(cis-4-{[4-(dimethylamino)-5,6-dimethylpyrimidin-2-yl]amino}cyclohexyl)-4-fluorobenzamide;
4-chloro-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-2-methoxybenzamide;
4-bromo-N-(cis-4-{[4-(dimethylamino)-5-methylpyrimidin-2-yl]amino}cyclohexyl)-benzamide;
N-(cis-4-{[4-(dimethylamino)-5-methylpyrimidin-2-yl]amino}cyclohexyl)-4-(trifluoromethyl)benzamide;
N-(cis-4-{[4-(dimethylamino)-5,6-dimethylpyrimidin-2-yl]amino}cyclohexyl)-3-methoxybenzamide;
5-bromo-N-(cis-4-{[4-(dimethylamino)-5,6-dimethylpyrimidin-2-yl]amino}cyclohexyl)-2-furamide;

N-(cis-4-{[4-(dimethylamino)-5,6-dimethylpyrimidin-2-yl]amino}cyclohexyl)-5-methylisoxazole-3-carboxamide;
2-(3,5-difluorophenyl)-N-(cis-4-{[4-(dimethylamino)-5,6-dimethylpyrimidin-2-yl]amino}-cyclohexyl)-2-hydroxyacetamide;
N-(cis-4-{[4-(dimethylamino)-5,6-dimethylpyrimidin-2-yl]amino}cyclohexyl)-2-methyl-1,3-oxazole-4-carboxamide;
N-(cis-4-{[4-(dimethylamino)-5,6-dimethylpyrimidin-2-yl]amino}cyclohexyl)-2,6-dimethoxynicotinamide;
4-bromo-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-4-(trifluoromethyl)benzamide;
4-bromo-N-(cis-4-{[4-(dimethylamino)-5,6-dimethylpyrimidin-2-yl]amino}cyclohexyl)-3-methylbenzamide;
N-(cis-4-{[4-(dimethylamino)-5,6-dimethylpyrimidin-2-yl]amino}cyclohexyl)-3-fluoro-4-methylbenzamide;
N-(cis-4-{[4-(dimethylamino)-5,6-dimethylpyrimidin-2-yl]amino}cyclohexyl)-4-fluoro-3-methylbenzamide;
N-(cis-4-{[4-(dimethylamino)-5,6-dimethylpyrimidin-2-yl]amino}cyclohexyl)-3-ethylbenzamide;
N-(cis-4-{[4-(dimethylamino)-5,6-dimethylpyrimidin-2-yl]amino}cyclohexyl)-3-(trifluoromethoxy)benzamide;
5-bromo-N-(cis-4-{[4-(dimethylamino)-5,6-dimethylpyrimidin-2-yl]amino}cyclohexyl)-nicotinamide;
N-(cis-4-{[4-(dimethylamino)-5,6-dimethylpyrimidin-2-yl]amino}cyclohexyl)-5-methylthiophene-2-carboxamide
N-(cis-4-{[4-(dimethylamino)-5,6-dimethylpyrimidin-2-yl]amino}cyclohexyl)-6-(trifluoromethyl)nicotinamide;
N-(cis-4-{[4-(dimethylamino)-5,6-dimethylpyrimidin-2-yl]amino}cyclohexyl)-3,5-diethoxybenzamide;
N-(cis-4-{[4-(dimethylamino)-5,6-climethylpyrimidin-2-yl]amino}cyclohexyl)-3-ethoxybenzamide;
N-(cis-4-{[4-(dimethylamino)-5,6-dimethylpyrimidin-2-yl]amino}cyclohexyl)-3-isopropoxybenzamide;
3,5-dichloro-N-(cis-4-{[4-(dimethylamino)-5-methylpyrimidin-2-yl]amino}cyclohexyl)-benzamide;
4-bromo-N-(cis-4-{[4-(dimethylamino)-5-methylpyrimidin-2-yl]amino}cyclohexyl)-3-methylbenzamide;
N-(cis-4-{[4-(dimethylamino)-5-methylpyrimidin-2-yl]amino}cyclohexyl)-4-ethoxybenzamide;
N-(cis-4-{[4-(dimethylamino)-5-methylpyrimidin-2-yl]amino}cyclohexyl)-4-fluoro-3-methylbenzamide;
N-(cis-4-{[4-(dimethylamino)-5-methylpyrimidin-2-yl]amino}cyclohexyl)-3-fluoro-4-methylbenzamide;
N-(cis-4-{[4-(dimethylamino)-5-methylpyrimidin-2-yl]amino}cyclohexyl)-3-ethylbenzamide;
N-(cis-4-{[4-(dimethylamino)-6-methylpyrimidin-2-yl]amino}cyclohexyl)-3,5-bis(trifluoromethyl)benzamide;
N-(cis-4-{[4-(dimethylamino)-6-methylpyrimidin-2-yl]amino}cyclohexyl)-3-fluoro-4-(trifluoromethyl)benzamide;
N-(cis-4-{[4-(dimethylamino)-6-methylpyrimidin-2-yl]amino}cyclohexyl)-3-fluoro-5-(trifluoromethyl)benzamide;
3-chloro-N-(cis-4-{[4-(dimethylamino)-6-methylpyrimidin-2-yl]amino}cyclohexyl)-4-fluorobenzamide;
N-(cis-4-{[4-(dimethylamino)-6-methylpyrimidin-2-yl]amino}cyclohexyl)-4-fluoro-3-methylbenzamide;
N-(cis-4-{[4-(dimethylamino)-6-methylpyrimidin-2-yl]amino}cyclohexyl)-3-fluoro-4-methylbenzamide;
3,5-dichloro-N-(cis-4-{[4-(dimethylamino)-6-methylpyrimidin-2-yl]amino}cyclohexyl)-benzamide;
N-(cis-4-{[4-(dimethylamino)-6-methylpyrimidin-2-yl]amino}cyclohexyl)-3-(trifluoromethoxy)benzamide;
N-(cis-4-{[4-(dimethylamino}-6-methylpyrimidin-2-yl]amino}cyclohexyl)-3,5-difluorobenzamide;
4-bromo-N-(cis-4-{[4-(dimethylarnino)-6-methylpyrimidin-2-yl]amino}cyclohexyl)-3-methylbenzamide;
N-(cis-4-{[4-(dimethylamino)-6-methylpyrimidin-2-yl]amino}cyclohexyl)-3-ethylbenzamide;
N-(cis-4-{[4-(dimethylamino)-6-methylpyrimidin-2-yl]amino}cyclohexyl)-4-(trifluoromethyl)benzamide;
4-bromo-N-(cis-4-{[4-(dimethylamino)-6-methylpyrimidin-2-yl]amino}cyclohexyl)-benzamide;
N-(cis-4-{[4-(dimethylamino)-6-methylpyrimidin-2-yl]amino}cyclohexyl)-4-ethylbenzamide;
N-(cis-4-{[4-(dimethylamino)-6-methylpyrimidin-2-yl]amino}cyclohexyl)-3,5-diethoxybenzamide;
N-(cis-4-{[4-(dimethylamino)-6-methylpyrimidin-2-yl]amino}cyclohexyl)-3-ethoxybenzamide;
N-(cis-4-{[4-(dimethylamino)-6-methylpyrimidin-2-yl]amino}cyclohexyl)-3-isopropoxybenzamide;
5-bromo-N-(cis-4-{[4-(dimethylamino)-6-methylpyrimidin-2-yl]amino}cyclohexyl)-nicotinamide;
5-bromo-N-(cis-4-{[4-(dimethylamino)-6-methylpyrimidin-2-yl]amino}cyclohexyl)-2-furamide;
5-chloro-N-(cis-4-{[4-(dimethylamino)-6-methylpyrimidin-2-yl]amino}cyclohexyl)-2-furamide;
N-(cis-4-{[4-(dimethylamino)-5-methylpyrimidin-2-yl]amino}cyclohexyl)-3-fluoro-5-(trifluoromethyl)benzamide;
N-(cis-4-{[4-(dimethylamino)-5-methylpyrimidin-2-yl]amino}cyclohexyl)-2,2-difluoro-1,3-benzodioxole-5-carboxamide;
N-(cis-4-{[4-(dimethylamino)-5-methylpyrimidin-2-yl]amino}cyclohexyl)-3-ethoxybenzamide;
N-(cis-4-{[4-(dimethylamino)-5-methylpyrimidin-2-yl]amino}cyclohexyl)-3-isopropoxybenzamide;
5-bromo-N-(cis-4-{[4-(dimethylamino)-5-methylpyrimidin-2-yl]amino}cyclohexyl)-2-furamide;
N-(cis-4-{[4-(dimethylamino)-5-methylpyrimidin-2-yl]amino}cyclohexyl)-3,5-diethoxybenzamide;
4-chloro-N-(cis-4-{[4-(dimethylamino)-5-methylpyrimidin-2-yl]amino}cyclohexyl)-3-(trifluoromethyl)benzamide;
5-bromo-N-(cis-4-{[4-(dimethylamino)-5-methylpyrimidin-2-yl]amino}cyclohexyl)-nicotinamide;
3,4-dichloro-N-(cis-4-{[4-(dimethylamino)-6-methylpyrimidin-2-yl]amino}cyclohexyl)-benzamide;
3-chloro-N-(cis-4-{[4-(dimethylamino)-5-methylpyrimidin-2-yl]amino}cyclohexyl)-4-(trifluoromethoxy)benzamide;
N-(cis-4-{[4-(dimethylamino)-5-methylpyrimidin-2-yl]amino}cyclohexyl)-4-methoxy-3-(trifluoromethyl)benzamide;
N-(cis-4-{[4-(dimethylamino)-6-methylpyrimidin-2-yl]amino}cyclohexyl)-4-methoxy-3-(trifluoromethyl)benzamide;
2-(4-chlorophenyl)-N-(cis-4-{[4-(dimethylamino)-5-methylpyrimidin-2-yl}amino]-cyclohexyl)-2-methylpropanamide;
1-(4-chlorophenyl)-N-(cis-4-{[4-(dimethylamino)-5-methylpyrimidin-2-yl]amino}-cyclohexyl)cyclopropanecarboxamide;
1-(4-chlorophenyl)-N-(cis-4-{[4-(dimethylamino)-5-methylpyrimidin-2-yl]amino}-cyclohexyl)cyclobutanecarboxamide;
1-(2,4-dichlorophenyl)-N-(cis-4-{[4-(dimethylamino)-5-methylpyrimidin-2-yl]amino}-cyclohexyl)cyclopropanecarboxamide;
2-(4-chlorophenyl)-N-(cis-4-{[4-(dimethylamino)-6-methylpyrimidin-2-yl]amino}-cyclohexyl)-2-methylpropanamide;
1-(4-chlorophenyl)-N-(cis-4-{[4-(dimethylamino)-6-methylpyrimidin-2-yl]amino}-cyclohexyl)cyclopropanecarboxamide;
1-(4-chlorophenyl)-N-(cis-4-{[4-(dimethylamino)-6-methylpyrimidin-2-yl]amino}-cyclohexyl)cyclobutanecarboxamide;
1-(2,4-dichlorophenyl)-N-(cis-4-{[4-(dimethylamino)-6-methylpyrimidin-2-yl]amino}-cyclohexyl)cyclopropanecarboxamide;
2-[3,5-bis(trifluoromethyl)phenyl]-N-(cis-4-{[4-(dimethylamino)-6-methylpyrimidin-2-yl]-amino}cyclohexyl)acetamide;
N-(cis-4-{[4-(dimethylamino)-5-methylpyrimidin-2-yl]amino}cyclohexyl)-4-[2,2,2-trifluoro-1-hydroxy-1-(trifluoromethyl)ethyl]benzamide;
2-(4-chlorophenyl)-N-(cis-4-{(4-(dimethylamino)-5-methylpyrimidin-2-yl]amino}-cyclohexyl)acetamide;
N-(cis-4-{[4-(dimethylamino)-5-methylpyrimidin-2-yl]amino}cyclohexyl)-1-(4-methylphenyl)cyclopropanecarboxamide;
2-(4-chiorophenyl)-N-(cis-4-{[4-(dimethylamino)-5-methylpyrimidin-2-yl]amino}-cyclohexyl)propanamide;
2-(4-chtorophenyl)-N-(cis-4-{[4-(dimethylamino)-5-methylpyrimidin-2-yl]amino}-cyclohexyl)-2-hydroxyacetamide;
N-(cis-4-{[4-(dimethylamino)-5-methylpyrimidin-2-yl]amino}cyclohexyl)-1-(4-methoxyphenyl)cyclopropanecarboxamide;
$N^2$-(3-chlorophenyl)-N-(cis-4-{[4-(dimethylamino)-5-methylpyrimidin-2-yl]amino}-cyclohexyl)-$N^2$-methylglycinamide;
$N^2$-(3,4-dichlorophenyl)-N-(cis-4-{[4-(dimethylamino)-5-methylpyrimidin-2-yl]amino}-cyclohexyl)-$N^2$-methylglycinamide;
N-(cis-4-{[4-(dimethylamino)-5-methylpyrimidin-2-yl]amino}cyclohexyl)-$N^2$-methyl-$N^2$-(3-methylphenyl)glycinamide;
N-(cis-4-{[4-(dimethylamino)-5-methylpyrimidin-2-yl]amino}cyclohexyl)-$N^2$-(3-fluorophenyl)-$N^2$-methylglycinamide;
N-(cis-4-{[4-(dimethylamino)-5-methylpyrimidin-2-yl]amino}cyclohexyl)-$N^2$-(4-fluorophenyl)-$N^2$-methylglycinamide;
$N^2$-(4-chlorophenyl)-N-(cis-4-{[4-(dimethylamino)-5-methylpyrimidin-2-yl]amino}-cyclohexyl)-$N^2$-methylglycinamide;
$N^2$-(3,4-difluorophenyl)-N-(cis-4-{[4-(dimethylamino)-5-methylpyrimidin-2-yl]amino}-cyclohexyl)-$N^2$-methylglycinamide;
N-(cis-4-{[4-(dimethylamino)-5-methylpyrimidin-2-yl]amino}cyclohexyl)-$N^2$-(3--methoxyphenyl)-$N^2$-methylglycinamide;
N-(cis-4-{[4-(dimethylamino)-5-methylpyrimidin-2-yl]amino}cyclohexyl)-$N^2$-(4-methoxyphenyl)-$N^2$-methylglycinamide;
2-[(3,4-difluorophenyl)amino]-N-(cis-4-{[4-(dimethylamino)-5-methylpyrimidin-2-yl]-amino}cyclohexyl)nicotinamide;
2-(3,4-dichlorophenoxy)-N-(cis-4-{[4-methyl-6-(methylamino)pyrimidin-2-yl]amino}-cyclohexyl)acetamide;
trans-2-(4-chlorophenyl)-N-(cis-4-{[4-(dimethylamino)-5-methylpyrimidin-2-yl]amino}-cyclohexyl)cyclopropanecarboxamide;
trans-2-(3-chlorophenyl)-N-(cis-4-{[4-(dimethylamino)-5-methylpyrimidin-2-yl]amino}-cyclohexyl)cyclopropanecarboxamide;
trans-2-(3,4-difluorophenyl)-N-(cis-4-{[4-(dimethylamino)-5-methylpyrimidin-2-yl]amino}-cyclohexyl)cyclopropanecarboxamide;
trans-2-(3,4-dichlorophenyl)-N-(cis-4-{[4-(dimethylamino)-5-methylpyrimidin-2-yl]-amino}cyclohexyl)cyclopropanecarboxamide;
trans-2-[3,5-bis(trifluoromethyl)phenyl]-N-(cis-4-{[4-(dimethylamino)-5-methylpyrimidin-2-yl]amino}cyclohexyl)cyclopropanecarboxamide;
2-[(4-chlorophenyl)sulfonyl]-N-(cis-4-{[4-(dimethylamino)-5-methylpyrimidin-2-yl]-amino}cyclohexyl)nicotinamide;

2-[(3-chlorophenyl)sulfonyl]-N-(cis-4-{[4-(dimethylamino)-5-methylpyrimidin-2-yl]-amino}cyclohexyl)nicotinamide;
2-[(4-bromophenyl)sulfonyl]-N-(cis-4-{[(4-(dimethylamino)-5-methylpyrimidin-2-yl]-amino}cyclohexyl)nicotinamide;
N-(cis-4-{[4-(dimethylamino)-5-methylpyrimidin-2-yl]amino}cyclohexyl)-2-{[4-(trifluoromethyl)phenyl]sulfonyl} nicotinamide;
N-(cis-4-{[4-(dimethylamino)-5-methylpyrimidin-2-yl]amino}cyclohexyl)-2-{[1-methyl-3-(trifluoromethyl)-1H-pyrazol-5-yl]oxy]acetamide;
2-[(2-chlorophenyl)sulfonyl]-N-(cis-4-{[4-(dimethylamino)-6-methylpyrimidin-2-yl]-amino}cyclohexyl)nicotinamide;
2-[(3-chlorophenyl)sulfonyl]-N-(cis-4-{[4-(dimethylamino)-6-methylpyrimidin-2-yl]-amino}cyclohexyl)nicotinamide;
3,4-dichloro-N-{cis-4-[(4-methoxy-5-methylpyrimidin-2-yl)amino]cyclohexyl}-benzamide;
N-[cis-4-(4-dimethylamino-5-methyl-pyrimidin-2-ylamino)-cyclohexyl]-2-phenoxy-nicotinamide;
N-[cis-4-(4-dimethylamino-6-methyl-pyrimidin-2-ylamino)-cyclohexyl]-2-phenoxy-nicotinamide;
3-chloro-N-[cis-4-(4-dimethylamino-5-methyl-pyrimidin-2-ylamino)-cyclohexyl]-4-fluoro-benzamide;
4-chloro-N-[cis-4-(4-dimethylamino-6-methyl-pyrimidin-2-ylamino)-cyclohexyl]-3-fluoro-benzamide;
3-chloro-N-[cis-4-(4-dimethylamino-6-methyl-pyrimidin-2-ylamino)-cyclohexyl]-5-fluoro-benzamide;
N-[cis-4-(4-dimethylamino-6-methyl-pyrimidin-2-ylamino)-cyclohexyl]-3,4,5-trifluoro-benzamide;
3-chloro-4-fluoro-N-[cis-4-(5-methyl-4-methylamino-pyrimidin-2-ylamino)-cyclohexyl]-benzamide;
4-chloro-N-[cis-4-(4-dimethylamino-5-methyl-pyrimidin-2-ylamino)-cyclohexyl]-3-fluoro-benzamide;
3-chloro-N-[cis-4-(4-dimethylamino-5-methyl-pyrimidin-2-ylamino)-cyclohexyl]-5-fluoro-benzamide;
N-[cis-4-(4-dimethylamino-5-methyl-pyrimidin-2-ylamino)-cyclohexyl]-3,4,5-trifluoro-benzamide;
N-[cis-4-(4-dimethylamino-5-methyl-pyrimidin-2-ylamino)-cyclohexyl]-3,5-difluorobenzamide; and
2-(3,4-difluoro-phenyl)-N-[cis-4-(4-dimethylamino-5-methyl-pyrimidin-2-ylamino}-cyclohexyl]-acetamide;

or a pharmaceutically acceptable salt, hydrate, or solvate thereof.

In some embodiments, compounds of the present invention are of Formula (I) wherein the compound is selected from the group consisting of:

N-[(cis-4-{[4-(dimethylamino)-5-methylpyrimidin-2-yl]amino}cyclohexyl)methyl]-3,5-bis(trifluoromethyl)benzamide;
N-[(cis-4-{[4-(dimethylamino)-5-methylpyrimidin-2-yl]amino}cyclohexyl)methyl]-3,5-dimethoxybenzamide;
N-[(cis-4-{[4-(dimethylamino)-5-methylpyrimidin-2-yl]amino}cyclohexyl)methyl]-3-(trifluoromethyl)benzamide;
4-bromo-N-[(cis-4-{[4-(dimethylamino)-5-methylpyrimidin-2-yl]amino}cyclohexyl)-methyl]-3-methylbenzamide;
3,5-dichloro-N-[(cis-4-{[4-(dimethylamino)-5-methylpyrimidin-2-yl]amino}cyclohexyl)-methyl]benzamide;
3,4-dichloro-N-[(cis-4-{[4-(dimethylamino)-5-methylpyrimidin-2-yl]amino}cyclohexyl)-methyl]benzamide;
3,5-dichloro-N-[(cis-4-({[4-(dimethylamino)-5-methylpyrimidin-2-yl]amino}methyl)-cyclohexyl]benzamide;
N-(cis-4-{[4-(dimethylamino)-5-methylpyrimidin-2-yl]amino}cyclohexyl)-2-(2-fluorophenoxy)nicotinamide;
N-(cis-4-{[4-(dimethylamino)-6-methylpyrimidin-2-yl]amino}cyclohexyl)-3,4,5-trimethoxybenzamide;
N-(4-{[4-(dimethylamino)-6-methylpyrimidin-2-yl]amino}cyclohexyl)-3-nitrobenzamide;
N-(cis-4-{[4-(dimethylamino)-6-methylpyrimidin-2-yl]amino}cyclohexyl)-2,2-diphenylacetamide;
4-chloro-N-(cis-4-{[4-(dimethylamino)-6-methylpyrimidin-2-yl]amino}cyclohexyl)-benzamide;
3-chloro-N-(cis-4-{[4-(dimethylamino)-6-methylpyrimidin-2-yl]amino}cyclohexyl)-benzamide;
N-(cis-4-{[4-(dimethylamino)-6-methylpyrimidin-2-yl]amino}cyclohexyl)-3,4-difluorobenzamide;
N-(cis-4-{[4-(dimethylamino)-6-methylpyrimidin-2-yl]amino}cyclohexyl)-3-methylbenzamide;
N-(cis-4-{[4-(dimethylamino)-6-methylpyrimidin-2-yl]amino}cyclohexyl)-3-methoxybenzamide;
N-(cis-4-{[4-(dimethylamino)-6-methylpyrimidin-2-yl]amino}cyclohexyl)-4-fluorobenzamide;
N-(cis-4-{([4-(dimethylamino)-5-methylpyrimidin-2-yl]amino}cyclohexyl)-3-methylbenzamide;
N-(cis-4-{[4-(dimethylamino)-5-methylpyrimidin-2-yl]amino}cyclohexyl)-3-methoxybenzamide;
N-(cis-4-{(4-(dimethylamino)-5-methylpyrimidin-2-yl]amino}cyclohexyl)-4-methylbenzamide;
N-(cis-4-{[4-(dimethylamino)-5-methylpyrimidin-2-yl]amino}cyclohexyl)-3,4-difluorobenzamide;
3-chloro-N-(cis-4-{[4-(dimethylamino)-5-methylpyrimidin-2-yl] amino}cyclohexyl)-benzamide;
N-(cis-4-{[4-(dimethylamino)-5-methylpyrimidin-2-yl]amino}cyclohexyl)-2-(3-methylphenoxy)nicotinamide;
2-(4-bromophenoxy)-N-(cis-4-{[4-(dimethylamino)-5-methylpyrimidin-2-yl]amino}-cyclohexyl)nicotinamide;
2-(4-chlorophenoxy)-N-(cis-4-{[4-(dimethylamino)-5-methylpyrimidin-2-yl]amino}-cyclohexyl)nicotinamide;
N-(cis-4-{[4-(dimethylamino)-5-methylpyrimidin-2-yl]amino}cyclohexyl)-2-(4-fluorophenoxy)nicotinamide;
N-(cis-4-{[4-(dimethylamino)-5-methylpyrimidin-2-yl]amino}cyclohexyl)-2-(3-fluorophenoxy)nicotinamide;
2-(2-bromophenoxy)-N-(cis-4-{[4-(dimethylamino)-5-methylpyrimidin-2-yl]amino}-cyclohexyl)nicotinamide;
N-(cis-4-{[4-(dimethylamino)-5-methylpyrimidin-2-yl]amino}cyclohexyl)-2-(3-methoxyphenoxy)nicotinamide;
N-(cis-4-{[4-(dimethylamino)-5-methylpyrimidin-2-yl]amino}cyclohexyl)-2-(4-methoxyphenoxy)nicotinamide;

N-(cis-4-{[4-(dimethylamino)-5-methylpyrimidin-2-yl]amino}cyclohexyl)-2-(4-iodophenoxy)nicotinamide;
2-(3,4-dichlorophenoxy)-N-(cis-4-{[5-methyl-4-(methylamino)pyrimidin-2-yl]amino}-cyclohexyl)acetamide;
2-(2,3-dichlorophenoxy)-N-(cis-4-{[5-methyl-4-(methylamino)pyrimidin-2-yl]amino}-cyclohexyl)acetamide;
2-[(3,4-difluorophenyl)sulfonyl]-N-(cis-4-{[4-(dimethylamino)-5-methylpyrimidin-2-yl]-amino}cyclohexyl)nicotinamide;
N-[cis-4-{(4-[ethyl(methyl)amino]-5-methylpyrimidin-2-yl}amino)cyclohexyl]-3,4-difluorobenzamide;
N-(cis-4-{[4-(dimethylamino)-6-methylpyrimidin-2-yl]amino}cyclohexyl)-3,5-dimethoxybenzamide;
N-(cis-4-{([4-(dimethylamino)-5-methylpyrimidin-2-yl]amino}cyclohexyl)-2-(2-methoxyphenoxy)nicotinamide;
2-(2-chlorophenoxy)-N-(cis-4-{[4-(dimethylamino)-5-methylpyrimidin-2-yl]amino}-cyclohexyl)nicotinamide;
2-(3-chlorophenoxy)-N-(cis-4-{[4-(dimethylamino)-5-methylpyrimidin-2-yl]amino}-cyclohexyl)nicotinamide;
2-(3-bromophenoxy)-N-(cis-4-{[4-(dimethylamino)-5-methylpyriniddin-2-yl]amino}-cyclohexyl)nicotinamide;
N-(cis-4-{([4-(dimethylamino)-5-methylpyrimidin-2-yl]amino}cyclohexyl)-2-[3-(trifluoromethyl)phenoxy]nicotinamide;
N-(cis-4-{[4-(dimethylamino)-5-methylpyrimidin-2-yl]amino}cyclohexyl)-2-(3-fluorophenoxy)acetamide;
N-(cis-4-{[4-(dimethylamino)-5-methylpyrimidin-2-yl]amino}cyclohexyl)-2-(3-methoxyphenoxy)acetamide;
N-(cis-4-{[4-(dimethylamino)-5-methylpyrimidin-2-yl]amino}cyclohexyl)-2-[3-(trifluoromethyl)phenoxy]acetamide;
2-(3-chlorophenoxy)-N-(cis-4-{[4-(dimethylamino)-5-methylpyrimidin-2-yl]amino}-cyclohexyl)acetamide;
2-[(5-chloropyridin-3-yl)oxy]-N-(cis-4-{[4-(dimethylamino)-5-methylpyrimidin-2-yl]-amino}cyclohexyl)acetamide;
N-(cis-4-{[4-(dimethylamino)-5,6-dimethylpyrimidin-2-yl]amino}cyclohexyl)-3,4-difluorobenzamide;
N-(cis-4-{[4-(dimethylamino)-5-methylpyrimidin-2-yl]amino}cyclohexyl)-2-hydroxy-2-(4-methoxyphenyl)acetamide;
2-(2,3-difluorophenyl)-N-(cis-4-{[4-(dimethylamino)-5-methylpyrimidin-2-yl]amino}-cyclohexyl)-2-hydroxyacetamide;
N-(cis-4-{[4-(dimethylamino)-5-methylpyrimidin-2-yl]amino}cyclohexyl)-2-hydroxy-2-[3-(trifluoromethyl)phenyl] acetamide;
N-(cis-4-{[4-(dimethylamino)-5-methylpyrimidin-2-yl]amino}cyclohexyl)-2-{[2-(trifluoromethyl)phenyl]sulfinyl} acetamide;
2-[(2-chlorophenyl)sulfinyl]-N-(cis-4-{[4-(dimethylamino)-5-methylpyrimidin-2-yl]amino}-cyclohexyl)acetamide;
2-[(3-bromophenyl)sulfinyl]-N-(cis-4-{[4-(dimethylamino)-5-methylpyrimidin-2-yl]amino}-cyclohexyl)acetamide;
N-(cis-4-{[4-(dimethylamino)-5-methylpyrimidin-2-yl]amino}cyclohexyl)-3-(trifluoromethyl)benzamide;
N-(cis-4-{[4-(dimethylamino)-5-methylpyrimidin-2-yl]amino}cyclohexyl)-3-fluorobenzamide;
3-bromo-N-(cis-4-{[4-(dimethylamino)-5-methylpyrimidin-2-yl]amino}cyclohexyl)-benzamide;
N-(cis-4-{[4-(dimethylamino)-5-methylpyrimidin-2-yl]amino}cyclohexyl)-4-(trifluoromethoxy)benzamide;
N-(cis-4-{[4-(dimethylamino)-5-methylpyrimidin-2-yl]amino}cyclohexyl)-4-fluorobenzamide;
3,4-dichloro-N-(cis-4-{[4-(dimethylamino)-5-methylpyrimidin-2-yl]amino}cyclohexyl)-benzamide;
N-(cis-4-{[4-(dimethylamino)-5-methylpyrimidin-2-yl]amino}cyclohexyl)-3,5-bis(trifluoromethyl)benzamide;
N-(cis-4-{[4-(dimethylamino)-5-methylpyrimidin-2-yl]amino}cyclohexyl)-3,5-dimethoxybenzamide;
N-(cis-4-{[4-(dimethylamino)-5-methylpyrimidin-2-yl]amino}cyclohexyl)-2,4-difluorobenzamide;
N-( cis-4-{[4-(dimethylamino)-5-methylpyrimidin-2-yl]amino}cyclohexyl)-2,5-difluorobenzamide;
N-(cis-4-{[4-(dimethylamino)-5-methylpyrimidin-2-yl]amino}cyclohexyl)-2,3,4-trifluorobenzamide;
4-chloro-N-(cis-4-{[4-(dimethylamino)-5-methylpyrimidin-2-yl]amino}cyclohexyl)-benzamide;
3-cyano-N-(cis-4-{[4-(dimethylamino)-5-methylpyrimidin-2-yl]amino}cyclohexyl)-benzamide;
4-cyano-N-(cis-4-{[4-(dimethylamino)-5-methylpyrimidin-2-yl]amino}cyclohexyl)-benzamide;
N-(cis-4-{[4-(dimethylamino)-5-methylpyrimidin-2-yl]amino}cyclohexyl)-2-(isopropylthio)nicotinamide;
N-(cis-4-{[4-(dimethylamino)-5-methylpyrimidin-2-yl]amino}cyclohexyl)-2-(propylthio)nicotinamide;
N-(cis-4-{[4-(dimethylamino)-6-methylpyrimidin-2-yl]amino]}cyclohexyl)-3-(trifluoromethyl)benzamide;
3-cyano-N-(cis-4-{[4-(dimethylamino)-6-methylpyrimidin-2-yl]amino}cyclohexyl)-benzamide;
4-cyano-N-(cis-4-{[4-(dimethylamino)-6-methylpyrimidin-2-yl]amino}cyclohexyl)-benzamide;
N-(cis-4-{[4-(dimethylamino)-5,6-dimethylpyrimidin-2-yl]amino}cyclohexyl)-3-(trifluoromethyl)benzamide;
3-cyano-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-3-methylbenzamide;
3-chloro-N-(cis-4-{[4-(dimethylamino)-5,6-dimethylpyrimidin-2-yl]amino}cyclohexyl)-benzamide;
3-bromo-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-3,5-dimethoxybenzamide;
N-(cis-4-{[4-(dimethylamino)-5,6-dimethylpyrimidin-2-yl]amino}cyclohexyl)-3,5-bis(trifluoromethyl)benzamide;
3,4-dichioro-N-(cis-4-{[4-(dimethylamino)-5,6-dimethylpyrimidin-2-yl]amino}cyclohexyl)-benzamide;
4-cyano-N-(cis-4-{[4-(dimethylamino)-5,6-dimethylpyrimidin-2-yl]amino}cyclohexyl)-benzamide;
N-(cis-4-{[4-(dimethylamino)-5,6-dimethylpyrimidin-2-yl]amino}cyclohexyl)-4-methylbenzamide;

N-(cis-4-{[4-(dimethylamino)-5,6-dimethylpyrimidin-2-yl]amino}cyclohexyl)-4-fluorobenzamide;
4-chloro-N-(cis-4-{[4-(dimethylamino)-5,6-dimethylpyrimidin-2-yl]amino}cyclohexyl)-benzamide;
4-bromo-N-(cis-4-{[4-(dimethylamino)-5-methylpyrimidin-2-yl]amino}cyclohexyl)-benzamide;
N-(cis-4-{[4-(dimethylamino)-5-methylpyrimidin-2-yl]amino}cyclohexyl)-4-(trifluoromethyl)benzamide;
N-(cis-4-{[4-(dimethylamino)-5,6-dimethylpyrimidin-2-yl]amino}cyclohexyl)-3-methoxybenzamide;
5-bromo-N-(cis-4-{[4-(dimethylamino)-5,6-dimethylpyrimidin-2-yl]amino}cyclohexyl)-2-furamide;
N-(cis-4-{[4-(dimethylamino)-5,6-dimethylpyrimidin-2-yl]amino}cyclohexyl)-2,6-dimethoxynicotinamide;
4-bromo-N-(cis-4-{[4-(dimethylamino)-5,6-dimethylpyrimidin-2-yl]amino}cyclohexyl)-benzamide;
N-(cis-4-{[4-(dimethylammo)-5,6-dimethylpyrimidin-2-yl]amino}cyclohexyl)-4-(trifluoromethyl)benzamide;
4-bromo-N-(cis-4-{[4-(dimethylamino)-5,6-dimethylpyrimidin-2-yl]amin}}cyclohexyl)-3-methylbenzamide;
N-(cis-4-{[4-(dimethylamino)-5,6-dimethylpyrimidin-2-yl]amino}cyclohexyl)-3-fluoro-4-methylbenzamide;
N-(cis-4-{[4-(dimethylamino)-5,6-dimethylpyrimidin-2-yl]amino}cyclohexyl)-4-fluoro-3-methylbenzamide;
N-(cis-4-{[4-(dimethylamino)-5,6-dimethylpyrimidin-2-yl]amino}cyclohexyl)-3-(trifluoromethoxy)benzamide;
5-bromo-N-(cis-4-{[4-(dimethylamino)-5,6-dimethylpyrimidin-2-yl]amino}cyclohexyl)-nicotinamide;
N-(cis-4-{[4-(dimethylamino)-5,6-dimethylpyrimidin-2-yl]amino}cyclohexyl)-5-methylthiophene-2-carboxamide;
N-(cis-4-{[4-(dimethylamino)-5,6-dimethylpyrimidin-2-yl]amino}cyclohexyl)-3,5-diethoxybenzamide;
N-(cis-4-{[4-(dimethylamino)-5,6-dimethylpyrimidin-2-yl]amino}cyclohexyl)-3-ethoxybenzamide;
N-(cis-4-{[4-(dimethylamino)-5,6-dimethylpyrimidin-2-yl]amino}cyclohexyl)-3-isopropoxybenzamide;
3,5-dichloro-N-(cis-4-{[4-(dimethylamino)-5-methylpyrimidin-2-yl]amino}cyclohexyl)-benzamide;
4-bromo-N-(cis-4-{[4-(dimethylamino)-5-methylpyrimidin-2-yl]amino}cyclohexyl)-3-methylbenzamide;
N-(cis-4-{[4-(dimethylamino)-5-methylpyrimidin-2-yl]amino}cydohexyl)-4-fluoro-3-methylbenzamide;
N-(cis-4-{[4-(dimethylamino)-5-methylpyrimidin-2-yl]amino}cyclohexyl)-3-fluoro-4-methylbenzamide;
N-(cis-4-{[4-(dimethylamino)-5-methylpyrimidin-2-yl]amino}cyclohexyl)-3-ethylbenzamide;
N-(cis-4-{[4-(dimethylamino)-6-methylpyrimidin-2-yl] amino}cyclohexyl)-3,5-bis(trifluoromethyl)benzamide;
N-(cis-4-{[4-(dimethylamino)-6-methylpyrimidin-2-yl]amino}cyclohexyl)-3-fluoro-4-(trifluoromethyl)benzamide;
N-(cis-4-{[4-(dimethylamino)-6-methylpyrimidin-2-yl]amino}cyclohexyl)-3-fluoro-5-(trifluoromethyl)benzamide;
3-chloro-N-(cis-4-{[4-(dimethylamino)-6-methylpyrimidin-2-yl]amino}cyclohexyl)-4-fluorobenzamide;
N-(cis-4-{[4-(dimethylamino)-6-methylpyrimidin-2-yl]amino}cyclohexyl)-4-fluoro-3-methylbenzamide;
N-(cis-4-{[4-(dimethylamino)-6-methylpyrimidin-2-yl]amino}cyclohexyl)-3-fluoro-4-methylbenzamide;
3,5-dichloro-N-(cis-4-{[4-(dimethylamino)-6-methylpyrimidin-2-yl]amino}cyclohexyl)-benzamide;
N-(cis-4-{[4-(dimethylamino)-6-methylpyrimidin-2-yl]amino}cyclohexyl)-3-(trifluoromethoxy)benzamide;
N-(cis-4-{[4-(dimethylamino)-6-methylpyrimidin-2-yl]amino}cyclohexyl)-3,5-difluorobenzamide;
4-bromo-N-(cis-4-{[4-(dimethylamino)-6-methylpyrimidin-2-yl]amino}cyclohexyl)-3-methylbenzamide;
N-(cis-4-{[4-(dimethylamino)-6-methylpyrimidin-2-yl]amino}cyclohexyl)-3-ethylbenzamide;
4-bromo-N-(cis-4-{[4-(dimethylamino)-6-methylpyrimidin-2-yl]amino}cyclohexyl)-benzamide;
N-(cis-4-{[4-(dimethylamino)-6-methylpyrimidin-2-yl]amino}cyclohexyl)-4-ethylbenzamide;
N-(cis-4-{[4-(dimethylamino)-6-methylpyrimidin-2-yl]amino}cyclohexyl)-3,5-diethoxybenzamide;
N-(cis-4-{[4-(dimethylamino)-6-methylpyrimidin-2-yl]amino}cyclohexyl)-3-ethoxybenzamide;
N-(cis-4-{[4-(dimethylamino)-6-methylpyrimidin-2-yl]amino}cyclohexyl)-3-isopropoxybenzamide;
5-bromo-N-(cis-4-{[4-(dimethylamino)-6-methylpyrimidin-2-yl]amino}cyclohexyl)-nicotinamide;
5-bromo-N-(cis-4-{[4-(dimethylamino)-6-methylpyrimidin-2-yl]amino}cyclohexyl)-2-furamide;
5-chloro-N-(cis-4-{[4-(dimethylamino)-6-methylpyrimidin-2-yl]amino}cyclohexyl)-2-furamide;
N-(cis-4-{[4-(dimethylamino)-5-methylpyrimidin-2-yl]amino}cyclohexyl)-3-fluoro-5-(trifluoromethyl)benzamide;
N-(cis-4-{[4-(dimethylamino)-5-methylpyrimidin-2-yl]amino}cyclohexyl)-2,2-difluoro-1,3-benzodioxole-5-carboxamide;
N-(cis-4-{[4-(dimethylamino)-5-methylpyrimidin-2-yl]amino}cyclohexyl)-3-ethoxybenzamide;
5-bromo-N-(cis-4-{[4-(dimethylamino)-5-methylpyrimidin-2-yl]amino}cyclohexyl)-2-furamide;
N-(cis-4-{[4-(dimethylamino)-5-methylpyrimidin-2-yl]amino}cyclohexyl)-3,5-diethoxybenzamide;
4-chloro-N-(cis-4-{[4-(dimethylamino)-5-methylpyrimidin-2-yl]amino}cyclohexyl)-3-(trifluoromethyl)benzamide;
5-bromo-N-(cis-4-{[4-(dimethylamino)-5-methylpyrimidin-2-yl]amino}cyclohexyl)-nicotinamide;
3,4-dichloro-N-(cis-4-{[4-(dimethylamino)-6-methylpyrimidin-2-yl]amino}cyclohexyl)-benzamide;
3-chloro-N-(cis-4-{[4-(dimethylamino)-5-methylpyrimidin-2-yl]amino}cyclohexyl)-4-(trifluoromethoxy)benzamide;
N-(cis-4-{[4-(dimethylamino)-5-methylpyrimidin-2-yl]amino}cyclohexyl)-4-methoxy-3-(trifluoromethyl)benzamide;
N-(cis-4-{[4-(dimethylamino)-6-methylpyrimidin-2-yl]amino}cyclohexyl)-4-methoxy-3-(trifluoromethyl)benzamide;
2-(4-chlorophenyl)-N-(cis-4-{[4-(dimethylamino)-5-methylpyrimidin-2-yl]amino}-cyclohexyl)-2-methylpropanamide
1-(4-chlorophenyl)-N-(cis-4-{[4-(dimethylamino)-5-methylpyrimidin-2-yl]amino}-cyclohexyl)cyclopropanecarboxamide;

1-(4-chlorophenyl)-N-(cis-4-{[4-(dimethylamino)-5-methylpyrimidin-2-yl]amino}-cyclohexyl)cyclobutanecarboxamide;
1-(2,4-dichlorophenyl)-N-(cis-4-{[4-(dimethylamino)-5-methylpyrimidin-2-yl]amino}-cyclohexyl)cyclopropanecarboxamide;
2-(4-chlorophenyl)-N-(cis-4-{[4-(dimethylamino)-6-methylpyrimidin-2-yl]amino}-cyclohexyl)-2-methylpropanamide
1-(4-chlorophenyl)-N-(cis-4-{[4-(dimethylamino)-6-methylpyrimidin-2-yl]amino}-cyclohexyl)cyclopropanecarboxamide;
1-(4-chlorophenyl)-N-(cis-4-{[4-(dimethylamino)-6-methylpyrimidin-2-yl]amino}-cyclohexyl)cyclobutanecarboxamide;
1-(2,4-dichlorophenyl)-N-(cis-4-{[4-(dimethylamino)-6-methylpyrimidin-2-yl]amino}-cyclohexyl)cyclopropanecarboxamide;
2-(4-chlorophenyl)-N-(cis-4-{[4-(dimethylamino)-5-methylpyrimidin-2-yl]amino}-cyclohexyl)acetamide;
N-(cis-4-{[4-(dimethylamino)-5-methylpyrimidin-2-yl]amino}cyclohexyl)-1-(4-methylphenyl)cyclopropanecarboxamide;
2-(4-chlorophenyl)-N-(cis-4-{[4-(dimethylamino)-5-methylpyrimidin-2-yl]amino}-cyclohexyl)propanamide
2-(4-chlorophenyl)-N-(cis-4-{[4-(dimethylamino)-5-methylpyrimidin-2-yl]amino}-cyclohexyl)-2-hydroxyacetamide;
N-(cis-4-{[4-(dimethylamino)-5-methylpyrimidin-2-yl]amino}cyclohexyl)-1-(4-methoxyphenyl)cyclopropanecarboxamide;
$N^2$-(3-chlorophenyl)-N-(cis-4-{[4-(dimethylamino)-5-methylpyrimidin-2-yl]amino}-cyclohexyl)-$N^2$-methylglycinamide;
$N^2$-(3,4-dichlorophenyl)-N-(cis-4-{[4-(dimethylamino)-5-methylpyrimidin-2-yl]amino}-cyclohexyl)-$N^2$-methylglycinamide;
N-(cis-4-{[4-(dimethylamino)-5-methylpyrimidin-2-yl]amino}cyclohexyl)-$N^2$-methyl-$N^2$-(3-methylphenyl)glycinamide;
N-(cis-4-{[4-(dimethylamino)-5-methylpyrimidin-2-yl]amino}cyclohexyl)-$N^2$-(3-fluorophenyl)-$N^2$-methylglycinamide;
N-(cis-4-{[4-(dimethylamino)-5-methylpyrimidin-2-yl]amino}cyclohexyl)-$N^2$-(4-fluorophenyl)-$N^2$-methylglycinamide;
$N^2$-(4-chlorophenyl)-N-(cis-4-{[4-(dimethylamino)-5-methylpyrimidin-2-yl]amino}-cyclohexyl)-$N^2$-methylglycinamide;
$N^2$-(3,4-difluorophenyl)-N-(cis-4-{[4-(dimethylamino)-5-methylpyrimidin-2-yl]amino}-cyclohexyl)-$N^2$-methylglycinamide;
N-(cis-4-{[4-(dimethylamino)-5-methylpyrimidin-2-yl]amino}cyclohexyl)-$N^2$-(3-methoxyphenyl)-$N^2$-methylglycinamide;
2-(3,4-dichlorophenoxy)-N-(cis-4-{[4-methyl-6-(methylamino)pyrimidin-2-yl]amino}-cyclohexyl)acetamide;
trans-2-(4-chlorophenyl)-N-(cis-4-{[4-(dimethylamino)-5-methylpyrimidin-2-yl]amino}-cyclohexyl)cyclopropanecarboxamide;
trans-2-(3-chlorophenyl)-N-(cis-4-{[4-(dimethylamino)-5-methylpyrimidin-2-yl]amino}-cyclohexyl)cyclopropanecarboxamide;
trans-2-(3,4-difluorophenyl)-N-(cis-4-{[4-(dimethylamino)-5-methylpyrimidin-2-yl]amino}-cyclohexyl)cyclopropanecarboxamide;
trans-2-(3,4-dichlorophenyl)-N-(cis-4-{[4-(dimethylamino)-5-methylpyrimidin-2-yl]-amino}cyclohexyl)cyclopropanecarboxamide;
trans-2-[3,5-bis(trifluoromethyl)phenyl]-N-(cis-4-{[4-(dimethylamino)-5-methylpyrimidin-2-yl]amino}cyclohexyl)cyclopropanecarboxamide;
2-[(4-chlorophenyl)sulfonyl]-N-(cis-4-{[4-(dimethylamino)-5-methylpyrimidin-2-yl]-amino}cyclohexyl)nicotinamide;
N-(cis-4-{[4-(dimethylamino)-5-methylpyrimidin-2-yl]amino}cyclohexyl)-2-{[1-methyl-3-(trifluoromethyl)-1H-pyrazol-5-yl]oxy}acetamide;
N-[cis-4-(4-dimethylamino-5-methyl-pyrimidin-2-ylamino)-cyclohexyl]-2-phenoxy-nicotinamide;
N-[cis-4-(4-dimethylamino-6-methyl-pyrimidin-2-ylamino)-cyclohexyl]-2-phenoxy-nicotinamide;
3-chloro-N-[cis-4-(4-dimethylamino-5-methyl-pyrimidin-2-ylamino)-cyclohexyl]-4-fluoro-benzamide;
4-chloro-N-[cis-4-(4-dimethylamino-6-methyl-pyrimidin-2-ylamino)-cyclohexyl]-3-fluoro-benzamide;
3-chloro-N-[cis-4-(4-dimethylamino-6-methyl-pyrimidin-2-ylamino)-cyclohexyl]-5-fluoro-benzamide;
N-[cis-4-(4-dimethylamino-6-methyl-pyrimidin-2-ylamino)-cyclohexyl]-3,4,5-trifluoro-benzamide;
3-chloro-4-fluoro-N-[cis-4-(5-methyl-4-methylamino-pyrimidin-2-ylamino)-cyclohexyl]-benzamide;
4-chloro-N-[cis-4-(4-dimethylamino-5-methyl-pyrimidin-2-ylamino)-cyclohexyl]-3-fluoro-benzamide;
3-chloro-N-[cis-4-(4-(4-dimethylamino-5-methyl-pyrimidin-2-ylamino)-cyclohexyl]-5-fluorobenzamide;

N-[cis-4-(4-dimethylamino-5-methyl-pyrimidin-2-ylamino)-cyclohexyl]-3,4,5-trifluorobenzamide;
N-[cis-4-(4-dimethylamino-5-methyl-pyrimidin-2-ylamino)-cyclohexyl]-3,5-difluorobenzamide; and
2-(3,4-difluoro-phenyl)-N-[cis-4-(4-dimethylamino-5-methyl-pyrimidin-2-ylamino)-cyclohexyl]-acetamide;

or a pharmaceutically acceptable salt, hydrate, or solvate thereof.

In some embodiments of the present invention, $R_1$ is selected from the group consisting of:

(i) $C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:

  - •• halogen,
  - •• nitro,
  - •• $C_{1-5}$ alkylcarbonylamino,
  - •• $C_{3-6}$ cycloalkylcarbonylamino,
  - •• $C_{1-5}$ alkyl,
  - •• $C_{1-5}$ alkyl substituted by halogen,
  - •• $C_{1-5}$ alkoxy, and
  - •• $C_{1-5}$ alkoxy substituted by halogen,

(ii) $C_{3-12}$ cycloalkyl, and
$C_{3-12}$ cycloalkyl substituted by carbocyclic aryl,
(iii) carbocyclic aryl, and
carbocyclic aryl substituted by substituent(s) independently selected from the group consisting of:

- halogen,
- $C_{1-10}$ alkyl,
- $C_{1-10}$ alkyl substituted by halogen,
- $C_{1-9}$ alkoxy, and
- $C_{1-5}$ alkylthio,

(iv) heterocyclyl,
L is Formula (XV);
Y is $-C(O)NR_5-$;
$R_2$ is selected from the group consisting of:

$-N(R_{2a}(R_{2b})$, wherein $R_{2a}$ is hydrogen or $C_{1-5}$ alkyl and $R_{2b}$ is $C_{1-5}$ alkyl;

wherein carbocyclic aryl is phenyl or naphthyl;
heterocyclyl is 3,4-dihydro-1*H*-isoquinolinyl; and
halogen is fluoro, chloro, bromo, or iodo;

or a pharmaceutically acceptable salt, hydrate, or solvate thereof.

In some embodiments of the present invention, $R_1$ is selected from the group consisting of:

(i) $C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:

  - •• halogen,
  - •• nitro,
  - •• $C_{1-5}$ alkyl,
  - •• $C_{1-5}$ alkyl substituted by halogen,
  - •• $C_{1-5}$ alkoxy, and

- •• $C_{1-5}$ alkoxy substituted by halogen,

(ii) $C_{3-12}$ cycloalkyl, and
$C_{3-12}$ cycloalkyl substituted by carbocyclic aryl,
(iii) carbocyclic aryl, and
carbocyclic aryl substituted by substituent(s) independently selected from the group consisting of:

- halogen,
- $C_{1-10}$ alkyl,
- $C_{1-10}$ alkyl substituted by halogen, and
- $C_{1-9}$ alkoxy,

$R_2$ is selected from the group consisting of:

- $N(R_{2a})(R_{2b})$, wherein $R_{2a}$ is hydrogen or $C_{1-5}$ alkyl and $R_{2b}$ is $C_{1-5}$ alkyl;

wherein carbocyclic aryl is phenyl or naphthyl;
heterocyclyl is 3,4-dihydro-*1H*-isoquinolinyl; and
halogen is fluoro, chloro, bromo, or iodo;

or a pharmaceutically acceptable salt, hydrate, or solvate thereof.

In some embodiments of the present invention, p is 1 and T is $C_{1-5}$ alkyl; $R_3$ and $R_4$ are both hydrogen; and A and B are both single bonds; $R_5$ is hydrogen: or a pharmaceutically acceptable salt, hydrate, or solvate thereof.

In some embodiments, compounds of the present invention are of Formula (I) wherein the compound is selected from the group consisting of:

cis-4-{[4-(dimethylamino)-6-methylpyrimidin-2-yl]amino}-N-(3-iodobenzyl)-cyclohexanecarboxamide;
cis-N-(2,4-dichlorobenzyl)-4-{[4-(dimethylamino)-6-methylpyrimidin-2-yl]amino}-cyclohexanecarboxamide;
cis-N-(2,5-dichlorobenzyl)-4-{[4-(dimethylamino)-6-methylpyrimidin-2-yl]amino}-cyclohexanecarboxamide;
cis-4-{[4-(dimethylamino)-6-methylpyrimidin-2-yl]amino}-N-(4-methylbenzyl)-cyclohexanecarboxamide;
cis-N-(3,5-dichlorobenzyl)-4-{[4-(dimethylamino)-6-methylpyrimidin-2-yl]amino}-cyclohexanecarboxamide;
cis-N-(3,5-dimethoxybenzyl)-4-{[4-(dimethylamino)-6-methylpyrimidin-2-yl]amino}-cyclohexanecarboxamide;
cis-N-(3-chlorobenzyl)-4-{[4-(dimethylamino)-6-methylpyrimidin-2-yl]amino}-cyclohexanecarboxamide;
cis-4-{[4-(dimethylamino)-6-methylpyrimidin-2-yl]amino}-N-[3-(trifluoromethyl)benzyl]-cyclohexanecarboxamide;
cis-N-[3,5-bis(trifluoromethyl)benzyl]-4-{[4-(dimethylamino)-6-methylpyrimidin-2-yl]-amino}cyclohexanecarboxamide;
cis-4-{[4-(dimethylamino)-6-methylpyrimidin-2-yl]amino}-N-(3-methoxybenzyl)-cyclohexanecarboxamide;
cis-N-(4-chlorobenzyl)-4-{[4-(dimethylamino)-6-methylpyrimidin-2-yl]amino}-cyclohexanecarboxamide;
cis-N-(3,4-dichlorobenzyl)-4-{[4-(dimethylamino)-6-methylpyrimidin-2-yl]amino}-cyclohexanecarboxamide;
cis-N-(2,5-difluorobenzyl)-4-{[4-(dimethylamino)-6-methylpyrimidin-2-yl]amino}-cyclohexanecarboxamide;
cis-N-(2,3-difluorobenzyl)-4-{[4-(dimethylamino)-6-methylpyrimidin-2-yl]amino}-cyclohexanecarboxamide;
cis-N-(4-bromo-2-fluorobenzyl)-4-{[4-(dimethylamino)-6-methylpyrimidin-2-yl]amino}-cyclohexanecarboxamide;
cis-N-(2,4-difluorobenzyl)-4-{[4-(dimethylamino)-6-methylpyrimidin-2-yl]amino}-cyclohexanecarboxamide;
cis-4-{[4-(dimethylamino)-6-methylpyrimidin-2-yl]amino}-N-(3-methylbenzyl)-cyclohexanecarboxamide;
cis-4-{[4-(dimethylamino)-6-methylpyrimidin-2-yl]amino}-N-[2-(trifluoromethoxy)benzyl]-cyclohexanecarboxamide;
cis-4-{[4-(dimethylamino)-5-methylpyrimidin-2-yl]amino}-N-[(1R)-1-phenylethyl]-cyclohexanecarboxamide;
cis-4-{[4-(dimethylamino)-5-methylpyrimidin-2-yl]amino}-N-[(1S)-1-(4-methylphenyl)-ethyl]cyclohexanecarboxamide;
cis-4-{[4-(dimethylamino)-5-methylpyrimidin-2-yl]amino}-N-[(1R)-1-(4-fluorophenyl)-ethyl]cyclohexanecarboxamide;
cis-4-{[4-(dimethylamino)-5-methylpyrimidin-2-yl]amino}-N-[(1S)-1-(4-fluorophenyl)-ethyl]cyclohexanecarboxamide;
cis-4-{[4-(dimethylamino)-5-methylpyrimidin-2-yl]amino}-N-[(1R)-1-(3-methoxyphenyl)-ethyl] cyclohexanecarboxamide;
cis-4-{[4-(dimethylamino)-5-methylpyrimidin-2-yl]amino}-N-[(1S)-1-(3-methoxyphenyl)-ethyl]cyclohexanecarboxamide;
cis-4-{[4-(dimethylamino)-5-methylpyrimidin-2-yl]amino}-N-[(1S)-1-(4-methoxyphenyl)-ethyl] cyclohexanecarboxamide;
cis-N-[(1R)-1-(4-chlorophenyl)ethyl]-4-{[4-(dimethylamino)-5-methylpyrimidin-2-yl]-amino}cyclohexanecarboxamide;
cis-N-[1-(4-bromophenyl)ethyl]-4-{[4-(dimethylamino)-5-methylpyrimidin-2-yl]amino}-cyclohexanecarboxamide;
cis-4-{[4-(dimethylamino)-5-methylpyrimidin-2-yl]amino}-N-[(1R)-1-(4-nitrophenyl)-ethyl]cyclohexanecarboxamide;
cis-4-{[4-(dimethylamino)-5-methylpyrimidin-2-yl]amino}-N-[(1S)-1-(4-nitrophenyl)ethyl]-cyclohexanecarboxamide;
cis-4-{[4-(dimethylamino)-5-methylpyrimidin-2-yl]amino}-N-(3-fluorophenyl)-cyclohexanecarboxamide;
cis-4-{[4-(dimethylamino)-5-methylpyrimidin-2-yl]amino}-N-(3-methoxyphenyl)-cyclohexanecarboxamide;
cis-N-(3-chlorophenyl)-4-{[4-(dimethylamino)-5-methylpyrimidin-2-yl]amino}-cyclohexanecarboxamide;
cis-4-{[4-(dimethylamino)-5-methylpyrimidin-2-yl]amino}-N-[(1S,2R)-2-phenylcyclopropyl]cyclohexanecarboxamide;
cis-4-{[4-(dimethylamino)-5-methylpyrimidin-2-yl]amino}-N-[4-(trifluoromethyl)phenyl]-cyclohexanecarboxamide;
cis-4-{[4-(dimethylamino)-6-methylpyrimidin-2-yl]amino}-N-[(1R)-1-(3-methoxyphenyl)-ethyl] cyclohexanecarboxamide;
cis-N-[(1S)-1-(4-chlorophenyl)ethyl]-4-{[4-(dimethylamino)-6-methylpyrimidin-2-yl]-amino}cyclohexanecarboxamide;
cis-N-benzyl-4-{[4-(dimethylamino)-6-methylpyrimidin-2-yl]amino}-cyclohexanecarboxamide;
cis-4-{[4-(dimethylamino)-6-methylpyrimidin-2-yl]amino}-N-(4-fluorobenzyl)-cyclohexanecarboxamide;
cis-N-(3,4-difluorobenzyl)-4-{[4-(dimethylamino)-6-methylpyrimidin-2-yl]amino}-cyclohexanecarboxamide;
cis-4-{[4-(dimethylamino)-6-methylpyrimidin-2-yl]amino}-N-[(1S)-1-(4-methoxyphenyl)-ethyl]cyclohexanecarboxamide;
cis-4-{[4-(dimethylamino)-6-methylpyrimidin-2-yl]amino}-N-[(1S)-1-(3-methoxyphenyl)-ethyl]cyclohexanecarboxamide;
cis-4-{[4-(dimethylamino)-6-methylpyrimidin-2-yl]amino}-N-[(1R)-1-(4-fluorophenyl)-ethyl]cyclohexanecarboxamide;
cis-N-[(1R)-1-(4-chlorophenyl)ethyl]-4-{[4-(dimethylamino)-6-methylpyrimidin-2-yl]-amino )cyclohexanecarboxamide;
cis-4-{[4-(dimethylamino)-5-methylpyrimidin-2-yl]amino}-N-[(1S)-1-(1-naphthyl)ethyl]-cyclohexanecarboxamide;
cis-N-[(1R)-1-(4-bromophenyl)ethyl]-4-{[4-(dimethylamino)-5-methylpyrimidin-2-yl]-amino}cyclohexanecarboxamide;
cis-N-[(1S)-1-(4-bromophenyl)ethyl]-4-{[4-(dimethylamino)-5-methylpyrimidin-2-yl]-amino}cyclohexanecarboxamide;
cis-4-{[4-(dimethylamino)-5-methylpyrimidin-2-yl]amino}-N-{(1S)-1-[4-(trifluoromethoxy)phenyl]ethyl}cyclohexanecarboxamide;
cis-4-{[4-(dimethylamino)-5-methylpyrimidin-2-yl]amino}-N-[(1R)-1-(2-fluorophenyl)-ethyl]cyclohexanecarboxamide;
cis-N-{(1S)-1-[3,5-bis(trifluoromethyl)phenyl]ethyl}-4-{[4-(dimethylamino)-5-methylpyrimidin-2-yl]amino}cyclohexanecarboxamide;
4-{[4-(dimethylamino)-5-methylpyrimidin-2-yl]amino}-N-{(1S)-1-[3-(trifluoromethyl)-phenyl]ethyl}cyclohexanecarboxamide;
4-{[4-(dimethylamino)-5-methylpyrimidin-2-yl]amino}-N-{(1S)-1-[2-(trifluoromethyl)-phenyl]ethyl}cyclohexanecarboxamide;
cis-4-{[4-(dimethylamino)-5-methylpyrimidin-2-yl]amino}-N-]{(1R)-1-[4-(trifluoromethoxy)phenyl]ethyl}cyclohexanecarboxamide;
cis-N-[(1S)-1-(4-chlorophenyl)ethyl]-4-{[4-(4-(dimethylamino)-5-methylpyrimidin-2-yl]-amino}cyclohexanecarboxamide;
cis-N-[(1R)-1-(4-chlorophenyl)ethyl]-4-{[4-(dimethylamino)-5-methylpyrimidin-2-yl]-amino}cyclohexanecarboxamide;
cis-N-[1-(4-chlorophenyl)-1-methylethyl]-4-{[4-(dimethylamino)-5-methylpyrimidin-2-yl]-amino}cyclohexanecarboxamide; and
cis-N-{1-[3,5-bis(trifluoromethyl)phenyl]-1-methylethyl}-4-{[4-(dimethylamino)-5-methylpyrimidin-2-yl]amino}cyclohexanecarboxamide;

or a pharmaceutically acceptable salt, hydrate, or solvate thereof.

In some embodiments, compounds of the present invention are of Formula (I) wherein the compound is selected from the group consisting of:

cis-4-{[4-(dimethylamino)-6-methylpyrimidin-2-yl]amino}-N-(3-iodobenzyl)-cyclohexanecarboxamide;
cis-N-(2,4-dichlorobenzyl)-4-{[4-(dimethylamino)-6-methylpyrimidin-2-yl]amino}-cyclohexanecarboxamide;
cis-N-(2,5-dichlorobenzyl)-4-{[4-(dimethylamino)-6-methylpyrimidin-2-yl]amino}-cyclohexanecarboxamide;
cis-4-{[4-(dimethylamino)-6-methylpyrimidin-2-yl]amino}-N-(4-methylbenzyl)-cyclohexanecarboxamide;
cis-N-(3,5-dichlorobenzyl)-4-{[4-(dimethylamino)-6-methylpyrimidin-2-yl]amino}-cyclohexanecarboxamide;
cis-N-(3,5-dimethoxybenzyl)-4-{[4-(dimethylamino)-6-methylpyrimidin-2-yl]amino}-cyclohexanecarboxamide;
cis-N-(3-chlorobenzyl)-4-{[4-(dimethylamino)-6-methylpyrimidin-2-yl]amino}-cyclohexanecarboxamide;
cis-N-[3,5-bis(trifluoromethyl)benzyl]-4-{[4-(dimethylamino)-6-methylpyrimidin-2-yl]-amino}cyclohexanecarboxamide;
cis-4-{[4-(dimethylamino)-6-methylpyrimidin-2-yl]amino}-N-(3-methoxybenzyl)-cyclohexanecarboxamide;
cis-N-(4-chlorobenzyl)-4-{[4-(dimethylamino)-6-methylpyrimidin-2-yl]amino}-cyclohexanecarboxamide;
cis-N-(3,4-dichlorobenzyl)-4-{[4-(dimethylamino)-6-methylpyrimidin-2-yl]amino}-cyclohexanecarboxamide;
cis-N-(2,5-difluorobenzyl)-4-{[4-(dimethylamino)-6-methylpyrimidin-2-yl]amino}-cyclohexanecarboxamide;
cis-N-(2,3-difluorobenzyl)-4-{[4-(dimethylamino)-6-methylpyrimidin-2-yl]amino}-cyclohexanecarboxamide;
cis-N-(4-bromo-2-fluorobenzyl)-4-{[4-(dimethylamino)-6-methylpyrimidin-2-yl] amino}-cyclohexanecarboxamide;
cis-N-(2,4-difluorobenzyl)-4-{[4-(dimethylamino)-6-methylpyrimidin-2-yl]amino}-cyclohexanecarboxamide;
cis-4-{[4-(dimethylamino)-6-methylpyrimidin-2-yl]amino}-N-(3-methylbenzyl)-cyclohexanecarboxamide;
cis-4-{[4-(dimethylamino)-6-methylpyrimidin-2-yl]amino}-N-[2-(trifluoromethoxy)benzyl]-cyclohexanecarboxamide;
cis-4-{[4-(dimethylamino)-5-methylpyrimidin-2-yl]amino}-N-[(1S)-1-(4-methylphenyl)-ethyl]cyclohexanecarboxamide;
cis-4-{[4-(dimethylamino)-5-methylpyrimidin-2-yl]amino}-N-[(1R)-1-(4-fluorophenyl)-ethyl]cyclohexanecarboxamide;
cis-4-{[4-(dimethylamino)-5-methylpyrimidin-2-yl]amino}-N-[(1R)-1-(3-methoxyphenyl)-ethyl]cyclohexanecarboxamide;
cis-4-{[4-(dimethylamino)-5-methylpyrimidin-2-yl]amino}-N-[(1S)-1-(3-methoxyphenyl)-ethyl]cyclohexanecarboxamide;
cis-4-{[4-(dimethylamino)-5-methylpyrimidin-2-yl]amino}-N-[(1S)-1-(4-methoxyphenyl)-ethyl]cyclohexanecarboxamide;
cis-N-[(1R)-1-(4-chlorophenyl)ethyl]-4-{[4-(dimethylamino)-5-methylpyrimidin-2-yl]-amino}cyclohexanecarboxamide;
cis-N-[1-(4-bromophenyl)ethyl]-4-{[4-(dimethylamino)-5-methylpyrimidin-2-yl]amino}-cyclohexanecarboxamide;
cis-4-{[4-(dimethylamino)-5-methylpyrimidin-2-yl]amino}-N-((1R)-1-(4-nitrophenyl)-ethyl] cyclohexanecarboxamide;
cis-4-{[4-(dimethylamino)-5-methylpyrimidin-2-yl]amino}-N-(3-methoxyphenyl)-cyclohexanecarboxamide;
cis-N-(3-chlorophenyl)-4-{[4-(dimethylamino)-5-methylpyrimidin-2-yl]amino}-cyclohexanecarboxamide;
cis-4-{[4-(dimethylamino)-5-methylpyrimidin-2-yl]amino}-N-[(1S,2R)-2-phenylcyclopropyl]cyclohexanecarboxamide;
cis-4-{[4-(dimethylamino)-5-methylpyrimidin-2-yl]amino}-N-[4-(trifluoromethyl)phenyl]-cyclohexanecarboxamide;
cis-N-[(1S)-1-(4-chlorophenyl)ethyl]-4-{[4-(dimethylamino)-6-methylpyrimidin-2-yl)-amino}cyclohexanecarboxamide;
cis-N-(3,4-difluorobenzyl)-4-{[4-(dimethylamino)-6-methylpyrimidin-2-yl]amino}-cyclohexanecarboxamide;
cis-4-{[4-(dimethylamino)-6-methylpyrimidin-2-yl)amino}-N-[(1S)-1-(4-methoxyphenyl)-ethyl]cyclohexanecarboxamide;
cis-4-{[4-(dimethylamino)-6-methylpyrimidin-2-yl]amino}-N-[(1S)-1-(3-methoxyphenyl)-ethyl]cyclohexanecarboxamide;
cis-N-[(1R)-1-(4-chlorophenyl)ethyl]-4-{[4-(dimethylamino)-6-methylpyrimidin-2-yl]-amino}cyclohexanecarboxamide;
cis-4-{[4-(dimethylamino)-5-methylpyrimidin-2-yl]amino}-N-[(1S)-1-(1-naphthyl)ethyl]-cyclohexanecarboxamide;
cis-N-[(1S)-1-(4-bromophenyl)ethyl]-4-{[4-(dimethylamino)-5-methylpyrimidin-2-yl]-amino}cyclohexanecarboxamide;
cis-4-{[4-(dimethylamino)-5-methylpyrimidin-2-yl]amino}-N-{(1S)-1-[4-(trifluoromethoxy)phenyl]ethyl}cyclohexanecarboxamide;
cis-4-{[4-(dimethylamino)-5-methylpyrimidin-2-yl]amino}-N-[(1R)-1-(2-fluorophenyl)-ethyl]cyclohexanecarboxamide;
cis-N-{(1S)-1-[3,5-bis(trifluoromethyl)phenyl]ethyl}-4-{[4-(dimethylamino)-5-methylpyrimidin-2-yl]amino}cyclohexanecarboxamide;
4-{[4-(dimethylamino)-5-methylpyrimidin-2-yl]amino}-N-{(1S)-1-[3-(trifluoromethyl)-phenyl]ethyl}cyclohexanecarboxamide;
4-{[4-(dimethylamino)-5-methylpyrimidin-2-yl]amino}-N-{(1S)-1-[2-(trifluoromethyl)-phenyl]ethyl)}cyclohexanecarboxamide; and
cis-N-[(1R)-1-(4-chlorophenyl)ethyl]-4-{[4-(dimethylamino)-5-methylpyrimidin-2-yl]-amino}cyclohexanecarboxamide;

or a pharmaceutically acceptable salt, hydrate, or solvate thereof.

In some embodiments of the present invention, $R_1$ is selected from the group consisting of:

(i) $C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:
  - •• halogen,
  - •• $C_{1-5}$ alkyl, and
  - •• $C_{1-5}$ alkyl substituted by halogen,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by halogen,

(iii) carbocyclic aryl, and
carbocyclic aryl substituted by substituent(s) independently selected from the group consisting of:

- halogen,
- $C_{1-10}$ alkyl,
- $C_{1-10}$ alkyl substituted by halogen,
- $C_{1-9}$ alkoxy,
- $C_{1-9}$ alkoxy substituted by substituent(s) independently selected from the group consisting of:
  - •• halogen, and
  - •• carbocyclic aryl,

L is Formula (VII);
Y is $-C(O)NR_5-$;
$R_2$ is $-N(R_{2a})(R_{2b})$ wherein $R_{2a}$ is hydrogen or $C_{1-5}$ alkyl and $R_{2b}$ is $C_{1-5}$ alkyl;
wherein carbocyclic aryl is phenyl; and
halogen is fluoro, chloro, bromo, or iodo;

or a pharmaceutically acceptable salt, hydrate, or solvate thereof.

In some embodiments of the present invention, p is 1 or 2 and each T is independently $C_{1-5}$ alkyl; $R_3$ is hydrogen; $R_4$ is hydrogen or $C_{1-5}$ alkyl; A and B are both single bonds; $R_5$ is hydrogen: or a pharmaceutically acceptable salt, hydrate, or solvate thereof.

In some embodiments, compounds of the present invention are of Formula (I) wherein the compound is selected from the group consisting of:

N-(3,4-dimethoxyphenyl)-N'-(cis-4-{[4-(dimethylamino)-5,6-dimethylpyrimidin-2-yl]-amino}cyclohexyl)urea;
N-(3-chlorophenyl)-N'-(cis-4-{[4-(dimethylamino)-5-methylpyrimidin-2-yl]amino}-cyclohexyl)-N-methylurea;
N-(3,4-dichlorophenyl)-N'-(cis-4-{[4-(dimethylamino)-5-methylpyrimidin-2-yl]amino}-cyclohexyl)-N-methylurea;
N'-(cis-4-{[4-(dimethylamino)-5-methylpyrimidin-2-yl]amino}cyclohexyl)-N-methyl-N-(3-methylphenyl)urea;
N'-(cis-4-{[4-(dimethylamino)-5-methylpyrimidin-2-yl]amino}cyclohexyl)-N-methyl-N-(4-methylphenyl)urea;
N'-(cis-4-{[4-(dimethylamino)-5-methylpyrimidin-2-yl]amino}cyclohexyl)-N-(3-fluorophenyl)-N-methylurea;
N'-(cis-4-{[4-(dimethylamino)-5-methylpyrimidin-2-yl]amino}cyclohexyl)-N-(4-fluorophenyl)-N-methylurea;
N-(4-chlorophenyl)-N'-(cis-4-{[4-(dimethylamino)-5-methylpyrimidin-2-yl]amino}-cyclohexyl)-N-methylurea;

N-(3,4-difluorophenyl)-N'-(cis-4-{[4-(dimethylamino)-5-methylpyrimidin-2-yl]amino}-cyclohexyl)-N-methylurea;
N'-(cis-4-{[4-(dimethylamino)-5-methylpyrimidin-2-yl]amino}cyclohexyl)-N-(3-methoxyphenyl)-N-methylurea;
N'-(cis-4-{[4-(dimethylamino)-5-methylpyrimidin-2-yl]amino}cyclohexyl)-N-(4-methoxyphenyl)-N-methylurea;
N-{1-[3,5-bis(trifluoromethyl)phenyl]-1-methylethyl)-N'-(cis-4-{[4-(dimethylamino)-5-methylpyrimidin-2-yl]amino} cyclohexyl)urea;
N-[1-(4-chlorophenyl)-1-methylethyl]-N'-(cis-4-{[4-(dimethylamino)-5-methylpyrimidin-2-yl]amino}cyclohexyl) urea;
N-[1-(4-chlorophenyl)-1-methylethyl]-N'-(cis-4-{[4-(dimethylamino)-6-methylpyrimidin-2-yl]amino}cyclohexyl) urea;
N-[1-(4-chlorophenyl)-1-methylethyl]-N'-(cis-4-{[4-(dimethylamino)-5-methylpyrimidin-2-yl]amino}cyclohexyl)-N-methylurea;
N-[1-(4-chlorophenyl)-1-methylethyl]-N'-(cis-4-{[4-(dimethylamino)-6-methylpyrimidin-2-yl]amino}cyclohexyl)-N-methylurea;
N-[1-(4-chlorophenyl)cyclopropyl]-N'-(cis-4-{[4-(dimethylamino)-5-methylpyrimidin-2-yl]-amino}cyclohexyl)-N-methylurea;
N-(cis-4-{[4-(dimethylamino)-5-methylpyrimidin-2-yl]amino}cyclohexyl)-N'-(2-methoxyphenyl)urea;
N-(cis-4-{[4-(dimethylamino)-5-methylpyrimidin-2-yl]amino}cyclohexyl)-N'-(3-methoxyphenyl)urea;
N-(3,4-dimethoxyphenyl)-N'-(cis-4-{[4-(dimethylamino)-5-methylpyrimidin-2-yl]amino}-cyclohexyl)urea;
N-(cis-4-{[4-(dimethylamino)-5-methylpyrimidin-2-yl]amino}cyclohexyl)-N'-(4-fluorophenyl)urea;
N-(3,4-difluorophenyl)-N'-(cis-4-{[4-(dimethylamino)-5-methylpyrimidin-2-yl]amino}-cyclohexyl)urea;
N-(cis-4-{[4-(dimethylamino)-5-methylpyrimidin-2-yl]amino}cyclohexyl)-N'-[2-(trifluoromethoxy)phenyl]urea;
N-(4-chlorophenyl)-N'-(cis-4-{[4-(dimethylamino)-5-methylpyrimidin-2-yl]amino}-cyclohexyl)urea;
N-[3,5-bis(trifluoromethyl)phenyl]-N'-(cis-4-{[4-(dimethylamino)-5-methylpyrimidin-2-yl]-amino}cyclohexyl)urea;
N-(4-bromophenyl)-N'-(cis-4-{[4-(dimethylamino)-5-methylpyrimidin-2-yl]amino}-cyclohexyl)urea;
N-(cis-4-{[4-(dimethylamino)-5-methylpyrimidin-2-yl]amino}cyclohexyl)-N'-(2-methylphenyl)urea;
N-benzyl-N'-(cis-4-{[4-(dimethylamino)-5-methylpyrimidin-2-yl]amino}cyclohexyl)urea;
N-[2-chloro-6-(trifluoromethyl)phenyl]-N'-(cis-4-{[4-(dimethylamino)-5-methylpyrimidin-2-yl]amino}cyclohexyl) urea;
N-(cis-4-{[4-(dimethylamino)-5-methylpyrimidin-2-yl]amino}cyclohexyl)-N'-(2,4,6-trichlorophenyl)urea;
N-(2,4-dichlorophenyl)-N'-(cis-4-{[4-(dimethylamino)-5-methylpyrimidin-2-yl]amino}-cyclohexyl)-N-methylurea;
N'-(cis-4-{[4-(dimethylamino)-5-methylpyrimidin-2-yl]amino}cyclohexyl)-N-methyl-N-[2-(trifluoromethoxy)phenyl] urea;
N-(4-chlorophenyl)-N'-(cis-4-{[4-(dimethylamino)-5-methylpyrimidin-2-yl]amino}-cyclohexyl)-N-ethylurea;
N-[3,5-bis(trifluoromethyl)phenyl]-N'-(cis-4-{[4-(dimethylamino)-5-methylpyrimidin-2-yl]amino}cyclohexyl)-N-ethylurea;
N'-(cis-4-{[4-(dimethylamino)-5-methylpyrimidin-2-yl]amino}cyclohexyl)-N-(2-fluorophenyl)-N-methylurea;
N'-(cis-4-{[4-(dimethylamino)-5-methylpyrimidin-2-yl]amino}cyclohexyl)-N-ethyl-N-[2-(trifluoromethoxy)phenyl] urea;
N'-(cis-4-{[4-(dimethylamino)-5-methylpyrimidin-2-yl]amino}cyclohexyl)-N-ethyl-N-phenylurea;
N'-(cis-4-}[4-(dimethylamino)-5-methylpyrimidin-2-yl]amino}cyclohexyl)-N-ethyl-N-(3-methylphenyl)urea; and
1-(2,3-dichloro-phenyl)-3-[cis-4-(4-dimethylamino-5-methyl-pyrimidin-2-ylamino)-cyclohexylmethyl]-urea;

or a pharmaceutically acceptable salt, hydrate, or solvate thereof.

In some embodiments, compounds of the present invention are of Formula (I) wherein the compound is selected from the group consisting of:

N-(3,4-dimethoxyphenyl)-N'-(cis-4-{[4-(dimethylamino)-5,6-dimethylpyrimidin-2-yl]-amino}cyclohexyl)urea;
N-(3-chlorophenyl)-N'-(cis-4-{[4-(dimethylamino)-5-methylpyrimidin-2-yl]amino}-cyclohexyl)-N-methylurea;
N-(3,4-dichlorophenyl)-N'-(cis-4-{[4-(dimethylamino)-5-methylpyrimidin-2-yl]amino}-cyclohexyl)-N-methylurea;
N'-(cis-4-{[4-(dimethylamino)-5-methylpyrimidin-2-yl]amino}cyclohexyl)-N-methyl-N-(3-methylphenyl)urea;
N'-(cis-4-{[4-(dimethylamino)-5-methylpyrimidin-2-yl]amino}cyclohexyl)-N-methyl-N-(4-methylphenyl)urea;
N'-(cis-4-{[4-(dimethylamino)-5-methylpyrimidin-2-yl]amino}cyclohexyl)-N-(3-fluorophenyl)-N-methylurea;
N'-(cis-4-{[4-(dimethylamino)-5-methylpyrimidin-2-yl]amino}cyclohexyl)-N-(4-fluorophenyl)-N-methylurea;
N-(4-chlorophenyl)-N'-(cis-4-{[4-(dimethylamino)-5-methylpyrimidin-2-yl]amino}-cyclohexyl)-N-methylurea;
N-(3,4-difluorophenyl)-N'-(cis-4-{[4-(dimethylamino)-5-methylpyrimidin-2-yl]amino}-cyclohexyl)-N-methylurea;
N'-(cis-4-{[4-(dimethylamino)-5-methylpyrimidin-2-yl]amino}cyclohexyl)-N-(3-methoxyphenyl)-N-methylurea;
N'-(cis-4-{[4-(dimethylamino)-5-methylpyrimidin-2-yl]amino}cyclohexyl)-N-(4-methoxyphenyl)-N-methylurea;
N-[1-(4-chlorophenyl)-1-methylethyl]-N'-(cis-4-{[4-(dimethylamino)-5-methylpyrimidin-2-yl]amino}cyclohexyl)urea;
N-[1-(4-chlorophenyl)-1-methylethyl]-N'-(cis-4-{[4-(dimethylamino)-6-methylpyrimidin-2-yl]amino}cyclohexyl) urea;
N-[1-(4-chlorophenyl)-1-methylethyl]-N'-(cis-4-{[4-(dimethylamino)-5-methylpyrimidin-2-yl]amino}cyclohexyl)-N-methylurea;
N-(cis-4-{[4-(dimethylamino)-5-methylpyrimidin-2-yl]amino}cyclohexyl)-N'-(4-fluorophenyl)urea;
N-(cis-4-{[4-(dimethylamino)-5-methylpyrimidin-2-yl]amino}cyclohexyl)-N'-[2-(trifluoromethoxyl)phenyl]area;
N-(4-bromophenyl)-N'-(cis-4-{[4-(dimethylamino)-5-methylpyrimidin-2-yl]amino}-cyclohexyl)urea;
N-(cis-4-{[4-(dimethylamino)-5-methylpyrimidin-2-yl]amino}cyclohexyl)-N'-(2-methylphenyl)urea;
N-(cis-4-{[4-(dimethylamino)-5-methylpyrimidin-2-yl]amino}cyclohexyl)-N'-(2,4,6-trichlorophenyl)urea;
N-(2,4-dichlorophenyl-N'-(cis-4-{[4-(dimethylamino)-5-methylpyrimidin-2-yl]amino}-cyclohexyl)-N-methylurea;
N'-(cis-4-{[4-(dimethylamino)-5-methylpyrimidin-2-yl]amino}cyclohexyl)-N-methyl-N-[2-(trifluoromethoxy)phenyl] urea;
N-(4-chlorophenyl)-N'-(cis-4-{[4-(dimethylamino)-5-methylpyrimidin-2-yl]amino}-cyclohexyl)-N-ethylurea;
N-[3,5-bis(trifluoromethyl)phenyl]-N'-(cis-4-{[4-(dimethylamino)-5-methylpyrimidin-2-yl]amino}cyclohexyl)-N-ethylurea;
N'-(cis-4-{[4-(dimethylamino)-5-methylpyrimidin-2-yl]amino}cyclohexyl)-N-ethyl-N-phenylurea;
N'-(cis-4-{[4-(dimethylamino)-5-methylpyrimidin-2-yl]amino}cyclohexyl)-N-ethyl-N-(3-methylphenyl)urea; and
1-(2,3-dichloro-phenyl)-3-[cis-4-(4-dimethylamino-5-methyl-pyrimidin-2-ylamino)-cyclohexylmethyl]-urea;

or a pharmaceutically acceptable salt, hydrate, or solvate thereof.

In some embodiments of the present invention, $R_1$ is selected from the group consisting of:

heterocyclyl, and
heterocyclyl substituted by substituent(s) independently selected from the group consisting of:

- carbocyclic aryloxy,
- carbocyclic aryloxy substituted by substituent(s) independently selected from the group consisting of:

  - •• halogen, and
  - •• $C_{1-5}$ alkoxy,

L is Formula (X) or (XI);
Y is -C(O)-;
$R_2$ is $-N(R_{2a})(R_{2b})$ wherein $R_{2a}$ is $C_{1-5}$ alkyl and $R_{2b}$ is $C_{1-5}$ alkyl;
wherein carbocyclic aryl is phenyl;
heterocyclyl is pyridyl; and
halogen is fluoro, chloro, bromo, or iodo;

or a pharmaceutically acceptable salt, hydrate, or solvate thereof.

In some embodiments of the present invention, p is 1 and T is $C_{1-5}$ alkyl; $R_3$ and $R_4$ are both hydrogen; A is a single bond and B is $-CH_2-$; or a pharmaceutically acceptable salt, hydrate, or solvate thereof.

In some embodiments of the present invention, $R_1$ is selected from the group consisting of:

(i) carbocyclic aryl, and
carbocyclic aryl substituted by substituent(s) independently selected from the group consisting of:

- halogen,
- $C_{1-10}$ alkyl, and
- $C_{1-10}$ alkyl substituted by halogen,

(ii) heterocyclyl,
L is Formula (VII); and
Y is $-S(O)_2-$;
$R_2$ is $-N(R_{2a})(R_{2b})$ wherein $R_{2a}$ is $C_{1-5}$ alkyl and $R_{2b}$ is $C_{1-5}$ alkyl;
wherein carbocyclic aryl is phenyl or naphthyl;
heterocyclyl is furyl; and
halogen is fluoro, chloro, bromo, or iodo;

or a pharmaceutically acceptable salt, hydrate, or solvate thereof.

In some embodiments of the present invention, p is 1 and T is $C_{1-10}$ alkyl; $R_3$ and $R_4$ are both hydrogen, and A and B are both single bonds; or a pharmaceutically acceptable salt, hydrate, or solvate thereof.

In some embodiments, a compound of the present invention is:

4-chloro-N-(cis-4-{[4-(dimethylamino)-5-methylpyrimidin-2-yl]amino}cyclohexyl)-benzenesulfonamide;

or a pharmaceutically acceptable salt, hydrate, or solvate thereof.

In some embodiments of the present invention, wherein $R_1$ is selected from hydrogen, $-CO_2{}^tBu$, or-$CO_2Bn$ (Bn is a benzyl group);

$R_2$ is selected from the group consisting of:

hydrogen, halogen, hydroxy, carboxy, carbamoyl, amino, $C_{1-5}$ alkyl, $C_{1-5}$ alkyl substituted by halogen, $C_{1-5}$ alkyl substituted by hydroxy, $C_{1-5}$ alkyl substituted by carboxy, $C_{1-5}$ alkyl substituted by carbamoyl, $C_{1-5}$ alkoxy, $C_{1-5}$ alkoxy substituted by halogen, $-N(R_{2a})(R_{2b})$;

wherein $R_{2a}$ is hydrogen or $C_{1-5}$ alkyl and $R_{2b}$ is $C_{1-5}$ alkyl, $C_{3-6}$ cycloalkyl, or $C_{1-5}$ alkyl substituted by substituent (s) independently selected from the group consisting of:

- halogen,
- hydroxy,
- carboxy,
- carbamoyl,
- $C_{1-5}$ alkoxy,
- amino,
- $C_{3-6}$ cycloalkyl,

or $R_2$ is methylamino or dimethylamino when Q is Formula (II);

Each T is independently selected from the group consisting of halogen, hydroxy, carboxy, carbamoyl, amino, cyano, nitro, $C_{1-5}$ alkyl, $C_{1-5}$ alkyl substituted by halogen, $C_{1-5}$ alkyl substituted by hydroxy, $C_{1-5}$ alkyl substituted by carboxy, $C_{1-5}$ alkyl substituted by carbamoyl, $C_{2-5}$ alkenyl, $C_{2-5}$ alkynyl, $C_{3-6}$ cycloalkyl, $C_{1-5}$ alkoxy, $C_{1-5}$ alkoxy substituted by halogen, carbocyclic aryl, heterocyclyl, and $-N(R_{2a})(R_{2b})$;

p is 0, 1, 2, 3, 4 or 5;

L is selected from the group consisting of Formula (VII), (X), (XI), (XV), (XVIII), or (XIX): wherein $R_3$ and $R_4$ are independently hydrogen or $C_{1-5}$ alkyl; and A and B are independently a single bond or $-CH_2-$; and

Y is a single bond;

or a pharmaceutically acceptable salt, hydrate, or solvate thereof.

One aspect of the present invention pertains to pharmaceutical compositions comprising at least one compound, as described herein, in combination with a pharmaceutically acceptable carrier.

One aspect of the present invention pertains to methods for the prophylaxis or treatment of improving memory function, sleeping and arousal, anxiety, depression, mood disorders, seizure, obesity, diabetes, appetite and eating disorders, cardiovascular disease, hypertension, dyslipidemia, myocardial infarction, binge eating disorders including bulimia, anorexia, mental disorders including manic depression, schizophrenia, delirium, dementia, stress, cognitive disorders, attention deficit disorder, substance abuse disorders and dyskinesias including Parkinson's disease, epilepsy, and addiction comprising administering to an individual suffering from the condition a therapeutically effective amount of a compound, as described herein, or a pharmaceutical composition thereof.

One aspect of the present invention pertains to methods for the prophylaxis or treatment of an eating disorder, obesity or an obesity related disorder comprising administering to an individual suffering from the condition a therapeutically effective amount of a compound, as described herein, or a pharmaceutical composition thereof.

One aspect of the present invention pertains to methods for the prophylaxis or treatment of anxiety, depression, schizophrenia, addiction, or epilepsy comprising administering to an individual suffering from the condition a therapeutically effective amount of a compound, as described herein, or a pharmaceutical composition.

One aspect of the present invention pertains to compounds of the present invention, as described herein, or a pharmaceutical composition thereof, for use in a method of treatment of the human or animal body by therapy.

One aspect of the present invention pertains to compounds of the present invention, as described herein, or a pharmaceutical composition thereof, for use in a method of prophylaxis or treatment of an eating disorder, obesity or an obesity related disorder of the human or animal body by therapy.

One aspect of the present invention pertains to compounds of the present invention, as described herein, or

a pharmaceutical composition thereof, for use in a method of prophylaxis or treatment of anxiety, depression, schizophrenia, addiction, or epilepsy of the human or animal body by therapy.

One aspect of the present invention pertains to compounds of the present invention, as described herein, for the manufacture of a medicament for use in the prophylaxis or treatment of an eating disorder, obesity or obesity related disorders.

One aspect of the present invention pertains to compounds of the present invention, as described herein, for the manufacture of a medicament for use in the prophylaxis or treatment of anxiety, depression, schizophrenia, addiction, or epilepsy.

One aspect of the present invention pertains to methods of decreasing food intake of an individual comprising administering to the individual a therapeutically effective amount of a compound, as described herein, or a pharmaceutical composition thereof.

One aspect of the present invention pertains to methods of inducing satiety in an individual comprising administering to said individual a therapeutically effective amount of a compound, as described herein, or a pharmaceutical composition thereof.

One aspect of the present invention pertains to methods of controlling or reducing weight gain in an individual comprising administering to said individual a therapeutically effective amount of a compound, as described herein, or a pharmaceutical composition thereof.

One aspect of the present invention pertains to methods of modulating a MCH receptor in an individual comprising contacting the receptor with a compound, as described herein. In some embodiments, the compound is an antagonist. In some embodiments, the modulation of the MCH receptor is for the prophylaxis or treatment of an eating disorder, obesity or obesity related disorder. In some embodiments, the modulation of the MCH receptor reduces food intake of the individual. In some embodiments, the modulation of the MCH receptor induces satiety in the individual. In some embodiments, the modulation of the MCH receptor controls or reduces weight gain of the individual. In some embodiments, the modulation of the MCH receptor is for prophylaxis or treatment of anxiety, depression, schizophrenia, addiction, or epilepsy.

In some embodiments, the individual is a mammal.

In some embodiments, the mammal is a human.

In some embodiments, the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

One aspect of the present invention pertains to methods of producing a pharmaceutical composition comprising admixing a compound, as described herein, and a pharmaceutically acceptable carrier.

One aspect of the present invention pertains to methods for the prophylaxis or treatment of improving memory function, sleeping and arousal, anxiety, depression, mood disorders, seizure, obesity, diabetes, appetite and eating disorders, cardiovascular disease, hypertension, dyslipidemia, myocardial infarction, binge eating disorders including bulimia, anorexia, mental disorders including manic depression, schizophrenia, delirium, dementia, stress, cognitive disorders, attention deficit disorder, substance abuse disorders and dyskinesias including Parkinson's disease, epilepsy, and addiction in mammals in need of such treatment comprising administering to the mammal a therapeutically effective amount of a compound, as described herein, or pharmaceutical composition thereof.

One embodiment of the invention includes any compound of the invention which selectively binds an MCH receptor, such selective binding is preferably demonstrated by a Ki for one or more other GPCR(s), preferably NPY, being at least 10-fold greater than the Ki for any particular MCH receptor, preferable MCHR1.

As used herein, the term "alkyl" is intended to denote hydrocarbon compounds including straight chain and branched chain, including for example but not limited to methyl, ethyl, n-propyl, isopropyl, n-butyl, see-butyl, tert-butyl, n-pentyl, isopentyl, tert-pentyl, n-hexyl, and the like.

The term "alkoxy" is intended to denote substituents of the formula -O-alkyl.

At various places in the present specification substituents of compounds of the invention are disclosed in groups. It is specifically intended that the invention include each and every individual subcombination of the members of such groups.

G-protein coupled receptors (GPCRs) represent a major class of cell surface receptors with which many neurotransmitters interact to mediate their effects. GPCRs are predicted to have seven membrane-spanning domains and are coupled to their effectors via G-proteins linking receptor activation with intracellular biochemical sequelae such as stimulation of adenylyl cyclase. Melanin Concentrating Hormone (MCH), a cyclic peptide, has been identified as the endogenous ligand of the orphan G-protein coupled receptor SLC-1. See, for example, Shimomura et al., Biochem. Biophys. Res. Commun. 261, 622-26 (1999). Studies have indicated that MCH acts as a neurotransmitter/modulator/regulator to alter a number of behavioral responses.

Mammalian MCH (19 amino acids) is highly conserved between rat, mouse, and human, exhibiting 100% amino acid identity, but its physiological roles are less clear. MCH has been reported to participate in a variety of

processes including feeding, water balance, energy metabolism, general arousal/attention state, memory and cognitive functions, and psychiatric disorders. For reviews, see 1. Baker, Int. Rev. Cytol. 126:1-47 (1991); 2. Baker, TEM 5: 120-126 (1994); 3. Nahon, Critical Rev. in Neurobiol 221:221-262, (1994); 4. Knigge et al., Peptides 18(7):1095-1097, (1996). The role of MCH in feeding or body weight regulation is supported by Qu et al., Nature 380:243-247, (1996), demonstrating that MCH is over expressed in the hypothalamus of ob/ob mice compared with ob/+mice, and that fasting further increased MCH mRNA in both obese and normal mice during fasting. MCH also stimulated feeding in normal rats when injected into the lateral ventricles as reported by Rossi et al., Endocrinology 138:351-355, (1997). MCH also has been reported to functionally antagonize the behavioral effects of $\alpha$-MSH; see: Miller et al., Peptides 14:1-10, (1993); Gonzalez et al, Peptides 17:171-177, (1996); and Sanchez et al., Peptides 18:3933-396, (1997). In addition, stress has been shown to increase POMC mRNA levels while decreasing the MCH precursor preproMCH (ppMCH) mRNA levels; Presse et al., Endocrinology 131:1241-1250, (1992). Thus MCH can serve as an integrative neuropeptide involved in the reaction to stress, as well as in the regulation of feeding and sexual activity; Baker, Int. Rev. Cytol. 126: 1-47, (1991); Knigge et al., Peptides 17:1063-1073, (1996).

The localization and biological activities of MCH peptide suggest that the modulation of MCH receptor activity can be useful in a number of therapeutic applications. MCH is expressed in the lateral hypothalamus, a brain area implicated in the regulation of thirst and hunger: Grillon et al., Neuropeptides 31:131-136, (1997); recently orexins A and B, which are potent orexigenic agents, have been shown to have very similar localization to MCH in the lateral hypothalamus; Sakurai et al., Cell 92:573-585 (1998). MCH mRNA levels in this brain region are increased in rats after 24 hours of food-deprivation; Herve and Fellmann, Neurpeptides 31:237-242 (1997); after insulin injection, a significant increase in the abundance and staining intensity of MCH immunoreactive perikarya and fibres was observed concurrent with a significant increase in the level of MCH mRNA; Bahjaoui-Bouhaddi et al., Neuropeptides 24:251-258, (1994). Consistent with the ability of MCH to stimulate feeding in rats; Rossi et al., Endocrinology 138:351-355, (1997); is the observation that MCH mRNA levels are upregulated in the hypothalami of obese ob/ob mice; Qu et al., Nature 380: 243-247, (1996); and decreased in the hypothalami of rats treated with leptin, whose food intake and body weight gains are also decreased; Sahu, Endocrinology 139:795-798, (1998). MCH appears to act as a functional antagonist of the melanocortin system in its effects on food intake and on hormone secretion within the HPA (hypothalamopituitary/ adrenal axis); Ludwig et al., Am. J. Physiol. Endocrinol. Metab. 274:E627-E633, (1998). Together these data suggest a role for endogenous MCH in the regulation of energy balance and response to stress, and provide a rationale for the development of specific compounds acting at MCH receptors for use in the treatment of obesity and stress-related disorders.

Accordingly, a MCH receptor antagonist is desirable for the prophylaxis or treatment of obesity or obesity related disorders. An obesity related disorder is a disorder that has been directly or indirectly associated to obesity, such as, type II diabetes, syndrome X, impaired glucose tolerance, dyslipidaemia, hypertension, coronary heart disease and other cardiovascular disorders including atherosclerosis, insulin resistance associated with obesity and psoriasis, for treating diabetic complications and other diseases such as polycystic ovarian syndrome (PCOS), certain renal diseases including diabetic nephropathy, glomerulonephritis, glomerular sclerosis, nephrotic syndrome, hypertensive nephrosclerosis, end-stage renal diseases and microalbuminuria as well as certain eating disorders.

In species studied to date, a major portion. of the neurons of the MCH cell group occupies a rather constant location in those areas of the lateral hypothalamus and subthalamus where they lie and can be a part of some of the so-called "extrapyramidal" motor circuits. These involve substantial striato- and pallidofugal pathways involving the thalamus and cerebral cortex, hypothalamic areas, and reciprocal connections to subthalamic nucleus, substantia nigra, and mid-brain centers; Bittencourt et al., J. Comp. Neurol. 319:218-245, (1992). In their location, the MCH cell group may offer a bridge or mechanism for expressing hypothalamic visceral activity with appropriate and coordinated motor activity. Clinically it can be of some value to consider the involvement of this MCH system in movement disorders, such as Parkinson's disease and Huntingdon's Chorea in which extrapyramidal circuits are known to be involved.

Human genetic linkage studies have located authentic hMCH loci on chromosome 12 (12q23-24) and the variant hMCH loci on chromosome 5 (5q12-13) (Pedeutour et al., 1994). Locus 12q23-24 coincides with a locus to which autosomal dominant cerebellar ataxia type II (SCA2) has been mapped; Auburger et al., Cytogenet. Cell. Genet. 61:252-256, (1992); Twells et al., Cytogenet. Cell. Genet. 61:262-265, (1992). This disease comprises neurodegenerative disorders, including an olivopontocerebellar atrophy. Furthermore, the gene for Darier's disease, has been mapped to locus 12q23-24; Craddock et al., Hum. Mol. Genet. 2:1941-1943, (1993). Dariers' disease is characterized by abnormalities I keratinocyte adhesion and mental illnesses in some families. In view of the functional and neuroanatomical patterns of the MCH neural system in the rat and human brains, the MCH gene can represent a good candidate for SCA2 or Darier's disease. Interestingly, diseases with high social impact have been mapped to this locus. Indeed, the gene responsible for chronic or acute forms of spinal muscular atrophies has been assigned to chromosome 5q12-13 using genetic linkage analysis; Melki et al., Nature (London) 344:767-768, (1990); Westbrook et al., Cytogenet. Cell. Genet. 61:225-231, (1992). Furthermore, independent lines of evidence support the assignment of a major schizophrenia locus to chromosome 5q11.2-13.3; Sherrington et al., Nature (London) 336:164-167, (1988); Bassett et al.,

Lancet 1:799-801, (1988); Gilliam et al., Genomics 5:940-944, (1989). The above studies suggest that MCH can play a role in neurodegenerative diseases and disorders of emotion.

Additional therapeutic applications for MCH-related compounds are suggested by the observed effects of MCH in other biological systems. For example, MCH can regulate reproductive functions in male and female rats. MCH transcripts and MCH peptide were found within germ cells in testes of adult rats, suggesting that MCH can participate in stem cell renewal and/or differentiation of early spermatocytes; Hervieu et al., Biology of Reduction 54:1161-1172, (1996). MCH injected directly into the medial preoptic area (MPOA) or ventromedial nucleus (VMN) stimulated sexual activity in female rats; Gonzalez et al., Peptides 17:171-177, (1996). In ovariectomized rats primed with estradiol, MCH stimulated luteinizing hormone (LH) release while anti-MCH antiserum inhibited LH release; Gonzalez et al., Neuroendocrinology 66:254-262, (1997). The zona incerta, which contains a large population of MCH cell bodies, has previously been identified as a regulatory site for the pre-ovulatory LH surge; MacKenzie et al., Neuroendocrinology 39:289-295, (1984). MCH has been reported to influence release of pituitary hormones including ACTH and oxytocin. MCH analogues can also be useful in treating epilepsy. In the PTZ seizure model, injection of MCH prior to seizure induction prevented seizure activity in both rats and guinea pigs, suggesting that MCH-containing neurons can participate in the neural circuitry underlying PTZ-induced seizure; Knigge and Wagner, Peptides 18:1095-1097, (1997). MCH has also been observed to affect behavioral correlates of cognitive functions. MCH treatment hastened extinction of the passive avoidance response in rats; McBride et al., Peptides 15:757-759, (1994); raising the possibility that MCH receptor antagonists can be beneficial for memory storage and/or retention. A possible role for MCH in the modulation or perception of pain is supported by the dense innervation of the periaqueductal grey (PAG) by MCH-positive fibers. Finally, MCH can participate in the regulation of fluid intake. ICV infusion of MCH in conscious sheep produced diuretic, natriuretic, and kaliuretic changes in response to increased plasma volume; Parkes, J. Neuroendocrinol. 8:57-63, (1996). Together with anatomical data reporting the presence of MCH in fluid regulatory areas of the brain, the results indicate that MCH can be an important peptide involved in the central control of fluid homeostasis in mammals.

In a recent citation MCHR1 antagonists surprisingly demonstrated their use as an anti-depressants and/or anti-anxiety agents. MCHR1 antagonists have been reported to show antidepressant and anxiolytic activities in rodent models, such as, social interaction, forced swimming test and ultrasonic vocalization. Therefore, MCHR1 antagonists could be useful to independently treat subjects with depression and/or anxiety. Also, MCHR1 antagonists could be useful to treat subjects that suffer from depression and/or anxiety and obesity.

This invention provides a method of treating an abnormality in a subject wherein the abnormality is alleviated by decreasing the activity of a mammalian MCH 1 receptor which comprises administering to the subject an amount of a compound which is a mammalian MCH1 receptor antagonist effective to treat the abnormality. In separate embodiments, the abnormality is a regulation of a steroid or pituitary hormone disorder, an epinephrine release disorder, an anxiety disorder, genta gastrointestinal disorder, a cardiovascular disorder, an electrolyte balance disorder, hypertension, diabetes, a respiratory disorder, asthma, a reproductive function disorder, an immune disorder, an endocrine disorder, a musculoskeletal disorder, a neuroendocrine disorder, a cognitive disorder, a memory disorder, a sensory modulation and transmission disorder, a motor coordination disorder, a sensory integration disorder, a motor integration disorder, a dopaminergic function disorder, a sensory transmission disorder, an olfaction disorder, a sympathetic innervation disorder, an affective disorder, a stress-related disorder, a fluid-balance disorder, a seizure disorder, pain, psychotic behavior, morphine tolerance, opiate addiction or migraine.

Compositions of the invention can conveniently be administered in unit dosage form and can be prepared by any of the methods well known in the pharmaceutical art, for example, as described in *Remington's Pharmaceutical Sciences* (Mack Pub. Co., Easton, PA, 1980).

The compounds of the invention can be employed as the sole active agent in a pharmaceutical or can be used in combination with other active ingredients which could facilitate the therapeutic effect of the compound.

Compounds of the present invention or a solvate or physiologically functional derivative thereof can be used as active ingredients in pharmaceutical compositions, specifically as a MCH receptor antagonists. By the term "active ingredient" is defined in the context of a "pharmaceutical composition" and shall mean a component of a pharmaceutical composition that provides the primary pharmaceutical benefit, as opposed to an "inactive ingredient" which would generally be recognized as providing no pharmaceutical benefit. The term "pharmaceutical composition" shall mean a composition comprising at one active ingredient and at least one ingredient that is not an active ingredient (for example and not limitation, a filler, dye, or a mechanism for slow release), whereby the composition is amenable to use for a specified, efficacious outcome in a mammal (for example, and not limitation, a human).

Pharmaceutical compositions, including, but not limited to, pharmaceutical compositions, comprising at least one compound of the present invention and/or an acceptable salt or solvate thereof (*e.g.*, a pharmaceutically acceptable salt or solvate) as an active ingredient combined with at least one carrier or excipient (*e.g.*, pharmaceutical carrier or excipient) can be used in the treatment of clinical conditions for which a MCH receptor antagonist is indicated. At least one compound of the present invention can be combined with the carrier in either solid or liquid form in a unit dose formulation. The pharmaceutical carrier must be compatible with the other ingredients in the composition and must be

tolerated by the individual recipient. Other physiologically active ingredients can be incorporated into the pharmaceutical composition of the invention if desired, and if such ingredients are compatible with the other ingredients in the composition. Formulations can be prepared by any suitable method, typically by uniformly mixing the active compound (s) with liquids or finely divided solid carriers, or both, in the required proportions, and then, if necessary, forming the resulting mixture into a desired shape.

Conventional excipients, such as binding agents, fillers, acceptable wetting agents, tabletting lubricants, and disintegrants can be used in tablets and capsules for oral administration. Liquid preparations for oral administration can be in the form of solutions, emulsions, aqueous or oily suspensions, and syrups. Alternatively, the oral preparations can be in the form of dry powder that can be reconstituted with water or another suitable liquid vehicle before use. Additional additives such as suspending or emulsifying agents, non-aqueous vehicles (including edible oils), preservatives, and flavorings and colorants can be added to the liquid preparations. Parenteral dosage forms can be prepared by dissolving the compound of the invention in a suitable liquid vehicle and filter sterilizing the solution before filling and sealing an appropriate vial or ampoule. These are just a few examples of the many appropriate methods well known in the art for preparing dosage forms.

It is noted that when the MCH receptor antagonists are utilized as active ingredients in a pharmaceutical composition, these are not intended for use only in humans, but in other non-human mammals as well. Indeed, recent advances in the area of animal health-care mandate that consideration be given for the use of MCH receptor antagonists for the treatment of obesity in domestic animals (*e.g.*, cats and dogs), and MCH receptor antagonists in other domestic animals where no disease or disorder is evident (*e.g.*, food-oriented animals such as cows, chickens, fish, etc.). Those of ordinary skill in the art are readily credited with understanding the utility of such compounds in such settings.

Pharmaceutically acceptable salts of the compounds of the invention can be prepared by reacting the free acid or base forms of these compounds with the appropriate base or acid in water, in an organic solvent, or in a mixture of the two; generally, nonaqueous media like ether, ethyl acetate, ethanol, isopropanol, dioxane, or acetonitrile are preferred. For instance, when the compound (I) possesses an acidic functional group, it can form an inorganic salt such as an alkali metal salt (e.g., sodium salt, potassium salt, etc.), an alkaline earth metal salt (e.g. calcium salt, magnesium salt, barium salt, etc.), and an ammonium salt. When the compound (I) possesses a basic functional group, it can form an inorganic salt (e.g., hydrochloride, sulfate, phosphate, hydrobromate, etc.) or an organic salt (e.g., acetate, maleate, fumarate, succinate, methanesulfonate, p-toluenesulfonate, citrate, tartrate, etc.).

## Other Utilities

Another object of the present invention relates to radiolabelled compounds of Formula (Ia) that would be useful not only in radio-imaging but also in assays, both in vitro and in vivo, for localizing and quantitating MCH in tissue samples, including human, and for identifying MCH ligands by inhibition binding of a radiolabelled compound. It is a further object of this invention to develop novel **MCH** assays of which comprise such radiolabelled compounds.

Suitable radionuclides that can be incorporated in compounds of the present invention include but are not limited to $^{3}H$ (also written as T), $^{11}C$ ,$^{14}C$, $^{18}F$, $^{125}I$ ,$^{82}Br$ ,$^{123}I$, $^{124}I$, $^{125}I$, $^{131}I$, $^{75}Br$ ,$^{76}Br$, $^{15}O$, $^{13}N$, $^{35}S$ and $^{77}Br$. The radionuclide that is incorporated in the instant radiolabelled compounds will depend on the specific application of that radiolabelled compound. Thus, for in vitro **MCH** labeling and competition assays, compounds that incorporate $^{3}H$, $^{14}C$, $^{125}I$, $^{131}I$, $^{35}S$ or $^{82}Br$ will generally be most useful. For radio-imaging applications $^{11}C$, $^{18}F$, $^{125}I$, $^{123}I$, $^{124}I$, $^{131}I$, $^{75}Br$, $^{76}Br$ or $^{77}Br$ will generally be most useful.

It is understood that a "radio-labelled " or "labelled compound" is a compound of Formula (**Ia**) that has incorporated at least one radionuclide; in some embodiments the radionuclide is selected from the group consisting of $^{3}M$, $^{14}C$, $^{125}I$ , $^{35}S$ and $^{82}$ Br; in some embodiments the radionuclide $^{3}H$ or $^{14}C$. Moreover, it should be understood that all of the atoms represented in the compounds of the invention can be either the most commonly occurring isotope of such atoms or the more scarce radio-isotope or nonradio-active isotope.

Synthetic methods for incorporating radio-isotopes into organic compounds including those applicable to those compounds of the invention are well known in the art and include incorporating activity levels of tritium into target molecules include: A. Catalytic Reduction with Tritium Gas-This procedure normally yields high specific activity products and requires halogenated or unsaturated precursors. B. Reduction with Sodium Borohydride [$^{3}H$] - This procedure is rather inexpensive and requires precursors containing reducible functional groups such as aldehydes, ketones, lactones, esters, and the like. C. Reduction with Lithium Aluminum Hydride [$^{3}H$] - This procedure offers products at almost theoretical specific activities. It also requires precursors containing reducible functional groups such as aldehydes, ketones, lactones, esters, and the like. **D.** Tritium Gas Exposure Labeling - This procedure involves exposing precursors containing exchangeable protons to tritium gas in the presence of a suitable catalyst. **E.** N-Methylation using Methyl Iodide [$^{3}H$] - This procedure is usually employed to prepare O-methyl or N-methyl ($^{3}H$) products by treating appropriate precursors with high specific activity methyl iodide ($^{3}H$). This method in general allows for high specific activity, such as about 80-87 Ci/mmol.

Synthetic methods for incorporating activity levels of $^{125}I$ into target molecules include: **A.** Sandmeyer and like reactions - This procedure transforms an aryl or heteroaryl amine into a diazonium salt, such as a tetrafluoroborate salt, and subsequently to $^{125}I$ labelled compound using $Na^{125}I$. A represented procedure was reported by Zhu, D.-G. and co-workers in *J. Org. Chem.* **2002**, *67*, 943-948. **B.** Ortho $^{125}$Iodination of phenols - This procedure allows for the incorporation of $^{125}I$ at the ortho position of a phenol as reported by Collier, T. L. and co-workers in *J. Labelled Compd Radiopharm.* 1999, *42*, S264-S266. **C.** Aryl and heteroaryl bromide exchange with $^{125}I$ - This method is generally a two step process. The first step is the conversion of the aryl or heteroaryl bromide to the corresponding tri-alkyltin intermediate using for example, a Pd catalyzed reaction [i.e. $Pd(Ph_3P)_4$] or through an aryl or heteroaryl lithium, in the presence of a tri-alkyltinhalide or hexaalkylditin [e.g., $(CH_3)_3SnSn(CH_3)_3$]. A represented procedure was reported by Bas, M.-D. and co-workers in *J. Labelled Compd Radiopharm.* **2001**, *44*, S280-S282.

A radiolabelled **MCH** compound of Formula (**I**) can be used in a screening assay to identify/evaluate compounds. In general terms, a newly synthesized or identified compound (i.e., test compound) can be evaluated for its ability to reduce binding of the "radiolabelled compound of Formula (**Ia**)" to the **MCH** receptor. Accordingly, the ability of a test compound to compete with the "radio-labelled compound of Formula (Ia)" for the binding to the **MCH** receptor directly correlates to its binding affinity.

The labelled compounds of the present invention bind to the **MCH** receptor. In one embodiment the labelled compound has an $IC_{50}$ less than about 500 μM, in another embodiment the labelled compound has an $IC_{50}$ less than about 100 μM, in yet another embodiment the labelled compound has an $IC_{50}$ less than about 10 μM, in yet another embodiment the labelled compound has an $IC_{50}$ less than about 1 μM, and in still yet another embodiment the labelled inhibitor has an $IC_{50}$ less than about 0.1 μ M.

**Preparation of Compound of Formula (I) - General synthetic methods**

The novel substituted quinolines, tetrahydroquinazolines, and pyrimidines of the present invention can be readily prepared according to a variety of synthetic manipulations, all of which would be familiar to one skilled in the art. Preferred methods for the preparation of compounds of the present invention include, but are not limited to, those described in Scheme 1-24.

The common intermediate (F) of the novel substituted quinolines can be prepared as shown in Scheme 1. Commercially available 2,4-dihydroxyquinoline (A) , wherein T and p is as defined above, is converted to 2,4-dihalo-quinoline (B) by a halogenating agent with or without a base (wherein X is halogen such as chloro, bromo, or iodo). The halogenating agent includes phosphorous oxychloride ($POCl_3$), phosphorous oxybromide ($POBr_3$), or phosphorus pentachloride ($PCl_5$). The base includes a tertiary amine (preferably *N,N*-diisopropylethylamine, etc.) or an aromatic amine (preferably *N,N*-dimethylaniline, etc.). Reaction temperature ranges from about 100°C to 200°C, preferably about 140°C to 180°C.

The halogen of 4-position of 2,4-dihalo-quinoline (B) is selectively substituted by a primary or secondary amine ($HNR_{2a}R_{2b}$, wherein $R_{2a}$ and $R_{2b}$ are as defined above) with or without a base in an inert solvent to provide the corresponding 4-substitued amino adduct (C). The base includes an alkali metal carbonate (preferably sodium carbonate or potassium carbonate, etc.), an alkali metal hydroxide (preferably sodium hydroxide, etc.), or a tertiary amine (preferably *N,N*-diisopropylethylan-tine, triethylamine, or *N*-methylmorpholine, etc.). The inert solvent includes lower alkyl alcohol solvents (preferably methanol, ethanol, 2-propanol, or butanol, etc.), ethereal solvents (preferably tetrahydrofuran or dioxane, etc.), or amide solvents (preferably *N*,*N*-dimethylformamide or 1-methyl-pyrrolidin-2-one, etc.). Reaction temperature ranges from about 0°C to 200°C, preferably about 10°C to 150°C.

In turn, this is substituted by the mono-protected diamine (D) , wherein $R_3$, $R_4$, A, and B are as defined above and P is a protective group, with or without a base in an inert solvent to provide 2,4-disubstituted amino quinoline (E). The base includes an alkali metal carbonate (preferably sodium carbonate or potassium carbonate, etc.), an alkali metal hydroxide (preferably sodium hydroxide, etc.), or a tertiary amine (preferably *N*,*N*-diisopropylethylamine, triethylamine, or *N*-methylmorpholine, etc.). The inert solvent includes lower alkyl alcohol solvents (preferably methanol, ethanol, 2-propanol, or butanol, etc.) or amide solvents (preferably *N,N*-dimethylformamide or 1-methyl-pyrrolidin-2-one, etc.). Reaction temperature ranges from about 50°C to 200°C, preferably about 80°C to 150°C. Also this reaction can be carried out under microwave conditions.

Representative protecting groups suitable for a wide variety of synthetic transformations are disclosed in Greene and Wuts, *Protective Groups in Organic Synthesis,* second edition, John Wiley & Sons, New York, 1991, the disclosure of which is incorporated herein by reference in its entirety. The deprotection of the protective group leads to the common intermediate (F) of the novel substituted quinolines.

## *Scheme 1*

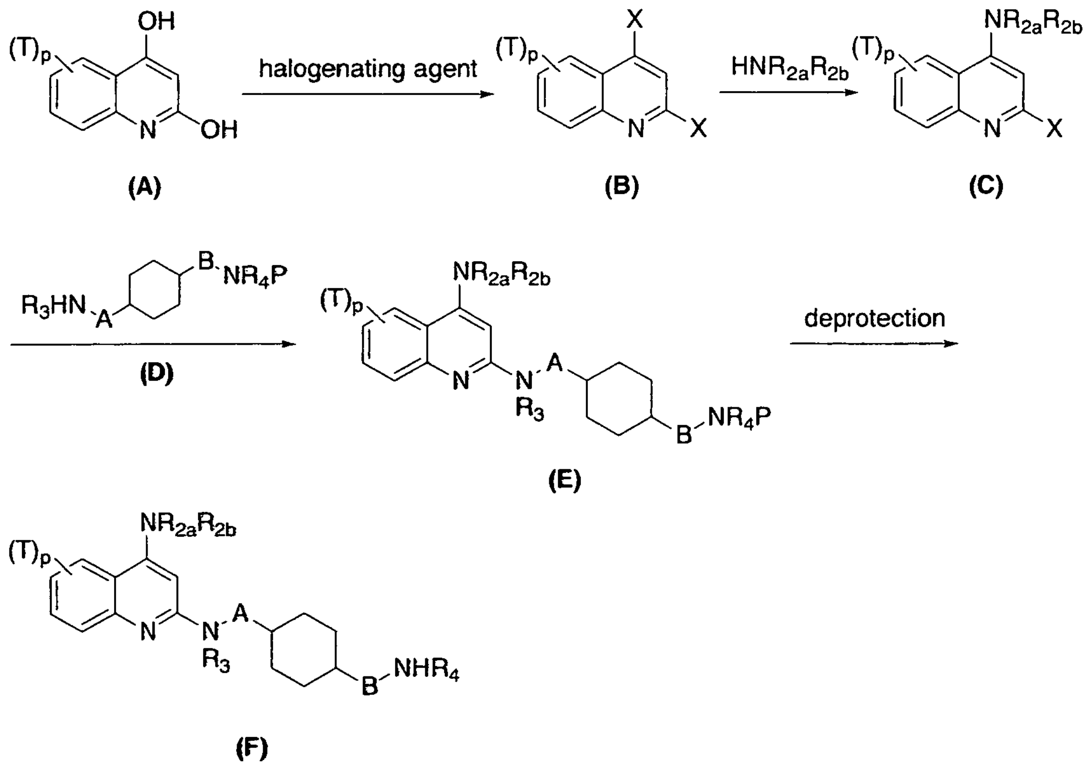


(A) (B) (C) (D) (E) (F)

The conversion of the common intermediate (F) to the novel substituted quinolines (G-K) of the present invention is outlined in Scheme 2.

The amine (F) is reacted with a carboxylic acid ($R_1CO_2H$) and a dehydrating condensing agent in an inert solvent with or without a base to provide the novel amide (G) of the present invention. The dehydrating condensing agent includes dicyclohexylcarbodiimide (DCC), 1-ethyl-3-(3-dimethylaminopropyl)carbodiimide hydrochloride (EDC•HCl), bromo-tris-pyrrolidino-phosnium hexafluorophosphate (PyBroP), *O*-(7-azabenzotriazol-1-yl)-1,1,3,3-tetramethyluronium hexafluorophosphate (HATU), or 1-cyclohexyl-3-methylpolystyrene-carbodiimide. The base includes a tertiary amine (preferably N,N-diisopropylethylamine or triethylamine, etc.). The inert solvent includes lower halocarbon solvents (preferably dichloromethane, dichloroethane, or chloroform, etc.), ethereal solvents (preferably tetrahydrofuran or dioxane), nitrile solvents (preferably acetonitrile, etc.), or amide solvents (preferably N,N-dimethylformamide, etc.). In case of need, 1-hydroxybenzotriazole (HOBT), HOBT-6-carboxaamidomethyl polystyrene, or 1-hydroxy-7-azabenzotriazole (HOAT) can be used as a reactant agent. Reaction temperature ranges from about -20°C to 50°C, preferably about 0°C to 40°C.

Alternatively, the novel amide (G) of the present invention can be obtained by amidation reaction using an acid chloride ($R_1COCl$) and a base in an inert solvent. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisoprogylethylamine, triethylamine, or *N*-methylmorpholine, etc.), or an aromatic amine (preferably pyridine, imidazole, poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), amide solvents (preferably *N*,*N*-dimethylformamide, etc.), or aromatic solvents (preferably toluene or pyridine, etc.). Reaction temperature ranges from about -20°C to 50°C, preferably about 0°C to 40°C.

The novel amide (G) of the present invention is reacted with a reducing agent in an inert solvent to provide the novel amine (H) of the present invention. The reducing agent includes alkali metal aluminum hydrides (preferably lithium aluminum hydride), alkali metal borohydrides (preferably lithium borohydride), alkali metal trialkoxyaluminum hydrides (preferably lithium tri-*tert*-butoxyaluminum hydride), dialkylaluminum hydrides (preferably di-isobutylaluminum hydride), borane, dialkylboranes (preferably di-isoamyl borane), alkali metal trialkylboron hydrides (preferably lithium triethylboron hydride). The inert solvent includes ethereal solvents (preferably tetrahydrofuran or dioxane) or

aromatic solvents (preferably toluene, etc.). Reaction temperature ranges from about -78°C to 200°C, preferably about 50°C to 120°C.

Alternatively, the novel amine (H) of the present invention can be obtained by reductive amination reaction using aldehyde ($R_1CHO$) and a reducing agent in an inert solvent with or without an acid. The reducing agent includes sodium triacetoxyborohydride, sodium cyanoborohydride, sodium borohydride, or boran-pyridine complex, preferably sodium triacetoxyborohydride or sodium cyanoborohydride. The inert solvent includes lower alkyl alcohol solvents (preferably methanol or ethanol, etc.), lower halocarbon solvents (preferably dichloromethane, dichloroethane, or chloroform, etc.), ethereal solvents (preferably tetrahydrofuran or dioxane), or aromatic solvents (preferably toluene, etc.). The acid includes an inorganic acid (preferably hydrochloric acid or sulfuric acid) or an organic acid (preferably acetic acid). Reaction temperature ranges from about -20°C to 120°C, preferably about 0°C to 100°C. Also this reaction can be carried out under microwave conditions.

The novel urea (I) of the present invention can be obtained by urea reaction using an isocyanate ($R_1NCO$) in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N,N*-dimethylformamide or dimethyl sulfoxide, etc.). Reaction temperature ranges from about -20°C to 120°C, preferably about 0°C to 100°C.

The amine (F) is reacted with a isothiocyanate ($R_1NCS$) in an inert solvent with or without a base to provide the novel thiourea (J) of the present invention. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or amide solvents (preferably *N*,*N*-dimethylformamide, etc.). Reaction temperature ranges from about -20°C to 120°C, preferably about 0°C to 100°C.

The novel urethane (K) of the present invention can be obtained by urethane reaction using $R_1OCOX$, wherein X is halogen such as chloro, bromo, or iodo, in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N,N*-diisopropylethylamine, triethylamine, or *N*-methylmorpholine, etc.), or an aromatic amine (preferably pyridine, imidazole, or poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N*,*N*-dimethylformamide or dimethyl sulfoxide, etc.). Reaction temperature ranges from about -20°C to 120°C, preferably about 0°C to 100°C.

***Scheme 2***

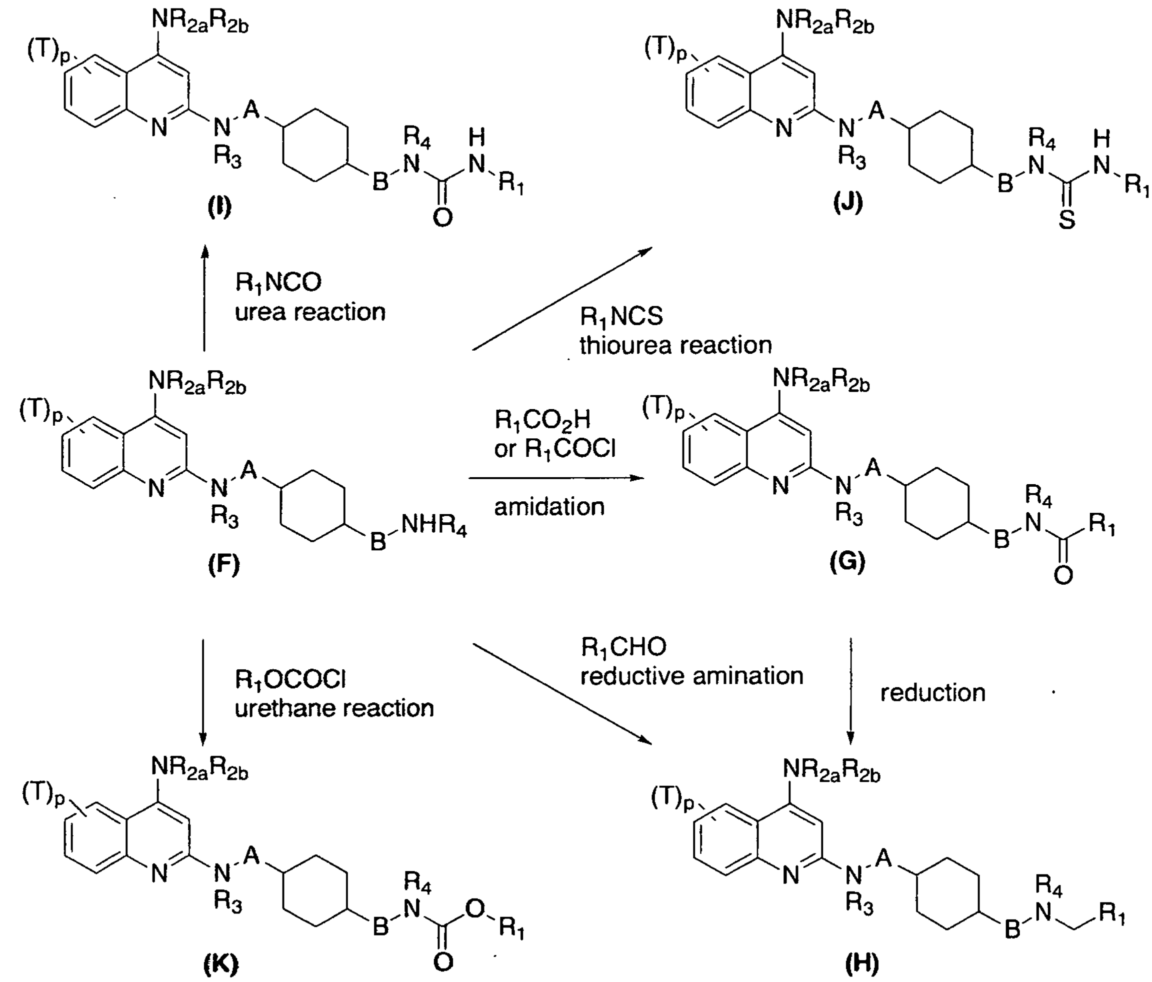


Compounds of Formula (N) can be prepared as shown in Scheme 3. [4-(Benzyloxycarbonylamino-methyl)-cyclohexyl]-carbamic acid *tert-butyl* ester (L) is synthesized by the method which is described in WO 01/72710. The deprotection of Boc-group is achieved by an acid to give the amine (M). The coupling of the amine with quinoline core (C), which is synthesized as scheme 1, gives 2,4-disubstituted amino quinoline. The deprotection of Z-group is achieved by hydrogen reduction to give compounds of Formula (N).

## *Scheme 3*

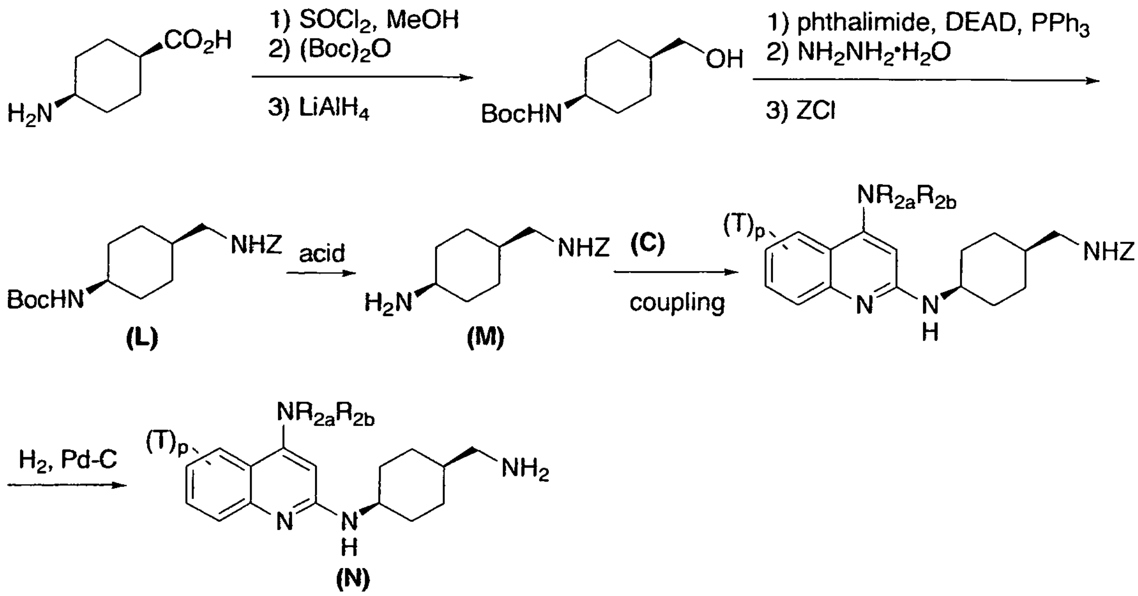


Compounds of Formula (P) can be prepared as shown in Scheme 4. The dicarboxylic acid of commercially available *cis*-cyclohexane-1,4-dicarboxylic acid is transformed to dibenzyl carbamate by curtius rearrangement. The deprotection of Z-group is achieved by hydrogen reduction to give the diamine. The mono-protection of the diamine can be achieved by the method described in *Synthetic communications,* **20,** 2559-2564 (1990) to give the compound (O). The coupling of the amine with quinoline core (C), which is synthesized as scheme 1, gives 2,4-disubstituted amino quinoline. The deprotection of Boc-group is achieved by an acid to give the amine (P).

## *Scheme 4*

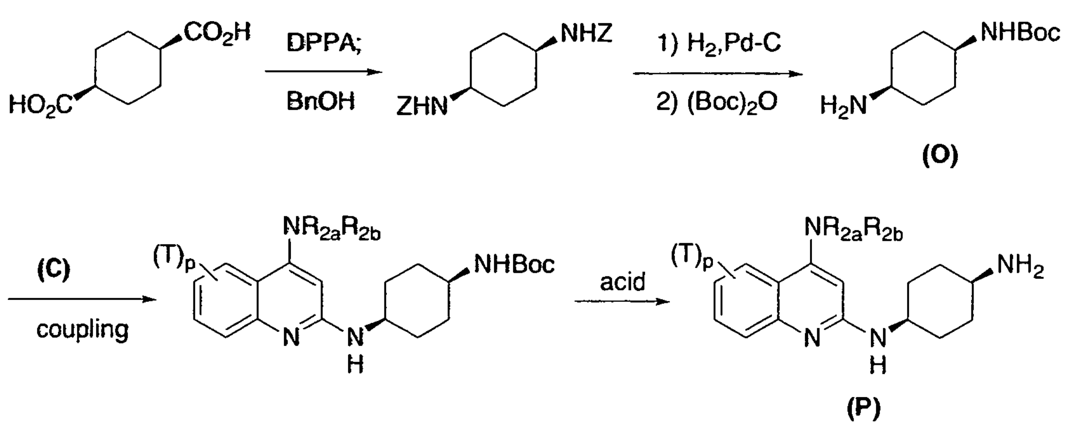


The common intermediate (V) of the novel substituted tetrahydroquinazolines can be prepared as shown in Scheme 5. Commercially available ethyl 2-cyclohexanonecarboxylate (Q), wherein T and p is as defined above, is transformed to 2,4-dihydroxytetrahydroquinazoline (R) according to the method described in EP 0604920. 2,4-Dihydroxytetrahydroquinazoline (R) is converted to 2,4-dihalo-tetrahydroquinazoline (S) by a halogenating agent with or without a base (wherein X is halogen such as chloro, bromo, or iodo). The halogenating agent includes phosphorous oxychloride ($POCl_3$), phosphorous oxybromide ($POBr_3$), or phosphorus pentachloride ($PCl_5$). The base includes a tertiary amine (preferably *N,N*-diisopropylethylamine, etc.) or an aromatic amine (preferably *N,N*-dimethylaniline, etc.). Reaction temperature ranges from about 100°C to 200°C, preferably about 140°C to 180°C.

The halogen of 4-position of 2,4-dihalo-tetrahydroquinazoline (S) is selectively substituted by a primary or secondary amine ($HNR_{2a}R_{2b}$, wherein $R_{2a}$ and $R_{2b}$ are as defined above) with or without a base in an inert solvent to provide the corresponding 4-substitued amino adduct (T). The base includes an alkali metal carbonate (preferably sodium carbonate or potassium carbonate, etc.), an alkali metal hydroxide (preferably sodium hydroxide, etc.), or a tertiary amine (preferably *N,N*-diisopropylethylamine, triethylamine, or *N*-methylmorpholine, etc.). The inert solvent includes lower alkyl alcohol solvents (preferably methanol, ethanol, 2-propanol, or butanol, etc.), ethereal solvents (preferably tetrahydrofuran or dioxane, etc.), or amide solvents (preferably *N,N*-dimethylformamide or 1-methyl-pyrrolidin-2-one, etc.). Reaction temperature ranges from about 0°C to 200°C, preferably about 10°C to 150°C.

In turn, this is substituted by the mono-protected diamine (D) , wherein $R_3$, $R_4$, A, and B are as defined above and P is a protective group, with or without a base in an inert solvent to provide 2,4-disubstituted amino tetrahydroquinazoline (U). The base includes an alkali metal carbonate (preferably sodium carbonate or potassium carbonate, etc.), an alkali metal hydroxide (preferably sodium hydroxide, etc.), or a tertiary amine (preferably *N,N*-diisopropylethylamine, triethylamine, or *N*-methylmorpholine, etc.). The inert solvent includes lower alkyl alcohol solvents (preferably methanol, ethanol, 2-propanol, or butanol, etc.) or amide solvents (preferably *N,N*-dimethylformamide or 1-methylpyrrolidin-2-one, etc.). Reaction temperature ranges from about 50°C to 200°C, preferably about 80°C to 150°C. Also this reaction can be carried out under microwave conditions.

The deprotection of the protective group leads to the common intermediate (V) of the novel substituted tetrahydroquinazolines.

***Scheme 5***

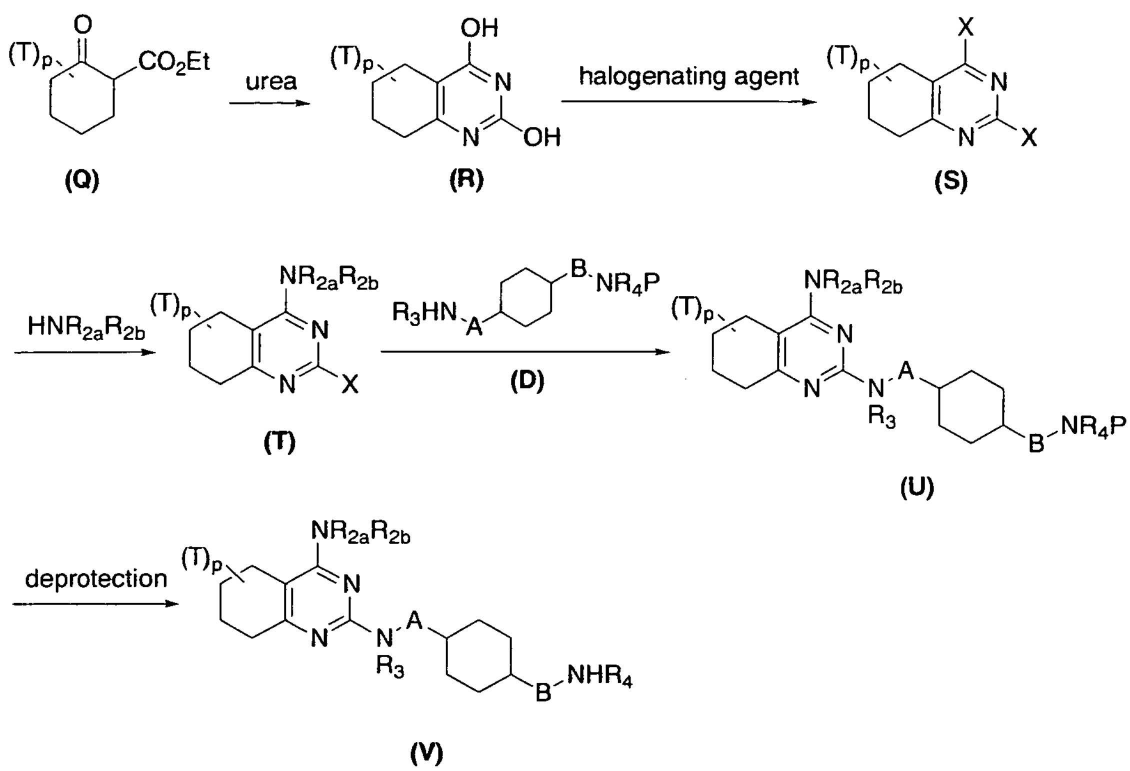


The conversion of the common intermediate (V) to the novel substituted tetrahydroquinazolines (W-A') of the present invention is outlined in Scheme 6.

The amine (V) is reacted with a carboxylic acid ($R_1CO_2H$) and a dehydrating condensing agent in an inert solvent with or without a base to provide the novel amide (W) of the present invention. The dehydrating condensing agent includes dicyclohexylcarbodiimide (DCC), 1-ethyl-3-(3-dimethylaminopropyl)carbodiimide hydrochloride (EDC•HCl), bromo-tris-pyrrolidino-phosnium hexafluorophosphate (PyBroP), *O*-(7-azabenzotriazol-1-yl)-1,1,3,3-tetramethyluronium hexafluorophosphate (HATU), or 1-cyclohexyl-3-methylpolystyrene-carbodiimide. The base includes a tertiary amine (preferably *N,N*-diisopropylethylamine or triethylamine, etc.). The inert solvent includes lower halocarbon solvents (preferably dichloromethane, dichloroethane, or chloroform, etc.), ethereal solvents (preferably tetrahydrofuran or dioxane), nitrile solvents (preferably acetonitrile, etc.), or amide solvents (preferably *N,N*-dimethylformamide, etc.). In case of need, 1-hydroxybenzotriazole (HOBT), HOBT-6-carboxaamidomethyl polystyrene, or 1-hydroxy-7-azabenzotriazole (HOAT) can be used as a reactant agent. Reaction temperature ranges from about -20°C to 50°C, preferably about 0°C to 40°C.

Alternatively, the novel amide (W) of the present invention can be obtained by amidation reaction using an acid chloride ($R_1COCl$) and a base in an inert solvent. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N,N*-diisopropylethylamine, triethylamine, or *N*-methylmorpholine, etc.), or an aromatic amine (preferably pyridine, imidazole, poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), amide solvents (preferably *N,N*-dimethylformamide, etc.), or aromatic solvents (preferably toluene or pyridine, etc.). Reaction temperature ranges from about -20°C to 50°C, preferably about 0°C to 40°C.

The novel amide (W) of the present invention is reacted with a reducing agent in an inert solvent to provide the novel amine (X) of the present invention. The reducing agent includes alkali metal aluminum hydrides (preferably lithium aluminum hydride), alkali metal borohydrides (preferably lithium borohydride), alkali metal trialkoxyaluminum hydrides (preferably lithium tri-*tert*-butoxyaluminum hydride), dialkylaluminum hydrides (preferably di-isobutylaluminum hydride), borane, dialkylboranes (preferably di-isoamyl borane), alkali metal trialkylboron hydrides (preferably lithium triethylboron hydride). The inert solvent includes ethereal solvents (preferably tetrahydrofuran or dioxane) or aromatic solvents (preferably toluene, etc.). Reaction temperature ranges from about -78°C to 200°C, preferably about 50°C to 120°C.

Alternatively, the novel amine (X) of the present invention can be obtained by reductive amination reaction using aldehyde ($R_1CHO$) and a reducing agent in an inert solvent with or without an acid. The reducing agent includes sodium triacetoxyborohydride, sodium cyanoborohydride, sodium borohydride, or boran-pyridine complex, preferably sodium triacetoxyborohydride or sodium cyanoborohydride. The inert solvent includes lower alkyl alcohol solvents (preferably methanol or ethanol, etc.), lower halocarbon solvents (preferably dichloromethane, dichloroethane, or chloroform, etc.), ethereal solvents (preferably tetrahydrofuran or dioxane), or aromatic solvents (preferably toluene, etc.). The acid includes an inorganic acid (preferably hydrochloric acid or sulfuric acid) or an organic acid (preferably acetic acid). Reaction temperature ranges from about -20°C to 120°C, preferably about 0°C to 100°C. Also this reaction can be carried out under microwave conditions.

The novel urea (Y) of the present invention can be obtained by urea reaction using an isocyanate ($R_1NCO$) in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N*,*N*-dimethylformamide or dimethyl sulfoxide, etc.). Reaction temperature ranges from about -20°C to 120°C, preferably about 0°C to 100°C.

The amine (V) is reacted with a isothiocyanate ($R_1NCS$) in an inert solvent with or without a base to provide the novel thiourea (Z) of the present invention. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or amide solvents (preferably *N,N*-dimethylformamide, etc.). Reaction temperature ranges from about -20°C to 120°C, preferably about 0°C to 100°C.

The novel urethane (A') of the present invention can be obtained by urethane reaction using $R_1OCOX$, wherein X is halogen such as chloro, bromo, or iodo, in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N,N*-diisopropylethylamine, triethylamine, or N-methylmorpholine, etc.), or an aromatic amine (preferably pyridine, imidazole, or poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N*,*N*-dimethylformamide or dimethyl sulfoxide, etc.). Reaction temperature ranges from about -20°C to 120°C, preferably about 0°C to 100°C.

## *Scheme 6*

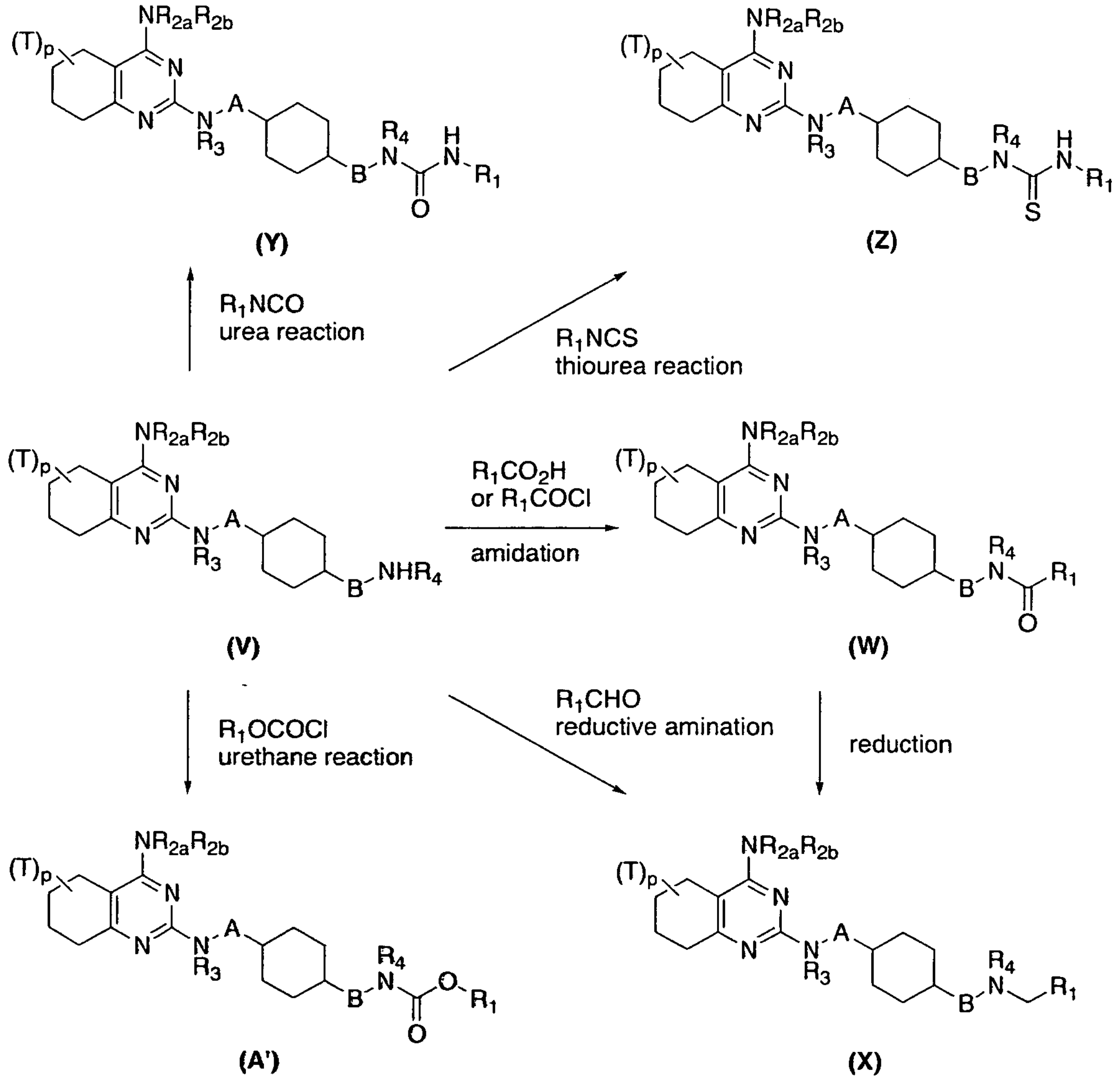


(Y) (Z) (V) (W) (A') (X)

Compounds of Formula (B') can be prepared as shown in Scheme 7. The coupling of the amine (M), which is synthesized as scheme 3, with tetrahydroquinazoline core (T), which is synthesized as scheme 5, gives 2,4-disubstituted amino tetrahydroquinazoline. The deprotection of Z-group is achieved by hydrogen reduction to give compounds of Formula (B').

***Scheme 7***

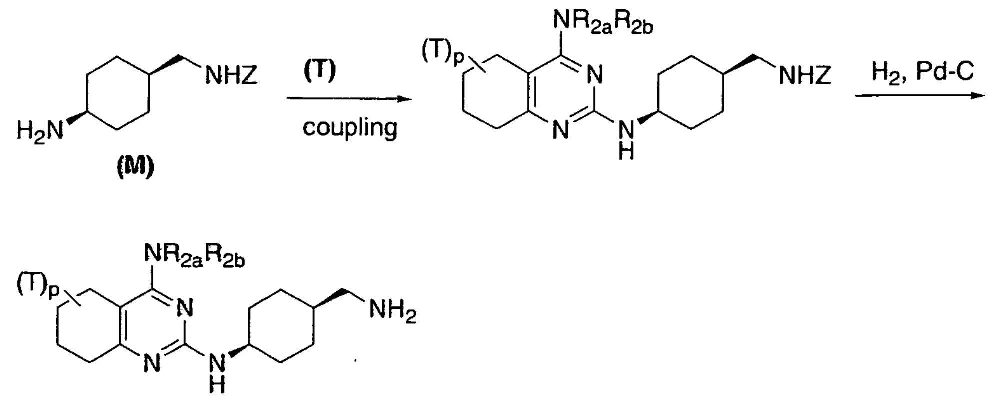


(M)

(B')

Compounds of Formula (C') can be prepared as shown in Scheme 8. The coupling of the amine (O), which is synthesized as scheme 4, with tetrahydroquinazoline core (T), which is synthesized as scheme 5, gives 2,4-disubstituted amino tetrahydroquinazoline. The deprotection of Boc-group is achieved by an acid to give the amine (C').

***Scheme 8***

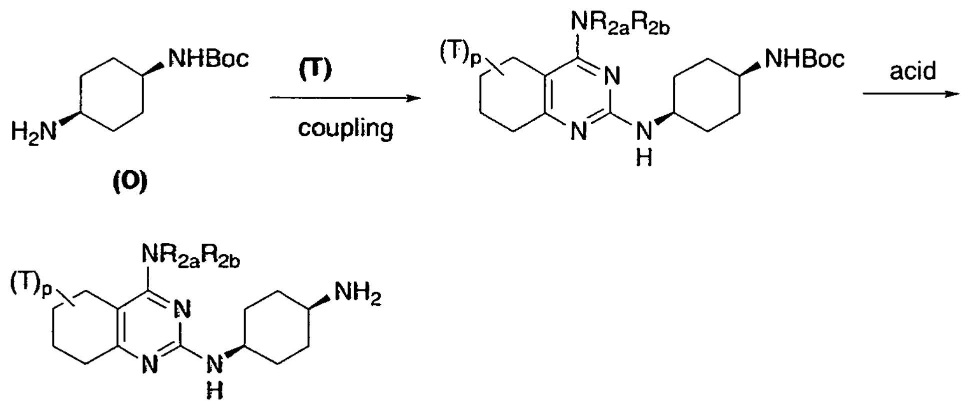


(O)

(C')

The common intermediate (H') of the novel substituted pyrimidines can be prepared as shown in Scheme 9. Commercially available substituted uracil (D') , wherein T and p is as defined above, is converted to substituted 2,4-dihalo- pyrimidines (E') by a halogenating agent with or without a base (wherein X is halogen such as chloro, bromo, or iodo). The halogenating agent includes phosphorous oxychloride ($POCl_3$), phosphorous oxybromide ($POBr_3$), or phosphorus pentachloride ($PCl_5$). The base includes a tertiary amine (preferably *N,N*-diisopropylethylamine, etc.) or an aromatic amine (preferably *N*,*N*-dimethylaniline, etc.). Reaction temperature ranges from about 100°C to 200°C, preferably about 140°C to 180°C.

The halogen of 4-position of substituted 2,4-dihalo-pyrimidines (E') is selectively substituted by a primary or secondary amine ($HNR_{2a}R_{2b}$, wherein $R_{2a}$ and $R_{2b}$ are as defined above) with or without a base in an inert solvent to provide the corresponding 4-substitued amino adduct (F'). The base includes an alkali metal carbonate (preferably sodium carbonate or potassium carbonate, etc.), an alkali metal hydroxide (preferably sodium hydroxide, etc.), or a tertiary amine (preferably *N,N*-diisopropylethylamine, triethylamine, or *N*-methylmorpholine, etc.). The inert solvent includes lower alkyl alcohol solvents (preferably methanol, ethanol, 2-propanol, or butanol, etc.), ethereal solvents (preferably tetrahydrofuran or dioxane, etc.), or amide solvents (preferably *N,N*-dimethylformamide or 1-methyl-pyrrolidin-2-one, etc.). Reaction temperature ranges from about 0°C to 200°C, preferably about 10°C to 150°C.

In turn, this is substituted by the mono-protected diamine (D) , wherein $R_3$, $R_4$, A, and B are as defined above and P is a protective group, with or without a base in an inert solvent to provide 2,4-disubstituted amino pyrimidines (G'). The base includes an alkali metal carbonate (preferably sodium carbonate or potassium carbonate, etc.), an alkali metal hydroxide (preferably sodium hydroxide, etc.), or a tertiary amine (preferably *N,N*-diisopropylethylamine, triethylamine, or *N*-methylmorpholine, etc.). The inert solvent includes lower alkyl alcohol solvents (preferably methanol, ethanol, 2-propanol, or butanol, etc.) or amide solvents (preferably *N,N*-dimethylformamide or 1-methyl-pyrrolidin-2-one, etc.). Reaction temperature ranges from about 50°C to 200°C, preferably about 80°C to 150°C. Also this reaction can be carried out under microwave conditions.

The deprotection of the protective group leads to the common intermediate (H') of the novel substituted pyrimidines.

***Scheme 9***

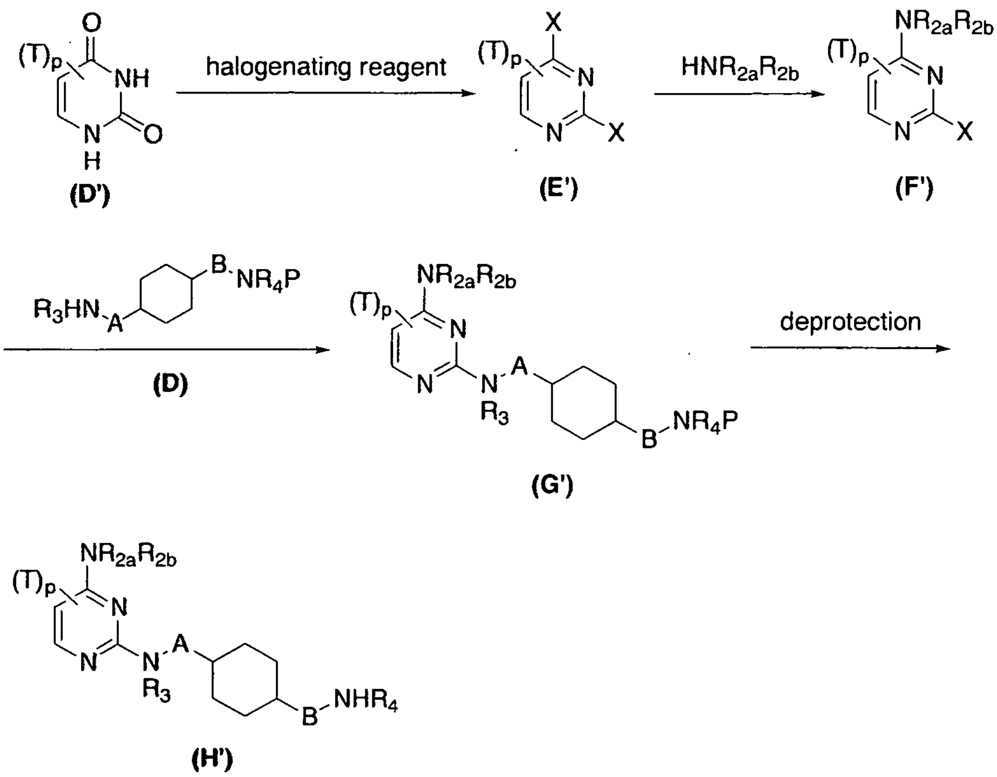


The conversion of the common intermediate (H') to the novel substituted pyrimidines (I'-M') of the present invention is outlined in Scheme 10.

The amine (H') is reacted with a carboxylic acid ($R_1CO_2H$) and a dehydrating condensing agent in an inert solvent with or without a base to provide the novel amide (I' ) of the present invention. The dehydrating condensing agent includes dicyclohexylcarbodiimide (DCC), 1-ethyl-3-(3-dimethylaminopropyl)carbodiimide hydrochloride (EDC•HCl), bromo-tris-pyrrolidino-phosnium hexafluorophosphate (PyBroP), *O*-(7-azabenzotriazol-1-yl)-1,1,3,3-tetramethyluronium hexafluorophosphate (HATU), or 1-cyclohexyl-3-methylpolystyrene-carbodiimide. The base includes a tertiary amine (preferably *N,N*-diisopropylethylamine or triethylamine, etc.). The inert solvent includes lower halocarbon solvents (preferably dichloromethane, dichloroethane, or chloroform, etc.), ethereal solvents (preferably tetrahydrofuran or dioxane), nitrile solvents (preferably acetonitrile, etc.), or amide solvents (preferably *N,N*-dimethylformamide, etc.). In case of need, 1-hydroxybenzotriazole (HOBT), HOBT-6-carboxaamidomethyl polystyrene, or 1-hydroxy-7-azabenzotriazole (HOAT) can be used as a reactant agent. Reaction temperature ranges from about -20°C to 50°C, preferably about 0°C to 40°C.

Alternatively, the novel amide (I') of the present invention can be obtained by amidation reaction using an acid chloride ($R_1COCl$) and a base in an inert solvent. The base includes an alkali metal carbonate (preferably sodium carbonate or potassium carbonate, etc.), an alkali metal hydrogencarbonate (preferably sodium hydrogencarbonate

or potassium hydrogencarbonate, etc.), an alkali hydroxide (preferably sodium hydroxide or potassium hydroxide, etc.), a tertiary amine (preferably *N,N*-diisoprogylethylamine, triethylamine, or *N*-methylmorpholine, etc.), or an aromatic amine (preferably pyridine, imidazole, poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), amide solvents (preferably *N,N*-dimethylformamide, etc.), or aromatic solvents (preferably toluene or pyridine, etc.). Reaction temperature ranges from about -20°C to 50°C, preferably about 0°C to 40°C.

The novel amide (I') of the present invention is reacted with a reducing agent in an inert solvent to provide the novel amine (J') of the present invention. The reducing agent includes alkali metal aluminum hydrides (preferably lithium aluminum hydride), alkali metal borohydrides (preferably lithium borohydride), alkali metal trialkoxyaluminum hydrides (preferably lithium tri-*tert*-butoxyaluminum hydride), dialkylaluminum hydrides (preferably di-isobutylaluminum hydride), borane, dialkylboranes (preferably di-isoamyl borane), alkali metal trialkylboron hydrides (preferably lithium triethylboron hydride). The inert solvent includes ethereal solvents (preferably tetrahydrofuran or dioxane) or aromatic solvents (preferably toluene, etc.). Reaction temperature ranges from about -78°C to 200°C, preferably about 50°C to 120°C.

Alternatively, the novel amine (J') of the present invention can be obtained by reductive amination reaction using aldehyde ($R_1CHO$) and a reducing agent in an inert solvent with or without an acid. The reducing agent includes sodium triacetoxyborohydride, sodium cyanoborohydride, sodium borohydride, or boran-pyridine complex, preferably sodium triacetoxyborohydride or sodium cyanoborohydride. The inert solvent includes lower alkyl alcohol solvents (preferably methanol or ethanol, etc.), lower halocarbon solvents (preferably dichloromethane, dichloroethane, or chloroform, etc.), ethereal solvents (preferably tetrahydrofuran or dioxane), or aromatic solvents (preferably toluene, etc.). The acid includes an inorganic acid (preferably hydrochloric acid or sulfuric acid) or an organic acid (preferably acetic acid). Reaction temperature ranges from about -20°C to 120°C, preferably about 0°C to 100°C. Also this reaction can be carried out under microwave conditions.

The novel urea (K') of the present invention can be obtained by urea reaction using an isocyanate ($R_1NCO$) in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N,N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N*,*N*-dimethylformamide or dimethyl sulfoxide, etc.). Reaction temperature ranges from about -20°C to 120°C, preferably about 0°C to 100°C.

The amine (H') is reacted with a isothiocyanate ($R_1NCS$) in an inert solvent with or without a base to provide the novel thiourea (L') of the present invention. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or amide solvents (preferably *N,N*-dimethylformamide, etc.). Reaction temperature ranges from about -20°C to 120°C, preferably about 0°C to 100°C.

The novel urethane (M') of the present invention can be obtained by urethane reaction using $R_1OCOCl$, wherein X is halogen such as chloro, bromo, or iodo, in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably N,N-diisopropylethylamine, triethylamine, or *N*-methylmorpholine, etc.), or an aromatic amine (preferably pyridine, imidazole, or poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N*,*N*-dimethylformamide or dimethyl sulfoxide, etc.). Reaction temperature ranges from about -20°C to 120°C, preferably about 0°C to 100°C.

## *Scheme 10*

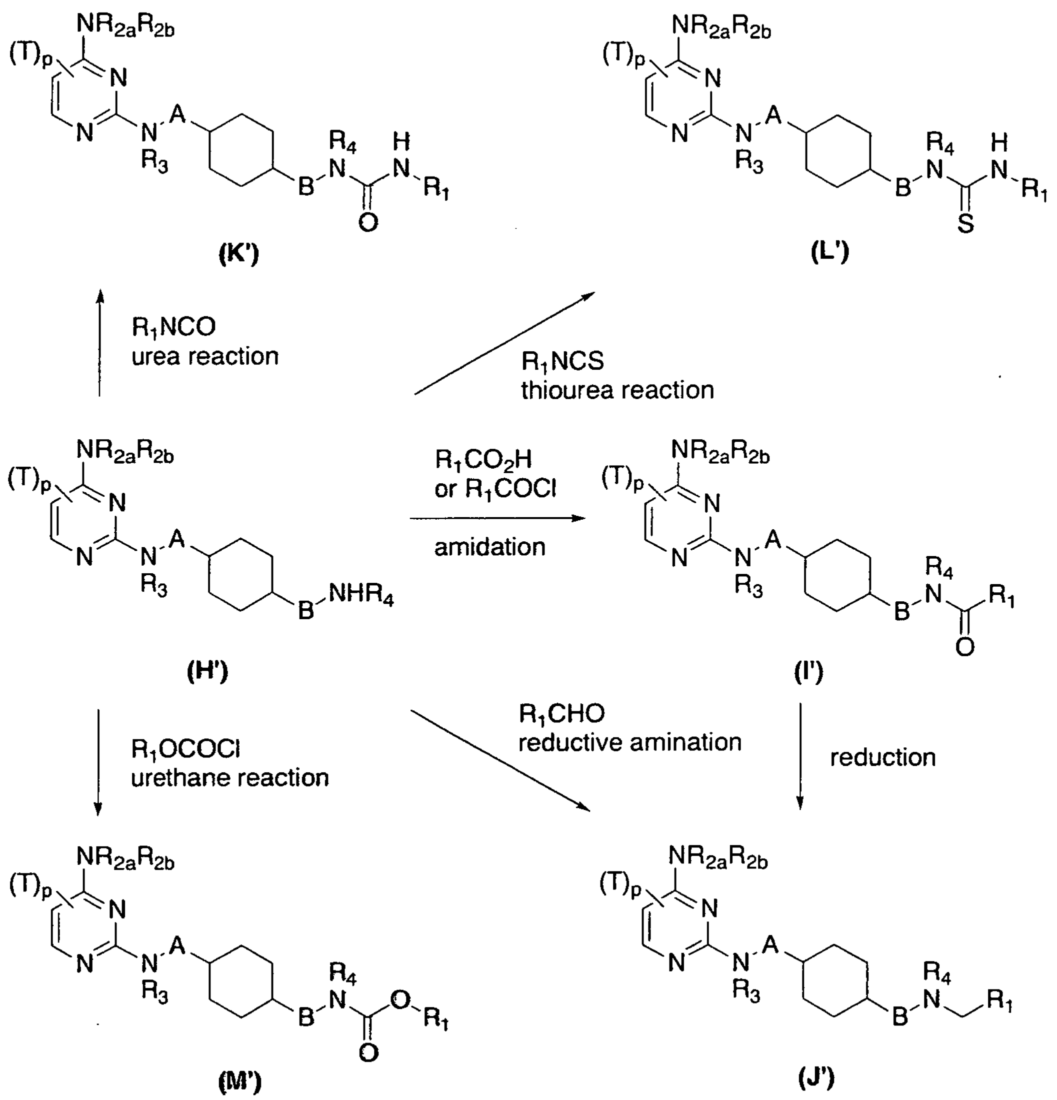


Compounds of Formula (N') can be prepared as shown in Scheme 11. The coupling of the amine (M), which is synthesized as scheme 3, with pyrimidine core (F'), which is synthesized as scheme 9, gives 2,4-disubstituted amino pyrimidine. The deprotection of Z-group is achieved by hydrogen reduction to give compounds of Formula (N').

## *Scheme 11*

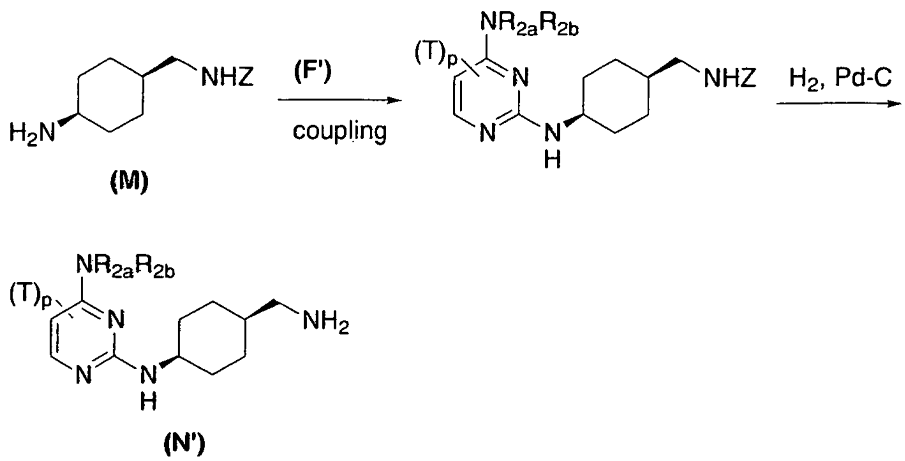


(M)

(N')

Compounds of Formula (O') can be prepared as shown in Scheme 12. The coupling of the amine (O), which is synthesized as scheme 4, with pyrimidine core (F'), which is synthesized as scheme 9, gives 2,4-disubstituted amino pyrimidine. The deprotection of Boc-group is achieved by an acid to give the amine (O').

## *Scheme 12*

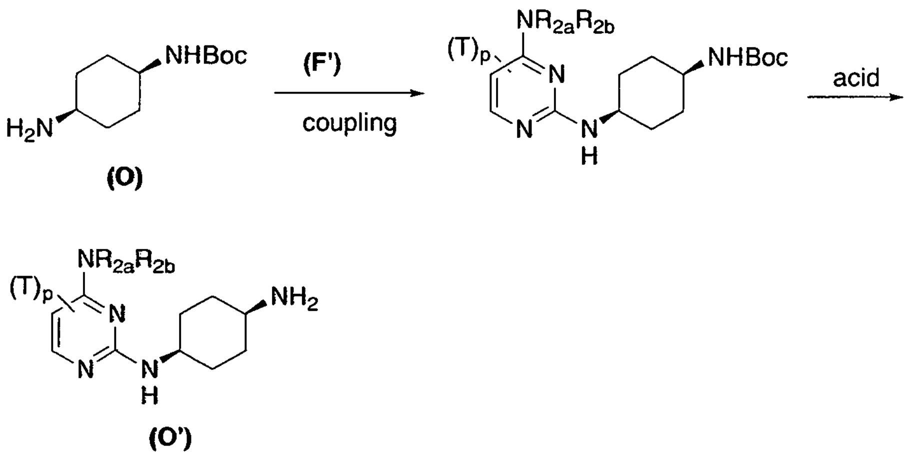


(O)

(O')

The common intermediate (S') of the novel substituted quinolines can be prepared as shown in Scheme 13. Commercially available substituted 2-hydroxy-quinoline (P') , wherein $R_2$, T, and p is as defined above, is converted to 2-halo-quinolines (Q') by a halogenating agent with or without a base (wherein X is halogen such as chloro, bromo, or iodo). The halogenating agent includes phosphorous oxychloride ($POCl_3$), phosphorous oxybromide ($POBr_3$), or phosphorus pentachloride ($PCl_5$). The base includes a tertiary amine (preferably *N,N*-diisopropylethylamine, etc.) or an aromatic amine (preferably *N,N*-dimethylaniline, etc.). Reaction temperature ranges from about 100°C to 200°C, preferably about 140°C to 180°C.

The halide (Q') is substituted by the mono-protected diamine (D) , wherein $R_3$, $R_4$, A, and B are as defined above and P is a protective group, with or without a base in an inert solvent to provide 2-substituted amino quinoline (R'). The base includes an alkali metal carbonate (preferably sodium carbonate or potassium carbonate, etc.), an alkali metal hydroxide (preferably sodium hydroxide, etc.), or a tertiary amine (preferably *N,N*-diisopropylethylamine, triethylamine, or N-methylmorpholine, etc.). The inert solvent includes lower alkyl alcohol solvents (preferably methanol, ethanol, 2-propanol, or butanol, etc.) or amide solvents (preferably *N,N*-dimethylformamide or 1-methyl-pyrrolidin-2-one,

etc.). Reaction temperature ranges from about 50°C to 200°C, preferably about 80°C to 150°C. Also this reaction can be carried out under microwave conditions.

The deprotection of the protective group leads to the common intermediate (S') of the novel substituted quinolines.

***Scheme 13***

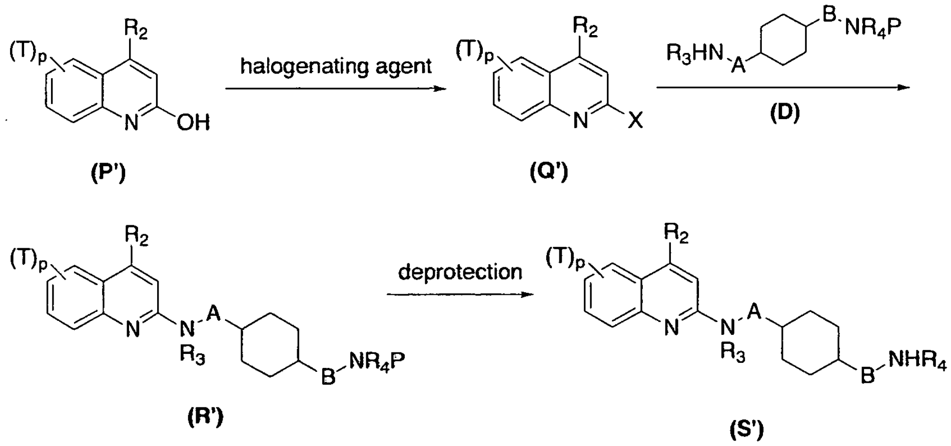


(P') (Q') (R') (S')

The conversion of the common intermediate (S') to the novel substituted quinolines (T'-X') of the present invention is outlined in Scheme 14.

The amine (S') is reacted with a carboxylic acid ($R_1CO_2H$) and a dehydrating condensing agent in an inert solvent with or without a base to provide the novel amide (T') of the present invention. The dehydrating condensing agent includes dicyclohexylcarbodiimide (DCC), 1-ethyl-3-(3-dimethylaminopropyl)carbodiimide hydrochloride (EDC•HCl), bromo-tris-pyrrolidino-phosnium hexafluorophosphate (PyBroP), *O*-(7-azabenzotriazol-1-yl)-1,1,3,3-tetramethyluronium hexafluorophosphate (HATU), or 1-cyclohexyl-3-methylpolystyrene-carbodiimide. The base includes a tertiary amine (preferably *N,N*-diisopropylethylamine or triethylamine, etc.). The inert solvent includes lower halocarbon solvents (preferably dichloromethane, dichloroethane, or chloroform, etc.), ethereal solvents (preferably tetrahydrofuran or dioxane), nitrile solvents (preferably acetonitrile, etc.), or amide solvents (preferably *N,N*-dimethylformamide, etc.). In case of need, 1-hydroxybenzotriazole (HOBT), HOBT-6-carboxaamidomethyl polystyrene, or 1-hydroxy-7-azabenzotriazole (HOAT) can be used as a reactant agent. Reaction temperature ranges from about -20°C to 50°C, preferably about 0°C to 40°C.

Alternatively, the novel amide (T') of the present invention can be obtained by amidation reaction using an acid chloride ($R_1COCl$) and a base in an inert solvent. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N,N*-diisopropylethylamine, triethylamine, or *N*-methylmorpholine, etc.), or an aromatic amine (preferably pyridine, imidazole, poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), amide solvents (preferably *N,N*-dimethylformamide, etc.), or aromatic solvents (preferably toluene or pyridine, etc.). Reaction temperature ranges from about -20°C to 50°C, preferably about 0°C to 40°C.

The novel amide (T') of the present invention is reacted with a reducing agent in an inert solvent to provide the novel amine (U') of the present invention. The reducing agent includes alkali metal aluminum hydrides (preferably lithium aluminum hydride), alkali metal borohydrides (preferably lithium borohydride), alkali metal trialkoxyaluminum hydrides (preferably lithium tri-*tert*-butoxyalurninum hydride), dialkylaluminum hydrides (preferably di-isobutylaluminum hydride), borane, dialkylboranes (preferably di-isoamyl borane), alkali metal trialkylboron hydrides (preferably lithium triethylboron hydride). The inert solvent includes ethereal solvents (preferably tetrahydrofuran or dioxane) or aromatic solvents (preferably toluene, etc.). Reaction temperature ranges from about -78°C to 200°C, preferably about 50°C to 120°C.

Alternatively, the novel amine (U') of the present invention can be obtained by reductive amination reaction using aldehyde ($R_1CHO$) and a reducing agent in an inert solvent with or without an acid. The reducing agent includes sodium triacetoxyborohydride, sodium cyanoborohydride, sodium borohydride, or boran-pyridine complex, preferably sodium triacetoxyborohydride or sodium cyanoborohydride. The inert solvent includes lower alkyl alcohol solvents

(preferably methanol or ethanol, etc.), lower halocarbon solvents (preferably dichloromethane, dichloroethane, or chloroform, etc.), ethereal solvents (preferably tetrahydrofuran or dioxane), or aromatic solvents (preferably toluene, etc.). The acid includes an inorganic acid (preferably hydrochloric acid or sulfuric acid) or an organic acid (preferably acetic acid). Reaction temperature ranges from about -20°C to 120°C, preferably about 0°C to 100°C. Also this reaction can be carried out under microwave conditions.

The novel urea (V') of the present invention can be obtained by urea reaction using an isocyanate ($R_1NCO$) in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N,N*-dimethylformamide or dimethyl sulfoxide, etc.). Reaction temperature ranges from about -20°C to 120°C, preferably about 0°C to 100°C.

The amine (S') is reacted with a isothiocyanate ($R_1NCS$) in an inert solvent with or without a base to provide the novel thiourea (W') of the present invention. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or amide solvents (preferably *N*,*N*-dimethylformamide, etc.). Reaction temperature ranges from about -20°C to 120°C, preferably about 0°C to 100°C.

The novel urethane (X') of the present invention can be obtained by urethane reaction using $R_1OCOCl$, wherein X is halogen such as chloro, bromo, or iodo, in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N,N*-diisopropylethylamine, triethylamine, or *N*-methylmorpholine, etc.), or an aromatic amine (preferably pyridine, imidazole, or poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N,N*-dimethylformamide or dimethyl sulfoxide, etc.). Reaction temperature ranges from about -20°C to 120°C, preferably about 0°C to 100°C.

***Scheme 14***

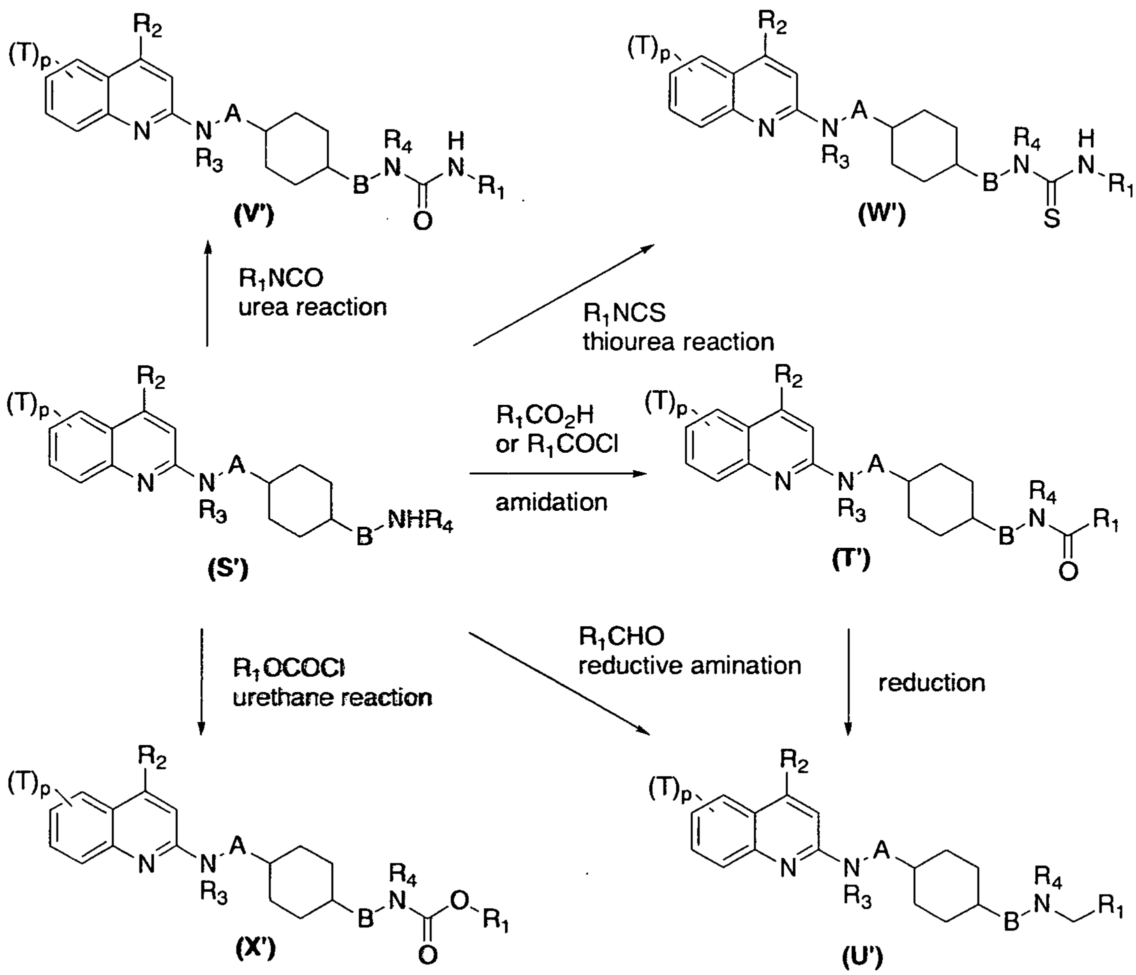


Compounds of Formula (Y') can be prepared as shown in Scheme 15. The coupling of the amine (M), which is synthesized as scheme 3, with quinoline core (Q'), which is synthesized as scheme 13, gives 2-substituted amino quinoline. The deprotection of Z-group is achieved by hydrogen reduction to give compounds of Formula (Y').

## *Scheme 15*

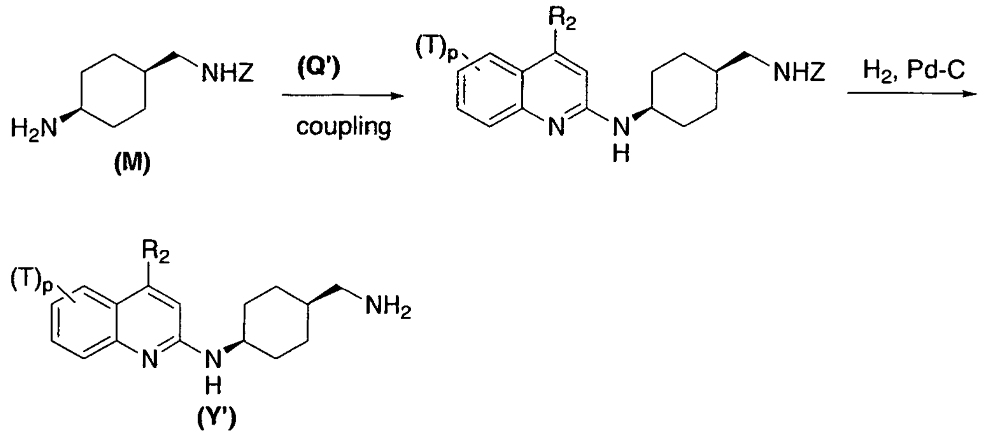


(M)

(Y')

Compounds of Formula (Z') can be prepared as shown in Scheme 16. The coupling of the amine (O), which is synthesized as scheme 4, with quinoline core (Q'), which is synthesized as scheme 13, gives 2-substituted amino quinoline. The deprotection of Boc-group is achieved by an acid to give the amine (Z').

## *Scheme 16*

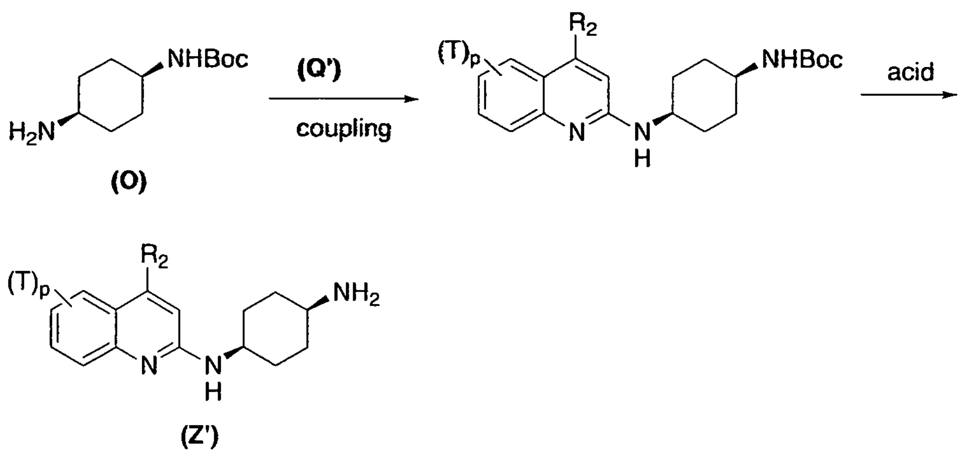


(O)

(Z')

The common intermediate (D") of the novel substituted pyrimidines can be prepared as shown in Scheme 17. Commercially available substituted 2-hydroxy-pyrimidines (A") , wherein $R_2$, T, and p is as defined above, is converted to 2-halo-pyrimidines (B") by a halogenating agent with or without a base (wherein X is halogen such as chloro, bromo, or iodo). The halogenating agent includes phosphorous oxychloride ($POCl_3$), phosphorous oxybromide ($POBr_3$), or phosphorus pentachloride ($PCl_5$). The base includes a tertiary amine (preferably *N*,*N*-diisopropylethylamine, etc.) or an aromatic amine (preferably *N*,*N*-dimethylaniline, etc.). Reaction temperature ranges from about 100°C to 200°C, preferably about 140°C to 180°C.

The halide (B") is substituted by the mono-protected diamine (D) , wherein $R_3$, $R_4$, A, and B are as defined above and P is a protective group, with or without a base in an inert solvent to provide 2-substituted amino pyrimidine (C"). The base includes an alkali metal carbonate (preferably sodium carbonate or potassium carbonate, etc.), an alkali metal hydroxide (preferably sodium hydroxide, etc.), or a tertiary amine (preferably *N*,*N*-diisopropylethylamine, triethylamine, or *N*-methylmorpholine, etc.). The inert solvent includes lower alkyl alcohol solvents (preferably methanol, ethanol, 2-propanol, or butanol, etc.) or amide solvents (preferably *N*,*N*-dimethylformamide or 1-methyl-pyrrolidin-2-one,

etc.). Reaction temperature ranges from about 50°C to 200°C, preferably about 80°C to 150°C. Also this reaction can be carried out under microwave conditions.

The deprotection of the protective group leads to the common intermediate (D") of the novel substituted pyrimidines.

***Scheme 17***

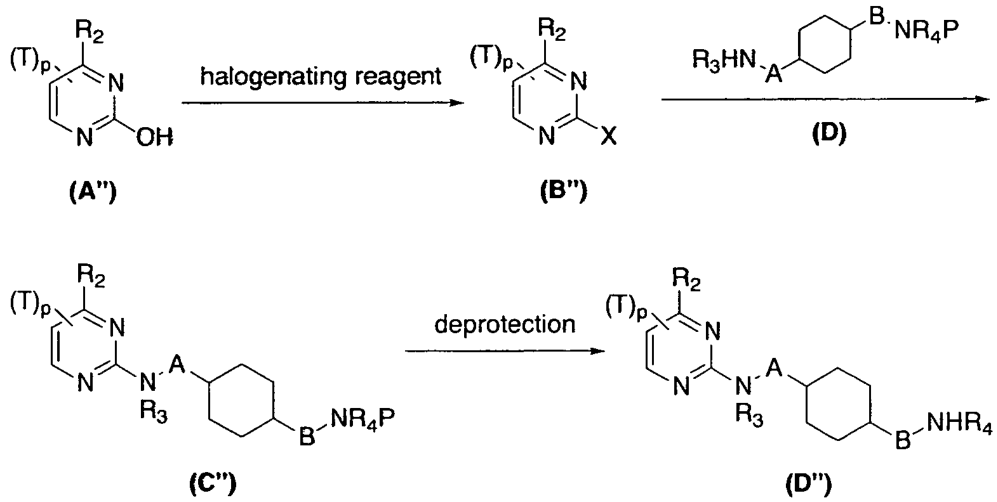


(A") (B") (C") (D")

The conversion of the common intermediate (D") to the novel substituted pyrimidines (E"-I") of the present invention is outlined in Scheme 18.

The amine (D") is reacted with a carboxylic acid ($R_1CO_2H$) and a dehydrating condensing agent in an inert solvent with or without a base to provide the novel amide (E") of the present invention. The dehydrating condensing agent includes dicyclohexylcarbodiimide (DCC), 1-ethyl-3-(3-dimethylaminopropyl)carbodiimide hydrochloride (EDC•HCl), bromo-tris-pyrrolidino-phosnium hexafluorophosphate (PyBroP), *O*-(7-azabenzotriazol-1-yl)-1,1,3,3-tetramethyluronium hexafluorophosphate (HATU), or 1-cyclohexyl-3-methylpolystyrene-carbodiimide. The base includes a tertiary amine (preferably *N*,*N*-diisopropylethylamine or triethylamine, etc.). The inert solvent includes lower halocarbon solvents (preferably dichloromethane, dichloroethane, or chloroform, etc.), ethereal solvents (preferably tetrahydrofuran or dioxane), nitrile solvents (preferably acetonitrile, etc.), or amide solvents (preferably *N,N*-dimethylformamide, etc.). In case of need, 1-hydroxybenzotriazole (HOBT), HOBT-6-carboxaamidomethyl polystyrene, or 1-hydroxy-7-azabenzotriazole (HOAT) can be used as a reactant agent. Reaction temperature ranges from about -20°C to 50°C, preferably about 0°C to 40°C.

Alternatively, the novel amide (E") of the present invention can be obtained by amidation reaction using an acid chloride ($R_1COCl$) and a base in an inert solvent. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine, imidazole, poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), amide solvents (preferably *N*,*N*-dimethylformamide, etc.), or aromatic solvents (preferably toluene or pyridine, etc.). Reaction temperature ranges from about -20°C to 50°C, preferably about 0°C to 40°C.

The novel amide (E") of the present invention is reacted with a reducing agent in an inert solvent to provide the novel amine (F") of the present invention. The reducing agent includes alkali metal aluminum hydrides (preferably lithium aluminum hydride), alkali metal borohydrides (preferably lithium borohydride), alkali metal trialkoxyaluminum hydrides (preferably lithium tri-*tert*-butoxyaluminum hydride), dialkylaluminum hydrides (preferably di-isobutylaluminum hydride), borane, dialkylboranes (preferably di-isoamyl borane), alkali metal trialkylboron hydrides (preferably lithium triethylboron hydride). The inert solvent includes ethereal solvents (preferably tetrahydrofuran or dioxane) or aromatic solvents (preferably toluene, etc.). Reaction temperature ranges from about -78°C to 200°C, preferably about 50°C to 120°C.

Alternatively, the novel amine (F") of the present invention can be obtained by reductive amination reaction using aldehyde ($R_1CHO$) and a reducing agent in an inert solvent with or without an acid. The reducing agent includes

sodium triacetoxyborohydride, sodium cyanoborohydride, sodium borohydride, or boran-pyridine complex, preferably sodium triacetoxyborohydride or sodium cyanoborohydride. The inert solvent includes lower alkyl alcohol solvents (preferably methanol or ethanol, etc.), lower halocarbon solvents (preferably dichloromethane, dichloroethane, or chloroform, etc.), ethereal solvents (preferably tetrahydrofuran or dioxane), or aromatic solvents (preferably toluene, etc.). The acid includes an inorganic acid (preferably hydrochloric acid or sulfuric acid) or an organic acid (preferably acetic acid). Reaction temperature ranges from about -20°C to 120°C, preferably about 0°C to 100°C. Also this reaction can be carried out under microwave conditions.

The novel urea (G") of the present invention can be obtained by urea reaction using an isocyanate ($R_1NCO$) in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N*,*N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N*,*N*-dimethylformamide or dimethyl sulfoxide, etc.). Reaction temperature ranges from about -20°C to 120°C, preferably about 0°C to 100°C.

The amine (D") is reacted with a isothiocyanate ($R_1NCS$) in an inert solvent with or without a base to provide the novel thiourea (H") of the present invention. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N,N*-diisopropylethylamine, triethylamine, or *N*-methylmorpholine, etc.), or an aromatic amine (preferably pyridine or imidazole, etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or amide solvents (preferably *N*,*N*-dimethylformamide, etc.). Reaction temperature ranges from about -20°C to 120°C, preferably about 0°C to 100°C.

The novel urethane (I") of the present invention can be obtained by urethane reaction using $R_1OCOCl$, wherein X is halogen such as chloro, bromo, or iodo, in an inert solvent with or without a base. The base includes an alkali metal carbonate (preferably sodium carbonate or potassium carbonate, etc.), an alkali metal hydrogencarbonate (preferably sodium hydrogencarbonate or potassium hydrogencarbonate, etc.), an alkali hydroxide (preferably sodium hydroxide or potassium hydroxide, etc.), a tertiary amine (preferably *N,N*-diisopropylethylamine, triethylamine, or N-methylmorpholine, etc.), or an aromatic amine (preferably pyridine, imidazole, or poly-(4-vinylpyridine), etc.). The inert solvent includes lower halocarbon solvents (preferably dichloromethane, dichloroethane, or chloroform, etc.), ethereal solvents (preferably tetrahydrofuran or dioxane), aromatic solvents (preferably benzene or toluene, etc.), or polar solvents (preferably *N*,*N*-dimethylformamide or dimethyl sulfoxide, etc.). Reaction temperature ranges from about -20°C to 120°C, preferably about 0°C to 100°C.

*Scheme 18*

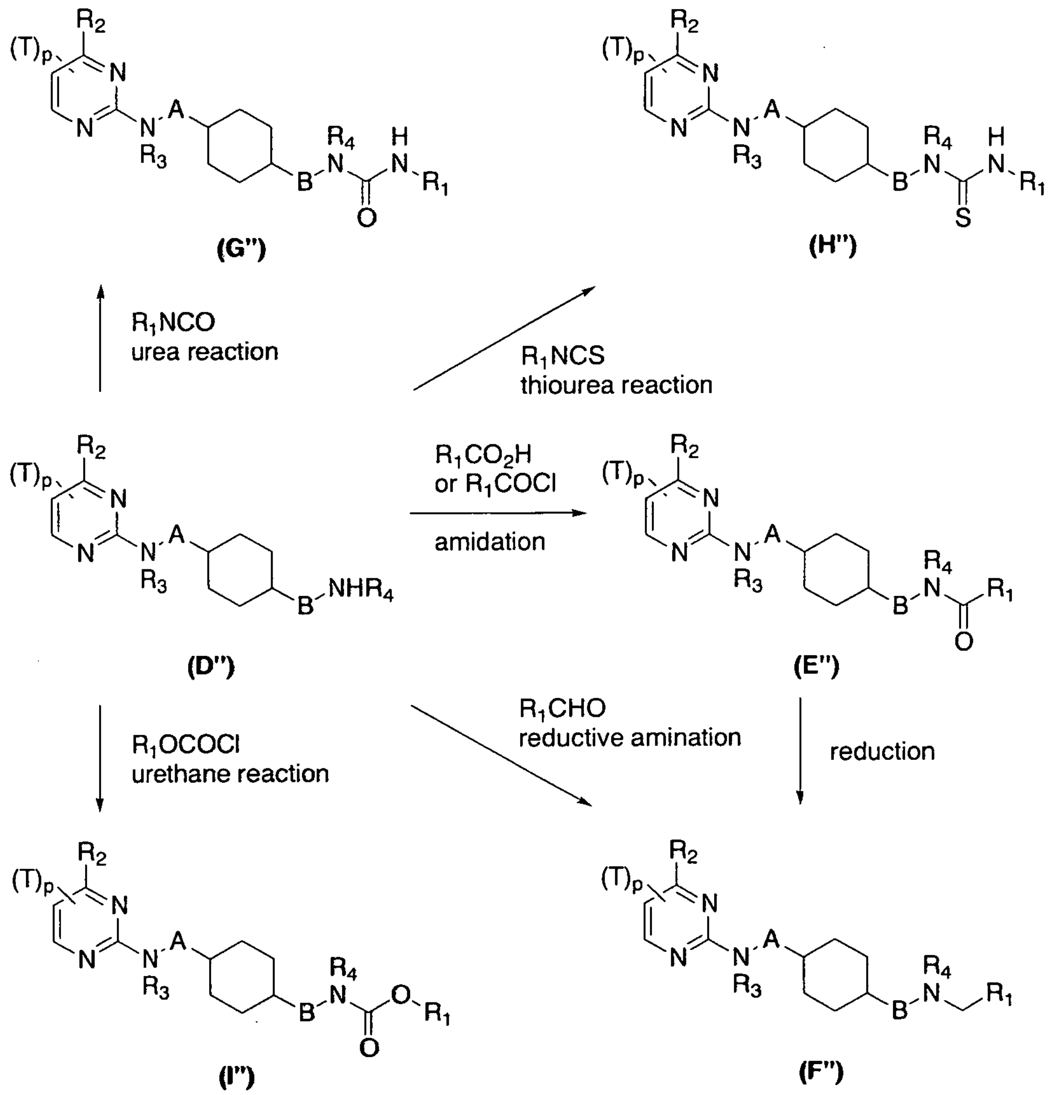


Compounds of Formula (J") can be prepared as shown in Scheme 19. The coupling of the amine (M), which is synthesized as scheme 3, with pyrimidine core (B"), which is synthesized as scheme 17, gives 2-substituted amino pyrimidine. The deprotection of Z-group is achieved by hydrogen reduction to give compounds of Formula (J").

## *Scheme 19*

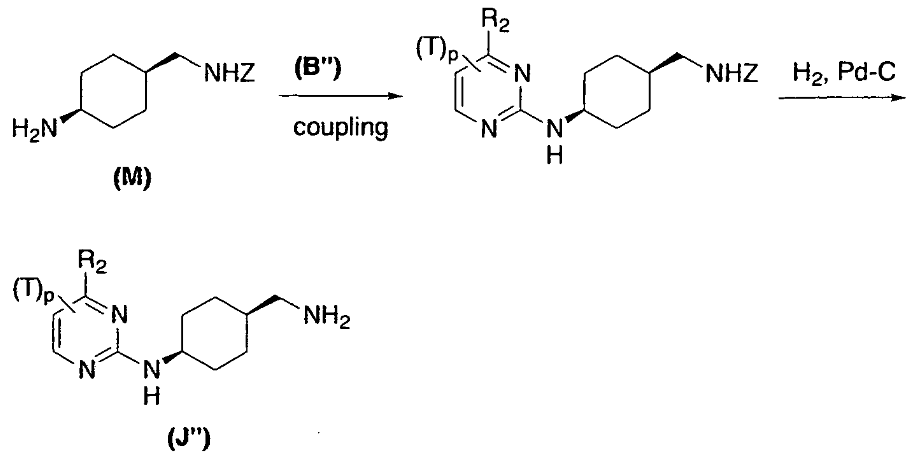


(M)

(J")

Compounds of Formula (K") can be prepared as shown in Scheme 20. The coupling of the amine (O), which is synthesized as scheme 4, with pyrimidine core (B"), which is synthesized as scheme 17, gives 2-substituted amino pyrimidine. The deprotection of Boc-group is achieved by an acid to give the amine (K").

## *Scheme 20*

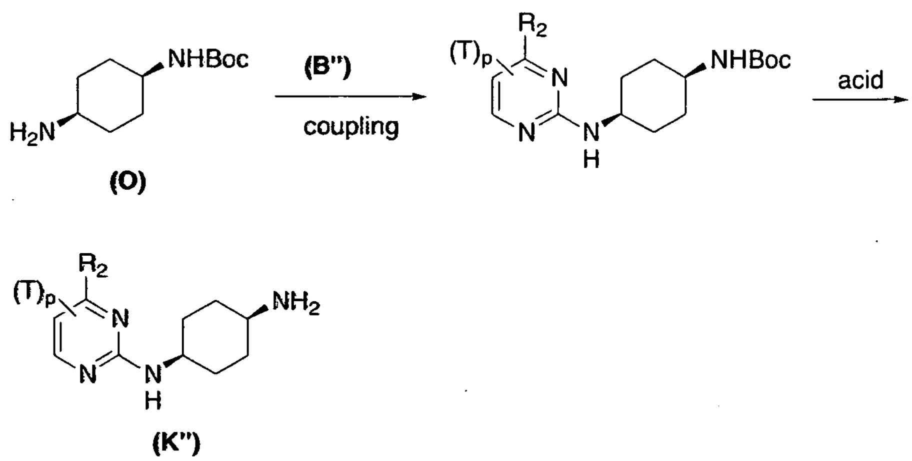


(O)

(K")

Alternatively, the novel quinoline (M"), the novel tetrahydroquinazoline (N"), the novel pyrimidine (O"), the novel quinoline (P"), and the novel pyrimidine (Q") of the present invention are directly synthesized from the quinoline core (C), which is synthesized in Scheme 1, the tetrahydroquinazoline core (T), which is synthesized in Scheme 5, the pyrimidine core (F'), which is synthesized in Scheme 9, the quinoline core (Q'), which is synthesized in Scheme 13, and the pyrimidine core (B"), which is synthesized in Scheme 17, as shown in Scheme 21. This coupling is performed with or without a base in an inert solvent. The base includes an alkali metal carbonate (preferably sodium carbonate or potassium carbonate, etc.), an alkali metal hydroxide (preferably sodium hydroxide, etc.), or a tertiary amine (preferably *N,N*-diisopropylethylamine, triethylamine, or *N*-methylmorpholine, etc.). The inert solvent includes lower alkyl alcohol solvents (preferably methanol, ethanol, 2-propanol, or butanol, etc.) or amide solvents (preferably *N,N*-dimethylformamide or 1-methyl-pyrrolidin-2-one, etc.). Reaction temperature ranges from about 50°C to 200°C, preferably about 80°C to 180°C. Also this reaction can be carried out under microwave conditions.

*Scheme 21*

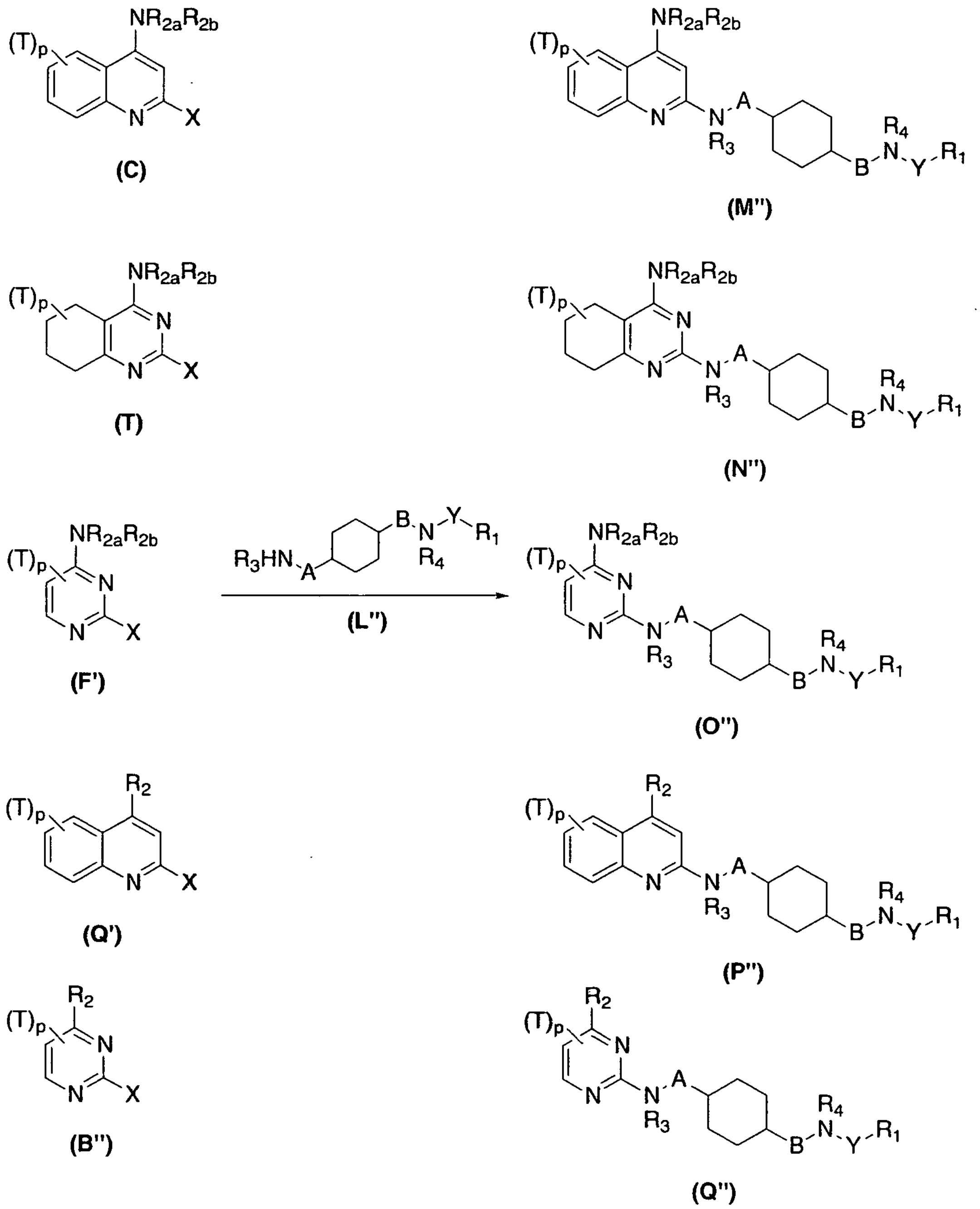

(C)

(M")

(T)

(N")

(F')

(L")

(O")

(Q')

(P")

(B")

(Q")

For example, compounds of Formula (T") can be prepared as shown in Scheme 22. The amine (O), which is synthesized in Scheme 4, is subjected to reductive amination by aldehyde ($R_1CHO$). The deprotection of Boc-group is achieved by an acid to give the amine. The coupling of the amine with pyrimidine core (F'), which is synthesized as scheme 9, gives the novel pyrimidine (T") of the present invention.

*Scheme 22*

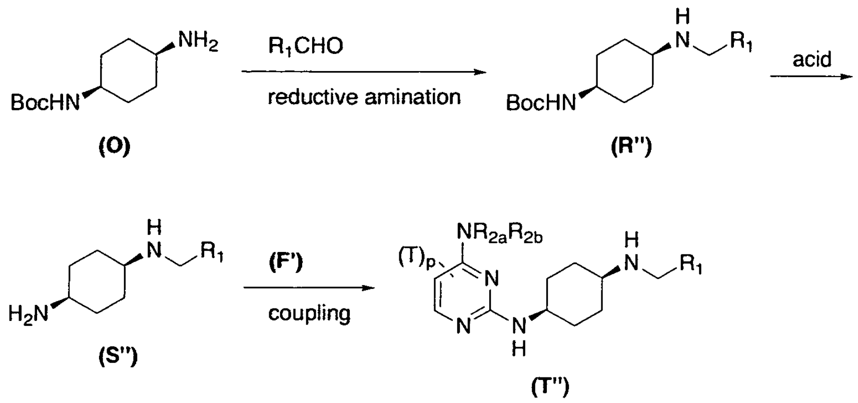


Compounds of Formula (W") can be prepared as shown in Scheme 23. The amine (O), which is synthesized in Scheme 4, is subjected to amidation by carboxylic acid ($R_1CO_2H$) or acid chloride ($R_1COCl$). The deprotection of Boc-group is achieved by an acid to give the amine. The coupling of the amine with quinoline core (Q'), which is synthesized as scheme 13, gives the novel quinoline (W") of the present invention.

*Scheme 23*

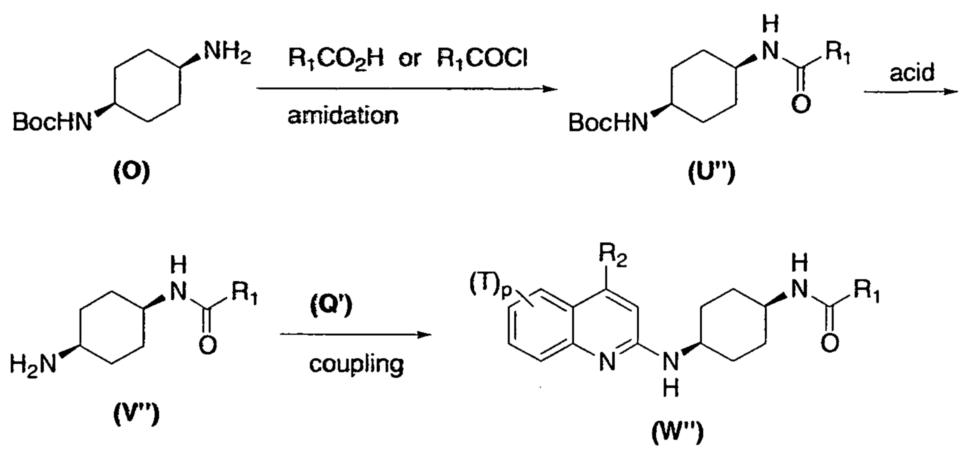


Compounds of Formula (Z") can be prepared as shown in Scheme 24. The amine (O), which is synthesized in Scheme 4, is subjected to amidation by carboxylic acid ($R_1CO_2H$) or acid chloride ($R_1COCl$). The deprotection of Boc-group is achieved by an acid to give the amine. The coupling of the amine with pyrimidine core (B"), which is synthesized as scheme 17, gives the novel pyrimidine (Z") of the present invention.

## *Scheme 24*

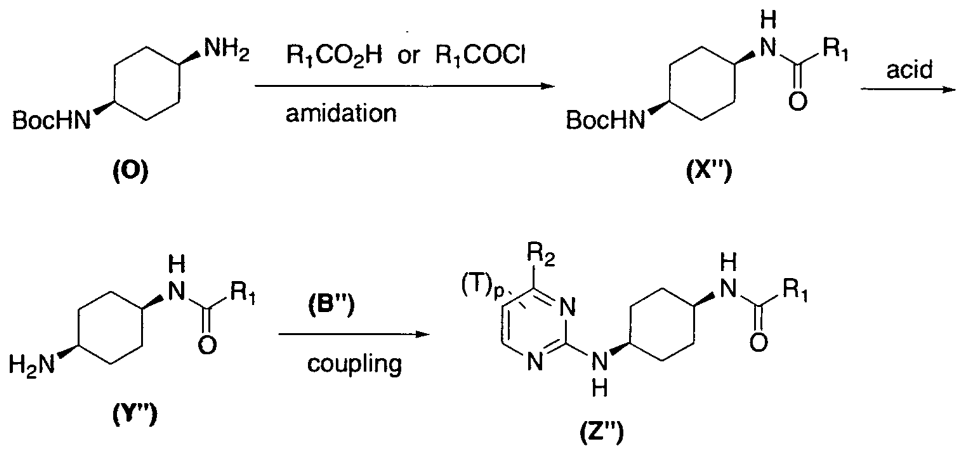


(O) (X") (Y") (Z")

When a compound of the invention contains optical isomers, stereoisomers, regio isomers, rotational isomers, a single substance and a mixture of them are included as a compound of the invention. For example, when a chemical formula is represented as showing no stereochemical designation(s), such as Formula VI, then all possible stereoisomer, optical isomers and mixtures thereof are considered within the scope of that formula. Accordingly, Formula VII, specifically designates the cis relationship between the two amino groups on the cyclohexyl ring and therefore this formula is also fully embraced by Formula VI.

Other uses of the disclosed invention will become apparent to those in the art based upon, inter alia, a review of this patent document.

The following examples are given to illustrate the invention and are not intended to be inclusive in any manner:

### Examples

The compounds of the invention and their synthesis are further illustrated by the following examples. The following examples are provided to further define the invention without, however, limiting the invention to the particulas of these examples. "Ambient temperature" as referred to in the following example is meant to indicate a temperature falling between 0 °C and 40 °C. The following compounds are named by Beilstein Auto Nom Version 4.0, CS Chem Draw Ultra Version 6.0, CS Chem Draw Ultra Version 6.0.2, CS Chem Draw Ultra Version 7.0.1, or ACD Name Version 7.0.

Abbreviations used in the instant specification, particularly the Schemes and Examples, are as follows:

| | |
|---|---|
| $^1H$ NMR : | proton nuclear magnetic resonance spectrum |
| AcOH : | acetic acid |
| APCI : | atmospheric pressure chemical ionization |
| $(Boc)_2O$ : | di-tertiary-butyl dicarbonate |
| BuLi : | butyl lithium |
| BuOH : | butanol |
| Cbz : | carbobenzoxy |
| $CDCl_3$: | deuterated chloroform |
| $CH_2Cl_2$ : | dichloromethane |
| $CHCl_3$: | chloroform |
| CI : | chemical ionization |
| mCPBA: | meta chloroperbenzoic acid |
| DMA: | N,N-dimethyl acetamide |
| DCM : | dichloromethane |
| DIEA : | diisopropylethylamine |
| DMSO : | dimethyl sulfoxide |
| Dppf: | bis-(diphenylphosphino)ferrocene |
| EI : | electron ionization |

| | |
|---|---|
| ESI : | electrospray ionization |
| $Et_2O$ : | diethyl ether |
| EtOAc : | acetic acid ethyl ester |
| EtOH : | ethanol |
| FAB : | fast atom bombardment |
| HATU : | *O*-(7-azabenzotriazol-1-yl)-*N*,*N*,*N*',*N*'-tetramethyluronium-Hexafluorophosphate |
| $H_2SO_4$ : | sulfuric acid |
| HCl : | hydrogen chloride |
| IPA: | isopropanol |
| $K_2CO_3$ : | potassium carbonate |
| $Me_2NH$ : | dimethylamine |
| $MeNH_2$ : | methylamine |
| MeOH : | methanol |
| $MgSO_4$ : | magnesium sulfate |
| MsOH : | methanesulfonic acid |
| $NaBH(OAc)_3$: | sodium triacetoxyborohydride |
| $NaBH_3CN$ : | sodium cyanoborohydride |
| $NaBH_4$ : | sodium borohydride |
| $NaHCO_3$ : | sodium hydrogencarbonate |
| Pd/C : | palladium carbon |
| $POCl_3$ : | phosphoryl chloride |
| PVP : | poly(4-vinylpyridine) |
| $SOCl_2$ : | thionyl chloride |
| TBME: | tert-butyl methyl ether |
| TFA : | trifluoroacetic acid |
| THF : | tetrahydrofuran |
| ZCl : | benzyloxycarbonyl chloride |
| s : | singlet |
| d : | doublet |
| t : | triplet |
| q : | qualtet |
| dd : | doublet doublet |
| dt : | doublet triplet |
| ddd : | doublet doublet doublet |
| brs : | broad singlet |
| m : | multiplet |
| *J* : | coupling constant |
| Hz : | Hertz |

**Example 1**

***N*²-[*cis*-4-(4-Bromo-2-trifluoromethoxy-benzyl)-amino-cyclohexyl]-*N*⁴-methyl-quinoline-2,4-diamine dihydrochloride**

**Step A: Synthesis of 2,4-dichloro-quinoline.**

A suspension of quinoline-2,4-diol (150 g, 931 mmol) in $POCl_3$ (975 mL, 10.4 mol) was stirred at reflux for 6 hr and the reaction mixture was concentrated. The residue was diluted with $CHCl_3$ (500 mL) and the solution was poured into ice water. The aqueous layer was extracted with $CHCl_3$ (three times). The combined organic layer was dried over $MgSO_4$, filtrated, concentrated, and purified by flash chromatography (silica gel, 20% EtOAc in hexane) to give 2,4-dichloro-quinoline (177 g, 96%) as a pale brown solid.
EI MS m/e 197, $M^+$ ; $^1H$ NMR (300 MHz, $CDCl_3$) $\delta$ 7.50 (s, 1 H), 7.65 (ddd, *J* = 8.3, 7.0, 1.3 Hz, 1 H), 7.79 (ddd, *J* = 8.5, 7.0, 1.3 Hz, 1 H), 8.00-8.06 (m, 1 H), 8.16-8.21 (m, 1 H).

**Step B: Synthesis of (2-chloro-quinolin-4-yl)-methyl-amine.**

To a solution of 2,4-dichloro-quinoline (29.8 g, 150 mmol) in THF (300 mL) was added 40% $MeNH_2$ in water (58.4 g, 752 mmol). The mixture was stirred at ambient temperature for 12 days and concentrated. The residue was

suspended in $CHCl_3$ and $H_2O$. The precipitate was collected by filtration, washed with acetone, and dried at 50 °C under reduced pressure to give (2-chloro-quinolin-4-yl)-methyl-amine (13.2 g, 45%) as a colorless solid.
ESI MS m/e 215, M + $Na^+$; $^1H$ NMR (300 MHz, DMSO-$d_6$) δ 2.91 (d, *J* = 4.7 Hz, 3 H), 6.35 (s, 1 H), 7.47 (ddd, *J* = 8.3, 6.6, 1.7 Hz, 1 H), 7.62-7.75 (m, 3 H), 8.16 (d, *J* = 8.6 Hz, 1 H).

**Step C: Synthesis of (*cis*-4-benzyloxycarbonylamino-cyclohexyl)-carbamic acid-benzyl ester.**

To a suspension of cis-cyclohexane-1,4-dicarboxylic acid (25.0 g, 145 mmol) in benzene (125 mL) were added phosphorazidic acid diphenyl ester (81.9 g, 298 mmol) and triethylamine (30.1 g, 297 mmol). The reaction mixture was stirred at reflux for 2.5 hr. Benzyl alcohol (32.2 g, 298 mmol) was added and the mixture was stirred at reflux for 24 hr. The reaction mixture was concentrated and the residue was dissolved in EtOAc and $H_2O$. The organic layer was separated and the aqueous layer was extracted with EtOAc (twice). The combined organic layer was washed with 1 M aqueous $KHSO_4$, saturated aqueous $NaHCO_3$, and brine, dried over $MgSO_4$, filtrated, concentrated, and purified by flash chromatography (silica gel, 33% EtOAc in hexane) to give (*cis*-4-benzyloxycarbonylaminocyclohexyl)-carbamic acid benzyl ester (52.0 g, 94%) as a colorless oil.
ESI MS m/e 405, M + $Na^+$; $^1H$ NMR (300 MHz, $CDCl_3$) δ 1.45-1.60 (m, 4 H), 1.60-1.80 (m, 4 H), 3.52-3.80 (m, 2 H), 4.70-5.00 (m, 2 H), 5.07 (s, 4 H), 7.15-7.40 (m, 10 H).

**Step D: Synthesis of (*cis*-4-amino-cyclohexyl)-carbamic acid *tert*-butyl ester.**

To a solution of (*cis*-4-benzyloxycarbonylamino-cyclohexyl)-carbamic acid benzyl ester (91.7 g, 240 mmol) in MeOH (460 mL) was added 5% Pd/C (9.17 g). The reaction mixture was stirred at ambient temperature under hydrogen atmosphere for 2.5 days, filtrated through a pad of celite, and concentrated to give a diamine as a colorless oil. To a solution of the diamine in MeOH (550 mL) was added a solution of $(Boc)_2O$ (6.59 g, 30.2 mmol) in MeOH (80 mL) dropwise over 4 hr. The reaction mixture was stirred at ambient temperature for 1.5 days and concentrated. After dissolution with $H_2O$, the aqueous layer was extracted with $CHCl_3$ (three times). The combined organic layer was dried over $MgSO_4$, filtrated, and concentrated to give (*cis*-4-amino-cyclohexyl)-carbamic acid *tert*-butyl ester (7.78 g, 15%, crude) as a colorless oil. The aqueous layer was concentrated and the residue was dissolved in MeOH, dried over $MgSO_4$, filtrated, and concentrated to give a recovered diamine (32.9 g) as a colorless oil. To a solution of the recovered diamine (32.9 g, 288 mmol) in MeOH (660 mL) was added a solution of $(Boc)_2O$ (6.29 g, 28.8 mmol) in MeOH (80 mL) dropwise over 5 hr. The reaction mixture was stirred at ambient temperature for 10 hr and concentrated. After dissolution with $H_2O$, the aqueous layer was extracted with $CHCl_3$ (three times). The combined organic layer was dried over $MgSO_4$, filtrated, and concentrated to give (*cis*-4-aminocyclohexyl)-carbamic acid *tert*-butyl ester (8.16 g, 16%, crude) as a colorless oil. The aqueous layer was concentrated and the residue was dissolved in MeOH, dried over $MgSO_4$, filtrated, and concentrated to give a recovered diamine (23.1 g) as a colorless oil. To a solution of the recovered diamine (23.1 g, 202 mmol) in MeOH (462 mL) was added a solution of $(BOC)_2O$ (4.42 g, 20.3 mmol) in MeOH (56 mL) dropwise over 4 hr. The reaction mixture was stirred at ambient temperature for 3.5 days and concentrated. After dissolution with $H_2O$, the aqueous layer was extracted with $CHCl_3$ (three times). The combined organic layer was dried over $MgSO_4$, filtrated, and concentrated to give (cis-4-amino-cyclohexyl)-carbamic acid *tert*-butyl ester (5.01 g, 10% based on starting material) as a colorless oil. The aqueous layer was concentrated and the residue was dissolved in MeOH, dried over $MgSO_4$, filtrated, and concentrated to give a recovered diamine (16.0 g) as a colorless oil. To a solution of the recovered diamine (16.0 g, 140 mmol) in MeOH (320 mL) was added a solution of $(Boc)_2O$ (3.06 g, 14.0 mmol) in MeOH (40 mL) dropwise over 4 hr. The reaction mixture was stirred at ambient temperature for 13 hr and concentrated. After dissolution with $H_2O$, the aqueous layer was extracted with $CHCl_3$ (three times). The combined organic layer was dried over $MgSO_4$, filtrated, and concentrated to give (*cis*-4-amino-cyclohexyl)-carbamic acid *tert*-butyl ester (3.53 g, 7% based on the starting material) as a colorless oil. The aqueous layer was concentrated and the residue was dissolved in MeOH, dried over $MgSO_4$, filtrated, and concentrated to give a recovered diamine (11.1 g) as a colorless oil.
ESI MS m/e 215, M + $H^+$ ; $^1H$ NMR (300 MHz, $CDCl_3$) δ 1.20-1.80 (m, 8 H), 1.44 (s, 9 H), 2.78-2.95 (m, 1 H), 3.50-3.80 (m, 1 H), 4.30-4.82 (m, 1 H).

**Step E: Synthesis of $N^2$-(*cis*-4-amino-cyclohexyl)-$N^4$-methyl-quinoline-2,4-diamine.**

A mixture of (2-chloro-quinolin-4-yl)-methyl-amine (2.00 g, 10.4 mmol) and (*cis*-4-aminocyclohexyl)-carbamic acid *tert*-butyl ester (2.45 g, 11.4 mmol) in butanol (3 mL) was stirred at 130 °C for 2 days in a sealed tube. The reaction mixture was poured into saturated aqueous $NaHCO_3$ and the aqueous layer was extracted with $CHCl_3$ (three times). The combined organic layer was dried over $MgSO_4$, filtrated, concentrated, and purified by medium-pressure liquid chromatography (NH-silica gel, 20% EtOAc in hexane) to give [*cis*-4-(4-methylamino-quinolin-2-ylamino)-cyclohexyl]-carbamic acid *tert*-butyl ester (1.45 g) as a pale yellow oil. To a solution of the above material (1.31 g) in EtOAc (15

mL) was added 4 M hydrogen chloride in EtOAc (30 mL). The reaction mixture was stirred at ambient temperature for 5 hr. The precipitate was collected by filtration and dissolved in saturated aqueous $NaHCO_3$. The aqueous layer was extracted with $CHCl_3$ (three times). The combined organic layer was dried over $MgSO_4$, filtrated, and concentrated to give *N*$^2$-(cis-4-amino-cyclohexyl)-*N*$^4$-methyl-quinoline-2,4-diamine (999 mg, 40%) as a pale yellow solid.
EI MS m/e 271 M + $H^+$ ; $^1$H NMR (300 MHz, DMSO-$d_6$) δ 1.42-1. (m, 8 H), 2.81 (d, *J* = 4.7 Hz, 3 H), 2.89-3.01 (m, 1 H), 3.17 (s, 2 H), 4.07 (brs, 1 H), 5.77 (s, 1 H), 6.32 (d, *J* = 6.5 Hz, 1 H), 6.69-6.80 (m, 1 H), 6.94-7.06 (m, 1 H), 7.34 (d, *J* = 3.7 Hz, 2 H), 7.85 (d, *J* = 8.2 Hz, 1 H).

**Step F: Synthesis of 4-bromo-2-trifluoromethoxy-benzaldehyde.**

A solution of 4-bromo-1-iodo-2-trifluoromethoxy-benzene (1.00 g, 2.72 mmol) in THF (15 mL) was cooled to -78 °C and 2.66 M BuLi in hexane (2.05 mL, 5.44 mmol) was added dropwise. The reaction mixture was stirred at -78 °C for 1.5 h and *N*-formylmorpholine (0.57 mL, 5.63 mmol) was added. The reaction mixture was stirred at -78 °C for 15 min and at ambient temperature for 80 min. The reaction was quenched with 0.25 M aqueous citric acid (10 mL) and the resulting mixture was extracted with EtOAc (three times). The combined organic layer was dried over $MgSO_4$, filtrated, concentrated, and purified by flash chromatography (silica gel, 2% to 5% EtOAc in hexane) to give 4-bromo-2-trifluoromethoxy-benzaldehyde (560 mg, 77%) as a pale brown solid.
CI MS m/e 269, M + $H^+$ ; $^1$H NMR (300 MHz, $CDCl_3$) δ 7.50-7.67 (m, 2 H), 7.85 (d, *J* = 8.1 Hz, 1 H), 10.33 (s, 1 H).

**Step G: Synthesis of *N*$^2$-[*cis*-4-(4-bromo-2-trifluoromethoxy-benzyl)-amino-cyclohexyl]-*N*$^4$-methyl-quinoline-2,4-diamine dihydrochloride.**

To a solution of *N*$^2$-(*cis*-4-amino-cyclohexyl)-*N*$^4$ -methyl-quinoline-2,4-diamine (370 mg, 1.37 mmol) in methanol (4 mL) were added 4-bromo-2-trifluoromethoxy-benzaldehyde (368 mg, 1.37 mmol), acetic acid (82 mg, 1.37 mmol), and $NaBH_3CN$ (129 mg, 2.05 mmol). The reaction mixture was stirred at ambient temperature for 20 hr. The reaction was quenched with saturated aqueous $NaHCO_3$ and the aqueous layer was extracted with $CHCl_3$ (three times). The combined organic layer was dried over $MgSO_4$, filtrated, concentrated, and purified by medium-pressure liquid chromatography (NH-silica gel, 20% EtOAc in hexane) and flash chromatography (silica gel, 5% MeOH in $CHCl_3$) to give a colorless oil. To a solution of the above oil in EtOAc (2 mL) was added 4 M hydrogen chloride in EtOAc (5 mL). The mixture was stirred at ambient temperature for 1 hr and concentrated. A suspension of the residue in $Et_2O$ (12 mL) was stirred at ambient tempareture for 1 hr. The precipitate was collected by filtration, washed with $Et_2O$, and dried under reduced pressure to give *N*$^2$-[*cis*-4-(4-bromo-2-trifluoromethoxy-benzyl)-amino-cyclohexyl]-*N*$^4$-methylquinoline-2,4-diamine dihydrochloride (365 mg, 45%) as a white solid.
ESI MS m/e 523, M (free) + $H^+$ ; $^1$H NMR (300 MHz, DMSO-$d_6$) δ 1.61-2.11 (m, 8 H), 2.96 (d, *J* = 4.4 Hz, 3 H), 3.19-3.41 (m, 2 H), 4.11-4.34 (m, 2 H), 5.92 (brs, 1 H), 7.40 (t, *J* = 8.2 Hz, 1 H), 7.63-7.79 (m, 3 H), 7.93 (d, *J* = 8.4 Hz, 1H), 8.22 (d, J = 8.2 Hz, 1H), 8.30-8.48 (m, 2 H), 9.59 (brs, 2H).

**Example 2**

***N*$^2$-{cis-4-[2-(4-Bromo-2-trifluoromethoxy-phenyl)-ethylamino]-cyclohexyl}-*N*$^4$-methylquinoline2,4-diamine dihydrochloride**

**Step A: Synthesis of (4-bromo-2-trifluoromethoxy-phenyl)-acetaldehyde.**

To a suspension of (methoxymethyl)-triphenylphosphonium chloride (5.29 g, 14.9 mol) in $Et_2O$ (50 mL) was added 1.8 M phenyl lithium in 30% $Et_2O$ in cyclohexane (8.58 mL, 15.5 mmol). The mixture was stirred at ambient temperature for 10 min. To the reaction mixture was added 4-bromo-2-trifluoromethoxy-benzaldehyde obtained in step F of example 1 (4.00 g, 14.9 mmol) in $Et_2O$ (18 mL). The mixture was stirred at ambient temperature for 4 hr, filtrated and concentrated. To the above residue was added 10% $H_2SO_4$ in AcOH (40 mL). The mixture was stirred at ambient temperature for 90 min. The solution was poured into $H_2O$ and the aqueous layer was extracted with $CHCl_3$ (three times). The combined organic layer was washed with saturated aqueous $NaHCO_3$ and brine, dried over $MgSO_4$, filtrated, concentrated, and purified by flash chromatography (silica gel, 9% EtOAc in hexane) to give (4-bromo-2-trifluoromethoxy-phenyl)-acetaldehyde (1.25 g, 30 %) as a pale brown oil.
ESI MS m/e 284, M + $H^+$; $^1$H NMR (200 MHz, $CDCl_3$) δ 3.75 (d, *J* = 1.5 Hz, 2 H), 7.16 (d, *J* = 8.4 Hz, 1 H), 7.41-7.51 (m, 2 H), 9.74 (t, *J* = 1.5 Hz, 1 H).

**Step B: Synthesis of *N*$^2$-{*cis*-4-[2-(4-bromo-2-trifluoromethoxy-phenyl)-ethylamino]-cyclohexyl)-*N*$^4$-methyl-quinoline-2,4-diamine dihydrochloride.**

Using the procedure for the step G of example 1, the title compound was obtained. ESI MS m/e 537, M (free) + $H^+$ ; $^1H$ NMR (300 MHz, DMSO-$d_6$) δ 1.62-2.06 (m, 8 H), 2.96 (d, J = 4.4 Hz, 3 H), 3.04-3.39 (m, 5 H), 4.17 (brs, 1 H), 5.90 (brs, 1 H), 7.40 (t, *J* = 8.2 Hz, 1 H), 7.52 (d, *J* = 8.7 Hz, 1 H), 7.57-7.85 (m, 3 H), 8.20 (d, *J* = 8.2 Hz, 1 H), 8.26-8.47 (m, 2 H), 9.23 (brs, 2 H).

**Example 3**

***N*$^2$-{*cis*-4-[(4-Bromo-2-trifluoromethoxy-benzyl)-amino-methyl]-cyclohexyl}-*N*$^4$-methylquinoline-2,4-diamine dihydrochloride**

**Step A: Synthesis of (*cis*-4-hydroxymethyl-cyclohexyl)-carbamic acid *tert*-butyl ester.**

A suspension of *cis*-4-amino-cyclohexanecarboxylic acid (244 g, 1.70 mol) in MeOH (2.45 L) was cooled to -8 °C . Thionyl chloride (45.0 mL, 617 mmol) was added dropwise. The resulting solution was stirred at ambient temperature for 4.5 hr and concentrated to give a white solid. To a suspension of the above solid in $CHCl_3$ (3.00 L) were added triethylamine (261 mL, 1.87 mol) and $(Boc)_2O$ (409 g, 1.87 mol) successively. The reaction mixture was stirred at ambient temperature for 5 hr and poured into water. The aqueous layer was extracted with $CHCl_3$ (three times). The combined organic layer was dried over $MgSO_4$, filtrated, concentrated, and purified by flash chromatography (silica gel, $CHCl_3$ only to 10% MeOH in $CHCl_3$) to give a colorless oil (531 g). To a suspension cooled at -4 °C of lithium aluminum hydride (78.3 g, 2.06 mol) in $Et_2O$ (7.9 L) was added a solution of the above oil (530.9 g) in $Et_2O$ (5.3 L) below 0 °C. The resulting suspension was stirred at ambient temperature for 2 hr. The reaction mixture was cooled on an ice-bath, quenched with cold water, and filtrated through a pad of celite. The filtrate was dried over $MgSO_4$, filtrated, and concentrated. The precipitate was suspended in hexane (300 mL), filtrated, washed with hexane, and dried under reduced pressure to give (*cis*-4-hydroxymethyl-cyclohexyl)-carbamic acid *tert*-butyl ester (301 g, 77%) as a white solid. ESI MS m/e 252, M + $Na^+$; $^1H$ NMR (300 MHz, $CDCl_3$) δ 1.16-1.36 (m, 2 H), 1.45 (s, 9 H), 1.52-1.77 (m, 7 H), 3.51 (d, *J* = 6.2 Hz, 2 H), 3.75 (brs, 1 H), 4.30-4.82 (m, 1 H).

**Step B: Synthesis of [*cis*-4-(benzyloxycarbonylamino-methyl)-cyclohexyl]-carbamic acid *tert*-butyl ester.**

To a solution of (*cis*-4-hydroxymethyl-cyclohexyl)-carbamic acid *tert*-butyl ester (17.7 g, 77.2 mmol) in THF (245 mL) were added triphenylphosphine (20.2 g, 77.0 mmol) and phthalimide (11.4 g, 77.5 mmol) successively. The resulting suspension was cooled on an ice-bath and 40% diethyl azodicarboxylate in toluene (33.6 mL, 74.1 mmol) was added over 1 hr. The reaction mixture was stirred at ambient temperature for 2.5 days, concentrated, and purified by flash chromatography (silica gel, 33% EtOAc in hexane) to give a white solid. To a suspension of the above solid (27.5 g) in EtOH (275 mL) was added hydrazine hydrate (5.76 g, 115 mmol). The mixture was stirred at reflux for 2.25 hr, cooled, and concentrated. The precipitate was dissolved in 10% aqueous sodium hydroxide (350 mL). The aqueous layer was extracted with $CHCl_3$ (three times). The combined organic layer was dried over $MgSO_4$, filtrated, and concentrated. To a solution of the above residue in $CHCl_3$ (275 mL) was added triethylamine (8.54 g, 84.4 mmol). The resulting solution was cooled to 0 °C and ZCl (14.4 g, 84.4 mmol) was added below 5 °C. The reaction mixture was stirred at ambient temperature for 16 hr and poured into saturated aqueous $NaHCO_3$. The aqueous layer was extracted with $CHCl_3$ (three times). The combined organic layer was dried over $MgSO_4$, filtrated, concentrated, and purified by flash chromatography (silica gel, 2% MeOH in $CHCl_3$) to give [*cis*-4-(benzyloxyearbonylamino-methyl)-cyclohexyl]-carbamic acid *tert*-butyl ester (25.3 g, 91 %) as a colorless oil.
ESI MS m/e 385, M + $Na^+$; $^1H$ NMR (300 MHz, $CDCl_3$) δ 1.13-1.31 (m, 2 H), 1.44 (s, 9 H), 1.48-1.75 (m, 7 H), 3.10 (t, *J* = 6.4 Hz, 2 H), 3.72 (brs, 1 H), 4.42-4.76 (m, 1 H), 4.76-4.92 (m, 1 H), 5.09 (s, 2 H), 7.27-7.38 (m, 5 H).

**Step C: Synthesis of (*cis*-4-amino-cyclohexylmethyl)-carbamic acid benzyl ester.**

To a solution of [*cis*-4-(benzyloxycarbonylamino-methyl)-cyclohexyl]-carbamic acid *tert-butyl* ester (12.9 g, 35.6 mmol) in EtOAc (129 mL) was added 4 M hydrogen chloride in EtOAc (129 mL). The reaction mixture was stirred at ambient temperature for 3 hr, filtrated, washed with EtOAc, and dried under reduced pressure. The solid was dissolved in saturated aqueous $NaHCO_3$. The aqueous layer was extracted with $CHCl_3$ (five times). The combined organic layer was dried over $MgSO_4$, filtrated, concentrated, and dried under reduced pressure to give (*cis*-4-aminocyclohexylmethyl)-carbamic acid benzyl ester (8.88 g, 95 % ) as a colorless oil.
ESI MS m/e 263, M + $H^+$; $^1H$ NMR (300 MHz, $CDCl_3$) δ 1.36-1.98 (m, 9 H), 2.96-3.32 (m, 3 H), 5.12 (brs, 3 H), 7.36

(s, 5 H).

**Step D: Synthesis of [*cis*-4-(4-methylamino-quinolin-2-ylamino)-cyclohexylmethyl]-carbamic acid benzyl ester.**

A mixture of (2-chloro-quinolin-4-yl)-methyl-amine obtained in step B of example 1 (2.00 g, 10.4 mmol) and (*cis*-4-amino-cyclohexylmethyl)-carbamic acid benzyl ester (3.27 g, 12.5 mmol) in butanol (3 mL) was stirred at 130 °C for 16 hr in a sealed tube. The reaction mixture was poured into saturated aqueous $NaHCO_3$, and the aqueous layer was extracted with $CHCl_3$ (three times). The combined organic layer was dried over $MgSO_4$, filtrated, concentrated, and purified by flash chromatography (silica, 10% MeOH in $CHCl_3$) to give [*cis*-4-(4-methylamino-quinolin-2-ylamino)-cyclohexylmethyl]-carbamic acid-benzyl ester (2.16 g, 49%) as a white solid.
ESI MS m/e 419, M + $H^+$ $^1H$ NMR (300 MHz, $CDCl_3$) δ 1.42-1.99 (m, 9 H), 3.05 (d, *J* = 4.7 Hz, 3 H), 3.08-3.16 (m, 2 H), 3.81 (brs,1 H), 5.07 (s, 2 H), 5.18-5.28 (m, 1 H), 5.34 (s, 1 H), 7.07-7.18 (m, 1H), 7.22-7.45 (m, 6 H), 7.56-7.70 (m, 1 H), 8.16 (d, *J* = 8.4 Hz, 1 H), 8.23 (d, *J* = 7.6 Hz, 1 H), 12.76 (brs, 1H).

**Step E: Synthesis of *N*$^2$-{cis-4-[(4-bromo-2-trifluoromethoxy-benzyl)-amino-methyl]-cyclohexyl}-N$^4$-methyl-quinoline-2,4-diamine dihydrochloride.**

To a solution of [*cis*-4-(4-methylamino-quinolin-2-ylamino)-cyclohexylmethyl]-carbamic acid-benzyl ester (2.02 g, 4.83 mmol) in MeOH (20 mL) was added 10% Pd/C (202 mg). The mixture was stirred at 50°C under hydrogen atmosphere for 23.5 hr. The reaction mixture was filtrated through a pad of celite and concentrated. To a solution of the residue (500 mg) in methanol (5 mL) were added 4-bromo-2-trifluoromethoxy-benzaldehyde obtained in step F of example 1 (497 mg, 1.85 mmol), acetic acid (111 mg, 1.85 mmol), and $NaBH_3CN$ (166 mg, 2.64 mmol). The reaction mixture was stirred at ambient temperature for 23 hr. The reaction was quenched with saturated aqueous $NaHCO_3$ and the aqueous layer was extracted with $CHCl_3$ (three times). The combined organic layer was dried over $MgSO_4$, filtrated, concentrated, and purified by medium-pressure liquid chromatography (NH-silica gel, 50% EtOAc in hexane) and flash chromatography (silica gel, 2% to 50% MeOH in $CHCl_3$) to give a colorless oil. To a solution of the above oil in EtOAc (2 mL) was added 4 M hydrogen chloride in EtOAc (5 mL). The mixture was stirred at ambient temperature for 1 hr and concentrated. A suspension of the residue in $Et_2O$ (12 mL) was stirred at ambient tempareture for 1 hr. The precipitate was collected by filtration, washed with $Et_2O$, and dried under reduced pressure to give *N*$^2$-{*cis*-4-[(4-bromo-2-trifluoromethoxy-benzyl)aminomethyl]-cyclohexyl}-*N*$^4$-methyl-quinoline-2,4-diamine dihydrochloride (147 mg, 14%) as a white solid.
ESI MS m/e 537, M (free) + $H^+$; $^1H$ NMR (300 MHz, $CDCl_3$) δ 1.34-2.15 (m, 9 H), 2.63-3.08 (m, 5 H), 3.41-3.88 (m, 1 H), 4.28 (s, 2 H), 7.00-7.62 (m, 6 H), 7.65-8.38 (m, 3 H), 10.01 (brs, 2 H), 11.76 (brs, 1 H).

**Example 4**

***N*$^4$-Methyl-*N*$^2$-{*cis*-4-[(2-trifluoromethoxy-benzyl)-amino-methyl]-cyclohexyl) -quinoline-2,4-diamine dihydrochloride**

**Step A: Synthesis of *N*$^4$-methyl-*N*$^2$-{*cis*-4-[(2-trifluoromethoxy-benzyl)-amino-methyl]-cyclohexyl}-quinoline-2,4-diamine dihydrochloride.**

To a solution of *N*$^2$-{cis-4-[(4-bromo-2-trifluoromethoxy-benzyl)amino-methyl]-cyclohexyl}-*N*$^4$-methyl-quinoline-2,4-diamine obtained in step E of example 3 (250 mg, 0.465 mmol) in EtOH (2.5 mL) was added 10% Pd/C (75 mg). The mixture was stirred at ambient temperature under hydrogen atmosphere for 15 hr. The reaction mixture was filtrated through a pad of celite and purified by flash chromatography (NH-silica gel, 50% EtOAc in hexane) to give a colorless oil. To a solution of the above oil in EtOAc (2 mL) was added 4 M hydrogen chloride in EtOAc (5 mL). The mixture was stirred at ambient temperature for 1 hr and concentrated. The residue was suspended with $Et_2O$ (10 mL) and stirred at ambient tempareture for 1 hr. The precipitate was collected by filtration, washed with $Et_2O$ and dried under reduced pressure to give *N*$^4$-methyl-*N*$^2$-{*cis-4-[(2*-trifluoromethoxy-benzyl)amino-methyl]-cyclohexyl}-quinoline-2,4-diamine dihydrochloride (114 mg, 46% ) as a white solid.
ESI MS m/e 459, M (free) + $H^+$ ; $^1H$ NMR (300 MHz, $CDCl_3$) δ 1.46-2.09 (m, 9 H), 2.84 (brs, 3 H), 2.92 (brs, 2 H), 3.60-3.82 (m, 1 H), 4.32 (s, 2 H), 7.05-7.49 (m, 6 H), 7.88 (d, *J* = 7.8 Hz, 1 H), 8.11-8.35 (m, 2 H), 9.91 (brs, 2 H), 11.83 (s, 1 H).

**Example 5**

***N*²-[*cis*-4-(4-Bromo-2-trifluoromethoxy-benzyl)-amino-cyclohexyl]-*N*⁴,*N*⁴-dimethyl-quinoline-2,4-diamine dihydrochloride**

**Step A: Synthesis of (2-chloro-quinolin-4-yl)-dimethyl-amine.**

To a solution of 2,4-dichloro-quinoline (177 g, 894 mmol) in THF (2.1 L) was added 50% aqueous $Me_2NH$ (234 mL, 2-23 mol). The mixture was stirred at ambient temperature for 68 hr. To the mixture was added 50% aqueous $Me_2NH$ (47 mL, 448 mmol) and stirred at ambient temperature for 3 hr. The solution was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 1 % to 3% EtOAc in hexane) to give (2-chloro-quinolin-4-yl)-dimethyl-amine (75.9 g, 41 %) as a pale yellow oil and (4-chloro-quinolin-2-yl)-dimethyl-amine (28.0 g, 15%) as a pale yellow oil.
(2-chloro-quinolin-4-yl)-dimethyl-amine;
ESI MS m/e 207, M + $H^+$; $^1H$ NMR (300 MHz, $CDCl_3$) δ 3.06 (s, 6 H), 6.71 (s, 1 H), 7.45 (ddd, *J* = 8.4, 7.0, 1.2 Hz, 1 H), 7.63 (ddd, *J* = 8.4, 6.9, 1.5 Hz, 1 H), 7.91-7.93 (m, 1 H), 7.97-8.03 (m, 1 H). (4-chloro-quinolin-2-yl)-dimethyl-amine; ESI MS m/e 229, M + $Na^+$; $^1H$ NMR (300 MHz, $CDCl_3$) δ 3.18 (s, 6 H), 6.97 (brs, 1 H), 7.18-7.31 (m, 1 H), 7.49-7.63 (m, 1 H), 7.66-7.72 (m, 1 H), 7.95-8.00 (m, 1 H).

**Step B: Synthesis of *N*²-(*cis*-4-amino-cyclohexyl)-*N*⁴, *N*⁴-dimethyl-quinoline-2,4-diamine.**

Using the procedure for the step E of example 1, the title compound was obtained. FAB MS m/e 285, M + $H^+$ ; $^1H$ NMR (200 MHz, $CDCl_3$) δ 1.12-2.00 (m, 9 H), 2.81-2.98 (m, 1 H), 2.93 (s, 6 H), 4.09 (brs, 1 H), 4.75 (d, J = 7.9 Hz, 1 H), 6.03 (s, 1 H), 7.14 (ddd, J = 8.2, 6.7, 1.3 Hz, 1 H), 7.45 (ddd, *J* = 8.4, 6.8, 1.5Hz, 1 H), 7.62 (m, 1 H), 7.84 (dd, *J* = 8.4, 1.3 Hz, 1 H).

**Step C: Synthesis of *N*²-[*cis*-4-(4-bromo-2-trifluoromethoxy-benzyl)-amino-cyclohexyl]-*N*⁴,*N*⁴-dimethyl-quinoline-2,4-diamine dihydrochloride.**

Using the procedure for the step G of example 1, the title compound was obtained. ESI MS m/e 537, M (free) + $H^+$; $^1H$ NMR (300 MHz, $CDCl_3$) δ 1.73-2.36 (m, 10 H), 3.05-3.31 (m, 2 H), 3.20 (s, 6 H), 4.32 (s, 2 H), 7.30-7.62 (m, 5 H), 7.86 (d, *J* = 8.6 Hz, 1 H), 8.21 (d, *J* = 8.4 Hz, 1 H), 8.53-8.64 (m, 1 H), 13.04 (brs, 1 H).

**Example 6**

***N*²-{*cis*-4-[2-(4-Bromo-2-trifluoromethoxy-phenyl)-ethylamino]-cyclohexyl}-*N*⁴,*N*⁴-dimethylquinoline-2,4-diamine dihydrochloride**

**Step A: Synthesis of *N*²-{*cis*-4-[2-(4-bromo-2-trifluoromethoxy-phenyl)-ethylamino]-cyclohexyl}-*N⁴*,*N⁴*-dimethyl-quinoline-2,4-diamine dihydrochloride.**

Using the procedure for the step G of example 1, the title compound was obtained. ESI MS m/e 551, M (free) + $H^+$ ;$^1H$ NMR (300 MHz, $CDCl_3$) δ 1.69-2.40 (m, 10 H), 3.11-3.46 (m, 10 H), 7.26-7.49 (m, 5 H), 7.59 (t, J = 7.3 Hz, 1 H), 7.86 (d, J = 7.5 Hz, 1 H), 8.53-8.70 (m, 1 H), 9.75-10.14 (m, 2 H), 13.05 (brs, 1 H).

**Example 7**

***N*²-{*cis*-4-[(4-Bromo-2-trifluoromethoxy-benzyl)amino-methyl]-cyclohexyl}-*N*⁴,*N*⁴-dimethylquinoline-2,4-diamine dihydrochloride**

**Step A: Synthesis of [cis-4-(4-dimethylamino-quinolin-2-ylamino)-cyclohexylmethyl]-carbamic acid benzyl ester.**

A mixture of (2-chloro-quinolin-4-yl)-dimethyl-amine obtained in step A of example 5 (23.6 g, 114 mmol) and (*cis*-4-amino-cyclohexylmethyl)-carbamic acid benzyl ester obtained in step C of example 3 (36.0 g, 137 mmol) in butanol (31 mL) was stirred at reflux for 14 days. The reaction mixture was poured into saturated aqueous $NaHCO_3$, and the aqueous layer was extracted with $CHCl_3$ (three times). The combined organic layer was dried over $MgSO_4$,

filtrated, concentrated, and purified by flash chromatography (NH-silica, 14% to 66% EtOAc in hexane) to give [*cis*-4-(4-dimethylaminoquinolin-2-ylamino)-cyclohexylmethyl]-carbamic acid benzyl ester (19.3 g, 39%) as a pale yellow solid.
ESI MS m/e 433, M + H$^+$; $^1$H NMR (200 MHz, $CDCl_3$) δ 1.12-1.97 (m, 9 H), 2.94 (s, 6 H), 3.13 (t, *J* = 6.4 Hz, 2 H), 4.06-4.26 (m, 1 H), 4.62-4.94 (m, 2 H), 5.11 (s, 2 H), 6.04 (s, 1 H), 7.14 (ddd, *J* = 8.4, 7.0, 1.3 Hz, 1 H), 7.29-7.40 (m, 5 H), 7.45 (ddd, *J* = 8.4, 6.8, 1.5 Hz, 1 H), 7.57-7.64 (m, 1 H), 7.84 (dd, *J*= 8.4, 1.3 Hz, 1 H).

**Step B: Synthesis of *N*$^2$-(*cis*-4-aminomethyl-cyclohexyl)-*N*$^4$,*N*$^4$-dimethyl-quinoline-2,4-diamine.**

To a solution of [*cis*-4-(4-dimethylamino-quinolin-2-ylamino)-cyclohexylmethyl]-carbamic acid benzyl ester (19.3 g, 44.6 mmol) in MeOH (200 mL) was added 5% Pd/C (1.93 g). The mixture was stirred at ambient temperature under hydrogen atmosphere for 6 days. The reaction mixture was filtrated through a pad of celite and concentrated. To a solution of the residue in methanol (200 mL) was added 10% Pd/C (1.93 g). The mixture was stirred at ambient temperature under hydrogen atmosphere for 1 day. The reaction mixture was filtrated through a pad of celite, concentrated, and purified by flash chromatography (silica gel, 5% to 14% 7 M $NH_3$/MeOH in $CHCl_3$) to give *N*$^2$-(*cis*-4-aminomethyl-cyclohexyl)-*N*$^4$,*N*$^4$-dimethyl-quinoline-2,4-diamine (12.7 g, 95%) as a pale yellow solid.
FAB MS m/e 299, M + H$^+$; $^1$H NMR (200 MHz, $CDCl_3$) δ 1.08-1.99 (m, 11 H), 2.60 (d, *J*= 6.2 Hz, 2 H), 2.94 (s, 6 H), 4.04-4.22 (m, 1 H), 4.77-4.93 (m, 1 H), 6.06 (s, 1 H), 7.14 (ddd, *J*= 8.4, 7.0, 1.3 Hz, 1 H), 7.45 (ddd, *J*= 8.4, 6.8, 1.5 Hz, 1 H), 7.61 (m, 1 H), 7.84 (dd, *J*= 8.4, 1.3 Hz, 1 H).

**Step C: Synthesis of *N*$^2$-{*cis*-4-[(4-bromo-2-trifluoromethoxy-benzyl)-amino-methyl]-cyclohexyl}-*N*$^4$,*N*$^4$-dimethyl-quinoline-2,4-diamine dihydrochloride.**

Using the procedure for the step G of example 1, the title compound was obtained. ESI MS m/e 551, M (free) +H$^+$; $^1$HNMR (300 MHz, $CDCl_3$) δ 1.50-2.20 (m, 9 H), 2.89 (s, 2 H), 3.20 (s, 6 H), 3.75-4.02 (m, 1 H), 4.23 (s, 2 H), 7.22-7.32 (m, 2 H), 7.40-7.46 (m, 1 H), 7.49-7.62 (m, 2 H), 7.83 (d, *J*= 8.7 Hz, 1 H), 8.17 (d, *J*= 8.4 Hz, 1 H), 8.53-8.69 (m, 1 H), 10.05 (brs, 2 H), 13.00 (brs, 1 H).

**Example 8**

***N*$^4$,*N*$^4$-Dimethyl-*N*$^2$-{*cis*-4-[(2-trifluoromethoxy-benzyl)-amino-methyl]-cyclohexyl}-quinoline-2,4-diamine dihydrochloride**

**Step A: Synthesis of *N*$^4$,*N*$^4$-dimethyl-*N*$^2$-{*cis*-4-[(2-trifluoromethoxy-benzyl)-amino-methyl]-cyclohexyl}-quinoline-2,4-diamine dihydrochloride.**

Using the procedure for the step G of example 1, the title compound was obtained. ESI MS m/e 473, M (free) + H$^+$; $^1$H NMR (300 MHz, $CDCl_3$) δ 1.54-2.20 (m, 9 H), 2.87 (brs, 2 H), 3.19 (s, 6 H), 3.70-4.03 (m, 1 H), 4.28 (brs, 2 H), 7.15-7.67 (m, 6 H), 7.81 (d, *J* = 8.4 Hz, 1 H), 8.17 (d, *J*= 7.3 Hz, 1 H), 8.63 (brs, 1 H), 9.92 (brs, 1 H), 13.13 (s, 1 H).

**Example 9**

***N*$^2$-[*cis*-4-(4-Bromo-2-trifluoromethoxy-benzyl)amino-cyclohexyl]-*N*$^4$-methyl-5,6,7,8-tetrahydro-quinazoline-2,4-diamine dihydrochloride**

**Step A: Synthesis of 5,6,7,8-tetrahydro-quinazoline-2,4-diol.**

To a solution of 2-oxo-cyclohexanecarboxylic acid ethyl ester (61.5 g, 361 mmol) in EtOH (61.5 mL) was added urea (73.8 g, 1.23 mol). The mixture was stirred at reflux for 10.5 days and stirred at ambient temperature for 30 min. The precipitate was filtrated, washed with acetone, and dried. A suspension ofthe above solid in $H_2O$ (100 mL) stirred on an ice-bath for 1 hr. The precipitate was filtrated, washed with hexane, and dried under reduced pressure to give 5,6,7,8-tetrahydro-quinazoline-2,4-diol (21.0 g, 35%) as a pale yellow solid.
CI MS m/e 167, M + H$^+$ ; $^1$H NMR (300 MHz, DMSO-$d_6$) δ 1.48-1.71 (m, 4 H), 2.09-2.19 (m, 2 H), 2.24-2.34 (m, 2 H), 10.41-10.98 (m, 2 H).

**Step B: Synthesis of 2,4-dichloro-5,6,7,8-tetrahydro-quinazoline.**

Using the procedure for the step A of example 1, the title compound was obtained. ESI MS m/e 203, M$^+$; $^1$H

NMR (300 MHz, $CDCl_3$) δ 1.83-1.94 (m, 4 H), 2.67-2.79 (m, 2 H), 2.84-2.95 (m, 2 H).

**Step C: Synthesis of (2-chloro-5,6,7,8-tetrahydro-quinazolin-4-yl)-methyl-amine.**

To a solution of 2,4-dichloro-5,6,7,8-tetrahydro-quinazolin (8.70 g, 42.8 mmol) in THF (87 mL) was added 40% aqueous $MeNH_2$ (8.32 g, 107 mmol). The mixture was stirred at ambient temperature for 8 hr. The solution was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 50% EtOAc in hexane) to give (2-chloro-5,6,7,8-tetrahydro-quinazolin-4-yl)-methyl-amine (7.04 g, 83%) as a white solid.
ESI MS m/e 220, M + $Na^+$ ; $^1H$ NMR (300 MHz, $CDCl_3$) δ 1.74-1.92 (m, 4 H), 2.26 (t, *J*= 5.5 Hz, 2 H), 2.67 (t, *J*= 5.6 Hz, 2 H), 3.05 (d, *J*= 5.0 Hz, 3 H), 4.81 (s, 1 H).

**Step D: Synthesis of *N*$^2$-(*cis*-4-amino-cyclohexyl)-*N*$^4$-methyl-5,6,7,8-tetrahydro-quinazoline-2,4-diamine.**

Using the procedure for the step E of example 1, the title compound was obtained.
ESI MS m/e 276, M + $H^+$ ; $^1H$ NMR (300 MHz, DMSO-$d_6$) δ 1.33-1.76 (m, 12 H), 2.11-2.21 (m, 2 H), 2.31-2.40 (m, 2 H), 2.70-2.77 (m, 2 H), 2.78 (d, *J*= 4.5 Hz, 3 H), 3.71-3.83 (m, 1 H), 5.50-5.63 (m, 1 H), 6.10-6.22 (m, 1 H).

**Step E: Synthesis of *N*$^2$-[*cis*-4-(4-bromo-2-trifluoromethoxy-benzyl)-amino-cyclohexyl]-*N*$^4$-methyl-5,6,7,8-tetrahydro-quinazoline-2,4-diamine dihydrochloride.**

Using the procedure for the step G of example 1, the title compound was obtained.
ESI MS m/e 528, M (free) + $H^+$ ; $^1H$ NMR (300 MHz, $CDCl_3$) δ 1.66-2.24 (m, 12 H), 2.41-2.56 (m, 4 H), 3.00 (d, *J*= 4.5 Hz, 3 H), 3.04 (brs, 1 H), 4.03 (brs, 1 H), 4.30 (brs, 2 H), 7.45-7.48 (m, 1 H), 7.52 (dd, *J* = 8.3, 1.8 Hz, 1 H), 7.61 (d, *J* = 5.8 Hz, 1 H), 7.74 (brs, 1 H), 8.14 (d, *J* = 8.2 Hz, 1 H), 11.84 (brs, 1 H).

**Example 10**

***N*$^2$-{*cis*-4-[2-(4-Bromo-2-trifluoromethoxy-phenyl)-ethylamino]-cyclohexyil}-*N*$^4$-methyl-5,6,7,8-tetrahydro-quinazoline-2,4-diamine dihydrochloride**

**Step A: Synthesis of *N*$^2$-{*cis*-4-[2-(4-bromo-2-triftuoromethoxy-phenyl)-ethylamino]-cyclohexyl}-*N*$^4$-methyl-5,6,7,8-tetrahydro-quinazoline-2,4-diamine dihydrochloride.**

Using the procedure for the step G of example 1, the title compound was obtained.
ESI MS m/e 542, M (free) + $H^+$ ; $^1H$ NMR (300 MHz, $CDCl_3$) δ 1.57-2.25 (m, 12 H), 2.35-2.60 (m, 4 H), 2.94-3.28 (m, 6 H), 3.32-3.45 (m, 2 H), 4.13 (brs, 1 H), 7.30-7.51 (m, 4 H), 7.72 (d, *J*= 6.2 Hz, 1 H), 9.86 (brs, 2 H) 11.90 (s, 1 H).

**Example 11**

***N*$^2$-{*cis*-4-[(4-Bromo-2-trifluoromethoxy-benzyl)amino-methyl]-cyetohexyl}-*N*$^4$-methyl-5,6,7,8-tetrahydro-quinazoline-2,4-diamine dihydrochloride**

**Step A: Synthesis of [*cis*-4-(4-methylamino-5,6,7,8-tetrahydro-quinazolin-2-ylamino) cyclohexylmethyl]-carbamic acid benzyl ester.**

A mixture of (2-chloro-5,6,7,8-tetrahydro-quinazolin-4-yl)-methyl-amine obtained in step C of example 9 (2.00 g, 10.1 mmol) and (cis-4-amino-cyclohexylmethyl)-carbamic acid benzyl ester obtained in step C of example 3 (3.19 g, 12.2 mmol) in butanol (3 mL) was stirred at 130 °C for 16 hr in a sealed tube. The reaction mixture was poured into saturated aqueous $NaHCO_3$, and the aqueous layer was extracted with $CHCl_3$ (three times). The combined organic layer was dried over $MgSO_4$, filtrated, concentrated, and purified by flash chromatography (silica gel, 10% MeOH in $CHCl_3$) to give [*cis*-4-(4-methylamino-5,6,7,8-tetrahydro-quinazolin-2-ylamino)-cyclohexylmethyl]-carbamic acid benzyl ester (1.38 g, 32%) as a pale yellow oil.
ESI MS m/e 424, M + $H^+$ ; $^1H$ NMR (300 MHz, $CDCl_3$) δ 1.31-2.02 (m, 13 H), 2.22-2.34 (m, 2 H), 2.52-2.64 (m, 2 H), 3.05 (d, *J* = 4.8 Hz, 3 H), 3.11 (t, *J* = 6.1 Hz, 2 H), 5.05-5.23 (m, 1 H), 5.08 (s, 2 H), 6.34-6.47 (m, 1 H), 7.23-7.42 (m, 5 H), 7.99 (d, *J* = 7.3 Hz, 1 H), 12.34 (brs, 1 H).

**Step B: Synthesis of *N*²-{*cis*-4-[(4-bromo-2-trifluoromethoxy-benzyl)-amino-methyl]-cyclohexyl}-*N*⁴-methyl-5,6,7,8-tetrahydro-quinazoline-2,4-diamine dihydrochloride.**

Using the procedure for the step E of example 3, the title compound was obtained. ESI MS m/e 542, M (free) + $H^+$ ; $^1H$ NMR (200 MHz, $CDCl_3$) δ 1.50-2.19 (m, 13 H), 2.58-2.61 (m, 2 H), 2.72-2.91 (m, 2 H), 2.83-2.97 (m, 2 H), 3.24 (s, 6 H), 4.15-4.20 (m, 1 H), 4.22-4.38 (m, 2 H), 7.43-7.50 (m, 1 H), 7.56-7.61 (m, 1H), 8.18-8.29 (m, 2 H), 10.06 (brs, 2 H), 12.30 (brs, 1 H).

**Example 12**

**$N^4$-Methyl-$N^2$-{*cis*-4-[(2-trifluoromethoxy-benzyl)-amino-methyl]-cyclohexyl}-5,6,7,8-tetrahydro-quinazoline-2,4-diamine dihydrochloride**

**Step A: Synthesis of $N^4$-methyl-$N^2$-{*cis*-4-[(2-trifluoromethoxy-benzyn-amino-methyl]-cyclohexyl)-5,6,7,8-tetrahydro-quinazoline-2,4-diamine dihydrochloride.**

Using the procedure for the step G of example 1, the title compound was obtained.
ESI MS m/e 464, M (free) + $H^+$ ; $^1H$ NMR (300 MHz, DMSO-$d_6$) δ 1.28-2.04 (m, 15 H), 2.14-2.30 (m, 2 H), 2.83-2.95 (m, 2 H), 2.91 (d, *J* = 4.5 Hz, 3 H), 4.13 (brs, 1 H), 4.22 (brs, 2 H), 7.43-7.62 (m, 3 H), 7.91 (dd, J = 7.5, 1.6 Hz, 1 H), 8.09 (d, *J* = 6.7 Hz, 2 H), 9.37 (brs, 2 H), 12.30-12.70 (m, 1 H).

**Example 13**

**$N^2$-[*cis*-4-(4-Bromo-2-trifluoromethoxy-benzyl)-amino-cyclohexyl]-$N^4$,$N^4$-dimethyl-5,6,7,8-tetrahydro-quinazoline-2,4-diamine dihydrochloride**

**Step A: Synthesis of (2-chloro-5,6,7,8-tetrahydro-quinazolin-4-yl)-dimethyl-amine.**

To a solution of 2,4-dichloro-5,6,7,8-tetrahydro-quinazolin (7.00 g, 34.5 mmol) in THF (70 mL) was added 50% aqueous $MeNH_2$ (7.77 g, 86.2 mmol). The mixture was stirred at ambient temperature for 2.25 hr. The solution was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 20% EtOAc in hexane) to give (2-chloro-5,6,7,8-tetrahydro-quinazolin-4-yl)-dimethyl-amine (6.08 g, 83%) as a white solid.
ESI MS m/e 234, M + $Na^+$; $^1H$ NMR (300 MHz, $CDCl_3$) δ 1.62-1.90 (m, 4 H), 2.59 (t, *J*= 6.0 Hz, 2 H), 2.76 (t, *J*= 6.6 Hz, 2 H), 3.06 (s, 6 H).

**Step B: Synthesis of $N^2$-(*cis*-4-amino-cyclohexyl)-$N^4$,$N^4$-dimethyl-5,6,7,8-tetrahydro-quinazoline-2,4-diamine.**

Using the procedure for the step E of example 1, the title compound was obtained.
FAB MS m/e 290, M + $H^+$ ; $^1H$ NMR (200 MHz, $CDCl_3$) δ 0.95-1.94 (m, 14 H), 2.49 (t, *J*= 5.9 Hz, 2 H), 2.61 (t, *J*= 7.0 Hz, 2 H), 2.72-2.94 (m, 1 H), 2.94 (s, 6 H), 3.89-4.11 (m, 1 H), 4.73 (d, *J*= 7.5 Hz, 1 H).

**Step C: Synthesis of $N^2$-[*cis*-4-(4-bromo-2-tritluoromethoxy-benzyl)-amino-cyclohexyl]-$N^4$,$N^4$-dimethyl-5,6,7,8-tetrahydro-quinazoline-2,4-diamine-dihydrochloride.**

Using the procedure for the step G of example 1, the title compound was obtained.
ESI MS m/e 542, M (free) + $H^+$ ; $^1H$ NMR (300 MHz, $CDCl_3$) δ 1.57-2.32 (m, 12 H), 2.52-2.60 (m, 2 H), 2.63-2.72 (m, 2 H), 3.11-3.24 (m, 7 H), 4.12-4.23 (m, 1 H), 4.28 (s, 2 H), 7.41 (d, J= 10.4 Hz, 1 H), 7.49 (dd, *J*=8.2, 1.9 Hz, 1 H), 8.19 (d, *J*=8.4 Hz, 1 H), 8.25 (d, *J*=8.1 Hz, 1 H), 10.02 (brs, 1 H), 12.43 (brs, 1 H).

**Example 14**

***N*²-{*cis*-4-[2-(4-Bromo-2-trifluoromethoxy-phenyl)-ethylamino]-cyclohexyl}-*N*⁴,*N*⁴-dimethyl-5, 6,7,8-tetrahydro-quinazoline-2,4-diamine dihydrochloride**

**Step A: Synthesis of *N*²-{*cis*-4-[2-(4-bromo-2-trifluoromethoxy-phenyl)-ethylamino]-cyclohexyl}-*N*⁴,*N*⁴-dimethyl-5,6,7,8-tetrahydro-quinazoline-2,4-diamine-dihydrochloride.**

Using the procedure for the step G of example 1, the title compound was obtained.
ESI MS m/e 556, M (free) + $H^+$; $^1H$ NMR (300 MHz, $CDCl_3$) δ 1.57-2.32 (m, 12 H), 2.56 (t, *J*= 5.8 Hz, 2 H), 2.69 (t, *J*= 6.2 Hz, 2 H), 3.14-3.41 (m, 9 H), 4.13-4.25 (m, 1 H), 7.35-7.44 (m, 2 H), 7.49-7.55. (m, 1 H), 8.20 (d, *J*= 7.8 Hz, 1 H).

**Example 15**

***N*²-{*cis*-4-[(4-Bromo-2-trifluoromethoxy-benzyl)-amino-methyl]-cyclohexyl}-*N*⁴,*N*⁴-dimethyl-5,6,7,8-tetrahydro-quinazoline-2,4-diamine dihydrochloride**

**Step A: Synthesis of [*cis*-4-(4-dimethylamino-quinolin-2-ylamino)-cyclohexylmethyl]-carbamic acid benzyl ester.**

Using the procedure for the step A of example 11, the title compound was obtained.
ESI MS m/e 438, M + $H^+$ ; $^1H$ NMR (300 MHz, $CDCl_3$) δ 1.18-1.39 (m, 2 H), 1.48-1.94 (m, 11 H), 2.49 (t, *J* = 5.9 Hz, 2 H), 2.60 (t, *J* = 6.6 Hz, 2 H), 2.94 (s, 6 H), 3.09 (t, *J* = 6.1 Hz, 2 H), 4.01-4.13 (m, 1 H), 4.70-4.91 (m, 2 H), 5.09 (s, 2 H), 7.27-7.39 (m, 5 H).

**Step B: Synthesis of *N*²-{*cis*-4-[(4-bromo-2-trifluoromethoxy-benzyl)-amino-methyl]-cyclohexyl}-*N*⁴,*N*⁴-dimethyl-5,6,7,8-tetrahydro-quinazoline-2,4-diamine dihydrochloride.**

Using the procedure for the step E of example 3, the title compound was obtained.
ESI MS m/e 556, M (free) + $H^+$ ; $^1H$ NMR (300 MHz, $CDCl_3$) δ 1.46-2.17 (m, 12 H), 2.55 (t, *J*= 5.8 Hz, 2 H), 2.69 (t, *J*= 6.1 Hz, 2 H), 2.79-2.92 (m, 2 H), 3.20 (s, 6 H), 4.08-4.18 (m, 1 H), 4.20-4.31 (m, 2 H), 7.43-7.47 (m, 1 H), 7.53 (dd, *J*= 8.4, 1.9 Hz, 1 H), 8.16 (d, *J*= 7.8 Hz, 1 H), 8.22 (d, *J*= 8.4 Hz, 1 H), 10.02 (brs, 2 H), 12.28 (brs, 1 H).

**Example 16**

***N*⁴,*N*⁴-Dimethyl-*N*²-{*cis*-4-[(2-trifluoromethoxy-benzyl)-amino-methyl]-cyclohexyl)-5,6,7,8-tetrahydro-quinazoline-2,4-diamine dihydrochloride**

**Step A: Synthesis of *N*⁴,*N*⁴-dimethyl-*N*²-{*cis*-4-[(2-trifluoromethoxy-benzyl)-amino-methyl]-cyclohexyl}-5,6,7,8-tetrahydro-quinazoline-2,4-diamine dihydrochloride.**

Using the procedure for the step G of example 1, the title compound was obtained.
ESI MS m/e 478, M (free) + $H^+$ ; $^1H$ NMR (300 MHz, $CDCl_3$) δ 1.48-2.15 (m, 13 H), 2.55 (t, *J* = 5.4 Hz, 2 H), 2.71 (t, *J* = 6.2 Hz, 2 H), 2.77-2.89 (m, 2 H), 3.19 (s, 6 H), 4.10 (brs, 1 H), 4.26-4.37 (m, 2 H), 7.27-7.34 (m, 1 H), 7.36-7.47 (m, 2 H), 8.15-8.25 (m, 2 H), 9.90 (s, 2 H), 12.52 (s, 1 H).

**Example 17**

***N*²-[cis-4-(4-Bromo-2-trifluoromethoxy-benzyl)-amino-cyclohexyl]-*N*⁴,*N*⁴-dimethyl-pyrimidin-2,4-diamine dihydrochloride**

**Step A: Synthesis of [cis-4-(4-bromo-2-trifluoromethoxy-benzyl)-amino-cyclohexyl]-carbamic acid *tert-butyl* ester.**

To a solution of (*cis*-4-amino-cyclohexyl)-carbamic acid tert-butyl ester obtained in step D of example 1 (6.72 g, 31.4 mmol) in $CHCl_3$ (67 mL) were added 4-bromo-2-trifluoromethoxybenzaldehyde obtained in step F of example 1 (8.44 g, 31.4 mmol), acetic acid (1.88 g, 31.3 mmol), and $NaBH(OAc)_3$ (9.97 g, 47.0 mmol). The reaction mixture was stirred at ambient temperature for 4 hr. The reaction was quenched with saturated aqueous $NaHCO_3$ and the

aqueous layer was extracted with $CHCl_3$ (three times). The combined organic layer was dried over $MgSO_4$, filtrated, concentrated, and purified by flash chromatography (silica gel, 33% EtOAc in hexane) to give [*cis*-4-(4-bromo-2-trifluoromethoxy-benzyl)-amino-cyclohexyl)-carbamic acid tert-butyl ester (10.28 g, 70%) as a pale yellow oil.
ESI MS m/e 467, M + $H^+$ ; $^1H$ NMR (300 MHz, $CDCl_3$) δ 1.16-1.78 (m, 17 H), 2.57-2.70 (m, 1 H), 3.62 (brs, 1 H), 3.78 (s, 2 H), 4.60 (brs, 1 H), 7.34-7.54 (m, 3 H).

**Step B: Synthesis of (2-chloro-pyrimidin-4-yl)-dimethyl-amine.**

To a solution of 2,4-dichloro-pyrimidine (15.0 g, 10.15 mmol) in THF (150 mL) was added 50% aqueous $MeNH_2$ (22.7 g, 25.2 mmol). The mixture was stirred at ambient temperature for 2 hr. The solution was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 20% EtOAc in hexane) to give (2-chloro-pyrimidin-4-yl)-dimethyl-amine (8.66 g, 55 %) as a white solid and (4-chloro-pyrimidin-2-yl)-dimethyl-amine (0.87 g, 6%) as a white solid.
(2-chloro-pyrimidin-4-yl)-dimethyl-amine;
CI MS m/e 158, M + $H^+$; $^1H$ NMR (300 MHz, $CDCl_3$) δ 3.12 (s, 6 H), 6.32 (d, *J*= 6.1 Hz, 1 H), 8.00 (d, *J*= 6.1 Hz, 1 H).
(4-chloro-pyrimidin-2-yl)-dimethyl-amine;
ESI MS m/e 157, $M^+$; $^1H$ NMR (300 MHz, $CDCl_3$) δ 3.21 (s, 6 H), 6.50 (d, *J*= 5.1 Hz, 1 H), 8.18 (d, *J*= 5.1 Hz, 1 H).

**Step C: Synthesis of *N*²-[cis-4-(4-bromo-2-trifluoromethoxy-benzyl)-amino-cyclohexyl]-*N*⁴,*N*⁴,-dimethyl-pyrimidine-2,4-diamine dihydrochloride.**

To a solution of [*cis*-4-(4-bromo-2-trifluoromethoxy-benzylamino)-cyclohexyl]-carbamic acid *tert-butyl* ester (3.00 g, 6.42 mmol) in EtOAc (30 mL) was added 4 M hydrogen chloride in EtOAc (60 mL). The reaction mixture was stirred at ambient temperature for 1 hr and concentrated. The residue was alkalized with saturated aqueous $NaHCO_3$. The aqueous layer was extracted with $CHCl_3$ (three times). The combined organic layer was dried over $MgSO_4$, filtrated, and concentrated. The above material (466 mg, 1.27 mmol) and (2-chloro-pyrimidin-4-yl)-dimethyl-amine (200 mg, 1.27 mmol) in butanol (1 mL) was stirred at 130 °C for 13.5 hr in a sealed tube. The reaction mixture was poured into saturated aqueous $NaHCO_3$ and the aqueous layer was extracted with $CHCl_3$ (three times). The combined organic layer was dried over $MgSO_4$, filtrated, concentrated, and purified by medium-pressure liquid chromatography (NH-silica, 20% EtOAc in hexane) to give a colorless oil. To a solution of the above oil in EtOAc (2 mL) was added 4 M hydrogen chloride in EtOAc (5 mL). The mixture was stirred at ambient temperature for 1 hr and concentrated. A suspension of the residue in $Et_2O$ (12 mL) was stirred at ambient tempareture for 1 hr. The precipitate was collected by filtration, washed with $Et_2O$, and dried under reduced pressure to $N^2$-[*cis*-4-(4-bromo-2-trifluoromethoxy-benzyl)-amino-cyclohexyl]-$N^4$,$N^4$-dimethyl-pyrimidine-2,4-diamine dihydrochloride (180 mg, 25%) as a white solid.
ESI MS m/e 488, M (free) + $H^+$; $^1H$ NMR (300 MHz, $CDCl_3$) δ 1.54-1.72 (m, 2 H), 2.01-2.29 (m, 6 H), 3.02 (brs, 1 H), 3.16 (s, 3 H), 3.24 (s, 3 H), 4.13 (brs, 1 H), 4.30 (s, 2 H), 6.02 (d, *J* = 7.5 Hz, 1 H), 7.40-7.43 (m, 1 H), 7.50 (dd, *J*= 8.4, 1.9 Hz, 1 H), 7.99 (d, J= 7.3 Hz, 1 H), 8.26 (d, *J*= 8.4 Hz, 1 H), 8.57 (d, J= 7.0 Hz, 1 H), 10.25 (s, 2 H).

**Example 18**

***N*²-{*cis*-4-[2-(4-Bromo-2-trifluoromethoxy-phenyl)-ethylamino]-cyclohexyl}-*N*⁴,*N*⁴-dimethyl-pyrimidine-2,4-diamine dihydrochloride**

**Step A: Synthesis of [*cis*-4-(4-dimethylamino-pyrimidin-2-ylamino)-cyclohexyl]-carbamic acid *tert*-butyl ester.**

A mixture of (2-chloro-pyrimidin-4-yl)-dimethyl-amine obtained in step B of example 17 (1.50 g, 9.52 mmol) and (*cis*-4-amino-cyclohexyl)-carbamic acid *tert*-butyl ester obtained in step D of example 1 (2.24 g, 10.5 mmol) in IPA (1.5 mL) was stirred at 130 °C for 22 hr in a sealed tube. The reaction mixture was poured into saturated aqueous $NaHCO_3$, and the aqueous layer was extracted with $CHCl_3$ (three times). The combined organic layer was dried over $MgSO_4$, filtrated, concentrated, and purified by medium-pressure liquid chromatography (NH-silica, 10% EtOAc in hexane) to give [*cis*-4-(4-dimethylamino-pyrimidin-2-ylamino)-cyclohexyl]-carbamic acid tert-butyl ester (1.34 g, 42%) as a white solid.
ESI MS m/e 358, M + $Na^+$ ; $^1H$ NMR (300 MHz, $CDCl_3$) δ 1.45 (s, 9 H), 1.48 (s, 8 H), 3.03 (s, 6 H), 3.61 (brs, 1 H), 3.89-4.04 (m, 1 H), 4.47-4.63 (m, 1 H),4.77-4.89 (m, 1 H), 5.80 (d, *J* = 6.1 Hz, 1 H), 7.84 (d, *J* = 6.1 Hz, 1 H).

**Step B: Synthesis of *N*2-(*cis*-4-amino-cyclohexyl)-*N4,N4*-dimethyl-pyrimidine-2,4-diamine.**

To a solution of [*cis*-4-(4-dimethylamino-pyrimidin-2-ylamino)-cyclohexyl]-carbamic acid *tert*-butyl ester (1.26 g, 3.76 mmol) in EtOAc (15 mL) was added 4 M hydrogen chloride in EtOAc (15 mL). The reaction mixture was stirred at ambient temperature for 1 hr and concentrated. The residue was alkalized with 1 M aqueous NaOH. The aqueous layer was extracted with $CHCl_3$ (six times). The combined organic layer was dried over $MgSO_4$, filtrated, and concentrated to give *N*2-(*cis*-4-amino -cyclohexyl)-*N4*,*N4*-dimethyl-pyrimidine-2,4-diamine(923 mg, quant.) as a pale yellow oil. ESI MS m/e 250, M + $H^+$; $^1H$ NMR (300 MHz, $CDCl_3$) δ 1.29-1.51 (m, 2 H), 1.61-1.91 (m, 6 H), 2.80-2.92 (m, 1 H), 3.03 (s, 6 H), 3.96-4.04 (m, 1 H), 4.85-4.98 (m, 1 H), 5.79 (d, *J* = 6.1 Hz, 1 H), 7.84 (d, *J* = 6.1 Hz, 1 H).

**Step C: Synthesis of *N*2-{*cis*-4-[2-(4-bromo-2-trifluoromethoxy-phenyl)-ethylamino]-cyclohexyl}-*N*4,*N*4-dimethyl-pyrimidine-2,4-diamine dihydrochloride.**

Using the procedure for the step G of example 1, the title compound was obtained.
ESI MS m/e 502, M (free) + $H^+$ ; $^1H$ NMR (300 MHz, $CDCl_3$) δ 1.62-1.82 (m, 2 H), 1.97-2.44 (m, 6 H), 3.16 (s, 3 H), 3.14-3.31 (m, 1 H), 3.25 (s, 3 H), 3.34-3.46 (m, 2 H), 4.18 (brs, 1 H), 6.02 (d, *J* = 6.8 Hz, 1 H), 7.34-7.43 (m, 2 H), 7.45-7.52 (m, 1 H), 7.85-7.97 (m, 1 H), 8.49-8.59 (m, 1 H), 9.95 (brs, 2 H), 12.42 (brs, 1H).

**Example 19**

***N*2-{*cis*-4-[(4-Bromo-2-trifluoromethoxy-benzyl)-amino-methyl]-cyclohexyl}-*N*4,*N*4-dimethyl-pyrimidine-2,4-diamine dihydrochloride**

**Step A: Synthesis of [*cis*-4-(4-dimethylamino-pyrimidin-2-ylamino)-cyclohexylmethyl]-carbamic acid benzyl ester.**

A mixture of (2-chloro-pyrimidin-4-yl)-dimethyl-amine obtained in step B of example 17 (1.50 g, 9.52 mmol) and *cis*-(4-amino-cyclohexylmethyl)-carbamic acid benzyl ester (2.75 g, 10.5 mmol) in IPA (1.5 mL) was stirred at 130 °C for 22 hr in a sealed tube. The reaction mixture was poured into saturated aqueous $NaHCO_3$, and the aqueous layer was extracted with $CHCl_3$ (three times). The combined organic layer was dried over $MgSO_4$, filtrated, concentrated, and purified by medium-pressure liquid chromatography (NH-silica, 10% EtOAc in hexane to EtOAc) to give [*cis*-4-(4-dimethylaniino-pyrimidin-2-ylamino)-cyclohexylmethyl]-carbamic acid benzyl ester (816 mg, 22%) as a pale yellow oil.
ESI MS m/e 406, M + $Na^+$ ; $^1H$ NMR (300 MHz, $CDCl_3$) δ 1.22-1.92 (m, 9 H), 3.03 (s, 6 H), 3.11 (t, *J* = 6.2 Hz, 2 H), 4.02-4.15 (m, 1 H), 4.82-4.93 (m, 2 H), 5.10 (s, 2 H), 5.79 (d, *J* = 6.1 Hz, 1 H), 7.28-7.42 (m, 5 H), 7.83 (d, *J* = 6.1 Hz, 1 H).

**Step B: Synthesis of *N*2-(*cis*-4-aminomethyl-cyclohexyl)-*N*4,*N*4-dimethyl-pyrimidine-2,4-diamine.**

Using the procedure for the step B of example 7, the title compound was obtained.
ESI MS m/e 250, M + $H^+$ ; $^1H$ NMR (300 MHz, $CDCl_3$) δ 1.40-1.88 (m, 9 H), 2.87 (d, *J* = 5.9 Hz, 2 H), 3.03 (s, 6 H), 4.11 (brs, 1 H), 5.63 (brs, 1 H), 5.78 (d, *J* = 6.2 Hz, 1 H), 7.08 (brs, 2 H), 7.82 (d, *J* = 6.2 Hz, 1 H).

**Step C: Synthesis of *N*2-{*cis*-4-[(4-bromo-2-trifluoromethoxy-benzyl)-amino-methyl]-cyclohexyl}-*N*4,*N*4-dimethyl-pyrimidine-2,4-diamine dihydrochloride.**

Using the procedure for the step G of example 1, the title compound was obtained.
ESI MS m/e 502, M (free) + $H^+$; $^1H$ NMR (300 MHz, $CDCl_3$) δ 1.52-2. (m, 9 H), 2.85 (d, *J*= 5.8 Hz, 2 H), 3.16 (s, 3 H), 3.24 (s, 3 H), 4.15-4.30 (m, 3 H), 6.00 (d, *J*= 7.6 Hz, 1 H), 7.43-7.47 (m, 1 H), 7.53 (dd, *J*= 8.3, 1.9 Hz, 1 H), 7.66 (d, *J*= 7.5 Hz, 1 H), 8.20 (d, *J*= 8.4 Hz, 1 H), 8.53 (d, *J*= 7.5 Hz, 1 H), 10.07 (brs, 2 H).

**Example 20-672**

To a solution of poly(4-vinylpyridine) (75 μL) in $CH_2Cl_2$ (200 μL) were added the amines (30 μmol) as shown below in $CH_2Cl_2$ (200 μL) and acid chloride (60 μmol) in $CH_2Cl_2$ (200 μL) at ambient temperature. After stirring at the same temperature for 19 hr, the reaction mixture was filtrated and concentrated by a stream of dry $N_2$. To the residue were added dry $CH_2Cl_2$ (700 μL) and PSA (300 μL). After the stirring at ambient temperature for 14 hr, the reaction mixture was purified by silica gel chromatography (NH-silica, 50% EtOAc in hexane to EtOAc only) to give the desired product. The product was determined by ESI-MS or APCI-MS.

Wherein the amines are selected from $N^2$-(*cis*-4-amino-cyclohexyl)-$N^4$,$N^4$-dimethyl-quinoline-2,4-diamine obtained in step B of example 5, $N^2$-(*cis*-4-aminomethyl-cyclohexyl)-$N^4$,$N^4$-dimethyl-quinoline-2,4-diamine obtained in step B of example 7, $N^2$-(*cis*-4-amino-cyclohexyl)-$N^4$,$N^4$-dimethyl-5,6,7,8-tetrahydro-quinazoline-2,4-diamine obtained in step B of example 13, $N^2$-(*cis*-4-aminomethyl-cyclohexyl)-$N^4$,$N^4$-dimethyl-5,6,7,8-tetrahydro-quinazoline-2,4-diamine obtained in intermediate of step B of example 15, $N^2$-(*cis*-4-amino-cyclohexyl)-$N^4$,$N^4$-dimethyl-pyrimidine-2,4-diamine obtained in step B of example 18, or $N^2$-(*cis*-4-aminomethyl-cyclohexyl)-$N^4$,$N^4$-dimethyl-pyrimidine-2,4-diamine obtained in step B of example 19.

**Example 673-1084**

To a solution of 1-cyclohexyl-3-methylpolystyrene-carbodiimide (150 μL) in $CH_2Cl_2$ (400 μL) were added the amines (30 μmol) as shown below in $CH_2Cl_2$ (200 μL) and carboxylic acid (60 μmol) in $CH_2Cl_2$ (200 μL) at ambient temperature. After stirring at the same temperature for 20 hr, the reaction mixture was filtrated through NH-silica gel, concentrated by a stream of dry $N_2$, and purified by silica gel chromatography (silica gel, 2% to 7% 2 M $NH_3$/MeOH in $CHCl_3$) to give the desired product. The product was determined by ESI-MS or APCI-MS.

Wherein the amines are selected from $N^2$-(*cis*-4-amino-cyclohexyl)-$N^4$,$N^4$-dimethyl-quinoline-2,4-diamine obtained in step B of example 5, $N^2$-(*cis*-4-aminomethyl-cyclohexyl)-$N^4$,$N^4$-dimethyl-quinoline-2,4-diamine obtained in step B of example 7, $N^2$-(*cis*-4-amino-cyclohexyl)-$N^4$,$N^4$-dimethyl-5,6,7,8-tetrahydro-quinazoline-2,4-diamine obtained in step B of example 13, $N^2$-( *cis*-4-aminomethyl-cyclohexyl)-$N^4$,$N^4$-dimethyl-5,6,7,8-tetrahydro-quinazoline-2,4-diamine obtained in intermediate of step B of example 15, $N^2$-(*cis*-4-amino-cyclohexyl)-$N^4$,$N^4$-dimethyl-pyrimidine-2,4-diamine obtained in step B of example 18, or $N^2$-(*cis*-4-aminomethyl-cyclohexyl)-$N^4$,$N^4$-dimethyl-pyrimidine-2,4-diamine obtained in step B of example 19.

**Example 1085-1446**

**-method A-**

To a solution of the amines (36 μmol) as shown below in MeOH (200 μL) were added aromatic aldehyde (30 μmol) in MeOH (200 μL) and AcOH (90 μmol) at ambient temperature. The reaction mixture was stirred at the same temperature for 1 hr. To the mixture was added $NaBH_3CN$ (120 μmol) in MeOH (200 μL). After stirring at the same temperature for 20 hr, the reaction mixture was concentrated by a stream of dry $N_2$. The residue was partitionated between $CHCl_3$ and 2 M aqueous sodium hydroxide. The aqueous layer was extracted with $CHCl_3$ (500 μL) and EtOAc (300 μL). The combined organic layers were dried over $MgSO_4$, concentrated by a stream of dry $N_2$, and purified by silica gel chromatography (silica gel, 2% to 7% 2 M $NH_3$/MeOH in $CHCl_3$) to give the desired product. The product was determined by ESI-MS or APCI-MS.

**-method B-**

To a solution of the amines (36 μmol) as shown below in MeOH (200 μL) were added aliphatic aldehyde (30 μmol) in MeOH (200 μL), AcOH (90 μmol), and $NaBH_3CN$ (120 μmol) in MeOH (200 μL) at ambient temperature. After stirring at the same temperature for 20 hr, the reaction mixture was concentrated by a stream of dry $N_2$. The residue was partitionated between $CHCl_3$ and 2 M aqueous sodium hydroxide. The aqueous layer was extracted with $CHCl_3$ (500 μL) and EtOAc (300 μL). The combined organic layers were dried over $MgSO_4$, concentrated by a stream of dry $N_2$, and purified by silica gel chromatography (silica gel, 2% to 7% 2 M $NH_3$/MeOH in $CHCl_3$) to give the desired product. The product was determined by ESI-MS or APCI-MS.

Wherein the amines are selected from $N^2$-(*cis*-4-amino-cyclohexyl)-$N^4$,$N^4$-dimethyl-quinoline-2,4-diamine obtained in step B of example 5, $N^2$-(*cis*-4-aminomethyl-cyclohexyl)-$N^4$,$N^4$-dimethyl-quinoline-2,4-diamine obtained in step B of example 7, $N^2$-(*cis*-4-amino-cyclohexyl)-$N^4$,$N^4$-dimethyl-5,6,7,8-tetrahydro-quinazoline-2,4-diamine obtained in step B of example 13,$N^2$-(*cis*-4-aminomethyl-cyclohexyl)-$N^4$,$N^4$-dimethyl-5,6,7,8-tetrahydro-quinazoline-2,4-diamine obtained in intermediate of step B of example 15, $N^2$-(*cis-4*-amino-cyclohexyl)-$N^4$,$N^4$-dimethyl-pyrimidine-2,4-diamine obtained in step B of example 18, or $N^2$-(*cis*-4-aminomethyl-cyclohexyl)-$N^4$,$N^4$-dimethyl-pyrimidine-2,4-diamine obtained in step B of example 19.

**Example 1457-1462,1478-1480,1491-1497, and 1510-1512**

To a solution of the amide product in THF (200 μl) was added 1 M borane-THF complex in THF (300 μl, 300 μmol). The mixture was stirred at 80 °C for 1 hr, and concentrated by a stream of dry $N_2$. To the residue were added 1 M aqueous HCl (300 μl) and THF (200 μl). The mixture was stirred at 80 °C for 1 hr and concentrated by a stream

of dry $N_2$. To the residue was partitionated between $CHCl_3$ and 2 M aqueous sodium hydroxide. The aqueous layer was extracted with $CHCl_3$ (300 μL, twice) and EtOAc (300 μL). The combined organic layers were dried over $MgSO_4$, concentrated by a stream of dry $N_2$, and the purified by silica gel chromatography (silica gel, 2% to 7% 2 M $NH_3$/MeOH in $CHCl_3$) to give the desired product. The product was determined by ESI-MS or APCI-MS.

**Example 1447-1456, 1463-1477, 1481-1490, 1498-1509, and 1513-1538**

To a suspension of Dess-Martin periodinane (63 μmol) in $CH_2Cl_2$ (200 μL) was added alcohol (35 μmol) in $CH_2Cl_2$ (200 μL) at ambient temperature, and the reaction mixture was stirred at the same temperature for 18 hr. To the reaction mixture were added amines (36 μmol) as shown below in MeOH (200 μL) and AcOH (90 μL). The mixture was stirred at the same temperature for 1 hr, and then $NaBH_3CN$ (120 μmol) in MeOH (200 μL) was added. After stirring at the same temperature for 17 hr, the reaction mixture was concentrated by a stream of dry $N_2$. The residue was partitionated between $CHCl_3$ and 2 M aqueous sodium hydroxide. The aqueous layer was extracted with $CHCl_3$ (500 mL) and EtOAc (300 μL). The combined organic layers were dried over $MgSO_4$, concentrated by a stream of dry $N_2$, and purified by silica gel chromatography (silica gel, 2% to 7% 2 M $NH_3$/MeOH in $CHCl_3$) to give the desired product. The product was determined by ESI-MS or APCI-MS.

Wherein the amines are selected from *N*$^2$-(cis-4-amino-cyclohexyl)-*N*$^4$,*N*$^4$-dimethyl-quinoline-2,4-diamine obtained in step B of example 5, *N*$^2$-(cis-4-aminomethyl-cyclohexyl)-*N*$^4$,*N*$^4$-dimethyl-quinoline-2,4-diamine obtained in step B of example 7, *N*$^2$-(cis-4-amino-cyclohexyl)-*N*$^4$,*N*$^4$-dimethyl-5,6,7,8-tetrahydro-quinazoline-2,4-diamine obtained in step B of example 13, *N*$^2$-(cis-4-aminomethyl-cyclohexyl)-*N*$^4$,*N*$^4$-dimethyl-5,6,7,8-tetrahydro-quinazoline-2,4-diamine obtained in intermediate of step B of example 15, *N*$^2$-(*cis-4*-amino-cyclohexyl)-*N*$^4$,*N*$^4$-dimethyl-pyrimidine-2,4-diamine obtained in step B of example 18, or *N*$^2$-(cis-4-aminomethyl-cyclohexyl)-*N*$^4$,*N*$^4$-dimethyl-pyrimidine-2,4-diamine obtained in step B of example 19.

**Example 1539-1658**

To a solution of poly(4-vinylpyridine) (75 μL) in $CH_2Cl_2$ (200 μL) were added the amines (30 μmol) as shown below in $CH_2Cl_2$ (200 μL) and chloroformate (60 μmol) in $CH_2Cl_2$ (200 μL) at ambient temperature. After stirring at the same temperature for 17 hr, the reaction mixture was filtrated and concentrated by a stream of dry $N_2$. To the residue were added $CH_2Cl_2$ (700 μL) and PSA (300 μL). After the stirring at ambient temperature for 19 hr, the reaction mixture was filtrated and purified by silica gel chromatography (NH-silica gel, 20% EtOAc in hexane to EtOAc only, and silica gel, 2% to 7% 2 M $NH_3$/MeOH in $CHCl_3$) to give the desired product. The product was determined by ESI-MS or APCI-MS.

Wherein the amines are selected from *N*$^2$-(*cis*-4-amino-cyclohexyl-*N*$^4$,*N*$^4$-dimethyl-quinoline-2,4-diamine obtained in step B of example 5, *N*$^2$-(*cis*-4-aminomethyl-cyclohexyl)-*N*$^4$,*N*$^4$-dimethyl-quinoline-2,4-diamine obtained in step B of example 7, *N*$^2$-(*cis*-4-amino-cyclohexyl)-*N*$^4$,*N*$^4$-dimethyl-5,6,7,8-tetrahydro-quinazoline-2,4-diamine obtained in step B of example 13, *N*$^2$-(*cis*-4-aminomethyl-cyclohexyl)-*N*$^4$,*N*$^4$-dimethyl-5,6,7,8-tetrahydro-quinazoline-2,4-diamine obtained in intermediate of step B of example 15, *N*$^2$-(*cis-4*-amino-cyclohexyl)-*N*$^4$,*N*$^4$-dimethyl-pyrimidine-2,4-diamine obtained in step B of example 18, or *N*$^2$-(*cis*-4-aminomethyl-cyclohexyl)-*N*$^4$,*N*$^4$-dimethyl-pyrimidine-2,4-diamine obtained in step B of example 19.

**Example 1659-2496**

To a solution of amines (30 μmol) as shown below in DMSO (300 μL) were added isocyanate or isothiocyanate (60 μmol) in DMSO (200 μL) at ambient temperature. The mixture was stirred at the same temperature for 22 hr. To the reaction mixture were added 2 M $MeNH_2$ in THF (30 μL, 60 μmol) or D-gulcamine (60 μmol) in DMSO (200 μL) at ambient temperature. After stirring at the same temperature for 20 hr, the reaction mixture was filtrated through a SCX, concentrated by a stream of dry $N_2$, and purified by silica gel chromatography (silica gel, 2% to 10% 2 M $NH_3$/MeOH in $CHCl_3$) and silica gel chromatography (NH-silica, 33% to 50% EtOAc in hexane) to give the desired product. The product was determined by ESI-MS or APCI-MS.

Wherein the amines are selected from *N*$^2$-(*cis*-4-amino-cyclohexyl)-*N*$^4$,*N*$^4$-dimethyl-quinoline-2,4-diamine obtained in step B of example 5, *N*$^2$-(*cis*-4-aminomethyl-cyclohexyl)-*N*$^4$,*N*$^4$-dimethyl-quinoline-2,4-diamine obtained in step B of example 7, *N*$^2$-(*cis*-4-amino-cyclohexyl)-*N*$^4$,*N*$^4$-dimethyl-5,6,7,8-tetrahydro-quinazoline-2,4-diamine obtained in step B of example 13, *N*$^2$-(*cis*-4-aminomethyl-cyclohexyl)-*N*$^4$,*N*$^4$-dimethyl-5,6,7,8-tetrahydro-quinazoline-2,4-diamine obtained in intermediate of step B of example 15, *N*$^2$-(*cis-4*-amino-cyclohexyl)-*N*$^4$,*N*$^4$-dimethyl-pyrimidine-2,4-diamine obtained in step B of example 18, or *N*$^2$-(*cis*-4-aminomethyl-cyclohexyl)-*N*$^4$,*N*$^4$-dimethyl-pyrimidine-2,4-diamine obtained in step B of example 19.

| Ex. No. | compound name | MS | class |
|---|---|---|---|
| 20 | N-(cis-4-{[4-(dimethylamino)quinolin-2-yl]amino}cyclohexyl)-3-methoxybenzamide | 419 (M + H) | 2 |
| 21 | 3-bromo-N-(cis-4-{[4-(dimethylamino)quinolin-2-yl]amino}cyclohexyl)benzamide | 467 (M + H) | 1 |
| 22 | 4-bromo-N-(cis-4-{[4-(dimethylamino)quinolin-2-yl]amino}cyclohexyl)benzamide | 467 (M + H) | 2 |
| 23 | N-(cis-4-{[4-(dimethylamino)quinolin-2-yl]amino}cyclohexyl)-2,1,3-benzoxadiazole-5-carboxamide | 431 (M + H) | 1 |
| 24 | 3-chloro-N-(cis-4-{[4-(dimethylamino)quinolin-2-yl]amino}cyclohexyl)benzamide | 423 (M + H) | 1 |
| 25 | 4-chloro-N-(cis-4-{[4-(dimethylamino)quinolin-2-yl]amino}cyclohexyl)benzamide | 423 (M + H) | 1 |
| 26 | (2E)-N-(cis-4-{[4-(dimethylamino)quinolin-2-yl]amino}cyclohexyl)-3-phenylacrylamide | 415 (M + H) | 3 |
| 27 | 4-chloro-N-(cis-4-{[4-(dimethylamino)quinolin-2-yl]amino}cyclohexyl)-3-nitrobenzamide | 468 (M + H) | 1 |
| 28 | 2-(4-chlorophenyl)-N-(cis-4-{[4-(dimethylamino)quinolin-2-yl]amino}cyclohexyl)acetamide | 437 (M + H) | 3 |
| 29 | 3-cyano-N-(cis-4-{[4-(dimethylamino)quinolin-2-yl]amino}cyclohexyl)benzamide | 414 (M + H) | 2 |
| 30 | 3,5-dichloro-N-(cis-4-{[4-(dimethylamino)quinolin-2-yl]amino}cyclohexyl)benzamide | 457 (M + H) | 2 |
| 31 | 3,4-dichloro-N-(cis-4-{[4-(dimethylamino)quinolin-2-yl]amino}cyclohexyl)benzamide | 457 (M + H) | 1 |
| 32 | N-(cis-4-{[4-(dimethylamino)quinolin-2-yl]amino}cyclohexyl)-2,2-diphenylacetamide | 479 (M + H) | 2 |
| 33 | N-(cis-4-{[4-(dimethylamino)quinolin-2-yl]amino}cyclohexyl)-3,4-difluorobenzamide | 425 (M + H) | 1 |
| 34 | N-(cis-4-{[4-(dimethylamino)quinolin-2-yl]amino}cyclohexyl)-3,5-difluorobenzamide | 425 (M + H) | 2 |
| 35 | 2-(2,5-dimethoxyphenyl)-N-(cis-4-{[4-(dimethylamino)quinolin-2-yl]amino}cyclohexyl)acetamide | 463 (M + H) | 3 |
| 36 | N-(cis-4-{[4-(dimethylamino)quinolin-2-yl]amino}cyclohexyl)-2-(ethylthio)nicotinamide | 450 (M + H) | 3 |
| 37 | N-(cis-4-{[4-(dimethylamino)quinolin-2-yl]amino}cyclohexyl)-4-fluorobenzamide | 407 (M + H) | 1 |
| 38 | N-(cis-4-{[4-(dimethylamino)quinolin-2-yl]amino}cyclohexyl)-3-fluoro-5-(trifluoromethyl)benzamide | 475 (M + H) | 2 |
| 39 | 2,4-dichloro-N-(cis-4-{[4-(dimethylamino)quinolin-2-yl]amino}cyclohexyl)-5-fluorobenzamide | 475 (M + H) | 3 |
| 40 | N-(cis-4-{[4-(dimethylamino)quinolin-2-yl]amino}cyclohexyl)hexanamide | 383 (M + H) | 3 |
| 41 | N-(cis-4-{[4-(dimethylamino)quinolin-2-yl]amino}cyclohexyl)-4-iodobenzamide | 515 (M + H) | 3 |

(continued)

| Ex. No. | compound name | MS | class |
|---|---|---|---|
| 42 | N-(cis-4-{[4-(dimethylamino)quinolin-2-yl]amino}cyclohexyl)-2-(methylthio)nicotinamide | 436 (M + H) | 3 |
| 43 | N-(cis-4-{[4-(dimethylamino)quinolin-2-yl]amino}cyclohexyl)-4-methyl-3-nitrobenzamide | 448 (M + H) | 2 |
| 44 | N-(cis-4-{[4-(dimethylamino)quinolin-2-yl)amino}cyclohexyl)-3-nitrobenzamide | 434 (M + H) | 1 |
| 45 | N-(cis-4-{[4-(dimethylamino)quinolin-2-yl]amino}cyclohexyl)-2-phenylacetamide | 403 (M + H) | 3 |
| 46 | (2R)-N-(cis-4-{[4-(dimethylamino)quinolin-2-yl]amino}cyclohexyl)-2-phenylcyclopropanecarboxamide | 429 (M + H) | 3 |
| 47 | N-(cis-4-{[4-(dimethylamino)quinolin-2-yl]amino}cyclohexyl)-1,3-benzodioxole-5-carboxamide | 433 (M + H) | 3 |
| 48 | N-(cis-4-{[4-(dimethylamino)quinolin-2-yl]amino}cyclohexyl)-2-phenoxybutanamide | 447 (M + H) | 1 |
| 49 | N-(cis-4-{[4-(dimethylamino)quinolin-2-yl]amino}cyclohexyl)-2-phenoxypropanamide | 433 (M + H) | 1 |
| 50 | N-(cis-4-{[4-(dimethylamino)quinolin-2-yl]amino}cyclohexyl)-3-methylbenzamide | 403 (M + H) | 1 |
| 51 | N-(cis-4-{[4-(dimethylamino)quinolin-2-yl]amino}cyclohexyl)-4-methylbenzamide | 403 (M + H) | 3 |
| 52 | N-(cis-4-{[4-(dimethylamino)quinolin-2-yl]amino}cyclohexyl) thiophene-2-carboxamide | 395 (M + H) | 3 |
| 53 | N-(cis-4-{[4-(dimethylamino)quinolin-2-yl]amino}cyclohexyl)-2-(2-thienyl)acetamide | 409 (M + H) | 3 |
| 54 | N-(cis-4-{[4-(dimethylamino)quinolin-2-yl]amino}cyclohexyl)-3-(trifluoromethoxy)benzamide | 473 (M + H) | 2 |
| 55 | benzyl (cis-4-{[4-(dimethylamino)quinolin-2-yl]amino}cyclohexyl) carbamate | 419 (M + H) | 3 |
| 56 | 4-nitrobenzyl (cis-4-{[4-(dimethylamino)quinolin-2-yl]amino} cyclohexyl)carbamate | 464 (M + H) | 3 |
| 57 | 4-bromo-N-(cis-4-{[4-(dimethylamino)quinolin-2-yl]amino} cyclohexyl)-3-methylbenzamide | 481 (M + H) | 1 |
| 58 | N-(cis-4-{[4-(dimethylamino)quinolin-2-yl]amino}cyclohexyl)-3-iodobenzamide | 515 (M + H) | 2 |
| 59 | 3-chloro-N-(cis-4-{[4-(dimethylamino)quinolin-2-yl]amino} cyclohexyl)-2-fluorobenzamide | 441 (M + H) | 3 |
| 60 | N-(cis-4-{[4-(dimethylamino)quinolin-2-yl]amino}cyclohexyl)-2,3-difluoro-4-methylbenzamide | 439 (M + H) | 3 |
| 61 | 2-chloro-N-(cis-4-{[4-(dimethylamino)quinolin-2-yl]amino} cyclohexyl)-4-fluorobenzamide | 441 (M + H) | 3 |
| 62 | 3-chloro-N-(cis-4-{[4-(dimethylamino)quinolin-2-yl]amino} cyclohexyl)-2,4-difluorobenzamide | 459 (M + H) | 2 |
| 63 | N-(cis-4-{[4-(dimethylamino)quinolin-2-yl]amino}cyclohexyl)-2-(phenylthio)acetamide | 435 (M + H) | 3 |

(continued)

| Ex. No. | compound name | MS | class |
|---|---|---|---|
| 64 | N-(cis-4-{[4-(dimethylamino)quinolin-2-yl]amino}cyclohexyl)-2-fluoro-3-(trifluoromethyl)benzamide | 475 (M + H) | 3 |
| 65 | N-(cis-4-({[4-(dimethylamino)quinolin-2-yl]amino}cyclohexyl)-2-fluoro-5-(trifluoromethyl)benzamide | 475(M + H) | 3 |
| 66 | N-(cis-4-{[4-(dimethylamino)quinolin-2-yl]amino}cyclohexyl)-2-phenylbutanamide | 431(M + H) | 3 |
| 67 | N-(cis-4-{[4-(dimethylamino)quinolin-2-yl]amino}cyclohexyl)-2-(3-methoxyphenyl)acetamide | 433(M + H) | 3 |
| 68 | N-(cis-4-{[4-(dimethylamino)quinolin-2-yl]amino}cyclohexyl)-2-(4-fluorophenyl)acetamide | 421 (M + H) | 3 |
| 69 | N-(cis-4-{[4-(dimethylamino)quinolin-2-yl]amino}cyclohexyl)-2-(4-methoxyphenyl)acetamide | 433 (M + H) | 3 |
| 70 | N-(cis-4-{[4-(dimethylamino)quinolin-2-yl]amin}cyclohexyl)-5-methyl-2-(trifluoromethyl)-3-furamide | 461 (M + H | 3 |
| 71 | N-(cis-4-{[4-(dimethylamino)quinolin-2-yl]amino}cyclohexyl)-2,5-dimethyl-3-furamide | 407 (M + H) | 1 |
| 72 | N-(cis-4-{[4-(dimethylamino)quinolin-2-yl]amino}cyclohexyl)-2-ethoxybenzamide | 433 (M + H) | 3 |
| 73 | 3-chloro-N-(cis-4-{[4-(dimethylamino)quinolin-2-yl]amino}cyclohexyl)-4-fluorobenzamide | 441 (M + H) | 1 |
| 74 | N-(cis-4-{[4-(dimethylamino)quinolin-2-yl]amino}cyclohexyl)-3-fluoro-4-methylbenzamide | 421 (M + H) | 2 |
| 75 | 2-cyclopentyl-N-(cis-4-{[4-(dimethylamino)quinolin-2-yl]amino]cyclohexyl)acetamide | 395 (M + H) | 3 |
| 76 | N-(cis-4-{[4-(dimethylamino)quinolin-2-yl]amino}cyclohexyl)-3,5-dimethoxybenzamide | 449 (M + H) | 1 |
| 77 | 4-cyano-N-(cis-4-{[4-(dimethylamino)quinolin-2-yl]amino}cyclohexyl)benzamide | 414 (M + H) | 3 |
| 78 | N-(cis-4-{[4-(dimethylamino)quinolin-2-yl]amino}cyclohexyl)-3,5-bis(trifluoromethyl)benzamide | 525 (M + H) | 2 |
| 79 | (2E)-N-(cis-4-{[4-(dimethylamino)quinolin-2-yl]amino}cyclohexyl)-3-(4-nitrophenyl)acrylamide | 460 (M + H) | 3 |
| 80 | 2-(2-bromophenyl)-N-(cis-4-{[4-(dimethylamino)quinolin-2-yl]amino}cyclohexyl)acetamide | 481 M + H) | 3 |
| 81 | N-(cis-4-{[4-(dimethylamino)quinolin-2-yl]amino}cyclohexyl)-4-fluoro-3-methylbenzamide | 421 (M + H) | 1 |
| 82 | 2-[(difluoromethyl)thio]-N-(cis-4-{[4-(dimethylamino)quinolin-2-yl]amino}cyclohexyl)benzamide | 471 (M + H) | 3 |
| 83 | 2,5-dichloro-N-(cis-4-{[4-(dimethylamino)quinolin-2-yl]amino}cyclohexyl)thiophene-3-carboxamide | 463 (M + H) | 2 |
| 84 | N-(cis-4-{[4-(dimethylamino)quinolin-2-yl]amino}cyclohexyl)-2-(propylthio)nicotinamide | 464 (M + H) | 3 |
| 85 | 1 -benzyl-3-tert-butyl-N-(cis-4-[4-(dimethylamino)quinolin-2-yl]amino}cyclohexyl)-1H-pyrazole-5-carboxamide | 525 (M + H) | 3 |

(continued)

| Ex. No. | compound name | MS | class |
|---|---|---|---|
| 86 | 3-tert-butyl-N-(cis-4-{[4-(dimethylamino)quinolin-2-yl]amino} cyclohexyl)-1-methyl-1 H-pyrazole-5-carboxamide | 449 (M + H) | 3 |
| 87 | (2E)-N-(cis-4-{[4-(dimethylamino)quinolin-2-yl]amino}cyclohexyl)-2-methyl-3-phenylacrylamide | 429 (M + H) | 3 |
| 88 | 5-bromo-N-(cis-4-{[4-(dimethylamino)quinolin-2-yl]amino} cyclohexyl)nicotinamide | 468 (M + H) | 3 |
| 89 | N-(cis-4-[4-(dimethylamino)quinolin-2-yl]amino}cyclohexyl)-2-(1-naphthyl)acetamide | 453 (M + H) | 3 |
| 90 | 1-tert-butyl-N-(cis-4-{[4-(dimethylamino)quinolin-2-yl]amino} cyclohexyl)-5-methyl-1H-pyrazole-3-carboxamide | 449 (M + H) | 3 |
| 91 | N-(cis-4-{[4-(dimethylamino)quinolin-2-yl]amino}cyclohexyl)-1-benzothiophene-3-carboxamide | 445 (M + H) | 3 |
| 92 | 2-[(cis-4-{[4-(dimethylamino)quinolin-2-yl]amino}cyclohexyl) amino]-2-oxo-1-phenylethyl acetate | 461 (M + H) | 3 |
| 93 | N-(cis-4-{[4-(dimethylamino)quinolin-2-yl]amino}cyclohexyl) benzamide | 389 (M + H) | 3 |
| 94 | N-(cis-4-{[4-(dimethylamino)quinolin-2-yl]amino}cyclohexyl)-1-benzothiophene-2-carboxamide | 445 (M + H) | 3 |
| 95 | 2-(benzyloxy)-N-(cis-4-{[4-(dimethylamino)quinolin-2-yl]amino} cyclohexyl)acetamide | 433 (M + H) | 3 |
| 96 | 2-(4-chlorophenoxy)-N-(cis-4-{[4-(dimethylamino)quinolin-2-yl] amino}cyclohexyl)acetamide | 453 (M + H) | 1 |
| 97 | N-(cis-4-{[4-(dimethylamino)quinolin-2-yl]amino}cyclohexyl) cyclohexanecarboxamide | 395 (M + H) | 3 |
| 98 | 3-(2-chlorophenyl)-N-(cis-4-{[4-(dimethylamino)quinolin-2-yl] amino}cyclohexyl)-5-methylisoxazole-4-carboxamide | 504 (M + H) | 1 |
| 99 | 1-(4-chlorophenyl)-N-(cis-4-{[4-(dimethylamino)quinolin-2-yl) amino}cyclohexyl)cyclopentanecarboxamide | 491 (M + H) | 2 |
| 100 | 3-(2-chloro-6-fluorophenyl)-N-(cis-4-{[4-(dimethylamino)quinolin-2-yl]amino}cyclohexyl)-5-methylisoxazole-4-carboxamide | 522 (M + H) | 1 |
| 101 | 3-chloro-N-(cis-4-{[4-(dimethylamino)quinolin-2-yl]amino} cyclohexyl)-4-(isopropylsulfonyl)thiophene-2-carboxamide | 535 (M + H) | 3 |
| 102 | 2-chloro-N-(cis-4-{[4-(dimethylamino)quinolin-2-yl]amino} cyclohexyl)-4-nitrobenzamide | 468 (M + H) | 3 |
| 103 | N (*cis*1-4-{[4-(dimethylamino)quinolin-2-yl]amino}cyclohexyl)-1,3-dimethyl-1H-pyrazole-5-carboxamide | 407 (M + H) | 3 |
| 104 | N-(cis-4-{[4-(dimethylamino)quinolin-2-yl]amino}cyclohexyl)-3,4-dimethoxybenzamide | 449 (M + H) | 3 |
| 105 | N-(cis-4-{[4-(dimethylamino)quinolin-2-yl]aminolcyclohexyl)-3-fluorobenzamide | 407 (M + H) | 2 |
| 106 | N-(cis-4-{[4-(dimethylamino)quinolin-2-yl]amino}cyclohexyl)-4-fluoro-3-(trifluoromethyl)benzamide | 475 (M + H) | 1 |
| 107 | N-(cis-4-{[4-(dimethylamino)quinolin-2-yl]amino}cyclohexyl)-5-methyl-2-phenyl-2H-1,2,3-triazole-4-carboxamide | 470 (M + H) | 2 |

(continued)

| Ex. No. | compound name | MS | class |
|---|---|---|---|
| 108 | N-(cis-4-{[4-(dimethylamino)quinolin-2-yl]amino}cyclohexyl)-2-(4-methoxyphenoxy)-5-nitrobenzamide | 556 (M + H) | 1 |
| 109 | N-(cis-4-{[4-(dimethylamino)quinolin-2-yl]amino}cyclohexyl)-1-naphthamide | 439 (M + H) | 3 |
| 110 | N-(cis-4-{[4-(dimethylamino)quinolin-2-yl]amino}cyclonexyl)-2-naphthamide | 439 (M + H) | 3 |
| 111 | N-(cis-4-{[4-(dimethylamino)quinolin-2-yl]amino}cyclohexyl)-5-nitro-2-furamide | 424 (M + H) | 1 |
| 112 | N-(cis-4-{[4-(dimethylamino)quinolin-2-yl]amino}cyclohexyl)-2-phenoxyacetamide | 419 (M+H) | 1 |
| 113 | N-(cis-4-{[4-(dimethylamino)quinolin-2-yl]amino}cyclohexyl)-2-(2-nitrophenoxy)acetamide | 464 (M + H) | 3 |
| 114 | N-(cis-4-{[4-(dimethylamino)quinolin-2-yl]amino}cyclohexyl) quinoxaline-2-carboxamide | 441 (M + H) | 2 |
| 115 | N-(cis-4-{[4-(dimethylamino)quinolin-2-yl]amino}cyclohexyl)-3,4,5-trimethoxybenzamide | 479 (M + H) | 3 |
| 116 | N-(cis-4-{[4-(dimethylamino)quinolin-2-yl]amino}cyclohexyl)-3-(trifluoromethyl)benzamide | 457 (M + H) | 3 |
| 117 | N-(cis-4-{[4-(dimethylamino)quinolin-2-yl]amino}cyclohexyl)-4-(trifluoromethyl)benzamide | 457 (M + H) | 3 |
| 118 | N-(cis-4-[{4-(dimethylamino)quinolin-2-yl]amino}cyclohexyl)-2-(trifluoromethoxy)benzamide | 473 (M + H) | 3 |
| 119 | 4,5-dimethoxy-2-nitrobenzyl (cis-4-{[4-(dimethylamino)quinolin-2-yl]amino}cyclohexyl)carbamate | 524 (M + H) | 3 |
| 120 | N-(cis-4-{[4-(dimethylamino)quinolin-2-yl]amino}cyclohexyl)-4-phenoxybutanamide | 447 (M + H) | 3 |
| 121 | 2-bromo-N-(cis-4-{[4-(dimethylamino)quinolin-2-yl]amino} cyclohexyl)-5-methoxybenzamide | 497 (M + H) | 3 |
| 122 | N-(cis-4-{[4-(dimethylamino)quinolin-2-yl]amino}cyclohexyl)-2-(pentafluorophenoxy)acetamide | 509 (M + H) | 3 |
| 123 | 2-(3,4-dimethoxyphenyl)-N-(cis-4-{[4-(dimethylamino)quinolin-2-yl]amino}cyclohexyl)acetamide | 463 (M + H) | 3 |
| 124 | N-(cis-4-{[4-(dimethylamino)quinolin-2-yl]amino}cyclohexyl)-2,3,4-trifluorobenzamide | 443 (M + H) | 3 |
| 125 | N-(cis-4-{[4-(dimethylamino)quinolin-2-yl]amino}cyclohexyl) cyclopentanecarboxamide | 381 (M + H) | 3 |
| 126 | N-(cis-4-{[4-(dimethylamino)quinolin-2-yl]amino}cyclohexyl)-2,4-difluorobenzamide | 425 (M+ H) | 3 |
| 127 | N-(cis-4-{[4-(dimethylamino)quinolin-2-yl]amino}cyclohexyl)-3-phenylpropanamide | 417 (M + H) | 3 |
| 128 | N-(cis-4-{[4-(dimethylamino)quinolin-2-yl]amino}cyclohexyl)-2,3,4,5-tetrafluorobenzamide | 461 (M + H) | 3 |
| 129 | N-(cis-4-{[4-(dimethylamino)quinolin-2-yl]amino}cyclohexyl)-2-ethoxy-1-naphthamide | 483 (M + H) | 3 |

(continued)

| Ex. No. | compound name | MS | class |
|---|---|---|---|
| 130 | N-(cis-4-{[4-(dimethylamino)quinolin-2-yl]amino}cyclohexyl)-2,3,4,5,6-pentafluorobenzamide | 479 (M + H) | 3 |
| 131 | N-(cis-4-{[4-(dimethylamino)quinolin-2-yl]amino}cyclohexyl)-4-[(trifluoromethyl)thio]benzamide | 489 (M + H) | 3 |
| 132 | 3,4,5-trichloro-N-(cis-4-{[4-(dimethylamino)quinolin-2-yl]amino}cyclohexyl)thiophene-2-carboxamide | 497 (M + H) | 3 |
| 133 | 2-(3-chlorophenoxy)-N-(cis-4-{[4-(dimethylamino)quinolin-2-yl]amino}cyclohexyl)acetamide | 453 (M + H) | 1 |
| 134 | 3-(2,6-dichlorophenyl)-N-(cis-4-{[4-(dimethylamino)quinolin-2-yl]amino}cyclohexyl)-5-methylisoxazole-4-carboxamide | 538 (M + H) | 1 |
| 135 | N-(cis-4-{[4-(dimethylamino)quinolin-2-yl]amino}cyclohexyl)-2-phenoxynicotinamide | 482 (M + H) | 1 |
| 136 | N-(cis-4-{[4-(dimethylamino)quinolin-2-yl]amino}cyclohexyl)-2-(phenylthio)nicotinamide | 498 (M + H) | 3 |
| 137 | N-(cis-4-{[4-(dimethylamino)quinolin-2-yl]amino}cyclohexyl)-2-(4-methylphenoxy)nicotinamide | 496 (M + H) | 1 |
| 138 | N-(cis-4-{[4-(dimethylamino)quinolin-2-yl]amino}cyclohexyl)-4-[(dipropylamino)sulfonyl]benzamide | 552 (M +H) | 3 |
| 139 | 2-(4-chlorophenoxy)-N-(cis-4-{[4-(dimethylamino)quinolin-2-yl]amino}cyclohexyl)-2-methylpropanamide | 481 (M + H) | 3 |
| 140 | 5-(4-chlorophenyl)-N-(cis-4-{[4-(dimethylamino)quinolin-2-yl]amino}cyclohexyl)-2-(trifluoromethyl)-3-furamide | 557 (M + H) | 3 |
| 141 | 2-(2,3-dihydro-1-benzofuran-5-yl)-N-(cis-4-{[4-(dimethylamino)quinolin-2-yl]amino}cyclohexyl)-1,3-thiazole-4-carboxamide | 514 (M + H) | 3 |
| 142 | 3-tert-butyl-1-(2,4-dichlorobenzyl)-N-(cis-4-{[4-(dimethylamino}quinolin-2-yl]amino)cyclohexyl)-1H-pyrazole-5-carboxamide | 593 (M + H) | 3 |
| 143 | 6-chloro-N-(cis-4-{[4-(dimethylamino)quinolin-2-yl]amino}cyclohexyl)-2H-chromene-3-carboxamide | 477 (M + H) | 3 |
| 144 | 3-chloro-N-(cis-4-{[4-(dimethylamino)quinolin-2-yl]amino}cyclohexyl)-4-(trifluoromethoxy)benzamide | 507 (M + H) | 3 |
| 145 | N-(cis-4-{[4-(dimethylamino)quinolin-2-yl]amino}cyclohexyl)-2-[(4-methyl-2-oxo-2H-chromen-8-yl)oxy]acetamide | 501 (M + H) | 3 |
| 146 | N-(cis-4-{[4-(dimethylamino)quinolin-2-yl]amino}cyclohexyl)-2-(2-thienyl)-1,3-thiazole-4-carboxamide | 478 (M + H) | 1 |
| 147 | N-[(cis-4-{[4-(dimethylamino)quinolin-2-yl]amino}cyclohexyl)methyl]-3-methoxybenzamide | 433 (M + H) | 3 |
| 148 | 3-bromo-N-[(cis-4-{[4-(dimethylamino)quinolin-2-yl]amino}cyclohexyl)methyl]benzamide | 481 (M + H) | 3 |
| 149 | 4-bromo-N-[(cis-4-{[4-(dimethylamino)quinolin-2-yl]amino}cyclohexyl)methyl]benzamide | 481 (M + H) | 3 |
| 150 | N-[(cis-4-({[4-(dimethylamino)quinolin-2-yl]amino}cyclohexyl)methyl]-2,1,3-benzoxadiazole-5-carboxamide | 445 (M + H) | 3 |
| 151 | 3-chloro-N-[(cis-4-{[4-(dimethylamino)quinolin-2-yl]amino}cyclohexyl)methyl]benzamide | 437 (M + H) | 3 |

(continued)

| Ex. No. | compound name | MS | class |
|---|---|---|---|
| 152 | 4-chloro-N-[(cis-4-{[4-(dimethylamino)quinolin-2-yl]amino} cyclohexyl)methyl]benzamide | 437 (M + H) | 3 |
| 153 | (2E)-N-[(cis-4-{[4-(dimethylamino)quinolin-2-yl]amino}cyclohexyl) methyl]-3-phenylacrylamide | 429 (M + H) | 3 |
| 154 | 4-chloro-N-[(cis-4-{[4-(dimethylamino)quinolin-2-yl]amino} cyclohexyl)methyl]-3-nitrobenzamide | 482 (M + H) | 3 |
| 155 | 2-(4-chlorophenyl)-N-[(cis-4-{[4-(dimethylamino)quinolin-2-yl] amino}cyclohexyl)methyl]acetamide | 451 (M + H) | 3 |
| 156 | 3-cyano-N-[(cis-4-{[4-(dimethylamino)quinolin-2-yl]amino} cyclohexyl)methyl]benzamide | 428 (M + H) | 3 |
| 157 | 3,5-dichloro-N-[(cis-4-{[4-(dimethylamino)quinolin-2-yl]amino} cyclohexyl)methyl]benzamide | 471 (M + H) | 3 |
| 158 | 3,4-dichloro-N-[(cis-4-{[4-(dimethylamino)quinolin-2-yl]amino} cyclohexyl)methyl]benzamide | 471 (M + H) | 3 |
| 159 | N-[(cis-4-{[4-(dimethylamino)quinolin-2-yl]amino}cyclohexyl) methyl]-2,2-diphenylacetamide | 493 (M + H) | 2 |
| 160 | N-[(cis-4-{[4-(dimethylamino)quinolin-2-yl]amino}cyclohexyl) methyl]-3,4-difluorobenzamide | 439 (M + H) | 3 |
| 161 | N-[(cis-4-{[4-(dimethylamino)quinolin-2-yl]amino}cyclohexyl) methyl]-3,5-difluorobenzamide | 439 (M + H) | 3 |
| 162 | 2-(2,5-dimethoxyphenyl)-N-[(cis-4-{[4-(dimethylamino)quinolin-2-yl]amino}cyclohexyl)methyl]acetamide | 477 (M + H) | 3 |
| 163 | N-[(cis-4-{[4-(dimethylamino)quinolin-2-yl]amino}cyclohexyl) methyl]-2-(ethylthio)nicotinamide | 464 (M + H) | 3 |
| 164 | N-[(cis-4-{[4-(dimethylamino)quinolin-2-yl]amino}cyclohexyl) methyl]-4-fluorobenzamide | 421 (M + H) | 3 |
| 165 | N-[(cis-4-{[4-(dimethylamino)quinolin-2-yl]amino}cyclohexyl) methyl]-3-fluoro-5-(trifluoromethyl)benzamide | 489 (M + H) | 3 |
| 166 | 2,4-dichloro-N-[(cis-4-{[4-(dimethylamino)quinolin-2-yl]amino} cyclohexyl)methyl]-5-fluorobenzamide | 489 (M + H) | 3 |
| 167 | N-[(cis-4-{[4-(dimethylamino)quinolin-2-yl]amino}cyclohexyl) methyl]hexanamide | 397 (M + H) | 3 |
| 168 | N-[(cis-4-{[4-(dimethylamino)quinolin-2-yl]amino}cyclohexyl) methyl)-4-iodobenzamide | 529 (M + H) | 3 |
| 169 | N-[(cis-4-{[4-(dimethylamino)quinolin-2-yl]amino}cyclohexyl) methyl]-2-(methylthio)nicotinamide | 450 (M + H) | 3 |
| 170 | N-[(cis-4-{[4-(dimethylamino)quinolin-2-yl]amino}cyclohexyl) methyl]-4-methyl-3-nitrobenzamide | 462 (M + H) | 3 |
| 171 | N-[(cis-4-{[4-(dimethylamino)quinolin-2-yl]amino}cyclohexyl) methyl]-3-nitrobenzamide | 448 (M + H) | 3 |
| 172 | N-[(cis-4-{[4-(dimethylamino)quinolin-2-yl]amino}cyclohexyl) methyl]-2-phenylacetamide | 417 (M + H) | 3 |
| 173 | (2R)-N-[(cis-4-{[4-(dimethylamino}quinolin-2-yl]amino}cyclohexyl) methyl]-2-phenylcyclopropanecarboxamide | 443 (M + H) | 3 |

(continued)

| Ex. No. | compound name | MS | class |
|---|---|---|---|
| 174 | N-[(cis-4-{[4-(dimethylamino)quinolin-2-yl]amino}cyclohexyl) methyl]-1,3-benzodioxole-5-carboxamide | 447 (M + H) | 3 |
| 175 | N-[(cis-4-{[4-(dimethylamino)quinolin-2-yl]amino}cyclohexyl) methyl]-2-phenoxybutanamide | 461 (M + H) | 3 |
| 176 | N-[(cis-4-{[4-(dimethylamino)quinolin-2-yl]amino}cyclohexyl) methyl]-2-phenoxypropanamide | 447 (M + H) | 3 |
| 177 | N-[(cis-4-{[4-(dimethylamino)quinolin-2-yl]amino}cyclohexyl) methyl]-3-methylbenzamide | 417 (M + H) | 3 |
| 178 | N-[(cis-4-{[4-(dimethylamino)quinolin-2-yl]amino}cyclohexyl) methyl)-4-methyl]benzamide | 417 (M + H) | 3 |
| 179 | N-[(cis-4-{[4-(dimethylamino)quinolin-2-yl]amino}cyclohexyl) methyl]thiophene-2-carboxamide | 409 (M + H) | 3 |
| 180 | N-[(cis-4-{[4-(dimethylamino)quinolin-2-yl]amino}cyclohexyl) methyl]-2-(2-thienyl)acetamide | 423 (M + H) | 3 |
| 181 | N-[(cis-4-{[4-(dimethylamino)quinolin-2-yl]amino}cyclohexyl) methyl)-3-(trifluoromethoxy)benzamide | 487 (M + H) | 3 |
| 182 | [4-(4-Dimethylamino-quinolin-2-ylamino)-cyclohexylmethyl]-carbamic acid benzyl ester | 433 (M + H) | 3 |
| 183 | [4-(4-Dimethylamino-quinolin-2-ylamino)-cyclohexylmethyl]-carbamic acid 4-nitro-benzyl ester | 478 (M + H) | 3 |
| 184 | 4-bromo-N-[(cis-4-{[4-(dimethylamino)quinolin-2-yl]amino} cyclohexylmethyl]-3-methyl]benzamide | 495 (M + H) | 3 |
| 185 | N-[(cis-4-{[4-(dimethylamino)quinolin-2-yl]amino}cyclohexyl) methyl]-3-iodobenzamide | 529 (M + H) | 3 |
| 186 | 3-chloro-N-[(cis-4-{[4-(dimethylamino)quinolin-2-yl]amino} cyclohexyl)methyl]-2-fluorobenzamide | 455 (M + H) | 3 |
| 187 | N-[(cis-4-{[4-(dimethylamino)quinolin-2-yl]amino}cyclohexyl) methyl]-2,3-difluoro-4-methylbenzamide | 453 (M + H) | 3 |
| 188 | 2-chloro-N-[(cis-4-{[4-(dimethylamino)quinolin-2-yl]amino} cyclohexyl)methyl]-4-fluorobenzamide | 455 (M + H) | 3 |
| 189 | 3-chloro-N-[(cis-4-{[4-(dimethylamino)quinolin-2-yl]ammo} cyclohexyl)methyl]-2,4-difluorobenzamide | 473 (M + H) | 3 |
| 190 | N-[(cis-4-{[4-(dimethylamino)quinolin-2-yl]amino}cyclohexyl) methyl]-2-(phenylthio)acetamide | 449 (M + H) | 3 |
| 191 | N-[(cis-4-{[4-(dimethylamino)quinolin-2-yl]amino}cyclohexyl) methyl]-2-fluoro-3-(trifluoromethyl)benzamide | 489 (M + H) | 3 |
| 192 | N-[(cis-4-{[4-(dimethylamino)quinolin-2-yl]amino}cyclohexyl)-methyl]-2-fluoro-5-(trifluoromethyl)benzamide | 489 (M + H) | 3 |
| 193 | N-[(cis-4-{[4-(dimethylamino)quinolin-2-yl]amino}cyclohexyl) methyl]-2-phenylbutanamide | 445 (M + H) | 3 |
| 194 | N-[(cis-4-{[4-(dimethylamino)quinolin-2-yl]amino}cyclohexyl) methyl]-2-(3-methoxyphenyl)acetamide | 447 (M + H) | 3 |
| 195 | N-[(cis-4-{[4-(dimethylamino)quinolin-2-yl]amino}cyclohexyl) methyl]-2-(4-fluorophenyl)acetamide | 435 (M + H) | 3 |

(continued)

| Ex. No. | compound name | MS | class |
|---|---|---|---|
| 196 | N-[(cis-4-{[4-(dimethylamino)quinolin-2-yl]amino}cyclohexyl) methyl]-2-(4-methoxyphenyl)acetamide | 447 (M + H) | 3 |
| 197 | N-[(cis-4-{[4-(dimethylamino)quinolin-2-yl]amino}cyclohexyl) methyl]-5-methyl-2-(trifluoromethyl)-3-furamide | 475 (M + H) | 3 |
| 198 | N-[(cis-4-{[4-(dimethylamino)quinolin-2-yl]amino}cyclohexyl) methyl]-2,5-dimethyl-3-furamide | 421 (M + H) | 3 |
| 199 | N-[(cis-4-{[4-(dimethylamino)quinolin-2-yl]amino}cyclohexyl) methyl]-2-ethoxybenzamide | 447 (M + H) | 3 |
| 200 | 3-chloro-N-[(cis-4-{[4-(dimethylamino)quinolin-2-yl]amino} cyclohexyl)methyl]-4-fluorobenzamide | 455 (M + H) | 3 |
| 201 | N-[(cis-4-{[4-(dimethylamino)quinolin-2-yl]amino}cyclohexyl) methyl]-3-fluoro-4-methylbenzamide | 435 (M + H) | 3 |
| 202 | 2-cyclopentyl-N-[(cis-4-{[4-(dimethylamino)quinolin-2-yl]amino} cyclohexyl)methyl]acetamide | 409 (M + H) | 3 |
| 203 | N-[(cis-4-{[4-(dimethylamino)quinolin-2-yl]amino}cyclohexyl) methyl]-3,5-dimethoxybenzamide | 463 (M + H) | 3 |
| 204 | 4-cyano-N-5[(cis-4-{[4-(dimethylamino)quinolin-2-yl]amino} cyclohexyl)methyl]benzamide | 428 (M + H) | 3 |
| 205 | N-[(cis-4-{[4-(dimethylamino)quinolin-2-yl]amino}cyclohexyl) methyl]-3,5-bis(trifluoromethyl)benzamide | 539 (M + H) | 3 |
| 206 | (2E)-N-[(cis-4-{[4-(dimethylamino)quinolin-2-yl]amino}cyclohexyl) methyl]-3-(4-nitrophenyl)acrylamide | 474 (M + H) | 2 |
| 207 | 2-(2-bromophenyl)-N-[(cis-4-{[4-(dimethylamino)quinolin-2-yl] amino}cyclohexyl)methyl]acetamide | 495 (M + H) | 3 |
| 208 | N-[(cis-4-{[4-(dimethylamino)quinolin-2-yl]amino}cyclohexyl) methyl]-4-fluoro-3-methylbenzamide | 435 (M + H) | 3 |
| 209 | 2-[(difluoromethyl)thio]-N-[(cis-4-{[4-(dimethylamino)quinolin-2-yl] amino}cyclohexyl)methyl]benzamide | 485 (M + H) | 3 |
| 210 | 2,5-dichloro-N-[(cis-4-{[4-(dimethylamino)quinolin-2-yl]amino} cyclohexyl)methyl]thiophene-3-carboxamide | 477 (M + H) | 3 |
| 211 | N-[(cis-4-{[4-(dimethylamino)quinolin-2-yl]amino}cyclohexyl) methyl]-2-(propylthio)nicotinamide | 478 (M + H) | 3 |
| 212 | 1-benzyl-3-tert-butyl-N-[(cis-4-{[4-(dimethylamino)quinolin-2-yl] amino}cyclohexyl)methyl]-1H-pyrazole-5-carboxamide | 539 (M + H) | 3 |
| 213 | 3-tert-butyl-N-[(cis-4-{[4-(dimethylamino)quinolin-2-yl]amino} cyclohexyl)methyl]-1-methyl-1H-pyrazole-5-carboxamide | 463 (M + H) | 3 |
| 214 | (2E)-N-[(cis-4-{[4-(dimethylamino)quinolin-2-yl]amino}cyclohexyl) methyl]-2-methyl-3-phenylacrylamide | 443 (M + H) | 3 |
| 215 | 5-bromo-N-[(cis-4-{[4-(dimethylamino)quinolin-2-yl]amino} cyclohexyl)methyl]nicotinamide | 482 (M + H) | 3 |
| 216 | N-[(cis-4-{[4-(dimethylamino)quinolin-2-yl]amino}cyclohexyl) methyl]-2-(1-naphthyl)acetamide | 467 (M + H) | 3 |
| 217 | 1-tert-butyl-N-[(cis-4-{[4-(dimethylamino)quinolin-2-yl]amino} cyclohexyl)methyl]-5-methyl-1H-pyrazole-3-carboxamide | 463 (M + H) | 3 |

(continued)

| Ex. No. | compound name | MS | class |
|---|---|---|---|
| 218 | N-[(cis-4-{[4-(dimethylamino)quinolin-2-yl]amino}cyclohexyl) methyl]-1-benzothiophene-3-carboxamide | 459 (M + H) | 3 |
| 219 | N-[(cis-4-{[4-(dimethylamino)quinolin-2-yl]amino}cyclohexyl) methyl]biphenyl-4-carboxamide | 479 (M + H) | 3 |
| 220 | 2-bromo-N-[(cis-4-{[4-(dimethylamino)quinolin-2-yl]amino} cyclohexyl)methyl]benzamide | 481 (M + H) | 3 |
| 221 | 2,6-dichloro-N-[(cis-4-{[4-(dimethylamino)quinolin-2-yl]amino} cyelohexyl)methyl]benzamide | 471 (M + H) | 2 |
| 222 | N-[(cis-4-{[4-(dimethylamino)quinolin-2-yl]amino}cyclohexyl) methyl]-2-iodobenzamide | 529 (M + H) | 3 |
| 223 | N-[(cis-4-{[4-(dimethylarnino)quinolin-2-yl]amino}cyclohexyl) methyl]-2-methylbenzamide | 417 (M + H) | 3 |
| 224 | 2,3-dichloro-N-[(cis-4-{[4-(dimethylamino)quinolin-2-yl]amino} cyclohexyl)methyl]benzamide | 471 (M + H) | 3 |
| 225 | 2-chloro-N-[(cis-4-{[4-(dimethylamino)quinolin-2-yl]amino} cyclohexyl)methyl]-5-fluorobenzamide | 455 (M + H) | 3 |
| 226 | N-[(cis-4-{[4-(dimethylamino)quinolin-2-yl]amino}cyclohexyl) methyl]-9-oxo-9H-fluorene-4-carboxamide | 505 (M + H) | 3 |
| 227 | N-[(cis-4-{[4-(dimethylamino)quinolin-2-yl]amino}cyclohexyl) methyl]-2,3,6-trifluorobenzamide | 457 (M + H) | 3 |
| 228 | N-[(cis-4-{[4-(dimethylamino)quinolin-2-yl]amino}cyclohexyl) methyl]-2,3-difluorobenzamide | 439 (M + H) | 3 |
| 229 | N-[(cis-4-{[4-(dimethylamino)quinolin-2-yl]amino}cyclohexyl) methyl]-2,6-difluorobenzamide | 439 (M + H) | 3 |
| 230 | N-[(cis-4-{[4-(dimethylamino)quinolin-2-yl]amino}cyclohexyl)-methyl]-2-fluoro-6-(trifluoromethyl)benzamide | 489 (M + H) | 3 |
| 231 | N-[(cis-4-{[4-(dimethylamino)quinolin-2-yl]amino}cyclohexyl) methyl]-2,4,6-trimethylbenzamide | 445 (M + H) | 1 |
| 232 | 2-chloro-N-[(cis-4-{[4-(dimethylamino)quinolin-2-yl]amino} cyclohexyl)methyl]-6-fluorobenzamide | 455 (M + H) | 3 |
| 233 | 2,4,6-trichloro-N-[(cis-4-{[4-(dimethylamino)quinolin-2-yl]amino} cyclohexyl)methyl]benzamide | 505 (M + H) | 1 |
| 234 | (2E)-3-(2-chlorophenyl)-N-[(cis-4-{[4-(dimethylamino)quinolin-2-yl]amino}cyclohexyl)methyl]acrylamide | 463 (M + H) | 2 |
| 235 | 6-chloro-N-[(cis-4-{[4-(dimethylamino)quinolin-2-yl]amino} cyclohexyl)methyl]-2-fluoro-3-methylbenzamide | 469 (M + H) | 3 |
| 236 | 2-chloro-N-[(cis-4-{[4-(dimethylamino)quinolin-2-yl]amino} cyclohexyl)methyl]-3,6-difluorobenzamide | 473 (M + H) | 3 |
| 237 | N-[(cis-4-{[4-(dimethylamino)quinolin-2-yl]amino}cyclohexyl) methyl]-2,3-dimethylbenzamide | 431 (M + H) | 3 |
| 238 | N-(cis-4-{[4-(dimethylamino)pyrimidin-2-yl]amino}cyclohexyl)-3-methoxybenzamide | 370 (M + H) | 2 |
| 239 | 3-bromo-N-(cis-4-{[4-(dimethylamino)pyrimidin-2-yl]amino} cyclohexyl)benzamide | 418 (M + H) | 1 |

(continued)

| Ex. No. | compound name | MS | class |
|---|---|---|---|
| 240 | 4-bromo-N-(cis-4-{[4-(dimethylamino)pyrimidin-2-yl]amino} cyclohexyl)benzamide | 418 (M + H) | 3 |
| 241 | N-(cis-4-{[4-(dimethylamino)pyrimidin-2-yl]amino}cyclohexyl)-2,1,3-benzoxadiazole-5-carboxamide | 382 (M + H) | 1 |
| 242 | 3-chloro-N-(cis-4-{[4-(dimethylamino)pyrimidin-2-yl]amino} cyclohexyl)benzamide | 374 (M + H) | 1 |
| 243 | 4-chloro-N-(cis-4-{[4-(dimethylamino)pyrimidin-2-yl]amino} cyclohexyl)benzamide | 374 (M + H) | 2 |
| 244 | (2E)-N-(cis-4-{[4-(dimethylamino)pyrimidin-2-yl]amino} cyclohexyl)-3-phenylacrylamide | 366 (M + H) | 3 |
| 245 | 4-chloro-N-(cis-4-{[4-(dimethylamino)pyrimidin-2-yl]amino} cyclohexyl)-3-nitrobenzamide | 419 (M + H) | 1 |
| 246 | 2-(4-chlorophenyl)-N-(cis-4-{[4-(dimethylamino)pyrimidin-2-yl] amino}cyclohexyl)acetamide | 388 (M + H) | 3 |
| 247 | 3-cyano-N-(cis-4-{[4-(dimethylamino)pyrimidin-2-yl]amino} cyclohexyl)benzamide | 365 (M + H) | 3 |
| 248 | 3,5-dichloro-N-(cis-4-{[4-(dimethylamino)pyrimidin-2-yl]amino} cyclohexyl)benzamide | 408 (M + H) | 1 |
| 249 | 3,4-dichloro-N-(cis-4-{[4-(dimethylamino)pyrimidin-2-yl]amino} cyclohexyl)benzamide | 408 (M + H) | 1 |
| 250 | N-(cis-4{[4-(dimethylamino)pyrimidin-2-yl]amino}cyclohexyl)-2,2-diphenylacetamide | 430 (M + H) | 2 |
| 251 | N-(cis-4-{[4-(dimethylamino)pyrimidin-2-yl]amino)cyclohexyl)-3,4-difluorobenzamide | 376 (M + H) | 1 |
| 252 | N-(cis-4-{[4-(dimethylamino)pyrimidin-2-yl]amino}cyclohexyl)-3,5-difluorobenzamide | 376 (M + H) | 2 |
| 253 | 2-(2,5-dimethoxyphenyl)-N-(cis-4-{[4-(dimethylamino)pyrimidin-2-yl]amino}cyclohexyl)acetamide | 414 (M + H) | 3 |
| 254 | N-(cis-4-{[4-(dimethylamino)pyrimidin-2-yl]amino}cyclohexyl)-2-(ethylthio)nicotinamide | 401 (M + H) | 3 |
| 255 | N-(cis-4-{[4-(dimethylamino)pyrimidin-2-yl]amino}cyclohexyl)-4-fluorobenzamide | 358 (M + H) | 3 |
| 256 | N-(cis-4-{[4-(dimethylamino)pyrimidin-2-yl]amino}cyclohexyl)-3-fluoro-5-(trifluoromethyl)benzamide | 426 (M + H) | 2 |
| 257 | 2,4-dichloro-N-(cis-4-{[4-(dimethylamino)pyrimidin-2-yl]amino} cyclohexyl)-5-fluorobenzamide | 426 (M + H) | 3 |
| 258 | N-(cis-4-{[4-(dimethylamino)pyrimidin-2-yl]amino}cyclohexyl) hexanamide | 334 (M + H) | 3 |
| 259 | N-(cis-4-{[4-(dimethylamino)pyrimidin-2-yl]amino}cyclohexyl)-4-iodobenzamide | 466 (M + H) | 3 |
| 260 | N-(cis-4-{[4-(dimethylamino)pyrimidin-2-yl]amino}cyclohexyl)-2-(methylthio)nicotinamide | 387 (M + H) | 3 |
| 261 | N-(cis-4-{[4-(dimethylamino)pyrimidin-2-yl]amino}cyclohexyl)-4-methyl-3-nitrobenzamide | 399 (M + H) | 2 |

(continued)

| Ex. No. | compound name | MS | class |
|---|---|---|---|
| 262 | N-(cis-4-{[4-(dimethylamino)pyrimidin-2-yl]amino}cyclohexyl)-3-nitrobenzamide | 385 (M + H) | 1 |
| 263 | N-(cis-4-{[4-(dimethylamino)pyrimidin-2-yl]amino}cyclohexyl)-2-phenylacetamide | 354 (M + H) | 3 |
| 264 | (2R)-N-(cis-4-{[4-(dimethylamino)pyrimidin-2-yl]amino}cyclohexyl)-2-phenylcyclopropanecarboxamide | 380 (M + H) | 3 |
| 265 | N-(cis-4-{(4-(dimethylamino)pyrimidin-2-yl]amino}cyclohexyl)-1,3-benzodioxole-5-carboxamide | 384 (M + H) | 3 |
| 266 | N-(cis-4-{[4-(dimethylamino)pyrimidin-2-yl]amino}cyclohexyl)-2-phenoxybutanamide | 398 (M + H) | 2 |
| 267 | N-(cis-4-{[4-(dimethylamino)pyrimidin-2-yl]amino}cyclohexyl)-2-phenoxypropanamide | 384 (M + H) | 3 |
| 268 | N-(cis-4-{[4-(dimethylamino)pyrimidin-2-yl]amino}cyclohexyl)-3-methylbenzamide | 354 (M + H) | 2 |
| 269 | N-(cis-4-{[4-(dimethylamino)pyrimidin-2-yl]amino}cyclohexyl)-4-methylbenzamide | 354 (M + H) | 3 |
| 270 | N-(cis-4-{[4-(dimethylamino)pyrimidin-2-yl]amino}cyclohexyl) thiophene-2-carboxamide | 346 (M+H) | 3 |
| 271 | N-(cis-4-{[4-(dimethylamino)pyrimidin-2-yl]amino}cyclohexyl)-2-(2-thienyl)acetamide | 360 (M + H) | 3 |
| 272 | N-(cis-4-{[4-(dimethylanzino)pyrimidin-2-yl]amino}cyclohexyl)-3-(trifluoromethoxy)benzamide | 424 (M + H) | 1 |
| 273 | [4-(4-Dimethylamino-pyrimidin-2-ylamino)-cyclohexyl]-carbamic acid benzyl ester | 370 (M + H) | 3 |
| 274 | [(4 (4 Dimethylamino-pyrimidin-2-ylamino)-cyclohexyl]-carbamic acid 4-nitro-benzyl ester | 415 (M + H) | 3 |
| 275 | 4-bromo-N-(cis-4-{[4-(dimethylamino)pyrimidin-2-yl]amino}cyclohexyl)-3-methylbenzamide | 432 (M + H) | 1 |
| 276 | N-(cis-4-{[4-(dimethylamino)pyrimidin-2-yl]amino)cyclohexyl)-3-iodobenzamide | 466 (M + H) | 1 |
| 277 | 3-chloro-N-(cis-4-{[4-(dimethylamino)pyrimidin-2-yl]amino}cyclohexyl)-2-fluorobenzamide | 392 (M + H) | 3 |
| 278 | N-(cis-4-{[4-(dimethylamino)pyrimidin-2-yl]amino]cyclohexyl)-2,3-difluoro-4-methylbenzamide | 390 (M + H) | 3 |
| 279 | 2-chloro-N-(cis-4-{[4-(dimethylamino)pyrimidin-2-yl]amino}cyclohexyl)-4-fluorobenzamide | 392 (M + H) | 3 |
| 280 | 3-chloro-N-(cis-4-{[4-(dimethylamino)pyrimidin-2-yl]amino}cyclohexyl)-2,4-difluorobenzamide | 410 (M + H) | 3 |
| 281 | N-(cis-4-{[4-(dimethylamino)pyrimidin-2-yl]amino}cyclohexyl)-2-(phenylthio)acetamide | 386 (M + H) | 3 |
| 282 | N-(cis-4-{[4-(dimethylamino)pyrimidin-2-yl]amino}cyclohexyl)-2-fluoro-3-(trifluoromethyl)benzamide | 426 (M + H) | 3 |
| 283 | N-(cis-4-{[4-(dimethylamino)pyrimidin-2-yl]amino}cyclohexyl)-2-fluoro-5-(trifluoromethyl)benzamide | 426 (M + H) | 3 |

(continued)

| Ex. No. | compound name | MS | class |
|---|---|---|---|
| 284 | N-(cis-4-{[4-(dimethylamino)pyrimidin-2-yl]amino}cyclohexyl)-2-phenylbutanamide | 382 (M + H) | 3 |
| 285 | N-(cis-4-{[4-(dimethylamino)pyrimidin-2-yl]amino}cyclohexyl)-2-(3-methoxyphenyl)acetamide | 384 (M + H) | 3 |
| 286 | N-(cis-4-{[4-(dimethylamino)pyrimidin-2-yl]amino}cyclohexyl)-2-(4-fluorophenyl)acetamide | 372 (M + H) | 3 |
| 287 | N-(cis-4-{[4-(dimethylamino)pyrimidin-2-yl]amino}cyclohexyl)-2-(4-methoxyphenyl)acetamide | 384 (M + H) | 3 |
| 288 | N-(cis-4-{[4-(dimethylamino)pyrimidin-2-yl]amino}cyclohexyl)-5-methyl-2-(trifluoromethyl)-3-furamide | 412 (M + H) | 3 |
| 289 | N-(cis-4-{[4-(dimethylamino)pyrimidin-2-yl]amino}cyclohexyl)-2,5-dimethyl-3-furamide | 358 (M + H) | 2 |
| 290 | N-(cis-4-{[4-(dimethylamino)pyrimidin-2-yl]amino}cyclohexyl)-2-ethoxybenzamide | 384 (M + H) | 3 |
| 291 | 3-chloro-N-(cis-4-{[4-(dimethylamino)pyrimidin-2-yl]amino}cyclohexyl)-4-fluorobenzamide | 392 (M + H) | 1 |
| 292 | N-(cis-4-{[4-(dimethylamino)pyrimidin-2-yl]amino}cyclohexyl)-3-fluoro-4-methylbenzamide | 372 (M + H) | 3 |
| 293 | 2-cyclopentyl-N-(cis-4-{[4-(dimethylamino)pyrimidin-2-yl]amino}cyclohexyl)acetamide | 346 (M + H) | 3 |
| 294 | N-(cis-4-{[4-(dimethylamino)pyrimidin-2-yl]amino}cyclohexyl)-3,5-dimethoxybenzamide | 400 (M + H) | 1 |
| 295 | 4-cyano-N-(cis-4-{[4-(dimethylamino)pyrimidin-2-yl]amino}cyclohexyl)benzamide | 365 (M + H) | 3 |
| 296 | N-(cis-4-{[4-(dimethylamino)pyrimidin-2-yl]amino}cyclohexyl)-3,5-bis(trifluoromethyl)benzamide | 476 (M + H) | 1 |
| 297 | (2E)-N-(cis-4-{[4-(dimethylamino)pyrimidin-2-yl]amino}cyclohexyl)-3-(4-nitrophenyl)acrylamide | 411 (M + H) | 3 |
| 298 | 2-(2-bromophenyl)-N-(cis-4-{[4-(dimethylamino)pyrimidin-2-yl]amino}cyclohexyl)acetamide | 432 (M + H) | 3 |
| 299 | N-(cis-4-{[4-(dimethylamino)pyrimidin-2-yl]amino}cyclohexyl)-4-fluoro-3-methylbenzamide | 372 (M + H) | 1 |
| 300 | 2-[(difluoromethyl)thio]-N-(cis-4-{[4-(dimethylamino)pyrimidin-2-yl]amino}cyclohexyl)benzamide | 422 (M + H) | 3 |
| 301 | 2,5-dichloro-N-(cis-4-{[4-(dimethylamino)pyrimidin-2-yl]amino}cyclohexyl)thiophene-3-carboxamide | 414 (M + H) | 2 |
| 302 | N-(cis-4-{[4-(dimethylamino)pyrimidin-2-yl]amino}cyclohexyl)-2-(propylthio)nicotinamide | 415 (M + H) | 3 |
| 303 | 1-benzyl-3-tert-butyl-N-(cis-4-{[4-(dimethylamino)pyrimidin-2-yl]amino}cyclohexyl)-1H-pyrazole-5-carboxamide | 476 (M + H) | 2 |
| 304 | 3-tert-butyl-N-(cis-4-{[4-(dimethylamino)pyrimidin-2-yl]amino}cyclohexyl)-1-methyl-1H-pyrazole-5-carboxamide | 400 (M + H) | 3 |
| 305 | (2E)-N-(cis-4-{[4-(dimethylamino)pyrimidin-2-yl]amino}cyclohexyl)-2-methyl-3-phenylacrylamide | 380 (M + H) | 3 |

(continued)

| Ex. No. | compound name | MS | class |
|---|---|---|---|
| 306 | 5-bromo-N-(cis-4-{[4-(dimethylamino)pyrimidin-2-yl]amino} cyclohexyl)nicotinamide | 419 (M + H) | 3 |
| 307 | N-(cis-4-{[4-(dimethylamino)pyrimidin-2-yl]amino}cyclohexyl)-2-(1-naphthyl)acetamide | 404 (M + H) | 2 |
| 308 | 1-tert-butyl-N-(cis-4-{[4-(dimethylamino)pyrimidin-2-yl]amino} cyclohexyl)-5-methyl-1H-pyrazole-3-carboxamide | 400 (M + H) | 3 |
| 309 | N-(cis-4-{[4-(dimethylamino)pyrimidin-2-yl]amino}cyclohexyl)-1-benzothiophene-3-carboxamide | 396 (M + H) | 3 |
| 310 | 2-[(cis-4-{[4-(dimethylamino)pyrimidin-2-yl]amino}cyclohexyl) amino]-2-oxo-1-phenylethyl acetate | 412 (M + H) | 3 |
| 311 | N-(cis-4-{[4-(dimethylamino)pyrimidin-2-yl]amino}cyclohexyl) benzamide | 340 (M + H) | 3 |
| 312 | N-(cis-4-{[4-(dimethylamino)pyrimidin-2-yl]amino}cyclohexyl)-1-benzothiophene-2-carboxamide | 396 (M + H) | 3 |
| 313 | 2-(benzyloxy)-N-(cis-4-{[4-(dimethylamino)pyrimidin-2-yl]amino} cyclohexyl)acetamide | 384 (M + H) | 3 |
| 314 | 2-(4-chlorophenoxy)-N-(cis-4-{[4-(dimethylamino)pyrimidin-2-yl] amino}cyclohexyl)acetamide | 404 (M + H) | 1 |
| 315 | N-(cis-4-{[4-(dimethylamino)pyrimidin-2-yl]amino}cyclohexyl) cyclohexanecarboxamide | 346 (M + H) | 3 |
| 316 | 3-(2-chlorophenyl)-N-(cis-4-{[4-(dimethylamino)pyrimidin-2-yl] amino}cyclohexyl)-5-methylisoxazole-4-carboxamide | 455(M + H) | 3 |
| 317 | 1-(4-chlorophenyl)-N-(cis-4-{[4-(dimethylamino)pyrimidin-2-yl] amino}cyclohexyl)cyclopentanecarboxamide | 442 (M + H) | 2 |
| 318 | 3-(2-chloro-6-fluorophenyl)-N-(cis-4-{[4-(dimethylamino) pyrimidin-2-yl]amino}cyclohexyl)-5-methylisoxazole-4-carboxamide | 473 (M + H) | 2 |
| 319 | 3-chloro-N-(cis-4-{[4-(dimethylamino)pyrimidin-2-yl]amino} cyclohexyl)-4-(isopropylsulfonyl)thiophene-2-carboxamide | 486 (M + H) | 3 |
| 320 | 2-chloro-N-(cis-4-{[4-(dimethylamino)pyrimidin-2-yl]amino} cyclohexyl)-4-nitrobenzamide | 419 (M + H) | 3 |
| 321 | N-(cis-4-{[4-(dimethylamino)pyrimidin-2-yl]amino}cyclohexyl)-1,3-dimethyl-1H-pyrazole-5-carboxamide | 358 (M + H) | 3 |
| 322 | N-(cis-4-{[4-(dimethylamino)pyrimidin-2-yl]amino}cyclohexyl)-3,4-dimethoxybenzamide | 400 (M + H) | 3 |
| 323 | N-(cis-4-{[4-(dimethylamino)pyrimidin-2-yl]amino}cyclohexyl)-3-fluorobenzamide | 358 (M + H) | 3 |
| 324 | N-(cis-4-{[4-(dimethylamino)pyrimidin-2-yl]amino}cyclohexyl)-4-fluoro-3-(trifluoromethyl)benzamide | 426 (M + H) | 1 |
| 325 | N-(cis-4-{(4-(dimethylamino)pyrimidin-2-yl]amino}cyclohexyl)-5-methyl-2-phenyl-2H-1,2,3-triazole-4-carboxamide | 421 (M + H) | 1 |
| 326 | N-(cis-4-{[4-(dimethylamino)pyrimidin-2-yl]amino}cyclohexyl)-2-(4-methoxyphenoxy)-5-nitrobenzamide | 507 (M + H) | 1 |
| 327 | N-(cis-4-{[4-(dimethylamino)pyrimidin-2-yl]amino}cyclohexyl)-1-naphthamide | 390 (M + H) | 3 |

(continued)

| Ex. No. | compound name | MS | class |
|---|---|---|---|
| 328 | N-(cis-4-{[4-(dimethylamino)pyrimidin-2-yl]amino}cyclohexyl)-2-naphthamide | 390 (M + H) | 3 |
| 329 | N-(cis-4-{[4-(dimethylamino)pyrimidin-2-yl]amino}cyclohexyl)-5-nitro-2-furamide | 375 (M + H) | 3 |
| 330 | N-(cis-4-{[4-(dimethylamino)pyrimidin-2-yl]amino}cyclohexyl)-2-phenoxyacetamide | 370 (M + H) | 2 |
| 331 | N-(cis-4-{[4-(dimethylamino)pyrimidin-2-yl]amino}cyclohexyl)-2-(2-nitrophenoxy)acetamide | 415 (M + H) | 3 |
| 332 | N-(cis-4-{[4-(dimethylamino)pyrimidin-2-yl]amino}cyclohexyl) quinoxaline-2-carboxamide | 392 (M + H) | 1 |
| 333 | N-(cis-4-{[4-(dimethylamino)pyrimidin-2-yl]amino}cyclohexyl)-3,4,5-trimethoxybenzamide | 430 (M + H) | 3 |
| 334 | N-(cis-4-{[4-(dimethylamino)pyrimidin-2-yl]amino}cyclohexyl)-3-(trifluoromethyl)benzamide | 408 (M + H) | 2 |
| 335 | N-(cis-4-{[4-(dimethylamino)pyrimidin-2-yl]amino}cyclohexyl)-4-(trifluoromethyl)benzamide | 408 (M + H) | 3 |
| 336 | N-(cis-4-{(4-(dimethylamino)pyrimidin-2-yl]amino}cyclohexyl)-2-(trifluoromethoxy)benzamide | 424 (M + H) | 3 |
| 337 | 4,5-dimethoxy-2-nitrobenzyl (cis-4-{[4-(dimethylamino)pyrimidin-2-yl]amino}cyclohexyl)carbamate | 475 (M + H) | 3 |
| 338 | N-(cis-4-{[4-(dimethylamino)pyrimidin-2-yl]amino}cyclohexyl)-4-phenoxybutanamide | 398 (M + H) | 3 |
| 339 | 2-bromo-N-(cis-4-{[4-(dimethylamino)pyrimidin-2-yl]amino} cyclohexyl)-5-methoxybenzamide | 448 (M + H) | 3 |
| 340 | N-(cis-4-{[4-(dimethylamino)pyrimidin-2-yl]amino}cyclohexyl)-2-(pentafluorophenoxy)acetamide | 460 (M + H) | 2 |
| 341 | 2-(3,4-dimethoxyphenyl)-N-(cis-4-{[4-(dimethylamino)pyrimidin-2-yl]amino}cyclohexyl)acetamide | 414 (M + H) | 3 |
| 342 | N-(cis-4-{[4-(dimethylamino)pyrimidin-2-yl]amino}cyclohexyl)-2,3,4-trifluorobenzamide | 394 (M + H) | 3 |
| 343 | N-(cis-4-{[4-(dimethylamino)pyrimidin-2-yl]amino}cyclohexyl) cyclopentanecarboxamide | 332 (M + H) | 3 |
| 344 | N-(cis-4-{[4-(dimethylamino)pyrimidin-2-yl]amino}cyclohexyl)-2,4-difluorobenzamide | 376 (M + H) | 3 |
| 345 | N-(cis-4-{[4-(dimethylamino)pyrimidin-2-yl]amino}cyclohexyl)-3-phenylpropanamide | 368 (M + H) | 3 |
| 346 | N-(cis-4-{[4-(dimethylamino)pyrimidin-2-yl]amino}cyclohexyl)-2,3,4,5-tetrafluorobenzamide | 412 (M + H) | 3 |
| 347 | N-(cis-4-{[4-(dimethylamino)pyrimidin-2-yl]amino}cyclohexyl)-2-ethoxy-1-naphthamide | 434 (M + H) | 3 |
| 348 | N-(cis-4-{[4-(dimethylamino)pyrimidin-2-yl]amino}cyclohexyl)-2,3,4,5,6-pentafluorobenzamide | 430 (M + H) | 3 |
| 349 | N-(cis-4-{[4-(dimethylamino)pyrimidin-2-yl]amino}cyclohexyl)-4-[(trifluoromethyl)thio]benzamide | 440 (M + H) | 3 |

(continued)

| Ex. No. | compound name | MS | class |
|---|---|---|---|
| 350 | 3,4,5-trichloro-N-(cis-4-{[4-(dimethylamino)pyrimidin-2-yl]amino} cyclohexyl)thiophene-2-carboxamide | 448 (M + H) | 3 |
| 351 | 2-(3-chlorophenoxy)-N-(cis-4-{[4-(dimethylamino)pyrimidin-2-yl] amino}cyclohexyl)acetamide | 404 (M + H) | 1 |
| 352 | 3-(2,6-dichlorophenyl)-N-(cis-4-{[4-(dimethylamino)pyrimidin-2-yl] amino}cyclohexyl)-5-methylisoxazole-4-carboxamide | 489 (M + H) | 1 |
| 353 | N-(cis-4-{[4-(dimethylamino)pyrimidin-2-yl]amino}cyclohexyl)-2-phenoxynicotinamide | 433 (M + H) | 2 |
| 354 | N-(cis-4-{[4-(dimethylamino)pyrimidin-2-yl]amino}cyclohexyl)-2-(phenylthio)nicotinamide | 449 (M + H) | 3 |
| 355 | N-(cis-4-{[4-(dimethylamino)pyrimidin-2-yl]amino}cyclohexyl)-2-(4-methylphenoxy)nicotinamide | 447 (M + H) | 1 |
| 356 | N-(cis-4-{(4-(dimethylamino)pyrimidin-2-yl]amino}cyclohexyl)-4-[(dipropylamino)sulfonyl]benzamide | 503 (M + H) | 1 |
| 357 | 2-(4-chlorophenoxy)-N-(cis-4-{[4-(dimethylamino)pyrimidin-2-yl] amino}cyclohexyl)-2-methylpropanamide | 432 (M + H) | 2 |
| 358 | 5-(4-chlorophenyl)-N-(cis-4-{[4-(dimethylamino)pyrimidin-2-yl) amino}cyclohexyl)-2-(trifluoromethyl)-3-furamide | 508 (M + H) | 3 |
| 359 | 2-(2,3-dihydro-1-benzofuran-5-yl)-N-(cis-4-{[4-(dimethylamino) pyrimidin-2-yl]amino}cyclohexyl)-1,3-thiazole-4-carboxamide | 465 (M + H) | 1 |
| 360 | 3-tert-butyl-1-(2,4-dichlorobenzyl)-N-(cis-4-([4-(dimethylamino) pyrimidin-2-yl]amino}cyclohexyl)-1H-pyrazole-5-carboxamide | 544 (M + H) | 2 |
| 361 | 6-chloro-N-(cis-4-{[4-(dimethylamino)pyrimidin-2-yl]amino} cyclohexyl)-2H-chromene-3-carboxamide | 428 (M + H) | 2 |
| 362 | 3-chloro-N-(cis-4-{[4-(dimethylamino)pyrimidin-2-yl]amino} cyclohexyl)-4-(trifluoromethoxy)benzamide | 458 (M + H) | 3 |
| 363 | N-(cis-4-{[4-(dimethylamino)pyrimidin-2-yl]amino}cyclohexyl)-2-[(4-methyl-2-oxo-2H-ehromen-8-yl)oxy]acetamide | 452 (M + H) | 3 |
| 364 | N-(cis-4-([4-(dimethylamino)pyrimidin-2-yl]amino}cyclohexyl)-2-(2-thienyl)-1,3-thiazole-4-carboxamide | 429 (M + H) | 1 |
| 365 | N-((cis-4-([4-(dimethylamino)pyrimidin-2-yl]amino cyclohexyl) methyl)-3-methoxybenzamide | 384 (M + H) | 3 |
| 366 | 3-bromo-N-[(cis-4-{[4-(dimethylamino)pyrimidin-2-yl]amino} cyclohexyl)methyl]benzamide | 432(M + H) | 3 |
| 367 | 4-bromo-N-[(cis-4-{[4-(dimethylamino)pyrimidin-2-yl]amino} cyclohexyl)methyl]benzamide | 432 (M + H) | 3 |
| 368 | N-[(cis-4-( {[4-(dimethylamino)pyrimidin-2-yl]amino)cyclohexyl) methyl]-2,1,3-benzoxadiazole-5-carboxamide | 396 (M + H) | 3 |
| 369 | 3-chloro-N-[(cis-4-3-(4-(dimethylamino)pyrimidin-2-yl]amino} cyclohexyl)methyl)benzamide | 388 (M + H) | 3 |
| 370 | 4-chloro-N-[(cis-4-{[4-(dimethylamino)pyrimidin-2-yl]amino} cyclohexyl)methyl]benzamide | 388 (M + H) | 2 |
| 371 | (2E)-N-[(cis-4-{[4-(dimethylamino)pyrimidin-2-yl]amino} cyclohexyl)methyl]-3-phenylacrylamide | 380 (M + H) | 2 |

(continued)

| Ex. No. | compound name | MS | class |
|---|---|---|---|
| 372 | 4-chloro-N-[(cis-4-{[4-(dimethylamino)pyrimidin-2-yl]amino} cyclohexyl)methyl]-3-nitrobenzamide | 433 (M + H) | 2 |
| 373 | 2-(4-chlorophenyl)-N-[(cis-4-{[4-(dimethylamino)pyrimidin-2-yl] amino)cyclohexyl)methyl]acetamide | 402 (M + H) | 2 |
| 374 | 3-cyano-N-[(cis-4-{[4-(dimethylamino)pyrimidin-2-yl]amino} cyclohexyl)methyl]benzamide | 379 (M + H) | 3 |
| 375 | 3,5-dichloro-N-[(cis-4-{[4-(dimethylamino)pyrimidin-2-yl]amino} cyclohexyl)methyl]benzamide | 422 (M + H) | 2 |
| 376 | 3,4-dichloro-N-[(cis-4-{[4-(dimethylamino)pyrimidin-2-yl]amino cyclohexyl)methyl]benzamide | 422 (M + H) | 2 |
| 377 | N-[(cis-4-{[4-(dimethylamino)pyrimidin-2-yl]amino}cyclohexyl) methyl]-2,2-diphenylacetamide | 444 (M + H) | 1 |
| 378 | N-[(cis-4-{[4-(dimethylamino)pyrimidin-2-yl]amino}cyclohexyl) methyl]-3,4-difluorobenzamide | 390 (M + H) | 3 |
| 379 | N-[(cis-4-{[4-(dimethylamino)pyrimidin-2-yl]amino}cyclohexyl) methyl]-3,5-difluorobenzamide | 390 (M + H) | 3 |
| 380 | -(2,5-dimethoxyphenyl)-N-[(cis-4-{[4-(dimethylamino)-pyrimidin-2-yl]amino}cyclohexyl)methyl]acetamide | 428 (M + H) | 3 |
| 381 | -[(cis-4-{[4-(dimethylamino)pyrimidin-2-yl]amino}cyclohexyl) methyl]-2-(ethylthio)nicotinamide | 415 (M + H) | 3 |
| 382 | N-[(cis-4-{[4-(dimethylamino)pyrimidin-2-yl]amino}cyclohexyl) methyl]-4-fluorobenzamide | 372 (M + H) | 3 |
| 383 | -[(cis-4-{[4-(dimethylamino)pyrimidin-2-yl]amino}cyclohexyl)-methyl]-3-fluoro-5-(trifluoromethyl)benzamide | 440 (M + H) | 3 |
| 384 | 2,4-dichloro-N-[(cis-4-{[4-(dimethylamino)pyrimidin-2-yl]amino} cyclohexyl)methyl]-5-fluorobenzamide | 440 (M + H) | 2 |
| 385 | N-[(cis-4-{[4-(dimethylamino)pyrimidin-2-yl]amino}cyclohexyl) methyl]hexanamide | 348 (M + H) | 3 |
| 386 | N-[(cis-4-{[4-(dimethylamino)pyrimidin-2-yl]amino}cyclohexyl) methyl]-4-iodobenzamide | 480 (M + H) | 3 |
| 387 | N-[(cis-4-{[4-(dimethylamino)pyrimidin-2-yl]aminocyclohexyl) methyl]-2-(methylthio)nicotinamide | 401 (M + H) | 3 |
| 388 | N-[(cis-4-{[4-(dimethylamino)pyrimidin-2-yl]amino}cyclohexyl) methyl]-4-methyl-3-nitrobenzamide | 413 (M + H) | 3 |
| 389 | N-[(cis-4-{[4-(dimethylamino)pyrimidin-2-yl]amino}cyclohexyl) methyl]-3-nitrobenzamide | 399 (M + H) | 3 |
| 390 | N-[(cis-4-{[4-(dimethylamino)Pyrimidin-2-yl]amino}cyclohexyl) methyl]-2-phenylacetamide | 368 (M + H) | 3 |
| 391 | (2R)-N-[(cis-4-{[4-(dimethylamino)pyrimidin-2-yl]jamino} cyclohexyl)methyl]-2-phenylcyclopropanecarboxamide | 394 (M + H) | 3 |
| 392 | N-[(cis-4-{[4-(dimethylamino)pyrimidin-2-yl]amino}cyclohexyl) methyl]-1,3-benzodioxole-5-carboxamide | 398 (M + H) | 3 |
| 393 | N-[(cis-4-{[4-(dimethylamino)pyrimidin-2-yl]amino}cyclohexyl) methyl]-2-phenoxybutanamide | 412 (M + H) | 2 |

(continued)

| Ex. No. | compound name | MS | class |
|---|---|---|---|
| 394 | N-[(cis-4-{[4-(dimethylamino)pyrimidin-2-yl]amino}cyclohexyl) methyl]-2-phenoxypropanamide | 398 (M + H) | 3 |
| 395 | N-[(cis-4-{[4-(dimethylamino)pyrimidin-2-yl]amino}cyclohexyl) methyl]-3-methylbenzamide | 368 (M + H) | 3 |
| 396 | N-[(cis-4-{[4-(dimethylamino)pyrimidin-2-yl]amino}cyclohexyl) methyl]-4-methylbenzamide | 368 (M + H) | 3 |
| 397 | N-[(cis-4-{[4-(dimethylamino)pyrimidin-2-yl]amino}cyclohexyl) methyl]thiophene-2-carboxamide | 360 (M + H) | 3 |
| 398 | N-[(cis-4-{[4-(dimethylamino)pyrimidin-2-yl]amino}cyclohexyl) methyl]-2-(2-thienyl)acetamide | 374 (M + H) | 3 |
| 399 | N-[(cis-4-{[4-(dimethylamino)pyrimidin-2-yl]amino}cyclohexyl) methyl]-3-(trifluoromethoxy)benzamide | 438 (M + H) | 3 |
| 400 | benzyl [(cis-4-{[4-(dimethylamino)pyrimidin-2-yl]amino} cyclohexyl)methyl]carbamate | 384 (M + H) | 3 |
| 401 | 4-nitrobenzyl [(cis-4-{[4-(dimethylamino)pyrimidin-2-yl]amino} cyclohexyl)methyl]carbamate | 429 (M + H) | 3 |
| 402 | 4-bromo-N-[(cis-4-{(4-(dimethylamino)pyrimidin-2-yl]amino} cyclohexyl)methyl]-3-methylbenzamide | 446 (M + H) | 3 |
| 403 | N-[(cis-4-{[4-(dimethylamino)pyrimidin-2-yl]amino}cyclohexyl) methyl]-3-iodobenzamide | 480 (M + H) | 3 |
| 404 | 3-chloro-N-[(cis-4-{[4-(dimethylamino)pyrimidin-2-yl]amino} cyclohexyl)methyl]-2-fluorobenzamide | 406 (M + H) | 3 |
| 405 | N-[(cis-4-{[4-(dimethylamino)pyrimidin-2-yl)amino}cyclohexyl) methyl]-2,3-difluoro-4-methylbenzamide | 404 (M + H) | 3 |
| 406 | 2-chloro-N-[(cis-4-{(4-(dimethylamino)pyrimidin-2-yl]amino} cyclohexyl)methyl]-4-fluorobenzamide | 406 (M + H) | 3 |
| 407 | 3-chloro-N-[(cis-4-{[4-(dimethylamino)pyrimidin-2-yl]amino} cyclohexyl)methyl]-2,4-difluorobenzamide | 424 (M + H) | 3 |
| 408 | N-[(cis-4-{[4-(dimethylamino)pyrimidin-2-yl]amino}cyclohexyl) methyl]-2-(phenylthio)acetamide | 400 (M + H) | 3 |
| 409 | N-[(cis-4-{[4-(dimethylamino)pyrimidin-2-yl]amino}cyclohexyl)-methyl]-2-fluoro-3-(trifluoromethyl)benzamide | 440 (M + H) | 3 |
| 410 | N-[(cis-4-{[4-(dimethylamino)pyrimidin-2-yl]amino}cyclohexyl)-methyl]-2-fluoro-5-(trifluoromethyl)benzamide | 440 (M + H) | 3 |
| 411 | N-[(cis-4-{[4-(dimethylamino)pyrimidin-2-yl]amino}cyclohexyl) methyl]-2-phenylbutanamide | 396 (M + H) | 1 |
| 412 | N-[(cis-4-{[4-(dimethylamino)pyrimidin-2-yl]amino}cyclohexyl) methyl]-2-(3-methoxyphenyl)acetamide | 398 (M + H) | 2 |
| 413 | N-[(cis-4-{[4-(dimethylamino)pyrimidin-2-yl]amino}cyclohexyl) methyl]-2-(4-fluorophenyl)acetamide | 386 (M + H) | 3 |
| 414 | N-[(cis-4-{[4-(dimethylamino)pyrimidin-2-yl]amino}cyclohexyl) methyl]-2-(4-methoxyphenyl)acetamide | 398 (M + H) | 2 |
| 415 | N-[(cis-4-{[4-(dimethylamino)pyrimidin-2-yl]amino}cyclohexyl)-methyl]-5-methyl-2-(trifluoromethyl)-3-furamide | 426 (M + H) | 3 |

(continued)

| Ex. No. | compound name | MS | class |
|---|---|---|---|
| 416 | N-[(cis-4-{[4-(dimethylamino)pyrimidin-2-yl]amino}cyclohexyl) methyl]-2,5-dimethyl-3-furamide | 372 (M + H) | 3 |
| 417 | N-[(cis-4-{[4-(dimethylamino)pyrimidin-2-yl]amino}cyclohexyl) methyl]-2-ethoxybenzamide | 398 (M + H) | 3 |
| 418 | 3-chloro-N-[(cis-4-{[4-(dimethylamino)pyrimidin-2-yl]amino} cyclohexyl)methyl]-4-fluorobenzamide | 406 (M + H) | 3 |
| 419 | N-[(cis-4-{[4-(dimethylamino)pyrimidin-2-yl]amino}cyclohexyl) methyl]-3-fluoro-4-methylbenzamide | 386 (M + H) | 3 |
| 420 | 2-cyclopentyl-N-[(cis-4-{[4-(dimethylamino)pyrimidin-2-yl]amino} cyclohexyl)methyl]acetamide | 360 (M + H) | 3 |
| 421 | N-[(cis-4-{[4-(dimethylamino)pyrimidin-2-yl]amino}cyclohexyl) methyl]-3,5-dimethoxybenzamide | 414 (M + H) | 3 |
| 422 | 4-cyano-N-[(cis-4-{[4-(dimethylamino)pyrimidin-2-yl]amino} cyclohexyl)methyl]benzamide | 379 (M + H) | 3 |
| 423 | N-[(cis-4-{[4-(dimethylamino)pyrimidin-2-yl]amino}cyclobexyl) methyl]-3,5-bis(trifluoromethyl)benzamide | 490 (M + H) | 2 |
| 424 | (2E)-N-[(cis-4-{[4-(dimethylamino)pyrimidin-2-yl]amino} cyclohexyl)methyl]-3-(4-nitrophenyl)acrylamide | 425 (M + H) | 1 |
| 425 | 2-(2-bromophenyl)-N-[(cis-4-{[4-(dimethylamino)pyrimidin-2-yl] amino}cyclohexyl)methyl]acetamide | 446 (M + H) | 2 |
| 426 | N-[(cis-4-{[4-(dimethylamino)pyrimidin-2-yl]amino}cyclohexyl) methyl]-4-fluoro-3-methylbenzamide | 386 (M + H) | 3 |
| 427 | 2-[(difluoromethyl)thio]-N-[(cis-4-{[4-(dimethylamino)-pyrimidin-2-yl]amino)cyclohexyl)methyl]benzamide | 436 (M + H) | 3 |
| 428 | 2,5-dichloro-N-[(cis-4-{[4-(dimethylamino)pyrimidin-2-yl]amino} cyclohexyl)methyl]thiophene-3-carboxamide | 428 (M + H) | 3 |
| 429 | N-((cis-4-{[4-(dimethylamino)pyrimidin-2-yl]amino}cyclohexyl) methyl]-2-(propylthio)nicotinamide | 429 (M + H) | 2 |
| 430 | 1-benzyl-3-tert-butyl-N-[(cis-4-{[4-(dimethylamino)pyrimidin-2-yl] amino}cyclohexyl)methyl]-1 H-pyrazole-5 -carboxamide | 490 (M + H) | 3 |
| 431 | 3-tert-butyl-N-[(cis-4-{[4-(dimethylamino)-pyrimidin-2-yl]amino} cyclohexyl)methyl]-1-methyl-1H-pyrazole-5-carboxamide | 414 (M + H) | 3 |
| 432 | (2E)-N-[(cis-4-{[4-(dimethylamino)pyrimidin-2-yl]amino) cyclohexyl)methyl]-2-methyl-3-phenylacrylamide | 394 (M + H) | 3 |
| 433 | 5-bromo-N-[(cis-4-{[4-(dimethylamino)pyrimidin-2-yl]amino} cyclohexyl)methyl]nicotinamide | 433 (M + H) | 3 |
| 434 | N-[(cis-4-{[4-(dimethylamino)pyrimidin-2-yl]amino}cyclohexyl) methyl]-2-(1-naphthyl)acetamide | 418 (M + H) | 1 |
| 435 | 1-tert-butyl-N-[(cis-4-{(4-(dimethylamino)pyrimidin-2-yl]amino} cyclohexyl)methyl]-5-methyl-1H-pyrazole-3-carboxamide | 414 (M + H) | 3 |
| 436 | N-[(cis-4-{[4-(dimethylamino)pyrimidin-2-yl]amino}cyclohexyl) methyl]-1-benzothiophene-3-carboxamide | 410 (M + H) | 3 |
| 437 | N-[(cis-4-{[4-(dimethylamino)pyrimidin-2-yl]amino}cyclohexyl) methyl]biphenyl-4-carboxamide | 430 (M + H) | 3 |

(continued)

| Ex. No. | compound name | MS | class |
|---|---|---|---|
| 438 | 2-bromo-N-[(cis-4-{[4-(dimethylamino)pyrimidin-2-yl]amino} cyclohexyl)methyl]benzamide | 432 (M + H) | 3 |
| 439 | 2,6-dichloro-N-[(cis-4-{[4-(dimethylamino)pyrimidin-2-yl]amino} cyclohexyl)methyl]benzamide | 422 (M + H) | 3 |
| 440 | N-[(cis-4-{[4-(dimethylamino)pyrimidin-2-yl]amino}cyclohexyl) methyl]-2-iodobenzamide | 480 (M + H) | 3 |
| 441 | N-[(cis-4-{[4-(dimethylamino)pyrimidin-2-yl]amino}cyclohexyl) methyl]-2-methylbenzamide | 368 (M + H) | 3 |
| 442 | 2,3-dichloro-N-[(cis-4-{[4-(dimethylamino)pyrimidin-2-yl]amino} cyclohexyl)methyl]benzamide | 422 (M + H) | 3 |
| 443 | 2-chloro-N-[(cis-4-{[4-(dimethylamino)pyrimidin-2-yl]amino} cyclohexyl)methyl]-5-fluorobenzamide | 406 M + H | 3 |
| 444 | N-[(cis-4-{[4-(dimethylamino)pyrimidin-2-yl]amino}cyclohexyl) methyl]-9-oxo-9H-fluorene-4-carboxamide | 456 (M + H) | 2 |
| 445 | N-[(cis-4-{[4-(dimethylamino)pyrimidin-2-yl]amino}cyclohexyl) methyl]-2,3,6-trifluorobenzamide | 408 (M + H) | 3 |
| 446 | N-[(cis-4-{[4-(dimethylamino)pyrimidin-2-yl]amino}cyclohexyl) methyl)-2,3-difluorobenzamide | 390 (M + H) | 3 |
| 447 | N-[(cis-4-{[4-(dimethylamino)pyrimidin-2-yl]amino}cyclohexyl) methyl]-2,6-difluorobenzamide | 390 (M + H) | 3 |
| 448 | N-[(cis-4-{[4-(dimethylamino)pyrimidin-2-yl]amino}cyclohexyl)-methyl]-2-fluoro-6-(trifluoromethyl)benzamide | 440 (M + H) | 3 |
| 449 | N-[(cis-4-{[4-(dimethylamino)pyrimidin-2-yl)amino}cyclohexyl) methyl)-2,4,6-trimethylbenzamide | 396 (M + H) | 2 |
| 450 | 2-chloro-N-[(cis-4-{[4-(dimethylamino)pyrimidin-2-yl]amino} cyclohexyl)methyl]-6-fluorobenzamide | 406 (M + H) | 3 |
| 451 | 2,4,6-trichloro-N-[(cis-4-{[4-(dimethylamino)pyrimidin-2-yl]amino} cyclohexyl)methyl]benzamide | 456 (M + H) | 2 |
| 452 | (2E)-3-(2-chlorophenyl)-N-[(cis-4-{[4-(dimethylamino)pyrimidin-2-yl]amino}cyclohexyl)methyl]acrylamide | 414 (M + H) | 2 |
| 453 | 6-chloro-N-[(cis-4-{[4-(dimethylamino)pyrimidin-2-yl]amino} cyclohexyl)methyl]-2-fluoro-3-methylbenzamide | 420 (M + H) | 3 |
| 454 | 2-chloro-N-[(cis-4-{[4-(dimethylamino)pyrimidin-2-yl]amino} cyclohexyl)methyl]-3,6-difluorobenzamide | 424 (M + H) | 3 |
| 455 | N-[(cis-4-{[4-(dimethylamino)pyrimidin-2-yl]amino}cyclohexyl) methyl]-2,3-dimethylbenzamide | 382 (M + H) | 3 |
| 456 | N-(cis-4-{[4-(dimethylamino)-5,6,7,8-tetrahydroquinazolin-2-yl] amino}cyclohexyl)-3-methoxybenzamide | 424 (M + H) | 1 |
| 457 | 3-bromo-N-(cis-4-{[4-(dimethylamino)-5,6,7,8-tetrahydroquinazolin-2-yl]amino}cyclohexyl)benzamide | 472 (M + H) | 1 |
| 458 | 4-bromo-N-(cis-4-{[4-(dimethylamino)-5,6,7,8-tetrahydroquinazolin-2-yl]amino}cyclohexyl)benzamide | 472 (M + H) | 2 |
| 459 | N-(cis-4-{[4-(dimethylamino)-5,6,7,8-tetrahydroquinazolin-2-yl] amino}cyclohexyl)-2,1,3-benzoxadiazole-5-carboxamide | 436 (M + H) | 1 |

(continued)

| Ex. No. | compound name | MS | class |
|---|---|---|---|
| 460 | 3-chloro-N-(cis-4-{[4-(dimethylamino)-5,6,7,8-tetrahydroquinazolin-2-yl]amino}cyclohexyl)benzamide | 428 (M + H) | 1 |
| 461 | 4-chloro-N-(cis-4-{[4-(dimethylamino)-5,6,7,8-tetrahydroquinazolin-2-yl]amino}cyclohexyl)benzamide | 428 (M + H) | 1 |
| 462 | (2E)-N-(cis-4-{[4-(dimethylamino)-5,6,7,8-tetrahydroquinazolin-2-yl]amino}cyclohexyl)-3-phenylacrylamide | 420 (M + H) | 3 |
| 463 | 4-chloro-N-(cis-4-{[4-(dimethylamino)-5,6,7,8-tetrahydroquinazolin-2-yl]amino}cyclohexyl)-3-nitrobenzamide | 473 (M + H) | 1 |
| 464 | 2-(4-chlorophenyl)-N-(cis-4-{[4-(dimethylamino)-5,6,7,8-tetrahydroquinazolin-2-yl]amino}cyclohexyl)acetamide | 442 (M + H) | 1 |
| 465 | 3-cyano-N-(cis-4-{[4-(dimethylamino)-5,6,7,8-tetrahydroquinazolin-2-yl]amino}cyclohexyl)benzamide | 419 (M + H) | 1 |
| 466 | 3,5-dichloro-N-(cis-4-{[4-(dimethylamino)-5,6,7,8-tetrahydroquinazolin-2-yl)amino}cyclohexyl)benzamide | 462 (M + H) | 1 |
| 467 | 3,4-dichloro-N-(cis-4-{[4-(dimethylamino)-5,6,7,8-tetrahydroquinazolin-2-yl]amino}cyclohexyl)benzamide | 462 (M + H) | 1 |
| 468 | N-(cis-4-{[4-(dimethylamino)-5,6,7,8-tetrahydroquinazolin-2-yl]amino}cyclohexyl)-2,2-diphenylacetamide | 484 (M + H) | 1 |
| 469 | N-(cis-4-{[4-(dimethylamino)-5,6,7,8-tetrahydroquinazolin-2-yl]amino}cyclohexyl)-3,4-difluorobenzamide | 430 (M + H) | 1 |
| 470 | N-(cis-4-{[4-(dimethylamino)-5,6,7,8-tetrahydroquinazolin-2-yl]amino}cyclohexyl)-3,5-difluorobenzamide | 430 (M + H) | 1 |
| 471 | 2-(2,5-dimethoxyphenyl)-N-(cis-4-{[4-(dimethylamino)-5,6,7,8-tetrahydroquinazolin-2-yl]amino}cyclohexyl)acetamide | 468 (M + H) | 3 |
| 472 | N-(cis-4-{[4-(dimethylamino)-5,6,7,8-tetrahydroquinazolin-2-yl]amino}cyclohexyl)-2-(ethylthio)nicotinamide | 455 (M + H) | 3 |
| 473 | N-(cis-4-{[4-(dimethylamino)-5,6,7,8-tetrahydroquinazolin-2-yl]amino}cyclohexyl)-4-fluorobenzamide | 412 (M + H) | 1 |
| 474 | N-(cis-4-{[4-(dimethylamino)-5,6,7,8-tetrahydroquinazolin-2-yl]amino}cyclohexyl)-3-fluoro-5-(trifluoromethyl)benzamide | 480 (M + H) | 1 |
| 475 | 2,4-dichloro-N-(cis-4-{[4-(dimethylamino)-5,6,7,8-tetrahydroquinazolin-2-yl]amino}cyclohexyl)-5-fluorobenzamide | 480 (M + H) | 3 |
| 476 | N-(cis-4-{[4-(dimethylamino)-5,6,7,8-tetrahydroquinazolin-2-yl]amino}cyclohexyl)hexanamide | 388 (M + H) | 2 |
| 477 | N-(cis-4-{[4-(dimethylamino)-5,6,7,8-tetrahydroquinazolin-2-yl]amino}cyclohexyl)-4-iodobenzamide | 520 (M + H) | 3 |
| 478 | N-(cis-4-{[4-(dimethylamino)-5,6,7,8-tetrahydroquinazolin-2-yl]amino}cyclohexyl)-2-(methylthio)nicotinamide | 441 (M + H) | 3 |
| 479 | N-(cis-4-{[4-(dimethylamino)-5,6,7,8-tetrahydroquinazolin-2-yl]amino}cyclohexyl)-4-methyl-3-nitrobenzamide | 453 (M + H) | 1 |
| 480 | N-(cis-4-{[4-(dimethylamino)-5,6,7,8-tetrahydroquinazolin-2-yl]amino}cyclohexyl)-3-nitrobenzamide | 439 (M + H) | 1 |

(continued)

| Ex. No. | compound name | MS | class |
|---|---|---|---|
| 481 | N-(cis-4-{[4-(dimethylamino)-5,6,7,8-tetrahydroquinazolin-2-yl] amino}cyclohexyl)-2-phenylacetamide | 408 (M + H) | 3 |
| 482 | (2R)-N-(cis-4-{[4-(dimethylamino)-5,6,7,8-tetrahydroquinazolin-2-yl]amino}cyclohexyl)-2-phenylcyclopropanecarboxamide | 434 (M + H) | 2 |
| 483 | N-(cis-4-{[4-(dimethylamino)-5,6,7,8-tetrahydroquinazolin-2-l] amino}cyclohexyl)-1,3-benzodioxole-5-carboxamide | 438 (M + H) | 3 |
| 484 | N-(cis-4-{[4-(dimethylamino)-5,6,7,8-tetrahydroquinazolin-2-yl] amino}cyclohexyl)-2-phenoxybutanamide | 452 (M + H) | 1 |
| 485 | N-(cis-4-{[4-(dimethylamino)-5,6,7,8-tetrahydroquinazolin-2-yl] amino}cyclohexyl)-2-phenoxypropanamide | 438 (M + H) | 1 |
| 486 | N-(cis-4-{[4-(dimethylamino)-5,6,7,8-tetrahydroquinazolin-2-yl] amino}cyclohexyl)-3-methylbenzamide | 408 (M + H) | 1 |
| 487 | N-(cis-4-{[4-(dimethylamino)-5,6,7,8-tetrahydroquinazolin-2-yl] amino}cyclohexyl)-4-methylbenzamide | 408 (M + H) | 2 |
| 488 | N-(cis-4-{[4-(dimethylamino)-5,6,7,8-tetrahydroquinazolin-2-yl] aminocyclohexyl)thiophene-2-carboxamide | 400 (M + H) | 3 |
| 489 | N-(cis-4-{[4-(dimethylamino)-5,6,7,8-tetrahydroquinazolin-2-yl] amino}cyclohexyl)-2-(2-thienyl)acetamide | 414 (M + H) | 3 |
| 490 | N-(cis-4-{[4-(dimethylamino)-5,6,7,8-tetrahydroquinazolin-2-yl] amino}cyclohexyl)3-(trifluoromethoxy)benzamide | 478 (M + H) | 2 |
| 491 | [4-(4-Dimethylamino-5,6,7,8-tetrahydro-quinazolin-2-ylamino)-yclohexyl]-carbamic acid benzyl ester | 424 (M + H) | 3 |
| 492 | [4-(4-Dimethylamino-5,6,7,8-tetrahydro-quinazolin-2-ylamino)-cyclohexyl]-carbamic acid 4-nitro-benzyl ester | 469 (M + H) | 3 |
| 493 | 4-bromo-N-(cis-4-{[4-(dimethylamino)-5,6,7,8-tetrahydroquinazolin-2-yl]amino}cyclohexyl)-3-methylbenzamide | 486 (M + H) | 2 |
| 494 | N-(cis-4-{[4-(dimethylamino)-5,6,7,8-tetrahydroquinazolin-2-yl] amino}cyclohexyl)-3-iodobenzamide | 520 (M + H) | 1 |
| 495 | 3-chloro-N-(cis-4-{[4-(dimethylamino)-5,6,7,8-tetrahydroquinazolin-2-yl]amino}cyclohexyl)-2-fluorobenzamide | 446 (M + H) | 3 |
| 496 | N-(cis-4-{[4-(dimethylamino)-5,6,7,8-tetrahydroquinazolin-2-yl] amino}cyclohexyl)-2,3-difluoro-4-methylbenzamide | 444 (M + H) | 3 |
| 497 | 2-chloro-N-(cis-4-{[4-(dimethylamino)-5,6,7,8-tetrahydroquinazolin-2-yl]amino cyclohexyl)-4-fluorobenzamide | 446 (M + H) | 2 |
| 498 | 3-chloro-N-(cis-4-([4-(dimethylamino)-5,6,7,8-tetrahydro-quinazolin-2-yl]amino}cydohexyl)-2,4-difluorobenzamide | 464 (M + H) | 3 |
| 499 | N-(cis-4-{[4-(dimethylamino)-5,6,7,8-tetrahydroquinazolin-2-yl] amino}cyclohexyl)-2-(phenylthio)acetamide | 440 (M + H) | 3 |
| 500 | N-(cis-4-{[4-(dimethylamino)-5,6,7,8-tetrahydroquinazolin-2-yl] amino}cyclohexyl)-2-fluoro-3-(trifluoromethyl)benzamide | 480 (M + H) | 3 |
| 501 | N-(cis-4-{[4-(dimethylamino)-5,6,7,8-tetrahydroquinazolin-2-yl] amino}cyclohexyl)-2-fluoro-5-(trifluoromethyl)benzamide | 480 (M + H) | 3 |

(continued)

| Ex. No. | compound name | MS | class |
|---|---|---|---|
| 502 | N-(cis-4-{[4-(dimethylamino)-5,6,7,8-tetrahydroquinazolin-2-yl] amino}cyclohexyl)-2-phenylbutanamide | 436 (M + H) | 3 |
| 503 | N-(cis-4-{[4-(dimethylamino)-5,6,7,8-tetrahydroquinazolin-2-yl] amino}cyclohexyl)-2-(3-methoxyphenyl)acetamide | 438 (M + H) | 2 |
| 504 | N-(cis-4-{(4-(dimethylamino)-5,6,7,8-tetrahydroquinazolin-2-yl] amino}cyclohexyl)-2-(4-fluorophenyl)acetamide | 426 (M + H) | 1 |
| 505 | N-(cis-4-{[4-(dimethylamino)-5,6,7,8-tetrahydroquinazolin-2-yl] amino}cyclohexyl)-2-(4-methoxyphenyl)acetamide | 438 (M + H) | 2 |
| 506 | N-(cis-4-{[4-(dimethylamino)-5,6,7,8-tetrahydroquinazolin-2-yl] amino}cyclohexyl)-5-methyl-2-(trifluoromethyl)-3-furamide | 466 (M + H) | 2 |
| 507 | N-(cis-4-{[4-(dimethylamino)-5,6,7,8-tetrahydroquinazolin-2-yl] amino}cyclohexyl)-2,5-dimethyl-3-furamide | 412 (M + H) | 1 |
| 508 | N-(cis-4-{[4-(dimethylamino)-5,6,7,8-tetrahydroquinazolin-2-yl] amino}cyclohexyl)-2-ethoxybenzamide | 438 (M + H) | 3 |
| 509 | 3-chloro-N-(cis-4-{[4-(dimethylamino)-5,6,7,8-tetrahydroquinazolin-2-yl]amino}cyclohexyl)-4-fluorobenzamide | 446 (M + H) | 1 |
| 510 | N-(cis-4-{[4-(dimethylaniino)-5,6,7,8-tetrahydroquinazolin-2-yl] amino)cyclohexyl)-3-fluoro-4-methylbenzamide | 426 (M+H) | 2 |
| 511 | 2-cyclopentyl-N-(cis-4-{[4-(dimethylamino)-5,6,7,8-tetrahydroquinazolin-2-yl]amino}cyclohexyl)acetamide | 400 (M + H) | 3 |
| 512 | N-(cis-4-{[4-(dimethylamino)-5,6,7,8-tetrahydroquinazolin-2-yl] amino ) cyclohexyl)-3,5-dimethoxybenzamide | 454 (M + H) | 1 |
| 513 | 4-cyano-N-(cis-4-{[4-(dimethylamino)-5,6,7,8-tetrahydroquinazolin-2-yl]amino}cyclohexyl)benzamide | 419 (M + H) | 3 |
| 514 | N-(cis-4-{[4-(dimethylamino)-5,6,7,8-tetrahydroquinazolin-2-yl] amino}cyclohexyl)-3,5-bis(trifluoromethyl)benzamide | 530 (M + H) | 1 |
| 515 | (2E)-N-(cis-4-{[4-(dimethylamino)-5,6,7,8-tetrahydroquinazolin-2-yl]amino}cyclohexyl)-3-(4-nitrophenyl)acrylamide | 465 (M + H) | 3 |
| 516 | 2-(2-bromophenyl)-N-(cis-4-{[4-(dimethylamino)-5,6,7,8 tetrahydroquinazolin-2-yl]amino}cyclohexyl)acetamide | 486 (M + H) | 3 |
| 517 | N-(cis-4-{[4-(dimethylamino)-5,6,7,8-tetrahydroquinazolin-2-yl] amino}cyclohexyl)-4-fluoro-3-methylbenzamide | 426 (M + H) | 1 |
| 518 | 2-[(difluoromethyl)thio]-N-(cis-4-)[4-(dimethylamino)-5,6,7,8-tetrahydroquinazolin-2-yl]amino}cyclohexyl)benzamide | 476 (M + H) | 3 |
| 519 | 2,5-dichloro-N-(cis-4-{[4-(dimethylamino)-5,6,7,8-tetrahydroquinazolin-2-yl]amino}cyclohexyl)thiophene-3-carboxamide | 468 (M + H) | 1 |
| 520 | N-(cis-4-{[4-(dimethylamino)-5,6,7,8-tetrahydroquinazolin-2-yl] amino}cyclohexyl)-2-(propylthio)nicotinamide | 469 (M + H) | 2 |
| 521 | 1-benzyl-3-tert-butyl-N-(cis-4-{[4-(dimethylamino)-5,6,7,8-tetrahydroquinazolin-2-yl]amino}cyclohexyl)-1H-pyrazole-5-carboxamide | 530 (M + H) | 2 |

(continued)

| Ex. No. | compound name | MS | class |
|---|---|---|---|
| 522 | 3-tert-butyl-N-(cis-4-{{[4-(dimethylamino)-5,6,7,8-tetrahydroquinazolin-2-yl]amino}cyclohexyl)-1-methyl-1 H-pyrazole-5-carboxamide | 454 (M + H) | 3 |
| 523 | (2E)-N-(cis-4-{[4-(dimethylamino)-5,6,7,8-tetrahydroquinazolin-2-yl]amino}cyclohexyl)-2-methyl-3-phenylacrylamide | 434 (M + H) | 3 |
| 524 | 5-bromo-N-(cis-4-{[4-(dimethylamino)-5,6,7,8-tetrahydroquinazolin-2-yl]amino}cyclohexyl)nicotinamide | 473 (M + H) | 1 |
| 525 | N-(cis-4-{[4-(dimethylamino)-5,6,7,8-tetrahydroquinazolin-2-yl]amino)cyclohexyl)-2-(1-naphthyl)acetamide | 458 (M + H) | 3 |
| 526 | 1-tert-butyl-N-(cis-4-{[4-(dimethylamino)-5,6,7,8-tetrahydroquinazolin-2-yl]amino)cyclohexyl)-5-methyl-1H-pyrazole-3-carboxamide | 454 (M + H) | 3 |
| 527 | N-(cis-4-{[4-(dimethylamino)-5,6,7,8-tetrahydroquinazolin-2-yl]amino)cyclohexyl)-1-benzothiophene-3-carboxamide | 450 (M + H) | 3 |
| 528 | 2-[(cis-4-{[4-(dimethylamino)-5,6,7,8-tetrahydroquinazolin-2-yl]amino)cyclohexyl)amino]-2-oxo-1-phenylethyl acetate | 466 (M + H) | 1 |
| 529 | N-(cis-4-{[4-(dimethylamino)-5,6,7,8-tetrahydroquinazolin-2-yl]amino)cyclohexyl)benzamide | 394 (M + H) | 2 |
| 530 | N-(cis-4-{[4-(dimethylamino)-5,6,7,8-tetrahydroquinazolin-2-yl]amino}cyclohexyl)-1-benzothiophene-2-carboxamide | 450 (M + H) | 3 |
| 531 | 2-(benzyloxy)-N-(cis-4-{[4-(dimethylamino)-5,6,7,8-tetrahydroquinazolin-2-yl]amino}cyclohexyl)acetamide | 438 (M + H) | 2 |
| 532 | 2-(4-chlorophenoxyl)-N-(cis-4-{[4-(dimethylamino)-5,6,7,8-tetrahydroquinazolin-2-yl]amino}cyclohexyl)acetamide | 458 (M + H) | 2 |
| 533 | N-(cis-4-{[4-(dimethylamino)-5,6,7,8-tetrahydroquinazolin-2-yl]amino}cyclohexyl)cyclohexanecarboxamide | 400 (M + H) | 3 |
| 534 | 3-(2-chloroghenyl)-N-(cis-4-{[4-(dimethylamino)-5,6,7,8-tetrahydroquinazolin-2-yl]amino}cyclohexyl)-5-methylisoxazole-4-carboxamide | 509 (M + H) | 2 |
| 535 | 1-(4-chlorophenyl)-N-(cis-4-{[4-(dimethylamino)-5,6,7,8-tetrahydroquinazolin-2-yl]amino}cyclohexyl) cyclopentanecarboxamide | 496 (M + H) | 2 |
| 536 | 3-(2-chloro-6-fluorophenyl)-N-(cis-4-{[4-(dimethylamino)-5,6,7,8-tetrahydroquinazolin-2-yl]amino}cyclohexyl)-5-methylisoxazole-4-carboxamide | 527 (M + H) | 1 |
| 537 | 3-chloro-N-(cis-4-{[4-(dimethylamino)-5,6,7,8-tetrahydroquinazolin-2-yl]amino}cyclohexyl)-4-(isopropylsulfonyl)thiophene-2-carboxamide | 540 (M + H) | 3 |
| 538 | 2-chloro-N-(cis-4-{[4-(dimethylamino)-5,6,7,8-tetrahydroquinazolin-2-yl]amino}cyclohexyl)-4-nitrobenzamide | 473 (M + H) | 3 |
| 539 | N-(cis-4-{[4-(dimethylamino)-5,6,7,8-tetrahydroquinazolin-2-yl]amino}cyclohexyl)-1,3-dimethy]-1H-pyrazole-5-carboxamide | 412 (M + H) | 2 |
| 540 | N-(cis-4-{[4-(dimethylamino}-5,6,7,8-tetrahydroquinazolin-2-yl]amino}cyclohexyl)-3,4-dimethoxybenzamide | 454 (M + H) | 3 |

(continued)

| Ex. No. | compound name | MS | class |
|---|---|---|---|
| 541 | N-(cis-4-{[4-(dimethylamino)-5,6,7,8-tetrahydroquinazolin-2-yl] amino}cyclohexyl)-3-fluorobenzamide | 412 (M + H) | 1 |
| 542 | N-(cis-4-{[4-(dimethylamino)-5,6,7,8-tetrahydroquinazolin-2-yl] amino}cyclohexyl)-4-fluoro-3-(thfluoromethyl)benzamide | 480 (M + H) | 1 |
| 543 | N-(cis-4-[4-(dimethylamino)-5,6,7,8-tetrahydroquinazolin-2-yl] amino}cyclohexyl)-5-methyl-2-phenyl-2H-1,2,3-triazole-4-carboxamide | 475 (M + H) | 2 |
| 544 | N-(cis-4-)[4-(dimethylamino)-5,6,7,8-tetrahydroquinazolin-2-yl] amino}cyclohexyl)-2-(4-methoxyphenoxy)-5-nitrobenzamide | 561 (M + H) | 1 |
| 545 | N-(cis-4-{[4-(dimethylamino)-5,6,7,8-tetrahydroquinazolin-2-yl] amino}cyclohexyl)-1-naphthamide | 444 (M + H) | 3 |
| 546 | N-(cis-4-{[4-(dimethylamino)-5,6,7,8-tetrahydroquinazolin-2-yl] amino}cyclohexyl)-2-naphthamide | 444 (M + H) | 3 |
| 547 | N-(cis-4-{[4-(dimethylamino)-5,6,7,8-tetrahydroquinazolin-2-yl] amino}cyclohexyl)-5-nitro-2-furamide | 429 (M + H) | 1 |
| 548 | N-(cis-4-{[4-(dimethylamino)-5,6,7,8-tetrahydroquinazolin-2-yl] amino}cyclohexyl)-2-phenoxyacetamide | 424 (M + H) | 1 |
| 549 | N-(cis-4-{[4-(dimethylamino)-5,6,7,8-tetrahydroquinazolin-2-yl] amino}cyclohexyl)-2-(2-nitrophenoxy)acetamide | 469 (M + H) | 3 |
| 550 | N-(cis-4-([4-(dimethylamino)-5,6,7,8-tetrahydroquinazolin-2-yl] amino}cyclohexyl)quinoxaline-2-carboxamide | 446 (M + H) | 1 |
| 551 | N-(cis-4-{[4-(dimethylamino)-5,6,7,8-tetrahydroquinazolin-2-yl] amino}cyclohexyl)-3,4,5-trimethoxybenzamide | 484 (M + H) | 3 |
| 552 | N-(cis-4-{[4-(dimethylamino)-5,6,7,8-tetrahydroquinazolin-2-yl] amino}cyclohexyl)-3-(thifluoromethyl)benzamide | 462 (M + H) | 1 |
| 553 | N-(cis-4-{[4-(dimethylamino)-5,6,7,8-tetrahydroquinazolin-2-yl] amino}cyclohexyl)-4-(trifluoromethyl)benzamide | 462 (M + H) | 3 |
| 554 | N-(cis-4-{[4-(dimethylamino)-5,6,7,8-tetrahydroquinazolin-2-yl] amino}cyclohexyl)-2-(trifluoromethoxy)benzamide | 478 (M + H) | 3 |
| 555 | 4,5-dimethoxy-2-nitrobenzyl (cis-4-{[4-(dimethylamino)-5,6,7,8-tetrahydroquinazolin-2-yl]amino cyclohexyl)carbamate | 529 (M + H) | 3 |
| 556 | N-(cis-4-{[4-(dimethylamino)-5,6,7,8-tetrahydroquinazolin-2-yl] amino}cyclohexyl)-4-phenoxybutanamide | 452 (M + H) | 3 |
| 557 | 2-bromo-N-(cis-4-{[4-(dimethylamino)-5,6,7,8-tetrahydro-quinazolin-2-yl]amino}cyclohexyl)-5-methoxybenzamide | 502 (M + H) | 3 |
| 558 | N-(cis-4-{[4-(dimethylamino)-5,6,7,8-tetrahydroquinazolin-2-yl] amino}cyclohexyl)-2-(pentafluorophenoxy)acetamide | 514 (M + H) | 3 |
| 559 | 2-(3,4-dimethoxyphenyl)-N-(cis-4-{[4-(dimethylamino)-5,6,7,8-tetrahydroquinazolin-2-yl]amino}cyclohexyl)acetamide | 468 (M + H) | 3 |
| 560 | N-(cis-4-{[4-(dimethylamino)-5,6,7,8-tetrahydroquinazolin-2-yl] amino}cyclohexyl)-2,3,4-trifluorobenzamide | 448 (M + H) | 3 |
| 561 | N-(cis-4-([4-(dimethylamino)-5,6,7,8-tetrahydroquinazolin-2-yl] amino}cyclohexyl)cyclopentanecarboxamide | 386 (M + H) | 3 |
| 562 | N-(cis-4-{[4-(dimethylamino)-5,6,7,8-tetrahydroquinazolin-2-yl] amino}cyclohexyl)-2,4-difluorobenzamide | 430 (M + H) | 3 |

(continued)

| Ex. No. | compound name | MS | class |
|---|---|---|---|
| 563 | N-(cis-4-{[4-(dimethylamino)-5,6,7,8-tetrahydroquinazolin-2-yljamino}cyclohexyl)-3-phenylpropanamide | 422 (M + H) | 3 |
| 564 | N-(cis-4-{[4-(dimethylamino)-5,6,7,8-tetrahydroquinazolin-2-yl]amino}cyclohexyl)-2,3,4,5-tetrafluorobenzamide | 466 (M + H) | 3 |
| 565 | N-(cis-4-{[4-(dimethylamino)-5,6,7,8-tetrahydroquinazolin-2-yl]amino}cyclohexyl)-2-ethoxy-1-naphthamide | 488 (M + H) | 3 |
| 566 | N-(cis-4-1{[4-(dimethylamino)-5,6,7,8-tetrahydroquinazolin-2-yl]amino}cyclohexyl)-2,3,4,5,6-pentafluorobenzamide | 484 (M + H) | 3 |
| 567 | N-(cis-4-{[4-(dimethylamino)-5,6,7,8-tetrahydroquinazolin-2-yl]amino}cyclohexyl)-4-[(trifluoromethyl)thio]benzamide | 494 (M + H) | 3 |
| 568 | 3,4,5-trichloro-N-(cis-4-{[4-(dimethylamino)-5,6,7,8-tetrahydro-quinazolin-2-yl]amino}cyclohexyl)thiophene-2-carboxamide | 502 (M + H) | 3 |
| 569 | 2-(3-chlorophenoxy)-N-(cis-4-)[4-(dimethylamino)-5,6,7,8-tetrahydroquinazolin-2-yl]amino}cyclohexyl)acetamide | 458 (M + H) | 1 |
| 570 | 3-(2,6-dichlorophenyl)-N-(cis-4-{[4-(dimethylamino)-5,6,7,8-tetrahydroquinazolin-2-yl]amino}cyclohexyl)-5-methylisoxazole-4-carboxamide | 543 (M + H) | 1 |
| 571 | N-(cis-4-{[4-(dimethylamino)-5,6,7,8-tetrahydroquinazolin-2-yl]amino}cyelohexyl)-2-phenoxynicotinamide | 487 (M + H) | 2 |
| 572 | N-(cis-4-{[4-(dimethylamino)-5,6,7,8-tetrahydroquinazolin-2-yl]amino)cyclohexyl)-2-(phenylthio)nicotinamide | 503 (M + H) | 3 |
| 573 | N-(cis-4-{[4-(dimethylamino)-5,6,7,8-tetrahydroquinazolin-2-yl]amino}cyclohexyl)-2-(4-methylphenoxy)nicotinamide | 501 (M + H) | 1 |
| 574 | N-(cis-4-{[4-(dimethylamino)-5,6,7,8-tetrahydroquinazolin-2-yl]amino}cyclohexyl)-4-[(dipropylamino)sulfonyl]benzamide | 557 (M + H) | 3 |
| 575 | 2-(4-chlorophenoxy)-N-(cis-4-{[4-(dimethylamino)-5,6,7,8-tetrahydroquinazolin-2-yl]amino}cyclohexyl)-2-methylpropanamide | 486 (M + H) | 3 |
| 576 | 5-(4-chlorophenyl)-N-(cis-4-{(4-(dimethylamino)-5,6,7,8-tetrahydroquinazolin-2-yl]amino}cyclohexyl)-2-(trifluoromethyl)-3-furamide | 562 (M + H) | 3 |
| 577 | 3-tert-butyl-1-(2,4-dichlorobenzyl)-N-(cis-4-{[4-(dimethylamino)-5,6,7,8-tetrahydroquinazolin-2-yl]amino}cyclohexyl)-1H-pyrazole-5-carboxamide | 598 (M + H) | 3 |
| 578 | 6-chloro-N-(cis-4-{[4-(dimethylamino)-5,6,7,8-tetrahydroquinazolin-2-yl]amino]cyclohexyl)-2H-chromene-3-carboxamide | 482 (M + H) | 3 |
| 579 | 3-chloro-N-(cis-4-{[4-(dimethylamino)-5,6,7,8-tetrahydro-quinazolin-2-yl]amino}cyclohexyl)-4-(trifluoromethoxy)-benzamide | 512 (M + H) | 3 |
| 580 | N-(cis-4-{[4-(dimethylamino)-5,6,7,8-tetrahydroquinazolin-2-yl]amino}cyclohexyl)-2-[(4-methyl-2-oxo-2H-chromen-8-yl)oxy]acetamide | 506 (M + H) | 3 |
| 581 | N-(cis-4-{[4-(dimethylamino)-5,6,7,8-tetrahydroquinazolin-2-yl]amino}cyclohexyl)-2-(2-thienyl)-1,3-thiazole-4-carboxamide | 483 (M + H) | 2 |

(continued)

| Ex. No. | compound name | MS | class |
|---|---|---|---|
| 582 | N-[(cis-4-{[4-(dimethylamino)-5,6,7,8-tetrahydroquinazolin-2-yl] amino}cyclohexyl)methyl]-3-methoxybenzamide | 438 (M + H) | 3 |
| 583 | 3-bromo-N-[(cis-4-{[4-(dimethylamino)-5,6,7,8-tetrahydroquinazolin-2-yl]amino}cyclohexyl)methyl] benzamide | 486 (M + H) | 2 |
| 584 | 4-bromo-N-[(cis-4-{[4-(dimethylamino)-5,6,7,8-tetrahydroquinazolin-2-yl]amino}cyclohexyl)methyl] benzamide | 486 (M + H) | 3 |
| 585 | N-[(cis-4-{[4-(dimethylamino)-5,6,7,8-tetrahydroquinazolin-2-yl] amino}cyclohexyl)methyl]-2,1,3-benzoxadiazole-5-carboxamide | 450 (M + H) | 3 |
| 586 | 3-chloro-N-[(cis-4-{[4-(dimethylamino)-5,6,7,8-tetrahydroquinazolin-2-yl]amino}cyclohexyl)methyl] benzamide | 442 (M + H) | 3 |
| 587 | 4-chloro-N-[(cis-4-{[4-(dimethylamino)-5,6,7,8-tetrahydroquinazolin-2-yl]amino}cyclohexyl)methyl] benzamide | 442 (M + H) | 3 |
| 588 | (2E)-N-[(cis-4-{[4-(dimethylamino)-5,6,7,8-tetrahydroquinazolin-2-yl]amino}cyclohexyl)methyl]-3-phenylacrylamide | 434 (M + H) | 3 |
| 589 | 4-chloro-N-[(cis-4-{[4-(dimethylamino)-5,6,7,8-tetrahydroquinazolin-2-yl]amino}cyclohexyl)methyl]-3-nitrobenzamide | 487 (M + H) | 3 |
| 590 | 2-(4-chlorophenyl)-N-[(cis-4-([4-(dimethylamino)-5,6,7,8-tetrahydroquinazolin-2-yl]amino}cyclohexyl)methyl] acetamide | 456 (M + H) | 3 |
| 591 | 3-cyano-N-[(cis-4-{[4-(dimethylamino)-5,6,7,8-tetrahydroquinazolin-2-yl]amino)cyclohexyl)methyl] benzamide | 433 (M + H) | 3 |
| 592 | 3,5-dichloro-N-[(cis-4-{[4-(dimethylamino)-5,6,7,8-tetrahydroquinazolin-2-yl]amino}cyclohexyl)methyl] benzamide | 476 (M + H) | 3 |
| 593 | 3,4-dichloro-N-[(cis-4-{[4-(dimethylamino)-5,6,7,8-tetrahydroquinazolin-2-yl]amino}cyclohexyl)methyl] benzamide | 476 (M + H) | 3 |
| 594 | N-[(cis-4-{[4-(dimethylamino)-5,6,7,8-tetrahydroquinazolin-2-yl] amino}cyclohexyl)methyl]-2,2-diphenylacetamide | 498 (M + H) | 3 |
| 595 | N-[(cis-4-{[4-(dimethylamino)-5,6,7,8-tetrahydroquinazolin-2-yl] amino}cyclohexyl)methyl]-3,4-difluorobenzamide | 444 (M + H) | 3 |
| 596 | N-[(cis-4-{[4-(dimethylamino)-5,6,7,8-tetrahydroquinazolin-2-yl] amino}cyclohexyl)methyl]-3,5-difluorobenzamide | 444 (M + H) | 3 |
| 597 | 2-(2,5-dimethoxyphenyl)-N-[(cis-4-{[4-(dimethylamino)-5,6,7,8-tetrahydroquinazolin-2-yl]amino}cyclohexyl)methyl] acetamide | 482 (M + H) | 3 |
| 598 | N-[(cis-4-{[4-(dimethylamino)-5,6,7,8-tetrahydroquinazolin-2-yl] amino}cyclohexyl)methyl]-2-(ethylthio)nicotinamide | 469 (M + H) | 1 |
| 599 | N-[(cis-4-{[4-(dimethylamino)-5,6,7,8-tetrahydroquinazolin-2-yl] amino}cyclohexyl)methyl]-4-fluorobenzamide | 426 (M + H) | 3 |

(continued)

| Ex. No. | compound name | MS | class |
|---|---|---|---|
| 600 | N-[(cis-4-{[4-(dimethylamino)-5,6,7,8-tetrahydroquinazolin-2-yl] amino}cyclohexyl)methyl]-3-fluoro-5-(trifluoromethyl)benzamide | 494 (M + H) | 3 |
| 601 | 2,4-dichloro-N-[(cis-4-{[4-(dimethylamino)-5,6,7,8-tetrahydroquinazolin-2-yl]amino}cyclohexyl)methyl]-5-fluorobenzamide | 494 (M + H) | 3 |
| 602 | N-[(cis-4-{[4-(dimethylamino)-5,6,7,8-tetrahydroquinazolin-2-yl] amino}cyclohexyl)methyl]hexanamide | 402 (M + H) | 3 |
| 603 | N-[(cis-4-{[4-(dimethylamino)-5,6,7,8-tetrahydroquinazolin-2-yl] amino}cyclohexyl)methyl]-4-iodobenzamide | 534 (M + H) | 3 |
| 604 | N-[(cis-4-{[4-(dimethylamino)-5,6,7,8-tetrahydroquinazolin-2-yl] amino}cyclohexyl)methyl]-2-(methylthio)nicotinamide | 455 (M + H) | 3 |
| 605 | N-[(cis-4-{[4-(dimethylamino)-5,6,7,8-tetrahydroquinazolin-2-yl] amino}cyclohexyl)methyl]-4-methyl-3-nitrobenzamide | 467 (M + H) | 3 |
| 606 | N-[(cis-4-{[4-(dimethylamino)-5,6,7,8-tetrahydroquinazolin-2-yl] amino}cyclohexyl)methyl]-3-nitrobenzamide | 453 (M + H) | 3 |
| 607 | N-((cis-4-{[4-(dimethylamino)-5,6,7,8-tetrahydroquinazolin-2-yl] amino}yclohexyl)methyl]-2-phenylacetamide | 422 (M + H) | 3 |
| 608 | (2R)-N-[(cis-4-3 {[4-(dimethymino)-5,6,7,8-tetrahydroquinazolin-2-yl]amino}cyclohexyl)methyl]-2-phenylcyclopropane-carboxamide | 448 (M + H) | 3 |
| 609 | N-[(cis-4-([4-(dimethylamino)-5,6,7,8-tetrahydroquinazolin-2-yl] amino}cyclohexyl)methyl]-1,3-benzodioxole-5-carboxamide | 452 (M + H) | 3 |
| 610 | N-[(cis-4-[4-(dimethylamino)-5,6,7,8-tetrahydroquinazolin-2-yl] amino}cyclohexyl)methyl]-2-phenoxybutanamide | 466 (M + H) | 3 |
| 611 | N-[(cis-4-{[4-(dimethylamino)-5,6,7,8-tetrahydroquinazolin-2-yl] amino}cyclohexyl)methyl]-2-phenoxypropanamide | 452 (M + H) | 3 |
| 612 | N-((cis-4-{[4-(dimethylamino)-5,6,7,8-tetrahydroquinazolin-2-yl] amino}cyclohexyl)methyl]-3-methylbenzamide | 422 (M + H) | 3 |
| 613 | N-[(cis-4-{[4-(dimethylamino)-5,6,7,8-tetrahydroquinazlin-2-yl] amino}cyclohexyl)methyl]-4-methylbenzamide | 422 (M + H) | 3 |
| 614 | N-[(cis-4-{[4-(dimethylamino)-5,6,7,8-tetrahydroquinazolin-2-yl] amino}cyclohexyl)methyl]thiophene-2-carboxamide | 414 (M + H) | 3 |
| 615 | N-[(cis-4-{[4-(dimethylamino)-5,6,7,8-tetrahydroquinazolin-2-yl] amino}cyclohexyl)methyl]-2-(2-thienyl)acetamide | 428 (M + H) | 3 |
| 616 | N-[(cis-4-{[4-(dimethylamino)-5,6,7,8-tetrahydroquinazolin-2-yl] amino}cyclohexyl)methyl]-3-(trifluoromethoxy)benzamide | 492 (M + H) | 3 |
| 617 | benzyl[(cis-4-{[4-(dimethylamino)-5,6,7,8-tetrahydroquinazolin-2-yl]amino}cyclohexyl)methyl]carbamate | 438 (M + H) | 3 |
| 618 | 4-nitrobenzyl[(cis-4-{[4-(dimethylamino)-5,6,7,8-tetrahydroquinazolin-2-yl]amino}cyclohexyl)methyl] carbamate | 483 (M + H) | 3 |
| 619 | 4-bromo-N-[(cis-4-({[4-(dimethylamino)-5,6,7,8-tetrahydroquinazolin-2-yl]amino}cyclohexyl)methyl]-3-methylbenzamide | 500 (M + H) | 3 |

(continued)

| Ex. No. | compound name | MS | class |
|---|---|---|---|
| 620 | N-{(cis-4-{[4-(dimethylamino)-5,6,7,8-tetrahydroquinazolin-2-yl] amino}cyclohexyl)methyl]-3-iodobenzamide | 534 (M + H) | 3 |
| 621 | 3-chloro-N-[(cis-4-{[4-(dimethylamino)-5,6,7,8-tetrahydro-quinazolin-2-yl]amino}cyclohexyl)methyl]-2-fluorobenzamide | 460 (M + H) | 3 |
| 622 | N-[(cis-4-{[4-(dimethylamino)-5,6,7,8-tetrahydroquinazolin-2-yl] amino cyclohexyl)methyl]-2,3-difluoro-4-methylbenzamide | 458 (M + H) | 3 |
| 623 | 2-chloro-N-[(cis-4-{[4-(dimethylamino)-5,6,7,8-tetrahydroquinazolin-2-yl]amino}cyclohexyl)methyl]-4-fluorobenzamide | 460 (M + H) | 3 |
| 624 | 3-chloro-N-[(cis-4-{[4-(dimethylamino)-5,6,7,8-tetrahydroquinazolin-2-yl]amino}cyclohexyl)methyl]-2,4-difluorobenzamide | 478 (M + H) | 3 |
| 625 | N-[(cis-4-{[4-(dimethylamino)-5,6,7,8-tetrahydroquinazolin-2-yl] amino}cyclohexyl)methyl]-2-(phenylthio)acetamide | 454 (M + H) | 3 |
| 626 | N-[(cis-4-{[4-(dimethylamino)-5,6,7,8-tetrahydroquinazolin-2-yl] amino}cyclohexyl)methyl]-2-fluoro-3-(trifluoromethyl)benzamide | 494 (M + H) | 3 |
| 627 | N-[(cis-4-{[4-(dimethylamino)-5,6,7,8-tetrahydroquinazolin-2-yl] amino}cyclohexyl)methyl]-2-fluoro-5-(trifluoromethyl)benzamide | 494 (M + H) | 3 |
| 628 | N-[(cis-4-{[4-(dimethylamino)-5,6,7,8-tetrahydroquinazolin-2-yl] amino}cyclohexyl)methyl]-2-phenylbutanamide | 450 (M + H) | 3 |
| 629 | N-[(cis-4-{[4-(dimethylamino)-5,6,7,8-tetrahydroquinazolin-2-yl] amino}cyclohexyl)methyl]-2-(3-methoxyphenyl)acetamide | 452 (M + H) | 3 |
| 630 | N-[(cis-4-{[4-(dimethylamino)-5,6,7,8-tetrahydroquinazolin-2-yl] amino}cyclohexyl)methyl]-2-(4-fluorophenyl)acetamide | 440 (M + H) | 3 |
| 631 | N-[(cis-4-{[4-(dimethylamino)-5,6,7,8-tetrahydroquinazolin-2-yl] amino}cyclohexyl)methyl]-2-(4-methoxyphenyl)acetamide | 452 (M + H) | 1 |
| 632 | N-[(cis-4-[4-(dimethylamino)-5,6,7,8-tetrahydroquinazolin-2-yl] amino}cyclohexyl)methyl]-5-methyl-2-(trifluoromethyl)-3-furamide | 480 (M + H) | 1 |
| 633 | N-[(cis-4-{[4-(dimethylamino)-5,6,7,8-tetrahydroquinazolin-2-yl] amino}cyclohexyl)methyl]-2,5-dimethyl-3-furamide | 426 (M + H) | 3 |
| 634 | N-[(cis-4-{[4-(dimethylamino)-5,6,7,8-tetrahydroquinazolin-2-yl] amino}cyclohexyl)methyl]-2-ethoxybenzamide | 452 (M + H) | 3 |
| 635 | 3-chloro-N-[(cis-4-{[4-(dimethylamino)-5,6,7,8-tetrahydroquinazolin-2-yl]amino}cyclohexyl)methyl]-4-fluorobenzamide | 460 (M + H) | 3 |
| 636 | N-[(cis-4-{[4-(dimethylamino)-5,6,7,8-tetrahydroquinazolin-2-yl] amino cyclohexyl)methyl]-3-fluoro-4-methylbenzamide | 440 (M + H) | 3 |
| 637 | 2-cyclopentyl-N-[(cis-4-{[4-(dimethylamino)-5,6,7,8-tetrahydroquinazolin-2-yl]amino}cyclohexyl)methyl] acetamide | 414 (M + H) | 3 |
| 638 | N-[(cis-4-{[4-(dimethylamino)-5,6,7,8-tetrahydroquinazolin-2-yl] amino}cyclohexyl)methyl]-3,5-dimethoxybenzamide | 468 (M + H) | 3 |
| 639 | 4-cyano-N-[(cis-4-{[4-(dimethylamino)-5,6,7,8-tetrahydroquinazolin-2-yl]amino}cyclohexyl)methyl] benzamide | 433 (M + H) | 3 |

(continued)

| Ex. No. | compound name | MS | class |
|---|---|---|---|
| 640 | N-[(cis-4-{[4-(dimethylamino)-5,6,7,8-tetrahydroquinazolin-2-yl] amino}cydohexyl)methyl]-3,5-bis(trifluoromethyl)benzamide | 544 (M + H) | 3 |
| 641 | (2E)-N-[(cis-4-{[4-(dimethylamino)-5,6,7,8-tetrahydroquinazolin-2-yl]amino]cyclohexyl)methyl]-3-(4-nitrophenyl)acrylamide | 479 (M + H) | 2 |
| 642 | 2-(2-bromophenyl)-N-[(cis-4-{(4-(dimethylamino)-5,6,7,8-tetrahydroquinazolin-2-yl]amino}cyclohexyl)methyl] acetamide | 500 (M + H) | 3 |
| 643 | N-[(cis-4-{[4-(dimethylamino)-5,6,7,8-tetrahydroquinazolin-2-yl] amino}cyclohexyl)methyl]-4-fluoro-3-methylbenzamide | 440 (M + H) | 2 |
| 644 | 2-[(difluoromethyl)thio]-N-[(cis-4-{[4-(dimethylamino)-5,6,7,8-tetrahydroquinazolin-2-yl]amino}cyclohexyl)methyl] benzamide | 490 (M + H) | 3 |
| 645 | 2,5-dichloro-N-[(cis-4-{[4-(dimethylamino)-5,6,7,8-tetrahydroquinazolin-2-yl]amino}cyclohexyl)methyl] thiophene-3-carboxamide | 482 (M + H) | 3 |
| 646 | N-[(cis-4-{[4-(dimethylamino)-5,6,7,8-tetrahydroquinazolin-2-yl] amino}cyclohexyl)methyl]-2-(propylthio) nicotinamide | 483 (M + H) | 1 |
| 647 | 1-benzyl-3-tert-butyl-N-[(cis-4-{[4-(dimethylamino)-5,6,7,8-1 tetrahydroquinazolin-2-yl]amino}cyclohexyl)methyl]-1H-pyrazole-5-carboxamide | 544 (M + H) | 3 |
| 648 | 3-tert-butyl-N-[(cis-4-{[4-(dimethylamino)-5,6,7,8-tetrahydroquinazolin-2-yl]amino]cyclohexyl)methyl]-1-methyl-1H-pyrazole-5-carboxamide | 468 (M + H) | 3 |
| 649 | (2E)-N-[(cis-4-{[4-(dimethylamino)-5,6,7,8-tetrahydroquinazolin-2-yl]amino}cyclohexyl)methyl]-2-methyl-3-phenylacrylamide | 448 (M + H) | 3 |
| 650 | 5-bromo-N-[(cis-4-{[4-(dimethylamino)-5,6,7,8-tetrahydro-quinazolin-2-yl]amino}cyclohexyl)-methyl]nicotinamide | 487 (M + H) | 3 |
| 651 | N-[(cis-4-{[4-(dimethylamino)-5,6,7,8-tetrahydroquinazolin-2-yl] amino}cyclohexyl)methyl]-2-(1-naphthyl)acetamide | 472 (M + H) | 3 |
| 652 | 1-tert-butyl-N-[(cis-4-{[4-(dimethylamino)-5,6,7,8-tetrahydroquinazolin-2-yl]amino}cyclohexyl)methyl]-5-methyl-H-pyrazole-3-carboxamide | 468 (M + H) | 3 |
| 653 | N-[(cis-4-{[4-(dimethylamino)-5,6,7,8-tetrahydroquinazolin-2-yl] amino}cyelohexyl)methyl]-1-benzothiophene-3-carboxamide | 464 (M + H) | 3 |
| 654 | N-[(cis-4-{[4-(dimethylamino)-5,6,7,8-tetrahydroquinazolin-2-yl] amino}cyclohexyl)methyl]biphenyl-4-carboxamide | 484 (M + H) | 3 |
| 655 | 2-bromo-N-[(cis-4-{[4-(dimethylamino)-5,6,7,8-tetrahydroquinazolin-2-yl]amino}cyclohexyl)methyl] benzamide | 486 (M + H) | 3 |
| 656 | 2,6-dichloro-N-[(cis-4-{[4-(dimethylamino)-5,6,7,8-tetrahydroquinazolin-2-yl]amino}cyclohexyl)methyl] benzamide | 476 (M + H) | 2 |
| 657 | N-[(cis-4-{[4-(dimethylamino)-5,6,7,8-tetrahydroquinazolin-2-yl] amino}cyclohexyl)methyl]-2-iodobenzamide | 534 (M + H) | 3 |
| 658 | N-[(cis-4-{[4-(dimethylamino)-5,6,7,8-tetrahydroquinazolin-2-yl] amino}cyclohexyl)methyl]-2-methylbenzamide | 422 (M + H) | 3 |

(continued)

| Ex. No. | compound name | MS | class |
|---|---|---|---|
| 659 | 2,3-dichloro-N-[(cis-4-{[4-(dimethylamino)-5,6,7,8-tetrahydroquinazolin-2-yl]amino}cyclohexyl)methyl] benzamide | 476 (M + H) | 3 |
| 660 | 2-chloro-N-[(cis-4-{[4-(dimethylamino)-5,6,7,8-tetrahydro-quinazolin-2-yl]amino}cyclohexyl)methyl]-5-fluorobenzamide | 460 (M + H) | 3 |
| 661 | N-[(cis-4-{[4-(dimethylamino)-5,6,7,8-tetrahydroquinazolin-2-yl] amino)cyclohexyl)methyl]-9-oxo-9H-fluorene-4-carboxamide | 510 (M + H) | 3 |
| 662 | N-[(cis-4-{[4-(dimethylamino)-5,6,7,8-tetrahydroquinazolin-2-yl] amino}cyclohexyl)methyl]-2,3,6-trifluorobenzamide | 462 (M + H) | 3 |
| 663 | N-[(cis-4-{[4-(dimethylamino)-5,6,7,8-tetrahydroquinazolin-2-yl] amino}cyclohexyl)methyl]-2,3-difluorobenzamide | 444 (M + H) | 3 |
| 664 | N-[(cis-4-{[4-(dimethylamino)-5,6,7,8-tetrahydroquinazolin-2-yl] amino}cyclohexyl)methyl]-2,6-difluorobenzamide | 444 (M + H) | 3 |
| 665 | N-[(cis-4-{{[4-(dimethylamino)-5,6,7,8-tetrahydroquinazolin-2-yl] amino}cyclohexyl)methyl]-2-fluoro-6-(trifluoromethyl)-benzamide | 494 (M + H) | 3 |
| 666 | N-[(cis-4-{[4-(dimethylamino)-5,6,7,8-tetrahydroquinazolin-2-yl] amino}cyclohexyl)methyl]-2,4,6-trimethylbenzamide | 450 (M + H) | 2 |
| 667 | 2-chloro-N-[(cis-4-{[4-(dimethylamino)-5,6,7,8-tetrahydro-quinazolin-2-yl]amino}cyclohexyl)methyl]-6-fluorobenzamide | 460 (M + H) | 2 |
| 668 | 2,4,6-trichloro-N-[(cis-4-{[4-(dimethylamino)-5,6,7,8-tetrahydroquinazolin-2-yl]amino}cyclohexyl)methyl] benzamide | 510 (M + H) | 1 |
| 669 | (2E)-3-(2-chlorophenyl)-N-[(cis-4-{[4-(dimethylamino)-5,6,7,8-tetrahydroquinazolin-2-yl]amino}cyclohexyl)methyl] acrylamide | 468 (M + H) | 3 |
| 670 | 6-chloro-N-[(cis-4-{[4-(dimethylamino)-5,6,7,8-tetrahydro-quinazolin-2-yl]amino)cyclohexyl)methyl]-2-fluoro-3-methylbenzamide | 474 (M + H) | 3 |
| 671 | 2-chloro-N-[(cis-4-{[4-(dimethylamino)-5,6,7,8-tetrahydro-quinazolin-2-yl]amino}cyclohexyl)methyl]-3,6-difluorobenzamide | 478 (M + H) | 3 |
| 672 | N [(cis 4{[4 (dimethylamino)-5,6,7,8-tetrahydroquinazolin-2-yl] amino}cyelohexyl)methyl]-2,3-dimethylbenzamide | 436 (M + H) | 3 |
| 673 | 5-bromo-N-(cis-4-{[4-(dimethylamino)quinolin-2-yl]amino} cyclohexyl)thiophene-2-carboxamide | 473 (M + H) | 2 |
| 674 | N-(cis-4-{[4-(dimethylamino)quinolin-2-yl]amino}cyclohexyl)-2-(2,3,6-trichlorophenyl)acetamide | 505 (M + H) | 3 |
| 675 | 2-(2-chloro-4-fluorophenyl)-N-(cis-4-{[4(dimethylamino)quinolin-2-yl]amino}cyclohexyl)acetamide | 455 (M + H) | 3 |
| 676 | 5-(4-chloro-2-nitrophenyl)-N-(cis-4-{[4-(dimethylamino)quinolin-2-yl]amino}cyclohexyl)-2-furamide | 534 (M + H) | 2 |
| 677 | 5-chloro-N-(cis-4-{[4-(dimethylamino)quinolin-2-yl]amino} cyclohexyl)thiophene-2-carboxamide | 429 (M + H) | 2 |
| 678 | N-(cis-4-{[4-(dimethylamino)quinolin-2-yl]amino}cyclohexyl)-2,3-diphenylpropanamide | 493 (M + H) | 3 |

(continued)

| Ex. No. | compound name | MS | class |
|---|---|---|---|
| 679 | N-(cis-4-{[4-(dimethylamino)quinolin-2-yl]amino}cyclohexyl)-3-(2-hydroxyphenyl)propanamide | 433 (M + H) | 3 |
| 680 | N-(cis-4-{[4-(dimethylamino)quinolin-2-yl]amino)cyclohexyl)-5-iodo-2-furamide | 505 (M + H) | 1 |
| 681 | N-(cis-4-{[4-(dimethylamino)quinolin-2-yl]amino]cyclohexyl)-2-(2-iodophenyl)acetamide | 529 (M + H) | 2 |
| 682 | N-(cis-4-{[4-(dimethylamino)quinolin-2-yl]amino}cyclohexyl)-2-(5-methoxy-2-methyl-1H-indol-3-yl)acetamide | 486 (M + H) | 2 |
| 683 | (2E)-N-(cis-4-{[4-(dimethylamino)quinolin-2-yl]amino}cyclohexyl)-3-(3-nitrophenyl)acrylamide | 460 (M + H) | 2 |
| 684 | N-(cis-4-{[4-(dimethylamino)quinolin-2-yl]amino}cyclohexyl)-3-oxoindane-1-carboxamide | 443 (M + H) | 3 |
| 685 | 2-benzyl-N-(cis-4-{[4-(dimethylamino)quinolin-2-yl]amino} cyclohexyl)benzamide | 479 (M + H) | 3 |
| 686 | 2,2-bis(4-chlorophenyl)-N-(cis-4-1[4-(dimethylamino)quinolin-2-yl]amino}cyclohexyl)acetamide | 547 (M + H) | 2 |
| 687 | N-(cis-4-{[4-(dimethylamino)quinolin-2-yl]amino)cyclohexyl)-5-(4-methyl-2-nitrophenyl)-2-furamide | 514 (M + H) | 3 |
| 688 | N-(cis-4-{[4-(dimethylamino)quinolin-2-yl]amino}cyclohexyl)-5-nitrothiophene-2-carboxamide | 440 (M + H) | 1 |
| 689 | N-(cis-4-{[4-(dimethylamino}quinolin-2-yl]amino cyclohexyl)-3-methyl-4-nitrobenzamide | 448 (M + H) | 1 |
| 690 | N-(cis-4-{[4-(dimethylamino)quinolin-2-yl]amino}cyclohexyl)-3-methoxy-4-nitrobenzamide | 464 (M + H) | 1 |
| 691 | 1-benzyl-N-(cis-4-{[4-(dimethylamino)quinolin-2-yl]amino} cyclohexyl)-1H-indole-3-carboxamide | 518 (M + H) | 3 |
| 692 | 3-acetyl-N-(cis-4-{[4-(dimethylamino)quinolin-2-yl]amino} cyclohexyl)benzamide | 431 (M + H) | 3 |
| 693 | (2R)-2-benzoyl-N-(cis-4-{[4-(dimethylamino)quinolin-2-yl]amino} cyclohexyl)cyclohexanecarboxamide | 499 (M + H) | 3 |
| 694 | 5-bromo-N-(cis-4-{[4-(dimethylamino)quinolin-2-yl]amino} cyclohexyl)-2-furamide | 457 M + H) | 1 |
| 695 | 3-cyclohexyl-N-(cis-4-{[4-(dimethylamino)quinolin-2-yl]amino} cyclohexyl)propanamide | 423 (M + H) | 3 |
| 696 | N-(cis-4-{[4-(dimethylamino)quinolin-2-yl]amino)cyclohexyl)-2-[(4-methylpyrimidin-2-yl)thio]acetamide | 451 (M + H) | 3 |
| 697 | 5(4-chlorophenyl)-N-(cis-4-{[4-(dimethylamino)quinolin-2-yl] amino}cyclohexyl)-2-furamide | 489 (M + H) | 3 |
| 698 | 3-(3,4-dichlorophenyl)-N-(cis-4-{[4-(dimethylamino)quinolin-2-yl] amino}cyclohexyl)propanamide | 485 (M + H) | 3 |
| 699 | 2-(3,4-dichlorophenyl)-N-(cis-4-{[4-(dimethylamino)quinolin-2-yl] amino}cyclohexyl)acetamide | 471 (M + H) | 3 |
| 700 | N-(cis-4-{[4-(dimethylamino)quinolin-2-yl]amino) cyclohexyl)-2-(4-hydroxy-3,5-dimethoxyphenyl)acetamide | 479 (M + H) | 3 |

(continued)

| Ex. No. | compound name | MS | class |
|---|---|---|---|
| 701 | 4,5-dibromo-N-(cis-4-{[4-(dimethylamino)quinolin-2-yl]amino} cyclohexyl)thiophene-2-carboxamide | 551 (M + H) | 2 |
| 702 | 2-(3,5-dimethoxyphenyl)-N-(cis-4-{[4-(dimethylamino)quinolin-2-yl]amino}cyclohexyl)acetamide | 463 (M + H) | 3 |
| 703 | 2-(3,5-di-tert-butyl-4-hydroxyphenyl)-N-(cis-4-{[4-(dimethylamino) quinolin-2-yl]amino}cyclohexyl)acetamide | 531 (M + H) | 3 |
| 704 | N-2-,N-6-dibenzoyl-N-(cis-4-{[4-(dimethylamino)quinolin-2-yl] amino}cyclohexyl)lysinamide | 621 (M + H) | 3 |
| 705 | 3-(dimethylamino)-N-(cis-4-{[4-(dimethylamino)quinolin-2-yl] anuno}cyclohexyl)benzamide | 432 (M + H) | 3 |
| 706 | 4,5-dibromo-N-(cis-4-{[4-(dimethylamino)quinolin-2-yl]amino} cyclohexyl)-2-furamide | 535 (M + H) | 1 |
| 707 | N-(cis-4-{[4-(dimethylamino)quinolin-2-yl]amino}cyclohexyl)-4-(4-fluorophenyl)-4-oxobutanamide | 463 (M + H) | 3 |
| 708 | N-(cis-4-{[4-(dimethylamino)quinolin-2-yl]amino}cyclohexyl)-2-(2-fluorobiphenyl-4-yl)propanamide | 511 (M + H) | 3 |
| 709 | tert-butyl {(1S)-1-[(1-benzyl-1H-imidazol-4-yl)methyl]-2-[(cis-4-{ [4-(dimethylamino)guinolin-2-yl]amino}cyclohexyl)amino]-2-oxoethyl}carbamate | 612 (M + H) | 3 |
| 710 | N-(cis-4-1[4-(dimethylamino)quinolin-2-yl]amino)cyclohexyl)-2-(M [4-(1-oxo-1,3-dihydro-2H-isoindol-2-yl)phenyl]propanamide | 548 (M + H) | 3 |
| 711 | N-(cis-4-{[4-(dimethylamino)quinolin-2-yl]amino)cyclohexyl)-2-(1H-indol-3-yl)acetamide | 442 (M + H) | 1 |
| 712 | N-(cis-4-{[4-(dimethylamino)quinolin-2-yl]amino}cyclohexyl)-2-(5-methyl-2-phenyl-1,3-thiazol-4-yl)acetamide | 500 (M + H) | 3 |
| 713 | N-(cis-4-{[4-(dimethylamino)quinolin-2-yl]amino)cyclohexyl)-2-(6-methoxy-3-oxo-2,3-dihydro-1H-inden-1-yl)acetamide | 487 (M + H) | 3 |
| 714 | N-(cis-4-{[4-(dimethylamino)quinolin-2-yl]amino}cyclohexyl)-2-{1-[(4-methoxybenzyl)thio]cyclohexyl}acetamide | 561 (M + H) | 3 |
| 715 | N-(cis-4-{[4-(dimethylamino)quinolin-2-yl]amino}cyclohexyl)-2-(7-methoxy-2-oxo-2H-chromen-4-yl)acetamide | 501 (M + H) | 3 |
| 716 | N-(cis-4-{[4-(dimethylamino)quinolin-2-yl]amino}cyclohexyl)-2-(1H-indol-3-yl)-4-oxo-4-phenylbutanamid | 560 (M + H) | 2 |
| 717 | 4-(4-bromophenyl)-N-(cis-4-{[4-(dimethylamino)quinolin-2-yl) amino}cyclohexyl)-2-(1H-indol-3-yl)-4-oxobutanamide | 638 (M + H) | 3 |
| 718 | N-(cis-4-{[4-(dimethylamino)quinolin-2-yl]amino}cyclohexyl)-3,5-dimethyl-2-[({[4(trifluoromethoxy)phenyl]amino}-carbonyl) amino]benzamide | 635 (M + H) | 3 |
| 719 | 3,5-dichloro-N-(cis-4-{[4-(dimethylamino)quinolin-2-yl]amino} cyclohexyl)-2-[(3-phenylprop-2-ynoyl)amino]benzamide | 600 (M + H) | 3 |
| 720 | 4-(4-tert-butylphenyl)-N-(cis-4-{[4-(dimethylamino)quinolin-2-yl] amino}cyclohexyl)-2-(7-ethy]-1H-indol-3-yl)-4-oxobutanamide | 644 (M + H) | 3 |
| 721 | 4-(4-tert-butylphenyl)-N-(cis-4-{[4-(dimethylamino)quinolin-2-yl] amino}cyclohexyl)-2-(1-methyl-1H-indol-3-yl)-4-oxobutanamide | 630 (M + H) | 3 |

(continued)

| Ex. No. | compound name | MS | class |
|---|---|---|---|
| 722 | N-(cis-4-{[4-(dimethylamino)quinolin-2-yl]amino}cyclohexyl)-2-(1-methyl-1H-indol-3-yl)-4-(4-methylphenyl)-4-oxobutanamide | 588 (M + H) | 3 |
| 723 | N-(2,4-dichlorophenyl)-2-{2-[(cis-4-{[4-(dimethylamino)quinolin-2-yl]amino}cyclohexyl)amino]-2-oxoethyl)benzamide | 590 (M + H) | 3 |
| 724 | N-(cis-4-{[4-(dimethylamino)quinolin-2-yl]amino}cyclohexyl)-2-methyl-1-(3-morpholin-4-ylpropyl)-5-phenyl-1H-pyrrole-3-carboxamide | 595 (M + H) | 3 |
| 725 | N-(cis-4-{[4-(dimethylamino)quinolin-2-yl]amino}cyclohexyl)-4-(4-nitrophenyl)butanamide | 476 (M + H) | 3 |
| 726 | (2E)-N-(cis-4-{[4-(dimethylamino)quinolin-2-yl]amino}cyclohexyl)-3-(2-nitrophenyl)acrylamide | 460 (M + H) | 3 |
| 727 | N-(cis-4-{[4-(dimethylamino)quinolin-2-yl)amino}cyclohexyl)-2-(3-phenoxyphenyl)acetamide | 495 (M + H) | 3 |
| 728 | N-(cis-4-{[4-(dimethylamino)quinolin-2-yl]amino}cyclohexyl)-2-(4-phenoxyphenyl)acetamide | 495 (M + H) | 3 |
| 729 | N-(cis-4-{[4-(dimethylamino)quinolin-2-yl]amino}cyclohexyl)-2-(2-phenyl-1H-indol-3-yl)acetamide | 518 (M + H) | 2 |
| 730 | N2-benzoyl-N5-(cis-4-{[4-(dimethylamino)quinolin-2-yl]amino}cyclohexyl)-N1,N1-dipropylglutamamide | 601 (M + H) | 3 |
| 731 | N-(cis-4-{[4-(dimethylamino)quinolin-2-yl]amino}cyclohexyl)-3-phenoxybenzamide | 481 (M + H) | 3 |
| 732 | N-(cis-4-{[4-(dimethylamino)quinolin-2-yl]amino}cyclohexyl)-2-(2-phenylethyl)benzamide | 493 (M + H) | 3 |
| 733 | 3-benzoyl-N-(cis-4-{[4-(dimethylamino)quinolin-2-yl]amino}cyclohexyl)benzamide | 493 (M + H) | 3 |
| 734 | N-(cis-4-{[4-(dimethylamino)quinolin-2-yl]amino}cyclohexyl)-2-(ethylthio)-2,2-diphenylacetamide | 539 (M + H) | 3 |
| 735 | 2-[(2-cyanophenyl)thio]-N-(cis-4-{[4-(dimethylamino)quinolin-2-yl]amino}cyclohexyl)benzamide | 522 (M + H) | 3 |
| 736 | 2-[4-(benzyloxy)-3-methoxyphenyl]-N-(cis-4-{[4-(dimethylamino)quinolin-2-yl]amino}cyclohexyl)acetamide | 539 (M + H) | 3 |
| 737 | N-(cis-4-{[4-(dimethylamino)quinolin-2-yl]amino}cyclohexyl)-N'-[(1R)-1-(1-naphthyl)ethyl]phthalamide | 586 (M + H) | 3 |
| 738 | (2S)-2-(3-benzoylphenyl)-N-(cis-4-{[4-(dimethylamino)quinolin-2-yl]amino}cyclohexyl)propanamide | 521 (M + H) | 3 |
| 739 | N'-(cis-4-{[4-(dimethylamino)quinolin-2-yl]amino}cyclohexyl)-N,N-bis[(1S)-1-phenylethyl]phthalamide | 640 (M + H) | 3 |
| 740 | (2S)-N-(cis-4-{[4-(dimethylamino)quinolin-2-yl]amino}cyclohexyl)-2-(2-fluorobiphenyl-4-yl)propanamide | 511 (M + H) | 3 |
| 741 | 2-[(4-chlorobenzyl)thio]-4-(4-chlorophenyl)-N-(cis-4-{[4-(dimethylamino)quinolin-2-yl]amino}cyclohexyl)-4-oxobutanamide | 635 (M + H) | 3 |
| 742 | 2-[(4-chlorobenzyl)thio]-N-(cis-4-{[4-(dimethylamino)quinolin-2-yl]amino}cyclohexyl)-4-(4-methylphenyl)-4-oxobutanamide | 615 (M + H) | 3 |

(continued)

| Ex. No. | compound name | MS | class |
|---|---|---|---|
| 743 | N-(cis-4-{[4-(dimethylamino)quinolin-2-yl]amino}cyclohexyl)-2-{(1E)-5-fluoro-2-methyl-1-[4-(methylsulfinyl)benzylidene]-1H-inden-3-yl}acetamide | 623 (M + H) | 3 |
| 744 | N-(cis-4-{[4-(dimethylamino)quinolin-2-yl]amino}cyclohexyl)-2-[4-(2-thienylcarbonyl)phenyl]propanamide | 527 (M + H) | 3 |
| 745 | N-(cis-4-{[4-(dimethylamino)quinolin-2-yl]amino}cyclohexyl)-4-oxo-4-(2-thienyl)butanamide | 451 (M + H) | 3 |
| 746 | N-(cis-4-{[4-(dimethylamino)quinolin-2-yl]amino}cyclohexyl)-4-(2-thienyl)butanamide | 437 (M + H) | 3 |
| 747 | N-(cis-4-{[4-(dimethylamino)quinolin-2-yl]amino}cyclohexyl)-2-(2,4,6-trichlorophenoxy)acetamide | 521 (M + H) | 2 |
| 748 | 2-[5-(benzyloxy)-1H-indol-3-yl]-N-(cis-4-{[4-(dimethylamino) quinolin-2-yl]amino}cyclohexyl)acetamide | 548(M + H) | 3 |
| 749 | N-(cis-4-{[4-(dimethylamino)quinolin-2-yl]amino}cyclohexyl)-2-(1-naphthoyl)benzamide | 543 (M + H) | 3 |
| 750 | 3-(benzyloxy)-N-(cis-4-{[4-(dimethylamino)quinolin-2-yl]amino} cyclohexyl)-4-methoxybenzamide | 525 (M + H) | 2 |
| 751 | N-(cis-4-{[4-(dimethylamino)quinolin-2-yl]amino}cyclohexyl)-2-methyl-1,5-diphenyl-1H-pyrrole-3-carboxamide | 544 (M + H) | 3 |
| 752 | 1-{2-[(2-chloro-6-fluorobenzyl)thio]ethyl-N-(cis-4-{[4-(dimethylamino)quinolin-2-yl]amino}cyclohexyl)-2-methyl-5-phenyl-1H-pyrrole-3-carboxamide | 670 (M + H) | 3 |
| 753 | N-(cis-4-{[4-(dimethylamino)quinolin-2-yl]amino}cyclohexyl) anthracene-9-carboxamide | 489 (M + H) | 3 |
| 754 | N-(cis-4-{[4-(dimethylamino)quinolin-2-yl]amino)cyclohexyl)-2-phenoxybenzamide | 481 (M + H) | 2 |
| 755 | N-(cis-4-{[4-(dimethylamino)quinolin-2-yl]amino}cyclohexyl) biphenyl-2-carboxamide | 465 (M + H) | 3 |
| 756 | N-(cis-4-{[4-(dimethylamino)quinolin-2-yl]amino}cyclohexyl)-3,3-diphenylpropanamide 3,3-diphenylpropanamide | 493 (M + H) | 3 |
| 757 | N-(cis-4-{[4-(dimethylamino)quinolin-2-yl]amino)cyclohexyl)-2-phenylquinoline-4-carboxamide phenylquinoline-4-carboxamide | 516 (M + H) | 2 |
| 758 | N-(cis-4-([4-(dimethylamino)quinolin-2-yl]amino)cyclohexyl) -N'-[(1S)-1-phenylethyl]phthalamide | 536 (M + H) | 3 |
| 759 | N-(cis-4-{[4-(dimethylamino)quinolin-2-yl]amino}cyclohexyl)-2-(4-methylbenzoyl)benzamide | 507 (M + H) | 3 |
| 760 | N-(cis-4-{[4-(dimethylamino)quinolin-2-yl]amino}cyclohexyl)-2-(phenoxymethyl)benzamide | 495 (M + H) | 3 |
| 761 | 2-[4-(4-chlorophenyl)-2-phenyl-1,3-thiazol-5-yl]-N-(cis-4-{[4-dimethylamino)quinolin-2-yl]amino}cyclohexyl)acetamide | 596 (M + H) | 3 |
| 762 | N-(cis-4-{[4-(dimethylamino)quinolin-2-yl]amino}cyclohexyl)-1-[(4-methylphenyl)sulfonyl]-1H-pyrrole-3-carboxamide | 532 (M + H) | 3 |
| 763 | N-(cis-4-{[4-(dimethylamino)quinolin-2-yl]amino}cyclohexyl)-5-(3-nitrophenyl)-2-furamide | 500 (M + H) | 1 |

(continued)

| Ex. No. | compound name | MS | class |
|---|---|---|---|
| 764 | 3-chloro-N-(cis-4-{[4-(dimethylamino)quinolin-2-yl]amino} cyclohexyl)-4-(methylsulfonyl)thiophene-2-carboxamide | 507 (M + H) | 3 |
| 765 | 3-chloro-N-(cis-4-{[4-(dimethylamino)quinolin-2-yl]amino} cyclohexyl)-4-(isopropylsulfonyl)-5-(methylthio)thiophene-2-carboxamide | 581 (M + H) | 3 |
| 766 | N-(cis-4-{[4-(dimethylamino)quinolin-2-yl]amino}cyclohexyl)-3-iodo-4-(isopropylsulfonyl)-5-(methylthio)thiophene-2-carboxamide | 673 (M + H) | 3 |
| 767 | N-(cis-4-{[4-(dimethylamino)quinolin-2-yl]amino)cyclohexyl)-5-nitrothiophene-3-carboxamide | 440 (M + H) | 1 |
| 768 | N-(cis-4-{[4-(dimethylamino)quinolin-2-yl]amino}cyclohexyl)-1-methyl-4-nitro-1H-pyrrole-2-carboxamide | 437 (M +H) | 1 |
| 769 | N-(cis-4-{[4-(dimethylamino)quinolin-2-yl]amino}cyclohexyl)-1-(phenylsulfonyl)-1H-indole-3-carboxamide | 568 (M + H) | 3 |
| 770 | N-(cis-4-{[4-(dimethylamino)quinolin-2-yl]amino}cyclohexyl)-4-nitrobenzamide | 434 (M + H) | 1 |
| 771 | N-(cis-4-{[4-(dimethylamino)quinolin-2-yl]amino}cyclohexyl)-2-methoxy-4-nitrobenzamide | 464 (M + H) | 2 |
| 772 | N-(cis-4-{[4-(dimethylamino)quinolin-2-yl]amino}cyclohexyl)-3-fluoro-4-(trifluoromethyl)benzamide | 475 (M + H) | 1 |
| 773 | N-(cis-4-{[4-(dimethylamino)quinolin-2-yl]amino}cyclohexyl)-2-fluoro-4-nitrobenzamide | 452 (M + H) | 3 |
| 774 | N-(cis-4-{[4-(dimethylamino)quinolin-2-yl]amino}cyclohexyl)-3,5-dimethyl-4-nitrobenzamide | 462 (M + H) | 2 |
| 775 | N-(cis-4-{[4-(dimethylamino)quinolin-2-yl]amino)cyclohexyl)-2-mesityl-2-oxoacetamide | 459 (M + H) | 2 |
| 776 | N-(cis-4-{[4-(dimethylamino)quinolin-2-yl]amino}cyclohexyl) quinolin-3-carboxamide | 440 (M + H) | 3 |
| 777 | N-(cis-4-{[4-(dimethylamino)quinolin-2-yl]amino}cyclohexyl)-2-methoxy-2-phenylacetamide | 433 (M + H) | 3 |
| 778 | N-(cis-4-{[4-(dimethylamino)quinolin-2-yl]amino}cyclohexyl)-1,2,3,4-tetrahydronaphthalene-2-carboxamide | 443 (M + H) | 3 |
| 779 | N-(cis-4-{[4-(dimethylamino)quinolin-2-yl]amino}cyclohexyl)-1,3-benzothiazole-6-carboxamide | 446 (M + H) | 3 |
| 780 | 5-chloro-N-(cis-4-{[4-(dimethylamino)quinolin-2-yl]amino} cyclohexyl)-2-hydroxybenzamide | 439 (M + H) | 2 |
| 781 | 2-chloro-N-(cis-4-{[4-(dimethylamino)quinolin-2-yl]amino} cyclohexyl)-5-(methylthio)benzamide | 469 (M + H) | 3 |
| 782 | N-(cis-4-{[4-(dimethylamino)quinolin-2-yl]amino}cyclohexyl)-7-methoxy-1-benzofuran-2-carboxamide | 459 (M + H) | 3 |
| 783 | 2-amino-N-(cis-4-{[4-(dimethylamino)quinolin-2-yl]amino} cyclohexyl)-3-methylbenzamide | 418 (M + H) | 3 |
| 784 | N-(cis-4-{[4-(dimethylamino)quinolin-2-yl]amino)cyclohexyl)-4-hydroxy-3,5-dimethoxybenzamide | 465 (M + H) | 3 |

(continued)

| Ex. No. | compound name | MS | class |
|---|---|---|---|
| 785 | N-(cis-4-{[4-(dimethylamino)quinolin-2-yl]amino}cyclohexyl) quinoline-4-carboxamide | 440 (M + H) | 3 |
| 786 | 2-(allylthio)-N-(cis-4-{[4-(dimethylamino)quinolin-2-yl]amino} cyclohexyl)nicotinamide yl]amino}cyclohexyl)nicotinamide | 462 (M + H) | 3 |
| 787 | 3,5-di-tert-butyl-N-(cis-4-{[4-(dimethylamino)quinolin-2-yl]amino} cyclohexyl)-4-hydroxybenzamide | 517 (M + H) | 3 |
| 788 | 5-bromo-N-[(cis-4-{[4-(dimethylamino)quinolin-2-yl]amino} cyclohexyl)methyl]thiophene-2-carboxamide | 487 (M + H) | 3 |
| 789 | N-[(cis-4-{[4-(dimethylamino)quinolin-2-yl]amino}cyclohexyl) methyl]-2-(2,3,6-trichlorophenyl)acetamide | 519 (M + H) | 1 |
| 790 | 2-(2-chtoro-4-nuorophenyl)-N-[(cis-4-{[4-(dimethylamino)-quinolin-2-yl]amino}cyclohexyl)-methyl]acetamide | 469 (M+H) | 3 |
| 791 | 5-(4-chloro-2-nitrophenyl)-N-[(cis-4-{[4-(dimethylamino)-quinolin-2-yl]amino}cyclohexyl)methyl]-2-furamide | 548 (M+H) | 3 |
| 792 | 5-chloro-N-[(cis-4-{[4-(dimethylamino)quinolin-2-yl]amino} cyclohexyl)methyl]thiophene-2-carboxamide | 443 (M+H) | 3 |
| 793 | N-[(cis-4-([4-(dimethylamino)quinolin-2-yl]amino}cyclohexyl) methyl}-2,3-diphenylpropanamide | 507 (M + H) | 3 |
| 794 | N-[(cis-4-)[4-(dimethylamino)quinolin-2-yl]amino}cyclohexyl) methyl)-3-(2-hydroxyphenyl)propanamide | 447 (M + H) | 3 |
| 795 | N-[(cis-4-{[4-(dimethylamino)quinolin-2-yl]amino}cyclohexyl) methyl]-5-iodo-2-furamide | 519 (M + H) | 3 |
| 796 | N-[(cis-4-{[4-(dimethylamino)quinolin-2-yl]amino}cyclohexyl} methyl]-2-(2-iodophenyl)acetamide | 543 (M + H) | 3 |
| 797 | (2E)-N-[(cis-4-{[4-(dimethylamino)quinolin-2-yl]amino}cyclohexyl) methyl]-3-(3-nitrophenyl)acrylamide | 474 (M + H) | 2 |
| 798 | N-[(cis-4-{[4-(dimethylamino)quinolin-2-yl]amino}cyclohexyl) methyl]-3-oxoindane-1-carboxamide | 457 (M + H) | 3 |
| 799 | 2-benzyl-N-[(cis-4-{[4-(dimethylamino)quinolin-2-yl]amino ) cyclohexyl)methyl]benzamide | 493 (M+H) | 3 |
| 800 | 2,2-bis(4-chlorophenyl)-N-[(cis-4-{[4-(dimethylamino)quinolin-2-yl]amino}cyclohexyl)methyl]acetamide | 561 (M + H) | 3 |
| 801 | N-[(cis-4-{[4-(dimethylamino)quinolin-2-yl]amino}cyclohexyl)-methyl]-5-(4-methyl-2-nitrophenyl)-2-furamide | 528 (M + H) | 3 |
| 802 | N-[(cis-4-{[4-(dimethylamino)quinolin-2-yl]amino}cyclohexyl) methyl]-5-nitrothiophene-2-carboxamide | 454 (M + H) | 3 |
| 803 | N-[(cis-4-{[4-(dimethylamino)quinolin-2-yl]amino}cyclohexyl) methyl]-3-methyl-4-nitrobenzamide | 462 (M + H) | 3 |
| 804 | N-[(cis-4-{[4-(dimethylamino)quinolin-2-yl]amino}cyclohexyl) methyl]-3-methoxy-4-nitrobenzamide | 478 (M+H) | 3 |
| 805 | 1-benzyl-N-[(cis-4-{[4-(dimethylamino)quinolin-2-yl]amino} cyclohexyl)methyl]-1H-indole-3-carboxamide | 532 (M+H) | 3 |
| 806 | 2-cyclohex-1-en-1-yl-N-[(cis-4-{[4-(dimethylamino)quinolin-2-yl] ammo)cyclohexyl)methyl]acetamide | 421 (M + H) | 3 |

(continued)

| Ex. No. | compound name | MS | class |
|---|---|---|---|
| 807 | N-[(cis-4-{[4-(dimethylamino)quinolin-2-yl]amino}cyclohexyl) methyl]-4-(4-ethoxyphenyl)-2-(3-methyl-5-oxo-1-phenyl-4,5-dihydro-1H-pyrazol-4-yl)-4-oxobutanamide | 675 (M + H) | 3 |
| 808 | N-[(cis-4-{[4-(dimethylamino)quinolin-2-yl]amino}cyclohexyl)-methyl]-2-[2-(trifluoromethoxy)phenyl]acetamide | 501 (M + H) | 3 |
| 809 | 4-(benzyloxy)-N-[(cis-4-{[4-(dimethylamino)quinolin-2-yl]amino} cyclohexyl)methyl]-3,5-dimethylbenzamide | 537 (M + H) | 3 |
| 810 | N-[(cis-4-{[4-(dimethylamino)quinolin-2-yl]amino)cyclohexyl) methyl]-9H-xanthene-9-carboxamide | 507 (M + H) | 3 |
| 811 | 2-(1-benzothien-3-yl)-N-[(cis-4-{[4-(dimethylamino)quinolin-2-yl] amino}cyclohexyl)methyl]acetamide | 473 (M + H) | 3 |
| 812 | 5-bromo-N-(cis-4-{[4-(dimethylamino)pyrimidin-2-yl]amino} cyclohexyl)thiophene-2-carboxamide | 424 (M + H) | 3 |
| 813 | N-(cis-4-{[4-(dimethylamino)pyrimidin-2-yl]amino}cyclohexyl)-2-(2,3,6-trichlorophenyl)acetamide | 456 (M + H) | 3 |
| 814 | 2-(2-chloro-4-fluorophenyl)-N-(cis-4-{[4-(dimethylamino) pyrimidin-2-yl]amino}cyclohexyl)acetamide | 406 (M + H) | 3 |
| 815 | 5-(4-chloro-2-nitrophenyl)-N-(cis-4-{[4-(dimethylamino)pyrimidin-2-yl)amino}cyclohexyl)-2-furamide | 485 (M + H) | 1 |
| 816 | 5-chloro-N-(cis-4-{[4-(dimethylamino)pyrimidin-2-yl]amino} cyclohexyl)thiophene-2-carboxamide | 380 (M + H) | 3 |
| 817 | N-(cis-4-{[4-(dimethylamino)pyrimidin-2-yl]amino}cyclohexyl)-2,3-diphenylpropanamide | 444 (M + H) | 3 |
| 818 | N-(cis-4-{[4-(dimethylamino)pyrimidin-2-yl]amino}cyclohexyl)-3-(2-hydroxyphenyl)propanamide | 384 (M + H) | 3 |
| 819 | N-(cis-4-{[4-(dimethylamino)pyrimidin-2-yl]amino}cyclohexyl)-5-iodo-2-furamide | 456 (M + H) | 2 |
| 820 | N-(cis-4-{[4-(dimethylamino)pyrimidin-2-yl]amino}cyclohexyl)-2-(2-iodophenyl)acetamide | 480 (M + H) | 3 |
| 821 | N-(cis-4-{[4-(dimethylamino)pyrimidin-2-yl]amino)cyclohexyl)-2-(5-methoxy-2-methyl-1H-indol-3-yl)acetamide | 437 (M +H) | 3 |
| 822 | (2E)-N-(cis-4-{[4-(dimethylamino)pyrimidin-2-yl]amino} cyclohexyl)-3-(3-nitrophenyl)acrylamide | 411 (M + H) | 3 |
| 823 | N-(cis-4-{[4-(dimethylamino)pyrimidin-2-yl]amino}cyclohexyl)-3-oxoindane-1-carboxamide | 394 (M + H) | 3 |
| 824 | 2-benzyl-N-(cis-4-{[4-(dimethylamino)pyrimidin-2-yl]amino} cyclohexyl)benzamide | 430 (M + H) | 3 |
| 825 | 2,2-bis(4-chlorophenyl)-N-(cis-4-{[4-(dimethylamino)pyrimidin-2-yl]amino}cyclohexyl)acetamide | 498 (M + H) | 3 |
| 826 | N-(cis-4-{[4-(dimethylamino)pyrimidin-2-yl]amino}cyclohexyl)-5-(4-methyl-2-nitrophenyl)-2-furamide | 465 (M + H) | 2 |
| 827 | N-(cis-4-{[4-(dimethylamino)pyrimidin-2-yl]amino}cyclohexyl)-5-nitrothiophene-2-carboxamide | 391 (M + H) | 2 |
| 828 | N-(cis-4-{[4-(dimethylamino)pyrimidin-2-yl]amino}cyclohexyl)-3-methyl-4-nitrobenzamide | 399 (M + H) | 2 |

(continued)

| Ex. No. | compound name | MS | class |
|---|---|---|---|
| 829 | N-(cis-4-{[4-(dimethylamino)pyrimidin-2-yl]amino}cyclohexyl)-3-methoxy-4-nitrobenzamide | 415 (M + H) | 1 |
| 830 | 1-benzyl-N-(cis-4-{[4-(dimethylamino)pyrimidin-2-yl]amino}cyclohexyl)-1H-indole-3-carboxamide | 469 (M + H) | 2 |
| 831 | 3-acetyl-N-(cis-4-{[4-(dimethylamino)pyrimidin-2-yl]amino}cyclohexyl)benzamide | 382 (M + H) | 2 |
| 832 | (2R)-2-benzoyl-N-(cis-4-{[4-(dimethylamino)pyrimidin-2-yl]amino}cyclohexyl)cyclohexanecarboxamide | 450 (M + H) | 3 |
| 833 | 5-bromo-N-(cis-4-{[4-(dimethylamino)pyrimidin-2-yl]amino}cyclohexyl)-2-furamide | 408 (M + H) | 1 |
| 834 | 3-cyclohexyl-N-(cis-4-{[4-(dimethylamino)pyrimidin-2-yl]amino}cyclohexyl)propanamide | 374 (M + H) | 3 |
| 835 | N-(cis-4-{[4-(dimethylamino)pyrimidin-2-yl]amino}cyclohexyl)-2-[(4-methylpyrimidin-2-yl)thio]acetamide | 402 (M + H) | 3 |
| 836 | 5-(4-chlorophenyl)-N-(cis-4-{[4-(dimethylamino)pyrimidin-2-yl]amino}cyclohexyl)-2-furamide | 440 (M + H) | 1 |
| 837 | 3-(3,4-dichlorophenyl)-N-(cis-4-{4-(dimethylamino)pyrimidin-2-yl]amino}cyclohexyl)propanamide | 436 (M + H) | 3 |
| 838 | 2-(3,4-dichlorophenyl)-N-(cis-4-{[4-(dimethylamino)pyrimidin-2-yl]amino}cyclohexyl)acetamide | 422 (M + H) | 3 |
| 839 | N-(cis-4-{[4-(dimethylamino)pyrimidin-2-yl]amino}cyclohexyl)-2-(4-hydroxy-3,5-dimethoxyphenyl)acetamide | 430 (M + H) | 3 |
| 840 | 4,5-dibromo-N-(cis-4-{[4-(dimethylamino)pyrimidin-2-yl]amino}cyclohexyl)thiophene-2-carboxamide | 501 (M + H) | 2 |
| 841 | 2-(3,5-dimethoxyphenyl)-N-(cis-4-{[4-(dimethylamino)pyrimidin-2-yl]amino}cyclohexyl)acetamide | 414 (M + H) | 3 |
| 842 | 2-(3,5-di-tert-butyl-4-hydroxyphenyl)-N-(cis-4-{[4-(dimethylamino)pyrimidin-2-yl]amino}cyclohexyl)acetamide | 482 (M + H) | 1 |
| 843 | N2,N6-dibenzoyl-N-(cis-4-{[4-(dimethylamino)pyrimidin-2-yl]amino}cyclohexyl)lysinamide | 572 (M + H) | 2 |
| 844 | 3-(dimethylamino)-N-(cis-4-{[4-(dimethylamino)pyrimidin-2-yl]amino}cyclohexyl)benzamide | 383 (M + H) | 2 |
| 845 | 4,5-dibromo-N-(cis-4-{[4-(dimethylamino)pyrimidin-2-yl]amino}cyclohexyl)-2-furamide | 486 (M + H) | 1 |
| 846 | N-(cis-4-{[4-(dimethylamino)pyrimidin-2-yl]amino}cyclohexyl)-4-(4-fluorophenyl)-4-oxobutanamide | 414 (M + H) | 3 |
| 847 | N-(cis-4-{[4-(dimethylamino)pyrimidin-2-yl]amino}cyclohexyl)-2-(2-fluorobiphenyl-4-yl)propanamide | 462 (M + H) | 3 |
| 848 | 1-benzyl-Nalpha-(tert-butoxycarbonyl)-N-(cis-4-{[4-(dimethylamino)pyrimidin-2-yl]amino ) cyclohexyl)-L-histidinamide | 563 (M + H) | 3 |
| 849 | N-(cis-4-{[4-(dimethylamino)pyrimidin-2-yl]amino}cyclohexyl)-2-[4-(1-oxo-1,3-dihydro-2H-isoindol-2-yl)phenyl]propanamide | 499 (M + H) | 3 |
| 850 | N-(cis-4-{[4-(dimethylamino)pyrimidin-2-yl]amino}cyclohexyl)-393 (M + H) 2-(1H-indol-3-yl)acetamide | 393 (M+H) | 2 |

(continued)

| Ex. No. | compound name | MS | class |
|---|---|---|---|
| 851 | N-(cis-4-{[4-(dimethylamino)pyrimidin-2-yl]amino}cyclohexyl)-2-(5-methyl-2-phenyl-1,3-thiazol-4-yl)acetamide | 451 (M + H) | 2 |
| 852 | N-(cis-4-{[4-(dimethylamino)pyrimidin-2-yl]amino}cyclohexyl)-2-(6-methoxy-3-oxo-2,3-dihydro-1H-inden-1-yl)acetamide | 438 (M + H) | 3 |
| 853 | N-(cis-4-{[4-(dimethylamino)pyrimidin-2-yl]amino}cyclohexyl)-2-{1-[(4-methoxybenzyl)thio]cyclohexyl acetamide | 512 (M + H) | 3 |
| 854 | N-(cis-4-{[4-(dimethylamino)pyrimidin-2-yl]amino}cyclohexyl)-2-(7-methoxy-2-oxo-2H-chromen-4-yl)acetamide | 452 (M + H) | 3 |
| 855 | N-(cis-4-{[4-(dimethylamino)pyrimidin-2-yl]amino}cyclohexyl)-2-(1H-indol-3-yl)-4-oxo-4-phenylbutanamide | 511 (M + H) | 1 |
| 856 | 4-(4-bromophenyl)-N-(cis-4-{[4-(dimethylamino)pyrimidin-2-yl] amino}cyclohexyl)-2-(1H-indol-3-yl)-4-oxobutanamide | 589 (M + H) | 2 |
| 857 | N-(cis-4-{[4-(dimethylamino)pyrimidin-2-yl]amino}cyclohexyl)-3,5-dimethyl-2-[({[4(trifluoromethoxy)phenyl]amino}carbonyl)-amino]benzamide | 586 (M + H) | 3 |
| 858 | 3,5-dichloro-N-(cis-4-{[4-(dimethylamino)pyrimidin-2-yl]amino} cyclohexyl)-2-[(3-phenylprop-2-ynoyl)amino]benzamide | 551 (M + H) | 2 |
| 859 | 3-[2-(4-bromophenyl)-6,6-dimethyl-4-oxo-4,5,6,7-tetrahydro-1H-indol-1-yl]-N-(cis-4-{[4-(dimethylamino)pyrimidin-2-yl]amino} cyclohexyl)benzamide | 655 (M + H) | 3 |
| 860 | 4-(4-tert-butylphenyl)-N-(cis-4-{[4-(dimethylamino)pyrimidin-2-yl] amino}cyclohexyl)-2-(7-ethyl-1H-indol-3-yl)-4-oxobutanamide | 595 (M + H) | 3 |
| 861 | 4-(4-tert-butylphenyl)-N-(cis-4-{[4-(dimethylamino)pyrimidin-2-yl] amino}cyclohexyl)-2-(1-methyl-1H-indol-3-yl)-4-oxobutanamide | 581 (M + H) | 3 |
| 862 | N-(cis-4-{[4-(dimethylamino)pyrimidin-2-yl]amino}cyclohexyl)-2-(1-methyl-1H-indol-3-yl)-4-(4-methylphenyl)-4-oxobutanamide | 539 (M + H) | 1 |
| 863 | N-(cis-4-{[4-(dimethylamino)pyrimidin-2-yl]amino}cyclohexyl)-2-methyl-1-(3-morpholin-4-ylpropyl)-5-phenyl-1H-pyrrole-3-carboxamide | 546 (M + H) | 2 |
| 864 | N-(cis-4-{[4-(dimethylamino)pyrimidin-2-yl]amino}cyclohexyl)-4-(4-nitrophenyl)butanamide | 427 (M + H) | 2 |
| 865 | N-(cis-4-{[4-(dimethylamino)pyrimidin-2-yl]amino}cyclohexyl)-2-[(3-nitropyridin-2-yl)thio]acetamide | 432 (M + H) | - |
| 866 | (2E)-N-(cis-4-1[4-(dimethylamino)pyrimidin-2-yl]amino} cyclohexyl)-3-(2-nitrophenyl)acrylamide | 411 (M + H) | 3 |
| 867 | N-(cis-4-{[4-(dimethylamino)pyrimidin-2-yl]amino}cyclohexyl)-2-(3-phenoxyphenyl)acetamide | 446 (M + H) | 3 |
| 868 | N-(cis-4-1[4-(dimethylamino)pyrimidin-2-yl]amino}cyclohexyl)-2-(4-phenoxyphenyl)acetamide | 446 (M +H) | 3 |
| 869 | N-(cis-4-{[4-(dimethylamino)pyrimidin-2-yl]amino}cyclohexyl)-2-(2-phenyl-1H-indol-3-yl)acetamide | 469 (M + H) | 1 |
| 870 | N2-benzoyl-N5-(cis-4-{[4-(dimethylamino)pyrimidin-2-yl]amino)} cyclohexyl)-N 1,N 1-dipropylglutamamide | 552 (M + H) | 2 |
| 871 | N-(cis-4-{[4-(dimethylamino)pyrimidin-2-yl]amino}cyclohexyl)-3-phenoxybenzamide | 432 (M + H) | 2 |

(continued)

| Ex. No. | compound name | MS | class |
|---|---|---|---|
| 872 | N-(cis-4-{[4-(dimethylamino)pyrimidin-2-yl]amino}cyclohexyl)-2-(2-phenylethyl)benzamide | 444 (M + H) | 3 |
| 873 | 3-benzoyl-N-(cis-4-{[4-(dimethylamino)pyrimidin-2-yl]amino}cyclohexyl)benzamide | 444 (M + H) | 2 |
| 874 | N-(cis-4-{[4-(dimethylamino)pyrimidin-2-yl]amino}cyclohexyl)-2-(ethylthio)-2,2-diphenylacetamide | 490 (M + H) | 1 |
| 875 | 2-[(2-cyanoghenyl)thio]-N-(cis-4-{[4-(dimethylamino)pyrimidin-2-yl]amino}cyclohexyl)benzamide | 473 (M + H) | 3 |
| 876 | 2-[4-(benzyloxy)-3-methoxyphenyl]-N-(cis-4-{[4-(dimethylamino)pyrimidin-2-yl]amino}cyclohexyl)acetamide | 490 (M + H) | 3 |
| 877 | N-(cis-4-{[4-(dimethylamino)pyrimidin-2-yl]amino}cyclohexyl) N'-[(1R)-1-(1-naphthyl)ethyl)phthalamide | 537 (M +H) | 2 |
| 878 | (2S)-2-(3-benzoylphenyl)-N-(cis-4-{[4-cyclohexyl)propanamide (dimethylamino)pyrimidin-2-yl]amino cyclohexyl)propanamide | 472 (M + H) | 2 |
| 879 | N'-(cis-4-{[4-(dimethylamino)pyrimidin-2-yl]amino}cyclohexyl)-N, N-bis[(1S)-1-phenylethyl]phthalamide | 591 (M + H) | 1 |
| 880 | (2S)-N-(cis-4-{[4-(dimethylamino)pyrimidin-2-yl]amino}cyclohexyl)-2-(2-fluorobiphenyl-4-yl)propanamide | 462 (M + H) | 3 |
| 881 | 2-[(4-chlorobenzyl)thio]-4-(4-chlorophenyl)-N-(cis-4-{[4-(dimethylamino)pyrimidin-2-yl]amino}cyclohexyl)-4-oxobutanamide | 586 (M + H) | 3 |
| 882 | 2-[(4-chlorobenzyl)thio]-N-(cis-4-{[4-(dimethylamino)pyrimidin-2-yl]amino}cyclohexyl)-4-(4-methylphenyl)-4-oxobutanamide | 566 (M + H) | 3 |
| 883 | N-(cis-4-{[4-(dimethylamino)pyrimidin-2-yl]amino}cyclohexyl)-2-{(1E)-5-fluoro-2-methyl-1-[4-(methylsulfinyl)benzylidene]-1H-inden-3-yl}acetamide | 574 (M + H) | 2 |
| 884 | N-(cis-4-{[4-(dimethylamino)pyrimidin-2-yl]amino}cyclohexyl)-2-[4-(2-thienylcarbonyl)phenyl]propanamide | 478 (M + H) | 2 |
| 885 | N-(cis-4-{[4-(dimethylamino)pyrimidin-2-yl]amino}cyclohexyl)-4-oxo-4-(2-thienyl)butanamide | 402 (M + H) | 3 |
| 886 | N-(cis-4-{[4-(dimethylamino)pyrimidin-2-yl]amino}cyclohexyl)-4-(2-thienyl)butanamide | 388 (M + H) | 3 |
| 887 | 2-[5-(benzyloxy)-1H-indol-3-yl]-N-(cis-4-{[4-(dimethylamino)pyrimidin-2-yl]amino}cyclohexyl)acetamide | 499 (M + H) | 3 |
| 888 | N-(cis-4-{[4-(dimethylamino)pyrimidin-2-yl]amino}cyclohexyl)-2-(1-naphthoyl)benzamide | 494 (M + H) | 3 |
| 889 | 3-(benzyloxy)-N-(cis-4-{[4-(dimethylamino)pyrimidin-2-yl]amino}cyclohexyl)-4-methoxybenzamide | 476 (M + H) | 1 |
| 890 | N-(cis-4-{[4-(dimethylamino)pyrimidin-2-yl]amino}cyclohexyl)-2-methyl-1,5-diphenyl-1H-pyrrole-3-carboxamide | 495 (M + H) | 1 |
| 891 | 1-{2-[(2-chloro-6-fluorobenzyl)thio]ethyl}-N-(cis-4-{[4-(dimethylamino)pyrimidin-2-yl]amino}cyclohexyl)-2-methyl-5-phenyl-1H-pyrrole-3-carboxamide | 621 (M + H) | 1 |
| 892 | N-(cis-4-{[4-(dimethylamino)pyrimidin-2-yl]amino}cyclohexyl) anthracene-9-carboxamide | 440 (M + H) | 3 |

(continued)

| Ex. No. | compound name | MS | class |
|---|---|---|---|
| 893 | N-(cis-4-{[4-(dimethylamino)pyrimidin-2-yl]amino}cyclohexyl)-2-phenoxybenzamide | 432 (M + H) | 2 |
| 894 | N-(cis-4-{[4-(dimethylamino)pyrimidin-2-yl]amino}cyclohexyl) biphenyl-2-carboxamide | 416 (M + H) | 3 |
| 895 | N-(cis-4-{[4-(dimethylamino)pyrimidin-2-yl]amino)cyclohexyl)-3,3-diphenylpropanamide | 444 (M + H) | 3 |
| 896 | N-(cis-4-{[4-(dimethylamino)pyrimidin-2-yl]amino}cyclohexyl)-2-phenylquinoline-4-carboxamide | 467 (M + H) | 2 |
| 897 | N-(cis-4-{[4-(dimethylamino)pyrimidin-2-yl]amino}cyclohexyl) -N'-[(1S)-1-phenylethyl]phthalamide | 487 (M + H) | 3 |
| 898 | N-(cis-4-{[4-(dimethylamino)pyrimidin-2-yl]amino}cyclohexyl)-2-(4-methylbenzoyl)benzamide | 458 (M + H) | 3 |
| 899 | N-(cis-4-{[4-(dimethylamino)pyrimidin-2-yl]amino)cyclohexyl)-2-(phenoxymethyl)benzamide | 446 (M + H) | 3 |
| 900 | 2-[4-(4-chlorophenyl)-2-phenyl-1,3-thiazol-5-yl]-N-(cis-4-{ [4-(dimethylamino)pyrimidin-2-yl]amino}cyclohexyl)acetamide | 547 (M + H) | 1 |
| 901 | N-(cis-4-{[4-(dimethylamino)pyrimidin-2-yl]amino}cyclohexyl)-1-[(4-methylphenyl)sulfonyl]-1H-pyrrole-3-carboxamide | 483 (M + H) | 2 |
| 902 | N-(cis-4-{[4-(dimethylamino)pyrimidin-2-yl]amino}cyclohexyl)-5-(3-nitrophenyl)-2-furamide | 451 (M + H) | 2 |
| 903 | 3-chloro-N-(cis-4-{[4-(dimethylamino)pyrimidin-2-yl]amino} cyclohexyl)-4-(methylsulfonyl)thiophene-2-carboxamide | 458 (M + H) | 3 |
| 904 | 3-chloro-N-(cis-4-{[4-(dimethylamino)pyrimidin-2-yl]amino} cyclohexyl)-4-(isopropylsulfonyl)-5-(methylthio)thiophene-2-carboxamide | 532 (M + H) | 2 |
| 905 | N-(cis-4-{[4-(dimethylamino)pyrimidin-2-yl]amino}cyclohexyl)-3-iodo-4-(isopropylsulfonyl)-5-(methylthio)thiophene-2-carboxamide | 624 (M + H) | 2 |
| 906 | N-(cis-4-{[4-(dimethylamino)pyrimidin-2-yl]amino}cyclohexyl)-5-nitrothiophene-3-carboxamide | 391 (M + H) | 1 |
| 907 | N-(cis-4-{[4-(dimethylamino)pyrimidin-2-yl]amino}cyclohexyl)-1-methyl-4-nitro-1H-pyrrole-2-carboxamide | 388 (M + H) | 1 |
| 908 | N-(cis-4-{[4-(dimethylamino)pyrimidin-2-yl]amino}cyclohexyl)-1 -(phenylsulfonyl)-1 H-indole-3-carboxamide | 519 (M + H) | 3 |
| 909 | N-(cis-4-{[4-(dimethylamino)pyrimidin-2-yl]amino}cyclohexyl)-4-nitrobenzamide | 385 (M + H) | 2 |
| 910 | N-(cis-4-{[4-(dimethylamino)pyrimidin-2-yl]amino}cyclohexyl)-2-methoxy-4-nitrobenzamide | 415 (M + H) | 3 |
| 911 | N-(cis-4-{[4-(dimethylamino)pyrimidin-2-yl]amino}cyclohexyl)-3-fluoro-4-(trifluoromethyl)benzamide | 426 (M + H) | 3 |
| 912 | N-(cis-4-{[4-(dimethylamino)pyrimidin-2-yl]amino}cyclohexyl)-2-fluoro-4-nitrobenzamide | 403 (M + H) | 3 |
| 913 | N-(cis-4-{[4-(dimethylamino)pyrimidin-2-yl]amino}cyclohexyl)-3,5-dimethyl-4-nitrobenzamide | 413 (M + H) | 2 |

(continued)

| Ex. No. | compound name | MS | class |
|---|---|---|---|
| 914 | N-(cis-4-{[4-(dimethylamino)pyrimidin-2-yl]amino}cyclohexyl)-2-mesityl-2-oxoacetamide | 410 (M + H) | 2 |
| 915 | N-(cis-4-{[4-(dimethylamino)pyrimidin-2-yl]amino}cyclohexyl) quinoline-3-carboxamide | 391 (M + H) | 3 |
| 916 | N-(cis-4-{[4-(dimethylamino)pyrimidin-2-yl)amino}cyclohexyl)-2-methoxy-2-phenylacetamide | 384 (M + H) | 3 |
| 917 | N-(cis-4-{[4-(dimethylamino)pyrimidin-2-yl]amino}cyclohexyl)-1,2,3,4-tetrahydronaphthalene-2-carboxamide | 394 (M + H) | 3 |
| 918 | N-(cis-4-{[4-(dimethylamino)pyrimidin-2-yl]amino}cyclohexyl)-1,3-benzothiazole-6-carboxamide | 397 (M + H) | 3 |
| 919 | 5-chloro-N-(cis-4-{[4-(dimethylamino)pyrimidin-2-yl]amino} cyclohexyl)-2-hydroxybenzamide | 390 (M + H) | 3 |
| 920 | 2-chloro-N-(cis-4-{[4-(dimethylamino)pyrimidin-2-yl]amino} cyclohexyl)-5-(methylthio)benzamide | 420 (M + H) | 3 |
| 921 | N-(cis-4-{[4-(dimethylamino)pyrimidin-2-yl]amino}cyclohexyl)-7-methoxy-1-benzofuran-2-carboxamide | 410 (M+H) | 3 |
| 922 | 2-amino-N-(cis-4-{[4-(dimethylamino)pyrimidin-2-yl]amino} cyclohexyl)-3-methylbenzamide | 369 (M + H) | 3 |
| 923 | 2-(allylthio)-N-(cis-4-{[4-(dimethylamino)pyrimidin-2-yl]amino} cyclohexyl)nicotinamide | 413 (M + H) | 3 |
| 924 | 3,5-di-tert-butyl-N-(cis-4-{[4-(dimethylamino)pyrimidin-2-yl] amino}cyclohexyl)-4-hydroxybenzamide | 468 (M + H) | 1 |
| 925 | 5-bromo-N-[(cis-4-{[4-(dimethylamino)pyrimidin-2-yl]amino} cyclohexyl)methyl)thiophene-2-carboxamide | 438 (M + H) | 3 |
| 926 | N-[(cis-4-{[4-(dimethylamino)pyrimidin-2-yl]amino}cyclohexyl) methyl]-2-(2,3,6-trichlorophenyl)acetamide | 470 (M + H) | 1 |
| 927 | 2-(2-chloro-4-fluorophenyl)-N-[(cis-4-{[4-(dimethylamino)-pyrimidin-2-yl]amino}-cyclohexyl)methyl]acetamide | 420 (M + H) | 3 |
| 928 | 5-(4-chloro-2-nitrophenyl)-N-[(cis-4-{[4-(dimethylamino)-pyrimidin-2-yl]amino}-cyclohexyl)methyl]-2-furamide | 499 (M + H) | 3 |
| 929 | 5-chloro-N-[(cis-4-{[4-(dimethylamino)pyrimidin-2-yl]amino} cyclohexyl)methyl]thiophene-2-carboxamide | 394 (M + H) | 3 |
| 930 | N-[(cis-4-{[4-(dimethylamino)pyrimidin-2-yl]amino}cyclohexyl) methyl]-2,3-diphenylpropanamide | 458 (M + H) | 2 |
| 931 | N-[(cis-4-{[4-(dimethylamino)pyrimidin-2-yl]amino}cyclohexyl) methyl]-3-(2-hydroxyphenyl)propanamide | 398 (M + H) | 3 |
| 932 | N-[(cis-4-{[4-(dimethylamino)pyrimidin-2-yl]amino}cyclohexyl) methyl]-5-iodo-2-furamide | 470 (M + H) | 2 |
| 933 | N-[(cis-4-{[4-(dimethylamino)pyrimidin-2-yl]amino}cyclohexyl)-methyl]-2-(5-methoxy-2-methyl-1H-indol-3-yl)acetamide | 451 (M + H) | 3 |
| 934 | (2E)-N-[(cis-4-{[4-(dimethylamino)pyrimidin-2-yl]amino} cyclohexyl)methyl]-3-(3-nitrophenyl)acrylamide | 425 (M + H) | 1 |
| 935 | N-((cis-4-{[4-(dimethylamino)pyrimidin-2-yl]amino}cyclohexyl) methyl]-3-oxoindane-1-carboxamide | 408 (M + H) | 1 |

(continued)

| Ex. No. | compound name | MS | class |
|---|---|---|---|
| 936 | 2-benzyl-N-[(cis-4-{[4-(dimethylamino)pyrimidin-2-yl]amino} cyclohexyl)methyl]benzamide | 444 (M + H) | 2 |
| 937 | 2,2-bis(4-chlorophenyl)-N-[(cis-4-{[4-(dimethylamino)pyrimidin-2-yl]amino}cyclohexyl)methyl]acetamide | 512 ( + H) | 1 |
| 938 | N-[(cis-4-{[4-(dimethylamino)pyrimidin-2-yl]amino}cyclohexyl)-methyl]-5-(4-methyl-2-nitrophenyl)-2-furamide | 479 (M + H) | 3 |
| 939 | N-[(cis-4-{[4-(dimethylamino) pyrimidin-2-yl]amino}cyclohexyl) methyl]-5-nitrothiophene-2-carboxamide | 405 (M + H) | 3 |
| 940 | N-[(cis-4-{[4-(dimethylamino)pyrimidin-2-yl]amino}cyclohexyl) methyl]-3-methyl-4-nitrobenzamide | 413 (M + H) | 1 |
| 941 | N-[(cis-4-{[4-(dimethylamino)pyrimidin-2-yl]aminolcyclohexyl) methyl]-3-methoxy-4-nitrobenzamide | 429 (M + H) | 1 |
| 942 | 1-benzyl-N-[(cis-4-{[4-(dimethylamino)pyrimidin-2-yl]amino} cyclohexyl)methyl]-1H-indole-3-carboxamide | 483 (M + H) | 3 |
| 943 | 2-cyclohex-1-en-1-yl-N-[(cis-4-{[4-(dimethylamino)pyrimidin-2-yl] amino}cyclohexyl)methyl]acetamide | 372 (M + H) | 3 |
| 944 | N-[(cis-4-{[4-(dimethylamino)pyrimidin-2-yl]amino}cyclohexyl) methyl]-4-(4-ethoxyphenyl)-2-(3-methyl-5-oxo-1-phenyl-4,5-dihydro-1H-pyrazol-4-yl)-4-oxobutanamide | 626 (M + H) | 3 |
| 945 | N-[(cis-4-{[4-(dimethylamino)pyrimidin-2-yl]amino}-cyclohexyl) methyl]-2-[2-(trifluoromethoxy)phenyl]-acetamide | 452 (M + H) | 1 |
| 946 | 4-(benzyloxy)-N-[(cis-4-{[4-(dimethylamino)pyrimidin-2-yl]amino} cyclohexyl)methyl]-3,5-dimethylbenzamide | 488 (M + H) | 3 |
| 947 | N-[(cis-4-{[4-(dimethylamino)pyrimidin-2-yl]amino}cyclohexyl) methyl]-9H-xanthene-9-carboxamide | 458 (M + H) | 1 |
| 948 | 2-(1-benzothien-3-yl)-N-[(cis-4-{[4-(dimethylamino)pyrimidin-2-yl] amino}cyclohexyl)methyl]acetamide | 424 (M + H) | 1 |
| 949 | 5-bromo-N-(cis-4-([4-(dimethylamino)-5,6,7,8-tetrahydro-quinazolin-2-yl]amino}cyclohexyl)-thiophene-2-carboxamide | 478 (M + H) | 2 |
| 950 | N-(cis-4-{[4-(dimethylamino)-5,6,7,8-tetrahydroquinazolin-2-yl] amino}cyclohexyl)-2-(2,3,6-trichlorophenyl)acetamide | 510 (M + H) | 2 |
| 951 | 2-(2-chloro-4-fluorophenyl)-N-(cis-4-{[4-(dimethylamino)-5,6,7,8-tetrahydroquinazolin-2-yl]amino}cyclohexyl)acetamide | 460 (M + H) | 1 |
| 952 | 5-(4-chloro-2-nitrophenyl)-N-(cis-4-{[4-(dimethylamino)-5,6,7,8-tetrahydroquinazolin-2-yl]amino}cyclohexyl)-2-furamide | 539 (M + H) | 1 |
| 953 | 5-chloro-N-(cis-4-{[4-(dimethylamino)-5,6,7,8-tetrahydroquinazolin-2-yl]amino}cyclohexyl)-thiophene-2-carboxamide | 434 (M + H) | 1 |
| 954 | N-(cis-4-{[4-(dimethylamino)-5,6,7,8-tetrahydroquinazolin-2-yl] amino}cyclohexyl)-2,3-diphenylpropanamide | 498 (M + H) | 2 |
| 955 | N-(cis-4-{[4-(dimethylanimo)-5,6,7,8-tetrahydroquinazolin-2-yl] amino}cyclohexyl)-3-(2-hydroxyphenyl)propanamide | 438 (M + H) | 2 |
| 956 | N-(cis-4-{[4-(dimethylamino)-5,6,7,8-tetrahydroquinazolin-2- yl] amino}cyclohexyl)-5-iodo-2-furamide | 510 (M + H) | 1 |

(continued)

| Ex. No. | compound name | MS | class |
|---|---|---|---|
| 957 | N-(cis-4-{[4-(dimethylamino)-5,6,7,8-tetrahydroquinazolin-2-yl] amino}cyclohexyl)-2-(2-iodophenyl)acetamide | 534 (M + H) | 2 |
| 958 | N-(cis-4-{[4-(dimethylamino)-5,6,7,8-tetrahydroquinazolin-2-yl] amino}cyclohexyl)-2-(5-methoxy-2-methyl-1H-indol-3-yl) acetamide | 491 (M + H) | 2 |
| 959 | (2E)-N-(cis-4-[4-(dimethylamino)-5,6,7,8-tetrahydroquinazolin-2-yl]amino]cyclohexyl)-3-(3-nitrophenyl)acrylamide | 465 (M + H) | 3 |
| 960 | N-(cis-4-([4-{(dimethylamino)-5,6,7,8-tetrahydroquinazolin-2-yl] amino}cyclohexyl)-3-oxoindane-1-carboxamide | 448 (M + H) | 2 |
| 961 | 2-benzyl-N-(cis-4-{[4-(dimethylamino)-5,6,7,8-tetrahydroquinazolin-2-yl]amino}cyclohexyl)benzamide | 484 (M+H) | 2 |
| 962 | 2,2-bis(4-chlorophenyl)-N-(cis-4-{[4-(dimethylamino)-5,6,7,8-tetrahydroquinazolin-2-yl]amino}cyclohexyl)acetamide | 552 (M+H) | 1 |
| 963 | N-(cis-4-{[4-(dimethylamino)-5,6,7,8-tetrahydroquinazolin-2-yl] amino}cydohexyl)-5-(4-methyl-2-nitrophenyl)-2-furamide | 519 (M + H) | 2 |
| 964 | N-(cis-4-{[4-(dimethylamino)-5,6,7,8-tetrahydroquinazolin-2-yl] amino}cyclohexyl)-5-nitrothiophene-2-carboxamide | 445 (M + H) | 1 |
| 965 | N-(cis-4-{[4-(dimethylamino)-5,6,7,8-tetrahydroquinazolin-2-yl] amino}cyclohexyl)-3-methyl-4-nitrobenzamide | 453 (M + H) | 1 |
| 966 | N-(cis-4-{[4-(dimethylamino)-5,6,7,8-tetrahydroquinazolin-2-yl] amino}cyclohexyl)-3-methoxy-4-nitrobenzamide | 469 (M + H) | 1 |
| 967 | 1-benzyl-N-(cis-4-{[4-(dimethylamino)-5,6,7,8-tetrahydro-quinazolin-2-yl]amino}cyclohexyl)-1H-indole-3-carboxamide | 523 (M + H) | 3 |
| 968 | 3-acetyl-N-(cis-4-{[4-(dimethylamino)-5,6,7,8-tetrahydroquinazolin-2-yl]amino}cyclohexyl)benzamide | 436 (M+H) | 1 |
| 969 | (2R)-2-benzoyl-N-(cis-4-{[4-(dimethylamino)-5,6,7,8-tetrahydroquinazolin-2-yl]amino}cyclohexyl)-cyclohexanecarboxamide | 504 (M + H) | 3 |
| 970 | 5-bromo-N-(cis-4-{[4-(dimethylamino)-5,6,7,8-tetrahydroquinazolin-2-yl]amino}cyclohexyl)-2-furamide | 462 (M + H) | 1 |
| 971 | 3-cyclohexyl-N-(cis-4-{[4-(dimethylamino)-5,6,7,8-tetrahydroquinazolin-2-yl]amino}cyclohexyl)propanamide | 428 (M + H) | 3 |
| 972 | N-(cis-4-{[4-(dimethylamino)-5,6,7,8-tetrahydroquinazolin-2-yl] amino}cyclohexyl)-2-[(4-methylpyrimidin-2-yl)thio]acetamide | 456 (M + H) | 2 |
| 973 | 5-(4-chlorophenyl)-N-(cis-4-{[4-(dimethylamino)-5,6,7,8-tetrahydroquinazolin-2-yl]amino}cyclohexyl)-2-furamide | 494 (M + H) | 1 |
| 974 | 3-(3,4-dichlorophenyl)-N-(cis-4-{[4-(dimethylamino)-5,6,7,8-tetrahydroquinazolin-2-yl]amino}cyclohexyl)propanamide | 490 (M + H) | 3 |
| 975 | 2-(3,4-dichlorophenyl)-N-(cis-4-{[4-(dimethylamino)-5,6,7,8-tetrahydroquinazolin-2-yl]amino}cyclohexyl)acetamide | 476 (M + H) | 1 |
| 976 | N-(cis-4-{[4-(dimethylamino)-5,6,7,8-tetrahydroquinazolin-2-yl] amino}cyclohexyl)-2-(4-hydroxy-3,5-dimethoxyphenyl)acetamide | 484 (M + H) | 1 |
| 977 | 4,5-dibromo-N-(cis-4-{[4-(dimethylamino)-5,6,7,8-tetrahydroquinazolin-2-yl]amino}cyclohexyl)thiophene-2-carboxamide | 556 (M + H) | 2 |

(continued)

| Ex. No. | compound name | MS | class |
|---|---|---|---|
| 978 | 2-(3,5-dimethoxyphenyl)-N-(cis-4-{[4-(dimethylamino)-5,6,7,8-tetrahydroquinazolin-2-yl]amino}cyclohexyl)acetamide | 468 (M + H) | 3 |
| 979 | 2-(3,5-di-tert-butyl-4-hydroxyphenyl)-N-(cis-{[4-(dimethylamino)-5,6,7,8-tetrahydroquinazolin-2-yl]amino}-cyclohexyl)acetamide | 536 (M + H) | 3 |
| 980 | N2,N6-dibenzoyl-N-(cis-4-{[4-(dimethylamino)-5,6,7,8-tetrahydroquinazolin-2-yl]amino}cyclohexyl)lysinamide | 626 (M + H) | 2 |
| 981 | 3-(dimethylamino)-N-(cis-4-{[4-(dimethylamino)-5,6,7,8-tetrahydroquinazolin-2-yl]amino}cyclohexyl)benzamide | 437 (M + H) | 2 |
| 982 | 4,5-dibromo-N-(cis-4-{[4-(dimethylamino)-5,6,7,8-tetrahydroquinazolin-2-yl]amino}cyclohexyl)-2-furamide | 540 (M + H) | 1 |
| 983 | N-(cis-4-{[4-(dimethylamino)-5,6,7,8-tetrahydroquinazolin-2-yl]amino}cyclohexyl)-4-(4-fluorophenyl)-4-oxobutanamide | 468 (M + H) | 2 |
| 984 | N-(cis-4-{[4-(dimethylamino)-5,6,7,8-tetrahydroquinazolin-2-yl]amino}cyclohexyl)-2-(2-fluorobiphenyl-4-yl)propanamide | 516 (M + H) | 2 |
| 985 | N-(cis-4-{[4-(dimethylamino)-5,6,7,8-tetrahydroquinazolin-2-yl]amino}cyclohexyl)-2-[4-(1-oxo-1,3-dihydro-2H-isoindol-2-yl)phenyl]propanamide | 553 (M + H) | 2 |
| 986 | N-(cis-4-{[4-(dimethylamino)-5,6,7,8-tetrahydroquinazolin-2-yl]amino}cyclohexyl)-2-(1H-indol-3-yl)acetamide | 447 (M + H) | 1 |
| 987 | N-(cis-4-{[4-(dimethylamino)-5,6,7,8-tetrahydroquinazolin-2-yl]amino}cyclohexyl)-2-(5-methyl-2-phenyl-1,3-thiazol-4-yl)acetamide | 505 (M + H) | 3 |
| 988 | N-(cis-4-{[4-(dimethylamino)-5,6,7,8-tetrahydroquinazolin-2-yl]amino}cyclohexyl)-2-(6-methoxy-3-oxo-2,3-dihydro-1H-inden-1-yl)acetamide | 492 (M + H) | 3 |
| 989 | N-(cis-4-{[4-(dimethylamino)-5,6,7,8-tetrahydroquinazolin-2-yl]amino}cyclohexyl)-2-{1-[(4-methoxybenzyl)thio]-cyclohexyl}acetamide | 566 (M + H) | 3 |
| 990 | N-(cis-4-{[4-(dimethylamino)-5,6,7,8-tetrahydroquinazolin-2-yl]amino}cyclohexyl)-2-(7-methoxy-2-oxo-2H-chromen-4-yl)acetamide | 506 (M + H) | 1 |
| 991 | N-(cis-4-{[4-(dimethylamino)-5,6,7,8-tetrahydroquinazolin-2-yl]amino}cyclohexyl)-2-(1H-indol-3-yl)-4-oxo-4-phenylbutanamide | 565 (M + H) | 2 |
| 992 | 4-(4-bromophenyl)-N-(cis-4-{[4-(dimethylamino)-5,6,7,8-tetrahydroquinazolin-2-yl]amino}cyclohexyl)-2-(1H-indol-3-yl)-4-oxobutanamide | 643 (M + H) | 3 |
| 993 | N-(cis-4-{[4-(dimethylamino)-5,6,7,8-tetrahydroquinazolin-2-yl]amino}cyclohexyl)-3,5-dimethyl-2-[({[4-(trifluoromethoxy)phenyl]amino}carbonyl)amino]benzamide | 640 (M + H) | 1 |
| 994 | 3,5-dichloro-N-(cis-4-{[4-(dimethylamino)-5,6,7,8-tetrahydroquinazolin-2-yl]amino}cyclohexyl)-2-[(3-phenylprop-2-ynoyl)amino]benzamide | 605 (M + H) | 1 |
| 995 | 3-[2-(4-bromophenyl)-6,6-dimethyl-4-oxo-4,5,6,7-tetrahydro-1H-indol-1-yl]-N-(cis-4-{[4-(dimethylamino)-5,6,7,8-tetrahydroquinazolin-2-yl]amino}cyclohexyl)benzamide | 709 (M + H) | 3 |

(continued)

| Ex. No. | compound name | MS | class |
|---|---|---|---|
| 996 | 4-(4-tert-butylphenyl)-N-(cis-4-{[4-(dimethylamino)-5,6,7,8-tetrahydroquinazolin-2-yl]amino}cyclohexyl)-2-(7-ethyl-1H-indol-3-yl)-4-oxobutanamide | 649 (M + H) | 1 |
| 997 | 4-(4-tert-butylphenyl)-N-(cis-4-{[4-(dimethylamino)-5,6,7,8-tetrahydroquinazolin-2-yl]amino}cyclohexyl)-2-(1-methyl-1H-indol-3-yl)-4-oxobutanamide | 635 (M + H) | 3 |
| 998 | N-(cis-4-{[4-(dimethylamino)-5,6,7,8-tetrahydroquinazolin-2-yl]amino}cyclohexyl)-2-(1-methyl-1H-indol-3-yl)-4-(4-methylphenyl)-4-oxobutanamide | 593 (M + H) | 2 |
| 999 | N-(2,4-dichlorophenyl)-2-{2-[(cis-4-{[4-(dimethylamino)-5,6,7,8-tetrahydroquinazolin-2-yl]amino}cyclohexyl)amino]-2-oxoethyl}benzamide | 595 (M + H) | 3 |
| 1000 | N-(cis-4-{[4-(dimethylamino)-5,6,7,8-tetrahydroquinazolin-2-yl]amino}cyclohexyl)-2-methyl-1-(3-morpholin-4-ylpropyl)-5-phenyl-1H-pyrrole-3-carboxamide | 600 (M + H) | 1 |
| 1001 | N-(cis-4-{[4-(dimethylamino)-5,6,7,8-tetrahydroquinazolin-2-yl]amino}cyclohexyl)-4-(4-nitrophenyl)butanamide | 481 (M + H) | 1 |
| 1002 | (2E) N (cis-4-{[4-(dimethylamino)-5,6,7,8-tetrahydroquinazolin-2-yl]amino}cyclohexyl)-3-(2-nitrophenyl)acrylamide | 465 (M + H) | 3 |
| 1003 | N-(cis-4-{[4-(dimethylamino)-5,6,7,8-tetrahydroquinazolin-2-yl]amino}cyclohexyl)-2-(3-phenoxyphenyl)acetamide | 500 (M + H) | 2 |
| 1004 | N-(cis-4-{[4-(dimethylamino)-5,6,7,8-tetrahydroquinazolin-2-yl]amino}cyclohexyl)-2-(4-phenoxyphenyl)acetamide | 500 (M + H) | 2 |
| 1005 | N-(cis-4-{[4-(dimethylamino)-5,6,7,8-tetrahydroquinazolin-2-yl]amino}cyclohexyl)-2-(2-phenyl-1H-indol-3-yl)acetamide | 523 (M + H) | 1 |
| 1006 | N2-benzoyl-N5-(cis-4-{[4-(dimethylamino)-5,6,7,8-tetrahydroquinazolin-2-yl]amino}cyclohexyl)-N1,N1-dipropylglutamamide | 606 (M + H) | 1 |
| 1007 | N-(cis-4-{[4-(dimethylamino)-5,6,7,8-tetrahydroquinazolin-2-yl]amino}cyclohexyl)-3-phenoxybenzamide | 486 (M + H) | 1 |
| 1008 | N-(cis-4-{[4-(dimethylamino)-5,6,7,8-tetrahydroquinazolin-2-yl]amino}cyclohexyl)-2-(2-phenylethyl)benzamide | 498 (M+H) | 3 |
| 1009 | 3-benzoyl-N-(cis-4-{[4-(dimethylamino)-5,6,7,8-tetrahydroquinazolin-2-yl]amino}cyclohexyl)benzamide | 498 (M + H) | 3 |
| 1010 | N-(cis-4-[4-(dimethylamino)-5,6,7,8-tetrahydroquinazolin-2-yl]amino}cyclohexyl)-2-(ethylthio)-2,2-diphenylacetamide | 544 (M + H) | 2 |
| 1011 | 2-[(2-cyanophenyl)thio]-N-(cis-4-[4-(dimethylamino)-5,6,7,8-tetrahydroquinazolin-2-yl]amino}cyclohexyl)benzamide | 527 (M + H) | 3 |
| 1012 | 2-[4-(benzyloxy)-3-methoxyphenyl]-N-(cis-4-{[4-(dimethylamino)-5,6,7,8-tetrahydroquinazolin-2-yl]amino}cyclohexyl)acetamide | 544 (M + H) | 3 |
| 1013 | N-(cis-4-{[4-(dimethylamino)-5,6,7,8-tetrahydroquinazolin-2-yl]aminol}cyclohexyl)-N'-[(1R)-1-(1-naphthyl)ethyl]phthalamide | 591 (M + H) | 3 |
| 1014 | (2S)-2-(3-benzoylphenyl)-N-(cis-4-{[4-(dimethylamino)-5,6,7,8-tetrahydroquinazolin-2-yl]amino}cyclohexyl)propanamide | 526 (M + H) | 3 |

(continued)

| Ex. No. | compound name | MS | class |
|---|---|---|---|
| 1015 | N'-(cis-4-{[4-(dimethylamino)-5,6,7,8-tetrahydroquinazolin-2-yl] amino}cyclohexyl)-N,N-bis[(1S)-1-phenylethyl]phthalamide | 645 (M + H) | 1 |
| 1016 | (2S)-N-(cis-4-{[4-(dimethylamino)-5,6,7,8-tetrahydroquinazolin-2-yl]amino}cyclohexyl)-2-(2-fluorobiphenyl-4-yl)propanamide | 516 (M + H) | 2 |
| 1017 | 2-[(4-chlorobenzyl)thio]-4-(4-chlorophenyl)-N-(cis-4-{ [4-(dimethylamino)-5,6,7,8-tetrahydroquinazolin-2-yl]amino} cyclohexyl)-4-oxobutanamide | 640 (M + H) | 3 |
| 1018 | 2-[(4-chlorobenzyl)thio]-N-(cis-4-{[4-(dimethylamino )-5,6, 7 , 8-tetrahydroquinazolin-2-yl]amino}cyclohexyl)-4-(4-methylphenyl)-4-oxobutanamide | 620 (M + H) | 2 |
| 1019 | N-(cis-4-{[4-(dimethylamino)-5,6,7,8-tetrahydroquinazolin-2-yl] amino}cyclohexyl)-2-{(1E)-5-fluoro-2-methyl-1-[4-(methylsulfinyl) benzylidene]-1H-inden-3-yl }acetamide | 628 (M + H) | 1 |
| 1020 | N-(cis-4-{[4-(dimethylamino)-5,6,7,8-tetrahydroquinazolin-2-yl] amino}cyclohexyl)-2-[4-(2-thienylcarbonyl)phenyl]propanamide | 532 (M + H) | 2 |
| 1021 | N-(cis-4-{[4-(dimethylamino)-5,6,7,8-tetrahydroquinazolin-2-yl] amino}cyclohexyl)-4-oxo-4-(2-thienyl)butanamide | 456 (M + H) | 3 |
| 1022 | N-(cis-4-{[4-(dimethylamino)-5,6,7,8-tetrahydroquinazolin-2-yl] amino}cyclohexyl)-4-(2-thienyl)butanamide | 442 (M + H) | 3 |
| 1023 | N-(cis-4-{[4-(dimethylamino)-5,6,7,8-tetrahydroquinazolin-2-yl] amino}cyclohexyl)-2-(2,4,6-trichlorophenoxy)acetamide | 526 (M + H) | 3 |
| 1024 | 2 [5-(benzyloxy)-1H-indol-3-yl]-N-(cis-4-{[4-(dimethylamino)-5,6,7,8-tetrahydroquinazolin-2-yl]amino}cyclohexyl)acetamide | 553 (M + H) | 3 |
| 1025 | N-(cis-4-{[4-(dimethylamino)-5,6,7,8-tetrahydroquinazolin-2-yl] amino}cyclohexyl)-2-(1 -naphthoyl)benzamide | 548 (M + H) | 3 |
| 1026 | 3-(benzyloxy)-N-(cis-4-{[4-{dimethylamino)-5,6,7,8-tetrahydroquinazolin-2-yl]amino}cyclohexyl)-4-methoxybenzamide | 530 (M + H) | 1 |
| 1027 | N-(cis-4-{[4-(dimethylamino)-5,6,7,8-tetrahydroquinazolin-2-yl] amino}cyclohexyl)-2-methyl-1,5-diphenyl-1H-pyrrole-3-carboxamide | 549 (M + H) | 2 |
| 1028 | 1-{2-[(2-chloro-6-fluorobenzyl)thio]ethyl}-N-(cis-4-{[4-(dimethy-lamino)-5,6,7,8-tetrahydroquinazolin-2-yl]amino}-cyclohexyl)-2-methyl-5-phenyl-1H-pyrrole-3-carboxamide | 675 (M + H) | 2 |
| 1029 | N-(cis-4-{[4-(dimethylamino)-5,6,7,8-tetrahydroquinazolin-2-yl] amino}cyclohexyl)anthracene-9-carboxamide | 494 (M + H) | 3 |
| 1030 | N-(cis-4-{[4-(dimethylamino)-5,6,7,8-tetrahydroquinazolin-2-yl] amino}cyclohexyl)-2-phenoxybenzamide | 486 (M + H) | 1 |
| 1031 | N-(cis-4-{[4-(dimethylamino)-5,6,7,8-tetrahydroquinazolin-2-yl] amino}cyclohexyl)biphenyl-2-carboxamide | 470 (M + H) | 3 |
| 1032 | N-(cis-4-{[4(dimethylamino)-5,6,7,8-tetrahydroquinazolin-2-yl] amino}cyclohexyl)-3,3-diphenylpropanamide | 498 (M + H) | 3 |
| 1033 | N-(cis-4-[4-(dimethylamino)-5,6,7,8-tetrahydroquinazolin-2-yl] amino}cyclohexyl)-2-phenylquinoline-4-carboxamide | 521 (M + H) | 2 |

(continued)

| Ex. No. | compound name | MS | class |
|---|---|---|---|
| 1034 | N-(cis-4-{[4-(dimethylamino)-5,6,7,8-tetrahydroquinazolin-2-yl] amino}cyclohexyl)-N'-[(1S)-1-phenylethyl]phthalamide | 541 (M + H) | 3 |
| 1035 | N-(cis-4-[4-(dimethylamino)-5,6,7,8-tetrahydroquinazolin-2-yl] amino}cyclohexyl)-2-(4-methylbenzoyl)benzamide | 512 (M + H) | 3 |
| 1036 | N-(cis-4-{[4-(dimethylamino)-5,6,7,8-tetrahydroquinazolin-2-yl] amino}cyclohexyl)-2-(phenoxymethyl)benzamide | 500 (M + H) | 3 |
| 1037 | 2-[4-(4-chlorophenyl)-2-phenyl-1,3-thiazol-5-yl]-N-(cis-4-{ [4-(dimethylamino)-5,6,7,8-tetrahydroquinazolin-2-yl]amino} cyclohexyl)acetamide | 601 (M + H) | 3 |
| 1038 | N-(cis-4-{[4-(dimethylamino)-5,6,7,8-tetrahydroquinazolin-2-yl] amino}cyclohexyl)-1-[(4-methylphenyl)sulfonyl]-1H-pyrrole-3-carboxamide | 537 (M + H) | 3 |
| 1039 | N-(cis-4-{[4-(dimethylamino)-5,6,7,8-tetrahydroquinazolin-2-yl] amino}cyclohexyl)-5-(3-nitrophenyl)-2-furamide | 505 (M + H) | 2 |
| 1040 | 3-chloro-N-(cis-4-{[4-(dimethylamino)-5,6,7,8-tetrahydro-quinazolin-2-yl]amino}cyclohexyl)-4-(methylsulfonyl)thiophene-2-carboxamide | 512 (M + H) | 3 |
| 1041 | 3-chloro-N-(cis-4-{[4-(dimethylamino)-5,6,7,8-tetrahydroquinazolin-2-yl]amino}cyclohexyl)-4-(isopropylsulfonyl)-5-(methylthio)thiophene-2-carboxamide | 586 (M + H) | 3 |
| 1042 | N-(cis-4-{[4-(dimethylamino)-5,6,7,8-tetrahydroquinazolin-2-yl] amino}cyclohexyl)-3-iodo-4-(isopropylsulfonyl)-5-(methylthio) thiophene-2-carboxamide | 678 (M + H) | 3 |
| 1043 | N-(cis-4-{[4-(dimethylamino)-5,6,7,8-tetrahydroquinazolin-2-yl] amino}cyclohexyl)-5-nitrothiophene-3-carboxamide | 445 (M + H) | 1 |
| 1044 | N-(cis-4-{[4-(dimethylamino)-5,6,7,8-tetrahydroquinazolin-2-yl] amino}cyclohexyl)-1-methyl-4-nitro-1H-pyrrole-2-carboxamide | 442 (M + H) | 1 |
| 1045 | N-(cis-4-{[4-(dimethylamino)-5,6,7,8-tetrahydroquinazolin-2-yl] amino}cyclohexyl)-1-(phenylsulfonyl)-1H-indole-3-carboxamide | 573 (M + H) | 3 |
| 1046 | N-(cis-4-{[4-(dimethylamino)-5,6,7,8-tetrahydroquinazolin-2-yl] amino}cyclohexyl)-4-nitrobenzamide | 439 (M + H) | 3 |
| 1047 | N-(cis-4-{[4-(dimethylamino)-5,6,7,8-tetrahydroquinazolin-2-yl] amino}cyclohexyl)-2-methoxy-4-nitrobenzamide | 469 (M + H) | 2 |
| 1048 | N-(cis-4-{[4-(dimethylamino)-5,6,7,8-tetrahydroquinazolin-2-yl] amino}cyclohexyl)-3-fluoro-4-(trifluoromethyl)benzamide | 480 (M + H) | 3 |
| 1049 | N-(cis-4-{[4-(dimethylamino)-5,6,7,8-tetrahydroquinazolin-2-yl] amino}cyclohexyl)-2-fluoro-4-nitrobenzamide | 457 (M + H) | 3 |
| 1050 | N-(cis-4-{[4-(dimethylamino)-5,6,7,8-tetrahydroquinazolin-2-yl] amino}cyclohexyl)-3,5-dimethyl-4-nitrobenzamide | 467 (M + H) | 3 |
| 1051 | N-(cis-4-{[4-(dimethylamino)-5,6,7,8-tetrahydroquinazolin-2-yl] amino}cyclohexyl)-2-mesityl-2-oxoacetamide | 464 (M + H) | 3 |
| 1052 | N-(cis-4-{[4-(dimethylamino)-5,6,7,8-tetrahydroquinazolin-2-yl] amino}cyclohexyl)-2-methoxy-2-phenylacetamide | 438 (M + H) | 2 |
| 1053 | N-(cis-4-{[4-(dimethylamino)-5,6,7,8-tetrahydroquinazolin-2-yl] amino}cyclohexyl)-1,2,3,4-tetrahydronaphthalene-2-carboxamide | 448 (M + H) | 3 |

(continued)

| Ex. No. | compound name | MS | class |
|---|---|---|---|
| 1054 | N-(cis-4-{[4-(dimethylamino)-5,6,7,8-tetrahydroquinazolin-2-yl]amino}cyclohexyl)-1,3-benzothiazole-6-carboxamide | 451 (M + H) | 3 |
| 1055 | 5-chloro-N-(cis-4-{[4-(dimethylamino)-5,6,7,8-tetrahydro-quinazolin-2-yl]amino}cyclohexyl)-2-hydroxybenzamide | 444 (M + H) | 1 |
| 1056 | 2-chloro-N-(cis-4-{[4-(dimethylamino)-5,6,7,8-tetrahydro-quinazolin-2-yl]amino}cyclohexyl)-5-(methylthio)-benzamide | 474 (M + H) | 3 |
| 1057 | N-(cis-4-{[4-(dimethylamino)-5,6,7,8-tetrahydroquinazolin-2-yl]amino}cyclohexyl)-7-methoxy-1-benzofuran-2-carboxamide | 464 (M + H) | 3 |
| 1058 | 2-amino-N-(cis-4-{[4-(dimethylamino)-5,6,7,8-tetrahydroquinazolin-2-yl]amino}cyclohexyl)-3-methylbenzamide | 423 (M + H) | 3 |
| 1059 | 2-(allylthio)-N-(cis-4-{[4-(dimethylamino)-5,6,7,8-tetrahydroquinazolin-2-yl]amino}cyclohexyl)nicotinamide | 467 (M + H) | 3 |
| 1060 | 3,5-di-tert-butyl-N-(cis-4-{[4-(dimethylamino)-5,6,7,8-tetrahydroquinazolin-2-yl]amino}cyclohexyl)-4-hydroxybenzamide | 522 (M + H) | 3 |
| 1061 | 5-bromo-N-[(cis-4-{[4-(dimethylamino)-5,6,7,8-tetrahydroquinazolin-2-yl]amino}cyclohexyl)methyl]thiophene-2-carboxamide | 492 (M + H) | 3 |
| 1062 | N-[(cis-4-{[4-(dimethylamino)-5,6,7,8-tetrahydroquinazolin-2-yl]amino}cyclohexyl)methyl]-2-(2,3,6-trichlorophenyl)acetamide | 524 (M + H) | 2 |
| 1063 | 2-(2-chloro-4-fluorophenyl)-N-[(cis-4-{[4-(dimethylamino)-5,6,7,8-tetrahydroquinazolin-2-yl]amino}cyclohexyl)methyl]-acetamide | 474 (M + H) | 3 |
| 1064 | 5-(4-chloro-2-nitrophenyl)-N-[(cis-4-{[4-(dimethylamino)-5,6,7,8-tetrahydroquinazolin-2-yl]amino}cyclohexyl)methyl]-2-furamide | 553 (M + H) | 3 |
| 1065 | 5-chloro-N-[(cis-4-{[4-(dimethylamino)-5,6,7,8-tetrahydro-quinazolin-2-yl]amino}cyclohexyl)methyl]thiophene-2-carboxamide | 448 (M + H) | 3 |
| 1066 | N-[(cis-4-{[4-(dimethylamino)-5,6,7,8-tetrahydroquinazolin-2-yl]amino}cyclohexyl)methyl]-2,3-diphenylpropanamide | 512 (M + H) | 3 |
| 1067 | N-[(cis-4-{[4-(dimethylamino)-5,6,7,8-tetrahydroquinazolin-2-yl]amino}cyclohexyl)methyl]-3-(2-hydroxyphenyl)propanamide | 452 (M + H) | 3 |
| 1068 | N-[(cis-4-{[4-(dimethylamino)-5,6,7,8-tetrahydroquinazolin-2-yl]amino}cyclohexyl)methyl]-5-iodo-2-furamide | 524 (M + H) | 3 |
| 1069 | N-[(cis-4-{[4-(dimethylamino)-5,6,7,8-tetrahydroquinazolin-2-yl]amino}cyclohexyl)methyl]-2-(2-iodophenyl)acetamide | 548 (M + H) | 3 |
| 1070 | (2E)-N-[(cis-4-{[4-(dimethylamino)-5,6,7,8-tetrahydroquinazolin-2-yl]amino}cyclohexyl)methyl]-3-(3-nitrophenyl)acrylamide | 479 (M + H) | 2 |
| 1071 | N-[(cis-4-{[4-(dimethylamino)-5,6,7,8-tetrahydroquinazolin-2-yl]amino}cyclohexyl)methyl]-3-oxoindane-1-carboxamide | 462 (M + H) | 3 |
| 1072 | 2-benzyl-N-[(cis-4-{[4-(dimethylamino)-5,6,7,8-tetrahydroquinazolin-2-yl]amino}cyclohexyl)methyl]benzamide | 498 (M + H) | 3 |

(continued)

| Ex. No. | compound name | MS | class |
|---|---|---|---|
| 1073 | 2,2-bis(4-chlorophenyl)-N-[(cis-4-{[4-(dimethylamino)-5,6,7,8-tetrahydroquinazolin-2-yl]amino}cyclohexyl)methyl] acetamide | 566 (M + H) | 3 |
| 1074 | N-[(cis-4-{[4-(dimethylamino)-5,6,7,8-tetrahydroquinazolin-2-yl] amino}cyclohexyl)methyl]-5-(4-methyl-2-nitrophenyl)-2-furamide | 533 (M + H) | 3 |
| 1075 | N-[(cis-4-{[4-(dimethylamino)-5,6,7,8-tetrahydroquinazolin-2-yl] amino}cyclohexyl)methyl]-5-nitrothiophene-2-carboxamide | 459 (M + H) | 3 |
| 1076 | N-[(cis-4-{[4-(dimethylamino)-5,6,7,8-tetrahydroquinazolin-2-yl] amino}cyclohexyl)methyl]-3-methyl-4-nitrobenzamide | 467 (M + H) | 3 |
| 1077 | N-[(cis-4-{[4-(dimethylamino)-5,6,7,8-tetrahydroquinazolin-2-yl] amino}cyclohexyl)methyl]-3-methoxy-4-nitrobenzamide | 483 (M + H) | 3 |
| 1078 | 1-benzyl-N-[(cis-4-{[4-(dimethylamino)-5,6,7,8-tetrahydroquinazolin-2-yl]amino}cyclohexyl)methyl]-1H-indole-3-carboxamide | 537 (M + H) | 3 |
| 1079 | 2-cyclohex-1-en-1-yl-N-[(cis-4-{[4-(dimethylamino)-5,6,7,8-tetrahydroquinazolin-2-yl]amino}cyclohexyl)methyl] acetamide | 426 (M + H) | 3 |
| 1080 | N-[(cis-4-{[4-(dimethylamino)-5,6,7,8-tetrahydroquinazolin-2-yl] amino}cyclohexyl)methyl]-4-(4-ethoxyphenyl)-2-(3-methyl-5-oxo-1-phenyl-4,5-dihydro-1H-pyrazol-4-yl)-4-oxobutanamide | 680 (M + H) | 3 |
| 1081 | N-[(cis-4-{[4-(dimethylamino)-5,6,7,8-tetrahydroquinazolin-2-yl] amino}cyclohexyl)methyl]-2-[2-(trifluoromethoxy)phenyl]-acetamide | 506 (M + H) | 3 |
| 1082 | 4-(benzyloxy)-N-[(cis-4-{[4-(dimethylamino)-5,6,7,8-tetrahydroquinazolin-2-yl]amino}cyclohexyl)methyl]-3,5-dimethylbenzamide | 542 (M + H) | 3 |
| 1083 | N-[(cis-4-{(4-(dimethylamino)-5,6,7,8-tetrahydroquinazolin-2-yl] amino}cyclohexyl)methyl]-9H-xanthene-9-carboxamide | 512 (M + H) | 3 |
| 1084 | 2-(1-benzothien-3-yl)-N-[(cis-4-{[4-(dimethylamino)-5,6,7,8-tetrahydroquinazolin-2-yl]amino}cyclohexyl)methyl] acetamide | 478 (M + H) | 3 |
| 1085 | N2-{cis-4-[(2,6-dimethoxybenzyl)amino]cyclohexyl } -N4,N4-dimethylquinoline-2,4-diamine | 435 (M + H) | 3 |
| 1086 | N2-{cis-4-[(2-ethoxybenzyl)amino]cyclohexyl} -N4,N4-dimethylquinoline-2,4-diamine | 419 (M + H) | 3 |
| 1087 | N2-{cis-4-[(1H-indol-3-ylmethyl)amino]cyclohexyl} -N4,N4-dimethylquinoline-2,4-diamine | 414 (M + H) | 3 |
| 1088 | N2-{cis-4-[(2,5-dimethoxybenzyl)amino]cyclohexyl} -N4,N4-dimethylquinoline-2,4-diamine | 435 (M + H) | 3 |
| 1089 | N2-(cis-4-{[(4-methoxy-1-naphthyl)methyl]amino}cyclohexyl) -N4,N4-dimethylquinoline-2,4-diamine | 455 (M + H) | 3 |
| 1090 | N2-(cis-4-{[(5-methoxy-1H-indol-3-yl)methyl]amino}-cyclohexyl) -N4,N4-dimethylquinoline-2,4-diamine | 444 (M + H) | 3 |
| 1091 | N2-(cis-4-{[(2-methoxy-1-naphthyl)methyl]amino}cyclohexyl) -N4,N4-dimethylquinoline-2,4-diamine | 455 (M + H) | 3 |

(continued)

| Ex. No. | compound name | MS | class |
|---|---|---|---|
| 1092 | 4-bromo-2-{[(cis-4-{[4-(dimethylamino)quinolin-2-yl]amino} cyclohexyl)amino}methyl}-6-methoxyphenol | 499 (M + H) | 3 |
| 1093 | N2-(cis-4-{[(5-bromo-1H-indol-3-yl)methyl]amino}cyclohexyl) -N4,N4-dimethylquinoline-2,4-diamine | 492 (M + H) | 3 |
| 1094 | N2-{cis-4-[(2,4-dimethoxybenzyl)amino]cyclohexyl} -N4,N4-dimethylquinoline-2,4-diamine | 435 (M + H) | 3 |
| 1095 | N4,N4-dimethyl-N2-{cis-4-[(2,3,4-trimethoxybenzyl)amino] cyclohexyl}quinoline-2,4-diamine | 465 (M + H) | 3 |
| 1096 | 4-{[(cis-4-{[4-(dimethylamino)quinolin-2-yl]amino}cyclohexyl) amino]methyl}-2,6-dimethoxyphenol | 451 (M + H) | 3 |
| 1097 | N2-{cis-4-[(3-ethoxy-4-methoxybenzyl)amino]cyclohexyl} -N4,N4-dimethylquinoline-2,4-diamine | 449 (M + H) | 3 |
| 1098 | N4,N4-dimethyl-N2-{cis-4-[({3-[4-(trifluoromethyl)phenyl]-1H-pyrazol-4-yl}methyl)amino]cyclohexyl}quinoline-2,4-diamine | 509 (M + H) | 3 |
| 1099 | N4,N4-dimethyl-N2-{cis-4-[(3,4,5-trimethoxybenzyl)amino] cyclohexyl}quinoline-2,4-diamine | 465 (M + H) | 3 |
| 1100 | N4,N4-dimethyl-N2-{cis-4-[(pentamethylbenzyl)amino] cyclohexyl}quinoline-2,4-diamine | 445 (M + H) | 3 |
| 1101 | N2-{cis-4-[(3,5-dimethoxybenzyl)amino]cyclohexyl} -N4,N4-dimethylquinoline-2,4-diamine | 435 (M + H) | 3 |
| 1102 | 4-{[(cis-4-{[4-(dimethylamino)quinolin-2-yl]amino}cyclohexyl) amino]methyl}-2-iodo-6-methoxyphenol | 547 (M + H) | 3 |
| 1103 | 4-{[(cis-4-{[4-(dimethylamino)quinolin-2-yl]amino}cyclohexyl) amino]methyl}-2,6-dimethylphenol | 419 (M + H) | 3 |
| 1104 | N2-{cis-4-[(3-methoxybenzyl)amino]cyclohexyl} -N4,N4-dimethylquinoline-2,4-diamine | 405 (M + H) | 3 |
| 1105 | N2-{cis-4-[(3-bromo-4-fluorobenzyl)amino]cyclohexyl} -N4,N4-dimethylquinoline-2,4-diamine | 471 (M + H) | 3 |
| 1106 | N4,N4-dimethyl-N2-{cis-4-[(3-phenylbutyl)amino]cyclohexyl} quinoline-2,4-diamine | 417 (M + H) | 3 |
| 1107 | 3-{[(cis-4-{[4-(dimethylamino)quinolin-2-yl]amino]cyclohexyl)-amino}methyl}-6-methyl-4H-chromen-4-one | 457 (M + H) | 3 |
| 1108 | 3-{[(cis-4-{[4-(dimethylamino)quinolin-2-yl]amino}cyclohexyl)-amino]methyl}-6,8-dimethyl-4H-chromen-4-one | 471 (M + H) | 3 |
| 1109 | N2-(cis-4-{[(2,5-dimethyl-1-phenyl-1H-pyrrol-3-yl)methyl]amino} cyclohexyl)-N4,N4-dimethylquinoline-2,4-diamine | 468 (M + H) | 3 |
| 1110 | N4,N4-dimethyl-N2-{cis-4-[(2-phenylpropyl)amino]cyclohexyl} quinoline-2,4-diamine | 403 (M + H) | 3 |
| 1111 | N2-(cis-4-{[(2E)-2-benzylideneheptyl]amino}cyclohexyl) -N4,N4-dimethylquinoline-2,4-diamine | 471 (M + H) | 3 |
| 1112 | N2-(cis-4-{[(2E)-3-(2-methoxyphenyl)prop-2-en-1-yl]amino} cyclohexyl)-N4,N4-dimethylquinoline-2,4-diamine | 431 (M + H) | 3 |
| 1113 | 6-chloro-3-{[(cis-4-{[4-(dimethylamino)quinolin-2-yl]amino} cyclohexyl)amino]methyl-4H-chromen-4-one | 477 (M + H) | 3 |

(continued)

| Ex. No. | compound name | MS | class |
|---|---|---|---|
| 1114 | N2-[cis-4-({[5-(4-fluorophenyl)pyridin-3-yl]methyl}amino)-cyclohexyl]-N4,N4-dimethylquinoline-2,4-diamine | 470 (M + H) | 3 |
| 1115 | ethyl 4,6-dichloro-3-{[(cis-4-{[4-(dimethylamino)quinolin-2-yl] amino}cyclohexyl)amino]methyl}-1H-indole-2-carboxylate | 552 (M - H) | 1 |
| 1116 | methyl 2-[(5-{[(cis-4-{[4-(dimethylamino)quinolin-2-yl]amino} cyclohexyl)amino]methyl}imidazo[2,1-b][1,3]thiazol-6-yl)thio] benzoate | 587 (M + H) | 3 |
| 1117 | N2-[cis-4-({[3-(4-fluorophenyl)-1H-pyrazol-4-yl]methyl}amino)-cyclohexyl]-N4,N4-dimethylquinoline-2,4-diamine | 459 (M + H) | 3 |
| 1118 | N4,N4-dimethyl-N2-(cis-4-{(4-(methylthio)benzyl]amino} cyclohexyl)quinoline-2,4-diamine | 421 (M + H) | 3 |
| 1119 | N4,N4-dimethyl-N2-{cis-4-[(1-naphthylmethyl)amino]cyclohexyl} quinoline-2,4-diamine | 425 (M + H) | 3 |
| 1120 | 4-{[(cis-4-{[4-(dimethylamino)quinolin-2-yl]amino}cyclohexyl) amino]methyl}-2-methoxyphenol | 421 (M + H) | 3 |
| 1121 | N2-{cis-4-[(3-chloro-4-fluorobenzyl)amino]cyclohexyl} -N4,N4-dimethylquinoline-2,4-diamine | 427 (M + H) | 3 |
| 1122 | N2-(cis-4-{[(2,6-dimethoxybenzyl)amino]methyl}cyclohexyl) -N4,N4-dimethylquinoline-2,4-diamine | 449 (M + H) | 2 2 |
| 1123 | N2-(cis-4-{[(2-ethoxybenzyl)amino]methyl}cyclohexyl) -N4,N4-dimethylquinoline-2,4-diamine | 433 (M + H) | 2 |
| 1124 | N2-(cis-4-{[(1H-indol-3-ylmethyl)amino]methyl}cyclohexyl) -N4,N4-dimethylquinoline-2,4-diamine | 428 (M + H) | 3 |
| 1125 | N2-(cis-4-{[(2,5-dimethoxybenzyl)amino]methyl}cyclohexyl) -N4,N4-dimethylquinoline-2,4-diamine | 449 (M + H) | 3 |
| 1126 | N2-[cis-4-({[(4-methoxy-1-naphthyl)methyl]amino}methyl)-cyclohexyl]-N4,N4-dimethylquinoline-2,4-diamine | 469 (M + H) | 2 |
| 1127 | N2-[cis-4-({[(5-methoxy-1H-indol-3-yl)methyl]amino}-methyl) cyclohexyl]-N4,N4-dimethylquinoline-2,4-diamine | 458 (M + H) | 3 |
| 1128 | N2-[cis-4-({[(2-methoxy-1-naphthyl)methyl]amino}methyl)-cyclohexyl]-N4,N4-dimethylquinoline-2,4-diamine | 469 (M + H) | 3 |
| 1129 | 4-bromo-2-({[(cis-4-{[4-(dimethylamino)quinolin-2-yl]amino} cyclohexyl)methyl]amino}methyl)-6-methoxyphenol | 513 (M + H) | 2 |
| 1130 | N2-[cis-4-({[(5-bromo-1H-indol-3-yl)methyl]amino}methyl)-cyclohexyl]-N4,N4-dimethylquinoline-2,4-diamine | 506 (M + H) | 2 |
| 1131 | N2-(cis-4-{[2,4-dimethoxybenzyl)amino]methyl}cyclohexyl) -N4,N4-dimethylquinoline-2,4-diamine | 449 (M + H) | 3 |
| 1132 | N4,N4-dimethyl-N2-(cis-4-{(2,3,4-trimethoxybenzyl)amino]-methyl}cyclohexyl)-quinoline-2,4-diamine | 479 (M + H) | 3 |
| 1133 | 4-({[(cis-4-{[4-(dimethylamino)quinolin-2-yl]amino}cyclohexyl)-methyl]amino}methyl)-2,6-dimethoxyphenol | 465 (M + H) | 3 |
| 1134 | N2-(cis-4-{[(3-ethoxy-4-methoxybenzyl)amino]methyl}-cyclohexyl)-N4,N4-dimethylquinoline-2,4-diamine | 463 (M + H) | 3 |

(continued)

| Ex. No. | compound name | MS | class |
|---|---|---|---|
| 1135 | N4,N4-dimethyl-N2-(cis-4-{[({3-[4-(trifluoromethyl)phenyl]-1H-pyrazol-4-yl}methyl)amino]methyl}cyclohexyl)-quinoline-2,4-diamine | 523 (M + H) | 3 |
| 1136 | N4,N4-dimethyl-N2-(cis-4-{[(3,4,5-trimethoxybenzyl)amino]-methyl}cyclohexyl)-quinoline-2,4-diamine | 479 (M + H) | 3 |
| 1137 | N4,N4-dimethyl-N2-(cis-4-{[(pentamethylbenzyl)amino]-methyl} cyclohexyl)-quinoline-2,4-diamine | 459 (M + H) | 3 |
| 1138 | N2-(cis-4-{[(3,5-dimethoxybenzyl)amino]methyl}cyclohexyl) -N4,N4-dimethylquinoline-2,4-diamine | 449 (M + H) | 3 |
| 1139 | 4-({[(cis-4-[4-(dimethylamino)quinolin-2-yl]amino}cyclohexyl)-methyl]amino}methyl}-2-iodo-6-methoxyphenol | 561 (M + H) | 3 |
| 1140 | 4({[(cis-4-{[4-(dimethylamino)quinolin-2-yl]amino}cyclohexyl) methyl]amino}methyl)-2,6-dimethylphenol | 433 (M + H) | 3 |
| 1141 | N2-(cis-4-{[(4-methoxybenzyl)amino]methyl}cyclohexyl) -N4,N4-dimethylquinoline-2,4-diamine | 419 (M + H) | 3 |
| 1142 | N2-(cis-4-{[(2,3-dihydro-1,4-benzodioxin-6-ylmethyl)amino]-methyl}cyclohexyl)-N4,N4-dimethylquinoline-2,4-diamine | 447 (M + H) | 3 |
| 1143 | N2-(cis-4-{[(3-bromobenzyl)amino]methyl}cyclohexyl) -N4,N4-dimethylquinoline-2,4-diamine | 467 (M + H) | 3 |
| 1144 | N2-(cis-4-{[(5-bromo-2,4-dimethoxybenzyl)amino]methyl} cyclohexyl)-N4,N4-dimethylquinoline-2,4-diamine | 527 (M + H) | 2 |
| 1145 | N2-(cis-4-{[(5-bromo-2-methoxybenzyl)amino]methyl}-cyclohexyl)-N4,N4-dimethylquinoline-2,4-diamine | 497 (M + H) | 3 |
| 1146 | 3-chloro-4-({[(cis-4-{[4-(dimethylamino)quinolin-2-yl]amino} cyclohexyl)methyl]amino}methyl)phenol | 439 (M + H) | 3 |
| 1147 | 2-({[(cis-4-{[4-(dimethylamino)quinolin-2-yl]amino}cyclohexyl) methyl]amino}methyl)benzonitrile | 414 (M + H) | 3 |
| 1148 | N2-(cis-4-{[(3-chlorobenzyl)amino]methyl}cyclohexyl) -N4,N4-dimethylquinoline-2,4-diamine | 423 (M + H) | ? |
| 1149 | N2-(cis-4-{[(4-chlorobenzyl)amino]methyl}cyclohexyl) -N4,N4-dimethylquinoline-2,4-diamine | 423 (M + H) | 3 |
| 1150 | N2-(cis-4-({[4-(diethylamino)benzyl]amino}methyl)cyclohexyl] -N4,N4-dimethylquinoline-2,4-diamine | 460 (M + H) | 3 |
| 1151 | N2-[cis-4-({[4-(dimethylamino)benzyl]amino}methyl)-cyclohexyl] -N4,N4-dimethylquinoline-2,4-diamine | 432 (M + H) | 3 |
| 1152 | N2-[cis-4-({[(9-ethyl-9H-carbazol-3-yl)methyl]amino}methyl)-cyclohexyl]-N4,N4-dimethylquinoline-2,4-diamine | 506 (M + H) | 3 |
| 1153 | N2-[cis-4-({[2-fluoro-5-(trifluoromethyl)benzyl]amino}methyl)-cyclohexyl]-N4,N4-dimethylquinoline-2,4-diamine | 475 (M + H) | 3 |
| 1154 | 4({[(cis-4-{[4-(dimethylamino)quinolin-2-yl]amino}cyclohexyl) methyl]amino methyl)phenol | 405 (M + H) | 3 |
| 1155 | [5-({[(cis-4-{[4-(dimethylamino)quinolin-2-yl]amino}cyclohexyl) methyl]amino}methyl)-2-furyl]methanol | 409 (M + H) | 3 |
| 1156 | N2-(cis-4-{[(4-isopropoxybenzyl)amino]methyl}cyclohexyl) -N4,N4-dimethylquinoline-2,4-diamine | 447 (M + H) | 3 |

(continued)

| Ex. No. | compound name | MS | class |
|---|---|---|---|
| 1157 | N2-[cis-4-({[(5-ethyl-2-thienyl)methyl]amino}methyl)-cyclohexyl]-N4,N4-dimethylquinoline-2,4-diamine | 423 (M + H) | 3 |
| 1158 | N2-(cis-4-{[(3,3-diphenylprop-2-en-1-yl)amino]methyl}-cyclohexyl)-N4,N4-dimethylquinoline-2,4-diamine | 491 (M + H) | 1 |
| 1159 | 4({[(cis-4-{[4-(dimethylamino)quinolin-2-yl]amino}cyclohexyl) methyl]amino}methyl)-2-ethoxyphenol | 449 (M + H) | 3 |
| 1160 | N2-{cis-4-[({(4-(dimethylamino)-1-naphthyl]methyl}amino)-methyl]cyclohexyl}-N4,N4-dimethylquinoline-2,4-diamine | 482 (M + H) | 3 |
| 1161 | N4,N4-dimethyl-N2-(cis-4-{[(2,4,6-trimethoxybenzyl)-amino] methyl}cyclohexyl)quinoline-2,4-diamine | 479 (M + H) | 2 |
| 1162 | 2-bromo-4-chloro-6-({[(cis-4-{[4-(dimethylamino)quinolin-2-yl] amino}cyclohexyl)methyl]amino}methyl)phenol | 517 (M + H) | 3 |
| 1163 | 3-({[(cis-4-{[4-(dimethylamino)quinolin-2-yl]amino}cyclohexyl) methyl]amino}methyl)benzonitrile | 414 (M + H) | 3 |
| 1164 | N2-(cis-4-{[(2-fluoro-5-methoxybenzyl)amino]methyl}-cyclohexyl)-N4,N4-dimethylquinoline-2,4-diamine | 437 (M + H) | 3 |
| 1165 | N4,N4-dimethyl-N2-{cis-4-[({2-[(trifluoromethyl)thio]benzyl}-amino)methyl]cyclohexyl}quinoline-2,4-diamine | 489 (M + H) | 3 |
| 1166 | N2-(cis-4-{[(5-bromo-2-ethoxybenzyl)amino]methyl}-cyclohexyl)-N4,N4-dimethylquinoline-2,4-diamine | 511 (M + H) | 3 |
| 1167 | N2-(cis-4-{[(2,4-dimethoxy-3-methylbenzyl)amino]methyl}-cyclohexyl)-N4,N4-dimethylquinoline-2,4-diamine | 463 (M + H) | 3 |
| 1168 | N4,N4-dimethyl-N2-[cis-4-({[2-(trifluoromethoxy)benzyl]-amino} methyl)cyclohexyl]-quinoline-2,4-diamine | 473 (M + H) | 3 |
| 1169 | N2-(cis-4-{[(2,5-diethoxybenzyl)amino]methyl}cyclohexyl)-N4,N4-dimethylquinoline-2,4-diamine | 477 (M + H) | 2 |
| 1170 | N2-(cis-4-{[(2,4-diethoxybenzyl)amino]methyl}cyclohexyl)-N4,N4-dimethylquinoline-2,4-diamine | 477 (M + H) | 2 |
| 1171 | N2-(cis-4-{[(3,5-dibromo-2-methoxybenzyl)amino]methyl}-cyclohexyl)-N4,N4-dimethylquinoline-2,4-diamine | 575 (M + H) | 2 |
| 1172 | N2-(cis-4-({[2-(difluoromethoxy)benzyl]amino}methyl)-cyclohexyl]-N4,N4-dimethylquinoline-2,4-diamine | 455 (M + H) | 3 |
| 1173 | N2-(cis-4-{[(5-fluoro-2-methoxybenzyl)amino]methyl}-cyclohexyl)-N4,N4-dimethylquinoline-2,4-diamine | 437 (M + H) | 3 |
| 1174 | N4,N4-dimethyl-N2-(cis-4-{[(2,4,5-triethoxybenzyl)amino]methyl} cyclohexyl)quinoline-2,4-diamine | 521 (M + H) | 2 |
| 1175 | N4,N4-dimethyl-N2-(cis-4-{[(2,4,5-trimethoxybenzyl)amino]-methyl}cyclohexyl)-quinoline-2,4-diamine | 479 (M + H) | 2 |
| 1176 | N2-(cis-4-{[(2,3-dimethoxybenzyl)amino]methyl]cyclohexyl)-N4,N4-dimethylquinoline-2,4-diamine | 449 (M + H) | 3 |
| 1177 | N2-[cis-4-({[2-(allyloxy)benzyl]amino}methyl}cyclohexyl)-N4,N4-dimethylquinoline-2,4-diamine | 445 (M + H) | 2 |
| 1178 | N2-(cis-4-{[(1-benzothien-3-ylmethyl)amino]methyl}-cyclohexyl)-N4,N4-dimethylquinoline-2,4-diamine | 445 (M + H) | 3 |

(continued)

| Ex. No. | compound name | MS | class |
|---|---|---|---|
| 1179 | N4,N4-dimethyl-N2-[cis-4-({[(1-methyl-1H-indol-3-yl)methyl] amino}methyl)cyclohexyl]quinoline-2,4-diamine | 442 (M + H) | 3 |
| 1180 | N4,N4-dimethyl-N2-[cis-4-({[(5-methyl-2-thienyl)methyl]amino} methyl)cyclohexyl]quinoline-2,4-diamine | 409 (M + H) | 3 |
| 1181 | N2-(cis-4-{[(mesitylmethyl)amino]methyl}cyclohexyl) -N4,N4-dimethylquinoline-2,4-diamine | 431 (M + H) | 3 |
| 1182 | N2-(cis-4-{[(1,3-benzodioxol-5-ylmethyl)amino]methyl}-cyclohexyl)-N4,N4-dimethylquinoline-2,4-diamine | 433 (M + H) | 3 |
| 1183 | N4,N4-dimethyl-N2-(cis-4-{[(3-thienylmethyl)amino]methyl} cyclohexyl)quinoline-2,4-diamine | 395 (M + H) | 3 |
| 1184 | N4,N4-dimethyl-N2-(cis-4-{[(3-methylbenzyl)amino]methyl} cyclohexyl)quinoline-2,4-diamine | 403 (M + H) | 3 |
| 1185 | N4,N4-dimethyl-N2-(cis-4-{[(2-methylbenzyl)amino]methyl} cyclohexyl)quinoline-2,4-diamine | 403 (M + H) | 3 |
| 1186 | N4,N4-dimethyl-N2-(cis-4-{[(4-methylbenzyl)amino]methyl} cyclohexyl)quinoline-2,4-diamine | 403 (M + H) | 3 |
| 1187 | N2-(cis-4-{[(3,5-dichlorobenzyl)amino]methyl}cyclohexyl) -N4,N4-dimethylquinoline-2,4-diamine | 457 (M + H) | 3 |
| 1188 | N2-[cis-4-({[(7-methoxy-1,3-benzodioxol-5-yl)methyl]amino}-methyl)cyclohexyl]-N4,N4-dimethylquinoline-2,4-diamine | 463 (M + H) | 2 |
| 1189 | N2-(cis-4-{[(3-bromo-4,5-dimethoxybenzyl)amino]methyl}-cyclohexyl)-N4,N4-dimethylquinoline-2,4-diamine | 527 (M + H) | 3 |
| 1190 | N2-(cis-4-{[(4-methoxy-3-methylbenzyl)amino]methyl}-cyclohexyl)-N4,N4-dimethylquinoline-2,4-diamine | 433 (M + H) | 3 |
| 1191 | N2-(cis-4-{[(2-bromo-4,5-dimethoxybenzyl)amino]methyl}-cyclohexyl)-N4,N4-dimethylquinoline-2,4-diamine | 527 (M + H) | 3 |
| 1192 | N4,N4-dimethyl-N2-[cis-4-({[(2-methyl-5-phenyl-3-furyl)methyl] amino}methyl)cyclohexyl]quinoline-2,4-diamine | 469 (M + H) | 3 |
| 1193 | N2-(cis-4-{[(3,4-dimethoxybenzyl)amino]methyl}cyclohexyl) -N4,N4-dimethylquinoline-2,4-diamine | 449 (M + H) | 3 |
| 1194 | 4-({[(cis-4-{[4-(dimethylamino)quinolin-2-yl]amino}cyclohexyl) methyl]amino}methyl)-2-methylphenol | 419 (M + H) | 3 |
| 1195 | N2-(cis-4-{[(4-methoxy-2,5-dimethylbenzyl)amino]methyl}-cyclohexyl)-N4,N4-dimethylquinoline-2,4-diamine | 447 (M + H) | 3 |
| 1196 | 2-({[(cis-4-{[4-(dimethylamino)quinolin-2-yl]amino}cyclohexyl) methyl]amino}methyl)-6-methoxyphenol | 435 (M +H) | 3 |
| 1197 | N2-[cis-4-({[3-chloro-2-fluoro-5-(trifluoromethyl)benzyl]-amino} methyl)cyclohexyl]-N4,N4-dimethylquinoline-2,4-diamine | 509 (M + H) | 3 |
| 1198 | N2-[cis-4-({[3-fluoro-5-(trifluoromethyl)benzyl]amino}-methyl) cyclohexyl]-N4,N4-dimethylquinoline-2,4-diamine | 475 (M + H) | 3 |
| 1199 | 4-({[(cis-4-{[4-(dimethylamino)quinolin-2-yl]amino}cyclohexyl)-methyl]amino}methyl)-2-fluoro-6-methoxyphenol | 453 (M + H) | 3 |
| 1200 | N2-(cis-4-{[(2-fluoro-4,5-dimethoxybenzyl)amino]methyl}-cyclohexyl)-N4,N4-dimethylquinoline-2,4-diamine | 467 (M + H) | 3 |

(continued)

| Ex. No. | compound name | MS | class |
|---|---|---|---|
| 1201 | N2-(cis-4-{[(2-ethylbenzyl)amino]methyl}cyclohexyl) -N4,N4-dimethylquinoline-2,4-diamine | 417 (M + H) | 3 |
| 1202 | 3-[[4-({(cis-4-{(4-(dimethylamino)quinolin-2-yl]amino}-cyclohexyl) methyl]amino}methyl)phenyl)(methyl)amino]-propanenitrile | 471 (M + H) | 3 |
| 1203 | N2-{cis-4-[({4-[(4-bromobenzyl)oxy]benzyl)amino}methyl]-cyclohexyl}-N4,N4-dimethylquinoline-2,4-diamine | 573 (M + H) | 3 |
| 1204 | N2-(cis-4-{[(3,5-dibromo-2-ethoxybenzyl)amino]methyl}-cyclohexyl)-N4,N4-dimethylquinoline-2,4-diamine | 589 (M + H) | 3 |
| 1205 | N2-{cis-4-[(2,6-dimethoxybenzyl)amino]cyclohexyl} -N4,N4-dimethylpyrimidine-2,4-diamine | 386 (M + H) | 3 |
| 1206 | N2-{cis-4-[(2-ethoxybenzyl)amino]cyclohexyl] -N4,N4-dimethylpyrimidine-2,4-diamine | 370 (M + H) | 3 |
| 1207 | N2-{cis-4-[(1H-indol-3-ylmethyl)amino]cyclohexyl) -N4,N4-dimethylpyrimidine-2,4-diamine | 365 (M + H) | 3 |
| 1208 | N2-(cis-4-{2,5-dimethoxybenzyl)amino]cyclohexyl} -N4,N4-dimethylpyrimidine-2,4-diamine | 386 (M + H) | 3 |
| 1209 | N2-(cis-4-{[(4-methoxy-1-naphthyl)methyl]amino}cyclohexyl) -N4,N4-dimethylpyrimidine-2,4-diamine | 406 (M + H) | 3 |
| 1210 | N2-(cis-4-{[(5-methoxy-1H-indol-3-yl)methyl]amino}-cyclohexyl) -N4,N4-dimethylpyrimidine-2,4-diamine | 395 (M + H) | 3 |
| 1211 | N2-(cis-4-{[(2-methoxy-1-naphthyl)methyl]amino}cyclohexyl) -N4,N4-dimethylpyrimidine-2,4-diamine | 406 (M + H) | 3 |
| 1212 | 4-bromo-2-{[(cis-4-{[4-(dimethylamino)pyrimidin-2-yl]amino} cyclohexyl)amino]methyl}-6-methoxyphenol | 450 (M + H) | 3 |
| 1213 | N2-(cis-4-{[(5-bromo-1H-indol-3-yl)methyl]amino}cyclohexyl) -N4,N4-dimethylpyrimidine-2,4-diamine | 443 (M + H) | 2 |
| 1214 | N2-{cis-4-[(2,4-dimethoxybenzyl)amino]cyclohexyl} -N4,N4-dimethylpyrimidine-2,4-diamine | 386 (M + H) | 3 |
| 1215 | N4,N4-dimethyl-N2-{cis-4-[(2,3,4-trimethoxybenzyl)amino] cyclohexyl}pyrimidine-2,4-diamine | 416 (M + H) | 3 |
| 1216 | 4-{[(cis-4-{[4-(dimethylamino)pyrimidin-2-yl]amino}cyclohexyl) amino]methyl}-2,6-dimethoxyphenol | 402 (M + H) | 3 |
| 1217 | N2-{cis-4-[(3-ethoxy-4-methoxybenzyl)amino]cyclohexyl} -N4,N4-dimethylpyrimidine-2,4-diamine | 400 (M + H) | 3 |
| 1218 | N4,N4-dimethyl-N2-{cis-4-[({3-[4-(trifluoromethyl)phenyl]-1H-pyrazol-4-yl}methyl)amino]cyclohexyl}pyrimidine-2,4-diamine | 460 (M + H) | 3 |
| 1219 | N4,N4-dimethyl-N2-{cis-4-[(3,4,5-trimethoxybenzyl)amino] cyclohexyl}pyrimidine-2,4-diamine | 416 (M + H) | 3 |
| 1220 | N4,N4-dimethyl-N2-{cis-4-[(pentamethylbenzyl)amino] cyclohexyl}pyrimidine-2,4-diamine | 396 (M + H) | 3 |
| 1221 | N2-{cis-4-[(3,5-dimethoxybenzyl)amino]cyclohexyl} -N4,N4-dimethylpyrimidine-2,4-diamine | 386 (M + H) | 3 |
| 1222 | 4{[(cis-4-{[4-(dimethylamino)pyrimidin-2-yl]amino}cyclohexyl) amino]methyl}-2-iodo-6-methoxyphenol | 498 (M + H) | 3 |

(continued)

| Ex. No. | compound name | MS | class |
|---|---|---|---|
| 1223 | 4-{[(cis-4-{[4-(dimethylamino)pyrimidin-2-yl]amino}cyclohexyl) amino]methyl}-2,6-dimethylphenol | 370 (M + H) | 3 |
| 1224 | N2-{cis-4-[(3-methoxybenzyl)amino]cyclohexyl} -N4,N4-dimethylpyrimidine-2,4-diamine | 356 (M + H) | 3 |
| 1225 | N2-{cis-4-[(3-bromo-4-fluorobenzyl)amino]cyclohexyl} -N4,N4-dimethylpyrimidine-2,4-diamine | 422 (M + H) | 3 |
| 1226 | N4,N4-dimethyl-N2-{cis-4-[(3-phenylbutyl)amino]cyclohexyl} pyrimidine-2,4-diamine | 368 (M + H) | 3 |
| 1227 | 3-{[(cis-4-{[4-(dimethylamino)pyrimidin-2-yl]amino}-cyclohexyl)-amino]methyl}-6-methyl-4H-chromen-4-one | 408 (M + H) | 3 |
| 1228 | 6-chloro-3-{[(cis-4-{[4-(dimethylamino)pyrimidin-2-yl]amino}-cyclohexyl)amino]methyl}-7-methyl-4H-chromen-4-one | 442 (M + H) | 3 |
| 1229 | 3-{[(cis-4-{[4-(dimethylamino)pyrimidin-2-yl]amino}-cyclohexyl)-amino]methyl}-6,8-dimethyl-4H-chromen-4-one | 422 (M + H) | 3 |
| 1230 | N2-(cis-4-{[(2,5-dimethyl-1-phenyl-1H-pyrrol-3-yl)methyl]-amino} cyclohexyl)-N4,N4-dimethylpyrimidine-2,4-diamine | 419 (M + H) | 3 |
| 1231 | N4,N4-dimethyl-N2-{cis-4-[(2-phenylpropyl)amino]cyclohexyl} pyrimidine-2,4-diamine | 354 (M + H) | 3 |
| 1232 | N2-(cis-4-{[(2E)-2-benzylideneheptyl]amino}cyclohexyl) -N4,N4-dimethylpyrinlidine-2,4-diamine | 422 (M + H) | 3 |
| 1233 | N2-(cis-4-{[(2E)-3-(2-methoxyphenyl)prop-2-en-1-yl]amino} cyclohexyl)-N4,N4-dimethylpyrimidine-2,4-diamine | 382 (M + H) | 3 |
| 1234 | 6-chloro-3-{[(cis-4-{[4-(dimethylamino)pyrimidin-2-yl]amino} cyclohexyl)amino]methyl}-4H-chromen-4-one | 428 (M + H) | 3 |
| 1235 | N2-(cis-4-({[5-(4-fluorophenyl)pyridin-3-yl]methyl}-amino) cyclohexyl]-N4,N4-dimethylpyrimidine-2,4-diamine | 421 (M + H) | 2 |
| 1236 | ethyl 4,6-dichloro-3-{[(cis-4-{[4-(dimethylamino)pyrimidin-2-yl] amino}cyclohexyl)amino]methyl}-1H-indole-2-carboxylate | 503 (M - H) | 1 |
| 1237 | methyl 2-[(5-{[(cis-4-{[4-(dimethylamino)pyrimidin-2-yl]amino} cyclohexyl)amino]methyl}imidazo[2,1-b][1,3]thiazol-6-yl)thio] benzoate | 538 (M + H) | 3 |
| 1238 | N2-[cis-4-({[3-(4-fluorophenyl)-1H-pyrazol-4-yl]methyl}-amino) cyclohexyl]-N4,N4-dimethylpyrimidine-2,4-diamine | 410 (M + H) | 3 |
| 1239 | N4,N4-dimethyl-N2-(cis-4-{[4-(methylthio)benzyl]amino} cyclohexyl)pyrimidine-2,4-diamine | 372 (M + H) | 3 |
| 1240 | N4,N4-dimethyl-N2-{cis-4-[(1-naphthylmethyl)amino]cyclohexyl} pyrimidine-2,4-diamine | 376 (M + H) | 3 |
| 1241 | 4-{[(cis-4-[4-(dimethylamino)pyrimidin-2-yl]amino}cyclohexyl) amino]methyl}-2-methoxyphenol | 372 (M + H) | 3 |
| 1242 | N2-{cis-4-[(3-chloro-4-fluorobenzyl)amino]cyclohexyl} -N4,N4-dimethylpyrimidine-2,4-diamine | 378 (M + H) | 3 |
| 1243 | N2-(cis-4-{[(2,6-dimethoxybenzyl)amino]methyl}cyclohexyl) -N4,N4-dimethylpyrimidine-2,4-diamine | 400 (M + H) | 2 |
| 1244 | N2-(cis-4-{[(2-ethoxybenzyl)amino]methyl}cyclohexyl) -N4,N4-dimethylpyrimidine-2,4-diamine | 384 (M + H) | 2 |

(continued)

| Ex. No. | compound name | MS | class |
|---|---|---|---|
| 1245 | N2-(cis-4-{[(1H-indol-3-ylmethyl)amino]methyl}cyclohexyl) -N4,N4-dimethylpyrimidine-2,4-diamine | 379 (M + H) | 3 |
| 1246 | N2-(cis-4-{[(2,5-dimethoxybenzyl)amino]methyl}cyclohexyl) -N4,N4-dimethylpyrimidine-2,4-diamine | 400 (M + H) | 3 |
| 1247 | N2-[cis-4-({[(4-methoxy-1-naphthyl)methyl]amino}methyl)-cyclohexyl]-N4,N4-dimethylpyrimidine-2,4-diamine | 420 (M + H) | 1 |
| 1248 | N2-[cis-4-({[(5-methoxy-1H-indol-3-yl)methyl]amino)}-methyl) cyclohexyl]-N4,N4-dimethylpyrimidine-2,4-diamine | 407 (M - H) | 2 |
| 1249 | N2-[cis-4-({[(2-methoxy-1-naphthyl)methyl]amino}methyl)-cyclohexyl]-N4,N4-dimethylpyrimidine-2,4-diamine | 420 (M + H) | 1 |
| 1250 | 4-bromo-2-({[(cis-4-{[4-(dimethylamino)pyrimidin-2-yl]amino} cyclohexyl)methyl]amino}methyl)-6-methoxyphenol | 462 (M - H) | 1 |
| 1251 | N2-[cis-4-({[(5-bromo-1H-indol-3-yl)methyl]amino}methyl)-cyclohexyl]-N4,N4-dimethylpyrimidine-2,4-diamine | 455 (M - H) | 1 |
| 1252 | N2-(cis-4-{[(2,4-dimethoxybenzyl)amino]methyl}cyclohexyl) -N4,N4-dimethylpyrimidine-2,4-diamine | 400 (M + H) | 2 |
| 1253 | N4,N4-dimethyl-N2-(cis-4-{[(2,3,4-trimethoxybenzyl)-amino] methyl}cyclohexyl)-pyrimidine-2,4-diamine | 430 (M+H) | 1 |
| 1254 | 4({[(cis-4-{[4-(dimethylamino)pyrimidin-2-yl]amino}-cyclohexyl) methyl]amino}methyl)-2,6-dimethoxyphenol | 414 (M - H) | 3 |
| 1255 | N2-(cis-4-{[(3-ethoxy-4-methoxybenzyl)amino]methyl}-cyclohexyl)-N4,N4-dimethyl,pyrimidine-2,4-diamine | 414 (M + H) | 1 |
| 1256 | N4,N4-dimethyl-N2-(cis-4-{[(3-[4-(trifluoromethyl)phenyl]-1H-pyrazol-4-yl}methyl)amino]methyl}cyclohexyl)-pyrimidine-2,4-diamine | 474 (M + H) | 1 |
| 1257 | N4,N4-dimethyl-N2-(cis-4-{[(3,4,5-trimethoxybenzyl)-amino] methyl}cyclohexyl)-pyrimidine-2,4-diamine | 430 (M + H) | 2 |
| 1258 | N4,N4-dimethyl-N2-(cis-4-{[(pentamethylbenzyl)-amino]methyl} cyclohexyl)-pyrimidine-2,4-diamine | 410 (M + H) | 3 |
| 1259 | N2-(cis-4-{[(3,5-dimethoxybenzyl)amino]methyl}cyclohexyl) -N4,N4-dimethylpyrimidine-2,4-diamine | 400 (M + H) | 3 |
| 1260 | 4-({[(cis-4-{[4-(dimethylamino)pyrimidin-2-yl]amino}-cyclohexyl) methyl]amino}methyl)-2-iodo-6-methoxyphenol | 512 (M + H) | 1 |
| 1261 | 4-({[(cis-4-{[4-(dimethylamino)pyrimidin-2-yl]amino}cyclohexyl) methyl]amino}methyl)-2,6-dimethylphenol | 382 (M - H) | 1 |
| 1262 | N2-(cis-4-{[(4-methoxybenzyl)amino]methyl}cyclohexyl) -N4,N4-dimethylpyrimidine-2,4-diamine | 370 (M + H) | 3 |
| 1263 | N2-(cis-4-{[(2,3-dihydro-1,4-benzodioxin-6-ylmethyl)amino]-methyl}cyclohexyl)-N4,N4-dimethylpyrimidine-2,4-diamine | 398 (M + H) | 3 |
| 1264 | N2-(cis-4-{[(3-bromobenzyl)amino]methyl}cyclohexyl) -N4,N4-dimethylpyrimidine-2,4-diamine | 418 (M + H) | 3 |
| 1265 | N2-(cis-4-{[(5-bromo-2,4-dimethoxybenzyl)amino]methyl}-cyclohexyl)-N4,N4-dimethylpyrimidine-2,4-diamine | 478 (M + H) | 1 |
| 1266 | N2-(cis-4-{[(5-bromo-2-methoxybenzyl)amino]methyl}-cyclohexyl)-N4,N4-dimethylpyrimidine-2,4-diamine | 448 (M + H) | 1 |

(continued)

| Ex. No. | compound name | MS | class |
|---|---|---|---|
| 1267 | 3-chloro-4-({[(cis-4-{[4-(dimethylamino)pyrimidin-2-yl]amino} cyclohexyl)methyl]amino}methyl)phenol | 388 (M - H) | 3 |
| 1268 | 2-({[(cis-4-{[4-(dimethylamino)pyrimidin-2-yl]amino}cyclohexyl) methyl]amino}methyl)benzonitrile | 365 (M + H) | 3 |
| 1269 | N2-(cis-4-{[(3-chlorobenzyl)amino]methyl}cyclohexyl) -N4,N4-dimethylpyrimidine-2,4-diamine | 374 (M + H) | 3 |
| 1270 | N2-(cis-4-{[(4-chlorobenzyl)amino]methyl}cyclohexyl) -N4,N4-dimethylpyrimidine-2,4-diamine | 374 (M + H) | 3 |
| 1271 | N2-[cis-4-({[4-(diethylamino)benzyl]amino}methyl)cyclohexyl] -N4,N4-dimethylpyrimidine-2,4-diamine | 411 (M + H) | 2 |
| 1272 | N2-[cis-4-({[4-(dimethylamino)benzyl]amino}methyl)-cyclohexyl] -N4,N4-dimethylpyrimidine-2,4-diamine | 383 (M + H) | 3 |
| 1273 | N2-[cis-4-({[(9-ethyl-9H-carbazol-3-yl)methyl)amino}-methyl] cyclohexyl]-N4,N4-dimethylpyrimidine-2,4-diamine | 457 (M + H) | 1 |
| 1274 | N2-[cis-4-({[2-fluoro-5-(trifluoromethyl)benzyl]amino}-methyl) cyclohexyl]-N4,N4-dimethylpyrimidine-2,4-diamine | 426 (M + H) | 3 |
| 1275 | 4-({{[(cis-4-{[4-(dimethylamino)pyrimidin-2-yl]aminocyclohexyl) methyl]amino}methyl)phenol | 354 (M - H) | 3 |
| 1276 | [5-({[(cis-4-{[4-(dimethylamino)pyrimidin-2-yl]aminocyclohexyl) methyl]amino}methyl)-2-furyl]methanol | 360 (M + H) | 3 |
| 1277 | N2-(cis-4-{[(4-isopropoxybenzyl)amino]methyl}cyclohexyl) -N4,N4-dimethylpyrimidine-2,4-diamine | 398 (M + H) | 2 |
| 1278 | N2-[cis-4-({[(5-ethyl-2-thienyl)methyl]amino}methyl)-cyclohexyl) -N4,N4-dimethylpyrirnidine-2,4-diamine | 374 (M + H) | 3 |
| 1279 | N2-(cis-4-{[(3,3-diphenylprop-2-en-1-yl)amino]methyl}- cyclohexyl)-N4,N4-dimethylpyrimidine-2,4-diamine | 442 (M + H) | 1 |
| 1280 | 4({[(cis-4-{[4-(dimethylamino)pyrimidin-2-yl]amino}cyclohexyl) methyl]amino}methyl)-2-ethoxyphenol | 400 (M + H) | 2 |
| 1281 | N2-{cis-4-[({[4-(dimethylamino)-1-naphthyl]methyl}amino)- methyl]cyclohexyl}-N4,N4-dimethylpyrimidine-2,4-diamine | 433 (M + H) | 2 |
| 1282 | N4,N4-dimethyl-N2-(cis-4-{[(2,4,6-trimethoxybenzyl)-amino] methyl}cyclohexyl)-pyrimidine-2,4-diamine | 430 (M + H) | 1 |
| 1283 | 2-bromo-4-chloro-6-({[(cis-4-{[4-(dimethylamino)pyrimidin-2-yl] amino}cyclohexyl)methyl]amino}methyl)phenol | 468 (M + H) | 3 |
| 1284 | 3 ({[(cis-4-{[4-(dimethylamino)pyrimidin-2-yl]amino}cyclohexyl) methyl]amino}methyl)benzonitrile | 365 (M + H) | 3 |
| 1285 | N2-(cis-4-{[(2-fluoro-5-methoxybenzyl)amino]methyl}-cyclohexyl) -N4,N4-dimethylpyrimidine-2,4-diamine | 388 (M + H) | 3 |
| 1286 | N4,N4-dimethyl-N2-{cis-4-[({2-((trifluoromethyl)thio]benzyl}- amino)methyl]cyclohexyl}pyrimidine-2,4-diamine | 440 (M + H) | 3 |
| 1287 | N2-(cis-4-{[(5-bromo-2-ethoxybenzyl)amino]methyl}-cyclohexyl) -N4,N4-dimethylpyrimidine-2,4-diamine | 462 (M + H) | 1 |
| 1288 | N2-(cis-4-{[(2,4-dimethoxy-3-methylbenzyl)amino]methyl}- cyclohexyl)-N4,N4-dimethylpyrimidine-2,4-diamine | 414 (M + H) | 1 |

(continued)

| Ex. No. | compound name | MS | class |
|---|---|---|---|
| 1289 | N4,N4-dimethyl-N2-[cis-4-({[2-(trifluoromethoxy)benzyl]-amino}methyl)cyclohexyl]pyrimidine-2,4-diamine | 424 (M + H) | 3 |
| 1290 | N2-(cis-4-{[(2,5-diethoxybenzyl)amino]methyl}cyclohexyl)-N4,N4-dimethylpyrimidine-2,4-diamine | 428 (M + H) | 1 |
| 1291 | N2-(cis-4-{[(2,4-diethoxybenzyl)amino]methyl}cyclohexyl)-N4,N4-dimethylpyrimidine-2,4-diamine | 428 (M + H) | 2 |
| 1292 | N2-(cis-4-{[(3,5-dibromo-2-methoxybenzyl)amino]methyl}-cyclohexyl)-N4,N4-dimethylpyrimidine-2,4-diamine | 526 (M + H) | 1 |
| 1293 | N2-[cis-4-({[2-(difluoromethoxy)benzyl]amino}methyl)-cyclohexyl]-N4,N4-dimethylpyrimidine-2,4-diamine | 406 (M + H) | 3 |
| 1294 | N2-(cis-4-{[(5-fluoro-2-methoxybenzyl)amino]methyl}-cyclohexyl)-N4,N4-dimethylpyrimidine-2,4-diamine | 388 (M + H) | 3 |
| 1295 | N4,N4-dimethyl-N2-(cis-4-{[(2,4,5-triethoxybenzyl)-amino]methyl}cyclohexyl)-pyrimidine-2,4-diamine | 472 (M + H) | 1 |
| 1296 | N4,N4-dimethyl-N2-(cis-4-{[(2,4,5-trimethoxybenzyl)-amino]methyl}cyclohexyl)-pyrimidine-2,4-diamine | 430 (M + H) | 2 |
| 1297 | N2-(cis-4-{[(2,3-dimethoxybenzyl)amino]methyl}cyclohexyl)-N4,N4-dinnethylpyrimidine-2,4-diamine | 400 (M + H) | 3 |
| 1298 | N2-[cis-4-({[2-(allyloxy)benzyl]amino}methyl)cyclohexyl]-N4,N4-dimethylpyrimidine-2,4-diamine | 396 (M + H) | 1 |
| 1299 | N2-(cis-4-{[(1-benzothien-3-ylmethyl)amino]methyl}-cyclohexyl)-N4,N4-dimethylpyrimidine-2,4-diamine | 396 (M + H) | 3 |
| 1300 | N4,N4-dimethyl-N2-(cis-4-({[(1-methyl-1H-indol-3-yl)methyl]amino}methyl)cyclohexyl]pyrimidine-2,4-diamine | 393 (M + H) | 2 |
| 1301 | N4,N4-dimethyl-N2-[cis-4-({[(5-methyl-2-thienyl)methyl]amino}methyl)cyclohexyl]pyrimidine-2,4-diamine | 360 (M + H) | 3 |
| 1302 | N2-(cis-4-{[(mesitylmethyl)amino]methyl}cyclohexyl)-N4,N4-dimethylpyrimidine-2,4-diamine | 382 (M + H) | 3 |
| 1303 | N2-(cis-4-{[(1,3-benzodioxol-5-ylmethyl)amino]methyl}-cyclohexyl)-N4,N4-dimethylpyrimidine-2,4-diamine | 384 (M + H) | 3 |
| 1304 | N4,N4-dimethyl-N2-(cis-4-{[(3-thienylmethyl)amino]methyl}cyclohexyl)pyrimidine-2,4-diamine | 346 (M + H) | 3 |
| 1305 | N4,N4-dimethyl-N2-(cis-4-{{(3-methylbenzyl)amino]methyl}cyclohexyl)pyrimidine-2,4-diamine | 354 (M + H) | 3 |
| 1306 | N4,N4-dimethyl-N2-(cis-4-{[(2-methylbenzyl)amino}methyl}cyclohexyl)pyrimidine-2,4-diamine | 354 (M + H) | 3 |
| 1307 | N4,N4-dimethyl-N2-(cis-4-{[(4-methylbenzyl)amino]methyl}cyclohexyl)pyrimidine-2,4-diamine | 354 (M + H) | 3 |
| 1308 | N2-(cis-4-{[(3,5-dichlorobenzyl)amino]methy}cyclohexyl)-N4,N4-dimethylpyrimidine-2,4-diamine | 408 (M + H) | 3 |
| 1309 | N2-[cis-4-({[(7-methoxy-1,3-benzodioxol-5-yl)methyl]amino}-methyl)cyclohexyl]-N4,N4-dimethylpyrimidine-2,4-diamine | 414 (M +H) | 1 |
| 1310 | N2-(cis-4-{[(3-bromo-4,5-dimethoxybenzyl)amino]methyl}-cyclohexyl)-N4,N4-dimethylpyrimidine-2,4-diamine | 478 (M + H) | 1 |

(continued)

| Ex. No. | compound name | MS | class |
|---|---|---|---|
| 1311 | N2-(cis-4-{[(4-methoxy-3-methylbenzyl)amino]methyl}-cyclohexyl)-N4,N4-dimethylpyrimidine-2,4-diamine | 384 (M + H) | 2 |
| 1312 | N2-(cis-4-{[(2-bromo-4,5-dimethoxybenzyl)amino]methyl}-cyclohexyl)-N4,N4-dimethylpyrimidine-2,4-diamine | 478 (M + H) | 2 |
| 1313 | N4,N4-dimethyl-N2-[cis-4-({[(2-methyl-5-phenyl-3-furyl)methyl] amino}methyl)cyclohexyl]pyrimidine-2,4-diamine | 420 (M + H) | 3 |
| 1314 | N2-(cis-4-{[(3,4-dimethoxybenzyl)amino]methyl}cyclohexyl) -N4,N4-dimethylpyrimidine-2,4-diamine | 400 (M + H) | 2 |
| 1315 | 4-({[(cis-4-{[4-(dimethylamino)pyrimidin-2-yl]amino}cyclohexyl) methyl]amino}methyl)-2-methylphenol | 368 (M - H) | 3 |
| 1316 | N2-(cis-4-{[(4-methoxy-2,5-dimethylbenzyl)amino]methyl}-cyclohexyl)-N4,N4-dimethylpyrimidine-2,4-diamine | 398 (M + H) | 2 |
| 1317 | 2-({[(cis-4-{[4-(dimethylamino)pyrimidin-2-yl]amino}cyclohexyl) methyl]amino}methyl)-6-methoxyphenol | 386 (M + H) | 3 |
| 1318 | N2-[cis-4-({[3-chloro-2-fluoro-5-(trifluoromethyl)benzyl]amino}-methyl)cyclohexyl]-N4,N4-dimethylpyrimidine-2,4-diamine | 460 (M + H) | 3 |
| 1319 | N2-[cis-4-({[3-fluoro-5-(trifluoromethyl)benzyl]amino}-methyl) cyclohexyl]-N4,N4-dimethylpyrimidine-2,4-diamine | 426 (M + H) | 3 |
| 1320 | 4-({[(cis-4-{[4-(dimethylamino)pyrimidin-2-yl]amino}-cyclohexyl) methyl]amino}methyl)-2-fluoro-6-methoxyphenol | 402 (M - H) | 3 |
| 1321 | N2-(cis-4-{[(2-fluoro-4,5-dimethoxybenzyl)amino]methyl}-cyclohexyl)-N4,N4-dimethylpyrimidine-2,4-diamine | 418 (M + H) | 3 |
| 1322 | N2-(cis-4-{[(2-ethylbenzyl)amino]methyl}cyclohexyl) -N4,N4-dimethylpyrimidine-2,4-diamine | 368 (M + H) | 3 |
| 1323 | 3-[[4-({[(cis-4-{[4-(dimethylamino)pyrimidin-2-yl]amino}-cyclohexyl)-methyl]amino}methyl)phenyl](methyl)amino]-propanenitrile | 422 (M + H) | 2 |
| 1324 | N2-{cis-4-[({4-[(4-bromobenzyl)oxy]benzyl}amino)methyl]-cyclohexyl}-N4,N4-dimethylpyrimidine-2,4-diamine | 524 (M + H) | 2 |
| 1325 | N2-(cis-4-{[(3,5-dibromo-2-ethoxybenzyl)amino]methyl}-cyclohexyl)-N4,N4-dimethylpyrimidine-2,4-diamine | 540 (M + H) | 2 |
| 1326 | N2-{cis-4-[(2,6-dimethoxybenzyl)amino]cyclohexyl} -N4,N4-dimethyl-5,6,7,8-tetrahydroquinazoline-2,4-diamine | 440 (M + H) | 3 |
| 1327 | N2-{cis-4-[(2-ethoxybenzyl)amino]cyclohexyl}-N4,N4-dimethyl-5,6,7,8-tetrahydroquinazoline-2,4-diamine | 424 (M + H) | 3 |
| 1328 | N2-{cis-4-[(1H-indol-3-ylmethyl)amino]cyclohexyl} -N4,N4-dimethyl-5,6,7,8-tetrahydroquinazoline-2,4-diamine | 419 (M + H) | 3 |
| 1329 | N2-{cis-4-[(2,5-dimethoxybenzyl)amino]cyclohexyl} -N4,N4-dimethyl-5,6,7,8-tetrahydroquinazoline-2,4-diamine | 440 (M + H) | 3 |
| 1330 | N2-(cis-4-{[(4-methoxy-1-naphthyl)methyl]amino}cyclohexyl) -N4,N4-dimethyl-5,6,7,8-tetrahydroquinazoline-2,4-diamine | 460 (M + H) | 3 |
| 1331 | N2-(cis-4-{[(5-methoxy-1H-indol-3-yl)methyl]amino}-cyclohexyl) -N4,N4-dimethyl-5,6,7,8-tetrahydroquinazoline-2,4-diamine | 449 (M + H) | 1 |
| 1332 | N2-(cis-4-{[(2-methoxy-1-naphthyl)methyl]amino}cyclohexyl) -N4,N4-dimethyl-5,6,7,8-tetrahydroquinazoline-2,4-diamine | 460 (M + H) | 3 |

(continued)

| Ex. No. | compound name | MS | class |
|---|---|---|---|
| 1333 | 4-bromo-2-{[(cis-4-{[4-(dimethylamino)-5,6,7,8-tetrahydro-quinazolin-2-yl]amino}cyclohexyl)amino]methyl]-6-methoxyphenol | 504 (M + H) | 3 |
| 1334 | N2-(cis-4-{[(5-bromo-1H-indol-3-yl)methyl]amino}cyclohexyl)-N4,N4-dimethyl-5,6,7,8-tetrahydroquinazoline-2,4-diamine | 497 (M + H) | 3 |
| 1335 | N2-{cis-4-[(2,4-dimethoxybenzyl)amino]cyclohexyl}-N4,N4-dimethyl-5,6,7,8-tetrahydroquinazoline-2,4-diamine | 440 (M + H) | 3 |
| 1336 | N4,N4-dimethyl-N2-{cis-4-[(2,3,4-trimethoxybenzyl)amino]-cyclohexyl}-5,6,7,8-tetrahydroquinazoline-2,4-diamine | 470 (M + H) | 3 |
| 1337 | 4-{[(cis-4-{[4-(dimethylamino)-5,6,7,8-tetrahydroquinazolin-2-yl]amino}cyclohexyl)amino]methyl }-2,6-dimethoxyphenol | 456 (M + H) | 2 |
| 1338 | N2-{cis-4-[(3-ethoxy-4-methoxybenzyl)amino]cyclohexyl}-N4,N4-dimethyl-5,6,7,8-tetrahydroquinazoline-2,4-diamine | 454 (M + H) | 3 |
| 1339 | N4,N4-dimethyl-N2-{cis-4-[(3-[4-(trifluoromethyl)phenyl]-1H-pyrazol-4-yl}methyl)amino]cyclohexyl}-5,6,7,8-tetrahydroquinazoline-2,4-diamine | 514 (M + H) | 3 |
| 1340 | N4,N4-dimethyl-N2-{cis-4-[(3,4,5-trimethoxybenzyl)amino]-cyclohexyl}-5,6,7,8-tetrahydroquinazoline-2,4-diamine | 470 (M + H) | 3 |
| 1341 | N4,N4-dimethyl-N2-{cis-4-[(pentamethylbenzyl)amino]-cyclohexyl}-5,6,7,8-tetrahydroquinazoline-2,4-diamine | 450 (M + H) | 2 |
| 1342 | N2-{cis-4-[(3,5-dimethoxybenzyl)amino]cyclohexyl}-N4,N4-dimethyl-5,6,7,8-tetrahydroquinazoline-2,4-diamine | 440 (M + H) | 2 |
| 1343 | 4-{[(cis-4-{[4-(dimethylamino)-5,6,7,8-tetrahydroquinazolin-2-yl]amino}cyclohexyl)amino]methyl}-2-iodo-6-methoxyphenol | 552 (M + H) | 2 |
| 1344 | 4-{[(cis-4-{[4-(dimethylamino)-5,6,7,8-tetrahydroquinazolin-2-yl]amino}cyclohexyl)amino]methyl}-2,6-dimethylphenol | 424 (M + H) | 3 |
| 1345 | N2-{cis-4-[(3-methoxybenzyl)amino]cyclohexyl}-N4,N4-dimethyl-5,6,7,8-tetrahydroquinazoline-2,4-diamine | 410 (M + H) | 3 |
| 1346 | N2-{cis-4-[(3-bromo-4-fluorobenzyl)amino]cyclohexyl}-N4,N4-dimethyl-5,6,7,8-tetrahydroquinazoline-2,4-diamine | 476 (M + H) | 3 |
| 1347 | N4,N4-dimethyl-N2-{cis-4-[(3-phenylbutyl)amino]cyclohexyl}-5,6,7,8-tetrahydroquinazoline-2,4-diamine | 422 (M + H) | 3 |
| 1348 | 3-[(cis-4-{[4-(dimethylamino)-5,6,7,8-tetrahydroquinazolin-2-yl]amino}cyclohexyl)amino]methyl}-6-methyl-4H-chromen-4-one | 462 (M + H) | 3 |
| 1349 | 6-chloro-3-{[(cis-4-{[4-(dimethylamino)-5,6,7,8-tetrahydroquinazolin-2-yl]amino}cyclohexyl)amino]methyl}-7-methyl-4H-chromen-4-one | 496 (M + H) | 3 |
| 1350 | 3-{[(cis-4-{[4-(dimethylamino)-5,6,7,8-tetrahydroquinazolin-2-yl]amino}cyclohexyl)amino]methyl}-6,8-dimethyl-4H-chromen-4-one | 476 (M + H) | 2 |
| 1351 | N2-(cis-4-{[2,5-dimethyl-1-phenyl-1H-pyrrol-3-yl)methyl]amino}cyclohexyl)-N4,N4-dimethyl-5,6,7,8-tetrahydroquinazoline-2,4-diamine | 473 (M + H) | 3 |
| 1352 | N4,N4-dimethyl-N2-{cis-4-[(2-phenylpropyl)amino]cyclohexyl}-5,6,7,8-tetrahydroquinazoline-2,4-diamine | 408 (M + H) | 3 |

(continued)

| Ex. No. | compound name | MS | class |
|---|---|---|---|
| 1353 | N2-(cis-4-{[(2E)-2-benzylideneheptyl]amino}cyclohexyl) -N4,N4-dimethyl-5,6,7,8-tetrahydroquinazoline-2,4-diamine | 476 (M + H) | 3 |
| 1354 | N2-(cis-4-{[(2E)-3-(2-methoxyphenyl)prop-2-en-1-yl]amino} cyclohexyl)-N4,N4-dimethyl-5,6,7,8-tetrahydroquinazoline-2,4-diamine | 436 (M + H) | 3 |
| 1355 | 6-chloro-3-{[(cis-4-{[4-(dimethylamino)-5,6,7,8-tetrahydroquinazolin-2-yl]amino}cyclohexyl)amino] methyl}-4H-chromen-4-one | 482 (M + H) | 3 |
| 1356 | N2-[cis-4-({[5-(4-fluorophenyl)pyridin-3-yl]methyl}-amino) cyclohexyl]-N4,N4-dimethyl-5,6,7,8-tetrahydroquinazoline-2,4-diamine | 475 (M + H) | 3 |
| 1357 | ethyl 4,6-dichloro-3-{[(cis-4-{[4-(dimethylamino)-5,6,7,8-tetrahydroquinazolin-2-yl]amino}cyclohexyl)amino] methyl}-1H-indole-2-carboxylate | 559 (M + H) | 1 |
| 1358 | methyl 2-[(5-{[(cis-4-{[4-(dimethylamino)-5,6,7,8-tetrahydroquinazolin-2-yl]amino}cyclohexyl)amino]-methyl}imidazo-[2,1-b][1,3]thiazol-6-yl)thio]benzoate | 592 (M + H) | 3 |
| 1359 | N2-[cis-4-({[3-(4-fluorophenyl)-1H-pyrazol-4-yl]methyl}amino) cyclohexyl]-N4,N4-dimethyl-5,6,7,8-tetrahydroquinazoline-2,4-diamine | 464 (M + H) | 1 |
| 1360 | N4,N4-dimethyl-N2-(cis-4-{[4-(methylthio)benzyl]amino}-cyclohexyl)-5,6,7,8-tetrahydroquinazoline-2,4-diamine | 426 (M + H) | 3 |
| 1361 | N4,N4-dimethyl-N2-{cis-4-[(1-naphthylmethyl)amino]-cyclohexyl}-5,6,7,8-tetrahydroquinazoline-2,4-diamine | 430 (M + H) | 3 |
| 1362 | 4-{[(cis-4-{[4-(dimethylamino)-5,6,7,8-tetrahydroquinazolin-2-yl] amino]cyclohexyl)amino]methyl-2-methoxyphenol | 426 (M + H) | 3 |
| 1363 | N2-{cis-4-[(3-chloro-4-fluorobenzyl)amino]cyclohexyl} -N4,N4-dimethyl-5,6,7,8-tetrahydroquinazoline-2,4-diamine | 432 (M + H) | 3 |
| 1364 | N2-(cis-4-{[(2,6-dimethoxybenzyl)amino]methyl}cyclohexyl) -N4,N4-dimethyl-5,6,7,8-tetrahydroquinazoline-2,4-diamine | 454 (M + H) | 1 |
| 1365 | N2-(cis-4-{[(2-ethoxybenzyl)amino]methyl}cyclohexyl) -N4,N4-dimethyl-5,6,7,8-tetrahydroquinazoline-2,4-diamine | 438 (M + H) | 2 |
| 1366 | N2-(cis-4-{[(1H-indol-3-ylmethyl)amino]methyl}cyclohexyl) -N4,N4-dimethyl-5,6,7,8-tetrahydroquinazoline-2,4-diamine | 433 (M + H) | 2 |
| 1367 | N2-(cis-4-{[(2,5-dimethoxybenzyl)amino]methyl}cyclohexyl) -N4,N4-dimethyl-5,6,7,8-tetrahydroquinazoline-2,4-diamine | 454 (M + H) | 2 |
| 1368 | N2-[cis-4-({[(4-methoxy-1-naphthyl)methyl]amino]methyl)-cyclohexyl]-N4,N4-dimethyl-5,6,7,8-tetrahydroquinazoline-2,4-diamine | 474 (M + H) | 2 |
| 1369 | N2-[cis-4-({[(5-methoxy-1H-indol-3-yl)methyl]amino}-methyl) cyclohexyl]-N4,N4-dimethyl-5,6,7,8-tetrahydro-quinazoline-2,4-diamine | 463 (M + H) | 1 |
| 1370 | N2-[cis-4-({[(2-methoxy-1-naphthyl)methyl]amino}methyl)-cyclohexyl]-N4,N4-dimethyl-5,6,7,8-tetrahydro-quinazoline-2,4-diamine | 474 (M + H) | 3 |

(continued)

| Ex. No. | compound name | MS | class |
|---|---|---|---|
| 1371 | 4-bromo-2-({[(cis-4-{[4-(dimethylamino)-5,6,7,8-tetrahydroquinazolin-2-yl]amino}cyclohexyl)methyl amino}methyl)-6-methoxyphenol | 518 (M + H) | 2 |
| 1372 | N2-[cis-4-({[(5-bromo-1H-indol-3-yl)methyl]amino}methyl)-cyclohexyl]-N4,N4-dimethyl-5,6,7,8-tetrahydro-quinazoline-2,4-diamine | 511 (M + H) | 1 |
| 1373 | N2-(cis-4-{[(2,4-dimethoxybenzyl)amino]methyl}cyclohexyl) N4,N4-dimethyl-5,6,7,8-tetrahydroquinazoline-2,4-diamine | 454 (M + H) | 3 |
| 1374 | N4,N4-dimethyl-N2-(cis-4-{[(2,3,4-trimethoxybenzyl)-amino] methyl}cyclohexyl)-5,6,7,8-tetrahydro-quinazoline-2,4-diamine | 484 (M + H) | 3 |
| 1375 | 4-({[(cis-4-{[4-(dimethylamino)-5,6,7,8-tetrahydroquinazolin-2-yl] amino}cyclohexyl)methyl]amino}methyl)-2,6-dimethoxyphenol | 470 (M + H) | 3 |
| 1376 | N2-(cis-4-{[(3-ethoxy-4-methoxybenzyl)amino]methyl}-cyclohexyl)-N4,N4-dimethyl-5,6,7,8-tetrahydro-quinazoline-2,4-diamine | 468 (M + H) | 1 |
| 1377 | N4,N4-dimethyl-N2-(cis-4-{[({3-[4-(trifluoromethyl)phenyl]-1H-pyrazol-4-yl}methyl)amino]methyl}cyclohexyl)-5,6,7,8-tetrahydroquinazoline-2,4-diamine | 528 (M+H) | 2 |
| 1378 | N4,N4-dimethyl-N2-(cis-4-{[(3,4,5-trimethoxybenzyl)amino] methyl}cyclohexyl)-5,6,7,8-tetrahydroquinazoline-2,4-diamine | 484 (M + H) | 2 |
| 1379 | N4,N4-dimethyl-N2-(cis-4-{[(pentamethylbenzyl)amino]-methyl} cyclohexyl)-5,6,7,8-tetrahydroquinazoline-2,4-diamine | 464 (M + H) | 3 |
| 1380 | N2-(cis-4-{[(3,5-dimethoxybenzyl)amino]methyl}cyclohexyl) -N4,N4-dimethyl-5,6,7,8-tetrahydroquinazoline-2,4-diamine | 454 (M + H) | 2 |
| 1381 | 4-({[(cis-4-{[4-(dimethylamino)-5,6,7,8-tetrahydroquinazolin-2-yl] amino}cyclohexyl)methyl]amino}methyl)-2-iodo-6-methoxyphenol | 566 (M + H) | 1 |
| 1382 | 4{([(cis-4-{[4-(dimethylamino)-5,6,7,8-tetrahydroquinazolin-2-yl] amino}cyclohexyl)methyl]amino}methyl)-2,6-dimethylphenol | 438 (M + H) | 2 |
| 1383 | N2-(cis-4-{[(4-methoxybenzyl)amino]methyl}cyclohexyl) -N4,N4-dimethyl-5,6,7,8-tetrahydroquinazoline-2,4-diamine | 424 (M + H) | 3 |
| 1384 | N2-(cis-4-{[(2,3-dihydro- 1,4-benzodioxin-6-ylmethyl)amino] methyl}cyclohexyl)-N4,N4-dimethyl-5,6,7,8-tetrahydroquinazoline-2,4-diamine | 452 (M + H) | 3 |
| 1385 | N2-(cis-4-{[(3-bromobenzyl)amino]methyl}cyclohexyl) -N4,N4-dimethyl-5,6,7,8-tetrahydroquinazoline-2,4-diamine | 472 (M + H) | 3 |
| 1386 | N2-(cis-4-{[(5-bromo-2,4-dimethoxybenzyl)amino]methyl)-cyclohexyl)-N4,N4-dimethyl-5,6,7,8-tetrahydroquinazoline-2,4-diamine | 532 (M + H) | 3 |
| 1387 | N2-(cis-4-{[(5-bromo-2-methoxybenzyl)amino]methyl}-cyclohexyl)-N4,N4-dimethyl-5,6,7,8-tetrahydroquinazoline-2,4-diamine | 502 (M + H) | 3 |
| 1388 | 3-chloro-4-({[(cis-4-{[4-(dimethylamino)-5,6,7,8-tetrahydroquinazolin-2-yl]amino}cyclohexyl)methyl] amino}-methyl)phenol | 444 (M + H) | 2 |
| 1389 | 2({[(cis-4-{[4-(dimethylamino)-5,6,7,8-tetrahydroquinazolin-2-yl] amino}cyclohexyl)methyl]amino}methyl)benzonitrile | 419 (M + H) | 3 |

(continued)

| Ex. No. | compound name | MS | class |
|---|---|---|---|
| 1390 | N2-(cis-4-{[(3-chlorobenzyl)amino]methyl}cyclohexyl) -N4,N4-dimethyl-5,6,7,8-tetrahydroquinazoline-2,4-diamine | 428 (M + H) | 3 |
| 1391 | N2-(cis-4-{[(4-chlorobenzyl)amino]methyl}cyclohexyl) -N4,N4-dimethyl-5,6,7,8-tetrahydroquinazoline-2,4-diamine | 428 (M + H) | 3 |
| 1392 | N2-[cis-4-({[4-(diethylamino)benzyl]amino}methyl)cyclohexyl] -N4,N4-dimethyl-5,6,7,8-tetrahydroquinazoline-2,4-diamine | 465 (M + H) | 2 |
| 1393 | N2-[cis-4-({[4-(dimethylamino)benzyl]amino}methyl)-cyclohexyl] -N4,N4-dimethyl-5,6,7,8-tetrahydroquinazoline-2,4-diamine | 437 (M + H) | 3 |
| 1394 | N2-[cis-4-({[(9-ethyl-9H-carbazol-3-yl)methyl]amino}-methyl) cyclohexyl]-N4,N4-dimethyl-5,6,7,8-tetrahydro-quinazoline-2,4-diamine | 511 (M + H) | 3 |
| 1395 | N2-[cis-4-({[2-fluoro-5-(trifluoromethyl)benzyl]amino}-methyl) cyclohexyl]-N4,N4-dimethyl-5,6,7,8-tetrahydro-quinazoline-2,4-diamine | 480 (M + H) | 3 |
| 1396 | 4-({[(cis-4-{[4-(dimethylamino)-5,6,7,8-tetrahydroquinazolin-2-yl] amino}cyclohexyl)methyl]amino}methyl)phenol | 410 (M + H) | 3 |
| 1397 | [5({[(cis-4-{[4-(dimethylamino)-5,6,7,8-tetrahydroquinazolin-2-yl] amino}cyclohexyl)rnethyl]amino}methyl)-2-furyl]methanol | 414 (M + H) | 3 |
| 1398 | N2-(cis-4-{[(4-isopropoxybenzyl)amino]methyl}cyclohexyl) -N4,N4-dimethyl-5,6,7,8-tetrahydroquinazoline-2,4-diamine | 452 (M + H) | 3 |
| 1399 | N2-[cis-4-({[(5-ethyl-2-thienyl)methyl]amino}methyl)-cyclohexyl] -N4,N4-dimethyl-5,6,7,8-tetrahydroquinazoline-2,4-diamine | 428 (M + H) | 3 |
| 1400 | N2-(cis-4-{[(3,3-diphenylprop-2-en-1-yl)amino]methyl}-cyclohexyl)-N4,N4-dimethyl-5,6,7,8-tetrahydroquinazoline-2,4-diamine | 496 (M + H) | 1 |
| 1401 | 4({[(cis-4-{[4-(dimethylamino)-5,6,7,8-tetrahydroquinazolin-2-yl] amino}cyclohexyl)methyl]amino}methyl)-2-ethoxyphenol | 454 (M + H) | 2 |
| 1402 | N2-{cis-4-[({[4-(dimethylamino)-1-naphthyl]methyl}amino)-methyl]cyclohexyl}-N4,N4-dimethyl-5,6,7,8-tetrahydro-quinazoline-2,4-diamine | 487 (M + H) | 2 |
| 1403 | N4,N4-dimethyl-N2-(cis-4-{[(2,4,6-trimethoxybenzyl)-amino] methyl}cyclohexyl)-5,6,7,8-tetrahydroquinazoline-2,4-diamine | 484 (M + H) | 1 |
| 1404 | 2-bromo-4-ch!oro-6-({[(cis-4-{[4-(dimethylamino)-5,6,7,8-tetrahydroquinazolin-2-yl]amino}cyclohexyl)methyl]-amino}methyl)phenol | 522 (M + H) | 2 |
| 1405 | 3-({[(cis-4-{[4-(dimethylamino)-5,6,7,8-tetrahydroquinazolin-2-yl] amino}cyclohexyl)methyl]amino}methyl)benzonitrile | 419 (M + H) | 3 |
| 1406 | N2-(cis-4-{[(2-fluoro-5-methoxybenzyl)amino]methyl}-cyclohexyl) -N4,N4-dimethyl-5,6,7,8-tetrahydroquinazoline-2,4-diamine | 442 (M + H) | 3 |
| 1407 | N4,N4-dimethyl-N2-{cis-4-[({2-[(trifluoromethyl)thio]-benzyl} amino)methyl]cyclohexyl}-5,6,7,8-tetrahydro-quinazoline-2,4-diamine | 494 (M + H) | 3 |
| 1408 | N2-(cis-4-{[(5-bromo-2-ethoxybenzyl)amino]methyl}-cyclohexyl) -N4,N4-dimethyl-5,6,7,8-tetrahydroquinazoline-2,4-diamine | 516 (M + H) | 3 |

(continued)

| Ex. No. | compound name | MS | class |
|---|---|---|---|
| 1409 | N2-(cis-4-{[(2,4-dimethoxy-3-methylbenzyl)amino]methyl}-cyclohexyl)-N4,N4-dimethyl-5,6,7,8-tetrahydroquinazoline-2,4-diamine | 468 (M + H) | 3 |
| 1410 | N4,N4-dimethyl-N2-[cis-4-({[2-(trifluoromethoxy)benzyl]-amino}methyl)cyclohexyl]-5,6,7,8-tetrahydroquinazoline-2,4-diamine | 478 (M + H) | 3 |
| 1411 | N2-(cis-4-{[(2,5-diethoxybenzyl)amino]methyl}cyclohexyl)-N4,N4-dimethyl-5,6,7,8-tetrahydroquinazoline-2,4-diamine | 482 (M + H) | 1 |
| 1412 | N2-(cis-4-{[(2,4-diethoxybenzyl)amino]methyl}cyclohexyl)-N4,N4-dimethyl-5,6,7,8-tetrahydroquinazoline-2,4-diamine | 482 (M + H) | 1 |
| 1413 | N2-(cis-4-{[(3,5-dibromo-2-methoxybenzyl)amino]methyl}-cyclohexyl)-N4,N4-dimethyl-5,6,7,8-tetrahydroquinazoline-2,4-diamine | 580 (M + H) | 1 |
| 1414 | N2-[cis-4-({[2-(difluoromethoxy)benzyl]amino}methyl)-cyclohexyl]-N4,N4-dimethyl-5,6,7,8-tetrahydroquinazoline-2,4-diamine | 460 (M + H) | 3 |
| 1415 | N2-(cis-4-{[(5-fluoro-2-methoxybenzyl)amino]methyl}-cyclohexyl)-N4,N4-dimethyl-5,6,7,8-tetrahydroquinazoline-2,4-diamine | 442 (M + H) | 3 |
| 1416 | N4,N4-dimethyl-N2-(cis-4-{[(2,4,5-triethoxybenzyl)amino]-methyl}cyclohexyl}-5,6,7,8-tetrahydroquinazoline-2,4-diamine | 526 (M + H) | 1 |
| 1417 | N4,N4-dimethyl-N2-(cis-4-{[(2,4,5-trimethoxybenzyl)amino]-methyl}cyclohexyl)-5,6,7,8-tetrahydroquinazoline-2,4-diamine | 484 (M + H) | 1 |
| 1418 | N2-(cis-4-{[(2,3-dimethoxybenzyl)amino]methyl}cyclohexyl)-N4,N4-dimethyl-5,6,7,8-tetrahydroquinazoline-2,4-diamine | 454 (M + H) | 3 |
| 1419 | N2-[cis-4-({[2-(allyloxy)benzyl]amino}methyl)cyclohexyl]-N4,N4-dimethyl-5,6,7,8-tetrahydroquinazoline-2,4-diamine | 450 (M + H) | 3 |
| 1420 | N2-(cis-4-{[(1-benzothien-3-ylmethyl)amino]methyl}-cyclohexyl)-N4,N4-dimethyl-5,6,7,8-tetrahydroquinazoline-2,4-diamine | 450 (M + H) | 3 |
| 1421 | N4,N4-dimethyl-N2-[cis-4-({[(1-methyl-1H-indol-3-yl)methyl]amino}methyl)cyclohexyl]-5,6,7,8-tetrahydroquinazoline-2,4-diamine | 447 (M + H) | 3 |
| 1422 | N4,N4-dimethyl-N2-[cis-4-({[(5-methyl-2-thienyl)methyl]-amino}methyl)cyclohexyl]-5,6,7,8-tetrahydroquinazoline-2,4-diamine | 414 (M + H) | 3 |
| 1423 | N2-(cis-4-{[(mesitylmethyl)amino]methyl}cyclohexyl)-N4,N4-dimethyl-5,6,7,8-tetrahydroquinazoline-2,4-diamine | 436 (M + H) | 3 |
| 1424 | N2-(cis-4-{[(1,3-benzodioxol-5-ylmethyl)amino]methyl}cyclohexyl)-N4,N4-dimethyl-5,6,7,8-tetrahydroquinazoline-2,4-diamine | 438 (M + H) | 3 |
| 1425 | N4,N4-dimethyl-N2-(cis-4-{[(3-thienylmethyl)amino]methyl}cyclohexyl)-5,6,7,8-tetrahydroquinazoline-2,4-diamine | 400 (M + H) | 3 |
| 1426 | N4,N4-dimethyl-N2-(cis-4-{[(3-methylbenzyl)amino]methyl}cyclohexyl)-5,6,7,8-tetrahydroquinazoline-2,4-diamine | 408 (M + H) | 3 |
| 1427 | N4,N4-dimethyl-N2-(cis-4-{[(2-methylbenzyl)amino]methyl}cyclohexyl)-5,6,7,8-tetrahydroquinazoline-2,4-diamine | 408 (M + H) | 3 |
| 1428 | N4,N4-dimethyl-N2-(cis-4-{[(4-methylbenzyl)amino]methyl}cyclohexyl)-5,6,7,8-tetrahydroquinazoline-2,4-diamine | 408 (M + H) | 3 |

(continued)

| Ex. No. | compound name | MS | class |
|---|---|---|---|
| 1429 | N2-(cis-4-{[(3,5-dichlorobenzyl)amino]methyl}cyclohexyl) -N4,N4-dimethyl-5,6,7,8-tetrahydroquinazoline-2,4-diamine | 462 (M + H) | 3 |
| 1430 | N2-[cis-4-({[(7-methoxy-1,3-benzodioxol-5-yl)methyl]amino} methyl)cyclohexyl]-N4,N4-dimethyl-5,6,7,8-tetrahydroquinazoline-2,4-diamine | 468 (M + H) | 2 |
| 1431 | N2-(cis-4-{([(3-bromo-4,5-dimethoxybenzyl)amino]methyl}-cyclohexyl)-N4,N4-dimethyl-5,6,7,8-tetrahydroquinazoline-2,4-diamine | 532 (M + H) | 3 |
| 1432 | N2-(cis-4-{[(4-methoxy-3-methylbenzyl)amino]methyl}-cyclohexyl)-N4,N4-dimethyl-5,6,7,8-tetrahydroquinazoline-2,4-diamine | 438 (M + H) | 3 |
| 1433 | N2-(cis-4-{[(2-bromo-4,5-dimethoxybenzyl)amino]methyl}-cyclohexyl)-N4,N4-dimethyl-5,6,7,8-tetrahydroquinazoline-2,4-diamine | 532 (M + H) | 3 |
| 1434 | N4,N4-dimethyl-N2-[cis-4-({[(2-methyl-5-phenyl-3-furyl)methyl] amino}methyl)cyclohexyl]-5,6,7,8-tetrahydroquinazoline-2,4-diamine | 474 (M + H) | 3 |
| 1435 | N2-(cis-4-{[(3,4-dimethoxybenzyl)amino]methyl}cyclohexyl) -N4,N4-dimethyl-5,6,7,8-tetrahydroquinazoline-2,4-diamine | 454 (M + H) | 3 |
| 1436 | 4-({[(cis-4-{[4-(dimethylamino)-5,6,7,8-tetrahydroquinazolin-2-yl] amino}cyclohexyl)methyl]amino}methyl)-2-methylphenol | 424 (M + H) | 2 |
| 1437 | N2-(cis-4-{([(4-methoxy-2,5-dimethylbenzyl)amino]methyl} cyclohexyl)-N4,N4-dimethyl-5,6,7,8-tetrahydroquinazoline-2,4-diamine | 452 (M + H) | 2 |
| 1438 | 2-({[(cis-4-{[4-(dimethylamino)-5,6,7,8-tetrahydroquinazolin-2-yl] amino}cyclohexyl)methyl]amino}methyl)-6-methoxyphenol | 440 (M + H) | 3 |
| 1439 | N2-[cis-4-({[3-chloro-2-fluoro-5-(trifluoromethyl)benzyl]-amino} methyl)cyclohexyl]-N4,N4-dimethyl-5,6,7,8-tetrahydroquinazoline-2,4-diamine | 514 (M + H) | 3 |
| 1440 | N2-[cis-4-({[3-fluoro-5-(trifluoromethyl)benzyl]amino}-methyl) cyclohexyl]-N4,N4-dimethyl-5,6,7,8-tetrahydroquinazoline-2,4-diamine | 480 (M + H) | 3 |
| 1441 | 4-({[(cis-4-{[4-(dimethylamino)-5,6,7,8-tetrahydroquinazolin-2-yl] amino}cyclohexyl)methyl]amino}methyl)-2-fluoro-6-methoxyphenol | 458 (M + H) | 2 |
| 1442 | N2-(cis-4-{[(2-fluoro-4,5-dimethoxybenzyl)amino]methyl}-cyclohexyl)-N4,N4-dimethyl-5,6,7,8-tetrahydroquinazoline-2,4-diamine | 472 (M + H) | 3 |
| 1443 | N2-(cis-4-{[(2-ethylbenzyl)amino]methyl}cyclohexyl) -N4,N4-dimethyl-5,6,7,8-tetrahydroquinazoline-2,4-diamine | 422 (M + H) | 3 |
| 1444 | 3-[[4-({[(cis-4-{[4-(dimethylamino)-5,6,7,8-tetrahydroquinazolin-2-yl]amino}cyclohexyl)methyl]amino}methyl)phenyl](methyl) amino]propanenitrile | 476 (M + H) | 3 |
| 1445 | N2-{cis-4-[({4-[(4-bromobenzyl)oxy]benzyl}amino)methyl]-cyclohexyl}-N4,N4-dimethyl-5,6,7,8-tetrahydroquinazoline-2,4-diamine | 578 (M + H) | 3 |

(continued)

| Ex. No. | compound name | MS | class |
|---|---|---|---|
| 1446 | N2-(cis-4-{[(3,5-dibromo-2-ethoxybenzyl)amino]methyl}-cyclohexyl)-N4,N4-dimethyl-5,6,7,8-tetrahydroquinazoline-2,4-diamine | 594 (M + H) | 3 |
| 1447 | N2-(cis-4-{[2-(4-bromophenyl)ethyl]amino}cyclohexyl) -N4,N4-dimethylquinoline-2,4-diamine | 467 (M + H) | 3 |
| 1448 | N2-(cis-4-{[2-(3-chlorophenyl)ethyl]amino}cyclohexyl) -N4,N4-dimethylquinoline-2,4-diamine | 423 (M + H) | - |
| 1449 | N2-(cis-4-{[2-(2-chlorophenoxy)ethyl]amino}cyclohexyl) -N4,N4-dimethylquinoline-2,4-diamine | 439 (M + H) | 3 |
| 1450 | N2-(cis-4-[(2-methoxy-2-phenylethyl)amino]cyclohexyl} -N4,N4-dimethylquinoline-2,4-diamine | 419 (M + H) | 3 |
| 1451 | N4,N4-Dimethyl-N2-[4-(pentamethylphenylmethyl-amino)-cyclohexyl]quinoline-2,4-diamine | 445 (M + H) | 3 |
| 1452 | N2-{cis-4-[(3-ethoxybenzyl)amino]cyclohexyl} -N4,N4-dimethylquinoline-2,4-diamine | 419 (M + H) | 3 |
| 1453 | N2-(cis-4-{[(2S)-2,3-bis(benzyloxy)propyl]amino}cyclohexyl) -N4,N4-dimethylquinoline-2,4-diamine | 539 (M + H) | 3 |
| 1454 | N2-(cis-4-{[(3-methoxy-2-naphthyl)methyl]amino}cyclohexyl) -N4,N4-dimethylquinoline-2,4-diamine | 455 (M + H) | 3 |
| 1455 | 3-[{2-[(cis-4-{[4-(dimethylamino)quinolin-2-yl]amino}cyclohexyl) amino]ethyl}(phenyl)amino]propanenitrile | 457 (M + H) | 2 |
| 1456 | N-{(1S)-1-benzyl-2-[(cis-4-{[4-(dimethylamino)quinolin-2-yl] amino}cyclohexyl)amino]ethyl}-4-methylbenzenesulfonamide | 572 (M + H) | 3 |
| 1457 | (2-{[4-(4-Dimethylamino-quinolin-2-ylamino)-cyclohexylamino] methyl}-cyclohexyl)-phenyl-methanol | 487 (M + H) | 3 |
| 1458 | N2-(cis-4-{[2-(3,5-dimethoxyphenyl)ethyl]amino}cyclohexyl) -N4,N4-dimethylquinoline-2,4-diamine | 449 (M + H) | 3 |
| 1459 | N4,N4-dimethyl-N2-(cis-4-{[2-(2-phenyl-1H-indol-3-yl)ethyl] amino}cyclohexyl)quinoline-2,4-diamine | 504 (M + H) | 2 |
| 1460 | N2-(cis-4-{[2,2-bis(4-chlorophenyl)ethyl]amino}cyclohexyl) -N4,N4-dimethylquinoline-2,4-diamine | 533 (M + H) | 3 |
| 1461 | (3-{(1S)-2-[(cis-4-{[4-(dimethylamino)quinolin-2-yl]amino} cyclohexyl)amino]-1-methylethyl}phenyl)-(phenyl)methanol | 509 (M + H) | 3 |
| 1462 | N2-[cis-4-({[1-(diphenylmethyl)azetidin-3-yl]methyl}amino)-cyclohexyl]-N4,N4-dimethylquinoline-2,4-diamine | 520 (M + H) | 1 |
| 1463 | N2-[cis-4-({[2-(4-bromophenyl)ethyl]amino}methyl)cyclohexyl] -N4,N4-dimethylquinoline-2,4-diamine | 481 (M + H) | 3 |
| 1464 | N2-[cis-4-({[4-(4-methoxyphenyl)butyl]amino}methyl)-cyclohexyl] -N4,N4-dimethylquinoline-2,4-diamine | 461 (M + H) | 3 |
| 1465 | N4,N4-dimethyl-N2-(cis-4-{[(6-phenylhexyl)amino]methyl} cyclohexyl)quinoline-2,4-diamine | 459 (M + H) | 3 |
| 1466 | N2-(cis-4-{[(2-mesitylethyl)amino]methyl}cyclohexyl) -N4,N4-dimethylquinoline-2,4-diamine | 445 (M + H) | 3 |
| 1467 | N4,N4-dimethyl-N2-(cis-4-{[(8-phenyloctyl)amino]methyl} cyclohexyl)quinoline-2,4-diamine | 487(M + H) | 3 |

(continued)

| Ex. No. | compound name | MS | class |
|---|---|---|---|
| 1468 | N2-[cis-4-({[2-(4-tert-butylphenyl)ethyl]amino}methyl)-cyclohexyl] -N4,N4-dimethylquinoline-2,4-diamine | 459 (M + H) | 3 |
| 1469 | N4,N4-dimethyl-N2-(cis-4-{[(5-phenylpent-4-yn-1-yl)amino} methyl}cyclohexyl)quinoline-2,4-diamine | 441 (M + H) | 3 |
| 1470 | N2-[cis-4-({[2-(2-methoxyphenyl)ethyl]amino)methyl)-cyclohexyl] -N4,N4-dimethylquinoline-2,4-diamine | 433 (M + H) | 3 |
| 1471 | N4,N4-dimethyl-N2-(cis-4-{[(3-phenoxypropyl)amino]methyl} cyclohexyl)quinoline-2,4-diamine | 433 (M + H) | 3 |
| 1472 | N4,N4-dimethyl-N2-(cis-4-{[(2,3,5,6-tetrafluorobenzyl)amino] methyl}cyclohexyl)quinoline-2,4-diamine | 461 (M + H) | 3 |
| 1473 | N2-(cis-4-{[(2,5-dichlorobenzyl)amino]methyl}cyclohexyl) -N4,N4-dimethylquinoline-2,4-diamine | 457 (M + H) | 3 |
| 474 | N2-(cis-4-{[(5-chloro-2-methoxybenzyl)amino]methyl}-cyclohexyl) -N4,N4-dimethylquinoline-2,4-diamine | 453 (M + H) | 3 |
| 1475 | N2-(cis-4-{[(4-chloro-2-methoxybenzyl)amino]methyl}-cyclohexyl) -N4,N4-dimethylquinoline-2,4-diamine | 453 (M + H) | 3 |
| 1476 | N2-(cis-4-{[(3-iodo-4-methylbenzyl)amino]methyl}cyclohexyl) -N4,N4-dimethylquinoline-2,4-diamine | 529 (M + H) | 3 |
| 1477 | N2-[cis-4-({[(2S)-2-(dibenzylamino)propyl]amino}methyl)- cyclohexyl]-N4,N4-dimethylquinoline-2,4-diamine | 536 (M + H) | 3 |
| 1478 | N4,N4-dimethyl-N2-[cis-4-({[(1-phenyl-5-propyl-1H-pyrazol-4-yl) methyl]amino}methyl)cyclohexyl]quinoline-2,4-diamine | 497 (M + H) | 1 |
| 1479 | N2-{cis-4-[({[1-(4-chlorophenyl)-5-propyl-1H-pyrazol-4-yl]methyl} amino)methyl]cyclohexyl}-N4,N4-dimethylquinoline-2,4-diamine | 531 (M + H) | 1 |
| 1480 | N4,N4-dimethyl-N2-[cis-4-({[4-(4-nitrophenyl)butyl]-amino} methyl)cyclohexyl]quinoline-2,4-diamine | 476 (M + H) | 3 |
| 1481 | N2-(cis-4-{[2-(4-bromophenyl)ethyl]amino}cyclohexyl) -N4,N4-dimethyl-5,6,7,8-tetrahydroquinazoline-2,4-diamine | 472 (M + H) | 3 |
| 1482 | N2-(cis-4-{[2-(3-chlorophenyl)ethyl]amino}cyclohexyl) -N4,N4-dimethyl-5,6,7,8-tetrahydroquinazoline-2,4-diamine | 428 (M + H) | 3 |
| 1483 | N2-{cis-4-[(2-methoxy-2-phenylethyl)amino]cyclohexyl} -N4,N4-dimethyl-5,6,7,8-tetrahydroquinazoline-2,4-diamine | 424 (M + H) | 3 |
| 1484 | N2-[4-(2-Methoxy-2-phenyl-ethylamino)-cyclohexyl] -N4,N4-dimethyl-5,6,7,8-tetrahydro-quinazoline-2,4-diamine | 424 (M + H) | 3 |
| 1485 | N4,N4-Dimethyl-N2-[4-(pentamethylphenylmethyl-amino)- cyclohexyl]-5,6,7,8-tetrahydro-quinazoline-2,4-diamine | 450 (M + H) | 2 |
| 1486 | N2-{cis-4-[(3-ethoxybenzyl)amino]cyclohexyl}-N4,N4-dimethyl- 5,6,7,8-tetrahydroquinazoline-2,4-diamine | 424 (M + H) | 3 |
| 1487 | N2-(cis-4-{[(2S)-2,3-bis(benzyloxy)propyl]amino}cyclohexyl) -N4,N4-dimethyl-5,6,7,8-tetrahydroquinazoline-2,4-diamine | 544 (M + H) | 3 |
| 1488 | N2-(cis-4-{[(3-methoxy-2-naphthyl)methyl]amino}cyclohexyl) -N4,N4-dimethyl-5,6,7,8-tetrahydroquinazoline-2,4-diamine | 460 (M + H) | 3 |
| 1489 | 3-[{2-[(cis-4-{[4-(dimethylamino)-5,6,7,8-tetrahydroquinazolin- 2-yl]amino}cyclohexyl)amino]ethyl}(3-methylphenyl)-amino] propanenitrile | 476 (M + H) | 2 |

(continued)

| Ex. No. | compound name | MS | class |
|---|---|---|---|
| 1490 | 3-[{2-[(cis-4-{[4-(dimethylamino)-5,6,7,8-tetrahydroquinazolin-2-yl]amino}cyclohexyl)amino]ethyl}(phenyl)amino]-propanenitrile | 462 (M + H) | 1 |
| 1491 | N-{(1S)-1-benzyl-2-[(cis-4-{[4-(dimethylamino)-5,6,7,8-tetrahydroquinazolin-2-yl]amino}cyclohexyl)amino]ethyl}-4-methylbenzenesulfonamide | 577 (M + H) | 1 |
| 1492 | (2-{[4-(4-Dimethylamino-5,6,7,8-tetrahydro-quinazolin-2-ylamino)-cyclohexylamino]-methyl}-cyclohexyl)-phenyl-methanol | 490 (M + H) | 3 |
| 1493 | N2-(cis-4-{[2-(3,5-dimethoxyphenyl)ethyl]amino}cyclohexyl)-N4,N4-dimethyl-5,6,7,8-tetrahydroquinazoline-2,4-diamine | 454 (M + H) | 2 |
| 1494 | N4,N4-dimethyl-N2-(cis-4-{[2-(2-phenyl-1H-indol-3-yl)ethyl]amino}cyclohexyl)-5,6,7,8-tetrahydroquinazoline-2,4-diamine | 509 (M + H) | 3 |
| 1495 | N2-(cis-4-{[2,2-bis(4-chlorophenyl)ethyl]amino}cyclohexyl)-N4,N4-dimethyl-5,6,7,8-tetrahydroquinazoline-2,4-diamine | 538 (M + H) | 3 |
| 1496 | (3-{(1S)-2-[(cis-4-{[4-(dimethylamino)-5,6,7,8-tetrahydroquinazolin-2-yl]amino}cyclohexyl)amino]-1-methylethyl }phenyl)(phenyl)methanol | 512 (M + H) | 3 |
| 1497 | N2-[cis-4-({[1-(diphenylmethyl)azetidin-3-yl]methyl}amino)-cyclohexyl]-N4,N4-dimethyl-5,6,7,8-tetrahydroquinazoline-2,4-diamine | 525 (M + H) | 1 |
| 1498 | N2-[cis-4-({[2-(4-bromophenyl)ethyl]amino}methyl)cyclohexyl]-N4,N4-dimethyl-5,6,7,8-tetrahydroquinazoline-2,4-diamine | 486 (M + H) | 3 |
| 1499 | N2-[cis-4-({[4-(4-methoxyphenyl)butyl]amino}methyl)-cyclohexyl]-N4,N4-dimethyl-5,6,7,8-tetrahydroquinazoline-2,4-diamine | 466 (M + H) | 3 |
| 1500 | N4,N4-dimethyl-N2-(cis-4-{[(6-phenylhexyl)amino]methyl}-cyclohexyl)-5,6,7,8-tetrahydroquinazoline-2,4-diamine | 464 (M + H) | 3 |
| 1501 | N2-(cis-4-{[(2-mesitylethyl)amino]methyl}cyclohexyl)-N4,N4-dimethyl-5,6,7,8-tetrahydroquinazoline-2,4-diamine | 450 (M + H) | 3 |
| 1502 | N4,N4-dimethyl-N2-(cis-4-{[(8-phenyloctyl)amino]methyl}-cyclohexyl)-5,6,7,8-tetrahydroquinazoline-2,4-diamine | 492 (M + H) | 3 |
| 1503 | N2-[cis-4-({[2-(4-tert-butylphenyl)ethyl]amino}methyl)-cyclohexyl]-N4,N4-dimethyl-5,6,7,8-tetrahydroquinazoline-2,4-diamine | 464 (M + H) | 3 |
| 1504 | N2-[cis-4-({[2-(2-methoxyphenyl)ethyl]amino}methyl)-cyclohexyl]-N4,N4-dimethyl-5,6,7,8-tetrahydroquinazoline-2,4-diamine | 438 (M + H) | 3 |
| 1505 | N4,N4-dimethyl-N2-(cis-4-{[(3-phenoxypropyl)amino]-methyl}cyclohexyl)-5,6,7,8-tetrahydroquinazoline-2,4-diamine | 438 (M + H) | 3 |
| 1506 | N2-(cis-4-{[(5-chloro-2-methoxybenzyl)amino]methyl}-cyclohexyl)-N4,N4-dimethyl-5,6,7,8-tetrahydroquinazoline-2,4-diamine | 458 (M + H) | 3 |
| 1507 | N2-(cis-4-{[(4-chloro-2-methoxybenzyl)amino]methyl}-cyclohexyl)-N4,N4-dimethyl-5,6,7,8-tetrahydroquinazoline-2,4-diamine | 458 (M + H) | 3 |
| 1508 | N2-(cis-4-{[(3-iodo-4-methylbenzyl)amino]methyl}cyclohexyl)-N4,N4-dimethyl-5,6,7,8-tetrahydroquinazoline-2,4-diamine | 534 (M + H) | 3 |
| 1509 | N2-[cis-4-({[(2S)-2-(dibenzylamino)propyl]amino}methyl)-cyclohexyl]-N4,N4-dimethyl-5,6,7,8-tetrahydroquinazoline-2,4-diamine | 541 (M + H) | 3 |

(continued)

| Ex. No. | compound name | MS | class |
|---|---|---|---|
| 1510 | N4,N4-dimethyl-N2-[cis-4-({[(1-phenyl-5-propyl-1H-pyrazol-4-yl) methyl]amino}methyl)cyclohexyl]-5,6,7,8-tetrahydroquinazoline-2,4-diamine | 502 (M + H) | 1 |
| 1511 | N2-{cis-4-[({[1-(4-chlorophenyl)-5-propyl-1H-pyrazol-4-yl]methyl} amino)methyl]cyclohexyl}-N4,N4-dimethyl-5,6,7,8-tetrahydroquinazoline-2,4-diamine | 536 (M + H) | 1 |
| 1512 | N4,N4-dimethyl-N2-[cis-4-({[4-(4-nitrophenyl)butyl]amino}-methyl)cyclohexyl]-5,6,7,8-tetrahydroquinazoline-2,4-diamine | 481 (M + H) | 3 |
| 1513 | N2-(cis-4-{[2-(4-bromophenyl)ethyl]amino}cyclohexyl) -N4,N4-dimethylpyrimidine-2,4-diamine | 418 (M + H) | 3 |
| 1514 | N2-(cis-4-{[2-(3-chlorophenyl)ethyl]amino}cyclohexyl) -N4,N4-(dimethylpyrimidine-2,4-diamine | 374 M + H) | 3 |
| 1515 | N2-(cis-4-{[2-(2-chlorophenoxy)ethyl]amino}cyclohexyl) -N4,N4-dimethylpyrimidine-2,4-diamine | 390 (M + H) | 3 |
| 1516 | N2-{cis-4-[(2-methoxy-2-phenylethyl)amino]cyclohexyl} -N4,N4-dimethylpyrimidine-2,4-diamine | 370 (M + H) | 3 |
| 1517 | N2-[4-(2-Methoxy-2-phenyl-ethylamino)-cyclohexyl] -N4,N4-dimethyl-pyrimidine-2,4-diamine | 370 (M + H) | 3 |
| 1518 | N2-(cis-4-{[2-(4-bromophenoxy)ethyl]amino}cyclohexyl) -N4,N4-dimethylpyrimidine-2,4-diamine | 434 (M + H) | 3 |
| 1519 | N4,N4-Dimethyl-N2-[4-(pentamethylphenylmethyl-amino)-cyclohexyl]-pyrimidine-2,4-diamine | 396 (M + H) | 3 |
| 1520 | N2-{cis-4-[(3-ethoxybenzyl)amino]cyclohexyl} -N4,N4-dimethylpyrimidine-2,4-diamine | 370 (M + H) | 3 |
| 1521 | N2-(cis-4-{[(2S)-2,3-bis(benzyloxy)propyl]amino}cyclohexyl) -N4,N4-dimethylpyrimidine-2,4-diamine | 490 (M + H) | 3 |
| 1522 | N2-(cis-4-{[(3-methoxy-2-naphthyl)methyl]amino}cyclohexyl) -N4,N4-dimethylpyrimidine-2,4-diamine | 406 (M + H) | 3 |
| 1523 | 3-[{2-[(cis-4-{[4-(dimethylamino)pyrimidin-2-yl]amino}cyclohexyl) amino]ethyl}(3-methylphenyl)-amino]propanenitrile | 422 (M + H) | 3 |
| 1524 | 3-[{2-[(cis-4-{[4-(dimethylamino)pyrimidin-2-yl]amino}cyclohexyl) amino]ethyl}(phenyl)amino]propanenitrile | 408 (M + H) | 3 |
| 1525 | N2-[cis-4-({[4-(4-methoxyphenyl)butyl]amino}methyl)-cyclohexyl] -N4,N4-dimethylpyrimidine-2,4-diamine | 412 (M + H) | 3 |
| 1526 | N4,N4-dimethyl-N2-(cis-4-{[(6-phenylhexyl)amino]methyl} cyclohexyl)pyrimidine-2,4-diamine | 410 (M + H) | 3 |
| 1527 | N2-(cis-4-{[(2-mesitylethyl)amino]methyl}cyclohexyl) -N4,N4-dimethylpyrimidine-2,4-diamine | 396 (M + H) | 3 |
| 1528 | N4,N4-dimethyl-N2-(cis-4-{[(8-phenyloctyl)amino]methyl} cyclohexyl)pyrimidine-2,4-diamine | 438 (M + H) | 3 |
| 1529 | N2-[cis-4-({[2-(4-tert-butylphenyl)ethyl]amino}methyl)-cyclohexyl] -N4,N4-dimethylpyrimidine-2,4-diamine | 410 (M + H) | 3 |
| 1530 | N4,N4-dimethyl-N2-(cis-4-{[(5-phenylpent-4-yn-1-yl)amino] methyl }cyclohexyl)pyrimidine-2,4-diamine | 392 (M + H) | 3 |

(continued)

| Ex. No. | compound name | MS | class |
|---|---|---|---|
| 1531 | N2-[cis-4-({[2-(2-methoxyphenyl)ethyl]amino}methyl)-cyclohexyl]-N4,N4-dimethylpyrimidine-2,4-diamine | 384 (M + H) | 3 |
| 1532 | N4,N4-dimethyl-N2-(cis-4-{[(3-phenoxypropyl)amino]-methyl}cyclohexyl)pyrimidine-2,4-diamine | 384 (M + H) | 3 |
| 1533 | N4,N4-dimethyl-N2-(cis-4-{[(2,3,5,6-tetrafluorobenzyl)amino]-methyl}cyclohexyl)pyrimidine-2,4-diamine | 412 (M + H) | 3 |
| 1534 | N2-(cis-4-{[(2,5-dichlorobenzyl)amino]methyl}cyclohexyl)-N4,N4-dimethylpyrimidine-2,4-diamine | 408 (M + H) | 3 |
| 1535 | N2-(cis-4-{[(5-chloro-2-methoxybenzyl)amino]methyl}-cyclohexyl)-N4,N4-dimethylpyrimidine-2,4-diamine | 404 (M + H) | 3 |
| 1536 | N2-(cis-4-{[(4-chloro-2-methoxybenzyl)amino]methyl}-cyclohexyl)-N4,N4-dimethylpyrimidine-2,4-diamine | 404 (M + H) | 3 |
| 1537 | N2-(cis-4-{[(3-iodo-4-methylbenzyl)amino]methyl}cyclohexyl)-N4,N4-dimethylpyrimidine-2,4-diamine | 480 (M + H) | 3 |
| 1538 | N2-[cis-4-({[(2S)-2-(dibenzylamino)propyl]amino}methyl)-cyclohexyl]-N4,N4-dimethylpyrimidine-2,4-diamine | 487 (M + H) | 3 |
| 1539 | 2-(benzyloxy)ethyl(cis-4-{[4-(dimethylamino)quinolin-2-yl]amino}cyclohexyl)carbamate | 463 (M + H) | 3 |
| 1540 | 2,2-dimethylpropyl(cis-4-{[4-(dimethylamino)quinolin-2-yl]amino)}cyclohexyl)carbamate | 399 (M + H) | 3 |
| 1541 | "[4-(4-Dimethylamino-quinolin-2-ylamino)-cyclohexyl]-carbamic acid 4,5-dimethoxy-2-nitro-benzyl ester | 524 (M + H) | 2 |
| 1542 | 3-(trifluoromethyl)phenyl(cis-4-{[4-(dimethylamino)quinolin-2-yl]amino}cyclohexyl)carbamate | 473 (M + H) | 3 |
| 1543 | 4-bromophenyl(cis-4-{[4-(dimethylamino)quinolin-2-yl]amino}cyclohexyl)carbamate | 483 (M + H) | 3 |
| 1544 | 2-methoxyphenyl(cis-4-{[4-(dimethylamino)quinolin-2-yl]amino}cyclohexyl)carbamate | 435 (M + H) | 3 |
| 1545 | 2-methoxyethyl(cis-4-{[4-(dimethylamino)quinolin-2-yl]amino}cyclohexyl)carbamate | 387 (M + H) | 3 |
| 1546 | octyl(cis-4-{[4-(dimethylamino)quinolin-2-yl]amino}cyclohexyl)carbamate | 441 (M + H) | 3 |
| 1547 | ethyl (cis-4-{[4-(dimethylamino)quinolin-2-yl]amino}cyclohexyl)carbamate | 357 (M + H) | 3 |
| 1548 | [4-(4-Dimethylamino-quinolin-2-ylamino)-cyclohexyl]-carbamic acid 4-nitro-benzyl ester | 464 (M + H) | 3 |
| 1549 | 2-naphthyl(cis-4-{[4-(dimethylamino)quinolin-2-yl]amino}cyclohexyl)carbamate | 455 (M + H) | 3 |
| 1550 | allyl (cis-4-{[4-(dimethylamino)quinolin-2-yl]amino}cyclohexyl)carbamate | 369 (M + H) | 3 |
| 1551 | [4-(4-Dimethylamino-quinolin-2-ylamino)-cyclohexyl]-carbamicacid benzyl ester | 419 (M + H) | 3 |
| 1552 | phenyl (cis-4-{[4-(dimethylamino)quinolin-2-yl]amino}cyclohexyl)carbamate | 405 (M + H) | 3 |

(continued)

| Ex. No. | compound name | MS | class |
|---|---|---|---|
| 1553 | (1R,2S,5R)-2-isopropyl-5-methylcyclohexyl (cis-4-{ [4-(dimethylamino)quinolin-2-yl]amino)}cyclohexyl)carbamate | 467 (M + H) | 3 |
| 1554 | 4-methylphenyl(cis-4-{[4-(dimethylamino)quinolin-2-yl]amino} cyclohexyl)carbamate | 419 (M + H) | 3 |
| 1555 | methyl(cis-4-{[4-(dimethylamino)quinolin-2-yl]amino}cyclohexyl) carbamate | 343 (M + H) | 3 |
| 1556 | 2-chlorobenzyl(cis-4-{[4-(dimethylamino)quinolin-2-yl]amino} cyclohexyl)carbamate | 453 (M + H) | 3 |
| 1557 | 9H-fluoren-9-ylmethyl(cis-4-{[4-(dimethylamino)quinolin-2-yl] amino}cyclohexyl)carbamate | 507 (M + H) | 3 |
| 1558 | 2,2,2-trichloroethyl(cis-4-{[4-(dimethylamino)quinolin-2-yl]amino} cyclohexyl)carbamate | 459 (M + H) | 3 |
| 1559 | 2-(benzyloxy)ethyl[(cis-4-{[4-(dimethylamino)quinolin-2-yl]amino} cyclohexyl)methyl]carbamate | 477 (M +H) | 3 |
| 1560 | 2,2-dimethylpropyl[(cis-4-{(4-(dimethylamino)quinolin-2-yl)amino} cyclohexyl)methyl]carbamate | 413 (M + H) | 3 |
| 1561 | 4,5-dimethoxy-2-nitrobenzyl[(cis-4-{[4-(dimethylamino)-quinolin-2-yl]amino}cyclohexyl)methyl]carbamate | 538 (M + H) | 3 |
| 1562 | 3-(trifluoromethyl)phenyl[(cis-4-{[4-(dimethylamino)quinolin-2-yl] amino}cyclohexyl)methyl]carbamate | 487 (M + H) | 3 |
| 1563 | 4-bromophenyl((cis-4-{[4-(dimethylamino)quinolin-2-yl]amino} cyclohexyl)methyl]carbamate | 497 (M + H) | 3 |
| 1564 | 2-methoxyphenyl((cis-4-{[4-(dimethylamino)quinolin-2-yl]amino} cyclohexyl)methyl]carbamate | 449 (M + H) | 3 |
| 1565 | 2-methoxyethyl[(cis-4-{[4-(dimethylamino)quinolin-2-yl]amino} cyclohexyl}methyl]carbamate | 401 (M + H) | 3 |
| 1566 | octyl [(cis-4-(4-{[dimethylamino)quinolin-2-yl]amino}cyclohexyl) methyl]carbamate | 455 (M + H) | 3 |
| 1567 | ethyl [(cis-4-{[4-(dimethylamino)quinolin-2-yl]amino}cyclohexyl) methyl]carbamate | 371 (M + H) | 3 |
| 1568 | 4-nitrobenzyl[(cis-4-{[4-(dimethylamino)quinolin-2-yl]amino} cyclohexyl)methyl]carbamate | 478 (M + H) | 3 |
| 1569 | 2-naphthyl[(cis-4-{[4-(dimethylamino)quinolin-2-yl]amino} cyclohexyl)methyl]carbamate | 469 (M + H) | 3 |
| 1570 | allyl [(cis-4-{[4-(dimethylamino)quinolin-2-yl]amino}cyclohexyl) methyl]carbamate | 383 (M + H) | 3 |
| 1571 | benzyl [(cis-4-{[4-(dimethylamino)quinolin-2-yl]amino}cyclohexyl) methyl]carbamate | 433 (M + H) | 3 |
| 1572 | phenyl [(cis-4-{[4-(dimethylamino)quinolin-2-yl]amino}cyclohexyl) methyl]carbamate | 419 (M + H) | 3 |
| 1573 | (1R,2S,5R)-2-isopropyl-5-methylcyclohexyl [(cis-4-{ [4-(dimethylamino)quinolin-2-yl]amino}cyclohexyl)methyl]-carbamate | 481 (M + H) | 3 |
| 1574 | 4-methylphenyl[(cis-4-{(4-(dimethylamino)quinolin-2-yl]amino} cyclohexyl)methyl]carbamate | 433 (M + H) | 3 |

(continued)

| Ex. No. | compound name | MS | class |
|---|---|---|---|
| 1575 | methyl [(cis-4-{(4-(dimethylamino)quinolin-2-yl]amino}cyclohexyl) methyl]carbamate | 357 (M + H) | 3 |
| 1576 | 2-chlorobenzyl[(cis-4-{[4-(dimethylamino)quinolin-2-yl]amino} cyclohexyl)methyl]carbamate | 467 (M + H) | 3 |
| 1577 | 9H-fluoren-9-ylmethyl[(cis-4-{[4-(dimethylamino)quinolin-2-yl] amino}cyclohexyl)methyl]carbamate | 521 (M + H) | 3 |
| 1578 | 2,2,2-trichloroethyl[(cis-4-{[4-(dimethylamino)quinolin-2-yl]amino} cyclohexyl)methyl]carbamate | 473 (M + H) | 3 |
| 1579 | 2-(benzyloxy)ethyl(cis-4-{[4-(dimethylamino)-5,6,7,8-tetrahydroquinazolin-2-yl]amino}cyclohexyl)carbamate | 468 (M + H) | 3 |
| 1580 | 2,2-dimethylpropyl(cis-4-{[4-(dimethylamino)-5,6,7,8-tetrahydroquinazolin-2-yl]amino)cyclohexyl)carbamate | 404 (M + H) | 3 |
| 1581 | [4-(4-Dimethylamino-5,6,7,8-tetrahydro-quinazolin-2-ylamino)-cyclohexyl]-carbamic acid 4,5-dimethoxy-2-nitro-benzyl ester | 529 (M + H) | 2 |
| 1582 | 3-(trifluoromethyl)phenyl(cis-4-{[4-(dimethylamino)-5,6,7,8-tetrahydroquinazolin-2-yl]amino}cyclohexyl)carbamate | 478 (M + H) | 3 |
| 1583 | 4-bromophenyl(cis-4-{[4-(dimethylamino)-5,6,7,8-tetrahydroquinazolin-2-yl]amino}cyclohexyl)carbamate | 488 (M + H) | 3 |
| 1584 | 2-methoxyphenyl(cis-4-{[4-(dimethylamino)-5,6,7,8-tetrahydroquinazolin-2-yl]amino}cyclohexyl)carbamate | 440 (M + H) | 3 |
| 1585 | 2-methoxyethyl(cis-4-{[4-(dimethylamino)-5,6,7,8-tetrahydroquinazolin-2-yl]amino}cyclohexyl)carbamate | 392 (M + H) | 3 |
| 1586 | octyl (cis-4-{[4-(dimethylamino)-5,6,7,8-tetrahydroquinazolin-2-yl] amino}cyclohexyl)carbamate | 446 (M + H) | 3 |
| 1587 | ethyl (cis-4-{[4-(dimethylamino)-5,6,7,8-tetrahydroquinazolin-2-yl] amino}cyclohexyl)carbamate | 362 (M + H) | 3 |
| 1588 | 4-nitrobenzyl(cis-4-{[4-(dimethylamino)-5,6,7,8-tetrahydroquinazolin-2-yl]amino}cyclohexyl)carbamate | 469 (M + H) | 3 |
| 1589 | 2-naphthyl(cis-4-{[4-(dimethylamino)-5,6,7,8-tetrahydroquinazolin-2-yl]amino}cyclohexyl)carbamate | 460 (M + H) | 3 |
| 1590 | allyl (cis-4-{[4-(dimethylamino)-5,6,7,8-tetrahydroquinazolin-2-yl] amino}cyclohexyl)carbamate | 374 (M + H) | 3 |
| 1591 | benzyl (cis-4-{[4-(dimethylamino)-5,6,7,8-tetrahydroquinazolin-2-yl]amino}cyclohexyl)carbamate | 424 (M + H) | 3 |
| 1592 | phenyl (cis-4-{[4-(dimethylamino)-5,6,7,8-tetrahydroquinazolin-2-yl]amino}cyclohexyl)carbamate | 410 (M + H) | 3 |
| 1593 | (2S,5R)-2-isopropyl-5-methylcyclohexyl (cis-4-{ [4-(dimethylamino)-5,6,7,8-tetrahydroquinazolin-2-yl]amino} cyclohexyl)carbamate | 472 (M + H) | 3 |
| 1594 | 4-methylphenyl (cis-4-{[4-(dimethylamino)-5,6,7,8-tetrahydroquinazolin-2-yl]amino}cyclohexyl)carbamate | 424 (M + H) | 3 |
| 1595 | methyl (cis-4-{[4-(dimethylamino)-5,6,7,8-tetrahydroquinazolin-2-yl]amino}cyclohexyl)carbamate | 348 (M + H) | 3 |
| 1596 | 2-chlorobenzyl (cis-4-{[4-(dimethylamino)-5,6,7,8-tetrahydroquinazolin-2-yl]amino}cyclohexyl)carbamate | 458 (M + H) | 3 |

(continued)

| Ex. No. | compound name | MS | class |
|---|---|---|---|
| 1597 | 9H-fluoren-9-ylmethyl(cis-4-{[4-(dimethylamino)-5,6,7,8-tetrahydroquinazolin-2-yl]amino}cyclohexyl)carbamate | 512 (M + H) | 3 |
| 1598 | 2,2,2-trichloroethyl(cis-4-{[4-(dimethylamino)-5,6,7,8-tetrahydroquinazolin-2-yl]amino}cyclohexyl)carbamate | 464 (M + H) | 3 |
| 1599 | 2-(benzyloxy)ethyl [(cis-4-{[4-(dimethylamino)-5,6,7,8-tetrahydroquinazolin-2-yl]amino}cyclohexyl)methyl] carbamate | 482 (M + H) | 3 |
| 1600 | 2,2-dimethylpropyl[(cis-4-{[4-(dimethylamino)-5,6,7,8-tetrahydroquinazolin-2-yl]amino}cyclohexyl)methyl] carbamate | 418 (M + H) | 3 |
| 1601 | 4,5-dimethoxy-2-nitrobenzyl[(cis-4-{[4-(dimethylamino)-5,6,7,8-tetrahydroquinazolin-2-yl]amino}cyclohexyl)methyl] carbamate | 543 (M + H) | 3 |
| 1602 | 3-(trifluoromethyl)phenyl[(cis-4-{[4-(dimethylamino)-5,6,7,8-tetrahydroquinazolin-2-yl]amino}cyclohexyl)methyl] carbamate | 492 (M + H) | 3 |
| 1603 | 4-bromophenyl[(cis-4-{[4-(dimethylamino)-5,6,7,8-tetrahydroquinazolin-2-yl]amino}cyclohexyl)methyl] carbamate | 502 (M + H) | 3 |
| 1604 | 2-methoxyphenyl[(cis-4-{[4-(dimethylamino)-5,6,7,8-tetrahydroquinazolin-2-yl]amino}cyclohexyl)methyl] carbamate | 454 (M + H) | 3 |
| 1605 | 2-methoxyethyl[(cis-4-{[4-(dimethylamino)-5,6,7,8-tetrahydroquinazolin-2-yl]amino}cyclohexyl)methyl] carbamate | 406 (M + H) | 3 |
| 1606 | octyl [(cis-4-{[4-(dimethylamino)-5,6,7,8-tetrahydroquinazolin-2-yl]amino}cyclohexyl)methyl]carbamate | 460 (M + H) | 3 |
| 1607 | ethyl [(cis-4-{[4-(dimethylamino)-5,6,7,8-tetrahydroquinazolin-2-yl]amino}cyclohexyl)methyl]carbamate | 376 (M + H) | 3 |
| 1608 | [4-(4-Dimethylamino-5,6,7,8-tetrahydro-quinazolin-2-ylamino)-cyclohexylmethyl]-carbamic acid 4-nitro-benzyl ester | 483 (M + H) | 3 |
| 1609 | 2-naphthyl[(cis-4-{[4-(dimethylamino)-5,6,7,8-tetrahydroquinazolin-2-yl]amino}cyclohexyl)methyl] carbamate | 474 (M + H) | 3 |
| 1610 | allyl [(cis-4-{[4-(dimethylamino)-5,6,7,8-tetrahydroquinazolin-2-yl] amino}cyclohexyl)methyl]carbamate | 388 (M + H) | 3 |
| 1611 | [4-(4-Dimethylamino-5,6,7,8-tetrahydro-quinazolin-2-ylamino)-cyclohexylmethyl]-carbamic acid benzyl ester | 438 (M + H) | 3 |
| 1612 | phenyl [(cis-4-{[4-(dimethylamino)-5,6,7,8-tetrahydroquinazolin-2-yl]amino}cyclohexyl)methyl]carbamate | 424 (M + H) | 3 |
| 1613 | (2S,5R)-2-isopropyl-5-methylcyclohexyl[(cis-4-{ [4-(dimethylamino)-5,6,7,8-tetrahydroquinazolin-2-yl]amino} cyclohexyl)methyl]carbamate | 486 (M + H) | 3 |
| 1614 | 4-methylphenyl[(cis-4-{[4-(dimethylamino)-5,6,7,8-tetrahydroquinazolin-2-yl]amino}cyclohexyl)methyl] carbamate | 438 (M + H) | 3 |

(continued)

| Ex. No. | compound name | MS | class |
|---|---|---|---|
| 1615 | methyl [(cis-4-{[4-(dimethylamino)-5,6,7,8-tetrahydroquinazolin-2-yl]amino}cyclohexyl)methyl]carbamate | 362 (M + H) | 3 |
| 1616 | 2-chlorobenzyl[(cis-4-{[4-(dimethylamino)-5,6,7,8-tetrahydroquinazolin-2-yl]amino}cyclohexyl)methyl] carbamate | 472 (M + H) | 3 |
| 1617 | 9H-fluoren-9-ylmethyl[(cis-4-{[4-(dimethylamino)-5,6,7,8-tetrahydroquinazolin-2-yl]amino}cyclohexyl)methyl] carbamate | 526 (M + H) | 3 |
| 1618 | 2,2,2 trichloroethyl[(cis-4-{[4-(dimethylamino)-5,6,7,8-tetrahydroquinazolin-2-yl]amino}cyclohexyl)methyl] carbamate | 478 (M + H) | 3 |
| 1619 | 2-(benzyloxy)ethyl(cis-4-{[4-(dimethylamino)pyrimidin-2-yl] amino}cyclohexyl)carbamate | 414 (M + H) | 3 |
| 1620 | 2,2-dimethylpropyl(cis-4-{[4-(dimethylamino)pyrimidin-2-yl] amino}cyclohexyl)carbamate | 350 (M + H) | 3 |
| 1621 | [4-(4-Dimethylamino-pyrimidin-2-ylamino)-cyclohexyl]-carbamic acid 4,5-dimethoxy-2-nitro-benzyl ester | 475 (M + H) | 3 |
| 1622 | 3-(trifluoromethyl)phenyl(cis-4-{[4-(dimethylamino)pyrimidin-2-yl] amino}cyclohexyl)carbamate | 424 (M + H) | 3 |
| 1623 | 4-bromophenyl(cis-4-{[4-(dimethylamino)pyrimidin-2-yl]amino} cyclohexyl)carbamate | 434 (M + H) | 3 |
| 1624 | 2-methoxyphenyl(cis-4-{[4-(dimethylamino)pyrimidin-2-yl]amino)} cyclohexyl)carbamate | 386 (M + H) | 3 |
| 1625 | 2-methoxyethyl(cis-4-{(4-(dimethylamino)pyrimidin-2-yl]amino} cyclohexyl)carbamate | 338 (M + H) | 3 |
| 1626 | octyl (cis-4-{[4-(dimethylamino)pyrimidin-2-yl]amino}cyclohexyl) carbamate | 392 (M + H) | 3 |
| 1627 | ethyl (cis-4-{[4-(dimethylamino)pyrimidin-2-yl]amino}cyclohexyl) carbamate | 308 (M + H) | 3 |
| 1628 | 4-nitrobenzyl (cis-4-{[4-(dimethylamino)pyrimidin-2-yl]amino} cyclohexyl)carbamate | 415 (M + H) | 3 |
| 1629 | 2-naphthyl(cis-4-{[4-(dimethylamino)pyrimidin-2-yl]amino} cyclohexyl)carbamate | 406 (M + H) | 3 |
| 1630 | allyl (cis-4-{[4-(dimethylamino)pyrimidin-2-yl]amino}cyclohexyl) carbamate | 320 (M + H) | 3 |
| 1631 | benzyl (cis-4-{[4-(dimethylamino)pyrimidin-2-yl]amino}cyclohexyl) carbamate | 370 (M + H) | 3 |
| 1632 | phenyl (cis-4-{[4-(dimethylamino)pyrimidin-2-yl]amino}cyclohexyl) carbamate | 356 (M + H) | 3 |
| 1633 | (2S,5R)-2-isopropyl-5-methylcyclohexyl (cis-4--{ [4-(dimethylamino)pyrimidin-2-yl]amino}cyclohexyl)carbamate | 418 (M + H) | 3 |
| 1634 | 4-methylphenyl (cis-4-{[4-(dimethylamino)pyrimidin-2-yl]amino} cyclohexyl)carbamate | 370 (M + H) | 3 |
| 1635 | methyl (cis-4-{[4-(dimethylamino)pyrimidin-2-yl]amino}cyclohexyl) carbamate | 294 (M + H) | 3 |

(continued)

| Ex. No. | compound name | MS | class |
|---|---|---|---|
| 1636 | 2-chlorobenzyl(cis-4-{(4-(dimethylamino)pyrimidin-2-yl]amino cyclohexyl)carbamate | 404 (M + H) | 3 |
| 1637 | 9H-fluoren-9-ylmethyl(cis-4-{[4-(dimethylamino)pyrimidin-2-yl] amino}cyclohexyl)carbamate | 458 (M + H) | 3 |
| 1638 | 2,2,2-trichloroethyl(cis-4-{[4-(dimethylamino)pyrimidin-2-yl] amino}cyclohexyl)carbamate | 410 (M + H) | 3 |
| 1639 | 2-(benzyloxy)ethyl[(cis-4-{[4-(dimethylamino)pyrimidin-2-yl] amino}cyclohexyl)methyl]carbamate | 428 (M + H) | 3 |
| 1640 | 2,2-dimethylpropyl[(cis-4-{[4-(dimethylamino)pyrimidin-2-yl] amino}cyclohexyl)methyl]carbamate | 364 (M + H) | 3 |
| 1641 | 4,5-dimethoxy-2-nitrobenzyl[(cis-4-{[4-(dimethylamino)-pyrimidin-2-yl]amino}cyclohexyl)methyl]carbamate | 489 (M + H) | 3 |
| 1642 | 3-(trifluoromethyl)phenyl[(cis-4-{[4-(dimethylamino)pyrimidin-2-yl] amino}cyclohexyl)methyl]carbamate | 438 (M + H) | 3 |
| 1643 | 4-bromophenyl[(cis-4-{[4-(dimethylamino)pyrimidin-2-yl]amino} cyclohexyl)methyl]carbamate | 448 (M + H) | 3 |
| 1644 | 2-methoxyphenyl[(cis-4-{[4-(dimethylamino)pyrimidin-2-yl]amino} cyclohexyl)methyl]carbamate | 400 (M + H) | 3 |
| 1645 | 2-methoxyethyl[(cis-4-{[4-(dimethylamino)pyrimidin-2-yl]amino} cyclohexyl)methyl]carbamate | 352 (M + H) | 3 |
| 1646 | octyl [(cis-4-{[4-(dimethylamino)pyrimidin-2-yl]amino}cyclohexyl) methyl]carbamate | 406 (M + H) | 3 |
| 1647 | ethyl [(cis-4-{[4-(dimethylamino)pyrimidin-2-yl]amino}cyclohexyl) methyl]carbamate | 322 (M + H) | 3 |
| 1648 | [4-(4-Dimethylamino-pyrimidin-2-ylamino)-cyclohexylmethyl]-carbamic acid 4-nitro-benzyl ester | 429 (M + H) | 3 |
| 1649 | 2-naphthyl[(cis-4-{[4-(dimethylamino)pyrimidin-2-yl]amino} cyclohexyl)methyl]carbamate | 420 (M + H) | 3 |
| 1650 | allyl [(cis-4-{[4-(dimethylamino)pyrimidin-2-yl]amino}cyclohexyl) methyl]carbamate | 334 (M + H) | 3 |
| 1651 | [4-(4-Dimethylamino-pyrimidin-2-ylamino)-cyclohexylmethyl]-carbamic acid benzyl ester | 384 (M + H) | 3 |
| 1652 | phenyl [(cis-4-{[4-(dimethylamino)pyrimidin-2-yl]amino} cyclohexyl)methyl]carbamate | 370 (M + H) | 3 |
| 1653 | (2S,5R)-2-isopropyl-5-methylcyclohexyl [(cis-4-{ [4-(dimethylamino)pyrimidin-2-yl]amino}cyclohexyl)methyl]-carbamate | 432 (M + H) | 3 |
| 1654 | 4-methylphenyl [(cis-4-{[4-(dimethylamino)pyrimidin-2-yl]amino} cyclohexyl)methyl]carbamate | 384 (M + H) | 3 |
| 1655 | methyl [(cis-4-{[4-(dimethylamino)pyrimidin-2-yl]amino} cyclohexyl)methyl]carbamate | 308 (M + H) | 3 |
| 1656 | 2-chlorobenzyl [(cis-4-{[4-(dimethylamino)pyrimidin-2-yl]amino} cyclohexyl)methyl]carbamate | 418 (M + H) | 3 |
| 1657 | 9H-fluoren-9-ylmethyl [(cis-4-{[4-(dimethylamino)pyrimidin-2-yl] amino}cyclohexyl)methyl]carbamate | 472 (M + H) | 3 |

(continued)

| Ex. No. | compound name | MS | class |
|---|---|---|---|
| 1658 | 2,2,2-trichloroethyl [(cis-4-{[4-(dimethylamino)pyrimidin-2-yl] amino}cyclohexyl)methyl]carbamate | 424 (M + H) | 3 |
| 1659 | N-(2-chlorophenyl)-N'-(cis-4-{[4-(dimethylamino)-5,6,7,8-tetrahydroquinazolin-2-yl]amino)cyclohexyl)urea | 443 (M + H) | 3 |
| 1660 | N-(cis-4-{[4-(dimethylamino)-5,6,7,8-tetrahydroquinazolin-2-yl] amino}cyclohexyl)-N'-(2,6-dimethylphenyl)urea | 437 (M + H) | 3 |
| 1661 | N-(2,4-difluorophenyl)-N'-(cis-4-{[4-(dimethylamino)-5,6,7,8-tetrahydroquinazolin-2-yl]amino}cyclohexyl)urea | 445 (M + H) | 3 |
| 1662 | N-(cis-4-{[4-(dimethylamino)-5,6,7,8-tetrahydroquinazolin-2-yl] amino}cyclohexyl)-N'-(2-ethyl-6-methylphenyl)urea | 451 (M + H) | 1 |
| 1663 | ethyl N-{[(cis-4-{[4-(dimethylamino)-5,6,7,8-tetrahydro-quinazolin-2-yl]amino}cyclohexyl)amino]carbonyl}leucinate | 475 (M + H) | 3 |
| 1664 | N-(cis-4-{[4-(dimethylamino)-5,6,7,8-tetrahydroquinazolin-2-yl] amino}cyclohexyl)-N'-(4-fluorophenyl)urea | 427 (M + H) | 2 |
| 1665 | N-(cis-4-{[4-(dimethylamino)-5,6,7,8-tetrahydroquinazolin-2-yl] amino}cyclohexyl)-N'-[4-(methylthio)phenyl]urea | 455 (M + H) | 3 |
| 1666 | N-(cis-4-{[4-(dimethylamino)-5,6,7,8-tetrahydroquinazolin-2-yl] amino}cyclohexyl)-N'-[2-(trifluoromethyl)phenyl]urea | 477 (M + H) | 3 |
| 1667 | N-(cis-4-{[4-(dimethylamino)-5,6,7,8-tetrahydroquinazolin-2-yl] amino}cyclohexyl)-N'-mesitylurea | 451 (M + H) | 1 |
| 1668 | N-(cis-4-{[4-(dimethylamino)-5,6,7,8-tetrahydroquinazolin-2-yl] amino}cyclohexyl)-N'-(2-methylphenyl)urea | 423 (M + H) | 3 |
| 1669 | N-(cis-4-{[4-(dimethylamino)-5,6,7,8-tetrahydroquinazolin-2-yl] amino}cyclohexyl)-N'-(2,4,6-trichlorophenyl)urea | 511 (M + H) | 2 |
| 1670 | N-(cis-4-{[4-(dimethylamino)-5,6,7,8-tetrahydroquinazolin-2-yl] amino}cyclohexyl)-N'-(2,4,6-tribromophenyl)urea | 642 (M + H) | 1 |
| 1671 | N-(2,4-dibromo-6-fluorophenyl)-N'-(cis-4-{[4-(dimethylamino)-5,6,7,8-tetrahydroquinazolin-2-yl]amino}cyclohexyl)urea | 583 (M + H) | 2 |
| 1672 | N-(2,6-diethylphenyl)-N'-(cis-4-{[4-(dimethylamino)-5,6,7,8-tetrahydroquinazolin-2-yl]amino}cyclohexyl)urea | 465 (M+ H) | 1 |
| 1673 | N-(2-chloro-6-(trifluoromethyl)phenyl]-N'-(cis-4-[4-(dimethyl {amino)-5,6,7,8-tetrahydroquinazolin-2-yl]amino-}cyclohexyl)urea | 511 (M + H) | 3 |
| 1674 | N-(2-chloro-6-methylphenyl)-N'-(cis-4-{[4-(dimethylamino)-5,6,7,8-tetrahydroquinazolin-2-yl]amino}cyclohexyl)urea | 457 (M + H) | 3 |
| 1675 | N-(2-chlorobenzyl)-N'-(cis-4-{[4-(dimethylamino)-5,6,7,8-tetrahydroquinazolin-2-yl]amino}cyclohexyl)urea | 457 (M + H) | 2 |
| 1676 | N-(cis-4-{[4-(dimethylamino)-5,6,7,8-tetrahydroquinazolin-2-yl] amino}cyclohexyl)-N'-(2-ethyl-6-isopropylphenyl)urea | 479 (M + H) | 2 |
| 1677 | N-(cis-4-{[4-(dimethylamino)-5,6,7,8-tetrahydroquinazolin-2-yl] amino}cyclohexyl)-N'-(2-ethylphenyl)urea | 437 (M + H) | 2 |
| 1678 | N-(cis-4-{[4-(dimethylamino)-5,6,7,8-tetrahydroquinazolin-2-yl] amino}cyclohexyl)-N'-(2-iodophenyl)urea | 535 (M + H) | 3 |
| 1679 | N-(cis-4-{[4-(dimethylamino)-5,6,7,8-tetrahydroquinazolin-2-yl] amino}cyclohexyl)-N'-(2-isopropyl-6-methylphenyl)urea | 465 (M + H) | 2 |

(continued)

| Ex. No. | compound name | MS | class |
|---|---|---|---|
| 1680 | N-(cis-4-{[4-(dimethylamino)-5,6,7,8-tetrahydroquinazolin-2-yl] amino}cyclohexyl)-N'-(2-isopropylphenyl)urea | 451 (M + H) | 3 |
| 1681 | N-(cis-4-{[4-(dimethylamino)-5,6,7,8-tetrahydroquinazolin-2-yl] amino}cyclohexyl)-N'-(2-methyl-3-nitrophenyl)urea | 468 (M + H) | 3 |
| 1682 | N-(cis-4-{[4-(dimethylamino)-5,6,7,8-tetrahydroquinazolin-2-yl] amino}cyclohexyl)-N'-(2-propylphenyl)urea | 451 (M + H) | 3 |
| 1683 | N-(2-tert-butyl-6-methylphenyl)-N'-(cis-4-{[4-(dimethylamino)-5,6,7,8-tetrahydroquinazolin-2-yl]amino}cyclohexyl)urea | 479 (M + H) | 2 |
| 1684 | N-(2-tert-butylphenyl)-N'-(cis-4-{[4-(dimethylamino)-5,6,7,8-tetrahydroquinazolin-2-yl]amino}cyclohexyl)urea | 465 (M+H) | 3 |
| 1685 | N-(3-chloro-2-methylphenyl)-N'-(cis-4-{[4-(dimethylamino)-5,6,7,8-tetrahydroquinazolin-2-yl]amino}cyclohexyl)urea | 457 (M + H) | 3 |
| 1686 | N-(4-bromo-2,6-difluorophenyl)-N'-(cis-4-{[4-(dimethylamino)-5,6,7,8-tetrahydroquinazolin-2-yl]amino}cyclohexyl)urea | 523 (M + H) | 3 |
| 1687 | N-[4-chloro-2-(trifluoromethyl)phenyl]-N'-(cis-4-{[4-(dimethyl-amino)-5,6,7,8-tetrahydroquinazolin-2-yl]amino}cyclohexyl)urea | 511 (M + H) | 3 |
| 1688 | N-(4-cyanophenyl)-N'-(cis-4-{[4-(dimethylamino)-5,6,7,8-tetrahydroquinazolin-2-yl]amino}cyclohexyl)urea | 434 (M + H) | 3 |
| 1689 | N-(cis-4-{[4-(dimethylamino)-5,6,7,8-tetrahydroquinazolin-2-yl] amino}cyclohexyl)-N'-(diphenylmethyl)urea | 499 (M + H) | 1 |
| 1690 | N-(4-bromo-2,6-dimethylphenyl)-N'-(cis-4-{[4-(dimethylamino)-5,6,7,8-tetrahydroquinazolin-2-yl)amino}cyclohexyl)urea | 515 (M + H) | 1 |
| 1691 | N-(cis-4-{[4-(dimethylamino)-5,6,7,8-tetrahydroquinazolin-2-yl] amino}cyclohexyl)-N'-(3-methyl-5-phenylisoxazol-4-yl)urea | 490 (M + H) | 1 |
| 1692 | N-(cis-4-{[4-(dimethylamino)-5,6,7,8-tetrahydroquinazolin-2-yl] amino}cyclohexyl)-N'-[5-methyl-2-(trifluoromethyl)-3-furyl]-urea | 481 (M + H) | 3 |
| 1693 | N-(2-bromophenyl)-N'-(cis-4-{[4-(dimethylamino)-5,6,7,8-tetrahydroquinazolin-2-yl]amino}cyclohexyl)urea | 487 (M + H) | 3 |
| 1694 | N-biphenyl-2-yl-N'-(cis-4-{[4-(dimethylamino)-5,6,7,8-tetrahydroquinazolin-2-yl]amino}cyclohexyl)urea | 485 (M + H) | 3 |
| 1695 | N-butyl-N'-(cis-4-{[4-(dimethylamino)-5,6,7,8-tetrahydroquinazolin-2-yl]amino}cyclohexyl)urea | 389 (M + H) | 3 |
| 1696 | N-(cis-4-{[4-(dimethylamino)-5,6,7,8-tetrahydroquinazolin-2-yl] amino}cyclohexyl)-N'-(2,3-dimethylphenyl)urea | 437 (M + H) | 3 |
| 1697 | ethyl 3-({[(cis-4-{[4-(dimethylamino)-5,6,7,8-tetrahydro-quinazolin-2-yl]amino}cyclohexyl)amino]carbonyl}amino)-benzoate | 481 (M + H) | 3 |
| 1698 | N-(cis-4-{[4-(dimethylamino)-5,6,7,8-tetrahydroquinazolin-2-yl] amino}cyclohexyl)-N'-[1-(3-isopropenylphenyl)-1-methyl-ethyl]-urea | 491 (M + H) | 3 |
| 1699 | methyl N-{[(cis-4-{[4-(dimethylamino)-5,6,7,8-tetrahydro-quinazolin-2-yl]amino}cyclohexyl)amino]carbonyl}methioninate | 479 (M + H) | 3 |
| 1700 | N-(cis-4-{[4-(dimethylamino)-5,6,7,8-tetrahydroquinazolin-2-yl] amino}cyclohexyl)-N'-1-naphthylurea | 459 (M + H) | 2 |

(continued)

| Ex. No. | compound name | MS | class |
|---|---|---|---|
| 1701 | N-(cis-4-{[4-(dimethylamino)-5,6,7,8-tetrahydroquinazolin-2-yl] amino}cyclohexyl)-N'-[(2S)-2-phenylcyclopropyl]urea | 449 (M + H) | 3 |
| 1702 | N-(cis-4-{[4-(dimethylamino)-5,6,7,8-tetrahydroquinazolin-2-yl] amino}cyclohexyl)-N'-(4-phenoxyphenyl)urea | 501 (M + H) | 3 |
| 1703 | N-(cis-4-{[4-(dimethylamino)-5,6,7,8-tetrahydroquinazolin-2-yl] amino}cyclohexyl)-N'-pentylurea | 403 (M + H) | 3 |
| 1704 | N-(cis-4-{[4-(dimethylamino)-5,6,7,8-tetrahydroquinazolin-2-yl] amino}cyclohexyl)-N'-[1-(1-naphthyl)ethyl]urea | 487 (M + H) | 1 |
| 1705 | methyl N-{[(cis-4-{[4-(dimethylamino)-5,6,7,8-tetrahydro-quinazolin-2-yl]amino}cyclohexyl)amino]carbonyl }-phenylalaninate | 495 (M + H) | 3 |
| 1706 | N-(cis-4-{[4-(dimethylamino)-5,6,7,8-tetrahydroquinazolin-2-yl] amino}cyclohexyl)-N'-(1-phenylethyl)urea | 437 (M + H) | 3 |
| 1707 | 1[4-(4-Dimethylamino-5,6,7,8-tetrahydro-quinazolin-2-ylamino)-cyclohexyl]-3-(1-phenyl-ethyl)-urea | 437 (M + H) | 3 |
| 1708 | N-(cis-4-{[4-(dimethylamino)-5,6,7,8-tetrahydroquinazolin-2-yl] amino}cyclohexyl)-N'-(2,3,5,6-tetrachlorophenyl)urea | 545 (M + H) | 3 |
| 1709 | N-(2,4-dibromophenyl)-N'-(cis-4-{[4-(dimethylamino)-5,6,7,8-tetrahydroquinazolin-2-yl]amino}cyclohexyl)urea | 565 (M + H) | 2 |
| 1710 | N-(2,4-dichlorobenzyl)-N'-(cis-4-{[4-(dimethylamino)-5,6,7,8-tetrahydroquinazolin-2-yl]amino}cyclohexyl)urea | 491 (M + H) | 2 |
| 1711 | N-(2,4-dimethoxyphenyl)-N'-(cis-4-{[4-(dimethylamino)-5,6,7,8-tetrahydroquinazolin-2-yl]amino}cyclohexyl)urea | 469 (M + H) | 2 |
| 1712 | N-(cis-4-{[4-(dimethylamino)-5,6,7,8-tetrahydroquinazolin-2-yl] amino}cyclohexyl)-N'-(2-ethoxyphenyl)urea | 453 (M + H) | 2 |
| 1713 | N-(cis-4-{[4-(dimethylamino)-5,6,7,8-tetrahydroquinazolin-2-yl] amino}cyclohexyl)-N'-(2-fluorobenzyl)urea | 441 (M + H) | 2 |
| 1714 | N-(cis-4-{[4-(dimethylamino)-5,6,7,8-tetrahydroquinazolin-2-yl] amino}cyclohexyl)-N'-(2-methyl-4-nitrophenyl)urea | 468 (M + H) | 3 |
| 1715 | N-(cis-4-{[4-(dimethylamino)-5,6,7,8-tetrahydroquinazolin-2-yl] amino}cyclohexyl)-N'-(2-methyl-5-nitrophenyl)urea | 468 (M + H) | 3 |
| 1716 | N-(cis-4-{[4-(dimethylamino)-5,6,7,8-tetrahydroquinazolin-2-yl] amino}cyclohexyl)-N'-(2-methylbenzyl)urea | 437 (M + H) | 3 |
| 1717 | N-(cis-4-{[4-(dimethylamino)-5,6,7,8-tetrahydroquinazolin-2-yl] amino}cyclohexyl)-N'-(2-nitrophenyl)urea | 454 (M + H) | 3 |
| 1718 | N-1,3-benzodioxol-5-yl-N'-(cis-4-{[4-(dimethylamino)-5,6,7,8-tetrahydroquinazolin-2-yl]amino}cyclohexyl)urea | 453 (M + H) | 3 |
| 1719 | N-(cis-4-{[4-(dimethylamino)-5,6,7,8-tetrahydroquinazolin-2-yl] amino}cyclohexyl)-N'-(3,4,5-trimethoxyphenyl)urea | 499 (M + H) | 1 |
| 1720 | N-(3,4-dimethoxyphenyl)-N'-(cis-4-{[4-(dimethylamino)-5,6,7,8-tetrahydroquinazolin-2-yl]amino}cyclohexyl)urea | 469 (M + H) | 2 |
| 1721 | N-(3-chloro-4-methoxyphenyl)-N'-(cis-4-{[4-(dimethylamino)-5,6,7,8-tetrahydroquinazolin-2-yl]amino}cyclohexyl)urea | 473 (M + H) | 3 |

(continued)

| Ex. No. | compound name | MS | class |
|---|---|---|---|
| 1722 | N-[4-bromo-2-(trifluoromethyl)phenyl]-N'-(cis-4-{[4-(dimethylamino)-5,6,7,8-tetrahydroquinazolin-2-yl]amino}cyclohexyl)urea | 555 (M + H) | 3 |
| 1723 | N-(4-bromobenzyl)-N'-(cis-4-{[4-(dimethylamino)-5,6,7,8-tetrahydroquinazolin-2-yl]amino}cyclohexyl)urea | 501 (M + H) | 3 |
| 1724 | N-(4-chloro-2-methylphenyl)-N'-(cis-4-{[4-(dimethylamino)-5,6,7,8-tetrahydroquinazolin-2-yl]amino}cyclohexyl)urea | 457 (M + H) | 2 |
| 1725 | N-(cis-4-{[4-(dimethylamino)-5,6,7,8-tetrahydroquinazolin-2-yl]amino}cyclohexyl)-N'-(4-fluorobenzyl)urea | 441 (M + H) | 2 |
| 1726 | N-(cis-4-{[4-(dimethylamino)-5,6,7,8-tetrahydroquinazolin-2-yl]amino}cyclohexyl)-N'-(4-methoxy-2-methylphenyl)urea | 453 (M + H) | 2 |
| 1727 | N-(5-chloro-2,4-dimethoxyphenyl)-N'-(cis-4-{[4-(dimethylamino)-5,6,7,8-tetrahydroquinazolin-2-yl]amino}cyclohexyl)urea | 503 (M + H) | 1 |
| 1728 | N-[1-(4-bromophenyl)ethyl]-N'-(cis-4-{[4-(dimethylamino)-5,6,7,8-tetrahydroquinazolin-2-yl]amino}cyclohexyl)urea | 515 (M + H) | 2 |
| 1729 | N-(4-bromo-2-methylphenyl)-N'-(cis-4-{[4-(dimethylamino)-5,6,7,8-tetrahydroquinazolin-2-yl]amino}cyclohexyl)urea | 501 (M + H) | 2 |
| 1730 | ethyl N-{[(cis-4-{[4-(dimethylamino)-5,6,7,8-tetrahydro-quinazolin-2-yl]amino}cyclohexyl)amino]carbonyl}-phenylalaninate | 509 (M + H) | 3 |
| 1731 | N-(2,3-dihydro-1,4-benzodioxin-6-yl)-N'-(cis-4-{[4-(dimethyl-amino)-5,6,7,8-tetrahydroquinazolin-2-yl]amino}cyclohexyl)urea | 467 (M + H) | 3 |
| 1732 | N-(2,6-dibromo-4-isopropylphenyl)-N'-(cis-4-{[4-(dimethylamino)-5,6,7,8-tetrahydroquinazolin-2-yl]amino}cyclohexyl)urea | 607 (M + H) | 3 |
| 1733 | N-[3-(cydopentyloxy)-4-methoxyphenyl]-N'-(cis-4-{[4-(dimethylamino)-5,6,7,8-tetrahydroquinazolin-2-yl]amino}cyclohexyl)urea | 523 (M + H) | 3 |
| 1734 | N-(3,4-dihydro-2H-1,5-benzodioxepin-7-yl)-N'-(cis-4-{[4-(dimethylamino)-5,6,7,8-tetrahydroquinazolin-2-yl]amino}cyclohexyl)urea | 481 (M + H) | 3 |
| 1735 | N-(4-butyl-2-methylphenyl)-N'-(cis-4-{[4-(dimethylamino)-5,6,7,8-tetrahydroquinazolin-2-yl]amino}cyclohexyl)urea | 479 (M + H) | 3 |
| 1736 | N-(cis-4-{[4-(dimethylamino)-5,6,7,8-tetrahydroquinazolin-2-yl]amino}cyclohexyl)-N'-(5-methyl-3-phenylisoxazol-4-yl)urea | 490 (M + H) | 1 |
| 1737 | N-(4-bromophenyl)-N'-(cis-4-{[4-(dimethylamino)-5,6,7,8-tetrahydroquinazolin-2-yl]amino}cyclohexyl)thiourea | 503 (M + H) | 3 |
| 1738 | N-(4-cyanophenyl)-N'-(cis-4-{[4-(dimethylamino)-5,6,7,8-8-tetrahydroquinazolin-2-yl]amino}cyclohexyl)thiourea | 450 (M + H) | 3 |
| 1739 | N-(2,4-dichlorophenyl)-N'-(cis-4-{[4-(dimethylamino)-5,6,7,8-tetrahydroquinazolin-2-yl]amino}cyclohexyl)thiourea | 493 (M + H) | 3 |
| 1740 | N-(2,4-dimethoxyphenyl)-N'-(cis-4-{[4-(dimethylamino)-5,6,7,8-tetrahydroquinazolin-2-yl]amino}cyclohexyl)thiourea | 485 (M + H) | 1 |
| 1741 | N-(cis-4-{[4-(dimethylamino)-5,6,7,8-tetrahydroquinazolin-2-yl]amino}cyclohexyl)-N'-(2,6-dimethylphenyl)thiourea | 453 (M + H) | 3 |
| 1742 | N-(cis-4-{[4-(dimethylamino)-5,6,7,8-tetrahydroquinazolin-2-yl]amino}cyclohexyl)-N'-(2-ethyl-6-isopropylphenyl)thiourea | 495 (M + H) | 3 |

(continued)

| Ex. No. | compound name | MS | class |
|---|---|---|---|
| 1743 | N-(cis-4-{[4-(dimethylamino)-5,6,7,8-tetrahydroquinazolin-2-yl]amino}cyclohexyl)-N'-(2-methoxyphenyl)thiourea | 455 (M + H) | 3 |
| 1744 | N-(cis-4-{[4-(dimethylamino)-5,6,7,8-tetrahydroquinazolin-2-yl]amino}cyclohexyl)-N'-1-naphthylthiourea | 475 (M + H) | 3 |
| 1745 | N-(cis-4-{[4-(dimethylamino)-5,6,7,8-tetrahydroquinazolin-2-yl]amino}cyclohexyl)-N'-(3,4,5-trimethoxyphenyl)thiourea | 515 (M + H) | 1 |
| 1746 | N-(3,4-dimethoxyphenyl)-N'-(cis-4-{[4-(dimethylamino)-5,6,7,8-tetrahydroquinazolin-2-yl]amino}cyclohexyl)thiourea | 485 (M + H) | 1 |
| 1747 | N-[4-(dimethylamino)-1-naphthyl]-N'-(cis-4-{[4-(dimethylamino)-5,6,7,8-tetrahydroquinazolin-2-yl]amino}cyclohexyl)thiourea | 518 (M + H) | 2 |
| 1748 | N-(cis-4-{[4-(dimethylamino)-5,6,7,8-tetrahydroquinazolin-2-yl]amino}cyclohexyl)-N'-(2-ethylphenyl)thiourea | 453 (M + H) | 3 |
| 1749 | N-(cis-4-{[4-(dimethylamino)-5,6,7,8-tetrahydroquinazolin-2-yl]amino}cyclohexyl)-N'-(2-methoxy-4-nitrophenyl)thiourea | 500 (M + H) | 3 |
| 1750 | N-(cis-4-{[4-(dimethylamino)-5,6,7,8-tetrahydroquinazolin-2-yl]amino}cyclohexyl)-N'-(2-methoxy-5-methylphenyl)thiourea | 469 (M + H) | 2 |
| 1751 | N-(4-bromo-2-chlorophenyl)-N'-(cis-4-{[4-(dimethylamino)-5,6,7,8-tetrahydroquinazolin-2-yl]amino}cyclohexyl)thiourea | 537 (M + H) | 1 |
| 1752 | N-(cis-4-{[4-(dimethylamino)-5,6,7,8-tetrahydroquinazolin-2-yl]amino}cyclohexyl)-N'-(4-iodophenyl)thiourea | 551 (M + H) | 2 |
| 1753 | N-(cis-4-{[4-(dimethylamino)-5,6,7,8-tetrahydroquinazolin-2-yl]amino}cyclohexyl)-N'-(2,4,6-tribromophenyl)thiourea | 658 (M + H) | 1 |
| 1754 | N-(cis-4-{[4-(dimethylamino)-5,6,7,8-tetrahydroquinazolin-2-yl]amino}cyclohexyl)-N'-(2,4,6-trichlorophenyl)thiourea | 527 (M + H) | 2 |
| 1755 | N-(cis-4-{[4-(dimethylamino)-5,6,7,8-tetrahydroquinazolin-2-yl]amino}cyclohexyl)-N'-mesitylthiourea | 467 (M + H) | 1 |
| 1756 | N-(cis-4-{[4-(dimethylamino)-5,6,7,8-tetrahydroquinazolin-2-yl]amino}cyclohexyl)-N'-(2,4-dimethylphenyl)thiourea | 453 (M + H) | 2 |
| 1757 | N-(2,6-diethylphenyl)-N'-(cis-4-{[4-(dimethylamino)-5,6,7,8-tetrahydroquinazolin-2-yl]amino}cyclohexyl)thiourea | 481 (M + H) | 1 |
| 1758 | N-(2-bromo-4-methylphenyl)-N'-(cis-4-{[4-(dimethylamino)-5,6,7,8-tetrahydroquinazolin-2-yl]amino}cyclohexyl)thiourea | 517 (M + H) | 3 |
| 1759 | N-(2-chlorobenzyl)-N'-(cis-4-{[4-(dimethylamino)-5,6,7,8-tetrahydroquinazolin-2-yl]amino}cyclohexyl)thiourea | 473 (M + H) | 3 |
| 1760 | N-(cis-4-{[4-(dimethylamino)-5,6,7,8-tetrahydroquinazolin-2-yl]amino}cyclohexyl)-N'-(2-ethyl-6-methylphenyl)thiourea | 467 (M + H) | 3 |
| 1761 | N-(cis-4-{[4-(dimethylamino)-5,6,7,8-tetrahydroquinazolin-2-yl]amino}cyclohexyl)-N'-(2-isopropylphenyl)thiourea | 467 (M + H) | 3 |
| 1762 | methyl 3-({[(cis-4-{[4-(dimethylamino)-5,6,7,8-tetrahydroquinazolin-2-yl]amino}cyclohexyl)amino]carbonothioyl}amino)-benzoate | 483 (M + H) | 3 |
| 1763 | N-(4-bromo-2,6-dimethylphenyl)-N'-(cis-4-{[4-(dimethylamino)-5,6,7,8-tetrahydroquinazolin-2-yl]amino)cyclohexyl)thiourea | 531 (M + H) | 1 |
| 1764 | N-(4-bromo-2-methylphenyl)-N'-(cis-4-{[4-(dimethylamino)-5,6,7,8-tetrahydroquinazolin-2-yl]amino}cyclohexyl)thiourea | 517 (M + H) | 1 |

(continued)

| Ex. No. | compound name | MS | class |
|---|---|---|---|
| 1765 | N-[4-bromo-2-(trifluoromethyl)phenyl]-N'-(cis-4-{ [4-(dimethylamino)-5,6,7,8-tetrahydroquinazolin-2-yl]amino} cyclohexyl)-thiourea | 571 (M + H) | 1 |
| 1766 | N-(4-chloro-2-methylphenyl)-N'-(cis-4-{[4-(dimethylamino)-5,6,7,8-tetrahydroquinazolin-2-yl]amino}cyclohexyl)thiourea | 473 (M + H) | 1 |
| 1767 | N-(cis-4-{[4-(dimethylamino)-5,6,7,8-tetrahydroquinazolin-2-yl] amino}cyclohexyl)-N'-(1 -naphthylmethyl)thiourea | 489 (M + H) | 3 |
| 1768 | N-(2,3-dimethoxybenzyl)-N'-(cis-4-{[4-(dimethylamino)-5,6,7,8-tetrahydroquinazolin-2-yl]amino}cyclohexyl)thiourea | 499 (M + H) | 3 |
| 1769 | N-(cis-4-{[4-(dimethylamino)-5,6,7,8-tetrahydroquinazolin-2-yl] amino}cyclohexyl)-N'-(2,4,5-trimethylphenyl)thiourea | 467 (M + H) | 3 |
| 1770 | N-biphenyl-2-yl-N'-(cis-4-{[4-(dimethylamino)-5,6,7,8-tetrahydroquinazolin-2-yl]amino}cyclohexyl)thiourea | 501 (M + H) | 3 |
| 1771 | N-(cis-4-{[4-(dimethylamino)-5,6,7,8-tetrahydroquinazolin-2-yl] amino}cyclohexyl)-N'-(2-methyl-4-nitrophenyl)thiourea | 482 (M - H) | 3 |
| 1772 | N-(3-chlorobenzyl)-N'-(cis-4-{[4-(dimethylamino)-5,6,7,8-tetrahydroquinazolin-2-yl]amino}cyclohexyl)thiourea | 473 (M + H) | 3 |
| 1773 | ethyl 3-({[(cis-4-{[4-(dimethylamino)-5,6,7,8-tetrahydroquinazolin-2-yl]amino)cyclohexyl)amino]carbonothioyl}amino)-benzoate | 497 (M + H) | 3 |
| 1774 | N-[4-chloro-2-(trifluoromethyl)phenyl]-N'-(cis-4-{ [4-(dimethylamino)-5,6,7,8-tetrahydroquinazolin-2-yl]amino} cyclohexyl)-thiourea | 527 (M + H) | 2 |
| 1775 | N-(cis-4-{[4-(dimethylamino)-5,6,7,8-tetrahydroquinazolin-2-yl] amino}cyclohexyl)-N'-(4-fluoro-2-methylphenyl)thiourea | 457 (M + H) | 2 |
| 1776 | N-(cis-4-{[4-(dimethylamino)-5,6,7,8-tetrahydroquinazolin-2-yl] amino}cyclohexyl)-N'-(4-methoxy-2-methylphenyl)thiourea | 469 (M + H) | 2 |
| 1777 | N-(5-chloro-2,4-dimethoxyphenyl)-N'-(cis-4-{[4-(dimethylamino)-5,6,7,8-tetrahydroquinazolin-2-yl]amino}cyclohexyl)-thiourea | 519 (M + H) | 1 |
| 1778 | N-(cis-4-{[4-(dimethylamino)-5,6,7,8-tetrahydroquinazolin-2-yl] amino}cyclohexyl)-N'-[(1R)-1-phenylethyl]thiourea | 453 (M + H) | 3 |
| 1779 | N-(cis-4-{[4-(dimethylamino)-5,6,7,8-tetrahydroquinazolin-2-yl] amino}cyclohexyl)-N'-(2,3-dimethylphenyl)thiourea | 453 (M + H) | 3 |
| 1780 | N-(2,4-dibromo-6-fluorophenyl)-N'-(cis-4-{[4-(dimethylamino)-5,6,7,8-tetrahydroquinazolin-2-yl]amino}cyclohexyl)thiourea | 599 (M + H) | 2 |
| 1781 | N-(2,4-dichloro-6-methylphenyl)-N'-(cis-4-{[4-(dimethylamino)-5,6,7,8-tetrahydroquinazolin-2-yl]amino}cyclohexyl)thiourea | 507 (M + H) | 1 |
| 1782 | N-(cis-4-{[4-(dimethylamino)-5,6,7,8-tetrahydroquinazolin-2-yl] amino}cyclohexyl)-N'-(2-ethoxyphenyl)thiourea | 469 (M + H) | 2 |
| 1783 | N-(cis-4-{[4-(dimethylamino)-5,6,7,8-tetrahydroquinazolin-2-yl] amino}cyclohexyl)-N'-(2-isopropyl-6-methylphenyl)thiourea | 481 (M + H) | 3 |
| 1784 | N-(2,3-dihydro-1,4-benzodioxin-6-yl)-N'-(cis-4-{ [4-(dimethylamino)-5,6,7,8-tetrahydroquinazolin-2-yl]amino} cyclohexyl)thiourea | 483 (M + H) | 3 |
| 1785 | N-1,3-benzodioxol-5-yl-N'-(cis-4-{[4-(dimethylamino)-5,6,7,8-tetrahydroquinazolin-2-yl]amino}cyclohexyl)thiourea | 469 (M + H) | 3 |

(continued)

| Ex. No. | compound name | MS | class |
|---|---|---|---|
| 1786 | N-(3-chloro-2-methylphenyl)-N'-(cis-4-{[4-(dimethylamino)-5,6,7,8-tetrahydroquinazolin-2-yl]amino}cyclohexyl)thiourea | 473 (M + H) | 3 |
| 1787 | N-[4-bromo-2-(trifluoromethoxy)phenyl]-N'-(cis-4-{[4-(dimethyl-amino)-5,6,7,8-tetrahydroquinazolin-2-yl]amino}-cyclohexyl)-thiourea | 587 (M + H) | 2 |
| 1788 | N-(4-chloro-2,5-dimethoxyphenyl)-N'-(cis-4-{[4-(dimethylamino)-5,6,7,8-tetrahydroquinazolin-2-yl]amino}cyclohexyl)-thiourea | 519 (M + H) | 2 |
| 1789 | N-(cis-4-{[4-(dimethylamino)-5,6,7,8-tetrahydroquinazolin-2-yl] amino}cyclohexyl)-N'-(5-methyl-3-phenylisoxazol-4-yl)-thiourea | 506 (M + H) | 3 |
| 1790 | 1-Bicyclo[2.2.1]hept-2-yl-3-[4-(4-dimethylamino-5,6,7,8-tetrahydro-quinazolin-2-ylamino)-cyclohexyl]-thiourea | 443 (M + H) | 3 |
| 1791 | methyl 3-({[(cis-4-{[4-(dimethylamino)-5,6,7,8-tetrahydro-quinazolin-2-yl]amino)cyclohexyl)amino}carbonothioyl]amino) -4-methylthiophene-2-carboxylate | 503 (M + H) | 3 |
| 1792 | methyl 3-({[(cis-4-{[4-(dimethylamino)-5,6,7,8-tetrahydro-quinazolin-2-yl]amino ) cyclohexyl)amino]carbonothioyl}amino)-thiophene-2-carboxylate | 489 (M + H) | 3 |
| 1793 | N-(4-butyl-2-methylphenyl)-N'-(cis-4-{[4-(dimethylamino)-5,6,7,8-tetrahydroquinazolin-2-yl]amino ) cyclohexyl)thiourea | 495 (M + H) | 3 |
| 1794 | N-(3,5-dichlorophenyl)-N'-(cis-4-{[4-(dimethylamino)-5,6,7,8-tetrahydroquinazolin-2-yl]amino}cyclohexyl)urea | 477 (M + H) | 3 |
| 1795 | N-(2,3-dichlorophenyl)-N'-(cis-4-{[4-(dimethylamino)-5,6,7,8-tetrahydroquinazolin-2-yl]amino)cyclohexyl)urea | 477 (M + H) | 2 |
| 1796 | N-(cis-4-{[4-(dimethylamino)-5,6,7,8-tetrahydroquinazolin-2-yl] amino}cyclohexyl)-N'-(4-methylphenyl)urea | 423 (M + H) | 2 |
| 1797 | N-(2,6-diisopropylphenyl)-N'-(cis-4-{[4-(dimethylamino)-5,6,7,8-tetrahydroquinazolin-2-yl]amino)cyclohexyl)urea | 493 (M + H) | 2 |
| 1798 | N-(cis-4-{[4-(dimethylamino)-5,6,7,8-tetrahydroquinazolin-2-yl] amino}cyclohexyl)-N'-(2,3-dimethyl-6-nitrophenyl)urea | 482 (M + H) | 3 |
| 1799 | N-(2,6-dibromo-4-fluorophenyl)-N'-(cis-4-{[4-(dimethylamino)-5,6,7,8-tetrahydroquinazolin-2-yl]amino}cyclohexyl)urea | 583 (M + H) | 3 |
| 1800 | N-(2,6-dichlorophenyl)-N'-(cis-4-{[4-(dimethylamino)-5,6,7,8-tetrahydroquinazolin-2-yl]amino}cyclohexyl)urea | 477 (M + H) | 3 |
| 1801 | N-(cis-4-{[4-(dimethylarnino)-5,6,7,8-tetrahydroquinazolin-2-yl] amino}cyclohexyl)-N'-(2-methoxy-5-methylphenyl)urea | 453 (M + H) | 3 |
| 1802 | N-(cis-4-{[4-(dimethylamino)-5,6,7,8-tetrahydroquinazolin-2-yl] amino}cyclohexyl)-N'-(2-methyl-6-nitrophenyl)urea | 468 (M + H) | 3 |
| 1803 | N-(3,4-difluorophenyl)-N'-(cis-4-{[4-(dimethylamino)-5,6,7,8-tetrahydroquinazolin-2-yl]amino}cyclohexyl)urea | 445 (M + H) | 3 |
| 1804 | N-(3,5-difluorophenyl)-N'-(cis-4-{[4-(dimethylamino)-5,6,7,8-tetrahydroquinazolin-2-yl]amino}cyclohexyl)urea | 445 (M + H) | 3 |
| 1805 | N-(3-chloro-4-fluorophenyl)-N'-(cis-4-{[4-(dimethylamino)-5,6,7,8-tetrahydroquinazolin-2-yl]amino}cyclohexyl)urea | 461 (M + H) | 3 |
| 1806 | N-(3-acetylphenyl)-N'-(cis-4-{[4-(dimethylamino)-5,6,7,8-tetrahydroquinazolin-2-yl]amino}cyclohexyl)urea | 451 (M + H) | 3 |

(continued)

| Ex. No. | compound name | MS | class |
|---|---|---|---|
| 1807 | N-1-adamantyl-N'-(cis-4-{[4-(dimethylamino)-5,6,7,8-tetrahydroquinazolin-2-yl]amino}cyclohexyl)urea | 467 (M + H) | 3 |
| 1808 | N-(4-acetylphenyl)-N'-(cis-4-{[4-(dimethylamino)-5,6,7,8-tetrahydroquinazolin-2-yl]amino}cyclohexyl)urea | 451 (M + H) | 3 |
| 1809 | N-{[(cis-4-{[4-(dimethylamino)-5,6,7,8-tetrahydroquinazolin-2-yl]amino}cyclohexyl)amino]carbonyl}benzamide | 437 (M + H) | 3 |
| 1810 | N-(tert-butyl)-N'-(cis-4-{[4-(dimethylamino)-5,6,7,8-tetrahydroquinazolin-2-yl]amino}cyclohexyl)urea | 389 (M + H) | 3 |
| 1811 | N-[3,5-bis(trifluoromethyl)phenyl]-N'-(cis-4-{[4-(dimethylamino)-5,6,7,8-tetrahydroquinazolin-2-yl]amino}cyclohexyl)urea | 545 (M + H) | 3 |
| 1812 | N-benzyl-N'-(cis-4-{[4-(dimethylamino)-5,6,7,8-tetrahydroquinazolin-2-yl]amino}cyclohexyl)urea | 423 (M + H) | 3 |
| 1813 | N-(4-bromophenyl)-N'-(cis-4-{[4-(dimethylamino)-5,6,7,8-tetrahydroquinazolin-2-yl]amino}cyclohexyl)urea | 487 (M + H) | 3 |
| 1814 | N-(3-chlorophenyl)-N'-(cis-4-{[4-(dimethylamino)-5,6,7,8-tetrahydroquinazolin-2-yl]amino}cyclohexyl)urea | 443 (M + H) | 3 |
| 1815 | N-(4-chlorophenyl)-N'-(cis-4-{[4-(dimethylamino)-5,6,7,8-tetrahydroquinazolin-2-yl]amino}cyclohexyl)urea | 443 (M + H) | 3 |
| 1816 | N-cyclohexyl-N'-(cis-4-{[4-(dimethylamino)-5,6,7,8-tetrahydroquinazolin-2-yl]amino}cyclohexyl)urea | 415 (M + H) | 3 |
| 1817 | N-(3-cyanophenyl)-N'-(cis-4-{[4-(dimethylamino)-5,6,7,8-tetrahydroquinazolin-2-yl]amino}cyclohexyl)urea | 434 (M + H) | 3 |
| 1818 | N-(3,4-dichlorophenyl)-N'-(cis-4-{[4-(dimethylamino)-5,6,7,8-tetrahydroquinazolin-2-yl]amino}cyclohexyl)urea | 477 (M + H) | 3 |
| 1819 | N-(2,4-dichlorophenyl)-N'-(cis-4-{[4-(dimethylamino)-5,6,7,8-tetrahydroquinazolin-2-yl]amino}cyclohexyl)urea | 477 (M + H) | 3 |
| 1820 | N-(2,6-difluorophenyl)-N'-(cis-4-{[4-(dimethylamino)-5,6,7,8-tetrahydroquinazolin-2-yl]amino}cyclohexyl)urea | 445 (M + H) | 3 |
| 1821 | N-(2,5-dichlorophenyl)-N'-(cis-4-{[4-(dimethylamino)-5,6,7,8-tetrahydroquinazolin-2-yl]amino}cyclohexyl)urea | 477 (M + H) | 3 |
| 1822 | ethyl N-{[(cis-4-{[4-(dimethylamino)-5,6,7,8-tetrahydroquinazolin-2-yl]amino}cyclohexyl)amino]carbonyl}glycinate | 419 (M + H) | 3 |
| 1823 | ethyl 4-({[(cis-4-{[4-(dimethylamino)-5,6,7,8-tetrahydroquinazolin-2-yl]amino}cyclohexyl)amino]carbonyl}amino)-benzoate | 481 (M + H) | 3 |
| 1824 | N-(cis-4-{[4-(dimethylamino)-5,6,7,8-tetrahydroquinazolin-2-yl]amino}cyclohexyl)-N'-(4-ethylphenyl)urea | 437 (M + H) | 3 |
| 1825 | N-(cis-4-{[4-(dimethylamino)-5,6,7,8-tetrahydroquinazolin-2-yl]amino}cyclohexyl)-N'-ethylurea | 361 (M + H) | 3 |
| 1826 | N-(cis-4-{[4-(dimethylamino)-5,6,7,8-tetrahydroquinazolin-2-yl]amino}cyclohexyl)-N'-(4-fluoro-3-nitrophenyl)urea | 472 (M + H) | 3 |
| 1827 | N-(cis-4-{[4-(dimethylamino)-5,6,7,8-tetrahydroquinazolin-2-yl]amino}cyclohexyl)-N'-(3-fluorophenyl)urea | 427 (M + H) | 3 |
| 1828 | N-(cis-4-{[4-(dimethylamino)-5,6,7,8-tetrahydroquinazolin-2-yl]amino}cyclohexyl)-N'-(2-fluorophenyl)urea | 427 (M + H) | 3 |

(continued)

| Ex. No. | compound name | MS | class |
|---|---|---|---|
| 1829 | N-(cis-4-([4-(dimethylamino)-5,6,7,8-tetrahydroquinazolin-2-yl] amino}cyclohexyl)-N'-(4-isopropylphenyl)urea | 451 (M + H) | 3 |
| 1830 | N-(cis-4-{[4-(dimethylamino)-5,6,7,8-tetrahydroquinazolin-2-yl] amino}cyclohexyl)-N'-isopropylurea | 375 (M + H) | 3 |
| 1831 | N-(cis-4-{[4-(dimethylamino)-5,6,7,8-tetrahydroquinazolin-2-yl] amino}cyclohexyl)-N'-(4-methoxyphenyl)urea | 439 (M + H) | 3 |
| 1832 | N-(cis-4-{[4-(dimethylamino)-5,6,7,8-tetrahydroquinazolin-2-yl] amino}cyclohexyl)-N'-(4-methyl-2-nitrophenyl)urea | 468 (M + H) | 3 |
| 1833 | N-(cis-4-{[4-(dimethylamino)-5,6,7,8-tetrahydroquinazolin-2-yl] amino cyclohexyl)-N'-(2-methoxyphenyi)urea | 439 (M + H) | 3 |
| 1834 | N-(cis-4-{[4-(dimethylamino)-5,6,7,8-tetrahydroquinazolin-2-yl] amino}cyclohexyl)-N'-(3-methoxyphenyl)urea | 439 (M + H) | 3 |
| 1835 | N-(cis-4-{[4-(dimethylamino)-5,6,7,8-tetrahydroquinazolin-2-yl] amino}cyclohexyl)-N'-(4-methoxybenzyl)urea | 453 (M + H) | 3 |
| 1836 | N-(cis-4-{[4-(dimethylamino)-5,6,7,8-tetrahydroquinazolin-2-yl] amino}cyclohexyl)-N'-(3-methylbenzyl)urea | 437 (M + H) | 3 |
| 1837 | N-(cis-4-{[4-(dimethylamino)-5,6,7,8-tetrahydroquinazolin-2-yl] amino}cyclohexyl)-N'-propylurea | 375 (M + H) | 3 |
| 1838 | N-(cis-4-{[4-(dimethylamino)-5,6,7,8-tetrahydroquinazolin-2-yl] amino}cyclohexyl)-N'-[3-(trifluoromethyl)phenyl]urea | 477 (M + H) | 3 |
| 1839 | N-(cis-4-{[4-(dimethylamino)-5,6,7,8-tetrahydroquinazolin-2-yl] amino}cyclohexyl)-N'-[3-(triethoxysilyl)propyl]urea | 537 (M + H) | 3 |
| 1840 | N-(cis-4-{[4-(dimethylamino)-5,6,7,8-tetrahydroquinazolin-2-yl] amino cyclohexyl)-N'-(3-methylphenyl)urea | 423 (M + H) | 3 |
| 1841 | N-(3-chloro-4-methylphenyl)-N'-(cis-4-{[4-(dimethylamino)-5,6,7,8-tetrahydroquinazolin-2-yl]amino}cyclohexyl) urea | 457 (M + H) | 3 |
| 1842 | 1-[4-(4-Dimethylamino-5,6,7,8-tetrahydro-quinazolin-2-ylamino)-cyclohexyl]-3-(1-naphthalen-1-yl-ethyl)-urea | 487 (M + H) | 3 |
| 1843 | N-[2-(difluoromethoxy)phenyl]-N'-(cis-4-{[4-(dimethylamino)-5,6,7,8-tetrahydroquinazolin-2-yl]amino}cyclohexyl)urea | 475 (M + H) | 3 |
| 1844 | methyl 2-({[(cis-4-{[4-(dimethylamino)-5,6,7,8-tetrahydro-quinazolin-2-yl]amino}cyclohexyl)amino]carbonyl}amino)-benzoate | 467 (M + H) | 3 |
| 1845 | N-(cis-4-{[4-(dimethylamino)-5,6,7,8-tetrahydroquinazolin-2-yl] amino}cyclohexyl)-N'-[2-(methylthio)phenyl]urea | 455 (M + H) | 3 |
| 1846 | N-(cis-4-{[4-(dimethylamino)-5,6,7,8-tetrahydroquinazolin-2-yl] amino}cyclohexyl)-N'-(2,4,5-trichlorophenyl)urea | 511 (M + H) | 2 |
| 1847 | N-(cis-4-{[4-(dimethylamino)-5,6,7,8-tetrahydroquinazolin-2-yl] amino}cyclohexyl)-N'-(2,4-dimethylphenyl)urea | 437 (M + H) | 3 |
| 1848 | N-(2,5-difluorophenyl)-N'-(cis-4-{[4-(dimethylamino)-5,6,7,8-tetrahydroquinazolin-2-yl]amino}cyclohexyl)urea | 445 (M + H) | 3 |
| 1849 | N-(2,5-dimethoxyphenyl)-N'-(cis-4-{[4-(dimethylamino)-5,6,7,8-tetrahydroquinazolin-2-yl]amino}cyclohexyl)urea | 469 (M + H) | 2 |
| 1850 | N-(cis-4-{[4-(dimethylamino)-5,6,7,8-tetrahydroquinazolin-2-yl] amino}cyclohexyl)-N'-,2, 5-dimethylphenyl) urea | 437 (M + H) | 3 |

(continued)

| Ex. No. | compound name | MS | class |
|---|---|---|---|
| 1851 | N-(2-benzylphenyl)-N'-(cis-4-{(4-(dimethylamino)-5,6,7,8-tetrahydroquinazolin-2-yl]amino}cyclohexyl)urea | 499 (M + H) | 3 |
| 1852 | N-(2-bromo-4,6-difluorophenyl)-N'-(cis-4-{[4-(dimethylamino)-5,6,7,8-tetrahydroquinazolin-2-yl]amino}cyclohexyl)urea | 523 (M + H) | 3 |
| 1853 | N-[2-chloro-4-(trifluoromethyl)phenyl]-N'-(cis-4-{[4-(dimethylamino)-5,6,7,8-tetrahydroquinazolin-2-yl]amino}cyclohexyl)-urea | 511 (M + H) | 3 |
| 1854 | N-(2-chloro-4-nitrophenyl)-N'-(cis-4-{[4-(dimethylamino)-,5,6,7,8-tetrahydroquinazolin-2-yl]amino}cyclohexyl)urea | 488 (M + H) | 3 |
| 1855 | N-[2-chloro-5-(trifluoromethyl)phenyl]-N'-(cis-4-{[4-(dimethylamino)-5,6,7,8-tetrahydroquinazolin-2-yl]amino}cyclohexyl)-urea | 511 (M + H) | 3 |
| 1856 | N-(2-chloro-5-methylphenyl)-N'-(cis-4-{[4-(dimethylamino)-5,6,7,8-tetrahydroquinazolin-2-yl]amino}cyclohexyl)urea | 457 (M + H) | 3 |
| 1857 | ethyl 2-({[(cis-4-{[4-(dimethylamino)-5,6,7,8-tetrahydro-quinazolin-2-yl]amino}cyclohexyl)amino]carbonyl}amino)-benzoate | 481 (M + H) | 3 |
| 1858 | N-(cis-4-{[4-(dimethylamino)-5,6,7,8-tetrahydroquinazolin-2-yl]amino}cyclohexyl)-N'-[2-fluoro-3-(trifluoromethyl)phenyl]-urea | 495 (M + H) | 3 |
| 1859 | N-(cis-4-{[4-(dimethylamino)-5,6,7,8-tetrahydroquinazolin-2-yl]amino}cyclohexyl)-N'-[2-fluoro-5-(trifluoromethyl)phenyl]-urea | 495 (M + H) | 3 |
| 1860 | N-(cis-4-{[4-(dimethylamino)-5,6,7,8-tetrahydroquinazolin-2-yl]amino}cyclohexyl)-N'-(2-fluoro-5-methylphenyl)urea | 441 (M + H) | 3 |
| 1861 | N-(cis-4-{[4-(dimethylamino)-5,6,7,8-tetrahydroquinazolin-2-yl]amino}cyclohexyl)-N'-(2-methoxy-4-nitrophenyl)urea | 484 (M + H) | 3 |
| 1862 | N-(cis-4-{[4-(dimethylamino)-5,6,7,8-tetrahydroquinazolin-2-yl]amino}cyclohexyl)-N'-(2-methoxy-5-nitrophenyl)urea | 484 (M + H) | 3 |
| 1863 | N-(cis-4-{[4-(dimethylamino)-5,6,7,8-tetrahydroquinazolin-2-yl]amino}cyclohexyl)-N'-2-naphthylurea | 459 (M + H) | 3 |
| 1864 | N-(cis-4-{[4-(dimethylamino)-5,6,7,8-tetrahydroquinazolin-2-yl]amino}cyclohexyl)-N'-(2-phenoxyphenyl)urea | 501 (M + H) | 3 |
| 1865 | N-(cis-4-{[4-(dimethylamino)-5,6,7,8-tetrahydroquinazolin-2-yl]amino}cyclohexyl)-N'-[3-(methylthio)phenyl]urea | 455 (M + H) | 3 |
| 1866 | N-(cis-4-{[4-(dimethylamino)-5,6,7,8-tetrahydroquinazolin-2-yl]amino}cyclohexyl)-N'- {3-[(trifluoromethyl)thio]phenyl}urea | 509 (M + H) | 3 |
| 1867 | N-(3,4-dichlorobenzyl)-N'-(cis-4-{[4-(dimethylamino)-5,6,7,8-tetrahydroquinazolin-2-yl]amino}cyclohexyl)urea | 491 (M + H) | 3 |
| 1868 | N-(3,5-dimethoxyphenyl)-N'-(cis-4-{[4-(dimethylamino)-5,6,7,8-tetrahydroquinazolin-2-yl]amino}cyclohexyl)urea | 469 (M + H) | 3 |
| 1869 | N-(cis-4-{[4-(dimethylamino)-5,6,7,8-tetrahydroquinazolin-2-yl]amino}cyclohexyl)-N'-(3,5-dimethylphenyl)urea | 437 (M + H) | 3 |
| 1870 | methyl 3-({[(cis-4-{[4-(dimethylamino)-5,6,7,8-tetrahydroquinazolin-2-yl]amino}cyclohexyl)amino]carbonyl}amino)-benzoate | 467 (M + H) | 3 |

(continued)

| Ex. No. | compound name | MS | class |
|---|---|---|---|
| 1871 | N-(cis-4-{[4-(dimethylamino)-5,6,7,8-tetrahydroquinazolin-2-yl] amino}cyclohexyl)-N'-(3-ethylphenyl)urea | 437 (M + H) | 3 |
| 1872 | N-(cis-4-{[4-(dimethylamino)-5,6,7,8-tetrahydroquinazolin-2-yl] amino}cyclohexyl)-N'-[3-fluoro-5-(trifluoromethyl)phenyl]-urea | 495 (M + H) | 3 |
| 1873 | N-(cis-4-{[4-(dimethylamino)-5,6,7,8-tetrahydroquinazolin-2-yl] amino)cyclohexyl)-N'-(3-fluorobenzyl)urea | 441 (M + H) | 3 |
| 1874 | N-(cis-4-{[4-(dimethylamino)-5,6,7,8-tetrahydroquinazolin-2-yl] amino}cyclohexyl)-N'-(3-nitrophenyl)urea | 454 (M + H) | 3 |
| 1875 | N-(cis-4-{[4-(dimethylamino)-5,6,7,8-tetrahydroquinazolin-2-yl] amino}cyclohexyl)-N'-(3-phenoxyphenyl)urea | 501 (M + H) | 3 |
| 1876 | N-[4-(difluoromethoxy)phenyl]-N'-(cis-4-{[4-(dimethylamino)-5,6,7,8-tetrahydroquinazolin-2-yl]amino}cyclohexyl)urea | 475 (M + H) | 3 |
| 1877 | butyl 4-({[(cis-4-{[4-(dimethylamino)-5,6,7,8-tetrahydroquinazolin-2-yl]amino}cyclohexyl)amino]carbonyl}amino)-benzoate | 509 (M + H) | 3 |
| 1878 | N-(cis-4-{[4-(dimethylamino)-5,6,7,8-tetrahydroquinazolin-2-yl] amino}cyclohexyl)-N'-[4-(trifluoromethyl)phenyl]urea | 477 (M + H) | 3 |
| 1879 | N-(cis-4-{[4-(dimethylamino)-5,6,7,8-tetrahydroquinazolin-2-yl] amino}cyclohexyl)-N'-{4-[(trifluoromethyl)thio]phenyl}urea | 509 (M + H) | 3 |
| 1880 | N-(cis-4-{[4-(dimethylamino)-5,6,7,8-tetrahydroquinazolin-2-yl] amino}cyclohexyl)-N'-(4,5-dimethyl-2-nitrophenyl)urea | 482 (M + H) | 3 |
| 1881 | N-[4-(benzyloxy)phenyl]-N'-(cis-4-{[4-(dimethylamino)-5,6,7,8-tetrahydroquinazolin-2-yl]amino}cyclohexyl)urea | 515 (M + H) | 3 |
| 1882 | N-(4-benzylphenyl)-N'-(cis-4-{[4-(dimethylamino)-5,6,7,8-tetrahydroquinazolin-2-yl]amino}cyclohexyl)urea | 499 (M + H) | 3 |
| 1883 | N-(4-bromo-2-chlorophenyl)-N'-(cis-4-{[4-(dimethylamino)-5,6,7,8-tetrahydroquinazolin-2-yl]amino}cyclohexyl)urea | 521 (M + H) | 2 |
| 1884 | N-(4-bromo-2-fluorophenyl)-N'-(cis-4-{[4-(dimethylamino)-5,6,7,8-tetrahydroquinazolin-2-yl]amino}cyclohexyl)urea | 505 (M + H) | 3 |
| 1885 | N-(4-bromo-3-methylphenyl)-N'-(cis-4-{[4-(dimethylamino)-5,6,7,8-tetrahydroquinazolin-2-yl]amino}cyclohexyl)urea | 501 (M + H) | 3 |
| 1886 | N-(4-chloro-2-nitrophenyl)-N'-(cis-4-{[4-(dimethylamino)-5,6,7,8-tetrahydroquinazolin-2-yl]amino}cyclohexyl)urea | 488 (M + H) | 3 |
| 1887 | N-[4-chloro-3-(trifluoromethyl)phenyl]-N'-(cis-4-{ [4-(dimethylamino)-5,6,7,8-tetrahydroquinazolin-2-yl]amino} cyclohexyl)urea | 511 (M + H) | 3 |
| 1888 | N-(cis-4-{[4-(dimethylamino)-5,6,7,8-tetrahydroquinazolin-2-yl] amino}cyclohexyl)-N'-(4-ethoxyphenyl)urea | 453 (M + H) | 3 |
| 1889 | N-(cis-4-{[4-(dimethylamino)-5,6,7,8-tetrahydroquinazolin-2-yl] amino}cyclohexyl)-N'-(4-fluoro-2-nitrophenyl)urea | 472 (M + H) | 3 |
| 1890 | N-(cis-4{[4-(dimethylamino)-5,6,7,8-tetrahydroquinazolin-2-yl] amino}cyclohexyl)-N'-[4-fluoro-3-(trifluoromethyl)phenyl]-urea | 495 (M + H) | 3 |
| 1891 | N-(cis-4-{[4-(dimethylamino)-5,6,7,8-tetrahydroquinazolin-2-yl] amino}cyclohexyl)-N'-[4-(heptyloxy)phenyl]urea | 523 (M + H) | 3 |
| 1892 | N-(cis-4-{[4-(dimethylamino)-5,6,7,8-tetrahydroquinazolin-2-yl] amino}cyclohexyl)-N'-(4-iodophenyl)urea | 535 (M + H) | 3 |

(continued)

| Ex. No. | compound name | MS | class |
|---|---|---|---|
| 1893 | N-(cis-4-{[4-(dimethylamino)-5,6,7,8-tetrahydroquinazolin-2-yl] amino}cyclohexyl)-N'-(4-methoxy-2-nitrophenyl)urea | 484 (M + H) | 3 |
| 1894 | N-(cis-4-{[4-(dimethylamino)-5,6,7,8-tetrahydroquinazolin-2-yl] amino}cyclohexyl)-N'-(4-methyl-3-nitrophenyl)urea | 468 (M + H) | 3 |
| 1895 | N-(cis-4-{[4-(dimethylamino)-5,6,7,8-tetrahydroquinazolin-2-yl] amino}cyclohexyl)-N'-(4-methylbenzyl)urea | 437 (M + H) | 3 |
| 1896 | N-(4-butoxyphenyl)-N'-(cis-4-{[4-(dimethylamino)-5,6,7,8-tetrahydroquinazolin-2-yl]amino}cyclohexyl)urea | 481 (M + H) | 3 |
| 1897 | N-(4-butylphenyl)-N'-(cis-4-{[4-(dimethylamino)-5,6,7,8-tetrahydroquinazolin-2-yl]amino}cyclohexyl)urea | 465 (M + H) | 3 |
| 1898 | N-biphenyl-4-yl-N'-(cis-4-{[4-(dimethylamino)-5,6,7,8-tetrahydroquinazolin-2-yl]amino}cyclohexyl)urea | 485 (M + H) | 3 |
| 1899 | N-(5-chloro-2-methoxyphenyl)-N'-(cis-4-{[4-(dimethylamino)-5,6,7,8-tetrahydroquinazolin-2-yl]amino)}cyclohexyl)urea | 473 (M + H) | 3 |
| 1900 | N-(5-chloro-2-methylphenyl)-N'-(cis-4-{[4-(dimethylamino)-5,6,7,8-tetrahydroquinazolin-2-yl]amino}cyclohexyl)urea | 457 (M + H) | 3 |
| 1901 | N-(5-chloro-2-nitrophenyl)-N'-(cis-4-{[4-(dimethylamino)-5,6,7,8-tetrahydroquinazolin-2-yl]amino}cyclohexyl)urea | 488 (M + H) | 3 |
| 1902 | N-(cis-4-{[4-(dimethylamino)-5,6,7,8-tetrahydroquinazolin-2-yl] amino}cyclohexyl)-N'-(5-fluoro-2-methylphenyl)urea | 441 (M + H) | 3 |
| 1903 | N-(2,3-dihydro-1H-inden-5-yl)-N'-(cis-4-{[4-(dimethylamino)-5,6,7,8-tetrahydroquinazolin-2-yl]amino}cyclohexyl)urea | 449 (M + H) | 3 |
| 1904 | N-(cis-4-{[4-(dimethylamino)-5,6,7,8-tetrahydroquinazolin-2-yl] amino}cyclohexyl)-N'-9H-fluoren-2-ylurea | 497 (M + H) | 3 |
| 1905 | N-(cis-4-{[4-(dimethylamino)-5,6,7,8-tetrahydroquinazolin-2-yl] amino}cyclohexyl)-N'-9H-fluoren-9-ylurea | 497 (M + H) | 3 |
| 1906 | N-(cis-4-{[4-(dimethylamino)-5,6,7,8-tetrahydroquinazolin-2-yl] amino}cyclohexyl)-N'-(2-phenylethyl)urea | 437 (M + H) | 3 |
| 1907 | N-cyclopentyl-N'-(cis-4-{[4-(dimethylamino)-5,6,7,8-tetrahydroquinazolin-2-yl]amino}cyclohexyl)urea | 401 (M + H) | 3 |
| 1908 | methyl 4-({[(cis-4-{(4-(dimethylamino)-5,6,7,8-tetrahydroquinazolin-2-yl]amino}cyclohexyl)amino] carbonyl}amino)-benzoate | 467 (M + H) | 3 |
| 1909 | N-(cis-4-{[4-(dimethylamino)-5,6,7,8-tetrahydroquinazolin-2-yl] amino}cyclohexyl)-N'-[2-(trifluoromethoxy)phenyl)urea | 493 (M + H) | 2 |
| 1910 | butyl 2-({[(cis-4-([4-(dimethylamino)-5,6,7,8-tetrahydro-quinazolin-2-yl]amino}cyclohexyl)amino]carbonyl}amino)-benzoate | 509 (M + H) | 3 |
| 1911 | dimethyl 5-({[(cis-4-{[4-(dimethylamino)-5,6,7,8-tetrahydroquinazolin-2-yl]amino}cyclohexyl)amino] carbonyl}amino)-isophthalate | 525 (M + H) | 3 |
| 1912 | N-(cis-4-{[4-(dimethylamino)-5,6,7,8-tetrahydroquinazolin-2-yl] amino}cyclohexyl)-N'-(4-(trifluoromethoxy)phenyl]urea | 493 (M + H) | 3 |

(continued)

| Ex. No. | compound name | MS | class |
|---|---|---|---|
| 1913 | N-(cis-4-{[4-(dimethylamino)-5,6,7,8-tetrahydroquinazolin-2-yl]amino}cyclohexyl)-N'-(2,2,4,4-tetrafluoro-4H-1,3-benzodioxin-6-yl)urea | 539 (M + H) | 3 |
| 1914 | N-(cis-4-{[4-(dimethylamino)-5,6,7,8-tetrahydroquinazolin-2-yl]amino}cyclohexyl)-N'-[2-(2-thienyl)ethyl]urea | 443 (M + H) | 3 |
| 1915 | N-(2-cyanophenyl)-N'-(cis-4-{[4-(dimethylamino)-5,6,7,8-tetrahydroquinazolin-2-yl]amino}cyclohexyl)urea | 434 (M + H) | 3 |
| 1916 | N-(cis-4-{[4-(dimethylamino)-5,6,7,8-tetrahydroquinazolin-2-yl]amino}cyclohexyl)-N'-2-thienylurea | 415 (M + H) | 3 |
| 1917 | N-(cis-4-{[4-(dimethylamino)-5,6,7,8-tetrahydroquinazolin-2-yl]amino}cyclohexyl)-N'-3-thienylurea | 415 (M + H) | 3 |
| 1918 | N-(4-tert-butylphenyl)-N'-(cis-4-{[4-(dimethylamino)-5,6,7,8-tetrahydroquinazolin-2-yl]amino}cyclohexyl)urea | 465 (M + H) | 3 |
| 1919 | N-(cis-4-{[4-(dimethylamino)-5,6,7,8-tetrahydroquinazolin-2-yl]amino}cyclohexyl)-N'-(5-phenyl-2-thienyl)urea | 491 (M + H) | 3 |
| 1920 | N-(cis-4-{[4-(dimethylamino)-5,6,7,8-tetrahydroquinazolin-2-yl]amino}cyclohexyl)-N'-(6-fluoro-4H-1,3-benzodioxin-8-yl)urea | 485 (M + H) | 3 |
| 1921 | benzyl 4-({[(cis-4-{[4-(dimethylamino)-5,6,7,8-tetrahydro quinazolin-2-yl]amino}cyclohexyl)amino]carbonyl}amino)-piperidine-1-carboxylate | 550 (M + H) | 3 |
| 1922 | N-[4-(dimethylamino)phenyl]-N'-(cis-4-{[4-(dimethylamino)-5,6,7,8-tetrahydroquinazolin-2-yl]amino)cyclohexyl)urea | 452 (M + H) | 3 |
| 1923 | N-(2,6-dichloropyridin-4-yl)-N'-(cis-4-{[4-(dimethylamino}-5,6,7,8-tetrahydroquinazolin-2-yl]amino}cyclohexyl)urea | 478 (M + H) | 3 |
| 1924 | N-(cis-4-{[4-(dimethylamino)-5,6,7,8-tetrahydroquinazolin-2-yl]amino}cyclohexyl)-N'-(3,5-dimethylisoxazol-4-yl)urea | 428 (M + H) | 3 |
| 1925 | N-(3-acetylphenyl)-N'-(cis-4-{[4-(dimethylamino)-5,6,7,8-tetrahydroquinazolin-2-yl]amino}cyclohexyl)thiourea | 467 (M + H) | 3 |
| 1926 | N-(4-acetylphenyl)-N'-(cis-4-{[4-(dimethylamino)-5,6,7,8-tetrahydroquinazolin-2-yl]amino}cyclohexyl)thiourea | 465 (M - H) | 3 |
| 1927 | N-[3,5-bis(trifluoromethyl)phenyl]-N'-(cis-4-{[4-(dimethylamino)-5,6,7,8-tetrahydroquinazolin-2-yl]amino}cyclohexyl)-thiourea | 561 (M + H) | 3 |
| 1928 | N-benzyl-N'-(cis-4-}[4-(dimethylamino)-5,6,7,8-tetrahydroquinazolin-2-yl]amino}cyclohexyl)thiourea | 439 (M + H) | 3 |
| 1929 | N-(3-bromophenyl)-N'-(cis-4-{[4-(dimethylamino)-5,6,7,8-tetrahydroquinazolin-2-yl]amino}cyclohexyl)thiourea | 503 (M + H) | 3 |
| 1930 | N-butyl-N'-(cis-4-{[4-(dimethylamino)-5,6,7,8-tetrahydroquinazolin-2-yl]amino}cyclohexyl)thiourea | 405 (M + H) | 3 |
| 1931 | N-cyclohexyl-N'-(cis-4-{[4-(dimethylamino)-5,6,7,8-tetrahydroquinazolin-2-yl]amino}cyclohexyl)thiourea | 431 (M + H) | 3 |
| 1932 | N-cyclopentyl-N'-(cis-4-{[4-(dimethylamino)-5,6,7,8-tetrahydroquinazolin-2-yl]amino}cyclohexyl)thiourea | 417 (M + H) | 3 |
| 1933 | N-(3-chlorophenyl)-N'-(cis-4-{[4-(dimethylamino)-5,6,7,8-tetrahydroquinazolin-2-yl]amino}cyclohexyl)thiourea | 459 (M + H) | 3 |

(continued)

| Ex. No. | compound name | MS | class |
|---|---|---|---|
| 1934 | N-(4-chlorophenyl)-N'-(cis-4-{[4-(dimethylamino)-5,6,7,8-tetrahydroquinazolin-2-yl]amino}cyclohexyl)thiourea | 459 (M + H) | 3 |
| 1935 | N-(2,5-difluorophenyl-N'-(cis-4-{[4-(dimethylamino)-5,6,7,8-tetrahydroquinazolin-2-yl]amino}cyclohexyl)thiourea | 461 (M + H) | 3 |
| 1936 | N-(2,5-dichlorophenyl)-N'-(cis-4-{[4-(dimethylamino)-5,6,7,8-tetrahydroquinazolin-2-yl]amino}cyclohexyl)thiourea | 493 (M + H) | 3 |
| 1937 | N-(3,4-dichlorophenyl)-N'-(cis-4-{[4-(dimethylamino)-5,6,7,8-tetrahydroquinazolin-2-yl]amino}cyclohexyl)thiourea | 493 (M + H) | 3 |
| 1938 | N-(2,6-dichlorophenyl)-N'-(cis-4-{[4-(dimethylamino)-5,6,7,8-tetrahydroquinazolin-2-yl]amino}cyclohexyl)thiourea | 493 (M + H) | 3 |
| 1939 | N-(cis-4-{[4-(dimethylamino)-5,6,7,8-tetrahydroquinazolin-2-yl]amino}cyclohexyl)-N'-(4-ethoxyphenyl)thiourea | 469 (M + H) | 3 |
| 1940 | N-(cis-4-{[4-(dimethylamino)-5,6,7,8-tetrahydroquinazolin-2-yl]amino}cyclohexyl)-N'-(2-furylmethyl)thiourea | 429 (M + H) | 3 |
| 1941 | N-(cis-4-{[4-(dimethylamino)-5,6,7,8-tetrahydroquinazolin-2-yl]amino}cyclohexyl)-N'-(4-fluorophenyl)thiourea | 443 (M + H) | 3 |
| 1942 | N-(cis-4-{[4-(dimethylamino)-5,6,7,8-tetrahydroquinazolin-2-yl]amino}cyclohexyl)-N'-hexylthiourea | 433 (M + H) | 3 |
| 1943 | N-(cis-4-{[4-(dimethylamino)-5,6,7,8-tetrahydroquinazolin-2-yl]amino}cyclohexyl)-N'-[4-(trans-4-propylcyclohexyl)phenyl]-thiourea | 549 (M + H) | 3 |
| 1944 | N-(cis-4-{[4-(dimethylamino)-5,6,7,8-tetrahydroquinazolin-2-yl]amino}cyclohexyl)-N'-isobutylthiourea | 405 (M + H) | 3 |
| 1945 | N-(cis-4-{[4-(dimethylamino)-5,6,7,8-tetrahydroquinazolin-2-yl]amino}cyclohexyl)-N'-(4-methoxybiphenyl-3-yl)thiourea | 531 (M + H) | 3 |
| 1946 | N-(1,3-benzodioxol-5-ylmethyl)-N'-(cis-4-{[4-(dimethylamino)-5,6,7,8-tetrahydroquinazolin-2-yl]amino}cyclohexyl)thiourea | 483 (M + H) | 3 |
| 1947 | N-(cis-4-{[4-(dimethylamino)-5,6,7,8-tetrahydroquinazolin-2-yl]amino}cyclohexyl)-N'-(3-methylphenyl)thiourea | 439 (M + H) | 3 |
| 1948 | N-(cis-4-{[4-(dimethylamino)-5,6,7,8-tetrahydroquinazolin-2-yl]amino}cyclohexyl)-N'-[4-(methylthio)phenyl]thiourea | 471 (M + H) | 3 |
| 1949 | N-(cis-4-{[4-(dimethylamino)-5,6,7,8-tetrahydroquinazolin-2-yl]amino}cyclohexyl)-N'-(4-methoxyphenyl)thiourea | 453 (M - H) | 3 |
| 1950 | N-(cis-4-{[4-(dimethylamino)-5,6,7,8-tetrahydroquinazolin-2-yl]amino}cyclohexyl)-N'-(2-methylprop-2-en-1-yl)thiourea | 403 (M + H) | 3 |
| 1951 | N-(cis-4-{[4-(dimethylamino)-5,6,7,8-tetrahydroquinazolin-2-yl]amino}cyclohexyl)-N'-methylthiourea | 363 (M + H) | 3 |
| 1952 | N-(cis-4-{[4-(dimethylamino)-5,6,7,8-tetrahydroquinazolin-2-yl]amino}cyclohexyl)-N'-(3-nitrophenyl)thiourea | 470 (M + H) | 3 |
| 1953 | N-(cis-4-{[4-(dimethylamino)-5,6,7,8-tetrahydroquinazolin-2-yl]amino}cyclohexyl)-N'-(4-nitrophenyl)thiourea | 470 (M + H) | 3 |
| 1954 | N-(cis-4-{[4-(dimethylamino)-5,6,7,8-tetrahydroquinazolin-2-yl]amino}cyclohexyl)-N'-(1,1,3,3-tetramethylbutyl)thiourea | 461 (M + H) | 3 |
| 1955 | N-(cis-4-{[4-(dimethylamino)-5,6,7,8-tetrahydroquinazolin-2-yl]amino}cyclohexyl)-N'-phenylthiourea | 425 (M + H) | 3 |

(continued)

| Ex. No. | compound name | MS | class |
|---|---|---|---|
| 1956 | N-(cis-4-{[4-(dimethylamino)-5,6,7,8-tetrahydroquinazolin-2-yl] amino}cyclohexyl)-N'-propylthiourea | 391 (M + H) | 3 |
| 1957 | N-(cis-4-{[4-(dimethylamino)-5,6,7,8-tetrahydroquinazolin-2-yl] amino}cyclohexyl)-N'-[3-(trifluoromethyl)phenyl]thiourea | 493 (M + H) | 3 |
| 1958 | N-(cis-4-{[4-(dimethylamino)-5,6,7,8-tetrahydroquinazolin-2-yl] amino}cyclohexyl)-N'-(tetrahydrofuran-2-ylmethyl)thiourea | 433 (M + H) | 3 |
| 1959 | N-(cis-4-{[4-(dimethylamino)-5,6,7,8-tetrahydroquinazolin-2-yl] amino}cyclohexyl)-N'-(4-methylphenyl)thiourea | 439 (M + H) | 3 |
| 1960 | N-(cis-4-{[4-(dimethylamino)-5,6,7,8-tetrahydroquinazolin-2-yl] amino}cyclohexyl)-N'-(2-methylphenyl)thiourea | 439 (M + H) | 3 |
| 1961 | N-(tert-butyl)-N'-(cis-4-{[4-(dimethylamino)-5,6,7,8-tetrahydroquinazolin-2-yl]amino}cyclohexyl)thiourea | 405 (M + H) | 3 |
| 1962 | N-1-adamantyl-N'-(cis-4-{[4-(dimethylamino)-5,6,7,8-tetrahydroquinazolin-2-yl]amino}cyclohexyl)thiourea | 483 (M + H) | 3 |
| 1963 | N-(2-bromophenyl)-N'-(cis-4-{[4-(dimethylamino)-5,6,7,8-tetrahydroquinazolin-2-yl]amino}cyclohexyl)thiourea | 503 (M + H) | 3 |
| 1964 | N-(2-chlorophenyl)-N'-(cis-4-{[4-(dimethylamino)-5,6,7,8-tetrahydroquinazolin-2-yl]amino}cyclohexyl)thiourea | 459 (M + H) | 3 |
| 1965 | N-(cis-4-{[4-(dimethylamino)-5,6,7,8-tetrahydroquinazolin-2-yl] amino}cyclohexyl)-N'-(2-phenylethyl)thiourea | 453 (M + H) | 3 |
| 1966 | N-(cis-4-{[4-(dimethylamino)-5,6,7,8-tetrahydroquinazolin-2-yl] amino}cyclohexyl)-N'-(4-ethylphenyl)thiourea | 453 (M + H) | 3 |
| 1967 | N-(cis-4-{[4-(dimethylamino)-5,6,7,8-tetrahydroquinazolin-2-yl] amino}cyclohexyl)-N'-[2-(methylthio)phenyl]thiourea | 471 (M + H) | 3 |
| 1968 | N-(cis-4-{[4-(dimethylamino)-5,6,7,8-tetrahydroquinazolin-2-yl] amino}cyclohexyl)-N'-(2-(trifluoromethoxy)phenyl]thiourea | 509 (M + H) | 3 |
| 1969 | N-(cis-4-{[4-(dimethylamino)-5,6,7,8-tetrahydroquinazolin-2-yl] amino}cyclohexyl)-N'-[2-(trifluoromethyl)phenyl]thiourea | 493 (M + H) | 3 |
| 1970 | N-(cis-4-{[4-(dimethylamino)-5,6,7,8-tetrahydroquinazolin-2-yl] amino}cyclohexyl)-N'-(2,3,4-trifluorophenyl)thiourea | 479 (M + H) | 3 |
| 1971 | N-(2,3-dichlorophenyl)-N'-(cis-4-{[4-(dimethylamino)-5,6,7,8-tetrahydroquinazolin-2-yl]amino}cyclohexyl)thiourea | 493 (M + H) | 3 |
| 1972 | N-(2,4-difluorophenyl)-N'-(cis-4-{[4-(dimethylamino)-5,6,7,8-tetrahydroquinazolin-2-yl]amino}cyclohexyl)thiourea | 461 (M + H) | 3 |
| 1973 | N-(2,5-dimethoxyphenyl)-N'-(cis-4-{[4-(dimethylamino)-5,6,7,8-tetrahydroquinazolin-2-yl]amino}cyclohexyl)thiourea | 485 (M + H) | 3 |
| 1974 | N-(2,6-difluorophenyl)-N'-(cis-4-{[4-(dimethylamino)-5,6,7,8-tetrahydroquinazolin-2-yl]amino}cyclohexyl)thiourea | 461 (M + H) | 3 |
| 1975 | N-(2-chloro-4-nitrophenyl)-N'-(cis-4-{[4-(dimethylamino)-5,6,7,8-tetrahydroquinazolin-2-yl]amino}cyclohexyl)thiourea | 504 (M + H) | 3 |
| 1976 | N-[2-(difluoromethoxy)phenyl]-N'-(cis-4-{[4-(dimethylamino)-5,6,7,8-tetrahydroquinazolin-2-yl]amino}cyclohexyl)thiourea | 491 (M + H) | 3 |
| 1977 | N-(cis-4-{[4-(dimethytamino)-5,6,7,8-tetrahydroquinazolin-2-yl] amino}cyclohexyl)-N'-[2-fluoro-5-(trifluoromethyl)phenyl]thiourea | 511 (M + H) | 3 |

(continued)

| Ex. No. | compound name | MS | class |
|---|---|---|---|
| 1978 | N-(cis-4-{[4-(dimethylamino)-5,6,7,8-tetrahydroquinazolin-2-yl] amino }cyclohexyl)-N'-(2-fluorophenyl)thiourea | 443 (M + H) | 3 |
| 1979 | N-(cis-4-{[4-(dimethylamino)-5,6,7,8-tetrahydroquinazolin-2-yl] amino}cyclohexyl)-N'-(2-iodophenyl)thiourea | 551 (M + H) | 3 |
| 1980 | N-(cis-4-{[4-(dimethylamino)-5,6,7,8-tetrahydroquinazolin-2-yl] amino}cyclohexyl)-N'-{3-[(trifluoromethyl)thio]phenyl}thiourea | 525 (M + H) | 3 |
| 1981 | N-(3,5-dichlorophenyl)-N'-(cis-4-{[4-(dimethylamino)-5,6,7,8-tetrahydroquinazolin-2-yl]amino}cyclohexyl)thiourea | 493 (M + H) | 3 |
| 1982 | N-(3,5-difluorophenyl)-N'-(cis-4-{[4-(dimethylamino)-5,6,7,8-tetrahydroquinazolin-2-yl]amino}cyclohexyl)thiourea | 461 (M + H) | 3 |
| 1983 | N-(3-cyanophenyl)-N'-(cis-4-{[4-(dimethylamino)-5,6,7,8-tetrahydroquinazolin-2-yl]amino}cyclohexyl)thiourea | 450 (M + H) | 3 |
| 1984 | N-(cis-4-{[4-(dimethylamino)-5,6,7,8-tetrahydroquinazolin-2-yl] amino}cyclohexyl)-N'-(3-fluorophenyl)thiourea | 443 (M + H) | 3 |
| 1985 | N-(cis-4-{[4-(dimethylamino)-5,6,7,8-tetrahydroquinazolin-2-yl] amino}cyclohexyl)-N'-(3-iodophenyl)thiourea | 551 (M + H) | 3 |
| 1986 | N-(cis-4-{[4-(dimethylamino)-5,6,7,8-tetrahydroquinazolin-2-yl] amino}cyclohexyl)-N'-(3-methoxyphenyl)thiourea | 455 (M + H) | 3 |
| 1987 | N-[4-(difluoromethoxy)phenyl]-N'-(cis-4-{[4-(dimethylamino)-5,6,7,8-tetrahydroquinazolin-2-yl]amino}cyclohexyl)thiourea | 491 (M + H) | 3 |
| 1988 | N-(cis-4-{[4-(dimethylamino)-5,6,7,8-tetrahydroquinazolin-2-yl] amino}cyclohexyl)-N'-[4-(trifluoromethoxy)phenyl]thiourea | 509 (M + H) | 3 |
| 1989 | N-(cis-4-{[4-(dimethylamino)-5,6,7,8-tetrahydroquinazolin-2-yl] amino}cyclohexyl)-N'-[4-(trifluoromethyl)phenyl]thiourea | 493 (M + H) | 3 |
| 1990 | N-(cis-4-{[4-(dimethylamino)-5,6,7,8-tetrahydroquinazolin-2-yl] amino}cyclohexyl)-N'-{4-[(trifluoromethyl)thio]phenyl}thiourea | 525 (M + H) | 3 |
| 1991 | N-(4-bromo-2-fluorophenyl)-N'-(cis-4-{[4-(dimethylamino)-5,6,7,8-tetrahydroquinazolin-2-yl]amino}cyclohexyl)thiourea | 520 (M) | 3 |
| 1992 | N-[4-chloro-3-(trifluoromethyl)phenyl]-N'-(cis-4-{ [4-(dimethylamino)-5,6,7,8-tetrahydroquinazolin-2-yl]amino} cyclohexyl)thiourea | 527 (M + H) | 3 |
| 1993 | N-(cis-4-{[4-(dimethylamino)-5,6,7,8-tetrahydroquinazolin-2-yl] amino}cyclohexyl)-N'-[4-fluoro-3-(trifluoromethyl)phenyl]thiourea | 511 (M + H) | 3 |
| 1994 | N-(5-chloro-2-methylphenyl)-N'-(cis-4-{[4-(dimethylamino)-5,6,7,8-tetrahydroquinazolin-2-yl]amino}cyclohexyl)thiourea | 473 (M + H) | 3 |
| 1995 | N-bicyclo[2.2.1]hept-2-yl-N'-(cis-4-{[4-(dimethylamino)-5,6,7,8-tetrahydroquinazolin-2-yl]amino}cyclohexyl)thiourea | 443 (M + H) | 3 |
| 1996 | tert-butyl [4-({[(cis-4-{[4-(dimethylamino)-5,6,7,8-tetrahydro-quinazolin-2-yl]amino}cyclohexyl)amino]carbonothioyl}amino) phenyl]carbamate | 540 (M + H) | 3 |
| 1997 | N-[2-(3,4-dimethoxyphenyl)ethyl]-N'-(cis-4-{[4-(dimethylamino)-5,6,7,8-tetrahydroquinazolin-2-yl]amino}cyclohexyl)thiourea | 513 (M + H) | 3 |
| 1998 | N-[2-(4-chlorophenyl)ethyl]-N'-(cis-4-{[4-(dimethylamino)-5,6,7,8-tetrahydroquinazolin-2-yl]amino}cyclohexyl)thiourea | 487 (M + H) | 3 |

(continued)

| Ex. No. | compound name | MS | class |
|---|---|---|---|
| 1999 | N-(cis-4-{[4-(dimethylamino)-5,6,7,8-tetrahydroquinazolin-2-yl] amino}cyclohexyl)-N'-(2,3,4,5-tetrachlorophenyl)thiourea | 561 (M + H) | 3 |
| 2000 | N-(cis-4-{[4-(dimethylamino)-5,6,7,8-tetrahydroquinazolin-2-yl] amino}cyclohexyl)-N'-(2,4,5-trichlorophenyl)thiourea | 527 (M + H) | 3 |
| 2001 | N-(cis-4-{[4-(dimethylamino)-5,6,7,8-tetrahydroquinazolin-2-yl] amino}cyclohexyl)-N'-(2,4,6-trifluorophenyl)thiourea | 479 (M + H) | 3 |
| 2002 | N-(2,6-diisopropylphenyl)-N'-(cis-4-([4-(dimethylamino)-5,6,7,8-tetrahydroquinazolin-2-yl]amino}cyclohexyl)thiourea | 509 (M + H) | 3 |
| 2003 | N-[2-chloro-5-(trifluoromethyl)phenyl)-N'-(cis-4-{ [4-(dimethylamino)-5,6,7,8-tetrahydroquinazolin-2-yl]amino} cyclohexyl)thiourea | 527 (M + H) | 3 |
| 2004 | N-(cis-4-{[4-(dimethylamino)-5,6,7,8-tetrahydroquinazolin-2-yl] amino}cyclohexyl)-N'-[3-(methylthio)phenyl]thiourea | 471 (M + H) | 3 |
| 2005 | N-(3,4-dichlorobenzyl)-N'-(cis-4-{[4-(dimethylamino)-5,6,7,8-tetrahydroquinazolin-2-yl]amino}cyclohexyl)thiourea | 507 (M + H) | 3 |
| 2006 | N-(3,5-dimethoxyphenyl)-N'-(cis-4-{[4-(dimethylamino)-5,6,7,8-tetrahydroquinazolin-2-yl]amino}cyclohexyl)thiourea | 485 (M + H) | 3 |
| 2007 | N-(cis-4-{[4-(dimethylamino)-5,6,7,8-tetrahydroquinazolin-2-yl] amino}cyclohexyl)-N'-(3,5-dimethylphenyl)thiourea | 453 (M + H) | 3 |
| 2008 | N-[3-(benzyloxy)phenyl]-N'-(cis-4-{[4-(dimethylamino)-5,6,7,8-tetrahydroquinazolin-2-yl]amino}cyclohexyl)thiourea | 531 (M + H) | 3 |
| 2009 | 3-({[(cis-4-{[4-(dimethylamino)-5,6,7,8-tetrahydroquinazolin-2-yl] amino}cyclohexyl)amino]carbonothioyl}amino)benzoic acid | 469 (M + H) | 3 |
| 2010 | N-(3-chloro-4-methylphenyl)-N'-(cis-4-{[4-(dimethylamino)-5,6,7,8-tetrahydroquinazolin-2-yl]amino}cyclohexyl)thiourea | 473 (M + H) | 3 |
| 2011 | N-(cis-4-{[4-(dimethylamino)-5,6,7,8-tetrahydroquinazolin-2-yl] amino}cyclohexyl)-N'-(3-phenylpropyl)thiourea | 467 (M + H) | 3 |
| 2012 | N-[4-(diethylamino)phenyl]-N'-(cis-4-{[4-(dimethylamino)-5,6,7,8-tetrahydroquinazolin-2-yl]amino}cyclohexyl)thiourea | 496 (M + H) | 3 |
| 2013 | ethyl 4-({[(cis-4-{[4-(dimethylamino)-5,6,7,8-tetrahydroquinazolin-2-yl]amino}cyclohexyl)amino]carbonothioyl}amino)benzoate | 497 (M + H) | 3 |
| 2014 | N-(cis-4-{[4-(dimethylamino)-5,6,7,8-tetrahydroquinazolin-2-yl] amino}cyclohexyl)-N'-[1-(4-fluorophenyl)ethyl]thiourea | 471 (M + H) | 3 |
| 2015 | N-(cis-4-{[4-(dimethylamino)-5,6,7,8-tetrahydroquinazolin-2-yl] amino}cyclohexyl)-N'-(4-fluorobenzyl)thiourea | 457 (M + H) | 3 |
| 2016 | N-(cis-4-{[4-(dimethylamino)-5,6,7,8-tetrahydroquinazolin-2-yl] amino}cyclohexyl)-N'-(4-isopropylphenyl)thiourea | 466 (M) | 3 |
| 2017 | N-(cis-4-{[4-(dimethylamino)-5,6,7,8-tetrahydroquinazolin-2- yl] amino}cyclohexyl)-N'-(4-methoxy-2-nitrophenyl)thiourea | 500 (M + H) | 3 |
| 2018 | N-(cis-4-{[4-(dimethylamino)-5,6,7,8-tetrahydroquinazolin-2-yl] amino}cyclohexyl)-N'-(4-methoxybenzyl)thiourea | 469 (M + H) | 3 |
| 2019 | methyl 4-({[(cis-4-{[4-(dimethylamino)-5,6,7,8-tetrahydroquinazolin-2-yl]amino}cyclohexyl)amino] carbonothioyl}amino)benzoate | 483 (M + H) | 3 |

(continued)

| Ex. No. | compound name | MS | class |
|---|---|---|---|
| 2020 | N-(cis-4-{[4-(dimethylamino)-5,6,7,8-tetrahydroquinazolin-2-yl] amino}cyclohexyl)-N'-(4-methyl-2-nitrophenyl)thiourea | 484 (M + H) | 3 |
| 2021 | N-(cis-4-{[4-(dimethylamino)-5,6,7,8-tetrahydroquinazolin-2-yl] amino}cyclohexyl)-N'-(4-methylbenzyl)thiourea | 453 (M + H) | 3 |
| 2022 | N-(4-butylphenyl)-N'-(cis-4-{[4-(dimethylamino)-5,6,7,8-tetrahydroquinazolin-2-yl]amino}cyclohexyl)thiourea | 481 (M + H) | 3 |
| 2023 | N-(5-chloro-2-methoxyphenyl)-N'-(cis-4-{[4-(dimethylamino)-5,6,7,8-tetrahydroquinazolin-2-yl]amino}cyclohexyl)thiourea | 489 (M + H) | 3 |
| 2024 | N-(cis-4-{[4-(dimethylamino)-5,6,7,8-tetrahydroquinazolin-2-yl] amino}cyclohexyl)-N'-(1-phenylethyl)thiourea | 453 (M + H) | 3 |
| 2025 | N-(cis-4-{[4-(dimethylamino)-5,6,7,8-tetrahydroquinazolin-2-yl] amino}cyclohexyl)-N'-(diphenylmethyl)thiourea | 515 (M + H) | 3 |
| 2026 | N-cyclododecyl-N'-(cis-4-{[4-(dimethylamino)-5,6,7,8-tetrahydroquinazolin-2-yl]amino}cyclohexyl)thiourea | 515 (M + H) | 3 |
| 2027 | N-(cyclohexylmethyl)-N'-(cis-4-{[4-(dimethylamino)-5,6,7,8-tetrahydroquinazolin-2-yl]amino}cyclohexyl)thiourea | 445 (M + H) | 3 |
| 2028 | N-cyclooctyl-N'-(cis-4-{[4-(dimethylamino)-5,6,7,8-tetrahydroquinazolin-2-yl]amino}cyclohexyl)thiourea | 459 (M + H) | 3 |
| 2029 | N-cyclopropyl-N'-(cis-4-{[4-(dimethylamino)-5,6,7,8-tetrahydroquinazolin-2-yl]amino}cyclohexyl)thiourea | 389 (M + H) | 3 |
| 2030 | N-(cis-4-{[4-(dimethylamino)-5,6,7,8-tetrahydroquinazolin-2-yl] amino}cyclohexyl)-N'-(2,2-diphenylethyl)thiourea | 529 (M) | 2 |
| 2031 | N-(cis-4-{[4-(dimethylamino)-5,6,7,8-tetrahydroquinazolin-2-yl] amino}cyclohexyl)-N'-(2,3,5,6-tetrachlorophenyl)thiourea | 561 (M + H) | 3 |
| 2032 | N-(2,4-dichlorobenzyl)-N'-(cis-4-{(4-(dimethylamino)-5,6,7,8-tetrahydroquinazolin-2-yl]amino}cyclohexyl)thiourea | 507 (M + H) | 3 |
| 2033 | N-(2,5-dibromophenyl)-N'-(cis-4-{[4-(dimethylamino)-5,6,7,8-tetrahydroquinazolin-2-yl]amino}cyclohexyl)thiourea | 581 (M + H) | 3 |
| 2034 | N-[2-(2,5-dimethoxyphenyl)ethyl]-N'-(cis-4-{[4-(dimethylamino)-5,6,7,8-tetrahydroquinazolin-2-yl]amino}cyclohexyl)thiourea | 513 (M + H) | 3 |
| 2035 | N-(2-chloro-5-nitrophenyl)-N'-(cis-4-{[4-(dimethylamino)-5,6,7,8-tetrahydroquinazolin-2-yl]amino}cyclohexyl)thiourea | 504 (M + H) | 3 |
| 2036 | N-(2-cyanophenyl)-N'-(cis-4-{[4-(dimethylamino)-5,6,7,8-tetrahydroquinazolin-2-yl]amino}cyclohexyl)thiourea | 450 (M + H) | 3 |
| 2037 | N-(cis-4-{[4-(dimethylamino)-5,6,7,8-tetrahydroquinazolin-2-yl] amino}cyclohexyl)-N'-(2-fluorobenzyl)thiourea | 457 (M + H) | 3 |
| 2038 | N-{[(cis-4-{[4-(dimethylamino)-5,6,7,8-tetrahydroquinazolin-2-yl] amino}cyclohexyl)amino]carbonothioyl}-2-furamide | 443 (M + H) | 3 |
| 2039 | N-(cis-4-{[4-(dimethylamino)-5,6,7,8-tetrahydroquinazolin-2-yl] amino}cyclohexyl)-N'-(2-methoxy-5-nitrophenyl)thiourea | 500 (M + H) | 3 |
| 2040 | N-(cis-4-{[4-(dimethylamino)-5,6,7,8-tetrahydroquinazolin-2-yl] amino}cyclohexyl)-N'-(2-methylbenzyl)thiourea | 453 (M + H) | 3 |
| 2041 | N-(3,4-dimethoxybenzyl)-N'-(cis-4-{[4-(dimethylamino)-5,6,7,8-tetrahydroquinazolin-2-yl]amino}cyclohexyl)thiourea | 499 (M + H) | 3 |

(continued)

| Ex. No. | compound name | MS | class |
|---|---|---|---|
| 2042 | N-(cis-4-{[4-(dimethylamino)-5,6,7,8-tetrahydroquinazolin-2-yl] amino}cyclohexyl)-N'-(3-ethylphenyl)thiourea | 453 (M + H) | 3 |
| 2043 | N-(cis-4-{[4-(dimethylamino)-5,6,7,8-tetrahydroquinazolin-2-yl] amino}cyclohexyl)-N'-(3-fluorobenzyl)thiourea | 457 (M + H) | 3 |
| 2044 | N-(cis-4-{[4-(dimethylamino)-5,6,7,8-tetrahydroquinazolin-2-yl] amino}cyclohexyl)-N'-(3-methoxybenzyl)thiourea | 469 (M + H) | 3 |
| 2045 | N-(cis-4-{[4-(dimethylamino)-5,6,7,8-tetrahydroquinazolin-2-yl] amino}cyclohexyl)-N'-(3-methylbenzyl)thiourea | 453 (M + H) | 3 |
| 2046 | N-(4-bromo-3-chlorophenyl)-N'-(cis-4-{[4-(dimethylamino)-5,6,7,8-tetrahydroquinazolin-2-yl]amino}cyclohexyl)thiourea | 537 (M + H) | 3 |
| 2047 | N-(4-bromo-3-methylphenyl)-N'-(cis-4-{[4-(dimethylamino)-5,6,7,8-tetrahydroquinazolin-2-yl]amino}cyclohexyl)thiourea | 517 (M + H) | 3 |
| 2048 | N-(4-decylphenyl)-N'-(cis-4-{[4-(dimethylamino)-5,6,7,8-tetrahydroquinazolin-2-yl]amino}cyclohexyl)thiourea | 565 (M + H) | 3 |
| 2049 | N-(cis-4-{[4-(dimethylamino)-5,6,7,8-tetrahydroquinazolin-2-yl] amino}cyclohexyl)-N'-[4-(4-nitrophenoxy)phenyl]thiourea | 562 (M + H) | 3 |
| 2050 | N-(cis-4-{[4-(dimethylamino)-5,6,7,8-tetrahydroquinazolin-2-yl] amino}cyclohexyl)-N'- (4-[(4-nitrophenyl)thio]phenyl}thiourea | 578 (M + H) | 3 |
| 2051 | 4-({[(cis-4-{[4-(dimethylamino)-5,6,7,8-tetrahydroquinazolin-2-yl] amino}cyclohexyl)amino]carbonothioyl}amino)-benzenesulfonamide | 502 (M - H) | 3 |
| 2052 | N-(cis-4-{[4-(dimethylamino)-5,6,7,8-tetrahydroquinazolin-2-yl] amino}cyclohexyl}-N'-[2-(4-methylphenyl)ethyl]thiourea | 467 (M + H) | 3 |
| 2053 | N-(cis-4-{[4-(dimethylamino)-5,6,7,8-tetrahydroquinazolin-2-yl] amino}cyclohexyl}-N'-(4-phenoxyphenyl)thiourea | 517 (M + H) | 3 |
| 2054 | N-(2,3-dihydro-1H-inden-5-yl)-N'-(cis-4-{[4-(dimethylamino)-5,6,7,8-tetrahydroquinazolin-2-yl]amino}cyclohexyl)thiourea | 465 (M + H) | 3 |
| 2055 | N-cycloheptyl-N'-(cis-4-{[4-(dimethylamino)-5,6,7,8-tetrahydroquinazolin-2-yl]amino}cyclohexyl)thiourea | 445 (M + H) | 3 |
| 2056 | N-(cis-4-)[4-(dimethylamino)-5,6,7,8-tetrahydroquinazolin-2-yl] amino}cyclohexyl)-N'-prop-2-yn-1-ylthiourea | 387 (M + H) | 3 |
| 2057 | N-(cis-4-{[4-(dimethylamino)-5,6,7,8-tetrahydroquinazolin-2-yl] amino}cyclohexyl)-N'-[4-(piperidin-1-ylsulfonyl)phenyl]-thiourea | 572 (M + H) | 3 |
| 2058 | N-(2-cyclohex-1-en-1-ylethyl)-N'-(cis-4-{[4-(dimethylamino)-5,6,7,8-tetrahydroquinazolin-2-yl]amino}cyclohexyl)thiourea | 457 (M + H) | 3 |
| 2059 | N-(cis-4-{[4-(dimethylamino)-5,6,7,8-tetrahydroquinazolin-2-yl] amino}cyclohexyl)-N'-(2,5-dimethylphenyl) thiourea | 453 (M + H) | 3 |
| 2060 | N-(2-bromo-4-isopropylphenyl)-N'-(cis-4-{[4-(dimethylamino)-5,6,7,8-tetrahydroquinazolin-2-yl]amino}cyclohexyl)thiourea | 545 (M + H) | 3 |
| 2061 | N-(2-bromo-5-fluorophenyl)-N'-(cis-4-{[4-(dimethylamino)-5,6,7,8-tetrahydroquinazolin-2-yl]amino}cyclohexyl)thiourea | 521 (M + H) | 3 |
| 2062 | N-(cis-4-{[4-(dimethylamino)-5,6,7,8-tetrahydroquinazolin-2-yl] amino}cyclohexyl)-N'-(2-methoxybenzyl)thiourea | 469 (M + H) | 3 |
| 2063 | N-(cis-4-{[4-(dimethylamino)-5,6,7,8-tetrahydroquinazolin-2-yl] amino}cyclohexyl)-N'-(3,4-dimethylphenyl)thiourea | 453 (M + H) | 3 |

(continued)

| Ex. No. | compound name | MS | class |
|---|---|---|---|
| 2064 | N-(cis-4-{[4-(dimethylamino)-5,6,7,8-tetrahydroquinazolin-2-yl] amino}cyclohexyl)-N'-(4-phenylbutyl)thiourea | 481 (M + H) | 3 |
| 2065 | N-(4-tert-butylphenyl)-N'-(cis-4-{[4-(dimethylamino)-5,6,7,8-tetrahydroquinazolin-2-yl]amino}cyclohexyl)thiourea | 481 (M + H) | 3 |
| 2066 | N-(5-chloro-2-fluorophenyl)-N'-(cis-4-([4-(dimethylamino)-5,6,7,8-tetrahydroquinazolin-2-yl]amino}cyclohexyl)thiourea | 477 (M + H) | 3 |
| 2067 | N-bicyclo[2.2.1]hept-5-en-2-yl-N'-(cis-4-{[4-(dimethylamino)-5,6,7,8-tetrahydroquinazolin-2-yl]amino}cyclohexyl)thiourea | 441 (M + H) | 3 |
| 2068 | N-(cyclopropylmethyl)-N'-(cis-4-{[4-(dimethylamino)-5,6,7,8-tetrahydroquinazolin-2-yl]amino}cyclohexyl)thiourea | 403 (M + H) | 3 |
| 2069 | ethyl 2-({[(cis-4-{[4-(dimethylamino)-5,6,7,8-tetrahydroquinazolin-2-yl]amino}cyclohexyl)amino]carbonothioyl}amino)-4,5,6,7-tetrahydro-1-benzothiophene-3-carboxylate | 557 (M + H) | 3 |
| 2070 | N-(2-bromo-4-fluorophenyl)-N'-(cis-4-{[4-(dimethylamino)-5,6,7,8-tetrahydroquinazolin-2-yl]amino}cyclohexyl)thiourea | 521 (M + H) | 3 |
| 2071 | N-(3-chloro-4-fluorophenyl)-N'-(cis-4-{[4-(dimethylamino)-5,6,7,8-tetrahydroquinazolin-2-yl]amino}cyclohexyl)thiourea | 477 (M + H) | 3 |
| 2072 | N-[4-(dimethylamino)phenyl]-N'-(cis-4-{[4-(dimethylamino)-5,6,7,8-tetrahydroquinazolin-2-yl]amino}cyclohexyl)thiourea | 468 (M + H) | 3 |
| 2073 | N-[3-(diethylamino)propyl]-N'-(cis-4-{[4-(dimethylamino)-5,6,7,8-tetrahydroquinazolin-2-yl]amino}cyclohexyl)thiourea | 462 (M + H) | 3 |
| 2074 | N-(cis-4-{[4-(dimethylamino)-5,6,7,8-tetrahydroquinazolin-2-yl] amino}cyclohexyl)-N'-(2-morpholin-4-ylethyl)thiourea | 462 (M + H) | 3 |
| 2075 | N-(cis-4-{[4-(dimethylamino)-5,6,7,8-tetrahydroquinazolin-2-yl] amino}cyclohexyl)-N'-(4-phenanthro[9,10-d][1,3]oxazol-2-ylphenyl)thiourea | 642 (M + H) | 3 |
| 2076 | N-(cis-4-{[4-(dimethylamino)-5,6,7,8-tetrahydroquinazolin-2-yl] amino}cyclohexyl)-N'-pyridin-3-ylthiourea | 426 (M + H) | 3 |
| 2077 | N-(4-{(E)-[4-(dimethylamino)phenyl]diazenyl}phenyl)-N'-(cis-4-{[4-(dimethylamino)-5,6,7,8-tetrahydroquinazolin-2-yl]amino} cyclohexyl)thiourea | 572 (M + H) | 3 |
| 2078 | N-(cis-4-{[4-(dimethylamino)-5,6,7,8-tetrahydroquinazolin-2-yl] amino}cyclohexyl)-N'-(3-morpholin-4-ylpropyl)thiourea | 476 (M + H) | 3 |
| 2079 | N-(4-chlorobenzyl)-N'-(cis-4-{[4-(dimethylamino)-5,6,7,8-tetrahydroquinazolin-2-yl]amino}cyclohexyl)thiourea | 473 (M + H) | 3 |
| 2080 | N-(cis-4-{[4-(dimethylamino)-5,6,7,8-tetrahydroquinazolin-2-yl] amino}cyclohexyl)-N'-{4-[(E)-phenyldiazenyl]phenyl}thiourea | 529 (M + H) | 3 |
| 2081 | N-(cis-4-{[4-(dimethylamino)-5,6,7,8-tetrahydroquinazolin-2-yl] amino}cyclohexyl)-N'-(2-piperidin-1-ylethyl)thiourea | 460 (M + H) | 3 |
| 2082 | N-(cis-4-{[4-(dimethylamino)-5,6,7,8-tetrahydroquinazolin-2-yl] amino}cycllohexyl)-N'-[4-(1H-pyrazol-1-yl)phenyl]thiourea | 401 (M + H) | 3 |
| 2083 | N-2,1,3-benzothiadiazol-4-yl-N'-(cis-4-{[4-(dimethylamino)-5,6,7,8-tetrahydroquinazolin-2-yl]amino}cyclohexyl)thiourea | 483 (M + H) | 3 |
| 2084 | N-2,1,3-benzothiadiazol-5-yl-N'-(cis-4-{[4-(dimethylamino)-5,6,7,8-tetrahydroquinazolin-2-yl]amino}cyclohexyl)thiourea | 483 (M + H) | 3 |

(continued)

| Ex. No. | compound name | MS | class |
|---|---|---|---|
| 2085 | N-(cis-4-{[4-(dimethylamino)-5,6,7,8-tetrahydroquinazolin-2-yl] amino}cyclohexyl)-N'-(3,5-dimethylisoxazol-4-yl)thiourea | 444 (M + H) | 3 |
| 2086 | N-(cis-4-{[4-(dimethylamino)-5,6,7,8-tetrahydroquinazolin-2-yl] amino}cyclohexyl)-N'-[4-(1,3-oxazol-5-yl)phenyl]thiourea | 492 (M + H) | 3 |
| 2087 | N-(cis-4-{[4-(dimethylamino)-5,6,7,8-tetrahydroquinazolin-2-yl] amino}cyclohexyl)-N'-(6-morpholin-4-ylpyridin-3-yl)thiourea | 511 (M + H) | 3 |
| 2088 | N-(cis-4-{[4-(dimethylamino)-5,6,7,8-tetrahydroquinazolin-2-yl] amino}cyclohexyl)-N'-(6-phenoxypyridin-3-yl) thiourea | 518 (M + H) | 3 |
| 2089 | N-(2-chlorophenyl)-N'-(cis-4-{[4-(dimethylamino)quinolin-2-yl] amino}cyclohexyl)urea | 438 (M + H) | 2 |
| 2090 | N-(cis-4-{[4-(dimethylamino)quinolin-2-yl]amino}cyclohexyl) -N'-(2,6-dimethylphenyl)urea | 432 (M + H) | 3 |
| 2091 | N-(2,4-difluorophenyl)-N'-(cis-4-{[4-(dimethylamino)quinolin-2-yl] amino}cyclohexyl)urea | 440 (M + H) | 3 |
| 2092 | N-(cis-4-{[4-(dimethylamino)quinolin-2-yl]amino}cyclohexyl) -N'-(2-ethyl-6-methylphenyl)urea | 446 (M + H) | 2 |
| 2093 | ethyl N-{[(cis-4-{[4-(dimethylamino)quinolin-2-yl]amino) cyclohexyl)amino]carbonyl}leucinate | 470 (M + H) | 3 |
| 2094 | N'-(cis-4-{[4-(dimethylamino)quinolin-2-yl]amino}cyclohexyl) -N'-(4-fluorophenyl)urea | 422 (M + H) | 3 |
| 2095 | N-(cis-4-{[4-(dimethylamino)quinolin-2-yl]amino}cyclohexyl) -N'-[4-(methylthio)phenyl]urea | 450 (M + H) | 3 |
| 2096 | N-(cis-4-{[4-(dimethylamino)quinolin-2-yl]amino}cyclohexyl) -N'-[2-(trifluoromethyl)phenyl]urea | 472 (M + H) | 3 |
| 2097 | N-(cis-4-{[4-(dimethylamino)quinolin-2-yl]amino}cyclohexyl)-N'-mesitylurea | 446 (M + H) | 1 |
| 2098 | N-(cis-4-{[4-(dimethylamino)quinolin-2-yl]amino}cyclohexyl) -N'-(2-methylphenyl)urea | 418 (M + H) | 3 |
| 2099 | N-(cis-4-{[4-(dimethylamino)quinolin-2-yl]amino}cyclohexyl) -N'-(2,4,6-trichlorophenyl)urea | 506 (M + H) | 2 |
| 2100 | N-(cis-4-{[4-(dimethylamino)quinolin-2-yl]amino}cyclohexyl) -N'-(2,4,6-tribromophenyl)urea | 637 (M + H) | 1 |
| 2101 | N-(2,4-dibromo-6-fluorophenyl)-N'-(cis-4-{[4-(dimethylamino) quinolin-2-yl]amino}cyclohexyl)urea | 578 (M + H) | 2 |
| 2102 | N-(2,6-diethylphenyl)-N'-(cis-4-{[4-(dimethylamino)quinolin-2-yl] amino}cyclohexyl)urea | 460 (M + H) | 1 |
| 2103 | N-[2-chloro-6-(trifluoromethyl)phenyl]-N'-(cis-4-{ [4-(dimethylamino)quinolin-2-yl]amino}cyclohexyl)urea | 506 (M + H) | 3 |
| 2104 | N-(2-chloro-6-methylphenyl)-N'-(cis-4-{[4-(dimethylamino) quinolin-2-yl]amino}cyclohexyl)urea | 452 (M + H) | 3 |
| 2105 | N-(2-chlorobenzyl)-N'-(cis-4-{[4-(dimethylamino)quinolin-2-yl] amino}cyclohexyl)urea | 452 (M + H) | 2 |
| 2106 | N-(cis-4-{[4-(dimethylamino)quinolin-2-yl]amino}cyclohexyl) -N'-(2-ethyl-6-isopropylphenyl)urea | 474 (M + H) | 2 |

(continued)

| Ex. No. | compound name | MS | class |
|---|---|---|---|
| 2107 | N-(cis-4-{[4-(dimethylamino)quinolin-2-yl]amino}cyclohexyl) -N'-(2-ethylphenyl)urea | 432 (M + H) | 3 |
| 2108 | N-(cis-4-{[4-(dimethylamino)quinolin-2-yl]amino}cyclohexyl) -N'-(2-iodophenyl)urea | 530 (M + H) | 3 |
| 2109 | N-(cis-4-{[4-(dimethylamino)quinolin-2-yl]amino}cyclohexyl) -N'-(2-isopropyl-6-methylphenyl)urea | 460 (M + H) | 2 |
| 2110 | N-(cis-4-{[4-(dimethylamino)quinolin-2-yl]amino}cyclohexyl) -N'-(2-isopropylphenyl)urea | 446 (M + H) | 3 |
| 2111 | N-(cis-4-{[4-(dimethylamino)quinolin-2-yl]amino}cyclohexyl) -N'-(2-methyl-3-nitrophenyl)urea | 463 (M + H) | 3 |
| 2112 | N-(cis-4-{[4-(dimethylamino)quinolin-2-yl]amino}cyclohexyl) -N'-(2-propylphenyl)urea | 446 (M + H) | 3 |
| 2113 | N-(2-tert-butyl-6-methylphenyl)-N'-(cis-4-{[4-(dimethylamino) quinolin-2-yl]amino}cyclohexyl)urea | 474 (M + H) | 1 |
| 2114 | N-(2-tert-butylphenyl)-N'-(cis-4-{[4-(dimethylamino)quinolin-2-yl] amino}cyclohexyl)urea | 460 (M + H) | 3 |
| 2115 | N-(3-chloro-2-methylphenyl)-N'-(cis-4-{[4-(dimethylamino) quinolin-2-yl]amino)}cyclohexyl)urea | 452 (M + H) | 3 |
| 2116 | N-(4-bromo-2,6-difluorophenyl)-N'-(cis-4-{[4-(dimethylamino) quinolin-2-yl]amino}cyclohexyl)urea | 518 (M + H) | 3 |
| 2117 | N-[4-chloro-2-(trifluoromethyl)phenyl]-N'-(cis-4-{ [4-(dimethylamino)quinolin-2-yl]amino}cyclohexyl)urea | 506 (M + H) | 3 |
| 2118 | N-(4-cyanophenyl)-N'-(cis-4-{[4-(dimethylamino)quinolin-2-yl] amino}cyclohexyl)urea | 429 (M + H) | 3 |
| 2119 | N-(cis-4-{[4-(dimethylamino)quinolin-2-yl]amino}cyclohexyl) -N'-(diphenylmethyl)urea | 494 (M + H) | 2 |
| 2120 | N-(4-bromo-2,6-dimethylphenyl)-N'-(cis-4-{[4-(dimethylamino) quinolin-2-yl]amino}cyclohexyl)urea | 510 (M + H) | 1 |
| 2121 | N-(cis-4-{[4-(dimethylamino)quinolin-2-yl]amino}cyclohexyl) -N'-(3-methyl-5-phenylisoxazol-4-yl)urea | 485 (M + H) | 2 |
| 2122 | N-(cis-4-{[4-(dimethylamino)quinolin-2-yl]amino}cyclohexyl) -N'-[5-methyl-2-(trifluoromethyl)-3-furyl]urea | 476 (M + H) | 3 |
| 2123 | N-(2-bromophenyl)-N'-(cis-4-{[4-(dimethylamino)quinolin-2-yl] amino}cyclohexyl)urea | 482 (M + H) | 3 |
| 2124 | N-biphenyl-2-yl-N'-(cis-4-{[4-(dimethylamino)quinolin-2-yl]amino} cyclohexyl)urea | 480 (M + H) | 3 |
| 2125 | N-butyl-N'-(cis-4-{[4-(dimethylamino)quinolin-2-yl]amino} cyclohexyl)urea | 384 (M + H) | 3 |
| 2126 | N-(cis-4-{[4-(dimethylamino)quinolin-2-yl]amino}cyclohexyl) -N'-(2,3-dimethylphenyl)urea | 432 (M + H) | 3 |
| 2127 | ethyl 3-({[(cis-4-{[4-(dimethylamino)quinolin-2-yl]amino} cyclohexyl)amino]carbonyl}amino)benzoate | 476 (M + H) | 3 |
| 2128 | N-(cis-4-{[4-(dimethylamino)quinolin-2-yl]amino}cyclohexyl) -N'-[1-(3-isopropenylphenyl)-1-methylethyl]urea | 486 (M + H) | 3 |

(continued)

| Ex. No. | compound name | MS | class |
|---|---|---|---|
| 2129 | methyl N-{[(cis-4-{[4-(dimethylamino)quinolin-2-yl]amino} cyclohexyl)amino]carbonyl}methioninate | 474 (M + H) | 3 |
| 2130 | N-(cis-4-{[4-(dimethylamino)quinolin-2-yl]amino}cyclohexyl)-N'-1-naphthylurea | 454 (M + H) | 1 |
| 2131 | N-(cis-4-{[4-(dimethylamino)quinolin-2-yl]amino}cyclohexyl) -N'-[(2S)-2-phenylcyclopropyl]urea | 444 (M + H) | 3 |
| 2132 | N-(cis-4-{[4-(dimethylamino)quinolin-2-yl]amino) cyclohexyl) -N'-(4-phenoxyphenyl)urea | 496 (M + H) | 3 |
| 2133 | N-(cis-4-{[4-(dimethylamino)quinolin-2-yl]amino}cyclohexyl)-N'-pentylurea | 398 (M + H) | 3 |
| 2134 | N-(cis-4-{[4-(dimethylamino)quinolin-2-yl]amino}cyclohexyl) -N'-[1-(1-naphthyl)ethyl]urea | 482 (M + H) | 1 |
| 2135 | methyl N-{[(cis-4-{[4-(dimethylamino)quinolin-2-yl]amino} cyclohexyl)amino]carbonyl}phenylalaninate | 490 (M + H) | 2 |
| 2136 | N-(cis-4-{[4-(dimethylamino)quinolin-2-yl]amino}cyclohexyl) -N'-(1-phenylethyl)urea | 432 (M + H) | 3 |
| 2137 | 1-[4-(4-Dimethylamino-quinolin-2-ylamino)-cyclohexyl]-3-(1-phenyl-ethyl)-urea | 432 (M + H) | 3 |
| 2138 | N-(cis-4-{[4-(dimethylamino)quinolin-2-yl]amino}cyclohexyl) -N'-(2,3,5,6-tetrachlorophenyl)urea | 540 (M + H) | 3 |
| 2139 | N-(2,4-dibromophenyl)-N'-(cis-4-{[4-(dimethylamino)quinolin-2-yl] amino}cyclohexyl)urea | 560 (M + H) | 3 |
| 2140 | N-(2,4-dichlorobenzyl)-N'-(cis-4-{[4-(dimethylamino)quinolin-2-yl] amino}cyclohexyl)urea | 486 (M + H) | 3 |
| 2141 | N-(2,4-dimethoxyphenyl)-N'-(cis-4-{[4-(dimethylamino)quinolin-2-yl]amino}cyclohexyl)urea | 464 (M + H) | 3 |
| 2142 | N-(cis-4-{[4-(dimethylamino)quinolin-2-yl]amino}cyclohexyl) -N'-(2-ethoxyphenyl)urea | 448 (M + H) | 3 |
| 2143 | N-(cis-4-{[4-(dimethylamino)quinolin-2-yl]amino}cyclohexyl) -N'-(2-fluorobenzyl)urea | 436 (M + H) | 3 |
| 2144 | N-(cis-4-{[4-(dimethylamino)quinolin-2-yl]amino}cyclohexyl) -N'-(2-methyl-4-nitrophenyl)urea | 463 (M + H) | 3 |
| 2145 | N-(cis-4-{[4-(dimethylamino)quinolin-2-yl]amino}cyclohexyl) -N'-(2-methyl-5-nitrophenyl)urea | 463 (M + H) | 3 |
| 2146 | N-(cis-4-{[4-(dimethylamino)quinolin-2-yl]amino}cyclohexyl) -N'-(2-methylbenzyl)urea | 432 (M + H) | 3 |
| 2147 | N-(cis-4-{[4-(dimethylamino)quinolin-2-yl]amino}cyclohexyl) -N'-(2-nitrophenyl)urea | 449 (M + H) | 3 |
| 2148 | N-1,3-benzodioxol-5-yl-N'-(cis-4-{[4-(dimethylamino)quinolin-2-yl] amino}cyclohexyl)urea | 448 (M + H) | 3 |
| 2149 | N-(cis-4-{[4-(dimethylamino)quinolin-2-yl]amino}cyclohexyl) -N'-(3,4,5-trimethoxyphenyl)urea | 494 (M + H) | 1 |
| 2150 | N-(3,4-dimethoxyphenyl)-N'-(cis-4-{[4-(dimethylamino)quinolin-2-yl]amino}cyclohexyl)urea | 464 (M + H) | 3 |

(continued)

| Ex. No. | compound name | MS | class |
|---|---|---|---|
| 2151 | N-(3-chloro-4-methoxyphenyl)-N'-(cis-4-{[4-(dimethylamino) quinolin-2-yl]amino}cyclohexyl)urea | 468 (M + H) | 3 |
| 2152 | N-[4-bromo-2-(trifluoromethyl)phenyl]-N'-(cis-4-{ [4-(dimethylamino)quinolin-2-yl]amino}cyclohexyl)urea | 550 (M + H) | 3 |
| 2153 | N-(4-bromobenzyl)-N'-(cis-4-{[4-(dimethylamino)quinolin-2-yl] amino}cyclohexyl)urea | 496 (M + H) | 3 |
| 2154 | N-(4-chloro-2-methylphenyl)-N'-(cis-4-{[4-(dimethylamino) quinolin-2-yl]amino}cyclohexyl)urea | 452 (M + H) | 3 |
| 2155 | N-(cis-4-{[4-(dimethylamino)quinolin-2-yl]amino}cyclohexyl) -N'-(4-fluorobenzyl)urea | 436 (M + H) | 3 |
| 2156 | N-(cis-4-{[4-(dimethylamino)quinolin-2-yl]amino}cyclohexyl) -N'-(4-methoxy-2-methylphenyl)urea | 448 (M + H) | 3 |
| 2157 | N-(5-chloro-2,4-dimethoxyphenyl)-N'-(cis-4-{[4-(dimethylamino) quinolin-2-yl]amino}cyclohexyl)urea | 498 (M + H) | 1 |
| 2158 | N-[1-(4-bromophenyl)ethyl]-N'-(cis-4-{[4-(dimethylamino)quinolin-2-yl]amino}cyclohexyl)urea | 510 (M + H) | 3 |
| 2159 | N-(4-bromo-2-methylphenyl)-N'-(cis-4-{[4-(dimethylamino) quinolin-2-yl]amino}cyclohexyl)urea | 496 (M + H) | 2 |
| 2160 | ethyl N-{[(cis-4-{[4-(dimethylamino)quinolin-2-yl]amino} cyclohexyl)amino]carbonyl }phenylalaninate | 504 (M + H) | 3 |
| 2161 | N-(2,3-dihydro-1,4-benzodioxin-6-yl)-N'-(cis-4-{ [4-(dimethylamino)quinolin-2-yl]amino}cyclohexyl)urea | 462 (M + H) | 3 |
| 2162 | N-(2,6-dibromo-4-isopropylphenyl)-N'-(cis-4-{[4-(dimethylamino) quinolin-2-yl]amino}cyclohexyl)urea | 602 (M + H) | 3 |
| 2163 | N-[3-(cyclopentyloxy)-4-methoxyphenyl]-N'-(cis-4-{ [4-(dimethylamino)quinolin-2-yl]amino}cyclohexyl)urea | 518 (M + H) | 3 |
| 2164 | N-(3,4-dihydro-2H-1,5-benzodioxepin-7-yl)-N'-(cis-4-{ [4-(dimethylamino)quinolin-2-yl]amino}cyclohexyl)urea | 476 (M + H) | 3 |
| 2165 | N-(4-butyl-2-methylphenyl)-N'-(cis-4-{[4-(dimethylamino)quinolin-2-yl]amino}cyclohexyl)urea | 474 (M + H) | 3 |
| 2166 | N-(cis-4-{[4-(dimethylamino)quinolin-2-yl]amino}cyclohexyl) -N'-(5-methyl-3-phenylisoxazol-4-yl)urea | 485 (M + H) | 3 |
| 2167 | N-(4-bromophenyl)-N'-(cis-4-{[4-(dimethylamino)quinolin-2-yl] amino}cyclohexyl)thiourea | 498 (M + H) | 3 |
| 2168 | N-(4-cyanophenyl)-N'-(cis-4-{[4-(dimethylamino)quinolin-2-yl] amino}cyclohexyl)thiourea | 445 (M + H) | 3 |
| 2169 | N-(2,4-dichlorophenyl)-N'-(cis-4-{[4-(dimethylamino)quinolin-2-yl] amino}cyclohexyl)thiourea | 488 (M + H) | 3 |
| 2170 | N-(2,4-dimethoxyphenyl)-N'-(cis-4-{[4-(dimethylamino)quinolin-2-yl]amino}cyclohexyl)thiourea | 480 (M + H) | 2 |
| 2171 | N-(cis-4-{[4-(dimethylamino)quinolin-2-yl]amino}cyclohexyl) -N'-(2,6-dimethylphenyl)thiourea | 448 (M + H) | 3 |
| 2172 | N-(cis-4-{[4-(dimethylamino)quinolin-2-yl]amino}cyclohexyl) -N'-(2-ethyl-6-isopropylphenyl)thiourea | 490 (M + H) | 3 |

(continued)

| Ex. No. | compound name | MS | class |
|---|---|---|---|
| 2173 | N-(cis-4-{[4-(dimethylamino)quinolin-2-yl]amino}cyclohexyl)-N'-(2-methoxyphenyl)thiourea | 450 (M + H) | 3 |
| 2174 | N-(cis-4-{[4-(dimethylamino)quinolin-2-yl]amino}cyclohexyl)-N'-1-naphthylthiourea | 470 (M + H) | 3 |
| 2175 | N-(cis-4-{[4-(dimethylamino)quinolin-2-yl]amino}cyclohexyl)-N'-(3,4,5-trimethoxyphenyl)thiourea | 510 (M + H) | 1 |
| 2176 | N-(3,4-dimethoxyphenyl)-N'-(cis-4-{[4-(dimethylamino)quinolin-2-yl]amino}cyclohexyl)thiourea | 480 (M + H) | 3 |
| 2177 | N-[4-(dimethylamino)-1-naphthyl]-N'-(cis-4-{[4-(dimethylamino)quinolin-2-yl]amino}cyclohexyl)thiourea | 513 (M + H) | 2 |
| 2178 | N-(cis-4-{[4-(dimethylamino)quinolin-2-yl]amino}cyclohexyl)-N'-(2-ethylphenyl)thiourea | 448 (M + H) | 3 |
| 2179 | N-(2-chlorophenyl)-N'-(cis-4-{[4-(dimethylamino)pyrimidin-2-yl]amino}cyclohexyl)urea | 389 (M + H) | 3 |
| 2180 | N-(cis-4-{[4-(dimethylamino)pyrimidin-2-yl]amino}cyclohexyl)-N'-(2,6-dimethylphenyl)urea | 383 (M + H) | 3 |
| 2181 | N-(2,4-difluorophenyl)-N'-(cis-4-{[4-(dimethylamino)pyrimidin-2-yl]amino}cyclohexyl)urea | 391 (M + H) | 3 |
| 2182 | N-(cis-4-{[4-(dimethylamino)pyrimidin-2-yl]amino}cyclohexyl)-N'-(2-ethyl-6-methylphenyl)urea | 397 (M + H) | 3 |
| 2183 | ethyl N-{[(cis-4-{[4-(dimethylamino)pyrimidin-2-yl]amino}cyclohexyl)amino]carbonyl}leucinate | 421 (M + H) | 3 |
| 2184 | N-(cis-4-{[4-(dimethylamino)pyrimidin-2-yl]amino}cyclohexyl)-N'-(4-fluorophenyl)urea | 373 (M + H) | 3 |
| 2185 | N-(cis-4-{[4-(dimethylamino)pyrimidin-2-yl]amino}cyclohexyl)-N'-[4-(methylthio)phenyl]urea | 401 (M + H) | 3 |
| 2186 | N-(cis-4-{[4-(dimethylamino)pyrimidin-2-yl]amino}cyclohexyl)-N'-[2-(trifluoromethyl)phenyl]urea | 445 (M + Na) | 3 |
| 2187 | N-(cis-4-{[4-(dimethylamino)pyrimidin-2-yl]amino}cyclohexyl)-N'-mesitylurea | 397 (M + H) | 2 |
| 2188 | N-(cis-4-{[4-(dimethylamino)pyrimidin-2-yl]amino}cyclohexyl)-N'-(2-methylphenyl)urea | 369 (M + H) | 3 |
| 2189 | N-(cis-4-{[4-(dimethylamino)pyrimidin-2-yl]amino}cyclohexyl)-N'-(2,4,6-trichlorophenyl)urea | 457 (M + H) | 1 |
| 2190 | N-(cis-4-{[4-(dimethylamino)pyrimidin-2-yl]amino}cyclohexyl)-N'-(2,4,6-tribromophenyl)urea | 588 (M + H) | 1 |
| 2191 | N-(2,4-dibromo-6-fluorophenyl)-N'-(cis-4-{[4-(dimethylamino)pyrimidin-2-yl]amino}cyclohexyl)urea | 529 (M + H) | 1 |
| 2192 | N-(2,6-diethylphenyl)-N'-(cis-4-{[4-(dimethylamino)pyrimidin-2- yl]amino}cyclohexyl)urea | 411 (M + H) | 3 |
| 2193 | N-[2-chloro-6-(trifluoromethyl)phenyl]-N'-(cis-4-{[4-(dimethylamino)pyrimidin-2-yl]amino}cyclohexyl)urea | 457 (M + H) | 3 |
| 2194 | N-(2-chloro-6-methylphenyl)-N'-(cis-4-{[4-(dimethylamino)pyrimidin-2-yl]amino}cyclohexyl)urea | 403 (M + H) | 3 |

(continued)

| Ex. No. | compound name | MS | class |
|---|---|---|---|
| 2195 | N-(2-chlorobenzyl)-N'-(cis-4-{[4-(dimethylamino)pyrimidin-2-yl]amino}cyclohexyl}urea | 403 (M + H) | 3 |
| 2196 | N-(cis-4-{[4-(dimethylamino)pyrimidin-2-yl]amino}cyclohexyl)-N'-(2-ethyl-6-isopropylphenyl)urea | 447 (M + Na) | 3 |
| 2197 | N-(cis-4-{[4-(dimethylamino)pyrimidin-2-yl]amino}cyclohexyl)-N'-(2-ethylphenyl)urea | 383 (M + H) | 3 |
| 2198 | N-(cis-4-{[4-(dimethylamino)pyrimidin-2-yl]amino}cyclohexyl)-N'-(2-iodophenyl)urea | 481 (M + H) | 3 |
| 2199 | N-(cis-4-{[4-(dimethylamino)pyrimidin-2-yl]amino}cyclohexyl)-N'-(2-isopropyl-6-methylphenyl)urea | 411 (M + H) | 3 |
| 2200 | N-(cis-4-{[4-(dimethylamino)pyrimidin-2-yl]amino}cyclohexyl)-N'-(2-isopropylphenyl)urea | 397 (M + H) | 3 |
| 2201 | N-(cis-4-{[4-(dimethylamino)pyrimidin-2-yl]amino}cyclohexyl)-N'-(2-methyl-3-nitrophenyl)urea | 414 (M + H) | 3 |
| 2202 | N-(cis-4-{[4-(dimethylamino)pyrimidin-2-yl]amino}cyclohexyl)-N'-(2-propylphenyl)urea | 397 (M + H) | 3 |
| 2203 | N-(2-tert-butyl-6-methylphenyl)-N'-(cis-4-{[4-(dimethylamino)pyrimidin-2-yl]amino}cyclohexyl)urea | 425 (M + H) | 3 |
| 2204 | N-(2-tert-butylphenyl)-N'-(cis-4-{[4-(dimethylamino)pyrimidin-2-yl]amino}cyclohexyl)urea | 411 (M + H) | 3 |
| 2205 | N-(3-chloro-2-methylphenyl)-N'-(cis-4-{[4-(dimethylamino)pyrimidin-2-yl]amino}cyclohexyl)urea | 403 (M + H) | 3 |
| 2206 | N-(4-bromo-2,6-difluorophenyl)-N'-(cis-4-{[4-(dimethylamino)pyrimidin-2-yl]amino}cyclohexyl)urea | 469 (M + H) | 3 |
| 2207 | N-[4-chloro-2-(trifluoromethyl)phenyl]-N'-(cis-4-{[4-(dimethylamino)pyrimidin-2-yl]amino}cyclohexyl)urea | 457 (M + H) | 3 |
| 2208 | N-(4-cyanophenyl)-N'-(cis-4-{[4-(dimethylamino)pyrimidin-2-yl]amino}cyclohexyl)urea | 380 (M + H) | 3 |
| 2209 | N-(cis-4-{[4-(dimethylamino)pyrimidin-2-yl]amino}cyclohexyl)-N'-(diphenylmethyl)urea | 445 (M + H) | 1 |
| 2210 | N-(4-bromo-2,6-dimethylphenyl)-N'-(cis-4-{[4-(dimethylamino)pyrimidin-2-yl]amino}cyclohexyl)urea | 461 (M + H) | 1 |
| 2211 | N-(cis-4-{[4-(dimethylamino)pyrimidin-2-yl]amino}cyclohexyl)-N'-(3-methyl-5-phenylisoxazol-4-yl)urea | 436 (M + H) | 3 |
| 2212 | N-(cis-4-{[4-(dimethylamino)pyrimidin-2-yl]amino)cyclohexyl)-N'-[5-methyl-2-(trifluoromethyl)-3-furyl]urea | 427 (M + H) | 3 |
| 2213 | N-(2-bromophenyl)-N'-(cis-4-{[4-(dimethylamino)pyrimidin-2-yl]amino}cyclohexyl)urea | 433 (M + H) | 3 |
| 2214 | N-biphenyl-2-yl-N'-(cis-4-{[4-(dimethylamino)pyrimidin-2-yl]amino}cyclohexyl)urea | 431 (M + H) | 3 |
| 2215 | N-butyl-N'-(cis-4-{[4-(dimethylamino)pyrimidin-2-yl]amino)cyclohexyl)urea | 335 (M + H) | 3 |
| 2216 | N-(cis-4-{[4-(dimethylamino)pyrimidin-2-yl]amino}cyclohexyl)-N'-(2,3-dimethylphenyl)urea | 383 (M + H) | 3 |

(continued)

| Ex. No. | compound name | MS | class |
|---|---|---|---|
| 2217 | ethyl 3-({[(cis-4-{[4-(dimethylamino)pyrimidin-2-yl]amino} cyclohexyl)amino]carbonyl}amino)benzoate | 427 (M + H) | 3 |
| 2218 | N-(cis-4-{[4-(dimethylamino)pyrimidin-2-yl]amino}cyclohexyl) -N'-[1-(3-isopropenylphenyl)-1-methylethyl]urea | 437 (M + H) | 3 |
| 2219 | methyl N-{[(cis-4-{[4-(dimethylamino)pyrimidin-2-yl]amino} cyclohexyl)amino]carbonyl}methioninate | 425 (M + H) | 3 |
| 2220 | N-(cis-4-{[4-(dimethylamino)pyrimidin-2-yl]amino}cyclohexyl)-N'-1-naphthylurea | 405 (M + H) | 3 |
| 2221 | N-(cis-4-{[4-(dimethylamino)pyrimidin-2-yl]amino}cyclohexyl) -N'-[(2S)-2-phenylcyclopropyl]urea | 395 (M + H) | 3 |
| 2222 | N-(cis-4-{[4-(dimethylamino)pyrimidin-2-yl]amino}cyclohexyl) -N'-(4-phenoxyphenyl)urea | 447 (M + H) | 3 |
| 2223 | N-(cis-4-{[4-(dimethylamino)pyrimidin-2-yl]amino}cyclohexyl)-N'-pentylurea | 349 (M + H) | 3 |
| 2224 | N-(cis-4-{[4-(dimethylamino)pyrimidin-2-yl]amino}cyclohexyl) -N'-[1-(1-naphthyl)ethyl]urea | 433 (M + H) | 1 |
| 2225 | methyl N-{[(cis-4-{[4-(dimethylamino)pyrimidin-2-yl]amino] cyclohexyl)amino]carbonyl}phenylalaninate | 441 (M + H) | 3 |
| 2226 | N-(cis-4-{[4-(dimethylamino)pyrimidin-2-yl]amino}cyclohexyl) -N'-(l-phenylethyl)urea | 383 (M + H) | 3 |
| 2227 | 1-[4-(4-Dimethylamino-pyrimidin-2-yl]amino}-cyclohexyl]-3-(1-phenyl-ethyl)-urea | 383 (M + H) | 3 |
| 2228 | N-(cis-4-{[4-(dimethylamino)pyrimidin-2-yl]amino}cyclohexyl) -N'-(2,3,5,6-tetrachlorophenyl)urea | 491 (M + H) | 3 |
| 2229 | N-(2,4-dibromophenyl)-N'-(cis-4-{[4-(dimethylamino)pyrimidin-2-yl]amino}cyclohexyl)urea | 511 (M + H) | 3 |
| 2230 | N-(2,4-dichlorobenzyl)-N'-(cis-4-{[4-(dimethylamino)pyrimidin-2-yl]amino}cyclohexyl)urea | 437 (M + H) | 3 |
| 2231 | N-(2,4-dimethoxyphenyl)-N'-(cis-4-{[4-(dimethylamino)pyrimidin-2-yl]amino}cyclohexyl)urea | 415 (M + H) | 3 |
| 2232 | N-(cis-4-{[4-(dimethylamino)pyrimidin-2-yl]amino}cyclohexyl) -N'-(2-ethoxyphenyl)urea | 399 (M + H) | 3 |
| 2233 | N-(cis-4-{[4-(dimethylamino)pyrimidin-2-yl]amino}cyclohexyl) -N'-(2-fluorobenzyl)urea | 387 (M + H) | 3 |
| 2234 | N-(cis-4-{[4-(dimethylamino)pyrimidin-2-yl]amino}cyclohexyl) -N'-(2-methyl-4-nitrophenyl)urea | 414 (M + H) | 3 |
| 2235 | N-(cis-4-{[4-(dimethylamino)pyrimidin-2-yl]amino}cyclohexyl) -N'-(2-methyl-5-nitrophenyl)urea | 414 (M + H) | 3 |
| 2236 | N-(cis-4-{[4-(dimethylamino)pyrimidin-2-yl]amino}cyclohexyl) -N'-(2-methylbenzyl)urea | 383 (M + H) | 3 |
| 2237 | N-(cis-4-{[4-(dimethylamino)pyrimidin-2-yl]amino}cyclohexyl) -N'-(2-nitrophenyl)urea | 400 (M + H) | 3 |
| 2238 | N-1,3-benzodioxol-5-yl-N'-(cis-4-{[4-(dimethylamino)pyrimidin-2-yl]amino}cyclohexyl)urea | 399 (M + H) | 3 |

(continued)

| Ex. No. | compound name | MS | class |
|---|---|---|---|
| 2239 | N-(cis-4-{[4-(dimethylamino)pyrimidin-2-yl]amino}cyclohexyl)-N'-(3,4,5-trimethoxyphenyl)urea | 445 (M + H) | 1 |
| 2240 | N-(3,4-dimethoxyphenyl)-N'-(cis-4-{[4-(dimethylamino)pyrimidin-2-yl]amino}cyclohexyl)urea | 415 (M + H) | 3 |
| 2241 | N-(3-chloro-4-methoxyphenyl)-N'-(cis-4-{[4-(dimethylamino)pyrimidin-2-yl]amino}cyclohexyl)urea | 419 (M + H) | 3 |
| 2242 | N-[4-bromo-2-(trifluoromethyl)phenyl]-N'-(cis-4-{[4-(dimethylamino)pyrimidin-2-yl]amino}cyclohexyl)urea | 501 (M + H) | 3 |
| 2243 | N-(4-bromobenzyl)-N'-(cis-4-{[4-(dimethylamino)pyrimidin-2-yl]amino}cyclohexyl)urea | 447 (M + H) | 3 |
| 2244 | N-(4-chloro-2-methylphenyl)-N'-(cis-4-{[4-(dimethylamino )pyrimidin-2-yl]amino}cyclohexyl)urea | 403 (M + H) | 2 |
| 2245 | N-(cis-4-{[4-(dimethylamino)pyrimidin-2-yl]amino}cyclohexyl)-N'-(4-fluorobenzyl)urea | 387 (M + H) | 3 |
| 2246 | N-(cis-4-{[4-(dimethylamino)pyrimidin-2-yl]amino}cyclohexyl)-N'-(4-methoxy-2-methylphenyl)urea | 399 (M + H) | 3 |
| 2247 | N-(5-chloro-2,4-dimethoxyphenyl)-N'-(cis-4-{[4-(dimethylamino)pyrimidin-2-yl]amino}cyclohexyl)urea | 449 (M + H) | 1 |
| 2248 | N-[1-(4-bromophenyl)ethyl]-N'-(cis-4-{[4-(dimethylamino)pyrimidin-2-yl]amino}cyclohexyl)urea | 461 (M + H) | 3 |
| 2249 | N-(4-bromo-2-methylphenyl)-N'-(cis-4-{[4-(dimethylamino)pyrimidin-2-yl]amino}cyclohexyl)urea | 447 (M + H) | 2 |
| 2250 | ethyl N-{[(cis-4-{[4-(dimethylamino)pyrimidin-2-yl]amino}cyclohexyl)amino]carbonyl}phenylalaninate | 455 (M + H) | 3 |
| 2251 | N-(2,3-dihydro-1,4-benzodioxin-6-yl)-N'-(cis-4-{[4-(dimethylamino)pyrimidin-2-yl]amino}cyclohexyl)urea | 413 (M + H) | 3 |
| 2252 | N-(2,6-dibromo-4-isopropylphenyl)-N'-(cis-4-{[4-(dimethylamino)pyrimidin-2-yl]amino}cyclohexyl)urea | 553 (M + H) | 2 |
| 2253 | N-[3-(cyclopentyloxy)-4-methoxyphenyl]-N'-(cis-4-{[4-(dimethylamino)pyrimidin-2-yl]amino}cyclohexyl)urea | 469 (M + H) | 2 |
| 2254 | N-(3,4-dihydro-2H-1,5-benzodioxepin-7-yl)-N'-(cis-4-{[4-(dimethylamino)pyrimidin-2-yl]amino}cyclohexyl)urea | 427 (M + H) | 3 |
| 2255 | N-(4-butyl-2-methylphenyl)-N'-(cis-4-{[4-(dimethylamino)pyrimidin-2-yl]amino}cyclohexyl)urea | 425 (M + H) | 3 |
| 2256 | N-(cis-4-{[4-(dimethylamino)pyrimidin-2-yl]amino}cyclohexyl)-N'-(5-methyl-3-phenylisoxazol-4-yl)urea | 436 (M + H) | 3 |
| 2257 | N-(4-bromophenyl)-N'-(cis-4-{[4-(dimethylamino)pyrimidin-2-yl]amino}cyclohexyl)thiourea | 449 (M + H) | 3 |
| 2258 | N-(4-cyanophenyl)-N'-(cis-4-{[4-(dimethylamino)pyrimidin-2-yl]amino}cyclohexyl)thiourea | 396 (M + H) | 2 |
| 2259 | N-(2,4-dichlorophenyl)-N'-(cis-4-{[4-(dimethylamino)pyrimidin-2-yl]amino}cyclohexyl)thiourea | 439 (M + H) | 3 |
| 2260 | N-(2,4-dimethoxyphenyl)-N'-(cis-4-{[4-(dimethylamino)pyrimidin-2-yl]amino}cyclohexyl)thiourea | 431 (M + H) | 2 |

(continued)

| Ex. No. | compound name | MS | class |
|---|---|---|---|
| 2261 | N-(cis-4-{[4-(dimethylamino)pyrimidin-2-yl]amino}cyclohexyl)-N'-(2,6-dimethylphenyl)thiourea | 399 (M + H) | 3 |
| 2262 | N-(cis-4-{[4-(dimethylamino)pyrimidin-2-yl]amino}cyclohexyl)-N'-(2-ethyl-6-isopropylphenyl)thiourea | 441 (M + H) | 3 |
| 2263 | N-(cis-4-{[4-(dimethylamino)pyrimidin-2-yl]amino}cyclohexyl)-N'-(2-methoxyphenyl)thiourea | 401 (M + H) | 3 |
| 2264 | N-(cis-4-{[4-(dimethylamino)pyrimidin-2-yl]amino}cyclohexyl)-N'-1-naphthylthiourea | 421 (M + H) | 3 |
| 2265 | N-(cis-4-{[4-(dimethylamino)pyrimidin-2-yl]amino}cyclohexyl)-N'-(3,4,5-trimethoxyphenyl)thiourea | 461 (M + H) | 1 |
| 2266 | N-(3,4-dimethoxyphenyl)-N'-(cis-4-{[4-(dimethylamino)pyrimidin-2-yl]amino}cyclohexyl)thiourea | 431 (M + H) | 2 |
| 2267 | N-[4-(dimethylamino)-1-naphthyl]-N'-(cis-4-{[4-(dimethylamino)pyrimidin-2-yl]amino}cyclohexyl)thiourea | 464 (M + H) | 2 |
| 2268 | N-(cis-4-{[4-(dimethylamino)pyrimidin-2-yl]amino}cyclohexyl)-N'-(2-ethylphenyl)thiourea | 399 (M + H) | 3 |
| 2269 | N-(cis-4-{[4-(dimethylamino)quinolin-2-yl]amino}cyclohexyl)-N'-(2-methoxy-4-nitrophenyl)thiourea | 495 (M + H) | 3 |
| 2270 | N-(cis-4-{[4-(dimethylamino)quinolin-2-yl]amino}cyclohexyl)-N'-(2-methoxy-5-methylphenyl)thiourea | 464 (M + H) | 3 |
| 2271 | N-(4-bromo-2-chlorophenyl)-N'-(cis-4-{[4-(dimethylamino}quinolin-2-yl]amino)cyclohexyl)thiourea | 532 (M + H) | 3 |
| 2272 | N-(cis-4-{[4-(dimethylamino)quinolin-2-yl]amino}cyclohexyl)-N'-(4-iodophenyl)thiourea | 546 (M + H) | 3 |
| 2273 | N-(cis-4-{[4-(dimethylamino)quinolin-2-yl]amino}cyclohexyl)-N'-(2,4,6-tribromophenyl)thiourea | 653 (M + H) | 1 |
| 2274 | N-(cis-4-{[4-(dimethylamino)quinolin-2-yl]amino}cyclohexyl)-N'-(2,4,6-trichlorophenyl)thiourea | 522 (M + H) | 2 |
| 2275 | N-(cis-4-{[4-(dimethylamino)quinolin-2-yl]amino}cyclohexyl)-N'-mesitylthiourea | 462 (M + H) | 1 |
| 2276 | N-(cis-4-{[4-(dimethylamino)quinolin-2-yl]amino}cyclohexyl)-N'-(2,4-dimethylphenyl)thiourea | 448 (M + H) | 3 |
| 2277 | N-(2,6-diethylphenyl)-N'-(cis-4-{[4-(dimethylamino)quinolin-2-yl]amino}cyclohexyl)thiourea | 476 (M + H) | 1 |
| 2278 | N-(2-bromo-4-methylphenyl)-N'-(cis-4-{[4-(dimethylamino)quinolin-2-yl]amino}cyclohexyl)thiourea | 512 (M + H) | 3 |
| 2279 | N-(2-chlorobenzyl)-N'-(cis-4-{[4-(dimethylamino)quinolin-2-yl]amino}cyclohexyl)thiourea | 468 (M + H) | 3 |
| 2280 | N-(cis-4-{[4-(dimethylamino)quinolin-2-yl]amino}cyclohexyl)-N'-(2-ethyl-6-methylphenyl)thiourea | 462 (M + H) | 3 |
| 2281 | N-(cis-4-{[4-(dimethylamino)quinolin-2-yl]amino}cyclohexyl)-N'-(2-isopropylphenyl)thiourea | 462 (M + H) | 3 |
| 2282 | methyl 3-({[(cis-4-{[4-(dimethylamino)quinolin-2-yl]amino}cyclohexyl)amino]carbonothioyl}amino)benzoate | 478 (M + H) | 3 |

(continued)

| Ex. No. | compound name | MS | class |
|---|---|---|---|
| 2283 | N-(4-bromo-2,6-dimethylphenyl)-N'-(cis-4-{[4-(dimethylamino) quinolin-2-yl]amino}cyclohexyl)thiourea | 526 (M + H) | 1 |
| 2284 | N-(4-bromo-2-methylphenyl)-N'-(cis-4-{[4-(dimethylamino) quinolin-2-yl]amino}cyclohexyl)thiourea | 512 (M + H) | 2 |
| 2285 | N-[4-bromo-2-(trifluoromethyl)phenyl]-N'-(cis-4-{ [4-(dimethylamino)quinolin-2-yl]amino}cyclohexyl)thiourea | 566 (M + H) | 2 |
| 2286 | N-(4-chloro-2-methylphenyl)-N'-(cis-4-{[4-(dimethylamino) quinolin-2-yl]amino}cyclohexyl)thiourea | 468 (M + H) | 3 |
| 2287 | N-(cis-4-{[4-(dimethylamino)quinolin-2-yl]amino}cyclohexyl) -N'-(1-naphthylmethyl)thiourea | 484 (M + H) | 3 |
| 2288 | N-(2,3-dimethoxybenzyl)-N'-(cis-4-{[4-(dimethylamino)quinolin-2-yl]amino}cyclohexyl)thiourea | 494 (M + H) | 3 |
| 2289 | N-(cis-4-{[4-(dimethylamino)quinolin-2-yl]amino}cyclohexyl) -N'-(2,4,5-trimethylphenyl)thiourea | 462 (M + H) | 3 |
| 2290 | N-biphenyl-2-yl-N'-(cis-4-{[4-(dimethylamino)quinolin-2-yl]amino} cyclohexyl)thiourea | 496 (M + H) | 3 |
| 2291 | N-(cis-4-{[4-(dimethylamino)quinolin-2-yl]amino}cyclohexyl) -N'-(2-methyl-4-nitrophenyl)thiourea | 479 (M + H) | 3 |
| 2292 | N-(3-chlorobenzyl)-N'-(cis-4-{[4-(dimethylamino)quinolin-2-yl] amino}cyclohexyl)thiourea | 468 (M + H) | 3 |
| 2293 | ethyl 3-({[(cis-4-{[4-(dimethylamino)quinolin-2-yl]amino} cyclohexyl)amino]carbonothioyl}amino)benzoate | 492 (M + H) | 3 |
| 2294 | N [4-chloro-2-(trifluoromethyl)phenyl]-N'-(cis-4-{ [4-(dimethylamino)quinolin-2-yl]amino}cyclohexyl)thiourea | 522 (M + H) | 3 |
| 2295 | N-(cis-4-{[4-(dimethylamino)quinolin-2-yl]amino}cyclohexyl) -N'-(4-fluoro-2-methylphenyl)thiourea | 452 (M + H) | 3 |
| 2296 | N-(cis-4-{[4-(dimethylamino)quinolin-2-yl]amino}cyclohexyl) -N'-(4-methoxy-2-methylphenyl)thiourea | 464 (M + H) | 3 |
| 2297 | N-(5-chloro-2,4-dimethoxyphenyl)-N'-(cis-4-{[4-(dimethylamino) quinolin-2-yl]amino}cyclohexyl)thiourea | 514 (M + H) | 1 |
| 2298 | N-(cis-4-{[4-(dimethylamino)quinolin-2-yl]amino}cyclohexyl) -N'-[(1R)-1-phenylethyl]thiourea | 448 (M + H) | 3 |
| 2299 | N-(cis-4-{[4-(dimethylamino)quinolin-2-yl]amino}cyclohexyl) -N'-(2,3-dimethylphenyl)thiourea | 448 (M + H) | 3 |
| 2300 | N-(2,4-dibromo-6-fluorophenyl)-N'-(cis-4-{[4-(dimethylamino) quinolin-2-yl]amino}cyclohexyl)thiourea | 594 (M + H) | 2 |
| 2301 | N-(2,4-dichloro-6-methylphenyl)-N'-(cis-4-{[4-(dimethylamino) quinolin-2-yl]amino}cyclohexyl)thiourea | 502 (M + H) | 1 |
| 2302 | N-(cis-4-{[4-(dimethylamino)quinolin-2-yl]amino)cyclohexyl) -N'-(2-ethoxyphenyl)thiourea | 464 (M + H) | 3 |
| 2303 | N-(cis-4-{[4-(dimethylamino)quinolin-2-yl]amino}cyclohexyl) -N'-(2-isopropyl-6-methylphenyl)thiourea | 476 (M + H) | 3 |
| 2304 | N-(2,3-dihydro-1,4-benzodioxin-6-yl)-N'-(cis-4-{ [4-(dimethylamino)quinolin-2-yl]amino}cyclohexyl)thiourea | 478 (M + H) | 3 |

(continued)

| Ex. No. | compound name | MS | class |
|---|---|---|---|
| 2305 | N-1,3-benzodioxol-5-yl-N'-(cis-4-{[4-(dimethylamino)quinolin-2-yl]amino}cyclohexyl)thiourea | 464 (M + H) | 3 |
| 2306 | N-(3-chloro-2-methylphenyl)-N'-(cis-4-{[4-(dimethylamino)quinolin-2-yl]amino}cyclohexyl)thiourea | 468 (M + H) | 3 |
| 2307 | N-[4-bromo-2-(trifluoromethoxy)phenyl]-N'-(cis-4-{[4-(dimethylamino)quinolin-2-yl]amino}cyclohexyl)thiourea | 582 (M + H) | 3 |
| 2308 | N-(4-chloro-2,5-dimethoxyphenyl)-N'-(cis-4-{[4-(dimethylamino)quinolin-2-yl]amino}cyclohexyl)thiourea | 514 (M + H) | 3 |
| 2309 | N-(cis-4-{[4-(dimethylamino)quinolin-2-yl]amino}cyclohexyl)-N'-(5-methyl-3-phenylisoxazol-4-yl)thiourea | 501 (M + H) | 3 |
| 2310 | N-bicyclo[2.2.1]hept-2-yl-N'-(cis-4-{[4-(dimethylamino)quinolin-2-yl]amino}cyclohexyl)thiourea | 438 (M + H) | 3 |
| 2311 | methyl 3-({[(cis-4-{[4-(dimethylamino)quinolin-2-yl]amino}cyclohexyl)amino]carbonothioyl}amino)-4-methylthiophene-2-carboxylate | 498 (M + H) | 2 |
| 2312 | methyl 3-({[(cis-4-{[4-(dimethylamino)quinolin-2-yl]amino}cyclohexyl)amino]carbonothioyl)amino)thiophene-2-carboxylate | 484 (M + H) | 3 |
| 2313 | N-(4-butyl-2-methylphenyl)-N'-(cis-4-{[4-(dimethylamino)quinolin-2-yl]amino}cyclohexyl)thiourea | 490 (M + H) | 3 |
| 2314 | N-(cis-4-{[4-(dimethylamino)pyrimidin-2-yl]amino}cyclohexyl)-N'-(2-methoxy-4-nitrophenyl)thiourea | 446 (M + H) | 3 |
| 2315 | N-(cis-4-{[4-(dimethylamino)pyrimidin-2-yl]amino}cyclohexyl)-N'-(2-methoxy-5-methylphenyl)thiourea | 413 (M - H) | 3 |
| 2316 | N-(4-bromo-2-chlorophenyl)-N'-(cis-4-{[4-(dimethylamimo)pyrimidin-2-yl]amino}cyclohexyl)thiourea | 483 (M + H) | 3 |
| 2317 | N-(cis-4-{([4-(dimethylamino)pyrimidin-2-yl]amino}cyclohexyl)-N'-(4-iodophenyl)thiourea | 497 (M + H) | 3 |
| 2318 | N-(cis-4-{[4-(dimethylamino)pyrimidin-2-yl]amino}cyclohexyl)-N'-(2,4,6-tribromophenyl)thiourea | 604 (M + H) | 1 |
| 2319 | N-(cis-4-{[4-(dimethylamino)pyrimidin-2-yl]amino}cyclohexyl)-N'-(2,4,6-trichlorophenyl)thiourea | 473 (M + H) | 3 |
| 2320 | N-(cis-4-{[4-(dimethylamino)pyrimidin-2-yl]amino}cyclohexyl)-N'-mesitylthiourea | 413 (M + H) | 1 |
| 2321 | N-(cis-4-{[4-(dimethylamino)pyrimidin-2-yl]amino}cyclohexyl)-N'-(2,4-dimethylphenyl)thiourea | 399 (M + H) | 3 |
| 2322 | N-(2,6-diethylphenyl)-N'-(cis-4-{[4-(dimethylamino)pyrimidin-2-yl]amino}cyclohexyl)thiourea | 427 (M + H) | 3 |
| 2323 | N-(2-bromo-4-methylphenyl)-N'-(cis-4-{[4-(dimethylamino)pyrimidin-2-yl]amino}cyclohexyl)thiourea | 463 (M + H) | 3 |
| 2324 | N-(2-chlorobenzyl)-N'-(cis-4-{[4-(dimethylamino)pyrimidin-2-yl]amino}cyclohexyl)thiourea | 419 (M + H) | 3 |
| 2325 | N-(cis-4-{[4-(dimethylamino)pyrimidin-2-yl]amino}cyclohexyl)-N'-(2-ethyl-6-methylphenyl)thiourea | 413 (M + H) | 3 |
| 2326 | N-(cis-4-{[4-(dimethylamino)pyrimidin-2-yl]amino}cyclohexyl)-N'-(2-isopropylphenyl)thiourea | 413 (M + H) | 3 |

(continued)

| Ex. No. | compound name | MS | class |
|---|---|---|---|
| 2327 | methyl 3-({[(cis-4-{[4-(dimethylamino)pyrimidin-2-yl]amino} cyclohexyl)amino]carbonothioyl}amino)benzoate | 429 (M + H) | 3 |
| 2328 | N-(4-bromo-2,6-dimethylphenyl)-N'-(cis-4-{[4-(dimethylamino) pyrimidin-2-yl]amino}cyclohexyl)thiourea | 477 (M + H) | 1 |
| 2329 | N-(4-bromo-2-methylphenyl)-N'-(cis-4-{[4-dimethylamino) pyrimidin-2-yl]amino}cyclohexyl)thiourea | 463 (M + H) | 3 |
| 2330 | N-{[4-bromo-2-(trifluoromethyl)phenyl]-N'-(cis-4-([4-(dimethylamino}pyrimidin-2-yl]amino]cyclohexyl)thiourea | 517 (M + H) | 3 |
| 2331 | N-(4-chloro-2-methylphenyl)-N'-(cis-4-{[4-(dimethylamino) pyrimidin-2-yl]amino}cyclohexyl)thiourea | 419 (M + H) | 3 |
| 2332 | N-(cis-4-{[4-(dimethylamino)pyrimidin-2-yl]amino}cyclohexyl) -N'-(1 -naphthylmethyl)thiourea | 435 (M + H) | 3 |
| 2333 | N-(2,3-dimethoxybenzyl)-N'-(cis-4-{[4-(dimethylamino)pyrimidin-2-yl]amino}cyclohexyl)thiourea | 443 (M - H) | 3 |
| 2334 | N-(cis-4-{[4-(dimethylamino)pyrimidin-2-yl]amino}cyclohexyl) -N'-(2,4,5-trimethylphenyl)thiourea | 413 (M + H) | 3 |
| 2335 | N-biphenyl-2-yl-N'-(cis-4-{[4-(dimethylamino)pyrimidin-2-yl] amino}cyclohexyl)thiourea | 447 (M + H) | 3 |
| 2336 | N-(cis-4-{[4-(dimethylamino)pyrimidin-2-yl]amino}cyclohexyl) -N'-(2-methyl-4-nitrophenyl)thiourea | 428 (M - H) | 3 |
| 2337 | N-(3-chlorobenzyl)-N'-(cis-4-{[4-(dimethylamino)pyrimidin-2-yl] amino}cyclohexyl)thiourea | 419 (M + H) | 3 |
| 2338 | ethyl 3-({[(cis-4-{[4-(dimethylamino)pyrimidin-2-yl]amino} cyclohexyl)amino]carbonothioyl}amino)benzoate | 441 (M - H) | 3 |
| 2339 | N-[4-chloro-2-(trifluoromethyl)phenyl]-N'-(cis-4-{[4-(dimethylamino)pyrimidin-2-yl]amino}cyclohexyl)thiourea | 473 (M + H) | 3 |
| 2340 | N-(cis-4-{[4-(dimethylamino)pyrimidin-2-yl]amino}cyclohexyl)-N'-(4-fluoro-2-methylphenyl)thiourea | 403 (M + H) | 3 |
| 2341 | N-(cis-4-{[4-(dimethylamino)pyrimidin-2-yl]amino}cyclohexyl) -N'-(4-methoxy-2-methylphenyl)thiourea | 415 (M + H) | 3 |
| 2342 | N-(5-chloro-2,4-dimethoxyphenyl)-N'-(cis-4-{[4-(dimethylamino) pyrimidin-2-yl]amino}cyclohexyl)thiourea | 465 (M + H) | 1 |
| 2343 | N-(cis-4-{[4-(dimethylamino)pyrimidin-2-yl]amino}cyclohexyl)-N'-[(1R)-1-phenylethyl]thiourea | 397 (M - H) | 3 |
| 2344 | N-(cis-4-{[4-(dimethylamino)pyrimidin-2-yl]amino}cyclohexyl) -N'-(2,3-dimethylphenyl)thiourea | 399 (M + H) | 3 |
| 2345 | N-(2,4-dibromo-6-fluorophenyl)-N'-(cis-4-{[4-(dimethylamino) pyrimidin-2-yl]amino}cyclohexyl)thiourea | 545 (M + H) | 2 |
| 2346 | N-(2,4-dichloro-6-methylphenyl)-N'-(cis-4-{[4-(dimethylamino) pyrimidin-2-yl]amino}cyclohexyl)thiourea | 453 (M + H) | 2 |
| 2347 | N-(cis-4-{[4-(dimethylamino)pyrimidin-2-yl]amino}cyclohexyl) -N'-(2-ethoxyphenyl)thiourea | 415 (M + H) | 3 |
| 2348 | N-(cis-4-{[4-(dimethylamino)pyrimidin-2-yl]amino}cyclohexyl) -N'-(2-isopropyl-6-methylphenyl)thiourea | 427 (M + H) | 3 |

(continued)

| Ex. No. | compound name | MS | class |
|---|---|---|---|
| 2349 | N-(2,3-dihydro-1,4-benzodioxin-6-yl)-N'-(cis-4-{[4-(dimethylamino)pyrimidin-2-yl]amino}cyclohexyl)thiourea | 429 (M + H) | 3 |
| 2350 | N-1,3-benzodioxol-5-yl-N'-(cis-4-{[4-(dimethylamino)pyrimidin-2-yl]amino}cyclohexyl)thiourea | 415 (M + H) | 3 |
| 2351 | N-(3-chloro-2-methylphenyl)-N'-(cis-4-{[4-(dimethylamino) pyrimidin-2-yl]amino}cyclohexyl)thiourea | 419 (M + H) | 3 |
| 2352 | N-[4-bromo-2-(trifluoromethoxy)phenyl]-N'-(cis-4-{[4-(dimethylamino)pyrimidin-2-yl]amino}cyclohexyl)thiourea | 533 (M + H) | 3 |
| 2353 | N-(4-chloro-2,5-dimethoxyphenyl)-N'-(cis-4-{[4-(dimethylamino) pyrimidin-2-yl]amino}cyclohexyl)thiourea | 465 (M + H) | 3 |
| 2354 | N-(cis-4-{[4-(dimethylamino)pyrimidin-2-yl]amino}cyclohexyl) -N'-(5-methyl-3-phenylisoxazol-4-yl)thiourea | 452 (M + H) | 3 |
| 2355 | N-bicyclo[2.2.1]hept-2-yl-N'-(cis-4-{[4-(dimethylamino)pyrimidin-2-yl]amino}cyclohexyl)thiourea | 387 (M-H) | 3 |
| 2356 | methyl 3-({[(cis-4-{[4-(dimethylamino)pyrimidin-2-yl]amino} cyclohexyl)amino]carbonothioyl}amino)-4-methylthiophene-2-carboxylate | 449 (M + H) | 3 |
| 2357 | methyl 3-({[(cis-4-{[4-(dimethylamino)pyrimidin-2-yl]amino} cyclohexyl)amino]carbonothioyl}amino)thiophene-2-carboxylate | 435 (M + H) | 3 |
| 2358 | N-(4-butyl-2-methylphenyl)-N'-(cis-4-([4-(dimethylamino) pyrimidin-2-yl]amino)cyclohexyl)thiourea | 441 (M + H) | 3 |
| 2359 | N-(2-chlorophenyl)-N'-[(cis-4-{[4-(dimethylamino)quinolin-2-yl] amino}cyclohexyl)methyl]urea | 452 (M + H) | 3 |
| 2360 | N-[(cis-4-{[4-(dimethylamino)quinolin-2-yl]amino}cyclohexyl) methyl]-N'-(2,6-dimethylphenyl)urea | 446 (M + H) | 3 |
| 2361 | N-(2,4-difluorophenyl)-N'-[(cis-4-{[4-(dimethylamino)quinolin-2-yl] amino}cyclohexyl)methyl]urea | 454 (M + H) | 3 |
| 2362 | N-[(cis-4-{[4-(dimethylamino)quinolin-2-yl]amino}cyclohexyl) methyl]-N'-(2-ethyl-6-methylphenyl)urea | 460 (M + H) | 2 |
| 2363 | ethyl N-({[(cis-4-{[4-(dimethylamino)quinolin-2-yl]amino} cyclohexyl)methyl]amino}carbonyl)leucinate | 484 (M + H) | 3 |
| 2364 | N-[(cis-4-{[4-(dimethylamino)quinolin-2-yl]amino}cyclohexyl) methyl]-N'-(4-fluorophenyl)urea | 436 (M + H) | 3 |
| 2365 | N-[(cis-4-{[4-(dimethylamino)quinolin-2-yl]amino}cyclohexyl) methyl]-N'-[4-(methylthio)phenyl]urea | 464 (M + H) | 3 |
| 2366 | N-[(cis-4-{[4-(dimethylamino)quinolin-2-yl]amino}cyclohexyl) methyl]-N'-[2-(trifluoromethyl)phenyl]urea | 486 (M + H) | 3 |
| 2367 | N-[(cis-4-{ [4-(dimethylamino)quinolin-2-yl]amino}cyclohexyl) methyl]-N'-mesitylurea | 460 (M + H) | 2 |
| 2368 | N-[(cis-4-{[4-(dimethylamino)quinolin-2-yl]amino}cyclohexyl) methyl]-N'-(2-methylphenyl)urea | 432 (M + H) | 3 |
| 2369 | N [(cis-4-{[4-(dimethylamino)quinolin-2-yl]amino}cyclohexyl) methyl]-N'-(2,4,6-trichlorophenyl)urea | 520 (M + H) | 1 |
| 2370 | N-[(cis-4-{[4-(dimethylamino)quinolin-2-yl]amino}cyclohexyl) methyl]-N'-(2,4,6-tribromophenyl)urea | 651 (M + H) | 1 |

(continued)

| Ex. No. | compound name | MS | class |
|---|---|---|---|
| 2371 | N-(2,4-dibromo-6-fluorophenyl)-N'-[(cis-4-{[4-(dimethylamino) quinolin-2-yl]amino}cyclohexyl)methyl]urea | 592 (M + H) | 1 |
| 2372 | N-(2,6-diethylphenyl)-N'-[(cis-4-([4-(dimethylamino)quinolin-2-yl] amino}cyclohexyl)methyl]urea | 474 (M + H) | 2 |
| 2373 | N-[2-chloro-6-(trifluoromethyl)phenyl]-N'-[(cis-4-{ [4-(dimethylamino)quinolin-2-yl]amino}cyclohexyl)methyl]urea | 520 (M + H) | 2 |
| 2374 | N-(2-chloro-6-methylphenyl)-N'-[(cis-4-{[4-(dimethylamino) quinolin-2-yl]amino}cyclohexyl)methyl]urea | 466 (M + H) | 3 |
| 2375 | N-(2-chlorobenzyl)-N'-[(cis-4-{[4-(dimethylamino)quinolin-2-yl] amino}cyclohexyl)methyl]urea | 466 (M + H) | 3 |
| 2376 | N-[(cis-4-{[4-(dimethylamino)quinolin-2-yl]amino}cyclohexyl) methyl]-N'-(2-ethyl-6-isopropylphenyl)urea | 488 (M + H) | 1 |
| 2377 | N-[(cis-4-{[4-(dimethylamino)quinolin-2-yl]amino}cyclohexyl) methyl]-N'-(2-ethylphenyl)urea | 446 (M + H) | 3 |
| 2378 | N-[(cis-4-{[4-(dimethylamino)quinolin-2-yl]amino}cyclohexyl) methyl]-N'-(2-iodophenyl)urea | 544 (M + H) | 3 |
| 2379 | N-[(cis-4-{[4-(dimethylamino)quinolin-2-yl]amino}cyclohexyl)-methyl]-N'-(2-isopropyl-6-methylphenyl)urea | 474 (M + H) | 2 |
| 2380 | N-[(cis-4-{[4-(dimethylamino)quinolin-2-yl]amino}cyclohexyl) methyl]-N'-(2-isopropylphenyl)urea | 460 (M + H) | 3 |
| 2381 | N-[(cis-4-{[4-(dimethylamino)quinolin-2-yl]amino}cyclohexyl) methyl]-N'-(2-methyl-3-nitrophenyl)urea | 477 (M + H) | 2 |
| 2382 | N-[(cis-4-{[4-(dimethylamino)quinolin-2-yl]amino}cyclohexyl) methyl]-N'-(2-propylphenyl)urea | 460 (M + H) | 3 |
| 2383 | N-(2-tert-butyl-6-methylphenyl)-N'-[(cis-4-{[4-(dimethylamino) quinolin-2-yl]amino}cyclohexyl)methyl]urea | 488 (M + H) | 1 |
| 2384 | N-(2-tert-butylphenyl)-N'-[(cis-4-{[4-(dimethylamino)quinolin-2-yl] amino}cyclohexyl)methyl]urea | 474 (M + H) | 1 |
| 2385 | N-(3-chloro-2-methylphenyl)-N'-[(cis-4-{[4-(dimethylamino) quinolin-2-yl]amino}cyclohexyl)methyl]urea | 466 (M + H) | 3 |
| 2386 | N-(4-bromo-2,6-difluorophenyl}-N'-[(cis-4-[4-(dimethylamino) quinolin-2-yl]amino}cyclohexyl)methyl]urea | 532 (M + H) | 3 |
| 2387 | N-[4-chloro-2-(trifluoromethyl)phenyl]-N'-[(cis-4-{ [4-(dimethylamino)quinolin-2-yl]amino}cyclohexyl)methyl]urea | 520 (M + H) | 3 |
| 2388 | N-(4-cyanophenyl)-N'-[(cis-4-{[4-(dimethylamino)quinolin-2-yl] amino}cyclohexyl)methyl]urea | 443 (M + H) | 3 |
| 2389 | N[(cis-4-{[4-(dimethylamino)quinolin-2-yl]amino}cyclohexyl) methyl]-N'-(diphenylmethyl)urea | 508 (M + H) | 2 |
| 2390 | N-(4-bromo-2,6-dimethylphenyl)-N'-[(cis-4-{[4-(dimethylamino) quinolin-2-yl]amino}cyclohexyl)methyl]urea | 524 (M + H) | 1 |
| 2391 | N-[(cis-4-{[4-(dimethylamino}quinolin-2-yl]amino)cyclohexyl)-methyl]-N'-(3-methyl-5-phenylisoxazol-4-yl)urea | 499 (M + H) | 3 |
| 2392 | N-[(cis-4-{[4-(dimethylamino)quinolin-2-yl]amino}cyclohexyl)-methyl]-N'-[5-methyl-2-(trifluoromethyl)-3-furyl]urea | 490 (M + H) | 3 |

(continued)

| Ex. No. | compound name | MS | class |
|---|---|---|---|
| 2393 | N-(3,5-dichlorophenyl)-N'-[(cis-4-{[4-(dimethylamino)quinolin-2-yl]amino}cyclohexyl)methyl]urea | 486 (M + H) | 3 |
| 2394 | N-(2,3-dichlorophenyl)-N'-[(cis-4-{[4-(dimethylamino)quinolin-2-yl]amino}cyclohexyl)methyl]urea | 486 (M + H) | 2 |
| 2395 | N-[(cis-4-{[4-(dimethylamino)quinolin-2-yl]amino}cyclohexyl) methyl]-N'-(4-methylphenyl)urea | 432 (M + H) | 3 |
| 2396 | N-(2,6-diisopropylphenyl)-N'-[(cis-4-{[4-(dimethylamino)quinolin-2-yl]amino}cyclohexyl)methyl]urea | 502 (M + H) | 1 |
| 2397 | N [(cis-4-{[4-(dimethylamino)quinolin-2-yl]amino}cyclohexyl)-methyl]-N'-(2,3-dimethyl-6-nitrophenyl)urea | 491 (M + H) | 3 |
| 2398 | N-(2,6-dibromo-4-fluorophenyl)-N'-[(cis-4-{[4-(dimethylamino) quinolin-2-yl]amino}cyclohexyl)methyl]urea | 592 (M + H) | 3 |
| 2399 | N-(2,6-dichlorophenyl)-N'-[(cis-4-{[4-(dimethylamino)quinolin-2-yl]amino}cyclohexyl)methyl]urea | 486 (M + H) | 3 |
| 2400 | N-[(cis-4-{[4-(dimethylamino)quinolin-2-yl]amino}cyclohexyl)-methyl]-N'-(2-methoxy-5-methylphenyl)urea | 462 (M + H) | 3 |
| 2401 | N-[(cis-4-{[4-(dimethylamino)quinolin-2-yl]amino}cyclohexyl) methyl]-N'-(2-methyl-6-nitrophenyl)urea | 477 (M + H) | 3 |
| 2402 | N-(3,4-difluorophenyl)-N'-[(cis-4-{[4-(dimethylamino)quinolin-2-yl] amino}cyclohexyl)methyl]urea | 454 (M + H) | 3 |
| 2403 | N-(3,5-difluorophenyl)-N'-[(cis-4-{[4-(dimethylamino)quinolin-2-yl] amino}cyclohexyl)methyl]urea | 454 (M + H) | 3 |
| 2404 | N-(3-chloro-4-fluorophenyl)-N'-[(cis-4-{[4-(dimethylamino) quinolin-2-yl]amino}cyclohexyl)methyl]urea | 470 (M + H) | 3 |
| 2405 | N-(2-chlorophenyl)-N'-[(cis-4-{[4-(dimethylamino)pyrimidin-2-yl] amino}cyclohexyl)methyl]urea | 403 (M + H) | 3 |
| 2406 | N-[(cis-4-{[4-(dimethylamino)pyrimidin-2-yl]amino}cyclohexyl) methyl]-N'-(2,6-dimethylphenyl)urea | 397 (M + H) | 1 |
| 2407 | N-(2,4-difluorophenyl)-N'-[(cis-4-{[4-(dimethylamino)pyrimidin-2-yl]amino}cyclohexyl)methyl]urea | 405 (M + H) | 2 |
| 2408 | N-[(cis-4-{[4-(dimethylamino)pyrimidin-2-yl]amino}cyclohexyl) methyl]-N'-(2-ethyl-6-methylphenyl)urea | 411 (M + H) | 1 |
| 2409 | ethyl N-({[(cis-4-{[4-(dimethylamino)pyrimidin-2-yl]amino} cyclohexyl)methyl]amino}carbonyl)leucinate | 435 (M + H) | 3 |
| 2410 | N-[(cis-4-{[4-(dimethylamino)pyrimidin-2-yl]amino}cyclohexyl) methyl]-N'-(4-fluorophenyl)urea | 387 (M + H) | 2 |
| 2411 | N-[(cis-4-{[(4-(dimethylamino)pyrimidin-2-yl]amino}cyclohexyl) methyl]-N'-[4-(methylthio)phenyl]urea | 415 (M + H) | 3 |
| 2412 | N- [(cis-4-{[4-(dimethylamino)pyrimidin-2-yl]amino cyclohexyl) methyl]-N'-[2-(trifluoromethyl)phenyl]urea | 435 (M - H) | 3 |
| 2413 | N-[(cis-4-{[4-(dimethylamino)pyrimidin-2-yl]amino}cyclohexyl) methyl]-N'-mesitylurea | 411 (M + H) | 1 |
| 2414 | N-[(cis-4-{[4-(dimethylamino)pyrimidin-2-yl]amino}cyclohexyl) methyl]-N'-(2-methylphenyl)urea | 383 (M + H) | 3 |

(continued)

| Ex. No. | compound name | MS | class |
|---|---|---|---|
| 2415 | N-[(cis-4-[4-{(dimethylamino)pyrimidin-2-yl]amino}cyclohexyl) methyl]-N'-(2,4,6-trichlorophenyl)urea | 471 (M + H) | 1 |
| 2416 | N-[(cis-4-{[4-(dimethylamino)pyrimidin-2-yl]amino}cyclohexyl) methyl]-N'-(2,4,6-tribromophenyl)urea | 602 (M + H) | 1 |
| 2417 | N-(2,4-dibromo-6-fluorophenyl)-N'-[(cis-4-[{4-(dimethylamino) pyrimidin-2-yl]amino}cyclohexyl)methyl]urea | 543 (M + H) | 1 |
| 2418 | N-(2,6-diethylphenyl)-N'-[(cis-4-{[4-(dimethylamino)pyrimidin-2-yl]amino}cyclohexyl)methyl]urea | 425 (M + H) | 1 |
| 2419 | N-[2-chloro-6-(trifluoromethyl)phenyl]-N'-[(cis-4-{[4-(dimethylamino)pyrimidin-2-yl]amino}cyclohexyl)methyl]urea | 471 (M + H) | 1 |
| 2420 | N-(2-chloro-6-methylphenyl)-N'-[(cis-4-{[4-(dimethylamino) pyrimidin-2-yl]amino}cyclohexyl)methyl]urea | 417 (M + H) | 1 |
| 2421 | N-(2-chlorobenzyl)-N'-[(cis-4-{[4-(dimethylamino)pyrimidin-2-yl] amino}cyclohexyl)methyl]urea | 417 (M + H) | 3 |
| 2422 | N-[(cis-4-{[4-(dimethylamino)pyrimidin-2-yl]amino}cyclohexyl) methyl]-N'-(2-ethyl-6-isopropylphenyl)urea | 437 (M-H) | 1 |
| 2423 | N-[(cis-4-{[4-(dimethylamino)pyrimidin-2-yl]amino}cyclohexyl) methyl]-N'-(2-ethylphenyl)urea | 397 (M + H) | 3 |
| 2424 | N-[(cis-4-{[4-(dimethylamino)pyrimidin-2-yl]amino}cyclohexyl) methyl]-N'-(2-iodophenyl)urea | 495 (M+H) | 3 |
| 2425 | N-[(cis-4-{[4-(dimethylamino)pyrimidin-2-yl]amino}cyclohexyl)-methyl]-N'-(2-isopropyl-6-methylphenyl)urea | 425 (M + H) | 1 |
| 2426 | N-[(cis-4-{[4-(dimethylamino)pyrimidin-2-yl]amino}cyclohexyl) methyl]-N'-(2-isopropylphenyl)urea | 411 (M + H) | 3 |
| 2427 | N-[(cis-4-{[4-(dimethylamino)pyrimidin-2-yl]amino}cyclohexyl) methyl]-N'-(2-methyl-3-nitrophenyl)urea | 428 (M+H) | 1 |
| 2428 | N-[(cis-4-{[4-(dimethylamino)pyrimidin-2-yl]amino}cyclohexyl) methyl]-N'-(2-propylphenyl)urea | 411 (M + H) | 2 |
| 2429 | N-(2-tert-butyl-6-methylphenyl)-N'-[(cis-4-{[4-(dimethylamino) pyrimidin-2-yl]amino}cyclohexyl)methyl]urea | 439 (M + H) | 1 |
| 2430 | N-(2-tert-butylphenyl)-N'-[(cis-4-{[4-(dimethylamino)pyrimidin-2-yl]amino}cyclohexyl)methyl]urea | 425 (M + H) | 1 |
| 2431 | N-(3-chloro-2-methylphenyl)-N'-[(cis-4-{[4-(dimethylamino) pyrimidin-2-yl]amino}cyclohexyl)methyl]urea | 417 (M + H) | 1 |
| 2432 | N-(4-bromo-2,6-difluorophenyl)-N'-[(cis-4-{[4-(dimethylamino) pyrimidin-2-yl]amino}cyclohexyl)methyl]urea | 483 (M+ H) | 1 |
| 2433 | N-[4-chloro-2-(trifluoromethyl)phenyl]-N'-[(cis-4-{[4-(dimethylamino)pyrimidin-2-yl]amino}cyclohexyl)methyl]urea | 471 (M + H) | 2 |
| 2434 | N-(4-cyanophenyl)-N'-[(cis-4-{[4-(dimethylamino)pyrimidin-2-yl] amino}cyclohexyl)methyl]urea | 394 (M + H) | 3 |
| 2435 | N-[(cis-4-{[4-(dimethylamino)pyrimidin-2-yl]amino}cyclohexyl) methyl]-N'-(diphenylmethyl)urea | 459 (M+H) | 1 |
| 2436 | N-(4-bromo-2,6-dimethylphenyl)-N'-[(cis-4-{[4-dimethylamino) pyrimidin-2-yl]amino cyclohexyl)methyl]urea | 475 (M + H) | 1 |

(continued)

| Ex. No. | compound name | MS | class |
|---|---|---|---|
| 2437 | N-[(cis-4-{[4-(dimethylamino)pyrimidin-2-yl]amino]cyclohexyl)-methyl]-N'-(3-methyl-5-phenylisoxazol-4-yl)urea | 450 (M+H) | 1 |
| 2438 | N-[(cis-4-{[4-(dimethylamino)pyrimidin-2-yl]amino}cyclohexyl)-methyl]-N'-[5-methyl-2-(trifluoromethyl)-3-furyl]urea | 441 (M + H) | 3 |
| 2439 | N-(3,5-dichlorophenyl)-N'-[(cis-4-{[4-(dimethylamino)pyrimidin-2-yl]amino}cyclohexyl)methyl]urea | 437 (M+H) | 2 |
| 2440 | N-(2,3-dichlorophenyl)-N'-[(cis-4-{[4-(dimethylamino)pyrimidin-2-yl]amino}cyclohexyl)methyl]urea | 437 (M + H) | 1 |
| 2441 | N-[(cis-4-{[4-(dimethylamino)pyrimidin-2-yl]amino}cyclohexyl) methyl]-N'-(4-methylphenyl)urea | 383 (M + H) | 3 |
| 2442 | N-(2,6-diisopropylphenyl)-N'-[(cis-4-{[4-(dimethylamino) pyrimidin-2-yl]amino}cyclohexyl)methyl]urea | 453 (M+ H) | 1 |
| 2443 | N-[(cis-4-{[4-(dimethylamino)pyrimidin-2-yl]amino}cyclohexyl)-methyl]-N'-(2,3-dimethyl-6-nitrophenyl)urea | 442 (M + H) | 1 |
| 2444 | N-(2,6-dibromo-4-fluorophenyl)-N'-[(cis-4-{[4-(dimethylamino) pyrimidin-2-yl]amino}cyclohexyl)methyl]urea | 543 (M + H) | 1 |
| 2445 | N-(2,6-dichlorophenyl)-N'-[(cis-4-{[4-(dimethylamino)pyrimidin-2-yl]amino}cyclohexyl)methyl]urea | 437 (M + H) | 1 |
| 2446 | N-[(cis-4-{[4-(dimethylamino)pyrimidin-2-yl]amino}cyclohexyl)-methyl]-N'-(2-methoxy-5-methylphenyl)urea | 413 (M + H) | 2 |
| 2447 | N-[(cis-4-{[4-(dimethylamino)pyrimidin-2-yl]amino}cyclohexyl) methyl]-N'-(2-methyl-6-nitrophenyl)urea | 428 (M + H) | 2 |
| 2448 | N-(3,4-difluorophenyl)-N'-[(cis-4-{[4-(dimethylamino)pyrimidin-2-yl]amino}cyclohexyl)methyl]urea | 405 (M + H) | 1 |
| 2449 | N-(3,5-difluorophenyl)-N'-[(cis-4-{[4-(dimethylamino)pyrimidin-2-yl]amino}cyclohexyl)methyl]urea | 405 (M + H) | 1 |
| 2450 | N-(3-chloro-4-fluorophenyl)-N'-[(cis-4-{[4-(dimethylamino) pyrimidin-2-yl]amino}cyclohexyl)methyl]urea | 421 (M + H) | 1 |
| 2451 | N-(2-chlorophenyl)-N'-[(cis-4-{[4-(dimethylamino)-5,6,7,8-tetrahydroquinazolin-2-yl]amino}cyclohexyl)methyl]urea | 457 (M + H) | 3 |
| 2452 | N-[(cis-4-{[4-(dimethylamino)-5,6,7,8-tetrahydroquinazolin-2-yl] amino}cyclohexyl)methyl]-N'-(2,6-dimethylphenyl)urea | 451 (M + H) | 1 |
| 2453 | N-(2,4-difluorophenyl)-N'-[(cis-4-{[4-(dimethylamino)-5,6,7,8-tetrahydroquinazolin-2-yl]amino}cyclohexyl)methyl]urea | 459 (M + H) | 2 |
| 2454 | N-[(cis-4-{[4-(dimethylamino)-5,6,7,8-tetrahydroquinazolin-2-yl] amino}cyclohexyl)methyl]-N'-(2-ethyl-6-methylphenyl)urea | 465 (M + H) | 1 |
| 2455 | ethyl N-({[(cis-4-{[4-(dimethylamino)-5,6,7,8-tetrahydro quinazolin-2-yl]amino}cyclohexyl)methyl]amino}carbonyl)-leucinate | 489 (M + H) | 2 |
| 2456 | N-[(cis-4-{[4-(dimethylamino)-5,6,7,8-tetrahydroquinazolin-2-yl] amino}cyclohexyl)methyl]-N'-(4-fluorophenyl)urea | 441 (M + H) | 2 |
| 2457 | N-[(cis-4-{[4-(dimethylamino)-5,6,7,8-tetrahydroquinazolin-2-yl] amino}cyclohexyl)methyl]-N'-[4-(methylthio)phenyl]urea | 469 (M + H) | 3 |
| 2458 | N-[(cis-4-{[4-(dimethylamino)-5,6,7,8-tetrahydroquinazolin-2-yl] amino}cyclohexyl)methyl]-N'-[2-(trifluoromethyl)phenyl]urea | 491 (M + H) | 3 |

(continued)

| Ex. No. | compound name | MS | class |
|---|---|---|---|
| 2459 | N-[(cis-4-{[4-(dimethylamino)-5,6,7,8-tetrahydroquinazolin-2-yl] amino}cyclohexyl)methyl]-N'-mesitylurea | 465 (M + H) | 1 |
| 2460 | N-[(cis-4-{[4-(dimethylamino)-5,6,7,8-tetrahydroquinazolin-2-yl] amino}cyclohexyl)methyl]-N'-(2-methylphenyl)urea | 437 (M + H) | 3 |
| 2461 | N-[(cis-4-{[4-(dimethylamino)-5,6,7,8-tetrahydroquinazolin-2-yl] amino}cyclohexyl)methyl]-N'-(2,4,6-trichlorophenyl)urea | 525 (M + H) | 1 |
| 2462 | N-[(cis-4-{[4-(dimethylamino)-5,6,7,8-tetrahydroquinazolin-2-yl] amino}cyclohexyl)methyl]-N'-(2,4,6-tribromophenyl)urea | 657 (M + H) | 1 |
| 2463 | N-(2,4-dibromo-6-fluorophenyl)-N'-[(cis-4-{[4-(dimethylamino)-5,6,7,8-tetrahydroquinazolin-2-yl]amino}cyclohexyl)methyl]urea | 597 (M + H) | 3 |
| 2464 | N-(2,6-diethylphenyl)-N'-[(cis-4-{[4-(dimethylamino)-5,6,7,8-tetrahydroquinazolin-2-yl]amino}cyclohexyl)methyl]urea | 479 (M + H) | 1 |
| 2465 | N-[2-chloro-6-(trifluoromethyl)phenyl]-N'-[(cis-4-{ [4-(dimethylamino)-5,6,7,8-tetrahydroquinazolin-2-yl]amino} cyclohexyl)-methyl]urea | 525 (M + H) | 1 |
| 2466 | N-(2-chloro-6-methylphenyl)-N'-[(cis-4-{[4-(dimethylamino)-5,6,7,8-tetrahydroquinazolin-2-yl]amino}cyclohexyl)methyl]urea | 471 (M + H) | 1 |
| 2467 | N-(2-chlorobenzyl)-N'-[(cis-4-{[4-(dimethylamino)-5,6,7,8-tetrahydroquinazolin-2-yl]amino}cyclohexyl)methyl]urea | 471 (M + H) | 3 |
| 2468 | N-[(cis-4-{[4-(dimethylamino)-5,6,7,8-tetrahydroquinazolin-2-yl] amino}cyclohexyl)methyl]-N'-(2-ethyl-6-isopropylphenyl)urea | 493 (M + H) | 1 |
| 2469 | N [(cis-4-{[4-(dimethylamino)-5,6,7,8-tetrahydroquinazolin-2-yl] amino}cyclohexyl)methyl]-N'-(2-ethylphenyl)urea | 451 (M + H) | 3 |
| 2470 | N-[(cis-4-{[4-(dimethylamino)-5,6,7,8-tetrahydroquinazolin-2-yl] amino}cyclohexyl)methyl]-N'-(2-iodophenyl)urea | 549 (M + H) | 3 |
| 2471 | N-[(cis-4-{[4-(dimethylamino)-5,6,7,8-tetrahydroquinazolin-2-yl] amino}cyclohexyl)methyl]-N'-(2-isopropyl-6-methylphenyl)-urea | 479 (M + H) | 2 |
| 2472 | N-[(cis-4-{[4-(dimethylamino)-5,6,7,8-tetrahydroquinazolin-2-yl] amino}cyclohexyl)methyl]-N'-(2-isopropylphenyl)urea | 465 (M + H) | 3 |
| 2473 | N-[(cis-4-{[4-(dimethylamino)-5,6,7,8-tetrahydroquinazolin-2-yl] amino}cyclohexyl)methyl]-N'-(2-methyl-3-nitrophenyl)urea | 482 (M + H) | 3 |
| 2474 | N-[(cis-4-{[4-(dimethylamino)-5,6,7,8-tetrahydroquinazolin-2-yl] amino}cyclohexyl)methyl]-N'-(2-propylphenyl)urea | 465 (M + H) | 3 |
| 2475 | N-(2-tert-butyl-6-methylphenyl)-N'-[(cis-4-{[4-(dimethylamino)-5,6,7,8-tetrahydroquinazolin-2-yl]amino}cyclohexyl)methyl]urea | 493 (M + H) | 1 |
| 2476 | N-(2-tert-butylphenyl)-N'-[(cis-4-{[4-(dimethylamino)-5,6,7,8-tetrahydroquinazolin-2-yl]amino}cyclohexyl)methyl]urea | 479 (M + H) | 2 |
| 2477 | N-(3-chloro-2-methylphenyl)-N'-[(cis-4-{[4-(dimethylamino)-5,6,7,8-tetrahydroquinazolin-2-yl]amino}cyclohexyl)methyl]urea | 471 M + H) | 2 |
| 2478 | N-(4-bromo-2,6-difluorophenyl)-N'-[(cis-4-{[4-(dimethylamino)-5,6,7,8-tetrahydroquinazolin-2-yl]amino}cyclohexyl)methyl]urea | 537 (M + H) | 3 |
| 2479 | N-[4-chloro-2-(trifluoromethyl)phenyl]-N'-[(cis-4-{ [4-(dimethylamino)-5,6,7,8-tetrahydroquinazolin-2-yl]amino} cyclohexyl)-methyl]urea | 525 (M + H) | 3 |

(continued)

| Ex. No. | compound name | MS | class |
|---|---|---|---|
| 2480 | N-(4-cyanophenyl)-N'-[(cis-4-{[4-(dimethylamino)-5,6,7,8-tetrahydroquinazolin-2-yl]amino}cyclohexyl)methyl]urea | 448 (M + H) | 3 |
| 2481 | N [(cis-4-{[4-(dimethylamino)-5,6,7,8-tetrahydroquinazolin-2-yl] amino}cyclohexyl)methyl]-N'-(diphenylmethyl)urea | 513 (M + H) | 3 |
| 2482 | N-(4-bromo-2,6-dimethylphenyl)-N'-[(cis-4-{[4-(dimethylamino)-5,6,7,8-tetrahydroquinazolin-2-yl]amino}cyclohexyl)methyl]urea | 529 (M + H) | 1 |
| 2483 | N-[(cis-4-[4-(dimethylamino)-5,6,7,8-tetrahydroquinazolin-2-yl] amino}cyclohexyl)methyl]-N'-(3-methyl-5-phenylisoxazol-4-yl) urea | 504 (M + H) | 3 |
| 2484 | N-[(cis-4-{[4-(dimethylamino)-5,6,7,8-tetrahydroquinazolin-2-yl] amino}cyclohexyl)methyl]-N'-[5-methyl-2-(trifluoromethyl)-3-furyl] urea | 495 (M + H) | 3 |
| 2485 | N-(3,5-dichlorophenyl)-N'-[(cis-4-{[4-(dimethylamino)-5,6,7,8-tetrahydroquinazolin-2-yl]amino}cyclohexyl)methyl]urea | 491 (M + H) | 3 |
| 2486 | N-(2,3-dichlorophenyl)-N'-[(cis-4-{[4-(dimethylamino)-5,6,7,8-tetrahydroquinazolin-2-yl]amino}cyclohexyl)methyl]urea | 491 (M + H) | 3 |
| 2487 | N [(cis-4-{[4-(dimethylamino)-5,6,7,8-tetrahydroquinazolin-2-yl] amino}cyclohexyl)methyl]-N'-(4-methylphenyl)urea | 437 (M + H) | 3 |
| 2488 | N-(2,6-diisopropylphenyl)-N'-[(cis-4-{[4-(dimethylamino)-5,6,7,8-tetrahydroquinazolin-2-yl]amino}cyclohexyl)methyl]urea | 507 (M + H) | 2 |
| 2489 | N-[(cis-4-{[4-(dimethylamino)-5,6,7,8-tetrahydroquinazolin-2-yl] amino}cyclohexyl)methyl]-N'-(2,3-dimethyl-6-nitrophenyl)-urea | 496 (M + H) | 2 |
| 2490 | N-(2,6-dibromo-4-fluorophenyl)-N'-[(cis-4-{[4-(dimethylamino)-5,6,7,8-tetrahydroquinazolin-2-yl]amino}cyclohexyl)methyl]urea | 597 (M + H) | 1 |
| 2491 | N-(2,6-dichlorophenyl)-N'-((cis-4-{[4-(dimethylamino)-5,6,7,8-tetrahydroquinazolin-2-yl]amino}cyclohexyl)methyl]urea | 491 (M + H) | 1 |
| 2492 | N-[(cis-4-{[4-(dimethylamino)-5,6,7,8-tetrahydroquinazolin-2-yl] amino}cyclohexyl)methyl]-N'-(2-methoxy-5-methylphenyl)-urea | 467 (M + H) | 3 |
| 2493 | N-[(cis-4-{[4-(dimethylamino)-5,6,7,8-tetrahydroquinazolin-2-yl] amino}cyclohexyl)methyl]-N'-(2-methyl-6-nitrophenyl)urea | 482 (M + H) | 3 |
| 2494 | N-(3,4-difluorophenyl)-N'-[(cis-4-{[4-(dimethylamino)-5,6,7,8-tetrahydroquinazolin-2-yl]amino}cyclohexyl)methyl]urea | 459 (M + H) | 3 |
| 2495 | N-(3,5-difluorophenyl)-N'-[(cis-4-{[4-(dimethylamino)-5,6,7,8-tetrahydroquinazolin-2-yl]amino}cyclohexyl)methyl]urea | 459 (M + H) | 3 |
| 2496 | N-(3-chloro-4-fluorophenyl)-N'-[(cis-4-{[4-(dimethylamino)-5,6,7,8-tetrahydroquinazolin-2-yl]amino}cyclohexyl)methyl]urea | 475 (M + H) | 3 |

**Example 2497**

**2,3,4-Trifluoro-*N*-{cis-4-[(4-methylquinolin-2-yl)aminolcyclohexyl)-benzamide trifluoroacetate**

**Step A: Synthesis of cis-(4-tert-butoxycarbonylamino-cyclohexyl)-carbamic acid benzyl ester.**

To a solution of cis-(4-amino-cyclohexyl)-carbamic acid tert-butyl ester (4 g, 0.019 mol) in 50 mL $CH_2Cl_2$ was added DIEA (4.9 mL, 0.028 mol). The solution was cooled on an ice bath and CbzCl (2.9 mL, 0.020 mol) was added slowly. The solution was removed from the ice bath and stirring continued for an additional hour. The solvent was evaporated and the material was subjected to chromatography (0-40% ethyl acetate in hexanes) to yield *cis-(4-tert-*

butoxycarbonylamino-cyclohexyl)-carbamic acid benzyl ester (6.2 g, 0.018 mol, 95%) as a white solid.
ESI MS m/e 349.0 M + H+; $^1$H NMR (400 MHz, DMSO-$d_6$) δ 7.34-7.28 (m, 5 H), 7.12 (d, J = 5.6 Hz, 1 H), 6.62 (brs, 1 H), 4.98 (s, 2 H), 3.39-3.37 (m, 2 H), 1.60-1.45 (m, 8 H), 1.37 (s, 9 H).

**Step B: Synthesis of *cis*-(4-amino-cyclohexyl)-carbamic acid benzyl ester.**

To a solution of cis-(4-tert-butoxycarbonylamino-cyclohexyl)-carbamic acid benzyl ester (6.2 g, 0.018 mol) in 40 mL $CH_2Cl_2$ was added TFA (2.7 mL, 0.36 mol). The solution was stirred at room temperature for 4 hours. The excess solvent was evaporated off and the resulting oil was dissolved in 30 mL $CH_2Cl_2$. The organic layer was extracted with 30 mL of a dilute NaOH (aq) / $NaHCO_3$ (aq) solution. The aqueous layer was back extracted twice with $CH_2Cl_2$ and the organic layers combined, dried over $MgSO_4$, and concentrated to yield cis-(4-amino-cyclohexyl)-carbamic acid benzyl ester (4.3 g, 97%) as a colorless oil. The oil was carried forward without further purification.
ESI MS m/e 249.2 M + H+.

**Step C: Synthesis of *c*is-[4-(4-methyl-quinolin-2-ylamino)-cyclohexyl)-carbamic acid benzyl ester.**

To a solution of cis-(4-amino-cyclohexyl)-carbamic acid benzyl ester (0.5 g, 0.0020 mol) in 1 mL 2-propanol was added 2-chloro-4-methyl-quinoline (0.43 g, 0.0024 mol) and IEA (526 uL, 0.0030 mol). The mixture was heated in a microwave synthesizer at 170 °C for 5 hours. The reaction was repeated 7 more times (4 g total material) and the reaction mixtures were pooled. The solvent was evaporated and the material subjected to chromatography (2-4 % 2M $NH_3$ in MeOH / $CH_2Cl_2$) to yield *c*is-[4-(4-methyl-quinolin-2-ylamino)-cyclohexyl]-carbamic acid benzyl ester (3.3 g, 53%) as a colorless oil.
ESI MS m/e 390.2 M + H+ ; $^1$H NMR (400 MHz, DMSO-$d_6$) δ 7.71 (d, J = 8 Hz, 1 H), 7.46-7.39 (m, 2 H), 7.37-7.19 (m, 7 H), 6.68 (m, 2 H), 5.01 (s, 2 H), 4.07 (m, 1 H), 3.46 (m, 1 H), 2.44 (s, 3 H), 1.79-1.71 (m, 2 H), 1.70-1.59 (m, 6 H).

**Step D: Synthesis of *cis-N*-(4-methyl-quinolin-2-yi)-cyclohexane-1,4-diamine.**

To a solution of cis-[4-(4-methyl-quinolin-2-ylamino)-cyclohexyl]-carbamic acid benzyl ester (3.3 g, 0.0085 mol) in 200 mL EtOH was added 10% Pd/C (330 mg). The reaction mixture was stirred at room temperature under $H_2$ (g) atmosphere for 3 hours. The $H_2$(g) atmosphere was removed and the mixture was through a pad of celite and washed with ethyl acetate. The solvent was concentrated and the material was subjected to chromatography (2-4 % 2M $NH_3$ in MeOH / $CH_2Cl_2$) to yield *cis-N-(4-methyl-quinolin-2-yl)-cyclohexane-1,4-diamine* (2.0 g, 92%) as a light brown solid.
ESI MS m/e 256.4 M + H+ ; $^1$H NMR (400 MHz, DMSO-$d_6$) δ 7.71 (d, J = 8 Hz, 1 H), 7.46-7.39 (m, 2H), 7.14-7.10 (m, 1H), 6.69-6.68 (m, 2 H), 4.07-4.05 (m, 1 H), 2.81-2.77 (m, 1 H), 2.44 (s, 3 H), 1.78-1.71 (m, 2 H), 1.62-1.40 (m, 6 H).

**Step E: Synthesis of 2,3,4-trifluoro-*N*-{*cis*-4-[(4-methylquinolin-2-yl)amino]cyclohexyl)-benzamide trifluoroacetate.**

To a solution of *cis*-*N*-(4-methyl -quinolin-2-yl)-cyclohexane- 1,4-diamine (23 mg, 0.090 mmol) in 0.5 mL DMF was added pyridine (12 uL, 0.15 mmol) and 2,3,4-trifluorobenzoyl chloride (12.8 uL, 0. 10 mmol). The reaction mixture was stirred overnight and then 0.5 mL ofDMSO was added to the mixture. The compound was then subjected to purification by prep LCMS to yield 2,3,4-trifluoro-*N*-{*cis*-4-[(4-methylquinolin-2-yl)amino]cyclohexyl}-benzamide trifluoroacetate (10.1 mg, 21 %) as a white solid.
ESI MS m/e 414.2 M + H+ ; $^1$H NMR (400 MHz, DMSO-$d_6$) δ 12.44 (brs, 1 H), 9.27 (brs, 1 H), 8.45 (d, J = 6.4 Hz, 1 H), 7.98-7.93 (m, 2 H), 7.80 (t, J = 7.6 Hz , 1 H), 7.53 (t, J = 8.0 Hz , 1 H), 7.43-7.37 (m, 2 H), 7.01 (s, 1 H), 4.05 (m, 1 H), 3.97 (m, 1 H), 2.69 (s, 3 H), 1.86-1.74 (m, 8 H).

**Example 2498**

**3,4-Difluoro-*N*-{*cis*-4-[(4-methylquinolin-2-yl)amino]cyclohexyl)benzamide trifluoroacetate**

**StepA: Synthesis of 3,4-difluoro-*N*-{cis-4-[(4-methylquinolin-2-yl)amino]cyclohexyl}-benzamide trifluoroacetate**

Using the procedure of step E of example 2497, the title compound was obtained.
ESI MS m/e 396.18 M + H+; $^1$H NMR (400 MHz, DMSO-$d_6$) δ 12.40 (brs, 1 H), 9.25 (brs, 1 H), 8.33 (d, J = 6.0 Hz , 1H), 7.98-7.90 (m, 3 H), 7.80-7.76 (m, 2 H), 7.58-7.50 (m, 2 H), 7.02 (brs, 1 H), 4.09 (m, 1 H), 3.94 (m, 1 H), 2.61 (s,

3 H), 1.84-1.74 (m, 8 H).

**Example 2499**

**4-Cyano-*N*-{cis-4-[(4-methylquino)in-2-yl)amino]cyclohexyl}benzamide trifluoroacetate**

**Step A: Synthesis of 4-cyano-*N*-{*cis*-4-[(4-methylquinolin-2-yl)amino]cyclohexyl}benzamide trifluoroacetate.**

Using the procedure of step E of example 2497, the title compound was obtained.
ESI MS m/e 385.2 M + $H^+$ ; $^1H$ NMR (400 MHz, DMSO-$d_6$) δ 12.38 (brs, 1 H), 9.27 (brs, 1 H), 8.51 (d, J = 6.0 Hz , 1 H), 8.01-7.95 (m, 6H), 7.80 (t, J = 7.2 Hz , 1 H), 7.54 (t, J = 8.0 Hz , 1 H), 7.02 (brs, 1 H), 4.09 (m, 1 H), 3.96 (m, 1 H), 2.66 (s, 3 H), 1.85-1.75 (m, 8 H).

**Example 2500**

**3-Fluoro-*N*-{cis-4-[(4-methylquinolin-2-yl)amino]cyclohexyl)benzamide trifluoroacetate**

**Step A: Synthesis of 3-fluoro-N-{*cis*-4-[(4-methylquinolin-2-yl)amino]cyclohexyl}benzamide trifluoroacetate.**

Using the procedure of step E of example 2497, the title compound was obtained.
ESI m/e 378 M + $H^+$ ; $^1H$ NMR (400 MHz, DMSO-$d_6$) δ 12.38 (brs, 1 H), 9.25 (brs, 1 H), 8.33 (d, J = 6.0 Hz , 1 H), 7.98-7.91 (m, 2 H), 7.80 (t, J = 7.6 Hz , 1 H), 7.71-7.64 (m, 2 H), 7.55-7.49 (m, 2 H), 7.41-7.36 (m, 1 H), 4.12 (m, 1 H), 4.08 (m, 1 H), 2.77 (s, 3 H), 1.85-1.74 (m, 8 H).

**Example 2501**

**3,5-Difluoro-*N*-{*cis*-4-[(4-methylquinolin-2-yl)amino]cyclohexyl}benzamide trifluoroacetate**

**Step A: Synthesis of 3,5-difluoro-*N*{cis-4-[(4-methylquinolin-2-yl)amino]cyclohexyl]-benzamide trifluoroacetate.**

Using the procedure of step E of example 2497, the title compound was obtained.
ESI MS m/e 396 M + $H^+$;$^1H$ NMR (400 MHz, DMSO-$d_6$) δ 12.40 (brs, 1 H), 9.25 (brs, 1 H), 8.40 (d, J = 6.0 Hz , 1 H), 7.98-7.96 (m, 2 H), 7.80 (t, *J* = 7.2 Hz, 1 H), 7.59-7.44 (m, 4 H), 7.02 (brs, 1 H), 4.09 (m, 1 H), 3.94 (m, 1 H), 2.68 (s, 3 H), 1.85-1.74 (m, 8 H).

**Example 2502**

***N*-(*cis*-4-[(4-Methylquinolin-2-yl)amino]cyclohexyl}-2-[4-(trifluoromethoxy)phenoxy]-acetamide trifluoroacetate**

**Step A: Synthesis of N-[cis-4-[(4-methylquinolin-2-yl)amino]cyclohexyl)-2-[4-(trifluoromethoxy)phenoxy]-acetamide trifluoroacetate.**

To a solution of *cis-N*(4-methyl-quinolin-2-yl)-cyclohexane-1,4-diamine (25.5 mg, 0.1 mmol) in 0.5 mL DMF was added 4-(trifluoromethoxy)phenoxyacetic acid (23.6 mg, 0.1 mmol), DIEA (0.026 mL, 0.15 mmol), and HATU (45.6 mg, 0.12 mmol). The reaction mixture was stirred overnight and then 0.5 mL of DMSO was added to the mixture. The compound was then subjected to purification by prep LCMS to yield *N*-{cis-4-[(4-methylquinolin-2-yl)amino]cyclohexyl}-2-[4-(trifluoromethoxy)phenoxy]-acetamide trifluoroacetate (22.3 mg, 38%) as a white solid.
ESI MS m/e 474.4 M + $H^+$; $^1H$ NMR (400 MHz, DMSO-$d_6$) δ 12.47 (s, 1 H), 9.25 (s, 1 H), 8.00-7.92 (m, 3 H), 7.80 (t, J = 7.2 Hz, 1 H), 7.53 (t, J = 8.0 Hz, 1 H), 7.31 (d, J = 8.8 Hz, 2 H), 7.04-7.01 (m, 3 H), 4.55 (s, 2 H), 4.06 (m, 1 H), 3.84 (m, 1 H), 2.69 (s, 3 H), 1.78-1.68 (m, 8 H).

**Example 2503**

**2-(3,4-Difluorophenyl)-*N*-{cis-4-[(4-methylquinolin-2-yl)amino]cyclohexyl}acetamide trifluoroacetate**

**Step A: Synthesis of 2-(3,4-difluorophenyl)-*N*-{cis-4-[(4-methylquinolin-2-yl)amino]cyclohexyl}acetamide trifluoroacetate.**

Using the procedure of step A of example 2502, the title compound was obtained.
ESI MS m/e 410 M + $H^+$ ; $^1$H NMR (400 MHz, DMSO-$d_6$) δ 12.42 (brs, 1 H), 9.26 (brs, 1 H), 8.09 (d, J = 6.4 Hz, 1 H), 7.98-7.92 (m, 2 H), 7.80 (t, J = 7.6 Hz, 1 H), 7.54 (t, J = 8.8 Hz, 1 H), 7.38-7.27 (m, 2 H), 7.10-7.07 (m, 1 H), 7.01 (brs, 1 H), 4.02 (m, 1 H), 3.94 (m, 1 H), 2.61 (s, 3 H), 1.79-1.69 (m, 8 H).

**Example 2504**

**2-(2-Bromo-4,5-dimethoxyphenyl)-N-{cis-4-[(4-methylquinolin-2-yl)amino]cyclohexyl)-acetamide trifluoroacetate**

**Step A: Synthesis of 2-(2-bromo-4,5-dimethoxyphenyl)-N-[cis-4-[(4-methylquinolin-2-yl)amino]cyclohexyl) acetamide trifluoroacetate.**

Using the procedure of step A of example 2502, the title compound was obtained.
ESI MS m/e 512.2 M + $H^+$ ; $^1$H NMR (400 MHz, DMSO-$d_6$) δ 12.45 (brs, 1 H), 9.25 (brs, 1H), 8.00-7.92 (m, 3 H), 7.80 (t, *J* = 7.6 Hz , 1 H), 7.53 (t, *J* = 7.6 Hz , 1H), 7.09 (s, 1 H), 7.01 (brs, I H), 6.95 (s, 1H), 4.10 (m, 1H), 3.78 (m, 1H), 3.74 (s, 3 H), 3.72 (s, 3 H), 3.53 (s, 2 H), 2.69 (s, 3 H), 1.78-1.67 (m, 8 H).

**Example 2505**

**4-(Benzyloxy)-*N*-{cis4-[(4-methylquinolin-2-yl)amino]cyclohexyl)benzamide trifluoroacetate**

**Step A: Synthesis of 4-(benzyloxy)-*N*-{cis-4-[(4-methylquinolin-2-yl)amino]cyclohexyl)-benzamide trifluoroacetate.**

Using the procedure of step A of example 2502, the title compound was obtained.
ESI MS m/e 466.2 M + $H^+$; $^1$H NMR (400 MHz, DMSO-$d_6$) δ 12.39 (brs, 1 H), 9.25 (brs, 1 H), 8.06 (d, J= 6.0 Hz , 1 H), 7.98-7.96 (m, 2 H), 7.84-7.76 (m, 3 H), 7.54 (t, J= 8.0 Hz , 1 H), 7.46 (d, J = 7.2 Hz, 2 H), 7.41 (t, J = 7.2 Hz, 2 H), 7.35-7.31 (m, 1 H), 7.08 (d, J= 8.8 Hz, 2 H), 7.02 (brs, 1 H), 5.17 (s, 2 H), 4.09 (m, I H), 3.93 (m, 1 H), 2.66 (s, 3 H), 1.84-1.72 (m, 8 H).

**Example 2506**

**2-(2-Methoxyphenoxy)-*N* {cis-4-[(4-methylquinolin-2-yl)amino]cyclohexyl}acetamide trifluoroacetate**

**Step A: Synthesis of 2-(2-methoxyphenoxy)-*N*-{cis-4-[(4-methylquinolin-2-yl)amino]cyclohexyl}acetamide trifluoroacetate.**

Using the procedure of step A of example 2502, the title compound was obtained.
ESI MS m/e 420.2 M + $H^+$ ; $^1$H NMR (400 MHz, DMSO-$d_6$) δ 12.50 (brs, 1 H), 9.25 (brs, 1 H), 7.98-7.93 (m, 2 H), 7.80-7.76 (m, 2 H), 7.53 (t, J = 5.6 Hz , 1 H), 7.02-6.85 (m, 5 H), 4.50 (s, 2 H), 4.07 (m, 1 H), 3.85 (m, 1 H), 3.79 (s, 3 H), 2.61 (s, 3 H), 1.84-1.69 (m, 8 H).

**Example 2507**

**2-(4-Fluorophenoxy)-*N*-{cis-4-[(4-methylquinolin-2-yl)amino]cyclohexyl}nicotinamide trifluoroacetate**

**Step A: Synthesis of 2-(4-fluorophenoxy)-N-icis-4-[(4-methylquinolin-2-yl)amino]cyclohexyl}nicotinamide trifluoroacetate.**

Using the procedure of step A of example 2502, the title compound was obtained.

ESI MS m/e 471.4 M + H+ ; $^1$H NMR (400 MHz, $CD_3OD$) δ 8.29 (dd, J = 7.6, 2.0 Hz, 1 H), 8.19 (dd, J = 4.8, 2.0 Hz, 1 H), 8.01 ( d, J = 8.0 Hz, 1 H), 7.88 (brs, 1 H), 7.80 (t, J = 8.4 Hz, 1 H), 7.57 (t, J = 8.0 Hz, 1 H), 7.25-7.15 (m, 5 H), 6.90 (brs, 1 H), 4.20 (brs, 1 H), 4.07 (brs, 1H), 2.67 (s, 3H), 2.02-1.81 (m, 8H).

**Example 2508**

**2- (4-Chlorophenoxy)- *N -lcis-4-[* (4-methylquinoJin- 2- y l)amino ]cy clohexy l) nicotinamide trifluoroacetate**

**Step A: Synthesis of 2-(4-Chlorophenoxy)-N-f cis-4-[(4-methylquinolin-2-yl)amino]cyclohexyl}nicotinamide trifluoroacetate.**

Using the procedure of step A of example 2502, the title compound was obtained.
ESI MS m/e 487.2 M + H+ ; $^1$H NMR (400 MHz, DMSO-$d_6$) δ 13.0 (brs, 1 H), 9.50 (d, J= 6.8 Hz, 1 H), 8.35 (m, 1 H), 8.19 (m, 1 H), 8.07 (d, J = 6.8 Hz, 1 H), 7.93 (d, *J* = 7.6 Hz, 1 H), 7.75 (t, J = 7.2 Hz, 1 H), 7.50 (m, 3 H), 7.30 (m, 3 H), 7.10 (brs, 1 H), 4.38 (brs, 1 H), 4.01 (brs, 1 H), 2.57 (s, 3 H), 1.83 (m, 8H).

**Example 2509**

**2,6-Dimethoxy-*N*-{*cis*-4-[(4-methylquinolin-2-yl)amino]cyclohexyl}nicotinamide trifluoroacetate**

**Step A: Synthesis of 2,6-dimethoxy-*N*-{cis-4-[(4-methylquinolin-2-yl)amino]cyclohexyl}nicotinamide trifluoroacetate.**

Using the procedure of step A of example 2502, the title compound was obtained.
ESI MS m/e 421.2 M + H+ ; $^1$H NMR (400 MHz, DMSO-$d_6$) δ 13.1 (brs, 1 H), 9.74 (d, J = 8.0 Hz, 1 H), 8.30 (d, J = 8.4 Hz, 1 H), 8.17 (d, J = 8.4 Hz, 1 H), 7.98 (m, 2 H), 7.60 (m, 1 H), 7.50 (t, *J* = 7.6 Hz, 1 H), 7.19 9 (brs, 1 H), 4.43 (brs, 1H), 3.94 (brs, 7H), 2.58 (s, 3H), 1.90 (m, 8 H).

**Example 2510**

**cis-N-[4-Bromo-2-(trifluoromethoxy)benzyl]-N'-(4-methylquinolin-2-yl)cyclohexane-1,4-diamine bis-trifluoroacetate**

**Step A: Synthesis of cis-N-[4-bromo-2-(trifluoromethoxy)benzyl]-N'-(4-methylquinolin-2-yl)cyclohexane-1,4-diamine bis-trifluoroacetate.**

To a solution of *cis*-N-(4-methyl-quinolin-2-yl)-cyclohexane-1,4-diamine (25.5 mg, 0.1 mmol) in 0.5 mL MeOH was added 4-bromo-2-trifluoromethoxybenzaldehyde (26.9 mg, 0.1 mmol). The reaction mixture was stirred for a half hour and then sodium triacetoxyborohydride (84.8 mg, 0.4 mmol) was added to the reaction. The mixture was stirred overnight and then 0.5 mL of DMSO was added. The compound was then subjected to purification by prep LCMS to yield *cis-N*-[4-bromo-2-(trifluoromethoxy)benzyl]-*N*-(4-methylquinolin-2-yl)cyclohexane-1,4-diamine bis-trifluoroacetate (9.6 mg, 13%) as a white solid.
ESI MS m/e 508.0 M + H+; $^1$H NMR (400 MHz, $CD_3OD$) δ 8.04 (d, J = 8.0 Hz, 1H), 7.84 (brs, I H), 7.81 (t, *J* = 7.2 Hz, 1 H), 7.69-7.63 (m, 3 H), 7.58 (t, J = 8.0 Hz, 1H), 7.16 (brs, 1 H), 4.36 (s, 2 H), 4.26 (m, 1 H), 3.32-3.30 (m, 1 H), 2.71 (s, 2 H), 2.66 (s, 3 H), 2.16-1.93 (m, 8 H).

**Example 2511**

***cis-N*-[(5-Bromo-1H-indol-3-yl)methyl]-*N'*-(4-methylquinolin-2-yl)cyclohexane-1,4-diamine bis-trifluoroacetate**

**Step A: Synthesis of *cis-N*-[(5-bromo-1H-indol-3-yl)methyl]-*N'*-(4-methylquinolin-2-yl)cyclohexane-1,4-diamine bis-trifluoroacetate.**

Using the procedure of step A of example 2510, the title compound was obtained.
ESI MS m/e 463.2 M + H+ ; $^1$H NMR (400 MHz, $CD_3OD$) δ 8.03 (d, J = 8.0 Hz, 1 H), 7.92 (s, 1 H), 7.87 (brs, 1 H), 7.80-7.76 (t, J = 7.2 Hz, 1 H), 7.57-7.53 (m, 2 H), 7.38 (d, J = 8.8 Hz, 1 H), 7.31 (d, J= 8.4 Hz, 1 H), 7.14 (brs, 1 H), 4.47 (s, 2 H), 4.23 (m, 1 H), 3.37 (m, 1 H), 2.71 (brs, 2 H), 2.65 (s, 3 H), 2.15-1.91 (m, 8 H).

**Example 2512**

**cis-*N*-(3,5-Dimethoxybenzyl)-*N*'-(4-methylquinolin-2-yl)cyclohexane-1,4-diamine bis-trifluoroacetate**

**Step A: Synthesis of *cis-N*-(3,5-dimethoxybenzyl)-*N*'-(4-methylquinolin-2-yl)cyclohexane-1,4-diamine bis-trifluoroacetate.**

Using the procedure of step A of example 2510, the title compound was obtained.
ESI MS m/e 406.2 M + $H^+$ ; $^1H$ NMR (400 MHz, $CD_3OD$) δ 8.03 (d, J= 8.0 Hz, 1 H), 7.88 (brs, 1 H), 7.80 (t, *J* = 7.2 Hz, 1 H), 7.57 (t, J = 8.4 Hz, 1 H), 7.17 (brs, 1 H), 6.71 (s, 2 H), 6.55 (s, 1 H), 4.24 (m, 1 H), 4.21 (s, 2 H), 3.81 (s, 6 H), 3.35 (m, 1 H), 2.70 (brs, 2 H), 2.66 (s, 3 H), 2.14-1.90 (m, 8 H).

**Example 2513**

***cis-N*-(3,5-Dichlorobenzyl)-*N*'-(4-methylquinolin-2-yl)cyclohexane-1,4-diamine bis-trifluoroacetate**

**Step A: Synthesis of cis-*N*-(3,5-dichiorobenzyl)-*N*'-(4-methylquinolin-2-yl)cyclohexane-1,4-diamine bis-trifluoroacetate.**

Using the procedure of step A of example 2510, the title compound was obtained.
ESI MS m/e 414.2 M + $H^+$; $^1H$ NMR (400 MHz, $CD_3OD$) δ 8.04 (d, J= 8.4 Hz, 1 H), 7.86 (brs, I H), 7.81 (t, J = 7.2 Hz, 1 H), 7.58-7.54 (m, 4 H), 7.16 (brs, 1 H), 4.30 (s, 2 H), 4.25 (m, 1 H), 3.41 (m, 1 H), 2.76 (brs, 2 H), 2.66 (s, 3 H), 2.12-1.92 (m, 8 H).

**Example 2514**

**cis-*N*-(3,4-Difluorobenzyl)-*N*'-(4-methylquinolin-2-yl)cyclohexane-1,4-diamine bis-trifluoroacetate**

**Step A: Synthesis of cis-N-(3,4-difluorobenzyl)-N'-(4-methylquinolin-2-yl)cyclohexane-1,4-diamine bis-trifluoroacetate.**

Using the procedure of step A of example 2510, the title compound was obtained.
ESI MS m/e 382.2 M + $H^+$; $^1H$ NMR (400 MHz, $CD_3OD$) δ 8.03 (d, J = 8.0 Hz, 1 H), 7.86 (brs, 1 H), 7.80 (t, *J* = 7.2 Hz, 1 H), 7.57-7.51 (m, 2H), 7.39-7.37 (m, 2 H), 7.16 (brs, 1H), 4.29 (s, 2 H), 4.25 (m, 1 H), 3.37 (m, 1H), 2.71 (brs, 2 H), 2.66 (s, 3 H), 2.11-1.95 (m, 8 H).

**Example 2515**

***N*-(3,5-Difluorophenyl)-N'-}*cis*-4-[(4-methylquinolin-2-yl)amino]cyclohexyl} urea trifluoroacetate**

**Step A: Synthesis of *N*-(3,5-difluorophenyl)-N'-{cis-4-[(4-methylquinolin-2-yl)amino]cyclohexyl}urea trifluoroacetate.**

To a solution of cis-*N*-(4-methyl-quinolin-2-yl)-cyclohexane-1,4-diamine (20 mg, 0.078 mmol) in 0.5 mL of DMSO was added 3,5-difluorophenyl isocyanate (9.3 uL, 0.078 mmol). The reaction mixture was stirred overnight and then 0.5 mL of DMSO was added to the mixture. The compound was then subjected to purification by prep LCMS to yield N-(3,5-difluorophenyl)-N-{cis-4-[(4-methylquinolin-2-yl)amino]cyclohexyl}urea trifluoroacetate (12 mg, 29%) as a white solid.ESI MS m/e 411.2 M + $H^+$ ; $^1H$ NMR (400 MHz, $CD_3OD$) δ 8.02 (d, J = 8.0 Hz, 1 H), 7.87 (brs, 1 H), 7.80 (t, J = 7.6 Hz, 1 H), 7.56 (t, J = 7.6 Hz, 1 H), 7.07 (s, 1 H), 7.03 (s, 1 H), 6.97 (brs, 1 H), 6.50 (t, J = 9.2 Hz, 1 H), 4.02 (m, 1 H), 3.89 (m, 1 H), 2.68 (brs, 3 H), 2.66 (s, 3 H), 1.99-1.78 (m, 8 H).

**Example 2516**

***N*-(3,5-Bis(trifluoromethyl)phenyl]-*N*'-[cis-4-[(4-methylquinolin-2-yl)amino]cyclohexyl} urea trifluoroacetate**

**Step A: Synthesis of N-[3,5-bis(tritluoromethyl)phenyl]-N'-icis-4-[(4-methylquinolin-2-yl)amino]cyclohexyl} urea trifluoroacetate.**

Using the procedure of step A of example 2515, the title compound was obtained.
ESI MS m/e 511.2 M + $H^+$ ; $^1H$ NMR (400 MHz, $CD_3OD$) δ 8.02 (s, 2 H), 8.00 (s, 1 H), 7.87 (brs, 1 H), 7.80 (t, *J* = 7.2 Hz, 1 H), 7.57 (t, J = 8.0 Hz, 1 H), 7.49 (s, 1 H), 6.98 (brs, 1 H), 4.04 (m, 1 H), 3.91 (m, 1 H), 2.69 (brs, 3 H), 2.66 (s, 3 H), 2.01-1.80 (m, 8 H).

**Example 2517**

***N*-(3-Chlorophenyl)-*N'*-{cis-4-[(4-methylquinolin-2-yl)amino]cyclohexyl}urea trifluoroacetate**

**Step A: Synthesis of *N*-(3-chlorophenyl)-*N'*-{cis-4-[(4-methylquinolin-2-yl)amino]cyclohexyl}urea trifluoroacetate.**

Using the procedure of step A of example 2515, the title compound was obtained.
ESI MS m/e 409.2 M + $H^+$; $^1H$ NMR (400 MHz, $CD_3OD$) δ 8.00 (d, J = 8.4 Hz, 1 H), 7.87 (brs, 1 H), 7.79 (t, J = 7.6 Hz, 1 H), 7.59 (s, 1 H), 7.56 (t, J = 7.6 Hz, 1 H), 7.21-7.15 (m, 2 H), 6.96 (brs, I H), 6.93-6.91 (m, 1 H), 4.01 (m, 1 H), 3.89 (t, 1 H), 2.66 (brs, 6 H), 1.99-1.78 (m, 8 H).

**Example 2518**

***N*-(3,4-Dichlorophenyl)-*N*'-{*cis*-4-[(4-methylquinolin-2-yl) amino]cyclohexyl}urea trifluoroacetate**

**Step A: Synthesis of *N*(3,4-dichlorophenyl)-*N*'-{cis-4-[(4-methylquinolin-2-yl)amino]cyclohexyl)urea trifluoroacetate.**

Using the procedure of step A of example 2515, the title compound was obtained.
ESI MS m/e 443.2 M+$H^+$ ; $^1H$ NMR (400 MHz, $CD_3OD$) δ 8.00 (d, *J* = 7.6 Hz, 1 H), 7.87 (brs, I H), 7.79-7.74 (m, 2 H), 7.56 (t, J = 7.6 Hz, 1 H), 7.34 (d, *J* = 8.4 Hz, 1 H), 7.20 (d, *J* = 8.4 Hz, 1 H), 6.97 (brs, 1 H), 4.02 (m, 1 H), 3.88 (m, 1 H), 2.66 (brs, 6 H), 1.98-1.78 (m, 8 H).

**Example 2519**

***N*- (3-Methoxyphenyl)-*N*'-{cis-4-[(4-methylquinolin-2-yl)amino] cyclohexyl}urea trifluoroacetate**

**Step A: Synthesis of *N* (3-methoxyphenyl)-*N*'-{cis-4-[(4-methylquinolin-2-yl)amino] cyclohexyl}urea trifluoroacetate.**

Using the procedure of step A of example 2515, the title compound was obtained.
ESI MS m/e 405.4 M + $H^+$ ; $^1H$ NMR (400 MHz, $CD_3OD$) δ 8.00 (d, J = 8.0 Hz, 1 H), 7.87 (brs, 1 H), 7.79 (t, J = 7.6 Hz, 1 H), 7.56 (t, J = 8.0 Hz, 1 H), 7.14-7.10 (m, 2 H), 6.96 (brs, 1H), 6.84 (d, J = 8.0 Hz, 1 H), 6.53 (d, J = 8.4 Hz, 1H), 4.01 (m, 1 H), 3.89 (m, 1H), 3.75 (s, 3H), 2.71 (brs, 6 H), 1.99-1.78 (m, 8 H).

**Example 2520**

**3-Methoxy-N-[cis-4-(quinolin-2-ylamino)cyclohexyl]benzamide trifluoroacetate**

**Step A: Synthesis of cis-[4-(3-methoxy-benzoylamino)-cyclohexyl]-carbamic acid *tert*-butyl ester.**

To a solution of cis-(4-amino-cyclohexyl)-carbamic acid tert-butyl ester (2.8 g, 0.013 mol) in 40 mL $CH_2Cl_2$ stirring on ice was added DIEA (3.41 mL, 0.020 mol). The solution was cooled on an ice bath and *m*-anisoyl chloride (1.84 mL, 0.013 mol) was added slowly. The solution was removed from the ice bath and stirring continued for an additional hour. The solvent was evaporated and the material was subjected to chromatography (0-40% ethyl acetate

in hexanes) to yield cis-[4-(3-methoxy-benzoylamino)-cyclohexyl]-carbamic acid tert-butyl ester (4.3 g, 94 %) as a white solid.
ESI MS m/e 349.0 M + $H^+$ ; $^1$H NMR (400 MHz, DMSO-$d_6$) δ 8.03 (d, J= 6.8 Hz, 1 H), 7.42-7.32 (m, 3 H), 7.07 (dd, J = 8.4, 2.4 Hz, 1 H), 6.62 (brs, 1 H), 3.79 (s, 3 H), 3.77 (m, 1 H), 3.41 (m, 1 H), 1.71-1.70 (m, 4 H), 1.52-1.46 (m, 4 H), 1.38 (s, 9H).

**Step B: Synthesis of cis-*N*-(4-amino-cyclohexyl)-3-methoxy-benzamide.**

To a solution of cis-[4-(3-methoxy-benzoylamino)-cyclohexyl]-carbamic acid *tert*-butyl ester (4.3 g, 0.012 mol) in 50 mL $CH_2Cl_2$ was added TFA (1.84 mL, 0.024 mol). The solution was stirred for 4 hours and the solvent evaporated. The resulting oil was re-dissolved in 50 mL $CH_2Cl_2$. The organic layer was extracted with 50 mL of a dilute NaOH (aq) / $NaHCO_3$ (aq) solution. The aqueous layer was extracted twice more with $CH_2Cl_2$ and the organic layers combined, dried over $MgSO_4$, and concentrated. The resulting precipitate was crystallized in ether and hexanes to yield cis-*N*-(4-amino-cyclohexyl)-3-methoxy-benzamide (2.4g, 78%) as a white solid.
ESI MS m/e 249.0 M + $H^+$ ; $^1$H NMR (400 MHz, DMSO-$d_6$) δ 8.10 (d, J = 7.2 Hz, 1 H), 7.42-7.32 (m, 3 H), 7.07 (dd, J = 8.0, 2.4 Hz, 1H), 3.79 (brs, 4 H), 2.91 (m, 1 H), 1.80-1.74 (m, 2 H), 1.52-1.46 (m, 6 H), 1.31 (brs, 2 H).

**Step C: Synthesis of 3-methoxy-*N*-[*cis*-4-(quinolin-2-ylamino)cyclohexyl]benzamide trifluoroacetate.**

To a solution of *cis*-*N*-(4-amino-cyclohexyl)-3-methoxy-benzamide (28.4 mg, 0.1 mmol) in 0.5 mL 2-propanol was added 2-chloroquinoline (32.7 mg, 0.2 mmol) and DIEA (34.8 uL, 0.2 mmol). The reaction mixture was heated in a microwave synthesizer at 170 °C for 10 hours. The solvent was removed and the resulting oil dissolved in 1 mL of DMSO. The compound was then subject to purification by prep LCMS to yield 3-methoxy-N-[cis-4-(quinolin-2-ylamino) cyclohexyl]benzamide trifluoroacetate (26 mg, 53%) as a colorless oil.
ESI MS m/e 376.2 M + $H^+$ ; $^1$H NMR (400 MHz, $CD_3OD$) δ 7.85 (d, J = 9.2 Hz, 1 H), 7.62 (t, J= 8.8 Hz, 2 H), 7.50 (t, *J* = 7.2 Hz, 1 H), 7.39-7.36 (m, 3 H), 7.19 (t, *J* = 7.2 Hz, 1 H), 7.10-7.07 (m, 1 H), 6.82 (d, J = 9.2 Hz, 1 H), 4.18 (m, 1 H), 4.02 (m, 1 H), 3.84 (s, 3 H), 1.95-1.22 (m, 1 H).

**Example 2521**

**3-methoxy-*N*-(*cis*-4-{[4-(trifluoromethyl)quinolin-2-yl]amino}cyclobexyl)benzamide trifluoroacetate**

**Step A: Synthesis of 2-chloro-4-trifluoromethyl-quinoline.**

To a solution of 4-trifluoromethyl-quinolin-2-ol (1.01 g, 0.0047 mol) in 10 mL $POCl_3$ was added N, *N*-dimethylaniline (661 uL, 0.0052 mol). The mixture was heated to reflux (125 °C) and stirred for 4 hours until the starting material completely dissolved and the solution turned dark purple in color. The solution was then cooled and poured slowly on ice (30 g; caution highly exothermic) to quench the reaction. The aqueous layer was then extracted three times with $CH_2Cl_2$ (25 mL). The organic layer was dried with $MgSO_4$, concentrated, and subjected to purification by chromatography (100% $CH_2Cl_2$) to yield 2-chloro-4-trifluoromethyl-quinoline (823 mg, 75%) as a slightly yellow solid.
ESI MS m/e 232.0 M + $H^+$ ; $^1$H NMR (400 MHz, DMSO-$d_6$) δ 8.15-8.09 (m, 2 H), 8.06 (s, 1 H), 8.01-7.97 (m, 1 H), 7.88-7.85 (m, 1 H).

**Step B: Synthesis of 3-methoxy-*N*-(cis-4-{[4-(trifluoromethyl)quinolin-2-yl]amino}cyclohexyl)benzamide trifluoroacetate.**

To a solution of cis-N-(4-amino-cyclohexyl)-3-methoxy-benzamide (50 mg, 0.20 mmol) in 0.5 mL 2-propanol was added 2-chloro-4-trifluoromethyl-quinoline (56 mg, 0.24 mmol), and DIEA (52.6 uL, 0.30 mmol). The reaction mixture was heated in a microwave synthesizer at 170° C for 5 hours. The solvent was removed and the resulting oil dissolved in 1 mL of DMSO. The compound was then subjected to purification by prep LCMS to yield 3-methoxy-N-(cis-4-{[4-(trifluoromethyl)quinolin-2-yl]amino}cyclohexyl)benzamide trifluoroacetate (71.8 mg, 64%) as a white solid.
ESI MS m/e 444.4 M + $H^+$ ; $^1$H NMR (400 MHz, DMSO-d6) δ 8.22 (d, J= 6.4 Hz, 1 H), 7.79-7.77 (m, 2 H), 7.69 (m, 1 H), 7.50 (s, 1 H), 7.44-7.34 (m, 4 H), 7.09 (dd, J= 8.0, 2.4 Hz 1 H), 4.14 (m, 1 H), 3.87 (m, 1 H), 3.80 (s, 3 H), 1.94-1.92 (m, 2 H), 1.82-1.72 (m, 6 H).

**Example 2522**

**3-Methoxy-*N*-{*cis*-4-[(quinolin-2-ylmethyl)amino]cyclohexyl}benzamide trifluoroacetate**

**Step A: Synthesis of 3-methoxy-*N*-{cis-4-[(quinolin-2-ylmethyl)amino]cyclohexyl}benzamide trifluoroacetate.**

Using the procedure of step A of example 2510, the title compound was obtained.
ESI MS m/e 390.2 M + $H^+$; $^1$H NMR (400 MHz, $CD_3OD$) δ 8.41 (d, J = 8:8 Hz, 1 H), 8.14 (d, *J*= 8.4 Hz, 1 H), 7.99 (d, *J* = 8.0 Hz, 1 H), 7.84 (t, *J* = 7.2 Hz, 1 H), 7.67 (t, *J* = 7.2 Hz, 1 H), 7.55 (d, *J* = 8.4 Hz, 1 H), 7.43-7.36 (m, 3 H), 7.12-7.10 (m, 1 H), 4.66 (s, 2 H), 4.13 (m, 1 H), 3.85 (s, 3 H), 3.46 (m, 1 H), 2.16-2.05 (m, 4 H), 2.05-1.96 (m, 2 H), 1.85-1.78 (m, 2 H).

**Example 2523**

**N-(cis-4-{[4-(Dimethylamino)-5-methylpyrimidin-2-yl]amino}cyclohexyl)-4-methylbenzamide trifluoroacetate**

**Step A: Synthesis of 2-chloro-4-dimethylamino-5-methylpyrimidine.**

In 8 mL tetrahydrofuran was dissolved 2,4-dichloro-5-methylpyrimidine (0.5 g, 3.07 mmol) at 0 °C. To the reaction mixture was added dimethylamine (2M in methanol, 3.4 mL, 6.8 mmol) dropwise. The reaction mixture was stirred at 10°C for 1.5 hour: *do not increase the reaction temperature.* The solution was concentrated and purified by flash chromatography (silica gel, 20% ethyl acetate and 5% methanol in hexanes) to give 2-chloro-4-dimethylamino-5-methylpyrimidine (307 mg, 58%) as a white solid.
ESI MS m/e 172 M+$H^+$; $^1$H NMR (400 MHz, $CDCl_3$) δ 7.8 (s, 1 H), 3.18 (s, 6 H), 2.23 (s, 3 H).

**Step B: Synthesis of cis-[4-(4-dimethylamino-5-methyl-pyrimidin-2-ylamino)-cyclohexyl]-carbamic acid *tert*-butyl ester.**

To a suspension of 2-chloro-4-dimethylamino-5-methylpyrimidine (250mg, 1.46 mmol) in 2-propanol (2.5 mL) was added cis-(4-amino-cyclohexyl)-carbamic acid tert-butyl ester (340 mg, 1.60mmol) and DIEA (507 µL, 2.91 mmol). The reaction was performed in the Smith synthesizer for 4.5 hours at 175° C. The solution was concentrated and purified by flash chromatography (silica gel, 1% MeOH in $CH_2Cl_2$) to give cis-[4-(4-dimethylamino-5-methyl-pyrimidin-2-ylamino) cyclohexyl]-carbamic acid *tert*-butyl ester (219 mg, 43 %) as a pale yellow solid.
ESI MS m/e 350.4 M + $H^+$; $^1$H NMR (400 MHz, $CDCl_3$) δ 7.80 (s, 1 H), 4.6 (brs, 1 H), 3.94 (brs, I H), 3.60 (brs, 1 H), 3.02 (s, 6 H), 2.18 (s, 3 H), 1.85-1.70 (m, 8 H), 1.41 (s, 9 H).

**Step C: Synthesis of *cis*-4-(4-dimethylamino-5-methyl-pyrimidin-2-ylamino)-0-amino-cyclohexane.**

To a suspension of cis-[4-(4-dimethylamino-5-methyl-pyrimidin-2-ylamino) cyclohexyl]-carbamic acid tert-butyl ester (219 mg, 0.627 mmol) in DCM (3 mL) was added trifluoroacetic acid (2mL). The reaction stirred at room temperature for 2 hours and concentrated. A few drops $NaHCO_3$ was added, followed by 1M NaOH until the solution was basic. The product was extracted with $H_2O$ and $CH_2Cl_2$ three times. The organic layers were combined, dried over $MgSO_4$, filtered and concentrated to give cis-4-(4-dimethylamino-5-methyl-pyrimidin-2-ylamino)-4-aminocyclohexane (115.9 mg, 74 %) as yellow oil.
ESI MS m/e 250.2 M + $H^+$; $^1$H NMR (400 MHz, $CDCl_3$) δ 7.60 (s, 1H), 4.95 (brs, 1H), 3.90 (brs, 1H), 2.98 (s, 6 H), 2.80 (brs, 1 H), 2.48 (brs, 2 H), 2.04 (s, 3 H), 1.78 (m, 2 H), 1.62 (m, 4 H), 1.4 (m, 2 H).

**Step D: Synthesis of *N*-(*cis*-4-{[4-(dimethylamino)-5-methylpyrimidin-2-yl]amino)-cyclohexyl)-4-methylbenzamide trifluoroacetate.**

To a suspension of *cis*-4-(4-dimethylamino-5-methyl-pyrimidin-2-ylamino)-4-aminocyclohexane (30 mg, 0.12 mmol) was added 4-methyl-benzoyl chloride (15.8 µL, 0.12 mmol) and DIEA (5 drops). The reaction was stirred overnight at room temperature under argon gas. The solution was concentrated and the product purified using prep HPLC to give *N-(cis*-4-{[4-(dimethylamino)-5-methylpyrimidin-2-yl]amino}cyclohexyl)-4-methylbenzamide trifluoroacetate (29.1 mg, 50.4 %) as a white solid.
ESI MS m/e 368 M + $H^+$; $^1$H NMR (400 MHz, DMSO-$d_6$) δ 8.70 (s, 1 H), 7.68 (d, J = 8.0 Hz, 2 H), 7.24 (d, J = 8.0 Hz, 2 H), 6.72 (s, 1 H), 4.25 (s, 1H), 3.23 (s, 6 H), 2.71 (s, 1 H), 2.34 (s, 3 H), 2.27 (s, 3 H), 1.7-1.88 (m, 8 H).

**Example 2524**

***N*-(*cis*-4-{[4-(Dimethylamino)-5-methylpyrimidin-2-yl]amino}cyclohexyl)-3,4-difluorobenzamide hydrochloride**

**Step A: Synthesis of *N*-(*cis*-4-{[4-(dimethylamino)-5-methylpyrimidin-2-yl]amino)-cyclohexyl)-3,4-difluorobenzamide hydrochloride.**

To a suspension of cis-4-(4-dimethylamino-5-methyl-pyrimidin-2-ylamino)-4-aminocyclohaexane (70 mg, 0.28 mmol) in DCM (5 mL) was added 3,4-difluorobenzoyl chloride (50 mg, 0.28 mmol) and DIEA (45 µL, 0.28 mmol). The reaction was stirred overnight and the product was purified by column chromatography (silica gel, DCM/MeOH = 100:0 to 90:10). The purified product was dissolved in DCM (3 mL), and 2M-HCl in ethyl ether (0.3 mL) was added. After stirring 20 min, removal of the volatile solvent gave N-(cis-4-{[4-(dimethylamino)-5-methylpyrimidin-2-y)]amino} cyclohexyl)-3,4-difluorobenzamide hydrochloride (26 mg, 23 %) as a white solid.
ESI MS m/e 390 M + $H^+$; $^1H$ NMR (400 MHz, DMSO-$d_6$) δ 11.8 (brs, 1 H), 8.23 (brs, 1 H), 7.80 (m, 2 H), 7.63 (m, 1 H), 7.51 (s, 1 H), 7.40 (d, J = 8.8 Hz, 1 H), 3.74 (brs, 2 H), 3.14 (s, 6 H), 2.11 (s, 3 H), 1.73-1.58 (m, 8 H).

**Example 2525**

**3-Chloro-*N*-(cis-4-{[4-(dimethylamino)-5-methylpyrimidin-2-yl]amino}cyclohexyl)benzamide hydrochloride**

**Step A: Synthesis of 3-chloro-*N*-(cis-4-{[4-(dimethylamino)-5-methylpyrimidin-2-yl]amino}cyclohexyl) benzamide hydrochloride.**

Using procedure of step A of example 2524, the title compound was obtained.
ESI MS m/e 388 M + $H^+$; $^1H$ NMR (400 MHz, DMSO-$d_6$) δ 11.7 (brs, 1H), 8.22 (brs, 1 H), 7.72 (m, 2 H), 7.62 (d, J = 8.0 Hz, 1 H), 7.45 (s, 1 H), 7.42 (d, J = 8.0 Hz, 1 H), 7.32 (t, J = 7.6 Hz, 1 H), 3.70 (brs, 2 H), 3.10 (s, 6 H), 2.06 (s, 3 H), 1.68-1.54 (m, 8 H).

**Example 2526**

***N-(cis-4-*{[4-(Dimethylamino)-6-methylpyrimidin-2-yl]amino}cyclohexyl)-3-methylbenzamide trifluoroacetate**

**Step A: Synthesis of 2-chloro-4-dimethylamino-6-methylpyrimidine.**

In 100 mL tetrahydrofuran was dissolved 2,4-dichloro-6-methylpyrimidine (10 g, 61.3 mmol) at 0° C. To the reaction mixture was added dimethylamine (2M in methanol, 67.4 mL, 134.8 mmol) dropwise. The reaction mixture was stirred at 10 °C for 2.5 hours. The solution was concentrated and purified by flash chromatography (silica gel, 20% ethyl acetate and 5% methanol in hexanes) to give 2-chloro-4-dimethylamino-6-methylpyrimidine (4.18g, 40 %) as a pale yellow solid.
ESI MS m/e 172 M + $H^+$; $^1H$ NMR (400 MHz, $CDCl_3$) δ 6.25 (s, 1 H), 3.2 (s, 6 H), 2.64 (s, 3 H).

**Step B: Synthesis of cis-[4-(4-dimethylamino-6-methyl-pyrimidin-2-ylamino) cyclohexyl]-carbamic acid *tert*-butyl ester.**

To a suspension of 2-chloro-4-dimethylamino-6-methylpyrimidine (15 mg, 0.0874 mmol) in 2-propanol (1.7 mL) was added *cis*-(4-amino-cyclohexyl)-carbamic acid tert- butyl ester (20.6 mg, 0.096 mmol) and DIEA (30.3 µL, 0.175 mmol). The reaction was performed in the Smith synthesizer for 4.5 hours at 175 °C. The solution was concentrated and purified by flash chromatography (silica gel, 1% MeOH in $CH_2Cl_2$) to give *cis*-[4-(4-dimethylamino-6-methyl-pyrimidin-2-ylamino) cyclohexyl]-carbamic acid tert-butyl ester (18.9 mg, 62 %) as a pale yellow solid.
ESI MS m/e 350.4 M + $H^+$; $^1H$ NMR (400 MHz, $CDCl_3$) δ 5.65 (s, 1 H), 4.75 (brs, 1 H), 4.0 (brs, I H), 3.60 (brs, 1 H), 3.05 (s, 6 H), 2.22 (s, 3 H), 1.78 (m, 6 H), 1.59 (m, 2 H), 1.44 (s, 9 H).

**Step C: Synthesis of *cis*-4-(4-dimethylamino-6-methyl-pyrimidin-2-ylamino)4-aminocyclohexane.**

To a suspension of cis-[4-(4-dimethylamino-6-methyl-pyrimidin-2-ylamino) cyclohexyl]-carbamic acid tert-butyl ester (617 mg, 1.77 mmol) in DCM (3 mL) was added trifluoroacetic acid (2mL). The reaction stirred at room temperature for 2 hours and concentrated. A few drops $NaHCO_3$ was added, followed by 1M NaOH until the solution was basic. The product was extracted with $H_2O$ and $CH_2Cl_2$ three times. The organic layers were combined, dried

over $MgSO_4$, filtered and concentrated to give cis-4-(4-dimethylamino-6-methyl-pyrimidin-2-ylamino)-4-aminocyclohexane (318 mg, 72 %) as yellow oil.
ESI MS m/e 250 M + $H^+$; $^1H$ NMR (400 MHz, $CDCl_3$) δ 5.52 (s, 1 H), 5.10 (brs, 1 H), 3.88 (brs, 1 H), 3.20 (brs, 2 H), 2.88 (s, 6 H), 2.75 (s, 1 H), 2.04 (s, 3 H), 1.70 (m, 2 H), 1.58 (m, 4 H), 1.38 (m, 2 H).

**Step D: Synthesis of *N*-(*cis*-4-{[4-(dimethylamino)-6-methylpyrimidin-2-yl]amino}cyclohexyl)-3-methylbenzamide trifluoroacetate.**

Using the procedure of step D of example 2523, the title compound was obtained.
ESI MS m/e 368.4 M + $H^+$; $^1H$ NMR (400 MHz, DMSO-$d_6$) 9.0 (s, 1 H), 7.6 (m, 2 H), 7.22 (s, 1 H), 6.72 (s, 1 H), 5.68 (s, 1 H), 4.2 (s, 1 H), 4.12 (s, 1 H), 3.18 (s, 3 H), 3.08 (s, 3 H), 2.35 (s, 3 H), 2.29 (s, 3 H), 1.85-1.62 (m, 8 H).

**Example 2527**

***cis*-4-{[4-(Dimethylamino)-6-methylpyrimidin-2-yl]amino}-N-[3-(trifluoromethyl) benzyl]-cyclohexanecarboxamide**

**Step A: Synthesis of cis-4-(4-dimethylamino-6-methyl-pyrimidin-2-ylamino) cyclohexanecarboxylic acid ethyl ester.**

To a suspension of 2-chloro-4-dimethylamino-6-methylpyrimidine (250 mg, 1.46 mmol) in 2-propanol (1.5 mL) was added cis-4-amino-cyclohexanecarboxylic acid ethyl ester hydrochloride (330 mg, 1.59 mmol) and DIEA (0.60 mL, 3.44 mmol). The reaction was performed in the Smith synthesizer for I hour at 155 °C. The solution was concentrated and purified by flash chromatography (silica gel, 1% MeOH in $CH_2Cl_2$) to give cis-4-(4-dimethylamino-6-methyl-pyrimidin-2-ylamino) cyclohexanecarboxylic acid ethyl ester (378.9 mg, 84.7%) as a pale yellow solid.
ESI MS m/e 307 M + $H^+$; $^1H$ NMR (400 MHz, DMSO-$d_6$) δ 7.62 (brs, 1 H), 6.21 (s, 1 H), 4.04 (q, J = 6.4 Hz, 2 H), 3.98 (brs, 1 H), 3.15 (s, 6 H), 2.20 (s, 3 H), 1.58-1.80 (m, 8 H), 1.20 (t, J = 6.0 Hz, 3 H).

**Step B: Synthesis of cis-4-(4-dimethylamino-6-methyl-pyrimidin-2-ylamino) cyclohexanecarboxylic acid.**

To a suspension of cis-4-(4-dimethylamino-6-methyl-pyrimidin-2-ylamino) cyclohexanecarboxylic acid ethyl ester (597.6 mg, 1.95 mmol) in $H_2O$ (10 mL) and ethanol (0.3 mL) was added KOH (547 mg, 9.75 mmol). The reaction was stirred at 70 °C for 2.5 hours until reaction was homogenous. The reaction was cooled in an ice bath and acidified with concentrated HCl. The product was purified using flash chromatography (silica gel, 0-10% MeOH in $CH_2Cl_2$) to give cis-4-(4-dimethylamino-6-methyl-pyrimidin-2-ylamino) cyclohexanecarboxylic acid (302 mg, 55%) as a white solid.
ESI MS m/e 279.2 M + $H^+$; $^1H$ NMR (400 MHz, $CDCl_3$) δ 8.50 (brs, 1 H), 5.79 (s, 1 H), 4.02 (brs, 1 H), 3.19 (brs, 6 H), 2.49 (brs, 1 H), 2.29 (s, 3 H), 2.05 (m, 2 H), 1.81 (m, 2 H), 1.64 (m, 4 H).

**Step C: Synthesis of *cis*-4-{[4-(dimethylamino)-6-methylpyrimidin-2-yl]amino}-*N*-[3-(trifluoromethyl)benzyl]-cyclohexanecarboxamide.**

To a suspension of 3-trifluoromethylbenzylamine (14μL, 0.0987 mmol) and *cis-4-(4*-dimethylamino-6-methyl-pyrimidin-2-ylamino) cyclohexanecarboxylic acid (25mg, 0.0898 mmol) in DCM (5 mL) was added HATU (37.5mg, 0.0987 mmol). The reaction stirred at room temperature under argon for 30 seconds and triethylamine (5 drops) was added. The reaction stirred under argon at room temperature for 16 hours. The reaction was quenched by diluting with 5 mL DCM, followed by washing twice with saturated $NaHCO_3$ (5mL), twice with 1M HCl (5mL) and once with $H_2O$ (5mL). The product was purified by filtering through silica gel with 0-10% MeOH in $CH_2Cl_2$ to give *cis*-4-{[4-(dimethylamino)-6-methylpyrimidin-2-yl]amino}-N-[3-(trifluoromethyl)benzyl]-cyclohexanecarboxamide (17.6mg, 45 %) as a white solid.
ESI MS m/e 436 M+$H^+$; $^1H$ NMR (400 MHz, $CDCl_3$) δ 8.01 (brs, 1 H), 7.59 (brs, 1 H), 7.53 (m, 2 H), 7.40 (m, 2 H), 5.76 (s, 1 H), 4.50 (d, J = 6.4 Hz, 2 H), 4.28 (brs, 1 H), 3.19 (s, 6 H), 2.39 (m, 1 H), 2.30 (s, 3 H), 2.0 (m, 2 H), 1.87 (m, 4 H), 1.60 (m, 2 H).

**Example 2528**

***cis-4*-{(4-(Dimethylamino)-5-methylpyrimidin-2-yl]amino}-*N*-(3-(propionylamino)benzyl]-cyclohexaneearboxamide**

**Step A: Synthesis of *cis*-[4-(3-nitrobenzylcarbamoyl)-cyclohexyl]-carbamic acid tert-butyl ester.**

cis-4-(tert-Butoxycarbonylamino)-cyclohexanecarboxylic acid (2.0 g, 8.2 mmol) and 3-nitrobenzyl amine hydrochloride (1.54 g, 8.2 mmol) in DCM (30 mL) was reacted in the presence of HATU (3.5 g, 9.02 mmol) and $Et_3N$ (4 mL). The reaction was diluted with DCM, washed with 1N-HCl and water, dried over $MgSO_4$, and concentrated. From column chromatography (silica gel, DCM/MeOH = 100:0 to 95 to 5), 2.7 g (90 %) of cis-[4-(3-nitrobenzylcarbamoyl)-cyclohexyl]-carbamic acid *tert*-butyl ester was isolated.
ESI MS m/e 378 M + $H^+$; $^1H$ NMR (400 MHz, $CDCl_3$) δ 8.11 (brs, 1H), 8.09 (s, 1 H), 7.60 (d, *J* = 8.0 Hz, 1 H), 7.48 (t, *J* = 7.6 Hz, 1 H), 6.17 (brs, 1 H), 4.72 (brs, 1 H), 4.53 (d, *J* = 6.0 Hz, 2 H), 3.74 (brs, 1 H), 2.27 (m, 1 H), 1.80-1.71 (m, 6 H), 1.65-1.59 (m, 2 H), 1.45 (s, 9 H).

**Step B: Synthesis of *cis*-4-amino-cyclohexanecarboxylic acid 3-nitro-benzamide hydrochloride.**

*cis*-[4-(3-Nitrobenzylcarbamoyl)-cyclohexyl]-carbamic acid *tert-butyl* ester (2.5 g, 6.6 mmol) was reacted in TFA/DCM (1:2 = 23 mL) for 2 hr at room temperature. After removal of the solvents, the residue was dissolved in DCM (15 mL), and added 2M-HCl in ethyl ether (2 eq.). After stirring for 20 min at room temperature, the volatile solvent was removed to give *cis-4*-amino-cyclohexanecarboxylic acid 3-nitro-benzamide hydrochloride (2.0 g, 95 %) as a yellowish white solid.
ESI MS m/e 278 M + $H^+$; $^1H$ NMR (400 MHz, DMSO-$d_6$) δ 8.53 (t, J = 6.0 Hz, 1 H), 8.07 (d, J= 7.6 Hz, 1 H), 8.06 (s, 1 H), 7.84 (brs, 2 H), 7.68 (d, J = 7.6 Hz, 1 H), 7.59 (t, J = 7.6 Hz, 1 H), 4.37 (d, *J* = 6.4 Hz, 2 H), 3.13 (m, 1 H), 2.40 (m, 1H), 1.89 (m, 2 H), 1.68 (m, 4 H), 1.57 (m, 2 H).

**Step C: Synthesis of cis-4-(4-dimethylamino-5-methyl-pyrimidin-2-ylamino)-cyclohexanecarboxylic acid 3-nitro-benzylamide.**

2-Chloro-4-dimethylamino-5-methylpyrimidine (0.31 g, 1.8 mmol) and *cis*-4-amino-cyclohexanecarboxylic acid 3-nitro-benzylamide hydrochloride (0.52 g, I eq.) in IPA (2.5 mL) and DIEA (0.7 mL) was reacted in a Smith synthesizer. The reaction was diluted with DCM, washed with1N-HCl and water, dried over $MgSO_4$, and concentrated. The crude compound was purified from column chromatography (silica gel, DCM/MeOH = 100:0 to 90:10) to give 0.23 g (31 %) of *cis*-4-(4-dimethylamino-5-methyl-pyrimidin-2-ylamino)-cyclohexanecarboxylic acid 3-nitro-benzylamide.
ESI MS m/e 413 M + $H^+$; $^1H$ NMR (400 MHz, $CDCl_3$) δ 8.11 (brs, 1H), 8.03 (d, *J* = 8.0 Hz, 1H), 7.95 (brs, 1H), 7.62 (d, *J*= 8.0 Hz, 1H), 7.43 (t, *J*= 7.6 Hz, 1H), 7.28 (s, 1H), 4.51 (d, *J*= 5.6 Hz, 2 H), 4.33 (m, 1 H), 3.23 (s, 6 H), 2.39 (m, 1 H), 2.22 (s, 3 H), 2.02 (m, 2 H), 1.86 (m, 4 H), 1.60 (m, 2 H).

**Step D: Synthesis of cis-4-(4-dimethylamino-5-methyl-pyrimidin-2-ylamino)-cyclohexanecarboxylic acid 3-amino-benzylamide.**

A solution of cis-4-(4-dimethylamino-5-methyl-pyrimidin-2-ylamino)-cyclohexane carboxylic acid 3-amino-benzylamide (0.21 g, 0.5 mmol) and 10 % Pd/C (50 mg) in EtOH (10 mL) was stirred overnight under $H_2$ atmosphere at room temperature. The reaction was filtered through a pad of celite. After removal of the volatile solvent, the residue was purified from a short pad of silica gel (DCM/MeOH = 100:0 to 80:20) to give 0.18 g (95 %) of cis-4-(4-dimethylamino-5-methyl-pyrimidin-2-ylamino)-cyclohexanecarboxylic acid 3-amino-benzylamide as the desired product.
ESI MS m/e 383 M + $H^+$; $^1H$ NMR (400 MHz, $CDCl_3$) δ 7.29 (s, 1 H), 7.03 (t, J = 7.6 Hz, 1 H), 6.64 (m, 2 H), 6.51 (d, J = 8.0 Hz, 1 H), 4.33 (d, J = 5.6 Hz, 2 H), 4.25 (brs, 1 H), 3.19 (s, 6 H), 2.32 (m, 1 H), 2.19 (s, 3 H), 1.97-1.78 (m, 6 H), 1.62 (m, 2 H).

**Step E: Synthesis of *cis*-4-{[4-(dimethylamino)-5-methylpyrimidin-2-yl]amino}-*N*-[3-(propionylamino)benzyl] cyclohexanecarboxamide.**

cis-4-(4-Dimethylamino-5-methyl-pyrimidin-2-ylamino)-cyclohexanecarboxylic acid 3-amino-benzylamide (30 mg, 0.075 mmol) and propionyl chloride (7 mg, 0.075 mmol) was reacted in the presence of catalytic $Et_3N$ (7 drops). The product was purified from column chromatography (silica gel, DCM/MeOH = 100:0 to 90:10) to give cis-4-{[4-(dimethylamino)-5-methylpyrimidin-2-yl]amino}-N-[3-(propionylamino)benzyl]cyclohexanecarboxamide (11 mg, 32

%).
ESI MS m/e 439 M + H+; $^1$H NMR (400 MHz, $CDCl_3$) δ 8.63 (brs, 1 H), 7.92 (brs, 1 H), 7.35 (s, I H), 7.28 (s, 1 H), 7.21 (t, J= 7.6 Hz, 1 H), 6.92 (d, *J* = 7.6 Hz, 1 H), 6.46 (brs, 1 H), 4.42 (d, *J* = 6.0 Hz, 2 H), 4.23 (m, 1 H), 3.22 (s, 6 H), 2.47 (d, J = 7.6 Hz, 2 H), 2.33 (m, 1 H), 2.22 (s, 3 H), 1.95-1.90 (m, 6 H), 1.63 (m, 2 H), 1.22 (t, J = 7.6 Hz, 3 H).

**Example 2529**

**cis-4-1[4-(Dimethylamino)-5-methylpyrimidin-2-yl]aminol-N-[3-(isobutyrylamino)benzyl]-cyclohexanecarboxamide**

**Step A: Synthesis of cis-4-{(4-(dimethylamino)-5-methylpyrimidin-2-yl]amino}-N-[3-(isobutyrylamino)benzyl] cyclohexanecarboxamide.**

Using the procedure of step E of example 2528, the title compound was obtained.
ESI MS m/e 453 M + H+;$^1$H NMR (400 MHz, $CDCl_3$) δ 8.80 (brs, 1 H), 8.10 (brs, 1H), 7.93 (brs, 1 H), 7.39 (s, 1H), 7.23 (s, 1 H), 7.18 (t, J= 7.6 Hz, 1 H), 6.91 (d, J= 7.6 Hz, 1H), 6.69 (brs, 1H), 4.40 (d, J = 5.6 Hz, 2 H), 4.22 (m, 1H), 3.23 (s, 6 H), 2.74 (m, 1 H), 2.33 (m, 1 H), 2.20 (s, 3H), 1.96-1.87 (m, 6 H), 1.60 (m, 2 H), 1.22 (d, J = 6.4 Hz, 6 H).

**Example 2530**

***cis*-4-{[4-(Dimethylamino)-5-methylpyrimidin-2-yl]amino}-*N*-{3-[(3-methylbutanoyl)amino]-benzyl} cyclohexanecarboxamide.**

**Step A: Synthesis of *cis*-4-{[4-(dimethylamino)-5-methylpyrimidin-2-yl]amino}-*N*-{3-[(3-methylbutanoyl)amino] benzyl}cyclohexanecarboxamide.**

Using the procedure of step E of example 2528, the title compound was obtained.
ESI MS m/e 467 M + H+; $^1$H NMR (400 MHz, $CDCl_3$) δ 8.62 (brs, 1 H), 8.04 (brs, 1 H), 7.91 (d, J = 7.2 Hz, 1 H), 7.35 (s, 1 H), 7.26 (s, 1 H), 7.20 (t, J = 7.6 Hz, 1 H), 6.93 (d, J = 7.6 Hz, 1 H), 6.59 (brs, 1 H), 4.42 (d, J= 5.2 Hz, 2 H), 4.22 (m, 1 H), 3.23 (s, 6 H), 2.33 (m, 1 H), 2.31 (d, J= 7.2 Hz, 2H), 2.23 (m, 1 H),2.21 (s, 3 H), 1.96-1.87 (m, 6 H), 1.62 (m, 2 H), 1.00 (d, J = 6.0 Hz, 6 H).

**Example 2531**

***cis-4-*{[4-(Dimethylamino)-5-methylpyrimidin-2-yl]amino}-N-{3-[(2,2-dimethyl-propanoyl)amino]benzyl} cyclohexanecarboxamide**

**Step A: Synthesis of *cis*-4-{[4-(dimethylamino)-5-methylpyrimidin-2-yl]amino}-N-(3-[(2,2-dimethylpropanoyl) amino]benzyl}cyclohexanecarboxamide.**

Using the procedure of step E of example 2528, the title compound was obtained.
ESI MS m/e 467 M + H+; $^1$H NMR (400 MHz, $CDCl_3$) δ 7.84 (brs, 1 H), 7.78 (d, *J* = 7.6 Hz, 1 H), 7.40 (s, 1 H), 7.25 (s, 1 H), 7.22 (t, *J* = 7.6 Hz, 1 H), 7.00 (d, *J* = 7.6 Hz, I H), 6.85 (brs, 1 H), 4.41 (d, *J*= 5.6 Hz, 2 H), 4.23 (m, 1 H), 3.23 (s, 6 H), 2.34 (m, 1 H), 2.22 (s, 3 H), 1.99-1.84 (m, 6 H), 1.60 (m, 2 H), 1.33 (s, 9 H).

**Example 2532**

**cis-N-)3-[(Cyclobutylcarbonyl)amino]benzyl}-4-{[4-(dimethylamino)-5-methylpyrimidin-2-yl]amino} cyclohexanecarboxamide**

**Step A: Synthesis of cis-N-{3-[(cyclobutylcarbonyl)amino]benzyl]-4-{[4-(dimethylamino)-5-methylpyrimidin-2-yl]amino}cyclohexanecarboxamide.**

Using the procedure of step E of example 2528, the title compound was obtained.
ESI MS m/e 465 M + H+; $^1$H NMR (400 MHz, $CDCl_3$) δ 8.50 (brs, 1 H), 8.23 (brs, 1 H), 7.93 (d, J = 6.4 Hz, 1 H), 7.33 (s, 1 H), 7.24 (s, 1 H), 7.20 (t, J = 8.0 Hz, 1 H), 6.92 (d, J= 8.0 Hz, 1 H), 6.76 (brs, 1 H), 4.41 (d, J= 5.6 Hz, 2 H), 4.23 (m, 1 H), 3.33 (m, 1 H), 3.24 (s, 6 H), 2.35 (m, 4 H), 2.21 (s, 3 H), 2.18 (m, 1 H), 2.00-1.87 (m, 8 H), 1.60 (m, 2 H).

**Example 2533**

***cis-N-*{3-[(Cyclopentylcarbonyl)amino]benzyl}-4-{[4-(dimethylamino)-5-methylpyrimidin-2-yl]amino} cyclohexanecarboxamide**

**Step A: Synthesis of *cis*-N-{3-[(cyclopentylcarbonyl)amino]benzyl}-4-{[4-(dimethylamino)-5-methylpyrimidin-2-yl]amino}cyclohexanecarboxamide.**

Using the procedure of step E of example 2528, the title compound was obtained.
ESI MS m/e 479 M + H$^+$; $^1$H NMR (400 MHz, $CDCl_3$) δ 8.47 (brs, 1 H), 8.10 (brs, 1 H), 7.91 (d, J = 6.4 Hz, 1 H), 7.35 (s, 1 H), 7.26 (s, 1 H), 7.20 (t, J = 8.0 Hz, 1 H), 6.93 (d, J = 7.6 Hz, 1 H), 6.61 (brs, 1 H), 4.42 (d, J = 5.6 Hz, 2 H), 4.22 (m, 1 H), 3.22 (s, 6 H), 2.85 (m, 1 H), 2.33 (m, 1 H), 2.21 (s, 3 H), 2.00-1.88 (m, 10 H), 1.75 (m, 2 H), 1.60 (m, 4 H).

**Example 2534**

***cis*-N-{3-[(Cyclohexylcarbonyl)amino }benzyl]-4-{[4-(dimethylamino)-5-methylpyrimidin-2-yl]amino} cyclohexanecarboxamide.**

**StepA: Synthesis of *cis-N-*{3-[(cyclohexylcarbonyl)amino]benzyl}-4-{[4-(dimethylamino)-5-methylpyrimidin-2-yl]amino]cyclohexanecarboxamide.**

Using the procedure of step E of example 2528, the title compound was obtained.
ESI MS m/e 493 M + H$^+$; $^1$H NMR (400 MHz, $CDCl_3$) δ 8.30 (brs, 1 H), 8.01 (brs, 1 H), 7.86 (d, J = 7.6 Hz, 1 H), 7.36 (s, 1 H), 7.26 (s, 1 H), 7.20 (t, J = 8.0 Hz, 1 H), 6.94 (d, J = 7.6 Hz, 1 H), 6.65 (brs, 1 H), 4.41 (d, J = 5.2 Hz, 2 H), 4.22 (m, 1 H), 3.22 (s, 6 H), 2.35 (m, 2 H), 2.21 (s, 3 H), 196-1.28 (m, 18 H).

**Example 2535**

***cis-N-*{3-[(Cyclopropylcarbonyl)amino]benzyl}-4-{[4-(dimethylamino)-5-methylpyrimidin-2-yl]amino} cyclohexanecarboxamide**

**Step A: Synthesis of *cis-N-*{3-[(cyclopropylcarbonyl)amino]benzyl}-4-{[4-(dimethylamino)-5-methylpyrimidin-2-yl]amino}cyclohexanecarboxamide.**

Using the procedure of step E of example 2528, the title compound was obtained.
ESI MS m/e 451 M + H$^+$; $^1$H NMR (400 MHz, $CDCl_3$) δ 9.10 (brs, 1 H), 7.93 (m, 1 H), 7.36 (s, I H), 7.25 (s, 1 H), 7.19 (t, *J*= 8.0 Hz, 1 H), 6.90 (d, *J* = 7.2 Hz, 1H), 6.50 (brs, 1 H), 4.43 (d, J = 6.0 Hz, 2 H), 4.23 (m, 1 H), 3.23 (s, 6 H), 2.33 (m, 1 H), 2.21 (s, 3 H), 1.96-1.88 (m, 7 H), 1.63 (m, 2 H), 1.03 (m, 2 H), 0.79 (m, 2 H).

**Example 2536**

***N*-[(*cis*-4-{[4-(Dimethylamino)-5-methylpyrimidin-2-yl]amino}cyclohexyl)methyl]-3-[(3-methylbutanoyl)amino] benzamide.**

**Step A: Synthesis of {*cis*-4-[(3-nitro-benzoylamino)-methyl]-cyciohexyl}-carbamic acid *tert-butyl* ester.**

A mixture of cis-(4-aminomethyl-cyclohexyl)-carbamic acid tert-butyl ester (1.55 g, 6.8 mmol) and 3-nitrobenzoyl chloride (1.25 g, 6.8 mmol) was stirred overnight at room temperature, diluted with DCM, washed with 1N-HCl and water, and concentrated. The crude product was preliminary purified by a short pad of silica gel with DCM/MeOH (100:0 to 90:10) to give 1.5 g (75 %) of {cis-4-[(3-nitro-benzoylamino)-methyl]-cyclohexyl}-carbamic acid tert-butyl ester.
ESI MS m/e 378 M + H$^+$; $^1$H NMR (400 MHz, $CDCl_3$) δ 8.54 (t, J = 2.0 Hz, 1 H), 8.33 (d, J = 8.0 Hz, 1 H), 8.14 (d, J = 8.0 Hz, 1 H), 7.63 (t, J = 7.6 Hz, 1 H), 6.31 (brs, 1 H), 4.62 (brs, 1 H), 3.73 (brs, 1 H), 3.41 (t, J = 6.4 Hz, 2 H), 1.72-1.57 (m, 7 H), 1.44 (s, 9 H), 1.32 (m, 2 H).

**Step B: Synthesis of cis-N-(4-amino-cyclohexylmethyl)-3-nitro-benzamide hydrochloride.**

{*cis*-4-[(3-Nitro-benzoylamino)-methyl]-cyclohexyl}-carbamic acid tert-butyl ester (1.4 g, 3.7 mmol) in DCM/TFA (1:1 = 13 mL) was stirred for 2 h at room temperature. After removal of the volatile solvent, the residue was

dissolved in DCM (10 mL), and 2M-HCl in ether (4 mL) was added. After stirring for 20 min at room temperature, removal of the volatile solvent gave 1.2 g (82 %) of *cis-N*-(4-amino-cyclohexylmethyl)-3-nitro-benzamide hydrochloride as the desired product.
ESI MS m/e 278 M + $H^+$; $^1H$ NMR (400 MHz, DMSO-$d_6$) δ 8.91 (t, *J* = 5.6 Hz, 1 H), 8.65 (m, 1 H), 8.36 (d, J = 2.0 Hz, 1 H), 8.29 (d, J = 8.0 Hz, 1 H), 7.97 (brs, 2 H), 7.74 (t, J = 8.0 Hz, 1 H), 3.25 (t, J = 6.8 Hz, 2 H), 3.13 (brs, 1 H), 1.77 (m, 1 H), 1.65-1.61 (m, 4 H), 1.51 (m, 4 H).

**Step C: Synthesis of cis-*N*-[4-(4-dimethylamino-5-methyl-pyrimidin-2-ylamino)-cyclohexylmethyl]-3-nitro-benzamide.**

A solution of 2-chloro-4-dimethylamino-5-methylpyrimidine (0.25 g, 1.46 mmol) and *cis*-N-(4-amino-cyclohexylmethyl)-3-nitro-benzamide hydrochloride (0.46 g) in IPA (2 mL) and DIEA (0.46 mL) was reacted for I hr 10 min. at 155 °C in a Smith synthesizer. The reaction was diluted with DCM, washed with 1N-HCl and water, dried over $MgSO_4$, and concentrated. The crude compound was purified from column chromatography (silica gel, DCM/MeOH = 100:0 to 90:10) to give 0.23 g (38 %) of *cis*-N-[4-(4-dimethylamino-5-methyl-pyrimidin-2-ylamino)-cyclohexylmethyl]-3-nitro-benzamide.
ESI MS m/e 413.2 M + $H^+$; $^1H$ NMR (400 MHz, $CDCl_3$) δ 8.7 (t, *J*= 2.0 Hz, 1 H), 8.34 (d, *J*= 7.6 Hz, 1 H), 8.28 (d, *J* = 8.0 Hz, , 1 H), 8.20 (brs, 1 H), 7.60 (t, J = 7.6 Hz, 1 H), 7.35 (brs, 1 H), 7.25 (s, 1 H), 4.18 (m, 1 H), 3.48 (t, J = 4.8 Hz, 2 H), 3.24 (s, 6 H), 2.21 (s, 3H), 1.89-1.57 (m, 9 H).

**Step D: Synthesis of *N*-[(cis-4-{[4-(dimethylamino)-5-methylpyrimidin-2-yl]amino}-cyclohexyl)methyl]-3-[(3-methylbutanoyl)aminolbenzamide.**

A solution of cis-N-[4-(4-dimethylamino-5-methyl-pyrimidin-2-ylamino)-cyclohexylmethyl)-3-nitro-benzamide (0.22 g, 0.53 mmol) and 10 % Pd/C (30 mg) in EtOH (15 mL) was stirred overnight under $H_2$ atmosphere at room temperature. The reaction was filtered through a pad of celite. After removal of the volatile solvent, the residue was purified from a short pad of silica gel (DCM/MeOH = 100:0 to 80:20) to give 0.19 g (95 %) as yellowish oil. 17 mg (0.04 mmol) of this oil was reacted with isovaleryl chlorides (5 mg, 0.04 mmol) using the procedure of step E of example 2528 to give *N*-[(*cis*-4-{[4-(dimethylamino)-5-methylpyrimidin-2-yl]amino}cyclohexyl)methyl]-3-[(3-methylbutanoyl)amino]benzamide (9 mg, 47 %).
ESI MS m/e 467.6 M + $H^+$; $^1H$ NMR (400 MHz, $CDCl_3$) δ 9.11 (brs, 1 H), 8.22 (d, J= 7.2 Hz, 1 H), 8.04 (s, 1 H), 7.55 (d, J = 7.6 Hz, 1 H), 7.34 (t, J = 7.6 Hz, 1 H), 7.30 (s, 1 H), 6.42 (m, 1 H), 4.14 (m, 1 H), 3.43 (t, J = 4.8 Hz, 2 H), 3.19 (s, 6 H), 2.33 (d, J = 6.8 Hz, 2 H), 2.25 (m, 1 H), 2.20 (s, 3 H), 1.94 (m, 2 H), 1.72~1.59 (m, 7 H), 1.02 (d, J = 6.4 Hz, 6 H).

**Example 2537**

***N*-[(*cis*-4-{[4-(Dimethylamino)-5-methylpyrimidin-2-yl]amino}cyclohexyl)methyl]-3-(propionylamino) benzamide**

**Step A: Synthesis of *N*-[(*cis*-4-{[4-(dimethylamino)-5-methylpyrimidin-2-yl]amino}-cyclohexyl)methyl]-3-(propionylamino)benzamide.**

Using the procedure of step D of example 2536, the title compound was obtained.
ESI MS m/e 439 M + $H^+$; $^1H$ NMR (400 MHz, $CDCl_3$) δ 9.10 (brs, 1 H), 8.23 (d, *J* = 7.2 Hz, 1 H), 8.02 (s, 1 H), 7.55 (d, J= 8.0 Hz, 1 H), 7.35 (t, J= 7.6 Hz, 1 H), 7.29 (s, 1 H), 6.36 (m, 1 H), 4.16 (brs, 1 H), 3.47 (m, 2 H), 3.21 (s, 6 H), 2.50 (q, *J*= 7.6 Hz, 2 H), 2.21 (s, 3 H), 1.95 (m, 2 H), 1.72-1.61 (m, 7 H), 1.25 (t, J = 7.2 Hz, 3 H).

**Example 2538**

***N*-[(*cis*-4-{[4-(Dimethylamino)-5-methylpyrimidin-2-yl]amino}cyclohexyl)methyl]-3-(isobutyrylamino) benzamide**

**Step A: Synthesis of *N*-[(*cis*-4-{[4-(dimethylamino)-5-methylpyrimidin-2-yl]amino}-cyclohexyl)methyl]-3-(isobutyrylamino)benzamide**

Using the procedure of step D of example 2536, the title compound was obtained.
ESI MS m/e 453 M + $H^+$; $^1H$ NMR (400 MHz, $CDCl_3$) δ 9.09 (brs, 1 H), 8.23 (d, *J* = 6.8 Hz, 1 H), 8.07 (s, 1 H), 7.55 (d, J= 7.6 Hz, 1 H), 7.34 (t, J= 7.6 Hz, 1 H), 7.29 (s, 1 H), 6.38 (m, 1 H), 4.16 (brs, 1 H), 3.45 (m, 2 H), 3.21 (s, 6 H), 2.73

(m, 1 H), 2.20 (s, 3 H), 1.95 (m, 2 H), 1.72-1.61 (m, 7 H), 1.26 (d, *J* = 6.8 Hz, 6 H).

**Example 2539**

**3-[(Cyclopropylearbonyi)amino]-N-[(cis4-{[4-(dimethylamino)-5-methylpyrimidin-2-yl]aminolcyclobexyl) methyl]benzamide**

**Step A: Synthesis of 3-[(cyclopropylcarbonyl)aminol-*N*-[(cis-4-1[4-(dimethylamino)-5-methylpyrimidin-2-yl] amino)cyclohexyl)methyl]benzamide**

Using the procedure of step D of example 2536, the title compound was obtained.
ESI MS m/e 451 M + H$^+$; $^1$H NMR (400 MHz, $CDCl_3$) δ 9.60 (brs, 1 H), 8.22 (d, J= 6.8 Hz, 1 H), 8.03 (s, 1 H), 7.56 (brs, 1 H), 7.55 (d, *J* = 7.6 Hz, 1 H), 7.32 (t, J = 7.6 Hz, 1 H), 7.28 (s, 1 H), 6.41 (m, 1 H), 4.15 (brs, 1 H), 3.45 (m, 2 H), 3.21 (s, 6 H), 2.20 (s, 3 H), 1.95 (m, 2 H), 1.89 (m, 1 H), 1.72~1.61 (m, 7 H), 1.05 (m, 2 H), 0.82 (m, 2 H).

***Ex*ample 2540**

**3-[(Cyclobutylcarbonyl)amino]-*N*-{(cis-4-{[4-(dimethylamino)-5-methylpyrimidin-2-yl)amino}cyclohexyl) methyl}benzamide**

**3-[(cyclobutylcarbonyl)amino]-*N*-[(*cis*-4-{[4-(dimethylamino)-5-methylpyrimidin-2-yl]amino}cyclohexyl) methyl]benzamide.**

Using the procedure of step D of example 2536, the title compound was obtained.
ESI MS m/e 465 M + H$^+$; $^1$H NMR (400 MHz, $CDCl_3$) δ 8.90 (brs, 1 H), 8.21 (d, *J* = 6.8 Hz, 1 H), 8.04 (s, 1 H), 7.54 (d, *J*= 8.0 Hz, 1 H), 7.34 (t, *J* = 7.6 Hz, 1 H), 7.31 (s, 1 H), 6.41 (m, 1 H), 4.14 (brs, 1 H), 3.43 (t, *J* = 5.2 Hz, 2 H), 3.34 (m, 1 H), 3.19 (s, 6 H), 2.41 (m, 2 H), 2.23 (m, 1 H), 2.20 (s, 3 H), 2.02-1.88 (m, 4 H), 1.72-1.55 (m, 8 H).

**Example 2541**

**3-[(Cyclopentylcarbonyl)amino]-*N*-[(*cis*-4-{[4-(dimethylamino)-5-methylpyrimidin-2-yl]amino}cyclohexyl) methyl]benzamide.**

**Step A: Synthesis of 3-[(cyclopentylcarbonyl)amino)-N-[(*cis*-4-3 [4-(dimethylamino)-5-methylpyrimidin-2-yl] amino}cyclohexyl)methyl]benzamide.**

Using the procedure of step D of example 2536, the title compound was obtained.
ESI MS m/e 479 M + H$^+$;$^1$H NMR (400 MHz, $CDCl_3$) δ 9.14 (brs, 1 H), 8.22 (d, J= 7.2 Hz, 1 H), 8.07 (s, 1 H), 7.54 (d, J = 8.0 Hz, 1 H), 7.33 (t, J = 8.0 Hz, 1 H), 7.29 (s, 1 H), 6.43 (m, 1 H), 4.14 (brs, 1 H), 3.44 (t, J = 5.2 Hz, 2 H), 3.19 (s, 6 H), 2.91 (m,1 H), 2.20 (s, 3 H), 1.98-1.59 (m, 17 H).

**Example 2542**

**3-[(Cyclohexylcarbonyl)amino)-*N*-[(cis-4-{[4-(dimethylamino)-5-methylpyrimidin-2-yl)amino}cyclohexyl) methyl]benzamide**

**Step A: Synthesis of 3-[(cyclohexylcarbonyl)amino]-*N*-[(*cis*-4-{[4-(dimethylamino)-5-methylpyrimidin-2-yl] amino}cyclohexyl)methyl]benzamide.**

Using the procedure of step D of example 2536, the title compound was obtained.
ESI MS m/e 479 M + H$^+$; $^1$H NMR (400 MHz, $CDCl_3$) δ 8.94 (brs, 1 H), 8.17 (d, J= 7.2 Hz, 1 H), 8.05 (s, 1 H), 7.54 (d, J = 8.0 Hz, 1 H), 7.33 (t, J = 8.0 Hz, 1 H), 7.30 (s, 1 H), 6.44 (m, 1 H), 4.13 (brs, 1 H), 3.42 (t, J = 5.2 Hz, 2 H), 3.19 (s, 6 H), 2.42 (m,1 H), 2.20 (s, 3 H), 1.98-1.52 (m, 15 H), 1.29 (m, 4 H).

**Examples 2543-2553**

Compounds 2543 to 2553 were prepared in a similar manner as described in Example 2497 using the appropriate acid chloride and amine intermediate from Step D.

**Examples 2554-2557**

Compounds 2554 to 2557 were prepared in a similar manner as described in Example 2502 using the appropriate carboxylic acid and amine intermediate from Example 2497 Step D.

**Examples 2558-2561**

Compounds 2558 to 2561 were prepared in a similar manner as described in Example 2515 using the appropriate isocyanate and amine intermediate from Example 2497 Step D.

**Examples 2562 and 2563**

Compounds 2562 and 2563 were prepared in a similar manner as described in Example 2523 using the appropriate acid chloride and amine intermediate from Step C.

**Examples 2564-2570**

Compounds 2564 to 2570 were prepared in a similar manner as described in Example 2526 using the appropriate acid chloride and amine intermediate from Step C.

**Examples 2571-2587**

Compounds 2571 to 2587 were prepared in a similar manner as described in Example 2527 using the appropriate benzyl amine and carboxylic acid intermediate from Step B.

| Ex. No. | compound name | MS | class |
|---|---|---|---|
| 2543 | 3-methyl-N-{cis-4-[(4-methylquinolin-2-yl)amino]cyclohexyl } benzamide | 374.2 (M + H) | 1 |
| 2544 | 4-methyl-N-{cis-4-[(4-methylquinolin-2-yl)amino]cyclohexyl) benzamide | 374.2 (M+H) | 1 |
| 2545 | 4-fluoro-N-{cis-4-[(4-methylquinolin-2-yl)amino]cyclohexyl} benzamide | 378.2 (M + H) | 1 |
| 2546 | N-{cis-4-[(4-methylquinolin-2-yl)amino]cyclohexyl} -3-(trifluoromethyl)benzamide | 428 (M+H) | 3 |
| 2547 | 3-chloro-N-{cis-4-[(4-methylquinolin-2-yl)amino]cyclohexyl} benzamide | 394.2 (M + H) | 1 |
| 2548 | N-{cis-4-[(4-methylquinolin-2-yl)amino]cyclohexyl}-3,5-bis (trifluoromethyl)benzamide | 496.2 (M+H) | 1 |
| 2549 | 3-methoxy-N-{cis-4-[(4-methylquinolin-2-yl)amino]cyclohexyl} benzamide | 390.4 (M+H) | 1 |
| 2550 | 3-cyano-N-{cis-4-[(4-methylquinolin-2-yl)amino)cyclohexyl) benzamide | 385.2 (M + H) | 1 |
| 2551 | 2-(4-chlorophenoxy)-N-{cis-4-[(4-methylquinolin-2-yl)amino] cyclohexyl}acetamide | 424.2 (M+H) | 1 |
| 2552 | 3,4,5-trimethoxy-N-{cis-4-[(4-methylquinolin-2-yl)amino] cyclohexyl }benzamide | 450.4 (M + H) | 1 |
| 2553 | 3,5-dimethoxy-N-{cis-4-[(4-methylquinolin-2-yl)amino] cyclohexyl} benzamide | 420.2 (M + H) | 1 |
| 2554 | 2-(3-methoxyphenoxy)-N-{cis-4-[(4-methylquinolin-2-yl)amino] cyclohexyl } acetamide | 420.2 (M + H) | 1 |

(continued)

| Ex. No. | compound name | MS | class |
|---|---|---|---|
| 2555 | (2R)-2-(3-chlorophenyl)-2-hydroxy-N-{cis-4-[(4-methylquinolin-2-yl)amino]cyclohexyl} acetamide | 424.2 (M + H) | 1 |
| 2556 | 2-(3-methylphenoxy)-N-{cis-4-[(4-methylquinolin-2-yl)amino] cyclohexyl} acetamide | 404.2 (M + H) | 1 |
| 2557 | 5-bromo-N-{cis-4-[(4-methylquinolin-2-yl)amino]cyclohexyl]-2-428 (M furamide | 428 (M +H) | 1 |
| 2558 | N-[4-(benzyloxy)phenyl]-N'-{cis-4-[(4-methylquinolin-2-yl)amino] cyclohexyl} urea | 481.2 (M+H) | 2 |
| 2559 | N-{cis-4-[(4-methylquinolin-2-yl)amino]cyclohexyl} -N'-(4-phenoxyphenyl)urea | 467.4 (M + H) | 1 |
| 2560 | N-{cis-4-[(4-methylquinolin-2-yl)amino]cyclohexyl } -N'-(3-phenoxyphenyl)urea | 467.4 (M + H) | 2 |
| 2561 | N-{cis-4-[(4-methylquinolin-2-yl)amino]cyclohexyl} -N'-(2-phenoxyphenyl)urea | 467.4 (M + H) | 1 |
| 2562 | N-(cis-4-{[4-(dimethylamino)-5-methylpyrimidin-2-yl]amino} cyclohexyl)-3-methylbenzamide | 368.2 (M+H) | 1 |
| 2563 | N-(cis-4-{[4-(dimethylamino)-5-methylpyrimidin-2-yl]amino} cyclohexyl)-3-methoxybenzamide | 384.2 (M + H) | 1 |
| 2564 | N-(cis-4-{[4-(dimethylamino)-6-methylpyrimidin-2-yl]amino} cyclohexyl)-3-methoxybenzamide | 384.2 (M + H) | 1 |
| 2565 | N-(cis-4-{[4-(dimethylamino)-6-methylpyrimidin-2-yl]amino} cyclohexyl)-4-methylbenzamide | 368.2 (M + H) | 1 |
| 2566 | 4-chloro-N-(cis-4-{[4-(dimethylamino)-6-methylpyrimidin-2-yl] amino}cyclohexyl)benzamide | 388.2(M+H) | 1 |
| 2567 | 3-chloro-N-(cis-4-{[4-(dimethylamino)-6-methylpyrimidin-2-yl] amino}cyclohexyl)benzamide | 388.4 (M + H) | 1 |
| 2568 | N-(cis-4-{[4-(dimethylamino)-6-methylpyrimidin-2-yl]amino} cyclohexyl)-3,4-difluorobenzamide | 390.2 (M + H) | 1 |
| 2569 | N-(cis-4-{[4-(dimethylamino)-6-methylpyrimidin-2-yl]amino} cyclohexyl)-4-(trifluoromethoxy)benzamide | 438.2 (M + H) | 3 |
| 2570 | N-(cis-4-{[4-(dimethylamino)-6-methylpyrimidin-2-yl]amino} cyclohexyl)-4-fluorobenzamide | 372.2 (M + H) | 1 |
| 2571 | cis-4-{[4-(dimethylamino)-6-methylpyrimidin-2-yl]amino} -N-(3-iodobenzyl)cyclohexanecarboxamide | 494.2 (M + H) | 1 |
| 2572 | cis-N-(2,4-dichlorobenzyl)-4-{[4-(dimethylamino)-6-methylpyrimidin-2-yl]amino}cyclohexanecarboxamide | 436.2 (M + H) | 1 |
| 2573 | cis-N-(2,5-dichlorobenzyl)-4-{[4-(dimethylamino)-6-methylpyrimidin-2-yl]amino}cyclohexanecarboxamide | 436.2 (M + H) | 1 |
| 2574 | cis-4-{[4-(dimethylamino)-6-methylpyrimidin-2-yl]amino}-N-(4-methylbenzyl)cyclohexanecarboxamide | 382 .2 (M + H) | 1 |
| 2575 | cis-N-(3,5-dichlorobenzyl)-4-{[4-(dimethylamino)-6-methylpyrimidin-2-yl]amino}cyclohexanecarboxamide | 436.2 (M + H) | 1 |
| 2576 | cis-N-(3,5-dimethoxybenzyl)-4-{[4-(dimethylamino)-6-methylpyrimidin-2-yl]amino}cyclohexanecarboxamide | 428.2 (M + H) | 1 |

(continued)

| Ex. No. | compound name | MS | class |
|---|---|---|---|
| 2577 | cis-N-(3-chlorobenzyl)-4-{[4-(dimethylamino)-6-methylpyrimidin-2-yl]amino}cyclohexanecarboxamide | 402.2 (M + H) | 1 |
| 2578 | cis-N-[3,5-bis(trifluoromethyl)benzyl]-4-{[4-(dimethylamino)-6-methylpyrimidin-2-yl]amino}cyclohexanecarboxamide | 504.2 (M + H) | 1 |
| 2579 | cis-4-{[4-(dimethylamino)-6-methylpyrimidin-2-yl]amino}-N-(3-methoxybenzyl)cyclohexanecarboxamide | 398.2 (M + H) | 1 |
| 2580 | cis-N-(4-chlorobenzyl)-4-{[4-(dimethylamino)-6-2580 methylpyrimidin-2-yl]amino}cyclohexanecarboxamide | 402.2 (M + H) | 1 |
| 2581 | cis-N-(3,4-dichlorobenzyl)-4-{[4-(dimethylamino)-6-methylpyrimidin-2-yl]amino}cyclohexanecarboxamide | 436.2 (M + H) | 1 |
| 2582 | cis-N-(2,4-difluorobenzyl)-4-{[4-(dimethylamino)-6-methylpyrimidin-2-yl]amino}cyclohexanecarboxamide | 404.2 (M + H) | 1 |
| 2583 | cis-N-(2,5-difluorobenzyl)-4-{[4-(dimethylamino)-6-methylpyrimidin-2-yl]amino}cyclohexanecarboxamide | 404.2 (M + H) | 1 |
| 2584 | cis-N-(2,3-difluorobenzyl)-4-{[4-(dimethylamino)-6-methylpyrimidin-2-yl]amino cyclohexanecarboxamide | 404.2 (M + H) | 1 |
| 2585 | cis-N-(4-bromo-2-fluorobenzyl)-4-{[4-(dimethylamino)-6-methylpyrimidin-2-yl]amino}cyclohexanecarboxamide | 464.2 (M + H) | 1 |
| 2586 | cis-4-{[4-(dimethylamino)-6-methylpyrimidin-2-yl]amino}-N-(3-methylbenzyl)cyclohexanecarboxamide | 382.4 (M + H) | 1 |
| 2587 | cis-4-{[4-(dimethylamino)-6-methylpyrimidin-2-yl]amino}-N-[2-(trifluoromethoxy)benzyl]cyclohexanecarboxamide | 452.2 (M + H) | 1 |

**Example 2588,**

***N*-(cis-4-[4-(Dimethylamino)-5-methylpyrimidin-2-yl]amino}cyclohexyl)-3,5-bis(trifluoromethyl)benzamide hydrochloride**

**Step A: Synthesis of N-(*cis*-4-amino-cyclohexyl)-3,5-bistrifluoromethyl-benzamide trifluoroacetate.**

To a solution of *cis*-(4-amino-cyclohexyl)-carbamic acid *tert-butyl* ester (2.18 g, 10 mmol) in anhydrous benzene (25 mL) was slowly added 3,5-bistrifluoromethyl benzoyl chloride (2.8 g, 1 eq.) and followed by $Et_3N$ (~3mL) at room temperature under $N_2$ atmosphere: formation of solid salts makes stirring difficult. The reaction was stirred vigorously for an additional 2 h at room temperature, washed with sat.-$NaHCO_3$ (3x) and water (1x), dried with $MgSO_4$, and concentrated to give [*cis*-4-[(3,5-bistrifluoromethyl-benzoylamino)-cyclohexyl]-carbamic acid *tert-butyl* ester (4.5 g, 99 %), which was used for the next reaction without further purification. ESI MS m/e 455 $(M + H)^+$;$^1$H NMR (400 MHz, $CDCl_3$) δ 8.16 (s, 2 H), 7.98 (s, 1 H), 6.12 ( bs, 1 H), 4.58 (bs, 1 H), 4.11 (m, 1 H), 3.69 (bs, 1 H), 1.95~1.65 (m, 8 H), 1.44 (s, 9 H).

[*cis*-4-[(3,5-Bistrifluoromethyl-benzoylamino)-cyclohexyl]-carbamic acid *tert-butyl* ester (4.5 g, 10 mmol) was dissolved in DCM (20 mL), and TFA (10 mL) was added to the reaction. After 1.5 h stirring at room temperature, removal of the volatile solvent gave the crude N-(cis-4-amino-cyclohexyl)-3,5-bistrifluoromethyl-benzamide trifluoroacetate as a sticky oil. With addition of water (~50 mL) to the crude product, shaking for 5 to 10 min provided formation of precipitates. The precipitates were filtered, washed with water, and dried. 3.98 (82 %) of N-(cis-4-amino-cyclohexyl)-3,5-bistrifluoromethyl-benzamide trifluoroacetate was isolated as a white powder. ESI MS m/e 355 $(M + H)^+$; $^1$H NMR (400 MHz, DMSO-$d_6$) δ 8.62 (bd, 1H, J = 4.8 Hz), 8.47 (s, 2 H), 8.29 (s, 1H), 7.84 ( bs, 3 H), 3.91 (bs, 1H), 3.16 (bs, 1H), 1.94 (m, 2 H), 1.75~1,66 (m, 6 H).

**Step B: Synthesis of N-(cis4-1[4-(dimethylamino)-5-methylpyrimidin-2-yl]amino}-cyclohexyl)-3,5-bis (trifluoromethyl)benzamide hydrochloride.**

A sealed tube containing 2-chloro-4-dimethylamino-5-methylpyrimidine (0.25 g, 1.45 mmol), N-(cis-4-amino-cyclohexyl)-3,5-bistrifluoromethyl-benzamide trifluoroacetate (0.68 g, 1 eq.), DIEA (0.5 mL, 2 eq.), and *tert*-BuOH (2.5 mL) was reacted for 2 h at 180°C in a Smith microwave synthesizer: over 95 % conversion was observed by LC-MS. The reaction was diluted with DCM, washed with diluted-HCl and water, dried, and concentrated. 0.35 g (48 %) of N-[cis-4-(4-dimethylamino-5-methyl-pyrimidin-2-ylamino)-cyclohexyl]-3,5-bistrifluoromethyl-benzamide was isolated by column chromatography (silica gel; DCM:MeOH = 100:0 to 95:5).

To a solution of this neutral compound in DCM (10 mL) was added 4M-HCl in dioxane (0.4 mL, 2 eq.). After 30 min stirring at room temperature, removal of the volatile solvent provided *N*-(cis-4-{[4-(dimethylamino)-5-methylpyrimidin-2-yl]amino}cyclohexyl)-3,5-bis(trifluoromethyl)benzamide hydrochloride as a white powder. ESI MS m/e 490 $(M + H)^+$; $^1H$ NMR (400 MHz, DMSO-$d_6$) δ 12.1 (bs, 1 H), 8.78 (bd, 1 H, J = 5.6 Hz), 8.48 (s, 2 H), 8.28 (s, 1 H), 8.05 (bd, 1 H, J = 6.4 Hz), 7.62 (s, 1 H), 3.91 (bs, 2 H), 3.26 (s, 6 H), 2.23 (s, 3 H), 1.87 (m, 2 H), 1.73 (bs, 6 H).

**Example 2589**

**N-[(cis-4-1[4-(Dimethylamino)-5-methylpyrimidin-2-yl] amino) cyclohexyl) methyl]-3,5-bis(trifluoromethyl) benzamide hydrochloride**

**Step A: Synthesis of N-(cis-4-amino-cyclohexylmethyl)-3,5-bistrifluoromethyl-benzamide trifluoroacetate.**

To a solution of cis-(4-aminomethyl-cyclohexyl)-carbamic acid tert-butyl ester (1.1 g, 4.8 mmol) in dry benzene (15 mL) was added 3,5-bistrifluoromethyl benzoyl chloride (1.33 g, 1 eg.) and followed by $Et_3N$ (2 mL) at room temperature under $N_2$. The reaction was stirred for an additional 2 h at room temperature, washed with sat.-$NaHCO_3$ (3x) and water (1x), dried with $MgSO_4$, and concentrated. The crude {*cis*-4-[(3,5-bistrifluoromethyl-benzoylamino)-methyl]-cyclohexyl}-carbamic acid tert-butyl ester was used for the next reaction without further purification.

To a solution of {*cis*-4-[(3,5-bistrifluoromethyl-benzoylamino)-methyl]-cyclohexyl)-carbamic acid *tert*-butyl ester (2.1 g, 4.5 mmol) in DCM (10 mL) was added TFA (5 mL) at room temperature. After 1.5 h stirring at ambient temperature, removal of the volatile solvent gave the crude N-(*cis*-4-amino-cyclohexylmethyl)-3,5-bistrifluoromethyl-benzamide trifluoroacetate as a sticky oil. After addition of water (~40 mL) to the crude product, 5 ~ 10 min shaking provided formation of precipitates. The ppts were filtered, washed with water, and dried. 1.40 (61 %) of N-(cis-4-amino-cyclohexylmethyl)-3,5-bistrifluoromethyl-benzamide trifluoroacetate was isolated as a white powder.
ESI MS m/e 369 $(M + H)^+$; $^1H$ NMR (400 MHz, DMSO-$d_6$) δ 8.97 (bs, 1 H), 8.47 (s, 2 H), 8.29 (s, 1 H), 7.78 (bs, 3 H), 3.29 (t, 2 H, J = 6.8 Hz), 3.15 (bs, 1 H), 1.78 (bs, 1 H), 1.66 (m, 4 H), 1.52 (m, 4 H).

**Step B: Synthesis of *N* [(cis-4-{[4-(dimethylamino)-5-methylpyrimidin-2-yl]amino}cyclohexyl)methyl]-3,5-bis (trifluoromethyl)benzamide hydrochloride.**

A sealed tube containing 2-chloro-4-dimethylamino-5-methylpyrimidine (0.21 g, 1.2 mmol), N-(*cis*-4-amino-cyclohexylmethyl)-3,5-bistrifluoromethyl-benzamide trifluoroacetate (0.6 g, 1 eq.), DIEA (0.45 mL, 2 eq.), and *tert-BuOH* (2.5 mL) was reacted for 1.6 h at 185 °C in a Smith microwave synthesizer. The reaction was diluted with DCM, washed with diluted HCl and water, dried, and concentrated. The crude product was purified by column chromatography (silica gel; DCM:MeOH = 100:0 to 95:5). 0.3 g (50 %) of N-[*cis*-4-(4-dimethylamino-5-methyl-pyrimidin-2-ylamino)-cyclohexylmethyl]-3,5-bistrifluoromethyl-benzamide was isolated.

To a solution of neutral compound in DCM (10 mL) was added 4M-HCl in dioxane (0.3 mL, 2 eq.). After 30 min stirring at ambient temperature, removal of the volatile solvent provided N-[(cis-4-{[4-(dimethylamino)-5-methyl-pyrimidin-2-yl]amino}cyclohexyl)methyl]-3,5-bis(trifluoromethyl)benzamide hydrochloride as a white powder. ESI MS m/e 504 $(M + H)^+$; $^1H$ NMR (400 MHz, $CDCl_3$) δ 12.5 (bs, 1 H), 8.79 (d, 1 H, J = 8.0 Hz), 8.43 (s, 2 H), 7.93 (s, 1 H), 7.50 (bs, 1 H), 7.15 (d, 1 H, J = 4.4 Hz), 4.23 (bs, 1 H), 3.51 (bs, 2 H), 3.27 (s, 6 H), 2.23 (s, 3 H), 1.89~1.82 (m, 5 H), 1.66~1.60 (m, 4 H).

**Example 2590**

***N*-[(*cis*-4-{[4-(Dimethylamino)-5-methylpyrimidin-2-yl]amino )cyclohexyl)methyl]-4-(trifluoromethoxy) benzamide trifluoroacetate**

**Step A: Synthesis of N-[cis-4-(4-dimethylamino-5-methyl-pyrimidin-2-ylamino)-cyclohexylmethyl]-carbamic acid benzyl ester.**

Six sealed tubes, each containing 2-chloro-4-dimethylamino-5-methyl pyrimidine (0.4 g, 2.3 mmol), cis-(4-amino-cyclohexylmethyl)-carbamic acid benzyl ester (0.61 g, I eq.), DIEA (0.8 mL, 2 eq.), and t-BuOH (2.5 mL), were reacted in a Smith microwave synthesizer for 6500 sec at 185°C. Completion of the reaction was confirmed by LC-MS. The combined reaction was diluted with DCM, washed with IN-HCl (2 x) and water (1 x), and dried with anhydrous $MgSO_4$. The organic was concentrated and purified by column chromatography (silica gel; hexane: DCM: MeOH = 1: 5:0 to 0:95:5). Removal of the solvent gave 3.2 g (58 %) of *N-[cis-4-(4*-dimethylamino-5-methyl-pyrimidin-2-ylamino)-cyclohexylmethyl]-carbamic acid benzyl ester. ESI MS m/z 398 $(M + H)^+$; $^1H$ NMR (400 MHz, $CDCl_3$) δ 8.00 (bs, 1H), 7.36 (m, 6 H), 5.10 (s, 3 H, NH-was overlapped), 4.12 (bs, 1H), 3.24 (s, 6 H), 3.14 (t, 2 H, J = 6.4 Hz), 2.22 (s, 3 H), 1.88~1.50 (m, 9 H).

**Step B: Synthesis of *N*-[*cis*-4-(4-dimethylamino-5-methyl-pyrimidin-2-ylamino)-cyclohexylmethyl] amine.**

The heterogenous mixture of N-[cis-4-(4-dimethylamino-5-methyl-pyrimidin-2-ylamino)-cyclohexylmethyl]-carbamic acid benzyl ester (3.0 g, 7.5 mmol) and 10 % Pd/C (0.12 g) in EtOH (20 mL) was stirred overnight under $H_2$ atmosphere at room temperature. Cbz of all starting material was cleaved, which was confirmed by ESI MS. The reaction was filtered through a pad of celite, and the organic was concentrated and purified by column chromatography (silica gel, DCM:MeOH = 100:0 to 80:20). 1.5 g (75 %) of *N*-[*cis*-4-(4-dimethylamino-5-methyl-pyrimidin-2-ylamino)-cyclohexylmethyl] amine was isolated as a yellowish powder.
ESI MS m/z 264 $(M + H)^+$; $^1H$ NMR (400 MHz, DMSO-d6) (7.70 (bs, 2 H), 7.60 (s, 1H), 6.05 (d, 1H, J = 6.4 Hz), 3.89 (bs, 1H), 2.96 (s, 6 H), 2.71 (d, 2H, J = 6.8 Hz), 2.08 (s, 3 H), 1.70~1.45 (m, 9H).

**Step C: Synthesis of *N*-[(*cis*-4-{[4-(dimethylamino)-5-methylpyrimidin-2-yl]amino}cyclohexyl)methyl]-4-(trifluoromethoxy)benzamide trifluoroacetate.**

To a solution of N-[*cis*-4-(4-dimethylamino-5-methyl-pyrimidin-2-ylamino)-cyclohexylmethyl] amine (25 mg, 0.01 mmol) in DCM (1.0 mL) was added 4-trifluoromethoxybenzoyl chloride (21 mg, leq.), and followed by Et3N (30 (L). The reaction was stirred for 4h at room temperature, concentrated, and purified by prep-HPLC. 20 mg (38 %) of N-[(c*is-4-*{[4-(dimethylamino)-5-methylpyrimidin-2-yl]amino}cyclohexyl)methyl]-4-(trifluoromethoxy)benzamide trifluoroacetate was isolated as a white powder.
ESI MS m/z 452 (M + H)+; 1H NMR (400 MHz, CDCl3) ( 13.9 (bs, 1 H), 8.36 (bd, 1 H, J = 6.4 Hz), 7.88 (d, 2 H, J = 8.4 Hz), 7.27 (s, 1 H), 7.23 (d, 2 H, J = 8.4 Hz), 7.08 (bs, 1 H), 4.17 (bs, 1 H), 3.42 (t, 2 H, J= 5.6 Hz), 3.28 (s, 6 H), 2.23 (s, 3 H), 1.91~1.78 (m, 3 H), 1.65~1.55 (m, 6 H).

**Example 2591**

**3,5-Dichloro-*N*-[cis4-({[4-(dimethylamino)-5-methylpyrimidin-2-yl] amino)-methyl)cyclohexyl]benzamide trifluoroacetate**

**Step A: Synthesis of *N*-cis4-[(4-dimethylamino-5-methyl-pyrimidin-2-ylamino)-methyl]-cyclohexylamine.**

Six sealed tubes, each containing 2-chloro-4-dimethylamino-5-methyl pyrimidine (0.4 g, 2.3 mmol), *cis*-(4-aminomethyl-cyclohexyl)-carbamic acid tert-butyl ester (0.53 g, 1 eq.), DIEA (0.7 mL, 2 eq.), and t-BuOH (2 mL), were reacted in a Smith microwave synthesizer for 7000 sec at 185°C. ESI MS confirmed that all starting material was consumed. The reactions were combined, diluted with DCM and washed with 1N-HCl (2 x) and water (1 x). The organic was concentrated and carried to the next step without a further purification.

The crude *N*-{cis-4-[(4-dimethylamino-5-methyl-pyrimidin-2-ylamino)-methyl]-cyclohexyl}-carbamic acid tert-butyl ester was dissolved in DCM (15 mL), and TFA (10 mL) was added. After 1.5 h stirring, removal of the volatile solvent gave *N*-cis-4-[(4-dimethylamino-5-methyl-pyrimidin-2-ylamino)-methyl]-cyclohexylamine trifluoroacetate as a sticky oil. The sticky oil was treated with sat. NaOH (15 mL), and the basic aqueous layer was extracted with DCM (2 x) to remove nonpolar organic impurity, and the aqueous was concentrated to give a solid residue. The solid residue

was extracted several times with DCM/MeOH (3/1), and removal of the solvent provided neutral *N*-cis-4-[(4-dimethyl-amino-5-methyl-pyrimidin-2-ylamino)-methyl]-cyclohexylamine (1.5 g, 41 %) as a yellowish powder.
ESI MS 264 $(M + H)^+$;$^1$H NMR (400 MHz, $CDCl_3$) δ 7.60 (s, 1 H), 5.05 (bs, 1 H), 3.29 (t, 2 H, J = 6.4 Hz), 3.03 (s, 7 H, CH-NH2 was overlapped), 2.54 (bs, 2 H), 2.13 (s, 3 H), 1.72 (bm, 1 H), 1.59~1.45 (m, 8 H).

**Step B: Synthesis of 3,5-dichloro-*N*-[*cis*-4-{[4-(dimethylamino)-5-methylpyrimidin-2-yl]amino}methyl] cyclohexyl]benzamide trifluoroacetate.**

To a solution of *N*-*cis*-4-[(4-dimethylamino-5-methyl-pyrimidin-2-ylamino)-methyl]-cyclohexylamine (28 mg, 0.01 mmol) in benzene/DCM (2/1, 1.5 mL) was added 3,5-dichlorobenzoyl chloride (22 mg, leq.), and followed by $Et_3N$ (30 μL). The reaction was stirred for 3h at room temperature, concentrated, and purified by prep-HPLC. 30 mg (51 %) of 3,5-dichloro-*N*-[*cis*-4-({[4-(dimethylamino)-5-methylpyrimidin-2-yl]amino}methyl)cyclohexyl]benzamide trifluoroacetate was isolated as a white powder.
ESI MS m/e 436 $(M + H)^+$; $^1$H NMR (400 MHz, $CDCl_3$) δ 13.7 (bs, 1 H), 8.71 (bs, 1 H), 7.61 (d, 2 H, J = 1.6 Hz), 7.44 (t, 1 H, J = 1.6 Hz), 7.29 (s, 1 H), 6.59 (d, 1 H, J = 6.4 Hz), 4.23 (bm, 1 H), 3.36 (t, 2 H, J = 6.0 Hz), 3.29 (s, 6 H), 2.24 (s, 3 H), 1.81 (m, 3 H), 1.68 (m, 4 H), 1.45 (m, 2 H).

**Example 2592**

**N-[cis-4-({[4-(Dimethylamino)-6-methylpyrimidin-2-yl]amino)methyl)cyclohexyl]-3,5-bis(trifluoromethyl) benzamide trifluoroacetate**

**Step A: Synthesis of *N*-*cis*-4-[(4-dimethylamino-6-methyl-pyrimidin-2-ylamino)-methyl]-cyclohexylamine.**

Six sealed tubes, each containing 2-chloro-4-dimethylamino-6-methyl pyrimidine (0.4 g, 2.3 mmol), cis-(4-aminomethyl-cyclohexyl)-carbamic acid *tert-butyl* ester (0.53 g, 1 eq.), DIEA (0.7 mL, 2 eq.), and t-BuOH (2 mL), were reacted in a Smith microwave synthesizer for 6500 sec at 185 °C. The reaction was monitored by LC-MS. The combined reaction was diluted with DCM and washed with 1N-HCl (2 x) and water (1 x). The organic was concentrated and performed deprotection without a further purification.
To a solution of *N*-{cis-4-[(4-dimethylamino-6-methyl-pyrimidin-2-ylamino)-methyl]-cyclohexyl}-carbamic acid *tert*-butyl ester in DCM (15 mL) was added TFA (10 mL). The reaction was stirred for 1.5 h at room temperature, and removal of the volatile solvent gave *N*-*cis*-4-[(4-dimethylamino-6-methyl-pyrimidin-2-ylamino)-methyl]-cyclohexylamine trifluoroacetate as a sticky oil. The sticky oil was treated with sat. NaOH (15 mL), and the basic aqueous layer was extracted with DCM (2 x), and the aqueous was concentrated to give a solid residue. The solid residue was extracted several times with DCM/MeOH (3/1), and removal of the solvent provided neutral *N*-*cis*-4-[(4-dimethylamino-6-methyl-pyrimidin-2-ylamino)-methyl]-cyclohexylamine (2.1 g, 57 %) as a yellowish powder.
ESI MS m/e 264 $(M + H)^+$; $^1$H NMR (400 MHz, $CDCl_3$) δ 5.91 (bs, 1 H), 5.65 (s, 1 H), 3.33 (t, 2 H, J = 6.4 Hz), 3.06 (s, 6 H), 2.97 (m, 1 H), 2.27 (bs, 2 H), 2.11 (s, 3 H), 1.70 (m, 1 H), 1.59~1.45 (m, 8 H).

**Step B: Synthesis of *N*-[*cis*-4-({[4-(dimethylamino)-6-methylpyrimidin-2-yl]amino}-methyl)cyclohexyl]-3,5-bis (trifluoromethyl)benzamide trifluoroacetate.**

To a solution of N-cis-4-[(4-dimethylamino-6-methyl-pyrimidin-2-ylamino)-methyl]-cyclohexylamine (20 mg, 0.008 mmol) in benzene/DCM (2/l, 1.5 mL) was added 3,5-bistrifluoromethylbenzoyl chloride (21 mg, leq.), and followed by $Et_3N$ (30 μL). The reaction was stirred for 3h at room temperature, concentrated, and purified by prep-HPLC. 25 mg (53 %) of *N*-[cis-4-({[4-(dimethylamino)-6-methylpyrimidin-2-yl]amino} methyl)cyclohexyl]-3,5-bis(trifluoromethyl) benzamide trifluoroacetate was isolated as a white powder.
ESI MS m/e 504 $(M + H)^+$; $^1$H NMR (400 MHz, $CDCl_3$) δ 13.9 (bs, 1 H), 8.86 (bs, 1 H), 8.25 (s, 2 H), 7.96 (s, 1 H), 7.30 (d, 1 H, J = 6.4 Hz), 5.74 (s, 1 H), 4.40 (bm, 1 H), 3.42 (t, 2 H, J = 6.0 Hz), 3.26 (s, 3 H), 3.13 (s, 3 H), 2.33 (s, 3 H), 1.91～1.60 (m, 9 H).

**Example 2593**

**4-Chloro-*N*-[*cis*-4-{[4-(dimethylamino)-6-methylpyrimidin-2-yl]amino}cyclohexyl)-methyl]benzamide trifluoroacetate**

**Step A: Synthesis of N-[*cis*-4-(4-dimethylamino-6-methyl-pyrimidin-2-ylamino)-cyclohexylmethyl]-carbamic acid benzyl ester.**

Six sealed tubes, each containing 2-chloro-4-dimethylamino-6-methyl pyrimidine (0.4 g, 2.3 mmol), *cis*-(4-amino-cyclohexylmethyl)-carbamic acid benzyl ester (0.61 g, l eq.), DIEA (0.8 mL, 2 eq.), and t-BuOH (2.5 mL), were reacted in a Smith microwave synthesizer for 6500 sec at 180 °C. The combined reaction was diluted with DCM, washed with 1N-HCl (2 x) and water (1 x), dried with MgSO4, and concentrated. Purification by column chromatography (silica gel; hexane: DCM: MeOH = 1:5:0 to 0:95:5) gave 4.8 g (86 %) of N-[cis-4-(4-dimethylamino-6-methyl-pyrimidin-2-ylamino)-cyclohexylmethyl]-carbamic acid benzyl ester.
ESI MS m/z 398 $(M + H)^+$; $^1H$ NMR (400 MHz, $CDCl_3$) δ 8.40 (bs, 1H), 7.38 (m, 5 H), 5.70 (s, 1 H), 5.10 (s, 3 H, NH-was overlapped), 4.17 (bs, 1 H), 3.14 (bs, 6 H), 3.12 (t, 2 H, J = 6.0 Hz), 2.32 (s, 3 H), 1.90~1.50 (m, 9 H).

**Step B: Synthesis of *N*-[*cis*-4-(4-dimethylamino-6-methyl-pyrimidin-2-ylamino)-cyclohexylmethyl] amine.**

The heterogenous solution of N-[cis-4-(4-dimethylamino-6-methyl-pyrimidin-2-ylamino)-cyclohexylmethyl]-carbamic acid benzyl ester (4.5 g, 11.3 mmol) and 10 % Pd/C (0.20 g) in EtOH (25 mL) was stirred overnight under $H_2$ atmosphere at room temperature. The reaction was filtered through a pad of celite, and the organic was concentrated and purified by column chromatography (silica gel, DCM:MeOH = 100:0 to 80:20). 2.2 g (76 %) of N-[cis-4-(4-dimethylamino-6-methyl-pyrimidin-2-ylamino)-cyclohexylmethyl] amine was isolated as a yellowish powder.
ESI MS m/e 264 $(M + H)^+$; $^1H$ NMR (400 MHz, $CDCl_3$) δ 7.82 (bs, 1 H), 5.72 (s, 1 H), 4.40 (bs, 2 H), 4.15 (bm, 1 H), 3.16 (s, 6 H), 2.84 (d, 2 H, J = 6.8 Hz), 2.32 (s, 3 H), 1.80 (m, 2 H), 1.70~1.45 (m, 7 H).

**Step C: Synthesis of 4-chloro-*N*-[(*cis*-4-{[4-(dimethylamino)-6-methylpyrimidin-2-yl]amino}cyclohexyl) methyl] benzamide trifluoroacetate.**

To a solution of N-[cis-4-(4-dimethylamino-6-methyl-pyrimidin-2-ylamino)-cyclohexylmethyl] amine (25 mg, 0.01 mmol) in DCM/benzene (3/1, 1.0 mL) was added 4-chlorobenzoyl chloride (17 mg, leq.), and followed by $Et_3N$ (30 μL). The reaction was stirred for 4h at room temperature, concentrated, and purified by prep-HPLC. 25 mg (51 %) of 4-chloro-N-[(*cis*-4-{[4-(dimethylamino)-6-methylpyrimidin-2-yl]amino}cyclohexyl)methyl]benzamide trifluoroacetate was isolated as a white powder.
ESI MS m/e 402 $(M + H)^+$; $^1H$ NMR (400 MHz, $CDCl_3$) δ 13.8 (bs, 1 H), 8.60 (bd, 1 H, J = 6.4 Hz), 7.78 (d, 2 H, J = 8.4 Hz), 7.35 (d, 2 H, J = 8.4 Hz), 7.03 (bm, 1 H), 5.71 (s, 1 H), 4.20 (bs, I H), 3.42 (t, 2 H, J = 6.0 Hz), 3.22 (s, 3 H), 3.10 (s, 3 H), 2.31 (s, 3 H), 1.91~1.78 (m, 3 H), 1.65~1.55 (m, 6 H).

**Example 2594**

***cis*-4-{[4-(Dimethylamino)-5-methylpyrimidin-2-yl]amino)-*N*-[(1S)-1-(4-methyl-phenyl)ethyl] cyclohexanecarboxamide hydrochloride**

**Step A: Synthesis of cis-4-(4-dimethylamino-5-methyl-pyrimidin-2-ylamino)-cyclohexanecarboxylic acid ethyl ester.**

A sealed tube containing a suspension of 2-chloro-4-dimethylamino-5-methylpyrimidine (0.28 g, 1.6 mmol), cis-4-amino-cyclohexanecarboxylic acid ethyl ester hydrochloride (0.33 g, I eq.), DIEA (0.65 mL, 2 eq.) in IPA (2 mL) was reacted in a Smith microwave synthesizer for 1 hour at 155° C. The solution was diluted with DCM, washed with 1N-HCl and water, concentrated, and purified by flash chromatography (silica gel, 1 % MeOH in $CH_2Cl_2$) to give *cis-4-(4*-dimethylamino-5-methyl-pyrimidin-2-ylamino) cyclohexanecarboxylic acid ethyl ester (0.3 g, 60 %) as a pale yellow solid.
ESI MS m/e 307 $(M + H)^+$; $^1H$ NMR (400 MHz, $CDCl_3$) δ 7.66 (s, 1 H), 4.72 (bd, 1 H, J = 6.8 Hz), 4.13 (q, 2 H, J = 6.8 Hz), 3.96 (bs, 1 H), 3.01 (s, 6 H), 2.44 (m, 1 H), 2.13 (s, 3 H), 1.89 (m, 2H), 1.72 (m, 6 H), 1.25 (t, 3 H, J = 6.8 Hz).

**Step B: Synthesis of *cis*-4-(4-dimethylamino-5-methyl-pyrimidin-2-ylamino)-cyclohexanecarboxylic acid.**

A suspension of cM-4-(4-dimethylamino-5-methy)-pyrimidin-2-y)amino) cyclohexanecarboxylic acid ethyl ester (0.25 g, 0.8 mmol) in 4N-HCl (10 mL) was stirred for 4 h at 85 °C. Progress of the reaction was monitored by LC-MS. The reaction was cooled to room temperature and completely removed the volatile solvent under a vacuum to give *cis-4-(4*-dimethylamino-5-methyl-pyrimidin-2-ylamino) cyclohexanecarboxylic acid (0.2 g, 90 %) as a white powder. ESI MS m/e 279 $(M + H)^+$; $^1H$ NMR (400 MHz, $CDCl_3$) δ 7.95 (bs, 1 H), 7.43 (s, 1 H), 3.94 (bs, 1 H), 3.28 (bs, 6 H), 2.49 (bs, 1 H), 2.25 (s, 3 H), 2.04 (m, 2 H), 1.82 (m, 2 H), 1.73 (m, 4 H), COOH was not observed.

**Step C: Synthesis of *cis*-4-{[4-(dimethylamino)-5-methylpyrimidin-2-yl]amino}-*N*-[(1S)-1-(4-methylphenyl) ethyl]cyclohexanecarboxamide hydrochloride.**

To a suspension of (S)-1-(4-methylphenyl)-ethylamine (12 mg, 1 eq.) and cis-4-(4-dimethylamino-5-methyl-pyrimidin-2-ylamino) cyclohexanecarboxylic acid (24 mg, 0.09 mmol) in DCM (2 mL) was added HATU (36 mg, 1.1 eq.). The reaction stirred for 30 seconds at room temperature under argon, and triethylamine (5 drops) was added. The reaction stirred overnight at room temperature. The reaction was diluted with DCM, washed with saturated $NaHCO_3$ (2x) and $H_2O$ (1x), and concentrated. Purification by column chromatography (silica gel; DCM:MeOH = 100:0 to 94:6) gave cis-4-(4-Dimethylamino-5-methyl-pyrimidin-2-ylamino)-cyclohexanecarboxylic acid (*S*)-(1-p-tolyl-ethyl)-amide (15 mg, 43 %). To a solution of the amide in DCM (1 mL) was added 4M-HCl in dioxane (50 µL,). The reaction was stirred for 30 min at room temperature, and removal of the volatile solvent gave *cis*-4-{[4-(dimethylamino)-5-methylpyrimidin-2-yl]amino)-N-[(1*S*-1-(4-methylphenyl)ethyl]cyclohexanecarboxamide hydrochloride as a white powder. ESI MS m/e 396 $(M + H)^+$; $^1H$ NMR (400 MHz, DMSO-$d_6$) δ 11.0 (bs, 1 H), 8.14 (d, 1 H, J = 8.4 Hz), 7.68 (bs, 1 H), 7.54 (s, 1 H), 7.14 (d, 2 H, J = 8.0 Hz), 7.07 (d, 2 H, J = 8.0 Hz), 4.84 (m, 1 H), 4.01 (bs, 1 H), 3.24 (s, 6 H), 2.27 (m, 1 H), 2.25 (s, 3 H), 2.22 (s, 3 H), 1.80~1.54 (m, 8 H), 1.29 (d, 3 H, J = 6.8 Hz).

**Example 2595**

**2,2-Difluoro-*N*-(cis-4-{[5-methyl-4-(methylamino)pyrimidin-2-yl]amino}cyclohexyl)-1,3-benzodioxole-5-carboxamide trifluoroacetate**

**Step A: Synthesis of 2,2-difluoro-N-(cis-4-{[5-methyl-4-(methylamino)pyrimidin-2-yl]amino}cyclohexyl)-1,3-benzodioxole-5-carboxamide trifluoroacetate.**

2-(3,5-Dimethoxy-4-formyl)phenoxy ethyl polystyrene resin (1.0 gram; 0.94mmol/gram) and methylamine (0.0122 mol) in 15 mL of $CH_2Cl_2$ was suspended in a fritted synthesis flask. To this suspension was added a solution of$NaBH(OAc)_3$ (0.0122 mol) in $CH_2Cl_2$ (15 mL). After shaking the mixture overnight in a rotary shaker, the solution was removed by filtration. The resulting resin bound N-methylamine was washed sequentially with $CH_2Cl_2$, DMF, and MeOH. The washing sequence was repeated four times. The resin bound N-methylamine was dried under vacuum for 20 minutes.

The resin bound Nmethylamine was suspended in DMF (10 mL). To the resin suspension was added 2,4-dichloro-5-methyl-pyrimidine (1.41 mmol) followed by triethylamine (0.393 mL, 2.82 mmol). The reaction mixture was shaken at 40 °C overnight. The solution was removed by filtration and the resin washed sequentially with DMF, $CH_2Cl_2$ and MeOH. The wash sequence was repeated four times. The resulting resin bound intermediate was dried under vacuum for 20 minutes

The resin bound intermediate was divided up into three portions and each portion was transferred into a 5ml Smith synthesizer reaction vessel. The resins (0.282 mmol) were separately suspended in a 1:1 solution of IPA/$H_2O$ (3 mL). To each suspension was added cis-1,4-diamino-cyclohexane (0.85 mmol) and DIEA (0.295ml; 1.69 mmol). The reactions were heated in a microwave synthesizer at 180°C for 4.5 hours. The resins were pooled together; and the solution removed by filtration. The resin was sequentially washed with IPA, $H_2O$, MeOH, $CH_2Cl_2$, and MeOH. The washing sequence was repeated three times. The resulting resin bound intermediate was dried under vacuum for 20 minutes.

The resin bound intermediate was suspended in DMF (10ml). To the resin suspension was added the 2,2-diflouro-benzo[1,3]dioxole 5-carbonyl chloride (0.94 mmol) and triethylamine (0.256 mL; 1.88 mol). The reaction was shaken in a rotary mixer at room temperature for 45 minutes. The solution was removed by filtration and the resin washed sequentially with DMF, $CH_2Cl_2$, MeOH. The wash sequence repeated three times.

After drying under vacuum for 20 minutes, the resin was treated with 15 mL of TFA solution (TFA /$CH_2Cl_2$ /$H_2O$ 20:20:1 v/v). The reaction was shaken for 2 hours and the TFA solution was collected after filtration. The TFA was removed by rotary evaporation and the compound subjected to purification by preparative HPLC to give 2,2-difluoro-N-(cis-4-{[5-methyl-4-(methylamino}pyrimidin-2-yl]amino}cyclohexyl)-1,3-benzodioxole-5-carboxamide trifluoroacetate (8.6 mg, 5%) as a white solid.
ESI MS m/e 420.5 M+H$^+$; $^1$H NMR (400MHz, $CD_3OD$) δ 8.21 (d, J= 4 Hz, 1H), 7.75-7.67 (m, 2H), 7.41 (s, 1H), 7.28 (d, J = 8 Hz, 1H), 3.99 (m, 2H), 3.05 (s, 3H), 1.99 (s, 3H), 1.95-1.71 (m, 8H).

**Example 2596**

**5-Bromo-*N*-(cis-4-{[5-methyl-4-(methylamino)pyrimidin-2-yl]amino)cyclohexyl)-2-furamide trifluoroacetate**

**Step A: Synthesis of 5-bromo-*N*-(*cis*-4-{[5-methyl-4-(methylamino)pyrimidin-2-yl]amino)cyclohexyl)-2-furamide trifluoroacetate.**

Using the procedure of Example 2595, the title compound was obtained.
ESI MS m/e 408.2 M+H$^+$; $^1$H NMR (400MHz, $CD_3OD$) δ 7.41 (s, 1H), 7.10 (s, 1H), 6.60 (s, 1H), 4.08-3.97 (m, 2H), 3.05 (s, 3H), 1.99 (s, 3H), 1.95-1.71 (m, 8H).

**Example 2597**

**3,5-Dibromo-*N*-(cis-4-{[5-methyl-4-(methylamino)pyrimidin-2-yl]amino}cyclohexyl)-benzamide trifluoroacetate**

**Step A: Synthesis of 3,5-dibromo-*N*-(*cis*-4-{[5-methyl-4-(methylamino)pyrimidin-2-yl]amino}cyclohexyl) benzamide trifluoroacetate.**

Using the procedure of Example 2595, the title compound was obtained.
ESI MS m/e 496.2 M+H$^+$; $^1$H NMR (400MHz, $CD_3OD$) δ 8.37 (m, J = 4 Hz, 1H), 8.02-7.91 (m, 3H) 7.41 (s, 1H), 4.12 -3.97 (m, 2H), 3.05 (s, 3H), 1.99 (s, 3H), 1.95-1.71 (m, 8H).

**Example 2598**

**3-Fluoro-N-(cis-4-{[5-methyl-4-(methylamino)pyrimidin-2-yl]amino}cyclohexyl)-5-(trifluoromethyl)benzamide trifluoroacetate**

**Step A: Synthesis of 3-fluoro-N-(cis-4-{[5-methyl-4-(methylamino)pyrimidin-2-yl]amino}cyclohexyl)-5-(trifluoromethyl)benzamide trifluoroacetate.**

Using the procedure of Example 2595, the title compound was obtained. ESI MS m/e 426.4 M+H$^+$; $^1$H NMR (400MHz, $CD_3OD$) δ 8.02 (m, 1H), 7.98 (d, *J* = 4 Hz , 1 H) 7.68 (d, *J*= 4 Hz 1H), 7.43-7.41 (s, 1H) 4.31 -3.81 (m, 2H), 3.05 (s, 3H), 1.87 (s, 3H), 1.87-1.73 (m, 8H).

**Example 2599**

***N-(cis*-4-{[5-Methyl-4-(methylamino)pyrimidin-2-yl]amino}cyclohexyl)-4-(trifluoromethoxy)benzamide trifluoroacetate**

**Step A: Synthesis of *N*-(*cis*-4-{[5-niethyl-4-(methylamino)pyrimidin-2-yl]amino}cyclohexyl)-4-(trifluoromethoxy)benzamide trifluoroacetate.**

Using the procedure of Example 2595, the title compound was obtained.
ESI MS m/e 424.3 M+H$^+$; $^1$H NMR (400MHz, $CD_3OD$) δ 8.34 (d, J = 4 Hz, 1H), 7.85 (d, J = 8 Hz, 1H) 7.72 -7.55 (s, 1H), 7.47-7.31 (m, 3H), 4.31 -3.82 (m, 2H), 3.05 (s, 3H), 1.98 (s, 3H), 1.96-1.72 (m, 8H).

**Example 2600**

***N-(cis-4-i* [5-methyl-4-(methylamino)pyrimidin-2-yl]amino) cyclohexyl)-3,5-bis(trifluoromethyl) benzamide hydrochloride**

**Step A: Synthesis of *N*-(cis-4-{[5-methyl-4-(methylamino)pyrimidin-2-yl]amino)cyclohexyl)-3,5-bis (trifluoromethyl)benzamide hydrochloride.**

To a solution of (2-chloro-5-methyl-pyrimidin-4yl)-methyl-amine (200mg, 1.27 mmol) in 1mL 2-propanol was added *cis-N*-(4-amino-cyclohexyl)-3,5-bis-trifluoromethyl-benzamide (676mg, 1.91mmol) and DIEA (2.54mmol). The mixture was heated in a microwave synthesizer at 180 °C for 2 hours. The solvent was evaporated and the material subjected to chromatography (70 ~ 95% ethyl acetate/ hexanes) The combined compound in $CH_2Cl_2$ was added 2 M HCl in diethyl ether (1.5ml, 0.38mmol). The solvents were removed in vacuo to yield *N*-(cis-4-{[5-methyl-4-(methylamino)pyrimidin-2-yl]amino) } cyc)ohexy))-3,5-bis(trifluoromethyl)benzamide hydrochloride (385.5mg, 0.75mmol, 59 %) as a white solid.
ESI MS 476.2 M+H$^+$ ; $^1$H NMR (400 MHz, DMSO-d6) δ 11.7 (s, 1H), 8.64 (s, 1H), 8.35 (s, 2H), 8.14 (s, 1H), 8.09 (bs, 1H), 8.00 (bs, 1H), 7.45 (s, 1H), 3.83 (bs, 1H), 3.75 (bs, 1H), 3.20 (s, 3H), 2.77-2.76 (d, J = 4 Hz, 3H), 1.76 (m, 2H), 1.58 (m, 6H).

**Example 2601**

***N*-(*cis*-4-{[4-(Ethylamino)-5-methylpyrimidin-2-yl]amino}cyclohexyl)-3,4-difluorobenzamide trifluoroacetate**

**Step A: Synthesis of *N*-(*cis*-4-{[4-(ethylamino)-5-methylpyrimidin-2-yl]amino)cyclohexyl)-3,4-difluorobenzamide trifluoroacetate.**

Using the procedure of Example 2595, the title compound was obtained. ESI MS m/e 390.4 M+H$^+$; $^1$H NMR (400MHz, $CD_3OD$) δ 8.22 (d, J = 4 Hz, 1 H), 7.78 (m, 1 H), 7.68 (m, 1H), 7.42 (s, 1H), 7.38 (m, 1 H), 4.22-3.99 (m, 2H), 3.63-3.56 (quartet, J = 4 Hz, 2H), 1.99 (s, 3H), 1.93-1.81 (m, 8H), 1.29-1.19 (t, J= 8 Hz, 3H).

**Example 2602**

**3,4-Difluoro-N-(*cis*-4-{[4-(isopropylamino)-5-methylpyrimidin-2yl]amino}cyclohexyl)-benzamide trifluoroacetate**

**Step A: Synthesis of 3,4-difluoro-*N*-(*cis*-4-{[4-(isopropylamino)-5-methylpyrimidin-2-yl]amino}cyclohexyl)-benzamide trifluoroacetate.**

Using the procedure of Example 2595, the title compound was obtained.
ESI MS m/e 404.4 M+H$^+$; $^1$H NMR (400MHz, $CD_3OD$) δ 8.10 (m, 1H), 7.80-7.75(m, 1H), 7.68 (m, 1H), 7.42 (s, 1H), 7.39-7.34 (m, 1H), 4.28-4.07 (m, 3H), 2.03 (s, 3H), 1.99-1.79 (m, 8H), 1.31-1.26 (d, J= 12 Hz, 6H).

**Example 2603**

***N-(cis-4-*{[4-(cyclopropylamino)-5-methylpyrimidin-2-yl]amino}cyclohexyl)-3,4-difluorobenzamide trifluoroacetate**

**Step A: Synthesis of *N*-(cis-4-{[4-(cyclopropylamino)-5-methylpyrimidin-2-yl]amino}-cyclohexyl)-3,4-difluorobenzamide trifluoroacetate.**

Using the procedure of Example 2595, the title compound was obtained.
ESI MS m/e 402.2 M+H$^+$; $^1$H NMR (400MHz, $CD_3OD$) δ 7.78-7.73 (m, 1H), 7.69-7.66 (m, 1H), 7.42 (s, 1H), 7.40-7.33 (m, 1H), 4.26-3.88 (m, 2H), 3.02-2.96 (m, 1H), 1.97-1.81 (m, 11H), 0.90-0.85 (m, 2H), 0.72-0.68 (m, 2H).

**Example 2604**

**3,4-Difluoro-N-(*cis*-4-{[5-methyl-4-(methylamino)pyrimidin-2-yl]amino}cyclohexyl)-benzamide trifluoroacetate**

**Step A: Synthesis of 3,4-difluoro-N-(*cis*-4-{[5-methyl-4-(methylamino)pyrimidin-2-yl]amino}cyclohexyl) benzamide trifluoroacetate.**

Using the procedure of Example 2595, the title compound was obtained.
ESI MS m/e 376.2 M+H$^+$; $^1$H NMR (400MHz, $CD_3OD$) δ 7.80-7.75 (m, 1 H), 7.68 (m, 1 H), 7.43-7.35 (m, 2H), 4.31-4.06 (m, 2H), 3.05 (s, 3H), 2.04 (s, 3H), 1.99-1.75 (m, 8H).

**Example 2605**

**2-(3,4-Dichlorophenoxy)-N-(*cis*-4-{[5-methyl-4-(methylamino)pyrimidin-2-yl]amino}cyclohexyl)acetamide trifluoroacetate**

**Step A: Synthesis of 2-(3,4-dichlorophenoxy)-N-(cis-4-{[5-methyl-4-(methylamino)-pyrimidin-2-yl]amino} cyclohexyl)acetamide trifluoroacetate.**

Using the procedure of Example 2595, the title compound was obtained.
ESI MS m/e 438.3 M+H$^+$; $^1$H NMR (400MHz, $CD_3OD$) δ 7.45-7.40 (m, 2H), 7.20 (s, 1H), 6.97-6.94 (m, 1H), 4.55 (s, 2H), 3.92-3.34 (s, 2H) 3.04 (s, 3H), 1.98 (s, 3H), 1.53-1.71 (m, 8H).

**Example 2606**

**2-(2,3-Dichlorophenoxy)-N-(cis-4-{[5-methyl-4-(methylamino)pyrimidin-2-yl]amino}cyclohexyl)acetamide trifluoroacetate**

**Step A: Synthesis of 2-(2,3-dichlorophenoxy)-N-(*cis*-4-{[5-methyl-4-(methylamino)-pyrimidin-2-yl]amino} cyclohexyl)acetamide trifluoroacetate.**

Using the procedure of Example 2595, the title compound was obtained.
ESI MS m/e 438.3 M+H$^+$; $^1$H NMR (400MHz, $CD_3OD$) δ 7.42 (s, 1H), 7.31-6.92 (m, 3H), 4.65 (s, 2H), 4.07-3.95 (m, 2H), 3.05 (s, 3H), 1.98 (s, 3H), 1.93-1.69 (m, 8H).

**Example 2607**

**N-(*cis*-4-{[4-(Dimethylamino)-5,6-dimethylpyrimidin-2-yl]amino}cyclohexyl)benzamide trifluoroacetate**

**Step A: Synthesis of 2,4-dichloro-5,6-dimethyl-pyrimidine.**

To a suspension of 2,4-dihydroxy-5,6-dimethylpyrimidine (6.2 g, 0.044 mol) in $POCl_3$ (25 mL) was slowly added *N,N*-dimethylaniline (6.18 mL, 0.049 mol). The mixture was then refluxed at 125 °C for 3 hours. After this time, the starting material completely dissolved indicating that the reaction was completed. The reaction mixture was cooled and then poured slowly onto ice to quench the $POCl_3$ (caution exothermic!). A precipitate formed, which was filtered and washed with ice-cold water. The precipitate was dried under high vacuum overnight to yield 2,4-dichloro-5,6-dimethyl-pyrimidine (7.2 g, 0.041 mol, 92 %) as a yellow solid.
$^1$H NMR (400 MHz, $CDCl_3$) δ 2.56 (s, 3H), 2.36 (s, 3H).

**Step B: Synthesis of (2-chloro-5,6-dimethyl-pyrimidin-4-yl)-dimethyl-amine.**

To a solution of 2,4-dichloro-5,6-dimethyl-pyrimidine (0.2 g, 0.0011 mol) in 1 mL 2-propanol was added DIEA (268 uL, 0.0017 mol) and dimethylamine (514 uL, 0.0010 mol). The mixture was then heated in a microwave at 170 °C for 10 minutes. The reaction mixture was cooled and concentrated and the resulting oil was purified by column (0-50% ethyl acetate in hexanes) to yield (2-chloro-5,6-dimethyl-pyrimidin-4-yl)-dimethyl-amine (75 mg, 0.40 mmol, 40%) as a white solid. ESI MS 186.0 M+H$^+$; $^1$H NMR (400 MHz, $CDCl_3$) δ 3.03 (s, 6H), 2.37 (s, 3H), 2.15 (s, 3H).

**Step C: Synthesis of cis-[4-(4-dimethylamino-5,6-dimethyl-pyrimidin-2-ylamino)-cyclohexyll-carbamic acid *tert-butyl* ester.**

To a solution of (2-chloro-5,6-dimethyl-pyrimidin-4-yl)-dimethyl-amine (0.5 g, 0.0027 mol) in 2 mL 2-propanol was added DIEA (514 uL, 0.0040 mol) and cis-(4-amino-cyclohexyl)-carbamic acid *tert*-butyl ester (635 mg, 0.0030 mol). The mixture was then heated in a microwave at 170 °C for 1 hour. The reaction mixture was cooled and concentrated and the resulting oil was purified by column (0-100% ethyl acetate in hexanes) to yield cis-[4-(4-dimethylamino-5,6-dimethyl-pyrimidin-2-ylamino)-cyclohexyl]-carbamic acid tert-butyl ester (875 mg, 2.4 mmol, 89 %) as a white solid. ESI MS 364.6 $M+H^+$ ; $^1H$ NMR (400 MHz, $CD_3OD$) δ 3.97 (m, 1H), 3.53 (m, 1H), 2.95 (s, 6H), 2.23 (s, 3H), 2.09 (s, 3H), 1.78-1.55 (m, 8H), 1.48 (s, 9H).

**Step D: Synthesis of *cis*-4-(4-dimethylamino-5,6-dimethyl-pyrimidin-2-ylamino)-1-aminocyclohexane.**

To a solution of cis-[4-(4-dimethylamino-5,6-dimethyl-pyrimidin-2-ylamino)-cyclohexyl]-carbamic acid tert-butyl ester (3.4 g, 0.0094 mol) in 40 mL $CH_2Cl_2$ was added TFA (1.4 mL, 0.019 mol). The solution was stirred at room temperature for 4 hours (or until the reaction was complete as judged by TLC). The excess solvent was evaporated off and the resulting oil was dissolved in 30 mL $CH_2Cl_2$. The organic layer was extracted with 30 mL of a dilute NaOH (aq) / $NaHCO_3$ (aq) solution (the aqueous layer was confirmed to remain basic during the extraction using pH paper indicator). The aqueous layer was back extracted twice with $CH_2Cl_2$ and the organic layers combined, dried over $MgSO_4$, and concentrated to yield cis-4-(4-dimethylamino-5,6-dimethyl-pyrimidin-2-ylamino)-1-aminocyclohexane (2.2 g, 0.0084 mol, 90%) as a white solid.
ESI MS 264.2 $M+H^+$; $^1H$ NMR (400 MHz, $CD_3OD$) δ 3.99 (m, 1H), 2.95 (s, 6H), 2.80 (m, 1H), 2.23 (s, 3H), 2.09 (s, 3H), 1.84-1.67 (m, 6H), 1.52-1.49 (m, 2H).

**Step E: Synthesis of N-(cis-4-{[4-(dimethylamino)-5,6-dimethylpyrimidin-2yl]amino}-cyclohexyl)benzamide trifluoroacetate.**

To a solution of cis-4-(4-dimethylamino-5,6-dimethyl-pyrimidin-2-ylamino)-1-aminocyclohexane (30 mg, 0.11 mmol) in 0.5 mL DMF was added pyridine (13.8 uL, 0.17 mmol) and benzoyl chloride (12.6 uL, 0. 11 mmol). The reaction mixture was stirred overnight and then 0.5 mL of DMSO was added to the mixture. The compound was then subject to purification by prep LCMS to yield *N-(cis*-4-{[4-(dimethylamino)-5,6-dimethy pyrimidin-2-yl]amino}cyclohexyl)benzamide trifluoroacetate (27 mg, 0.056 mmol, 52%) as a white solid TFA salt.
ESI MS m/e 368.2 $M+H^+$ ; $^1H$ NMR (400 MHz, CD3OD) δ 7.85-7.83 (m, 2H), 7.58-7.54 (m, 1 H), 7.51-7.47 (m, 2H), 4.15 (m, 1H), 4.03 (m, 1H), 3.28 (s, 6H), 2.34 (s, 3H), 2.19 (s, 3H), 2.00-1.80 (m, 8H).

**Example 2608**

***N*-(*cis*-4-{[4-(Dimethylamino)-5,6-dimethylpyrimidin-2-yl]amino}cyclohexyl)-3-(trifluoromethyl)benzamide trifluoroacetate**

**Step A: Synthesis of *N*-(*cis*-4-{[4-(dimethylamino)-5,6-dimethylpyrimidin-2yl]amino}-cyclohexyl)-3-(trifluoromethyl)benzamide trifluoroacetate.**

Using the procedure of Example 2607, the title compound was obtained as a white solid TFA salt.
ESI MS m/e 436.4 $M+H^+$ ; $^1H$ NMR (400 MHz, $CD_3OD$) δ 8.16 (s, 1H), 8.12 (d, 1H, J = 7.6 Hz), 7.89 (d, 1H, J = 8.0 Hz), 7.71 (t, 1H, J = 8.0 Hz), 4.16 (m, 1H), 4.05 (m, 1 H), 3.28 (s, 6H), 2.34 (s, 3H), 2.20 (s, 3H), 2.00-1.82 (m, 8H).

**Example 2609**

***N*-(*cis*-4-{[4-(Dimethylamino)-5,6-dimethylpyrimidin-2-yl]amino}cyclohexyl)-2-hydroxynicotinamide trifluoroacetate**

**Step A: Synthesis of *N*-(*cis*-4-{[4-(dimethylamino)-5,6-dimethylpyrimidin-2yl]amino}-cyclohexyl)-2-hydroxynicotinamide trifluoroacetate.**

To a solution of *cis*-4-(4-dimethylamino-5,6-dimethyl-pyrimidin-2-ylamino)-1-aminocyclohexane (30 mg, 0.11 mmol) in 0.5 mL DMF was added 2-hydroxynicotinic acid (15 mg, 0.11 mmol), DIEA (29.8 uL, 0.17 mmol), and HATU (52 mg, 0.14 mmol). The reaction mixture was stirred overnight and then 0.5 mL DMSO was added to the mixture. The

compound was then subject to purification by prep LCMS to yield *N*-(*cis*-4-([4-(dimethylamino)-5,6-dimethylpyrimidin-2-yl]amino}cyclohexyl)-2-hydroxy nicotinamide trifluoroacetate (17 mg, 0.034 mmol, 31 %) as a white solid.
ESI MS m/e 385.2 M+H$^+$; $^1$H NMR (400 MHz, $CD_3OD$) δ 8.53 (dd, 1H, $J_1$ = 7.2 Hz, $J_2$ = 2.0 Hz), 7.70 (dd, 1H, $J_1$ = 6.4 Hz, $J_2$ = 2.0 Hz), 6.61 (t, 1H, J = 6.8 Hz), 4.17 (m, 1H), 4.01 (m, 1H), 3.28 (s, 6H), 2.33 (s, 3H), 2.19 (s, 3H), 1.98-1.72 (m, 8H).

**Example 2610**

**5-Bromo-*N*-(*cis*-4-{[4-(dimethylamino)-5,6-dimethylpyrimidin-2-yl]amino}cyclohexyl)-2-furamide trifluoroacetate**

**Step A: Synthesis of 5-Bromo-*N*-(*cis*-4-{[4-(dimethylamino)-5,6-dimethylpyrimidin-2-yl]amino}cyclohexyl)-2-furamide trifluoroacetate.**

Using a similar procedure of Example 2609, the title compound was obtained as a white solid TFA salt.
ESI MS m/e 436.2 M+H$^+$; $^1$H NMR (400 MHz, $CD_3OD$) δ 7.15 (d, 1H, *J* = 3.6 Hz), 6.63 (d, 1H, *J* = 3.2 Hz), 4.16 (m, 1H), 3.99 (m, 1H), 3.27 (s, 6H), 2.34 (s, 3H), 2.19 (s, 3H), 1.98-1.95 (m, 2H), 1.89-1.76 (m, 6H).

**Example 2611**

***N*$^2$-{cis-4-[(3,5-Dimethoxybenzyl)amino]cyclohexyl}-*N*$^4$,*N*$^4$,5,6-tetramethylpyrimidine-2,4-diamine bis-trifluoroacetate**

**Step A: Synthesis of *N*$^2$-{*cis-4*-[(3,5-dimethoxybenzyl)amino]cyclohexyl} -N$^4$,N$^4$,5,6-tetramethylpyrimidine-2,4-diamine bis-trifluoroacetate.**

To a solution of cis-4-(4-dimethylamino-5,6-dimethyl-pyrimidin-2-ylamino)-1-aminocyclohexane (26.3 mg, 0.1 mmol) in 0.5 mL MeOH was added 3,5-dimethoxybenzaldehyde (15.0 mg, 0.09 mmol). The mixture was stirred at room temperature for a half an hour and then sodium triacetoxyborohydride (84.8 mg, 0.4 mmol) was added. The mixture was stirred at room temperature overnight and then 0.5 mL of DMSO was added to the mixture. The compound was then subjected to purification by prep LCMS to yield *N*$^2$-{*cis-4-[(3,5*-dimethoxybenzyl)amino]cyclohexyl}-*N*$^4$,*N*$^4$,5,6-tetramethylpyrimidine-2,4-diamine bis-trifluoroacetate (24 mg, 0.037 mmol, 42%) as a white solid TFA salt.
ESI MS m/e 414.6 M+H$^+$ ; $^1$H NMR (400 MHz, $CD_3OD$) δ 6.71 (d, 2H, *J*= 2.0 Hz), 6.59 (t, 1H, *J* = 2.0 Hz), 4.28 (m, 1 H), 4.21 (s, 2H), 3.84 (s, 6H), 3.28 (m, 1 H), 3.27 (s, 6H), 2.34 (s, 3H), 2.19 (s, 3H), 2.10-2.08 (m, 4H), 1.85-1.83 (m, 4H).

**Example 2612**

***N*$^2$-{*cis*-4-[(3-Bromobenzyl)amino]cyclohexyl)-*N*$^4$,*N*$^4$,5,6-tetramethylpyrimidine-2,4-diamine bis-trifluoroacetate**

**Step A: Synthesis of *N*$^2$-{*cis*-4-[(3-bromobenzyl)amino]cyclohexyl}-*N*$^4$,*N*$^4$,5,6-tetramethylpyrimidine-2,4-diamine bis-trifluoroacetate.**

Using the procedure of Example 2611, the title compound was obtained as a white solid TFA salt.
ESI MS m/e 432.4 M+H$^+$; $^1$H NMR (400 MHz, $CD_3OD$) δ 7.78 (s, 1 H), 7.68 (d, 1H, *J* = 8.0 Hz), 7.54 (d, 1 H, J = 7.6 Hz), 7.45 (t, 1 H, *J* = 4 Hz), 4.29 (m, 3H), 3.28 (m, 1 H), 3.27 (s, 6H), 2.34 (s, 3H), 2.20 (s, 3H), 2.11-2.09 (m, 4H), 1.87-1.82 (m, 4H).

**Example 2613**

***N-(cis*-4-{[4-(Dimethylamino)-5,6-dimethylpyrimidin-2-yl]amino}cyclohexyl)-N'-(3-methoxyphenyl)urea trifluoroacetate**

**Step A: Synthesis of *N*-(*cis*-4-{[4-(dimethylamino)-5,6-dimethylpyrimidin-2yl]amino}-cyclohexyl)-N -(3-methoxyphenyl)urea trifluoroacetate.**

To a solution of cis-4-(4-dimethylamino-5,6-dimethyl-pyrimidin-2-ylamino)-1-aminocyclohexane (26.3 mg, 0.1 mmol) in 0.5 mL DMSO was added 3-methoxyphenyl isocyanate (13.1 uL, 0.1 mmol). The mixture was stirred at room

temperature overnight and then 0.5 mL of DMSO was added to the mixture. The compound was then subject to purification by prep LC MS to yield N-(cis-4-{[4-(dimethylamino)-5,6-dimethyl pyrimidin-2-yl]amino}cyclohexyl)-N'-(3-methoxyphenyl)urea trifluoroacetate (28 mg, 0.053 mmol, 53%) as a white solid.
ESI MS m/e 413.6 M+H$^+$ ; $^1$H NMR (400 MHz, $CD_3OD$) δ 7.18 (m, 2H), 6.86 (dd, 1H, *J,* = 8.0 Hz, $J_2$ = 2.0 Hz), 6.58 (dd, 1H, *J,* = 8.4 Hz, $J_2$ = 2.4 Hz), 4.03 (m, 1H), 3.82 (m, 1H), 3.79 (s, 3H), 3.27 (s, 6H), 2.33 (s, 3H), 2.19 (s, 3H), 1.92-1.73 (m, 8H).

**Example 2614**

***N*-(3,5-Difluorophenyl)-N'-(*cis*-4-} [4-(dimethylamino)-5,6-dimethylpyrimidin-2-yl]amino}cyclohexyl)urea trifluoroacetate**

**Step A: Synthesis of *N*-(3,5-difluorophenyl)-N'-(*cis*-4-}[4-(dimethylamino)-5,6-dimethylpyrimidin-2-yl]amino} cyclohexyl)urea trifluoroacetate.**

Using the procedure of Example 2613, the title compound was obtained as a white solid TFA salt.
ESI MS m/e 419.3 M+H$^+$ ; $^1$H NMR (400 MHz, $CD_3OD$) δ 7.08-7.03 (m, 2H), 6.55-6.49 (m, 1 H), 4.04 (m, 1H), 3.81 (m, 1H), 3.28 (s, 6H), 2.33 (s, 3H), 2.20 (s, 3H), 1.93-1.73 (m, 8H).

**Example 2615**

**1-(4-Chlorophenyl)-N-(cis-4-; [4-(dimethylamino)-5-methylpyrimidin-2-yl]amino}cyclohexyl) cyclobutanecarboxamide trifluoroacetate**

**Step A: Synthesis of 1-(4-chlorophenyl)-N-(cis-4-1[4-(dimethylamino)-5-methylpyrimidin-2-yl)amino} cyclohexyl)cyclobutanecarboxamide trifluoroacetate.**

To a solution of cis-4-(4-dimethylamino-5-methyl-pyrimidin-2-ylamino)-1-aminocyclohexane (35 mg, 0.14 mmol), 1-(4-chlorophenyl)-cyclobutanecarboxylic acid (30 mg, 1 eq.) in DCM (2 mL) was added HATU (58 mg, 1.1 eq.) and followed by $Et_3N$ (40 μL, 2 eq.). The reaction was stirred at room temperature for 4 h, and completion of the reaction was confirmed by LCMS. After removal of the volatile solvent, the residue was purified by prep-HPLC to give 32 mg (41 %) of 1-(4-chlorophenyl)-N-(*cis*-4-{[4-(dimethylamino)-5-methylpyrimidin-2-yl]amino}cyclohexyl)cyclobutanecarboxamide trifluoroacetate as a white solid.
ESI MS m/e 442 M + H$^+$; $^1$H NMR (400 MHz, $CDCl_3$) δ 13.6 (bs, 1 H), 8.38 (d, 1 H, J = 7.2 Hz), 7.32-7.22 (m, 5 H), 5.76 (d, 1 H, J = 8.8 Hz), 4.09 (bs, 1 H), 3.81 (m, 1 H), 3.26 (s, 6 H), 2.77 (m, 2 H), 2.44 (m, 2 H), 2.22 (s, 3 H), 2.02 (m, 1 H), 1.86 (m, 1 H), 1.65~1.50 (m, 8 H).

**Example 2616**

**2-(4-Chlorophenyl)-*N*-(*cis*-4-{[4-(dimethylamino)-5-methylpyrimidin-2-yl]amino}cyclohexyl)-2-methylpropanamide trifluoroacetate**

**Step A: Synthesis of 2-(4-chlorophenyl)-*N*-(*cis*-4-{[4-(dimethylamino)-5-methylpyrimidin-2-yl]amino} cyclohexyl)-2-methylpropanamide trifluoroacetate.**

Using the procedure of Example 2615, the title compound was obtained.
ESI MS m/e 430 M + H$^+$; $^1$H NMR (400 MHz, $CDCl_3$) δ 13.3 (bs, 1 H), 8.21 (d, 1 H, J = 7.6 Hz), 7.28 (bs, 4 H), 7.22 (m, 1 H), 5.67 (d, 1 H, J = 8.4 Hz), 4.09 (bs, 1 H), 3.85 (m, 1 H), 3.26 (s, 6 H), 2.22 (s, 3 H), 1.71~1.61 (m, 6 H), 1.54 (s, 6 H), 1.50 (m, 2 H).

**Example 2617**

**2-[3,5-bis(Trifluoromethyl)phenyl]-N-(cis-4-{[4-(dimethylamino)-5-methylpyrimidin-2-yl]amino}cyclohexyl)-2-methylpropanamide trifluoroacetate**

**Step A: Synthesis of 2-[3,5-bis(trifluoromethyl)phenyl]-N-(cis-4-{[4-(dimethylamino)-5-methylpyrimidin-2-yl]amino}cyclohexyl)-2-methylpropanamide trifluoroacetate.**

Using the procedure of Example 2615, the title compound was obtained.
ESI MS m/e 532 M + $H^+$; $^1H$ NMR (400 MHz, $CDCl_3$) δ 13.9 (bs, 1H), 8.68 (d, 1H, *J* = 7.6 Hz), 7.78 (s, 2 H), 7.72 (s, 1 H), 7.21 (d, 1H, *J* = 4.4 Hz), 6.14 (d, 1H, *J* = 8.4 Hz), 4.20 (bs, 1H), 3.93 (m, 1H), 3.26 (s, 6 H), 2.22 (s, 3 H), 1.77~1.56 (m, 8 H), 1.61 (s, 6 H).

**Example 2618**

**2-[3,5-bis(Trifluoromethyl)phenyl]-N-(cis-4-{[4-(dimethylamino)-5-methylpyrimidin-2-yl]amino}cyclohexyl)acetamide trifluoroacetate**

**Step A: Synthesis of 2-[3,5-bis(trifluoromethyl)phenyl]-N-(cis-4-{[4-(dimethylamino)-5-methylpyrimidin-2-yl]amino}cyclohexyl)acetamide trifluoroacetate.**

Using the procedure of Example 2615, the title compound was obtained.
ESI MS m/e 504 M + $H^+$; $^1H$ NMR (400 MHz, $CDCl_3$) δ 13.8 (bs, 1 H), 8.51 (d, 1 H, J = 7.8 Hz), 7.78 (s, 2 H), 7.73 (s, 1 H), 7.22 (m, 1 H), 5.87 (d, 1 H, J = 8.0 Hz), 4.15 (bs, 1 H), 3.96 (m, 1 H), 3.62 (s, 2 H), 3.28 (s, 6 H), 2.24 (s, 3 H), 1.80~1.65 (m, 8 H).

**Example 2619**

**1-(4-Chlorophenyl)-N-(cis-4-{[4-(dimethylamino)-6-methylpyrimidin-2-yl]amino}cyclohexyl)cyclopropanecarboxamide trifluoroacetate**

**Step A: Synthesis of 1-(4-Chlorophenyl)-N (cis-4-{[4-(dimethylamino)-6-methylpyrimidin-2-yl]amino}cyclohexyl)cyclopropanecarboxamide trifluoroacetate.**

To a solution of *cis*-4-(4-dimethylamino-6-methyl-pyrimidin-2-ylamino)-1-aminocyclohexane (36 mg, 0.14 mmol), 1-(4-chlorophenyl)-cyclopropanecarboxylic acid (31 mg, 1 eq.) in DCM (2 mL) was added HATU (60 mg, 1.1 eq.) and followed by $Et_3N$ (40 µL, 2 eq.). The reaction was stirred at room temperature for 4 h, and completion of the reaction was confirmed by ESI MS. After removal of the volatile solvent, the residue was purified by prep-HPLC to give 45 mg (72 %) of 1-(4-Chlorophenyl)-*N*-(*cis*-4-{[4-(dimethylamino)-6-methylpyrimidin-2-yl]amino}cyclohexyl)cyclopropane carboxamide trifluoroacetate as a white solid.
ESI MS m/e 428 M + $H^+$; $^1H$ NMR (400 MHz, $CDCl_3$) δ 13.4 (bs, 1 H), 8.61 (d, 1 H, J = 7.2 Hz), 7.32 (m, 4 H), 5.70 (s, 1 H), 5.46 (d, 1 H, *J* = 8.0 Hz), 4.04 (bs, 1 H), 3.79 (m, 1 H), 3.21 (s, 3 H), 3.10 (s, 3 H), 2.31 (s, 3 H), 1.68~1.47 (m, 9 H), 1.22 (m, 1 H), 1.00 (m, 2H).

**Example 2620**

**1-(4-Chlorophenyl)-*N*-(*cis*-4-{[4-(dimethylamino)-6-methylpyrimidin-2-yl]amino}cyclohexyl)cyclobutanecarboxamide trifluoroacetate**

**Step A: Synthesis of 1-(4-chlorophenyl)-*N*-(*cis*-4-{[4-(dimethylamino)-6-methylpyrimidin-2-yl]amino}cyclohexyl)cyclobutanecarboxamide trifluoroacetate.**

Using the procedure of Example 2619, the title compound was obtained.
ESI MS m/e 442 M + $H^+$; $^1H$ NMR (400 MHz, $CDCl_3$) δ 13.1 (bs, 1 H), 8.41 (d, 1 H, J = 7.6 Hz), 7.28 (s, 4 H), 5.95 (d, 1 H, J = 8.8 Hz), 5.72 (s, 1 H), 4.14 (bs, 1 H), 3.82 (m, 1 H), 3.21 (s, 3 H), 3.11 (s, 3 H), 2.77 (m, 2 H), 2.44 (m, 2 H), 2.31 (s, 3 H), 2.01 (m, 1 H), 1.83 (m, 1 H), 1.70~1.50 (m, 8 H).

**Example 2621**

**1-(2,4-Dichlorophenyl)-N-(*cis*-4-{[4-(dimethylamino)-6-methylpyrimidin-2-yl]amino}cyclohexyl) cyclopropanecarboxamide trifluoroacetate**

**Step A: Synthesis of 1-(2,4-dichlorophenyl)-*N*-(*cis*-4-{[4-(dimethylamino)-6-methylpyrimidin-2-yl]amino) cyclohexyl)cyclopropanecarboxamide trifluoroacetate.**

Using the procedure of Example 2619, the title compound was obtained.
ESI MS m/e 462 M + $H^+$; $^1$H NMR (400 MHz, $CDCl_3$) δ 13.4 (bs, 1 H), 8.54 (bs, 1 H), 7.43 (s, 1 H), 7.28 (d, 1 H, J = 8.4 Hz), 7.26 (d, 1 H, J = 8.0 Hz), 5.70 (s, 1 H), 5.39 (d, 1 H, J = 8.0 Hz), 4.06 (bs, 1 H), 3.84 (m, 1 H), 3.20 (s, 3 H), 3.10 (s, 3 H), 2.30 (s, 3 H), 1.69~1.62 (m, 8 H), 1.50 (m, 2 H), 1.01 (m, 2H).

**Example 2622**

**2-[3,5-bis(Trifluoromethyl)phenyl]-*N*-(*cis*-4-{[4-(dimethylamino)-6-methylpyrimidin-2-yl]amino}cyclohexyl)-2-methylpropanamide trifluoroacetate**

**Step A: Synthesis of 2-[3,5-bis(trifluoromethyl)phenyl]-*N*-(*cis*-4-{[4-(dimethylamino)-6-methylpyrimidin-2-yl] amino}cyclohexyl)-2-methylpropanamide trifluoroacetate.**

Using the procedure of Example 2619, the title compound was obtained.
ESI MS m/e 532 M + $H^+$; $^1$H NMR (400 MHz, $CDCl_3$) δ 13.8 (bs, 1 H), 8.80 (d, 1 H, J = 8.4 Hz), 7.79 (s, 2 H), 7.72 (s, 1 H), 6.20 (d, 1 H, J = 8.4 Hz), 5.70 (s, 1 H), 4.24 (bs, 1 H), 3.94 (bm, 1 H), 3.22 (s, 3 H), 3.10 (s, 3 H), 2.30 (s, 3 H), 1.79~1.60 (m, 8 H), 1.61 (s, 6 H).

**Example 2623**

**2-(3,4-dinuorophenyl)-*N*-(*cis*-4-{[4-(dimethylamino)-5-methylpyrimidin-2-yl]amino}cyclohexyl)-2-hydroxyacetamide hydrochloride**

**Step A: 2-(3,4-difluorophenyl)-*N*-(*cis*-4-{[4-(dimethylamino)-5-methylpyrimidin-2-yl]amino}cyclohexyl)-2-hydroxyacetamide hydrochloride.**

To a solution of *cis*-4-(4-dimethylamino-5-methyl-pyrimidin-2-ylamino)-1-aminocyclohexane (43 mg, 0.17 mmol), 3,4-difluoro mandelic acid (34 mg, 1 eq.) in DCM (2 mL) was added HATU (68 mg, 1.1 eq.) and followed by $Et_3N$ (50 μL, 2 eq.). The reaction was stirred at room temperature for 4 h and quenched. After removal of the volatile solvent, the residue was purified by column chromatography (DCM : MeOH = 100 : 0 to 94 : 6). 28 mg (39 %) of the product was isolated and converted into HCl salt.
ESI MS m/e 420 M + $H^+$; $^1$H NMR (400 MHz, $CDCl_3$) δ 8.48 (d, 1 H, *J* = 8.0 Hz), 7.39~7.20 (m, 3 H), 7.04 (m, 1H), 5.05 (s, 1 H), 4.08 (bs, 1 H), 3.89 (bs, 1 H), 3.26 (s, 6 H), 2.22 (s, 3 H), 1.78~1.60 (m, 8 H), two exchangeable protons (-NH- and -OH) were not detected.

**Example 2624**

***N*-(*cis*-4-{[4-(Dimethylamino)-5-methylpyrimidin-2-yl]amino}cyclohexyl)-2-hydroxy-2-[3-(trifluoromethyl) phenyl]acetamide**

**Step A: Synthesis of *N*-(*cis*-4-{[4-(dimethylamino)-5-methylpyrimidin-2-yl]amino}-cyclohexyl)-2-hydroxy-2-[3-(trifluoromethyl)phenyl]acetamide.**

Using the procedure of Example 2623, the title compound was obtained.
ESI MS m/e 452 M + $H^+$; $^1$H NMR (400 MHz, $CDCl_3$) δ 7.83 (bs, 1 H), 7.22 (s, 1 H), 7.65 (d, 1 H, *J* = 8.0 Hz), 7.51 (d, 1 H, 7= 8.0 Hz), 7.42 (t, 1 H, *J* = 8.0 Hz), 7.22 (s, 1 H), 7.00 (d, 1 H, *J*= 8.0 Hz), 5.10 (s, 1 H), 4.04 (bs, 1 H), 3.89 (bs, 1 H), 3.20 (s, 6 H), 2.18 (s, 3 H), 1.78~1.64 (m, 8 H), one exchangeable proton (-OH) was not detected.

**Example 2625**

**N-(cis-4-{[4-(Dimethylamino)-5-methylpyrimidin-2-yl]amino}cyclohexyl)-2-hydroxy-2-(4-methoxyphenyl) acetamide**

**Step A: Synthesis of N-(cis-4-{[4-(dimethylamino)-5-methylpyrimidin-2-yl]amino}-cyclohexyl)-2-hydroxy-2-(4-methoxyphenyl)acetamide.**

Using the procedure of Example 2623, the title compound was obtained.
ESI MS m/e 414 M + $H^+$; $^1$H NMR (400 MHz, $CDCl_3$) δ 8.72 (d, 1 H, J = 6.8 Hz), 7.31 (d, 2 H, J = 8.4 Hz), 7.22 (s, 1 H), 6.83 (d, 2 H, J = 8.4 Hz), 6.78 (d, 1 H, J = 7.6 Hz), 4.98 (s, 1 H), 4.06 (bs, I H), 3.90 (bs, 1 H), 3.76 (s, 3 H), 3.25 (s, 6 H), 2.20 (s, 3 H), 1.78~1.64 (m, 8 H.

**Example 2626**

**2-(3-Chlorophenyl)-*N*-(*cis*-4-{[4-(dimethylamino)-5-methylpyrimidin-2-yl]amino}cyclohexyl)-2-hydroxyacetamide**

**Step A: Synthesis of 2-(3-chlorophenyl)-*N*-(*cis*-4-{[4-(dimethylamino)-5-methylpyrimidin-2-yl]amino} cyclohexyl)-2-hydroxyacetamide.**

Using the procedure of Example 2623, the title compound was obtained.
ESI MS m/e 418 M + $H^+$; $^1$H NMR (400 MHz, $CDCl_3$) δ 8.63 (bs, 1 H), 7.44 (s, 1 H), 7.33 (m, 1 H), 7.21 (m, 2 H), 7.12 (bs, 1 H), 5.03 (s, 1 H), 4.08 (bs, 1 H), 3.88 (bs, 1 H), 3.24 (s, 6 H), 2.19 (s, 3 H), 1.78∼1.63 (m, 8 H).

**Example 2627**

**2-(2,3-Difluorophenyl)-*N*-(*cis*-4-{[4-(dimethylamino)-5-methylpyrimidin-2-yl]amino}cyclohexyl)-2-hydroxyacetamide**

**Step A: Synthesis of 2-(2,3-difluorophenyl)-*N*-(cis-4-{[4-(dimethylamino)-5-methylpyrimidin-2-yl]amino} cyclohexyl)-2-hydroxyacetamide.**

Using the procedure of Example 2623, the title compound was obtained.
ESI MS m/e 420 M + $H^+$; $^1$H NMR (400 MHz, $CDCl_3$) δ 7.26 (s, 1 H), 7.14 (m, 1H), 7.06 (m, 2 H), 6.73 (d, 1 H, J = 8.0 Hz), 5.32 (s, 1 H), 4.06 (bs, 1 H), 3.93 (bs, 1 H), 3.22 (s, 6 H), 2.20 (s, 3 H), 1.78~1.64 (m, 8 H).

**Example 2628**

***N*-(*cis*-4-{[4-(Dimethylamino)-5-methylpyrimidin-2-yl]amino}cyclohexyl)-2-(trifluoromethyl) benzenesulfonamide hydrochloride**

**Step A: Synthesis of *N*-(*cis*-4-{[4-(dimethylamino)-5-methylpyrimidin-2-yl]amino}-cyclohexyl)-2-(trifluoromethyl)benzenesulfonamide hydrochloride.**

To a solution of cis-4-(4-dimethylamino-5-methyl-pyrimidin-2-ylamino)-1-aminocyclohexane (45 mg, 0.18 mmol) in IPA (2 mL) was added 2-trifluoromethyl benzenesulfonyl chloride (44 mg, 1 eq.) and followed by DIEA (50 µL, 2 eq.). The reaction was stirred at room temperature for 1.5 h under an inert atmosphere, and the progress of the reaction was monitored by ESI MS. The reaction was diluted with DCM (7 mL), washed with saturated $NaHCO_3$ (1 x 5 mL) and water (1 x 5 mL), and concentrated. The crude product was purified by column chromatography (DCM : MeOH = 100 : 0 to 95 : 5). 31 mg (38 %) of the product was isolated and converted into HCl salt.
ESI MS m/e 458 M + $H^+$; $^1$H NMR (400 MHz, DMSO-$d_6$) δ 12.2 (bs, 1 H), 8.13 (m, 2 H), 8.06 (d, 1 H, J = 6.0 Hz), 7.93 (d, 1 H, *J* = 8.0 Hz), 7.87 (t, 1 H, *J* = 7.6 Hz), 7.79 (t, I H, *J* = 7.6 Hz), 7.62 (bs, 1 H), 3.78 (bs, 1 H), 3.22 (s, 6 H), 3.21 (bs, 1 H), 2.20 (s, 3 H), 1.78∼1.54 (m, 8 H).

**Example 2629**

**4-Chloro-*N*-(cis-4-{[4-(dimethylamino)-5-methylpyrimidin-2-yl]amino}cyclohexyl)-benzenesulfonamide hydrochloride**

**Step A: Synthesis of 4-chloro-*N*-(cis-4-{[4-(dimethylamino)-5-methylpyrimidin-2-yl]amino}cyclohexyl) benzenesulfonamide hydrochloride.**

Using the procedure of Example 2628, the title compound was obtained.
ESI MS m/e 424 M + $H^+$; $^1H$ NMR (400 MHz, DMSO-$d_6$) δ 11.9 (bs, 1 H), 7.92 (bs, 1 H), 7.83 (s, 1 H, overlapped with the doublet of 7.81 ppm), 7.81 (d, 2 H, *J* = 8.4 Hz), 7.64 (d, 2 H, *J* = 8.4 Hz), 7.58 (bs, 1 H), 3.74 (bs, 1 H), 3.21 (s, 6 H), 3.08 (bs, 1 H), 2.20 (s, 3 H), 1.70～1.44 (m, 8 H).

**Example 2630**

**2-Bromo-*N*-(*cis*-4-{[4-(dimethylamino)-5-methylpyrimidin-2-yl]amino}cyclohexyl)-benzenesulfonamide hydrochloride**

**Step A: Synthesis of 2-bromo-*N*-(*cis*-4-{[4-(dimethylamino)-5-methylpyrimidin-2-yl]amino}cyclohexyl) benzenesulfonamide hydrochloride.**

Using the procedure of Example 2628, the title compound was obtained.
ESI MS m/e 468 M + $H^+$; $^1H$ NMR (400 MHz, DMSO-$d_6$) δ 11.9 (bs, 1 H), 8.00 (d, 1 H, *J* = 7.2 Hz), 7.92 (bs, 1 H), 7.82 (d, 2 H, J = 7.6 Hz), 7.59~7.48 (m, 3 H), 3.73 (bs, 1 H), 3.21 (s, 6 H), 3.20 (bs, 1 H), 2.20 (s, 3 H), 1.72 (m, 2 H), 1.58 (m, 6 H).

**Example 2631**

***N*-(cis-4-{[4-(Dimethylamino)-5-methylpyrimidin-2-yl]amino}cyclohexyl)thiophene-2-sulfonamide hydrochloride**

**Step A: Synthesis of *N*-(cis-4-{[4-(dimethylamino)-5-methylpyrimidin-2-yl]amino}-cyclohexyl)thiophene-2-sulfonamide hydrochloride.**

Using the procedure of Example 2628, the title compound was obtained.
ESI MS m/e 396 M + $H^+$; $^1H$ NMR (400 MHz, DMSO-$d_6$) δ 12.1 (bs, 1 H), 7.99 (bs, 1 H), 7.92 (bs, 1 H), 7.88 (d, 1 H, J = 4.8 Hz), 7.60 (bs, 1 H), 7.57 (d, 1 H, *J* = 2.8 Hz), 7.14 (t, 1 H, *J* = 4.8 Hz), 3.75 (bs, 1 H), 3.22 (s, 6 H), 3.17 (bs, 1 H), 2.20 (s, 3 H), 1.70～1.51 (m, 8 H).

**Example 2632**

***N*$^4$,*N*$^4$,5-Trimethyl-*N*$^2$-(cis-4-{[3-(trifluoromethyl)benzyl]amino}cyclohexyl)pyrimidine-2,4-diamine bistrifluoroacetate**

**Step A: Synthesis of *N*$^4$,*N*$^4$,5-trimethyl-*N*$^2$-(cis-4-{[3-(trifluoromethyl)benzyl]amino}-cyclohexyl)pyrimidine-2,4-diamine bistrifluoroacetate.**

A solution of cis-4-(4-dimethylamino-5-methyl-pyrimidin-2-ylamino)-1-aminocyclohexane (31 mg, 0.12 mmol) and 3-trifluoromethyl benzaldehyde (22 mg, 1 eq.) in MeOH (1.5 mL) was stirred at room temperature for 4 h. NaBH$(OAc)_3$ (85 mg, ~ 4 eq.) was added into the reaction, and the reaction was stirred overnight. The reaction was quenched with water, extracted with DCM, concentrated, and purified by prep-HPLC. 35 mg (54 %) of N$^4$,N$^4$,5-trimethyl-N$^2$-(cis-4-{[3-(trifluoromethyl)benzyl]amino}cyclohexyl)pyrimidine-2,4-diamine bistrifluoroacetate was isolated as a white powder.
ESI MS m/e 408 M + $H^+$; $^1H$ NMR (400 MHz, $CDCl_3$) δ 13.7 (bs, 1 H), 9.70 (bs, 2 H), 8.60 (d, 1 H, J = 8.8 Hz), 7.70 (m, 2 H), 7.59 (d, 1 H, J = 8.0 Hz), 7.48 (t, 1 H, J = 8.4 Hz), 4.31 (m, 1 H), 4.23 (s, 2 H), 3.30 (m, 1 H), 3.29 (s, 6 H), 2.25 (s, 3 H), 2.05 (m, 2 H), 1.93 (m, 4 H), 1.64 (m, 2 H).

**Example 2633**

***$N^2$-(cis-4-*{[4-(Difluoromethoxy)benzyl]amino) cyclohexyl)-$N^4$,$N^4$,5-trimethylpyrimidine-2,4-diamine bistrifluoroacetate**

**Step A: Synthesis of $N^2$-(cis-4-{[4-(difluoromethoxy)benzyl]amino}cyclohexyl)-$N^4$,$N^4$,5-trimethylpyrimidine-2,4-diamine bistrifluoroacetate.**

Using the procedure of Example 2632, the title compound was obtained.
ESI MS m/e 406 M + $H^+$; $^1H$ NMR (400 MHz, $CDCl_3$) δ 13.8 (bs, 1 H), 9.60 (bs, I H), 8.60 (d, 1H, *J* = 8.8 Hz), 7.46 (d, 2 H, *J* = 8.8 Hz), 7.24 (s, 1H), 7.07 ( d, 2 H, J = 8.8 Hz), 6.48 (t, 1 H, $J_{F-H}$ = 73.6 Hz), 4.31 (m, 1 H), 4.15 (s, 2 H), 3.40 (bs, 1 H), 3.29 (s, 6 H), 2.24 (s, 3 H), 2.05 (m, 2 H), 1.90 (m, 4 H), 1.63 (m, 2 H).

**Example 2634**

***$N^2$*-{*cis*-4-[(3-Bromo-4-methoxybenzyl)amino]cyclohexyl)-*$N^4$*,*$N^4$*,5-trimethylpyrimidine-2,4-diamine bistrifluoroacetate**

**Step A: Synthesis of *$N^2$-)cis-4-[(3-bromo-4-methoxybenzyl)amino]cyclohexyl]-$N^4$,$N^4$,5*-trimethylpyrimidine-2,4-diamine bistrifluoroacetate.**

Using the procedure of Example 2632, the title compound was obtained.
ESI MS m/e 448 M + $H^+$; $^1H$ NMR (400 MHz, $CDCl_3$) δ 13.8 (bs, 1H), 9.44 (bs, 1H), 8.57 (d, 1 H, *J* = 8.0 Hz), 7.58 (d, 1H, J = 2.4 Hz), 7.41 (dd, 1 H, *J* = 8.8 and 2.0 Hz), 7.24 (s, 1 H), 6.86 ( d, 1 H, J = 8.0 Hz), 4.29 (m, 1H), 4.07 (s, 2 H), 3.86 (s, 3 H), 3.28 (s, 6 H), 3.25 (bs, 1 H), 2.24 (s, 3 H), 2.05~1.85 (m, 6 H), 1.64 (m, 2 H).

**Example 2635**

***$N^2$*-(3,4-Dichlorophenyl)-*N*-(*cis*-4-{[4-(dimethylamino)-5-methylpyrimidin-2-yl]amino}cyclohexyl)-*$N^2$*-methylglycinamide bistrifluoroacetate**

**Step A: Synthesis of 2-bromo-N-[4-(4-dimethylamino-5-methyl-pyrimidin-2-ylamino)-cyclohexyl]-acetamide.**

cis-[4-(4-dimethylamino-5-methyl-pyrimidin-2-ylamino) cyclohexyl]-carbamic acid *tert*-butyl ester (3.5 g, 14.0 mmol) was dissolved in 20 mL of methylene chloride, and cooled to 0°C in an ice bath. Bromo-acetyl bromide (1.26 mL, 14.0 mmol) was added dropwise into the stirring solution over the ice bath. The reaction mixture was stirred at room temperature for 10 minutes. Methylene chloride was evaporated off to yield 2-bromo-*N*-[4-(4-dimethylamino-5-methyl-pyrimidin-2-ylamino)-cyclohexyl]-acetamide as a pinkish crude solid. (6.1 g, 95%).
ESI MS m/z 370.1 (M + $H^+$) ; $^1H$ NMR (400 MHz, $CDCl_3$) δ 12.20 (s, 1 H), 8.21 (d, *J* = 7.2 Hz, 1H), 6.85 (d, J = 6.8 Hz, 1H), 4.15 (s, 1H), 3.97-3.89 (m, 3H), 3.31 (s, 6H), 2.27 (s, 3H), 1.93-1.72 (m, 8H).

**Step B: Synthesis of *$N^2$*-(3,4-dichlorophenyl)-*N*-(*cis*-4-{[4-(dimethylamino)-5-methylpyrimidin-2-yl]amino} cyclohexyl)-*$N^2$*-methylglycinamide bistrifluoroacetate.**

2-Bromo-*N*-[4-(4-dimethylamino-5-methyl-pyrimidin-2-ylamino)-cyclohexyl]-acetamide (50 mg, 0.135 mmol) and (3,4-dichloro-phenyl)-methyl-amine (48 mg, 0.270 mmol) were dissolved in 0.8 mL of DMF. The reaction mixture was heated via Smith Synthesizer at 180°C for 50 minutes. The crude was purified by HPLC to give $N^2$-(3,4-dichlorophenyl)-N-(cis-4-{[4-(dimethylamino)-5-methylpyrimidin-2-yl]amino}cyclohexyl)-*$N^2$*-methylglycinamide bistrifluoroacetate as a white solid. (12.8 mg, 18%)
ESI MS m/z 465.3 (M + $H^+$) ; $^1H$ NMR (400 MHz, $CDCl_3$) δ 8.75 (d, J= 6.0 Hz, 1H), 6.80 (d, J= 2.8 Hz, 1 H), 6.67-6.65 (m, 1 H), 6.57 (dd, J = 9.0, 3.0 Hz, 1 H), 4.13 (s, 1 H), 3.98 (s, 1 H), 3.86 (s, 2H), 3.29 (s, 6H), 3.06 (s, 3H), 2.25 (s, 3H), 1.73-1.62 (m, 8H).

**Example 2636**

***N*-[((1*R*,3S)-3-{[4-(Dimethylamino)-5-methylpyrimidin-2-yl]amino)cyclopentyl)methyl]-2-(4-fluorophenoxy) nicotinamide trifluoroacetate**

**Step A: Synthesis of (3-{[(2-Chloro-pyridine-3-carbonyl)-amino]-methyl}-cyclopentyl)-carbamic acid tert-butyl ester.**

(3-Aminomethyl-cyclopentyl)-carbamic acid tert-butyl ester (0.050 g, 0.23 mmol), 2-chloronicotinoyl chloride (0.041 g, 0.23 mmol), and diisopropylethylamine (0.061 mL, 0.34 mmol) were combined in dichloromethane (2.00 mL) at ambient temperature and stirred 18 hrs. The mixture was concentrated and purified by flash silica chromatography (5% methanol in ethyl acetate) to give (3-{[(2-chloro-pyridine-3-carbonyl)-amino]-methyl}-cyclopentyl)-carbamic acid tert-butyl ester (0.035 g, 43%) as a solid.
ESI MS m/e 354, M + $H^+$; $^1$H NMR (400 MHz, $CDCl_3$) δ 8.47 (dd, *J*aa = 1.5 Hz, *J*ab = 4.7 Hz, 1 H), 8.11 (dd, *J*aa = 1.5 Hz, *J*ab = 7.6 Hz, 1H), 7.35 (dq, *J*aa = 1.2 Hz, *J*ab = 4.8 Hz, *J*ac = 7.6 Hz, 1H), 6.56 (bs, 1H), 4.59 (bs, 1 H), 3.97 (m, 1H), 3.48 (m, 2 H), 2.27 (m, 2 H), 1.94 (m, 2 H), 1.49 (m, 1 H), 1.44 (s, 9 H), 1.25 (m, 2 H).

**Step B: Synthesis of *N*-(3-Amino-cyclopentylmethyl)-2-(4-fluoro-phenoxy)-nicotinamide.**

(3-{[(2-Chloro-pyridine-3-carbonyl)-amino]-methyl}-cyclopentyl)-carbamic acid tert-butyl ester (0.23 mmol), 4-fluorophenol (0.026 g, 0.23 mmol), cesium carbonate (0.152 g, 0.46 mmol), and dioxane (2.00 mL) were combined and heated to 180°C for 1 hr. utilizing a SmithSynthesizer microwave apparatus. Trifluoroacetic acid (3.00 mL) was added and the mixture stirred 18 hrs. Then it was concentrated, neutralized with saturated aqueous $NaHCO_3$, extracted with dichloromethane, and concentrated to give N-(3-amino-cyclopentylmethyl)-2-(4-fluoro-phenoxy)-nicotinamide as the crude product.
ESI MS m/e 330, M + $H^+$; $^1$H NMR (400 MHz, $CDCl_3$) δ 8.52 (dd, Jaa = 1.0 Hz, Jab = 7.6 Hz, 1 H), 8.19 (dd, Jaa = 1.9 Hz, Jab = 3.9 Hz, 1 H), 8.06 (t, J = 5.8 Hz, 1 Hz), 6.91 (t, J = 8.2 Hz, 1 H), 6.77 (dd, Jaa = 3.6 Hz, Jab = 3.2 Hz, 1 H), 3.62 (m, 2 H), 2.26 (m, 2 H), 2.05 (m, 1 H), 1.81 (m, 2 H), 1.62 (m, 1 H), 1.48 (m, 2 H).

**Step C: Synthesis of *N*-[((1*R*,3*S*)-3-{[4-(dimethylamino)-5-methylpyrimidin-2-yl]-amino) cyclopentyl) methyl]-2-(4-fluorophenoxy)nicotinamide trifluoroacetate.**

5-Methyl-4-dimethylamino-2-chloropyrimidine (0.040 g, 0.23 mmol), *N*-(3-amino-cyclopentylmethyl)-2-(4-fluoro-phenoxy)-nicotinamide (0.23 mmol), diisopropyl-ethylamine (0.061 mL, 0.34 mmol), and isopropanol (2.00 mL) were combined and heated to 180°C for 2 hrs. utilizing a SmithSynthesizer microwave apparatus. The mixture was then purified by prep LCMS (gradient: 15-95% acetonitrile-water with 0.05% TFA) to give N-[((1*R*,3*S*)-3-{[4-(dimethylamino)-5-methylpyrimidin-2-yl]amino}cyclopentyl) methyl]-2-(4-fluorophenoxy)nicotinamide trifluoroacetate as a white solid (0.018 g, 13.5% over two steps).
ESI MS m/e 465, M + $H^+$; $^1$H NMR (400 MHz, DMSO-$d_6$) δ 11.63 (bs, 1 H), 8.44 (t, J = 5.7 Hz, I H), 8.16 (dd, Jaa = 1.9 Hz, Jab = 4.8 Hz, 1 H), 8.04 (dd, Jaa = 1.8 Hz, Jab = 7.4 Hz, 1 H), 7.98 (bs, 1 H), 7.53 (s, 1 H), 7.25-7.19 (m, 2 H), 4.08 (bs, 1 H), 3.22 (s, 6 H), 2.53 (s, 3 H), 2.19 (m, 2 H), 1.95 (m, 1 H), 1.71 (m, 1 H), 1.54 (m, 2 H), 1.46 (m, 2 H), 1.22 (m, 2 H).

**Example 2637**

***N*-[((1*R*,3S)-3-{[4-(Dimethylamino)-5-methylpyrimidin-2-yl]amino}cyclopentyl)methyl]-6-(2-methoxyphenoxy) nicotinamide trifluoroacetate**

**Step A: Synthesis of (3-{[(6-Chloro-pyridine-3-carbonyl)-amino]-methyl}-cyclopentyl)-carbamic acid tert-butyl ester.**

(3-Aminomethyl-cyclopentyl)-carbamic acid tert-butyl ester (0.050 g, 0.23 mmol), 6-chloronicotinoyl chloride (0.041 g, 0.23 mmol), and diisopropylethylamine (0.061 mL, 0.34 mmol) were combined in dichloromethane (2.00 mL) at ambient temperature and stirred 18 hrs. The mixture was concentrated and purified by flash silica chromatography (5% methanol in ethyl acetate) to give an orange gel.
ESI MS m/e 354, M + $H^+$; $^1$H NMR (400 MHz, $CDCl_3$) δ 8.75 (d, J = 2.1 Hz, 1 H), 8.09 (dd, Jaa = 1.8 Hz, Jab = 8.3 Hz, 1 H), 7.41 (d, J = 8.3 Hz, 1 H), 6.48 (bs, 1 H), 4.65 (d, J = 8 Hz, 1 H), 3.92 (m, 1 H), 3.46 (m, 2 H), 2.25 (m, 2 H), 1.98 (m, 2 H), 1.81 (m, 1 H), 1.47 (s, 9 H), 1.18 (m, 2 H).

**Step B: Synthesis of N-[((1*R*,3S)-3-{[4-(dimethylamino)-5-methylpyrimidin-2-yl]amino}cyclopentyl)methyl]-6-(2-methoxyphenoxy)nicotinamide trifluoroacetate.**

(3-{[(6-Chloro-pyridine-3-carbonyl)-amino]-methyl}-cyclopentyl)-carbamic acid tert-butyl ester (0.23 mmol), 2-methoxyphenol (0.029 g, 0.23 mmol), cesium carbonate (0.152 g, 0.46 mmol), and dioxane (2.00 mL) were combined and heated to 180°C for 1 hr. utilizing a SmithSynthesizer microwave apparatus. Trifluoroacetic acid (3.00 mL) was added and the mixture stirred 18 hrs. Then it was concentrated, neutralized with saturated aqueous $NaHCO_3$, extracted with dichloromethane, and concentrated to give a foam. 5-Methyl-4-dimethylamino-2-chloropyrimidine (0.040 g, 0.23 mmot), diisopropylethylamine (0.061 mL, 0.34 mmol), and isopropanol (2.00 mL) were added and the combined mixture was heated to 180 °C for 2 hrs utilizing a Smith synthesizer microwave apparatus. The mixture was then purified by prep-LCMS (gradient: 15-95% acetonitrile-water with 0.05% TFA) to give N-[((1R,3,S)-3-{[4-(dimethylamino)-5-methylpyrimidin-2-yl]amino}cyclopentyl)methyl]-6-(2-methoxyphenoxy)nicotinamide trifluoroacetate as a white solid (0.011 g, 8.1% over four steps).
ESI MS m/e 477, M + $H^+$; $^1H$ NMR (400 MHz, DMSO-$d_6$) δ 9.05 (bs, 1H), 8.63 (s, 1H), 8.16 (dd, Jaa = 2.2 Hz, Jab = 8.6 Hz, 1H), 7.58 (bs, 1H), 7.23 (s, 1H), 7.19 (d, *J* = 6.2 Hz, 1H), 7.16 (dd, Jaa = 1.5 Hz, Jab = 7.7 Hz, 1H), 7.00 (t, *J* = 8.8 Hz, 1H), 6.91 (d, *J* = 12 Hz, 1H), 4.25 (bs, 1H), 3.75 (s, 3 H), 3.66 (m, 1 H), 3.29 (s, 6 H), 3.11 (m, 2 H), 2.52 (m, 2 H), 2.23 (s, 3 H), 2.10 (m, 2 H), 1.78 (m, 1H), 1.62 (m, 2H).

**Example 2638**

***N-(cis-4-*{[4-(Dimethylamino)-5-methylpyrimidin-2-yl]amino}cyclohexyl)-2-(3-fluorophenoxy)acetamide**

**Step A: Synthesis of *N*-[cis-4-(4-dimethylamino-5-methyl-pyrimidin-2-ylamino)-cyclohexyl]-bromoacetamide.**

To a solution of *cis*-4-(4-dimethylamino-5-methyl-pyrimidin-2-ylamino)-1-aminocyclohexane (150 mg, 0.6 mmol) in DCM (10 mL) was added dropwise bromacetylbromide (120 mg, I eq.) at 0 °C under an inert atmosphere. After 5 min stirring, DIEA (0.1 mL, 1 eq.) was added into the reaction. The reaction was stirred for an additional 3 h at below 15 °C, quenched, and purified by column chromatography.
0.12 g (55 %) of the product was isolated.

**Step B: Synthesis of *N*-(*cis*-4-{[4-(dimethylamino)-5-methylpyrimidin-2-yl]amino}-cyclohexyl)-2-(3-fluorophenoxy)acetamide.**

A sealed tube containing a heterogenous solution of N-[cis-4-(4-dimethylamino-5-methyl-pyrimidin-2-ylamino)-cyclohexyl]-bromoacetamide (30 mg, 0.08 mmol), 3-fluorophenol (27 mg, 3 eq.), and $Cs_2CO_3$ (30 mg, 1.1 eq.) in dioxane (~0.7 mL) was reacted in a Smith microwave synthesizer for 3000 sec at 180 °C. The reaction was diluted with DCM, washed with sat.-$NaHCO_3$ (2 x) and water (1 x), concentrated, and purified by column chromatography to give 11 mg (34 %) of the product.
ESI MS m/e 402 M + $H^+$; $^1H$ NMR (400 MHz, $CDCl_3$) δ 7.58 (s, 1 H), 7.26 (m, 1 H), 6.74~6.63 (m, 3 H), 6.51 (d, 1 H, J = 8.0 Hz), 5.15 (bs, 1 H), 4.45 (s, 2 H), 4.01 (m, 1 H), 3.97 (bs, 1 H), 3.05 (s, 6 H), 2.15 (s, 3 H), 1.82~1.61 (m, 8 H).

**Example 2639**

**2-[(5-Chloropyridin-3-yl)oxy]-*N*-(*cis*-4-{[4-(dimethylamino)-5-methylpyrimidin-2-yl]amino}cyclohexyl) acetamide**

**Step A: Synthesis of 2-[(5-chloropyridin-3-yl)oxy]-*N*-(*cis*-4-{[4-(dimethylamino)-5-methylpyrimidin-2-yl]amino} cyclohexyl)acetamide.**

Using the procedure of Example 2638, the title compound was obtained. ESI MS m/e 419 M + $H^+$; $^1H$ NMR (400 MHz, $CDCl_3$) δ 8.25 (m, 2 H), 7.53 (s, 1 H), 7.27 (t, 1 H, J = 2.4 Hz), 6.56 (d, 1 H, J = 7.6 Hz), 5.57 (bs, 1 H), 4.50 (s, 2 H), 4.01 (bs, 2 H), 3.08 (s, 6 H), 2.16 (s, 3 H), 1.83~1.64 (m, 8 H).

**Example 2640**

***N*-(*cis*-4-{[4-(Dimethylamino)-5-ethylpyrimidin-2-yl]amino}cyclohexyl)-3,4-difluorobenzamide**

**Step A: Synthesis of 2,4-dichloro-5-ethylpyrimidine.**

To a suspension of 5-ethyluracil (1 g, 7.1 mmol) in $POCl_3$ (4.5 mL) was slowly added *N,N*-dimethylaniline (1 mL). The reaction was heated at reflux (~120 °C) for 5 h until the starting material was completely dissolved and the entire solution turned a purple color. The mixture was allowed to cool and poured very slowly into ice (~40 g). The resulting ppt was filtered and washed with ice water. The ppt was dissolved with a minimal amount of DCM and poured onto a short column of silica gel, and the product (1.2 g, ~ 100 %) was obtained by column chromatography with DCM. $^1H$ NMR (400 MHz, $CDCl_3$) δ 8.42 (s, 1 H), 2.75 (q, 2 H, J = 7.6 Hz), 1.29 (t, 3 H, *J* = 7.6 Hz).

**Step B: Synthesis of *N*-(cis-4-1{[4-(dimethylamino)-5-ethylpyrimidin-2-yl]amino)cyclohexyl)-3,4-difluorobenzamide.**

A solution of 2,4-dichloro-5-ethylpyrimidine (1.2 g, 6.8 mmol), in THF (15 mL) was cooled to 5 °C in an ice bath, and 2M-dimethylamine (7 mL, 2 eq.) was slowly added. The reaction was stirred for 2 h at around 10 °C, and the volatile solvent was removed. The residue was purified by column chromatography (hexane:DCM = 50:50 to 10:90) to give 0.89 g (70 %) of 2-chloro-4-dimethylamino-5-ethylpyrimidine: ESI MS m/e = 186 M + $H^+$.

A sealed tube containing 2-chloro-4-dimethylamino-5-ethylpyrimidine (35 mg, 0.019 mmol), *cis*-(4-amino-cyclohexyl)-3,4-difluoro-benzamide (48 mg, 1 eq.), DIEA (50 mg, 2 eq.), and IPA (1 mL) was reacted in a Smith microwave synthesizer for 2 h at 180 °C. The reaction was diluted with DCM, washed with 1-N HCl and water, concentrated, and purified from column chromatography (DCM:MeOH = 100:0 to 95:5) to give 11 mg (14 %) of the product.
ESI MS m/e 404 M + $H^+$; $^1H$ NMR (400 MHz, $CDCl_3$) δ 7.68 (s, 1 H), 7.61 (m, 1 H), 7.48 (m, 1 H), 7.19 (m, 1 H), 5.99 (d, 1 H, J = 7.2 Hz), 4.38 (d, 1 H, J = 6.0 Hz), 4.20 (m, I H), 4.12 (m, 1 H), 3.10 (s, 6 H), 2.29 (q, 2 H, J = 7.2 Hz), 1.96~1.64 (m, 8 H), 1.18 (t, 3 H, J = 7.6 Hz).

**Example 2641**

***N*-[*cis-4*-({4-[Ethyl(methyl)amino]-5-methylpyrimidin-2-yl}amino)cyclohexyl]-3,4-difluorobenzamide hydrochloride**

**Step A: Synthesis of N [cis-4-({4-[ethyl(methyl)amino]-5-methylpyrimidin-2-yl}amino)cyclohexyl]-3,4-difluorobenzamide hydrochloride.**

A solution of 2,4-dichloro-5-methylpyrimidine (2.6 g, 16 mmol) and ethyl methylamine (2.7 mL, 2 eq.) in THF (20 mL) was stirred at < 10 °C for 4 h. After removal of the volatile solvent, the residue was purified by column chromatography. 1.3 g (45 %) of 2-chloro-4-(ethyl-methyl-amino)-5-methylpyrimidine was isolated.
ESI MS m/e 186 M + $H^+$.

A sealed tube containing 2-chloro-4-(ethyl-methyl-amino)-5-methylpyrimidine (80 mg, 0.019 mmol), *cis*-(4-amino-cyclohexyl)-3,4-difluoro-benzamide (100 mg, 1 eg.), DIEA (0.14 mL, 2 eq.), and IPA (1 mL) was reacted in a Smith microwave synthesizer for 2 h at 180 °C. The reaction was diluted with DCM, washed with 1-N HCl and water, concentrated, and purified by column chromatography (DCM:MeOH = 100:0 to 95:5) to give 35 mg (20 %) of the product, which was converted to HCl salt.
ESI MS m/e 404 M + $H^+$; $^1H$ NMR (400 MHz, DMSO-$d_6$) δ 12.0 (bs, 1 H), 8.36 (bs, 1 H), 7.97 (d, 1 H, J = 6.0 Hz), 7.90 (m, 1 H), 7.73 (m, 1 H), 7.63 (s, 1 H), 7.51 (m, 1 H), 3.85 (bm, 2 H), 3.65 (q, 2 H, *J* = 7.2 Hz), 3.25 (s, 3 H), 2.22 (s, 3 H), 1.84 (m, 2 H), 1.69 (m, 6 H), 1.18 (t, 3 H, *J* = 7.2 Hz).

**Example 2642**

***N-(cis-4-*{[4-(Dimethylamino)-5-(trifluoromethyl)pyrimidin-2-yl]amino}cyclohexy 1)-3,5-bis(trifluoromethyl) benzamide trifluoroacetate**

**Step A: Synthesis of 2-chloro-4-dimethylamino-S-trifluoromethylpyrimidine.**

To a solution of 2,4-dichloro-5-trifluoromethylpyrimidine (1 g, 4.6 mmol) in THF (15 mL) was added 2M-dimethylamine (4.6 mL, 2 eq.) at 0 °C. The reaction was stirred for an additional 1.5 h at < 5 °C, concentrated, and purified

by column chromatography (DCM:hexane:MeOH = 90:10:0 to 95:0:5). 0.49 g (47 %) of 2-chloro-4-dimethylamino-5-trifluoromethylpyrimidine was isolated.
ESI MS m/e 226 M + H+; $^1$H NMR (400 MHz, $CDCl_3$) δ 8.36 (s, 1 H), 3.21 (s, 6 H).

**Step B: Synthesis of *cis*-[4-(4-Dimethylamino-5-trifluoromethyl-pyrimidin-2-ylamino)-cyclohexyl]-carbamic acid tert-butyl ester.**

A sealed tube containing 2-chloro-4-dimethylamino-5-trifluoromethylpyrimidine (0.49 g, 2.0 mmol), *cis*-(4-amino-cyclohexyl)-carbamic acid tert-butyl ester (0.47 g, 1 eq.), DIEA (0.7 mL, 2 eq.) in IPA (2.5 mL) was reacted in a Smith microwave synthesizer for 2 h at 175 °C. The solution was concentrated and purified by column chromatography (DCM:MeOH = 100:0 to 96:4). 0.57 g (65 %) of cis-[4-(4-dimethylamino-5-trifluoromethyl-pyrimidin-2-ylamino)-cyclohexyl]-carbamic acid tert-butyl ester was isolated.
ESI MS m/e 404 M + H+; $^1$H NMR (400 MHz, $CDCl_3$) δ 8.15 (s, 1 H), 5.10 (bs, 1 H), 4.53 (bs, 1 H), 3.94 (bs, 1 H), 3.61 (bs, 1 H), 3.09 (s, 6 H), 1.78~1.49 (m, 8 H), 1.44 (s, 9 H).

**Step C: Synthesis of cis-*N*-(4-dimethylamino-5-trifluoromethyl-pyrimidin-2-yl)-cyclohexane-1,4-diamine.**

To a solution of cis-[4-(4-dimethylamino-5-trifluoromethyl-pyrimidin-2-ylamino)-cyclohexyl]-carbamic acid tert-butyl ester (0.55g, 1.3 mmol) in DCM (10 mL) was added TFA (7 mL). The reaction was stirred at room temperature for 2 h and concentrated. The residue was neutralized with sat-NaOH, and the aqueous layer was extracted with DCM (3 x). The combined organic layers were washed with water, dried, and concentrated to give 0.25 g (65 %) of *cis-N-(4-*dimethylamino-5-trifluoromethyl-pyrimidin-2-yl)-cyclohexane-1,4-diamine.
ESI MS m/e 304 M + H+; $^1$H NMR (400 MHz, $CDCl_3$) δ 8.16 (s, 1 H), 5.42 (bs, 1 H), 3.98 (bs, 1 H), 3.09 (s, 6 H), 2.87 (bs, 1 H), 1.81 (m, 2 H), 1.73~1.65 (m, 4 H), 1.43 (m, 4 H).

**Step D: Synthesis of *N*-(*cis*-4-{[4-(dimethylamino)-5-(trifluoromethyl)pyrimidin-2-yl]amino}cyclohexyl)-3,5-bis (trifluoromethyl)benzamide trifluoroacetate.**

To a solution of *cis*-*N*-(4-dimethylamino-5-trifluoromethyl-pyrimidin-2-yl)-cyclohexane-1,4-diamine (30 mg, 0.01 mmol) in dry benzene (2 mL) was added 3,5-bistrifluoromethyl benzoyl chloride (27 mg, 1 eq.) and followed by $Et_3N$ (20 μL, 2.5 eq). The reaction was stirred overnight, concentrated, and purified by prep-HPLC. 32 mg (49 %) of N-(*cis*-4-{[4-(dimethylamino)-5-(trifluoromethyl)pyrimidin-2-yl]amino}cyclohexyl)-3,5-bis(trifluoromethyl)benzamide trifluoroacetate was isolated as a white powder.
ESI MS m/e 544 M + H+; $^1$H NMR (400 MHz, $CDCl_3$) δ 9.35 (d, 1 H, J = 8.0 Hz), 8.47 (s, 1 H), 8.32 (s, 2 H), 8.07 (s, 1 H), 7.61 (d, 1 H, J = 8.4 Hz), 4.31 (bs, 1 H), 4.20 (bs, 1 H), 3.33 (s, 6 H), 1.93~1.79 (m, 8 H.

**Example 2643**

***N*-(*cis*-4-{[4-(Dimethylamino)-5-(trifluoromethyl)pyrimidin-2-yl]amino}cyclohexyl)-4-(trifluoromethoxy) benzamide trifluoroacetate**

**Step A: Synthesis of *N*-(cis-4-{[4-(dimethylamino)-5-(trifluoromethyl)pyrimidin-2-yl]amino}cyclohexyl)-4-(trifluoromethoxy)benzamide trifluoroacetate.**

Using the procedure of Example 2642, the title compound was obtained.
ESI MS m/e 492 M + H+; $^1$H NMR (400 MHz, $CDCl_3$) δ 9.45 (d, 1 H, J = 8.0 Hz), 8.05 (s, 1 H), 7.88 (d, 2 H, J= 8.8 Hz), 7.24 (m, 2 H, overlapped with solvent), 7.04 (d, 1 H, J = 8.4 Hz), 4.27 (bs, 1 H), 4.18 (bs, 1 H), 3.31 (s, 6 H), 1.89~1.77 (m, 8 H).

**Example 2644**

***N*-(*cis*-4-{[4-(Dimethylamino)-5-methylpyrimidin-2-yl]amino}cyclohexyl)-2-{[3-(trifluoromethyl)phenyl] sulfinyl}acetamide hydrochloride**

**Step A: Synthesis of (3-trifluoromethyl-phenylsulfanyl)-acetic acid ethyl ester.**

A solution of ethyl bromoacetate (0.65g, 3.2 mmol), 3-trifluoromethyl thiophenol (0.88 g, 1.5 eq.), and $Et_3N$ (1.5 mL) in THF (15 mL) was stirred for 2 h at 62 °C. The mixture was diluted with DCM, washed with sat.-$NaHCO_3$

(3x) and water, dried with $MgSO_4$, and concentrated. The crude product (0.73 g, 85 %) was used to next reaction without a further purification.
$^1$H NMR (400 MHz, $CDCl_3$) δ 7.62 (s, 1 H), 7.55 (d, 1 H, J = 8.0 Hz), 7.46~7.37 (m, 2 H), 4.16 (q, 2 H, J = 7.2 Hz), 3.66 (s, 2 H), 1.22 (t, 3 H, J = 7.2 Hz).

**Step B: Synthesis of (3-trifluoromethyl-phenylsulfinyl)-acetic acid ethyl ester.**

To a solution of (3-trifluoromethyl-phenylsulfanyl)-acetic acid ethyl ester (0.5 g, 1.9 mmol) in DCM (10 mL) was added 77 %-MCPBA (0.42 g, I eq.) under Ar atmosphere at 0 °C. The reaction was stirred for an additional 3 h, diluted with DCM, washed with sat-$NaHCO_3$ and water, and concentrated. (3-trifluoromethyl-phenylsulfinyl)-acetic acid ethyl ester (0.34 g, 64 %) and (3-trifluoromethyl-phenylsulfonyl)-acetic acid ethyl ester (0.15 g, 27 %) were isolated by column chromatography (hexane:EtOAc = 95:5 to 80:20).
(3-Trifluoromethyl-phenylsulfinyl)-acetic acid ethyl ester:
$^1$H NMR (400 MHz, $CDCl_3$) δ 7.95 (s, 1H), 7.87 (d, 1H, J = 8.0 Hz), 7.78 (d, 1 H, J = 8.0 Hz), 7.67 (t, 1 H, *J* = 8.0 Hz), 4.15 (q, 2 H, *J* = 7.2 Hz), 3.86 (d, 1 H, *J* = 14.0 Hz), 3.70 (d, 1 H, *J* = 14.0 Hz), 1.22 (t, 3 H, *J* = 7.2 Hz).
(3-Trifluoromethyl-phenylsulfonyl)-acetic acid ethyl ester:
$^1$H NMR (400 MHz, $CDCl_3$) δ 8.20 (s, 1 H), 8.14 (d, 1 H, J = 7.6 Hz), 7.94 (d, 1 H, J = 7.6 Hz), 7.74 (t, 1 H, *J* = 7.6 Hz), 4.15 (s, 2 H), 4.14 (q, 2 H, J = 7.6 Hz), 1.20 (t, 3 H, *J* = 7.2 Hz).

**Step C: Synthesis of (3-trifluoromethyl-phenylsulfinyl)-acetic acid.**

To a heterogenous solution of (3-trifluoromethyl-phenylsulfinyl)-acetic acid ethyl ester (0.2 g, 0.7 mmol) in $H_2O$ (5 mL)/EtOH (0.5 mL) was added KOH (120 mg, 3 eq.). The reaction was stirred for 2 h at 85 °C, concentrated to about half of the reaction volume, and acidified with conc-HCl at an ice bath. (3-Trifluoromethyl-phenylsulfinyl)-acetic acid (100 mg, 56 %) was filtered and dried.
$^1$H NMR (400 MHz, DMSO-$d_6$) δ 8.05 (s, 1 H), 8.01 (d, 1 H, J = 8.0 Hz), 7.92 (d, 1 H, J = 8.0 Hz), 7.81 (t, 1 H, *J* = 8.0 Hz), 4.16 (d, 1 H, J = 14.4 Hz), 3.87 (d, 1 H, J = 14.4 Hz).

**Step D: Synthesis of *N*-(*cis*-4-{[4-(dimethylamino)-5-methylpyrimidin-2-yl]amino}-cyclohexyl)-2-{[3-(trifluoromethyl)phenyl]sulfinyl}acetamide hydrochloride.**

To a solution of *cis*-4-(4-dimethylamino-5-methyl-pyrimidin-2-ylamino)-1-aminocyclohexane (60 mg, 0.024 mmol) in DCM (5 mL) was added (3-trifluoromethyl-phenylsulfinyl)-acetic acid (60 mg, 1 eq.), followed by HATU (85 mg, 1.1 eg.), and $Et_3N$ (30 μL). The reaction was stirred for 16 h at room temperature and concentrated. The residue was purified by column chromatography to give *N*-(*cis*-4-{[4-(dimethylamino)-5-methylpyrimidin-2-y)]amino}cyclohexyl)-2-{[3-(trif!uoromethy))pheny)]su)finy)} acetamide (52 mg, 45%), which was converted to HCl salt with 4M-HCl in dioxane.
ESI MS m/e 484 M + H$^+$; $^1$H NMR (400 MHz, DMSO-$d_6$) δ 11.7 (bs, 1 H), 8.08 (d, 1 H, J = 6.4 Hz), 7.99 (m, 2 H), 7.92 (d, 1 H, J= 8.0 Hz), 7.90 (bs, 1 H), 7.82 (t, 1 H, J= 8.0 Hz), 7.59 (s, 1 H), 3.94 (d, 1 H, J = 12.8 Hz), 3.86 (d, 1 H, J = 12.8 Hz), 3.80 (bs, 1 H), 3.68 (bs, 1 H), 3.25 (s, 6 H), 2.23 (s, 3 H), 1.70~1.50 (m, 8 H).

**Example 2645**

**2-[(3,4-Dichlorophenyl)sulfinyl]-*N*-(*cis*-4-{[4-(dimethylamino)-5-methylpyrimidin-2-yl]amino}cyclohexyl) acetamide hydrochloride**

**Step A: Synthesis of 2-[(3,4-dichlorophenyl)sulfinyl]-N-(cis-4-{[4-(dimethyl amino)-5-methylpyrimidin-2-yl] amino}cyclohexyl)acetamide hydrochloride.**

Using the procedure of Example 2644, the title compound was obtained.
ESI MS m/e 484 M + H$^+$; $^1$H NMR (400 MHz, DMSO-$d_6$) δ 11.9 (bs, 1H), 8.13 (d, 1H, *J*= 6.8 Hz), 7.98 (bs, 1H), 7.87 (s, 1H), 7.86 (d, 1H, *J*= 8.8 Hz), 7.65 (d, 1H, *J* = 8.8 Hz), 7.61 (bs, 1H), 3.93 (d, 1H, *J* = 12.8 Hz), 3.87 (d, 1H, *J* = 12.8 Hz), 3.81 (bs, 1H), 3.64 (bs, 1H), 3.25 (s, 6 H), 2.23 (s, 3 H), 1.70~1.50 (m, 8 H).

**Example 2646**

***N-(cis-4-*{[4-(Dimethylamino)-5-methylpyrimidin-2-yl]amino}cyclohexyl)-2-{[3-(trifluoromethyl)phenyl] sulfonyl}acetamide hydrochloride**

**Step A: Synthesis of *N*-(cis-4-{[4-(dimethylamino)-5-methylpyrimidin-2-yl]amino}- cyclohexyl)-2-{ [3-(trifluoromethyl]phenyl]sulfonyl]acetamide hydrochloride.**

(3-trifluoromethyl-phenylsulfonyl)-acetic acid ethyl ester was obtained from step B in Example 2644. The ester was hydrolyzed to (3-trifluoromethyl-phenylsulfonyl)-acetic acid using the procedure of step C in Example 2644. $^1$H NMR (400 MHz, *DMSO-$d_6$)* δ 8.22 (d, 1 H, J = 8.0 Hz), 8.21 (s, 1 H), 8.14 (d, 1 H, J= 8.0 Hz), 7.90 (t, 1 H, J = 8.0 Hz), 4.69 (s, 2 H).

To a solution of *cis*-4-(4-dimethylamino-5-methyl-pyrimidin-2-ylamino)-1-aminocyclohexane (56 mg, 0.023 mmol) in DCM (5 mL) was added (3-trifluoromethyl-phenylsulfonyl)-acetic acid (60 mg, 1 eq.), followed by HATU (85 mg, 1.1 eq.), and $Et_3N$ (30 μL). The reaction was stirred for 16 h at room temperature and concentrated. The residue was purified by column chromatography to give *N*-(*cis*-4-{[4-(dimethylamino)-5-methylpyrimidin-2-yl]amino}cyclohexyl)-2-{[3-(trifluoromethyl)phenyl] sulfonyl}acetamide (50 mg, 45%), which was converted to HCl salt with 4M HCl in dioxane.
ESI MS m/e 500 M + H$^+$; $^1$H NMR (400 MHz, DMSO-$d_6$) δ 11.6 (bs, 1 H), 8.22 (d, 1 H, J = 6.4 Hz), 8.17~8.12 (m, 3 H), 7.90 (t, 1 H, J = 7.6 Hz), 7.87 (bs, 1 H), 7.57 (s, 1 H), 4.45 (s, 2 H), 3.79 (bs, 1 H), 3.61 (bs, 1 H), 3.25 (s, 6 H), 2.23 (s, 3 H), 1.70～1.47 (m, 8 H).

**Example 2647**

***N*-(*cis*-4-{[4-(Dimethylamino)-5-methylpyrimidin-2-yl]amino)cyclohexyl)-2-(4-fluorophenoxy)nicotinamide hydrochloride**

**Step A: Synthesis of 2-chloro-*N*-[*cis*-4-(4-dimethylamino-5-methyl-pyrimidin-2-ylamino)-cyclohexyl]-nicotinamide.**

To a solution of *cis*-4-(4-dimethylamino-5-methyl-pyrimidin-2-ylamino)-1-aminocyclohexane (0.6 g, 2.4 mmol) in DCM (20 mL) was added 2-chloronicotinoyl chloride (0.44 g, 1.01 eq.) and followed by DIEA (0.4 mL, ~ 1.1 eq.). The reaction was stirred overnight at room temperature, washed with sat-$NaHCO_3$ (2x) and water (1x), dried with $MgSO_4$, and concentrated. The crude residue was purified by column chromatography to give 2-chloro-N-[cis-4-(4-dimethylamino-5-methyl-pyrimidin-2-ylamino)-cyclohexyl]-nicotinamide (0.57 g, 65 %).
ESI MS m/e 389 M + H$^+$; $^1$H NMR (400 MHz, $CDCl_3$) δ 8.72 (bs, 1 H), 8.47 (d, 1 H, J = 5.0 Hz), 7.98 (d, 1 H, J = 7.0 Hz), 7.32 (dd, 1 H, J= 8.0 and 5.0 Hz), 7.28 (s, 1 H), 6.88 (d, 1 H, J = 8.0 Hz), 4.18 (m, 2 H), 3.27 (s, 6 H), 2.23 (s, 3 H), 1.90~1.80 (m, 8 H).

**Step B: Synthesis of *N*-(*cis*-4-{[4-(dimethylamino)-5-methylpyrimidin-2-yl]aminol-cyclohexyl)-2-(4-fluorophenoxy)nicotinamide hydrochloride.**

A sealed tube containing 2-chloro-N-[cis-4-(4-dimethylamino-5-methyl-pyrimidin-2-ylamino)-cyclohexyl]-nicotinamide (0.35 g, 0.9 mmol), 4-fluorophenol (0.25 g, 2.5 eq.), $Cs_2CO_3$ (0.33 g, 1.1 eq.), and dioxane (3 mL) was reacted in a Smith microwave synthesizer for 1 h at 180 °C. The reaction was diluted with DCM, washed with sat-$NaHCO_3$ (3x) and water (1x), dried, and concentrated. The residue was purified by column chromatography (DCM: MeOH = 100:0 to 95:5) to give *N*-[*cis*-4-(4-dimethylamino-5-methyl-pyrimidin-2-ylamino)-cyclohexyl]-2-(4-fluoro-phenoxy)-nicotinamide (0.33 g, 80 %). The neutral compound was dissolved in DCM (5 mL), and 4M-HCl (0.45 mL, 2.5 eq.) in dioxane was added. After 20 min stirring, removal of the volatile solvent gave *N- (cis-4-*{[4-(dimethylamino)-5-methylpyrimidin-2-yl]amino}cyclohexyl)-2-(4-fluorophenoxy)nicotinamide hydrochloride.
ESI MS m/e 465 M + H$^+$; $^1$H NMR (400 MHz, DMSO-$d_6$) δ 12.1 (bs, 1 H), 8.34 (d, 1H, *J* = 7.2 Hz), 8.15 (dd, 1 H, J = 5.2 and 2.0 Hz), 8.06 (d, 1 H, J = 6.8 Hz), 8.01 (d, 1 H, J = 7.6 Hz), 7.63 (s, 1 H), 7.26～7.18 (m, 5 H), 3.94 (bs, 1 H), 3.88 (bs, 1 H), 3.25 (s, 6 H), 2.21 (s, 3 H), 1.72 (bs, 8 H).

**Example 2648**

**2-(2-Bromophenoxy)-*N*-(cis-4-{[4-(dimethylamino)-5-methylpyrimidin-2 yl]amino}cyclohexyl)nicotinamide hydrochloride**

**Step A: Synthesis of 2-(2-bromophenoxy)-*N*-(*cis*-4-([4-(dimethylamino)-5-methylpyrimidin-2-yl]amino} cyclohexyl)nicotinamide hydrochloride.**

Using the procedure of Example 2647, the title compound was obtained.
ESI MS m/e 525 M + $H^+$; $^1$H NMR (400 MHz, DMSO-$d_6$) δ 11.8 (bs, 1 H), 8.20 (d, 1 H, J = 7.6 Hz), 8.16~8.11 (m, 2 H), 7.96 (bs, 1 H), 7.70 (dd, 1 H, J = 8.0 and 1.6 Hz), 7.60 (s, 1 H), 7.47~7.38 (m, 2 H), 7.25~7.19 (m, 2 H), 3.97 (bs, 1 H), 3.89 (bs, 1 H), 3.24 (s, 6 H), 2.22 (s, 3 H), 1.74 (bs, 8 H).

**Example 2649**

**2-(4-Bromophenoxy)-*N*-(*cis*-4-{[4-(dimethylamino)-5-methylpyrimidin-2-yl]amino}cyclohexyl)nicotinamide hydrochloride**

**Step A: Synthesis of 2-(4-bromophenoxy)-*N*-(*cis*-4-{[4-(dimethylamino)-5-methylpyrimidin-2-yl]amino} cyclohexyl)nicotinamide hydrochloride.**

Using the procedure of Example 2647, the title compound was obtained.
ESI MS m/e 525 M + $H^+$; $^1$H NMR (400 MHz, DMSO-$d_6$) δ 11.9 (bs, 1 H), 8.28 (d, 1 H, J = 7.0 Hz), 8.12 (dd, 1 H, J = 4.4 and 1.6 Hz), 7.97 (d, 1 H, J = 7.6 Hz), 7.91 (bs, 1 H), 7.56 (bs, 1 H), 7.54 (d, 2 H, J = 8.8 Hz), 7.17 (m, 1 H), 7.14 (d, 2 H, J = 8.8 Hz), 3.87 (bs, 1 H), 3.81 (bs, 1 H), 3.19 (s, 6 H), 2.16 (s, 3 H), 1.65 (bs, 8 H).

**Example 2650**

**2-(4-Chlorophenoxy)-*N*-(*cis*-4-{[4-(dimethylamino)-5-methylpyrimidin-2-yl]amino}cyclohexyl)nicotinamide hydrochloride**

**Step A: Synthesis of 2-(4-chlorophenoxy)-*N*-(*cis*-4-{[4-(dimethylamino)-5-methylpyrimidin-2-yl]amino} cyclohexyl)nicotinamide hydrochloride.**

Using the procedure of Example 2647, the title compound was obtained. ESI MS m/e 481 M + $H^+$; $^1$H NMR (400 MHz, DMSO-$d_6$) δ 11.8 (bs, 1 H), 8.27 (d, I H, J = 6.6 Hz), 8.12 (dd, 1 H, J = 4.8 and 1.6 Hz), 7.97 (dd, 1 H, *J* = 7.0 and 1.6 Hz), 7.86 (bs, 1 H), 7.55 (s, 1 H), 7.41 (d, 2 H, *J* = 8.8 Hz), 7.20 (d, 2 H, *J* = 8.8 Hz), 7.17 (m, 1 H), 3.88 (bs, 1 H), 3.81 (bs, 1 H), 3.19 (s, 6 H), 2.16 (s, 3 H), 1.65 (bs, 8 H).

**Example 2651**

**2-[(5-chloropyridin-3-yl)oxy]-*N*-(cis-4-{[4-(dimethylamino)-5-methylpyrimidin-2-yl]amino}cyclohexyl) nicotinamide hydrochloride**

**Step A: Synthesis of 2-[(5-chloropyridin-3-yl)oxy]-*N*-(cis-4-{[4-(dimethylamino)-5-methylpyrimidin-2-yl]amino} cyclohexyl)nicotinamide hydrochloride.**

Using the procedure of Example 2647, the title compound was obtained.
ESI MS m/e 482 M + $H^+$; $^1$H NMR (400 MHz, DMSO-$d_6$) δ 11.6 (bs, 1 H), 8.46 (s, 1 H), 8.31 (d, 1 H, J = 1.6 Hz), 8.01 (bm, 1 H), 7.83 (t, 1 H, J = 2.0 Hz), 7.56 (d, 1 H, *J* = 5.2 Hz), 7.49 (bm, 1 H), 7.25 (bs, 1 H), 6.07 (bs, 1 H), 5.74 (s, 1 H), 4.51 (bs, 1 H), 4.00 (bs, 1 H), 3.23 (s, 6 H), 2.19 (s, 3 H), 1.90 (m, 2 H), 1.75 (m, 4 H), 1.39 (m, 2 H).

**Example 2652**

**2-(*tert*-butylthio)-*N*-(*cis*-4-{[4-(dimethylamino)-5-methylpyrimidin-2-yl]amino]-cyclohexyl)nicotinamide hydrochloride**

**Step A: Synthesis of 2-(*tert*-butylthio)-*N*-(*cis*-4-{[4-(dimethylamino)-5-methylpyrimidin-2-yl]amino)cyclohexyl) nicotinamide hydrochloride.**

A sealed tube containing 2-chloro-*N*-[*cis*-4-(4-dimethylamino-5-methyl-pyrimidin-2-ylamino)-cyclohexyl]-nicotinamide (70 mg, 0.018 mmol), 2-methyl-2-propanethiol (80 mg, 5 eq.), $Cs_2CO_3$ (60 mg, 1.1 eq) in dioxane (0.8 mL) was reacted in a Smith microwave synthesizer for 1.5 h at 180 °C. The reaction was diluted with DCM, washed with sat-$NaHCO_3$ (3x) and water (1x), dried, and concentrated. The residue was purified by column chromatography (DCM:MeOH = 100:0 to 95:5) to give 2-(*tert*-butylthio)-*N*-(*cis*-4-{[4-(dimethylamino)-5-methylpyrimidin-2-yl]amino}cyclohexyl)nicotinamide (50 mg, 62 %), which was converted to HCl salt.
ESI MS m/e 443 M + $H^+$; $^1H$ NMR (400 MHz, DMSO-$d_6$) δ 12.2 (bs, 1 H), 8.47 (dd, 1 H, J = 4.8 and 1.6 Hz), 8.40 (d, 1 H, J = 6.0 Hz), 8.00 (bm, 1 H), 7.62 (s, 1 H), 7.56 (dd, 1 H, J = 7.6 and 1.6 Hz), 7.15 (m, 1 H), 3.90 (bs, 2 H), 3.25 (s, 6 H), 2.21 (s, 3 H), 1.80~1.65 (m, 8 H), 1.49 (s, 9 H).

**Example 2653**

**N-(*cis*-4-{[4-(dimethylamino)-5-methylpyrimidin-2-yl]amino}cyclohexyl)-2-(propylthio)nicotinamide hydrochloride**

**Step A: Synthesis of *N*-(*cis*-4-{[4-(dimethylamino)-5-methylpyrimidin-2-yl]amino}cyclohexyl)-2-(propylthio) nicotinamide hydrochloride.**

Using the procedure of Example 2652, the title compound was obtained.
ESI MS m/e 429 M + $H^+$; $^1H$ NMR (400 MHz, DMSO-$d_6$) δ 12.4 (bs, 1 H), 8.44 (m, 2 H), 8.04 (d, 1 H, *J* = 6.8 Hz), 7.63 (d, 2 H, *J* = 6.4 Hz), 7.12 (m, 1 H), 3.85 (bs, 2 H), 3.24 (s, 6 H), 3.06 (t, 2 H, *J* = 6.8 Hz), 2.21 (s, 3 H), 1.83~1.65 (m, 8 H), 1.62 (m, 2 H), 0.95 (t, 3 H, *J* = 7.2 Hz).

**Example 2654**

***N-(cis*-4-{[4-(Dimethylamino)-5-methylpyrimidin-2-yl] amino}cyclohexyl)-2-(isopropylthio)nicotinamide hydrochloride**

**Step A: Synthesis of *N*-(*cis*-4-{[4-(dimethylamino)-5-methylpyrimidin-2-yl]amino}-cyclohexyl)-2-(isopropylthio)nicotinamide hydrochloride.**

Using the procedure of Example 2652, the title compound was obtained.
ESI MS m/e 429 M + $H^+$; $^1H$ NMR (400 MHz, DMSO-$d_6$) δ 12.2 (bs, 1 H), 8.46 (dd, 1 H, J = 4.8 and 1.6 Hz), 8.42 (bs, 1 H), 8.02 (d, 1 H, J= 6.4 Hz), 7.62 (m, 2 H), 7.12 (m, 1 H), 3.95 (sept, 1 H, *J* = 6.4 Hz), 3.83 (bs, 2 H), 3.25 (s, 6 H), 2.21 (s, 3H), 1.82~1.65 (m, 8 H), 1.30 (d, 6 H, *J* = 6.8 Hz).

**Example 2655**

**2-(*tert*-Butylsulfinyl)-*N*-(*cis*-4-{[4-(dimethylamino)-5-methylpyrimidin-2-yl]amino) cyclohexyl)nicotinamide**

**Step A: Synthesis of 2-(*tert*-butylsulfinyl)-*N*-(cis-4-{[4-(dimethylamino)-S-methylpyrimidin-2-yl)amino) cyclohexyl)nicotinamide.**

To a solution of *N*-[*cis*-4-(4-dimethylamino-5-methyl-pyrimidin-2-ylamino)-cyclohexyl]-2-tert-butyl sulfanyl-nicotinamide (30 mg, 0.07 mmol) in DCM (5 mL) was added MCPBA (16 mg, 1.1 eq) at 0 °C. The reaction was stirred for an additional 2 h at < 10 °C with monitoring the progress by ESI MS. The reaction was diluted with DCM, washed with sat.-$NaHCO_3$ (2x) and water (1x), dried, concentrated, and purified by column chromatography (DCM:MeOH = 100:0 to 94:6). 26 mg (85 %) of 2-(*tert*-butylsulfinyl)-*N*-(*cis*-4-{[4-(dimethylamino)-5-methylpyrimidin-2-yl]amino}cyclohexyl)nicotinamide was isolated.
ESI MS m/e 459 M + $H^+$; $^1H$ NMR (400 MHz, $CDCl_3$) δ 8.71 (dd, 1 H, J= 4.8 and 1.6 Hz), 8.54 (d, 1 H, *J* = 6.8 Hz),

8.20 (d, 1 H, J = 8.0 Hz), 7.61 (s, 1 H), 7.43 (dd, 1 H, *J* = 8.0 and 4.0 Hz), 5.03 (d, 1 H, J = 6.0 Hz), 4.12 (bs, 1 H), 3.98 (bs, 1 H), 2.99 (s, 6 H), 2.12 (s, 3 H), 1.87～1.75 (m, 8 H), 1.23 (s, 9 H).

**Example 2656**

**2-[(3,4-Difluorophenyl)sulfonyl]-N-(cis-4-{[4-(dimethylamino)-5-methylpyrimidin-2-yl]amino)cyclohexyl) nicotinamide hydrochloride**

**Step A: Synthesis of N-[cis-4-(4-dimethylamino-5-methyl-pyrimidin-2-ylamino)-cyclohexyl]-2-(3,4-difluorophenyl)-sulfanyl-nicotinamide.**

A sealed tube containing 2-chloro-*N*-[*cis*-4-(4-dimethylamino-5-methyl-pyrimidin-2-ylamino)-cyclohexyl]-nicotinamide (100 mg, 0.025 mmol), 3,4-difluorothiophenol (90 mg, 2.5 eq.), $Cs_2CO_3$ (150 mg, 2 eq), and dioxane (2 mL) was reacted in a Smith microwave synthesizer for 1.0 h at 180 °C. The reaction was diluted with DCM, washed with sat-$NaHCO_3$ (3x) and water (1x), dried, and concentrated. The residue was purified by column chromatography (DCM:MeOH = 100:0 to 95:5) to give N-[cis-4-(4-dimethylamino-5-methyl-pyrimidin-2-ylamino)-cyclohexyl]-2-(3,4-difluorophenyl)-sulfanyl-nicotinamide (70 mg, 55 %).
ESI MS m/e 499 M + $H^+$; $^1H$ NMR (400 MHz, $CDCl_3$) δ 8.34 (dd, 1H, *J* = 4.8 and 1.6 Hz), 7.79 (dd, 1H, *J* = 7.2 and 2.0 Hz), 7.62 (s, 1H), 7.35 (m, 1H), 7.25 (m, 1H), 7.16
(m, 1H), 7.08 (dd, 1 H, *J* = 7.6 and 4.8 Hz), 6.28 (d, 1H, *J* = 7.2 Hz), 4.71 (d, 1H, *J* = 7.2 Hz), 4.18 (m, 1H), 3.97 (m, 1H), 3.02 (s, 6 H), 2.13 (s, 3 H), 1.92~1.85 (m, 4 H), 1.80~1.74 (m, 4 H).

**Step B: Synthesis of 2-[(3,4-difluorophenyl)sulfonyl]-N-(cis-4-{[4-(dimethylamino)-5-methylpyrimidin-2-yl] amino}cyclohexyl)nicotinamide hydrochloride.**

To a solution of N-[cis-4-(4-dimethylamino-5-methyl-pyrimidin-2-ylamino)-cyclohexyl]-2-(3,4-difluorophenyl)-sulfanyl-nicotinamide (45 mg, 0.09 mmol) in DCM (6 mL) was added MCPBA (77 %, 31 mg, 2 eq.) at 0 °C under Ar atmosphere. The reaction was stirred overnight, washed with sat.-$NaHCO_3$ (2 x) and water, concentrated, and purified by column chromatography (DCM:MeOH = 100:0 to 94:6). 25 mg (53 %) of 2-[(3,4-difluorophenyl)sulfonyl]-N-(cis-4-{[4-(dimethylamino)-5-methylpyrimidin-2-yl]amino}cyclohexyl)nicotinamide was isolated and converted to its HCl salt.
ESI MS m/e 531 M + $H^+$; $^1H$ NMR (400 MHz, DMSO-$d_6$) δ 11.8 (bs, 1 H), 8.70 (m, 2 H), 8.04 (m, 1 H), 7.95 (dd, 1 H, J = 7.6 and 1.6 Hz), 7.89 (m, 1 H), 7.78~7.70 (m, 2 H), 7.60 (s, 1 H), 3.95 (bs, 1 H), 3.87 (bs, 1 H), 3.25 (s, 6 H), 2.22 (s, 3 H), 1.76 (bs, 8 H).

**Example 2657**

***N*-(3,4-Difluorophenyl)-*N'*-(*cis*-4-{[4-(dimethylamino)-5-methylpyrimidin-2-yl]amino}cyclohexyl)-*N*-methylurea trifluoroacetate**

**Step A: Synthesis of ethyl 3,4-difluorophenylcarbamate.**

3,4-Difluoroaniline (2.8 mL, 28 mmol) and *N*,*N'*-diisopropylethylamine (5.4 mL, 31 mmol) were dissolved in 10 mL of anhydrous THF, and cooled to 0°C in an ice bath. Ethyl chloroformate (5.4 mL, 31 mmol) was added slowly into the stirring solution over the ice bath. The solution was allowed to warm up to room temperature and stir for 30 minutes. The solvent was removed via vacuo and the crude solid was purified by column chromatography using ethyl acetate and hexane mixture (3:97) to yield ethyl 3,4-difluorophenylcarbamate as an off-white solid. (5.59 g, 99%)
ESI MS m/z 202.1 (M + $H^+$) ; $^1H$ NMR (400 MHz, DMSO-$d_6$) δ 9.79 (s, 1H), 7.55-7.50 (m, 1H), 7.29-7.22 (m, 1H), 7.16-7.15 (m, 1 H), 4.10 (q, *J* = 7.2 Hz, 2H), 1.22 (t, *J* = 7.2 Hz, 3H).

**Step B: Synthesis of (3,4-difluoro-phenyl)-methyl-amine.**

Lithium aluminum hydride (22 g, 56 mmol) was placed in a 500 mL round bottom flask. THF (100 mL) was syringed into the flask under argon. The solution was cooled to 0°C in an ice bath. To the ice-cold solution, 3,4-difluorophenylcarbamate (5.59 g, 28 mmol) was added slowly into the flask. The solution was refluxed for 3 hours. After cooling the reaction mixture to 0°C, $H_2O$ (3 mL), 1 N NaOH (3 mL), and then more $H_2O$ (15 mL) were added for quenching. The precipitate was filtered off and THF was evaporated from the filtrate. The crude was dissolved in 150 mL of ethyl acetate, washed with water, and dried over $Na_2SO_4$. The organic solvent was removed via vacuo to yield (3,4-difluoro-phenyl)-methyl-amine as a light brown oil. (2.86 g, 71 %) ESI MS m/z 144.2 (M + $H^+$) ; $^1H$ NMR (400 MHz,

$CDCl_3$) δ 7.04-6.97 (m, 1H), 6.45-6.39 (m, 1H), 6.32-6.28 (m, 1H), 3.69 (b, 1H), 2.86 (s, 3H).

**Step C: Synthesis of N-(3,4-difluorophenyl)-*N'*-(cis-4-{[4-(dimethylamino)-5-methyl-pyrimidin-2-yl]amino} cyclohexyl)-*N*-methylurea trifluoroacetate.**

cis-[4-(4-dimethlamino-5-methyl-pyrimidin-2-ylamino) cyclohexyl]-carbamic acid *tert*-butyl ester (100 mg, 0.402 mmol) and 1,1 -carbonyldiimidazole (78.1 mg, 0.482 mmol) were dissolved in 1 mL of methylene chloride and allowed to stir at room temperature overnight. To the vial, (3,4-difluoro-phenyl)-methyl-amine (88 mg, 0.603 mmol) was added. The solution was heated via Smith Synthesizer at 130°C for 15 minutes. The solvent was evaporated, and 1 mL of methanol was added to the crude. The crude was purified by HPLC to yield N (3,4-difluorophenyl)-N-(cis-4-{[4-(dimethylamino)-5-methylpyrimidin-2-yl]amino}cyclohexyl)-N-methylurea trifluoroacetate as a white solid. (47.8 mg, 22%)
ESI MS m/z 419.3 (M + $H^+$) ; $^1$H NMR (400 MHz, $CDCl_3$) δ 14.0 (s, 1 H), 8.62 (d, J = 6.4 Hz, 1 H), 7.29-7.21 (m, 2H), 7.13-7.01 (m, 2H), 4.61 (bs, 1 H), 4.10 (m, 1 H), 3.78 (m, 1 H), 3.46-3.29 (b, 3H), 3.24 (s, 6H), 2.24 (s, 3H), 1.77-1.56 (m, 8H).

**Example 2658**

***N- [(cis-4-* [4-(Dimethylamino)-6-methylpyrimidin-2-yl] amino j cyclobexyl) methyl]-3,5-bis(trifluoromethyl) benzamide hydrochloride**

**Step A: Synthesis of N-(cis-4-amino-cyclohexylmethyl)-3,5-bistrifluoromethyl-benzamide trifluoroacetate.**

To a solution of cis-(4-aminomethyl-cyclohexyl)-carbamic acid tert-butyl ester (1.1 g, 4.8 mmol) in dry benzene (15 mL) was added 3,5-bistrifluoromethyl benzoyl chloride (1.33 g, 1 eq.) and followed by $Et_3N$ (~2 mL) at room temperature under $N_2$. The reaction was stirred for an additional 2 h at room temperature, washed with sat.-$NaHCO_3$ (3x) and water (1x), dried with $MgSO_4$, and concentrated. The crude {cis-{4-[(3,5-Bis-trifluoromethy)-benzoylamino)-methy)]-cyclohexyl}-carbamic acid tert-butyl ester was pure enough to use for the next deprotection without a further purification.
{cis-{4-[(3,5-Bis-trifluoromethyl-benzoy)amino)-methy)]-cyclohexy)}-carbamic acid tert-butyl ester (2.1 g, 4.5 mmol) was dissolved in DCM (10 mL), and TFA (5 mL) was added to the reaction. After 1.5 h stirring at room temperature, removal of the volatile solvent gave crude *N-(* 4-amino-cyclohexylmethyl)-3,5-bis-trifluoromethyl-benzamide trifluoroacetate as a sticky oil. Addition of water (~40 mL) to the crude product and shaking well for 5 ~ 10 min provided formation of precipitates, and the ppts were filtered, washed with water, and dried; 1.40 (61 %) of N-(4-amino-cyclohexylmethyl)-3,5-bis-trifluoromethyl-benzamide trifluoroacetate was isolated as a white powder.
ESI MS m/e 369 M + $H^+$; $^1$H NMR (400 MHz, DMSO-$d_6$) δ 8.97 (bs, 1 H), 8.47 (s, 2 H), 8.29 (s, 1 H), 7.78 (bs, 3 H), 3.29 (t, 2 H, J = 6.8 Hz), 3.15 (bs, 1 H), 1.78 (bs, 1 H), 1.66 (m, 4 H), 1.52 (m, 4 H).

**Step B: Synthesis of *N*-[(cis4-{[4-(dimethylamino)-6-methylpyrimidin-2-yl]amino)-cyclohexyl)methyl]-3,5-bis (trifluoromethyl)benzamide hydrochloride.**

A sealed tube containing 2-chloro-4-dimethylamino-6-methylpyrimidine (0.21 g, 1.2 mmol), N-(cis-4-amino-cyclohexylmethyl)-3,5-bistrifluoromethyl-benzamide trifluoroacetate (0.6 g, 1 eq.), DIEA (0.45 mL, 2 eq.), and *tert-BuOH* (2.5 mL) was reacted for 1.6 h at 185 °C in a Smith microwave synthesizer. The reaction was diluted with DCM, washed with diluted-HCl and water, dried, and concentrated. The crude product was purified by column chromatography (silica gel; DCM:MeOH = 100:0 to 95:5). 0.3 g (50 %) of N-[(cis-4-{[4-(dimethylamino)-6-methylpyrimidin-2-yl]amino} cyclohexyl) methyl]-3,5-bis(trifluoromethyl)benzamide was isolated and converted to HCl-salt.
ESI MS m/e 504 M + $H^+$; $^1$H NMR (400 MHz, $CDCl_3$) δ 12.8 (bs, 1H), 8.72 (d, 1 H, J = 8.0 Hz), 8.39 (s, 2 H), 7.93 (s, 1 H), 7.43 (bs, 1H), 5.70 (s, 1 H), 4.24 (bm, 1 H), 3.49 (t, 2 H, *J* = 4.4 Hz), 3.22 (s, 3 H), 3.11 (s, 3 H), 2.31 (s, 3 H), 1.91~1.79 (m, 5 H), 1.64~1.56 (m, 4 H).

**Example 2659**

**$N^2$-[cis-4-({6-[(3,4-Difluorophenyl)sulfinyl)pyrazin-2-yl }amino)cyclohexyl]-*$N^4$*,*$N^4$*,5-trimethylpyrimidine-2,4-diamine**

**Step A: Synthesis of *cis*-[1-(6-chloro-pyrazin-2-ylamino)-4-(4-dimethylamino-5-methyl-pyrimidin-2-ylamino)]-cyclohexane.**

A sealed tube containing *cis*-4-(4-dimethylamino-5-methyl-pyrimidin-2-ylamino)-1-aminocyclohexane hydrochloride (0.2 g, 0.7 mmol), 2,6-dichloropyrazine (0.1 g, 1 eq.), DIEA (0.3 mL, 2 eq.), and IPA (2 mL) was reacted for 1.5 h at 170 °C in a Smith microwave synthesizer. The reaction was diluted with DCM, washed with IN-HCl and water, concentrated, and purified by column chromatography (DCM:MeOH = 100:0 to 96:4). 0.15 g (61 %) of *cis*-[1-(6-chloro-pyrazin-2-ylamino)-4-(4-dimethylamino-5-methyl-pyrimidin-2-ylamino)]-cyclohexane was isolated.
ESI MS m/e 362 M + $H^+$; $^1H$ NMR (400 MHz, $CDCl_3$) δ 8.70 (bs, 1 H), 7.76 (s, 1 H), 7.71 (s, 1 H), 7.29 (s, 1 H), 5.32 (bs, 1 H), 4.11 (bs, 1 H), 4.00 (bs, 1 H), 3.27 (s, 6 H), 2.23 (s, 3 H), 1.80 (m, 8 H).

**Step B: Synthesis of *cis*-{1-[6-(3,4-difluoro-phenylsulfonyl)-pyrazin-2-ylamino]-4-(4-dimethylamino-5-methyl-pyrimidin-2-ylamino)}-cyclohexane.**

A sealed tube containing cis-4-(4-dimethylamino-5-methyl-pyrimidin-2-ylamino)-1-(6-chloro-pyrazin-2-ylamino)-cyclohexane (0.1 g, 0.27 mmol), 3,4-difluorothiophenol (0.1 g, 2.5 eq.), $Cs_2CO_3$ (0.15 g, 2 eq.), and dioxane (2 mL) was reacted for I h at 180 °C in a Smith microwave synthesizer. The reaction was diluted with DCM, washed with sat-$NaHCO_3$ (3x) and water, concentrated, and purified by column chromatography to give 85 mg (65 %) of *cis*-{1-[6-(3,4-difluoro-phenylsulfanyl)-pyrazin-2-ylamino]-4-(4-dimethylamino-5-methyl-pyrimidin-2-ylamino)}-cyclohexane.
ESI MS m/e 472 M + $H^+$; $^1H$ NMR (400 MHz, $CDCl_3$) δ 7.60 (s, 1 H), 7.48 (s, 1 H), 7.42 (m, 2 H), 7.29 (m, 1 H), 7.15 (m, 1 H), 6.70 (bs, 1 H), 5.15 (d, 1 H, J = 7.6 Hz), 4.03 (bs, 1 H), 3.67 (bm, 1 H), 3.16 (s, 6 H), 2.19 (s, 3 H), 1.81~1.61 (m, 8 H).

**Step C: Synthesis of *$N^2$*-[*cis*-4- ({6-[(3,4-difluorophenyl)sulfinyl]pyrazin-2-yl] amino)-cyclohexyl]-*$N^4$*,*$N^4$*, 5-trimethylpyrimidine-2,4-diamine.**

To a solution of cis-4-(4-dimethylamino-5-methyl-pyrimidin-2-ylamino)-1-[6-(3,4-difluoro-phenylsulfanyl)-pyrazin-2-ylamino]-cyclohexane (35 mg, 0.07 mmol) in DCM (5 mL) was added MCPBA (33 mg, 2 eq.) at room temperature under an Ar atmosphere. The reaction was stirred overnight, washed with sat-$NaHCO_3$ (2x) and water, concentrated, and purified by column chromatography (DCM:MeOH = 100:0 to 95:5). 12 mg (33 %) of *$N^2$-[cis*-4-({6-[(3,4-difluorophenyl)sulfnyl]pyrazin-2-yl}amino) cyclohexyl]-*$N^4$*,*$N^4$*,5-trimethylpyrimidine-2,4-diamine was isolated.
ESI MS m/e 488 M + $H^+$; $^1H$ NMR (400 MHz, $CDCl_3$) δ 8.25 (s, 1H), 7.87 (s, 1 H), 7.63 (m, 1 H), 7.57 (s, 1 H), 7.53 (m, 1 H), 7.26 (m, 1 H), 5.36 (bs, 1 H), 5.14 (d, 1 H, J = 6.8 Hz), 4.01 (bs, 1 H), 3.82 (bm, 1 H), 3.06 (s, 6 H), 2.15 (s, 3 H), 1.87~1.60 (m, 8 H).

**Example 2660**

***cis-N*-[1-(4-Bromophenyl)ethyl]-4-{[4-(dimethylamino)-5-methylpyrimidin-2-yl]amino} cyclohexanecarboxamide hydrochloride**

**Step A: Synthesis of *cis-N*-[1-(4-bromophenyl)ethyl]-4-{[4-(dimethylami*no)-5 methylpyrimidin-2-yl]amino) cyclohexanecarboxamide hydrochloride.**

To a solution of *cis*-4-(4-dimethylamino-5-methyl-pyrimidin-2-ylamino)-cyclohexanecarboxylic acid obtained from step B of Example 2594 (24 mg, 0.08 mmol) in DCM (3 mL) was added 1-(4-bromophenyl)-ethylamine (18 mg, 1 eq.), and followed by HATU (36 mg, 1.1 eq.) and $Et_3N$ (20 μL). The reaction was stirred overnight, concentrated, and purified by column chromatography (DCM:MeOH = 100:0 to 95:5). 16 mg (41 %) of *cis-N*-[1-(4-bromophenyl)ethyl]-4-{[4-(dimethylamino)-5-methylpyrimidin-2-yl]amino}cyclohexanecarboxamide was isolated and converted to HCl salt.
ESI MS m/e 460 M + $H^+$; $^1H$ NMR (400 MHz, DMSO-$d_6$) δ 11.0 (bs, 1 H), 8.20 (d, 1 H, J = 7.6 Hz), 7.66 (bs, 1 H), 7.50 (s, 1 H), 7.43 (d, 2 H, J = 8.4 Hz), 7.18 (d, 2 H, J = 8.4 Hz), 4.79 (m, 1 H), 3.95 (bs, 1 H), 3.19 (s, 6 H), 2.23 (bs, 1 H), 2.16 (s, 3 H), 1.70～1.50 (m, 8 H), 1.24 (d, 3 H, *J*= 7.2 Hz).

### Example 2661

***N*-[(*cis*-4-{[5-methyl-4-(methylamino)pyrimidin-2-yl]amino }cyclohexyl)methyl]-3,5-bis(trifluoromethyl) benzamidehydrochloride**

**Step A: Synthesis of (2-Chloro-5-methyl-pyrimidin-4-yl)-methyl-amine.**

2,4- Dichloro-5-methylpyrimidine (3.8g, 23.4mmol) in 20ml in $CH_2Cl_2$ was added 2.0 M methylamine in methyl alcohol (14.05ml, 28.1mmol) at 0 °C. The reaction mixture was stirred overnight and then the excess solvent was evaporated off and the material subjected to chromatography (50% hexanes in ethyl acetate) to yield (2-Chloro-5-methyl-pyrimidin-4-yl)-methyl-amine (968.7mg, 6.17mmol, 26%) as a white solid.
ESI MS 158.0 $M+H^+$; $^1$H NMR (400 MHz, DMSO-$d_6$) δ 7.86 (s, 1H), 7.39 (s, 1 H), 2.93-2.92 (d, J = 4 Hz, 3H), 2.04 (s, 3H).

**Step B: Synthesis of *N*-[(*cis*-4-{[5-methyl-4-(methylamino)pyrimidin-2-yl]amino}-cyclohexyl)methyl]-3,5-bis (trifluoromethyl)benzamidehydrochloride.**

To a solution of (2-Chloro-5-methyl-pyrimidin-4-yl)-methyl-amine (200mg, 1.27mmol) in 1 mL 2-propanol was added cis-N-(4-amino-cyclohexylmethyl)-3,5-bis-trifluoromethyl-benzamide in TFA salt (736mg, 1.52mmol) and DIEA (2.54mmol). The mixture was heated in a microwave synthesizer at 180°C for 2 hours. The solvent was evaporated and the material subjected to chromatography (70 ~ 95% ethyl acetate/ hexanes) The combined compound was dissolved in $CH_2Cl_2$ and was added 2 M HCl in diethyl ether (5.6ml, 1.42mmol) to yield N-[(cis-4-{[5-methyl-4-(methylamino)pyrimidin-2-yl]amino}cyclohexyl)methyl]-3,5-bis(trifluoromethyl)benzamidehydrochloride (443mg, 0.84mmol, 66%) as a white solid.
ESI MS 490.4 $M+H^+$; $^1$H NMR (400 MHz, DMSO-$d_6$) δ 11.5 (s, 1H), 8.86-8.83 (t, *J* = 4 Hz, 8 Hz, 1H), 8.32 (s, 2H), 8.11 (s, 1H), 8.03 (bs, 1H), 7.97 (bs, 1H), 7.40 (s, 1H), 3.90 (bs, 1H), 3.24 (s, 3H), 3.06-3.04 (d, *J* = 8 Hz, 2H), 2.72-2.71 (d, *J* = 4 Hz, 3H), 1.54 (bs, 4H), 1.42 (m, 4H), 1.20 (2H).

### Example 2662

***cis*-4-{[4-(Dimethylamino)-5-methylpyrimidin-2-yl]amino}-*N*-[(1R)-1-(3-methoxyphenyl)ethyl] cyclohexanecarboxamide hydrochloride**

**Step A: Synthesis of *cis*-4-{[4-(dimethylamino)-5-methylpyrimidin-2-yl]amino}-*N*-[(1R)-1-(3-methoxyphenyl) ethyl]cyclohexanecarboxamide hydrochloride.**

Using the procedure of Example 2660, the title compound was obtained.
ESI MS m/e 412 M + $H^+$; $^1$H NMR (400 MHz, DMSO-$d_6$) δ 10.9 (bs, 1 H), 7.98 (d, 1 H, J = 8.0 Hz), 7.53 (bs, 1 H), 6.98 (t, 1 H, J = 8.0 Hz), 6.63 (d, 1 H, *J* = 7.4 Hz), 6.62 (s, 1 H), 6.54 (d, 2 H, *J* = 8.0 Hz), 4.64 (m, 1 H), 3.79 (bs, 1 H), 3.50 (s, 3 H), 3.03 (s, 6 H), 2.08 (bs, 1 H), 1.97 (s, 3 H), 1.60~1.30 (m, 8 H), 1.10 (d, 3 H, *J*= 6.8 Hz).

### Example 2663

***cis*-4-{[4-(Dimethylamino)-5-methylpyrimidin-2-yl]amino}-*N*-[(1R)-1-(1-naphthyl)ethyl] cyclohexanecarboxamide hydrochloride**

**Step A: Synthesis of *cis*-4-{[4-(dimethylamino)-5-methylpyrimidin-2-yl]amino}-*N*-[(1R)-1-(1-naphthyl)ethyl] cyclohexanecarboxamide hydrochloride.**

Using the procedure of Example 2660, the title compound was obtained.
ESI MS m/e 432 M + $H^+$; $^1$H NMR (400 MHz, DMSO-$d_6$) δ 11.1 (bs, 1 H), 8.39 (d, 1 H, J = 8.0 Hz), 8.09 (d, 1 H, J = 8.0 Hz), 7.94 (m, 1 H), 7.82 (d, 1 H, J = 8.0 Hz), 7.73 (bs, 1 H), 7.56~7.49 (m, 5 H), 5.69 (m, 1 H), 4.01 (bs, 1 H), 3.25 (s, 6 H), 2.33 (bs, 1 H), 2.23 (s, 3 H), 1.85~1.55 (m, 8 H), 1.49 (d, 3 H, J = 6.8 Hz).

**Example 2664**

***N*-(*cis*-4-{[4-(Dimethylamino)-5-methylpyrimidin-2-yl]amino}cyclohexyl)-3-methylbenzamide hydrochloride**

**Step A: Synthesis of *N*-(*cis*-4-{[4-(dimethylamino)-5-methylpyrimidin-2-yl]amino}-cyclohexyl)-3-methylbenzamide hydrochloride.**

Using the procedure of example 2523, the title compound was obtained.
ESI MS m/e 368 M + $H^+$; $^1H$ NMR (400 MHz, DMSO-$d_6$) δ 12.2 (bs, 1 H), 8.28 (bs, 1 H), 7.98 (bd, 1 H, J= 6.0 Hz), 7.64 (m, 3 H), 7.31 (s, 1H), 7.30 (s, 1H), 3.91 (bs, 1H), 3.85 (bs, 1H), 3.25 (s, 6 H), 2.35 (s, 3 H), 2.22 (s, 3 H), 1.85 (bs, 2 H), 1.70 (bs, 6 H).

**Example 2665**

**N-{cis-4-[(4-Methylquinolin-2-yl)aminolcyclohexyl)-3,5-bis(trifluoromethyl)benzamide hydrochloride**

**Step A: Synthesis of *cis*-*N*-(4-amino-cyclohexyl)-3,5-bis(trifluoromethyl)-benzamide.**

To a solution of cis-(4-amino-cyclohexyl)-carbamic acid tert-butyl ester (3.2 g, 0.015 mol) in $CH_2Cl_2$ (50 mL) was added DIEA (3.9 mL, 0.022 mol). The mixture was cooled on an ice bath and 3,5-bis(trifluormethyl)benzoyl chloride (2.9 mL, 0.015 mol) was slowly added. The mixture was brought to room temperature and stirred for I hour. After this time, the solvent and excess DIEA was evaporated in vacuo. The resulting oil was re-dissolved in $CH_2Cl_2$ (30 mL) and extracted with $H_2O$ (30 mL),1M NaOH (30 mL), and brine (30 mL). The brine layer was twice back extracted with $CH_2Cl_2$ and the organic layers were combined, dried over $MgSO_4$, and concentrated. The resulting precipitate was re-dissolved in $CH_2Cl_2$ (50 mL) and TFA (4.6 mL, 0.060 mol) was added. The solution was stirred at room temperature for 4 hours (or until the reaction was complete as judged by TLC). The excess solvent was evaporated off and the resulting oil was dissolved in 30 mL $CH_2Cl_2$. The organic layer was extracted with 30 mL of a dilute NaOH (aq) / $NaHCO_3$ (aq) solution (the aqueous layer was confirmed to remain basic during the extraction using pH paper indicator). The aqueous layer was back extracted twice with $CH_2Cl_2$ and the organic layers combined, dried over $MgSO_4$, and concentrated. A precipitate formed that was subsequently filtered and washed with a cold 50% ether in hexanes solution to yield *cis*-*N*-(4-amino-cyclohexy])-3,5-bis(trifluoromethyl)-benzamide (4.0 g, 0.011 mol, 77%) as a white solid.
ESI MS 355.0 M+$H^+$ ; $^1H$ NMR (400 MHz, $CD_3OD$) δ 8.44 (s, 2H), 8.18 (s, 1H), 4.04 (m, 1H), 3.00 (m, 1H), 1.89-1.84 (m, 2H), 1.79-1.74 (m, 4H), 1.74-1.64 (m, 2H).

**Step B Synthesis of *N*-{*cis*-4-[(4-methylquinolin-2-yl)amino]cyclohexyl}-3,5-bis(trifluoromethyl)benzamide hydrochloride.**

To a solution of 2-chloro-4-methyl-quinoline (326 mg, 1.84 mmol) in 2 mL *t*-BuOH was added DIEA (369 uL, 2.12 mmol) and *cis*-*N*-(4-amino-cyclohexyl)-3,5-bis(trifluoromethyl)-benzamide (500 mg, 1.41 mmol). The mixture was then heated in a microwave at 180 °C for 12 hours. The reaction mixture was cooled and concentrated and the resulting oil was purified by column (<5 % MeOH in $CH_2Cl_2$). The organic solvents were evaporated and the resulting oil was re-dissolved into 4 mL $CH_2Cl_2$ and HCl (1.4 mL, 2.82 mol) was added. The reaction was stirred for 30 minutes and the solvent was removed. A precipitate formed that was subsequently filtered and washed with a cold 50% ether in hexanes solution to yield *N*-{*cis*-4-[(4-methylquinolin-2-yl)amino]cyclohexyl}-3,5-bis(trifluoromethyl)benzamide hydrochloride (620 mg, 1.17 mmol, 83%).
ESI MS 496.4 M+$H^+$; $^1H$ NMR (400 MHz,$CD_3OD$) δ 8.47 (s, 2H), 8.21 (s, 1H), 8.05 (d, 1H, *J*= 8.0 Hz), 7.93 (bs, 1 H), 7.82 (t, 1 H, J = 7.8 Hz), 7.59 (t, 1 H, J = 8.2 Hz), 7.09 (bs, 1 H), 4.17 (m, 1 H), 4.15 (m, 1 H), 2.73 (s, 3H), 2.08-1.95 (m, 8H).

**Example 2666**

***N-(cis-4-*{[4-(Dimethylamino)-5-methylpyrimidin-2-yl]amino}cyclohexyl)-3-(trifluoromethoxy)benzamide hydrochloride**

**Step A: Synthesis of N-(*cis*-4-{[4-(dimethylamino)-5-methylpyrimidin-2-yl]amino}cyclohexyl)-3-(trifluoromethoxy)benzamide hydrochloride.**

Using the procedure of example 2523, the title compound was obtained.

ESI MS m/e 438 M + H+; $^1$H NMR (400 MHz, $CDCl_3$) δ 12.9 (bs, 1 H), 8.59 (bd, 1 H, J= 6.8 Hz), 7.69 (s, 1 H), 7.68 (d, 1 H, J = 8.4 Hz), 7.43 (t, 1 H, J = 8.0 Hz), 7.30 (d, 1 H, J = 7.6 Hz), 7.20 (d, I H, *J*= 5.2 Hz), 6.55 (d, 1 H, *J*= 8.0 Hz), 4.17 (bs, 1 H), 4.10 (bs, 1 H), 3.29 (s, 6 H), 2.24 (s, 3 H), 1.98~1.83 (m, 6 H), 1.73 (m, 2 H).

**Example 2667**

***N-(cis*-4-{[4-(Dimethylamino)-5-methylpyrimidin-2-yl]amino}cyclohexyl)-4-(trifluoromethoxy)benzamide hydrochloride**

**Step A: Synthesis of *N*-(*cis*-4-{[4-(dimethylamino)-5-methylpyrimidin-2-yl]aminojcyclohexyl)-4-(trifluoromethoxy)benzamide hydrochloride.**

Using the procedure of example 2523, the title compound was obtained.
ESI MS m/e 438 M + H+; $^1$H NMR (400 MHz, $CDCl_3$) δ 12.3 (bs, 1 H), 8.54 (bd, 1 H, J = 6.8 Hz), 7.86 (d, 2 H, J = 8.8 Hz), 7.22 (d, 2 H, *J* = 8.8 Hz), 7.21 (s, 1 H), 6.68 (d, 1 H, J = 8.0 Hz), 4.17 (bs, 1 H), 4.10 (bs, 1 H), 3.28 (s, 6 H), 2.24 (s, 3 H), 1.95~1.85 (m, 6 H), 1.72 (m, 2 H).

**Example 2668**

**3-Chloro-*N*-(*cis*-4-{[4-(dimethylamino)-5-methylpyrimidin-2-yl]amino}cyclohexyl)-4-(trifluoromethoxy) benzamide hydrochloride**

**Step A: Synthesis of 3-chloro-N-(cis-4-{[4-(dimethylamino)-5-methylpyrimidin-2-yl]amino}cyclohexyl)-4-(trifluoromethoxy)benzamide hydrochloride.**

Using the procedure of example 2523, the title compound was obtained.
ESI MS m/e 472 M + H+; $^1$H NMR (400 MHz, $CDCl_3$) δ 12.5 (bs, 1 H), 8.37 (bd, 1 H, J = 7.2 Hz), 8.06 (s, 1 H), 7.86 (d, 1 H, J = 8.4 Hz), 7.51 (d, 1H, *J* = 8.4 Hz), 7.30 (d, 1H, J = 8.0 Hz), 7.24 (s, 1 H), 4.17 (bs, 1 H), 4.08 (bm, 1 H), 3.28 (s, 6 H), 2.23 (s, 3 H), 1.92~1.85 (m, 6 H), 1.71 (m, 2 H).

**Example 2669**

**4-Chloro-N-(cis4-{[4-(dimethylamino)-5-methylpyrimidin-2-yl]amino)cyclohexyl)-3-(trifluoromethyl) benzamide hydrochloride**

**Step A: Synthesis of 4-chloro-N-(cis4-{[4-(dimethylamino)-5-methylpyrimidin-2-yl]aminojcyclohexyl)-3-(trifluoromethyl)benzamide hydrochloride.**

Using the procedure of example 2523, the title compound was obtained.
ESI MS m/e 456 M + H+; $^1$H NMR (400 MHz, $CDCl_3$) δ 12.8 (bs, 1 H), 8.58 (bd, 1 H, J = 6.8 Hz), 8.19 (s, 1 H), 7.90 (d, 1 H, J = 8.4 Hz), 7.54 (d, I H, J = 8.4 Hz), 7.19 (bd, 1 H, J = 5.2 Hz), 6.76 (d, 1 H, J = 8.4 Hz), 4.19 (bs, 1 H), 4.10 (bm, 1 H), 3.29 (s, 6 H), 2.24 (s, 3H), 1.94~1.83 (m, 6 H), 1.72 (m, 2 H).

**Example 2670**

**3,5-Dichloro-*N*-(*cis*-4-{[4-(dimethylamino)-5-methylpyrimidin-2-yl]amino}cyclohexyl)benzamide hydrochloride**

**Step A: Synthesis of 3,5-dichloro-*N*-(*cis*-4-{[4-(dimethylamino)-5-methylpyrimidin-2-yl]amino}cyclohexyl) benzamide hydrochloride.**

Using the procedure of example 2523, the title compound was obtained.
ESI MS m/e 422 M + H+; $^1$H NMR (400 MHz, *DMSO-$d_6$)* δ 12.1 (bs, 1 H), 8.50 (bs, 1 H), 8.02 (bd, 1 H, *J* = 5.2 Hz), 7.86 (d, 2 H, J = 1 .6 Hz), 7.77 (t, 1 H, *J* = 1.6 Hz), 7.63 (s, 1 H), 3.90 (bs, 1 H), 3.85 (bs, 1 H), 3.25 (s, 6 H), 2.22 (s, 3 H), 1.85 (bs, 2 H), 1.70 (bs, 6 H).

**Example 2671**

**3,4-Dichloro-*N-(cis*-4-{[4-(dimethylamino)-5-methylpyrimidin-2yl]amino) cyclohexyl)-benzamide hydrochloride**

**Step A: Synthesis of 3,4-dichloro-*N*-(*cis*-4-{[4-(dimethylamino)-5-methylpyrimidin-2-yl]amino}cyclohexyl) benzamide hydrochloride.**

Using the procedure of example 2523, the title compound was obtained. ESI MS m/e 422 M + $H^+$; $^1H$ NMR (400 MHz, DMSO-$d_6$) δ 12.2 (bs, 1H), 8.47 (bs, 1H), 8.09 (d, 1H, *J* = 2.0 Hz), 8.05 (d, 1 H, *J* = 6.4 Hz), 7.82 (dd, 1H, *J* = 8.0 and 1.6 Hz), 7.71 (d, 1H, *J* = 8.4 Hz), 7.63 (s, 1H), 3.90 (bs, 1 H), 3.85 (bs, 1H), 3.25 (s, 6 H), 2.22 (s, 3 H), 1.85 (bs, 2 H), 1.70 (bs, 6H).

**Example 2672**

**5-Bromo-N-(cis-4-{[4-(dimethylamino)-5-methylpyrimidin-2-yl]amino}cyclohexyl)-2-furamide**

**Step A: Synthesis of 5-bromo-N-(cis-4-{[4-(dimethylamino)-5-methylpyrimidin-2-yl]amino}cyclohexyl)-2-furamide.**

Using the procedure of example 2523, the title compound was obtained. ESI MS m/e 422 M + $H^+$; $^1H$ NMR (400 MHz, $CDCl_3$) δ 7.64 (s, 1H), 7.02 (d, 1H, J = 3.6 Hz), 6.41 (d, 1 H, J = 3.6 Hz), 6.23 (bs, 1 H), 4.77 (bs, 1 H), 4.08 (bs, 1 H), 3.96 (bs, 1 H), 3.02 (s, 6 H), 2.14 (s, 3H), 1.88~1.60 (m, 8 H).

**Example 2673**

**N-(cis-4-{[4-(Dimethylamino)-5-methylpyrimidin-2-yl]amino}cyclohexyl)-2-(methylsulfonyl)benzamide**

**Step A: Synthesis of N-(cis-4-{[4-(dimethylamino)-5-methylpyrimidin-2-yl]amino}cyclohexyl)-2-(methylsulfonyl)benzamide.**

Using the procedure of example 2523, the title compound was obtained. ESI MS m/e 432 M + $H^+$; $^1H$ NMR (400 MHz, $CDCl_3$) δ 8.02 (d, 1 H, J = 7.6 Hz), 7.69 (t, 1 H, *J* = 8.0 Hz), 7.59 (t, 2 H, J = 7.6 Hz), 6.39 (d, 1 H, J = 8.0 Hz), 6.34 (bs, 1 H), 4.10 (bs, 2 H), 3.33 (s, 3 H), 3.25 (s, 6 H), 2.25 (s, 3 H), 1.93～1.71 (m, 8 H) .

**Example 2674**

***N*-(*cis*-4-{[4-(Dimethylamino)-5-methylpyrimidin-2-yl]amino}cyclohexyl)-3-(methylsulfonyl)benzamide**

**Step A: Synthesis of *N*-(*cis*-4-{[4-(dimethylamino)-5-methylpyrimidin-2-yl]amino}cyclohexyl)-3-(methylsulfonyl)benzamide.**

Using the procedure of example 2523, the title compound was obtained. ESI MS m/e 432 M + $H^+$; $^1H$ NMR (400 MHz, $CDCl_3$) δ 8.40 (s, 1H), 8.18 (d, 1H, *J* = 7.6 Hz), 8.08 (d, 1H, *J* = 7.6 Hz), 7.67 (t, 1H, *J* = 7.6 Hz), 7.34 (s, 1H), 6.99 (d, 1H, *J* = 8.0 Hz), 6.57 (bd, 1H, *J* = 6.4 Hz), 4.17 (bm, 2 H), 3.32 (s, 6 H), 3.16 (s, 3 H), 2.27 (s, 3 H), 1.90~1.71 (m, 8 H).

**Example 2675**

***N*-(*cis*-4-{[4-(Dimethylamino)-5-methylpyrimidin-2-yl]amino}cyclohexyl)-4-(methylsulfonyl)benzamide**

**Step A: Synthesis of *N*-(*cis*-4-{[4-(dimethylamino)-5-methylpyrimidin-2-yl]amino}cyclohexyl)-4-(methylsulfonyl)benzamide.**

Using the procedure of example 2523, the title compound was obtained. ESI MS m/e 432 M + $H^+$; $^1H$ NMR (400 MHz, $CDCl_3$) δ 8.04 (d, 2 H, *J* = 8.4 Hz), 7.98 (d, 2 H, *J* = 8.4 Hz), 7.28 (s, 1 H), 6.86 (d, 1 H, *J* = 8.4 Hz), 6.41 (d, 1 H, *J* = 7.6 Hz), 4.14 (bm, 2 H), 3.32 (s, 6 H), 3.07 (s, 3 H), 2.27 (s, 3 H), 1.90~1.71

(m, 8 H).

**Example 2676**

**Methyl *2-{[(cis-4-*{[4-(dimethylamino)-5-methylpyrimidin-2-yl]amino}cyclohexyl)amino]carbonyl}benzoate**

**Step A: Synthesis of methyl 2-{[(*cis*-4-{[4-(dimethylamino)-5-methylpyrimidin-2-yl]amino}cyclohexyl)amino]carbonyl}benzoate.**

Using the procedure of example 2523, the title compound was obtained.
ESI MS m/e 428 M + $H^+$; $^1H$ NMR (400 MHz, $CDCl_3$) δ 8.10 (bs, 1 H), 7.87 (d, 1 H, J = 7.6 Hz), 7.52 (t, 1 H, *J* = 7.6 Hz), 7.46 (m, 2 H), 7.30 (s, 1 H), 6.56 (d, 1 H, *J* = 8.0 Hz), 4.13 (bm, 2 H), 3.87 (s, 3 H), 3.24 (s, 6 H), 2.22 (s, 3 H), 1.93~1.75 (m, 8 H).

**Example 2677**

**Methyl 3-{[(*cis*-4-{[4-(dimethylamino)-5-methylpyrimidin-2-yl]amino}cyclohexyl)amino]carbonyl}benzoate**

**Step A: Synthesis of methyl 3-{[(*cis*-4-{[4-(dimethylamino)-5-methylpyrimidin-2-yl]amino}cyclohexyl)amino]carbonyl)benzoate.**

Using the procedure of example 2523, the title compound was obtained.
ESI MS m/e 428 M + $H^+$; $^1H$ NMR (400 MHz, $CDCl_3$) δ 8.48 (s, 1 H), 8.17 (bs, 1 H), 8.14 (d, 1 H, *J* = 7.6 Hz), 8.08 (d, 1 H, *J* = 7.6 Hz), 7.51 (t, 1 H, *J* = 8.0 Hz), 7.31 (s, 1 H), 7.16 (d, 1 H, *J* = 7.6 Hz), 4.14 (bm, 2 H), 3.94 (s, 3 H), 3.26 (s, 6 H), 2.23 (s, 3 H), 1.93～1.73 (m, 8 H).

**Example 2678**

**2-{[(*cis*-4-{[4-(Dimethylamino)-5-methylpyrimidin-2-yl]amino}cyclohexyl)amino]carbonyl)benzoic acid hydrochloride**

**Step A: Synthesis of 2-{[(*cis*-4-{[4-(dimethylamino)-5-methylpyrimidin-2-yl]amino}cyclohexyl)amino]carbonyl)benzoic acid hydrochloride.**

Using the procedure of example 2523, the title compound was obtained.
ESI MS m/e 398 M + $H^+$; $^1H$ NMR (400 MHz, DMSO-$d_6$) δ 12.5 (bs, 2 H), 8.32 (bs, 1 H), 8.04 (d, 1 H, *J* = 6.4 Hz), 7.80 (d, 1 H, *J* = 7.6 Hz), 7.68 (s, 1 H), 7.58 (m, 1 H), 7.51 (t, 1 H, *J* = 7.6 Hz), 7.39 (d, 1H, *J* = 7.6 Hz), 3.89 (bs, 2 H), 3.28 (s, 6 H), 2.25 (s, 3 H), 1.85~1.70 (m, 8 H).

**Example 2679**

**3-{[(*cis*-4-{[4-(Dimethylamino)-5-methylpyrimidin-2-yl]amino}cyclohexyl)amino]carbonyl}benzoic acid hydrochloride**

**Step A: Synthesis of 3-{[(*cis*-4-{[4-(dimethylamino)-5-methylpyrimidin-2-yl]amino}cyclohexyl)amino]carbonyl}benzoic acid hydrochloride.**

Using the procedure of example 2523, the title compound was obtained.
ESI MS m/e 398 M + $H^+$; $^1H$ NMR (400 MHz, DMSO-$d_6$) δ 13.2 (bs, 1 H), 12.3 (bs, 1 H), 8.59 (bs, 1 H), 8.47 (m, 1H), 8.16~8.11 (m, 3 H), 7.72 (s, 1H), 7.64 (t, 1 H, *J* = 8.0 Hz), 3.95 (bs, 2 H), 3.32 (s, 6 H), 2.29 (s, 3 H), 1.93 (bs, 2 H), 1.78 (bs, 6 H).

**Example 2680**

***N*-(*cis*-4-{[4-(Dimethylamino)-6-methylpyrimidin-2-yl]amino}cyclohexyl)-3,4-difluorobenzamide hydrochloride**

**Step A: Synthesis of *N*-(*cis*-4-{[4-(dimethylamino)-6-methylpyrimidin-2-yl]amino)cyclohexyl)-3,4-difluorobenzamide hydrochloride.**

Using the procedure of example 2526, the title compound was obtained.
ESI MS m/e 390 M + $H^+$; $^1H$ NMR (400 MHz, DMSO-$d_6$) δ 12.7 (bs, 1 H), 8.37 (bs, 1 H), 7.93~7.88 (m, 2 H), 7.73 (m, 1 H), 7.51 (dd, 1 H, *J* = 18.8 and 8.4 Hz), 6.26 (s, 1 H), 3.96 (bs, 1 H), 3.84 (bs, 1 H), 3.17 (s, 3 H), 3.13 (s, 3 H), 2.25 (s, 3 H), 1.85 (bm, 2 H), 1.70 (bs, 6 H).

**Example 2681**

***N*-(cis-4-{[4-(Dimethylamino)-6-methylpyrimidin-2-yl]amino}cyclohexyl)-3,5-bis(trifluoromethyl)benzamide hydrochloric acid**

**Step A: Synthesis of *N*-(*cis*-4-{[4-(dimethylamino)-6-methylpyrimidin-2-yl] amino)-cyclohexyl)-3,5-bis (trifluoromethyl)benzamide hydrochloric acid.**

To a solution of (2-chloro-6-methyl-pyrimidin-4-yl)-dimethyl-amine (242 mg, 1.41 mmol) in 2 mL *t*-BuOH was added DIEA (369 uL, 2.12 mmol) and cis-N-(4-amino-cyclohexyl)-3,5-bis(trifluoromethyl)-benzamide (500 mg, 1.41 mmol). The mixture was then heated in a microwave at 180 °C for 1.7 hours. The reaction mixture was cooled and concentrated and the resulting oil was purified by column (<5 % MeOH in $CH_2Cl_2$). The organic solvents were evaporated and the resulting oil was re-dissolved into 4 mL $CH_2Cl_2$ and HCl (1.4 mL, 2.82 mol) was added. The reaction was stirred for 30 minutes and the solvent was removed. A precipitate formed that was subsequently filtered and washed with a cold 50% ether in hexanes solution to yield *N*-(*cis*-4-{[4-(dimethylamino)-6-methylpyrimidin-2-yl]amino} cyclohexyl)-3,5-bis(trifluoromethyl)benzamide hydrochloric acid (653 mg, 1.24 mmol, 88%).
ESI MS 490.4 M+$H^+$ ; $^1H$ NMR (400 MHz, CD3OD) δ 12.58 (bs, 1H), 8.81 (d, 1H, *J* = 6.4 Hz), 8.50 (s, 2H), 8.30 (s, 1H), 7.89 (bs, 1H), 6.28 (s, 1H), 4.00 (m, 1H), 3.90 (m, 1H), 3.18 (s, 3H), 3.12 (s, 3H), 2.25 (s, 3H), 1.87-1.71 (m, 8H).

**Example 2682**

***N*-(*cis*-4-) [4-(Dimethylamino)-6-methylpyrimidin-2-yl]amino}cyclohexyl)-4-(trifluoromethoxy)benzamide hydrochloride**

**Step A: Synthesis of *N*-(*cis*-4-{[4-(dimethylamino)-6-methylpyrimidin-2-yl]amino)cyclohexyl)-4-(trifluoromethoxy)benzamide hydrochloride.**

Using the procedure of example 2526, the title compound was obtained.
ESI MS m/e 438 M + $H^+$; $^1H$ NMR (400 MHz, $CDCl_3$) δ 13.0 (bs, 1 H), 8.52 (bd, 1 H, *J* = 7.6 Hz), 7.87 (d, 2 H, *J* = 8.8 Hz), 7.23 (d, 2 H, *J* = 8.8 Hz), 6.84 (d, 1 H, *J* = 8.0 Hz), 5.72 (s, 1 H), 4.22 (bm, 1 H), 4.11 (bm, 1 H), 3.24 (s, 3 H), 3.12 (s, 3 H), 2.34 (s, 3 H), 1.95~1.85 (m, 6 H), 1.72 (m, 2 H).

**Example 2683**

**3-Chloro-*N*-(*cis*-4-{[4-(dimethylamino)-6-methylpyrimidin-2-yl]amino}cyclohexyl)-4-(trifluoromethoxy) benzamide hydrochloride**

**Step A: Synthesis of 3-chloro-*N*-(*cis*-4-{[4-(dimethylamino)-6-methylpyrimidin-2-yl]amino}cyclohexyl)-4-(trifluoromethoxy)benzamide hydrochloride.**

Using the procedure of example 2526, the title compound was obtained.
ESI MS m/e 472 M + $H^+$; $^1H$ NMR (400 MHz, $CDCl_3$) δ 12.9 (bs, 1 H), 8.52 (d, 1 H, *J* = 7.6 Hz), 7.96 (d, 1 H, *J* = 2.4 Hz), 7.73 (dd, 1 H, *J* = 8.8 and 2.0 Hz), 7.34 (d, 1 H, *J* = 8.4 Hz), 6.59 (d, 1 H, *J* = 8.0 Hz), 5.72 (s, 1 H), 4.22 (bm, 1 H), 4.10 0 (bm, 1 H), 3.24 (s, 3 H), 3.12 (s, 3 H), 2.34 (s, 3 H), 1.95~1.83 (m, 6 H), 1.72 (m, 2 H).

**Example 2684**

**4-Chloro-*N*-(*cis*-4-{[4-(dimethylamino)-6-methylpyrimidin-2-yl]amino}cyclohexyl)benzamide hydrochloride**

**Step A: Synthesis of 4-chloro-*N*-(*cis*-4-([4-(dimethylamino)-6-methylpyrimidin-2-yl]amino)cyclohexyl) benzamide hydrochloride.**

Using the procedure of example 2526, the title compound was obtained.
ESI MS m/e 388 M + H$^+$; $^1$H NMR (400 MHz, $CDCl_3$) δ 13.1 (bs, 1 H), 8.57 (bd, 1 H, *J* = 8.0 Hz), 7.73 (d, 2 H, *J*= 8.4 Hz), 7.37 (d, 2 H, *J* = 8.4 Hz), 6.46 (d, 1 H, *J*= 6.0 Hz), 5.71 (s, 1 H), 4.20 (bs, 1 H), 4.10 (bs, 1 H), 3.24 (s, 3 H), 3.12 (s, 3 H), 2.34 (s, 3 H), 1.94~1.82 (m, 6 H), 1.73 (m, 2 H).

**Example 2685**

**3,4-Dichloro-*N*-(*cis*-4-{[4-(dimethylamino)-6-methy]pyrimidin-2yl]amino}cyclohexyl)benzamide hydrochloride**

**Step A: Synthesis of 3,4-dichloro-*N*-(*cis*-4-{[4-(dimethylamino)-6-methylpyrimidin-2-yl]amino]cyclohexyl) benzamide hydrochloride.**

Using the procedure of example 2526, the title compound was obtained. ESI MS m/e 422 M + H$^+$; $^1$H NMR (400 MHz, $CDCl_3$) δ 13.0 (bs, 1 H), 8.51 (d, 1 H, *J* = 7.6 Hz), 7.94 (d, 1 H, *J* = 2.0 Hz), 7.64 (dd, 1 H, *J* = 8.4 and 2.0 Hz), 7.47 (d, 1 H, *J* = 8.4 Hz), 6.88 (d, 1 H, J = 8.8 Hz), 5.72 (s, 1 H), 4.22 (bm, 1 H), 4.09 (bm, 1 H), 3.24 (s, 3 H), 3.13 (s, 3 H), 2.34 (s, 3 H), 1.94~1.82 (m, 6 H), 1.72 (m, 2 H).

**Example 2686**

***N-(cis*-4-{[4-(Dimethylamino)-6-methylpyrimidin-2-yl]amino}cyclohexyl)-3,5-dimethoxybenzamide**

**Step A: Synthesis of *N*- (cis-4-{[4-(dimethylamino)-6-methylpyrimidin-2-yl]amino)yclohexyl)-3,5-dimethoxybenzamide.**

Using the procedure of example 2526, the title compound was obtained.
ESI MS m/e 414 M + H$^+$; $^1$H NMR (400 MHz, $CDCl_3$) δ 6.88 (d, 2 H, J = 2.0 Hz), 6.57 (t, 1 H, *J* = 2.0 Hz), 6.15 (d, 1 H, *J* = 7.6 Hz), 5.69 (s, 1 H), 5.10 (bs, 1 H), 4.06 (bm, 2 H), 3.82 (s, 6 H), 3.04 (s, 6 H), 2.21 (s, 3 H), 1.90~1.81 (m, 6 H), 1.67 (m, 2 H).

**Example 2687**

**5-Bromo-*N*-(*cis*-4-{[4-(dimethylamino)-5-methylpyrimidin-2-yl]amino}cyclohexyl)nicotinamide hydrochloride**

**Step A: Synthesis of 5-bromo-*N*-(*cis*-4-{[4-(dimethylamino)-5-methylpyrimidin-2-yl]amino}cyclohexyl) nicotinamide hydrochloride.**

Using the procedure of example 2526, the title compound was obtained.
ESI MS 433.2 M+H$^+$ ; $^1$H NMR (400 MHz, DMSO-$d_6$) δ 12.2 (s, 1H), 8.85 (d, *J* = 4 Hz, 1H), 8.73 (d, *J* =4 Hz, 1H), 8.51 (bs, 1H), 8.34-8.33 (m, 1H), 7.55 (bs, 1H), 3.76 (bs, 2H), 3.14 (bs, 6H), 2.10 (s, 3 H), 1.74-1.59 (m, 8H).

**Example 2688**

***N*-(*cis*-4-{[4-(Dimethytamino)-5-methylpyrimidin-2-yl]amino}cyc!ohexyl)-4-[2,2,2-trinuoro-1-hydroxy-1-(trifluoromethyl)ethyl]benzamide hydrochloride**

**Step A: Synthesis of *N*-(*cis*-4-{[4-(dimethylamino)-5-methylpyrimidin-2-yl]amino}cyclohexyl)-4-[2,2,2-trifluoro-1-hydroxy-1-(trifluoromethyl)ethyl]benzamide hydrochloride.**

Using the procedure of example 2526, the title compound was obtained.
ESI MS 520.4 M+H$^+$ ; $^1$H NMR (400 MHz, DMSO-$d_6$) δ 12.0 (s, 1H), 8.84 (s, 1H), 8.36 (bs, 1H), 7.91 (bs, 1H), 7.88 (d, *J* = 8 Hz, 2H), 7.73-7.71 (d, *J* = 8 Hz, 2H), 7.60 (s, 1H), 3.85 (bs, 2H), 3,23 (s, 6H), 2.20 (s, 3H), 1.82 (m, 2H), 1.68 (m, 6H).

**Example 2689**

**3-Bromo-4-chloro-*N*-(*cis*-4-{[4-(dimethylamino)-5-methylpyrimidin-2-yl]amino}cyclohexyl)benzamide hydrochloride**

**Step A: Synthesis of 3-bromo-4-chloro-*N*-(*cis*-4-{[4-(dimethylamino)-5-methylpyrimidin-2-yl]amino} cyclohexyl)benzamide hydrochloride.**

Using the procedure of example 2526, the title compound was obtained.
ESI MS 466.0 $M+H^+$ ; $^1H$ NMR (400 MHz, DMSO-$d_6$) δ 12.0 (s, 1H), 8.32-8.31 (d, *J* = 4 Hz, 1H), 8.08-8.07 (d, *J*= 2 Hz, 1H), 7.88-7.86 (d, *J*= 8 Hz, 1H), 7.73-7.70 (dd, $J_1$ = 4 Hz, $J_{2-4}$ Hz, 1H), 7.57-7.55 (d, *J* = 8 Hz, 1H ), 7.49 (s, 1H), 3.76-3.69 (m, 2H), 3.16 (s, 6H), 2.07 (s, 3H), 1.70 (bs, 2H), 1.55 (bs, 6H).

**Examples 2690-2711**

Compounds 2690 to 2711 were prepared in a similar manner as described in Example 2590 using the appropriate acid chloride and amine intermediate from Step B.

**Examples 2712-2731**

Compounds 2712 to 2731 were prepared in a similar manner as described in Example 2591 using the appropriate acid chloride and amine intermediate from Step A.

**Examples 2732-2750**

Compounds 2732 to 2750 were prepared in a similar manner as described in Example 2592 using the appropriate acid chloride and amine intermediate from Step A.

**Examples 2751-2770**

Compounds 2751 to 2770 were prepared in a similar manner as described in Example 2593 using the appropriate acid chloride and amine intermediate from Step B.

**Examples 2771-2794**

Compounds 2771 to 2794 were prepared in a similar manner as described in Example 2594 using the appropriate amine and the carboxylic acid intermediate from Step B.

**Examples 2795-2823**

Compounds 2795 to 2823 were prepared in a similar manner as described in Example 2527 using the appropriate amine and the carboxylic acid intermediate from Step B.

**Examples 2824-2864**

Compounds 2824 to 2864 were prepared in a similar manner as described in Example 2607 using the appropriate acid chloride and the amine intermediate from Step D.

**Examples 2865-2866**

Compounds 2865 and 2866 were prepared in a similar manner as described in Example 2611 using the appropriate benzaldehyde and the amine from Step A.

**Examples 2867-2869**

Compounds 2867 to 2869 were prepared in a similar manner as described in Example 2613 using the appropriate isocyanate and the amine from Step A.

**Examples 2870-2875**

Compounds 2870 to 2875 were prepared in a similar manner as described in Example 2615 using the appropriate carboxylic acid and the amine from Step A.

**Example 2876**

Compound 2876 was prepared in a similar manner as described in Example 2623 using the appropriate 4-chloro mandelic acid and the amine of Step A.

**Examples 2877-2879**

Compounds 2877 to 2879 were prepared in a similar manner as described in Example 2638 using the appropriate phenol and the bromoacetamide intermediate of Step B.

**Examples 2880-2884**

Compounds 2880 to 2884 were prepared in a similar manner as described in Example 2644 using the appropriate thiophenol.

**Examples 2885-2895**

Compounds 2885 to 2895 were prepared in a similar manner as described in Example 2647 using the appropriate phenol and the chloropyridyl intermediate of Step A.

**Examples 2896-2940**

Compounds 2896 to 2940 were prepared in a similar manner as described in Example 2523 using the appropriate acid chloride and the amine of Step C.

**Examples 2941-2948**

Compounds 2941 to 2948 were prepared in a similar manner as described in Example 2635 using the appropriate *N*-methylaniline and the bromoacetamide intermediate from step A.

**Examples 2949-2950**

Compounds 2949 and 2950 were prepared in a similar manner as described in Example 2619 using the appropriate carboxylic acid and the amine of Step A.

**Examples 2951-2994**

Compounds 2951 to 2994 were prepared in a similar manner as described in Example 2526 using the appropriate acid chloride and the amine of Step C.

**Example 2995**

Compound 2995 was prepared in a similar manner as described in Example 2628 using phenylsulfonyl chloride and the amine of Step A.

**Examples 2996-3004**

Compounds 2996 to 3004 were prepared in a similar manner as described in Example 2632 using the appropriate benzaldehyde and the amine of Step A.

**Example 3005**

Compound 3005 was prepared in a similar manner as described in Example 2632 using 3-trifluoromethoxy

benzaldehyde and the amine from step C of Example 2526.

**Example 3006**

Compound 3006 was prepared in a similar manner as described in Example 2642 using 3,4-difluorobenzoyl chloride and the amine from Step C.

**Examples 3007-3011**

Compounds 3007 to 3011 were prepared in a similar manner as described in Example 2637 using the appropriate phenol and the chloropyridyl intermediate from Step A.

**Examples 3012-3020**

Compounds 3012 to 3020 were prepared in a similar manner as described in Example 2636 using the appropriate phenol and the chloropyridyl intermediate of Step A.

**Examples 3021-3029**

Compounds 3021 to 3029 were prepared in a similar manner as described in Example 2657 using the appropriate N-methylaniline and the intermediate prepared in Step C.

**Example 3030**

Compound 3030 was prepared in a similar manner as described in Example 2595 using 3,4-dichlorobenzoyl chloride and the amine of Step A.

Specific compounds as shown in the Examples and in the Tables herein are represented as a mono or di-salt, for example, trifluoroacetate, hydrochloride, and the like; or as a free base. It is understood that these specific representations of the compounds in no way limit the scope of the invention to the respective salt or free base. For example, a trifluoroacetate salt can be readily converted to the corresponding free amine by treatment with a sufficient amount of base and if desired converted to another salt, for example, a pharmaceutically acceptable salt as described herein.

It is understood that the present invention embraces compounds, as disclosed herein, as free bases, inorganic salts, and organic salts; and as solvates, and hydrates thereof.

Compounds in the subsequent table are listed specifically as the free base and may have been specifically isolated as a trifluoroacetate, hydrochloride, or like salt as dictated by the specific synthetic procedure.

| Ex. No. | compound name | MS | class |
|---|---|---|---|
| 2690 | N-[(cis-4-{[4-(dimethylamino)-5-methylpyrimidin-2-yl]amino}cyclohexyl)methyl] benzamide | 368 (M + H) | 3 |
| 2691 | N-[(cis-4-{[4-(dimethylamino)-5-methylpyrimidin-2-yl]amino}cyclohexyl)methyl]-4-methylbenzamide | 382 (M + H) | 3 |
| 2692 | N-[(cis-4-{[4-(dimethylamino)-5-methylpyrimidin-2-yl]amino}cyclohexyl)methyl]-3,4-difluorobenzamide | 404 (M + H) | 2 |
| 2693 | N-[(cis-4-{[4-(dimethylamino)-5-methylpyrimidin-2-yl]amino}cyclohexyl)methyl]-4-methoxybenzamide | 398 (M + H) | 3 |
| 2694 | N-[(cis-4-{[4-(dimethylamino)-5-methylpyrimidin-2-yl]amino}cyclohexyl)methyl]-3,5-dimethoxybenzamide | 428 (M + H) | 1 |
| 2695 | N-[(cis-4-{[4-(dimethylamino)-5-methylpyrimidin-2-yl]amino]cyclohexyl)methyl]-3-fluoro-4-methylbenzamide | 400 (M + H) | 2 |
| 2696 | N-[(cis-4-{[4-(dimethylamino)-5-methylpyrimidin-2-yl]amino]cyclohexyl)methyl]-4-fluoro-3-methylbenzamide | 400 (M + H) | 2 |

(continued)

| Ex. No. | compound name | MS | class |
|---|---|---|---|
| 2697 | N-[(cis-4-{[4-(dimethylamino)-5-methylpyrimidin-2-yl]amino] cyclohexyl) methyl]-3-(trifluoromethyl)benzamide | 436 (M + H) | 1 |
| 2698 | N-((cis-4-{[4-(dimethylamino)-5-methylpyrimidin-2-yl]amino}cyclohexyl) methyl]-4-(trifluoromethyl)benzamide | 436 (M + H) | 3 |
| 2699 | N-[(cis-4-{[4-(dimethylamino)-5-methylpyrimidin-2-yl]amino]cyclohexyl)methyl]-3-(trifluoromethoxy)benzamide | 452 (M + H) | 2 |
| 2700 | N-[(cis-4-{[4-(dimethylamino)-5-methylpyrimidin-2-yl]amino]cyclohexyl)methyl]-4-(trifluoromethoxy)benzamide | 452 (M + H) | 2 |
| 2701 | 4-cyano-N-[(cis-4-{[4-(dimethylamino)-5-methylpyrimidin-2-yl]amino] cyclohexyl)methyl]benzamide | 393 (M + H) | 2 |
| 2702 | 4-bromo-N-[(cis-4-{[4-(dimethylamino)-5-methylpyrimidin-2-yl]amino] cyclohexyl)methyl]benzamide | 446 (M + H) | 1 |
| 2703 | 4-bromo-N-[(cis-4-{[4-(dimethylamino)-5-methylpyrimidin-2-yl]amino] cyclohexyl)methyl]-3-methylbenzamide | 460 (M + H) | 1 |
| 2704 | 3-chloro-N-[(cis-4-{[4-(dimethylamino)-5-methylpyrimidin-2-yl]amino] cyclohexyl)methyl)-4-fluorobenzamide | 420 (M + H) | 1 |
| 2705 | N-[(cis-4-{[4-(dimethylamino)-5-methylpyrimidin-2-yl]amino}cyclohexyl)methyl]-3-fluoro-4-(trifluoromethyl)benzamide | 454 (M + H) | 2 |
| 2706 | 3,5-dichloro-N-[(cis-4-{[4-(dimethylamino)-5-methylpyrimidin-2-yl]amino} cyclohexyl)methyl]benzamide | 436 (M + H) | 1 |
| 2707 | 3,4-dichloro-N-[(cis-4-{[4-(dimethylamino)-5-methylpyrimidin-2-yl]amino} cyclohexyl)methyl]benzamide | 436 (M + H) | 1 |
| 2708 | N-[(cis-4-{[4-(dimethylamino)-5-methylpyrimidin-2-yl]amino}cyclohexyl)methyl]-2,2-difluoro-1,3-benzodioxole-5-carboxamide | 448 (M + H) | 2 |
| 2709 | N-[(cis-4-{[4-(dimethylamino)-5-methylpyrimidin-2-yl]amino}cyclohexyl)methyl] biphenyl-4-carboxamide | 444 (M + H) | 3 |
| 2710 | 4-chloro-N-[(cis-4-{[4-(dimethylamino)-5-methylpyrimidin-2-yl]amino} cyclohexyl)methyl]benzamide | 402 (M + H) | 2 |
| 2711 | N-[(cis-4-{[4-(dimethylamino)-6-methylpyrimidin-2-yl]amino }cyclohexyl) methyl]-3,5-dimethoxybenzamide | 428 (M + H) | 2 |
| 2712 | N-(cis-4-({[4-(dimethylamino)-5-methylpyrimidin-2-yl]amino}methyl)cyclohexyl] benzamide | 368 (M + H) | 3 |
| 2713 | N-[cis-4-({[4-(dimethylamino)-5-methylpyrimidin-2-yl]amino}methyl)cyclohexyl]-4-methylbenzamide | 382(M + H) | 3 |
| 2714 | N-[cis-4-({[4-(dimethylamino)-5-methylpyrimidin-2-yl]amino}methyl)cyclohexyl]-3,4-difluorobenzamide | 404 (M + H) | 3 |
| 2715 | N-[cis-4-({[4-(dimethylamino)-5-methylpyrimidin-2-yl]amino}methyl)cyclohexyl]-4-methoxybenzamide | 398 (M + H) | 3 |
| 2716 | N-[cis-4-({[4-(dimethylamino)-5-methylpyrimidin-2-yl]amino}methyl)cyclohexyl]-3,5-dimethoxybenzamide | 428 (M + H) | 2 |
| 2717 | N-[cis-4-({[4-(dimethylamino)-5-methylpyrimidin-2-yl]amino}methyl)cyclohexyl]-3-fluoro-4-methylbenzamide | 400 (M + H) | 3 |
| 2718 | N-[cis-4-(1[4-(dimethylamino)-5-methylpyrimidin-2-yl]amino}methyl) cyclohexyl]-4-fluoro-3-methylbenzamide | 400 (M + H) | 2 |

(continued)

| Ex. No. | compound name | MS | class |
|---|---|---|---|
| 2719 | N-[cis-4-({[4-(dimethylamino)-5-methylpyrimidin-2-yl]amino}methyl)cyclohexyl]-3-(trifluoromethyl)benzamide | 436 (M + H) | 3 |
| 2720 | N-[cis-4-({[4-(dimethylamino)-5-methylpyrimidin-2-yl]amino}methyl)cyclohexyl]-4-(trifluoromethyl)benzamide | 436 (M + H) | 3 |
| 2721 | N-[cis-4-({[4-(dimethylamino)-5-methylpyrimidin-2-yl]amino }methyl) cyclohexyl]-3-(triftuoromethoxy)benzamide | 452 (M + H) | 3 |
| 2722 | N-(cis-4-({[4-(dimethylamino)-5-methylpyrimidin-2-yl]amino}methyl) cyclohexyl]-4-(trifluoromethoxy)benzamide | 452 (M + H) | 3 |
| 2723 | 4-cyano-N-[cis-4-({[4-(dimethylamino)-5-methylpyrimidin-2-yl]amino}methyl) cyclohexyl]benzamide | 393 (M + H) | 3 |
| 2724 | 4-bromo-N-[cis-4-({[4-(dimethylamino)-5-methylpyrimidin-2-yl]amino}methyl) cyclohexyl]benzamide | 446 (M + H) | 3 |
| 2725 | 4-bromo-N-[cis-4-({[4-(dimethylamino)-5-methylpyrimidin-2-yl]amino}methyl) cyclohexyl]-3-methylbenzamide | 460 (M + H) | 2 |
| 2726 | 3-chloro-N-[cis-4-({[4-(dimethylamino)-5-methylpyrimidin-2-yl]amino}methyl) cyclohexyl]-4-fluorobenzamide | 420 (M + H) | 2 |
| 2727 | N-[cis-4-({[4-(dimethylamino)-5-methylpyrimidin-2-yl]amino}methyl)cyclohexyl]-3-fluoro-4-(trifluoromethyl)-benzamide | 454 (M + H) | 3 |
| 2728 | '3,5-dichloro-N-[cis-4-({[4-(dimethylamino)-5-methylpyrimidin-2-yl]amino} methyl)cyclohexyl]benzamide | 436 (M + H) | 2 |
| 2729 | 3,4-dichloro-N-[cis-4-({[4-(dimethylamino)-5-methylpyrimidin-2-yl]amino} methyl)cyclohexyl]benzamide | 436 (M + H) | 3 |
| 2730 | N-[cis-4-({[4-(dimethylamino)-5-methylpyrimidin-2-yl]amino}methyl)cyclohexyl]-2,2-difluoro-1,3-benzodioxole-5-carboxamide | 448 (M + H) | 3 |
| 2731 | N-[cis-4-({[4-(dimethylamino)-5-methylpyrimidin-2-yl]amino}methyl)cyclohexyl]-3,5-bis(trifluoromethyl)benzamide | 504 (M + H) | 2 |
| 2732 | N-[cis-4-({[4-(dimethylamino)-6-methylpyrimidin-2-yl]amino}methyl)cyelohexyl] benzamide | 368 (M+H) | 3 |
| 2733 | N-[cis-4-({[4-(dimethylamino)-6-methylpyrimidin-2-yl]amino} methyl) cyclohexyl]-4-methylbenzamide | 382 (M+H) | 3 |
| 2734 | N-[cis-4-({[4-(dimethylamino)-6-methylpyrimidin-2-yl]amino} methyl) cyclohexyl]-3,4-difluorobenzamide | 404 (M + H) | 3 |
| 2735 | N-[cis-4-({[4-(dimethylamino)-6-methylpyrimidin-2-yl]amino}methyl)cyclohexyl]-4-methoxybenzamide | 398 (M + H) | 3 |
| 2736 | N-[cis-4-({[4-(dimethylamino)-6-methylpyrimidin-2-yl]amino}methyl)cyclohexyl]-3,5-dimethoxybenzamide | 428 (M + H) | 3 |
| 2737 | N-[cis-4-({[4-(dimethylamino)-6-methylpyrimidin-2-yl]amino}methyl)cyclohexyl]-3-fluoro-4-methylbenzamide | 400 (M + H) | 3 |
| 2738 | N-[cis-4-({[4-(dimethylamino)-6-methylpyrimidin-2-yl]amino}methyl)cyclohexyl]-4-fluoro-3-methylbenzamide | 400 (M + H) | 3 |
| 2739 | N-[cis-4-({[4-(dimethylamino)-6-methylpyrimidin-2-yl]amino}methyl)cyclohexyl]-3-(trifluoromethyl)benzamide | 436 (M + H) | 3 |
| 2740 | N-[cis-4-({[4-(dimethylamino)-6-methylpyrimidin-2-yl]amino}methyl)cyclohexyl]-4-(trifluoromethyl)benzamide | 436 (M + H) | 3 |

(continued)

| Ex. No. | compound name | MS | class |
|---|---|---|---|
| 2741 | N-[cis-4-({[4-(dimethylamino)-6-methylpyrimidin-2-yl]amino}methyl)cyclohexyl]-3-(trifluoromethoxy)benzamide | 452 (M + H) | 3 |
| 2742 | N-[cis-4-({[4-(dimethylamino)-6-methylpyrimidin-2-yl]amino}methyl)cyclohexyl]-4-(trifluoromethoxy)benzamide | 452 (M + H) | 3 |
| 2743 | 4-cyano-N-[cis-4-({[4-(dimethylamino)-6-methylpyrimidin-2-yl]amino}methyl) cyclohexyl]benzamide | 393 (M + H) | 3 |
| 2744 | 4-bromo-N-[cis-4-({[4-(dimethylamino)-6-methylpyrimidin-2-yl]amino}methyl) cyclohexyl]benzamide | 446 (M + H) | 2 |
| 2745 | 4-bromo-N-[cis-4-({[4-(dimethylamino)-6-methylpyrimidin-2-yl]amino}methyl) cyclohexyl]-3-methylbenzamide | 460 (M + H) | 2 |
| 2746 | 3-chloro-N-[cis-4-({[4-(dimethylamino)-6-methylpyrimidin-2-yl]amino}methyl) cyclohexyl]-4-fluorobenzamide | 420 (M + H) | 3 |
| 2747 | N-[cis-4-({[4-(dimethylamino)-6-methylpyrimidin-2-yl]amino]methyl)cyclohexyl]-3-fluoro-4-(trifluoromethyl)-benzamide | 454 (M + H) | 3 |
| 2748 | 3,5-dichloro-N-[cis-4-({[4-(dimethylamino)-6-methylpyrimidin-2-yl]amino} methyl)cyclohexyl]benzamide | 436 (M + H) | 2 |
| 2749 | 3,4-dichloro-N-[cis-4-({[4-(dimethylamino)-6-methylpyrimidin-2-yl]amino} methyl)cyclohexyl]benzamide | 436 (M + H) | 2 |
| 2750 | N-[cis-4-({[4-(dimethylamino)-6-methylpyrimidin-2-yl]amino}methyl)cyclohexyl]-2,2-difluoro-1,3-benzodioxole-5-carboxamide | 448 (M + H) | 3 |
| 2751 | N-[(cis-4-{[4-(dimethylamino)-6-methylpyrimidin-2-yl]amino}cyclohexyl)methyl] benzamide | 368 (M + H) | 3 |
| 2752 | N-[(cis-4-{[4-(dimethylamino)-6-methylpyrimidin-2-yl]amino}cyclohexyl)methyl]-4-methylbenzamide | 382 (M + H) | 3 |
| 2753 | N-[(cis-4-{[4-(dimethylamino)-6-methylpyrimidin-2-yl]amino}cyclohexyl)methyl]-3,4-difluorobenzamide | 404 (M + H) | 3 |
| 2754 | N-[(cis-4-{[4-(dimethylamino)-6-methylpyrimidin-2-yl]amino}cyclohexyl)methyl]-4-methoxybenzamide | 398 (M + H) | 3 |
| 2755 | N-[(cis-4-{[4-(dimethylamino)-6-methylpyrimidin-2-yl]amino}cyclohexyl)methyl]-3-fluoro-4-methylbenzamide | 400 (M + H) | 2 |
| 2756 | N-[(cis-4-{[4-(dimethylamino)-6-methylpyrimidin-2-yl]amino}cyclohexyl)methyl]-4-fluoro-3-methylbenzamide | 400 (M + H) | 3 |
| 2757 | N-[(cis-4-{[4-(dimethylamino)-6-methylpyrimidin-2-yl]amino}cyclohexyl)methyl]-3-(trifluoromethyl)benzamide | 436 (M + H) | 3 |
| 2758 | N-[(cis-4-{[4-(dimethylamino)-6-methylpyrimidin-2-yl]amino}cyclohexyl)methyl]-4-(trifluoromethyl)benzamide | 436 (M + H) | 2 |
| 2759 | N-[(cis-4-{[4-(dimethylamino)-6-methylpyrimidin-2-yl]amino}cyclohexyl)methyl]-3-(trifluoromethoxy)benzamide | 452 (M + H) | 3 |
| 2760 | N-[(cis-4-{[4-(dimethylamino)-6-methylpyrimidin-2-yl]amino}cyclohexyl)methyl]-4-(trifluoromethoxy)benzamide | 452 (M + H) | 3 |
| 2761 | 4-cyano-N-[(cis-4-{[4-(dimethylamino)-6-methylpyrimidin-2-yl]amino} cyclohexyl)methyl]benzamide | 393 (M + H) | 3 |
| 2762 | 4-bromo-N-[(cis-4-{[4-(dimethylamino)-6-methylpyrimidin-2-yl]amino} cyclohexyl)methyl]benzamide | 446 (M + H) | 1 |

(continued)

| Ex. No. | compound name | MS | class |
|---|---|---|---|
| 2763 | 4-bromo-N-[(cis-4-{[4-(dimethylamino)-6-methylpyrimidin-2-yl]amino} cyclohexyl)methyl]-3-methylbenzamide | 460 (M + H) | 1 |
| 2764 | 3-chloro-N-[(cis-4-{[4-(dimethylamino)-6-methylpyrimidin-2-yl]amino} cyclohexyl)methyl]-4-fluorobenzamide | 420 (M + H) | 1 |
| 2765 | N-[(cis-4-{[4-(dimethylamino)-6-methylpyrimidin-2-yl]amino}cyclohexyl)methyl]-3-fluoro-4-(trifluoromethyl)-benzamide | 454 (M + H) | 2 |
| 2766 | 3,5-dichloro-N-[(cis-4-{[4-(dimethylamino)-6-methylpyrimidin-2-yl]amino} cyclohexyl)methyl]benzamide | 436 (M + H) | 2 |
| 2767 | 3,4-dichloro-N-[(cis-4-{[4-(dimethylamino)-6-methylpyrimidin-2-yl]amino} cyclohexyl)methyl]benzamide | 436 (M + H) | 2 |
| 2768 | N-[(cis-4-{[4-(dimethylamino)-6-methylpyrimidin-2-yl]amino}cyclohexyl)methyl]-2,2-difluoro-1,3-benzodioxole-5-carboxamide | 448 (M + H) | |
| 2769 | N-[(cis-4-{[4-(dimethylamino)-6-methylpyrimidin-2-yl]amino}cyclohexyl)methyl] biphenyl-4-carboxamide | 444 (M + H) | |
| 2770 | 4-chloro-N-[(cis-4-{[4-(dimethylamino)-6-methylpyrimidin-2-yl]amino} cyclohexyl)methyl]benzamide | 402 (M + H) | 2 |
| 2771 | cis-4-{[4-(dimethylamino)-5-methylpyrimidin-2-yl]amino}-N-[(1R)-1-phenylethyl] cyclohexanecarboxamide | 382 (M + H) | 2 |
| 2772 | cis-4-{[4-(dimethylamino)-5-methylpyrimidin-2-yl]amino}-N-[(1S)-1-(4-methylphenyl)ethyl]cyclohexanecarboxamide | 396 (M + H) | 1 |
| 2773 | cis-4-{[4-(dimethylamino)-5-methylpyrimidin-2-yl]amino}-N-[(1R)-1-(4-fluorophenyl)ethyl]cyclohexanecarboxamide | 400 (M + H) | 1 |
| 2774 | cis-4-{[4-(dimethylamino)-5-methylpyrimidin-2-yl]amino}-N-[(1S)-1-(4-fluorophenyl)ethyl]cyclohexanecarboxamide | 400 (M + H) | 2 |
| 2775 | cis-4-{[4-(dimethylamino)-5-methylpyrimidin-2-yl]amino}-N-[(1R)-1-(3-methoxyphenyl)ethyl]cyclohexanecarboxamide | 412 (M + H) | 1 |
| 2776 | cis-4-{[4-(dimethylamino)-5-methylpyrimidin-2-yl]amino}-N-[(1S)-1-(3-methoxyphenyl)ethyl]cyclohexanecarboxamide | 412 (M + H) | 1 |
| 2777 | cis-4-{[4-(dimethylamino)-5-methylpyrimidin-2-yl]amino}-N-[(1S)-1-(4-methoxyphenyl)ethyl]cyclohexanecarboxamide | 412 (M + H) | 1 |
| 2778 | cis-N-[(1R)-1-(4-chlorophenyl)ethyl]-4-{[4-(dimethylamino)-5-methylpyrimidin-2-yl]amino}cyclohexanecarboxamide | 416 (M + H) | 1 |
| 2779 | cis-N-[1-(4-bromophenyl)ethyl]-4-{[4-(dimethylamino)-5-methylpyrimidin-2-yl] amino}cyclohexanecarboxamide | 460 (M + H) | 1 |
| 2780 | cis-4-{[4-(dimethylamino)-5-methylpyrimidin-2-yl]amino}-N-[(1R)-1-(4-nitrophenyl)ethyl]cyclohexanecarboxamide | 427 (M + H) | 1 |
| 2781 | cis-4-{[4-(dimethylamino)-5-methylpyrimidin-2-yl]amino}-N-[(1S)-1-(4-nitrophenyl)ethyl]cyclohexanecarboxamide | 427 (M + H) | 2 |
| 2782 | cis-4-{[4-(dimethylamino)-5-methylpyrimidin-2-yl]amino}-N-[(1R)-1-(1-naphthyl) ethyl]cyclohexanecarboxamide | 432 (M + H) | 1 |
| 2783 | cis-4-{[4-(dimethylamino)-5-methylpyrimidin-2-yl]amino}-N-[(1S)-1-(1-naphthyl) ethyl]cyclohexanecarboxamide | 432 (M + H) | 1 |
| 2784 | cis-4-{(4-(dimethylamino)-5-methylpyrimidin-2-yl]amino}-N-(3-fluorophenyl) cyclohexanecarboxamide | 372 (M + H) | 2 |

(continued)

| Ex. No. | compound name | MS | class |
|---|---|---|---|
| 2785 | cis-4-{[4-(dimethylamino)-5-methylpyrimidin-2-yl]amino}-N-(4-propylphenyl) cyclohexanecarboxamide | 396 (M + H) | 2 |
| 2786 | cis-4-{[4-(dimethylamino)-5-methylpyrimidin-2-yl]amino}-N-(4-methoxyphenyl) cyclohexanecarboxamide | 396(M+H) | 2 |
| 2787 | cis-4-{[4-(dimethylamino)-5-methylpyrimidin-2-yl]amino}-N-(3-methoxyphenyl) cyclohexanecarboxamide | 384 (M + H) | 1 |
| 2788 | cis-N-(3-chlorophenyl)-4-{[4-(dimethylamino)-5-methylpyrimidin-2-yl]amino} cyclohexanecarboxamide | 388 (M + H) | 1 |
| 2789 | cis-N-(2-bromophenyl)-4-{[4-(dimethylamino)-5-methylpyrimidin-2-yl]amino} cyclohexanecarboxamide | 432 (M + H) | 3 |
| 2790 | cis-4-{[4-(dimethylamino)-5-methylpyrimidin-2-yl]amino}-N-[(1S,2R)-2-phenylcyclopropyl]cyclohexanecarboxamide | 394 (M + H) | 1 |
| 2791 | cis-4-{[4-(dimethylamino)-5-methylpyrimidin-2-yl]amino}-N-[4-(trifluoromethyl) phenyl]cyclohexanecarboxamide | 422 (M + H) | 1 |
| 2792 | cis-4-{[4-(dimethylamino)-5-methylpyrimidin-2-yl]amino}-N-[2-(methylthio) phenyl]cyclohexanecarboxamide | 400 (M + H) | 2 |
| 2793 | N2-[cis-4-(3,4-dihydroisoquinolin-2(1H)-ylcarbonyl)cyclohexyl] -N4,N4,5-trimethylpyrimidine-2,4-diamine | 394 (M + H) | 3 |
| 2794 | cis-N-(4-chlorophenyl)-4-{[4-(dimethylamino)-5-methylpyrimidin-2-yl]amino}-N-methylcyclohexanecarboxamide | 402 (M + H) | |
| 2795 | cis-4-{[4-(dimethylamino)-6-methylpyrimidin-2-yl]amino}-N-[(1S)-1-(4-methylphenyl)ethyl]cyclohexanecarboxamide | 396 (M + H) | 1 |
| 2796 | cis-4-{[4-(dimethylamino)-6-methylpyrimidin-2-yl]amino}-N-[(1R)-1-(3-methoxyphenyl)ethyl]cyclohexanecarboxamide | 412 (M + H) | 2 |
| 2797 | cis-N-[(1S)-1-(4-chlorophenyl)ethyl]-4-{[4-(dimethylamino)-6-methylpyrimidin-2-yl]amino}cyclohexanecarboxamide | 416 (M + H) | 1 |
| 2798 | cis-N-benzyl-4-{[4-(dimethylamino)-6-methylpyrimidin-2-yl]amino} cyclohexanecarboxamide | 368 (M + H) | 2 |
| 2799 | cis-4-{[4-(dimethylamino)-6-methylpyrimidin-2-yl]amino}-N-(4-fluorobenzyl) cyclohexanecarboxamide | 386 (M + H) | 2 |
| 2800 | cis-4-{[4-(dimethylamino)-6-methylpyrimidin-2-yl]amino}-N-(2-fluorobenzyl) cyclohexanecarboxamide | 386 (M + H) | 2 |
| 2801 | cis-N-(3,4-difluorobenzyl)-4-{[4-(dimethylamino)-6-methylpyrimdin-2-yl]amino} cyclohexanecarboxamide | 404 (M + H) | 1 |
| 2802 | cis-4-{[4-(dimethylamino)-6-methylpyrimidin-2-yl]amino}-N-[(1S)-1-(4-methoxyphenyl)ethyl]cyclohexanecarboxamide | 412 (M + H) | 1 |
| 2803 | cis-4-{[4-(dimethylamino)-6-methylpyrimidin-2-yl]amino}-N-[(1S)-1-(3-methoxyphenyl)ethyl]cyclohexanecarboxamide | 412 (M + H) | 1 |
| 2804 | cis-4-{[4-(dimethylamino)-6-methylpyrimidin-2-yl]amino}-N- [(1R)-1-(4-fluorophenyl)ethyl]cyclohexanecarboxamide | 400 (M + H) | 2 |
| 2805 | cis-N-[(1R)-1-(4-chlorophenyl)ethyl]-4-{[4-(dimethylamino)-6-methylpyrimidin-2-yl]amino}cyclohexanecarboxamide | 416 (M + H) | 1 |
| 2806 | cis-4-{[4-(dimethylamino)-6-methylpyrimidin-2-yl]amino}-N-(3-iodobenzyl) cyclohexanecarboxamide | 494 (M + H) | 1 |

(continued)

| Ex. No. | compound name | MS | class |
|---|---|---|---|
| 2807 | cis-N-(2,4-dichlorobenzyl)-4-{[4-(dimethylamino}-6-methylpyrimidin-2-yl]amino cyclohexanecarboxamide | 436 (M + H) | 1 |
| 2808 | cis-N-(2,5-dichlorobenzyl)-4-{[4-(dimethylamino)-6-methylpyrimidin-2-yl]amino} cyclohexanecarboxamide | 436 (M + H) | 1 |
| 2809 | cis-4-{[4-(dimethylamino)-6-methylpyrimidin-2-yl]amino}-N-(4-methylbenzyl) cyclohexanecarboxamide | 382 (M + H) | 1 |
| 2810 | cis-N-(3,5-dichlorobenzyl)-4-{[4-(dimethylamino)-6-methylpyrimidin-2-yl]amino} cyclohexanecarboxamide | 436 (M + H) | 1 |
| 2811 | cis-N-(3,5-dimethoxybenzyl)-4-{[4-(dimethylamino)-6-methylpyrimidin-2-yl] amino}cyclohexanecarboxamide | 428 (M + H) | 1 |
| 2812 | cis-N-(3-chlorobenzyl)-4-{[4-(dimethylamino)-6-methylpyrimidin-2-yl]amino} cyclohexanecarboxamide | 402 (M + H) | 1 |
| 2813 | cis-4-{[4-(dimethylamino)-6-methylpyrimidin-2-yl]amino}-N-[3-(trifluoromethyl) benzyl]cyclohexanecarboxamide | 436 (M + H) | 2 |
| 2814 | cis-N-[3,5-bis(trifluoromethyl)benzyl]-4-{[4-(dimethylamino)-6-methylpyrimidin-2-yl]amino}cyclohexanecarboxamide | 504 (M + H) | 1 |
| 2815 | cis-4-{[4-(dimethylamino)-6-methylpyrimidin-2-yl]amino}-N-(3-methoxybenzyl) cyclohexanecarboxamide | 398 (M + H) | 1 |
| 2816 | cis-N-(4-chlorobenzyl)-4-{[4-(dimethylamino)-6-methylpyrimidin-2-yl]amino} cyclohexanecarboxamide | 402 (M + H) | 1 |
| 2817 | cis-N-(3,4-dichlorobenzyl)-4-{[4-(dimethylamino)-6-methylpyrimidin-2-yl]amino} cyclohexanecarboxamide | 436 (M + H) | 1 |
| 2818 | cis-N-(2,4-difluorobenzyl)-4-{[4-(dimethylamino)-6-methylpyrimidin-2-yl]amino} cyclohexanecarboxamide | 404 (M + H) | 1 |
| 2819 | cis-N-(2,5-difluorobenzyl)-4-{[4-(dimethylamino)-6-methylpyrimidin-2-yl]amino} cyclohexanecarboxamide | 404 (M + H) | 1 |
| 2820 | cis-N-(2,3-difluorobenzyl)-4-{[4-(dimethylamino)-6-methylpyrimidin-2-yl]amino} cyclohexanecarboxamide | 404 (M + H) | 1 |
| 2821 | cis-N-(4-bromo-2-fluorobenzyl)-4-{[4-(dimethylamino)-6-methylpyrimidin-2-yl] amino}cyclohexanecarboxamide | 464 (M + H) | 1 |
| 2822 | cis-4-{[4-(dimethylamino)-6-methylpyrimidin-2-yl]amino}-N-(3-methylbenzyl) cyclohexanecarboxamide | 382 (M + H) | 1 |
| 2823 | cis-4-{[4-(dimethylamino)-6-methylpyrimidin-2-yl]amino} -N-[2-(trifluoromethoxy)benzyl]cyclohexanecarboxamide | 452 (M + H) | 1 |
| 2824 | N-(cis-4-{[4-(dimethylamino)-5,6-dimethylpyrimidin-2-yl]amino}cyclohexyl)-3-methoxybenzamide | 398 (M + H) | 1 |
| 2825 | N-(cis-4-{[4-(dimethylamino)-5,6-dimethylpyrimidin-2-yl]amino}cyclohexyl)-2,6-dihydroxyisonicotinamide | 401 (M + H) | 3 |
| 2826 | N-(cis-4-{[4-(dimethylamino)-5,6-dimethylpyrimidin-2-yl]amino}cyclohexyl) pyrazine-2-carboxamide | 370 (M + H) | 3 |
| 2827 | N-(cis-4-{[4-(dimethylamino)-5,6-dimethylpyrimidin-2-yl]amino}cyclohexyl)-6-hydroxynicotinamide | 385 (M + H) | 3 |
| 2828 | N-(cis-4-{[4-(dimethylamino)-5,6-dimethylpyrimidin-2-yl]amino}cyclohexyl)-5-methylisoxazole-3-carboxamide | 373 (M + H) | 2 |

(continued)

| Ex. No. | compound name | MS | class |
|---|---|---|---|
| 2829 | 2-(3,5-difluorophenyl)-N-(cis-4-{[4-(dimethylamino)-5,6-dimethylpyrimidin-2-yl] amino}cyclohexyl)-2-hydroxyacetamide | 434 (M + H) | 2 |
| 2830 | N-(cis-4-{[4-(dimethylamino)-5,6-dimethylpyrimidin-2-yl]amino}cyclohexyl)-2-methyl-1,3-oxazole-4-carboxamide | 373 (M + H) | 2 |
| 2831 | N-(cis-4-{[4-(dimethylamino)-5,6-dimethylpyrimidin-2-yl]amino}cyclohexyl)-2-methylnicotinamide | 383 (M + H) | 3 |
| 2832 | N-(cis-4-{[4-(dimethylamino)-5,6-dimethylpyrimidin-2-yl]amino}cyclohexyl)-2,6-dimethoxynicotinamide | 429 (M + H) | 1 |
| 2833 | 3-amino-N-(cis-4-{[4-(dimethylamino)-5,6-dimethylpyrimidin-2-yl]amino} cyclohexyl)pyrazine-2-carboxamide | 385 (M + H) | 2 |
| 2834 | N-(cis-4-{[4-(dimethylamino)-5,6-dimethylpyrimidin-2-yl]amino}cyclohexyl)-2-ethoxynicotinamide | 413 (M + H) | 3 |
| 2835 | N-(cis-4-{[4-(dimethylamino)-5,6-dimethylpyrimidin-2-yl]amino}cyclohexyl) pyridine-2-carboxamide | 369 (M + H) | 3 |
| 2836 | 3-cyano-N-(cis-4-{[4-(dimethylamino)-5,6-dimethylpyrimidin-2-yl]amino} cyclohexyl)benzamide | 393 (M + H) | 1 |
| 2837 | N-(cis-4-{[4-(dimethylamino)-5,6-dimethylpyrimidin-2-yl]amino}cyclohexyl)-3-methylbenzamide | 382 (M + H) | 1 |
| 2838 | 3-chloro-N-(cis-4-{[4-(dimethylamino)-5,6-dimethylpyrimidin-2-yl]amino} cyclohexyl)benzamide | 402 (M + H) | 1 |
| 2839 | 3-bromo-N-(cis-4-{[4-(dimethylamino)-5,6-dimethylpyrimidin-2-yl]amino} cyclohexyl)benzamide | 446 (M + H) | 1 |
| 2840 | N-(cis-4-{[4-(dimethylamino)-5,6-dimethylpyrimidin-2-yl]amino}cyclohexyl)-3,5-dimethoxybenzamide | 428 (M + H) | 1 |
| 2841 | N-(cis-4-{[4-(dimethylamino)-5,6-dimethylpyrimidin-2-yl]amino}cyclohexyl)-3,5-bis(trifluoromethyl)benzamide | 504 (M + H) | 1 |
| 2842 | 3,4-dichloro-N-(cis-4-{[4-(dimethylamino)-5,6-dimethylpyrimidin-2-yl]amino} cyclohexyl)benzamide | 436 (M + H) | 1 |
| 2843 | N-(cis-4-{[4-(dimethylamino)-5,6-dimethylpyrimidin-2-yl]amino}cyclohexyl)-4-(trifluoromethoxy)benzamide | 452 (M + H) | 2 |
| 2844 | 4-cyano-N-(cis-4-{[4-(dimethylamino)-5,6-dimethylpyrimidin-2-yl]amino} cyclohexyl)benzamide | 393 (M + H) | 1 |
| 2845 | N-(cis-4-{[4-(dimethylamino)-5,6-dimethylpyrimidin-2-yl]amino}cyclohexyl)-4-methylbenzamide | 382 (M + H) | 1 |
| 2846 | N-(cis-4-{[4-(dimethylamino)-5,6-dimethylpyrimidin-2-yl]amino}cyclohexyl)-4-fluorobenzamide | 386 (M + H) | 1 |
| 2847 | 4-chloro-N-(cis-4-{[4-(dimethylamino)-5,6-dimethylpyrimidin-2-yl]amino} cyclohexyl)benzamide | 402 (M + H) | 1 |
| 2848 | N-(cis-4-{[4-(dimethylamino)-5,6-dimethylpyrimidin-2-yl]amino}cyclohexyl)-2-methoxybenzamide | 398 (M + H) | 2 |
| 2849 | 4-bromo-N-(cis-4-{[4-(dimethylamino)-5,6-dimethylpyrimidin-2-yl]amino} cyclohexyl)benzamide | 446 (M + H) | 1 |
| 2850 | N-(cis-4-{[4-(dimethylamino)-5,6-dimethylpyrimidin-2-yl]amino}cyclohexyl)-4-(trifluoromethyl)benzamide | 436 (M + H) | 1 |

(continued)

| Ex. No. | compound name | MS | class |
|---|---|---|---|
| 2851 | N-(cis-4-{[4-(dimethylamino)-5,6-dimethylpyrimidin-2-yl]amino}cyclohexyl)-4-ethoxybenzamide | 412 (M + H) | 3 |
| 2852 | 4-bromo-N-(cis-4-{[4-(dimethylamino)-5,6-dimethylpyrimidin-2-yl]amino}cyclohexyl)-3-methylbenzamide | 460 (M + H) | 1 |
| 2853 | N-(cis-4-{[4-(dimethylamino)-5,6-dimethylpyrimidin-2-yl]amino}cyclohexyl)-3-fluoro-4-methylbenzamide | 400 (M + H) | 1 |
| 2854 | N-(cis-4-{[4-(dimethylamino)-5,6-dimethylpyrimidin-2-yl]amino}cyclohexyl)-4-fluoro-3-methylbenzamide | 400 (M + H) | 1 |
| 2855 | N-(cis-4-{[4-(dimethylamino)-5,6-dimethylpyrimidin-2-yl]amino}cyclohexyl)-3-ethylbenzamide | 396 (M + H) | 2 |
| 2856 | N-(cis-4-{[4-(dimethylamino)-5,6-dimethylpyrimidin-2-yl]amino}cyclohexyl)-3-(trifluoromethoxy)benzamide | 452 (M + H) | 1 |
| 2857 | 5-bromo-N-(cis-4-{[4-(dimethylamino)-5,6-dimethylpyrimidin-2-yl]amino}cyclohexyl)nicotinamide | 447 (M + H) | 1 |
| 2858 | N-(cis-4-{[4-(dimethylamino)-5,6-dimethylpyrimidin-2-yl]amino}cyclohexyl)-5-methylthiophene-2-carboxamide | 388 (M + H) | 1 |
| 2859 | N-(cis-4-{[4-(dimethylamino)-5,6-dimethylpyrimidin-2-yl]amino}cyclohexyl)-6-(trifluoromethyl)nicotinamide | 437 (M + H) | 2 |
| 2860 | N-(cis-4-{[4-(dimethylamino)-5,6-dimethylpyrimidin-2-yl]amino}cyclohexyl)-3,5-diethoxybenzamide | 456 (M + H) | 1 |
| 2861 | N-(cis-4-{[4-(dimethylamino)-5,6-dimethylpyrimidin-2-yl]amino}cyclohexyl)-3-ethoxybenzamide | 412 (M + H) | 1 |
| 2862 | N-(cis-4-{[4-(dimethylamino)-5,6-dimethylpyrimidin-2-yl]amino}cyclohexyl)-3-isopropoxybenzamide | 426 (M + H) | 1 |
| 2863 | N-(cis-4-{[4-(dimethylamino)-5,6-dimethylpyrimidin-2-yl]amino}cyclohexyl)-6-hydroxypyridine-2-carboxamide | 385 (M + H) | 3 |
| 2864 | N-(cis-4-{[4-(dimethylamino)-5,6-dimethylpyrimidin-2-yl]amino}cyclohexyl)-3,4-difluorobenzamide | 404 (M + H) | 1 |
| 2865 | N4,N4,5,6-tetramethyl-N2-(cis-4-{[3-(trifluoromethoxy)benzyl]amino}cyclohexyl)pyrimidine-2,4-diamine | 438 (M + H) | 3 |
| 2866 | N2-{cis-4-[(3,4-difluorobenzyl)amino]cyclohexyl}-N4,N4,5,6-tetramethylpyrimidine-2,4-diamine | 390 (M + H) | 2 |
| 2867 | N-(3,4-dimethoxyphenyl)-N'-(cis-4-{[4-(dimethylamino)-5,6-dimethylpyrimidin-2-yl]amino}cyclohexyl)urea | 433 (M + H) | 1 |
| 2868 | N-(cis-4-{[4-(dimethylamino)-5,6-dimethylpyrimidin-2-yl]amino}cyclohexyl)-N'-(2-ethoxyphenyl)urea | 427 (M + H) | 1 |
| 2869 | N-[4-(benzyloxy)phenyl]-N'-(cis-4-{[4-(dimethylamino)-5,6-dimethylpyrimidin-2-yl]amino}cyclohexyl)urea | 489 (M + H) | 3 |
| 2870 | 1-(4-chlorophenyl)-N-(cis-4-{[4-(dimethylamino)-5-methylpyrimidin-2-yl]amino}cyclohexyl)cyclopropanecarboxamide | 428 (M + H) | 1 |
| 2871 | 1-(2,4-dichlorophenyl)-N-(cis-4-{[4-(dimethylamino)-5-methylpyrimidin-2-yl]amino}cyclohexyl)cyclopropanecarboxamide | 462 (M + H) | 1 |
| 2872 | 2-(4-chlorophenyl)-N-(cis-4-{[4-(dimethylamino)-5-methylpyrimidin-2-yl]amino}cyclohexyl)acetamide | 402 (M + H) | 1 |

(continued)

| Ex. No. | compound name | MS | class |
|---|---|---|---|
| 2873 | N-(cis-4-{[4-(dimethylamino)-5-methylpyrimidin-2-yl]amino}cyclohexyl)-1-(4-methylphenyl)-cyclopropanecarboxamide | 408 (M + H) | 1 |
| 2874 | 2-(4-chlorophenyl)-N-(cis-4-{[4-(dimethylamino)-5-methylpyrimidin-2-yl]amino}cyclohexyl)propanamide | 416 (M + H) | 1 |
| 2875 | N-(cis-4-{[4-(dimethylamino)-5-methylpyrimidin-2-yl]amino}cyclohexyl)-1-(4-methoxyphenyl)-cyclopropanecarboxamide | 424 (M + H) | 1 |
| 2876 | 2-(4-chlorophenyl)-N-(cis-4-{[4-(dimethylamino)-5-methylpyrimidin-2-yl]amino}cyclohexyl)-2-hydroxyacetamide | 418 (M + H) | 1 |
| 2877 | N-(cis-4-{[4-(dimethylamino)-5-methylpyrimidin-2-yl]amino}cyclohexyl)-2-(3-methoxyphenoxy)acetamide | 414 (M + H) | 1 |
| 2878 | N-(cis-4-{[4-(dimethylamino)-5-methylpyrimidin-2-yl]amino}cyclohexyl)-2-[3-(trifluoromethyl)phenoxy]acetamide | 452 (M + H) | 1 |
| 2879 | 2-(3-chlorophenoxy)-N-(cis-4-{[4-(dimethylamino)-5-methylpyrimidin-2-yl]amino}cyclohexyl)acetamide | 418 (M + H) | 1 |
| 2880 | N-(cis-4-{[4-(dimethylamino)-5-methylpyrimidin-2-yl]amino}cyclohexyl)-2-{[2-(trifluoromethyl)phenyl]-sulfinyl}acetamide | 484 (M + H) | 1 |
| 2881 | 2-[(2-chlorophenyl)sulfinyl]-N-(cis-4-{[4-(dimethylamino)-5-methylpyrimidin-2-yl]amino}cyclohexyl)acetamide | 450 (M + H) | 1 |
| 2882 | 2-[(3-bromophenyl)sulfinyl]-N-(cis-4-{[4-(dimethylamino)-5-methylpyrimidin-2-yl]amino}cyclohexyl)acetamide | 494 (M + H) | 1 |
| 2883 | 2-[(3,4-difluorophenyl)sulfinyl]-N-(cis-4-)[4-(dimethylamino)-5-methylpyrimidin-2-yl]amino}cyclohexyl)acetamide | 452 (M+ H) | 2 |
| 2884 | 2-[(3,4-difluorophenyl)sulfonyl]-N-(cis-4-{[4-(dimethylamino)-5-methylpyrimidin-2-yl]amino}cyclohexyl)acetamide | 468 (M + H) | |
| 2885 | N-(cis-4-{[4-(dimethylamino)-5-methylpyrimidin-2-yl]amino}cyclohexyl)-2-(3-methylphenoxy)nicotinamide | 461 (M + H) | 1 |
| 2886 | N-(cis-4-{[4-(dimethylamino)-5-methylpyrimidin-2-yl]amino}cyclohexyl)-2-(3-fluorophenoxy)nicotinamide | 465 (M + H) | 1 |
| 2887 | N-(cis-4-{[4-(dimethylamino)-5-methylpyrimidin-2-yl]amino}cyclohexyl)-2-(3-methoxyphenoxy)nicotinamide | 477 (M + H) | 1 |
| 2888 | N-(cis-4-{[4-(dimethylamino)-5-methylpyrimidin-2-yl]amino}cyclohexyl)-2-(4-methoxyphenoxy)nicotinamide | 477 (M + H) | 1 |
| 2889 | N-(cis-4-{[4-(dimethylamino)-5-methylpyrimidin-2-yl]amino}cyclohexyl)-2-(4-iodophenoxy)nicotinamide | 573 (M + H) | 1 |
| 2890 | N-(cis-4-{[4-(dimethylamino)-5-methylpyrimidin-2-yl]amino}cyclohexyl)-2-(2-methoxyphenoxy)nicotinamide | 477 (M + H) | 1 |
| 2891 | N-(cis-4-{[4-(dimethylamino)-5-methylpyrimidin-2-yl]amino}cyclohexyl)-2-(2-fluorophenoxy)nicotinamide | 465 (M + H) | 1 |
| 2892 | 2-(2-chlorophenoxy)-N-(cis-4-{[4-(dimethylamino)-5-methylpyrimidin-2-yl]amino}cyclohexyl)nicotinamide | 481 (M + H) | 1 |
| 2893 | 2-(3-chlorophenoxy)-N-(cis-4-{[4-(dimethylamino)-5-methylpyrimidin-2-yl]amino}cyclohexyl)nicotinamide | 481 (M + H) | 1 |
| 2894 | 2-(3-bromophenoxy)-N-(cis-4-{[4-(dimethylamino)-5-methylpyrimidin-2-yl]amino}cyclohexyl)nicotinamide | 525 (M + H) | 1 |

(continued)

| Ex. No. | compound name | MS | class |
|---|---|---|---|
| 2895 | N-(cis-4-{[4-(dimethylamino)-5-methylpyrimidin-2-yl]amino}cyclohexyl)-2-[3-(trifluoromethyl)phenoxy]-nicotinamide | 515 (M + H) | 1 |
| 2896 | N-(cis-4-{[4-(dimethylamino)-5-methylpyrimidin-2-yl]amino}cyclohexyl)-3-(trifluoromethyl)benzamide | 422 (M + H) | 1 |
| 2897 | N-(cis-4-{[4-(dimethylamino)-5-methylpyrimidin-2-yl]amino}cyclohexyl)-3-fluorobenzamide | 372 (M + H) | 1 |
| 2898 | 3-bromo-N-(cis-4-{[4-(dimethylamino)-5-methylpyrimidin-2-yl]amino}cyclohexyl)benzamide | 432 (M + H) | 1 |
| 2899 | N-(cis-4-{[4-(dimethylamino)-5-methylpyrimidin-2-yl]amino}cyclohexyl)-4-fluorobenzamide | 372 (M + H) | 1 |
| 2900 | N-(cis-4-{[4-(dimethylamino)-5-methylpyrimidin-2-yl]amino}cyclohexyl)-3,5-dimethoxybenzamide | 414 (M + H) | 1 |
| 2901 | N-(cis-4-{[4-(dimethylamino)-5-methylpyrimidin-2-yl]amino}cyclohexyl)-2,4-difluorobenzamide | 390 (M + H) | 1 |
| 2902 | N-(cis-4-{[4-(dimethylamino)-5-methylpyrimidin-2-yl]amino}cyclohexyl)-2,5-difluorobenzamide | 390 (M + H) | 1 |
| 2903 | N-(cis-4-{[4-(dimethylamino)-5-methylpyrimidin-2-yl]amino}cyclohexyl)-2,3,4-trifluorobenzamide | 408 (M + H) | 1 |
| 2904 | N-(cis-4-{[4-(dimethylamino)-5-methylpyrimidin-2-yl]amino}cyclohexyl) benzamide | 354 (M + H) | 2 |
| 2905 | 4-tert-butyl-N-(cis-4-{[4-(dimethylamino)-5-methylpyrimidin-2-yl]amino}cyclohexyl)benzamide | 410 (M + H) | 1 |
| 2906 | 4-butyl-N-(cis-4-{[4-(dimethylamino)-5-methylpyrimidin-2-yl]amino}cyclohexyl) benzamide | 410 (M + H) | |
| 2907 | 4-chloro-N-(cis-4-{[4-(dimethylamino)-5-methylpyrimidin-2-yl]amino}cyclohexyl)benzamide | 388 (M + H) | 1 |
| 2908 | 3-cyano-N-(cis-4-{[4-(dimethylamino)-5-methylpyrimidin-2-yl]amino}cyclohexyl)benzamide | 379(M + H) | 1 |
| 2909 | 4-cyano-N-(cis-4-{(4-(dimethylamino)-5-methylpyrimidin-2-yl]amino)cyclohexyl)benzamide | 379 (M + H) | 1 |
| 2910 | N-(cis-4-{[4-(dimethylamino)-5-methylpyrimidin-2-yl]amino} cyclohexyl)-2-methoxybenzamide | 384 (M + H) | 3 |
| 2911 | 4-bromo-N-(cis-4-{[4-(dimethylamino)-5-methylpyrimidin-2-yl]amino}cyclohexyl)benzamide | 432 (M + H) | 1 |
| 2912 | N-(cis-4-{[4-(dimethylamino)-5-methylpyrimidin-2-yl]amino}cyclohexyl)-4-(trifluoromethyl)benzamide | 422 (M + H) | 1 |
| 2913 | N-(cis-4-{[4-(dimethylamino)-5-methylpyrimidin-2-yl]amino}cyclohexyl)-4-methoxybenzamide | 384 (M+H) | 3 |
| 2914 | 2-bromo-N-(cis-4-{[4-(dimethylamino)-5-methylpyrimidin-2-yl]amino}cyclohexyl)benzamide | 432 (M + H) | 3 |
| 2915 | 2-chloro-N-(cis-4-{[4-(dimethylamino)-5-methylpyrimidin-2-yl]amino}cyclohexyl)benzamide | 388 (M + H) | 3 |
| 2916 | N-(cis-4-{[4-(dimethylamino)-5-methylpyrimidin-2-yl]amino}cyclohexyl)-2-fluorobenzamide | 372 (M + H) | 3 |

(continued)

| Ex. No. | compound name | MS | class |
|---|---|---|---|
| 2917 | N-(cis-4-{[4-(dimethylamino)-5-methylpyrimidin-2-yl]amino}cyclohexyl)-2-methylbenzamide | 368 (M + H) | 3 |
| 2918 | N-(cis-4-{[4-(dimethylamino)-5-methylpyrimidin-2-yl]amino}cyclohexyl)-2-(trifluoromethyl)benzamide | 422 (M+H) | 3 |
| 2919 | N-(cis-4-{[4-(dimethylamino)-5-methylpyrimidin-2-yl]amino}cyclohexyl)-4-fluoro-3-(trifluoromethyl)benzamide | 440 (M + H) | 1 |
| 2920 | 4-bromo-N-(cis-4-{[4-(dimethylamino)-5-methylpyrimidin-2-yl]amino}cyclohexyl)-3-methylbenzamide | 446 (M + H) | 1 |
| 2921 | N-(cis-4-{ [4-(dimethylamino)-5-methylpyrimidin-2-yl]amino}cyclohexyl)-4-ethoxybenzamide | 398 (M + H) | 2 |
| 2922 | 3-(dimethylamino)-N-(cis-4-{[4-(dimethylamino)-5-methylpyrimidin-2-yl]amino}cyclohexyl)benzamide | 397 (M + H) | |
| 2923 | N-(cis-4-{[4-(dimethylamino)-5-methylpyrimidin-2-yl]amino}cyclohexyl)-4-fluoro-3-methylbenzamide | 386 (M + H) | 1 |
| 2924 | N-(cis-4-{[4-(dimethylamino)-5-methylpyrimidin-2-yl]amino}cyclohexyl)-3-fluoro-4-methylbenzamide | 386 (M + H) | 1 |
| 2925 | N-(cis-4-{(4-(dimethylamino)-5-methylpyrimidin-2-yl]amino}cyclohexyl)-3-ethylbenzamide | 382 (M + H) | 1 |
| 2926 | N-(cis-4-{[4-(dimethylamino)-5-methylpyrimidin-2-yl]amino}cyclohexyl)-2,2-difluoro-1,3-benzodioxole-5-carboxamide | 434 (M + H) | 1 |
| 2927 | N-(cis-4-{[4-(dimethylamino)-5-methylpyrimidin-2-yl]amino}cyclohexyl)-3-ethoxybenzamide | 398 (M + H) | 2 |
| 2928 | N-(cis-4-{[4-(dimethylamino)-5-methylpyrimidin-2-yl]amino}cyclohexyl)-3-isopropoxybenzamide | 412 (M + H) | 2 |
| 2929 | N-(cis-4-{[4-(dimethylamino)-5-methylpyrimidin-2-yl]amino}cyclohexyl)-3,5-diethoxybenzamide | 442 (M + H) | 1 |
| 2930 | N-(cis-4-{[4-(dimethylamino)-5-methylpyrimidin-2-yl]amino}cyclohexyl)-3-fluoro-5-(triftuoromethyl)benzamide | 440 (M + H) | 1 |
| 2931 | N-(cis-4-{[4-(dimethylamino)-5-methylpyrimidin-2-yl]amino}cyclohexyl)-3-fluoro-4-(trifluoromethyl)benzamide | 440 (M + H) | 3 |
| 2932 | 3-chloro-N-(cis-4-{[4-(dimethylamino)-5-methylpyrimidin-2-yl]amino}cyclohexyl)-4-fluorobenzamide | 406 (M + H) | 3 |
| 2933 | 3,5-dibromo-N-(cis-4-{[4-(dimethylamino)-5-methylpyrimidin-2- yl]amino}cyclohexyl)benzamide | 510 (M + H) | 1 |
| 2934 | N-(cis-4-{[4-(dimethylamino)-5-methylpyrimidin-2-yl]amino}cyclohexyl)-3,5-dimethylbenzamide | 382 (M + H) | 1 |
| 2935 | 4-chloro-N-(cis-4-{[4-(dimethylamino)-5-methylpyrimidin-2-yl]amino}cyclohexyl)-3-methylbenzamide | 402 (M + H) | 1 |
| 2936 | N-(cis-4-{[4-(dimethylamino)-5-methylpyrimidin-2-yl]amino}cyclohexyl)-4-methoxy-3-(trifluoromethyl)benzamide | 452 (M + H) | 1 |
| 2937 | N-(cis-4-{[4-(dimethylamino)-5-methylpyrimidin-2-yl]amino}cyclohexyl)-3-methylbenzamide | 368 (M + H) | 1 |
| 2938 | N-(cis-4-{[4-(dimethylamino)-5-methylpyrimidin-2-yl]amino}cyclohexyl)-3-methoxybenzamide | 384 (M + H) | 1 |

(continued)

| Ex. No. | compound name | MS | class |
|---|---|---|---|
| 2939 | N-(cis-4-{[4-(dimethylamino)-5-methylpyrimidin-2-yl]amino}cyclohexyl)-4-methylbenzamide | 368 (M + H) | 1 |
| 2940 | 3-chloro-N-(cis-4-{[4-(dimethylamino)-5-methylpyrimidin-2-yl]amino}cyclohexyl)benzamide | 388 (M + H) | 1 |
| 2941 | N~ 2~ -(3-chlorophenyl)-N-(cis-4-{[4-(dimethylamino)-5-methylpyrimidin-2-yl]amino}cyclohexyl)-N2-methylglycinamide | 431 (M + H) | 1 |
| 2942 | N-(cis-4-{[4-(dimethylamino)-5-methylpyrimidin-2-yl]amino}cyclohexyl)-N2-methyl-N2-(3-methylphenyl)-glycinamide | 411 (M + H) | 1 |
| 2943 | N-(cis-4-{[4-(dimethylamino)-5-methylpyrimidin-2-yl]amino}cyclohexyl)-N2-(3-fluorophenyl)-N2-methylglycinamide | 415 (M + H) | 1 |
| 2944 | N-(cis-4-{[4-(dimethylamino)-5-methylpyrimidin-2-yl]amino}cyclohexyl)-N2-(4-fluorophenyl)-N2-methylglycinamide | 415 (M + H) | 1 |
| 2945 | N2-(4-chlorophenyl)-N-(cis-4-{[4-(dimethylamino)-5-methylpyrimidin-2-yl]amino}cyclohexyl)-N2-methylglycinamide | 431 (M + H) | 1 |
| 2946 | N2-(3,4-difluorophenyl)-N-(cis-4-{[4-(dimethylamino)-5-methylpyrimidin-2-yl]amino}cyclohexyl)-N2-methylglycinamide | 433 (M + H) | 1 |
| 2947 | N-(cis-4-[4-(dimethylamino)-5-methylpyrimidin-2-yl]amino}cyclohexyl)-N2-(3-methoxyphenyl)-N2-methylglycinamide | 427 (M + H) | 1 |
| 2948 | N-(cis-4-[4-(dimethylamino)-5-methylpyrimidin-2-yl]amino}cyclohexyl)-N2-(4-methoxyphenyl)-N2-methylglycinamide | 427 (M + H) | 2 |
| 2949 | 2-(4-chlorophenyl)-N-(cis-4-{[4-(dimethylamino)-6-methylpyrimidin-2-yl]amino}cyclohexyl)-2-methylpropanamide | 430 (M + H) | 1 |
| 2950 | 2-[3,5-bis(trifluoromethyl)phenyl]-N-(cis-4-{[4-(dimethylamino)-6-methylpyrimidin-2-yl]amino}cyclohexyl)acetamide | 504 (M + H) | 2 |
| 2951 | N-(cis-4-{[4-(dimethylamino)-6-methylpyrimidin-2-yl]amino}cyclohexyl)benzamide | 354 (M + H) | 3 |
| 2952 | 4-butyl-N-(cis-4-{[4-(dimethylamino)-6-methylpyrimidin-2-yl]amino}cyclohexyl)benzamide | 410 (M + H) | 3 |
| 2953 | N-(cis-4-{[4-(dimethylamino)-6-methylpyrimidin-2-yl]amino}cyclohexyl)-3-fluorobenzamide | 372 (M + H) | 2 |
| 2954 | N-(cis-4-{[4-(dimethylamino)-6-methylpyrimidin-2-yl]amino}cyclohexyl)-3-(trifluoromethyl)benzamide | 422 (M + H) | 1 |
| 2955 | N-(cis-4-{[4-(dimethylamino)-6-methylpyrimidin-2-yl]amino}cyclohexyl)-2-methoxybenzamide | 384 (M + H) | 3 |
| 2956 | N-(cis-4-([4-(dimethylamino)-6-methylpyrimidin-2-yl]amino}cyclohexyl)-4-methoxybenzamide | 384 (M + H) | 3 |
| 2957 | 3-cyano-N-(cis-4-{[4-(dimethylamino)-6-methylpyrimidin-2-yl]amino}cyclohexyl)benzamide | 379 (M + H) | 1 |
| 2958 | 4-cyano-N-(cis-4-{[4-(dimethylamino)-6-methylpyrimidin-2-yl]amino}cyclohexyl)benzamide | 379 (M + H) | 1 |
| 2959 | N-(cis-4-{[4-(dimethylamino)-6-methylpyrimidin-2-yl]amino}cyclohexyl)-3,5-dimethoxybenzamide | 414 (M + H) | 1 |
| 2960 | N-(cis-4-([4-(dimethylamino)-6-methylpyrimidin-2-yl]amino) cyclohexyl)-3-fluoro-4-(trifluoromethyl)benzamide | 440 (M + H) | 2 |

(continued)

| Ex. No. | compound name | MS | class |
|---|---|---|---|
| 2961 | N-(cis-4-{[4-(dimethylamino)-6-methylpyrimidin-2-yl]amino}cyclohexyl)-4-fluoro-3-(trifluoromethyl)benzamide | 440 (M + H) | 1 |
| 2962 | N-(cis-4-{[4-(dimethylamino)-6-methylpyrimidin-2-yl]amino}cyclohexyl)-3-fluoro-5-(trifluoromethyl)benzamide | 440 (M + H) | 1 |
| 2963 | 3-chloro-N-(cis-4-{[4-(dimethylamino)-6-methylpyrimidin-2-yl]amino} cyclohexyl)-4-fluorobenzamide | 406 (M + H) | 1 |
| 2964 | N-(cis-4-{[4-(dimethylamino)-6-methylpyrimidin-2-yl]amino}cyclohexyl)-4-fluoro-3-methylbenzamide | 386 (M + H) | 1 |
| 2965 | N-(cis-4-{[4-(dimethylamino)-6-methylpyrimidin-2-yl]amino ) cyclohexyl)-3-fluoro-4-methylbenzamide | 386 (M + H) | 1 |
| 2966 | 3,5-dichloro-N-(cis-4-{[4-(dimethylamino)-6-methylpyrimidin-2-yl]amino} cyclohexyl)benzamide | 422 (M + H) | 1 |
| 2967 | N-(cis-4-{[4-(dimethylamino)-6-methylpyrimidin-2-yl]amino}cyclohexyl)-3-(trifluoromethoxy)benzamide | 438 (M + H) | 1 |
| 2968 | N-(cis-4-{[4-(dimethylamino)-6-methylpyrimidin-2-yl]aminocyclohexyl)-3,5-difluorobenzamide | 390 (M + H) | 1 |
| 2969 | 4-bromo-N-(cis-4-{[4-(dimethylamino)-6-methylpyrimidin-2-yl]amino} cyclohexyl)-3-methylbenzamide | 446 (M + H) | 1 |
| 2970 | N-(cis-4-{[4-(dimethylamino)-6-methylpyrimidin-2-yl]amino}cyclohexyl)-3-ethylbenzamide | 382 (M + H) | 3 |
| 2971 | N-(cis-4-{[4-(dimethylamino)-6-methylpyrimidin-2-yl]amino}cyclohexyl)-4-ethoxybenzamide | 398 (M + H) | 3 |
| 2972 | N-(cis-4-{[4-(dimethylamino)-6-methylpyrimidin-2-yl]amino}cyclohexyl)-4-(trifluoromethyl)benzamide | 422 (M + H) | 2 |
| 2973 | 4-bromo-N-(cis-4-{[4-(dimethylamino)-6-methylpyrimidin-2-yl]amino} cyclohexyl)benzamide | 432 (M + H) | 1 |
| 2974 | N-(cis-4-{[4-(dimethylamino)-6-methylpyrimidin-2-yl]amino}cyclohexyl)-4-ethylbenzamide | 382 (M + H) | 2 |
| 2975 | N-(cis-4-{[4-(dimethylamino)-6-methylpyrimidin-2-yl]amino}cyclohexyl)-3,5-diethoxybenzamide | 442 (M + H) | 1 |
| 2976 | N-(cis-4-{[4-(dimethylamino)-6-methylpyrimidin-2-yl]amino}cyclohexyl)-3-ethoxybenzamide | 398 (M + H) | 2 |
| 2977 | N-(cis-4-{[4-(dimethylamino)-6-methylpyrimidin-2-yl]amino}cyclohexyl)-3-isopropoxybenzamide | 412 (M + H) | 1 |
| 2978 | 5-bromo-N-(cis-4-{[4-(dimethylamino)-6-methylpyrimidin-2-yl]amino} cyclohexyl)nicotinamide | 433 (M + H) | 1 |
| 2979 | 5-bromo-N-(cis-4-{[4-(dimethylamino)-6-methylpyrimidin-2-yl]amino} cyclohexyl)-2-furamide | 422 (M + H) | 1 |
| 2980 | 5-chloro-N-(cis-4-{[4-(dimethylamino)-6-methylpyrimidin-2-yl]amino} cyclohexyl)-2-furamide | 378 (M + H) | 1 |
| 2981 | N-(cis-4-{[4-(dimethylamino)-6-methylpyrimidin-2-yl]amino}cyclohexyl)-4-methoxy-3-(trifluoromethyl)benzamide | 452 (M + H) | 2 |
| 2982 | 4-chloro-N-(cis-4-{[4-(dimethylamino)-6-methylpyrimidin-2-yl]amino} cyclohexyl)-3-(trifluoromethyl)benzamide | 456 (M + H) | 1 |

(continued)

| Ex. No. | compound name | MS | class |
|---|---|---|---|
| 2983 | N-(cis-4-{[4-(dimethylamino)-6-methylpyrimidin-2-yl]amino}cyclohexyl)-3-methylbenzamide | 368 (M + H) | 1 |
| 2984 | N-(cis-4-{[4-(dimethylamino)-6-methylpyrimidin-2-yl]amino}cyclohexyl)-3-methoxybenzamide | 384 (M + H) | 1 |
| 2985 | N-(cis-4-{[4-(dimethylamino)-6-methylpyrimidin-2-yl]amino}cyclohexyl)-4-methylbenzamide | 368 (M + H) | 1 |
| 2986 | 4-chloro-N-(cis-4-{[4-(dimethylamino)-6-methylpyrimidin-2-yl]amino} cyclohexyl)benzamide | 388 (M + H) | 1 |
| 2987 | 3-chloro-N-(cis-4-{[4-(dimethylamino)-6-methylpyrimidin-2-yl]amino} cyclohexyl)benzamide | 388 (M + H) | 1 |
| 2988 | N-(cis-4-{[4-(dimethylamino)-6-methylpyrimidin-2-yl]amino}cyclohexyl)-3,4-difluorobenzamide | 390 (M + H) | 1 |
| 2989 | N-(cis-4-{[4-(dimethylamino)-6-methylpyrimidin-2-yl]amino}cyclohexyl)-4-(trifluoromethoxy)benzamide | 438 (M + H) | 3 |
| 2990 | N-(cis-4-{[4-(dimethylamino)-6-methylpyrimidin-2-yl]amino}cyclohexyl)-4-fluorobenzamide | 372 (M + H) | 1 |
| 2991 | N-(cis-4-{[4-(dimethylamino)-6-methylpyrimidin-2-yl]amino}cyclohexyl)-3,4,5-trimethoxybenzamide | 444 (M + H) | 1 |
| 2992 | N-(4-{[4-(dimethylamino)-6-methylpyrimidin-2-yl]amino}cyclohexyl)-3-nitrobenzamide | 399 (M + H) | 1 |
| 2993 | N-(cis-4-{[4-(dimethylamino)-6-methylpyrimidin-2-yl]amino}cyclohexyl)-2,2-diphenylacetamide | 444 (M + H) | 1 |
| 2994 | 3,4-dichloro-N-(cis-4-{[4-(dimethylamino)-6-methylpyrimidin-2-yl]amino} cyclohexyl)benzamide | 422 (M + H) | 1 |
| 2995 | N-(cis-4-{[4-(dimethylamino)-5-methylpyrimidin-2-yl]amino}cyclohexyl) benzenesulfonamide | 390 (M + H) | 3 |
| 2996 | N4,N4,5-trimethyl-N2-{cis-4-[(4-methylbenzyl)amino]cyclohexyl } pyrimidine-2,4-diamine | 354 (M + H) | 3 |
| 2997 | N2-{cis-4-[(3,4-difluorobenzyl)amino]cyclohexyl}-N4,N4,5-trimethylpyrimidine-2,4-diamine | 376 (M + H) | 3 |
| 2998 | N2-{cis-4-[(3-chlorobenzyl)amino]cyclohexyl}-N4,N4,5-trimethylpyrimidine-2,4-diamine | 374 (M + H) | 3 |
| 2999 | N2-{cis-4-[(3-bromobenzyl)amino]cyclohexyl}-N4,N4,5-trimethylpyrimidine-2,4-diamine | 418 (M + H) | 3 |
| 3000 | N2-{cis-4-[(3,5-dimethoxybenzyl)amino]cyclohexyl} -N4,N4,5-trimethylpyrimidine-2,4-diamine | 400 (M + H) | 2 |
| 3001 | N2-{cis-4-[(3,5-dichlorobenzyl)amino]cyclohexyl)-N4,N4,5-trimethylpyrimidine-2,4-diamine | 408 (M +H) | 3 |
| 3002 | N2-{cis-4-[(3,4-dichlorobenzyl)amino]cyclohexyl}-N4,N4,5-trimethylpyrimidine-2,4-diamine | 408 (M + H) | 3 |
| 3003 | N2-{cis-4-[(4-methoxy-3-methylbenzyl)amino]cyclohexyl} -N4,N4,5-trimethylpyrimidine-2,4-diamine | 384 (M + H) | 3 |
| 3004 | N4,N4,5-trimethyl-N2-(cis-4-{[3-(trifluoromethoxy)benzyl]amino}cyclohexyl) pyrimidine-2,4-diamine | 424 (M + H) | 3 |

(continued)

| Ex. No. | compound name | MS | class |
|---|---|---|---|
| 3005 | N4,N4,6-trimethyl-N2-(cis-4-{[3-(trifluoromethoxy)benzyl]amino}cyclohexyl) pyrimidine-2,4-diamine | 424 (M + H) | 3 |
| 3006 | N-(cis-4-{[4-(dimethylamino)-5-(trifluoromethyl)pyrimidin-2-yl]amino} cyclohexyl)-3,4-difluorobenzamide | 444 (M + H) | 3 |
| 3007 | N-[((1R,3S)-3-{[4-(dimethylamino)-5-methylpyrimidin-2-yl]amino}cyclopentyl) methy]-6-(3-fluorophenoxy)nicotinamide | 465 (M + H) | |
| 3008 | 6-(3-chlorophenoxy)-N-[((1R,3S)-3-{[4-(dimethylamino)-5-methylpyrimidin-2-yl] amino}cyclopentyl)methyl]nicotinamide | 481 (M + H) | |
| 3009 | 3009 N-[((1R,3S)-3-{[4-(dimethylamino)-5-methylpyrimidin-2-yl]amino} cyclopentyl)methyl]-6-(3-methoxyphenoxy) nicotinamide | 477 (M + H) | |
| 3010 | N-[((1R,3S)-3-{[4-(dimethylamino)-5-methylpyrimidin-2-yl]amino}cyclopentyl) methyl]-6-(2-methoxyphenoxy)nicotinamide | 477 (M + H) | |
| 3011 | N-[((1R,3S)-3-{[4-(dimethylamino)-5-methylpyrimidin-2-yl]amino}cyclopentyl) methyl]-6-(2-fluorophenoxy)nicotinamide | 465 (M + H) | |
| 3012 | 2-(4-bromophenoxy)-N-[((1R,3S)-3-{[4-(dimethylamino)-5-methylpyrimidin-2-yl]amino}cyclopentyl)methyl]nicotinamide | 525 (M + H) | 2 |
| 3013 | N-[((1R,3S)-3-{[4-(dimethylamino)-5-methylpyrimidin-2-yl]amino}cyclopentyl) methyl]-2-(2-methoxyphenoxy)nicotinamide | 477 (M + H) | 1 |
| 3014 | 2-(2-bromophenoxy)-N-[((1R,3S)-3-{[4-(dimethylamino)-5-methylpyrimidin-2-yl]amino}cyclopentyl)methyl]nicotinamide | 525 (M + H) | 3 |
| 3015 | N-[((1R,3S)-3-{[4-(dimethylamino)-5-methylpyrimidin-2-yl]amino}cyclopentyl) methyl]-2-(2-fluorophenoxy)nicotinamide | 465 (M + H) | 1 |
| 3016 | N-[((1R,3S)-3-{[4-(dimethylamino)-5-methylpyrimidin-2-yl]amino}cyclopentyl) methyl]-2-(4-methoxyphenoxy)-nicotinamide | 477 (M + H) | |
| 3017 | N-[((1R,3S)-3-{[4-(dimethylamino)-5-methylpyrimidin-2-yl]amino}cyclopentyl) methyl]-2-(3-fluorophenoxy)nicotinamide | 465 (M + H) | 3 |
| 3018 | 2-(3-chlorophenoxy)-N-[((1R,3S)-3-{[4-(dimethylamino)-5-methylpyrimidin-2-yl] amino}cyclopentyl)methyl]nicotinamide | 481 (M + H) | 3 |
| 3019 | 2-(3-chloro-4-fluorophenoxy)-N-[((1R,3S)-3-{[4-(dimethylamino)-5-methylpyrimidin-2-yl]amino}cyclopentyl)methyl]nicotinamide | 499 (M + H) | 2 |
| 3020 | 2-(4-chloro-3-fluorophenoxy)-N-[((1R,3S)-3-{[ 4-(dimethylamino)-5-methylpyrimidin-2-yl]amino}cyclopentyl)methyl]nicotinamide | 499 (M + H) | 3 |
| 3021 | N-(3-chlorophenyl)-N'-(cis-4-{[4-(dimethylamino)-5-methylpyrimidin-2-yl] amino}cyclohexyl)-N-methylurea | 417 (M + H) | 1 |
| 3022 | N-(3,4-dichlorophenyl)-N'-(cis-4-{[4-(dimethylamino)-5-methylpyrimidin-2-yl] amino}cyclohexyl)-N-methylurea | 451 (M + H) | 1 |
| 3023 | N'-(cis-4-{[4-(dimethylamino)-5-methylpyrimidin-2-yl]amino}cyclohexyl)-N-methyl-N-(3-methylphenyl)urea | 397 (M + H) | 1 |
| 3024 | N'-(cis-4-{[4-(dimethylamino)-5-methylpyrimidin-2-yl]amino}cyclohexyl)-N-methyl-N-(4-methylphenyl)urea | 397 (M + H) | 1 |
| 3025 | N'-(cis-4-{[4-(dimethylamino)-5-methylpyrimidin-2-yl]amino}cyclohexyl) -N-(3-fluorophenyl)-N-methylurea | 401 (M + H) | 1 |
| 3026 | N'-(cis-4-{[4-(dimethylamino)-5-methylpyrimidin-2-yl]amino}cyclohexyl) -N-(4-fluorophenyl)-N-methylurea | 401 (M + H) | 1 |

(continued)

| Ex. No. | compound name | MS | class |
|---|---|---|---|
| 3027 | N-(4-chlorophenyl)-N'-(cis-4-{[4-(dimethylamino)-5-methylpyrimidin-2-yl]amino}cyclohexyl)-N-methylurea | 417 (M + H) | 1 |
| 3028 | N'-(cis-4-{[4-(dimethylamino)-5-methylpyrimidin-2-yl]amino}cyclohexyl)-N-(3-methoxyphenyl)-N-methylurea | 413 (M + H) | 1 |
| 3029 | N'-(cis-4-{[4-(dimethylamino)-5-methylpyrimidin-2-yl]amino}cyclohexyl)-N-(4-methoxyphenyl)-N-methylurea | 413 (M + H) | 1 |
| 3030 | 3,4-dichloro-N-(cis-4-{[5-methyl-4-(methylamino)pyrimidin-2-yl]amino}cyclohexyl)benzamide | 408 (M + H) | 3 |

**Example 3031**

**3,4-Difluoro-*N*-[*cis*-4-(quinolin-2-ylamino)-cyclohexyl]-benzamide hydrochloride**

**Step A: Synthesis of (*cis*-4-benzyloxycarbonylamino-cyclohexyl)-carbamic acid benzyl ester.**

To a suspension of *cis*-cyclohexane-1,4-dicarboxylic acid (25.0 g, 145 mmol) in benzene (125 mL) were added phosphorazidic acid diphenyl ester (81.9 g, 298 mmol) and triethylamine (30.1 g, 297 mmol). The reaction mixture was stirred at reflux for 2.5 hr. Benzyl alcohol (32.2 g, 298 mmol) was added and the mixture was stirred at reflux for 24 hr. The reaction mixture was concentrated and the residue was dissolved in EtOAc and $H_2O$. The organic layer was separated and the aqueous layer was extracted with EtOAc (twice). The combined organic layer was washed with 1 M aqueous $KHSO_4$, saturated aqueous $NaHCO_3$, and brine, dried over $MgSO_4$, filtered, concentrated, and purified by flash chromatography (silica gel, 33% EtOAc in hexane) to give (*cis*-4-benzyloxycarbonylamino-cyclohexyl)-carbamic acid benzyl ester (52.0 g, 94%) as a colorless oil.
ESI MS m/e 405, M + $Na^+$ ; $^1H$ NMR (300 MHz, $CDCl_3$) $\delta$ 7.15-7.40 (m, 10 H), 5.07 (s, 4 H), 4.70-5.00 (m, 2 H), 3.52-3.80 (m, 2 H), 1.60-1.80 (m, 4 H), 1.45-1.60 (m, 4 H).

**Step B: Synthesis of (*cis*-4-amino-cyclohexyl)-carbamic acid *tert*-butyl ester.**

To a solution of (*cis*-4-benzyloxycarbonylamino-cyclohexyl)-carbamic acid benzyl ester (91.7 g, 240 mmol) in MeOH (460 mL) was added 5% Pd/C (9.17 g). The reaction mixture was stirred at ambient temperature under hydrogen atmosphere for 2.5 days, filtered through a pad of celite, and concentrated to give a diamine as a colorless oil. To a solution of the diamine in MeOH (550 mL) was added a solution of $(Boc)_2O$ (6.59 g, 30.2 mmol) in MeOH (80 mL) dropwise over 4 hr. The reaction mixture was stirred at ambient temperature for 1.5 days and concentrated. After dissolution with $H_2O$, the aqueous layer was extracted with $CHCl_3$ (three times). The combined organic layer was dried over $MgSO_4$, filtered, and concentrated to give *cis*-(4-amino-cyclohexyl)-carbamic acid *tert*-butyl ester (7.78 g, 15%, crude) as a colorless oil. The aqueous layer was concentrated and the residue was dissolved in MeOH, dried over $MgSO_4$, filtered, and concentrated to give a recovered diamine (32.9 g) as a colorless oil. To a solution of the recovered diamine (32.9 g, 288 mmol) in MeOH (660 mL) was added a solution of $(Boc)_2O$ (6.29 g, 28.8 mmol) in MeOH (80 mL) dropwise over 5 hr. The reaction mixture was stirred at ambient temperature for 10 hr and concentrated. After dissolution with $H_2O$, the aqueous layer was extracted with $CHCl_3$ (three times). The combined organic layer was dried over $MgSO_4$, filtered, and concentrated to give *cis*-(4-amino-cyclohexyl)-carbamic acid *tert*-butyl ester (8.16 g, 16%, crude) as a colorless oil. The aqueous layer was concentrated and the residue was dissolved in MeOH, dried over $MgSO_4$, filtered, and concentrated to give a recovered diamine (23.1 g) as a colorless oil. To a solution of the recovered diamine (23.1 g, 202 mmol) in MeOH (462 mL) was added a solution of $(Boc)_2O$ (4.42 g, 20.3 mmol) in MeOH (56 mL) dropwise over 4 hr. The reaction mixture was stirred at ambient temperature for 3.5 days and concentrated. After dissolution with $H_2O$, the aqueous layer was extracted with $CHCl_3$ (three times). The combined organic layer was dried over $MgSO_4$, filtered, and concentrated to give *cis*-(4-amino-cyclohexyl)-carbamic acid *tert*-butyl ester (5.01 g, 10% based on starting material) as a colorless oil. The aqueous layer was concentrated and the residue was dissolved in MeOH, dried over $MgSO_4$, filtered, and concentrated to give a recovered diamine (16.0 g) as a colorless oil. To a solution of the recovered diamine (16.0 g, 140 mmol) in MeOH (320 mL) was added a solution of $(Boc)_2O$ (3.06 g, 14.0 mmol) in MeOH (40 mL) dropwise over 4 hr. The reaction mixture was stirred at ambient temperature for 13 hr and concentrated. After dissolution with $H_2O$, the aqueous layer was extracted with $CHCl_3$ (three times). The combined organic layer was dried over $MgSO_4$, filtered, and concentrated to give *cis*-(4-amino-cyclohexyl)-carbamic acid *tert*-butyl ester (3.53 g,

7% based on the starting material) as a colorless oil. The aqueous layer was concentrated and the residue was dissolved in MeOH, dried over $MgSO_4$, filtered, and concentrated to give a recovered diamine (11.1 g) as a colorless oil.
ESI MS m/e 215, M + $H^+$; $^1H$ NMR (300 MHz, $CDCl_3$) δ 4.30-4.82 (m, 1 H), 3.50-3.80 (m, 1 H), 2.78-2.95 (m, 1 H), 1.44 (s, 9H), 1.20-1.80 (m, 8 H).

**Step C: Synthesis of (*cis*-4-{[1-(3,4-difluoro-phenyl)-methanoyl]-amino}-cyclohexyl)-carbamic acid tert-butyl ester.**

To a solution of 3,4-difluoro-benzoic acid (4.10 g, 25.9 mmol) and (*cis*-4-amino-cyclohexyl)-carbamic acid *tert*-butyl ester (5.05 g, 23.6 mmol) in DMF (50 mL) were added $Et_3N$ (90 mL, 56.7 mmol), HOBt-$H_2O$ (5.41 g, 35.3 mmol), and EDC-HCl (4.97 g, 25.9 mmol). The reaction mixture was stirred at ambient temperature for 17 hr. To the reaction mixture was added water (200 mL) and the suspension was stirred at ambient temperature for 10 min. The precipitated was collected by filtration, washed with $H_2O$ and EtOH, and dried at 80 °C under reduced pressure to give (*cis*-4-{[1-(3,4-difluoro-phenyl)-methanoyl]-amino}-cyclohexyl)-carbamic acid *tert*-butyl ester (5.20 g, 62.3%) as a white solid.
ESI MS m/e 377, M + $Na^+$; $^1H$ NMR (300 MHz, $CDCl_3$) δ 1.45 (s, 9 H), 1.53-1.95 (m, 8 H), 3.60-3.74 (m, 1 H), 4.00-4.16 (m, 1 H), 4.50-4.68 (m, 1 H), 5.95-6.09 (m, 1 H), 7.15-7.28 (m, 1 H), 7.43-7.68 (m, 2 H).

**Step D: Synthesis of *N*-(*cis*-4-amino-cyclohexyl)-3,4-difluoro-benzamide.**

A solution of (*cis*-4-{[1-(3,4-difluoro-phenyl)-methanoyl]-amino}-cyclohexyl)-carbamic acid *tert*-butyl ester (5.20 g, 14.7 mmol) in EtOAc (52 mL) was cooled on an ice-bath and 4 M hydrogen chloride in EtOAc (104 mL) was added. The mixture was stirred at ambient temperature for 1 hr and concentrated. The residue was dissolved in 1 M aqueous NaOH and the aqueous layer was extracted with $CHCl_3$ (three time). The combined organic layer was dried over $MgSO_4$, filtered, concentrated, and dried at 60 °C under reduced pressure to give *N*-(*cis*-4-amino-cyclohexyl)-3,4-difluoro-benzamide (3.00 g, 80%) as a white solid.
ESI MS m/e 255, M + $H^+$ ; $^1H$ NMR (300 MHz, $CDCl_3$) δ 1.15-1.52 (m, 3 H), 1.59-1.89 (m, 5 H), 2.94-3.06 (m, 1 H), 4.06-4.20 (m, 1 H), 6.01-6.18 (m, 1 H), 7.13-7.26 (m, 1 H), 7.43-7.50 (m, 1 H), 7.57-7.67 (m, 1 H).

**Step E: Synthesis of 3,4-difluoro-*N*-[*cis*-4-(quinolin-2-ylamino)-cyclohexyl]-benzamide hydrochloride.**

A mixture of 2-chloro-quinoline (200 mg, 1.22 mmol) and *N*-(*cis*-4-amino-cyclohexyl)-3,4-difluoro-benzamide (342 mg, 1.34 mmol) in butanol (1 mL) was stirred at 130 °C for 60 hr in a sealed tube. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane). To a solution of the above material in EtOAc (2 mL) was added 4 M hydrogen chloride in EtOAc (10 mL). The mixture was stirred at ambient temperature for 1 hr and concentrated. The residue was suspended in $Et_2O$ (20 mL) and the suspension was stirred at ambient temperature for 2 hr. The precipitate was filtered, washed with $Et_2O$, and dried at 80 °C under reduced pressure to give 3,4-difluoro-*N*-[*cis*-4-(quinolin-2-ylamino)-cyclohexyl]-benzamide hydrochloride (189 mg, 37%) as a white solid.
ESI MS m/e 382, M (free) + $H^+$ ; $^1H$ NMR (300 MHz, $CDCl_3$) δ 1.80-2.09 (m, 8 H), 3.96-4.24 (m, 2 H), 6.90-7.03 (m, 2 H), 7.14-7.25 (m, 1 H), 7.41-7.48 (m, 1 H), 7.57-7.64 (m, 1 H), 7.69-7.79 (m, 4 H), 8.18 (d, *J* = 9.6 Hz, 1 H), 9.73-9.76 (m, 1 H).

**Example 3032**

**2-Phenoxy-*N*-[*cis*-4-(quinolin-2-ylamino)-cyclohexyl]-nicotinamide hydrochloride**

**Step A: Synthesis of *N*-(*cis*-4-amino-cyclohexyl)-2-phenoxy-nicotinamide.**

To a solution of (*cis*-4-amino-cyclohexyl)-carbamic acid *tert*-butyl ester obtained in step B of example 3031 (6.00 g, 27.8 mmol) in $CHCl_3$ (60 mL) was added *i*-$Pr_2NEt$ (9.67 mL, 55.6 mmol). The mixture was cooled on an ice-bath and 2-phenoxy-nicotinoyl chloride (6.50 g, 27.8 mmol) was added. The mixture was stirred at ambient temperature for 17 hr. The reaction was quenched with saturated aqueous $NaHCO_3$ and the aqueous layer was extracted with $CHCl_3$ (three times). The combined organic layer was dried over $MgSO_4$, filtered, and concentrated. To a solution of the above material in EtOAc (100 mL) and $CHCl_3$ (40 mL) was added 4 M hydrogen chloride in EtOAc (100 mL). The mixture was stirred at ambient temperature for 1 hr and concentrated. The residue was dissolved in 1 M aqueous NaOH and the aqueous layer was extracted with $CHCl_3$ (three time). The combined organic layer was dried over

$MgSO_4$, filtered, concentrated, and purified by medium-pressure liquid chromatography (NH-silica gel, 0.2% to 1% MeOH in $CHCl_3$) to give *N*-(*cis*-4-amino-cyclohexyl)-2-phenoxy-nicotinamide (3.51 g, 41%) as a pale yellow oil.
ESI MS m/e 312, M + $H^+$ ; $^1$H NMR (300 MHz, $CDCl_3$) δ 1.12-1.39 (m, 3 H), 1.65-1.94 (m, 5 H), 2.80-2.91 (m, 1 H), 4.18-4.30 (m, 1 H), 7.13-7.22 (m, 3 H), 7.25-7.33 (m, 1 H), 7.41-7.51 (m, 2 H), 8.04-8.14 (m, 1 H), 8.22 (dd, *J* = 4.7, 2.1 Hz, 1 H), 8.62 (dd, *J* = 7.6, 2.0 Hz, 1 H).

**Step B: Synthesis of 2-phenoxy-*N*-[*cis*-4-(quinolin-2-ylamino)-cyclohexyl]-nicotinamide hydrochloride.**

A mixture of 2-chloro-quinoline (200 mg, 1.22 mmol) and *cis*-*N*-(4-amino-cyclohexyl)-2-phenoxy-nicotinamide (418 mg, 1.34 mmol) in butanol (1 mL) was stirred at 130 °C for 6 days in a sealed tube. The reaction mixture was poured into saturated aqueous $NaHCO_3$ and the aqueous layer was extracted with $CHO_3$ (three times). The combined organic layer was dried over $MgSO_4$, filtered, concentrated, and purified by medium-pressure liquid chromatography (NH-silica gel, 10% to 16% EtOAc in hexane). To a solution of the above material in EtOAc (10 mL) was added 4 M hydrogen chloride in EtOAc (0.2 mL). The mixture was stirred at ambient temperature for 1 hr, and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give 2-phenoxy-*N*-[*cis*-4-(quinolin-2-ylamino)-cyclohexyl]-nicotinamide hydrochloride (138 mg, 37%) as a white solid.
ESI MS m/e 461, M (free) + $Na^+$; $^1$H NMR (300 MHz, $CDCl_3$) δ 1.87-2.10 (m, 8 H), 3.83-3.97 (m, 1 H) 4.12-4.24 (m, 1 H), 6.90 (d, J = 9.5 Hz, 1 H), 7.12 (dd, J = 7.6,4.5 Hz, 1 H), 7.20-7.32 (m, 3 H), 7.37-7.50 (m, 3 H), 7.66-7.80 (m, 3 H), 7.95 (d, J = 7.0 Hz, 1 H) 8.13 (d, J = 9.6 Hz, 1 H), 8.21 (dd, *J* = 4.6, 2.2 Hz, 1 H), 8.53 (dd, *J* = 7.6,1.9 Hz, 1 H), 9.77-9.92 (m, 1 H).

**Example 3033**

**3-Methyl-*N*-[*cis*-4-(quinolin-2-ylamino)-cyclohexyl]-benzamide hydrochloride**

**Step A: Synthesis of *cis*-*N*-quinolin-2-yl-cyclohexane-1,4-diamine.**

A mixture of 2-chloro-quinoline (16.0 g, 97.8 mol) and (cis-4-amino-cyclohexyl)-carbamic acid *tert*-butyl ester obtained in step B of example 3031 (23.0 g, 107.5 mol) in butanol (16 mL) was stirred at 130 °C for 3 days. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 10% EtOAc in hexane) to give a pale yellow oil. To a solution of the above material in EtOAc (160 mL) was added 4 M hydrogen chloride in EtOAc (80 mL). The mixture was stirred at ambient temperature for 12 hr and concentrated. The residue was dissolved in 1 M aqueous NaOH and the aqueous layer was extracted with $CHCl_3$ (three time). The combined organic layer was dried over $MgSO_4$, filtered, and purified by medium-pressure liquid chromatography (NH-silica gel, 2% to 5% MeOH in $CHCl_3$) to give *cis*-*N*-quinolin-2-yl-cyclohexane-1,4-diamine (6.30 g, 27%) as pale yellow solid.
ESI MS m/e 242, M + $H^+$ ; $^1$H NMR (300 MHz, $CDCl_3$) δ 1.12-1.53 (m, 4 H), 1.65-1.93 (m, 6 H), 2.84-2.98 (m, 1 H), 4.04-4.16 (m, 1 H), 4.78-4.91 (m, 1 H), 6.61 (d, *J* = 9.0 Hz, 1 H), 7.17 (ddd, *J* = 8.0, 6.9, 1.2 Hz, 1 H), 7.46-7.58 (m, 2 H), 7.61-7.66 (m, 1 H), 7.79 (d, *J* = 8.9 Hz, 1 H).

**Step B: Synthesis of 3-methyl-*N*-[*cis*-4-(quinolin-2-ylamino)-cyclohexyl]-benzamide hydrochloride.**

To a solution of *cis*-*N*-quinolin-2-yl-cyclohexane-1,4-diamine (300 mg, 1.24 mmol) in $CHCl_3$ (2 mL) were added *i*-$Pr_2$NEt (0.45 mL, 2.60 mmol) and 3-methyl-benzoyl chloride (210 mg, 1.36 mmol) in $CHCl_3$ (1 mL). The mixture was stirred at ambient temperature for 17 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% EtOAc in hexane) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give 3-methyl-*N*-[*cis*-4-(quinolin-2-ylamino)-cyclohexyl]-benzamide hydrochloride (294 mg, 60%) as a white solid.
ESI MS m/e 382, M (free) + $Na^+$; $^1$H NMR (300 MHz, $CDCl_3$) δ 1.82-2.07 (m, 8 H), 2.40 (s, 3 H), 3.93-4.04 (m, 1 H), 4.08-4.26 (m, 1 H), 6.49-6.58 (m, 1 H), 6.94 (d, J = 9.5 Hz, 1 H), 7.25-7.32 (m, 2 H), 7.40-7.48 (m, 1 H), 7.56-7.66 (m, 2 H), 7.67-7.81 (m, 3 H) 8.17 (d, *J* = 9.5 Hz, 1 H), 9.74-9.87 (m, 1 H).

**Example 3034**

**3-Methoxy-*N*-[*cis*-4-(quinolin-2-ylamino)-cyclohexyl]-benzamide hydrochloride**

**Step A: Synthesis of 3-methoxy-*N*-[*cis*-4-(quinolin-2-ylamino)-cyclohexyl]-benzamide hydrochloride.**

Using the procedure for the step B of example 3033, the title compound was obtained.
ESI MS m/e 398, M (free) + $Na^+$ ; $^1H$ NMR (300 MHz, $CDCl_3$) δ 1.78-2.09 (m, 8 H), 3.86 (s, 3 H), 3.94-4.06 (m, 1 H), 4.08-4.25 (m, 1 H), 6.63 (d, *J* = 8.6 Hz, 1 H), 6.91-7.05 (m, 2 H), 7.28-7.48 (m, 4 H), 7.68-7.80 (m, 3 H), 8.17 (d, *J* = 9.3 Hz, 1 H), 9.75-9.84 (m, 1 H).

**Example 3035**

**3-Chloro-*N*-[*cis*-4-(quinolin-2-ylamino)-cyclohexyl]-benzamide hydrochloride**

**Step A: Synthesis of 3-chloro-*N*-[*cis*-4-(quinolin-2-ylamino)-cyclohexyl] -benzamide hydrochloride.**

Using the procedure for the step B of example 3033, the title compound was obtained.
ESI MS m/e 402, M (free) + $Na^+$; $^1H$ NMR (300 MHz, $CDCl_3$) δ 1.82-2.10 (m, 8 H), 3.96-4.07 (m, 1 H), 4.09-4.26 (m, 1 H), 6.75 (d, *J* = 7.8 Hz, 1 H), 6.96 (d, *J* = 9.3 Hz, 1 H), 7.33-7.49 (m, 3 H), 7.66-7.79 (m, 4 H), 7.83-7.88 (m, 1 H), 8.19 (d, *J* = 9.3 Hz, 1 H), 9.80 (d, *J* = 8.5 Hz, 1 H).

**Example 3036**

**5-Nitro-thiophene-3-carboxylic acid [*cis*-4-(quinolin-2-ylamino)-cyclohexyl]-amide hydrochloride**

**Step A: Synthesis of 5-nitro-thiophene-3-carboxylic acid [*cis*-4-(quinolin-2-ylamino)-cyclohexyl]-amide hydrochloride.**

To a solution of 5-nitro-thiophene-3-carboxylic acid (516 mg, 2.98 mmol) and *cis*-*N*-quinolin-2-yl-cyclohexane-1,4-diamine obtained in step A of example 3033 (600 mg, 2.48 mmol) in DMF (6 mL) were added $Et_3N$ (0.83 mL, 5.95 mmol), HOBt-$H_2O$ (570 mg, 3.72 mmol), and EDC-HCl (571 g, 2.98 mmol). The reaction mixture was stirred at ambient temperature for 12 hr. To the reaction mixture was added water (20 mL) and the suspension was stirred at ambient temperature for 30 min. The precipitated was collected by filtration, washed with $H_2O$, and purified by medium-pressure liquid chromatography (NH-silica gel, 33% to 50% EtOAc in hexane and silica gel, 2% to 5% MeOH in $CHCl_3$) to give a pale yellow oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give 5-nitro-thiophene-3-carboxylic acid [*cis*-4-(quinolin-2-ylamino)-cyclohexyl]-amide hydrochloride (329 mg, 60%) as a yellow solid.
ESI MS m/e 419, M (free) + $Na^+$ ; $^1H$ NMR (300 MHz, $CDCl_3$) δ 1.80-2.18 (m, 8 H), 3.96-4.25 (m, 2 H), 6.97 (d, *J* = 9.3 Hz, 1 H), 7.39-7.53 (m, 2 H), 7.67-7.80 (m, 3 H), 8.20 (d, *J* = 9.4 Hz, 1 H), 8.26-8.30 (m, 1 H), 8.39-8.42 (m, 1 H), 9.59 (d, *J* = 8.6 Hz, 1 H).

**Example 3037**

**2-Chloro-*N*-[*cis*-4-(quinolin-2-ylamino)-cyclohexyl]-nicotinamide hydrochloride**

**Step A: Synthesis of 2-chloro-*N*-[*cis*-4-(quinolin-2-ylamino)-cyclohexyl]-nicotinamide hydrochloride.**

Using the procedure for the step A of example 3036, the title compound was obtained.
ESI MS m/e 403, M (free) + $Na^+$; $^1H$ NMR (300 MHz, $CDCl_3$) δ 1.84-2.17 (m, 8 H), 3.93-4.05 (m, 1 H), 4.13-4.30 (m, 1 H), 6.89-7.02 (m, 2 H), 7.30-7.50 (m, 2 H), 7.67-7.81 (m, 3 H), 7.96 (d, *J* = 7.5 Hz, 1 H), 8.19 (d, *J* = 9.6 Hz, 1 H), 8.44-8.50 (m, 1 H), 9.73-9.87 (m, 1 H).

**Example 3038**

**3-Chloro-4-fluoro-*N*-[*cis*-4-(quinolin-2-ylamino)-cyclohexyl]-benzamide hydrochloride**

**Step A: Synthesis of 3-chloro-4-fluoro-*N*-[*cis*-4-(quinolin-2-ylamino)-cyclohexyl]-benzamide hydrochloride.**

Using the procedure for the step B of example 3033, the title compound was obtained.
ESI MS m/e 420, M (free) + $Na^+$; $^1H$ NMR (300 MHz, $CDCl_3$) δ 1.78-2.08 (m, 8 H), 3.95-4.06 (m, 1 H), 4.07-4.23 (m, 1 H), 6.68-6.78 (m, 1 H), 6.95 (d, *J* = 9.6 Hz, 1 H), 7.18 (t, *J* = 8.6 Hz, 1 H), 7.41-7.48 (m, 1 H), 7.68-7.79 (m, 4 H), 7.95 (dd, *J* = 7.0, 2.2 Hz, 1 H), 8.18 (d, *J* = 9.6 Hz, 1 H), 9.79 (d, *J* = 8.4 Hz, 1 H).

**Example 3039**

**3,5-Dimethoxy-*N*-[*cis*-4-(quinolin-2-ylamino)-cyclohexyl]-benzamide hydrochloride**

**Step A: Synthesis of 3,5-dimethoxy-*N*-[*cis*-4-(quinolin-2-ylamino)-cyclohexyl]-benzamide hydrochloride.**

Using the procedure for the step B of example 3033, the title compound was obtained.
ESI MS m/e 428, M (free) + $Na^+$; $^1H$ NMR (300 MHz, $CDCl_3$) δ 1.80-2.14 (m, 8 H), 3.85 (s, 6 H), 3.95-4.26 (m, 2 H), 6.53-6.66 (m, 2 H), 6.89-7.01 (m, 3 H), 7.40-7.51 (m, 1 H), 7.68-7.82 (m, 3 H), 8.18 (d, *J* = 9.6 Hz, 1H), 9.76-9.85 (m, 1H).

**Example 3040**

**3,4-Dichloro-*N*-[*cis*-4-(quinolin-2-ylamino)-cyclohexyl]-benzamide hydrochloride**

**Step A: Synthesis of 3,4-dichloro-*N*-[*cis*-4-(quinolin-2-ylamino)-cyclohexyl]-benzamide hydrochloride.**

Using the procedure for the step B of example 3033, the title compound was obtained.
ESI MS m/e 436, M (free) + $Na^+$; $^1H$ NMR (300 MHz, $CDCl_3$) δ 1.81-2.15 (m, 8 H), 3.98-4.25 (m, 2 H), 6.87-7.00 (m, 2 H), 7.42-7.52 (m, 2 H), 7.65-7.80 (m, 4 H), 7.98 (d, *J* = 2.0 Hz, 1H), 8.19 (d, *J* = 9.5 Hz, 1H), 9.87 (d, *J* = 8.6 Hz, 1H).

**Example 3041**

**Benzo[2,1,3]oxadiazole-5-carboxylic acid-[*cis*-4-(quinolin-2-ylamino)-cyclohexyl]-amide hydrochloride**

**Step A: Synthesis of benzo[2,1,3]oxadiazole-5-carboxylic acid-[*cis*-4-(quinolin-2-ylamino)-cyclohexyl]-amide hydrochloride.**

Using the procedure for the step B of example 3033, the title compound was obtained.
ESI MS m/e 388, M (free) + $H^+$ ; $^1H$ NMR (300 MHz, $CDCl_3$) δ 1.81-2.23 (m, 8 H), 3.98-4.31 (m, 2 H), 6.97 (d, *J* = 9.5 Hz, 1 H), 7.38-7.50 (m, 2 H), 7.70-7.78 (m, 3 H), 7.88 (ddd, *J* = 14.3, 9.3, 1.2 Hz, 2 H), 8.20 (d, *J* = 9.5 Hz, 1 H), 8.41 (t, *J* = 1.2 Hz, 1 H), 9.75 (d, *J* = 8.1 Hz, 1 H).

**Example 3042**

**1-Methyl-4-nitro-1*H*-pyrrole-2-carboxylic acid [*cis*-4-(quinolin-2-ylamino)-cyclohexyl]-amide hydrochloride**

**Step A: Synthesis of 1-methyl-4-nitro-1*H*-pyrrole-2-carboxylic acid [*cis*-4-(quinolin-2-ylamino)-cyclohexyl]-amide hydrochloride.**

Using the procedure for the step A of example 3036, the title compound was obtained.
ESI MS m/e 394, M (free) + $H^+$; $^1H$ NMR (300 MHz, $CDCl_3$) δ 1.80-2.14 (m, 8 H), 3.91-4.15 (m, 5 H), 6.96 (d, *J* = 9.4 Hz, 1 H), 7.09 (d, *J* = 9.0 Hz, 1 H), 7.28-7.31 (m, 1 H), 7.41-7.54 (m, 2 H), 7.67-7.79 (m, 3 H), 8.19 (d, *J* = 9.6 Hz, 1H), 9.66 (m, 1 H).

**Example 3043**

**9*H*-Xanthene-9-carboxylic acid [*cis*-4-(quinolin-2-ylamino)-cyclohexyl]-amide hydrochloride**

**Step A: Synthesis of 9H-Xanthene-9-carboxylic acid [cis-4-(quinolin-2-ylamino)-cyclohexyl]-amide hydrochloride.**

Using the procedure for the step A of example 3036, the title compound was obtained.
ESI MS m/e 472, M (free) + $Na^+$ ; $^1H$ NMR (300 MHz, $CDCl_3$) δ 1.65-1.89 (m, 8 H), 3.76-3.94 (m, 2 H), 5.99-6.09 (m, 1 H), 6.87 (d, *J* = 10.1 Hz, 1 H), 7.05-7.18 (m, 4 H), 7.24-7.47 (m, 5 H), 7.65-7.79 (m, 3 H) 8.13 (d, *J* = 9.6 Hz, 1 H), 9.62 (d, *J* = 7.6 Hz, 1 H).

**Example 3044**

**5-(4-Chloro-phenyl)-furan-2-carboxylic acid [*cis*-4-(quinolin-2-ylamino)-cyclohexyl]-amide hydrochloride**

**Step A: Synthesis of 5-(4-chloro-phenyl)-furan-2-carboxylic acid[*cis*-4-(quinolin-2-ylamino)-cyclohexyl]-amide hydrochloride.**

Using the procedure for the step A of example 3036, the title compound was obtained.
ESI MS m/e 468, M (free) + $Na^+$ ; $^1H$ NMR (300 MHz, $CDCl_3$) δ 1.77-2.13 (m, 8 H), 3.93-4.07 (m, 1 H), 4.10-4.28 (m, 1 H), 6.65-7.03 (m, 3 H), 7.12-7.23 (m, 1 H), 7.32-7.52 (m, 3 H), 7.63-7.85 (m, 5 H), 8.12-8.26 (m, 1 H), 9.74-9.94 (m, 1 H).

**Example 3045**

**3-Nitro-*N*-[*cis*-4-(quinolin-2-ylamino)-cyclohexyl]-benzamide hydrochloride**

**Step A: Synthesis of 3-nitro-*N*-[*cis*-4-(quinolin-2-ylamino)-cyclohexyl]-benzamide hydrochloride.**

Using the procedure for the step A of example 3036, the title compound was obtained.
ESI MS m/e 413, M (free) + $Na^+$; $^1H$ NMR (300 MHz, $CDCl_3$) δ 1.83-2.30 (m, 8 H), 3.99-4.10 (m, 1 H), 4.13-4.31 (m, 1 H), 6.97 (d, *J* = 9.5 Hz, 1 H), 7.24-7.33 (m, 1 H), 7.42-7.51 (m, 1 H), 7.63 (t, *J* = 7.9 Hz, 1 H), 7.70-7.79 (m, 3 H), 8.17-8.24 (m, 2 H), 8.30-8.35 (m, 1 H), 8.75-8.77 (m, 1 H), 9.76 (d, *J* = 7.3 Hz, 1 H).

**Example 3046**

**4-Fluoro-3-methyl-*N*-[*cis*-4-(quinolin-2-ylamino)-cyclohexyl]-benzamide hydrochloride**

**Step A: Synthesis of 4-fluoro-3-methyl-*N*-[*cis*-4-(quinolin-2-ylamino)-cyclohexyl]-benzamide hydrochloride.**

To a solution of *cis*-*N*-quinolin-2-yl-cyclohexane-1,4-diamine obtained in step A of example 3033 (250 mg, 1.04 mmol) in $CHCl_3$ (5 mL) were added $Et_3N$ (0.3 mL, 2.15 mmol) and 4-fluoro-3-methyl-benzoyl chloride (197 mg, 1.14 mmol). The mixture was stirred at ambient temperature for 12 hr. The reaction was quenched with saturated aqueous $NaHCO_3$ and the aqueous layer was extracted with $CHCl_3$ (three times). The combined organic layer was dried over $MgSO_4$, filtered, concentrated, purified by medium-pressure liquid chromatography (NH-silica gel, 20% to 50% EtOAc in hexane). To a solution of the above material in EtOAc (2 mL) was added 4 M hydrogen chloride in EtOAc (10 mL). The mixture was stirred at ambient temperature for 2 hr and concentrated. The residue was suspended in $Et_2O$ (20 mL) and the suspension was stirred at ambient temperature for 6 hr. The precipitate was collected by filtration, washed with $Et_2O$, and dried at 80 °C under reduced pressure to give 4-fluoro-3-methyl-*N*-[*cis*-4-(quinolin-2-ylamino)-cyclohexyl]-benzamide hydrochloride (363 mg, 85%) as a white solid.
ESI MS m/e 400, M (free) + $Na^+$; $^1H$ NMR (300 MHz, $CDCl_3$) δ 1.82-2.10 (m, 8 H), 2.32 (d, *J* = 1.9 Hz, 3 H), 3.96-4.07 (m, 1 H), 4.09-4.27 (m, 1 H), 6.62-6.72 (m, 1 H), 6.96 (d, *J* = 9.5 Hz, 1 H), 7.04 (t, *J* = 8.9 Hz, 1 H), 7.40-7.51 (m, 1 H), 7.61-7.83 (m, 5 H) 8.19 (d, *J* = 9.6 Hz, 1 H), 9.71-9.85 (m, 1 H).

**Example 3047**

**3-Bromo-*N*-[*cis*-4-(quinolin-2-ylamino)-cyclohexyl]-benzamide hydrochloride**

**Step A: Synthesis of 3-bromo-*N*-[*cis*-4-(quinolin-2-ylamino)-cyclohexyl]-benzamide hydrochloride.**

Using the procedure for the step A of example 3046, the title compound was obtained.
ESI MS m/e 446, M (free) + $Na^+$; $^1H$ NMR (300 MHz, $CDCl_3$) δ 1.81-2.13 (m, 8 H), 3.96-4.08 (m, 1 H), 4.09-4.27 (m, 1 H), 6.84 (d, *J* = 7.8 Hz, 1 H), 6.96 (d, *J* = 9.6 Hz, 1 H), 7.30 (t, *J* = 7.9 Hz, 1 H), 7.41-7.50 (m, 1 H), 7.57-7.64 (m, 1 H), 7.69-7.80 (m, 4 H), 8.01 (t, *J* = 1.6 Hz, 1 H), 8.19 (d, *J* = 9.3 Hz, 1 H), 9.71 (m, 1 H).

**Example 3048**

**2-(2-Bromo-phenoxy)-*N*-[*cis*-4-(quinolin-2-ylamino)-cyclohexyl]-nicotinamide hydrochloride**

**Step A: Synthesis of 2-(2-bromo-phenoxy)-*N*-[*cis*-4-(quinolin-2-ylamino)-cyclohexyl]-nicotinamide hydrochloride.**

To a solution of 2-bromo-phenol (453 mg, 2.62 mmol) in DMA (4 mL) was added 60% NaH in oil (210 mg, 5.24 mmol). The mixture was stirred at ambient temperature for 1 hr. To the mixture was added 2-chloro-*N*-[*cis*-4-(quinolin-2-ylamino)-cyclohexyl]-nicotinamide obtained in step A of example 3037 (1.00 g, 2.62 mmol) in DMA (2 mL). The mixture was stirred at 120 °C for 3 hr and the reaction was quenched with water. The aqueous layer was extracted with $CHCl_3$ (three times). The combined organic layer was dried over $MgSO_4$, filtered, concentrated, and purified by medium-pressure liquid chromatography (NH-silica gel, 20% EtOAc in hexane and silica gel, 1% to 2% MeOH in $CHCl_3$) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give 2-(2-bromo-phenoxy)-*N*-[*cis*-4-(quinolin-2-ylamino)-cyclohexyl]-nicotinamide hydrochloride (262 mg, 18%) as a white solid.
ESI MS m/e 517, M (free) + $H^+$ ; $^1H$ NMR (300 MHz, $CDCl_3$) δ 1.89-2.17 (m, 8 H), 3.81-3.98 (m, 1 H), 4.14-4.30 (m, 1 H), 6.92 (d, *J* = 9.5 Hz, 1 H), 7.11-7.20 (m, 2 H), 7.34-7.47 (m, 3 H), 7.63-7.80 (m, 4 H), 7.92-8.00 (m, 1 H), 8.10-8.20 (m, 2 H), 8.54 (dd, *J* = 7.5, 2.0 Hz, 1 H), 9.71-9.88 (m, 1 H).

**Example 3049**

**3-Cyano-*N*-[*cis*-4-(quinolin-2-ylamino)-cyclohexyl]-benzamide hydrochloride**

**Step A: Synthesis of 3-cyano-*N*-[*cis*-4-(quinolin-2-ylamino)-cyclohexyl]-benzamide hydrochloride.**

Using the procedure for the step A of example 3046, the title compound was obtained.
ESI MS m/e 393, M (free) + $Na^+$ ; $^1H$ NMR (300 MHz, $CDCl_3$) δ 1.80-2.17 (m, 8 H), 3.98-4.30 (m, 2 H), 6.97 (d, *J* = 9.3 Hz, 1 H), 7.07-7.18 (m, 1 H), 7.42-7.50 (m, 1 H) 7.56 (t, *J* = 7.8 Hz, 1 H), 7.70-7.80 (m, 4 H), 8.08 (d, *J* = 7.9 Hz, 1 H), 8.17-8.25 (m, 2 H), 9.69-9.84 (m, 1 H).

**Example 3050**

***N*-[*cis*-4-(Quinolin-2-ylamino)-cyclohexyl]-3-trifluoromethyl-benzamide hydrochloride**

**Step A: Synthesis of *N*-[*cis*-4-(quinolin-2-ylamino)-cyclohexyl]-3-trifluoromethyl-benzamide hydrochloride.**

Using the procedure for the step A of example 3046, the title compound was obtained.
ESI MS m/e 436, M (free) + $Na^+$ ; $^1H$ NMR (300 MHz, $CDCl_3$) δ 1.77-2.13 (m, 8 H), 3.97-4.09 (m, 1 H), 4.12-4.33 (m, 1 H), 6.92-7.05 (m, 2 H), 7.41-7.50 (m, 1 H), 7.57 (t, *J* = 7.7 Hz, 1 H), 7.69-7.79 (m, 4 H), 8.02 (d, *J* = 8.1 Hz, 1 H), 8.13-8.26 (m, 2 H), 9.72-9.85 (m, 1 H).

**Example 3051**

***N*-[*cis*-4-(Quinolin-2-ylamino)-cyclohexyl]-*2-m*-tolyloxy-acetamide hydrochloride**

**Step A: Synthesis of *N*-[*cis*-4-(quinolin-2-ylamino)-cyclohexyl]-2-*m*-tolyloxy-acetamide hydrochloride.**

To a solution of *m*-tolyloxy-acetic acid (189 mg, 1.14 mmol) and *cis*-*N*-quinolin-2-yl-cyclohexane-1,4-diamine obtained in step A of example 3036 (250 mg, 1.04 mmol) in DMF (15 mL) were added $Et_3N$ (0.35 mL, 2.50 mmol), HOBt-$H_2O$ (238 mg, 1.56 mmol), and EDC-HCl (219 g, 1.14 mmol). The reaction mixture was stirred at ambient temperature for 13 hr. To the reaction mixture was added water (30 mL) and the aqueous layer was extracted with $CHCl_3$ (three times). The combined organic layer was dried over $MgSO_4$, filtered, concentrated, and purified by medium-pressure liquid chromatography (silica gel, 1% to 5% MeOH in $CHCl_3$) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give *N*-[*cis*-4-(quinolin-2-ylamino)-cyclohexyl]-2-*m*-tolyloxy-acetamide hydrochloride (140 mg, 32%) as a white solid.
ESI MS m/e 412, M (free) + $Na^+$ ; $^1H$ NMR (300 MHz, $CDCl_3$) δ 1.78-2.06 (m, 8 H), 2.33 (s, 3 H), 3.88-4.12 (m, 2 H), 4.44 (s, 2 H), 6.72-6.96 (m, 5 H), 7.18 (t, *J* = 8.0 Hz, 1 H), 7.39-7.47 (m, 1 H), 7.68-7.81 (m, 3 H), 8.17 (d, *J* = 9.3 Hz, 1 H), 9.72-9.89 (m, 1 H).

**Example 3052**

**2,2-Diphenyl-*N*-[*cis*-4-(quinolin-2-ylamino)-cyclohexyl]-acetamide hydrochloride**

**Step A: Synthesis of 2,2-diphenyl-*N*-[*cis*-4-(quinolin-2-ylamino)-cyclohexyl]-acetamide hydrochloride.**

Using the procedure for the step A of example 3046, the title compound was obtained.
ESI MS m/e 458, M (free) + $Na^+$ ; $^1H$ NMR (300 MHz, $CDCl_3$) δ 1.71-1.97 (m, 8 H), 3.84-4.10 (m, 2 H), 4.87 (s, 1 H), 6.16-6.25 (m, 1 H), 6.90 (d, *J* = 9.5 Hz, 1 H), 7.20-7.36 (m, 10 H), 7.39-7.48 (m, 1 H), 7.67-7.79 (m, 3 H), 8.15 (d, *J* = 9.2 Hz, 1 H), 9.63-9.77 (m, 1 H).

**Example 3053**

**5-Bromo-furan-2-carboxylic acid [*cis*-4-(quinolin-2-ylamino)-cyclohexyl)-amide hydrochloride**

**Step A: Synthesis of 5-bromo-furan-2-carboxylic acid [*cis*-4-(quinolin-2-ylamino)-cyclohexyl)-amide hydrochloride.**

Using the procedure for the step A of example 3036, the title compound was obtained.
ESI MS m/e 436, M (free) + $Na^+$; $^1H$ NMR (300 MHz, $CDCl_3$) δ 1.84-2.09 (m, 8 H), 3.92-4.18 (m, 2 H), 6.42 (d, *J* = 3.5 Hz, 1 H), 6.61 (d, *J* = 8.6 Hz, 1 H), 6.95 (d, *J* = 8.2 Hz, 1 H), 7.05 (d, *J* = 3.5 Hz, 1 H), 7.42-7.48 (m, 1 H), 7.69-7.83 (m, 3 H), 8.19 (d, *J* = 9.5 Hz, 1 H), 9.85 (d, *J* = 8.6 Hz, 1 H).

**Example 3054**

**2-(4-Fluoro-phenoxy)-*N*-[*cis*-4-(quinolin-2-ylamino)-cyclohexyl]-nicotinamide hydrochloride**

**Step A: 2-(4-fluoro-phenoxy)-nicotinic acid ethyl ester.**

To a solution of 2-fluoro-phenol (3.02 g, 26.9 mmol) in DMA (20 mL) was added 60% NaH in oil (1.08 mg, 26.9 mmol). The mixture was stirred at ambient temperature for 1.5 hr. To the mixture was added 2-chloro-nicotinic acid ethyl ester (5.00 g, 26.9 mmol) in DMA (5 mL). The mixture was stirred at 120 °C for 2.hr and the reaction was quenched with saturated aqueous $NaHCO_3$. The aqueous layer was extracted with $CHCl_3$ (three times). The combined organic layer was dried over $MgSO_4$, filtered, and concentrated. The residue was suspended in 10% $Et_2O$ in hexane (50 mL) and the suspension was stirred at ambient tempereture for 1 hr. The precipitate was collected by filtration, washed with hexane, and dried at 70 °C under reduced pressure to give 2-(4-fluoro-phenoxy)-nicotinic acid ethyl ester (3.08 g, 44%) as a white solid.

ESI MS m/e 284, M + $Na^+$; $^1H$ NMR (300 MHz, $CDCl_3$) δ 1.40 (td, *J* = 7.1, 1.6 Hz, 3 H), 4.41 (qd, *J* = 7.1, 1.6 Hz, 2 H), 6.99-7.21 (m, 5 H), 8.23-8.29 (m, 2 H).

**Step B: Synthesis of 2-(4-fluoro-phenoxy)-nicotinic acid.**

To a solution of 2-(4-fluoro-phenoxy)-nicotinic acid ethyl ester (3.00 g, 11.5 mmol) in EtOH (90 mL) was added 2 M aqueous NaOH (6 mL). The mixture was stirred at ambient temperature for 4 hr. To the mixture was added 1 M aqueous HCl (pH=3) and concentrated. The residue was dissolved in water and the aqueous layer was extracted with $CHCl_3$ (three times). The combined organic layer was dried over $MgSO_4$, filtered, and concentrated. The residue was suspended in 10% $Et_2O$ in hexane (80 mL) and the suspension was stirred at ambient tempereture for 1 hr. The precipitate was collected by filtration, washed with hexane, and dried at 70 °C under reduced pressure to give 2-(4-fluoro-phenoxy)-nicotinic acid (2.39 g, 89%) as a white solid.
ESI MS m/e 233, $M^+$; $^1H$ NMR (300 MHz, $CDCl_3$) δ 7.05-7.25 (m, 5 H), 8.32 (dd, *J* = 4.8, 2.0Hz, 1 H), 8.49 (dd, *J* = 7.6, 2.0 Hz, 1 H).

**Step C: Synthesis of 2-(4-fluoro-phenoxy)-*N*-[*cis*-4-(quinolin-2-ylamino)-cyclohexyl]-nicotinamide hydrochloride.**

Using the procedure for the step A of example 3036, the title compound was obtained.
ESI MS m/e 479, M (free) + $Na^+$; $^1H$ NMR (300 MHz, $CDCl_3$) δ 1.83-2.14 (m, 8H), 3.88-4.01 (m, 1 H), 4.13-4.30 (m, 1 H), 6.93 (d, *J* = 9.3 Hz, 1 H), 7.11-7.20 (m, 3 H), 7.25-7.31 (m, 2 H), 7.43 (t, *J* = 7.9 Hz, 1 H), 7.67-7.81 (m, 3 H), 7.93 (d, *J* = 7.2 Hz, 1 H), 8.16 (d, *J* = 9.2 Hz, 1 H), 8.21 (dd, *J* = 4.8, 2.0 Hz, 1 H), 8.54 (dd, *J* = 7.6, 2.0 Hz, 1 H), 9.81-9.94 (m, 1 H).

**Example 3055**

**2-(3,4-Difluoro-phenoxy)-*N*-[*cis*-4-(quinolin-2-ylamino)-cyclohexyl]-nicotinamide hydrochloride**

**Step A: Synthesis of 2-(3,4-difluoro-phenoxy)-nicotinic acid.**

To a solution of 3,4-difluoro-phenol (3.77 g, 28.9 mmol) in DMA (20 mL) was added 60% NaH in oil (1.16 mg, 28.9 mmol). The mixture was stirred at ambient temperature for 1 hr. To the mixture was added 2-chloro-nicotinic acid ethyl ester (5.36 g, 28.9 mmol) in DMA (5 mL). The mixture was stirred at 120 °C for 2.5 hr and the reaction was quenched with saturated aqueous $NaHCO_3$. The aqueous layer was extracted with $CHCl_3$ (three times). The combined organic layer was dried over $MgSO_4$, filtered, and concentrated. The residue was suspended in 10% $Et_2O$ in hexane (150 mL) and the suspension was stirred at ambient tempereture for 1 hr. The precipitate was filtered, washed with hexane, and dried at 70 °C under reduced pressure to give a white solid. To a solution of the above material in EtOH (150 mL) was added 2 M aqueous NaOH (15.2 mL). The mixture was stirred at ambient temperature for 12 hr. To the mixture was added I M aqueous HCl (46 mL, pH=3) and concentrated. The residue was dissolved in water and the aqueous layer was extracted with $CHCl_3$ (three times). The combined organic layer was dried over $MgSO_4$, filtered, and concentrated. The residue was suspended in 10% $Et_2O$ in hexane (150 mL) and the suspension was stirred at ambient tempereture for 30 min. The precipitate was collected by filtration, washed with hexane, and dried at 70 °C under reduced pressure to give 2-(3,4-difluoro-phenoxy)-nicotinic acid (4.85 g, 67) as a white solid.
ESI MS m/e 251, $M^+$ ; $^1H$ NMR (300 MHz, $CDCl_3$) δ 6.90-7.30 (m, 4 H), 8.30-8.35 (m, 1 H), 8.46-8.52 (m, 1 H).

**Step B: Synthesis of 2-(3,4-difluoro-phenoxy)-*N*-[*cis*-4-(quinolin-2-ylamino)-cyclohexyl]-nicotinamide hydrochloride**

Using the procedure for the step A of example 3036, the title compound was obtained.
ESI MS m/e 475, M (free) + $H^+$; $^1H$ NMR (300 MHz, $CDCl_3$) δ 1.85-2.13 (m, 8 H), 3.91-4.03 (m, 1 H), 4.13-4.29 (m, 1 H), 6.94 (d, *J* = 9.6 Hz, 1 H), 7.11-7.34 (m, 4 H), 7.40-7.47 (m, 1 H), 7.67-7.85 (m, 4 H), 8.17 (d, *J* = 9.5 Hz, 1 H), 8.22 (dd, *J* = 4.8, 2.0 Hz, 1 H), 8.53 (dd, *J* = 7.6, 2.0 Hz, 1 H), 9.84-9.98 (m, 1 H).

**Example 3056**

***N*-[*cis*-4-(Quinolin-2-ylamino)-cyclohexyl]-2-*p*-tolyloxy-nicotinamide hydrochloride**

**Step A: Synthesis of 2-*p*-tolyloxy-nicotinic acid.**

Using the procedure for the step A of example 3055, the title compound was obtained.
ESI MS m/e 229, $M^+$; $^1H$ NMR (300 MHz, $CDCl_3$) δ 2.40 (s, 3 H) 7.05-7.31 (m, 5 H), 8.30-8.35 (m, 1 H), 8.52-8.57 (m, 1 H).

**Step B: Synthesis of *N*-[*cis*-4-(quinolin-2-ylamino)-cyclohexyl]-2-*p*-tolyloxy-nicotinamide hydrochloride.**

Using the procedure for the step A of example 3036, the title compound was obtained.
ESI MS m/e 475, M (free) + $Na^+$ ; $^1H$ NMR (300 MHz, $CDCl_3$) δ 1.86-2.13 (m, 8 H), 2.36 (s, 3 H), 3.86-3.98 (m, 1 H), 4.11-4.29 (m, 1 H), 6.86-7.00 (m, 1 H), 7.07-7.31 (m, 5 H), 7.43 (t, *J* = 7.6 Hz, 1 H), 7.64-7.82 (m, 3 H), 7.92-8.28 (m, 3 H), 8.53 (d, *J* = 7.0 Hz, 1 H), 9.74-9.87 (m, 1 H).

**Example 3057**

**2-(4-Chloro-phenoxy)-*N*-[*cis*-4-(quinolin-2-ylamino)-cyclohexyl]-nicotinamide hydrochloride**

**Step A: Synthesis of 2-(4-chloro-phenoxy)-nicotinic acid.**

Using the procedure for the step A of example 3055, the title compound was obtained.
ESI MS m/e 250, M + $H^+$ ; $^1H$ NMR (300 MHz, $CDCl_3$) δ 7.10-7.21 (m, 3 H), 7.36-7.45 (m, 2 H), 8.30-8.36 (m, 1 H), 8.45-8.51 (m, 1 H).

**Step B: Synthesis of 2-(4-chloro-phenoxy)-*N*-[*cis*-4-(quinolin-2-ylamino)-cyclohexyl]-nicotinamide hydrochloride.**

Using the procedure for the step A of example 3036, the title compound was obtained.
ESI MS m/e 473, M + $H^+$ ; $^1H$ NMR (300 MHz, $CDCl_3$) δ 1.83-2.13 (m, 8 H), 3.87-4.04 (m, 1 H), 4.10-4.33 (m, 1 H), 6.87-7.01 (m, 1 H), 7.10-7.35 (m, 3 H), 7.38-7.50 (m, 3 H), 7.65-7.93 (m, 4 H), 8.10-8.26 (m, 2 H), 8.53 (d, *J* = 6.4 Hz, 1 H), 9.78-9.97 (m, 1 H).

**Example 3058**

**2-(4-Bromo-phenoxy)-*N*-[*cis*-4-(quinolin-2-ylamino)-cyclohexyl]-nicotinamide hydrochloride**

**Step A: 2-(4-bromo-phenoxy)-nicotinic acid**

Using the procedure for the step A of example 3055, the title compound was obtained.
ESI MS m/e 294, M + $H^+$ ; $^1H$ NMR (300 MHz, $CDCl_3$) δ 7.05-7.12 (m, 2 H), 7.18 (dd, *J* = 7.6, 4.8 Hz, 1 H), 7.52-7.62 (m, 2 H), 8.31-8.35 (m, 1 H), 8.48 (dd, *J* = 7.6, 2.0 Hz, 1 H).

**Step B: Synthesis of 2-(4-bromo-phenoxy)-*N*-[*cis*-4-(quinolin-2-ylamino)-cyclohexyl]-nicotinamide hydrochloride**

Using the procedure for the step A of example 3036, the title compound was obtained.
ESI MS m/e 517, M (free) + $H^+$; $^1H$ NMR (300 MHz, $CDCl_3$) δ 1.80-2.11 (m, 8 H), 3.87-4.02 (m, 1 H), 4.12-4.30 (m, 1 H), 6.86-7.01 (m, 1 H), 7.09-7.29 (m, 3 H), 7.38-7.48 (m, 1 H), 7.51-7.62 (m, 2 H), 7.64-7.93 (m, 4 H), 8.11-8.25 (m, 2 H), 8.53 (d, *J* = 7.6 Hz, 1 H), 9.78-9.96 (m, 1 H).

**Example 3059**

**2-(4-Methoxy-phenoxy)-*N*-[*cis*-4-(guinolin-2-ylamino)-cyclohexyl]-nicotinamide hydrochloride**

**Step A: Synthesis of 2-(4-methoxy-phenoxy)-nicotinic acid.**

Using the procedure for the step A of example 3055, the title compound was obtained. ESI MS m/e 245, $M^+$ ; $^1H$ NMR (300 MHz, $CDCl_3$) δ 6.94-7.01 (m, 2 H), 7.09-7.20 (m, 3 H), 8.31-8.35 (m, 1 H), 8.50-8.55 (m, 1 H).

**Step B: Synthesis of 2-(4-methoxy-phenoxy)-*N*-[*cis*-4-(quinolin-2-ylamino)-cyclohexyl]-nicotinamide hydrochloride.**

Using the procedure for the step A of example 3036, the title compound was obtained. ESI MS m/e 491, M (free) + $Na^+$; $^1H$ NMR (300 MHz, $CDCl_3$) δ 1.85-2.12 (m, 8 H), 3.81 (brs, 3 H), 3.86-3.99 (m, 1 H), 4.12-4.30 (m, 1 H), 6.84-7.29 (m, 6 H), 7.37-7.49 (m, 1 H), 7.64-7.82 (m, 3 H), 7.96-8.28 (m, 3 H), 8.48-8.60 (m, 1 H), 9.71-9.86 (m, 1 H).

**Example 3060**

**2-(3-Chloro-4-fluoro-phenoxy)-*N*-[*cis*-4-(quinolin-2-ylamino)-cyclohexyl]-nicotinamide hydrochloride**

**Step A: Synthesis of 2-(3-chloro-4-fluoro-phenoxy)-nicotinic acid.**

Using the procedure for the step A of example 3055, the title compound was obtained. ESI MS m/e 268, M + $H^+$ ; $^1H$ NMR (200 MHz, $CDCl_3$) δ 7.03-7.32 (m, 4 H), 8.29-8.37 (m, 1 H), 8.44-8.53 (m, 1 H).

**Step B: Synthesis of 2-(3-chloro-4-fluoro-phenoxy)-*N*-[*cis*-4-(quinolin-2-ylamino)-cyclohexyl]-nicotinamide hydrochloride.**

Using the procedure for the step A of example 3036, the title compound was obtained. ESI MS m/e 491, M (free) + $H^+$ ; $^1H$ NMR (300 MHz, $CDCl_3$) δ 1.83-2.10 (m, 8 H), 3.88-4.04 (m, 1 H), 4.11-4.27 (m, 1 H), 6.92 (d, *J* = 9.6 Hz, 1 H) 7.16 (dd, *J* = 7.6, 4.8 Hz, 1 H), 7.21-7.46 (m, 4 H), 7.67-7.81 (m, 4 H), 8.15 (d, *J* = 9.5 Hz, 1 H), 8.20 (dd, *J* = 4.8, 2.0 Hz, 1 H), 8.52 (dd, *J* = 7.6, 2.0 Hz, 1 H), 9.83-9.95 (m, 1 H).

**Example 3061**

***N*-[*cis*-4-(Quinolin-2-ylamino)-cyclohexyl]-2-*m*-tolyloxy-nicotinamide hydrochloride**

**Step A: Synthesis of 2-*m*-tolyloxy-nicotinic acid**

Using the procedure for the step A of example 3055, the title compound was obtained. ESI MS m/e 229, $M^+$ ; $^1H$ NMR (200 MHz, $CDCl_3$) δ 2.40 (s, 3 H), 6.95-7.41 (m, 5 H), 8.33 (dd, *J* = 4.8, 1.8 Hz, 1 H), 8.54 (dd, *J* = 7.7, 1.9 Hz, 1 H).

**Step B: Synthesis of *N*-[*cis*-4-(quinolin-2-ylamino)-cyclohexyl]-2-*m*-tolyloxy-nicotinamide hydrochloride.**

Using the procedure for the step A of example 3036, the title compound was obtained. ESI MS m/e 475, M + $Na^+$; $^1H$ NMR (300 MHz, $CDCl_3$) δ 1.87-2.07 (m, 8 H), 2.40 (s, 3 H), 3.85-3.98 (m, 1 H), 4.10-4.25 (m, 1 H), 6.88-6.99 (m, 1 H), 7.01-7.18 (m, 4 H), 7.33 (t, *J* = 7.8 Hz, 1 H), 7.42 (t, *J* = 7.5 Hz, 1 H), 7.66-7.81 (m, 3 H), 7.93-8.03 (m, 1 H), 8.12-8.20 (m, 1 H), 8.23 (dd, *J* = 4.7, 1.9 Hz, 1 H), 8.52 (dd, *J* = 7.5, 1.9 Hz, 1 H), 9.71-9.83 (m, 1 H).

**Example 3062**

**2-(3-Methoxy-phenoxy)-*N*-[*cis*-4-(quinolin-2-ylamino)-cyclohexyl]-acetamide hydrochloride**

**Step A: Synthesis of (3-methoxy-phenoxy)-acetic acid ethyl ester.**

To a solution of 3-methoxy-phenol (3.54 g, 28.5 mmol) in DMA (20 mL) was added 60% NaH in oil (1.14 g, 28.5 mmol). The mixture was stirred at ambient temperature for 1.5 hr. To the mixture was added bromo-acetic acid ethyl esterr (4.53 g, 28.5 mmol) in DMA (10 mL). The mixture was stirred at ambient temperature for 1.5 hr and the reaction was quenched with saturated aqueous $NaHCO_3$. The aqueous layer was extracted with $CHCl_3$ (three times). The combined organic layer was dried over $MgSO_4$, filtered, concentrated, and purified by medium-pressure liquid chromatography (silica gel, 20% EtOAc in hexane) to give (3-methoxy-phenoxy)-acetic acid ethyl ester (5.19 g, 91 %) as a colorless oil.
ESI MS m/e 233, M + $Na^+$; $^1H$ NMR (300 MHz, $CDCl_3$) δ 1.30 (t, *J* = 7.1 Hz, 3 H), 3.79 (s, 3 H), 4.27 (q, *J* = 7.1 Hz, 2 H), 4.60 (s, 2 H), 6.44-6.61 (m, 3 H), 7.15-7.23 (m, 1 H).

**Step B: Synthesis of (3-methoxy-phenoxy)-acetic acid.**

To a solution of (3-methoxy-phenoxy)-acetic acid ethyl ester (5.06 g, 24.1 mmol) in EtOH (100 mL) was added 1 M aqueous NaOH (25.3 mL). The mixture was stirred at ambient temperature for 1 hr. To the mixture was added 1 M aqueous HCl (pH=3) and concentrated. The residue was dissolved in water and the aqueous layer was extracted with $CHCl_3$ (three times). The combined organic layer was dried over $MgSO_4$, filtered, and concentrated to give (3-methoxy-phenoxy)-acetic acid (4.05 g, 92%) as a white solid.
ESI MS m/e 182, $M^+$ ; $^1H$ NMR (300 MHz, DMSO-$d_6$) δ 3.73 (s, 3 H), 4.65 (s, 2 H), 6.45-6.57 (m, 3 H), 7.13-7.23 (m, 1 H), 12.97 (brs, 1 H).

**Step C: Synthesis of 2-(3-methoxy-phenoxy)-*N*-[*cis*-4-(quinolin-2-ylamino)-cyclohexyl]-acetamide hydrochloride.**

Using the procedure for the step A of example 3036, the title compound was obtained.
ESI MS m/e 406, M (free) + $H^+$ ; $^1H$ NMR (300 MHz, $CDCl_3$) δ 1.79-2.05 (m, 8 H), 3.82 (s, 3 H), 3.90-4.11 (m, 2 H), 4.46 (s, 2 H), 6.52-6.61 (m, 3 H), 6.80-6.87 (m, 1 H), 6.93 (d, *J* = 9.5 Hz, 1 H), 7.16-7.24 (m, 1 H), 7.41-7.48 (m, 1 H), 7.69-7.82 (m, 3 H), 8.17 (d, *J* = 9.5 Hz, 1 H) 9.78-9.88 (m, 1 H).

**Example 3063**

**2-(3-Chloro-phenoxy)-*N*-[*cis*-4-(quinolin-2-ylamino)-cyclohexyl]-acetamide hydrochloride**

**Step A: Synthesis of (3-chloro-phenoxy)-acetic acid ethyl ester.**

Using the procedure for the step A of example 3062, the title compound was obtained.
ESI MS m/e 237, M + $Na^+$; $^1H$ NMR (300 MHz, $CDCl_3$) δ 1.30 (t, *J* = 7.2 Hz, 3 H), 4.28 (q, *J* = 7.2 Hz, 2 H), 4.60 (s, 2 H), 6.73-7.02 (m, 3 H), 7.14-7.30 (m, 1 H).

**Step B: Synthesis of (3-chloro-phenoxy)-acetic acid.**

Using the procedure for the step B of example 3062, the title compound was obtained.
ESI MS m/e 187, M + $H^+$ ; $^1H$ NMR (300 MHz, $CDCl_3$) δ 4.73 (d, *J* = 1.2 Hz, 2 H), 6.87-6.94 (m, 1 H), 6.98-7.05 (m, 2 H), 7.27-7.35 (m, 1 H), 13.07 (s, 1 H).

**Step C: Synthesis of 2-(3-chloro-phenoxy)-*N*-[*cis*-4-(quinolin-2-ylamino)-cyclohexyl]-acetamide hydrochloride.**

Using the procedure for the step A of example 3036, the title compound was obtained.
ESI MS m/e 410, M (free) + $H^+$; $^1H$ NMR (300 MHz, $CDCl_3$) δ 1.78-2.07 (m, 8 H), 3.90-4.14 (m, 2 H), 4.45 (s, 2 H) 6.74-6.84 (m, 1 H), 6.86-7.03 (m, 4 H), 7.20-7.29 (m, 1 H), 7.40-7.49 (m, 1 H), 7.69-7.82 (m, 3 H), 8.18 (d, *J* = 9.3 Hz, 1 H), 9.79-9.93 (m, 1 H).

**Example 3064**

**2-(3-Chloro-4-fluoro-phenoxy)-*N*-[*cis*-4-(quinolin-2-ylamino)-cyclohexyl]-acetamide hydrochloride**

**Step A: Synthesis of (3-chloro-4-fluoro-phenoxy)-acetic acid ethyl ester.**

Using the procedure for the step A of example 3062, the title compound was obtained.
ESI MS m/e 233, M + H$^+$ ; $^1$H NMR (300 MHz, $CDCl_3$) δ 1.30 (t, *J* = 7.1 Hz, 3 H), 4.28 (q, *J* = 7.1 Hz, 2 H), 4.58 (s, 2 H), 6.75-6.82 (m, 1 H), 6.95 (dd, *J* = 5.9, 3.1 Hz, 1 H), 7.01-7.11 (m, 1 H).

**Step B: Synthesis of (3-chloro-4-fluoro-phenoxy)-acetic acid.**

Using the procedure for the step B of example 3062, the title compound was obtained.
ESI MS m/e 205, M + H$^+$ ; $^1$H NMR (300 MHz, DMSO-$d_6$) δ 4.72 (s, 2 H), 6.92-6.97 (m, 1 H), 7.17 (dd, *J* = 6.1, 3.1 Hz, 1 H), 7.34 (t, *J* = 9.1 Hz, 1 H), 13.08 (brs, 1 H).

**Step C: Synthesis of 2-(3-chloro-4-fluoro-phenoxy)-*N*-[*cis*-4-(quinolin-2-ylamino)-cyclohexyl]-acetamide hydrochloride.**

Using the procedure for the step A of example 3036, the title compound was obtained.
ESI MS m/e 450, M (free) + Na$^+$ ; $^1$H NMR (300 MHz, $CDCl_3$) δ 1.76-2.08 (m, 8 H), 3.91-4.13 (m, 2 H), 4.42 (s, 2 H), 6.73-6.97 (m, 3 H), 7.00-7.14 (m, 2 H), 7.41-7.49 (m, 1 H), 7.70-7.80 (m, 3 H), 8.18 (d, *J* = 9.5 Hz, 1 H), 9.79-9.90 (m, 1 H).

**Example 3065**

**2-(3,4-Dichloro-phenoxy)-*N*-[*cis*-4-(quinolin-2-ylamino)-cyclohexyl]-acetamide hydrochloride**

**Step A: Synthesis of (3,4-dichloro-phenoxy)-acetic acid ethyl ester.**

Using the procedure for the step A of example 3062, the title compound was obtained.
CI MS m/e 249, M$^+$ ;$^1$H NMR (300 MHz, $CDCl_3$) δ 1.30 (t, *J* = 7.1 Hz, 3 H), 4.28 (q, *J* = 7.1 Hz, 2 H), 4.59 (s, 2 H), 6.78 (dd, *J* = 9.0, 2.9 Hz, 1 H), 7.01 (d, *J* = 2.8 Hz, 1 H), 7.34 (d, *J* = 9.1 Hz, 1 H).

**Step B: Synthesis of (3,4-dichloro-phenoxy)-acetic acid.**

Using the procedure for the step B of example 3062, the title compound was obtained.
ESI MS m/e 221, M + H$^+$ ; $^1$H NMR (300 MHz, DMSO-$d_6$) δ 4.76 (s, 2 H), 6.96 (dd, *J* = 8.9, 2.9 Hz, 1 H), 7.24 (d, *J* = 2.9 Hz, 1 H), 7.53 (d, *J* = 8.9 Hz, 1 H), 13.12 (brs, 1 H).

**Step C: Synthesis of 2-(3,4-dichloro-phenoxy)-*N*-[*cis*-4-(quinolin-2-ylamino)-cyclohexyl]-acetamide hydrochloride.**

Using the procedure for the step A of example 3036, the title compound was obtained.
ESI MS m/e 466, M (free) + Na$^+$ ; $^1$H NMR (300 MHz, $CDCl_3$) δ 1.75-2.07 (m, 8 H), 3.92-4.13 (m, 2 H), 4.44 (s, 2 H), 6.78 (d, *J* = 8.1 Hz, 1 H), 6.86-6.97 (m, 2 H), 7.10 (d, *J* = 2.9 Hz, 1 H), 7.37 (d, *J* = 8.9 Hz, 1 H), 7.41-7.49 (m, 1 H), 7.67-7.82 (m, 3 H), 8.18 (d, *J* = 9.5 Hz, 1 H), 9.80-9.90 (m, 1 H).

**Example 3066**

***C*-(Methyl-phenyl-amino)-*N*-[*cis*-4-(quinolin-2-ylamino)-cyclohexyl]-acetamide dihydrochloride**

**Step A: Synthesis of (methyl-phenyl-amino)-acetic acid ethyl ester.**

To a solution of bromo-acetic acid ethyl ester (5.00 g, 29.9 mmol) in IPA (10 mL) were added *i*-$Pr_2NEt$ (4.06 g, 31.4 mmol) and methyl-phenyl-amine (3.37 g, 31.4 mmol). The mixture was stirred at reflux for 2.5 hr and the reaction was quenched with saturated aqueous $NaHCO_3$. The aqueous layer was extracted with $CHCl_3$ (three times). The combined organic layer was dried over $MgSO_4$, filtered, concentrated, and purified by medium-pressure liquid chromatography (silica gel, 20% EtOAc in hexane) to give (methyl-phenyl-amino)-acetic acid ethyl ester (5.61 g, 97%) as

a yellow oil.
ESI MS m/e 216, M + Na$^+$; $^1$H NMR (300 MHz, $CDCl_3$) δ 1.24 (t, *J* = 7.1 Hz, 3 H), 3.07 (s, 3 H), 4.05 (s, 2 H), 4.17 (q, *J* = 7.1 Hz, 2 H), 6.63-6.79 (m, 3 H), 7.18-7.29 (m, 2 H).

**Step B: Synthesis of (methyl-phenyl-amino)-acetic acid.**

To a solution of (methyl-phenyl-amino)-acetic acid ethyl ester (5.48 g, 28.4 mmol) in EtOH (100 mL) was added 1 M aqueous NaOH (29.8 mL). The mixture was stirred at ambient temperature for 1.5 hr. To the mixture was added 1 M aqueous HCl (pH=3) and concentrated. The residue was dissolved in water and the aqueous layer was extracted with $CHCl_3$ (three times). The combined organic layer was dried over $MgSO_4$, filtered, concentrated, and purified by medium-pressure liquid chromatography (silica gel, 20% EtOAc in hexane) to give (methyl-phenyl-amino)-acetic acid (1.73 g, 37%) as a yellow oil.
ESI MS m/e 165, M$^+$ ;$^1$H NMR (300 MHz, $CDCl_3$) δ 3.05 (s, 3 H), 4.07 (s, 2 H), 6.65-6.85 (m, 3 H), 7.18-7.30 (m, 2 H), 8.62 (brs, 1 H).

**Step C: Synthesis of *C*-(methyl-phenyl-amino)-*N*-[*cis*-4-(quinolin-2-ylamino)-cyclohexyl]-acetamide dihydrochloride.**

Using the procedure for the step A of example 3036, the title compound was obtained.
ESI MS m/e 411, M (free) + Na$^+$ ; $^1$H NMR (300 MHz, $CDCl_3$) δ 1.73-1.99 (m, 8 H), 3.05-3.16 (m, 3 H), 3.79-4.02 (m, 4 H), 6.82-7.00 (m, 4 H), 7.06-7.49 (m, 5 H), 7.65-7.80 (m, 3 H), 8.15 (d, *J* = 9.9 Hz, 1 H), 9.57-9.68 (m, 1 H).

**Example 3067**

**2-(3,4-Dichloro-phenylamino)-*N*-[*cis*-4-(quinolin-2-ylamino)-cyclohexyl]-acetamide dihydrochloride**

**Step A: Synthesis of (3,4-dichloro-phenylamino)-acetic acid ethyl ester.**

Using the procedure for the step A of example 3066, the title compound was obtained.
CI MS m/e 248, M + H$^+$; $^1$H NMR (300 MHz, $CDCl_3$) δ 1.31 (t, *J* = 7.1 Hz, 3 H), 3.85 (d, *J* = 5.4 Hz, 2 H), 4.26 (q, *J* = 7.1 Hz, 2 H), 4.33-4.42 (m, 1 H), 6.45 (dd, *J* = 8.7, 2.8 Hz, 1 H), 6.66 (d, *J* = 2.8 Hz, 1 H), 7.21 (d, *J* = 8.7 Hz, 1 H).

**Step B: Synthesis of (3,4-dichloro-phenylamino)-acetic acid.**

Using the procedure for the step B of example 3054, the title compound was obtained.
ESI MS m/e 220, M + H$^+$;$^1$H NMR (300 MHz, $CDCl_3$) δ 3.84 (s, 2 H), 6.37 (brs, 1 H), 6.57 (dd, *J* = 8.8, 2.7 Hz, 1 H), 6.76 (d, *J* = 2.6 Hz, 1 H), 7.26 (d, *J* = 8.8 Hz, 1 H), 12.67 (brs, 1 H).

**Step C: Synthesis of 2-(3,4-dichloro-phenylamino)-*N*-[*cis*-4-(quinolin-2-ylamino)-cyclohexyl]-acetamide dihydrochloride**

Using the procedure for the step A of example 3036, the title compound was obtained.
ESI MS m/e 465, M (free) + Na$^+$ ; $^1$H NMR (300 MHz, $CDCl_3$) δ 1.72-2.05 (m, 8 H), 3.80 (s, 2 H), 3.87-4.10 (m, 2 H), 6.48-6.57 (m, 1 H), 6.73 (brs, 1 H), 6.86-7.05 (m, 2 H), 7.18 (d, *J* = 8.7 Hz, 1 H), 7.39-7.50 (m, 1 H), 7.66-7.80 (m, 3 H), 8.11-8.24 (m, 1 H), 9.55-9.68 (m, 1 H).

**Example 3068**

**3,4-Difluoro-*N*-[*cis*-4-(quinolin-2-ylamino)-cyclohexylmethyl]-benzamide hydrochloride**

**Step A: Synthesis of (*cis*-4-hydroxymethyl-cyclohexyl)-carbamic acid *tert*-butyl ester.**

A suspension of *cis*-4-amino-cyclohexanecarboxylic acid (244 g, 1.70 mol) in MeOH (2.45 L) was cooled to-8 °C. Thionyl chloride (45.0 mL, 617 mmol) was added dropwise. The resulting solution was stirred at ambient temperature for 4.5 hr and concentrated to give a white solid. To a suspension of the above solid in $CHCl_3$ (3.00 L) were added triethylamine (261 mL, 1.87 mol) and $(Boc)_2O$ (409 g, 1.87 mol) successively. The reaction mixture was stirred at ambient temperature for 5 hr and poured into water. The aqueous layer was extracted with $CHCl_3$ (three times). The combined organic layer was dried over $MgSO_4$, filtrated, concentrated, and purified by flash chromatography (silica

gel, $CHCl_3$ only to 10% MeOH in $CHCl_3$) to give a colorless oil (531 g). To a suspension cooled at -4 °C of lithium aluminum hydride (78.3 g, 2.06 mol) in $Et_2O$ (7.9 L) was added a solution of the above oil (530.9 g) in $Et_2O$ (5.3 L) below 0°C. The resulting suspension was stirred at ambient temperature for 2 hr. The reaction mixture was cooled on an ice-bath, quenched with cold water, and filtrated through a pad of celite. The filtrate was dried over $MgSO_4$, filtrated, and concentrated. The precipitate was suspended in hexane (300 mL), filtrated, washed with hexane, and dried under reduced pressure to give (*cis*-4-hydroxymethyl-cyclohexyl)-carbamic acid *tert*-butyl ester (301 g, 77%) as a white solid.
ESI MS m/e 252, M + $Na^+$ ; $^1H$ NMR (300 MHz, $CDCl_3$) δ 1.16-1.36 (m, 2 H), 1.45 (s, 9 H), 1.52-1.77 (m, 7 H), 3.51 (d, *J* = 6.2 Hz, 2 H), 3.75 (brs, 1 H), 4.30-4.82 (m, 1 H).

**Step B: Synthesis of [*cis*-4-(benzyloxycarbonylamino-methyl)-cyclohexyl]-carbamic acid *t*ert-butyl ester.**

To a solution of (*cis*-4-hydroxymethyl-cyclohexyl)-carbamic acid *tert*-butyl ester (17.7 g, 77.2 mmol) in THF (245 mL) were added triphenylphosphine (20.2 g, 77.0 mmol) and phthalimide (11.4 g, 77.5 mmol) successively. The resulting suspension was cooled on an ice-bath and 40% diethyl azodicarboxylate (DEAD) in toluene (33.6 mL, 74.1 mmol) was added over 1 hr. The reaction mixture was stirred at ambient temperature for 2.5 days, concentrated, and purified by flash chromatography (silica gel, 33% EtOAc in hexane) to give a white solid. To a suspension of the above solid (27.5 g) in EtOH (275 mL) was added hydrazine hydrate (5.76 g, 115 mmol). The mixture was stirred at reflux for 2.25 hr, cooled, and concentrated. The precipitate was dissolved in 10% aqueous sodium hydroxide (350 mL). The aqueous layer was extracted with $CHCl_3$ (three times). The combined organic layer was dried over $MgSO_4$, filtrated, and concentrated. To a solution of the above residue in $CHCl_3$ (275 mL) was added triethylamine (8.54 g, 84.4 mmol). The resulting solution was cooled to 0 °C and ZCl (14.4 g, 84.4 mmol) was added below 5 °C. The reaction mixture was stirred at ambient temperature for 16 hr and poured into saturated aqueous $NaHCO_3$. The aqueous layer was extracted with $CHCl_3$ (three times). The combined organic layer was dried over $MgSO_4$, filtrated, concentrated, and purified by flash chromatography (silica gel, 2% MeOH in $CHCl_3$) to give [*cis*-4-(benzyloxycarbonylamino-methyl)-cyclohexyl]-carbamic acid *tert*-butyl ester (25.3 g, 91%) as a colorless oil.
ESI MS m/e 385, M + $Na^+$; $^1H$ NMR (300 MHz, $CDCl_3$) δ 1.13-1.31 (m, 2 H), 1.44 (s, 9 H), 1.48-1.75 (m, 7 H), 3.10 (t, *J* = 6.4 Hz, 2 H), 3.72 (brs, 1 H), 4.42-4.76 (m, 1 H), 4.76-4.92 (m, 1 H), 5.09 (s, 2 H), 7.27-7.38 (m, 5 H).

**Step C: Synthesis of (*cis*-4-amino-cyclohexylmethyl)-carbamic acid benzyl ester.**

To a solution of [*cis*-4-(benzyloxycarbonylamino-methyl)-cyclohexyl]-carbamic acid *tert*-butyl ester (12.9 g, 35.6 mmol) in EtOAc (129 mL) was added 4 M hydrogen chloride in EtOAc (129 mL). The reaction mixture was stirred at ambient temperature for 3 hr, filtrated, washed with EtOAc, and dried under reduced pressure. The solid was dissolved in saturated aqueous $NaHCO_3$. The aqueous layer was extracted with $CHCl_3$ (five times). The combined organic layer was dried over $MgSO_4$, filtrated, concentrated, and dried under reduced pressure to give (*cis*-4-amino-cyclohexylmethyl)-carbamic acid benzyl ester (8.88 g, 95%) as a colorless oil.
ESI MS m/e 263, M + $H^+$ ;$^1H$ NMR (300 MHz, $CDCl_3$) δ 1.36-1.98 (m, 9 H), 2.96-3.32 (m, 3 H), 5.12 (brs, 3 H), 7.36 (s, 5 H).

**Step D: Synthesis of (*cis*-4-aminomethyl-cyclohexyl)-quinolin-2-yl-amine.**

A mixture of 2-chloro-quinoline (10.0 g, 61.1 mmol) and (*cis*-4-amino-cyclohexylmethyl)-carbamic acid benzyl ester (17.6 g, 67.2 mmol) in butanol (10 mL) was stirred at reflux for 2 days. The reaction mixture was poured into saturated aqueous $NaHCO_3$, and the aqueous layer was extracted with $CHCl_3$ (three times). The combined organic layer was dried over $MgSO_4$, filtrated, concentrated, and purified by flash chromatography (NH-silica, 33% to 50% EtOAc in hexane) to give a pale yellow oil. To a solution of the above oil in MeOH (100 mL) was added 10% Pd/C (1.00 g). The mixture was stirred at ambient temperature under hydrogen atmosphere for 1.5 days. The reaction mixture was filtrated through a pad of celite, concentrated, and purified by medium-pressure liquid chromatography (NH-silica gel, 2% MeOH in $CHCl_3$) to give (*cis*-4-aminomethyl-cyclohexyl)-quinolin-2-yl-amine (6.20 g, 40%) as a pale yellow solid.
ESI MS m/e 256, M + $H^+$ $^1H$ NMR (300 MHz, $CDCl_3$) δ 1.12-1.51 (m, 4 H), 1.59-1.93 (m, 5 H), 2.60 (d, *J* = 6.2 Hz, 2 H), 4.08-4.20 (m, 1 H), 4.94 (d, *J* = 7.4 Hz, 1 H), 6.65 (d, *J* = 9.0 Hz, 1 H), 7.18 (ddd, *J* = 7.9, 6.8, 1.1 Hz, 1 H) 7.47-7.59 (m, 2 H), 7.61-7.67 (m, 1 H) 7.81 (d, *J* = 8.9 Hz, 1 H).

**Step E: Synthesis of 3,4-difluoro-*N*-[*cis*-4-(quinolin-2-ylamino)-cyclohexylmethyl]-benzamide hydrochloride.**

To a solution of *cis*-(4-an-amino methyl-cyclohexyl)-quinolin-2-yl-amine (300 mg, 1.17 mmol) and 3,4-difluoro-benzoic acid (223 mg, 1.41 mmol) in DMF (3 mL) were added $Et_3N$ (0.40 mL, 2.87 mmol), HOBt-$H_2O$ (270 mg, 1.76

mmol), and EDC-HCl (270 mg, 1.41 mmol). The reaction mixture was stirred at ambient temperature for 16 hr. To the reaction mixture was added water (20 mL) and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane). To a solution of the above material in EtOAc (2 mL) was added 4 M hydrogen chloride in EtOAc (10 mL). The mixture was stirred at ambient temperature for 2 hr, filtered, washed with $Et_2O$, and dried at 80 °C under reduced pressure to give 3,4-difluoro-*N*-[*cis*-4-(quinolin-2-ylamino)-cyclohexylmethyl]-benzamide hydrochloride (390 mg, 77%) as a white solid.
ESI MS m/e 418, M (free) + $Na^+$ ; $^1H$ NMR (300 MHz, $CDCl_3$) δ 1.65-2.08 (m, 9 H), 3.48-3.56 (m, 2 H), 3.98-4.09 (m, 1 H), 6.92-7.07 (m, 2 H), 7.18-7.29 (m, 1 H), 7.39-7.47 (m, 1 H), 7.67-7.76 (m, 3 H), 7.81-7.93 (m, 2 H), 8.15 (d, *J* = 9.6 Hz, 1 H), 9.86-9.95 (m, 1 H).

**Example 3069**

**2-Phenoxy-N-[*cis*-4-(quinolin-2-ylamino)-cyclohexylmethyl]-nicotinamide hydrochloride**

**Step A: Synthesis of 2-phenoxy-*N*-[*cis*-4-(quinolin-2-ylamino)-cyclohexylmethyl]-nicotinamide hydrochloride.**

Using the procedure for the step E of example 3068, the title compound was obtained.
ESI MS m/e 475, M (free) + $Na^+$ ; $^1H$ NMR (300 MHz, $CDCl_3$) δ 1.54-2.02 (m, 9 H), 3.42-3.52 (m, 2 H), 3.91-4.05 (m, 1 H), 6.91 (d, *J* = 9.5 Hz, 1 H), 7.10-7.20 (m, 3 H), 7.23-7.31 (m, 1 H), 7.38-7.50 (m, 3 H), 7.65-7.82 (m, 3 H), 8.06-8.17 (m, 2 H), 8.20 (dd, *J* = 4.7, 2.0 Hz, 1 H) 8.60 (dd, *J* = 7.7,1.9 Hz, 1 H), 9.65-9.78 (m, 1 H).

**Example 3070**

***N*-[*cis*-4-(4-Methyl-quinolin-2-ylamino)-cyclohexyl]-2-phenoxy-nicotinamide hydrochloride**

**Step A: Synthesis of *N*-(*cis*-4-methyl-quinolin-2-yl)-cyclohexane-1,4-diamine.**

A mixture of 2-Chloro-4-methyl-quinoline (10.0 g, 56.3 mmol) and (*cis*-4-amino-cyclohexyl)-carbamic acid *tert-butyl* ester obtained in step B of example 3031 (13.3 g, 62.1 mmol) in IPA (10 mL) was stirred at reflux for 7 days. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane) to give a pale yellow oil. To a solution of the above material in EtOAc (150 mL) was added 4 M hydrogen chloride in EtOAc (150 mL). The mixture was stirred at ambient temperature for 1 hr and concentrated. The residue was dissolved in 1 M aqueous NaOH and the aqueous layer was extracted with $CHCl_3$ (three time). The combined organic layer was dried over $MgSO_4$, filtered, and purified by medium-pressure liquid chromatography (NH-silica gel, 1% to 5% MeOH in $CHCl_3$) to give *N*-(*cis*-4-methyl-quinolin-2-yl)-cyclohexane-1,4-diamine (3.41 g, 24%) as pale yellow solid.
ESI MS m/e 256, M + $H^+$ ; $^1H$ NMR (300 MHz, $CDCl_3$) δ 1.19-1.55 (m, 4 H), 1.67-1.94 (m, 4 H), 2.56 (s, 3 H), 2.85-2.98 (m, 1 H), 4.03-4.15 (m, 1 H), 4.77 (d, *J* = 6.8 Hz, 1 H), 6.49 (s, 1 H), 7.17-7.25 (m, 1 H), 7.47-7.55 (m, 1 H), 7.62-7.68 (m, 1 H), 7.72-7.77 (m, 1 H).

**Step B: Synthesis of *N*-[*cis*-4-(4-methyl-quinolin-2-ylamino)-cyclohexyl]-2-phenoxy-nicotinamide hydrochloride.**

To a solution of *N*-(*cis*-4-methyl-quinolin-2-yl)-cyclohexane-1,4-diamine (300 mg, 1.17 mmol) in $CHCl_3$ (2 mL) were added $Et_3N$ (0.45 mL, 2.60 mmol) and 2-phenoxy-nicotinoyl chloride (411 mg, 1.76 mmol) in $CHCl_3$ (1 mL). The mixture was stirred at ambient temperature for 14 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% EtOAc in hexane) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give *N*-[*cis*-4-(4-methyl-quinolin-2-ylamino)-cyclohexyl]-2-phenoxy-nicotinamide hydrochloride (85 mg, 15%) as a white solid.
ESI MS m/e 453, M (free) + $H^+$; $^1H$ NMR (300 MHz, $CDCl_3$) δ 1.85-2.12 (m, 8 H), 2.70 (s, 3 H), 3.83-4.00 (m, 1 H), 4.11-4.28 (m, 1 H), 6.74 (s, 1 H), 7.08-7.18 (m, 1 H), 7.19-7.34 (m, 3 H), 7.38-7.53 (m, 3 H), 7.63-7.85 (m, 3 H), 7.91-7.99 (m, 1 H), 8.20-8.24 (m, 1 H), 8.54 (d, *J* = 7.4 Hz, 1 H).

**Example 3071**

**3,4-Difluoro-*N*-[*cis*-4-(4-methyl-quinolin-2-ylamino)-cyclohexyl]-benzamide hydrochloride**

**Step A: Synthesis of 3,4-difluoro-*N*-[*cis*-4-(4-methyl-quinolin-2-ylamino)-cyclohexyl]-benzamide hydrochloride.**

To a solution of 3,4-difluoro-benzoic acid (222 mg, 1.40 mmol) and *N*-(*cis*-4-methyl-quinolin-2-yl)-cyclohexane-1,4-diamine obtained in step A of example 3070 (300 mg, 1.17 mmol) in DMF (3 mL) were added $Et_3N$ (0.39 mL, 2.80 mmol), HOBt-$H_2O$ (268 mg, 1.76 mmol), and EDC-HCl (268 g, 1.40 mmol). The reaction mixture was stirred at ambient temperature for 12 hr. To the reaction mixture was added water (20 mL) and the suspension was stirred at ambient temperature for 30 min. The precipitated was collected by filtration, washed with $H_2O$, and purified by medium-pressure liquid chromatography (NH-silica gel, 20% to 50% EtOAc in hexane and silica gel, 2% to 5% MeOH in $CHCl_3$) to give a yellow oil. To a solution of the above material in EtOAc (8 mL) was added 4 M hydrogen chloride in EtOAc (0.5 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give 3,4-difluoro-*N*-[*cis*-4-(4-methyl-quinolin-2-ylamino)-cyclohexyl]-benzamide hydrochloride (377 mg, 75%) as a white solid.
ESI MS m/e 396, M (free) + $H^+$ ; $^1H$ NMR (300 MHz, $CDCl_3$) δ 1.75-2.17 (m, 8 H), 2.73 (s, 3 H), 3.95-4.26 (m, 2 H), 6.71 (d, *J* = 7.1 Hz, 1 H), 6.79 (s, 1 H), 7.14-7.28 (m, 1 H), 7.41-7.51 (m, 1 H), 7.54-7.64 (m, 1 H), 7.66-7.79 (m, 3 H), 7.85 (d, *J* = 8.2 Hz, 1 H), 9.57-9.67 (m, 1 H).

**Example 3072**

**1-(2,3-Dichloro-phenyl)-3-[*cis*-4-(4-methyl-quinolin-2-ylamino)-cyclohexyl]-urea hydrochloride**

**Step A: Synthesis of 1-(2,3-dichloro-phenyl)-3-[*cis*-4-(4-methyl-quinolin-2-ylamino)-cyclohexyl]-urea hydrochloride.**

To a solution of *N*-(*cis*-4-methyl-quinolin-2-yl)-cyclohexane-1,4-diamine obtained in step A of example 3070 (300 mg, 1.17 mmol) in DMSO (3 mL) was added 1,2-dichloro-3-isocyanato-benzene (242 mg, 1.29 mmol). The mixture was stirred at ambient temperature for 5 hr and poured into water (20 mL). The precipitate was collected by filtration, washed with water, and purified by medium-pressure liquid chromatography (NH-silica gel, 20% to 33% EtOAc in hexane) and flash chromatography (silica gel, 2% MeOH in $CHCl_3$) to give a pale yellow oil. To a solution of the above material in EtOAc (10 mL) was added 4 M hydrogen chloride in EtOAc (0.2 mL). The mixture was stirred at ambient temperature for 1 hr and concentrated. A suspension of the residue in $Et_2O$ (20 mL) was stirred at ambient tempareture for 1 hr. The precipitate was collected by filtration, washed with $Et_2O$, and dried at 60 °C under reduced pressure to give 1-(2,3-dichloro-phenyl)-3-[*cis*-4-(4-methyl-quinolin-2-ylamino)-cyclohexyl]-urea hydrochloride (421 mg, 68%) as a white solid.
ESI MS m/e 465, M (free) + $Na^+$ ; $^1H$ NMR (300 MHz, $CDCl_3$) δ 1.76-2.17 (m, 8 H), 2.70 (s, 3 H), 3.69-4.08 (m, 2 H), 6.65-6.83 (m, 2 H), 6.95-7.17 (m, 2 H), 7.41 (t, *J* = 8.1 Hz, 1 H), 7.54-7.89 (m, 4 H), 8.05-8.17 (m, 1 H), 9.13-9.27 (m, 1 H).

**Example 3073**

**3-Chloro-*N*-[*cis*-4-(4-methyl-quinolin-2-ylamino)-cyclohexyl]-benzamide hydrochloride**

**Step A: Synthesis of 3-chloro-*N*-[*cis*-4-(4-methyl-quinolin-2-ylamino)-cyclohexyl]-benzamide hydrochloride.**

To a solution of *N*-(*cis*-4-methyl-quinolin-2-yl)-cyclohexane-1,4-diamine obtained in step A of example 3070 (300 mg, 1.17 mmol) in $CHCl_3$ (3 mL) were added $Et_3N$ (0.35 mL, 2.51 mmol) and 3-chloro-benzoyl chloride (226 mg, 1.29 mmol). The mixture was stirred at ambient temperature for 1.5 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane). To a solution of the above material in EtOAc (2 mL) was added 4 M hydrogen chloride in EtOAc (10 mL). The mixture was stirred at ambient temperature for 1 hr, and concentrated. The residue was suspended in $Et_2O$ (20 mL) and the suspension was stirred at ambient temperature for 2 hr. The precipitate was collected by filtration, washed with $Et_2O$, and dried at 80 °C under reduced pressure to give 3-chloro-*N*-[*cis*-4-(4-methyl-quinolin-2-ylamino)-cyclohexyl]-benzamide hydrochloride (441 mg, 87%) as a white solid.

ESI MS m/e 416, M (free) + $Na^+$; $^1H$ NMR (300 MHz, $CDCl_3$) δ 1.78-2.11 (m, 8 H), 2.72 (s, 3 H), 3.92-4.29 (m, 2 H), 6.78 (s, 1 H), 6.94 (d, *J* = 9.0 Hz, 1 H), 7.33-7.50 (m, 3 H), 7.68-7.76 (m, 3 H), 7.83-7.88 (m, 2 H), 9.58 (d, *J* = 9.0 Hz, 1 H).

**Example 3074**

**5-Nitro-thiophene-3-carboxylic acid [*cis*-4-(4-methyl-quinolin-2-ylamino)-cyclohexyl]-amide hydrochloride**

**Step A: Synthesis of 5-nitro-thiophene-3-carboxylic acid [*cis*-4-(4-methyl-quinolin-2-ylamino)-cyclohexyl]-amide hydrochloride.**

Using the procedure for the step A of example 3071, the title compound was obtained.
ESI MS m/e 411, M (free) + $H^+$ ; $^1H$ NMR (300 MHz, $CDCl_3$) δ 1.78-2.14 (m, 8 H), 2.73 (s, 3 H), 3.97-4.26 (m, 2 H), 6.79 (s, 1 H), 7.41-7.57 (m, 2 H), 7.68-7.76 (m, 2 H), 7.85 (d, *J* = 8.2 Hz, 1 H), 8.26 (d, *J* = 1.4 Hz, 1 H), 8.38 (d, *J* = 1.4 Hz, 1 H), 9.41 (d, *J* = 9.0 Hz, 1 H).

**Example 3075**

**3-Methyl-*N*-[*cis*-4-(4-methyl-quinolin-2-ylamino)-cyclohexyl]-benzamide hydrochloride**

**Step A: Synthesis of 3-methyl-N-[*cis*-4-(4-methyl-quinolin-2-ylamino)-cyclohexyl]-benzamide hydrochloride.**

Using the procedure for the step B of example 3070, the title compound was obtained.
ESI MS m/e 374, M (free) + $H^+$ ; $^1H$ NMR (300 MHz, $CDCl_3$) δ 1.66-2.10 (m, 8 H), 2.41 (s, 3 H), 2.72 (d, *J* = 0.8 Hz, 3 H), 3.94-4.05 (m, 1 H), 4.08-4.25 (m, 1 H), 6.62 (d, *J* = 8.1 Hz, 1 H), 6.78 (s, 1 H), 7.28-7.36 (m, 2 H), 7.42-7.49 (m, 1 H), 7.58-7.66 (m, 2 H), 7.67-7.79 (m, 2 H), 7.84 (d, *J* = 8.1 Hz, 1 H), 9.62 (d, *J* = 8.1 Hz, 1 H).

**Example 3076**

**3-Methoxy-*N*-[*cis*-4-(4-methyl-quinolin-2-ylamino)-cyclohexyl]-benzamide hydrochloride**

**Step A: Synthesis of 3-methoxy-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 390, M (free) + $H^+$ ; $^1H$ NMR (300 MHz, $CDCl_3$) δ 1.66-2.10 (m, 8 H), 2.72 (s, 3 H), 3.87 (s, 3 H), 3.94-4.26 (m, 2 H), 6.69-6.81 (m, 2 H), 6.99-7.07 (m, 1 H), 7.28-7.51 (m, 4 H), 7.66-7.79 (m, 2 H), 7.84 (d, *J* = 7.9 Hz, 1 H), 9.55-9.68 (m, 1 H).

**Example 3077**

**4-Cyano-*N*-[*cis*-4-(4-methyl-quinolin-2-ylamino)-cyclohexyl]-benzamide hydrochloride**

**Step A: Synthesis of 4-cyano-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 385, M (free) + $H^+$ ; $^1H$ NMR (300 MHz, $CDCl_3$) δ 1.79-2.16 (m, 8 H), 2.73 (d, *J* = 0.9 Hz, 3 H), 3.99-4.29 (m, 2 H), 6.79 (s, 1 H), 7.20-7.28 (m, 1 H), 7.42-7.51 (m, 1 H), 7.69-7.76 (m, 4 H), 7.86 (d, *J* = 8.2 Hz, 1 H), 7.95-8.02 (m, 2 H), 9.54 (d, *J* = 8.9 Hz, 1 H).

**Example 3078**

**3,4-Dichloro-*N*-[*cis*-4-(4-methyl-quinolin-2-ylamino)-cyclohexyl]-benzamide hydrochloride**

**Step A: Synthesis of 3,4-dichloro-*N*-[*cis*-4-(4-methyl-quinolin-2-ylamino)-cyclohexyl]-benzamide hydrochloride**

Using the procedure for the step B of example 3070, the title compound was obtained.
ESI MS m/e 428, M (free) + $H^+$ ; $^1H$ NMR (300 MHz, $CDCl_3$) δ 1.80-2.14 (m, 8 H), 2.73 (d, *J* = 0.6 Hz, 3 H), 3.95-4.24 (m, 2 H), 6.75-6.87 (m, 2 H), 7.42-7.52 (m, 2 H), 7.64-7.76 (m, 3 H), 7.85 (d, *J* = 8.2 Hz, 1 H), 7.98 (d, *J* = 1.9 Hz, 1

H), 9.60 (d, *J* = 7.9 Hz, 1 H).

**Example 3079**

**3-Chloro-4-fluoro-*N*-[*cis*-4-(4-methyl-quinolin-2-ylamino)-cyclohexyl]-benzamide hydrochloride**

**Step A: Synthesis of 3-chloro-4-fluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 412, M (free) + $H^+$ ; $^1H$ NMR (300 MHz, $CDCl_3$) δ 1.79-2.14 (m, 8 H), 2.73 (d, *J* = 0.8 Hz, 3 H), 3.96-4.26 (m, 2 H), 6.70-6.82 (m, 2 H), 7.18 (t, *J* = 8.6 Hz, 1 H), 7.42-7.51 (m, 1 H), 7.68-7.78 (m, 3 H), 7.85 (d, *J* = 8.2 Hz, 1 H), 7.96 (dd, *J* = 7.0, 2.2 Hz, 1 H), 9.61 (d, *J* = 8.4 Hz, 1 H).

**Example 3080**

**4-Fluoro-3-methyl-*N*-[*cis*-4-(4-methyl-quinolin-2-ylamino)-cyclohexyl]-benzamide hydrochloride**

**Step A: Synthesis of 4-fluoro-3-methyl-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 414, M (free) + $Na^+$ ; $^1H$ NMR (300 MHz, $CDCl_3$) δ 1.73-2.10 (m, 8 H), 2.33 (d, *J* = 1.9 Hz, 3 H), 2.72 (s, 3 H), 3.95-4.25 (m, 2 H), 6.45-6.54 (m, 1 H), 6.78 (s, 1 H), 7.00-7.08 (m, 1 H), 7.42-7.50 (m, 1 H), 7.60-7.80 (m, 4 H), 7.84 (d, *J* = 8.6 Hz, 1 H), 9.58-9.70 (m, 1 H).

**Example 3081**

**1-Methyl-4-nitro-1*H*-pyrrole-2-carboxylic acid-[*cis*-4-(4-methyl-quinolin-2-ylamino)-cyclohexyl]-amide hydrochloride**

**Step A: Synthesis of 1-methyl-4-nitro-1*H*-pyrrole-2-carboxylic acid-[*cis*-4-(4-methyl-quinolin-2-ylamino)-cyclohexyl]-amide hydrochloride.**

Using the procedure for the step A of example 3071, the title compound was obtained.
ESI MS m/e 408, M (free) + $H^+$ ; $^1H$ NMR (300 MHz, $CDCl_3$) δ 1.77-2.11 (m, 8 H), 2.72 (s, 3 H), 3.94-4.14 (m, 5 H), 6.77 (s, 1 H), 7.09-7.16 (m, 1 H), 7.26-7.29 (m, 1 H), 7.41-7.55 (m, 2 H), 7.67-7.78 (m, 2 H), 7.84 (d, *J* = 8.2 Hz, 1 H), 9.51-9.63 (m, 1 H).

**Example 3082**

**9*H*-Xanthene-9-carboxylic acid-[*cis*-4-(4-methyl-quinolin-2-ylamino)-cyclohexyl]-amide hydrochloride**

**Step A: Synthesis of 9*H*-xanthene-9-carboxylic acid [*cis*-4-(4-methyl-quinolin-2-ylamino)-cyclohexyl]-amide hydrochloride.**

Using the procedure for the step A of example 3071, the title compound was obtained.
ESI MS m/e 486, M (free) + $Na^+$ ; $^1H$ NMR (300 MHz, $CDCl_3$) δ 1.63-1.91 (m, 8 H), 2.68 (s, 3 H), 3.75-3.97 (m, 2 H), 4.88 (s, 1 H), 6.14-6.27 (m, 1 H), 6.69 (brs, 1 H), 7.03-7.18 (m, 4 H), 7.23-7.49 (m, 5 H), 7.62-7.86 (m, 3 H), 9.34-9.47 (m, 1 H).

**Example 3083**

**5-Bromo-furan-2-carboxylic acid-[*cis*-4-(4-methyl-quinolin-2-ylamino)-cyclohexyl]-amide hydrochloride**

**Step A: Synthesis of 5-bromo-furan-2-carboxylic acid [*cis*-4-(4-methyl-quinolin-2-ylamino)-cyclohexyl]-amide hydrochloride.**

Using the procedure for the step A of example 3071, the title compound was obtained.
ESI MS m/e 428, M (free) + $H^+$ ; $^1$H NMR (300 MHz, $CDCl_3$) δ 1.62-2.08 (m, 8 H), 2.72 (s, 3 H), 3.90-4.19 (m, 2 H), 6.42 (d, *J* = 3.6 Hz, 1 H), 6.67-6.80 (m, 2 H), 7.05 (d, *J* = 3.6 Hz, 1 H), 7.41-7.51 (m, 1 H), 7.67-7.81 (m, 2 H), 7.85 (d, *J* = 8.4 Hz, 1 H), 9.59-9.72 (m, 1 H).

**Example 3084**

***N*-[*cis*-4-(4-Methyl-quinolin-2-ylamino)-cyclohexyl]-2-*m*-tolyloxy-acetamide hydrochloride**

**Step A: Synthesis of *N*-[*cis*-4-(4-methyl-quinolin-2-ylamino)-cyclohexyl]-2-*m*-tolyloxy-acetamide hydrochloride.**

Using the procedure for the step A of example 3071, the title compound was obtained.
ESI MS m/e 426, M (free) + $Na^+$ ; $^1$H NMR (300 MHz, $CDCl_3$) δ 1.75-2.07 (m, 8 H), 2.34 (s, 3 H), 2.72 (s, 3 H), 3.86-4.14 (m, 2 H), 4.46 (s, 2 H), 6.70-6.95 (m, 5 H), 7.15-7.24 (m, 1 H), 7.41-7.50 (m, 1 H), 7.67-7.88 (m, 3 H), 9.58-9.69 (m, 1 H).

**Example 3085**

**Benzo[2,1,3]oxadiazole-5-carboxylic acid-[*cis*-4-(4-methyl-quinolin-2-ylamino)-cyclohexyl]-amide hydrochloride**

**Step A: Synthesis of benzo[2,1,3]oxadiazole-5-carboxylic acid [*cis*-4-(4-methyl-quinolin-2-ylamino)-cyclohexyl]-amide hydrochloride.**

Using the procedure for the step A of example 3073, the title compound was obtained.
ESI MS m/e 402, M (free) + $H^+$ ; $^1$H NMR (300 MHz, $CDCl_3$) δ 1.79-2.28 (m, 8 H), 2.73 (s, 3 H), 3.98-4.11 (m, 1 H), 4.12-4.32 (m, 1 H), 6.79 (s, 1 H), 7.37-7.50 (m, 2 H), 7.71 (s, 1 H), 7.72 (s, 1 H), 7.81-7.96 (m, 3 H), 8.40 (s, 1 H), 9.56 (d, *J* = 8.7 Hz, 1 H).

**Example 3086**

**3-Bromo-*N*-[*cis*-4-(4-methyl-quinolin-2-ylamino)-cyclohexyl]-benzamide hydrochloride**

**Step A: Synthesis of 3-bromo-*N*-[*cis*-4-(4-methyl-quinolin-2-ylamino)-cyclohexyl]-benzamide hydrochloride.**

Using the procedure for the step A of example 3073, the title compound was obtained.
ESI MS m/e 438, M (free) + $H^+$ ; $^1$H NMR (300 MHz, $CDCl_3$) δ 1.81-2.13 (m, 8 H), 2.72 (s, 3 H), 3.96-4.06 (m, 1 H), 4.08-4.26 (m, 1 H), 6.75-6.85 (m, 2 H), 7.26-7.34 (m, 1 H), 7.42-7.50 (m, 1 H), 7.57-7.64 (m, 1 H), 7.66-7.79 (m, 3 H), 7.85 (d, *J* = 8.2 Hz, 1 H), 8.01 (s, 1 H), 9.55-9.66 (m, 1 H).

**Example 3087**

**3-Cyano-*N*-[*cis*-4-(4-methyl-quinolin-2-ylamino)-cyclohexyl]-benzamide hydrochloride**

**Step A: Synthesis of 3-cyano-*N*-[*cis*-4-(4-methyl-quinolin-2-ylamino)-cyclohexyl]-benzamide hydrochloride.**

Using the procedure for the step A of example 3073, the title compound was obtained.
ESI MS m/e 385, M (free) + $H^+$ ; $^1$H NMR (300 MHz, $CDCl_3$) δ 1.81-2.18 (m, 8 H), 2.73 (s, 3 H), 3.98-4.29 (m, 2 H), 6.80 (s, 1 H), 7.13-7.22 (m, 1 H), 7.43-7.60 (m, 2 H), 7.68-7.79 (m, 3 H), 7.85 (d, *J* = 8.1 Hz, 1 H), 8.08 (d, *J* = 7.2 Hz, 1 H), 8.22 (s, 1 H), 9.49-9.62 (m, 1 H).

**Example 3088**

***N*-[*cis*-4-(4-Methyl-quinolin-2-ylamino)-cyclohexyl]-3-trifluoromethyl-benzamide hydrochloride**

**Step A: Synthesis of *N*-[*cis*-4-(4-methyl-quinolin-2-ylamino)-cyclohexyl]-3-trifluoromethyl-benzamide hydrochloride.**

Using the procedure for the step A of example 3073, the title compound was obtained.
ESI MS m/e 428, M (free) + $H^+$ ; $^1$H NMR (300 MHz, $CDCl_3$) δ 1.81-2.14 (m, 8 H), 2.73 (s, 3 H), 3.95-4.09 (m, 1 H), 4.12-4.31 (m, 1 H), 6.79 (s, 1 H), 6.85-6.99 (m, 1 H), 7.43-7.50 (m, 1 H), 7.57 (t, *J* = 7.8 Hz, 1 H), 7.64-7.79 (m, 3 H), 7.85 (d, *J* = 8.2 Hz, 1 H), 8.01 (d, *J* = 7.8 Hz, 1 H), 8.15 (s, 1 H), 9.56-9.68 (m, 1 H).

**Example 3089**

***N*-[*cis*-4-(4-Methyl-quinolin-2-ylamino)-cyclohexyl]-2,2-diphenyl-acetamide hydrochloride**

**Step A: Synthesis of *N*-[*cis*-4-(4-methyl-quinolin-2-ylamino)-cyclohexyl]-2,2-diphenyl-acetamide hydrochloride.**

Using the procedure for the step A of example 3073, the title compound was obtained.
ESI MS m/e 472, M (free) + $Na^+$ ; $^1$H NMR (300 MHz, $CDCl_3$) δ 1.56-2.10 (m, 8 H), 2.51-2.87 (m, 3 H), 3.81-4.16 (m, 2 H), 4.94 (s, 1 H), 6.40-6.88 (m, 2 H), 7.17-7.51 (m, 11 H), 7.63-7.89 (m, 3 H), 9.44 (brs, 1 H).

**Example 3090**

**2-(4-Fluoro-phenoxy)-*N*-[*cis*-4-(4-methyl-quinolin-2-ylamino)-cyclohexyl]-nicotinamide hydrochloride**

**Step A: Synthesis of 2-(4-fluoro-phenoxy)-*N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 493, M (free) + $Na^+$ ; $^1$H NMR (300 MHz, $CDCl_3$) δ 1.85-2.12 (m, 8 H), 2.71 (s, 3 H), 3.87-4.00 (m, 1 H), 4.11-4.30 (m, 1 H), 6.76 (brs, 1 H), 7.09-7.21 (m, 3 H), 7.24-7.35 (m, 2 H), 7.44 (t, *J* = 7.1 Hz, 1 H), 7.65-7.99 (m, 4 H), 8.19-8.25 (m, 1 H), 8.54 (d, *J* = 6.2 Hz, 1 H), 9.60-9.73 (m, 1 H).

**Example 3091**

**2-(3,4-Difluoro-phenoxy)-*N*-[*cis*-4-(4-methyl-quinolin-2-ylamino)-cyclohexyl]-nicotinamide hydrochloride**

**Step A: Synthesis of 2-(3,4-difluoro-phenoxy)-*N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 511, M (free) + $Na^+$ ; $^1$H NMR (300 MHz, $CDCl_3$) δ 1.81-2.13 (m, 8 H), 2.71 (s, 3 H), 3.90-4.03 (m, 1 H), 4.13-4.30 (m, 1 H), 6.76 (s, 1 H), 7.10-7.51 (m, 5 H), 7.65-7.88 (m, 4 H), 8.18-8.27 (m, 1 H), 8.50-8.58 (m, 1 H), 9.67-9.81 (m, 1 H).

**Example 3092**

***N*-[*cis*-4-(4-Methyl-quinolin-2-ylamino)-cyclohexyl]-2-*p*-tolyloxy-nicotinamide hydrochloride**

**Step A: Synthesis of *N*-[*cis*-4-(4-methyl-quinolin-2-ylamino)-cyclohexyl]-2-*p*-tolyloxy-nicotinamide hydrochloride.**

Using the procedure for the step A of example 3071, the title compound was obtained.
ESI MS m/e 489, M (free) + $Na^+$ ; $^1$H NMR (300 MHz, $CDCl_3$) δ 1.83-2.15 (m, 8 H), 2.36 (s, 3 H), 2.71 (s, 3 H), 3.78-4.03 (m, 1 H), 4.10-4.32 (m, 1 H), 6.67-6.84 (m, 1 H), 7.06-7.51 (m, 6 H), 7.62-7.90 (m, 3 H), 7.95-8.08 (m, 1 H), 8.19-8.30 (m, 1 H), 8.48-8.61 (m, 1 H), 9.62 (brs, 1 H).

**Example 3093**

**2-(4-Chloro-phenoxy)-*N*-[*cis*-4-(4-methyl-quinolin-2-ylamino)-cyclohexyl]-nicotinamide hydrochloride**

**Step A: Synthesis of 2-(4-chloro-phenoxy)-*N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 487, M (free) + $H^+$ ; $^1H$ NMR (300 MHz, $CDCl_3$) δ 1.58-2.13 (m, 8 H), 2.71 (s, 3 H), 3.87-4.02 (m, 1 H), 4.10-4.31 (m, 1 H), 6.75 (brs, 1 H), 7.15 (dd, *J* = 7.5, 4.8 Hz, 1 H), 7.22-7.33 (m, 2 H), 7.37-7.49 (m, 3 H), 7.64-7.92 (m, 4 H), 8.21 (dd, *J* = 4.8, 2.0 Hz, 1 H), 8.53 (dd, *J* = 7.6, 2.0 Hz, 1 H), 9.63-9.78 (m, 1 H).

**Example 3094**

**2-(4-Bromo-phenoxy)-*N*-[*cis*-4-(4-methyl-quinolin-2-ylamino)-cyclohexyl]-nicotinamide hydrochloride**

**Step A: Synthesis of 2-(4-bromo-phenoxy)-*N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 531, M (free) + $H^+$ ; $^1H$ NMR (300 MHz, $CDCl_3$) δ 1.81-2.20 (m, 8 H), 2.72 (s, 3 H), 3.83-4.31 (m, 2 H), 6.66-6.85 (m, 1 H), 7.03-7.93 (m, 10 H), 8.16-8.28 (m, 1 H), 8.46-8.61 (m, 1 H), 9.55-9.61 (m, 1 H).

**Example 3095**

**2-(4-Methoxy-phenoxy)-*N*-[*cis*-4-(4-methyl-quinolin-2-ylamino)-cyclohexyl]-nicotinamide hydrochloride**

**Step A: Synthesis of 2-(4-methoxy-phenoxy)-*N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 483, M (free) + $H^+$ ; $^1H$ NMR (300 MHz, $CDCl_3$) δ 1.84-2.19 (m, 8 H), 2.71 (s, 3 H), 3.74-4.00 (m, 4 H), 4.12-4.28 (m, 1 H), 6.68-6.82 (m, 1 H), 6.91-7.30 (m, 5 H), 7.38-7.50 (m, 1 H), 7.63-7.88 (m, 3 H), 7.96-8.09 (m, 1 H), 8.17-8.33 (m, 1 H), 8.48-8.61 (m, 1 H), 9.50-9.70 (m, 1 H).

**Example 3096**

**2-(3-Chloro-4-fluoro-phenoxy)-*N*-[*cis*-4-(4-methyl-quinolin-2-ylamino)-cyclohexyl]-nicotinamide hydrochloride**

**Step A: Synthesis of 2-(3-chloro-4-fluoro-phenoxy)-*N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 505, M (free) + $H^+$ ; $^1H$ NMR (300 MHz, $CDCl_3$) δ 1.67-2.13 (m, 8 H), 2.71 (s, 3H), 3.89-4.02 (m, 1 H), 4.13-4.29 (m, 1 H), 6.76 (brs, 1 H), 7.17 (dd, *J* = 7.6, 4.8 Hz, 1 H), 7.22-7.49 (m, 4 H), 7.65-7.87 (m, 4 H), 8.21 (dd, *J* = 4.8, 2.0 Hz, 1 H), 8.52 (dd, *J* = 7.6, 2.0 Hz, 1 H), 9.65-9.77 (m, 1 H).

**Example 3097**

***N*-[*cis*-4-(4-Methyl-quinolin-2-ylamino)-cyclohexyl]-2-*m*-tolyloxy-nicotinamide hydrochloride**

**Step A: Synthesis of *N*-[*cis*-4-(4-Methyl-quinolin-2-ylamino)-cyclohexyl]-2-*m*-tolyloxy-nicotinamide hydrochloride.**

Using the procedure for the step A of example 3071, the title compound was obtained.
ESI MS m/e 467, M (free) + $H^+$ ; $^1H$ NMR (300 MHz, $CDCl_3$) δ 1.85-2.10 (m, 8 H), 2.40 (s, 3 H), 2.70 (s, 3 H), 3.84-3.98 (m, 1 H), 4.10-4.24 (m, 1 H), 6.76 (brs, 1 H), 7.00-7.21 (m, 4 H), 7.28-7.48 (m, 2 H), 7.62-7.87 (m, 3 H), 7.94-8.06 (m,

1 H), 8.21-8.29 (m, 1 H), 8.53 (d, *J* = 6.4 Hz, 1 H), 9.51-9.64 (m, 1 H).

**Example 3098**

**2-(3-Methoxy-phenoxy)-*N*-[*cis*-4-(4-methyl-quinolin-2-ylamino)-cyclohexyl]-acetamide hydrochloride**

**Step A: Synthesis of 2-(3-methoxy-phenoxy)-*N*-[*cis*-4-(4-methyl-quinolin-2-ylamino)-cyclohexyl]-acetamide hydrochloride.**

Using the procedure for the step A of example 3071, the title compound was obtained.
ESI MS m/e 442, M (free) + $Na^+$ ; $^1$H NMR (300 MHz, $CDCl_3$) δ 1.71-2.06 (m, 8 H), 2.72 (s, 3 H), 3.82 (s, 3 H), 3.89-4.11 (m, 2 H), 4.46 (s, 3 H), 6.52-6.61 (m, 3 H), 6.75 (s, 1 H) 6.84-6.92 (m, 1 H), 7.16-7.24 (m, 1 H), 7.41-7.49 (m, 1 H), 7.67-7.80 (m, 1 H), 7.84 (d, *J* = 8.2 Hz, 1 H), 9.57-9.70 (m, 1 H).

**Example 3099**

**2-(3-Chloro-phenoxy)-*N*-[*cis*-4-(4-methyl-quinolin-2-ylamino)-cyclohexyl]-acetamide hydrochloride**

**Step A: Synthesis of 2-(3-chloro-phenoxy)-*N*-[*cis*-4-(4-methyl-quinolin-2-ylamino)-cyclohexyl]-acetamide hydrochloride.**

Using the procedure for the step A of example 3071, the title compound was obtained.
ESI MS m/e 446, M (free) + $Na^+$ ; $^1$H NMR (300 MHz, $CDCl_3$) δ 1.80-2.06 (m, 8 H), 2.72 (s, 3 H), 3.91-4.13 (m, 2 H), 4.45 (s, 2 H), 6.73-7.03 (m, 5 H), 7.19-7.28 (m, 1 H), 7.41-7.50 (m, 1 H), 7.67-7.87 (m, 3 H), 9.58-9.72 (m, 1 H).

**Example 3100**

**2-(3-Chloro-4-fluoro-phenoxy)-*N*-[*cis*-4-(4-methyl-quinolin-2-ylamino)-cyclohexyl]-acetamide hydrochloride**

**Step A: Synthesis of 2-(3-chloro-4-fluoro-phenoxy)-*N*-[*cis*-4-(4-methyl-quinolin-2-ylamino)-cyclohexyl]-acetamide hydrochloride.**

Using the procedure for the step A of example 3071, the title compound was obtained.
ESI MS m/e 464, M (free) + $Na^+$ ; $^1$H NMR (300 MHz, $CDCl_3$) δ 1.70-2.07 (m, 8 H), 2.72 (s, 3 H), 3.91-4.14 (m, 2 H), 4.42 (s, 2 H), 6.76 (s, 1 H), 6.83-6.95 (m, 2 H), 6.99-7.16 (m, 2 H), 7.42-7.50 (m, 1 H), 7.67-7.80 (m, 2 H), 7.84 (d, *J* = 7.9 Hz, 1 H), 9.59-9.70 (m, 1 H).

**Example 3101**

**2-(3,4-Dichloro-phenoxy)-*N*-[*cis*-4-(4-methyl-quinolin-2-ylamino)-cyclohexyl]-acetamide hydrochloride**

**Step A: Synthesis of 2-(3,4-dichloro-phenoxy)-*N*-[*cis*-4-(4-methyl-quinolin-2-ylamino)-cyclohexyl]-acetamide hydrochloride.**

Using the procedure for the step A of example 3071, the title compound was obtained.
ESI MS m/e 480, M (free) + $Na^+$ ; $^1$H NMR (300 MHz, $CDCl_3$) δ 1.78-2.13 (m, 8 H), 2.72 (s, 3 H), 3.91-4.14 (m, 2 H), 4.44 (s, 2 H), 6.76 (brs, 1 H), 6.84-6.93 (m, 2 H), 7.09 (d, *J* = 2.8 Hz, 1 H), 7.37 (d, *J* = 8.9 Hz, 1 H), 7.42-7.49 (m, 1 H), 7.67-7.80 (m, 2 H), 7.84 (d, *J* = 8.1 Hz, 1 H), 9.54-9.72 (m, 1 H).

**Example 3102**

***C*-(Methyl-phenyl-amino)-*N*-[*cis*-4-(4-methyl-quinolin-2-ylamino)-cyclohexyl]-acetamide dihydrochloride**

**Step A: Synthesis of *C*-(methyl-phenyl-amino)-*N*-[*cis*-4-(4-methyl-quinolin-2-ylamino)-cyclohexyl]-acetamide dihydrochloride.**

Using the procedure for the step A of example 3071, the title compound was obtained.
ESI MS m/e 403, M (free) + $H^+$ ; $^1$H NMR (300 MHz, $CDCl_3$) δ 1.67-1.99 (m, 8 H), 2.70 (s, 3 H), 3.11 (s, 3 H), 3.76-4.06

(m, 4 H), 6.63-7.01 (m, 4 H), 7.08-7.50 (m, 4 H), 7.60-7.92 (m, 3 H), 9.34-9.51 (m, 1 H).

**Example 3103**

**2-(3,4-Dichloro-phenylamino)-*N*-[*cis*-4-(4-methyl-quinolin-2-ylamino)-cyclohexyl}-acetamide dihydrochloride**

**Step A: Synthesis of 2-(3,4-dichloro-phenylamino)-*N*-[*cis*-4-(4-methyl-quinolin-2-ylamino)-cyclohexyl]-acetamide dihydrochloride.**

Using the procedure for the step A of example 3071, the title compound was obtained.
ESI MS m/e 479, M (free) + $Na^+$ ; $^1H$ NMR (300 MHz, $CDCl_3$) δ 1.75-2.02 (m, 8 H), 2.71 (s, 3 H), 3.74-4.08 (m, 4 H), 6.45-6.56 (m, 1 H), 6.67-6.78 (m, 2 H), 7.04-7.19 (m, 2 H), 7.39-7.50 (m, 1 H), 7.62-7.87 (m, 3 H), 9.31-9.46 (m, 1 H).

**Example 3104**

**3,4-Difluoro-*N*-[*cis*-4-(4-methyl-quinolin-2-ylamino)-cyclohexylmethyl]-benzamide hydrochloride**

**Step A: Synthesis of (*cis*-4-aminomethyl-cyclohexyl)-(4-methyl-quinolin-2-yl)-amine.**

A mixture of 2-chloro-4-methyl-quinoline (10.0 g, 56.3 mmol) and (*cis*-4-amino-cyclohexylmethyl)-carbamic acid benzyl ester obtained in step C of example 3068 (17.7 g, 67.6 mmol) in butanol (10 mL) was stirred at reflux for 5 days. The reaction mixture was poured into saturated aqueous $NaHCO_3$ and the aqueous layer was extracted with $CHCl_3$ (three times). The combined organic layer was dried over $MgSO_4$, filtrated, concentrated, and purified by medium-pressure liquid chromatography (NH-silica gel, 10% to 20% EtOAc in hexane and silica gel, 2% to 10% MeOH in $CHCl_3$) to give a pale yellow oil. To a solution of the above oil in MeOH (140 mL) was added 10% Pd/C (1.40 g). The mixture was stirred at ambient temperature under hydrogen atmosphere for 6 days. The reaction mixture was filtrated through a pad of celite, concentrated, and purified by medium-pressure liquid chromatography (NH-silica gel, 2% to 10% MeOH in $CHCl_3$) to give (*cis*-4-aminomethyl-cyclohexyl)-(4-methyl-quinolin-2-yl)-amine (5.74 g, 38%) as a pale yellow solid.
ESI MS m/e 470, M + $H^+$;$^1H$ NMR (300 MHz, $CDCl_3$) δ 1.14-1.51 (m, 4 H), 1.60-1.94 (m, 5 H), 2.56 (s, 3 H), 2.60 (d, *J*= 6.4 Hz, 2 H), 4.08-4.22 (m, 1 H), 4.82-4.92 (m, 1 H), 6.52 (s, 1 H), 7.17-7.24 (m, 1 H), 7.47-7.54 (m, 1 H), 7.62-7.67 (m, 1 H), 7.72-7.77 (m, 1 H).

**Step B: Synthesis of 3,4-difluoro-*N*-[*cis*-4-(4-methyl-quinolin-2-ylamino)-cyclohexylmethyl]-benzamide hydrochloride.**

To a solution of (*cis*-4-aminomethyl-cyclohexyl)-(4-methyl-quinolin-2-yl)-amine (300 mg, 0.90 mmol) in $CHCl_3$ (2 mL) were added *i*-$Pr_2NEt$ (0.33 mL, 1.89 mmol) and 3,4-difluoro-benzoyl chloride (175 mg, 0.99 mmol) in $CHCl_3$ (1 mL). The mixture was stirred at ambient temperature for 6 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 25% EtOAc in hexane) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give 3,4-difluoro-*N*-[*cis*-4-(4-methyl-quinolin-2-ylamino)-cyclohexylmethyl]-benzamide hydrochloride (289 mg, 72%) as a white solid.
ESI MS m/e 432, M (free) + $Na^+$ ; $^1H$ NMR (300 MHz, $CDCl_3$) δ 1.56-2.05 (m, 9 H), 2.70 (s, 3 H), 3.49-3.54 (m, 2 H), 3.97-4.09 (m, 1 H), 6.75 (s, 1 H), 6.89-6.98 (m, 1 H), 7.19-7.30 (m, 1 H), 7.40-7.47 (m, 1 H), 7.66-7.75 (m, 2 H), 7.79-7.93 (m, 3 H), 9.72-9.85 (m, 1 H).

**Example 3105**

***N*-[*cis*-4-(4-Methyl-quinolin-2-ylamino)-cyclohexylmethyl)-2-phenoxy-nicotinamide hydrochloride**

**Step A: Synthesis of *N*-[*cis*-4-(4-methyl-quinolin-2-ylamino)-cyclohexylmethyl]-2-phenoxy-nicotinamide hydrochloride.**

Using the procedure for the step C of example 3104, the title compound was obtained.

ESI MS m/e 467, M (free) + $H^+$ ; $^1H$ NMR (300 MHz, $CDCl_3$) δ 1.61-2.14 (m, 9 H), 2.69 (s, 3 H), 3.42-3.50 (m, 2 H), 3.92-4.04 (m, 1 H), 6.73 (brs, 1 H), 7.10-7.32 (m, 4 H), 7.38-7.49 (m, 3 H), 7.64-7.84 (m, 3 H), 8.06-8.15 (m, 1 H), 8.19-8.24 (m, 1 H), 8.57-8.63 (m, 1 H), 9.49-9.62 (m, 1 H).

**Example 3106**

**1-(2,3-Dichloro-phenyl)-3-[*cis*-4-(4-methyl-quinolin-2-ylamino)-cyclohexylmethyl]-urea hydrochloride**

**Step A: Synthesis of 1-(2,3-dichloro-phenyl)-3-[*cis*-4-(4-methyl-quinolin-2-ylamino)-cyclohexylmethyl]-urea hydrochloride.**

To a solution of (*cis*-4-aminomethyl-cyclohexyl)-(4-methyl-quinolin-2-yl)-amine obtained in step B of example 3014 (300 mg, 1.11 mmol) in DMSO (3 mL) was added 1,2-dichloro-3-isocyanato-benzene (230 mg, 1.22 mmol). The mixture was stirred at ambient temperature for 21 hr and poured into water (20 mL). The precipitate was collected by filtration, washed with water, and purified by medium-pressure liquid chromatography (NH-silica gel, 20% EtOAc in hexane) to give a pale yellow oil. To a solution of the above material in EtOAc (10 mL) was added 4 M hydrogen chloride in EtOAc (0.2 mL). The mixture was stirred at ambient temperature for 1 hr and concentrated. A suspension of the residue in $Et_2O$ (20 mL) was stirred at ambient tempareture for 1 hr. The precipitate was collected by filtration, washed with $Et_2O$, and dried at 60 °C under reduced pressure to give 1-(2,3-dichloro-phenyl)-3-[*cis*-4-(4-methyl-quinolin-2-ylamino)-cyclohexylmethyl]-urea hydrochloride (247 mg, 45%) as a white solid.
ESI MS m/e 479, M (free) + $Na^+$ ; $^1H$ NMR (300 MHz, $CDCl_3$) δ 1.51-2.18 (m, 9 H), 2.71 (d, *J* = 0.8 Hz, 3 H), 3.37-3.44 (m, 2 H), 4.04-4.14 (m, 1 H), 6.78 (s, 1 H), 6.89-7.13 (m, 3 H), 7.42-7.50 (m, 1 H), 7.70-7.76 (m, 2 H), 7.84 (d, *J* = 8.1 Hz, 1 H), 8.13-8.22 (m, 2 H), 9.38 (d, *J* = 9.2 Hz, 1 H), 13.95 (brs, 1 H).

**Example 3107**

***N*-[*cis*-4-(4-Dimethylamino-5,6,7,8-tetrahydro-quinazolin-2-ylamino)-cyclohexyl]-2-phenoxy-nicotinamide hydrochloride**

**Step A: Synthesis of 5,6,7,8-tetrahydro-quinazoline-2,4-diol.**

To a solution of 2-oxo-cyclohexanecarboxylic acid ethyl ester (61.5 g, 361 mmol) in EtOH (61.5 mL) was added urea (73.8 g, 1.23 mol). The mixture was stirred at reflux for 10.5 days and stirred at ambient temperature for 30 min. The precipitate was filtrated, washed with acetone, and dried. A suspension of the above solid in $H_2O$(100 mL) stirred on an ice-bath for 1hr. The precipitate was filtrated, washed with hexane, and dried under reduced pressure to give 5,6,7,8-tetrahydro-quinazoline-2,4-diol (21.0 g, 35%) as a pale yellow solid.
CI MS m/e 167, M + $H^+$ ;$^1H$ NMR (300 MHz, DMSO-$d_6$) δ 1.48-1.71 (m, 4 H), 2.09-2.19 (m, 2 H), 2.24-2.34 (m, 2 H), 10.41-10.98 (m, 2 H).

**Step B: Synthesis of (2-chloro-5,6,7,8-tetrahydro-quinazolin-4-yl)-dimethyl-amine.**

A suspension of 5,6,7,8-tetrahydro-quinazoline-2,4-diol (20.9 g, 100 mmol) in $POCl_3$ (105 mL) was stirred at reflux for 2 hr and the reaction mixture was concentrated. The residue was poured into ice water. The aqueous layer was extracted with EtOAc (three times). The combined organic layer was dried over $MgSO_4$, filtrated, and concentrated. To the solution of residue (7.00 g) in THF (70 mL) was added 50% aqueous $Me_2NH$ (7.77 g, 86.2 mmol) and the mixture stirred at ambient temperature for 2 hr. To the reaction was added saturated aqueous $NaHCO_3$ and the aqueous layer was extracted with $CHCl_3$ (three times). The combined organic layer was dried over $MgSO_4$, filtered, concentrated, and purified flash chromatography (silica gel, 20% EtOAc in hexane) to give (2-chloro-5,6,7,8-tetrahydro-quinazolin-4-yl)-dimethyl-amine (6.08 g, 64%) as a white solid.
ESI MS m/e 234, M + $Na^+$; $^1H$ NMR (300 MHz, $CDCl_3$) δ 1.62-1.90 (m, 4 H), 2.59 (t, *J*= 6.0 Hz, 2 H), 2.76 (t, *J*= 6.6 Hz, 2 H), 3.06 (s, 6 H).

**Step C: Synthesis of (*cis*-4-amino-cyclohexyl)-carbamic acid benzyl ester.**

To a solution of (*cis*-4-amino-cyclohexyl)-carbamic acid tert-butyl ester obtained in step B of example 3031 (75.0 g, 350 mmol) in $CHCl_3$ (750 mL) were added $Et_3N$ (53.7 mL, 385 mmol) and benzyl chloroformate (55 mL, 385 mmol). The mixture was stirred at ambient temperature for 20 hr. The reaction was quenched with saturated aqueous $NaHCO_3$ and the aqueous layer was extracted with $CHCl_3$ (three times). The combined organic layer was dried over

$MgSO_4$, filtered, concentrated, purified by flash chromatography (silica gel, 0.4% to 5% MeOH in $CHCl_3$) to give a pale yellow oil. To a solution of the residue in EtOAc (200 mL) was added 4 M hydrogen chloride in EtOAc (200 mL). The mixture was stirred at ambient temperature for 2 hr and concentrated. The residue was dissolved in 1 M aqueous NaOH and the aqueous layer was extracted with $CHCl_3$ (three time). The combined organic layer was dried over $MgSO_4$, filtered, concentrated, and purified flash chromatography (silica gel, 25% to 50% EtOAc in hexane) to give (*cis*-4-amino-cyclohexyl)-carbamic acid benzyl ester (37.6 g, 43%) as a pale brown oil.
ESI MS m/e 249, $M^+ + H^+$ ; $^1H$ NMR (200 MHz, $CDCl_3$) $\delta$ 1.13-1.83 (m, 8 H), 2.77-2.97 (m, 1 H), 3.63-3.83 (m, 1 H), 4.92-5.20 (m, 3 H), 7.25-7.47 (m, 5 H).

**Step D: Synthesis of *N*$^2$-(*cis*-4-amino-cyclohexyl)-*N*$^4$,*N*$^4$-dimethyl-5,6,7,8-tetrahydro-quinazoline-2,4-diamine.**

A mixture of (2-chloro-5,6,7,8-tetrahydro-quinazolin-4-yl)-dimethyl-amine (16.0 g, 75.7 mmol) and (*cis*-4-amino-cyclohexyl)-carbamic acid benzyl ester (18.8 g, 75.7 mmol) in butanol (21 mL) was stirred at reflux for 6 days. The reaction mixture was poured into saturated aqueous $NaHCO_3$, and the aqueous layer was extracted with $CHCl_3$ (three times). The combined organic layer was dried over $MgSO_4$, filtrated, concentrated, and purified by flash chromatography (NH-silica, 33% to 50% EtOAc in hexane) to give a pale yellow oil. To a solution of the above oil in MeOH (270 mL) was added 10% Pd/C (2.70 g). The mixture was stirred at ambient temperature under hydrogen atmosphere for 1.5 days. The reaction mixture was filtrated through a pad of celite, concentrated, and purified by medium-pressure liquid chromatography (NH-silica gel, 1% to 5% MeOH in $CHCl_3$) to give *N*$^2$-(cis-4-amino-cyclohexyl)-*N*$^4$,*N*$^4$-dimethyl-5,6,7,8-tetrahydro-quinazoline-2,4-diamine (15.8 g, 72%) as a pale yellow solid.
FAB MS m/e 290, $M^+ + H^+$; $^1H$ NMR (200 MHz, $CDCl_3$) $\delta$ 1.00-1.90 (m, 14 H), 2.49 (t, *J*= 5.9 Hz, 2 H), 2.61 (t, *J*= 6.6 Hz, 2 H), 2.71-3.00 (m, 7 H), 3.93-4.07 (m, 1 H), 4.67-4.80 (m, 1 H).

**Step E: Synthesis of *N*-[*cis*-4-(4-dimethylamino-5,6,7,8-tetrahydro-quinazolin-2-ylamino)-cyclohexyl]-2-phenoxy-nicotinamide hydrochloride**

To a solution of *N*$^2$-(*cis*-4-amino-cyclohexyl)-*N*$^4$,*N*$^4$-dimethyl-5,6,7,8-tetrahydro-quinazoline-2,4-diamine (300 mg, 1.04 mmol) in $CHCl_3$ (3 mL) were added $Et_3N$ (0.31 mL, 2.22 mmol) and 2-phenoxy-nicotinoyl chloride (266 mg, 1.14 mmol). The mixture was stirred at ambient temperature for 3 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% EtOAc in hexane). To a solution of the above material in EtOAc (2 mL) was added 4 M hydrogen chloride in EtOAc (10 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 80 °C under reduced pressure to give *N*-[*cis*-4-(4-dimethylamino-5,6,7,8-tetrahydro-quinazolin-2-ylamino)-cyclohexyl]-2-phenoxy-nicotinamide hydrochloride (159 mg, 29%) as a white solid.
ESI MS m/e 487, M (free) + $H^+$ ; $^1H$ NMR (300 MHz, $CDCl_3$) $\delta$ 1.61-1.98 (m, 12 H), 2.54 (t, *J*= 5.9 Hz, 2 H), 2.74 (t, *J*= 6.5 Hz, 2 H), 3.20 (s, 6 H), 4.02-4.20 (m, 2 H), 7.12 (dd, *J*= 7.5, 4.8 Hz, 1 H), 7.21-7.30 (m, 3 H), 7.42-7.50 (m, 2 H), 7.87-7.93 (m, 1 H), 8.21 (dd, *J*= 4.8, 2.2 Hz, 1 H), 8.25-8.32 (m, 1 H), 8.52 (dd, *J* = 7.6, 2.0 Hz, 1 H), 13.18 (s, 1 H).

**Example 3108**

**3-Chloro-*N*-[*cis*-4-(4-dimethylamino-5,6,7,8-tetrahydro-quinazolin-2-ylamino)-cyclohexyl]-4-fluoro-benzamide hydrochloride**

**Step A: Synthesis of 3-chloro-*N*-[*cis*-4-(4-dimethylamino-5,6,7,8-tetrahydro-quinazolin-2-ylamino)-cyclohexyl]-4-fluoro-benzamide hydrochloride.**

Using the procedure for the step E of example 3107, the title compound was obtained.
ESI MS m/e 468, M (free) + $Na^+$ ; $^1H$ NMR (300 MHz, $CDCl_3$) $\delta$ 1.61-2.00 (m, 12 H), 2.51-2.61 (m, 2 H), 2.68-2.81 (m, 2 H), 3.23 (s, 6 H), 4.02-4.26 (m, 2 H), 6.73-6.90 (m, 1 H), 7.13-7.23 (m, 1 H), 7.65-7.82 (m, 1 H), 7.96 (d, *J* = 6.8 Hz, 1 H), 8.22-8.44 (m, 1 H), 12.63-12.89 (m, 1 H).

**Example 3109**

***N*-[*cis*-4-(4-Dimethylamino-5,6,7,8-tetrahydro-quinazolin-2-ylamino)-cyclohexyl]-4-fluoro-3-methyl-benzamide hydrochloride**

**Step A: Synthesis of *N*-[*cis*-4-(4-dimethylamino-5,6,7,8-tetrahydro-quinazolin-2-ylamino)-cyclohexyl]-4-fluoro-3-methyl-benzamide hydrochloride.**

Using the procedure for the step E of example 3107, the title compound was obtained.
ESI MS m/e 448, M (free) + $Na^+$; $^1H$ NMR (300 MHz, $CDCl_3$) δ 1.60-2.04 (m, 12 H), 2.27-2.36 (m, 3 H), 2.50-2.61 (m, 2 H), 2.65-2.84 (m, 2 H), 3.23 (s, 6 H), 4.03-4.27 (m, 2 H), 6.42-6.58 (m, 1 H), 6.96-7.11 (m, 1 H), 7.56-7.75 (m, 2 H), 8.25-8.47 (m, 1 H).

**Example 3110**

***N*-[*cis*-4-(4-Dimethylamino-5,6,7,8-tetrahydro-quinazolin-2-ylamino)-cyclohexyl]-3,5-dimethoxy-benzamide hydrochloride**

**Step A: Synthesis of *N*-[*cis*-4-(4-dimethylamino-5,6,7,8-tetrahydro-quinazolin-2-ylamino)-cyclohexyl]-3,5-dimethoxy-benzamide hydrochloride.**

Using the procedure for the step E of example 3107, the title compound was obtained.
ESI MS m/e 476, M (free) + $Na^+$ ; $^1H$ NMR (300 MHz, $CDCl_3$) δ 1.63-2.04 (m, 12 H), 2.51-2.62 (m, 2 H), 2.66-2.86 (m, 2 H), 3.23 (s, 6 H), 3.85 (s, 6 H), 4.04-4.27 (m, 2 H), 6.50-6.70 (m, 2 H), 6.95 (brs, 2 H), 8.19-8.47 (m, 1 H).

**Example 3111**

**Benzo[2,1,3]oxadiazole-5-carboxylic acid-[*cis*-4-(4-dimethylamino-5,6,7,8-tetrahydro-quinazolin-2-ylamino)-cyclohexyl]-amide hydrochloride**

**Step A: Synthesis of benzo[2,1,3]oxadiazole-5-carboxylic acid-[*cis*-4-(4-dimethylamino-5,6,7,8-tetrahydro-quinazolin-2-ylamino)-cyclohexyl]-amide hydrochloride.**

Using the procedure for the step E of example 3107, the title compound was obtained.
ESI MS m/e 458, M (free) + $Na^+$ ; $^1H$ NMR (300 MHz, $CDCl_3$) δ 1.62-2.01 (m, 12 H), 2.56 (t, *J* = 5.8 Hz, 2 H), 2.71 (t, *J* = 6.5 Hz, 2 H), 3.23 (s, 6 H), 4.04-4.27 (m, 2 H), 7.71 (d, *J* = 8.2 Hz, 1 H), 7.85 (dd, *J* = 9.5, 1.1 Hz, 1 H), 7.91-7.96 (m, 1 H), 8.27 (d, *J* = 8.1 Hz, 1 H), 8.42 (t, *J* = 1.2 Hz, 1 H).

**Example 3112**

***N*-[*cis*-4-(4-Dimethylamino-5,6,7,8-tetrahydro-quinazolin-2-ylamino)-cyclohexyl]-3-nitro-benzamide hydrochloride**

**Step A: Synthesis of *N*-[*cis*-4-(4-dimethylamino-5,6,7,8-tetrahydro-quinazolin-2-ylamino)-cyclohexyl]-3-nitro-benzamide hydrochloride.**

Using the procedure for the step E of example 3107, the title compound was obtained.
ESI MS m/e 461, M (free) + $Na^+$ ; $^1H$ NMR (300 MHz, $CDCl_3$) δ 1.65-2.04 (m, 12 H), 2.50-2.85 (m, 4 H), 3.24 (s, 6 H), 4.11-4.29 (m, 2 H), 7.04-7.20 (m, 1 H), 7.56-7.68 (m, 1 H), 8.13-8.38 (m, 3 H), 8.72-8.79 (m, 1 H).

**Example 3113**

***N*-[*cis*-4-(4-Dimethylamino-5,6,7,8-tetrahydro-quinazolin-2-ylamino)-cyclohexylmethyl]-2-phenoxy-nicotinamide hydrochloride**

**Step A: Synthesis of *N*$^2$-(*cis*-4-aminomethyl-cyclohexyl)-*N*$^4$,*N*$^4$-dimethyl-5,6,7,8-tetrahydro-quinazoline-2,4-diamine.**

A mixture of (*cis*-4-amino-cyclohexylmethyl)-carbamic acid benzyl ester obtained in step C of example 3068 (3.10 g, 11.8 mmol) and (2-chloro-5,6,7,8-tetrahydro-quinazolin-4-yl)-dimethyl-amine obtained in step B of example 3107 (2.00 g, 9.44 mmol) in butanol (3 mL) was stirred at reflux for 19 hr. The reaction mixture was poured into saturated aqueous $NaHCO_3$, and the aqueous layer was extracted with $CHCl_3$ (three times). The combined organic layer was dried over $MgSO_4$, filtrated, concentrated, and purified by flash chromatography (NH-silica gel, 33% to 50% EtOAc in hexane) to give a pale yellow oil. To a solution of the above oil (2.48 g) in MeOH (25 mL) was added 10% Pd/C (248 mg). The mixture was stirred at 50 °C under hydrogen atmosphere for 8 hr. The reaction mixture was filtrated through a pad of celite, concentrated, and purified by medium-pressure liquid chromatography (NH-silica gel, 1% to 5% MeOH in $CHCl_3$) to give *N*$^2$-(*cis*-4-aminomethyl-cyclohexyl)-*N*$^4$,*N*$^4$-dimethyl-5,6,7,8-tetrahydro-quinazoline-2,4-diamine (1.70 g, 59%) as a pale yellow solid.
FAB MS m/e 304, M (free) + $H^+$

**Step B: Synthesis of *N*-[*cis*-4-(4-dimethylamino-5,6,7,8-tetrahydro-quinazolin-2-ylamino)-cyclohexylmethyl]-2-phenoxy-nicotinamide hydrochloride.**

Using the procedure for the step A of example 3071, the title compound was obtained.
ESI MS m/e 501, M (free) + $H^+$

**Example 3114**

***N*-[*cis*-4-(4-Dimethylamino-quinolin-2-ylamino)-cyclohexyl]-2-phenoxy-nicotinamide hydrochloride**

**Step A: Synthesis of 2,4-dichloro-quinoline.**

A suspension of quinoline-2,4-diol (150 g, 931 mmol) in $POCl_3$ (975 mL, 10.4 mol) was stirred at reflux for 6 hr and the reaction mixture was concentrated. The residue was diluted with $CHCl_3$ (500 mL) and the solution was poured into ice water. The aqueous layer was extracted with $CHCl_3$ (three times). The combined organic layer was dried over $MgSO_4$, filtrated, concentrated, and purified by flash chromatography (silica gel, 20% EtOAc in hexane) to give 2,4-dichloro-quinoline (177 g, 96%) as a pale brown solid.
EI MS m/e 197, $M^+$ ; $^1$H NMR (300 MHz, $CDCl_3$) $\delta$ 7.50 (s, 1 H), 7.65 (ddd, *J* = 8.3, 7.0, 1.3 Hz, 1 H), 7.79 (ddd, *J* = 8.5, 7.0, 1.3 Hz, 1 H), 8.00-8.06 (m, 1 H), 8.16-8.21 (m, 1 H).

**Step B: Synthesis of (2-chloro-quinolin-4-yl)-dimethyl-amine.**

To a solution of 2,4-dichloro-quinoline (177 g, 894 mmol) in THF (2.1 L) was added 50% aqueous $Me_2NH$ (234 mL, 2.23 mol). The mixture was stirred at ambient temperature for 68 hr. To the mixture was added 50% aqueous $Me_2NH$ (47 mL, 448 mmol) and stirred at ambient temperature for 3 hr. The solution was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 1% to 3% EtOAc in hexane) to give (2-chloro-quinolin-4-yl)-dimethyl-amine (75.9 g, 41%) as a pale yellow oil and (4-chloro-quinolin-2-yl)-dimethyl-amine (28.0 g, 15%) as a pale yellow oil. (2-chloro-quinolin-4-yl)-dimethyl-amine;
ESI MS m/e 207, M + $H^+$ ; $^1$H NMR (300 MHz, $CDCl_3$) $\delta$ 3.06 (s, 6 H), 6.71 (s, 1 H), 7.45 (ddd, *J* = 8.4,7.0,1.2 Hz, 1 H), 7.63 (ddd, *J* = 8.4, 6.9, 1.5 Hz, 1 H), 7.91-7.93 (m, 1 H), 7.97-8.03 (m, 1 H).
(4-chloro-quinolin-2-yl)-dimethyl-amine;
ESI MS m/e 207, M + $H^+$ ; $^1$H NMR (300 MHz, $CDCl_3$) $\delta$ 3.18 (s, 6 H), 6.97 (brs, 1 H), 7.18-7.31 (m, 1 H), 7.49-7.63 (m, 1 H), 7.66-7.72 (m, 1 H), 7.95-8.00 (m, 1 H).

**Step C: Synthesis of *N*$^2$-(*cis*-4-amino-cyclohexyl)-*N*$^4$,*N*$^4$-dimethyl-quinoline-2,4-diamine.**

A mixture of (2-chloro-quinolin-4-yl)-dimethyl-amine (15.6 g, 75.7 mmol) and (*cis*-4-amino-cyclohexyl)-carbamic acid benzyl ester obtained in step C of example 3107 (18.8 g, 75.7 mmol) in butanol (20 mL) was stirred at reflux for 6 days. The reaction mixture was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 33% to 50% EtOAc in hexane) to give a pale yellow oil. To a solution of the above oil in MeOH (170 mL) was added 10% Pd/C (1.70 g). The mixture was stirred at ambient temperature under hydrogen atmosphere for 1.5 days. The reaction mixture was filtrated through a pad of celite, concentrated, and purified by medium-pressure liquid chromatography (NH-silica gel, 1% to 5% MeOH in $CHCl_3$) to give *N*$^2$-(*cis*-4-amino-cyclohexyl) -*N*$^4$,*N*$^4$-dimethyl-quinoline-2,4-diamine (11.7 g, 55%) as a pale yellow solid.
FAB MS m/e 285, M + $H^+$ ; ;$^1$H NMR (300 MHz, $CDCl_3$) δ 1.19-1.96 (m, 10 H), 2.81-3.03 (m, 7 H), 4.02-4.17 (m, 1 H), 4.66-4.83 (m, 1 H), 6.03 (s, 1 H), 7.06-7.21 (m, 1 H), 7.39-7.52 (m, 1 H), 7.55-7.67 (m, 1 H), 7.80-7.90 (m, 1 H).

**Step D: Synthesis of *N*-[*cis*-4-(4-dimethylamino-quinolin-2-ylamino)-cyclohexyl]-2-phenoxy-nicotinamide hydrochloride.**

To a solution of *N*$^2$-(*cis*-4-amino-cyclohexyl)-*N*$^4$,*N*$^4$-dimethyl-quinoline-2,4-diamine (300 mg, 1.05 mmol) in $CHCl_3$ (3 mL) were added $Et_3N$ (0.31 mL, 2.22 mmol) and 2-phenoxy-nicotinoyl chloride (271 mg, 1.16 mmol). The mixture was stirred at ambient temperature for 3 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% EtOAc in hexane). To a solution of the above material in EtOAc (2 mL) was added 4 M hydrogen chloride in EtOAc (10 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 80 °C under reduced pressure to give *N*-[*cis*-4-(4-dimethylamino-quinolin-2-ylamino)-cyclohexyl]-2-phenoxy-nicotinamide hydrochloride (160 mg, 29%) as a white solid.
ESI MS m/e 482, M (free) + $H^+$ ; $^1$H NMR (300 MHz, $CDCl_3$) δ 1.57-2.15 (m, 8 H), 3.21 (s, 6 H), 3.73-3.88 (m, 1 H), 4.06-4.27 (m, 1 H), 5.79 (s, 1 H), 7.12 (dd, *J* = 7.6, 4.8 Hz, 1 H), 7.19-7.33 (m, 4 H), 7.41-7.71 (m, 4 H), 7.81-7.97 (m, 2 H), 8.21 (dd, *J* = 4.8, 2.0 Hz, 1 H), 8.52 (dd, *J* = 7.6, 2.0 Hz, 1 H), 8.94-9.08 (m, 1 H), 13.81 (brs, 1 H).

**Example 3115**

***N*-[*cis*-4-(4-Chloro-quinolin-2-ylamino)-cyclohexyl]-2-phenoxy-nicotinamide hydrochloride**

**Step A: Synthesis of *N*-[*cis*-4-(4-chloro-quinolin-2-ylamino)-cyclohexyl]-2-phenoxy-nicotinamide hydrochloride.**

A mixture of 2,4-dichloro-quinoline obtained in step A of example 3114 (1.5 g, 7.57 mmol) and *N*-(*cis*-4-amino-cyclohexyl)-2-phenoxy-nicotinamide obtained in step A of example 2 (2.3 g, 7.57 mmol) in butanol (2 mL) was stirred at 130 °C for 3 days in a sealed tube. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% EtOAc in hexane) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give *N*-[*cis*-4-(4-chloro-quinolin-2-ylamino)-cyclohexyl]-2-phenoxy-nicotinamide hydrochloride (295 mg, 8%) as a white solid and *N*-[*cis*-4-(2-chloro-quinolin-4-ylamino)-cyclohexyl]-2-phenoxy-nicotinamide hydrochloride (283 mg, 7%) as a white solid.
*N*-[*cis*-4-(4-chloro-quinolin-2-ylamino)-cyclohexyl]-2-phenoxy-nicotinamide hydrochloride;
ESI MS m/e 495, M (free) + $Na^+$ ; $^1$H NMR (300 MHz, $CDCl_3$) δ 1.86-2.10 (m, 8 H), 3.82-3.96 (m, 1 H), 4.13-4.28 (m, 1 H), 7.04 (s, 1 H), 7.10-7.34 (m, 4 H), 7.41-7.55 (m, 3 H), 7.71-7.84 (m, 2 H), 7.92-8.11 (m, 2 H), 8.20-8.26 (m, 1 H), 8.50-8.59 (m, 1 H), 9.83 (brs, 1 H).
*N*-[*cis*-4-(2-chloro-quinolin-4-ylamino)-cyclohexyl]-2-phenoxy-nicotinamide hydrochloride;
ESI MS m/e 495, M (free) + $Na^+$ ; $^1$H NMR (300 MHz, DMSO-$d_6$) δ 1.72-2.37 (m, 8 H), 3.64-3.84 (m, 1 H), 4.36 (brs, 1 H), 6.33 (brs, 1 H), 7.05-7.60 (m, 8 H), 8.06-8.66 (m, 6 H).

**Example 3116**

**3,4-Difluoro-*N*-[*cis*-4-(4-methoxy-quinolin-2-ylamino)-cyclohexyl]-benzamide hydrochloride**

**Step A: Synthesis of 2-chloro-quinolin-4-ol.**

A mixture of 2,4-dichloro-quinoline obtained in step A of example 3114 (3.00 g, 15.1 mmol) and MeOH (485 mg, 15.1 mmol) in butanol (3 mL) was stirred at reflux for 3 hr. The reaction mixture was suspended in $CHCl_3$ (15 mL) and stirred at ambient temperature for 30 min. The precipitate was collected by filtration, washed with $CHCl_3$, and dried at 50 °C under reduced pressure to give 2-chloro-quinolin-4-ol (1.47 g, 54%) as a pale yellow solid.
ESI MS m/e 179, $M^+$ ; $^1H$ NMR (300 MHz, DMSO-$d_6$) δ 6.83 (s, 1 H), 7.27-7.43 (m, 2 H), 7.60-7.67 (m, 1 H), 7.86 (d, *J* = 7.9 Hz, 1 H), 12.05 (brs, 1 H).

**Step B: Synthesis of 2-chloro-4-methoxy-quinoline.**

To a solution of 2-chloro-quinolin-4-ol (500 mg, 2.78 mmol) in DMF (5 mL) were added $K_2CO_3$ (462 mg, 3.37 mmol) and MeI (210 μL, 3.37 mmol). The mixture was stirred at 50°C for 3 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 10% EtOAc in hexane) to give 2-chloro-4-methoxy-quinoline (440 mg, 82%) as a white solid.
ESI MS m/e 194, M + $H^+$ ; $^1H$ NMR (300 MHz, $CDCl_3$) δ 3.71 (s, 3 H), 6.89 (s, 1 H), 7.27-7.43 (m, 2 H), 7.60-7.69 (m, 1 H), 8.01 (d, *J* = 8.1 Hz, 1 H).

**Step C: Synthesis of 3,4-difluoro-*N*-[*cis*-4-(4-methoxy-quinolin-2-ylamino)-cyclohexyl]-benzamide hydrochloride.**

A mixture of 2-chloro-4-methoxy-quinoline (250 mg, 1.29 mmol) and *N*-(*cis*-4-amino-cyclohexyl)-3,4-difluoro-benzamide obtained in step D of example 3031 (361 mg, 1.42 mmol) in butanol (1 mL) was stirred at 130 °C for 5 days in a sealed tube. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane). To a solution of the above material in EtOAc (2 mL) was added 4 M hydrogen chloride in EtOAc (10 mL). The mixture was stirred at ambient temperature for 1 hr and concentrated. The residue was suspended in $Et_2O$ (20 mL) and the suspension was stirred at ambient temperature for 2 hr. The precipitate was filtered, washed with $Et_2O$, and dried at 80 °C under reduced pressure to give *cis*-3,4-difluoro-*N*-[4-(4-methoxy-quinolin-2-ylamino)-cyclohexyl]-benzamide hydrochloride (79 mg, 14%) as a white solid.
ESI MS m/e 434, M (free) + $Na^+$ ; $^1H$ NMR (300 MHz, DMSO-$d_6$) δ 1.58-2.09 (m, 8 H), 3.55-3.72 (m, 4 H), 3.88-4.06 (m, 1 H), 5.93 (s, 1 H), 7.03-8.09 (m, 7 H), 8.25-8.45 (m, 2 H).

**Example 3117**

***N*-[*cis*-4-(4-Chloro-quinolin-2-ylamino)-cyclohexyl]-3,4-difluoro-benzamide hydrochloride**

**Step A: Synthesis of *N*-[*cis*-4-(4-chloro-quinolin-2-ylamino)-cyclohexyl]-3,4-difluoro-benzamide hydrochloride.**

Using the procedure for the step A of example 3115, the title compound was obtained.
*N*-[*cis*-4-(4-chloro-quinolin-2-ylamino)-cyclohexyl]-3,4-difluoro-benzamide hydrochloride; ESI MS m/e 416, M (free) + $H^+$ ; $^1H$ NMR (300 MHz, $CDCl_3$) δ 1.82-2.22 (m, 8 H), 3.93-4.28 (m, 2 H), 6.65-6.77 (m, 1 H), 7.08 (s, 1 H), 7.14-7.29 (m, 1 H), 7.48-7.64 (m, 2 H), 7.68-7.88 (m, 3 H), 8.09 (d, *J* = 8.1 Hz, 1 H), 9.82-9.90 (m, 1 H).
*N*-[*cis*-4-(2-chloro-quinolin-4-ylamino)-cyclohexyl]-3,4-difluoro-benzamide hydrochloride; ESI MS m/e 438, M (free) + $Na^+$; $^1H$ NMR (300 MHz, $CDCl_3$) δ 1.72-2.37 (m, 8 H), 3.76-3.95 (m, 1 H), 4.49-4.65 (m, 1 H), 6.37 (brs, 1 H), 6.94-7.12 (m, 1 H), 7.18-7.33 (m, 1 H), 7.39-7.55 (m, 1 H), 7.60-7.76 (m, 1 H), 7.85-7.95 (m, 1 H), 8.06-8.20 (m, 2 H), 8.46-8.58 (m, 1 H), 8.70-8.87 (m, 1 H).

**Example 3118**

***N*-[*cis*-4-(4-Dimethylamino-quinolin-2-ylamino)-cyclohexylmethyl]-2-phenoxy-nicotinamide hydrochloride**

**Step A: Synthesis of [*cis*-4-(4-dimethylamino-quinolin-2-ylamino)-cyclohexylmethyl]-carbamic acid benzyl ester.**

A mixture of (2-chloro-quinolin-4-yl)-dimethyl-amine obtained in step B of example 3114 (23.6 g, 114 mmol) and (*cis*-4-amino-cyclohexylmethyl)-carbamic acid benzyl ester obtained in step C of example 3068 (36.0 g, 137 mmol) in butanol (31 mL) was stirred at reflux for 14 days. The reaction mixture was poured into saturated aqueous $NaHCO_3$, and the aqueous layer was extracted with $CHCl_3$ (three times). The combined organic layer was dried over $MgSO_4$, filtrated, concentrated, and purified by flash chromatography (NH-silica, 14% to 66% EtOAc in hexane) to give [*cis*-4-(4-dimethylamino-quinolin-2-ylamino)-cyclohexylmethyl]-carbamic acid benzyl ester (19.3 g, 39%) as a pale yellow solid.
ESI MS m/e 433, M (free) + $H^+$ ; $^1H$ NMR (200 MHz, $CDCl_3$) δ 1.12-1.97 (m, 9 H), 2.94 (s, 6 H), 3.13 (t, $J$ = 6.4 Hz, 2 H), 4.06-4.26 (m, 1 H), 4.62-4.94 (m, 2 H), 5.11 (s, 2 H), 6.04 (s, 1 H), 7.14 (ddd, $J$ = 8.4, 7.0, 1.3 Hz, 1 H), 7.29-7.40 (m, 5 H), 7.45 (ddd, $J$ = 8.4, 6.8, 1.5 Hz, 1 H), 7.57-7.64 (m, 1 H), 7.84 (dd, $J$ = 8.4, 1.3 Hz, 1 H).

**Step B: Synthesis of *N*$^2$-(*cis*-4-aminomethyl-cyclohexyl)-*N*$^4$,*N*$^4$-dimethyl-quinoline-2,4-diamine.**

To a solution of [*cis*-4-(4-dimethylamino-quinolin-2-ylamino)-cyclohexylmethyl]-carbamic acid benzyl ester (19.3 g, 44.6 mmol) in MeOH (200 mL) was added 5% Pd/C (1.93 g). The mixture was stirred at ambient temperature under hydrogen atmosphere for 6 days. The reaction mixture was filtrated through a pad of celite and concentrated. To a solution of the residue in methanol (200 mL) was 10% Pd/C (1.93 g). The mixture was stirred at ambient temperature under hydrogen atmosphere for 1 day. The reaction mixture was filtrated through a pad of celite, concentrated, and purified by flash chromatography (silica gel, 5% to 14% 7 M $NH_3$/MeOH in $CHCl_3$) to give *N*$^2$-(*cis*-4-aminomethyl-cyclohexyl)-*N*$^4$,*N*$^4$-dimethyl-quinoline-2,4-diamine (12.7 g, 95%) as a pale yellow solid.
FAB MS m/e 299, $M^+$ + $H^+$; $^1H$ NMR (200 MHz, $CDCl_3$) δ 1.08-1.99 (m, 11 H), 2.60 (d, $J$ = 6.2 Hz, 2 H), 2.94 (s, 6 H), 4.04-4.22 (m, 1 H), 4.77-4.93 (m, 1 H), 6.06 (s, 1 H), 7.14 (ddd, $J$ = 8.4, 7.0, 1.3 Hz, 1 H), 7.45 (ddd, $J$ = 8.4, 6.8, 1.5 Hz, 1 H), 7.61 (s, 1 H), 7.84 (dd, $J$ = 8.4, 1.3 Hz, 1 H).

**Step C: Synthesis of *N*-[*cis*-4-(4-dimethylamino-quinolin-2-ylamino)-cyclohexylmethyl]-2-phenoxy-nicotinamide hydrochloride.**

To a solution of 2-Phenoxy-nicotinic acid (190 mg, 1.20 mmol) and *N*$^2$-(*cis*-4-aminomethyl-cyclohexyl)-*N*$^4$,*N*$^4$-dimethyl-quinoline-2,4-diamine (300 mg, 1.00 mmol) in DMF (3 mL) were added $Et_3N$ (0.33 mL, 2.40 mmol), HOBt-$H_2O$ (230 mg, 1.50 mmol), and EDC-HCl (230 g, 1.20 mmol). The reaction mixture was stirred at ambient temperature for 20 hr. To the reaction mixture was added water (20 mL) and the suspension was stirred at ambient temperature for 30 min. The precipitated was collected by filtration, washed with $H_2O$, and purified by medium-pressure liquid chromatography (NH-silica gel, 20% EtOAc in hexane) to give a pale yellow oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$ and dried at 60 °C under reduced pressure to give *N*-[*cis*-4-(4-dimethylamino-quinolin-2-ylamino)-cyclohexylmethyl]-2-phenoxy-nicotinamide hydrochloride (164 mg, 31%) as a white solid.
ESI MS m/e 496, M (free) + $H^+$

**Example 3119**

***N*-[*cis*-4-(4-Dimethylamino-5-methyl-pyrimidin-2-ylamino)-cyclohexyl]-3,4-difluoro-benzamide hydrochloride**

**Step A: Synthesis of 2-chloro-4-dimethylamino-5-methylpyrimidine.**

To the solution of 2,4-dichloro-5-methylpyrimidine (20.0 g, 123 mmol) in THF (200 mL) was added 50% aqueous $Me_2NH$ (13.3 g, 143 mol) and the mixture was stirred at ambient temperature for 5 days. To the reaction was added saturated aqueous $NaHCO_3$ and the aqueous layer was extracted with $CHCl_3$ (three times). The combined organic layer was dried over $MgSO_4$, filtered, concentrated, and purified flash chromatography (NH-silica gel, 2% EtOAc in hexane) to give 2-chloro-4-dimethylamino-5-methylpyrimidine (19.9 g, 94 %) as a white solid and 4-chloro-2-dimethylamino-5-methylpyrimidine (1.53 g, 7%) as a white solid.
2-chloro-4-dimethylamino-5-methylpyrimidine;
ESI MS m/e 172, M + $H^+$ ; $^1H$ NMR (300 MHz, $CDCl_3$) δ 2.27 (s, 3 H), 3.15 (s, 6 H), 7.82 (s, 1 H).
4-chloro-2-dimethylamino-5-methylpyrimidine;
ESI MS m/e 194, M + $Na^+$ ; $^1H$ NMR (300 MHz, $CDCl_3$) δ 2.14 (s, 3 H), 3.15 (s, 6 H), 8.06 (s, 1 H).

**Step B: Synthesis of [*cis*-4-(4-dimethylamino-5-methyl-pyrimidin-2-ylamino)-cyclohexyl]-carbamic acid *tert*-butyl ester.**

A mixture of 2-chloro-4-dimethylamino-5-methylpyrimidine (7.00 g, 40.8 mmol) and (*cis*-4-amino-cyclohexyl)-carbamic acid *tert*-butyl ester obtained in step B of example 3031 (9.61 g, 44.8 mmol) in butanol (7 mL) was stirred at 130 °C for 26 hr. The reaction mixture was poured into saturated aqueous $NaHCO_3$ and the aqueous layer was extracted with $CHCl_3$ (three times). The combined organic layer was dried over $MgSO_4$, filtered, concentrated, and purified by flash chromatography (NH-silica gel, 3% to 50% EtOAc in hexane) to give [*cis*-4-(4-dimethylamino-5-methyl-pyrimidin-2-ylamino)cyclohexyl]-carbamic acid *tert*-butyl ester (5.90 g, 42%) as a colorless oil.
ESI MS m/e 350, M + $H^+$ ; $^1H$ NMR (300 MHz, $CDCl_3$) δ 1.40-1.84 (m, 17 H), 2.14 (d, *J* = 0.8 Hz, 3 H), 3.02 (s, 6 H), 3.53-3.71 (m, 1 H), 3.85-3.99 (m, 1 H), 4.51-4.64 (m, 1 H), 4.68-4.78 (m, 1 H), 7.66 (s, 1 H).

**Step C: Synthesis of $N^2$-(*cis*-4-amino-cyclohexyl)-5,$N^4$,$N^4$-trimethyl-pyrimidine-2,4-diamine.**

A solution of [*cis*-4-(4-dimethylamino-5-methyl-pyrimidin-2-ylamino)cyclohexyl]-carbamic acid *tert*-butyl ester (5.71 g, 16.3 mmol) in EtOAc (60 mL) was cooled on an ice-bath and 4 M hydrogen chloride in EtOAc (120 mL) was added. The mixture was stirred at ambient temperature for 1.5 hr and concentrated. The residue was dissolved in 1 M aqueous NaOH and the aqueous layer was extracted with $CHCl_3$ (three time). The combined organic layer was dried over $MgSO_4$, filtered, concentrated, and dried under reduced pressure to give $N^2$-(*cis*-4-amino-cyclohexyl)-5,$N^4$,$N^4$-trimethyl-pyrimidine-2,4-diamine (3.99 g, 98%) as a pale yellow oil.
ESI MS m/e 250, M + $H^+$; $^1H$ NMR (300 MHz, $CDCl_3$) δ 1.39-1.91 (m, 8 H), 2.12 (s, 3 H), 2.79-2.97 (m, 1 H), 3.00 (s, 6 H), 3.86-4.05 (m, 1 H), 4.71-4.92 (m, 1 H), 7.66 (s, 1 H).

**Step D: Synthesis of *N*-[*cis*-4-(4-dimethylamino-5-methyl-pyrimidin-2-ylamino)-cyclohexyl]-3,4-difluoro-benzamide hydrochloride.**

To a solution of $N^2$-(*cis*-4-amino-cyclohexyl)-5,$N^4$,$N^4$-trimethyl-pyrimidine-2,4-diamine (200 mg, 0.80 mmol) in $CHCl_3$ (4 mL) were added $Et_3N$ (0.25 mL, 1.79 mmol) and 1,3-difluoro-benzoyl chloride (156 mg, 0.88 mmol). The mixture was stirred at ambient temperature for 22 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane and silica gel, 3% MeOH in $CHCl_3$). To a solution of the above material in EtOAc (2 mL) was added 4 M hydrogen chloride in EtOAc (10 mL). The mixture was stirred at ambient temperature for 1 hr, and concentrated. The residue was suspended in $Et_2O$ (20 mL) and the suspension was stirred at ambient temperature for 2 hr. The precipitate was collected by filtration, washed with $Et_2O$, and dried at 80 °C under reduced pressure to give *N*-[*cis*-4-(4-dimethylamino-5-methyl-pyrimidin-2-ylamino)-cyclohexyl]-3,4-difluoro-benzamide hydrochloride (56 mg, 16%) as a white solid.
ESI MS m/e 412, M (free) + $Na^+$ ; $^1H$ NMR (300 MHz, $CDCl_3$) δ 1.64-1.99 (m, 8 H), 2.26 (s, 3 H), 3.30 (s, 6 H), 4.02-4.25 (m, 2 H), 6.65-6.74 (m, 1 H), 7.13-7.26 (m, 2 H), 7.53-7.62 (m, 1 H), 7.67-7.79 (m, 1 H), 8.55-8.65 (m, 1 H).

**Example 3120**

***N*-[*cis*-4-(4-Dimethylamino-5-methyl-pyrimidin-2-ylamino)-cyclohexyl]-2-phenoxy-nicotinamide hydrochloride**

**Step A: Synthesis of *N*-[*cis*-4-(4-dimethylamino-5-methyl-pyrimidin-2-ylamino)-cyclohexyl]-2-phenoxy-nicotinamide hydrochloride.**

Using the procedure for the step D of example 3119, the title compound was obtained.
ESI MS m/e 447, M (free) + $H^+$ ; $^1H$ NMR (300 MHz, $CDCl_3$) δ 1.64-1.97 (m, 8 H), 2.23 (s, 3 H), 3.28 (s, 6 H), 4.01-4.21 (m, 2 H), 7.13 (dd, *J* = 7.6, 4.8 Hz, 1 H), 7.19-7.32 (m, 4 H), 7.42-7.52 (m, 2 H), 7.86-7.95 (m, 1 H), 8.21 (dd, *J* = 4.8, 2.0 Hz, 1 H), 8.39-8.48 (m, 1 H), 8.53 (dd, *J* = 7.6, 2.0 Hz, 1 H).

**Example 3121**

***N*-[*cis*-4-(4-Dimethylamino-5-methyl-pyrimidin-2-ylamino)-cyclohexyl]-3-methyl-benzamide hydrochloride**

**Step A: Synthesis of *N*-[*cis*-4-(4-dimethylamino-5-methyl-pyrimidin-2-ylamino)-cyclohexyl]-3-methyl-benzamide hydrochloride.**

Using the procedure for the step D of example 3119, the title compound was obtained.
ESI MS m/e 390, M (free) + $Na^+$ ; $^1H$ NMR (300 MHz, $CDCl_3$) δ 1.67-2.01 (m, 8 H), 2.25 (s, 3 H), 2.41 (s, 3 H), 3.30 (s, 6 H), 4.04-4.22 (m, 2 H), 6.41-6.52 (m, 1 H), 7.19-7.34 (m, 3 H), 7.56-7.66 (m, 2 H), 8.53-8.63 (m, 1 H), 13.04 (s, 1 H).

**Example 3122**

***N*-[*cis*-4-(4-Dimethylamino-5-methyl-pyrimidin-2-ylamino)-cyclohexyl]-3-methoxy-benzamide hydrochloride**

**Step A: Synthesis of *N*-[*cis*-4-(4-dimethylamino-5-methyl-pyrimidin-2-ylamino)-cyclohexyl]-3-methoxy-benzamide hydrochloride.**

Using the procedure for the step D of example 3119, the title compound was obtained.
ESI MS m/e 406, M (free) + $Na^+$ ; $^1H$ NMR (300 MHz, $CDCl_3$) δ 1.66-1.99 (m, 8 H), 2.25 (s, 3 H), 3.30 (s, 6 H), 3.86 (s, 3 H), 4.06-4.23 (m, 2 H), 6.72-6.81 (m, 1 H), 6.98-7.05 (m, 1 H), 7.20-7.43 (m, 4 H), 8.47-8.57 (m, 1 H).

**Example 3123**

***N*-[*cis*-4-(4-Dimethylamino-5-methyl-pyrimidin-2-ylamino)-cyclohexyl]-2-(4-fluoro-phenoxy)-nicotinamide hydrochloride**

**Step A: Synthesis of *N*-[*cis*-4-(4-dimethylamino-5-methyl-pyrimidin-2-ylamino)-cyclohexyl]-2-(4-fluoro-phenoxy)-nicotinamide hydrochloride.**

To a solution of 4-fluoro-phenol (317 mg, 2.83 mmol) in DMA (4 mL) was added 60% NaH in oil (226 mg, 5.56 mmol). The mixture was stirred at ambient temperature for 1 hr. To the mixture was added 2-chloro-*N*-[*cis*-4-(dimethyl-amino-methyl-pyrimidin-2-ylamino)-cyclohexyl]-nicotinamide (1.10 g, 2.83 mmol) in DMA (3 mL). The mixture was stirred at 120 °C for 2 hr and the reaction was quenched with water (60 mL). The aqueous layer was extracted with $CHCl_3$ (three times). The combined organic layer was dried over $MgSO_4$, filtered, concentrated, and purified by medium-pressure liquid chromatography (NH-silica gel, 33% to 50% EtOAc in hexane) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give *N*-[*cis*-4-(4-dimethylamino-5-methyl-pyrimidin-2-ylamino)-cyclohexyl]-2-(4-fluoro-phenoxy)-nicotinamide hydrochloride (154 mg, 11%) as a white solid.
ESI MS m/e 487, M (free) + $Na^+$; $^1H$ NMR (200 MHz, $CDCl_3$) δ 1.61-2.02 (m, 8 H), 2.24 (s, 3 H), 3.28 (s, 6 H), 4.03-4.25 (m, 2 H), 7.06-7.33 (m, 6 H), 7.79-7.91 (m, 1 H), 8.16-8.23 (m, 1 H), 8.46-8.59 (m, 2 H).

**Example 3124**

**2-(2-Bromo-phenoxy)-*N*-[*cis*-4-(4-dimethylamino-5-methyl-pyrimidin-2-ylamino)-cyclohexyl]-nicotinamide hydrochloride**

**Step A: Synthesis of 2-(2-bromo-phenoxy)-*N*-[*cis*-4-(4-dimethylamino-5-methyl-pyrimidin-2-ylamino)-cyclohexyl]-nicotinamide hydrochloride.**

Using the procedure for the step A of example 3123, the title compound was obtained.
ESI MS m/e 547, M (free) + $Na^+$ ; $^1H$ NMR (200 MHz, $CDCl_3$) δ 1.72-2.02 (m, 8 H), 2.23 (s, 3 H), 3.28 (s, 6 H), 3.97-4.27 (m, 2 H), 7.09-7.48 (m, 5 H), 7.66 (dd, *J* = 7.9, 1.3 Hz, 1 H), 7.84-7.95 (m, 1 H), 8.13-8.19 (m, 1 H), 8.31-8.43 (m, 1 H), 8.53 (dd, *J* = 7.4, 2.2 Hz, 1 H), 13.32 (s, 1 H).

**Example 3125**

**1-(2,3-Dichloro-phenyl)-3-[*cis*-4-(4-dimethylamino-5-methyl-pyrimidin-2-ylamino)-cyclohexylmethyl]-urea hydrochloride**

**Step A: Synthesis of *N*²-(*cis*-4-aminomethyl-cyclohexyl)-5,*N*⁴,*N*⁴-trimethyl-pyrimidine-2,4-diamine.**

A mixture of 2-chloro-4-dimethylamino-5-methylpyrimidine obtained in step A of example 3119 (3.00 g, 17.4 mmol) and (*cis*-4-amino-cyclohexylmethyl)-carbamic acid benzyl ester obtained in step C of example 3068 (5.48 g, 20.9 mmol) in butanol (3 mL) was stirred at reflux for 70 hr. The reaction mixture was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 33% to 50% EtOAc in hexane) to give a pale yellow oil. To a solution of the above oil in MeOH (30 mL) was added 10% Pd/C (600 mg). The mixture was stirred at ambient temperature under hydrogen atmosphere for 1.5 days. The reaction mixture was filtrated through a pad of celite, concentrated, and purified by medium-pressure liquid chromatography (NH-silica gel, 2% MeOH in $CHCl_3$) to give $N^2$-(*cis*-4-aminomethyl-cyclohexyl)-5,$N^4$,$N^4$-trimethyl-pyrimidine-2,4-diamine (1.03 g, 22%) as a pale yellow solid.

ESI MS m/e 264, M (free) + $H^+$ ; $^1H$ NMR (300 MHz, $CDCl_3$) δ 1.15-1.89 (m, 11 H), 2.13 (s, 3 H), 2.59 (d, *J* = 6.4 Hz, 2 H), 3.02 (s, 6 H), 4.03-4.13 (m, 1 H), 4.77-4.85 (m, 1 H), 7.67 (s, 1 H).

**Step B: Synthesis of 1-(2,3-dichloro-phenyl)-3-[*cis*-4-(4-dimethylamino-5-methyl-pyrimidin-2-ylamino)-cyclohexylmethyl)-urea hydrochloride.**

To a solution of $N^2$-(*cis*-4-aminomethyl-cyclohexyl)-5,$N^4$,$N^4$-trimethyl-pyrimidine-2,4-diamine (300 mg, 1.14 mmol) in DMSO (3 mL) was added 1,2-dichloro-3-isocyanato-benzene (236 mg, 1.25 mmol). The mixture was stirred at ambient temperature for 16 hr and poured into water (20 mL). The precipitate was collected by filtration, washed with water, and purified by medium-pressure liquid chromatography (NH-silica gel, 20% EtOAc in hexane and silica gel, 3% MeOH in $CHCl_3$) to give a pale yellow oil. To a solution of the above material in EtOAc (10 mL) was added 4 M hydrogen chloride in EtOAc (0.2 mL). The mixture was stirred at ambient temperature for 1 hr and concentrated. A suspension of the residue in $Et_2O$ (20 mL) was stirred at ambient tempareture for 1 hr. The precipitate was collected by filtration, washed with $Et_2O$, and dried at 60 °C under reduced pressure to give 1-(2,3-dichloro-phenyl)-3-[*cis*-4-(4-dimethylamino-5-methyl-pyrimidin-2-ylamino)-cyclohexylmethyl]-urea hydrochloride (326 mg, 59%) as a white solid.

ESI MS m/e 473, M (free) + $Na^+$ ; $^1H$ NMR (200 MHz, $CDCl_3$) δ 1.45-1.99 (m, 9 H), 2.24 (s, 3 H), 3.30 (s, 6 H), 3.32-3.43 (m, 2 H), 4.22-4.38 (m, 2 H), 6.85-7.15 (m, 3 H), 7.22 (brs, 1 H), 8.14-8.26 (m, 2 H), 8.49-8.62 (m, 1 H), 12.14 (s, 1 H).

**Example 3126**

***N*-[*cis*-4-(4-Dimethylamino-6-methyl-pyrimidin-2-ylamino)-cyclohexyl]-2-phenoxy-nicotinamide hydrochloride**

**Step A: Synthesis of (2-chloro-6-methyl-pyrimidin-4-yl)-dimethyl-amine.**

To the solution of 2,4-dichloro-6-methylpyrimidine (20.0 g, 123 mmol) in THF (200 mL) was added 50% aqueous $Me_2NH$ (13.3 g, 147 mmol) and the mixture was stirred at ambient temperature for 24 hr. To the reaction was added saturated aqueous $NaHCO_3$ and the aqueous layer was extracted with $CHCl_3$ (three times). The combined organic layer was dried over $MgSO_4$, filtered, concentrated, and purified flash chromatography (NH-silica gel, 5% to 16% EtOAc in hexane) to give (2-chloro-6-methyl-pyrimidin-4-yl)-dimethyl-amine (14.4 g, 68 %) as a pale yellow solid and (4-chloro-6-methyl-pyrimidin-2-yl)-dimethyl-amine (6.57 g, 31%) as a pale yellow solid. (2-chloro-6-methyl-pyrimidin-4-yl)-dimethyl-amine;

ESI MS m/e 194, $M^+$ + $Na^+$;$^1H$ NMR (300 MHz, $CDCl_3$) δ 2.34 (s, 3 H), 3.10 (s, 6 H), 6.16 (s, 1 H).

(4-chloro-6-methyl-pyrimidin-2-yl)-dimethyl-amine;

CI MS m/e 172, M + $H^+$ ; $^1H$ NMR (300 MHz, $CDCl_3$) δ 2.29 (s, 3 H), 3.16 (s, 6 H), 6.34 (s, 1 H).

**Step B: Synthesis of *N*-[*cis*-4-(4-dimethylamino-6-methyl-pyrimidin-2-ylamino)-cyclohexyl]-2-phenoxy-nicotinamide hydrochloride.**

A mixture of (2-chloro-6-methyl-pyrimidin-4-yl)-dimethyl-amine (300 mg, 1.75 mmol) and *N*-(*cis*-4-amino-cyclohexyl)-2-phenoxy-nicotinamide obtained in step A of example 3032 (598 mg, 1.92 mmol) in butanol (1 mL) was

stirred at 130 °C for 40 hr in a sealed tube. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane). To a solution of the above material in EtOAc (2 mL) was added 4 M hydrogen chloride in EtOAc (10 mL). The mixture was stirred at ambient temperature for 1 hr and concentrated. The residue was suspended in $Et_2O$ (20 mL) and the suspension was stirred at ambient temperature for 2 hr. The precipitate was filtered, washed with $Et_2O$, and dried at 60 °C under reduced pressure to give *N*-[*cis*-4-(4-dimethylamino-6-methyl-pyrimidin-2-ylamino)-cyclohexyl]-2-phenoxy-nicotinamide hydrochloride (549 mg, 65%) as a white solid.
ESI MS m/e 447, M (free) + $H^+$ ; $^1H$ NMR (300 MHz, $CDCl_3$) δ 1.67-2.05 (m, 8 H), 2.34 (s, 3 H), 3.12 (s, 3 H), 3.23 (s, 3 H), 4.03-4.22 (m, 2 H), 5.71 (s, 1 H), 7.13 (dd, *J* = 7.5, 4.8 Hz, 1 H), 7.21-7.32 (m, 3 H), 7.41-7.51 (m, 2 H), 7.84-7.95 (m, 1 H), 8.21 (dd, *J* = 4.7, 2.1 Hz, 1 H), 8.45-8.57 (m, 2 H), 13.43 (brs, 1 H).

**Example 3127**

***N*-[*cis*-4-(4-dimethylamino-6-methyl-pyrimidin-2-ylamino)-cyclohexyl]-3,4-difluoro-benzamide hydrochloride**

**Step A: Synthesis of $N^2$-(*cis*-4-amino-cyclohexyl)-6,$N^4$,$N^4$-trimethyl-pyrimidine-2,4-diamine.**

A mixture of (2-chloro-6-methyl-pyrimidin-4-yl)-dimethyl-amine (6.00 g, 35.0 mmol) and (*cis*-4-amino-cyclohexyl)-carbamic acid tert-butyl ester obtained in step B of example 3031 (8.30 g, 38.5 mmol) in butanol (6 mL) was stirred at 130 °C for 48 hr. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 16% to 50% EtOAc in hexane). To a solution of the above material in EtOAc (60 mL) was added 4 M hydrogen chloride in EtOAc (60 mL). The mixture was stirred at ambient temperature for 2 hr and concentrated. The residue was dissolved in 1 M aqueous NaOH and the aqueous layer was extracted with $CHCl_3$ (three time). The combined organic layer was dried over $MgSO_4$, filtered, concentrated, and purified by medium-pressure liquid chromatography (NH-silica gel, 2% to 10% MeOH in $CHCl_3$) to give $N^2$-(cis-4-amino-cyclohexyl)-6,$N^4$,$N^4$-trimethyl-pyrimidine-2,4-diamine (2.29 g, 26%) as a pale yellow oil.
ESI MS m/e 250, M + $H^+$ ;$^1H$ NMR (300 MHz, $CDCl_3$) δ 1.18-1.50 (m, 4 H), 1.58-1.93 (m, 6 H), 2.19 (s, 3 H), 2.76-2.87 (m, 1 H), 3.03 (s, 6 H), 3.96-4.06 (m, 1 H), 4.78-4.89 (m, 1 H), 5.67 (s, 1 H).

**Step B: Synthesis of *N*-[*cis*-4-(4-dimethylamino-6-methyl-pyrimidin-2-ylamino)-cyclohexyl]-3,4-difluoro-benzamide hydrochloride**

To a solution of $N^2$-(*cis*-4-amino-cyclohexyl)-6,$N^4$,$N^4$-trimethyl-pyrimidine-2,4-diamine (300 mg, 1.20 mmol) in $CHCl_3$ (2 mL) were added *i*-$Pr_2NEt$ (0.44 mL, 2.52 mmol) and 3,4-difluoro-benzoyl chloride (233 mg, 1.32 mmol) in $CHCl_3$ (1 mL). The mixture was stirred at ambient temperature for 15 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% EtOAc in hexane) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give *N*-[*cis*-4-(4-dimethylamino-6-methyl-pyrimidin-2-ylamino)-cyclohexyl]-3,4-difluoro-benzamide hydrochloride (359 mg, 70%) as a white solid.
ESI MS m/e 390, M (free) + $H^+$ ; $^1H$ NMR (300 MHz, $CDCl_3$) δ 1.64-2.00 (m, 8 H), 2.35 (d, *J* = 0.6 Hz, 3 H), 3.14 (s, 3 H), 3.26 (s, 3 H), 4.03-4.29 (m, 2 H), 5.74 (d, *J* = 0.7 Hz, 1 H), 6.61-6.72 (m, 1 H), 7.14-7.26 (m, 1 H), 7.53-7.62 (m, 1 H), 7.67-7.78 (m, 1 H), 8.59 (d, *J* = 7.8 Hz, 1 H).

**Example 3128**

**3-Chloro-*N*-[*cis*-4-(4-dimethylamino-6-methyl-pyrimidin-2-ylamino)-cyclohexyl]-benzamide hydrochloride**

**Step A: Synthesis of 3-chloro-*N*-[*cis*-4-(4-dimethylamino-6-methyl-pyrimidin-2-ylamino)-cyclohexyl]-benzamide hydrochloride.**

Using the procedure for the step B of example 3127, the title compound was obtained.
ESI MS m/e 410, M (free) + $Na^+$ ; $^1H$ NMR (300 MHz, $CDCl_3$) δ 1.67-2.00 (m, 8 H), 2.35 (s, 3 H), 3.13 (s, 3 H), 3.25 (s, 3 H), 4.04-4.26 (m, 2 H), 5.75 (s, 1 H), 6.53 (d, *J* = 8.6 Hz, 1 H), 7.32-7.48 (m, 2 H), 7.64-7.70 (m, 1 H), 7.83 (t, *J*

= 1.9 Hz, 1 H), 8.60 (d, *J* = 7.9 Hz, 1 H), 13.11 (brs, 1 H).

**Example 3129**

***N*-[*cis*-4-(4-Dimethylamino-pyrimidin-2-ylamino)-cyclohexyl]-2-phenoxy-nicotinamide hydrochloride**

**Step A: Synthesis of (2-chloro-pyrimidin-4-yl)-dimethyl-amine.**

To a solution of 2,4-dichloro-pyrimidine (15.0 g, 10.15 mmol) in THF (150 mL) was added 50% aqueous $MeNH_2$ (22.7 g, 25.2 mmol). The mixture was stirred at ambient temperature for 2 hr. The solution was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 20% EtOAc in hexane) to give (2-chloro-pyrimidin-4-yl)-dimethyl-amine (8.66 g, 55%) as a white solid and (4-chloro-pyrimidin-2-yl)-dimethyl-amine (0.87 g, 6%) as a white solid.
(2-chloro-pyrimidin-4-yl)-dimethyl-amine;
CI MS m/e 158, M + $H^+$ ; $^1$H NMR (300 MHz, $CDCl_3$) δ 3.12 (s, 6 H), 6.32 (d, *J* = 6.1 Hz, 1 H), 8.00 (d, *J* = 6.1 Hz, 1 H).
(4-chloro-pyrimidin-2-yl)-dimethyl-amine;
ESI MS m/e 157, $M^+$ ; $^1$H NMR (300 MHz, $CDCl_3$) δ 3.21 (s, 6 H), 6.50 (d, *J* = 5.1 Hz, 1 H), 8.18 (d, *J* = 5.1 Hz, 1 H).

**Step B: Synthesis of [*cis*-4-(4-dimethylamino-pyrimidin-2-ylamino)-cyclohexyl]-carbamic acid *tert*-butyl ester.**

A mixture of (2-chloro-pyrimidin-4-yl)-dimethyl-amine (1.50 g, 9.52 mmol) and (*cis*-4-amino-cyclohexyl)-carbamic acid *tert*-butyl ester obtained in step B of example 3031 (2.24 g, 10.5 mmol) in IPA (1.5 mL) was stirred at 130 °C for 22 hr in a sealed tube. The reaction mixture was poured into saturated aqueous $NaHCO_3$, and the aqueous layer was extracted with $CHCl_3$ (three times). The combined organic layer was dried over $MgSO_4$, filtrated, concentrated, and purified by medium-pressure liquid chromatography (NH-silica, 10% EtOAc in hexane) to give [*cis*-4-(4-dimethylamino-pyrimidin-2-ylamino)-cyclohexyl]-carbamic acid *tert*-butyl ester (1.34 g, 42%) as a white solid.
ESI MS m/e 358, M + $Na^+$ ; $^1$H NMR (300 MHz, $CDCl_3$) δ 1.45 (s, 9 H), 1.48 (s, 8 H), 3.03 (s, 6 H), 3.61 (brs, 1 H), 3.89-4.04 (m, 1 H), 4.47-4.63 (m, 1 H),4.77-4.89 (m, 1 H), 5.80 (d, *J* = 6.1 Hz, 1 H), 7.84 (d, *J* = 6.1 Hz, 1 H).

**Step C: Synthesis of *N*²-(cis-4-amino-cyclohexyl)-*N*⁴,*N*⁴-dimethyl-pyrimidine-2,4-diamine.**

To a solution of [*cis*-4-(4-dimethylamino-pyrimidin-2-ylamino)-cyclohexyl]-carbamic acid *tert*-butyl ester (1.26 g, 3.76 mmol) in EtOAc (15 mL) was added 4 M hydrogen chloride in EtOAc (15 mL). The reaction mixture was stirred at ambient temperature for 1 hr and concentrated. The residue was alkalized with 1 M aqueous NaOH. The aqueous layer was extracted with $CHCl_3$ (six times). The combined organic layer was dried over $MgSO_4$, filtrated, and concentrated to give $N^2$-(*cis*-4-amino-cyclohexyl)-$N^4$,$N^4$-dimethyl-pyrinlidine-2,4-diamine (923 mg, quant.) as a pale yellow oil.
ESI MS m/e 250, M + $H^+$ ; $^1$H NMR (300 MHz, $CDCl_3$) δ 1.29-1.51 (m, 2 H), 1.61-1.91 (m, 6 H), 2.80-2.92 (m, 1 H), 3.03 (s, 6 H), 3.96-4.04 (m, 1 H), 4.85-4.98 (m, 1 H), 5.79 (d, *J* = 6.1 Hz, 1 H), 7.84 (d, *J* = 6.1 Hz, 1 H).

**Step D: Synthesis of *N*-[*cis*-4-(4-dimethylamino-pyrimidin-2-ylamino)-cyclohexyl]-2-phenoxy-nicotinamide hydrochloride.**

To a solution of $N^2$-(*cis*-4-amino-cyclohexyl)-$N^4$,$N^4$-dimethyl-pyrimidine-2,4-diamine (300 mg, 1.20 mmol) in $CHCl_3$ (3 mL) were added $Et_3N$ (0.35 mL, 2.51 mmol) and 2-phenoxy-nicotinoyl chloride (309 mg, 1-32 mmol). The mixture was stirred at ambient temperature for 22 hr. The reaction was quenched with saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane and silica gel, 3% MeOH in $CHCl_3$). To a solution of the above material in EtOAc (2 mL) was added 4 M hydrogen chloride in EtOAc (10 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 80 °C under reduced pressure to give *N*-[*cis*-4-(4-dimethylamino-pyrimidin-2-ylamino)-cyclohexyl]-2-phenoxy-nicotinamide hydrochloride (150 mg, 26%) as a white solid.
ESI MS m/e 433, M (free) + $H^+$

**Example 3130**

**3,4-Difluoro-*N*-[*cis*-4-(4-trifluoromethyl-pyrimidin-2-yl)amino-cyclohexyl]-benzamide hydrochloride**

**Step A: Synthesis of 3,4-difluoro-*N*-[*cis*-4-(4-trifluoromethyl-pyrimidin-2-yl)amino-cyclohexyl]-benzamide hydrochloride.**

A mixture of 2-chloro-4-trifluoromethyl-pyrimidine (200 mg, 1.09 mmol) and *N*-(*cis*-4-amino-cyclohexyl)-3,4-difluoro-benzamide obtained in step D of example 3031 (306 mg, 1.20 mmol) in butanol (1 mL) was stirred at 130 °C for 12 hr in a sealed tube. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane). To a solution of the above material in EtOAc (2 mL) was added 4 M hydrogen chloride in EtOAc (10 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 80 °C under reduced pressure to give 3,4-difluoro-*N*-[*cis*-4-(4-trifluoromethyl-pyrimidin-2-yl)amino-cyclohexyl]-benzamide hydrochloride (123 mg, 26%) as a white solid.
ESI MS m/e 423, $M^+$ (free) + $Na^+$

**Example 3131**

**3,4-Difluoro-*N*-[*cis*-4-(4-methoxy-pyrimidin-2-ylamino)-cyclohexyl]-benzamide hydrochloride**

**Step A: Synthesis of 3,4-difluoro-*N*-[*cis*-4-(4-methoxy-pyrimidin-2-ylamino)-cyclohexyl]-benzamide hydrochloride.**

Using the procedure for the step A of example 3130, the title compound was obtained.
ESI MS m/e 385, M (free) + $Na^+$

**Example 3132**

***N*-[*cis*-4-(4,6-Dimethoxy-pyrimidin-2-ylamino)-cyclohexyl]-3,4-difluoro-benzamide hydrochloride**

**Step A: Synthesis of *N*-[*cis*-4-(4,6-dimethoxy-pyrimidin-2-ylamino)-cyclohexyl]-3,4-difluoro-benzamide hydrochloride.**

Using the procedure for the step A of example 3130, the title compound was obtained.
ESI MS m/e 415, M (free) + $Na^+$

**Example 3133**

**2-Phenoxy-*N*-[*cis*-4-(4-trifluoromethyl-pyrimidin-2-yl)-amino-cyclohexyl]-nicotinamide hydrochloride**

**Step A: Synthesis of 2-phenoxy-*N*-[*cis*-4-(4-trifluoromethyl-pyrimidin-2-yl)-amino-cyclohexyl]-nicotinamide hydrochloride.**

A mixture of 2-chloro-4-trifluoromethyl-pyrimidine (200 mg, 1.10 mmol) and *N*-(*cis*-4-amino-cyclohexyl)-2-phenoxy-nicotinamide obtained in step A of example 3032 (375 mg, 1.20 mmol) in butanol (1 mL) was stirred at 130 °C for 3 days in a sealed tube. The reaction mixture was poured into saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 33% EtOAc in hexane) to give a pale yellow oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 60 °C under reduced pressure to give 2-phenoxy-*N*-[*cis*-4-(4-trifluoromethyl-pyrimidin-2-yl)-amino-cyclohexyl]-nicotinamide hydrochloride (111 mg, 21 %) as a white solid.
ESI MS m/e 480, M (free) + $Na^+$

**Example 3134**

***N*-[*cis*-4-(4-Methoxy-pyrimidin-2-ylamino)-cyclohexyl]-2-phenoxy-nicotinamide hydrochloride**

**Step A: Synthesis of *N*-[*cis*-4-(4-methoxy-pyrimidin-2-ylamino)-cyclohexyl]-2-phenoxy-nicotinamide hydrochloride.**

Using the procedure for the step A of example 3133, the title compound was obtained.
ESI MS m/e 442, M (free) + $Na^+$

**Example 3135**

***N*-[*cis*-4-(4,6-Dimethoxy-pyrimidin-2-ylamino)-cyclohexyl]-2-phenoxy-nicotinamide hydrochloride**

**Step A: Synthesis of *N*-[*cis*-4-(4,6-dimethoxy-pyrimidin-2-ylamino)-cyclohexyl]-2-phenoxy-nicotinamide hydrochloride.**

Using the procedure for the step A of example 3133, the title compound was obtained.
ESI MS m/e 472, M (free) + $Na^+$

**Example 3136**

***N*-[*cis*-4-(4-Dimethylamino-5-phenyl-pyrimidin-2-ylamino)-cyclohexyl]-2-phenoxy-nicotinamide hydrochloride**

**Step A: Synthesis of (5-bromo-2-chloro-pyrimidin-4-yl)-dimethyl-amine.**

Using the procedure for the step A of example 3129, the title compound was obtained.
ESI MS m/e 236, M + $H^+$

**Step B: Synthesis of (2-chloro-5-phenyl-pyrimidin-4-yl)-dimethyl-amine.**

To a solution of (5-bromo-2-chloro-pyrimidin-4-yl)-dimethyl-amine (2.00 g, 8.46 mmol) in toluene (30 mL) were added 2 M aqueous $K_2CO_3$ (15 mL), phenylboronic acid (1.03 g, 8.45mmol), and tetrakis-(triphenylphosphine)-palladium (977 mg, 0.845 mmol). The reaction mixture was stirred at reflux for 8 hr. The mixture was poured into water and the aqueous layer was extracted with $CHCl_3$ (three times). The combined organic layer was dried over $MgSO_4$, filtered, concentrated, and purified by flash chromatography (NH-silica gel, 3% EtOAc in hexane) to give (2-chloro-5-phenyl-pyrimidin-4-yl)-dimethyl-amine (1.44 g, 73%).
ESI MS m/e 256, M + $Na^+$

**Step C: Synthesis of *N*-[*cis*-4-(4-dimethylamino-5-phenyl-pyrimidin-2-ylamino)-cyclohexyl]-2-phenoxy-nicotinamide hydrochloride.**

Using the procedure for the step A of example 3133, the title compound was obtained.
ESI MS m/e 531, M (free) + $Na^+$

**Example 3137**

***N*-[*cis*-4-(5-Chloro-4-dimethylamino-pyrimidin-2-ylamino)-cyclohexyl]-2-phenoxy-nicotinamide hydrochloride**

**Step A: Synthesis of (2,5-dichloro-pyrimidin-4-yl)-dimethyl-amine.**

Using the procedure for the step A of example 3129, the title compound was obtained.
ESI MS m/e 191, $M^+$

**Step B: Synthesis of *N*-[*cis*-4-(5-chloro-4-dimethylamino-pyrimidin-2-ylamino)-cyclohexyl]-2-phenoxy-nicotinamide hydrochloride.**

Using the procedure for the step A of example 3133, the title compound was obtained.

ESI MS m/e 467, M (free) + $H^+$

**Example 3138**

***N*-[*cis*-4-(4-Dimethylamino-5-phenyl-pyrimidin-2-ylamino)-cyclohexyl]-3,4-difluoro-benzamide hydrochloride**

**Step A: Synthesis of *N*-[*cis*-4-(4-dimethylamino-5-phenyl-pyrimidin-2-ylamino)-cyclohexyl]-3,4-difluoro-benzamide hydrochloride.**

Using the procedure for the step A of example 3130, the title compound was obtained.
ESI MS m/e 474, M (free) + $Na^+$

**Example 3139**

***N*-[*cis*-4-(5-Chloro-4-dimethylamino-pyrimidin-2-ylamino)-cyclohexyl]-3,4-difluoro-benzamide hydrochloride**

**Step A: Synthesis of *N*-[*cis*-4-(5-chloro-4-dimethylamino-pyrimidin-2-ylamino)-cyclohexyl]-3,4-difluoro-benzamide hydrochloride.**

Using the procedure for the step A of example 3130, the title compound was obtained.
ESI MS m/e 432, M (free) + $Na^+$

**Example 3140**

***N*-[*cis*-4-(4-Dimethylamino-5-fluoro-pyrimidin-2-ylamino)-cyclohexyl]-2-phenoxy-nicotinamide hydrochloride**

**Step A: Synthesis of (2-chloro-5-fluoro-pyrimidin-4-yl)-dimethyl-amine.**

Using the procedure for the step A of example 3129, the title compound was obtained.
ESI MS m/e 176, M + $H^+$

**Step B : Synthesis of *N*-[*cis*-4-(4-dimethylamino-5-fluoro-pyrimidin-2-ylamino)-cyclohexyl]-2-phenoxy-nicotinamide hydrochloride.**

Using the procedure for the step A of example 3133, the title compound was obtained.
ESI MS m/e 451, M (free) + $H^+$

**Example 3141**

***N*-[*cis*-4-(5-Bromo-4-dimethylamino-pyrimidin-2-ylamino)-cyclohexyl]-2-phenoxy-nicotinamide hydrochloride**

**Step A: Synthesis of *N*-[*cis*-4-(5-bromo-4-dimethylamino-pyrimidin-2-ylamino)-cyclohexyl]-2-phenoxy-nicotinamide hydrochloride.**

Using the procedure for the step A of example 3133, the title compound was obtained.
ESI MS m/e 533, M (free) + $Na^+$

**Example 3142**

***N*-[*cis*-4-(4,6-Dimethyl-pyrimidin-2-ylamino)-cyclohexyl]-3,4-difluoro-benzamide hydrochloride**

**Step A: Synthesis of *N*-[*cis*-4-(4,6-dimethyl-pyrimidin-2-ylamino)-cyclohexyl]-3,4-difluoro-benzamide hydrochloride.**

Using the procedure for the step A of example 3130, the title compound was obtained.
ESI MS m/e 383, M (free) + $Na^+$

**Example 3143**

***N*-[*cis*-4-(4,6-Dimethyl-pyrimidin-2-ylamino)-cyclohexyl]-2-phenoxy-nicotinamide hydrochloride**

**Step A: Synthesis of *N*-[*cis*-4-(4,6-dimethyl-pyrimidin-2-ylamino)-cyclohexyl]-2-phenoxy-nicotinamide hydrochloride.**

Using the procedure for the step A of example 3133, the title compound was obtained.
ESI MS m/e 440, M (free) + $Na^+$

**Example 3144**

**3,4-Difluoro-*N*-[*cis*-4-(pyrimidin-2-ylamino)-cyclohexyl]-benzamide hydrochloride**

**Step A: Synthesis of 3,4-difluoro-*N*-(*cis*-4-(pyrimidin-2-ylamino)-cyclohexyl]-benzamide hydrochloride.**

Using the procedure for the step A of example 3130, the title compound was obtained.
ESI MS m/e 355, M (free) + $Na^+$

**Example 3145**

***N*-[*cis*-4-(4-Dimethylamino-pyrimidin-2-ylamino)-cyclohexylmethyl]-2-phenoxy-nicotinamide hydrochloride**

**Step A: Synthesis of [*cis*-4-(4-dimethylamino-pyrimidin-2-ylamino)-cyclohexylmethyl]-carbamic acid benzyl ester.**

A mixture of (2-chloro-pyrimidin-4-yl)-dimethyl-amine obtained in step A of example 3129 (1.50 g, 9.52 mmol) and (*cis*-4-amino-cyclohexylmethyl)-carbamic acid benzyl ester obtained in step C of example 3068 (2.75 g, 10.5 mmol) in IPA (1.5 mL) was stirred at 130 °C for 22 hr in a sealed tube. The reaction mixture was poured into saturated aqueous $NaHCO_3$ and the aqueous layer was extracted with $CHCl_3$ (three times). The combined organic layer was dried over $MgSO_4$, filtrated, concentrated, and purified by medium-pressure liquid chromatography (NH-silica, 10% EtOAc in hexane to EtOAc) to give [*cis*-4-(4-dimethylamino-pyrimidin-2-ylamino)-cyclohexylmethyl]-carbamic acid benzyl ester (816 mg, 22%) as a pale yellow oil.
ESI MS m/e 406, M + $Na^+$ ; $^1H$ NMR (300 MHz, $CDCl_3$) δ 1.22-1.92 (m, 9 H), 3.03 (s, 6 H), 3.11 (t, *J* = 6.2 Hz, 2 H), 4.02-4.15 (m, 1 H), 4.82-4.93 (m, 2 H), 5.10 (s, 2 H), 5.79 (d, *J* = 6.1 Hz, 1 H), 7.28-7.42 (m, 5 H), 7.83 (d, *J* = 6.1 Hz, 1 H).

**Step B: Synthesis of $N^2$-(*cis*-4-aminomethyl-cyclohexyl)-$N^4$,$N^4$-dimethyl-pyrimidine-2,4-diamine.**

Using the procedure for the step B of example 3118, the title compound was obtained.
ESI MS m/e 250, M + $H^+$; $^1H$ NMR (300 MHz, $CDCl_3$) δ 1.40-1.88 (m, 9 H), 2.87 (d, *J* = 5.9 Hz, 2 H), 3.03 (s, 6 H), 4.11 (brs, 1 H), 5.63 (brs, 1 H), 5.78 (d, *J* = 6.2 Hz, 1 H), 7.08 (brs, 2 H), 7.82 (d, *J* = 6.2 Hz, 1 H).

**Step C: Synthesis of *N*-[*cis*-4-(4-dimethylamino-pyrimidin-2-ylamino)-cyclohexylmethyl]-2-phenoxy-nicotinamide hydrochloride.**

To a solution of $N^2$-(*cis*-4-aminomethyl-cyclohexyl)-$N^4$,$N^4$-dimethyl-pyrimidine-2,4-diamine (400 mg, 1.60 mmol) in $CHCl_3$ (2 mL) were added *i*-$Pr_2NEt$ (0.56 mL, 3.36 mmol) and 2-phenoxy-nicotinoyl chloride (523 mg, 2.24 mmol) in $CHCl_3$ (2 mL). The mixture was stirred at ambient temperature for 5 hr. The reaction was quenched with saturated aqueous $NaHCO_3$ and the aqueous layer was extracted with $CHCl_3$ (three times). The combined organic layer was dried over $MgSO_4$ filtered, concentrated, and purified by medium-pressure liquid chromatography (NH-silica gel, 20% to 50% EtOAc in hexane) to give a colorless oil. To a solution of the above material in EtOAc (10 mL) was added 4 M hydrogen chloride in EtOAc (0.2 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$ and dried at 60 °C under reduced pressure to give *N*-[*cis*-4-(4-dimethylamino-pyrimidin-2-ylamino)-cyclohexylmethyl]-2-phenoxy-nicotinamide hydrochloride (199 mg, 26%) as a white solid.
ESI MS m/e 469, M (free) + $Na^+$

**Example 3146**

**3-Hydroxy-*N*-[*cis*-4-(quinolin-2-ylamino)-cyclohexyl]-benzamide hydrochloride**

**Step A: Synthesis of 3-hydroxy-*N*-[*cis*-4-(quinolin-2-ylamino)-cyclohexyl]-benzamide hydrochloride.**

Using the procedure for the step A of example 3036, the title compound was obtained.
ESI MS m/e 362, M (free) + $H^+$; $^1H$ NMR (300 MHz, DMSO-$d_6$) δ 1.60-2.09 (m, 8 H), 3.83-4.02 (m, 1 H), 4.22-4.49 (m, 1 H), 6.79-7.02 (m, 1 H), 7.12-7.59 (m, 5 H), 7.67-8.45 (m, 5 H), 9.40-9.78 (m, 2 H), 12.91-13.17 (m, 1 H).

**Example 3147**

***N*-[*cis*-4-(Quinolin-2-ylamino)-cyclohexyl]-isophthalamic acid methyl ester hydrochloride**

**Step A: Synthesis of *N*-[*cis*-4-(quinolin-2-ylamino)-cyclohexyl]-isophthalamic acid methyl ester hydrochloride.**

Using the procedure for the step A of example 3036, the title compound was obtained.
ESI MS m/e 404, M (free) + $H^+$ ; $^1H$ NMR (300 MHz, $CDCl_3$) δ 1.54-2.12 (m, 8 H), 3.89-4.31 (m, 5 H), 6.89-7.05 (m, 2 H), 7.41-7.58 (m, 2 H), 7.68-7.82 (m, 3 H), 8.00-8.22 (m, 3 H), 8.46-8.51 (m, 1 H), 9.66-9.85 (m, 1 H).

**Example 3148**

***N*-[*cis*-4-(Quinolin-2-ylamino)-cyclohexyl]-3,5-bis-trifluoromethyl-benzamide hydrochloride**

**Step A: Synthesis of *N*-[*cis*-4-(quinolin-2-ylamino)-cyclohexyl]-3,5-bis-trifluoromethyl-benzamide hydrochloride.**

Using the procedure for the step A of example 3046, the title compound was obtained.
ESI MS m/e 482, M (free) + $H^+$ ; $^1H$ NMR (300 MHz, $CDCl_3$) δ 1.75-2.27 (m, 8 H), 4.00-4.32 (m, 2 H), 6.97 (d, *J* = 9.4 Hz, 1 H), 7.42-7.65 (m, 2 H), 7.69-7.80 (m, 3 H), 7.96-8.02 (m, 1 H), 8.20 (d, *J* = 9.3 Hz, 1 H), 8.35-8.42 (m, 2 H), 9.69-9.79 (m, 1 H).

**Example 3149**

***N*-[*cis*-4-(Quinolin-2-ylamino)-cyclohexyl]-3-trifluoromethoxy-benzamide hydrochloride**

**Step A: Synthesis of *N*-[*cis*-4-(Quinolin-2-ylamino)-cyclohexyl]-3-trifluoromethoxy-benzamide hydrochloride.**

Using the procedure for the step A of example 3046, the title compound was obtained.
ESI MS m/e 430, M (free) + $H^+$ ; $^1H$ NMR (300 MHz, $CDCl_3$) δ 1.77-2. (m, 8 H), 3.96-4.29 (m, 2 H), 6.88-7.03 (m, 2 H), 7.29-7.51 (m, 3 H), 7.69-7.82 (m, 5 H), 8.19 (d, *J* = 9.5 Hz, 1 H), 9.73-9.86 (m, 1 H).

**Example 3150**

***N*-[*cis*-4-(Quinolin-2-ylamino)-cyclohexyl]-isophthalamic acid**

**Step A: Synthesis of *N*-[*cis*-4-(quinolin-2-ylamino)-cyclohexyl]-isophthalamic acid methyl ester.**

To a solution of isophthalic acid monomethyl ester (435 mg) and *cis*-*N*-quinolin-2-yl-cyclohexane-1,4-diamine obtained in step A of example 3033 (500 mg) in DMF (5 mL) were added $Et_3N$ (0.96 mL), HOBt-$H_2O$ (476 mg), and EDC-HCl (437 mg). The reaction mixture was stirred at ambient temperature for 18 hr. To the reaction mixture was added water (20 mL) and the suspension was stirred at ambient temperature for 30 min. The precipitated was collected by filtration, washed with $H_2O$, and purified by medium-pressure liquid chromatography (NH-silica gel, 25% EtOAc in hexane) to give *N*-[*cis*-4-(quinolin-2-ylamino)-cyclohexyl]-isophthalamic acid methyl ester (740 mg) as a white solid.
ESI MS m/e 404, M + $H^+$ ; $^1H$ NMR (300 MHz, $CDCl_3$) δ 1.71-2.05 (m, 8 H), 3.96 (s, 3 H), 4.10-4.28 (m, 2 H), 4.80-4.90 (m, 1 H), 6.16-6.26 (m, 1 H), 6.66 (d, *J* = 8.8 Hz, 1 H), 7.18-7.20 (m, 1 H), 7.49-7.68 (m, 4 H), 7.84 (d, *J* = 83 Hz, 1 H), 8.03-8.10 (m, 1 H), 8.15-8.22 (m, 1 H), 8.35-8.38 (m, 1 H)

**Step B: Synthesis of *N*-[*cis*-4-(Quinolin-2-ylamino)-cyclohexyl]-isophthalamic acid.**

To a solution of *N*-[*cis*-4-(quinolin-2-ylamino)-cyclohexyl]-isophthalamic acid methyl ester (400 mg) in EtOH (12 mL) was added 2 M aqueous NaOH (0.52 mL). The reaction mixture was stirred at ambient temperature for 11 hr. To the reaction mixture was added 1 M aqueous HCl (0.6 mL) and the aqueous layer was extracted with $CHCl_3$ (three times). The combined organic layer was dried over $MgSO_4$, filtered, concentrated under reduced pressure, purified by medium-pressure liquid chromatography (silica gel, 1% to 5% MeOH in $CHCl_3$) to give a white solid. The suspension of above solid in $Et_2O$ (20 mL) was stirred at ambient temperature for 1 hr and filtered to to give *N*-[*cis*-4-(Quinolin-2-ylamino)-cyclohexyl]-isophthalamic acid (183 mg) as a white solid.
ESI MS m/e 412, M (free) + $Na^+$ ; $^1H$ NMR (300 MHz, DMSO-$d_6$) δ 1.63-2.09 (m, 8 H), 3.84-4.18 (m, 2 H), 6.83-6.91 (m, 2 H), 7.07-7.17 (m, 1 H), 7.39-7.64 (m, 4 H), 7.83 (d, *J* = 9.0 Hz, 1 H), 8.03-8.13 (m, 2 H), 8.39-8.53 (m, 2 H).

**Example 3151**

***C*-(Ethyl-phenyl-amino)-*N*-[*cis*-4-(quinolin-2-ylamino)-cyclohexyl]-acetamide dihydrochloride**

**Step A: Synthesis of *C*-(ethyl-phenyl-amino)-*N*-[*cis*-4-(quinolin-2-ylamino)-cyclohexyl]-acetamide dihydrochloride.**

Using the procedure for the step A of example 3036, the title compound was obtained.
ESI MS m/e 403, M (free) + $H^+$ ; $^1H$ NMR (300 MHz, $CDCl_3$) δ 1.18-1.36 (m, 3 H), 1.54-2.15 (m, 8 H), 3.39-3.65 (m, 3 H), 3.68-4.11 (m, 3 H), 6.80-7.20 (m, 3 H), 7.29-7.86 (m, 8 H), 8.07-8.23 (m, 1 H), 9.48-9.68 (m, 1 H).

**Example 3152**

**3,5-Difluoro-*N*-[*cis*-4-(quinolin-2-ylamino)-cyclohexyl]-benzamide hydrochloride**

**Step A: Synthesis of 3,5-Difluoro-*N*-[*cis*-4-(quinolin-2-ylamino)-cyclohexyl]-benzamide hydrochloride.**

Using the procedure for the step A of example 3046, the title compound was obtained.
ESI MS m/e 404, M (free) + $Na^+$ ; $^1H$ NMR (300 MHz, DMSO-$d_6$) δ 1.71-2.02 (m, 8 H), 3.87-4.13 (m, 1 H), 4.24-4.53 (m, 1 H), 7.21-8.01 (m, 7 H), 8.18-8.60 (m, 3 H), 9.48-9.81 (m, 1 H), 13.09-13.28 (m, 1 H).

**Example 3153**

**-Chloro-3-fluoro-*N*-[*cis*-4-(quinolin-2-ylamino)-cyclohexyl]-benzamide-hydrochloride**

**Step A: Synthesis of 4-chloro-3-fluoro-*N*-[*cis*-4-(quinolin-2-ylamino)-cyclohexyl]-benzamide hydrochloride.**

Using the procedure for the step A of example 3036, the title compound was obtained.
ESI MS m/e 398, M (free) + $H^+$ ; $^1H$ NMR (300 MHz, $CDCl_3$) δ 1.80-2.10 (m, 8 H), 3.97-4.27 (m, 2 H), 6.88-7.03 (m, 2 H), 7.39-7.50 (m, 2 H), 7.54-7.62 (m, 1 H), 7.66-7.83 (m, 4 H), 8.19 (d, *J* = 9.4 Hz, 1 H), 9.65-9.82 (m, 1 H).

**Example 3154**

**C-[(4-Chloro-phenyl)-ethyl-amino]-*N*-[*cis*-4-(quinolin-2-ylamino)-cyclohexyl]-acetamide dihydrochloride**

**Step A: Synthesis of *C*-[(4-chloro-phenyl)-ethyl-amino]-*N*-[*cis*-4-(quinolin-2-ylamino)-cyclohexyl]-acetamide dihydrochloride.**

Using the procedure for the step A of example 3036, the title compound was obtained.
ESI MS m/e 459, M (free) + $Na^+$ ; $^1H$ NMR (300 MHz, DMSO-$d_6$) δ 0.99-1.19 (m, 3 H), 1.42-1.96 (m, 8 H), 3.30-3.55 (m, 2 H), 3.71-3.87 (m, 1 H), 3.94 (s, 2 H), 4.29-4.51 (m, 1 H), 6.57-6.77 (m, 2 H), 7.02-7.58 (m, 4 H), 7.65-8.04 (m, 3 H), 8.15-8.44 (m, 2 H), 9.61-9.85 (m, 1 H), 13.17-13.42 (m, 1 H).

**Example 3155**

***N*-[*cis*-4-(Quinolin-2-ylamino)-cyclohexyl]-isophthalamide hydrochloride**

**Step A: Synthesis of *N*-[*cis*-4-(quinolin-2-ylamino)-cyclohexyl]-isophthalamide hydrochloride.**

To a solution of *N*-[*cis*-4-(quinolin-2-ylamino)-cyclohexyl]-isophthalamic acid obtained in step B of example 3150 (160 mg) in DMF (2 mL) were added 28% aqueous $NH_3$ (30 mg), $Et_3N$ (0.14 mL), HOBt-$H_2O$ (94 mg), and EDC-HCl (95 mg). The reaction mixture was stirred at ambient temperature for 16 hr. To the reaction mixture was added water (20 mL) and the aqueous layer extracted with $CHCl_3$ (three times). The combined organic layer was dried over $MgSO_4$, filtered, concentrated under reduced pressure, purified by medium-pressure liquid chromatography (NH-silica gel, 20% to 50% EtOAc in hexane). The solution of above purified material in EtOH (3 mL) was added 4 M hydrogen chloride in EtOAc (0.3 mL). The mixture was stirred at ambient temperature for 2 hr, filtered, and dried under reduced pressure to give *N*-[*cis*-4-(quinolin-2-ylamino)-cyclohexyl]-isophthalamide hydrochloride (9 mg) as a white solid.
ESI MS m/e 411, M (free) + $Na^+$ ; $^1H$ NMR (300 MHz, DMSO-$d_6$) δ 1.70-2.06 (m, 8 H), 3.89-4.08 (m, 1 H), 4.19-4.39 (m, 1 H), 7.17-7.60 (m, 4 H), 7.71-8.46 (m, 8 H), 12.84-12.97 (m, 1 H).

**Example 3156**

**3,4-Difluoro-*N*-{*cis*-4-[(quinolin-2-ylmethyl)-amino]-cyclohexyl}-benzamide dihydrochloride**

**Step A: Synthesis of 3,4-difluoro-*N*-{*cis*-4-[(quinolin-2-ylmethyl)-amino]-cyclohexyl}-benzamide dihydrochloride.**

To a solution of *N*-(*cis*-4-amino-cyclohexyl)-3,4-difluoro-benzamide obtained in step D of example 3031 (250 mg) in $CHCl_3$ (5 mL) were added quinoline-2-carbaldehyde (185 mg), acetic acid (71 mg), and $NaBH(OAc)_3$ (316 mg). The reaction mixture was stirred at ambient temperature for 16 hr. The reaction was quenched with saturated aqueous $NaHCO_3$ and the aqueous layer was extracted with $CHCl_3$ (three times). The combined organic layer was dried over $MgSO_4$, filtrated, concentrated under reduced pressure, and purified by medium-pressure liquid chromatography (NH-silica gel, 20% EtOAc in hexane and silica gel, 2% to 5% MeOH in $CHCl_3$) to give a colorless oil. To a solution of the above oil in EtOAc (10 mL) was added 4 M hydrogen chloride in EtOAc (0.2 mL). The mixture was stirred at ambient temperature for 1 hr and concentrated under reduced pressure. A suspension of the above material in $Et_2O$ (12 mL) was stirred at ambient tempareture for 1 hr. The precipitate was collected by filtration, washed with $Et_2O$, and dried under reduced pressure to give 3,4-difluoro-*N*-{*cis*-4-[(quinolin-2-ylmethyl)-amino]-cyclohexyl}-benzamide dihydrochloride (100 mg) as a white solid.
ESI MS m/e 418, M (free) + $Na^+$ ; $^1H$ NMR (300 MHz, DMSO-$d_6$) δ 1.50-1.68 (m, 2 H), 1.90-2.15 (m, 6 H), 3.20-3.37 (m, 1 H), 3.91-4.01 (m, 1 H), 4.53-4.66 (m, 2 H), 7.46-8.29 (m, 9 H), 8.52 (d, *J* = 8.5 Hz, 1 H), 9.44-9.62 (m, 2 H).

**Example 3157**

***N*-[*cis*-4-(4-Methyl-quinolin-2-ylamino)-cyclohexyl]-3,5-bis-trifluoromethyl-benzamide hydrochloride**

**Step A: Synthesis of *N*-[*cis*-4-(4-methyl-quinolin-2-ylamino)-cyclohexyl]-3,5-bis-trifluoromethyl-benzamide hydrochloride.**

Using the procedure for the step B of example 3070, the title compound was obtained.
ESI MS m/e 496, M (free) + $H^+$ ; $^1H$ NMR (300 MHz, $CDCl_3$) δ 1.77-2.19 (m, 8 H), 2.74 (s, 3 H), 3.98-4.31 (m, 2 H), 6.78-6.81 (m, 1 H), 7.40-7.52 (m, 1 H), 7.58-7.78 (m, 3 H), 7.85 (d, *J* = 9.2 Hz, 1 H), 7.96-8.01 (m, 1 H), 8.36-8.41 (m, 2 H), 9.49-9.64 (m, 1 H).

**Example 3158**

***N*-[*cis*-4-(4-Methyl-quinolin-2-ylamino)-cyclohexyl]-3-trifluoromethoxy-benzamide hydrochloride**

**Step A: Synthesis of *N*-[*cis*-4-(4-methyl-quinolin-2-ylamino)-cyclohexyl]-3-trifluoromethoxy-benzamide hydrochloride.**

Using the procedure for the step B of example 3070, the title compound was obtained.
ESI MS m/e 444, M (free) + $H^+$ ; $^1H$ NMR (300 MHz, $CDCl_3$) δ 1.68-2.20 (m, 8 H), 2.71-2.75 (m, 3 H), 3.96-4.30 (m, 2 H), 6.76-6.87 (m, 2 H), 7.30-7.39 (m, 1 H), 7.42-7.52 (m, 2 H), 7.67-7.89 (m, 5 H), 9.50-9.72 (m, 1 H).

**Example 3159**

***C*-(Ethyl-phenyl-amino)-*N*-[*cis*-4-(4-methyl-quinolin-2-ylamino)-cyclohexyl]-acetamide dihydrochloride**

**Step A: Synthesis of *C*-(ethyl-phenyl-amino)-*N*-[*cis*-4-(4-methyl-quinolin-2-ylamino)-cyclohexyl]-acetamide dihydrochloride.**

Using the procedure for the step A of example 3071, the title compound was obtained.
ESI MS m/e 417, M (free) + $H^+$ ; $^1H$ NMR (300 MHz, $CDCl_3$) δ 1.10-1.38 (m, 3 H), 1.59-2.05 (m, 8 H), 2.45-2.84 (m, 3 H), 3.35-4.15 (m, 6 H), 6.57-6.81 (m, 1 H), 6.85-7.52 (m, 7 H), 7.57-7.89 (m, 4 H), 9.20-9.50 (m, 1 H).

**Example 3160**

**3-Hydroxy-*N*-[*cis*-4-(4-methyl-quinotin-2-ylamino)-cyclohexyl]-benzamide hydrochloride**

**Step A: Synthesis of 3-hydroxy-*N*-[*cis*-4-(4-methyl-quinolin-2-ylamino)-cyclohexyl]-benzamide hydrochloride.**

Using the procedure for the step A of example 3071, the title compound was obtained.
ESI MS m/e 398, M (free) + $Na^+$ ; $^1H$ NMR (300 MHz, DMSO-$d_6$) δ 1.67-2.02 (m, 8 H), 2.53-2.70 (m, 3 H), 3.86-3.99 (m, 1 H), 4.24-4.40 (m, 1 H), 6.88-6.96 (m, 1 H), 7.06-7.31 (m, 4 H), 7.46-7.57 (m, 1 H), 7.73-7.83 (m, 1 H), 7.92-8.28 (m, 3 H), 9.66 (s, 1 H), 12.83-12.94 (m, 1 H).

**Example 3161**

**2-Amino-*N*-[*cis*-4-(4-methyl-quinolin-2-ylamino)-cyclohexyl]-nicotinamide dihydrochloride**

**Step A: Synthesis of 2-amino-*N*-[*cis*-4-(4-methyl-quinolin-2-ylamino)-cyclohexyl]-nicotinamide dihydrochloride.**

Using the procedure for the step A of example 3071, the title compound was obtained.
ESI MS m/e 376, M (free) + $H^+$ ; $^1H$ NMR (300 MHz, DMSO-$d_6$) δ 1.63-2.06 (m, 8 H), 2.53-2.70 (m, 3 H), 3.87-4.04 (m, 1 H), 4.36-4.59 (m, 1 H), 6.92-7.06 (m, 1 H), 7.15-7.27 (m, 1 H), 7.45-7.58 (m, 1 H), 7.69-7.84 (m, 1 H), 7.89-8.01 (m, 1 H), 8.14-8.58 (m, 4 H), 8.69-8.86 (m, 1 H), 9.54-9.72 (m, 1 H).

**Example 3162**

**2,3-Difluoro-*N*-[*cis*-4-(4-methyl-quinolin-2-ylamino)-cyclohexyl]-benzamide hydrochloride**

**Step A: Synthesis of 2,3-difluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 418, M (free) + $Na^+$ ; $^1H$ NMR (300 MHz, $CDCl_3$) δ 1.56-2.17 (m, 8 H), 2.72 (s, 3 H), 3.88-4.04 (m, 1 H), 4.09-4.30 (m, 1 H), 6.67-6.92 (m, 2 H), 7.10-7.35 (m, 2 H), 7.41-7.52 (m, 1 H), 7.60-7.93 (m, 4 H), 9.53-9.75 (m, 1 H).

**Example 3163**

**2,4-Difluoro-*N*-[*cis*-4-(4-methyl-quinolin-2-ylamino)-cyclohexyl]-benzamide hydrochloride**

**Step A: Synthesis of 2,4-difluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 418, M (free) + $Na^+$ ; $^1H$ NMR (300 MHz, $CDCl_3$) δ 1.57-2.22 (m, 8 H), 2.73 (s, 3 H), 3.87-4.06 (m, 1 H), 4.11-4.31 (m, 1 H), 6.69-7.06 (m, 4 H), 7.40-7.56 (m, 1 H), 7.65-7.88 (m, 3 H), 7.98-8.14 (m, 1 H), 9.51-9.83 (m, 1 H).

**Example 3164**

**2,5-Difluoro-*N*-[*cis*-4-(4-methyl-quinolin-2-ylamino)-cyclohexyl]-benzamide hydrochloride**

**Step A: Synthesis of 2,5-difluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 418, M (free) + $Na^+$ ; $^1H$ NMR (300 MHz, $CDCl_3$) δ 1.46-2.14 (m, 8 H), 2.72 (s, 3 H), 3.84-4.04 (m, 1 H), 4.09-4.32 (m, 1 H), 6.77 (s, 1 H), 6.82-7.21 (m, 3 H), 7.37-7.54 (m, 1 H), 7.63-7.89 (m, 4 H), 9.54-9.72 (m, 1 H).

**Example 3165**

**2,6-Difluoro-*N*-[*cis*-4-(4-methyl-quinolin-2-ylamino)-cyclohexyl]-benzamide hydrochloride**

**Step A: Synthesis of 2,6-difluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 418, M (free) + $Na^+$ ; $^1H$ NMR (300 MHz, $CDCl_3$) δ 1.72-2.08 (m, 8 H), 2.72 (s, 3 H), 3.91-4.03 (m, 1 H), 4.13-4.33 (m, 1 H), 6-42-6.54 (m, 1 H), 6.77 (s, 1 H), 6.88-6.99 (m, 2 H), 7.27-7.50 (m, 2 H), 7.66-7.78 (m, 2 H), 7.84 (d, *J* = 8.2 Hz, 1 H), 9.53-9.70 (m, 1 H).

**Example 3166**

**3,5-Difluoro-*N*-[*cis*-4-(4-methyl-quinolin-2-ylamino)-cyclohexyl]-benzamide hydrochloride**

**Step A: Synthesis of 3,5-difluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 418, M (free) + $Na^+$ ; $^1H$ NMR (300 MHz, $CDCl_3$) δ 1.78-2.16 (m, 8 H), 2.72 (s, 3 H), 3.96-4.26 (m, 2 H), 6.78 (s, 1 H), 6.86-7.02 (m, 2 H), 7.33-7.52 (m, 3 H), 7.67-7.78 (m, 2 H), 7.85 (d, *J* = 8.2 Hz, 1 H), 9.48-9.71 (m, 1 H).

**Example 3167**

***C*-[(4-Chloro-phenyl)-ethyl-amino]-*N*-[*cis*-4-(4-methyl-quinolin-2-ylamino)-cyclohexyl]-acetamide dihydrochloride**

**Step A: Synthesis of *C*-[(4-chloro-phenyl)-ethyl-amino]-*N*-[*cis*-4-(4-methyl-quinolin-2-ylamino)-cyclohexyl]-acetamide dihydrochloride.**

Using the procedure for the step A of example 3071, the title compound was obtained.
ESI MS m/e 451, M (free) + $H^+$; $^1H$ NMR (300 MHz, $CDCl_3$) δ 1.14-1.26 (m, 3 H), 1.69-2.00 (m, 8 H), 2.60 (s, 3 H), 3.39-3.61 (m, 2 H), 3.75-4.03 (m, 4 H), 6.63-7.06 (m, 4 H), 7.14-7.32 (m, 2 H), 7.39-7.51 (m, 1 H), 7.64-7.89 (m, 3 H), 9.44-9.59 (m, 1 H).

**Example 3168**

**4-Chloro-3-fluoro-*N*-[*cis*-4-(4-methyl-quinolin-2-ylamino)-cyclohexyl]-benzamide hydrochloride**

**Step A: Synthesis of 4-chloro-3-fluoro-*N*-[*cis*-4-(4-methyl-quinolin-2-ylamino)-cyclohexyl]-benzamide hydrochloride.**

Using the procedure for the step A of example 3071, the title compound was obtained.
ESI MS m/e 412, M (free) + $H^+$ ; $^1H$ NMR (300 MHz, $CDCl_3$) δ 1.78-2.13 (m, 8 H), 2.70-2.76 (m, 3 H), 3.95-4.28 (m, 2 H), 6.65-6.81 (m, 2 H), 7.41-7.50 (m, 2 H), 7.53-7.59 (m, 1 H), 7.65-7.77 (m, 3 H), 7.82-7.88 (m, 1 H), 9.57-9.71 (m, 1 H).

**Example 3169**

**4-Fluoro-*N*-[*cis*-4-(4-methyl-quinolin-2-ylamino)-cyclohexyl]-benzamide hydrochloride**

**Step A: Synthesis of 4-fluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 378, M (free) + $H^+$ ;$^1H$ NMR (300 MHz, $CDCl_3$) δ 1.81-2.10 (m, 8 H), 2.72 (s, 3 H), 3.95-4.29 (m, 2 H), 6.65-6.81 (m, 2 H), 7.10 (t, *J* = 8.6 Hz, 2 H), 7.42-7.51 (m, 1 H), 7.67-7.91 (m, 5 H), 9.55-9.67 (m, 1 H).

**Example 3170**

**3-Fluoro-*N*-[*cis*-4-(4-methyl-quinolin-2-ylamino)-cyclohexyl]-benzamide hydrochloride**

**Step A: Synthesis of 3-Fluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 378, M (free) + $H^+$ ; $^1H$ NMR (300 MHz, $CDCl_3$) δ 1.77-2.09 (m, 8 H), 2.71-2.76 (m, 3 H), 3.94-4.25 (m, 2 H), 6.54-6.65 (m, 1 H), 6.76-6.81 (m, 1 H), 7.13-7.23 (m, 1 H), 7.35-7.61 (m, 4 H), 7.67-7.88 (m, 3 H), 9.58-9.73 (m, 1 H).

**Example 3171**

**2-Fluoro-*N*[*cis*-4-(4-methyl-quinolin-2-ylamino)-cyclohexyl]-benzamide hydrochloride**

**Step A: Synthesis of 2-Fluoro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 378, M (free) + $H^+$ ; $^1H$ NMR (300 MHz, $CDCl_3$) δ 1.84-2.15 (m, 8 H), 2.72 (s, 3 H), 3.87-4.01 (m, 1 H), 4.13-4.29 (m, 1 H), 6.73-6.89 (m, 2 H), 7.07-7.28 (m, 2 H), 7.40-7.51 (m, 2 H), 7.66-7.87 (m, 3 H), 7.96-8.05 (m, 1 H), 9.62-9.72 (m, 1 H).

**Example 3172**

**4-Chloro-*N* [*cis*-4-(4-methyl-quinolin-2-ylamino)-cyclohexyl]-benzamide hydrochloride**

**Step A: Synthesis of 4-chloro-*N*-[*cis*-4-(4-methyl-quinolin-2-ylamino)-cyclohexyl]-benzamide hydrochloride.**

Using the procedure for the step B of example 3070, the title compound was obtained.
ESI MS m/e 394, M (free) + $H^+$ ; $^1H$ NMR (300 MHz, DMSO-$d_6$) δ 1.64-2.04 (m, 8 H), 2.55-2.70 (m, 3 H), 3.87-4.04 (m, 1 H), 4.27-4.52 (m, 1 H), 7.07-7.18 (m, 1 H), 7.46-7.58 (m, 3 H), 7.73-8.02 (m, 4 H), 8.23-8.38 (m, 2 H), 9.39-9.52 (m, 1 H), 12.96-13.10 (m, 1 H).

**Example 3173**

**2-Hydroxy-*N*-[*cis*-4-(4-methyl-quinolin-2-ylamino)-cyclohexyl]-nicotinamide hydrochloride**

**Step A: Synthesis of 2-Hydroxy-*N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 399, M (free) + $Na^+$ ; $^1H$ NMR (300 MHz, DMSO-$d_6$) δ 1.46-1.99 (m, 8 H), 2.53-2.72 (m, 3 H), 4.02-4.15 (m, 1 H), 4.20-4.45 (m, 1 H), 6.46-6.56 (m, 1 H), 6.95-7.08 (m, 1 H), 7.45-7.57 (m, 1 H), 7.69-7.83 (m, 2 H), 7.90-8.47 (m, 3 H), 10.08-10.27 (m, 1 H), 12.48-12.63 (m, 1 H).

**Example 3174**

***N*-[*cis*-4-(4-Methyl-quinolin-2-ylamino)-cyclohexyl]-isophthalamic acid-methyl ester hydrochloride**

**Step A: Synthesis of *N*-[*cis*-4-(4-methyl-quinolin-2-ylamino)-cyclohexyl]-isophthalamic acid methyl ester hydrochloride.**

Using the procedure for the step A of example 3071, the title compound was obtained.
ESI MS m/e 440, M (free) + $Na^+$ ; $^1H$ NMR (300 MHz, $CDCl_3$) δ 1.78-2.21 (m, 8 H), 2.73 (d, *J* = 1.1 Hz, 3 H), 3.92-4.07 (m, 4 H), 4.13-4.29 (m, 1 H), 6.78 (s, 1 H), 6.99-7.10 (m, 1 H), 7.40-7.57 (m, 2 H), 7.67-7.79 (m, 2 H), 7.82-7.89 (m, 1 H), 8.02-8.19 (m, 2 H), 8.46-8.52 (m, 1 H), 9.46-9.65 (m, 1 H).

**Example 3175**

**6-Chloro-*N*-[*cis*-4-(4-methyl-quinolin-2-ylamino)-cyclohexyl]-nicotinamide hydrochloride**

**Step A: Synthesis of 6-chloro-*N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 417, M (free) + $Na^+$ ; $^1H$ NMR (300 MHz, DMSO-$d_6$) δ 1.67-2.03 (m, 8 H), 2.54-2.72 (m, 3 H), 3.91-4.06 (m, 1 H), 4.26-4.42 (m, 1 H), 7.05-7.18 (m, 1 H), 7.45-7.57 (m, 1 H), 7.63-7.69 (m, 1 H), 7.73-7.83 (m, 1 H), 7.91-8.04 (m, 1 H), 8.17-8.31 (m, 2 H), 8.51-8.62 (m, 1 H), 8.83-8.89 (m, 1 H), 9.33-9.51 (m, 1 H), 12.86-13.03 (m, 1 H).

**Example 3176**

**6-Dimethylamino-*N*-[*cis*-4-(4-methyl-quinolin-2-ylamino)-cyclohexyl]-nicotinamide dihydrochloride**

**Step A: Synthesis of 6-dimethylamino-*N*-[*cis*-4-(4-methyl-quinolin-2-ylamino)-cyclohexyl]-nicotinamide dihydrochloride.**

To a solution of 6-chloro-*N*-[*cis*-4-(4-methyl-quinolin-2-ylamino)-cyclohexyl]-nicotinamide obtained in step A of example 3175 (250 mg) in IPA (1 mL) were added 50% aqueous $Me_2NH$ (63 mg) and $iPr_2NEt$ (172 mg). The mixture was stirred at reflux for 5 hr, added 50% aqueous $Me_2NH$ (120 mg), and stirred at reflux for 5 hr. The reaction was quenched with saturated aqueous $NaHCO_3$ and the aqueous layer was extracted with $CHCl_3$ (three times). The combined organic layer was dried over $MgSO_4$, filtrated, concentrated under reduced pressure, and purified by medium-pressure liquid chromatography (NH-silica gel, EtOAc). To a solution of the above material in EtOH (3 mL) was added 4 M hydrogen chloride in EtOAc (0.47 mL). The mixture was stirred at ambient temperature for 1 hr and concentrated under reduced pressure. A suspension of the above material in $Et_2O$ (3 mL) was stirred at ambient tempareture for 4 hr. The precipitate was collected by filtration, washed with $Et_2O$, and dried under reduced pressure to give 6-dimethylamino-*N*-[*cis*-4-(4-methyl-quinolin-2-ylamino)-cyclohexyl]-nicotinamide dihydrochloride (200 mg) as a white solid.
ESI MS m/e 426, M (free) + $Na^+$; $^1H$ NMR (300 MHz, $CDCl_3$) δ 1.73-2.13 (m, 8 H), 2.63-2.80 (m, 3 H), 3.34-3.61 (m, 6 H), 3.91-4.28 (m, 2 H), 6.70-7.07 (m, 2 H), 7.35-8.10 (m, 5 H), 8.29-8.46 (m, 1 H), 8.82-8.98 (m, 1 H), 9.36-9.51 (m, 1 H).

**Example 3177**

**3-Hydroxymethyl-*N*-[*cis*-4-(4-methyl-quinolin-2-ylamino)-cyclohexyl]-benzamide hydrochloride**

**Step A: Synthesis of 3-hydroxymethyl-*N*-[*cis*-4-(4-methyl-quinolin-2-ylamino)-cyclohexyl]-benzamide hydrochloride.**

To a suspension of LiAIH (18 mg) in $Et_2O$ (5 mL) was added *N*-[*cis*-4-(4-methyl-quinolin-2-ylamino)-cyclohexyl]-isophthalamic acid methyl ester obtained in step A of example 3174 (200 mg) in $Et_2O$ (2 mL) . The mixture was stirred at ambient temperature for 3 hr. The reaction was quenched with water and the aqueous layer was extracted with $CHCl_3$ (three times). The combined organic layer was dried over $MgSO_4$, filtrated, concentrated under reduced pressure and purified by medium-pressure liquid chromatography (silica gel, 3% to 10% MeOH in $CHCl_3$). To a solution of the above material in EtOH (2 mL) was added 4 M hydrogen chloride in EtOAc (0.24 mL). The mixture was stirred at ambient temperature for 2 hr and concentrated under reduced pressure. A suspension of the above material in $Et_2O$ (3 mL) was stirred at ambient tempareture for 1 hr. The precipitate was collected by filtration, washed with $Et_2O$, and dried at 70 °C under reduced pressure to give 3-hydroxymethyl-*N*-[*cis*-4-(4-methyl-quinolin-2-ylamino)-cyclohexyl]-benzamide hydrochloride (93 mg) as a white solid.
ESI MS m/e 390, M (free) + $H^+$ ; $^1H$ NMR (300 MHz, DMSO-$d_6$) δ 1.66-2.02 (m, 8 H), 2.61 (s, 3 H), 3.87 (brs, 1 H), 4.22-4.42 (m, 1 H), 4.55 (s, 2 H), 7.03-7.17 (m, 1 H), 7.35-7.59 (m, 3 H), 7.67-7.87 (m, 3 H), 7.91-8.04 (m, 1 H), 8.11-8.31 (m, 2 H), 12.75-12.96 (m, 1 H).

**Example 3178**

***N*-[*cis*-4-(4-Methyl-quinolin-2-ylamino)-cyclohexyl]-isophthalamide hydrochloride**

**Step A: Synthesis of *N*-[*cis*-4-(4-methyl-quinolin-2-ylamino)-cyclohexyl]-isophthalamic acid methyl ester.**

To a solution of isophthalic acid monomethyl ester (400 mg) and *N*-(*cis*-4-methyl-quinolin-2-yl)-cyclohexane-1,4-diamine in step A of example 3070 (400 mg) in DMF (4 mL) were added $Et_3N$ (0.52 mL), HOBt $H_2O$ (358 mg), and EDC-HCl (330 mg). The reaction mixture was stirred at ambient temperature for 12 hr. To the reaction mixture was added water (20 mL) and the suspension was stirred at ambient temperature for 30 min. The precipitated was collected by filtration, washed with $H_2O$, and purified by medium-pressure liquid chromatography (silica gel, 9% MeOH in $CHCl_3$) to give *N*-[*cis*-4-(4-methyl-quinolin-2-ylamino)-cyclohexyl]-isophthalamic acid methyl ester (740 mg) as a white solid.
ESI MS m/e 440, M + $Na^+$ ; $^1H$ NMR (200 MHz, $CDCl_3$) δ 1.59-2.09 (m, 8 H), 2.58 (s, 3 H), 3.96 (s, 3 H), 4.02-4.29 (m, 2 H), 4.72-4.87 (m, 1 H), 6.12-6.27 (m, 1 H), 6.48-6.59 (m, 1 H), 7.17-7.30 (m, 1 H), 7.45-7.82 (m, 4 H), 8.00-8.22 (m, 2 H), 8.32-8.39 (m, 1 H).

**Step B: Synthesis of *N*-[*cis*-4-(4-methyl-quinolin-2-ylamino)-cyclohexyl]-isophthalamide hydrochloride.**

To a solution of *N*-[*cis*-4-(4-methyl-quinolin-2-ylamino)-cyclohexyl]-isophthalamic acid methyl ester (150 mg) in EtOH (4.5 mL) was added 2 M aqueous NaOH (0.27 mL). The reaction mixture was stirred at ambient temperature for 13 hr. To the reaction mixture was added 1 M aqueous HCl (0.3 mL) and the aqueous layer was extracted with $CHCl_3$ (three times). The combined organic layer was dried over $MgSO_4$, filtered, concentrated under reduced pressure, purified by medium-pressure liquid chromatography (silica gel, 1% to 5% MeOH in $CHCl_3$) to give a white solid. To a solution of the above solid in DMF (2 mL) was added 28% aqueous $NH_3$ (21 mg), $Et_3N$ (0.1 mL), HOBt-$H_2O$ (67 mg), and EDC-HCl (67 mg). The reaction mixture was stirred at ambient temperature for 12 hr. To the reaction mixture was added water (20 mL) and the aqueous layer was extracted with $CHCl_3$ (three times). The combined organic layer was dried over $MgSO_4$, filtered, concentrated under reduced pressure, purified by medium-pressure liquid chromatography (NH-silica gel, 3% to 9% MeOH in $CHCl_3$). The solution of above material in EtOAc (10 mL) was added 4 M hydrogen chloride in EtOAc (0.2 mL). The mixture was stirred at ambient temperature for 1 hr and concentrated under reduced pressure. A suspension of the above material in $Et_2O$ (12 mL) was stirred at ambient tempareture for 2 hr. The precipitate was collected by filtration, washed with $Et_2O$, and under reduced pressure to give *N*-[*cis*-4-(4-methyl-quinolin-2-ylamino)-cyclohexyl]-isophthalamide hydrochloride
ESI MS m/e 403, M (free) + $H^+$ ; $^1H$ NMR (300 MHz, DMSO-$d_6$) δ 1.69-2.04 (m, 8 H), 2.56-2.63 (m, 3 H), 3.92-4.06 (m, 1 H), 4.28-4.48 (m, 1 H), 7.06-7.17 (m, 1 H), 7.41-7.58 (m, 3 H), 7.70-8.04 (m, 3 H), 8.06-8.43 (m, 3 H), 9.35-9.54 (m, 1 H), 12.87-13.07 (m, 1 H).

**Example 3179**

**3-Chloro-5-fluoro-*N*-[*cis*-4-(4-methyl-quinolin-2-ylamino)-cyclohexyl]-benzamide hydrochloride**

**Step A: Synthesis of 3-chloro-5-fluoro-*N*-[*cis*-4-(4-methyl-quinolin-2-ylamino)-cyclohexyl]-benzamide hydrochloride.**

Using the procedure for the step A of example 3071, the title compound was obtained.
ESI MS m/e 412, M (free) + $H^+$ ; $^1H$ NMR (300 MHz, $CDCl_3$) δ 1.79-2.12 (m, 8 H), 2.73 (d, *J* = 0.9 Hz, 3 H), 3.96-4.22 (m, 2 H), 6.75-6.90 (m, 2 H), 7.17-7.25 (m, 1 H), 7.42-7.51 (m, 2 H), 7.59-7.89 (m, 4 H), 9.51-9.72 (m, 1 H).

**Example 3180**

**3,4,5-Trifluoro-*N* [*cis*-4-(4-methyl-quinolin-2-ylamino)-cyclohexyl]-benzamide hydrochloride**

**Step A: Synthesis of 3,4,5-trifluoro-*N*-[*cis*-4-(4-methyl-quinolin-2-ylamino)-cyclohexyl]-benzamide hydrochloride.**

Using the procedure for the step A of example 3071, the title compound was obtained.
ESI MS m/e 414, M (free) + $H^+$ ; $^1H$ NMR (300 MHz, $CDCl_3$) δ 1.76-2.16 (m, 8 H), 2.73 (d, *J* = 1.1 Hz, 3 H), 3.97-4.24 (m, 2 H), 6.78 (s, 1 H), 6.92-7.04 (m, 1 H), 7.41-7.60 (m, 3 H), 7.68-7.77 (m, 2 H), 7.82-7.89 (m, 1 H), 9.50-9.64 (m, 1 H).

**Example 3181**

**Pyridine-2-carboxylic acid [*cis*-4-(4-methyl-quinolin-2-ylamino)-cyclohexyl]-amide hydrochloride**

**Step A: Synthesis of pyridine-2-carboxylic acid [*cis*-4-(4-methyl-guinolin-2-ylamino)-cyclohexyl]-amide hydrochloride.**

Using the procedure for the step A of example 3071, the title compound was obtained.
ESI MS m/e 383, M (free) +$Na^+$ ; $^1H$ NMR (300 MHz, DMSO-$d_6$) δ 1.63-2.07 (m, 8 H), 2.54-2.71 (m, 3 H), 4.00-4.13 (m, 1 H), 4.49-4.62 (m, 1 H), 7.10-7.20 (m, 1 H), 7.46-7.56 (m, 1 H), 7.61-8.12 (m, 5 H), 8.33-8.42 (m, 2 H), 8.65-8.72 (m, 1 H), 9.46-9.60 (m, 1 H), 13.23-13.38 (m, 1 H).

**Example 3182**

***N*-[*cis*-4-(4-Methyl-quinolin-2-ylamino)-cyclohexyl]-nicotinamide hydrochloride**

**Step A: Synthesis of *N*-[*cis*-4-(4-methyl-quinolin-2-ylamino)-cyclohexyl]-nicotinamide hydrochloride.**

Using the procedure for the step A of example 3071, the title compound was obtained.
ESI MS m/e 383, M (free) + $Na^+$ ; $^1H$ NMR (300 MHz, DMSO-$d_6$) δ 1.76-2.05 (m, 8 H), 2.54-2.73 (m, 3 H), 3.93-4.07 (m, 1 H), 4.29-4.48 (m, 1 H), 7.10-7.19 (m, 1 H), 7.47-7.57 (m, 1 H), 7.72-7.85 (m, 2 H), 7.92-8.04 (m, 1 H), 8.21-8.33 (m, 1 H), 8.48-8.57 (m, 1 H), 8.65-8.73 (m, 1 H), 8.82-8.89 (m, 1 H), 9.14-9.20 (m, 1 H), 9.42-9.58 (m, 1 H), 12.93-13.08 (m, 1 H).

**Example 3183**

***N*-[*cis*-4-(4-Methyl-quinolin-2-ylamino)-cyclohexyl]-isonicotinamide hydrochloride**

**Step A: Synthesis of *N*-[*cis*-4-(4-methyl-quinolin-2-ylamino)-cyclohexyl)-isonicotinamide hydrochloride.**

Using the procedure for the step A of example 3071, the title compound was obtained.
ESI MS m/e 383, M (free) + $Na^+$ ; $^1H$ NMR (300 MHz, DMSO-$d_6$) δ 1.68-2.08 (m, 8 H), 2.53-2.71 (m, 3 H), 3.92-4.08 (m, 1 H), 4.33-4.54 (m, 1 H), 7.11-7.22 (m, 1 H), 7.43-7.60 (m, 1 H), 7.69-7.86 (m, 1 H), 7.89-8.41 (m, 4 H), 8.81-9.07 (m, 3 H), 9.48-9.67 (m, 1 H), 13.03-13.24 (m, 1 H).

**Example 3184**

**4-Chloro-pyridine-2-carboxylic acid [*cis*-4-(4-methyl-quinolin-2-ylamino)-cyclohexyl)-amide hydrochloride**

**Step A: Synthesis of 4-Chloro-pyridine-2-carboxylic acid [*cis*-4-(4-methyl-quinolin-2-ylamino)-cyclohexyl]-amide hydrochloride.**

Using the procedure for the step A of example 3071, the title compound was obtained.
ESI MS m/e 417, M (free) + $Na^+$ ; $^1H$ NMR (300 MHz, DMSO-$d_6$) δ 1.62-2.05 (m, 8 H), 2.53-2.72 (m, 3 H), 3.99-4.51 (m, 2 H), 7.04-7.15 (m, 1 H), 7.46-7.57 (m, 1 H), 7.72-7.85 (m, 2 H), 7.92-8.10 (m, 2 H), 8.16-8.29 (m, 1 H), 8.39 (d, *J* = 8.1 Hz, 1 H), 8.66 (d, *J* = 5.3 Hz, 1 H), 9.32-9.51 (m, 1 H), 12.93-13.08 (m, 1 H).

**Example 3185**

**5-Bromo-*N*-[*cis*-4-(4-methyl-quinolin-2-ylamino)-cyclohexyl]-nicotinamide dihydrochloride**

**Step A: Synthesis of 5-bromo-*N*-[*cis*-4-(4-methyl-quinolin-2-ylamino)-cyclohexyl]-nicotinamide dihydrochloride.**

Using the procedure for the step A of example 3071, the title compound was obtained.
ESI MS m/e 439, M (free) + $H^+$ ; $^1H$ NMR (300 MHz, DMSO-$d_6$) δ 1.71-2.02 (m, 8 H), 2.54-2.71 (m, 3 H), 3.88-4.08 (m, 1 H), 4.25-4.50 (m, 1 H), 7.06-7.18 (m, 1 H), 7.47-7.56 (m, 1 H), 7.70-7.83 (m, 1 H), 7.91-8.04 (m, 1 H), 8.19-8.33 (m, 1 H), 8.43-8.64 (m, 2 H), 8.86-8.88 (m, 1 H), 8.97-8.99 (m, 1 H), 9.35-9.50 (m, 1 H), 12.89-13.08 (m, 1 H).

**Example 3186**

***N*-[*cis*-4-(4-Methyl-quinolin-2-ylamino)-cyclohexyl]-6-trifluoromethyl-nicotinamide hydrochloride**

**Step A: Synthesis of *N*-[*cis*-4-(4-methyl-quinolin-2-ylamino)-cyclohexyl]-6-trifluoromethyl-nicotinamide hydrochloride.**

Using the procedure for the step A of example 3071, the title compound was obtained.
ESI MS m/e 451, M (free) + $Na^+$ ; $^1H$ NMR (300 MHz, DMSO-$d_6$) δ 1.58-2.04 (m, 8 H), 2.53-2.75 (m, 3 H), 3.91-4.09 (m, 1 H), 4.22-4.44 (m, 1 H), 7.03-7.22 (m, 1 H), 7.45-7.59 (m, 1 H), 7.71-7.85 (m, 1 H), 7.91-8.10 (m, 2 H), 8.15-8.30 (m, 1 H), 8.42-8.54 (m, 1 H), 8.64-8.81 (m, 1 H), 9.12-9.21 (m, 1 H), 9.33-9.54 (m, 1 H), 12.88-13.00 (m, 1 H).

**Example 3187**

**6-Imidazol-1-yl-*N*-[*cis*-4-(4-methyl-quinolin-2-ylamino)-cyclohexyl]-nicotinamide dihydrochloride**

**Step A: Synthesis of 6-imidazol-1-yl-*N*-[*cis*-4-(4-methyl-quinolin-2-ylamino)-cyclohexyl]-nicotinamide dihydrochloride.**

To a solution of 6-chloro-*N*-[*cis*-4-(4-methyl-quinolin-2-ylamino)-cyclohexyl]-nicotinamide obtained in step A of example 3175 (250 mg) in BuOH (1 mL) were added imidazole (47 mg) and $iPr_2NEt$ (172 mg). The mixture was heated in a microwave synthesizer at 220°C for 10 min and 230°C for 20 min. To the mixture was added saturated aqueous $NaHCO_3$ and the aqueous layer was extracted with $CHCl_3$ (three times). The combined organic layer was dried over $MgSO_4$, filtrated, concentrated under reduced pressure, and purified by medium-pressure liquid chromatography (NH-silica gel, 50% in EtOAc in nexane). To a solution of the above material in EtOAc (10 mL) was added 4 M hydrogen chloride in EtOAc (0.2 mL). The mixture was stirred at ambient temperature for 1 hr and concentrated under reduced pressure. A suspension of the above material in $Et_2O$ (12 mL) was stirred at ambient tempareture for 4 hr. The precipitate was collected by filtration, washed with $Et_2O$, and dried under reduced pressure to give 6-imidazol-1-yl-*N*-[*cis*-4-(4-methyl-quinolin-2-ylamino)-cyclohexyl]-nicotinamide dihydrochloride (83 mg) as a white solid.
ESI MS m/e 427, M (free) + $H^+$ ; $^1H$ NMR (300 MHz, DMSO-$d_6$) δ 1.35-2.39 (m, 8 H), 2.60-2.81 (m, 3 H), 3.92-4.28 (m, 2 H), 6.63-6.92 (m, 1 H), 7.09-8.23 (m, 8 H), 8.53-8.82 (m, 1 H), 8.95-9.41 (m, 2 H), 9.96-10.17 (m, 1 H), 13.97-14.19 (m, 1 H).

**Example 3188**

***N*-[*cis*-4-(4-Dimethylamino-quinolin-2-ylamino)-cyclohexyl]-3,4-difluoro-benzamide hydrochloride**

**Step A: Synthesis of N-[*cis*-4-(4-dimethylamino-quinolin-2-ylamino)-cyclohexyl]-3,4-difluoro-benzamide hydrochloride.**

To a solution of 3,4-difluoro-benzoic acid (199 mg) and $N^2$-(*cis*-4-amino-cyclohexyl)-$N^4$-methyl-quinoline-2,4-diamine obtained in step E of example 1 (300 mg) in DMF (3 mL) were added $Et_3N$ (0.35 mL), HOBt-$H_2O$ (241 mg), and EDC-HCI (242 mg). The reaction mixture was stirred at ambient temperature for 15 hr. To the mixture was added water (4.8 mL) and the aqueous layer was extracted with $CHCl_3$ (three times). The combined organic layer was dried over $MgSO_4$, filtered, concentrated under reduced pressure, and purified by medium-pressure liquid chromatography (NH-silica gel, 20% EtOAc in hexane). To a solution of the above material in EtOAc (4 mL) was added 4 M hydrogen chloride in EtOAc (0.5 mL). The mixture was stirred at ambient temperature for 1 hr. The precipitate was collected by filtration, washed with EtOAc, and dried under reduced pressure to give *N*-[*cis*-4-(4-dimethylamino-quinolin-2-ylamino)-cyclohexyl)-3,4-difluoro-benzamide hydrochloride (263 mg) as a white solid.
ESI MS m/e 425, M (free) + $H^+$ ; $^1H$ NMR (300 MHz, $CDCl_3$) $\delta$ 1.69-2.20 (m, 8 H), 3.24 (s, 6 H), 3.81-4.30 (m, 2 H), 5.82 (s, 1 H), 6.74-6.88 (m, 1 H), 7.10-7.40 (m, 2 H), 7.51-7.98 (m, 5 H), 8.86-8.99 (m, 1 H), 13.44-13.63 (m, 1 H).

**Example 3189**

**5-Nitro-thiophene-3-carboxylic acid [*cis*-4-(4-dimethylamino-quinolin-2-ylamino)-cyclohexyl]-amide hydrochloride**

**Step A: Synthesis of 5-nitro-thiophene-3-carboxylic acid [*cis*-4-(4-dimethylamino-quinolin-2-ylamino)-cyclohexyl]-amide hydrochloride.**

Using the procedure for the step A of example 3188, the title compound was obtained.
ESI MS m/e 462, M (free) + $Na^+$ ; $^1H$ NMR (300 MHz, $CDCl_3$) $\delta$ 1.65-2.17 (m, 8 H), 3.25 (s, 6 H), 3.82-4.00 (m, 1 H), 4.00-4.23 (m, 1 H), 5.82 (s, 1 H), 7.25-7.40 (m, 1 H), 7.58-7.97 (m, 4 H) 8.28-8.42 (m, 2 H), 8.56-8.73 (m, 1 H), 13.02-13.30 (m, 1 H).

**Example 3190**

***N*-[*cis*-4-(4-Dimethylamino-quinolin-2-ylamino)-cyclohexylmethyl]-3,4-difluoro-benzamide hydrochloride**

**Step A: Synthesis of *N*-[*cis*-4-(4-dimethylamino-quinolin-2-ylamino)-cyclohexylmethyl]-3,4-difluoro-benzamide hydrochloride.**

To a solution of $N^2$-(*cis*-4-aminomethyl-cyclohexyl)-$N^4$,$N^4$-dimethyl-quinoline-2,4-diamine obtained in step B of example 7 (300 mg) in $CHCl_3$ (3 mL) were added $iPr_2NEt$ (0.36 mL) and 3,4-difluoro-benzoyl chloride (194 mg). The mixture was stirred at ambient temperature for 6 hr. The reaction was quenched with saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (NH-silica gel, 9% to 20 % EtOAc in hexane and 2% to 9% MeOH in $CHCl_3$) to give *N*-[*cis*-4-(4-dimethylamino-quinolin-2-ylamino)-cyclohexylmethyl]-3,4-difluoro-benzamide hydrochloride (272 mg) as white solid.
ESI MS m/e 439, M (free) + $H^+$ ; $^1H$ NMR (300 MHz, $CDCl_3$) $\delta$ 1.53-2.08 (m, 9 H), 3.21 (s, 6 H), 3.47-3.56 (m, 2 H), 3.86-3.98 (m, 1 H), 5.81 (s, 1 H), 6.95-7.09 (m, 1 H), 7.16-7.34 (m, 2 H), 7.53-7.68 (m, 2 H), 7.80-7.95 (m, 3 H), 9.08-9.22 (m, 1 H), 13.40-13.51 (m, 1 H).

**Example 3191**

**1-(2,3-Dichloro-phenyl)-3-[*cis*-4-(4-dimethylamino-quinolin-2-ylamino)-cyclohexylmethyl]-urea hydrochloride**

**Step A: Synthesis of 1-(2,3-dichloro-phenyl)-3-[*cis*-4-(4-dimethylamino-quinolin-2-ylamino)-cyclohexylmethyl]-urea hydrochloride.**

To a solution of $N^2$-(*cis*-4-aminomethyl-cyclohexyl)-$N^4$,$N^4$-dimethyl-quinoline-2,4-diamine obtained in step B

of example 7 (300 mg) in DMSO (3 mL) was added 1,2-dichloro-4-isocyanato-benzene (207 mg). The mixture was stirred at ambient temperature for 12 hr and poured into water. The precipitate was filtrated, washed with water, and purified by medium-pressure liquid chromatography (NH-silica gel, 25% to 50% EtOAc in hexane). To a solution of the above material in EtOAc (2 mL) was added 4 M hydrogen chloride in EtOAc (10 mL). The mixture was stirred at ambient temperature for 1 hr and concentrated under reduced pressure. A suspension of the residue in $Et_2O$ (20 mL) was stirred at ambient tempareture for 1 hr. The precipitate was collected by filtration, washed with $Et_2O$, and dried under reduced pressure to give 1-(2,3-dichloro-phenyl)-3-[*cis*-4-(4-dimethylamino-quinolin-2-ylamino)-cyclohexylmethyl]-urea hydrochloride (170 mg) as a white solid.
ESI MS m/e 486, $M^+$; $^1H$ NMR (300 MHz, $CDCl_3$) δ 1.51-2.18 (m, 9 H), 3.23 (s, 6 H), 3.36-3.44 (m, 2 H), 3.91-4.02 (m, 1 H), 5.78-5.88 (m, 1 H), 6.97-7.12 (m, 3 H), 7.26-7.35 (m, 1 H), 7.58-7.66 (m, 1 H), 7.86 (m, *J* = 9.0 Hz, 2 H), 8.16 (dd, *J* = 8.2, 1.7 Hz, 1 H), 8.20-8.31 (m, 1 H), 8.65-8.76 (m, 1 H), 12.98-13.21 (m, 1 H).

**Example 3192**

**_N_-[_cis_-4-(4-dimethylamino-5,6,7,8-tetrahydro-quinazolin-2-ylamino)-cyclohexyl]-3,4-difluoro-benzamide hydrochloride**

**Step A: Synthesis of _N_-[_cis_-4-(4-dimethylamino-5,6,7,8-tetrahydro-quinazolin-2-ylamino)-cyclohexyl]-3,4-difluoro-benzamide hydrochloride.**

To a solution of 3,4-difluoro-benzoic acid (199 mg) and $N^2$-(*cis*-4-amino-cyclohexyl)-$N^4$,$N^4$-dimethyl-5,6,7,8-tetrahydro-quinazoline-2,4-diamine in step D of example 3107 (304 mg) in DMF (4 mL) were added $Et_3N$ (0.35 mL), HOBt-$H_2O$ (241 mg), and EDC-HCl (242 mg). The reaction mixture was stirred at ambient temperature for 7 hr. To the reaction mixture was added water (20 mL) and the suspension was stirred at ambient temperature for 1 hr. The precipitated was collected by filtration, washed with $H_2O$, and purified by medium-pressure liquid chromatography (NH-silica gel, 20% EtOAc in hexane). To a solution of the above material in EtOAc (10 mL) was added 4 M hydrogen chloride in EtOAc (0.2 mL). The mixture was stirred at ambient temperature for 1 hr. The precipitate was collected by filtration, washed with EtOAc, and dried under reduced pressure to give *N*-[*cis*-4-(4-dimethylamino-5,6,7,8-tetrahydro-quinazolin-2-ylamino)-cyclohexyl]-3,4-difluoro-benzamide hydrochloride (252 mg) as a white solid.
ESI MS m/e 430, M (free) + $H^+$; $^1H$ NMR (300 MHz, $CDCl_3$) δ 1.56-2.22 (m, 12 H), 2.48-2.84 (m, 4 H), 3.23 (s, 6 H), 3.92-4.33 (m, 2 H), 6.51-6.77 (m, 1 H), 7.01-7.30 (m, 1 H), 7.43-7.86 (m, 2 H), 8.28-8.57 (m, 1 H), 12.56 (m, 1 H).

**Example 3193**

**5-Nitro-thiophene-3-carboxylic acid [_cis_-4-(4-dimethylamino-5,6,7,8-tetrahydro-quinazolin-2-ylamino)-cyclohexyl]-amide hydrochloride**

**Step A: Synthesis of 5-nitro-thiophene-3-carboxylic acid [_cis_-4-(4-dimethylamino-5,6,7,8-tetrahydro-quinazolin-2-ylamino)-cyclohexyl]-amide hydrochloride.**

Using the procedure for the step A of example 3192, the title compound was obtained.
ESI MS m/e 467, M (free) + $Na^+$; $^1H$ NMR (300 MHz, $CDCl_3$) δ 1.51-2.24 (m, 12 H), 2.51-2.62 (m, 2 H), 2.67-2.81 (m, 2 H), 3.23 (s, 6 H), 3.98-4.29 (m, 2 H), 7.42-7.48 (m, 1 H), 8.22-8.29 (m, 2 H), 8.37 (s, 1 H).

**Example 3194**

**1-Methyl-4-nitro-1_H_-pyrrole-2-carboxylic acid [_cis_-4-(4-dimethylamino-5,6,7,8-tetrahydro-quinazolin-2-ylamino)-cyclohexyl]-amide hydrochloride**

**Step A: Synthesis of 1-methyl-4-nitro-1_H_-pyrrole-2-carboxylic acid [_cis_-4-(4-dimethylamino-5,6,7,8-tetrahydro-quinazolin-2-ylamino)-cyclohexyl]-amide hydrochloride**

Using the procedure for the step A of example 3192, the title compound was obtained.
ESI MS m/e 442, M (free) + $H^+$; $^1H$ NMR (300 MHz, $CDCl_3$) δ 1.57-2.13 (m, 12 H), 2.49-2.61 (m, 2 H), 2.68-2.81 (m, 2 H), 3.22 (s, 6 H), 3.93-4.04 (m, 4 H), 4.14-4.24 (m, 1 H), 7.04-7.12 (m, 1 H), 7.23-7.27 (m, 1 H), 7.49-7.54 (m, 1 H), 8.30-8.41 (m, 1 H), 12.66-12.92 (m, 1 H).

**Example 3195**

***N*-[*cis*-4-(4-Dimethylamino-5,6,7,8-tetrahydro-quinazolin-2-ylamino)-cyclohexylmethyl]-3,4-difluoro-benzamide hydrochloride**

**Step A: Synthesis of *N*-[*cis*-4-(4-dimethylamino-5,6,7,8-tetrahydro-quinazolin-2-ylamino)-cyclohexylmethyl]-3,4-difluoro-benzamide hydrochloride.**

To a solution of $N^2$-(*cis*-4-aminomethyl-cyclohexyl)-$N^4$,$N^4$-dimethyl-5,6,7,8-tetrahydro-quinazoline-2,4-diamine in step A of example 3113 (300 mg) in $CHCl_3$ (3 mL) were added $iPr_2NEt$ (0.36 mL) and 3,4-difluoro-benzoyl chloride (194 mg). The mixture was stirred at ambient temperature for 17 hr. The reaction was quenched with saturated aqueous $NaHCO_3$ and the aqueous layer was extracted with $CHCl_3$ (three times). The combined organic layer was dried over $MgSO_4$ filtered, concentrated under reduced pressure, and purified by medium-pressure liquid chromatography (NH-silica gel, 33% EtOAc in hexane). To a solution of the above material in EtOAc (2 mL) was added 4 M hydrogen chloride in EtOAc (10 mL). The mixture was stirred at ambient temperature for 1 hr and concentrated under reduced pressure. A suspension of the residue in $Et_2O$ (20 mL) was stirred at ambient tempareture for 1 hr. The precipitate was collected by filtration, washed with $Et_2O$, and dried under reduced pressure to give *N*-[*cis*-4-(4-dimethylamino-5,6,7,8-tetrahydro-quinazolin-2-ylamino)-cyclohexylmethyl]-3,4-difluoro-benzamide hydrochloride (263 mg) as a white solid.
ESI MS m/e 466, M (free) + $Na^+$; $^1H$ NMR (300 MHz, $CDCl_3$) δ 1.50-1.96 (m, 13 H), 2.49-2.59 (m, 2 H), 2.66-2.77 (m, 2H), 3.21 (s, 6 H), 3.42-3.51 (m, 2 H), 4.16-4.28 (m, 1 H), 6.91-7.01 (m, 1 H), 7.17-7.26 (m, 1 H), 7.80-7.92 (m, 2 H), 8.55 (d, *J* = 8.2 Hz, 1 H), 12.61-12.77 (m, 1 H).

**Example 3196**

**1-(2,3-Dichloro-phenyl)-3-[*cis*-4-(4-dimethylamino-5,6,7,8-tetrahydro-quinazolin-2-ylamino)-cyclohexylmethyl]-urea hydrochloride**

**Step A: Synthesis of 1-(2,3-dichloro-phenyl)-3-[*cis*-4-(4-dimethylamino-5,6,7,8-tetrahydro-quinazolin-2-ylamino)-cyclohexylmethyl]-urea hydrochloride.**

To a solution of $N^2$-(*cis*-4-aminomethyl-cyclohexyl)-$N^4$,$N^4$-dimethyl-5,6,7,8-tetrahydro-quinazoline-2,4-diamine in step A of example 3113 (300 mg) in DMSO (3 mL) was added 1,2-dichloro-4-isocyanato-benzene (207 mg). The mixture was stirred at ambient temperature for 12 hr and poured into water. The precipitate was filtrated, washed with water, and purified by medium-pressure liquid chromatography (NH-silica gel, 25% to 50% EtOAc in hexane). To a solution of the above material in EtOAc (2 mL) was added 4 M hydrogen chloride in EtOAc (10 mL). The mixture was stirred at ambient temperature for 1 hr and concentrated under reduced pressure. A suspension of the residue in $Et_2O$ (20 mL) was stirred at ambient tempareture for 1 hr. The precipitate was collected by filtration, washed with $Et_2O$, and dried under reduced pressure to give 1-(2,3-dichloro-phenyl)-3-[*cis*-4-(4-dimethylamino-5,6,7,8-tetrahydro-quinazolin-2-ylamino)-cyclohexylmethyl]-urea hydrochloride (113 mg) as a white solid.
ESI MS m/e 491, $M^+$; $^1H$ NMR (200 MHz, $CDCl_3$) δ 1.42-2.04 (m, 13 H), 2.46-2.80 (m, 4 H), 3.21 (s, 6 H), 3.29-3.44 (m, 2 H), 4.18-4.38 (m, 1 H), 6.80-7.22 (m, 3 H), 8.06-8.45 (m, 3 H), 12.04-12.29 (m, 1 H).

**Example 3197**

***N*-[*cis*-4-(4-Dimethylamino-pyrimidin-2-ylamino)-cyclohexyl]-3,4-difluoro-benzamide hydrochloride**

**Step A: Synthesis of *N*-[*cis*-4-(4-dimethylamino-pyrimidin-2-ylamino)-cyclohexyl]-3,4-difluoro-benzamide hydrochloride.**

Using the procedure for the step D of example 3129, the title compound was obtained. ESI MS m/e 376, M (free) + $H^+$; $^1H$ NMR (300 MHz, $CDCl_3$) δ 1.61-2.01 (m, 8 H), 3.17 (s, 3 H), 3.28 (s, 3 H), 3.98-4.32 (m, 2 H), 5.98 (d, *J* = 7.3 Hz, 1 H), 6.45-6.63 (m, 1 H), 7.11-7.30 (m, 1 H), 7.41-7.79 (m, 3 H), 8.67-8.94 (m, 1 H), 12.89-13.06 (m, 1 H).

**Example 3198**

**5-Nitro-thiophene-3-carboxylic acid [*cis*-4-(4-dimethylamino-pyrimidine-2-ylamino)-cyclohexyl]-amide hydrochloride**

**Step A: Synthesis of 5-nitro-thiophene-3-carboxylic acid [*cis*-4-(4-dimethylamino-pyrimidine-2-ylamino)-cyclohexyl]-amide hydrochloride.**

To a solution of 5-nitro-thiophene-3-carboxylic acid (265 mg) and *cis*-$N^2$-(4-amino-cyclohexyl)-$N^4$,$N^4$-dimethyl-pyrimidine-2,4-diamine in step C of example 3129 (300 mg) in DMF (3 mL) were added $Et_3N$ (0.43 mL), HOBt-$H_2O$ (293 mg), and EDC-HCl (293 mg). The reaction mixture was stirred at ambient temperature for 12 hr. To the reaction mixture was added water (20 mL) and the suspension was stirred at ambient temperature for 1 hr. The precipitated was collected by filtration, washed with $H_2O$ and purified by medium-pressure liquid chromatography (NH-silica gel, 20% to 50% EtOAc in hexane). To a solution of the above material in EtOAc (10 mL) was added 4 M hydrogen chloride in EtOAc (2 mL). The mixture was stirred at ambient temperature for 1 hr. The precipitate was collected by filtration, washed with EtOAc, and dried under reduced pressure to give 5-nitro-thiophene-3-carboxylic acid [*cis*-4-(4-dimethylamino-pyrimidine-2-ylamino)-cyclohexyl]-amide hydrochloride (71 mg) as a white solid.
ESI MS m/e 413, M (free) + $Na^+$; $^1H$ NMR (300 MHz, $CDCl_3$) $\delta$ 1.62-2.02 (m, 8 H), 3.18 (s, 3 H), 3.27 (s, 3 H), 3.99-4.29 (m, 2 H) 5.99 (d, $J$ = 7.5 Hz, 1 H), 7.48-7.64 (m, 2 H), 8.34 (d, J = 1.8 Hz, 1 H), 8.48 (d, $J$ = 1.8 Hz, 1 H), 8.50-8.67 (m, 1 H), 12.58-12.76 (m, 1 H).

**Example 3199**

**5-(4-Chloro-phenyl)-furan-2-carboxylic acid [*cis*-4-(4-dimethylamino-pyrimidin-2-ylamino)-cyclohexyl]-amide hydrochloride**

**Step A: Synthesis of 5-(4-Chloro-phenyl)-furan-2-carboxylic acid [*cis*-4-(4-dimethylamino-pyrimidin-2-ylamino)-cyclohexyl]-amide hydrochloride.**

Using the procedure for the step A of example 3198, the title compound was obtained. ESI MS m/e 462, M (free) + $Na^+$; $^1H$ NMR (300 MHz, $CDCl_3$) $\delta$ 1.67-2.07 (m, 8 H), 3.17 (s, 3 H), 3.28 (s, 3 H), 4.01-4.27 (m, 2 H), 5.97 (d, $J$ = 6.9 Hz, 1 H), 6.71 (d, $J$ = 3.5 Hz, 1 H), 6.76-6.87 (m, 1 H), 7.17 (d, $J$ = 3.5 Hz, 1 H), 7.36-7.55 (m, 3 H), 7.69-7.79 (m, 2 H), 8.65-8.86 (m, 1 H), 13.08-13.30 (m, 1 H).

**Example 3200**

**4'-Fluoro-biphenyl-4-carboxylic acid [*cis*-4-(4-dimethylamino-pyrimidin-2-ylamino)-cyclohexyl]-amide hydrochloride**

**Step A: Synthesis of 4'-fluoro-biphenyl-4-carboxylic acid [*cis*-4-(4-dimethylamino-pyrimidin-2-ylamino)-cyelohexyl]-amide hydrochloride.**

Using the procedure for the step A of example 3198, the title compound was obtained. ESI MS m/e 456, M (free) + $Na^+$; $^1H$ NMR (300 MHz, $CDCl_3$) $\delta$ 1.66-2.06 (m, 8 H), 3.17 (s, 3 H), 3.28 (s, 3 H), 4.06-4.32 (m, 2 H), 5.97 (d, $J$ = 7.3 Hz, 1 H), 6.50-6.60 (m, 1 H), 7.09-7.20 (m, 2 H), 7.43-7.64 (m, 5 H), 7.85-7.91 (m, 2 H), 8.74-8.86 (m, 1 H), 12.98-13.23 (m, 1 H).

**Example 3201**

***N*-[*cis*-4-(4-Dimethylamino-pyrimidin-2-ylamino)-cyclohexyl]-2-(4-fluoro-phenoxy)-nicotinamide hydrochloride**

**Step A: Synthesis of *N*-[*cis*-4-(4-dimethylamino-pyrimidin-2-ylamino)-cyclohexyl]-2-(4-fluoro-phenoxy)-nicotinamide hydrochloride.**

Using the procedure for the step A of example 3198, the title compound was obtained.
ESI MS m/e 473, M (free) + $Na^+$; $^1H$ NMR (300 MHz, $CDCl_3$) $\delta$ 1.62-2.05 (m, 8 H), 3.16 (s, 3 H), 3.26 (s, 3 H), 4.07-4.24 (m, 2 H), 5.94 (d, $J$ = 7.3 Hz, 1 H), 7.09-7.20 (m, 3 H), 7.23-7.32 (m, 2 H), 7.42-7.52 (m, 1 H), 7.8 1 -7.94 (m, 1 H),

8.20 (dd, *J* = 4.8, 2.0 Hz, 1 H), 8.54 (dd, *J* = 7.5, 2.1 Hz, 1 H), 8.70-8.80 (m, 1 H), 13.23-13.38 (m, 1 H).

**Example 3202**

***N*-[*cis*-4-(4-Dimethylamino-pyrimidin-2-ylamino)-cyclohexyl]-*C*-(ethyl-phenyl-amino)-acetamide dihydrochloride**

**Step A: Synthesis of *N*-[*cis*-4-(4-dimethylamino-pyrimidin-2-ylamino)-cyclohexyl]-*C*-(ethyl-phenyl-amino)-acetamide dihydrochloride.**

Using the procedure for the step A of example 3198, the title compound was obtained.
ESI MS m/e 419, M (free) + $Na^+$; $^1H$ NMR (300 MHz, $CDCl_3$) δ 1.14-1.35 (m, 3 H), 1.55-1.92 (m, 8 H), 3.15 (s, 3 H), 3.24 (s, 3 H), 3.45-3.64 (m, 2 H), 3.75-4.06 (m, 4 H), 5.91-6.03 (m, 1 H), 7.00-7.64 (m, 7 H), 8.32-8.48 (m, 1 H), 13.12-13.34 (m, 1 H).

**Example 3203**

***C*-[*cis*-(4-Chloro-phenyl)-ethyl-amino]-N-[4-(4-dimethylamino-pyrimidin-2-ylamino)-cyclohexyl]-acetamide dihydrochloride**

**Step A: Synthesis of *C*-[*cis*-(4-chloro-phenyl)-ethyl-amino]-N-[4-(4-dimethylamino-pyrimidin-2-ylamino)-cyclohexyl]-acetamide dihydrochloride.**

Using the procedure for the step A of example 3198, the title compound was obtained.
ESI MS m/e 431, M (free) + $H^+$; $^1H$ NMR (300 MHz, $CDCl_3$) δ 1.12-1.24 (m, 3 H), 1.51-1.96 (m, 8 H), 3.15 (s, 3 H), 3.25 (s, 3 H), 3.43-3.55 (m, 2 H), 3.74-3.98 (m, 3 H), 4.01-4.18 (m, 1 H), 5.88-6.02 (m, 1 H), 6.68-6.87 (m, 3 H), 7.15-7.24 (m, 2 H), 7.43-7.52 (m, 1 H), 8.49-8.62 (m, 1 H), 13.11-13.28 (m, 1 H).

**Example 3204**

**2-(3,4-Difluoro-phenyl)-*N*-[*cis*-4-(4-dimethylamino-pyrimidin-2-ylamino)-cyclohexyl]-acetamide hydrochloride**

**Step A: Synthesis of 2-(3,4-difluoro-phenyl)-*N*-[*cis*-4-(4-dimethylamino-pyrimidin-2-ylamino)-cyclohexyl]-acetamide hydrochloride.**

Using the procedure for the step A of example 3198, the title compound was obtained.
ESI MS m/e 390, M (free) + $H^+$; $^1H$ NMR (300 MHz, DMSO-$d_6$) δ1.46-1.87 (m, 8 H), 3.15 (s, 3 H), 3.18 (s, 3 H), 3.46 (s, 2 H), 3.58-3.75 (m, 1 H), 3.86-4.04 (m, 1 H), 6.36 (d, *J* = 7.4 Hz, 1 H), 7.05-7.13 (m, 1 H), 7.27-7.40 (m, 2 H), 7.84-7.94 (m, 1 H), 8.10-8.19 (m, 1 H), 8.27-8.38 (m, 1 H), 12.14-12.23 (m, 1 H).

**Example 3205**

***N*-[*cis*-4-(4-Dimethylamino-pyrimidin-2-ylamino)-cyclohexyl]-3,5-difluoro-benzamide hydrochloride**

**Step A: Synthesis of *N*-[*cis*-4-(4-dimethylamino-pyrimidin-2-ylamino)-cyclohexyl]-3,5-difluoro-benzamide hydrochloride.**

Using the procedure for the step D of example 3129, the title compound was obtained.
ESI MS m/e 376, M (free) + $H^+$; $^1H$ NMR (300 MHz, $CDCl_3$) δ 1.64-2.02 (m, 8 H), 3.17 (s, 3 H), 3.28 (s, 3 H), 4.01-4.31 (m, 2 H), 5.97 (d, *J* = 7.4 Hz, 1 H), 6.46-6.57 (m, 1 H), 6.87-6.98 (m, 1 H), 7.30-7.40 (m, 2 H), 7.49 (d, *J* = 7.4 Hz, 1 H), 8.77-8.93 (m, 1 H).

**Example 3206**

**3-Chloro-*N*-[*cis*-4-(4-dimethylamino-pyrimidin-2-ylamino)-cyclohexyl]-4-fluoro-benzamide hydrochloride**

**Step A: Synthesis of 3-Chloro-*N*-[*cis*-4-(4-dimethylamino-pyrimidin-2-ylamino)-cyclohexyl]-4-fluoro-benzamide hydrochloride.**

Using the procedure for the step A of example 3198, the title compound was obtained.
ESI MS m/e 392, M (free) + $H^+$; $^1H$ NMR (300 MHz, $CDCl_3$) δ 1.65-2.00 (m, 8 H), 3.17 (s, 3 H), 3.28 (s, 3 H), 4.03-4.30 (m, 2 H), 5.97 (d, *J* = 7.5 Hz, 1 H), 6.43-6.53 (m, 1 H), 7.19 (t, J = 8.5 Hz, 1 H), 7.43-7.54 (m, 1 H), 7.65-7.75 (m, 1 H), 7.90-7.97 (m, 1 H), 8.76-8.94 (m, 1 H), 12.95-13.14 (m, 1 H).

**Example 3207**

**4-Chloro-*N*-[*cis*-4-(4-dimethylamino-pyrimidin-2-ylamino)-cyclohexyl]-3-fluoro-benzamide hydrochloride**

**Step A: Synthesis of 4-Chloro-*N*-[*cis*-4-(4-dimethylamino-pyrimidin-2-ylamino)-cyclohexyl]-3-fluoro-benzamide hydrochloride.**

Using the procedure for the step A of example 3198, the title compound was obtained.
ESI MS m/e 392, M (free) + $H^+$; $^1H$ NMR (300 MHz, DMSO-$d_6$) δ 1,56-1.98 (m, 8 H), 3.05-3.27 (m, 6 H), 3.76-4.10 (m, 2 H), 6.37 (d, *J* = 7.6 Hz, 1 H), 7.65-7.80 (m, 2 H), 7.84-7.97 (m, 2 H), 8.21-8.34 (m, 1 H), 8.39-8.56 (m, 1 H), 12.09-12.27 (m, 1 H).

**Example 3208**

**Pyridine-2-carboxylic acid [*cis*-4-(4-dimethylamino-pyrimidin-2-ylamino)-cyclohexyl]-amide hydrochloride**

**Step A: Synthesis of pyridine-2-carboxylic acid [*cis*-4-(4-dimethylamino-pyrimidin-2-ylamino)-cyclohexyl]-amide hydrochloride.**

Using the procedure for the step A of example 3198, the title compound was obtained. ESI MS m/e 341, M (free) + $H^+$; $^1H$ NMR (300 MHz, $CDCl_3$) δ 1.72-2.07 (m, 8 H), 3.17 (s, 3 H), 3.27 (s, 3 H), 4.02-4.22 (m, 2 H), 5.97 (d, *J* = 7.4 Hz, 1 H), 7.36-7.55 (m, 2 H), 7.76-7.88 (m, 1 H), 8.10-8.29 (m, 2 H), 8.52-8.70 (m, 2 H).

**Example 3209**

***N*-[*cis*-4-(4-Dimethylamino-pyrimidin-2-ylamino)-cyclohexyl]-nicotinamide dihydrochloride**

**Step A: Synthesis of *N*-[*cis*-4-(4-dimethylamino-pyrimidin-2-ylamino)-cyclohexyl]-nicotinamide dihydrochloride.**

Using the procedure for the step A of example 3198, the title compound was obtained.
ESI MS m/e 341, M (free) + $H^+$; $^1H$ NMR (300 MHz, DMSO-$d_6$) δ 1.62-2.03 (m, 8 H), 3.15 (s, 3 H), 3.20 (s, 3 H), 3.83-4.08 (m, 2 H), 6.37 (d, *J* = 7.4 Hz, 1 H), 7.81-7.98 (m, 2 H), 8.34-8.48 (m, 1 H), 8.58-8.66 (m, 1 H), 8.76-8.93 (m, 2 H), 9.17-9.23 (m, 1 H), 12.30-12.48 (m, 1 H).

**Example 3210**

***N*-[*cis*-4-(4-Dimethylamino-pyrimidin-2-ylamino)-cyclohexyl]-isonicotinamide dihydrochloride**

**Step A: Synthesis of *N*-[*cis*-4-(4-dimethylamino-pyrimidin-2-ylamino)-cyclohexyl]-isonicotinamide dihydrochloride.**

Using the procedure for the step A of example 3198, the title compound was obtained.
ESI MS m/e 341, M (free) + $H^+$; $^1H$ NMR (300 MHz, DMSO-$d_6$) δ 1.67-1.99 (m, 8 H), 3.16 (s, 3 H), 3.20 (s, 3 H), 3.84-4.07 (m, 2 H), 6.37 (d, *J* = 7.4 Hz, 1 H), 7.86-8.02 (m, 1 H), 8.25 (d, *J* = 6.5 Hz, 2 H), 8.48-8.57 (m, 1 H), 8.95-9.13 (m, 3 H), 12.53-12.69 (m, 1 H).

**Example 3211**

**5-Bromo-*N*-[*cis*-4-(4-dimethylamino-pyrimidin-2-ylamino)-cyclohexyl]-nicotinamide hydrochloride**

**Step A: Synthesis of 5-Bromo-*N*-[*cis*-4-(4-dimethylamino-pyrimidin-2-ylamino)-cyclohexyl]-nicotinamide hydrochloride.**

Using the procedure for the step A of example 3198, the title compound was obtained.
ESI MS m/e 419, M (free) + $H^+$; $^1$H NMR (300 MHz, $CDCl_3$) δ1.64-2.07 (m, 8 H), 3.18 (s, 3 H), 3.28 (s, 3 H), 4.04-4.31 (m, 2 H), 5.95-6.04 (m, 1 H), 7.37-7.65 (m, 2 H), 8.42 (brs, 1 H), 8.63-8.74 (m, 1 H), 8.79 (brs, 1 H), 9.12 (brs, 1 H), 12.72-12.97 (m, 1 H).

**Example 3212**

***N*-[*cis*-4-(4-Dimethylamino-pyrimidin-2-ylamino)-cyclohexyl]-6-trifluoromethyl-nicotinamide hydrochloride**

**Step A: Synthesis of *N*-[*cis*-4-(4-dimethylamino-pyrimidin-2-ylamino)-cyclohexyl]-6-trifluoromethyl-nicotinamide hydrochloride.**

Using the procedure for the step A of example 3198, the title compound was obtained.
ESI MS m/e 409, M (free) + $H^+$; $^1$H NMR (300 MHz, $CDCl_3$) δ 1.63-2.06 (m, 8 H), 3.18 (s, 3 H),3.27 (s, 3 H), 4.07-4.34 (m, 2 H), 5.98 (d, *J* = 7.4 Hz, 1 H), 7.47-7.62 (m, 2 H), 7.72 (d, *J* = 8.0 Hz, 1 H), 8.35-8.45 (m, 1 H), 8.57-8.74 (m, 1 H), 9.24-9.31 (m, 1 H).

**Example 3213**

**4-Chloro-pyridine-2-carboxylic acid [*cis*-4-(4-dimethylamino-pyrimidin-2-ylamino)-cyclohexyl]-amide hydrochloride**

**Step A: Synthesis of 4-chloro-pyridine-2-carboxylic acid [*cis*-4-(4-dimethylamino-pyrimidin-2-ylamino)-cyclohexyl]-amide hydrochloride.**

Using the procedure for the step A of example 3198, the title compound was obtained.
ESI MS m/e 375, M (free) + $H^+$; $^1$H NMR (300 MHz, $CDCl_3$) δ 1.71-2.09 (m, 8 H), 3.18 (s, 3 H), 3.28 (s, 3 H), 4.01-4.24 (m, 2 H), 5.88-6.08 (m, 1 H), 7.39-7.59 (m, 2 H), 8.05-8.35 (m, 2 H), 8.43-8.72 (m, 2 H), 13.20-13.45 (m, 1 H).

**Example 3214**

***N*-[*ci*s-4-(4-Dimethylamino-pyrimidin-2-ylamino)-cyclohexyl]-4-fluoro-benzamide hydrochloride**

**Step A: Synthesis of *N*-[*cis*-4-(4-dimethylamino-pyrimidin-2-ylamino)-cyclohexyl]-4-fluoro-benzamide hydrochloride.**

Using the procedure for the step D of example 3129, the title compound was obtained.
ESI MS m/e 380, M (free) + $Na^+$; $^1$H NMR (300 MHz, $CDCl_3$) δ 1.63-2.24 (m, 8 H), 3.17 (s, 3 H), 3.27 (s, 3 H), 4.01-4.32 (m, 2 H), 5.97 (d, *J* = 7.3 Hz, 1 H), 6.38-6.57 (m, 1 H), 7.01-7.17 (m, 2 H), 7.41-7.54 (m, 1 H), 7.77-7.91 (m, 2 H), 8.76-8.84 (m, 1 H), 12.86-13.14 (m, 1 H).

**Example 3215**

**3-Chloro-*N*-[*cis*-4-(4-dimethylamino-pyrimidin-2-ylamino)-cyclohexyl]-5-fluoro-benzamide hydrochloride**

**Step A: Synthesis of 3-Chloro-*N*-[*cis*-4-(4-dimethylamino-pyrimidin-2-ylamino)-cyclohexyl]-5-fluoro-benzamide hydrochloride.**

Using the procedure for the step A of example 3198, the title compound was obtained.
ESI MS m/e 414, M (free) + $Na^+$; $^1$H NMR (300 MHz, $CDCl_3$) δ 1.64-2.03 (m, 8 H), 3.17 (s, 3 H), 3.28 (s, 3 H), 4.02-4.31 (m, 2 H), 5.97 (d, J = 7.4 Hz, 1 H), 6.53-6.67 (m, 1 H), 7.16-7.23 (m, 1 H), 7.41-7.51 (m, 2 H), 7.58-7.64 (m, 1 H),

8.76-8.91 (m, 1 H).

**Example 3216**

***N*-[*cis*-4-(4-Dimethylamino-pyrimidin-2-ylamino)-cyclohexyl]-3,4,5-trifluoro-benzamide hydrochloride**

**Step A: Synthesis of *N*-[*cis*-4-(4-dimethylamino-pyrimidin-2-ylamino)-cyclohexyl]-3,4,5-trifluoro-benzamide hydrochloride.**

Using the procedure for the step A of example 3198, the title compound was obtained.
ESI MS m/e 416, M (free) + $Na^+$; $^1H$ NMR (300 MHz, $CDCl_3$) δ 1.66-2.03 (m, 8 H), 3.18 (s, 3 H), 3.28 (s, 3 H), 4.01-4.34 (m, 2 H), 5.98 (d, *J* = 7.4 Hz, 1 H), 6.70-6.79 (m, 1 H), 7.42-7.63 (m, 3 H), 8.73-8.86 (m, 1 H).

**Example 3217**

**3,5-Di-tert-butyl-*N*-[*cis*-4-(4-dimethylamino-pyrimidin-2-ylamino)-cyclohexyl]-4-hydroxy-benzamide hydrochloride**

**Step A: Synthesis of 3,5-di-tert-butyl-*N*-[*cis*-4-(4-dimethylamino-pyrimidin-2-ylamino)-cyclohexyl]-4-hydroxy-benzamide hydrochloride.**

Using the procedure for the step A of example 3198, the title compound was obtained.
ESI MS m/e 490, M (free) + $Na^+$;$^1H$ NMR (300 MHz, $CDCl_3$) δ 1.47 (s, 18 H), 1.63-2.13 (m, 8 H), 3.17 (s, 3 H), 3.28 (s, 3 H), 4.05-4.27 (m, 2 H), 5.52 (s, 1 H), 5.90-6.02 (m, 1 H), 6.57-6.73 (m, 1 H), 7.41-7.55 (m, 1 H), 7.63 (s, 2 H), 8.60-8.77 (m, 1 H), 13.00-13.24 (m, 1 H).

**Example 3218**

**1-(2,3-Dichloro-phenyl)-3-[*cis*-4-(4-dimethylamino-pyrimidin-2-ylamino)-cyclohexyl]-urea hydrochloride**

**Step A: Synthesis of 1-(2,3-dichloro-phenyl)-3-[*cis*-4-(4-dimethylamino-pyrimidin-2-ylamino)-cyclohexyl]-urea hydrochloride.**

To a solution of $N^2$-(*cis*-4-amino-cyclohexyl)-$N^4$,$N^4$-dimethyl-pyrimidine-2,4-diamine in step C of example 3129 (300 mg) in DMSO (3 mL) was added 1,2-dichloro-3-isocyanato-benzene (264 mg). The mixture was stirred at ambient temperature for 12 hr and poured into water. The precipitate was filtrated, washed with water, and purified by medium-pressure liquid chromatography (NH-silica gel, 25% to 50% EtOAc in hexane). To a solution of the above material in EtOAc (2 mL) was added 4 M hydrogen chloride in EtOAc (10 mL). The mixture was stirred at ambient temperature for 1 hr and concentrated under reduced pressure. A suspension of the residue in $Et_2O$ (20 mL) was stirred at ambient tempareture for 1 hr. The precipitate was collected by filtration, washed with $Et_2O$, and dried under reduced pressure to give 1-(2,3-dichloro-phenyl)-3-[*cis*-4-(4-dimethylamino-pyrimidin-2-ylamino)-cyclohexyl]-urea hydrochloride (421 mg) as a white solid.
ESI MS m/e 445, M (free) + $Na^+$; $^1H$ NMR (200 MHz, $CDCl_3$) δ 1.63-2.19 (m, 8 H), 3.15 (s, 3 H), 3.25 (s, 3 H), 3.80-4.22 (m, 2 H), 5.94 (d, *J* = 7.4 Hz, 1 H), 7.00-7.19 (m, 2 H), 7.43-7.64 (m, 2 H), 8.16 (dd, *J* = 8.3, 1.7 Hz, 1 H), 8.37-8.52 (m, 1 H), 12.70-13.00 (m, 1 H).

**Example 3219**

***N*-[*cis*-4-(4-Dimethylamino-pyrimidin-2-ylamino)-cyclohexylmethyl]-3,4-difluoro-benzamide hydrochloride**

**Step A: Synthesis of *N-[cis*-4-(4-dimethylamino-pyrimidin-2-ylamino)-cyclohexylmethyl]-3,4-difluoro-benzamide hydrochloride.**

To a solution of $N^2$-(*cis*-4-aminomethyl-cyclohexyl)-$N^4$,$N^4$-dimethyl-pyrimidine-2,4-diamine in step B of example 3145 (300 mg) in $CHCl_3$ (3 mL) were added $iPr_2NEt$ (0.59 mL) and 3,4-difluoro-benzoyl chloride (233 mg). The mixture was stirred at ambient temperature for 17 hr. The reaction was quenched with saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (NH-silica gel,

33 % EtOAc in hexane). To a solution of the above material in EtOAc (2 mL) was added 4 M hydrogen chloride in EtOAc (10 mL). The mixture was stirred at ambient temperature for 1 hr and concentrated under reduced pressure. A suspension of the residue in $Et_2O$ (20 mL) was stirred at ambient tempareture for 1 hr. The precipitate was collected by filtration, washed with $Et_2O$, and dried under reduced pressure to give *N*-[*cis*-4-(4-dimethylamino-pyrimidin-2-ylamino)-cyclohexylmethyl]- 3,4-difluoro-benzamide hydrochloride (155 mg) as a white solid.
ESI MS m/e 412, M (free) + $Na^+$; $^1H$ NMR (200 MHz, $CDCl_3$) δ 1.26-2.03 (m, 9 H), 3.16 (s, 3 H), 3.26 (s, 3 H), 3.37-3.61 (m, 2 H), 4.18-4.35 (m, 1 H), 5.94 (d, *J* = 7.4 Hz, 1 H), 6.82-7.33 (m, 2 H), 7.46 (d, *J* = 7.4 Hz, 1 H), 7.74-8.07 (m, 2 H), 8.83-9.12 (m, 1 H).

**Example 3220**

***N*-[*cis*-4-(4-Dimethylamino-pyrimidin-2-ylamino)-cyclohexylmethyl]-2-(2,3,6-trichloro-phenyl)-acetamide hydrochloride**

**Step A: Synthesis of *N*-[*cis*-4-(4-dimethylamino-pyrimidin-2-ylamino)-cyclohexylmethyl]-2-(2,3,6-trichloro-phenyl)-acetamide hydrochloride.**

Using the procedure for the step C of example 3118, the title compound was obtained.
ESI MS m/e 492, M (free) + $Na^+$; $^1H$ NMR (300 MHz, $CDCl_3$) δ 1.57-1.98 (m, 9 H), 3.16 (s, 3 H), 3.21-3.33 (m, 4 H), 4.16 (s, 2 H), 4.20-4.34 (m, 1 H), 5.95-5.99 (m, 1 H), 6.51-6.64 (m, 1 H), 7.23-7.51 (m, 3 H), 8.75-8.83 (m, 1 H), 12.80-12.95 (m, 1 H).

**Example 3221**

**9*H*-Xanthene-9-carboxylic acid [*cis*-4-(4-dimethylamino-pyrimidin-2-ylamino)-cyclohexylmethyl]-amide hydrochloride**

**Step A: Synthesis of 9*H*-xanthene-9-carboxylic acid [*cis*-4-(4-dimethylamino-pyrimidin-2-ylamino)-cyclohexylmethyl]-amide hydrochloride.**

Using the procedure for the step C of example 3118, the title compound was obtained.
ESI MS m/e 480, M (free) + $Na^+$; $^1H$ NMR (300 MHz, $CDCl_3$) δ 1.27-1.94 (m, 9 H), 3.05-3.19 (m, 5 H), 3.24 (s, 3 H), 4.14-4.28 (m, 1 H), 5.10 (s, 1 H), 5.91 (d, *J* = 7.4 Hz, 1 H), 6.19-6.33 (m, 1 H), 6.98-7.18 (m, 3 H), 7.20-7.31 (m, 2 H), 7.37-7.54 (m, 3 H), 8.62-8.82 (m, 1 H) 12.88-13.08 (m, 1 H).

**Example 3222**

**1-(2,3-Dichloro-phenyl)-3-[*cis*-4-(4-dimethylamino-pyrimidin-2-ylamino)-cyclohexylmethyl]-urea hydrochloride**

**Step A: Synthesis of 1-(2,3-dichloro-phenyl)-3-[*cis*-4-(4-dimethylamino-pyrimidin-2-ylamino)-cyclohexylmethyl]-urea hydrochloride.**

To a solution of $N^2$-(*cis*-4-aminomethyl-cyclohexyl)-$N^4$,$N^4$-dimethyl-pyrimidine-2,4-diamine in step B of example 3145 (300 mg) in DMSO (3 mL) was added 1,2-dichloro-3-isocyanato-benzene (249 mg). The mixture was stirred at ambient temperature for 15 hr and poured into water (20 mL). The precipitate was filtrated, washed with water, and purified by medium-pressure liquid chromatography (NH-silica gel, 25% to 50% EtOAc in hexane). To a solution of the above material in EtOAc (2 mL) was added 4 M hydrogen chloride in EtOAc (10 mL). The mixture was stirred at ambient temperature for 1 hr and concentrated under reduced pressure. A suspension of the residue in $Et_2O$ (20 mL) was stirred at ambient tempareture for 1 hr. The precipitate was collected by filtration, washed with $Et_2O$, and dried under reduced pressure to 1-(2,3-dichloro-phenyl)-3-[*cis*-4-(4-dimethylamino-pyrimidin-2-ylamino)-cyclohexylmethyl]-urea hydrochloride (260 mg) as a white solid.
ESI MS m/e 437, $M^+$; $^1H$ NMR (200 MHz, $CDCl_3$) δ 1.35-2.10 (m, 9 H), 3.16 (s, 3 H), 3.26 (s, 3 H), 3.32-3.47 (m, 2 H), 4.27-4.47 (m, 1 H), 5.96 (d, *J* = 7.5 Hz, 1 H), 6.80-7.20 (m, 3 H), 7.47 (d, *J* = 7.5 Hz, 1 H), 8.08-8.37 (m, 2 H), 8.63-8.93 (m, 1 H).

**Example 3223**

**3,4-Difluoro-*N*-[*cis*-4-(4-methylamino-pyrimidin-2-ylamino)-cyclohexyl]-benzamide-hydrochloride**

**Step A: Synthesis of (2-chloro-pyrimidin-4-yl)-methyl-amine.**

To a solution of 2,4-dichloro-pyrimidine (15.0 g) in THF (150 mL) was added 40% aqueous $MeNH_2$ (19.5 g). The mixture was stirred at ambient temperature for 1.5 hr. The solution was poured into saturated aqueous $NaHCO_3$ and the aqueous layer was extracted with $CHCl_3$ (three times). The combined organic layer was dried over $MgSO_4$, filtrated, concentrated, and purified by flash chromatography (NH-silica, 20% EtOAc in hexane) to give (2-chloro-pyrimidin-4-yl)-methyl-amine (10.0 g) as a white solid and (4-chloro-pyrimidin-2-yl)-methyl-amine (0.87 g, 6%) as a white solid. (2-chloro-pyrimidin-4-yl)-methyl-amine;
ESI MS m/e 143, $M^+$; $^1H$ NMR (300 MHz, $CDCl_3$) δ 3.01 (d, *J* = 5.0 Hz, 3 H), 5.58-5.96 (m, 1 H), 6.55 (d, J = 5.1 Hz, 1 H), 8.09-8.23 (m, 1 H).
(4-chloro-pyrimidin-2-yl)-methyl-amine;
ESI MS m/e 143, $M^+$; $^1H$ NMR (300 MHz, $CDCl_3$) δ 2.98 (d, *J* = 5.0 Hz, 3 H), 6.27 (d, *J* = 6.1 Hz, 1 H), 7.93-8.20 (m, 1 H).

**Step B: Synthesis of [*cis*-4-(4-methylamino-pyrimidin-2-ylamino)-cyclohexyl] carbamic acid tert-butyl ester.**

A mixture of (2-chloro-pyrimidin-4-yl)-methyl-amine (2.50 g) and (*cis*-4-amino-cyclohexyl)-carbamic acid *tert*-butyl ester obtained in step B of example 1 (4.10 g) in BuOH (2.50 mL) was stirred at reflux for 24 hr. The reaction mixture was poured into saturated aqueous $NaHCO_3$, and the aqueous layer was extracted with $CHCl_3$ (three times). The combined organic layer was dried over $MgSO_4$, filtrated, concentrated under reduced pressure, and purified by medium-pressure liquid chromatography (NH-silica, 25% to 66% EtOAc in hexane) to give [*cis*-4-(4-methylamino-pyrimidin-2-ylamino)-cyclohexyl]-carbamic acid tert-butyl ester (2.63 g) as a white solid.
ESI MS m/e 344, M + $Na^+$; $^1H$ NMR (300 MHz, $CDCl_3$) δ 1.36-1.88 (m, 17 H), 2.89 (d, *J* = 5.1 Hz, 3 H), 3.53-3.69 (m, 1 H), 3.84-4.04 (m, 1 H), 4.44-4.70 (m, 2 H), 4.76-4.86 (m, 1 H), 5.69-5.72 (m, = 1 H), 7.80-7.91 (m, 1 H).

**Step C: Synthesis of *N*$^2$-(*cis*-4-amino-cyclohexyl)-*N*$^4$-methyl-pyrimidine-2,4-diamine.**

A solution of [*cis*-4-(4-methylamino-pyrimidin-2-ylamino)-cyclohexyl]-carbamic acid tert-butyl ester (4.76 g) in EtOAc (48 mL) was cooled on an ice-bath and 4 M hydrogen chloride in EtOAc (24 mL) was added. The mixture was stirred at ambient temperature for 4 hr and concentrated under reduced pressure. The residue was dissolved in 1 M aqueous NaOH and the aqueous layer was extracted with $CHCl_3$ (five times). The combined organic layer was dried over $MgSO_4$, filtered, concentrated under reduced pressure, and dried under reduced pressure to give *N*$^2$-(*cis*-4-amino-cyclohexyl)-*N*$^4$-methyl-pyrimidine-2,4-diamine (3.00 g, 80%) as a white solid.
ESI MS m/e 222, M + $H^+$; $^1H$ NMR (300 MHz, $CDCl_3$) δ 0.95-1.92 (m, 10 H), 2.78-2.99 (m, 4 H), 3.92-4.08 (m, 1 H), 4.56-4.75 (m, 1 H), 4.84-4.97 (m, 1 H), 5.68 (d, *J* = 5.9 Hz, 1 H), 7.85 (d, *J* = 5.7 Hz, 1 H).

**Step D: Synthesis of 3,4-difluoro-N-[*cis*-4-(4-methylamino-pyrimidin-2-ylamino)-cyclohexyl]-benzamide hydrochloride.**

To a solution of 3,4-difluoro-benzoic acid (196 mg) and *N*$^2$-(*cis*-4-amino-cyclohexyl)-*N*$^4$-methyl-pyrimidine-2,4-diamine (250 mg) in DMF (4 mL) were added $Et_3N$ (0.38 mL), HOBt-$H_2O$ (259 mg), and EDC-HCl (238 mg). The reaction mixture was stirred at ambient temperature for 12 hr. To the mixture was added water (20 mL) and the aqueous layer was extracted with $CHCl_3$ (three times). The combined organic layer was dried over $MgSO_4$, filtered, concentrated under reduced pressure, and purified by medium-pressure liquid chromatography (NH-silica gel, 33 % to 75% EtOAc in hexane). To a solution of the above material in EtOAc (10 mL) was added 4 M hydrogen chloride in EtOAc (0.2 mL). The mixture was stirred at ambient temperature for 1 hr. The precipitate was collected by filtration, washed with EtOAc, and dried under reduced pressure to give 3,4-difluoro-*N*-[*cis*-4-(4-methylamino-pyhmidin-2-ylamjno)-cyclohexyl]-benzamide hydrochloride (317 mg) as a white solid.
ESI MS m/e 362, M (free) + $H^+$; 1 H NMR (300 MHz, DMSO-$d_6$) δ 1.59-1.90 (m, 8 H), 2.89 (d, *J* = 4.6 Hz, 3 H), 3.80-4.11 (m, 2 H), 6.03-6.13 (m, 1 H), 7.47-8.03 (m, 4 H), 8.27-8.49 (m, 2 H), 8.82-9.06 (m, 1 H), 11.92-12.11 (m, 1 H).

**Example 3224**

**3-Chloro-4-fluoro-*N*-[*cis*-4-(4-methylamino-pyrimidin-2-ylamino)-cyclohexyl]-benzamide hydrochloride**

**Step A: Synthesis of 3-chloro-4-fluoro-*N*-(*cis*-4-(4-methylamino-pyrimidin-2-ylamino)-cyclohexyl]-benzamide hydrochloride.**

Using the procedure for the step C of example 3223, the title compound was obtained.
ESI MS m/e 378, M (free) + $H^+$; $^1H$ NMR (300 MHz, DMSO-$d_6$) δ 1.59-1.90 (m, 8 H), 2.89 (d, *J* = 4.6 Hz, 3 H), 3.77-4.10 (m, 2 H), 6.00-6.12 (m, 1 H), 7.49-7.60 (m, 1 H), 7.67-7.76 (m, 1 H), 7.85-7.94 (m, 1 H), 8.11 (dd, *J* = 7.1, 2.2 Hz, 1 H), 8.24-8.51 (m, 2 H), 8.82-8.94 (m, 1 H), 11.80-11.98 (m, 1 H).

**Example 3225**

***N*-[*cis*-4-(4-Ethylamino-pyrimidin-2-ylamino)-cyclohexyl]-3,4-difluoro-benzamide hydrochloride**

**Step A: Synthesis of (2-chloro-pyrimidin-4-yl)-ethyl-amine.**

To the solution of 2,4-dichloro-pyrimidine (5.00 g) in THF (50 mL) was added 70% aqueous $EtNH_2$ (5.40 g). The mixture was stirred at ambient temperature for 1 hr. To the residue was added saturated aqueous $NaHCO_3$ and the aqueous layer was extracted with $CHCl_3$ (two times). The combined organic layer was dried over $MgSO_4$, filtered, concentrated under reduced pressure, and purified flash chromatography (silica gel, 17% to 50% EtOAc in hexane) to give (2-chloro-pyrimidin-4-yl)-ethyl-amine (3.69 g) as a white solid and (4-chloro-pyrimidin-2-yl)-ethyl-amine (1.28 g) as a white solid.
(2-chloro-pyrimidin-4-yl)-ethyl-amine;
ESI MS m/e 157, $M^+$;$^1H$ NMR (500 MHz, $CDCl_3$) δ 1.26 (t, *J* = 7.3 Hz, 3 H), 3.16-3.62 (m, 2 H), 4.80-5.95 (m, 1 H), 6.23 (d, *J* = 5.8 Hz, 1 H), 8.02-8.22 (m, 1 H).
(4-chloro-pyrimidin-2-yl)-ethyl-amine;
CI MS m/e 158, M + $H^+$;$^1H$ NMR (500 MHz, $CDCl_3$) δ 1.23 (t, *J* = 7.5 Hz, 3 H), 3.42-3.49 (m, 2 H), 5.30-5.62 (m, 1 H), 6.54 (d, *J* = 5.2 Hz, 1 H), 8.02-8.22 (m, 1 H).

**Step B: Synthesis of *N*-[*cis*-4-(4-ethylamino-pyrimidin-2-ylamino)-cyclohexyl]-3,4-difluoro-benzamide hydrochloride.**

To a solution of *N-(cis*-4-amino-cyclohexyl)-3,4-difluoro-benzamide obtained in step D of example 3031 (300 mg) in BuOH (1 mL) was added (2-chloro-pyrimidin-4-yl)-ethyl-amine (532 mg). The mixture was heated in a microwave synthesizer at 200°C for 30 min. To the mixture was added saturated aqueous $NaHCO_3$ and the aqueous layer was extracted with $CHCl_3$ (three times). The combined organic layer was dried over $MgSO_4$, filtrated, concentrated under reduced pressure, and purified by medium-pressure liquid chromatography (NH-silica gel, 20% to 50% in EtOAc in nexane). To a solution of the above material in EtOAc (10.0 mL) was added 4 M hydrogen chloride in EtOAc (5.00 mL). The mixture was stirred at ambient temperature for 1 hr and concentrated under reduced pressure. A suspension of the above material in $Et_2O$ (20 mL) was stirred at ambient tempareture for 4 hr. The precipitate was collected by filtration, washed with $Et_2O$, and dried under reduced pressure to give *N*-[*cis*-4-(4-ethylamino-pyrimidin-2-ylamino)-cyclohexyl]-3,4-difluoro-benzamide hydrochloride (398 mg) as a white solid.
ESI MS m/e 398, M (free) + $Na^+$; $^1H$ NMR (500 MHz, $CDCl_3$) δ 1.19-1.42 (m, 3 H), 1.61-2.05 (m, 8 H), 3.46-3.65 (m, 2 H), 4.00-4.34 (m, 2 H), 5.85-6.00 (m, 1 H), 6.42-6.72 (m, 2 H), 7.11-7.37 (m, 2 H), 7.52-7.82 (m, 2 H), 8.68-8.90 (m, 1 H).

**Example 3226**

**N-{*cis*-4-[4-(Ethyl-methyl-amino)-pyrimidin-2-ylamino]-cyclohexyl}-3,4-difluoro benzamide hydrochloride**

**Step A: Synthesis of (2-chloro-pyrimidin-4-yl)-ethyl-methyl-amine.**

To the solution of 2,4-dichloro-pyrimidine (5.00 g) in THF (50 mL) was added ethyl-methyl-amine (2.08 g). The mixture was stirred at ambient temperature for 1 hr. To the residue was added saturated aqueous $NaHCO_3$ and the aqueous layer was extracted with $CHCl_3$ (two times). The combined organic layer was dried over $MgSO_4$, filtered, concentrated under reduced pressure, and purified flash chromatography (silica gel, 17% to 50% EtOAc in hexane) to give (2-chloro-pyrimidin-4-yl)-ethyl-methyl-amine (4.49 g) as a white solid and (4-chloro-pyrimidin-2-yl)-ethyl-methyl-amine (0.91 g) as a colorless oil.
(2-chloro-pyrimidin-4-yl)-ethyl-methyl-amine;
CI MS m/e 172, M (free) + H+; $^1$H NMR (500 MHz, $CDCl_3$) δ 1.18 (t, *J* = 3.0 Hz, 3H), 3.06 (brs, 3 H), 3.35-3.70 (m, 2 H), 6.29 (d, *J* = 4.8 Hz, 1 H), 7.99(d, *J* = 6.1 Hz, 1 H).
(4-chloro-pyrimidin-2-yl)-ethyl-methyl-amine;
CI MS m/e 172, M + H+; $^1$H NMR (500 MHz, $CDCl_3$) δ 1.17 (t, *J* = 3.0 Hz, 3 H), 3.10 (s, 3 H), 3.66 (q, *J* = 7.0 Hz, 2 H), 6.45 (d, *J* = 5.0 Hz, 1 H), 8.14 (d, *J* = 5.0 Hz, 1 H).

**Step B: Synthesis of *N*-{*cis*-4-[4-(ethyl-methyl-amino)-pyrimidin-2-ylamino)]-cyclohexyl}-3,4-difluoro-benzamide hydrochloride.**

Using the procedure for the step B of example 3225, the title compound was obtained.
ESI MS m/e 412, M (free) + Na+; $^1$H NMR (300 MHz, $CDCl_3$) δ 1.18-1.33 (m, 3 H), 1.64-2.03 (m, 8 H), 3.13-3.32 (m, 3 H), 3.44-3.56 (m, 1 H), 3.67-3.82 (m, 1 H), 4.04-4.31 (m, 2 H), 5.90-6.00 (m, 1 H), 6.59-6.72 (m, 1 H), 7.14-7.27 (m, 1 H), 7.43-7.62 (m, 2 H), 7.68-7.79 (m, 1 H), 8.71-8.83 (m, 1 H).

**Example 3227**

**3,4-Difluoro-*N*-(*cis*-4-{4-[(2-hydroxy-ethyl)-methyl-amino]-pyrimidin-2-ylamino}-cyclohexyl)-benzamide hydrochloride**

**Step A: Synthesis of [(2-chloro-pyrimidin-4-yl)-methyl-amino]-ethanol.**

To the solution of 2,4-dichloro-pyrimidine (5.00 g) in THF (50 mL) was added 2-methylamino-ethanol (2.65 g). The mixture was stirred at ambient temperature for 1hr. To the residue was added saturated aqueous $NaHCO_3$ and the aqueous layer was extracted with $CHCl_3$ (two times). The combined organic layer was dried over $MgSO_4$, filtered, concentrated under reduced pressure, and purified flash chromatography (silica gel, 17% to 50% EtOAc in hexane) to give [(2-chloro-pyrimidin-4-yl)-methyl-amino]-ethanol (3.50 g) as a white solid and [(4-chloro-pyrimidin-2-yl)-methyl-amino]-ethanol (827 mg) as a white solid.
[(2-chloro-pyrimidin-4-yl)-methyl-amino]-ethanol;
ESI MS m/e 188, M (free) + H+; $^1$H NMR (500 MHz, $CDCl_3$) δ 2.91 (brs, 3 H), 3.13 (s, 3 H), 3.64-3.92 (m, 4 H), 6.46-6.49 (m, 1 H), 7.99 (d, *J* = 6.1 Hz, 1 H).
[(4-chloro-pynmidin-2-yl)-methyl-amino]-ethanol
ESI MS m/e 210, M + Na+; $^1$H NMR (500 MHz, $CDCl_3$) δ 3.23 (s, 3 H), 3.76-3.92 (m, 4 H), 6.52 (d, *J* =5.2 Hz, 1 H), 8.12 (d, *J* = 4.6 Hz, 1 H).

**Step B: Synthesis of 3,4-difluoro-*N*-(*cis*-4-{4-[(2-hydroxy-ethyl)-methyl-amino]-pyrimidin-2-ylamino}-cyclohexyl)-benzamide hydrochloride.**

Using the procedure for the step B of example 3225, the title compound was obtained.
ESI MS m/e 428, M (free) + Na+; $^1$H NMR (300 MHz, DMSO-$d_6$) δ 1.61-1.98 (m, 8 H), 3.13-3.25 (m, 3 H), 3.54-4.31 (m, 5 H), 4.76-5.02 (m, 1 H), 6.26-6.52 (m, 1 H), 7.48-7.62 (m, 1 H), 7.68-8.17 (m, 4 H), 8.28-8.47 (m, 1 H), 11.74-11.95 (m, 1 H).

**Example 3228**

**3-Chloro-*N*-[*cis*-4-(4-dimethylamino-5-methyl-pyrimidin-2-ylamino)-cyclohexyl]-4-fluoro-benzamide hydrochloride**

**Step A: Synthesis of *N*-(*cis*-4-amino-cyclohexyl)-3-chloro-4-fluoro-benzamide.**

To a solution of 3-chloro-4-fluoro-benzoic acid (26.9 g) and (cis-4-amino-cyclohexyl)-carbamic acid *tert*-butyl ester (30.0 g) in DMF (300 mL) were added $Et_3N$ (46.8 mL), HOBt-$H_2O$ (32.2 g), and EDC-HCl (29.5 g). The reaction mixture was stirred at ambient temperature for 20 hr. To the mixture was added water (1.20 L) and the aqueous layer was extracted with $CHCl_3$ (three times). The combined organic layer was dried over $MgSO_4$, filtered, and concentrated under reduced pressure. A solution of the above material in EtOAc (650 mL) was cooled on an ice-bath and 4 M hydrogen chloride in EtOAc (325 mL) was added. The mixture was stirred at ambient temperature for 16 hr and concentrated under reduced pressure. The residue was dissolved in 1 M aqueous NaOH (300 mL) and the aqueous layer

was extracted with $CHCl_3$ (three time). The combined organic layer was dried over $MgSO_4$, filtered, concentrated under reduced pressure, and dried under reduced pressure to give *N*-(*cis*-4-amino-cyclohexyl)-3-chloro-4-fluoro-benzamide (44.4 g) as a brown solid.
ESI MS m/e 271, M (free) + $H^+$; $^1H$ NMR (300 MHz, $CDCl_3$) δ 1.37-1.92 (m, 8 H), 2.94-3.08 (m, 1 H), 4.06-4.22 (m, 1 H), 6.13-6.31 (m, 1 H), 7.19 (t, *J* = 8.5 Hz, 1 H), 7.61-7.70 (m, 1 H), 7.79-7.87 (m, 1 H).

**Step B: Synthesis of 3-chloro-*N*-[*cis*-4-(4-dimethylamino-5-methyl-pyrimidin-2-ylamino)-cyclohexyl]-4-fluoro-benzamide hydrochloride.**

To a solution of *N*-(*cis*-4-amino-eyclohexyl)-3-chloro-4-fluoro-benzamide (432 mg) in BuOH (1 mL) was added 2-chloro-4-dimethylamino-5-methylpyrimidine obtained in step A of example 3119 (250 mg). The mixture was heated in a microwave synthesizer at 200°C for 10 min. To the mixture was added saturated aqueous $NaHCO_3$ and the aqueous layer was extracted with $CHCl_3$ (three times). The combined organic layer was dried over $MgSO_4$, filtrated, concentrated under reduced pressure, and purified by medium-pressure liquid chromatography (NH-silica gel, 20% to 50% EtOAc in hexane) to give a pale yellow oil. To a solution of the above material in EtOAc (10 mL) was added 4 M hydrogen chloride in EtOAc (0.2 mL). The mixture was stirred at ambient temperature for 1 hr and concentrated under reduced pressure. A suspension of the above material in $Et_2O$ (20 mL) was stirred at ambient tempareture for 4 hr. The precipitate was collected by filtration, washed with $Et_2O$, and dried under reduced pressure to give 3-chloro-*N*-[*cis*-4-(4-dimethyl-amino-5-methyl-pyrimidin-2-ylamino)-cyclohexyl]-4-fluoro-benzamide hydrochloride (174 mg) as a white solid.
ESI MS m/e 406, M (free) + $H^+$; $^1H$ NMR (300 MHz, $CDCl_3$) δ 1.61-2.02 (m, 8 H), 2.25 (s, 3 H), 3.30 (s, 6 H), 4.02-4.26 (m, 2 H), 6.81-6.93 (m, 1 H), 7.13-7.27 (m, 2 H), 7.70-7.78 (m, 1 H), 7.93-8.00 (m, 1 H), 8.50-8.63 (m, 1 H), 12.68-12.85 (m, 1 H).

**Example 3229**

**3-Chloro-*N*-[*cis*-4-(4-dimethylamino-5-fluoro-pyrimidin-2-ylamino)-cyclohexyl]-4-fluoro-benzamide hydrochloride**

**Step A: Synthesis of 3-chloro-*N*-[*cis*-4-(4-dimethylamino-5-fluoro-pyrimidin-2-ylamino)-cyclohexyl]-4-fluoro-benzamide hydrochloride.**

Using the procedure for the step B of example 3228, the title compound was obtained.
ESI MS m/e 410, M (free) + $H^+$; $^1H$ NMR (300 MHz, $CDCl_3$) δ 1.64-2.03 (m, 8 H), 3.36 (s, 6 H), 4.00-4.23 (m, 2 H), 6.73-6.84 (m, 1 H), 7.18 (t, *J* = 8.6 Hz, 1 H), 7.45 (d, *J* = 7.6 Hz, 1 H), 7.67-7.76 (m, 1 H), 7.95 (dd, *J* = 7.0, 2.2 Hz, 1 H), 8.64-8.78 (m, 1 H).

**Example 3230**

**3-Chloro-*N*-[*cis*-4-(4-dimethylamino-6-methyl-pyrimidin-2-ylamino)-cyclohexyl]-4-fluoro-benzamide hydrochloride**

**Step A: Synthesis of 3-chloro-*N*-[*cis*-4-(4-dimethylamino-6-methyl-pyrimidin-2-ylamino)-cyclohexyl]-4-fluoro-benzamide hydrochloride.**

Using the procedure for the step B of example 3228, the title compound was obtained.
ESI MS m/e 406, M (free) + $H^+$; $^1H$ NMR (300 MHz, $CDCl_3$) δ 1.62-2.04 (m, 8 H), 2.36 (s, 3 H), 3.15 (s, 3 H), 3.27 (s, 3 H), 4.01-4.31 (m, 2 H), 5.76 (s, 1 H), 6.73-6.84 (m, 1 H), 7.19 (t, *J* = 8.6 Hz, 1 H), 7.68-7.79 (m, 1 H), 7.97 (dd, *J* = 6.9, 2.2 Hz, 1 H), 8.50-8.63 (m, 1 H), 12.94-13.16 (m, 1 H).

**Example 3231**

***N*-[*cis*-4-(4-Dimethylamino-6-ethyl-pyrimidin-2-ylamino)-cyclohexyl]-3,4-difluoro-benzamide hydrochloride**

**Step A: Synthesis of (2,6-Dichloro-pyrimidin-4-yl)-dimethyl-amine.**

To the solution of 2,4,6-trichloro-pyrimidine (10.0 g) in THF (50 mL) were added 50% aqueous $Me_2NH$ (4.92 g) and $iPr_2NEt$ (8.46 g). The mixture was stirred at ambient temperature for 1.5 hr and concentrated under reduced pressure. To the residue was added saturated aqueous $NaHCO_3$ and the aqueous layer was extracted with $CHCl_3$

(three times). The combined organic layer was dried over $MgSO_4$, filtered, concentrated under reduced pressure, and purified flash chromatography (NH-silica gel, 3% EtOAc in hexane) to give (2,6-dichloro-pyrimidin-4-yl)-dimethyl-amine (6.03 g) as white solid.
ESI MS m/e 192, $M^+$; $^1$H NMR (300 MHz, $CDCl_3$) δ 2.77-3.46 (m, 6 H), 6.34 (s, 1 H).

**Step B: Synthesis of (2-chloro-6-ethyl-pyrimidin-4-yl)-dimethyl-amine.**

A solution of $ZnBr_2$ (3.87 g) in THF (60 mL) was cooled to -60°C and 1 M EtMgBr in THF (17.2 mL) was added. The mixture was stirred at -60°C for 1 hr and warmed to ambient temperature. To the mixture was added (2,6-dichloro-pyrimidin-4-yl)-dimethyl-amine in THF (60 mL) and stirred at reflux for 5 days. To the mixture was added saturated aqueous $NH_4Cl$ and the aqueous layer was extracted with $CHCl_3$ (three times). The combined organic layer was dried over $MgSO_4$, filtered, concentrated under reduced pressure, and purified by medium-pressure liquid chromatography (silica gel, 17% to 33% EtOAc in hexane) to give (2-chloro-6-ethyl-pyrimidin-4-yl)-dimethyl-amine (352 mg) as pale yellow solid and (6-chloro-2-ethyl-pyrimidin-4-yl)-dimethyl-amine (622 mg) as pale yellow solid.
(2-chloro-6-ethyl-pyrimidin-4-yl)-dimethyl-amine;
ESI MS m/e 208, M (free) + $Na^+$; $^1$H NMR (300 MHz, $CDCl_3$) δ 1.25 (t, *J* = 7.6 Hz, 3 H), 2.54-2.66 (m, 2 H), 3.11 (s, 6 H), 6.15 (s, 1 H).
(6-chloro-2-ethyl-pyrimidin-4-yl)-dimethyl-amine;
ESI MS m/e 186, M + $H^+$; $^1$H NMR (300 MHz, $CDCl_3$) δ 1.29 (t, *J* = 7.6 Hz, 3 H), 2.74 (q, *J* = 7.7 Hz, 2 H), 3.10 (s, 6 H), 6.24 (s, 1 H).

**StepC: Synthesis of *N*-[*cis*-4-(4-dimethylamino-6-ethyl-pyrimidin-2-ylamino)-cyclohexyl]-3,4-difluoro-benzamide hydrochloride**

Using the procedure for the step B of example 3225, the title compound was obtained.
ESI MS m/e 426, M (free) + $Na^+$; $^1$H NMR (300 MHz, $CDCl_3$) δ 1.29-1.44 (m, 3 H), 1.58-2.19 (m, 8 H), 2.54-2.77 (m, 2 H), 3.15 (s, 3 H), 3.26 (s, 3 H), 3.98-4.34 (m, 2 H), 5.74 (s, 1 H), 6.41-6.63 (m, 1 H), 7.08-7.32 (m, 1 H), 7.46-7.81 (m, 2 H), 8.58-8.81 (m, 1 H), 12.83-13.09 (m, 1 H).

**Example 3232**

***N*-[*cis*-4-(4,6-Bis-dimethylamino-pyrimidin-2-ylamino)-cyclohexyl]-3,4-difluoro-benzamide hydrochloride**

**Step A: Synthesis of 2-chloro-*N,N,N',N'*-tetramethyl-pyrimidine-4,6-diamine.**

To the solution of (2,6-dichloro-pyrimidin-4-yl)-dimethyl-amine obtained in step A of example 3231 (1.60 g) in THF (2 mL) was added 50% aqueous $Me_2NH$ (789 mg). The mixture was stirred at reflux for 3.5 hr in a sealed tube. To the residue was added saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (silica gel, 20% EtOAc in hexane) to give 2-chloro-*N,N,N',N'*-tetramethyl-pyrimidine-4,6-diamine (203 mg) as a pale brown solid and 6-chloro-*N,N,N',N'*-tetramethyl-pyrimidine-2,4-diamine (1.43 g) as a pale yellow solid.
2-chloro-*N,N,N',N'*-tetramethyl-pyrimidine-4,6-diamine;
ESI MS m/e 201, M (free) + $H^+$; $^1$H NMR (300 MHz, $CDCl_3$) δ 3.05 (s, 12 H), 5.15 (s, 1 H).
6-chloro-*N,N,N',N'*-tetramethyl-pyrimidine-2,4-diamine;
ESI MS m/e 201, M + $H^+$; $^1$H NMR (300 MHz, $CDCl_3$) δ 3.04 (s, 6 H), 3.13 (s, 6 H), 5.76 (s, 1 H).

**Step B: Synthesis of *N*-[*cis*-4-(4,6-bis-dimethylamino-pyrimidin-2-ylamino)-cyclohexyl]-3,4-difluoro-benzamide hydrochloride.**

Using the procedure for the step B of example 3225, the title compound was obtained.
ESI MS m/e 441, M (free) + $Na^+$; $^1$H NMR (300 MHz, $CDCl_3$) δ 1.61-2.09 (m, 8 H), 2.96-3.38 (m, 12 H), 4.00-4.31 (m, 2 H), 4.73 (s, 1 H), 6.65-6.82 (m, 1 H), 7.13-7.25 (m, 1 H), 7.55-7.63 (m, 1 H), 7.68-7.78 (m, 1 H), 8.70-8.82 (m, 1 H), 11.79-11.99 (m, 1 H).

**Example 3233**

***N*-[*cis*-4-(6-Chloro-4-dimethylamino-pyrimidin-2-ylamino)-cyclohexyl]-2-phenoxy-nicotinamide hydrochloride**

**Step A: Synthesis of *N*-[*cis*-4-(6-chloro-4-dimethylamino-pyrimidin-2-ylamino)-cyclohexyl]-2-phenoxy-nicotinamide hydrochloride.**

Using the procedure for the step B of example 3032, the title compound was obtained.
ESI MS m/e 489, M (free) $^+$ $Na^+$; $^1H$ NMR (300 MHz, $CDCl_3$) δ 1.52-2.10 (m, 8 H), 2.96-3.38 (m, 6 H), 4.02-4.29 (m, 2 H), 5.82-6.03 (m, 1 H), 7.04-7.55 (m, 6 H), 7.80-8.01 (m, 1 H), 8.15-8.28 (m, 1 H), 8.47-8.61 (m, 1 H).

**Example 3234**

***N*-[*cis*-4-(6-Chloro-4-dimethylamino-pyrimidin-2-ylamino)-cyclohexyl]-3,4-difluoro-benzamide hydrochloride**

**Step A: Synthesis of *N*-[*cis*-4-(6-chloro-4-dimethylamino-pyrimidin-2-ylamino)-cyclohexyl]-3,4-difluoro-benzamide hydrochloride.**

Using the procedure for the step B of example 3225, the title compound was obtained.
ESI MS m/e 432, M (free) + $Na^+$; $^1H$ NMR (300 MHz, $CDCl_3$) δ 1.63-2.05 (m, 8 H), 3.04-3.37 (m, 6 H), 4.02-4.37 (m, 2 H), 5.88-6.03 (m, 1 H), 6.56-6.86 (m, 1 H), 7.14-7.27 (m, 1 H), 7.51-7.63 (m, 1 H), 7.66-7.82 (m, 1 H), 8.85-9.02 (m, 1 H).

**Example 3235**

***N*-[*cis*-4-(4-Amino-quinolin-2-ylamino)-cyclohexyl]-3,4-difluoro-benzamide hydrochloride**

**Step A: Synthesis of 2-chloro-quinolin-4-ylamine.**

To the solution of 2,4-dichloro-quinoline obtained in step A of example 1 (4.00 g) in IPA (40 mL) was added 28% aqueous $NH_3$ (40.0 mL). The mixture was stirred at reflux for 10 days in a sealed tube. To the residue was added saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (silica gel, 9% to 17% EtOAc in hexane) to give 2-chloro-quinolin-4-ylamine (1.39 g) as a white solid and 4-chloro-quinolin-2-ylamine (1.17 g) as a white solid.
2-chloro-quinolin-4-ylamine;
ESI MS m/e 178, $M^+$; $^1H$ NMR (200 MHz, $CDCl_3$) δ 4.69-4.97 (m, 2 H), 6.61 (s, 1 H), 7.37-7.78 (m, 3 H), 7.84-8.02 (m, 1 H).
4-chloro-quinolin-2-ylamine
ESI MS m/e 178, $M^+$;$^1H$ NMR (300 MHz, $CDCl_3$) δ 4.58-4.96 (m, 2 H), 6.85 (s, 1 H), 7.23-7.41 (m, 1 H), 7.53-7.72 (m, 2 H), 7.98-8.09 (m, 1 H).

**Step B: Synthesis of *N*-[*cis*-4-(4-amino-quinolin-2-ylamino)-cyclohexyl]-3,4-difluoro-benzamide hydrochloride.**

Using the procedure for the step B of example 3225, the title compound was obtained.
ESI MS m/e 397, M (free) + $H^+$; $^1H$ NMR (300 MHz, $CDCl_3$) δ 1.29-2.15 (m, 8 H), 3.75-3.90 (m, 1 H) 4.05,4.26 (m, 1 H), 5.44-5.59 (m, 2 H), 5.89 (s, 1 H), 6.99-7.43 (m, 3 H), 7.55-7.84 (m, 5 H), 8.81-8.98 (m, 1 H).

**Example 3236**

**2-(*cis*-4-{[1-(3,4-Difluoro-phenyl)-methanoyl]-amino}-cyclohexylamino)-quinoline-4-carboxylic acid amide**

**Step A: Synthesis of 2-chloro-quinoline-4-carboxylic acid amide.**

To a solution of 2-chloro-quinoline-4-carboxylic acid (3.00 g) in DMF (30 mL) were added 28% aqueous $NH_3$ (1.05 g), $Et_3N$ (5.04 mL), HOBt-$H_2O$ (3.32 g), and EDC-HCl (3.32 g). The reaction mixture was stirred at ambient temperature for 16 hr. To the reaction mixture was added water (20 mL) and the aqueous layer extracted with $CHCl_3$ (three times). The combined organic layer was dried over $MgSO_4$, filtered, concentrated under reduced pressure,

purified by medium-pressure liquid chromatography (NH-silica gel, 20% to 50% EtOAc in hexane) to give 2-chloro-quinoline-4-carboxylic acid amide (1.77 g) as a white solid.
ESI MS m/e 207, M (free) + $H^+$; $^1H$ NMR (300 MHz, DMSO-$d_6$) δ 7.65 (s, 1 H), 7.68-7.77 (m, 1 H), 7.83-7.93 (m, 1 H), 7.98-8.09 (m, 2 H), 8.18-8.25 (m, 1 H), 8.30-8.40 (m, 1 H).

**Step B: Synthesis of 2-(*cis*-4-{[1-(3,4-difluoro-phenyl)-methanoyl]-amino)-cyclohexylamino}-quinoline-4-carboxylic acid amide.**

A mixture of 2-chloro-quinoline-4-carboxylic acid amide (300 mg) and *N*-(*cis*-4-amino-cyclohexyl)-3,4-difluoro-benzamide obtained in step A of example 3031 (406 mg) in butanol (1 mL) and DMSO (1 mL) was stirred at reflux for 24 hr. The reaction mixture was poured into saturated aqueous $NaHCO_3$ and the aqueous layer was extracted with $CHCl_3$ (three times). The combined organic layer was dried over $MgSO_4$, filtered, concentrated under reduced pressure, purified by medium-pressure liquid chromatography (NH-silica gel, EtOAc), and concentrated under reduced pressure. The above material was washed with and dried under reduced pressure to give 2-(*cis*-4-{[1-(3,4-difluoro-phenyl)-methanoyl]-amino}-cyclohexylamino)-quinoline-4-carboxylic acid amide (136 mg) as a white solid.
ESI MS m/e 447, M (free) + $Na^+$; $^1H$ NMR (300 MHz, DMSO-$d_6$) δ 1.61-2.03 (m, 8 H), 3.78-3.93 (m, 1 H), 4.05-4.20 (m, 1 H), 6.89 (s, 1 H), 6.99-7.07 (m, 1 H), 7.11-7.21 (m, 1 H), 7.42-7.61 (m, 3 H), 7.65-7.82 (m, 3 H), 7.88-7.99 (m, 1 H), 8.02-8.10 (m, 1 H), 8.28-8.36 (m, 1 H).

**Example 3237**

**3,4-Difluoro-*N*-[*cis*-4-(4-trifluoromethyl-quinolin-2-yl)-amino-cyclohexyl]-benzamide hydrochloride**

**Step A: Synthesis of 3,4-difluoro-*N*-[*cis*-4-(4-trifluoromethyl-quinolin-2-yl)-amino-cyclohexyl]-benzamide hydrochloride.**

Using the procedure for the step B of example 3225, the title compound was obtained.
ESI MS m/e 472, M (free) + $Na^+$; $^1H$ NMR (300 MHz, $CDCl_3$) δ 1.80-2.10 (m, 8 H), 3.99-4.28 (m, 2 H), 6.46-6.63 (m, 1 H), 7.12-7.34 (m, 2 H), 7.48-7.63 (m, 2 H), 7.66-7.90 (m, 3 H), 7.94-8.05 (m, 1 H), 10.14-10.35 (m, 1 H).

**Example 3238**

**3,4-Difluoro-*N*-[*cis*-4-(4-methyl-quinolin-2-ylamino)-cyclohexyl]-benzamide methanesulfonic acid**

**Step A: Synthesis of 3,4-difluoro-*N*-[*cis*-4-(4-methyl-quinolin-2-ylamino)-cyclohexyl]-benzamide methanesulfonic acid.**

To a solution of *N*-(*cis*-4-methyl-quinolin-2-yl)-cyclohexane-1,4-diamine obtained in step A of example 3070 (3.00 g) in $CHCl_3$ (30 mL) were added $Et_3N$ (3.40 mL) and 3,4-difluoro-benzoyl chloride (2.28 g). The mixture was stirred at ambient temperature for 6 hr. To the mixture was added saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (NH-silica gel, 20% to 33% EtOAc in hexane and silica gel, 2% to 5% MeOH in $CHCl_3$) to give 3,4-difluoro-*N*-[*cis*-4-(4-methyl-quinolin-2-ylamino)-cyclohexyl]-benzamide (3.52 g) as colorless solid. To a solution of 3,4-difluoro-*N*-[*cis*-4-(4-methyl-quinolin-2-ylamino)-cyclohexyl]-benzamide (700 mg) in EtOH (7 mL) was added MsOH (179 mg). The mixture was stirred at ambient temperature for 3 hr. The precipitate was collected by filtration, washed with EtOH, and dried at 70 °C under reduced pressure to give 3,4-difluoro-*N*-[*cis*-4-(4-methyl-quinolin-2-ylamino)-cyclohexyl]-benzamide methanesulfonic acid (769 mg) as a white solid.
ESI MS m/e 396, M (free) + $H^+$; $^1H$ NMR (300 MHz, DMSO-$d_6$) δ 1.69-2.01 (m, 8 H), 2.42 (s, 3 H), 2.62 (brs, 3 H), 3.90-4.21 (m, 2 H), 7.02-7.13 (m, 1 H), 7.47-7.61 (m, 2 H), 7.75-8.04 (m, 5 H), 8.35, (d, *J* = 6.4 Hz, 1 H), 9.15-9.42 (m, 1 H), 12.27-12.51 (m, 1 H).

**Example 3239**

**3-Chloro-4-fluoro-*N*-[*cis*-4-(4-methyl-quinolin-2-ylamino)-cyclohexyl]-benzamide methanesulfonic acid**

**Step A: Synthesis of 3-chloro-4-fluoro-*N*-[*cis*-4-(4-methyl-quinolin-2-ylamino)-cyclohexyl]-benzamide methanesulfonic acid.**

To a solution of 3-chloro-4-fluoro-benzoic acid (2.26 g) and *N*-(*cis*-4-methyl-quinolin-2-yl)-cyclohexane-1,4-diamine obtained in step A of example 3070 (3.00 g) in DMF (30 mL) were added $Et_3N$ (3.93 mL), HOBt-$H_2O$ (2.70 g), and EDC-HCl (2.47 g). The reaction mixture was stirred at ambient temperature for 6 hr. To the reaction mixture was added water (200 mL) and the suspension was stirred at ambient temperature for 30 min. The precipitated was collected by filtration, washed with $H_2O$, and purified by medium-pressure liquid chromatography (NH-silica gel, 20% to 33% EtOAc in hexane) to give 3-chloro-4-fluoro-*N*-[*cis*-4-(4-methyl-quinolin-2-ylamino)-cyclohexyl]-benzamide (4.40 g) as a colorless solid. To a solution of 3-chloro-4-fluoro-*N*-[*cis*-4-(4-methyl-quinolin-2-ylamino)-cyclohexyl]-benzamide (800 mg) in EtOH (8 mL) was added MsOH (196 mg). The mixture was stirred at ambient temperature for 4 hr. The precipitate was collected by filtration, washed with EtOH, and dried at 80 °C under reduced pressure to give 3-chloro-4-fluoro-*N*-[*cis*-4-(4-methyl-quinolin-2-ylamino)-cyclohexyl]-benzamide methanesulfonic acid (845 mg) as a white solid. ESI MS m/e 434, M (free) +$Na^+$; $^1H$ NMR (300 MHz, DMSO-$d_6$) $\delta$ 1.66-1.99 (m, 8 H), 2.38 (s, 3 H), 2.56-2.73 (m, 3 H), 3.87-4.21 (m, 2 H), 6.99-7.14 (m, 1 H) 7.48-7.58 (m, 2 H), 7.74-7.84 (m, 1 H), 7.87-8.05 (m, 3 H), 8.12 (dd, $J$ = 7.2, 2.2 Hz, 1 H), 8.36-8.41 (m, 1 H), 9.14-9.39 (m, 1 H), 12.28-12.55 (m, 1 H).

**Example 3240**

**3-Methoxy-*N*-[*cis*-4-(quinolin-2-ylamino)-cyclohexyl]-benzamide methanesulfonic acid**

**Step A: Synthesis of 3-methoxy-*N*-[*cis*-4-(quinolin-2-ylamino)-cyclobexyl]-benzamide methanesulfonic acid.**

To a solution of *cis*-*N*-quinolin-2-yl-cyclohexane-1,4-diamine obtained in step A of example 3033 (4.00 g) in $CHCl_3$ (40 mL) were added $Et_3N$ (4.85 mL) and 3-methoxy-benzoyl chloride (3.10 g). The mixture was stirred at ambient temperature for 6 hr. The reaction was quenched with saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (NH-silica gel, 20% EtOAc in hexane) to give 3-methoxy-*N*-[*cis*-4-(quinolin-2-ylamino)-cyclohexyl]-benzamide (5.42 g) as colorless solid. To a solution of 3-methoxy-*N*-[*cis*-4-(quinolin-2-ylamino)-cyclohexyl]-benzamide (700 mg) in EtOH (7 mL) was added MsOH (188 mg). The mixture was stirred at ambient temperature for 24 hr. The precipitate was collected by filtration, washed with EtOH, and dried at 80 °C under reduced pressure to give 3-methoxy-*N*-[*cis*-4-(quinolin-2-ylamino)-cyclohexyl]-benzamide methanesulfonic acid (741 mg) as a white solid.
ESI MS m/e 398, M (free) + $Na^+$; $^1H$ NMR (300 MHz, DMSO-$d_6$) $\delta$ 1.70-1.99 (m, 8 H), 2.35 (s, 3 H), 3.81 (s, 3 H) 3.90-4.04 (m, 1 H), 4.08-4.22 (m, 1 H), 7.06-7.26 (m, 2 H), 7.32-7.56 (m, 4 H), 7.73-8.02 (m, 3 H), 8.17-8.38 (m, 2 H), 12.41-12.58 (m, 1 H).

**Example 3241**

***N*-{*cis*-4-[(4-Amino-5-methylpyrimidin-2-yl)amino]cyclohexyl}-3,5-bis(trifluoromethyl)benzamide hydrochloride**

**Step A: Synthesis of 2-chloro-5-methyl-pyrimidin-4-ylamine.**

A solution of 2,4-dichloro-5-methyl-pyrimidine (4.1 g, 0.025 mol) was dissolved in THF (30 mL) and cooled with stirring on an ice bath. To the mixture was added 7 N $NH_3$ in MeOH (14.4 mL, 0.10 mol) and stirring was continued overnight (in which time the ice melted and the reaction warmed to room temperature). The excess solvent was removed in vacuo and the precipitate was suspended in $CH_2Cl_2$ (20 mL). The organic layer was extracted with a $NaHCO_3$ (aq) solution (20 mL) and both layers of the extraction were filtered to collect the resulting insoluble precipitate. This precipitate was washed with cold $H_2O$ and dried to yield 2-chloro-5-methyl-pyrimidin-4-ylamine (1.0 g, 0.0070 mol, 27 %) as a white solid.
ESI-MS m/e 144.2 M+$H^+$; $^1H$ NMR (400 MHz, DMSO-$d_6$) $\delta$ 7.80 (s, 1H), 7.22 (bs, 2H), 1.93 (s, 3H).

**Step B: Synthesis of *N*-{*cis*-4-[(4-amino-5-methylpyrimidin-2-yl)amino]cyclohexyl}-3,5-bis(trifluoromethyl) benzamide hydrochloride.**

To a solution of 2-chloro-5-methyl-pyrimidin-4-ylamine (292 mg, 2.03 mmol) in 2 mL 2-propanol was added DIEA (531 uL, 3.05 mmol) and *cis*-*N*-(4-amino-cyclohexyl)-3,5-bis(trifluoromethyl)-benzamide (720 mg, 2.03 mmol). The mixture was then heated in a microwave at 170 °C for 1 hour. The reaction mixture was cooled and concentrated and the resulting oil was purified by column (0-5 % MeOH in $CH_2Cl_2$). The organic solvents were evaporated and the resulting oil was re-dissolved into 4 mL $CH_2Cl_2$ and 2M HCl in $Et_2O$ (2.0 mL, 4.0 mmol) was added. The reaction was stirred for 30 minutes and the solvent was removed. A precipitate formed that was subsequently filtered and washed with a cold 50% ether in hexanes solution to yield *N*-{*cis*-4-[(4-amino-5-methylpyrimidin-2-yl)amino]cyclohexyl}-3,5-bis (trifluoromethyl)benzamide hydrochloride (500 mg, 1.00 mmol, 49%) as a HCl salt.
ESI-MS m/e 462.2 M+H$^+$ ; $^1$H NMR (400 MHz, DMSO-$d_6$) δ 11.86 (s, 1H), 8.79 (s, 1H), 8.51 (s, 1H), 8.39 (s, 1H), 8.31 (s, 1H), 8.01 (s, 1H), 7.85 (s, 1H), 7.66 (s, 1H), 3.90 (bs, 2H), 1.90 (s, 3H), 1.89-1.61 (m, 8H).

**Example 3242**

**2-[(*cis*-4-{[4-(Dimethylamino)-5-methylpyrimidin-2-yl]amino}cyclohexyl)amino]-1-[4-(trifluoromethoxy) phenyl]ethanone trifluoroacetate**

**Step A: Synthesis of 2-[(*cis*-4-{[4-(dimethylamino)-5-methylpyrimidin-2-yl]amino}cyclohexyl)amino]-1-[4-(tritluoromethoxy)phenyl]ethanone trifluoroacetate.**

To a solution of *cis*-4-(4-dimethylamino-5-methyl-pyrimidin-2-ylamino)-1-cyclohexylamine (37 mg, 0.14 mmol) and 4-trifluoromethoxy bromoacetophenone (42 mg, 0.14 mmol) in THF (2 mL) was added DIEA (20 µL). The reaction was stirred for 2 h at 65 °C, concentrated, dissolved in DMSO (1 mL), and purified by prep-HPLC to give 2-[(*cis*-4-{[4-(dimethylamino)-5-methylpyrimidin-2-yl]amino}cyclohexyl)amino]-1-[4-(trifluoromethoxy)phenyl]ethanone trifluoroacetate 24 mg (30 %) as a white powder.
ESI-MS m/e 452 (M + H)$^+$; $^1$H NMR (400 MHz, $CDCl_3$) δ 8.28 (bs, 2 H), 8.09 (d, 2 H, *J* = 8.8 Hz), 7.29 (m, 2 H), 7.20 (m, 1 H), 4.13 (bs, 1 H), 3.45 (bs, 1 H), 3.33 (s, 6 H), 3.27 (bm, 2 H), 2.28 (s, 3 H), 2.02-1.71 (m, 8 H).

**Example 3243**

***N*-{1-[3,5-Bis(trifluoromethyl)phenyl]-1-methylethyl}-N'-(*cis*-4-{[4-dimethylamino)-5-methylpyrimidin-2-yl] amino}cyclohexyl)urea trifluoroacetate**

**Step A: Synthesis of *N*-{1-[3,5-bis(trifluoromethyl)phenyl]-1-methylethyl}-N'-(*cis*-4-{[4-(dimethylamino)-5-methylpyrimidin-2-yl]amino}cyclohexyl)urea trifluoroacetate.**

To a solution of 2-(3,5-bistrifluoromethyl-phenyl)-2-methyl propionic acid (0.4 g, 1.3 mmol) and $Et_3N$ (0.17 mL, 1.3 mmol) in dry benzene (4 mL) was added diphenylphosphoryl azide (0.36 g, 1.3 mmol). During the reaction being refluxed for about 3 h, 3,5-bistrifluoromethyl-4-(isocyanato-1-methyl-ethyl)-benzene was formed as the reaction intermediate, which was directly used to prepare urea derivatives.

To a solution of *cis*-4-(4-dimethylamino-5-methyl-pyrimidin-2-ylamino)-1-aminocyclohexane (40 mg, 0.16 mmol) in EtOH (1 mL) was added 3,5-bistrifluoromethyl-4-(isocyanato-1-methyl-ethyl)-benzene (48 mg, 0.16 mmol) from the above reaction. The reaction mixture was stirred at 60 °C for 1 h, and completed consumption of the starting material was observed by LC-MS. After removal of the volatile solvent, the residue was dissolved in DMSO (1.5 mL) and purified by prep-HPLC to give 35 mg (35 %) of *N*-{1-[3,5-bis(trifluoromethyl) phenyl]-1-methylethyl}-N'-(*cis*-4-{[4-(dimethylamino)-5-methylpyrimidin-2-yl]amino}cyclohexyl)urea trifluoroacetate.
ESI-MS m/e 547 (M + H)$^+$; $^1$H NMR (400 MHz, $CDCl_3$) δ 13.4 (bs, 1 H), 8.37 (bd, 1 H, *J* = 6.4 Hz), 7.84 (s, 3 H), 7.71 (s, 1 H), 5,56 (bs, 1 H), 4.01 (bs, 1 H), 3.75 (m, 1 H), 3.29 (s, 6 H), 2.25 (s, 3 H), 1.75-1.60 (m, 14 H).

**Example 3244**

***N*-{1-[3,5-Bis(trifluoromethyl)phenyl]-1-methytethyl)-*N'*-(*cis*-4-{[4-(dimethylamino)-5-methylpyrimidin-2-yl] amino}cyclohexyl)-*N*-methylurea trifluoroacetate**

**Step A: Synthesis of *N*-{1-[3,5-bis(trifluoromethyl)phenyl]-1-methylethyl}-*N'*-(*cis*-4-{[4-(dimethylamino)-5-methylpyrimidin-2-yl]amino)cyclohexyl}-*N*-methylurea trifluoroacetate.**

3,5-Bistrifluoromethyl-4-(isocyanato-1-methyl-ethyl)-benzene (36 mg, 0.12 mmol) was added to a solution of *cis*-4-(4-dimethylamino-5-methyl-pyrimidin-2-ylamino)-1-aminocyclohexane (30 mg, 0.12 mmol) and $CH_3I$ (0.17 g, 1.2 mmol) in anhydrous benzene (1 mL) under an inert atmosphere. The reaction mixture was stirred at 50 °C for 2 h, and formation of the methylated and protonated products were observed by LC-MS. After removal of the volatile solvent, the residue was dissolved in DMSO (1.5 mL) and purified by prep-HPLC. 20 mg (25 %) of *N* {1-[3,5-bis(trifluoromethyl) phenyl]-1-methylethyl}-*N'*-(*cis*-4-{[4-(dimethyl amino)-5-methy)pyrimidin-2-yl]amino}cyclohexy))-*N*-methylurea trifluoroacetate was isolated as a white powder.
ESI-MS m/e 561 $(M + H)^+$; $^1H$ NMR (400 MHz, $CDCl_3$) δ 14.5 (bs, 1 H), 9.19 (bd, 1 H, *J* = 6.0 Hz), 7.84 (s, 2 H), 7.79 (s, 1 H), 7.70 (s, 1 H), 4.87 (s, 1 H), 4.23 (bs, 1 H), 4.14 (m, 1 H), 3.26 (s, 6 H), 2.98 (s, 3 H), 2.23 (s, 3 H), 1.75-1.65 (m, 14 H).

**Example 3245**

***cis-N* {1-[3,5-Bis(trifluoromethyl)phenyl]-1-methylethyl}-4-{[4-(dimethylamino)-5-methylpyrimidin-2-yl]amino} cyclohexanecarboxamide trifluoroacetate**

**Step A: Synthesis of 1-(3,5-bistrifluoromethyl-phenyl)-1-methyl-ethylamine.**

3,5-Bistrifluoromethyl-4-(isocyanato-1-methyl-ethyl)-benzene (0.1 g, 0.33 mmol) was treated with 8-N HCl (4 mL). The acidic aqueous solution was heated for 1 h at 60 °C. After cooling the reaction, NaOH pellets were added to make the aqueous mixture alkaline. The solid precipitates were filtered off, and the basic aqueous was extracted with DCM (2x). The combined organic was washed with $H_2O$, dried, and concentrated to give 1-(3,5-bistrifluoromethyl-phenyl)-1-methyl-ethylamine: 1-(3,5-bistrifluoromethyl-phenyl)-1-methyl-ethylamine appeared to be unstable in neat. The product was kept in DCM solution.
ESI-MS m/e 272 $(M + H)^+$

**Step B: Synthesis of *cis-N*-{1-[3,5-bis(trifluoromethyl)phenyl]-1-methylethyl}-4-{[4-(dimethylamino)-5-methylpyrimidin-2-yl]amino}cyclohexanecarboxamide trifluoroacetate.**

To a solution of *cis*-4-(4-dimethylamino-5-methyl-pyrimidin-2-ylamino)-cyclohexanecarboxylic acid (15 mg, 0.05 mmol) and 1-(3,5-bistrifluoromethyl-pheny])-1-methy)-ethylamine (15 mg, 0.05 mmol) in DCM (1.5 mL) was added HATU (25 mg, 0.06 mmol) and followed by $Et_3N$ (10 mg, 0.1 mmol). After 4h stirring at room temperature, the reaction was concentrated, dissolved in DMSO (1.5 mL), and purified by prep-HPLC to give 11 mg (30 %) of *cis-N*-{1-[3,5-bis (trifluoromethyl)phenyl]-1-methylethyl}-4-{[4-(dimethylamino)-5-methylpyrimidin-2-yl]amino}cyclohexanecarboxamide trifluoroacetate.
ESI-MS m/e 532 $(M + H)^+$;$^1H$ NMR (400 MHz, $CDCl_3$) δ 14.6 (bs, 1 H), 8.64 (bd, 1 H, *J* = 6.0 Hz), 7.78 (s, 2 H), 7.69 (s, 1 H), 7.30 (d, 1 H, *J* = 7.2 Hz), 7.16 (s, 1 H), 4.40 (bs, 1 H), 3.30 (s, 6 H), 2.26 (s, 3 H), 2.18 (m, 1 H), 2.07-1.80 (m, 8 H), 1.70 (s, 6 H).

**Example 3246**

**3,4-Difluoro-*N*-{*cis*-4-[(4-methoxy-5-methylpyrimidin-2-yl)amino]cyclohexyl}benzamide trifluoroacetate**

**Step A: Synthesis of 2-chloro-4-methoxy-5-methyl pyrimidine.**

2,4-dichloro-5-methyl pyrimidine (0.8 g, 5 mmol) was dissolved in MeOH (10 mL), and 0.5 M-$NaOCH_3$ in MeOH (10 mL, 5 mmol) was slowly added into the solution. The reaction was stirred for 40 min at room temperature, diluted with $H_2O$, and extracted with DCM (3x). The combined organic was washed with $H_2O$ (2x) and saline (1x), dried, and concentrated. 0.8 g (99 %) of 2-chloro-4-methoxy-5-methyl pyrimidine was isolated, which was directly used for the next reaction without a further purification.

$^1H$ NMR (400 MHz, $CDCl_3$) δ 8.10 (s, 1 H), 4.03 (s, 3 H), 2.12 (s, 3 H).

**Step B: Synthesis of N-[*cis*-4-(4-methoxy-5-methyl-pyrimidin-2-ylamino)-cyclohexyl]carbamic acid *tert*-butyl ester.**

A sealed tube containing 2-chloro-4-methoxy-5-methyl pyrimidine (0.35 g, 2.2 mmol), *cis-(4*-amino-cyclohexyl)-carbamic acid *tert*-butyl ester (0.56 g, 2.4 mmol), DIEA (0.8 mL, 4.5 mmol), and IPA (2 mL) was reacted for 4000 sec at 175 °C in a Personal Microwave Synthesizer. The reaction was diluted with DCM, washed with 1N-HCl and $H_2O$, dried, and concentrated. The crude product was purified by column chromatography [silica gel, DCM:MeOH (100:0 to 97:3)]. 0.25 g (34 %) of N-[*cis*-4-(4-methoxy-5-methyl-pyrimidin-2-ylamino)-cyclohexyl] carbamic acid *tert*-butyl ester was isolated.
ESI-MS m/e 337 $(M + H)^+$;$^1H$ NMR (400 MHz, $CDCl_3$) δ 7.80 (s, 1 H), 4.86 (bd, 1 H, *J* = 6.0 Hz), 4.55 (bs, 1 H), 3.93 (bm, 1 H), 3.89 (s, 3 H), 3.62 (bs, 1 H), 1.97 (s, 3 H), 1.83-1.55 (m, 8 H), 1.45 (s, 9 H).

**Step C: Synthesis of *cis*-4-(4-methoxy-5-methyl-pyrimidin-2-ylamino)-aminocyclohexane.**

To a solution of N-[*cis*-4-(4-methoxy-5-methyl-pyrimidin-2-ylamino)-cyclohexyl] carbamic acid tert-butyl ester (0.24 g, 0.7 mmol) in DCM (10 mL) was added TFA (5 mL). The reaction was stirred for 1.5 h at room temperature. After removal of the volatile solvent, the residue was treated with 4N-NaOH (3 mL). The basic aqueous was extracted with DCM (3x), and combined organic was washed with $H_2O$ (2x) and brine (1x), and concentrated. 0.13 g (82 %) of *cis*-4-(4-methoxy-5-methyl-pyrimidin-2-ylamino)-aminocyclohexane was isolated as a yellowish solid.
ESI-MS m/e 237 $(M + H)^+$; $^1H$ NMR (400 MHz, $CDCl_3$) δ 7.79 (s, I H), 5.05 (bd, 1 H, J = 6.4 Hz), 3.99 (bs, 1 H), 3.89 (s, 3 H), 2.92 (bm, 1 H), 2.45 (bs, 2 H), 1.96 (s, 3 H), 1.83-1.45 (m, 8 H).

**Step D: Synthesis of 3,4-difluoro-*N*-{*cis*-4-[(4-methoxy-5-methylpyrimidin-2-yl)amino]cyclohexyl}benzamide trifluoroacetate.**

To a solution of *cis*-4-(4-methoxy-5-methyl-pyrimidin-2-ylamino)-aminocyclohexane (20 mg, 0.08 mmol) in DCM (1 mL) was added 3,4-difluorobenzoyl chloride (14 mg, 0.08 mmol), and followed by $Et_3N$ (25 µL). The reaction was stirred for 2 h at room temperature, and MeOH (0.2 mL) was added to quench the reaction. After removal of the volatile solvent, the residue was dissolved in DMSO (1.5 mL) and purified by prep-HPLC to give 12 mg (40 %) of 3,4-difluoro-*N*-{*cis*-4-[(4-methoxy-5-methylpyrimidin-2-yl)amino]cyclohexyl}benzamide trifluoroacetate as a white powder.
ESI-MS m/e 377 $(M + H)^+$; $^1H$ NMR (400 MHz, $CDCl_3$) δ 15.7 (bs, 1 H), 9.55 (d, I *H, J* = 7.2 Hz), 7.73 (m, 1 H), 7.59 (m, 1 H), 7.57 (s, 1 H), 7.20 (m, 1 H), 6.80 (d, 1 H, *J* = 8.0 Hz), 4.37 (bs, 1 H), 4.18 (bm, 1 H), 4.09 (s, 3 H), 2.04 (s, 3 H), 1.89-1.75 (m, 8 H).

**Example 3247**

***N*-(*cis*-4-{[4-Methyl-6-(methylamino)pyrimidin-2-yl]amino}cyclohexyl)-4-(trifluoromethoxy)benzamide hydrochloride**

**Step A: Synthesis of (2-chloro-6-methyl-pyrimidin-4-yl)-methyl-amine.**

2,4-Dichloro-6-methylpyrimidine (10 g, 61.34 mmol) in 50 mL in $CH_2Cl_2$ was added 2 M methylamine in methyl alcohol (46.01 ml, 92.02 mmol) at 0°C. The reaction mixture was stirred overnight and then the excess solvent was evaporated off and the material subjected to chromatography (50% hexanes in ethyl acetate) to yield (2-chloro-6-methyl-pyrimidin-4yl)-methyl-amine (5.835 g, 37.17 mmol, 60.59%) as a white solid.
ESI-MS 158.0 $M+H^+$ ; $^1H$ NMR (400 MHz, DMSO-$d_6$) δ 7.62 (s, 1H), 6.18 (s, 1H), 2.70 (bs, 3H), 2.10 (bs, 3H).

**Step B: Synthesis *N*-(*cis*-4-{[4-methyl-6-(methylamino)pyrimidin-2-yl]amino}cyclohexyl)-4-(trifluoromethoxy) benzamide hydrochloride.**

To a solution of (2-chloro-6-methyl-pyrimidin-4yl)-methyl-amine (500 mg, 3.18 mmol) in 3 mL 2-propanol was added *cis*-*N*-(4-amino-cyclohexyl)-4-trifluoromethoxy-benzamide (1.25 g, 4.14 mmol) and DIEA (1.108 mL, 6.36 mmol). The mixture was heated in a microwave synthesizer at 180 °C for 2 hours. The solvent was evaporated and obtained compound was dissolved in $CH_2Cl_2$ and was added 2 M HCl in diethyl ether (6.2 mL) to give *N*-(*cis*-4-{[4-methyl-6-(methylamino)pyrimidin-2-yl]amino}cyclohexyl)-4-(trifluoromethoxy)benzamide hydrochloride (1.3014 g, 2.83 mmol,

89 %) as a yellowish solid.
ESI-MS 424.2 M+H$^+$;$^1$H NMR (400 MHz, DMSO-$d_6$) δ 8.72 (s, 1H), 8.44 (s,1H), 7.99-7.96 (d, *J* = 8 Hz, 2H), 7.86 (s, 1H), 7.47-7.45 (d, *J* = 8 Hz, 2H, 4.03 (s, 1H), 3.87 (s, 1H), 2.89-2.88 (d, *J* = 4 Hz, 3H), 2.20 (s, 3H), 1.85 (bs, 2H), 1.72 (bs, 6H).

**Example 3248**

***N*-({*cis*-4-[(4-Amino-5-methylpyrimidin-2-yl)amino]cyclohexyl}methyl)-3,5-bis(trifluoromethyl)benzamide hydrochloride**

**Step A: Synthesis of *N*-({*cis*-4-[(4-amino-5-methylpyrimidin-2-yl)amino]cyclohexyl}methyl)-3,5-bis (trifluoromethyl)benzamide hydrochloride.**

To a solution of 2-chloro-5-methyl-pyrimidin-4-ylamine (269 mg, 1.87 mmol) in 1 mL 2-propanol was added *cis*-*N*-(4-amino-cyclohexylmethyl)-3,5-bis-trifluoromethyl-benzamide (689.8 mg, 1.87 mmol) and DIEA (489.5.4 µl, 2.81 mmol). The mixture was heated in a microwave synthesizer at 180 °C for 2 hours. The solvent was evaporated and the material subjected to chromatography (1-2% methanol/ $CH_2Cl_2$). The obtained compound was dissolved in $CH_2Cl_2$ and was added 2 M HCl in diethyl ether (2.2 mL) to give *N*-({*cis*-4-[(4-amino-5-methylpyrimidin-2-yl)amino]cyclohexyl} methyl)-3,5-bis(trifluoromethyl)benzamide hydrochloride (667.1 mg, 1.30 mmol, 70%) as a white solid.
ESI-MS 476.2 M+H$^+$; $^1$H NMR (400 MHz, DMSO-$d_6$) δ 9.16-9.13 (t, *J* = 4 Hz, *J* = 8 Hz, 1H), 8.55 (s, 2H), 8.36-8.31 (bs, 2H), 7.86 (bs, 1H), 7.71 (bs, 1H), 4.07 (bs, 1H), 3.27-3.24 (t, *J* = 8 Hz, *J* = 4 Hz, 2H), 1.91 (bs, 3H), 1.73-1.42 (m, 8H).

**Example 3249**

**2-[(2-Chlorophenyl)sulfonyl]-*N*-(*cis*-4-{[4-(dimethylamino)-6-methylpyrimidin-2-yl]amino}cyclohexyl) nicotinamide trifluoroacetate**

**Step A: Synthesis of *cis*-2-chloro-*N*-[4-(4-dimethylamino-6-methyl-pyrimidin-2ylamino)-cyclohexyl]-nicotinamide.**

*cis*-$N^2$-(4-Amino-cyclohexyl)-6, $N^4$,$N^4$-trimethyl-pyrimidine-2,4-diamine (2.86 g, 11.5 mmol) in 20 mL $CH_2Cl_2$ was added 2-chloronicotinoyl chloride (2.02 g, 11.5 mmol), and DIEA (3.9 mL, 23 mmol). The reaction mixture was stirred for an hour. The solvent was evaporated off and the compound was crystallized (2% hexanes in ether) to yield *cis*-2-chloro-*N*-[4-(4-dimethylamino-6-methyl-pyrimidin-2ylamino)-cyclohexyl]-nicotinamide (4.2 g, 10.8 mmol, 94%).
ESI-MS 389.2 M+H$^+$ ; $^1$H NMR (400 MHz, DMSO-$d_6$) δ 13.1 (bs, 1H), 8.72-8.70 (d, *J* = 8 Hz, 1H), 8.49-8.46 (dt, *J* = 8 Hz, *J* = *4* Hz, 1H), 8.04 (s, 1H), 7.89-7.87 (dd, *J* = 4 Hz, *J* = 4Hz, 1H), 7.52-7.47 (q, *J* = 8 Hz, *J* = 4Hz, 1H), 6.27 (s, 1H), 3.95 (bs, 2H), 3.27 (bs, 6H), 2.31 (s, 3H), 1.82-1.74 (m, 8H).

**Step B: Synthesis of 2-[(2-chlorophenyl)sulfonyl)-*N*-(*cis*-4-{[4-(dimethylamino)-6-methylpyrimidin-2-yl]amino} cyclohexyl)nicotinamide trifluoroacetate.**

To a solution of *cis*-2-chloro-*N*-[4-(4-dimethylamino-6-methyl-pyrimidin-2ylamino)-cyclohexyl]-nicotinamide (50 mg, 0.128 mmol) in 1 mL dioxane was added 2-chlorobenzenethiol (37.1 mg, 0.256 mmol), and $Cs_2CO_3$; (83.4 mg, 0.256 mmol). The mixture was heated in a microwave synthesizer at 180 °C for 1 hour. After the solvent was evaporated, the compound was then subjected to purification by prep HPLC to give *cis*-2-(2-chloro-phenylsulfanyl) -*N*-[4-(4-dimethylamino-6-methyl-pyrimidin-2-ylamino)-cyclohexyl]-nicotinamide trifluoroacetate (23.2 mg, 30 %) as a white solid.
ESI-MS m/e 497.4 M+H$^+$ ;

To a solution of *cis*-2-(2-chloro-phenylsulfanyl)-*N*-[4-(4-dimethylamino-6-methyl-pyrimidin-2-ylamino)-cyclohexyl]-nicotinamide trifluoroacetate (23.2 mg, 0.038 mmol) in 1mL $CH_2Cl_2$ was added 3-chloroperoxybenzoic acid (31.5 mg 0.14 mmol). The reaction mixture was stirred for 15 h and quenching with $NaHCO_3$. The solvent was evaporated and compound was then subjected to purification by prep HPLC to give 2-[(2-chlorophenyl)sulfonyl]-*N*-(*cis*-4-{ [4-(dimethylamino)-6-methylpyrimidin-2-yl]amino}cyclohexyl)nicotinamide trifluoroacetate (8.9 mg, 0.014 mmol, 36%) as a white solid.
ESI-MS m/e 529.2 M+H$^+$ ; $^1$H NMR (400 MHz, DMSO-$d_6$) δ 11.98 (s, 1H), 8.61-8.59 (m, 2H), 8.24-8.21 (dd, *J*= 4 Hz, 4 Hz, 1H), 8.08-8.06 (d, J= 8Hz, 1H), 7.79-7.74 (m, 2H), 7.71-7.69 (t, *J* = 4Hz, 1H), 7.64-7.62 (d, *J*= 8 Hz, 1H), 7.58 (bs, 1H), 6.32 (s, 1H), 3.94 (bs, 2H), 3.21 (s, 3H), 3.15 (s, 3H), 2.28 (s, 3H), 1.84-1.78 (m, 8 H).

**Example 3250**

***N*-(*cis*-4-{[(4-Methylquinolin-2-yl)methyl]amino}cyclohexyl)-3,5-bis(trifluoromethyl)benzamide trifluoroacetate**

**Step A: Synthesis of 4-methyl-2-vinyl-quinoline.**

To 50 mL toluene in a 150 mL rounded-bottom flask, was added 2-chlorolepidine (1 g, 63 mmol), tetrakis ( triphenylphonsine ) palladium (0) (65 mg, 0.63 mmol), triphenyl phosphine (0.495 g, 1.89 mmol) and vinyltributyl tin (2.2 g,6.76 mmol). The mixture was refluxed at 116 °C under $N_2$ for 2 hours. The reaction mixture was concentrated and purified by silica gel with 0-10% EtOAc/Hexane to yield 4-methyl-2-vinyl-quinoline (720 mg, 4.26 mmol, 76%).
ESI MS m/e: 170.0 M+H$^+$;$^1$H NMR (400 MHz, $CDCl_3$) δ 7.96 (d, *J* = 8 Hz,1H), 7.85 (d, *J* = 8 Hz, 1H), 7.58 (dd, $J_1$ = $J_2$ = 8 Hz, 1H), 7.43 (dd, $J_1$ = $J_2$ = 8 Hz, 1 H ), (7.15 (s, 1H), 6.89 (dd, $J_1$ = 16 Hz, $J_2$ = 12 Hz, 1H), 6.15 (d, J = 16 Hz, 1H), 5.54 (d, *J* = 8 Hz, 1H), 2.60 (s, 3H)

**Step B: Synthesis of 4-methyl-quinoline-2-carbaldehyde.**

To a 500 mL rounded bottom flask filled with 40 mL 90% THF/$H_2O$ was added 4-methyl-2-vinyl-quinoline (1.2 g, 7.1 mmol) NMO (1.29 g, 10.65 mmol), and $OsO_4$ (1.3 mL, 0.21 mmol) under $N_2$. The mixture was stirred at room temperature overnight under $N_2$. The reaction mixture was quenched with saturated solution of $Na_2SO_3$ , and the organic phase was then extracted EtOAc (100 mL x 4. The organic layer was combined and washed with brine, and concentrated. The crude product, 1-(4-methyl-quinolin-2-yl)-ethane-1,2-diol (1.5 g), was directly used to next Step without further purification.
To 60 mL 90%THF/ $H_2O$, was added 1.5 g of the crude 1-(4-methyl-quinolin-2-yl)-ethane-1,2-diol and $NaIO_4$ (1.4 g, 8.86 mmol). The mixture was stirred at room temperature under $N_2$ for 6 hours. The organic phase was extracted with EtOAc (100 mL x4, combined, and dried by anhydrous $MgSO_4$. It was concentrated to purify by silica gel column using 0-5% EtOAc /Hexane to yield 4-methyl-quinoline-2-carbaldehyde (600 mg, 3.5 mL, 49.4%).
ESI MS m/e: 172.0 M+H$^+$; $^1$H NMR (400 MHz, $CDCl_3$) δ 10.2 (s, 1H), 8.25 (d, *J* = 8 Hz, 1H), 8.07 (d, *J* = 8 Hz, 1H), 7.88 (s, 1H), 7.82 (dd, $J_1$ = $J_2$ = 8 Hz, 1H), 7.71 (dd, $J_1$ = $J_2$ = 8 Hz, 1H), 2.79 (s, 3H).

**Step C: Synthesis of resin bound *cis*-(4-amino cyclohexyl) carbamic acid fluorenylmethyl ester.**

In a 30 mL manual synthesis vessel, 2-(3,5 dimethoxy-4-formyl) phenoxy ethyl polystyrene resin (0.5 gram; 0.90 mmol/gram) and *cis*-(4-amino cyclohexyl) carbamic acid fluorenylmethyl ester 2 (453mg, 1.35 mmol) were suspended in 4 mL of DMF. To this suspension was added a solution of $NaBH(OAc)_3$ (299 mg, 1.35 mmol) in 1% acetic acid/DMF solution (4 mL). After shaking the mixture overnight in a rotary shaker, the solution was removed by filtration and the resin washed sequentially with DMF, 10% DIEA/DMF, DMF, DCM and MeOH. The washing sequence was repeated four times. The resulting resin bound intermediate was dried under vacuum for 20 minutes.

**Step D: Synthesis of resin bound-*cis* -[4-(4-methyl-quinolin-2-methyl-amino)-cyclohexyl]-carbamic acid flourenylmethyl ester.**

To the resin bound intermediate (0.315 mmol) was added 4-methyl-quinoline-2-carbaldehyde (96 mg, 0.564 mmol) in dimethyl acetamide (5 mL) and 1% acetic acid (.050 mL). The resin suspension was mixed in a rotary shaker for 1 hour at room temperature. Sodium cyanoborohydride (195 mg, 3.15 mmol) was added to the resin suspension and the reaction was mixed overnight at room temperature. At the completion of the reaction, the solution was filtered and the resin washed sequentially with DMF, 10%DIEA/DMF, DMF, DCM and MeOH. The washing sequence was repeated four times. The resulting resin bound intermediate 5 was dried under vacuum for 20 minutes

**Step E: Synthesis of *N*-(*cis*-4-{[(4-methylquinolin-2-yl)methyl]amino}cyclohexyl)-3,5-bis(trifluoromethyl) benzamide trifluoroacetate.**

The resin bound intermediate (0.171 mmol) was treated with 20% piperidine in DMF (3 mL) for 30 minutes at room temperature. After 30 minutes, the solution was filtered and the resin washed with DMF, DCM and MeOH. The washing sequence was repeated four times.
The deprotected resin bound intermediate was suspended in DMF (1.0 mL). 3,5 bis-trifluoromethylbenzoyl chloride (47 mg, 0.171 mmol) was added to the resin suspension followed by triethylamine (0.0519 mL, 0.513 mmol). The reaction was mixed for 30 minutes at room temperature. The solution was then filtered and the resin washed

sequentially with DMF, DCM and MeOH. The washing sequence was repeated four times.

After drying under vacuum for 20 minutes, the resin bound intermediate was treated with 5 mL of TFA solution (TFA /$CH_2Cl_2$ /$H_2O$ 20:20:1 v/v). The reaction was shaken for 2 hours and the TFA solution was collected after filtration. The TFA was removed by rotary evaporation and the compound subjected to purification by preparative HPLC to give *N*-(*cis*-4-{[(4-methyl quinolin-2-yl)methyl]amino}cyclohexyl)-3,5-bis(trifluoromethyl)benzamide trifluoroacetate (3.8 mg; 8%) as a white solid.
ESI MS m/e 510.2 M+H$^+$; $^1$H NMR (400MHz, $CD_3OD$) δ (ppm): 8.56 (m, 1H), 8.42 (s, 2H), 8.19 (m, 3H), 7.82 (m, 1H), 7.69 (m, 1H), 7.39 (s, 1H), 4.6 (s, 2H), 4.14 (m, 1H), 3.40 (m, 1H), 2.78 (s, 3H), 2.22-1.81 (m, 8H).

## Example 3251

### *cis-N*-[(1S)-1-(4-Chlorophenyl)ethyl]-4-{[4-(dimethylamino)-5-methylpyrimidin-2-yl]amino} cyclohexanecarboxamide trifluoroacetate

#### Step A: Synthesis of *cis*-4-(4-dimethylamino-5-methyl-pyrimidin-2-ylamino)-cyclohexanecarboxylic acid.

A mixture of (2-chloro-5-methyl-pyrimidin-4-yl)-dimethyl-amine (28.9 g, 0.186 mol) and 4-amino-cyclohexanecarboxylic acid (20 g, 0.140 mol) in 100 mL of toluene was stirred at room temperature for 5 minutes to form a slurry under $N_2$. To the slurry, was added $Pd(OAc)_2$ (0.34 g, 1.5 x $10^{-3}$ mol), 2-(di-t-butylphosphine) biphenyl (0.24, 0.8 mmol) and NaOtBu (33.64 g, 0.35 mol). The mixture was heated and refluxed at 118°C under $N_2$ for 2 hours. The reaction mixture was concentrated to give a brown solid. The above brown solid was dissolved with 100 mL MeOH and 5 mL $H_2O$, neutralized with acetic acid. The precipitate was filtered and washed with cold water (5mL x 2) and toluene (100 mL x 2) to yield *cis*-4-(4-dimethylamino-5-methyl-pyrimidin-2-ylamino)-cyclohexanecarboxylic acid (36.7 g, 0.132 mol, 94%) as a white solid.
ESI MS m/e 279 M+H$^+$; $^1$H NMR (400 MHz, $CDCl_3$) δ 7.46 (s, 1H), 4.20 (s, 1H), 3.3 (s, 6H), 3.2 (s, 1H), 2.48 (m, 1H), 2.27 (s, 3H), 2.15-1.63 (m, 8H).

#### Step B: Synthesis of *cis-N*-[(1S)-1-(4-chlorophenyl)ethyl]-4-{[4-(dimethylamino)-5-methylpyrimidin-2-yl]amino} cyclohexanecarboxamide trifluoroacetate.

To a solution of (*S*)-1-(4-chloro-phenyl)-ethylamine (61.5 mg, 0.395 mmol) in 10 mL DCM was added *cis*-4-(4-dimethylamino-5-methyl-pyrimidin-2-ylamino)-cyclohexanecarboxylic (100 mg, 0.395 mmol), HATU (150 mg, 0.395 mmol), and 5 drops of $Et_3N$. The reaction mixture was stirred at room temperature under $N_2$ overnight. The solvent was evaporated and the material subjected to prep-HPLC to give *cis-N*-[(1*S*)-1-(4-chlorophenyl)ethyl]-4-{ [4-(dimethyl amino)-5-methylpyrimidin-2-yl]amino}cyclohexanecarboxamide trifluoroacetate (20 mg, 0.048 mmol, 13.4%) as a white solid.
ESI MS m/e 416.3 M+H$^+$; $^1$H NMR (400 MHz, DMSO-$d_6$) δ 8.24-8.12 (d, 1H), 7.55 (s, 1H), 7.32-7.10 (m, 4H), 4.87 (m, 1H), 2.47 (s, 6H), 2.28 (bs, 1H), 2.18 (s, 3H), 1.81-1.39 (m, 8H), 1.31 (d, 3H).

## Example 3252

### *cis-N*-[(*1R*)-1-(4-Bromophenyl)ethyl]-4-[(4-methylquinolin-2-yl)amino]cyclohexanecarboxamide trifluoroacetate

#### Step A: Synthesis of *cis*-4-(4-methylguinolin-2-ylamino)cyclohexanecarboxylic acid.

A mixture of 2-chloro-4-methyl-quinoline (6.67, 0.0375 mol) and 4-amino-cyclohexanecarboxylic acid (4.48 g, 0.0312 mol) was dissolved in 100 mL of toluene and stirred at room temperature for 5 minutes to form a slurry under $N_2$. To the slurry, was added $Pd(OAc)_2$ (0.077 g, 3.43x $10^{-4}$ mol), 2-(di-t-butylphosphine) biphenyl (0.093, 3.12x$10^{-4}$ mol) and NaOtBu (7.5g, 0.078 mol). The above material was heated and refluxed at 118°C for 2 hours. The reaction mixture was concentrated under reduced pressure to give a brown solid. The above brown solid was dissolved with 100 mL MeOH and 5 mL $H_2O$, neutralized with acetic acid. The precipitates were filtered and washed with cold water (5 mL x 2) and toluene (100 mL x 2) to yield *cis*-4-(4-methylquinolin-2-ylamino)cyclohexanecarboxylic acid (7.45 g, 0.026 mol, 84%) as a white solid.
ESI MS m/e 285.1 M+H$^+$; $^1$H NMR (400 MHz, DMSO-$d_6$) δ 7.78 (d, 1H), 7.49 (m, 1H), 7.21 (m, 1H), 6.85 (d, 1H), 6.72 (s, 1H), 4.19 (s, 1H), 2,54-2.53 (m, 2H), 2.46 (s, 3H).

**Step B: Synthesis of *cis-N*-[(1*R*)-1-(4-bromophenyl)ethyl]-4-[(4-methylquinolin-2-yl)amino] cyclohexanecarboxamide trifluoroacetate.**

To a solution of (*R*)-1-(4-bromo-phenyl)-ethylamine (77.4 mg, 0.39 mmol) in 10 mL DCM was added *cis*-4-(4-methylquinolin-2-ylamino)cyclohexanecarboxylic acid (100 mg, 0.35 mmol) , HATU (148 mg, 0.39 mmol), and 5 drops of $Et_3N$. The reaction mixture was stirred at room temperature under $N_2$ overnight. The solvent was evaporated and the material subjected to prep HPLC to give *cis-N*-[(1*R*)-1-(4-bromophenyl)ethyl]-4-[(4-methylquinolin-2-yl)amino] cyclohexanecarboxamide trifluoroacetate (24 mg, 0.052 mmol, 14.7%) as white solid.
ESI MS m/e 468.2 $M+H^+$; $^1H$ NMR (400 MHz, DMSO-$d_6$) δ 9.18-9.07 (s, 1H), 7.94-7.84 (t, 1H), 7.74-7.68 (t, 1H), 7.46-7.42 (m, 2H), 7.22-7.17 (m, 2H), 7.00-6.94 (s, 1H), 4.86 (m, 1H), 4.11 (s,1H), 2.58 (s, 3H), 2.40-2.23 (m, 2H), 1.88-1.49 (m, 8H), 1.33-1.19 (d, 3H).

**Example 3253**

***trans*-2-(4-Chlorophenyl)-*N*-(*cis*-4-{[4-(dimethylamino)-5-methylpyrimidin-2-yl]amino}cyclohexyl) cyclopropanecarboxamide trifluoroacetate**

**Step A: Synthesis of *trans*-3-(4-chlorophenyl)-N-methoxy-N-methylacrylamide.**

A solution of 4-chlorobenzalehyde (3 g, 21.34 mmol) and N-methoxy-N-methyl-2-(triphenylphosphoranylidene)acetamide (8.5 g, 23.47 mmol) in $CH_2Cl_2$ was stirred at room temperature for 16 h. The solvent was removed in vacuo, and the crude product was purified by column chromatography on silica gel (0-20% EtOAc / Hex) to afford *trans*-3-(4-chlorophenyl)-N-methoxy-N-methylacrylamide (4.78 g, 99%) as colorless crystals.
ESI MS m/e 226.1 $M+H^+$; $^1H$ NMR (400 MHz, $CDCl_3$) δ 7.66 (d, *J* = 5.6 Hz, 1H), 7.45 (d, *J* = 8.4 Hz, 2H), 7.33 (d, *J* = 8.4 Hz, 2H), 6.99 d, *J* = 5.6 Hz, 1H), 3.75 (s, 3H), 3.29 (s, 3H)

**Step B: Synthesis of N-methoxy-N-methyl-*trans*-2-(4-chlorophenyl) cyclo-propanecarbxoamide.**

To a solution of trimethylsulfoxonium iodide (9.3 g, 42.4 mmol) in DMSO (40 mL) was added sodium hydride (1.7 g, 42.4 mmol) at room temperature in portions. After 1h, a solution of *trans*-3-(4-chlorophenyl)-N-methoxy-N-methylacrylamide (4.78 g, 21.2 mmol) in DMSO (20 mL) was added via cannula at r.t. The mixture was stirred for another 6 h, and then it was quenched with saturated aqueous $NH_4Cl$ solution, extracted with $CH_2Cl_2$, washed with brine and dried over anhydrous $MgSO_4$. The crude product was purified by column chromatography (0-50 % EtOAc / Hex)to afford N-methoxy-N-methyl -*trans*-2-(4-chlorophenyl)cyclopropanecarboxamide as colorless oil (4.76 g, 88.5 %).
ESI MS m/e 239.9 $M+H^+$; $^1H$ NMR (400 MHz, $CDCl_3$) δ 7.24 (d, *J* = 8 Hz, 2H), 7.06 (d, *J* = 8 Hz, 2H), 3.69 (s, 3H), 3.23 (s, 3H), 2.47 (m, 1H), 2.37 (bs, 1H), 1.63 (m, 1H), 1.27 (m, 1H)

**Step C: Synthesis of trans-2-(4-chloro-phenyl)cyclopropanecarboxylic acid.**

A suspension of afford N-methoxy-N-methyl -*trans-2-(4*-chlorophenyl)cyclopropanecarboxamide (4.76 g, 18.76 mmol) and potassium tert-butoxide (4.76 g, 18.76 mmol) in the TBME (130 mL) and water (0.68 mL, 37.5 mmol) was stirred at room temperature for 16h. The mixture was acidified by slowly adding concentrated HCl, and the aqueous mixture was extracted with $CH_2Cl_2$ (3x60 mL). The combined organic layers were washed with brine and dried over anhydrous $MgSO_4$. The solvent was removed in vacuo and the product was obtained as white solid (3.447 g, 93.5 %)
ESI MS m/e 197.0 $M+H^+$; $^1H$ NMR (400 MHz, $CDCl_3$) δ 11.4 (bs, 1H), 7.28 (d, *J* = 8.4 Hz, 2H), 7.06 (d, *J*= 8.4 Hz, 2H), 2.60 (ddd, $J_1$ = 9.5 Hz, $J_2$ = 6.6 Hz, $J_3$ = 4.1 Hz, 1H), 1.89 (ddd, $J_1$ = 9.5 Hz, $J_2$ = 5.2 Hz, $J_3$ = 4.2 Hz, 1 H), 1.69 (dt, $J_1$ = 9.5 Hz, $J_2$ = 5.1 Hz, 1H), 1.40 (ddd, $J_1$ = 8.4 Hz, $J_2$ = 5.2 Hz, $J_3$ = 4.3 Hz, 1H)

**Step D: Synthesis of *trans*-2-(4-chlorophenyl)-*N*-(*cis*-4-{[4-(dimethylamino)-5-methylpyrimidin-2-yl]amino} cyclohexyl)cyclopropanecarboxamide trifluoroacetate.**

To a mixture of *trans*-2-(4-chloro-phenyl)cyclopropanecarboxylic acid (22.1 mg, 0. 112 mmol) and 2-chloro-4-dimethylamino-5-methylpyrimidine (28 mg, 0.112 mmol) in $CH_2Cl_2$ (5 mL) was added HATU (42.6 mg, 0.112 mmol) at r.t. After 30 sec $Et_3N$ (5 drops) was added dropwise. The mixture was stirred overnight. *trans*-2-(4-chlorophenyl) -*N*-(*cis*-4-{[4-(dimethylamino)-5-methylpyrimidin-2-yl]amino}cyclohexyl)cyclopropanecarboxamide trifluoroacetate (20 mg, 37%) was obtained from prep-HPLC.
ESI MS m/e: 428.4 $M+H^+$; $^1H$ NMR (400 MHz, $CDCl_3$) δ 8.49 (bs, 1H), 7.23 (d, *J* = 8 Hz, 2H), 7.02( d, *J*= 8 Hz, 2H),

6.28 (d, $J$ = 8 Hz, 1H), 4.11 (m, 1H), 3.99 (m, 1H), 3.29 (s, 6H), 2.45 (ddd, $J_1$ = 12 Hz, $J_2$ = 8 Hz, $J_3$ = 4 Hz, 1H), 2.25 (s, 3H), 1.85-1.65 (m, 1H), 1.58 (dt, $J_1$ = 8 Hz, $J_2$ = 4 Hz, 1H), 1.18 (dt, $J_1$ = 8 Hz, $J_2$= 4 Hz, 1H)

**Example 3254**

***N*-{*cis*-4-[(4,5-Dimethytpyrimidin-2-yl)amino]cyclohexyl}-3,5-bis(trifluoromethyl)benzamide trifluoroacetate**

**Step A: Synthesis of 2-chloro-4, 5-dimethylpyrimidine.**

A mixture of 2, 4-dichloro-5-methylpyrimidine (0.3 g, 1.84 mmol), $AlMe_3$ (0.3 mL, 2.0M) and $Pd(PPh_3)_4$ (85 mg, 4%mol) in dry THF (5 mL) was heated in a microwave synthesizer at 150°C for 20 min. The solvent was removed in vacuo and the crude product subjected to chromatography (0-40 % EtOAC/Hex) to yield 2-chloro-4, 5-dimethylpyrimidine (0.13 g, 50 %) as yellow solid.
ESI MS m/e: 143.1 M+H$^+$; $^1$H NMR (400 MHz, $CDCl_3$) δ 8.24 (s, 1H), 2.45 (s, 3H), 2.22 (s, 3H)

**Step B: Synthesis of *N*-{*cis*-4-[(4,5-dimethylpyrimidin-2-yl)amino]cyclohexyl}-3,5-bis(trifluoromethyl) benzamide trifluoroacetate.**

A mixture of 2-chloro-4, 5-dimethylpyrimidine (30 mg, 0.21 mmol), N-(*cis*-4-aminocyclohexyl)-3,5-bis(trifluoromethyl)benzamide (74.6 mg, 0.21 mmol), $Pd(OAc)_2$ (0.47 mg, 0.01 equiv.), dppf (1.16 mg, 0.01 equiv.) and KOtBu (59 mg, 0.53 mmol) in toluene (3 mL) was heated in a microwave synthesizer at 150°C for 20 min. The solvent was removed in vacuo and the crude product subjected to purification by HPLC to give *N*-{*cis*-4-[(4,5-dimethyl pyrimidin-2-yl)amino]cyclohexyl}-3,5-bis(trifluoromethyl)benzamide trifluoroacetate (25 mg, 21%) as yellow solid.
ESI MS m/e 461.2 M+H$^+$; $^1$H NMR (400 MHz, $CDCl_3$) δ 8.36 (s, 3H), 7.99 (s, 1H), 4.47 (d, 1H), 4.23 (bs, 1H), 2.52 (s, 3H), 2.13 (s, 3H), 1.95-1.65 (m, 8H)

**Example 3255**

***N*-(3,4-Difluorophenyl)-*N'*-(*cis*-4-{[4-(dimethylamino)-5-methylpyrimidin-2-yl]amino}cyclohexyl)urea trifluoroacetate**

**Step A: Synthesis of *N*-(3,4-difluorophenyl)-*N'*-(*cis*-4-{[4-(dimethylamino)-5-methylpyrimidin-2-yl]amino} cyclohexyl)urea trifluoroacetate.**

*cis*-$N^2$-(4-Amino-cyclohexyl)-5,$N^4$,$N^4$-trimethyl-pyrimidine-2,4-diamine (30 mg, 0.12 mmol) was dissolved in 1 mL of DMSO. 1,2-Difluoro-4-isocyanato-benzene was added to the solution, and the solution was stirred overnight. The crude was purified by HPLC to give *N*-(3,4-difluoro phenyl)-*N'*-(*cis*-4-{[4-(dimethylamino)-5-methylpyrimidin-2-yl] amino}cyclohexyl)urea trifluoroacetate as a white solid. (32.2 mg, 54.0%).
ESI MS m/e 405.3 (M + H$^+$); $^1$H NMR (400 MHz, $CDCl_3$) δ 13.45 (s, 1H), 8.35 (d, J = 8.0 Hz, 1H), 7.67 (s, 1H), 7.52-7.47 (m, 1H), 7.28-7.26 (m, 1H), 7.07-6.99 (m, 2H), 4.00 (m, 1H), 3.96 (m, 1H), 3.32 (s, 6H), 2.27 (s, 3H), 1.78-1.67 (m, 8H).

**Example 3256**

**2-[(3,4-Difluorophenyl)amino]-*N*-(*cis*-4-{ [4-(dimethylamino)-5-methylpyrimidin-2-yl]amino}cyclohexyl) nicotinamide**

**Step A: Synthesis of 2-[(3,4-difluorophenyl)amino]-*N*-(*cis*-4-{[4-(dimethylamino)-5-methylpyrimidin-2-yl] amino}cyclohexyl)nicotinamide.**

3,4-Difluoro-aniline (20.6 uL, 0.204 mmol) was dissolved in 1.0 mL of DMF. NaH (8.2 mg, 0.204 mmol) was added to the solution and allowed to stir for 10 minutes. 2-Chloro-*N*-[4-(4-dimethylamino-5-methyl-pyrimidin-2-ylamino) cyclohexyl]-nicotinamide (40 mg, 0.102 mmol) was added and the mixture was stirred for another 5 minutes. The reaction was heated via Smith Synthesizer at 200 °C for 1 hour. The crude was purified by silica column chromatography. The column was flushed with 200 mL mixture methanol and methylene (1:9) and 100 mL of methanol to give 2-[(3,4-difluoropheny)amino]-*N*-(*cis*-4-{[4-(dimethylamino)-5-methylpyrimidin-2-yl]amino}cyclohexyl)nicotinamide as a white solid. (30.0 mg, 61.2%)
ESI-MS m/z 482.5 (M + H$^+$) ; $^1$H NMR (400 MHz, $CDCl_3$) δ 8.32 (dd, $J$ = 4.8, 1.6 Hz, 1H), 7.92-7.86 (m, 1H), 7.71 (dd, $J$ = 7.6, $J$= 1.6 Hz, 1H), 7.64 (s, 1H), 7.19-7.03 (m, 2H), 6.76-6.73 (m, 1H), 6.34 (d, $J$ = 6.8 Hz, 1H), 4.95 (s, 1H),

4.11-4.03 (m, 2H), 2.96 (s, 6H), 2.15 (s, 3H), 1.90-1.68 (m, 8H).

**Example 3257**

***N*-(4-Chlorophenyl)-*N'*-(*cis*-4-{[4-(dimethylamino)-5-methylpyrimidin-2-yl]amino}cyclohexyl)-*N*-ethylurea trifluoroacetate**

**Step A: Synthesis of *N*-(4-chlorophenyl)-*N'*-(*cis*-4-{[4-(dimethylamino)-5-methylpyrimidin-2-yl]amino} cyclohexyl)-*N*-ethylurea trifluoroacetate.**

*cis*-$N^2$-(4-Amino-cyclohexyl)-5,$N^4$,$N^4$-trimethyl-pyrimidine-2,4-diamine (75 mg, 0.30 mmol) and 1,1 -carbonyldiimidazole (58 mg, 0.36 mmol) were dissolved in 1 mL of methylene chloride in a Smith Synthesizer vial and allowed to stir at room temperature overnight. To the vial, (4-chloro-phenyl)-ethyl-amine (94 mg, 0.60 mmol) was added. The solution was heated via Smith Synthesizer at 130 °C for 30 minutes. The solvent was evaporated, and 1 mL of methanol was added to the crude to redissolve it. The crude was then purified by HPLC to give *N*-(4-chlorophenyl)-*N'*-(*cis*-4-{[4-(dimethylamino)-5-methylpyrimidin-2-yl]amino}cyclohexyl)-*N*-ethylurea trifluoroacetate as a white solid. (25.0 mg, 15%)
ESI MS m/e 431.3 (M + $H^+$) ;$^1$H NMR (400 MHz, $CDCl_3$) δ 14.0 (s, 1H), 8.65 (d, *J* = 6.4 Hz, 1H), 7.53 (dd, *J*= 9.2, *J* = 2.4 Hz, 2H), 7.43 (b, 1H), 7.28 (dd, *J*= 9.2, *J* = 2.4 Hz, 2H), 4.48 (bs, 1H), 4.16 (bs, 1H), 3.99 (m, 2H), 3.39 (s, 6H), 2.34 (s, 3H), 1.84-1.60 (m, 8H), 1.21 (t, *J* = 7.0 Hz, 3H).

**Example 3258**

***N*-(*cis*-4-{[5-Methyl-4-(methylamino)pyrimidin-2-yl]amino}cyclohexyl)-2-{[2-(trifluoromethyl)pyrimidin-4-yl] oxy}acetamide trifluoroacetate**

**Step A: Synthesis of resin bound N-methylamine.**

2-(3,5 Dimethoxy-4-formyl)phenoxy ethyl polystyrene resin (1.0 gram; 0.90 mmol/gram) and methylamine 2 M in methanol (5.85 mL, 11.7 mmol) in 15 mL of $CH_2Cl_2$ was suspended in a fritted synthesis flask. To this suspension was added a solution of $NaBH(OAc)_3$ (.0117 mol) in $CH_2Cl_2$ (15 mL). After shaking the mixture overnight in a rotary shaker, the solution was removed by filtration. The resulting resin bound N-methylamine was washed sequentially with $CH_2Cl_2$, DMF, and MeOH. The washing sequence was repeated four times. The resin bound N-methylamine was dried under vacuum for 20 minutes.

**Step B: Synthesis of resin bound-4-(N-methyl-5 methyl-2-chloro)-pyrimidine.**

The resin bound N-methylamine was suspended in DMF (10 mL). To the resin suspension was added 2,4 dichloro-5-methyl-pyrimidine (1.35 mmol) followed by triethylamine (0.273 mL, 2.70 mmol). The reaction mixture was shaken overnight at room temperature. The solution was removed by filtration and the resin washed sequentially with DMF, $CH_2Cl_2$ and MeOH. The wash sequence was repeated four times. The resulting resin bound intermediate was dried under vacuum for 20 minutes..

**Step C: Synthesis of resin bound *cis*-N-(4-N-methyl-5 methyl-pyrimidyl-2yl)cyclohexane-1,4-diamine.**

The resin bound intermediate was divided up into three portions and each portion was transferred into a 5 mL Smith synthesizer reaction vessel. The resins (0.272 mmol) were separately suspended in anhydrous dioxane (3 mL). To each suspension was added *cis* 1,4 diamino cyclohexane (0.405 mmol), tris(dibenzylidineacetone)dipalladium (O) (0.027 mol), 2,2 bisdiphenylphosphino-1,1 binapthyl (BINAP) (0.081mmol) and sodium tert-butoxide (1.35 mmol). The reactions were heated in a microwave synthesizer at 140°C for 20 minutes. At the completion of the reaction, the resin suspension was transferred to 8 mL fritted tubes. The solutions were removed by filtration. The resins were sequentially washed with MeOH, $H_2O$, MeOH, $CH_2Cl_2$, and MeOH. The washing sequence was repeated three times. The resulting resin bound intermediate was dried under vacuum for 20 minutes.

**Step D: Synthesis of *cis*-N-[4-(4-N-methyl-5 methyl-pyrimidyl-2yl-amino)-cyclohexyl]-bromoacetamide.**

The resin bound intermediate (0.27 mmol) was suspended in DCM (3 mL). To the resin suspension was added bromoacetyl bromide (0.27 mmol) and DIEA (.094 mL; 0.54 mmol). The reaction was mixed in a rotary shaker for 45

minutes at room temperature. At the completion of the reaction, the solution was removed by filtration. The resin was sequentially washed with DCM, DMF, DCM, and MeOH. The washing sequence was repeated three times. The resulting resin bound intermediate was dried under vacuum for 20 minutes.

**Step E: Synthesis of *N*-(*cis*-4-{[5-methyl-4-(methylamino)pyrimidin-2-yl]amino}cyclohexyl)-2-{[2-(trifluoromethyl)pyrimidin-4-yl]oxy}acetamide trifluoroacetate.**

The resin bound intermediate from Step D (0.27 mmol) was transferred into a 5 mL microwave synthesizer vial. The resin was suspended in anhydrous DMF ( 2 mL). To the resin suspension was added 4 hydroxy-2-trifluoromethyl pyrimidine (0.54 mmol) and potassium carbonate (0.54 mmol) The reaction was heated in a microwave oven at 140°C for 30 minutes. At the completion of the reaction, the resin suspension was transferred to an 8 mL fritted tube. The solution was removed by filtration and the resin washed sequentially with DMF, DCM, MeOH. The wash sequence repeated three times.

After drying under vacuum for 20 minutes, the resin bound intermediate was treated with 5 mL of TFA solution (TFA /$CH_2Cl_2$ /$H_2O$ 20:20:1 v/v). The reaction was shaken for 2 hours and the TFA solution was collected after filtration. The TFA was removed by rotary evaporation and the compound subjected to purification by preparative HPLC to give *N*-(*cis*-4-{[5-methyl-4-(methylamino)pyrimidin-2-yl]amino}cyclohexyl)-2-{[2-(trifluoromethyl)pyrimidin-4-yl]oxy}acetamide trifluoroacetate (2.1 mg. 5%) as a white solid.

ESI MS m/e 440.3 M+H$^+$; $^1$H NMR (400MHz, $CD_3OD$) δ (ppm): 8.69 (m, 1H), 7.45 (m, 1H), 7.21-7.17 (m, 1H), 4.95 (m, 2H), 4.03 (bs, 1H), 3.82 (bs, 1H), 3.04 (s, 3H), 1.98 (s, 3H), 1.93-1.61 (m, 8H).

**Example 3259**

**2,2-Difluoro-*N*-(*cis*-4-{[4-methyl-6-(methylamino)pyrimidin-2-yl]amino}cyclohexyl)-1,3-benzodioxole-5-carboxamide trifluoroacetate**

**Step A: Synthesis of resin bound N-methylamine.**

2-(3,5 Dimethoxy-4-formyl)phenoxy ethyl polystyrene resin (1.0 gram; 0.94mmol/gram) and methylamine (0.0122 mol) in 15 mL of $CH_2Cl_2$ was suspended in a fritted synthesis flask. To this suspension was added a solution of $NaBH(OAC)_3$ (0.0122 mol) in $CH_2Cl_2$ (15 mL). After shaking the mixture overnight in a rotary shaker, the solution was removed by filtration. The resulting resin bound N-methylamine was washed sequentially with $CH_2Cl_2$, DMF, and MeOH. The washing sequence was repeated four times. The resin bound N-methylamine was dried under vacuum for 20 minutes.

**Step B: Synthesis of resin bound-4-(N-methyl-6 methyl-2-chloro)-pyrimidine.**

The resin bound N-methylamine was suspended in DMF (10 mL). To the resin suspension was added 2,4 dichloro-6-methyl-pyrimidine (1.41 mmol) followed by triethylamine (0.393 mL, 2.82 mmol). The reaction mixture was shaken at 40 °C overnight. The solution was removed by filtration and the resin washed sequentially with DMF, $CH_2Cl_2$ and MeOH. The wash sequence was repeated four times. The resulting resin bound intermediate was dried under vacuum for 20 minutes.

**Step C: Synthesis of resin bound *cis*-N-(4-N-methyl-6methyl-pyrimidyl-2yl)cyclohexane-1,4-diamine.**

The resin bound intermediate was divided up into three portions and each portion was transferred into a 5 mL Smith synthesizer reaction vessel. The resins (0.282 mmol) were separately suspended in a 1:1 solution of IPA/H20 (3 mL). To each suspension was added *cis* 1,4 diamino cyclohexane (0.85 mmol) and DIEA (0.295ml; 1.69 mmol). The reactions were heated in a microwave synthesizer at 180°C for 4.5 hours. The resins were pooled together; and the solution removed by filtration. The resin was sequentially washed with IPA, $H_2O$, MeOH, $CH_2Cl_2$, and MeOH. The washing sequence was repeated three times. The resulting resin bound intermediate was dried under vacuum for 20 minutes.

**Step D: Synthesis of 2,2-difluoro-*N*-(*cis*-4-{[4-methyl-6-(methylamino)pyrimidin-2-yl]amino}cyclohexyl)-1,3-benzodioxole-5-carboxamide trifluoroacetate.**

The resin bound intermediate was suspended in DMF (8mL). To the resin suspension was added the 2,2 diflouro 1,3 benzodioxole 5-carbonyl chloride (0.846 mmol) and triethylamine ( 0.256 mL; 1.69 mmol). The reaction

was shaken in a rotary mixer at room temperature for 45 minutes. The solution was removed by filtration and the resin washed sequentially with DMF, $CH_2Cl_2$, MeOH. The wash sequence repeated three times.

After drying under vacuum for 20 minutes, the resin bound intermediate was treated with 15 mL of TFA solution (TFA /$CH_2Cl_2$ /$H_2O$ 20:20:1 v/v). The reaction was shaken for 2 hours and the TFA solution was collected after filtration. The TFA was removed by rotary evaporation and the compound subjected to purification by preparative HPLC to give 2,2-difluoro-*N*-(*cis*-4-{[4-methyl-6-(methylamino)pyrimidin-2-yl]amino}cyclohexyl)-1,3-benzodioxole-5-carboxamide trifluoroacetate (2.0 mg. 2%) as a white solid.

ESI MS m/e 420.5 $M+H^+$; $^1H$ NMR (400MHz, $CD_3OD$) δ (ppm): 8.24 (m, 1H), 7.72-7.68 (m, 2H), 7.31-7.29 (m, 1H), 5.86 (s, 1H), 4.18-3.99 (m, 2H), 2.99 (s, 3H), 2.25 (s, 3H), 1.93-1.80 (m, 8H).

**Examples 3260-3262**

Compounds 3260 to 3262 were prepared in a similar manner as described in Example 3242 using the appropriate bromoacetophemone and amine intermediate from Step A.

**Examples 3263-3267**

Compounds 3263 to 3267 were prepared in a similar manner as described in Example 3243 using the appropriate acid and amine intermediate from Step A.

**Examples 3268-3272**

Compounds 3268 to 3272 were prepared in a similar manner as described in Example 3244 using the appropriate isocyanate and amine intermediate from Step A.

**Examples 3273-3275**

Compounds 3723 to 3275 were prepared in a similar manner as described in Example 3245 using the appropriate amine and carboxylic intermediate from Step A.

**Examples 3276-3280**

Compounds 3276 to 3280 were prepared in a similar manner as described in Example 3246 using the appropriate acid chloride and amine intermediate from Step D.

**Examples 3281-3291**

Compounds 3281 to 3291 were prepared in a similar manner as described in Example 2656 using the appropriate thioderivative and amine intermediate from Step A.

**Examples 3292-3303**

Compounds 3292 to 3303 were prepared in a similar manner as described in Example 3251 using the appropriate amine and carboxylic intermediate from Step B.

**Examples 3304-3307**

Compounds 3304 to 3307 were prepared in a similar manner as described in Example 3252 using the appropriate amine and carboxylic intermediate from Step B.

**Examples 3308**

Compounds 3308 were prepared in a similar manner as described in Example 3251 using the appropriate amine and carboxylic intermediate from Step B.

**Examples 3309-3315**

Compounds 3309 to 3315 were prepared in a similar manner as described in Example 3252 using the appropriate amine and carboxylic intermediate from Step B.

**Examples 3316-3320**

Compounds 3316 to 3320 were prepared in a similar manner as described in Example 3253 using the appropriate aldehyde and amine intermediate from Step D.

**Examples 3321-3345**

Compounds 3321 to 3345 were prepared in a similar manner as described in Example 3255 using the appropriate isocyate and amine intermediate from Step A.

**Examples 3346-3355**

Compounds 3346 to 3355 were prepared in a similar manner as described in Example 3257 using the appropriate aniline and amine intermediate from Step A.

**Examples 3356-3357**

Compounds 3356 to 3357 were prepared in a similar manner as described in Example 2638 using the appropriate hydroxyaryl derivative and bromide intermediate from Step B.

**Examples 3358-3359**

Compounds 3358 to 3359 were prepared in a similar manner as described in Example 3259 using the appropriate acid chloride and amine intermediate from Step D.

**Examples 3360-3365**

Compounds 3360 to 3365 were prepared in a similar manner as described in Example 3259 using the appropriate hydroxyaryl derivative and bromide intermediate from Step E.

**Examples 3366-3367**

Compounds 3366 to 3367 were prepared in a similar manner as described in Example 3250 using the appropriate acid chloride derivative and amine intermediate from Step E.

**Examples 3368-3381**

Compounds 3368 to 3381 were prepared in a similar manner as described in Example 3249 using the appropriate thiophenol and nicotinamide intermediate from Step A.

**Example 3382**

Compound 3382 was prepared in a similar manner as described in Example 2497 using 4-trifluoromethoxybenzoyl chloride and the amine intermediate from Step E.

| Ex. No. | compound name | MS | class |
|---|---|---|---|
| 3260 | 1-(4-chlorophenyl)-2-[(cis-4-{[4-(dimethylamino)-5-methylpyrimidin-2-yl] amino}cyclohexyl)amino]ethanone | 402.4 (M + H) | 2 |
| 3261 | 1-(3,4-difluorophenyl)-2-[(cis-4-{[4-(dimethylamino)-5-methylpyrimidin-2-yl] amino}cyclohexyl)amino]ethanone | 404.4 (M + H) | 3 |
| 3262 | 1-(4-bromophenyl)-2-[(cis-4-{[4-(dimethylamino)-5-methylpyrimidin-2-yl] amino}cyclohexyl)amino]ethanone | 446.3 (M + H) | 3 |

(continued)

| Ex. No. | compound name | MS | class |
|---|---|---|---|
| 3263 | N-{1-[3,5-bis(trifluoromethyl)phenyl]-1-methylethyl}-N'-(cis-4-{[4-(dimethylamino)-6-methylpyrimidin-2-yl]amino}-cyclohexyl)urea | 547.6 (M + H) | 2 |
| 3264 | N [1-(4-chlorophenyl)-1-methylethyl]-N'-(cis-4-{[4-(dimethylamino)-5-methylpyrimidin-2-yl]amino)}cyclohexyl)urea | 445.3 (M + H) | 1 |
| 3265 | N-[1-(4-chlorophenyl)-1-methylethyl]-N'-(cis-4-{[4-(dimethylamino)-6-methylpyrimidin-2-yl]amino}cyclohexyl)urea | 445.2 (M + H) | 2 |
| 3266 | N-[1-(4-chlorophenyl)cyclopropyl]-N'-(cis-4-{[4-(dimethylamino)-5-methylpyrimidin-2-yl]amino}cyclohexyl)urea | 443.3 (M + H) | 1 |
| 3267 | N-[1-(4-chlorophenyl)cyclopropyl]-N'-(cis-4-{[4-(dimethylamino)-6-methylpyrimidin-2-yl]amino}cyclohexyl)urea | 443.4 (M + H) | 1 |
| 3268 | N-[1-[3,5-bis(trifluoromethyl)phenyl]-1-methylethyl}-N'-(cis-4-{[4-(dimethylamino)-6-methylpyrimidin-2-yl]amino}cyclohexyl)-N-methylurea | 561.4 (M + H) | 3 |
| 3269 | N-[1-(4-chlorophenyl)-1-methylethyl]-N'-(cis-4-{[4-(dimethylamino)-5-methylpyrimidin-2-yl]amino}cyclohexyl)-N-methylurea | 459.6 (M + H) | 1 |
| 3270 | N-[1-(4-chlorophenyl)-1-methylethyl]-N'-(cis-4-{[4-(dimethylamino)-6-methylpyrimidin-2-yl]amino}cyclohexyl)-N-methylurea | 459.5 (M + H) | 2 |
| 3271 | N-[1-(4-chlorophenyl)cyclopropyl]-N'-(cis-4-{[4-(dimethylamino)-5-methylpyrimidin-2-yl]amino}cyclohexyl)-N-methylurea | 457.5 (M + H) | 2 |
| 3272 | N-[1-(4-chlorophenyl)cyclopropyl]-N'-(cis-4-{[4-(dimethylamino)-6-methylpyrimidin-2-yl]amino}cyclohexyl)-N-methylurea | 457.2 (M + H) | 3 |
| 3273 | cis-N-[1-(4-chlorophenyl)-1-methylethyl]-4-{[4-(dimethylamino)-5-methylpyrimidin-2-yl]amino}cyclohexanecarboxamide | 430.3 (M + H) | 2 |
| 3274 | cis-N-[1-(4-chlorophenyl)-1-methylethyl]-4-{[4-(dimethylamino)-6-methylpyrimidin-2-yl]amino}cyclohexanecarboxamide | 430.4 (M + H) | 3 |
| 3275 | cis-N-{1-[3,5-bis(trifluoromethyl)phenyl]-1-methylethyl}-4-{[4-(dimethylamino)-6-methylpyrimidin-2-yl]amino}cyclohexanecarboxamide | 532.3 (M + H) | 3 |
| 3276 | 4-chloro-N-{cis-4-[(4-methoxy-5-methylpyrimidin-2-yl)amino]cyclohexyl}benzamide | 375.1 (M + H) | 3 |
| 3277 | N-{cis-4-[(4-methoxy-5-methylpyrimidin-2-yl)amino]cyclohexyl}-4-(trifluoromethoxy)benzamide | 425.1 (M + H) | 3 |
| 3278 | 3,4-dichloro-N-{cis-4-[(4-methoxy-5-methylpyrimidin-2-yl)amino]cyclohexyl}benzamide | 408.9 (M + H) | 2 |
| 3279 | 3,5-dichloro-N-{cis-4-[(4-methoxy-5-methylpyrimidin-2-yl)amino]cyclohexyl}benzamide | 409.1 (M + H) | 3 |
| 3280 | N-{cis-4-[(4-methoxy-5-methylpyrimidin-2-yl)amino]cyclohexyl}-3,5-bis(trifluoromethyl)benzamide | 477.2 (M + H) | 3 |
| 3281 | N-(cis-4-{[4-(dimethylamino)-5-methylpyrimidin-2-yl]amino}cyclohexyl)-2-[(4-fluorophenyl)sulfonyl]nicotinamide | 513.4 (M + H) | 1 |
| 3282 | 2-[(4-chlorophenyl)sulfonyl]-N-(cis-4-{[4-(dimethylamino)-5-methylpyrimidin-2-yl]amino}cyclohexyl)nicotinamide | 529.4 (M + H) | 1 |
| 3283 | 2-[(3-chlorophenyl)sulfonyl]-N-(cis-4-{[4-(dimethylamino)-5-methylpyrimidin-2-yl]amino}cyclohexyl)nicotinamide | 529.4 (M + H) | 2 |
| 3284 | 2-[(2-chlorophenyl)sulfonyl]-N-(cis-4-{[4-(dimethylamino)-5-methylpyrimidin-2-yl]amino}cyclohexyl)nicotinamide | 529.3 (M + H) | 2 |

(continued)

| Ex. No. | compound name | MS | class |
|---|---|---|---|
| 3285 | 2-[(3-bromophenyl)sulfonyl]-N-(cis-4-{[4-(dimethylamino)-5-methylpyrimidin-2-yl]amino}cyclohexyl)nicotinamide | 573.6 (M + H) | 2 |
| 3286 | N-(cis-4-{[4-(dimethylamino)-5-methylpyrimidin-2-yl]amino}cyclohexyl)-2-[(4-methoxyphenyl)sulfonyl]-nicotinamide | 525.4 (M + H) | 3 |
| 3287 | N-(cis-4-{[4-(dimethylamino)-5-methylpyrimidin-2-yl]amino}cyclohexyl)-2-{[3-(trifluoromethyl)phenyl]sulfonyl}-nicotinamide | 563.5 (M + H) | 2 |
| 3288 | N-(cis-4-{[4-(dimethylamino)-5-methylpyrimidin-2-yl]amino}cyclohexyl)-2-[(4-methylphenyl)sulfonyl]nicotinamide | 509.6 (M + H) | 2 |
| 3289 | 2-[(4-bromophenyl)sulfonyl]-N-(cis-4-{[4-(dimethylamino)-5- methylpyrimidin-2-yl]amino}cyclohexyl)nicotinamide | 573.5 (M + H) | 2 |
| 3290 | N-(cis-4-{[4-(dimethylamino)-5-methylpyrimidin-2-yl]amino}cyclohexyl)-2-[(2-methyl-3-furyl)sulfonyl]nicotinamide | 499.4 (M + H) | 3 |
| 3291 | N-(cis-4-{[4-(dimethylamino)-5-methylpyrimidin-2-yl]amino}cyclohexyl)-2-{[4-(trifluoromethyl)phenyl]sulfonyl}-nicotinamide | 563.5 (M + H) | 2 |
| 3292 | cis-N-[(1S)-1-(4-chlorophenyl)ethyl]-4-{[4-(dimethylamino)-5-methylpyrimidin-2-yl]amino}cyclohexanecarboxamide | 416.3 (M + H) | 2 |
| 3293 | cis-N-{(1S)-1-[3,5-bis(trifluoromethyl)phenyl]ethyl)}-4-{[4-(dimethylamino)-5-methylpyrimidin-2-yl]amino}cyclohexanecarboxamide | 518.4 (M + H) | 3 |
| 3294 | cis-4-{[4-(dimethylamino)-5-methylpyrimidin-2-yl]amino}-N-[(1R)-1-(2-fluorophenyl)ethyl]cyclohexanecarboxamide | 400.3 (M + H) | 3 |
| 3295 | cis-4-{[4-(dimethylamino)-5-methylpyrimidin-2-yl]amino}-N-[(1S)-1-(2-fluorophenyl)ethyl]cyclohexanecarboxamide | 400.3 (M + H) | 3 |
| 3296 | cis-N-[(1S)-1-(4-bromophenyl)ethyl]-4-{[4-(dimethylamino)-5-methylpyrimidin-2-yl]amino}cyclohexanecarboxamide | 460.3 (M + H) | 1 |
| 3297 | cis-N-[(1R)-1-(4-bromophenyl)ethyl]-4-{[4-(dimethylamino)-5-methylpyrimidin-2-yl]amino}cyclohexanecarboxamide | 460.3 (M + H) | 2 |
| 3298 | 4-{[4-(dimethylamino)-5-methylpyrimidin-2-yl]amino}-N-{(1S)-1-[3-(trifluoromethyl)phenyl]ethyl}cyclohexanecarboxamide | 450.2 (M + H) | 1 |
| 3299 | 4 {[4-(dimethylamino)-5-methylpyrimidin-2-yl]amino}-N-{(1R)-1-[3-(trifluoromethyl)phenyl]ethyl}cyclohexanecarboxamide | 450.3 (M + H) | 1 |
| 3300 | 4-{[4-(dimethylamino)-5-methylpyrimidin-2-yl]amino}-N-{(1S)-1-[2-(trifluoromethyl)phenyl]ethyl}cyclohexanecarboxamide | 450.4 (M + H) | 1 |
| 3301 | 4-{[4-(dimethylamino)-5-methylpyrimidin-2-yl]amino)-N-[(1R)-1-(2-(trifluoromethyl)phenyl]ethyl}cyclohexanecarboxamide | 450 (M + H) | 3 |
| 3302 | cis-4-{[4-(dimethylamino)-5-methylpyrimidin-2-yl]amino}-N-{(1S)-1-[4-(trifluoromethoxy)phenyl]ethyl}-cyclohexanecarboxamide | 466.4 (M + H) | 1 |
| 3303 | cis-4-{[4-(dimethylamino)-5-methylpyrimidin-2-yl]amino}-N-{(1R)-1-[4-(trifluoromethoxy)phenyl]ethyl}-cyclohexanecarboxamide | 466.4 (M + H) | 2 |
| 3304 | cis-N-[(1S)-1-(4-bromophenyl)ethyl]-4-[(4-methylquinolin-2-yl)amino] cyclohexanecarboxamide | 466.4 (M + H) | 3 |
| 3305 | cis-N-[(1R)-1-(4-chlorophenyl)ethyl]-4-[(4-methylquinolin-2-yl)amino] cyclohexanecarboxamide | 422.3 (M + H) | 2 |
| 3306 | cis-N-[(1S)-1-(4-chlorophenyl)ethyl]-4-[(4-methylquinolin-2-yl)amino] cyclohexanecarboxamide | 422.4 (M + H) | 2 |

(continued)

| Ex. No. | compound name | MS | class |
|---|---|---|---|
| 3307 | cis-4-[(4-methylquinolin-2-yl)amino]-N-{(1R)-1-[3-(trifluoromethyl)phenyl] ethyl}cyclohexanecarboxamide | 456.3 (M + H) | 1 |
| 3308 | cis-N-[(1S)-1-(4-bromophenyl)ethyl]-4-{[4-(dimethylamino)-5-methylpyrimidin-2-yl]amino}cyclohexanecarboxamide | 460 (M + H) | 1 |
| 3309 | cis-N-{(1S)-1-[3,5-bis(trifluoromethyl)phenyl]ethyl}-4-[(4-methylquinolin-2-yl) amino]cyclohexanecarboxamide | 524.2 (M + H) | 2 |
| 3310 | cis-4-[(4-methylquinolin-2-yl)amino]-N-{(1R)-1-[4-(trifluoromethoxy)phenyl] ethyl}cyclohexanecarboxamide | 472.4 (M + H) | 3 |
| 3311 | cis-N-[(1R)-1-(2-fluorophenyl)ethyl]-4-[(4-methylquinolin-2-yl)amino] cyclohexanecarboxamide | 406.2 (M + H) | 3 |
| 3312 | cis-N-[(1S)-1-(2-fluorophenyl)ethyl]-4-[(4-methylquinolin-2-yl)amino] cyclohexanecarboxamide | 406.3 (M + H) | 1 |
| 3313 | cis-4-[(4-methylquinolin-2-yl)amino]-N-{(1R)-1-[2-(trifluoromethyl)phenyl] ethyl}cyclohexanecarboxamide | 456.2 (M + H) | 2 |
| 3314 | cis-4-[(4-methylquinolin-2-yl)amino]-N-{(1S)-1-[2-(trifluoromethyl)phenyl] ethyl}cyclohexanecarboxamide | 456.3 (M + H) | 1 |
| 3315 | cis-4-[(4-methylquinolin-2-yl)amino]-N-{(1S)-1-[3-(trifluoromethyl)phenyl] ethyl}cyclohexanecarboxamide | 456 (M + H) | 1 |
| 3316 | *trans*-2-(4-chlorophenyl)-N-(cis-4-{[4-(dimethylamino)-5-methylpyrimidin-2-yl] amino}cyclohexyl)-cyclopropanecarboxamide | 428.4 (M + H) | 1 |
| 3317 | *trans*-2-(3-chlorophenyl)-N-(cis-4-{[4-(dimethylamino)-5-methylp)yrimidin-2-yl]aminol}cyclohexyl)-cyclopropanecarboxamide | 428 (M + H) | 1 |
| 3318 | *trans*-2-(3,4-difluorophenyl)-N-(cis-4-{(4-(dimethylamino)-5-methylpyrimidin-2-yl]amino}cyclohexyl)-cyclopropanecarboxamide | 430.2 (M + H) | 1 |
| 3319 | *trans*-2-(3,4-dichlorophenyl)-N-(cis-4-{[4-(dimethylamino)-5-methylpyrimidin-2-yl]amino}cyclohexyl)-cyclopropanecarboxamide | 462.3 (M + H) | 1 |
| 3320 | *trans*-2-[3,5-bis(trifluoromethyl)phenyl]-N-(cis-4-{[4-(dimethylamino)-5-methylpyrimidin-2-yl]amino}cyclohexyl)-cyclopropanecarboxamide | 530.2 (M + H) | 1 |
| 3321 | N-(cis-4-{[4-(dimethylamino)-5-methylpyrimidin-2-yl]amino}cyclohexyl) -N'-(2-methoxyphenyl)urea | 399.3 (M + H) | 2 |
| 3322 | N-(cis-4-{[4-(dimethylamino)-5-methylpyrimidin-2-yl]amino}cyclohexyl) -N'-(3-methoxyphenyl)urea | 3993 (M + H) | 3 |
| 3323 | N-(3,4-dimethoxyphenyl)-N'-(cis-4-{[4-(dimethylamino)-5-methylpyrimidin-2-yl]amino}cyclohexyl)urea | 429.4 (M + H) | 1 |
| 3324 | N-(cis-4-{[4-(dimethylamino)-5-methylpyrimidin-2-yl]amino}cyclohexyl) -N'-(2-fluorophenyl)urea | 387.5 (M+H) | 2 |
| 3325 | N-(cis-4-{[4-(dimethylamino)-5-methylpyrimidin-2-yl]amino}cyclohexyl) -N'-(3-fluorophenyl)urea | 387.4 (M + H) | 2 |
| 3326 | N-(cis-4-{[4-(dimethylamino)-5-methylpyrimidin-2-yl]amino}cyclohexyl) -N'-(4-fluorophenyl)urea | 387.4 (M + H) | 1 |
| 3327 | N-(3,4-difluorophenyl)-N'-(cis-4-{[4-(dimethylamino)-5-methylpyrimidin-2-yl] amino}cyclohexyl)urea | 405.3 (M + H) | 2 |
| 3328 | N-(cis-4-{[4-(dimethylamino)-5-methylpyrimidin-2-yl]amino}cyclohexyl) -N'-[3-(trifluoromethyl)phenyl]urea | 437.3 (M+H) | |

(continued)

| Ex. No. | compound name | MS | class |
|---|---|---|---|
| 3329 | N-(cis-4-{[4-(dimethylamino)-5-methylpyrimidin-2-yl]amino}cyclohexyl) -N'-[4-(trifluoromethyl)phenyl] urea | 437.2 (M + H) | |
| 3330 | N-(cis-4-{[4-(dimethylamino)-5-methylpyrimidin-2-yl]amino}cyclohexyl) -N'-[2-(trifluoromethoxy)phenyl]urea | 453.1 (M + H) | 1 |
| 3331 | N-(3-chloro-4-fluorophenyl)-N'-(cis-4-{[4-(dimethylamino)-5-methylpyrimidin-2-yl]amino}cyclohexyl)urea | 421.1 (M + H) | 2 |
| 3332 | N-(cis-4-{[4-(dimethylamino)-5-methylpyrimidin-2-yl]amino}cyclohexyl) -N'-[4-fluoro-3-(trifluoromethyl)-phenyl]urea | 455.3 (M + H) | |
| 3333 | N-(4-chlorophenyl)-N'-(cis-4-{[4-(dimethylamino)-5-methylpyrimidin-2-yl] amino}cyclohexyl)urea | 403.2 (M + H) | 2 |
| 3334 | N-[3,5-bis(trifluoromethyl)phenyl]-N'-(cis-4-{[4-(dimethylamino)-5-methylpyrimidin-2-yl]amino}cyclohexyl)urea | 505.3 (M + H) | 2 |
| 3335 | N-(4-bromophenyl)-N'-(cis-4-{[4-(dimethylamino)-5-methylpyrimidin-2-yl] amino}cyclohexyl)urea | 447.1 (M + H) | 1 |
| 3336 | N-(cis-4-{[4-(dimethylamino)-5-methylpyrimidin-2-yl]amino}cyclohexyl) -N'-(2-methylphenyl)urea | 383.2 (M + H) | 2 |
| 3337 | N-(3,4-dichlorophenyl)-N'-(cis-4-{[4-(dimethylamino)-5-methylpyrimidin-2-yl] amino}cyclohexyl)urea | 437.3 (M + H) | 2 |
| 3338 | N-(2,4-dichlorophenyl)-N'-(cis-4-{[4-(dimethylamino)-5-methylpyrimidin-2-yl] amino)}cyclohexyl)urea | 437.3 (M + H) | 2 |
| 3339 | N-(3,5-dichlorophenyl)-N'-(cis-4-{[4-(dimethylamino)-5-methylpyrimidin-2-yl] amino}cyclohexyl)urea | 437.3 (M + H) | 2 |
| 3340 | N-(3-chlorophenyl)-N'-(cis-4-{[4-(dimethylamino)-5-methylpyrimidin-2-yl] amino}cyclohexyl)urea | 403.4 (M + H) | |
| 3341 | N-benzyl-N'-(cis-4-{[4-(dimethylamino)-5-methylpyrimidin-2-yl]amino} cyclohexyl)urea | 383.5 (M + H) | 2 |
| 3342 | N-(2,5-dichlorophenyl)-N'-(cis-4-{[4-(dimethylamino)-5-methylpyrimidin-2-yl] amino}cyclohexyl)urea | 437.3 (M + H) | 2 |
| 3343 | N-(2'3-dichlorophenyl)-N'-(cis-4-{[4-(dimethylamino)-5-methylpyrimidin-2-yl] amino}cyclohexyl)urea | 437.3 (M + H) | 3 |
| 3344 | N-[2-chloro-6-(trifluoromethyl)phenyl]-N'-(cis-4-{[4-(dimethylamino)-5-methylpyrimidin-2-yl]amino}cyclohexyl)urea | 471.3 (M + H) | 3 |
| 3345 | N-(cis-4-{[4-(dimethylamino)-5-methylpyrimidin-2-yl]amino}cyclohexyl) -N'-(2,4,6-trichlorophenyl)urea | 471.3 (M + H) | 1 |
| 3346 | N'-(cis-4-{[4-(dimethylamino)-5-methylpyrimidin-2-yl]amino}cyclohexyl) -N-(2-fluorophenyl)-N-methylurea | 401.2 (M + H) | 3 |
| 3347 | N-(2-chlorophenyl)-N'-(cis-4-{[4-(dimethylamino)-5-methylpyrimidin-2-yl] amino}cyclohexyl)-N-methylurea | 417.1 (M + H) | 2 |
| 3348 | N-(2,4-dichlorophenyl)-N'-(cis-4-{[4-(dimethylamino)-5-methylpyrimidin-2-yl] amino}cyclohexyl)-N-methylurea | 451.2 (M + H) | 1 |
| 3349 | N'-(cis-4-{[4-(dimethylamino)-5-methylpyrimidin-2-yl]amino}cyclohexyl)-N-ethyl-N-[2-(trifluoromethoxy)-phenyl]urea | 481.3 (M + H) | 2 |
| 3350 | N'-(cis-4-{[4-(dimethylamino)-5-methylpyrimidin-2-yl]amino}cyclohexyl)-N-ethyl-N-phenylurea | 397.1 (M + H) | 1 |

(continued)

| Ex. No. | compound name | MS | class |
|---|---|---|---|
| 3351 | N'-(cis-4-{[4-(dimethylamino)-5-methylpyrimidin-2-yl]amino}cyclohexyl)-N-ethyl-N-[4-(trifluoromethoxy)-phenyl]urea | 481.1 (M + H) | 1 |
| 3352 | N'-(cis-4-{[4-(dimethylamino)-5-methylpyrimidin-2-yl]amino}cyclohexyl)-N-methyl-N-[2-(trifluoromethoxy)-phenyl]urea | 467.2 (M + H) | 1 |
| 3353 | N-(4-chlorophenyl)-N'-(cis-4-{[4-(dimethylamino)-5-methylpyrimidin-2-yl] amino}cyclohexyl)-N-ethylurea | 431.3 (M + H) | 1 |
| 3354 | N-[3,5-bis(trifluoromethyl)phenyl]-N'-(cis-4-{[4-(dimethylamino)-5-methylpyrimidin-2-yl]amino}cyclohexyl)-N-ethylurea | 533.1 (M + H) | 1 |
| 3355 | N'-(cis-4-{[4-(dimethylamino)-5-methylpyrimidin-2-yl]amino}cyclohexyl)-N-ethyl-N-(3-methylphenyl)urea | 411.3 (M + H) | 1 |
| 3356 | N-(cis-4-{[4-(dimethylamino)-5-methylpyrimidin-2-yl]amino}cyclohexyl)-2-{[1-methyl-3-(trifluoromethyl)-1H-pyrazol-5-yl]oxy}acetamide | 456.4 (M + H) | 1 |
| 3357 | N-(cis-4-{[4-(dimethylamino)-5-methylpyrimidin-2-yl]amino}cyclohexyl)-2-{[6-(trifluoromethyl)pyrimidin-4-yl]oxy acetamide | 454.2 (M + H) | 3 |
| 3358 | 2,2-difluoro-N-(cis-4-{[4-methyl-6-(methylamino)pyrimidin-2-yl]amino} cyclohexyl)-1,3-benzodioxole-5-carboxamide | 420.5 (M + H) | |
| 3359 | 4-chloro-N-(cis-4-{[4-methyl-6-(methylamino)pyrimidin-2-yl]amino} cyclohexyl)-3-(trifluoromethyl)benzamide | 442.1 (M + H) | |
| 3360 | 2-(3,4-dichlorophenoxy)-N-(cis-4-{[4-methyl-6-(methylamino)pyrimidin-2-yl] amino}cyclohexyl)acetamide | 438.3 (M + H) | 1 |
| 3361 | N-(cis-4-{[5-methyl-4-(methylamino)pyrimidin-2-yl]amino}-cyclohexyl)-2-{(2-(trifluoromethyl)pyrimidin-4-yl]oxy}-acetamide | 440.3 (M + H) | |
| 3362 | N-(cis-4-{[5-methyl-4-(methylamino)pyrimidin-2-yl]amino}-cyclohexyl)-2-{[1-methyl-3-(trifluoromethyl)-1H-pyrazol-5-yl]oxy} acetamide | 442.5 (M + H) | 3 |
| 3363 | N-(cis-4-{[5-methyl-4-(methylamino)pyrimidin-2-yl]amino}-cyclohexyl)-2-{[6-(trifluoromethyl)pyrimidin-4-yl]oxy}acetamide | 440.3 (M + H) | 3 |
| 3364 | N-(cis-4-{[5-methyl-4-(methylamino)pyrimidin-2-yl]amino}-cyclohexyl)-2-{[1-methyl-5-(trifluoromethyl)-1H-pyrazol-3-yl]oxy} acetamide | 442.4 (M + H) | 3 |
| 3365 | N-(cis-4-{(5-methyl-4-(methylamino)pyrimidin-2-yl]amino}-cyclohexyl)-2-{[3-(trifluoromethyl)-1H-pyrazol-5-yl]oxy}-acetamide | 428.2 (M + H) | |
| 3366 | 3,4-difluoro-N-(cis-4-{[(4-methylquinolin-2-yl)methyl]amino}cyclohexyl) benzamide | 410.3 (M + H) | 3 |
| 3367 | 3-chloro-N-(cis-4-{[(4-methylquinolin-2-yl)methyl]amino}cyclohexyl) benzamide | 408.3 (M + H) | |
| 3368 | N-(cis-4-{[4-(dimethylamino)-6-methylpyrimidin-2-yl]amino}cyclohexyl)-2-[(4-fluorophenyl)sulfonyl]nicotinamide | 513.5 (M + H) | 2 |
| 3369 | 2-[(2-chlorophenyl)sulfonyl]-N-(cis-4-{[4-(dimethylamino)-6-methylpyrimidin-2-yl]amino}cyclohexyl)nicotinamide | 513.5 (M + H) | 2 |
| 3370 | 2-[(3-chlorophenyl)sulfonyl]-N-(cis-4-{[4-(dimethylamino)-6-methylpyrimidin-2-yl]amino}cyclohexyl)nicotinamide | 529.1 (M + H) | 2 |
| 3371 | 2-[(4-chlorophenyl)sulfonyl]-N-(cis-4-{[4-(dimethylamino)-6-methylpyrimidin-2-yl]amino}cyclohexyl)nicotinamide | 529.1 (M + H) | 3 |
| 3372 | 2-[(2-bromophenyl)sulfonyl]-N-(cis-4-{[4-(dimethylamino)-6-methylpyrimidin-2-yl]amino}cyclohexyl)nicotinamide | 573.3 (M + H) | 3 |

(continued)

| Ex. No. | compound name | MS | class |
|---|---|---|---|
| 3373 | 2-[(3-bromophenyl)sulfonyl]-N-(cis-4-{[4-(dimethylamino)-6-3373 methylpyrimidin-2-yl]amino}cyclohexyl)nicotinamide | 575.4 (M + H) | 2 |
| 3374 | 2-[(4-bromophenyl)sulfonyl]-N-(cis-4-{[4-(dimethylamino)-6-methylpyrimidin-2-yl]amino}cyclohexyl)nicotinamide | 573.2 (M + H) | 3 |
| 3375 | N-(cis-4-{[4-(dimethylamino)-6-methylpyrimidin-2-yl]amino}cyclohexyl)-2-[(2-methylphenyl)sulfonyl]nicotinamide | 509.5 (M + H) | 2 |
| 3376 | N-(cis-4-{[4-(dimethylamino)-6-methylpyrimidin-2-yl]amino}cyclohexyl)-2-[(3-methylphenyl)sulfonyl]nicotinamide | 509.5 (M + H) | 3 |
| 3377 | N-(cis-4-{[4-(dimethylamino)-6-methylpyrimidin-2-yl]amino}cyclohexyl)-2-[(4-methylphenyl)sulfonyl]nicotinamide | 509.5 (M + H) | 2 |
| 3378 | N-(cis-4-{[4-(dimethylamino)-6-methylpyrimidin-2-yl]amino}cyclohexyl)-2-[(2-methoxyphenyl)sulfonyl]-nicotinamide | 525.3 (M + H) | 3 |
| 3379 | N-(cis-4-{[4-(dimethylamino)-6-methylpyrimidin-2-yl]amino}cyclohexyl)-2-[(3-methoxyphenyl)sulfonyl]-nicotinamide | 525.3 (M + H) | 3 |
| 3380 | N-(cis-4-{[4-(dimethylamino)-6-methylpyrimidin-2-yl]amino}cyclohexyl)-2-[(4-methoxyphenyl)sulfonyl]-nicotinamide | 525.3 (M + H) | 3 |
| 3381 | N-(cis-4-{(4-(dimethylamino)-6-methylpyrimidin-2-yl]amino}cyclohexyl)-2-{[2-(trifluoromethyl)phenyl]-sulfonyl}nicotinamide | 563.4 (M + H) | 3 |
| 3382 | N-{cis-4-[(4-methylquinolin-2-yl)amino]cyclohexyl}-4-(trifluoromethoxy) benzamide | 444.4 (M + H) | 1 |

**Example 3383**

**4-Chloro-*N*-[*cis*-4-(4-dimethylamino-6-methyl-pyrimidin-2-ylamino)-cyclohexyl]-3- fluoro-benzamide hydrochloride**

**Step A: Synthesis of 4-chloro-*N*-[*cis*-4-(4-dimethylamino-6-methyl-pyrimidin-2-ylamino)-cyclohexyl]-3-fluoro-benzamide hydrochloride.**

To a solution of $N^2$-(*cis*-4-amino-cyclohexyl)-6,$N^4$,$N^4$-trimethyl-pyrimidine-2,4-diamine obtained in step A of example 3127 (300 mg) in DMF (3 mL) were added 4-chloro-3-fluoro-benzoic acid (252 mg), $Et_3N$ (0.42 mL), HOBt-$H_2O$ (276 mg), and EDC-HCl (277 mg). The reaction mixture was stirred at ambient temperature for 1 day. The reaction mixture was poured into saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (NH-silica gel, 15% to 60% EtOAc in hexane). The solution of the above purified material in EtOAc (5 mL) was added 4 M hydrogen chloride in EtOAc (10 mL). The mixture was stirred at ambient temperature for 1 hr and concentrated. The residue was suspended in $Et_2O$ (20 mL) and the suspension was stirred at ambient temperature for 2 hr. The precipitate was collected by filtration, washed with $Et_2O$, and dried at 80 °C under reduced pressure to give 4-chloro-*N*-[*cis*-4-(4-dimethylamino-6-methyl-pyrimidin-2-ylamino)-cyclohexyl]-3-fluoro-benzamide hydrochloride (335 mg) as a white solid.
ESI MS m/e 406, M (free) + $H^+$;$^1$H NMR (300 MHz, $CDCl_3$) $\delta$ 1.64-2.01 (m, 8 H), 2.35 (s, 3 H), 3.14 (s, 3H), 3.26 (s, 3 H), 4.02-4.31 (m, 2 H), 5.74 (s, 1 H), 6.84-6.96 (m, 1 H), 7.40-7.49 (m, 1 H), 7.53-7.60 (m, 1 H), 7.69 (dd, *J* = 9.7, 1.9 Hz, 1 H), 8.48-8.65 (m, 1 H), 12.93-13.08 (m, 1 H).

**Example 3384**

**3-Chloro-*N*-[*cis*-4-(4-dimethylamino-6-methyl-pyrimidin-2-ylamino)-cyclohexyl]-5-fluoro-benzamide hydrochloride**

**Step A: Synthesis of 3-chloro-*N*-[*cis*-4-(4-dimethylamino-6-methyl-pyrimidin-2-ylamino)-cyclohexyl]-5-fluoro-benzamide hydrochloride.**

Using the procedure for the step A of example 3383, the title compound was obtained.
ESI MS m/e 406, M (free) + $H^+$; $^1H$ NMR (300 MHz, $CDCl_3$) δ 1.64-2.05 (m, 8 H), 2.36 (s, 3 H), 3.15 (s, 3 H), 3.26 (s, 3 H), 4.01-4.30 (m, 2 H), 5.75 (s, 1 H), 6.45-6.54 (m, 1 H), 7.17-7.23 (m, 1 H), 7.40-7.47 (m, 1 H), 7.57-7.61 (m, 1 H), 8.60-8.71 (m, 1 H), 13.07-13.19 (m, 1H).

**Example 3385**

***N*-[*cis*-4-(4-Dimethylamino-6-methyl-pyrimidin-2-ylamino)-cyclohexyl]-3,4,5-trifluoro-benzamide hydrochloride**

**Step A: Synthesis of *N*-[*cis*-4-(4-dimethylamino-6-methyl-pyrimidin-2-ylamino)-cyclohexyl]-3,4,5-trifluoro-benzamide hydrochloride.**

Using the procedure for the step A of example 3383, the title compound was obtained.
ESI MS m/e 408, M (free) + $H^+$; $^1H$ NMR (300 MHz, $CDCl_3$) δ 1.62-2.01 (m, 8 H), 2.36 (s, 3 H), 3.14 (s, 3 H), 3.26 (s, 3 H), 4.00-4.32 (m, 2 H), 5.75 (s, 1 H), 6.70-6.81 (m, 1 H), 7.47-7.59 (m, 2 H), 8.54-8.66 (m, 1 H), 12.92-13.08 (m, 1 H).

**Example 3386**

**3-Chloro-4-fluoro-*N*-[*cis*-4-(5-methyl-4-methylamino-pyrimidin-2-ylamino)-cyclohexyl]-benzamide hydrochloride**

**Step A: Synthesis of (2-chloro-5-methyl-pyrimidin-4-yl)-methyl-amine.**

To the solution of 2,4-dichloro-5-methylpyrimidine (5.00 g) in THF (50 mL) were added $iPr_2NEt$ (6.4 mL) and 40% aqueous $MeNH_2$ (4.78 mL). The mixture was stirred at ambient temperature for 12 hr and concentrated under reduced pressure. To the residue was added saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 9% to 20% EtOAc in hexane) to give (2-chloro-5-methyl-pyrimidin-4-yl)-methyl-amine (3.55 g) as a white solid.
ESI MS m/e 408, M + $H^+$; $^1H$ NMR (300 MHz, $CDCl_3$) δ 2.01 (d, $J$ = 0.8 Hz, 3 H), 3.07 (d, $J$ = 5.0 Hz, 3 H), 4.89-5.06 (m, 1 H), 7.79 (s, 1 H).

**Step B: Synthesis of 3-chloro-4-fluoro-*N*-[*cis*-4-(5-methyl-4-methylamino-pyrimidin-2-ylamino)-cyclohexyl]-benzamide hydrochloride.**

Using the procedure for the step B of example 3228, the title compound was obtained.
ESI MS m/e 392, M (free) + $H^+$; $^1H$ NMR (300 MHz, DMSO-$d_6$) δ 1.64-1.98 (m, 1 H), 2.94 (d, $J$ = 4.5 Hz, 3 H), 3.80-4.08 (m, 2 H), 7.48-7.67 (m, 2 H), 7.87-7.95 (m, 1 H), 8.08-8.51 (m, 4 H), 11.95-12.03 (m, 1H).

**Example 3387**

**4-Chloro-*N*-[*cis*-4-(4-dimethylamino-5-methyl-pyrimidin-2-ylamino)-cyclohexyl]-3-fluoro-benzamide hydrochloride**

**Step A: Synthesis of 4-chloro-*N*-[*cis*-4-(4-dimethylamino-5-methyl-pyrimidin-2-ylamino)-cyclohexyl]-3-fluoro-benzamide hydrochloride.**

To a solution of $N^2$-(*cis*-4-amino-cyclohexyl)-5,$N^4$,$N^4$-trimethyl-pyrimidine-2,4-diamine obtained in step C of example 3119 (250 mg) in DMF (4 mL) were added 4-chloro-3-fluoro-benzoic acid (209 mg), $Et_3N$ (0.36 mL), HOBt-

$H_2O$ (230 mg), and EDC-HCl (230 mg). The reaction mixture was stirred at ambient temperature for 16 hr. The reaction mixture was poured into saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (NH-silica gel, 20% to 40% EtOAc in hexane). The solution of the above purified material in EtOAc (10 mL) was added 4 M hydrogen chloride in EtOAc (0.5 mL). The mixture was stirred at ambient temperature for 1 hr and concentrated. The residue was suspended in $Et_2O$ (10 mL) and the suspension was stirred at ambient temperature for 4 hr. The precipitate was collected by filtration, washed with $Et_2O$ and dried at 80 °C under reduced pressure to give 4-chloro-*N*-[*cis*-4-(4-dimethylamino-5-methyl-pyrimidin-2-ylamino)-cyclohexyl]-3-fluoro-benzamide hydrochloride (208 mg) as a white solid.
ESI MS m/e 406, M (free) + $H^+$; $^1H$ NMR (300 MHz, $CDCl_3$) $\delta$ 1.65-2.00 (m, 8 H), 2.26 (s, 3 H), 3.31 (s, 6 H), 3.98-4.27 (m, 2 H), 6.53-6.72 (m, 1 H), 7.20-7.27 (m, 1 H), 7.41-7.59 (m, 2 H), 7.64-7.73 (m, 1 H), 8.53-8.73 (m, 1 H), 12.76-12.95 (m, 1 H).

**Example 3388**

**3-Chloro-*N*-[*cis*-4-(4-dimethylamino-5-methyl-pyrimidin-2-ylamino)-cyclohexyl]-5-fluoro-benzamide hydrochloride**

**Step A: Synthesis of 3-chloro-*N*-[*cis*-4-(4-dimethylamino-5-methyl-pyrimidin-2-ylamino)-cyclohexyl]-5-fluoro-benzamide hydrochloride.**

Using the procedure for the step A of example 3387, the title compound was obtained.
ESI MS m/e 406, M (free) + $H^+$; $^1H$ NMR (300 MHz, $CDCl_3$) $\delta$ 1.64-2.01 (m, 8 H), 2.26 (s, 3 H), 3.30 (s, 6 H), 4.02-4.25 (m, 2 H), 7.02-7.28 (m, 3 H), 7.46-7.53 (m, 1 H), 7.63-7.68 (m, 1 H), 8.48-8.60 (m, 1 H), 12.70-12.84 (m, 1 H).

**Example 3389**

***N*-[*cis*-4-(4-Dimethylamino-5-methyl-pyrimidin-2-ylamino)-cyclohexyl]-3,4,5-trifluoro-benzamide hydrochloride**

**Step A: Synthesis of *N*-[*cis*-4-(4-dimethylamino-5-methyl-pyrimidin-2-ylamino)-cyclohexyl]-3,4,5-trifluoro-benzamide hydrochloride.**

Using the procedure for the step A of example 3387, the title compound was obtained.
ESI MS m/e 408, M (free) + $H^+$; $^1H$ NMR (300 MHz, $CDCl_3$) $\delta$ 1.60-2.02 (m, 8 H), 2.26 (s, 3 H), 3.31 (s, 6 H), 4.01-4.26 (m, 2 H), 6.65-6.76 (m, 1 H), 7.21-7.29 (m, 1 H), 7.48-7.60 (m, 2 H), 8.57-8.69 (m, 1 H), 12.73-12.91 (m, 1 H).

**Example 3390**

***N*-[*cis*-4-(4-Dimethylamino-5-methyl-pyrimidin-2-ylamino)-cyclohexyl]-3,5-difluoro-benzamide hydrochloride**

**Step A: Synthesis of *N*-[*cis*-4-(4-dimethylamino-5-methyl-pyrimidin-2-ylamino)-cyclohexyl]-3,5-difluoro-benzamide hydrochloride.**

Using the procedure for the step D of example 3119, the title compound was obtained.
ESI MS m/e 390, M (free) + $H^+$; $^1H$ NMR (300 MHz, $CDCl_3$) $\delta$ 1.61-2.06 (m, 8 H), 2.26 (s, 3 H), 3.31 (s, 6 H), 4.01-4.29 (m, 2 H), 6.55-6.70 (m, 1 H), 6.84-7.01 (m, 1 H), 7.18-7.43 (m, 3 H), 8.54-8.71 (m, 1 H), 12.77-12.97 (m, 1 H).

**Example 3391**

**2-(3,4-Difluoro-phenyl)-*N*-[*cis*-4-(4-dimethylamino-5-methyl-pyrimidin-2-ylamino)-cyclohexyl]-acetamide hydrochloride**

**Step A: Synthesis of 2-(3,4-difluoro-phenyl)-*N*-[*cis*-4-(4-dimethylamino-5-methyl-pyrimidin-2-ylamino)-cyclohexyl]-acetamide hydrochloride.**

Using the procedure for the step A of example 3387, the title compound was obtained.
ESI MS m/e 404, M (free) + $H^+$; $^1H$ NMR (300 MHz, $CDCl_3$) $\delta$ 1.57-1.94 (m, 8 H), 2.24 (s, 3 H), 3.29 (s, 6 H), 3.47 (s,

2 H), 3.80-3.97 (m, 1 H), 4.05-4.18 (m, 1 H), 6.01-6.15 (m, 1 H), 6.95-7.28 (m, 4 H), 8.46-8.86 (m, 1 H), 12.81-13.01 (m, 1 H).

**Example 3392**

***N*-[*cis*-4-(4-Amino-5-methyl-pyrimidin-2-ylamino)-cyclohexyl]-3-chloro-4-fluoro-benzamide hydrochloride**

**Step A: Synthesis of 2-chloro-5-methyl-pyrimidin-4-ylamine.**

To the solution of 2,4-dichloro-5-methylpyrimidine (1.00 g) in IPA (2 mL) was added 28% aqueous $NH_3$ (2 mL). The mixture was heated in a microwave synthesizer at 120°C for 20 min. To the mixture was added saturated aqueous $NaHCO_3$ and the aqueous layer was extracted with $CHCl_3$ (three times). The combined organic layer was dried over $MgSO_4$, filtered, concentrated, and purified by medium-pressure liquid chromatography (NH-silica gel, 20% to 50% EtOAc in hexane) to give 2-chloro-5-methyl-pyrimidin-4-ylamine (151 mg) as a white solid.
ESI MS m/e 143, $M^+$; $^1H$ NMR (300 MHz, DMSO-$d_6$) δ 1.94 (s, 3 H), 7.81 (s, 1 H).

**Step B: Synthesis of *N*-[*cis*-4-(4-amino-5-methyl-pyrimidin-2-ylamino)-cyclohexyl]-3-chloro-4-fluoro-benzamide hydrochloride.**

Using the procedure for the step B of example 3228, the title compound was obtained.
ESI MS m/e 378, M (free) + $H^+$; $^1H$ NMR (300 MHz, DMSO-$d_6$) δ 1.63-1.94 (m, 8 H), 1.91 (s, 3 H), 3.79-4.00 (m, 2 H), 7.52 (t, J = 8.9 Hz, 1 H), 7.63-7.70 (m, 1 H), 7.78-7.99 (m, 2 H), 8.07-8.13 (m, 1 H), 8.28-8.48 (m, 1 H), 11.86-11.96 (m, 1 H).

**Example 3393**

**2-(3,4-Dichloro-phenoxy)-*N*-[*cis*-4-(4-dimethylamino-pyrimidin-2-ylamino)-cyclohexyl]-acetamide hydrochloride**

**Step A: Synthesis of 2-(3,4-dichloro-phenoxy)-*N*-[*cis*-4-(4-dimethylamino-pyrimidin-2-ylamino)-cyclohexyl]-acetamide hydrochloride.**

Using the procedure for the step A of example 3198, the title compound was obtained.
ESI MS m/e 438, M $(free)^+$ ; $^1H$ NMR (300 MHz, $CDCl_3$) δ 1.59-2.03 (m, 8 H), 3.17 (s, 3 H), 3.27 (s, 3 H), 3.88-4.08 (m, 1 H), 4.11-4.25 (m, 1 H), 4.43 (s, 2 H), 5.96 (d, *J* = 7.5 Hz, 1 H), 6.66-6.79 (m, 1 H), 6.88 (dd, *J* = 8.9,3.0 Hz, 1 H), 7.10 (d, *J* = 3.0 Hz, 1 H), 7.37 (d, *J* = 8.9 Hz, 1 H), 7.43-7.53 (m, 1 H), 8.69-8.83 (m, 1 H), 13.21 (brs, 1 H).

**Example 3394**

***N*-[*cis*-4-(4-Dimethylamino-pyrimidin-2-ylamino)-cyclohexyl]-2-(3-methoxy-phenoxy)-acetamide hydrochloride**

**Step A: Synthesis of *N*-[*cis*-4-(4-dimethylamino-pyrimidin-2-ylamino)-cyclohexyl]-2-(3-methoxy-phenoxy)-acetamide hydrochloride.**

Using the procedure for the step A of example 3198, the title compound was obtained.
ESI MS m/e 400, M (free) + $H^+$; $^1H$ NMR (300 MHz, $CDCl_3$) δ 1.63-2.03 (m, 8 H), 3.16 (s, 3 H), 3.27 (s, 3 H), 3.82 (s, 3 H), 3.92-4.08 (m, 1 H), 4.09-4.23 (m, 1 H), 4.45 (s, 2 H), 5.96 (d, *J* = 7.3 Hz, 1 H), 6.47-6.64 (m, 3 H), 6.75-6.90 (m, 1 H), 7.14-7.25 (m, 1 H), 7.40-7.56 (m, 1 H), 8.62-8.79 (m, 1 H), 13.29 (brs, 1 H).

**Example 3395**

***N*-[*cis*-4-(4-Dimethylamino-pyrimidin-2-ylamino)-cyclohexyl]-*C*-(ethyl-phenyl-amino)-acetamide dihydrochloride**

**Step A: Synthesis of *N*-[*cis*-4-(4-dimethylamino-pyrimidin-2-ylamino)-cyclohexyl]-*C*-(ethyl-phenyl-amino)-acetamide dihydrochloride.**

Using the procedure for the step A of example 3198, the title compound was obtained.
ESI MS m/e 397, M (free) + $H^+$ ; $^1$H NMR (300 MHz, DMSO-d6) δ 1.09 (t, *J* = 7.0 Hz, 3 H), 1.41-1.87 (m, 8 H), 3.14 (s, 3 H), 3.18 (s, 3 H), 3.43 (q, *J* = 7.0 Hz, 2 H), 3.60-3.80 (m, 1 H), 3.82-4.01 (m, 3H), 6.36 (d, *J* = 7.5 Hz, 1 H), 6.57-6.80 (m, 3 H), 7.06-7.28 (m, 2 H), 7.72-8.05 (m, 2 H), 8.20-8.42 (m, 1 H), 12.19 (brs, 1 H).

**Example 3396**

**5-Chloro-*N*-[*cis*-4-(4-methyl-quinolin-2-ylamino)-cyclohexyl]-nicotinamide**

**Step A: Synthesis of 5-bromo-*N*-[*cis*-4-(4-methyl-quinolin-2-ylamino)-cyclohexyl]-nicotinamide.**

To a solution of *N*-(*cis*-4-methyl-quinolin-2-yl)-cyclohexane-1,4-diamine obtained in step A of example 3070 (5.00 g) in DMF (50 mL) were added 5-bromo-nicotinic acid (4.74 g), $Et_3N$ (6.55 mL), HOBt-$H_2O$ (4.50 g), and EDC-HCl (4.51 g). The reaction mixture was stirred at ambient temperature for 16 hr. The reaction mixture was poured into saturated aqueous $NaHCO_3$ and the aqueous layer was extracted with $CHCl_3$ (three times). The combined organic layer was dried over $MgSO_4$, filtered, concentrated under reduced pressure, and purified by medium-pressure liquid chromatography (NH-silica gel, 20% to 40% EtOAc in hexane) to give 5-bromo-*N*-[*cis*-4-(4-methyl-quinolin-2-ylamino)-cyclohexyl]-nicotinamide (9.81 g) as a white solid.
ESI MS m/e 439, M + $H^+$; $^1$H NMR (300 MHz, $CDCl_3$) δ 1.67-2.84 (m, 8 H), 2.58 (s, 3 H), 4.07-4.24 (m, 2 H), 4.72-4.83 (m, 1 H), 6.11-6.20 (m, 1 H), 6.52 (s, 1 H), 7.20-7.28 (m, 1 H), 7.49-7.81 (m, 3 H), 8.23-8.29 (m, 1 H), 8.79 (d, *J* = 2.3 Hz, 1 H), 8.86 (d, *J* = 1.9 Hz, 1 H).

**Step B: Synthesis of 5-amino-*N*-[*cis*-4-(4-methyl-quinolin-2-ylamino)-cyclohexyl]-nicotinamide.**

To the solution of 5-bromo-*N*-[*cis*-4-(4-methyl-quinolin-2-ylamino)-cyclohexyl]-nicotinamide (6.00 g) in EtOH (40 mL) were added copper (2.17 g), cuprous chloride (3.37 g), and 28 % aqueous $NH_3$ (40.0 mL). The mixture was stirred at 180°C for 4 hr in a sealed tube. The mixture was filtrated through a pad of celite and the aqueous layer was extracted with $CHCl_3$ (three times). The combined organic layer was dried over $MgSO_4$, filtered, concentrated under reduced pressure, and purified by medium-pressure liquid chromatography (silica gel, 25% to 50% EtOAc in hexane) to give 5-amino-*N*-[*cis*-4-(4-methyl-quinolin-2-ylamino)-cyclohexyl]-nicotinamide (3.92 g) as a white solid.
ESI MS m/e 376, M + $H^+$; $^1$H NMR (300 MHz, $CDCl_3$) δ 1.66-2.04 (m, 8 H), 2.58 (s, 3 H), 3.88-4.24 (m, 4 H), 4.75-4.90 (m, 1 H), 6.18-6.31 (m, 1 H), 6.52 (s, 1 H), 7.19-7.29 (m, 1 H), 7.39-7.44 (m, 1 H), 7.48-7.58 (m, 1 H), 7.62-7.70 (m, 1 H), 7.73-7.80 (m, 1 H), 8.19 (d, *J* = 2.8 Hz, 1 H), 8.29 (d, *J* = 1.6 Hz, 1 H).

**Step C: Synthesis of 5-chloro-*N*-[*cis*-4-(4-methyl-quinolin-2-ylamino)-cyclohexyl]-nicotinamide.**

A mixture of conc. HCl (1.33 mL) and $NaNO_2$ (137.8 mg) was stirred at 70 °C for 10 min and cooled to ambient temperature. To the reaction mixture was added a solution of 5-amino-*N*-[*cis*-4-(4-methyl-quinolin-2-ylamino)-cyclohexyl]-nicotinamide (500 mg) in AcOH (45 mL) and the mixture was stirred at ambient temperature for 30 min. To the reaction mixture was added a solution of CuCl (460.8 mg) in conc. HCl (3.0 mL) and the mixture was stirred at 80 °C for 6 hr. The reaction mixture was alkalized with 1M aqueous NaOH and the aqueous layer was extracted with $CHCl_3$ (three times). The combined organic layer was dried over $MgSO_4$, filtered, concentrated, and purified by flash chromatography (NH-silica gel, 20% EtOAc in hexane and silica gel, 2% MeOH in $CHCl_3$) to give 5-chloro-*N*-[*cis*-4-(4-methyl-quinolin-2-ylamino)-cyclohexyl]-nicotinamide (52.3 mg) as a yellow solid.
ESI MS m/e 395, M (free) + $H^+$ ; $^1$H NMR (300 MHz, $CDCl_3$) δ 1.65-2.03 (m, 8 H), 2.57 (s, 3 H), 4.03-4.29 (m, 2 H), 5.05 (brs, 1 H), 6.33-6.44 (m, 1 H), 6.53 (s, 1 H), 7.19-7.28 (m, 1 H), 7.48-7.56 (m, 1 H), 7.61-7.67 (m, 1 H), 7.73-7.79 (m, 1 H), 8.08-8.13 (m, 1 H), 8.66 (d, *J* = 2.3 Hz, 1 H), 8.83 (d, *J* = 1.9 Hz, 1 H).

**Example 3397**

**5-Fluoro-*N*-[*cis*-4-(4-methyl-quinolin-2-ylamino)-cyclohexyl]-nicotinamide**

**Step A: Synthesis of 5-fluoro-*N*-[*cis*-4-(4-methyl-quinolin-2-ylamino)-cyclohexyl]-nicotinamide.**

To a solution of 5-amino-*N*-[*cis*-4-(4-methyl-quinolin-2-ylamino)-cyclohexyl]-nicotinamide obtained in step B of example 3396 (500 mg) in 48% aqueous $HBF_4$ (3.95 mL) and EtOH (4.00 mL) was added $CuF_2$ (132.0 mg) at ambient temperature. To the reaction mixture was added a solution of $NaNO_2$ (183.5 mg) in $H_2O$ (3.95 mL) and the mixture was stirred at ambient temperature for 1 hr. Then the mixture was stirred at 50°C for 2 hr and 80°C for 2 hr. The reaction mixture was alkalized with 1M aqueous NaOH and the aqueous layer was extracted with EtOAc (three times). The combined organic layer was dried over $MgSO_4$, filtered, concentrated, and purified by flash chromatography (NH-silica gel, 50% EtOAc in hexane) to give 5-fluoro-*N*-[*cis*-4-(4-methyl-quinolin-2-ylamino)-cyclohexyl]-nicotinamide (20.9 mg) as a yellow amorphous.
ESI MS m/e 379, M (free) + $H^+$ ; $^1H$ NMR (300 MHz, $CDCl_3$) δ 1.67-2.05 (m, 8 H), 2.57 (s, 3 H), 4.08-4.25 (m, 2 H), 4.72 (brs, 1 H), 6.17-6.29 (m, 1 H), 6.52 (s, 1 H), 7.19-7.28 (m, 1 H), 7.48-7.57 (m, 1 H), 7.62-7.69 (m, 1 H), 7.73-7.80 (m, 1 H), 7.82-7.91 (m, 1 H), 8.60 (d, *J* = 2.8 Hz, 1 H), 8.76 (t, *J* = 1.5 Hz, 1 H).

**Example 3398**

**3-chloro-*N*-[*cis*-4-(4-dimethylamino-5-methyl-pyrimidin-2-ylamino)-cyclohexyl]-4-fluoro-benzamide methanesulfonic acid**

**Step A: Synthesis of 3-chloro-*N*-[*cis*-4-(4-dimethylamino-5-methyl-pyrimidin-2-ylamino)-cyclohexyl]-4-fluoro-benzamide methanesulfonic acid.**

To a solution of *N*-(cis-4-amino-cyclohexyl)-3-chloro-4-fluoro-benzamide obtained in step A of example 3228 (1.76 g) in BuOH (2.5 mL) was added 2-chloro-4-dimethylamino-5-methylpyrimidine obtained in step A of example 3119 (1.00 g). The mixture was heated in a microwave synthesizer at 200°C for 15 min. The reaction was repeated 3 more times and the reaction mixtures were pooled. The mixture was poured into saturated aqueous $NaHCO_3$ and the aqueous layer was extracted with $CHCl_3$ (three times). The combined organic layer was dried over $MgSO_4$, filtrated, concentrated under reduced pressure, and purified by medium-pressure liquid chromatography (NH-silica gel, 15 % to 80% EtOAc in nexane) to give a colorless solid. To a solution of the above solid (1.85 g) in EtOH (18 mL) was added MsOH (460 mg). The mixture was stirred at ambient temperature for 30 min and stirred on an ice-bath for 4 hr. The precipitate was collected by filtration, washed with EtOH, and dried at 80°C under reduced pressure to give 3-chloro-*N*-[*cis*-4-(4-dimethylamino-5-methyl-pyrimidin-2-ylamino)-cyclohexyl]-4-fluoro-benzamide methanesulfonic acid (1.41 g) as a white solid.
ESI MS m/e 406, M (free) + $H^+$; $^1H$ NMR (300 MHz, $CDCl_3$) δ 1.60-2.03 (m, 8 H), 2.25 (s, 3 H), 2.89 (s, 3 H), 3.30 (s, 6 H), 4.07-4.30 (m, 2 H), 7.13-7.29 (m, 2 H), 7.38-7.49 (m, 1 H), 7.81-7.89 (m, 1 H), 7.96-8.05 (m, 1 H), 8.07 (dd, *J* = 7.1, 2.3 Hz, 1 H), 12.07-12.23 (m, 1 H).

**Assay Procedures**

**Example 3399**

**ASSAY FOR DETERMINATION OF CONSTITUTIVE ACTIVITY OF GPCRs**

**A. Intracellular $IP_3$ Accumulation Assay**

On day 1, cells to be tranfected can be plated onto 24 well plates, usually $1x10^5$ cells/well (although his umber can be optimized. On day 2 cells can be transfected by firstly mixing 0.25ug DNA (e.g., pCMV vector or pCMV vector comprising polynucleotide enocoding receptor) in 50 ul serum free DMEM/well and 2 ul lipofectamine in 50 µl serum-free DMEM/well. The solutions are gently mixed and incubated for 15-30 min at room temperature. Cells are washed with 0.5 ml PBS and 400 µl of serum free media is mixed with the transfection media and added to the cells. The cells are then incubated for 3-4 hrs at 37°C/5%$CO_2$ and then the transfection media is removed and replaced with 1ml/well of regular growth media. On day 3 the cells are labeled with $^3H$-myo-inositol. Briefly, the media is removed and the cells are washed with 0.5 ml PBS. Then 0.5 ml inositol-free/serum free media (GIBCO BRL) is added/well with 0.25 µCi of $^3H$-myo-inositol/ well and the cells are incubated for 16-18 hrs o/n at 37°C/5%$CO_2$ On Day 4 the cells are washed

with 0.5 ml PBS and 0.45 ml of assay medium is added containing inositol-free/serum free media 10µM pargyline 10 mM lithium chloride or 0.4 ml of assay medium and 50 ul of 10x ketanserin (ket) to final concentration of 10µM. The cells are then incubated for 30 min at 37°C. The cells are then washed with 0.5 ml PBS and 200 ul of fresh/ice cold stop solution (1M KOH; 18 mM Na-borate; 3.8 mM EDTA) is added/well. The solution is kept on ice for 5-10 min or until cells were lysed and then neutralized by 200 µl of fresh/ice cold neutralization sol. (7.5 % HCL). The lysate is then transferred into 1.5 ml eppendorf tubes and 1 ml of chloroform/methanol (1:2) is added/tube. The solution is vortexed for 15 sec and the upper phase is applied to a Biorad AG1-X8™ anion exchange resin (100-200 mesh). Firstly, the resin is washed with water at 1:1.25 W/V and 0.9 ml of upper phase is loaded onto the column. The column is washed with 10 mls of 5 mM myo-inositol and 10 ml of 5 mM Na-borate/60mM Na-formate. The inositol tris phosphates are eluted into scintillation vials containing 10 ml of scintillation cocktail with 2 ml of 0.1 M formic acid/ 1 M ammonium formate. The columns are regenerated by washing with 10 ml of 0.1 M formic acid/3M ammonium formate and rinsed twice with $H_2O$ and stored at 4°C in water.

**Example 3400**

**High Throughput Functional Screening: FLIPR™**

Subsequently, a functional based assay was used to confirm the lead hits, referred to as FLIPR™ (the Fluorometric Imaging Plate Reader) and FDSS6000™ (Functional Drug Screening System). This assay utilized a non-endogenous, constitutively active version of the MCH receptor.

The FLIPR and FDSS assays are able to detect intracellular $Ca^{2+}$ concentration in cells, which can be utilized to assess receptor activation and determine whether a candidate compound is an, for example, antagonist, inverse agonist or agonist to a Gq-coupled receptor. The concentration of free $Ca^{2+}$ in the cytosol of any cell is extremely low, whereas its concentration in the extracellular fluid and endoplasmic reticulum (ER) is very high. Thus, there is a large gradient tending to drive $Ca^{2+}$ into the cytosol across both the plasma membrane and ER. The FLIPR™ and FDSS6000™' systems (Molecular Devices Corporation, HAMAMATSU Photonics K.K.) are designed to perform functional cell-based assays, such as the measurement of intracellular calcium for high-throughput screening. The measurement of fluorescent is associated with calcium release upon activation of the Gq-coupled receptors. Gi or Go coupled receptors are not as easily monitored through the FLIPR™ and FDSS6000™ systems because these G proteins do not couple with calcium signal pathways.

Fluorometric Imaging Plate Reader system was used to allow for rapid, kinetic measurements of intracellular fluorescence in 96 well microplates (or 384 well microplates). Simultaneous measurements of fluorescence in all wells can be made by FLIPR or FDSS6000™ every second with high sensitivity and precision. These systems are ideal for measuring cell-based functional assays such as monitoring the intracellular calcium fluxes that occur within seconds after activation of the Gq coupled receptor.

Briefly, the cells are seeded into 96 well at $5.5 \times 10^4$ cells/well with complete culture media (Dulbecco's Modified Eagle Medium with 10 % fetal bovine serum, 2 mM L-glutamine, 1 mM sodium pyruvate and 0.5 mg/ml G418, pH7.4) for the assay next day. On the day of assay, the media is removed and the cells are incubated with 100 µl of loading buffer (4 µM Fluo4-AM in complete culture media containing 2.5 mM Probenicid, 0.5 mg/ml and 0.2% bovine serum albumin) in 5% $CO_2$ incubator at 37°C for 1 hr. The loading buffer is removed, and the cells are washed with wash buffer (Hank's Balanced Salt Solution containing 2.5 mM Probenicid, 20 mM HEPES, 0.5 mg/ml and 0.2% bovine serum albumin, pH 7.4)). One hundred fifty µl of wash buffer containing various concentrations of test compound is added to the cells, and the cells are incubated in 5% $CO_2$ incubator at 37°C for 30 min. Fifty µl of wash buffer containing various concentration of MCH are added to each well, and transient changes in $[Ca^{2+}]i$ evoked by MCH are monitored using the FLIPR or FDSS in 96 well plates at Ex. 488 nm and Em. 530 nm for 290 second. When antagonist activity of compound is tested, 50 nM of MCH is used.

Use of FLIPR™ and FDSS6000™ can be accomplished by following manufacturer's instruction (Molecular Device Corporation and HAMAMATSU Photonics K.K.).

Representative examples are shown below.

| Compound No. | $IC_{50}$ (nM) |
|---|---|
| Example 7 | 11 |
| Example 15 | 19 |
| Example 19 | 21 |
| Example 2524 | 2.1 |

(continued)

| Compound No. | $IC_{50}$ (nM) |
|---|---|
| Example 2526 | 7.6 |

The results were shown on the tables in the Examples section and the table in the next page in accordance with the classification as defined below.

Class 1 : The value of percent of control at $10^{-7}$ M was less than 40% or the value of $IC_{50}$ was less than 50 nM.
Class 2 : The value of percent of control at $10^{-7}$ M was from 40% to 60% or the value of $IC_{50}$ was from 50 nM to 200 nM.
Class 3 : The value of percent of control at $10^{-7}$ M was more than 60% or the value of $IC_{50}$ was more than 200 nM.

The compounds in Examples 2497 to 2542, 2588 to 2689, and 3241 to 3259 were tested and they showed $IC_{50}$ activities less than about 50 μM.

| Ex. No. | class | Ex. No. | class | Ex. No. | class | Ex. No. | class | Ex. No. | class | Ex. No. | class |
|---|---|---|---|---|---|---|---|---|---|---|---|
| 1 | 2 | 3058 | 1 | 3104 | 2 | 3150 | 3 | 3196 | 2 | 3384 | 1 |
| 2 | 2 | 3059 | 1 | 3105 | 2 | 3151 | 1 | 3197 | 2 | 3385 | 1 |
| 3 | 1 | 3060 | 2 | 3106 | 1 | 3152 | 1 | 3198 | 1 | 3386 | 1 |
| 4 | 1 | 3061 | 1 | 3107 | 1 | 3153 | 1 | 3199 | 1 | 3387 | 1 |
| 5 | 2 | 3062 | 1 | 3108 | 1 | 3154 | 1 | 3200 | 3 | 3388 | 1 |
| 6 | 2 | 3063 | 1 | 3109 | 1 | 3155 | 3 | 3201 | 1 | 3389 | 1 |
| 7 | 1 | 3064 | 1 | 3110 | 1 | 3156 | 3 | 3202 | 1 | 3390 | 1 |
| 8 | 1 | 3065 | 1 | 3111 | 1 | 3157 | 2 | 3203 | 1 | 3391 | 1 |
| 9 | 3 | 3066 | 1 | 3112 | 1 | 3158 | 1 | 3204 | 2 | 3392 | 3 |
| 10 | 2 | 3067 | 2 | 3113 | 3 | 3159 | 1 | 3205 | 2 | 3393 | 1 |
| 11 | 1 | 3068 | 2 | 3114 | 1 | 3160 | 1 | 3206 | 1 | 3394 | 1 |
| 12 | 2 | 3069 | 2 | 3115 | 1 | 3161 | 2 | 3207 | 1 | 3395 | 1 |
| 13 | 3 | 3070 | 1 | 3116 | 3 | 3162 | 2 | 3208 | 3 | 3396 | 1 |
| 14 | 1 | 3071 | 1 | 3117 | 1 | 3163 | 1 | 3209 | 3 | 3397 | 1 |
| 15 | 1 | 3072 | 1 | 3118 | 3 | 3164 | 2 | 3210 | 3 | 3398 | 1 |
| 16 | 1 | 3073 | 1 | 3119 | 1 | 3165 | 2 | 3211 | 1 | | |
| 17 | 2 | 3074 | 1 | 3120 | 1 | 3166 | 1 | 3212 | 3 | | |
| 18 | 1 | 3075 | 1 | 3121 | 1 | 3167 | 1 | 3213 | 3 | | |
| 19 | 1 | 3076 | 1 | 3122 | 1 | 3168 | 1 | 3214 | 2 | | |
| 3031 | 1 | 3077 | 1 | 3123 | 1 | 3169 | 1 | 3215 | 2 | | |
| 3032 | 1 | 3078 | 1 | 3124 | 1 | 3170 | 1 | 3216 | 1 | | |
| 3033 | 1 | 3079 | 1 | 3125 | 1 | 3171 | 2 | 3217 | 1 | | |
| 3034 | 1 | 3080 | 1 | 3126 | 1 | 3172 | 1 | 3218 | 3 | | |
| 3035 | 1 | 3081 | 1 | 3127 | 1 | 3173 | 3 | 3219 | 2 | | |
| 3036 | 1 | 3082 | 1 | 3128 | 1 | 3174 | 1 | 3220 | 1 | | |
| 3037 | 3 | 3083 | 1 | 3129 | 2 | 3175 | 1 | 3221 | 1 | | |
| 3038 | 1 | 3084 | 1 | 3130 | 3 | 3176 | 2 | 3222 | 1 | | |
| 3039 | 1 | 3085 | 1 | 3131 | 3 | 3177 | 2 | 3223 | 3 | | |
| 3040 | 1 | 3086 | 1 | 3132 | 3 | 3178 | 2 | 3224 | 2 | | |
| 3041 | 1 | 3087 | 1 | 3133 | 3 | 3179 | 1 | 3225 | 3 | | |
| 3042 | 1 | 3088 | 1 | 3134 | 3 | 3180 | 1 | 3226 | 3 | | |
| 3043 | 1 | 3089 | 1 | 3135 | 3 | 3181 | 2 | 3227 | 3 | | |
| 3044 | 2 | 3090 | 1 | 3136 | 3 | 3182 | 3 | 3228 | 1 | | |
| 3045 | 1 | 3091 | 1 | 3137 | 2 | 3183 | 3 | 3229 | 1 | | |
| 3046 | 1 | 3092 | 1 | 3138 | 2 | 3184 | 2 | 3230 | 1 | | |
| 3047 | 1 | 3093 | 1 | 3139 | 2 | 3185 | 1 | 3231 | 2 | | |
| 3048 | 1 | 3094 | 1 | 3140 | 2 | 3186 | 2 | 3232 | 2 | | |
| 3049 | 1 | 3095 | 1 | 3141 | 2 | 3187 | 3 | 3233 | 3 | | |
| 3050 | 1 | 3096 | 1 | 3142 | 3 | 3188 | 1 | 3234 | 3 | | |
| 3051 | 1 | 3097 | 1 | 3143 | 3 | 3189 | 1 | 3235 | 1 | | |
| 3052 | 1 | 3098 | 1 | 3144 | 3 | 3190 | 2 | 3236 | 3 | | |
| 3053 | 1 | 3099 | 1 | 3145 | 3 | 3191 | 2 | 3237 | 3 | | |
| 3054 | 1 | 3100 | 1 | 3146 | 1 | 3192 | 1 | 3238 | 1 | | |
| 3055 | 1 | 3101 | 1 | 3147 | 2 | 3193 | 1 | 3239 | 1 | | |
| 3056 | 1 | 3102 | 1 | 3148 | 3 | 3194 | 1 | 3240 | 1 | | |
| 3057 | 1 | 3103 | 2 | 3149 | 2 | 3195 | 2 | 3383 | 1 | | |

**Example 3401**

**Receptor Binding Assay**

In addition to the methods described herein, another means for evaluating a test compound is by determining binding affinities to the **MCH** receptor. This type of assay generally requires a radiolabelled ligand to the **MCH** receptor. Absent the use of known ligands for the **MCH** receptor and radiolabels thereof, compounds of Formula (**I**) can be labelled with a radioisotope and used in an assay for evaluating the affinity of a test compound to the **MCH** receptor.

A radiolabelled **MCH** compound of Formula (**I**) can be used in a screening assay to identify/evaluate compounds. In general terms, a newly synthesized or identified compound (i.e., test compound) can be evaluated for its ability to reduce binding of the "radiolabelled compound of Formula (**I**)" to the **MCH** receptor. Accordingly, the ability to compete with the "radio-labelled compound of Formula (**I**)" or **Radiolabelled MCH** Ligand for the binding to the **MCH** receptor directly correlates to its binding affinity of the test compound to the **MCH** receptor.

**ASSAY PROTOCOL FOR DETERMINING RECEPTOR BINDING FOR MCH:**

**A. MCH RECEPTOR PREPARATION**

293 cells (human kidney, ATCC), transiently transfected with 10 ug human **MCH** receptor and 60 ul Lipofectamine (per 15-cm dish), are grown in the dish for 24 hours (75% confluency) with a media change and removed with 10 ml/dish of Hepes-EDTA buffer (20mM Hepes +10 mM EDTA, pH 7.4). The cells are then centrifuged in a Beckman Coulter centrifuge for 20 minutes, 17,000 rpm (JA-25.50 rotor). Subsequently, the pellet is resuspended in 20 mM Hepes + 1 mM EDTA, pH 7.4 and homogenized with a 50-ml Dounce homogenizer and again centrifuged. After removing the supernatant, the pellets can be stored at -80°C, until used in binding assay. When used in the assay, membranes are thawed on ice for 20 minutes and then 10 mL of incubation buffer (20 mM Hepes, 1 mM $MgCl_2$,100 mM NaCl, pH 7.4) added. The membranes are then vortexed to resuspend the crude membrane pellet and homogenized with a Brinkmann PT-3100 Polytron homogenizer for 15 seconds at setting 6. The concentration of membrane protein is determined using the BRL Bradford protein assay.

**B. BINDING ASSAY**

For total binding, a total volume of 50µl of appropriately diluted membranes (diluted in assay buffer containing 50mM Tris HCl (pH 7.4), 10mM $MgCl_2$, and 1mM EDTA; 5-50ug protein) is added to 96-well polyproylene microtiter plates followed by addition of 100µl of assay buffer and 50ul of Radiolabelled MCH Ligand. For nonspecific binding, 50 ul of assay buffer is added instead of 100ul and an additional 50ul of 10uM cold MCH is added before 50ul of Radiolabelled MCH Ligand is added. Plates are then incubated at room temperature for 60-120 minutes. The binding reaction is terminated by filtering assay plates through a Microplate Devices GF/C Unifilter filtration plate with a Brandell 96-well plate harvestor followed by washing with cold 50 mM Tris HCl, pH 7.4 containing 0.9% NaCl. Then, the bottom of the filtration plate are sealed, 50 µl of Optiphase Supermix is added to each well, the top of the plates are sealed, and plates are counted in a Trilux MicroBeta scintillation counter. For compound competition studies, instead of adding 100 µl of assay buffer, 100 µl of appropriately diluted test compound is added to appropriate wells followed by addition of 50 µl of **Radiolabelled MCH** Ligand.

**C. CALCULATIONS**

The test compounds are initially assayed at 1 and 0.1 µM and then at a range of concentrations chosen such that the middle dose would cause about 50% inhibition of a Radiolabelled MCH Ligand binding (i.e., $IC_{50}$). Specific binding in the absence of test compound ($B_O$) is the difference of total binding ($B_T$) minus non-specific binding (NSB) and similarly specific binding (in the presence of test compound) (B) is the difference of displacement binding ($B_D$) minus non-specific binding (NSB). $IC_{50}$ is determined from an inhibition response curve, logit-log plot of % $B/B_O$ vs concentration of test compound.

$K_i$ is calculated by the Cheng and Prustoff transformation:

$$K_i = IC_{50} / (1 + [L]/K_D)$$

wherein [**L**] is the concentration of a Radiolabelled MCH Ligand used in the assay and $K_D$ is the dissociation constant of a Radiolabelled MCH Ligand determined independently under the same binding conditions.

It is intended that each of the patents, applications, printed publications, and other published documents mentioned or referred to in this specification be herein incorporated by reference in their entirety.

Those skilled in the art will appreciate that numerous changes and modifications may be made to the preferred embodiments of the invention and that such changes and modifications may be made without departing from the spirit of the invention. It is therefore intended that the appended claims cover all such equivalent variations as fall within the true spirit and scope of the invention.

## Claims

**1.** A compound of Formula (I):

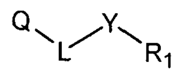

(I)

wherein Q is:

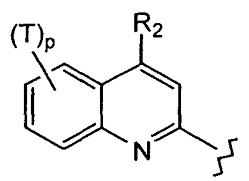

,

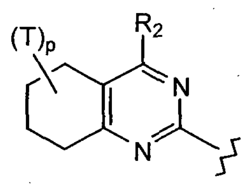

or

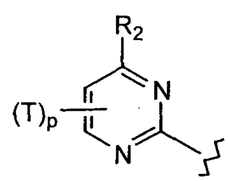

(II) (III) (IV)

$R_1$ is selected from the group consisting of:

(i) $C_{1-16}$ alkyl, and
$C_{1-16}$ alkyl substituted by substituent(s) independently selected from the group consisting of:

- halogen,
- hydroxy,
- oxo,
- $C_{1-5}$ alkoxy,
- $C_{1-5}$ alkoxy substituted by substituent(s) independently selected from the group consisting of:
  - •• carbocyclic aryl,
  - •• heterocyclyl, and
  - •• heterocyclyl substituted by $C_{1-5}$ alkyl,
- $C_{1-5}$ alkylcarbonyloxy,
- carbocyclyloxy,
- carbocyclic aryloxy,
- carbocyclic aryloxy substituted by substituent(s) independently selected from the group consisting of:
  - •• halogen,
  - •• hydroxy,
  - •• carboxy,
  - •• carbamoyl,
  - •• nitro,
  - •• cyano,
  - •• amino,
  - •• carbocyclic aryl,

- •• carbocyclic aryl substituted by $C_{1-5}$ alkoxy,
- •• $C_{1-5}$ alkoxy,
- •• $C_{1-5}$ alkoxy substituted by halogen,
- •• $C_{1-5}$ alkyl, and
- •• $C_{1-5}$ alkyl substituted by substituent(s) independently selected from the group consisting of:
  - ••• halogen,
  - ••• hydroxy,
  - ••• carboxy,
  - ••• oxo,
  - ••• mono-$C_{1-5}$ alkylamino,
  - ••• di-$C_{1-5}$ alkylamino,
  - ••• mono-$C_{1-5}$ alkylamino substituted by carbocyclic aryl,
  - ••• di-$C_{1-5}$ alkylamino substituted by carbocyclic aryl,
  - ••• mono-$C_{1-5}$ alkylamino substituted by halogenated carbocyclic aryl,
  - ••• di-$C_{1-5}$ alkylamino substituted by halogenated carbocyclic aryl,
  - ••• carbocyclic arylcarbonylamino, and
  - ••• carbocyclic arylcarbonylamino substituted by halogen,

- • heterocyclyloxy,
- • heterocyclyloxy substituted by substituent(s) independently selected from the group consisting of:
  - •• halogen,
  - •• hydroxy,
  - •• carboxy,
  - •• carbamoyl,
  - •• nitro,
  - •• cyano,
  - •• amino,
  - •• carbocyclic aryl,
  - •• carbocyclic aryl substituted by $C_{1-5}$ alkoxy,
  - •• $C_{1-5}$ alkoxy,
  - •• $C_{1-5}$ alkoxy substituted by substituent(s) independently selected from the group consisting of:
    - ••• halogen,
    - ••• hydroxy, and
    - ••• carboxy,
  - •• $C_{1-5}$ alkyl, and
  - •• $C_{1-5}$ alkyl substituted by substituent(s) independently selected from the group consisting of:
    - ••• halogen,
    - ••• hydroxy, and
    - ••• carboxy,

- • substituted heterocyclyl-ethylideneaminooxy,
- • $C_{1-5}$ alkoxycarbonyl,
- • $C_{1-5}$ alkoxycarbonyl substituted by carbocyclic aryl,
- • mono-$C_{1-5}$ alkylaminocarbonyl,
- • di-$C_{1-5}$ alkylaminocarbonyl,
- • mono-$C_{1-5}$ alkylamino,
- • mono-$C_{1-5}$ alkylamino substituted by substituent(s) independently selected from, the group consisting of:
  - •• cyano,
  - •• carbocyclic aryl, and
  - •• heterocyclyl,

- • di-$C_{1-5}$ alkylamino,

- di-$C_{1-5}$ alkylamino substituted by substituent(s) independently selected from the group consisting of:
  - •• cyano,
  - •• carbocyclic aryl, and
  - •• heterocyclyl,
- mono-carbocyclic arylamino,
- mono-carbocyclic arylamino substituted by substituent(s) independently selected from the group consisting of:
  - •• halogen,
  - •• hydroxy,
  - •• carboxy,
  - •• carbamoyl,
  - •• nitro,
  - •• cyano,
  - •• amino,
  - •• carbocyclic aryl,
  - •• carbocyclic aryl substituted by $C_{1-5}$ alkoxy,
  - •• $C_{1-5}$ alkoxy,
  - •• $C_{1-5}$ alkoxy substituted by substituent(s) independently selected from the group consisting of:
    - ••• halogen,
    - ••• hydroxy,and
    - ••• carboxy,
  - •• $C_{1-5}$ alkyl, and
  - •• $C_{1-5}$ alkyl substituted by substituent(s) independently selected from the group consisting of:
    - ••• halogen,
    - ••• hydroxy, and
    - ••• carboxy,
- di-carbocyclic arylamino,
- di-carbocyclic arylamino substituted by substituent(s) independently selected from the group consisting of:
  - •• halogen,
  - •• hydroxy,
  - •• carboxy,
  - •• carbamoyl,
  - •• nitro,
  - •• cyano,
  - •• amino,
  - •• carbocyclic aryl,
  - •• carbocyclic aryl substituted by $C_{1-5}$ alkoxy,
  - •• $C_{1-5}$ alkoxy,
  - •• $C_{1-5}$ alkoxy substituted by substituent(s) independently selected from the group consisting of:
    - ••• halogen,
    - ••• hydroxy, and
    - ••• carboxy,
  - •• $C_{1-5}$ alkyl, and
  - •• $C_{1-5}$ alkyl substituted by substituent(s) independently selected from the group consisting of:
    - ••• halogen,
    - ••• hydroxy, and
    - ••• carboxy,

- mono-heterocyclylamino,
- mono-heterocyclylamino substituted by substituent(s) independently selected from the group consisting of:
    - halogen,
    - hydroxy,
    - carboxy,
    - carbamoyl,
    - nitro,
    - cyano,
    - amino,
    - carbocyclic aryl,
    - carbocyclic aryl substituted by $C_{1-5}$ alkoxy,
    - $C_{1-5}$ alkoxy,
    - $C_{1-5}$ alkoxy substituted by substituent(s) independently selected from the group consisting of:
        - halogen,
        - hydroxy, and
        - carboxy,
    - $C_{1-5}$ alkyl, and
    - $C_{1-5}$ alkyl substituted by substituent(s) independently selected from the group consisting of:
        - halogen,
        - hydroxy, and
        - carboxy,
- di-heterocyclylamino,
- di-heterocyclylamino substituted by substituent(s) independently selected from the group consisting of:
    - halogen,
    - hydroxy,
    - carboxy,
    - carbamoyl,
    - nitro,
    - cyano,
    - amino,
    - carbocyclic aryl,
    - carbocyclic aryl substituted by $C_{1-5}$ alkoxy,
    - $C_{1-5}$ alkoxy,
    - $C_{1-5}$ alkoxy substituted by substituent(s) independently selected from the group consisting of:
        - halogen,
        - hydroxy, and
        - carboxy,
    - $C_{1-5}$ alkyl, and
    - $C_{1-5}$ alkyl substituted by substituent(s) independently selected from the group consisting of:
        - halogen,
        - hydroxy, and
        - carboxy,
- $C_{1-5}$ alkylcarbonylamino,
- $C_{1-5}$ alkylcarbonylamino substituted by substituent(s) independently selected from the group consisting of:
    - $C_{1-5}$ alkylcarbonylamino,
    - carbocyclic arylcarbonylamino, and

  - •• heterocyclyl,
- $C_{1-5}$ alkoxycarbonylamino,
- carbocyclic arylcarbonylamino,
- heterocyclyl carbonylamino,
- carbocyclic arylsulfonylamino,
- carbocyclic arylsulfonylamino substituted by substituent(s) independently selected from the group consisting of:
  - •• nitro,
  - •• $C_{1-5}$ alkyl,
  - •• mono-$C_{1-5}$ alkylamino, and
  - •• di-$C_{1-5}$ alkylamino,
- $C_{1-5}$ alkylthio,
- $C_{1-5}$ alkylthio substituted by substituent(s) independently selected from the group consisting of:
  - •• mono-carbocyclic arylaminocarbonyl,
  - •• mono-carbocyclic arylaminocarbonyl substituted by halogen,
  - •• di-carbocyclic arylaminocarbonyl,
  - •• di-carbocyclic arylaminocarbonyl substituted by halogen,
  - •• mono-carbocyclic arylamino,
  - •• mono-carbocyclic arylamino substituted by halogen,
  - •• di-carbocyclic arylamino,
  - •• di-carbocyclic arylamino substituted by halogen,
  - •• carbocyclic aryl, and
  - •• carbocyclic aryl substituted by substituent(s) independently selected from the group consisting of:
    - ••• halogen, and
    - ••• $C_{1-5}$ alkoxy,
- carbocyclic arylthio,
- carbocyclic arylthio substituted by substituent(s) independently selected from the group consisting of:
  - •• halogen,
  - •• $C_{1-5}$ alkyl, and
  - •• $C_{1-5}$ alkyl substituted by halogen,
- carbocyclic arylsulfinyl,
- carbocyclic arylsulfinyl substituted by substituent(s) independently selected from the group consisting of:
  - •• halogen,
  - •• $C_{1-5}$ alkyl, and
  - •• $C_{1-5}$ alkyl substituted by halogen,
- carbocyclic arylsulfonyl,
- carbocyclic arylsulfonyl substituted by substituent(s) independently selected from the group consisting of:
  - •• halogen,
  - •• $C_{1-5}$ alkyl, and
  - •• $C_{1-5}$ alkyl substituted by halogen,
- heterocyclylthio,
- heterocyclylthio substituted by substituent(s) independently selected from the group consisting of:
  - •• nitro, and
  - •• $C_{1-5}$ alkyl,

- $C_{3-6}$ cycloalkyl,
- $C_{3-6}$ cycloalkyl substituted by $C_{1-5}$ alkyl,
- $C_{3-6}$ cycloalkyl substituted by carbocyclic aryl,
- $C_{3-6}$ cycloalkenyl,
- carbocyclyl,
- carbocyclyl substituted by substituent(s) independently selected from the group consisting of:

    - •• halogen,
    - •• $C_{1-5}$ alkyl,
    - •• $C_{1-5}$ alkoxy,
    - •• $C_{2-5}$ alkenyl, and
    - •• $C_{2-5}$ alkenyl substituted by substituent(s) independently selected from the group consisting of:

        - ••• carbocyclic aryl, and
        - ••• carbocyclic aryl substituted by $C_{1-5}$ alkylsulfinyl,

- carbocyclic aryl,
- carbocyclic aryl substituted by substituent(s) independently selected from the group consisting of:

    - •• halogen,
    - •• hydroxy,
    - •• carboxy,
    - •• carbamoyl,
    - •• cyano,
    - •• nitro,
    - •• amino,
    - •• $C_{1-5}$ alkylcarbonylamino,
    - •• $C_{3-6}$ cycloalkylcarbonylamino,
    - •• $C_{1-5}$ alkyl,
    - •• $C_{1-5}$ alkyl substituted by substituent(s) independently selected from the group consisting of:

        - ••• halogen,
        - ••• hydroxy,
        - ••• carboxy,
        - ••• carbamoyl,
        - ••• oxo,
        - ••• carbocyclic aryl,
        - ••• heterocyclyl,
        - ••• mono-carbocyclic arylamino,
        - ••• di-carbocyclic arylamino,
        - ••• mono-carbocyclic arylamino substituted by substituent(s) independently selected from the group consisting of:

            - •••• halogen,
            - •••• nitro,
            - •••• $C_{1-5}$ alkyl,
            - •••• $C_{1-5}$ alkoxy, and
            - •••• $C_{1-5}$ alkoxy substituted by halogen,

        - ••• di-carbocyclic arylamino substituted by substituent(s) independently selected from the group consisting of:

            - •••• halogen,
            - •••• nitro,
            - •••• $C_{1-5}$ alkyl,
            - •••• $C_{1-5}$ alkoxy, and
            - •••• $C_{1-5}$ alkoxy substituted by halogen,

- $C_{2-5}$ alkenyl,
- $C_{1-5}$ alkoxy,
- $C_{1-5}$ alkoxy substituted by substituent(s) independently selected from the group consisting of:

  - halogen, and
  - carbocyclic aryl,

- carbocyclic aryloxy,
- $C_{1-5}$ alkoxycarbonyl,
- $C_{1-5}$ alkylcarbonyloxy,
- mono-$C_{1-5}$ alkylamino,
- di-$C_{1-5}$ alkylamino,
- mono-carbocyclic arylamino,
- mono-carbocyclic arylamino substituted by halogen,
- di-carbocyclic arylamino,
- di-carbocyclic arylamino substituted by halogen,
- mono-carbocyclic arylaminocarbonyl,
- mono-carbocyclic arylaminocarbonyl substituted by substituent(s) selected from the group consisting of:

  - halogen,
  - nitro,
  - $C_{1-5}$ alkyl,
  - $C_{1-5}$ alkoxy, and
  - $C_{1-5}$ alkoxy substituted by halogen,

- di-carbocyclic arylaminocarbonyl,
- di-carbocyclic arylaminocarbonyl substituted by substituent(s) selected from the group consisting of:

  - halogen,
  - nitro,
  - $C_{1-5}$ alkyl,
  - $C_{1-5}$ alkoxy, and
  - $C_{1-5}$ alkoxy substituted by halogen,

- mercapto,
- $C_{1-5}$ alkylthio,
- $C_{1-5}$ alkylthio substituted by halogen,
- $C_{1-5}$ alkylsulfonyl,
- $C_{3-6}$ cycloalkyl,
- carbocyclic aryl, and
- heterocyclyl,

- heterocyclyl, and
- heterocyclyl substituted by substituent(s) independently selected from the group consisting of:

  - halogen,
  - hydroxy,
  - carboxy,
  - carbamoyl,
  - cyano,
  - nitro,
  - amino,
  - $C_{1-5}$ alkyl,
  - $C_{1-5}$ alkyl substituted by substituent(s) independently selected from the group consisting of:

    - halogen,
    - hydroxy,

      - carboxy, and
      - carbamoyl,

    - $C_{1-5}$ alkyl substituted by carbocyclic aryl,
    - $C_{1-5}$ alkoxy,
    - $C_{1-5}$ alkoxy substituted by halogen,
    - $C_{1-5}$ alkoxy substituted by carbocyclic aryl,
    - carbocyclic aryl, and
    - carbocyclic aryl substituted by halogen,

(ii) $C_{2-8}$ alkenyl, and
$C_{2-8}$ alkenyl substituted by substituent(s) independently selected from the group consisting of:

- halogen,
- oxo,
- $C_{1-5}$ alkoxy,
- $C_{1-5}$ alkoxy substituted by carbocyclic aryl,
- carbocyclic aryl,
- carbocyclic aryl substituted by substituent(s) independently selected from the group consisting of:

    - halogen,
    - hydroxy,
    - nitro,
    - $C_{1-5}$ alkyl,
    - $C_{1-5}$ alkyl substituted by halogen,
    - $C_{1-5}$ alkoxy, and
    - $C_{1-5}$ alkoxy substituted by halogen,

- heterocyclyl, and
- heterocyclyl substituted by substituent(s) independently selected from the group consisting of:

    - hydroxy,
    - nitro,
    - $C_{1-5}$ alkyl, and
    - $C_{1-5}$ alkoxy,

(iii) $C_{2-5}$ alkynyl, and
$C_{2-5}$ alkynyl substituted by carbocyclic aryl,
(iv) $C_{3-12}$ cycloalkyl, and
$C_{3-12}$ cycloalkyl substituted by substituent(s) independently selected from the group consisting of:

- $C_{1-5}$ alkyl,
- $C_{1-5}$ alkyl substituted by substituent(s) independently selected from the group consisting of:

    - hydroxy,
    - oxo, and
    - carbocyclic aryl,

- mono-$C_{1-5}$ alkylamino,
- mono-$C_{1-5}$ alkylamino substituted by carbocyclic aryl,
- di-$C_{1-5}$ alkylamino,
- di-$C_{1-5}$ alkylamino substituted by carbocyclic aryl,
- carbocyclic arylcarbonylamino,
- carbocyclic aryl, and
- carbocyclic aryl substituted by substituent(s) independently selected from the group consisting of:

    - halogen,
    - $C_{1-5}$ alkoxy,

- •• $C_{1-5}$ alkyl, and
- •• $C_{1-5}$ alkyl substituted by halogen,

(v) $C_{3-6}$ cycloalkenyl, and
$C_{3-6}$ cycloalkenyl substituted by $C_{1-5}$ alkyl,
(vi) carbocyclyl, and
carbocyclyl substituted by substitutent(s) independently selected from the group consisting of:

- • hydroxy, and
- • nitro,

(vii) carbocyclic aryl, and
carbocyclic aryl substituted by substituent(s) independently selected from the group consisting of:

- • halogen,
- • hydroxy,
- • cyano,
- • nitro,
- • $C_{1-10}$ alkyl,
- • $C_{1-10}$ alkyl substituted by substituent(s) independently selected from the group consisting of:

  - •• halogen,
  - •• hydroxy,
  - •• carboxy,
  - •• carbamoyl,
  - •• oxo,
  - •• $C_{1-5}$ alkoxy,
  - •• carbocyclic aryloxy,
  - •• mono-$C_{1-5}$ alkylamino-N-oxy,
  - •• di-$C_{1-5}$ alkylamino-N-oxy,
  - •• mono-$C_{1-5}$ alkylamino,
  - •• di-$C_{1-5}$ alkylamino,
  - •• mono-$C_{1-5}$ alkylamino substituted by carbocyclic aryl,
  - •• di-$C_{1-5}$ alkylamino substituted by carbocyclic aryl,
  - •• mono-carbocyclic arylamino,
  - •• di-carbocyclic arylamino,
  - •• carbocyclylimino,
  - •• carbocyclylimino substituted by carbocyclic aryl,
  - •• mono-carbocyclic arylamino,
  - •• di-carbocyclic arylamino,
  - •• mono-carbocyclic arylamino substituted by $C_{1-5}$ alkoxy,
  - •• di-carbocyclic arylamino substituted by $C_{1-5}$ alkoxy,
  - •• mono-carbocyclic arylaminocarbonyl,
  - •• di-carbocyclic arylaminocarbonyl,
  - •• mono-carbocyclic arylaminocarbonyl substituted by $C_{1-5}$ alkoxy,
  - •• di-carbocyclic arylaminocarbonyl substituted by $C_{1-5}$ alkoxy,
  - •• carbocyclic aryl,
  - •• carbocyclic aryl substituted by substituent(s) independently selected from the group consisting of:

    - ••• halogen,
    - ••• $C_{1-5}$ alkyl, and
    - ••• $C_{1-5}$ alkyl substituted by halogen,

  - •• heterocyclyl, and
  - •• heterocyclyl substituted by $C_{1-5}$ alkyl,

- • $C_{2-5}$ alkenyl,
- • $C_{2-5}$ alkenyl substituted by carbocyclic aryl,

- $C_{1-9}$ alkoxy,
- $C_{1-9}$ alkoxy substituted by substituent(s) independently selected from the group consisting of:

  - •• hydroxy,
  - •• halogen,
  - •• carboxy,
  - •• mono-$C_{1-5}$ alkylamino,
  - •• di-$C_{1-5}$ alkylamino,
  - •• carbocyclic aryl,
  - •• halogenated carbocyclic aryl,
  - •• heterocyclyl,
  - •• heterocyclyl substituted by substituent(s) independently selected from the group consisting of:

    - ••• halogen,
    - ••• heterocyclyl, and
    - ••• heterocyclyl substituted by substituent(s) independently selected from the group consisting of:

      - •••• halogen,
      - •••• $C_{1-5}$ alkyl, and
      - •••• $C_{1-5}$ alkyl substituted by halogen,

- $C_{2-5}$ alkenyloxy,
- $C_{3-6}$ cycloalkoxy,
- $C_{1-5}$ alkylcarbonyloxy,
- carbocyclic aryloxy,
- carbocyclic aryloxy substituted by substituent(s) independently selected from the group consisting of:

  - •• halogen,
  - •• hydroxy,
  - •• carboxy,
  - •• carbamoyl,
  - •• cyano,
  - •• nitro,
  - •• amino,
  - •• $C_{1-5}$ alkyl,
  - •• $C_{1-5}$ alkyl substituted by substituent(s) independently selected from the group consisting of:

    - ••• halogen,
    - ••• hydroxy,
    - ••• carboxy, and
    - ••• carbamoyl,

  - •• $C_{1-5}$ alkoxy, and
  - •• $C_{1-5}$ alkoxy substituted by halogen,

- heterocyclyloxy,
- heterocyclyloxy substituted by substituent(s) independently selected from the group consisting of:

  - •• halogen,
  - •• hydroxy,
  - •• carboxy,
  - •• carbamoyl,
  - •• cyano,
  - •• nitro,
  - •• amino,
  - •• $C_{1-5}$ alkyl,
  - •• $C_{1-5}$ alkyl substituted by substituent(s) independently selected from the group consisting of:

      - halogen,
      - hydroxy,
      - carboxy, and
      - carbamoyl,

    - $C_{1-5}$ alkoxy, and
    - $C_{1-5}$ alkoxy substituted by halogen,

- (carbocyclic aryl)$S(O)_2O$,
- carboxy,
- carbamoyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- mono-carbocyclic arylaminocarbonyl,
- di-carbocyclic arylaminocarbonyl,
- mono-carbocyclic arylaminocarbonyl substituted by $C_{1-5}$ alkyl,
- di-carbocyclic arylaminocarbonyl substituted by $C_{1-5}$ alkyl,
- amino,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- mono-carbocyclic arylamino,
- di-carbocyclic arylamino,
- $C_{1-5}$ alkylcarbonylamino,
- $C_{3-6}$ cycloalkylcarbonylamino,
- $C_{2-5}$ alkynylcarbonylamino,
- $C_{2-5}$ alkynylcarbonylamino substituted by carbocyclic aryl,
- $C_{1-5}$ alkoxycarbonylamino,
- carbocyclic arylsulfonylamino,
- carbocyclic arylsulfonylamino substituted by $C_{1-5}$ alkyl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- carbocyclic aryl azo,
- carbocyclic aryl azo substituted by mono-$C_{1-5}$ alkylamino,
- carbocyclic aryl azo substituted by di-$C_{1-5}$ alkylamino,
- $C_{1-5}$ alkylthio,
- $C_{1-5}$ alkylthio substituted by halogen,
- carbocyclic arylthio,
- carbocyclic arylthio substituted by substituent(s) independently selected from the group consisting of:

    - halogen,
    - nitro,
    - cyano, and
    - $C_{1-5}$ alkyl,

- aminosulfonyl,
- heterocyclylthio,
- $C_{1-5}$ alkylsulfonyl,
- mono-$C_{1-5}$ alkylaminosulfonyl,
- di-$C_{1-5}$ alkylaminosulfonyl,
- heterocyclylsulfonyl,
- $C_{3-6}$ cycloalkyl,
- $C_{3-6}$ cycloalkyl substituted by $C_{1-5}$ alkyl,

- carbocyclic aryl,
- carbocyclic aryl substituted by substituent(s) independently selected from the group consisting of:

  - $C_{1-7}$ alkyl, and
  - $C_{1-7}$ alkyl substituted by halogen,

- heterocyclyl, and
- heterocyclyl substituted by substituent(s) independently selected from the group consisting of:

  - $C_{1-5}$ alkyl,
  - carbocyclic aryl, and
  - halogenated carbocyclic aryl,

- $C_{1-5}$ alkoxycarbonyl substituted by carbocyclic aryl, and

(viii) heterocyclyl, and
heterocyclyl substituted by substituent(s) independently selected from the group consisting of:

- halogen,
- hydroxy,
- carboxy,
- carbamoyl,
- cyano,
- nitro,
- amino,
- $C_{1-5}$ alkyl,
- $C_{1-5}$ alkyl substituted by substituent(s) independently selected from the group consisting of:

  - halogen,
  - hydroxy,
  - carboxy,
  - carbamoyl,
  - oxo,
  - $C_{1-5}$ alkylcarbonyloxy,
  - carbocyclic arylcarbonylamino,
  - carbocyclic arylcarbonylamino substituted by halogen,
  - $C_{1-5}$ alkoxycarbonyl,
  - $C_{1-5}$ alkylthio,
  - $C_{1-5}$ alkylthio substituted by carbocyclic aryl,
  - $C_{1-5}$ alkylthio substituted by halogenated carbocyclic aryl,
  - carbocyclic aryl,
  - carbocyclic aryl substituted by substituent(s) independently selected from the group consisting of:

    - halogen, and
    - nitro,

  - heterocyclyl, and
  - heterocyclyl substituted by substituent(s) independently selected from the group consisting of:

    - halogen,
    - $C_{1-5}$ alkyl, and
    - $C_{1-5}$ alkyl substituted by halogen,

- $C_{1-5}$ alkoxy,
- $C_{1-5}$ alkoxy substituted by halogen,
- $C_{1-5}$ alkoxy substituted by carbocyclic aryl,
- carbocyclic aryloxy,
- carbocyclic aryloxy substituted by substituent(s) independently selected from the group consisting of:

- •• halogen,
- •• nitro,
- •• cyano,
- •• hydroxy,
- •• carboxy,
- •• carbamoyl,
- •• amino,
- •• $C_{1-5}$ alkyl,
- •• $C_{1-5}$ alkyl substituted by substituent(s) independently selected from the group consisting of:

  - ••• halogen,
  - ••• hydroxy,
  - ••• carboxy, and
  - ••• carbamoyl,

- •• mono-$C_{1-5}$ alkylamino,
- •• di-$C_{1-5}$ alkylamino,
- •• $C_{1-5}$ alkylcarbonylamino,
- •• $C_{3-6}$ cycloalkycarbonylamino,
- •• $C_{1-5}$ alkoxy,
- •• $C_{1-5}$ alkoxy substituted by halogen,
- •• $C_{3-6}$ cycloalkyl,
- •• $C_{2-5}$ alkenyl,
- •• $C_{2-5}$alkynyl,
- •• carboxy,
- •• $C_{1-5}$ alkoxycarbonyl,
- •• mono-$C_{1-5}$ alkylaminocarbonyl,
- •• di-$C_{1-5}$ alkylaminocarbonyl,
- •• mono-$C_{3-6}$ cycloalkylaminocarbonyl,
- •• di-$C_{3-6}$ cycloalkylaminocarbonyl,
- •• mono-$C_{1-5}$ alkylaminocarbonylamino,
- •• di-$C_{1-5}$ alkylaminocarbonylamino,
- •• mono-$C_{3-6}$ cycloalkylaminocarbonylamino,
- •• di-$C_{3-6}$ cycloalkylaminocarbonylamino,
- •• $C_{1-5}$ alkylthio,
- •• $C_{1-5}$ alkylthio substituted by halogen,
- •• $C_{1-5}$ alkylsulfinyl,
- •• $C_{1-5}$ alkylsulfinyl substituted by halogen,
- •• $C_{1-5}$ alkylsulfonyl, and
- •• $C_{1-5}$ alkylsulfonyl substituted by halogen,

- • heterocyclyloxy,
- • heterocyclyloxy substituted by substituent(s) independently selected from the group consisting of:

- •• halogen,
- •• nitro,
- •• hydroxy,
- •• carboxy,
- •• carbamoyl,
- •• cyano,
- •• amino,
- •• $C_{1-5}$ alkyl,
- •• $C_{1-5}$ alkyl substituted by substituent(s) independently selected from the group consisting of:

  - ••• halogen,
  - ••• hydroxy,
  - ••• carboxy, and
  - ••• carbamoyl,

  - •• $C_{1-5}$ alkoxy, and
  - •• $C_{1-5}$ alkoxy substituted by halogen,

- mono-$C_{1-5}$ alkylamino,
- di-$C_{1-5}$ alkylamino,
- mono-carbocyclic arylamino,
- mono-carbocyclic arylamino substituted by halogen,
- $C_{1-5}$ alkylcarbonylamino,
- $C_{1-5}$ alkylthio,
- $C_{2-5}$ alkenylthio,
- carbocyclic arylthio,
- carbocyclic arylthio substituted by halogen,
- carbocyclic arylthio substituted by $C_{1-5}$ alkoxycarbonyl,
- heterocyclylthio,
- heterocyclylthio substituted by $C_{1-5}$ alkyl,
- $C_{1-5}$ alkylsulfinyl,
- $C_{1-5}$ alkylsulfonyl,
- carbocyclic arylsulfinyl,
- carbocyclic arylsulfinyl substituted by halogen,
- carbocyclic arylsulfonyl,
- carbocyclic arylsulfonyl substituted by substituent(s) independently selected from the group consisting of:

  - •• halogen,
  - •• $C_{1-5}$ alkoxy,
  - •• $C_{1-5}$ alkyl, and
  - •• $C_{1-5}$ alkyl substituted by halogen,

- $C_{1-5}$ alkoxycarbonyl,
- $C_{1-5}$ alkoxycarbonyl substituted by carbocyclic aryl,
- carbocyelie aryl,
- carbocyclic aryl substituted by substituent(s) independently selected from the group consisting of:

  - •• halogen,
  - •• nitro,
  - •• $C_{1-5}$ alkyl,
  - •• $C_{1-5}$ alkyl substituted by halogen,
  - •• $C_{1-5}$ alkoxy, and
  - •• $C_{1-5}$ alkoxy substituted by halogen,

- heterocyclyl, and
- heterocyclyl substituted by substituent(s) independently selected from the group consisting of:

  - •• halogen,
  - •• $C_{1-5}$ alkyl,
  - •• $C_{1-5}$ alkyl substituted by halogen,
  - •• $C_{1-5}$ alkoxy, and
  - •• $C_{1-5}$ alkoxycarbonyl;

$R_2$ is selected from the group consisting of:

hydrogen, halogen, hydroxy, carboxy, carbamoyl, amino, $C_{1-5}$ alkyl, $C_{1-5}$ alkyl substituted by halogen, $C_{1-5}$ alkyl substituted by hydroxy, $C_{1-5}$ alkyl substituted by carboxy, $C_{1-5}$ alkyl substituted by carbamoyl, $C_{1-5}$ alkoxy, $C_{1-5}$ alkoxy substituted by halogen, $-NHNH_2$, -NHNHBoc, $-N(R_{2a})(R_{2b})$, morpholino, 4-acetyl-piperazyl, or 4-phenyl-piperazyl,

wherein $R_{2a}$ is hydrogen or $C_{1-5}$ alkyl and $R_{2b}$ is $C_{1-5}$ alkyl, $C_{3-6}$ cycloalkyl, or $C_{1-5}$ alkyl substituted by substituent (s) independently selected from the group consisting of:

- halogen,
- hydroxy,
- carboxy,
- carbamoyl,
- $C_{1-5}$ alkoxy,
- amino,
- -NHBoc,
- $C_{3-6}$ cycloalkyl,
- carbocyclic aryl,
- carbocyclic aryl substituted by substituent(s) independently selected from the group consisting of:
    - •• halogen,
    - •• $C_{1-5}$ alkyl,
    - •• $C_{1-5}$ alkoxy, and
    - •• $SO_2NH_2$,
- heterocyclyl, and

$C_{3-6}$ cycloalkyl, carbocyclic aryl, carbocyclic aryl substituted by substituent(s) independently selected from the group consisting of:

- halogen,
- $C_{1-5}$ alkyl,
- $C_{1-5}$ alkoxy, and
- a group of Formula (V):

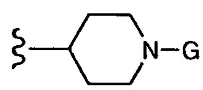

**(V)**

wherein Boc is carbamic acid *tert*-butyl ester and G is $C_{1-5}$ alkyl or $C_{1-5}$ alkyl substituted by substituent(s) independently selected from the group consisting of:

- carbocyclic aryl,
- halogenated carbocyclic aryl, and
- carbocyclic aryl substituted by $C_{1-5}$ alkoxy;

or $R_2$ is methylamino or dimethylamino when Q is Formula (II) and Y is a single bond or $-CH_2-$;
Each T is independently selected from the group consisting of halogen, hydroxy, carboxy, carbamoyl, amino, cyano, nitro, $C_{1-5}$ alkyl, $C_{1-5}$ alkyl substituted by halogen, $C_{1-5}$ alkyl substituted by hydroxy, $C_{1-5}$ alkyl substituted by carboxy, $C_{1-5}$ alkyl substituted by carbamoyl, $C_{2-5}$ alkenyl, $C_{2-5}$ alkynyl, $C_{3-6}$ cycloalkyl, $C_{1-5}$ alkoxy, $C_{1-5}$ alkoxy substituted by halogen, carbocyclic aryl, heterocyclyl, and $-N(R_{2a})(R_{2b})$;
p is 0, 1, 2, 3, 4 or 5;
L is selected from the group consisting of Formulae (VI) to (XXI):

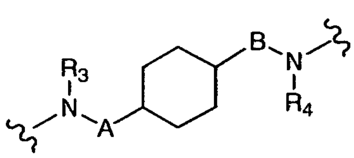

**(VI)**

,

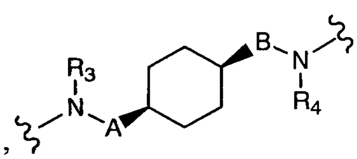

**(VII)**

,

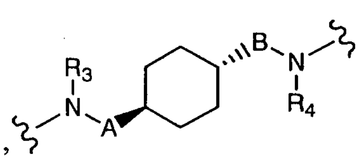

**(VIII)**

,

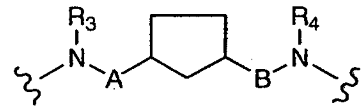 , 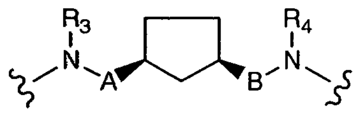 , 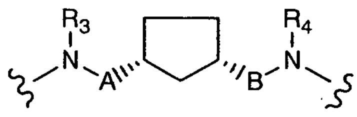 ,

(IX) (X) (XI)

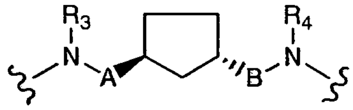 , 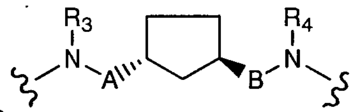

(XII) (XIII)

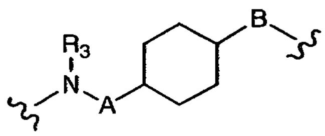 , 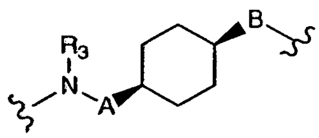 , 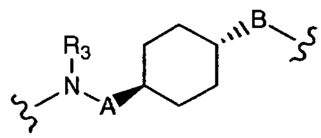 ,

(XIV) (XV) (XVI)

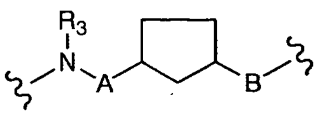 , 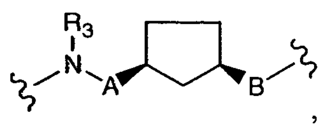 , 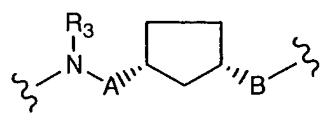 ,

(XVII) (XVIII) (XIX)

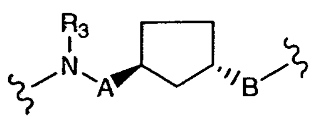 , 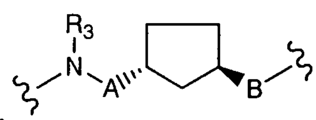 ;

(XX) (XXI)

wherein $R_3$ and $R_4$ are independently hydrogen or $C_{1-5}$ alkyl; and A and B are independently a single bond, $-CH_2-$, or $-(CH_2)_2-$;

and

Y represents:

(i) $-C(O)NR_5-$, $-C(S)NR_5-$, $-C(O)O-$, $-S(O)_2-$, $-C(O)-$, $-C(S)-$, a single bond, or $-CH_2-$ when L is selected from the group consisting of Formulae (VI) to (XIII); or

(ii) $-C(O)NR_5-$, $-C(S)NR_5-$, $-C(O)O-$ or $-OC(O)-$ when L is selected from the group consisting of Formulae (XIV) to (XXI);

wherein $R_5$ is hydrogen or $C_{1-5}$ alkyl, or when Y is $-C(O)NR_5-$ then $R_5$ and $R_1$ together with the nitrogen they are bonded form a heterocyclyl group;

wherein carbocyclic aryl is phenyl, naphthyl, anthranyl, phenanthryl, or biphenyl;

carbocyclyl is 10,11-dihydro-5-oxo-dibenzo[a,d]cycloheptyl, 1-oxo-indanyl, 7,7-dimethyl-2-oxo-bicyclo[2.2.1]heptyl, 9*H*-fluorenyl, 9-oxo-fluorenyl, acenaphthyl, anthraquinonyl, *C*-fluoren-9-ylidene, indanyl, indenyl,

1,2,3,4-tetrahydro-naphthyl, or bicyclo[2.2.1]heptenyl;

heterocyclyl is 1,2,3,4-tetrahydro-isoquinolyl, 1,2,3-thiadiazolyl, 1,2,3-triazolyl, 1,2-dihydro-3-oxo-pyrazolyl, 1,3,4-thiadiazolyl, 1,3-dioxo-isoindolyl, 1,3-dioxolanyl, 1*H*-indolyl, 1*H*-pyrrolo[2,3-c]pyridyl, 1*H*-pyrrolyl, 1-oxo-3*H*-isobenzofuranyl, 2,2',5',2''-terthiophenyl, 2,2'-bithiophenyl, 2,3-dihydro-1-oxo-isoindolyl, 2,3-dihydro-benzo[1,4]dioxinyl, 2,3-dihydro-benzofuryl, 2,4-dihydro-3-oxo-pyrazolyl, 2*H*-benzopyranyl, 2-oxo-benzopyranyl, 2-oxo-pyrrolidinyl, 3,4-dihydro-2*H*-benzo[1,4]oxazinyl, 3,4-dihydro-2*H*-benzo[b][1,4]dioxepinyl, 4*H*-benzo[1,3]dioxinyl, 4*H*-benzopyranyl, 4-oxo-1,5,6,7-tetrahydro-indolyl, 4-oxo-3,4-dihydro-phthalazinyl, 4-oxo-benzopyranyl, 9,10,10-trioxo-thioxanthenyl, 9*H*-carbazolyl, 9*H*-xanthenyl, azetidinyl, benzimidazolyl, benzo[1,3]dioxolyl, benzo[2,1,3]oxadiazolyl, benzo[1,2,5]oxadiazolyl, benzo[b]thienyl, benzofuryl, benzothiazolyl, cinnolyl, furyl, imidazo[2,1-b]thiazolyl, imidazolyl, isoxazolyl, morpholino, morpholinyl, oxazolyl, oxolanyl, piperazyl, piperidyl, piridyl, pyrazolo[5,1-b]thiazolyl, pyrazolyl, pyrazinyl, pyridyl, pyrimidyl, pyrrolidyl, quinolyl, quinoxalyl, thiazolidyl, thiazolyl, thienyl, thiolanyl, 2,3-dihydro-benzofuryl, tetrahydro-thienyl, or benzofuranyl; and

halogen is fluoro, chloro, bromo, or iodo;

or a pharmaceutically acceptable salt, hydrate, or solvate thereof.

**2.** The compound according to claim 1 wherein $R_1$ is selected from the group consisting of:

(i) $C_{1-8}$ alkyl, and

$C_{1-8}$ alkyl substituted by substituent(s) independently selected from the group consisting of:

- halogen,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by substituent(s) independently selected from the group consisting of:

  •• halogen,
  •• nitro,
  •• $C_{1-5}$ alkyl, and
  •• $C_{1-5}$ alkoxy,

- heterocyclyloxy,
- heterocyelyloxy substituted by $C_{1-5}$ alkyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by cyano,
- mono-$C_{1-5}$ alkylamino substituted by carbocyclic aryl,
- di-$C_{1-5}$ alkylamino,
- di-$C_{1-5}$ alkylamino substituted by cyano,
- di-$C_{1-5}$ alkylamino substituted by carbocyclic aryl,
- mono-carbocyclic arylamino,
- mono-carbocyclic arylamino substituted by halogen,
- mono-carbocyclic arylamino substituted by $C_{1-5}$ alkyl,
- di-carbocyclic arylamino,
- di-carbocyclic arylamino substituted by halogen,
- di-carbocyelic arylamino substituted by $C_{1-5}$ alkyl,
- $C_{1-5}$ alkoxycarbonylamino,
- carbocyclic arylcarbonylamino,
- carbocyclic arylsulfonylamino,
- carbocyclic arylsulfonylamino substituted $C_{1-5}$ alkyl,
- $C_{1-5}$ alkylthio,
- $C_{1-5}$ alkylthio substituted by substituent(s) independently selected from the group consisting of:

  •• carbocyclic aryl,

    - •• carbocyclic aryl substituted by halogen, and
    - •• carbocyclic aryl substituted by $C_{1-5}$ alkoxy,

- • carbocyclic arylthio,
- • heterocyclylthio,
- • heterocyclylthio substituted by nitro,
- • heterocyclylthio substituted by $C_{1-5}$ alkyl,
- • $C_{3-6}$ cycloalkyl,
- • $C_{3-6}$ cycloalkenyl,
- • carbocyclyl,
- • carbocyclyl substituted by substituent(s) independently selected from the group consisting of:

    - •• halogen,
    - •• $C_{1-5}$ alkyl,
    - •• $C_{1-5}$ alkoxy,
    - •• $C_{2-5}$ alkenyl, and
    - •• $C_{2-5}$ alkenyl substituted by substituent(s) independently selected from the group consisting of:

        - ••• carbocyclic aryl, and
        - ••• carbocyclic aryl substituted by $C_{1-5}$ alkylsulfinyl,

- • carbocyclic aryl,
- • carbocyclic aryl substituted by substituent(s) independently selected from the group consisting of:

    - •• halogen,
    - •• hydroxy,
    - •• nitro,
    - •• $C_{1-5}$ alkyl,
    - •• $C_{1-5}$ alkyl substituted by substituent(s) independently selected from the group consisting of:

        - ••• oxo,
        - ••• carbocyclic aryl, and
        - ••• heterocyclyl,

    - •• $C_{2-5}$ alkenyl,
    - •• $C_{1-5}$ alkoxy,
    - •• $C_{1-5}$ alkoxy substituted by halogen,
    - •• $C_{1-5}$ alkoxy substituted by carbocyclic aryl,
    - •• carbocyclic aryloxy,
    - •• mono-carbocyclic arylaminocarbonyl,
    - •• mono-carbocyclic arylaminocarbonyl substituted by halogen,
    - •• di-carbocyclic arylaminocarbonyl,
    - •• di-carbocyclic arylaminocarbonyl substituted by halogen,
    - •• carbocyclic aryl, and
    - •• heterocyclyl,

- • heterocyclyl, and
- • heterocyclyl substituted by substituent(s) independently selected from the group consisting of:

    - •• $C_{1-5}$ alkyl,
    - •• $C_{1-5}$ alkyl substituted by carbocyclic aryl,
    - •• $C_{1-5}$ alkoxy,
    - •• $C_{1-5}$ alkoxy substituted by carbocyclic aryl,
    - •• carbocyclic aryl, and
    - •• carbocyclic aryl substituted by halogen,

(ii) $C_{2-7}$ alkenyl, and
$C_{2-7}$ alkenyl substituted by substituent(s) independently selected from the group consisting of:

- carbocyclic aryl,
- carbocyclic aryl substituted by substituent(s) independently selected from the group consisting of:

  - •• halogen,
  - •• nitro, and
  - •• $C_{1-5}$ alkoxy,

(iii) $C_{2-5}$ alkynyl, and
$C_{2-5}$ alkynyl substituted by carbocyclic aryl,
(iv) $C_{3-12}$ cycloalkyl, and
$C_{3-12}$ cycloalkyl substituted by substituent(s) independently selected from the group consisting of:

- $C_{1-5}$ alkyl,
- $C_{1-5}$ alkyl substituted by oxo,
- $C_{1-5}$ alkyl substituted by carbocyclic aryl, and
- carbocyclic aryl,

(v) carbocyclyl,
(vi) carbocyclic aryl, and
carbocyclic aryl substituted by substituent(s) independently selected from the group consisting of:

- halogen,
- hydroxy,
- cyano,
- nitro,
- carboxy,
- carbamoyl,
- $C_{1-10}$ alkyl,
- $C_{1-10}$ alkyl substituted by substituent(s) independently selected from the group consisting of:

  - •• halogen,
  - •• hydroxy,
  - •• oxo,
  - •• carbocyclic aryloxy,
  - •• carbocyclic aryl, and
  - •• carbocyclic aryl substituted by $C_{1-5}$ alkyl,

- $C_{1-7}$ alkoxy,
- $C_{1-7}$ alkoxy substituted by substituent(s) independently selected from the group consisting of:

  - •• halogen,
  - •• carbocyclic aryl, and
  - •• halogenated carbocyclic aryl,

- $C_{2-5}$ alkenyloxy,
- $C_{3-6}$ cycloalkoxy,
- carbocyclic aryloxy,
- carbocyclic aryloxy substituted by nitro,
- carbocyclic aryloxy substituted by $C_{1-5}$ alkoxy,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- mono-$C_{1-5}$ alkylamino substituted by cyano,

- di-$C_{1-5}$ alkylamino substituted by cyano,
- $C_{2-5}$ alkynylcarbonylamino,
- $C_{2-5}$ alkynylcarbonylamino substituted by carbocyclic aryl,
- $C_{1-5}$ alkoxycarbonylamino,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- carbocyclic aryl azo,
- carbocyclic aryl azo substituted by mono-$C_{1-5}$ alkylamino,
- carbocyclic aryl azo substituted by di-$C_{1-5}$ alkylamino,
- $C_{1-5}$ alkylthio,
- $C_{1-5}$ alkylthio substituted by halogen,
- carbocyclic arylthio,
- carbocyclic arylthio substituted by nitro,
- carbocyclic arylthio substituted by cyano,
- aminosulfonyl,
- mono-$C_{1-5}$ alkylaminosulfonyl,
- di-$C_{1-5}$ alkylaminosulfonyl,
- heterocyclylsulfonyl,
- $C_{3-6}$ cycloalkyl,
- $C_{3-6}$ cycloalkyl substituted by $C_{1-5}$ alkyl,
- carbocyclic aryl,
- heterocyclyl, and
- heterocyclyl substituted by substituent(s) independently selected from the group consisting of:

    - •• $C_{1-5}$ alkyl,
    - •• carbocyclic aryl, and
    - •• halogenated carbocyclic aryl,

(vii) heterocyclyl, and
heterocyclyl substituted by substituent(s) independently selected from the group consisting of:

- halogen,
- nitro,
- amino,
- hydroxy,
- $C_{1-5}$ alkyl,
- $C_{1-5}$ alkyl substituted by substituent(s) independently selected from the group consisting of:

    - •• halogen,
    - •• hydroxy,
    - •• $C_{1-5}$ alkylthio,
    - •• $C_{1-5}$ alkylthio substituted by carbocyclic aryl,
    - •• $C_{1-5}$ alkylthio substituted by halogenated carbocyclic aryl,
    - •• carbocyclic aryl,
    - •• carbocyclic aryl substituted by halogen, and
    - •• heterocyclyl,

- $C_{1-5}$ alkoxy,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,
- di-$C_{1-5}$ alkylamino,
- $C_{1-5}$ alkylthio,
- $C_{2-5}$ alkenylthio,
- carbocyclic arylthio,

- carbocyclic arylthio substituted by $C_{1-5}$ alkoxycarbonyl,
- $C_{1-5}$ alkylsulfonyl,
- carbocyclic arylsulfonyl,
- carbocyclic arylsulfonyl substituted by $C_{1-5}$ alkyl,
- $C_{1-5}$ alkoxycarbonyl,
- $C_{1-5}$ alkoxycarbonyl substituted by carbocyclic aryl,
- carbocyclic aryl,
- carbocyclic aryl substituted by substituent(s) independently selected from the group consisting of:

  - •• halogen,
  - •• nitro,
  - •• $C_{1-5}$ alkyl, and
  - •• $C_{1-5}$ alkyl substituted by halogen,

- heterocyclyl;

wherein carbocyclic aryl is phenyl, naphthyl, or anthranyl;

carbocyclyl is 1,2,3,4-tetrahydronaphthyl, 1-oxo-indanyl, 9-fluorenyl, 9*H*-fluorenyl, 9-oxo-9*H*-fluorenyl, adamantly, bicyclo[2.2.1]heptenyl, bicyclo[2.2.1]heptyl, indanyl, indenyl, or menthyl;

heterocyclyl is 1,2,3-triazolyl, 1*H*-indolyl, 1*H*-pyrrolyl, 2,3-dihydro-1-oxo-isoindolyl, 2,3-dihydro-benzo[1,4]dioxinyl, 2,3-dihydro-benzofuryl, 2,4-dihydro-3-oxo-pyrazolyl, 2*H*-benzopyranyl, 2-oxo-benzopyranyl, 3,4-dihydro-2*H*-benzo[b][1,4]dioxepinyl, 4,5,6,7-tetrahydro-benzo[b]thienyl, 4*H*-benzo[1,3]dioxinyl, 4-oxo-1,5,6,7-tetrahydro-indolyl, 4-oxo-benzopyranyl, 9*H*-carbazolyl, 9*H*-xanthenyl, azetidinyl, benzo[1,3]dioxolyl, benzo[2,1,3]oxadiazolyl, benzo[1,2,5]oxadiazolyl, benzo[2,1,3]thiadiazolyl, benzo[b]thienyl, benzofuryl, benzothiazolyl, fury], imidazo [2,1-b]thiazolyl, imidazolyl, isoxazolyl, morpholino, morpholinyl, oxazolyl, phenanthro[9,10-d]oxazolyl, piperidyl, pyrazolyl, pyridyl, pyrimidyl, quinolyl, quinoxalyl, tetrahydrofuryl, thiazolyl, or thienyl; and

halogen is fluoro, chloro, bromo, or iodo;

or a pharmaceutically acceptable salt, hydrate, or solvate thereof.

**3.** The compound according to claim 2 wherein Q is Formula (II);

$R_1$ is selected from the group consisting of:

(i) $C_{1-8}$ alkyl, and

$C_{1-8}$ alkyl substituted by substituent(s) independently selected from the group consisting of:

- halogen,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- heterocyclyloxy,
- heterocyclyloxy substituted by $C_{1-5}$ alkyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by cyano,
- mono-$C_{1-5}$ alkylamino substituted by carbocyclic aryl,
- di-$C_{1-5}$ alkylamino,
- di-$C_{1-5}$ alkylamino substituted by cyano,
- di-$C_{1-5}$ alkylamino substituted by carbocyclic aryl,
- mono-carbocyclic arylamino,
- di-carbocyclic arylamino,
- $C_{1-5}$ alkoxycarbonylamino,
- carbocyclic arylcarbonylamino,

- carbocyclic arylsulfonylamino,
- carbocyclic arylsulfonylamino substituted $C_{1-5}$ alkyl,
- $C_{1-5}$ alkylthio,
- $C_{1-5}$ alkylthio substituted by substituent(s) independently selected from the group consisting of:
  - •• carbocyclic aryl,
  - •• carbocyclic aryl substituted by halogen, and
  - •• carbocyclic aryl substituted by $C_{1-5}$ alkoxy,
- carbocyclic arylthio,
- heterocyclylthio,
- heterocyclylthio substituted by $C_{1-5}$ alkyl,
- $C_{3-6}$ cycloalkyl,
- $C_{3-6}$ cycloalkenyl,
- carbocyclyl,
- carbocyclyl substituted by substituent(s) independently selected from the group consisting of:
  - •• halogen,
  - •• $C_{1-5}$ alkyl,
  - •• $C_{1-5}$ alkoxy,
  - •• $C_{2-5}$ alkenyl, and
  - •• $C_{2-5}$ alkenyl substituted by substituent(s) independently selected from the group consisting of:
    - ••• carbocyclic aryl, and
    - ••• carbocyclic aryl substituted by $C_{1-5}$ alkylsulfinyl,
- carbocyclic aryl,
- carbocyclic aryl substituted by substituent(s) independently selected from the group consisting of:
  - •• halogen,
  - •• hydroxy,
  - •• nitro,
  - •• $C_{1-5}$ alkyl,
  - •• $C_{1-5}$ alkyl substituted by substituent(s) independently selected from the group consisting of:
    - ••• oxo,
    - ••• carbocyclic aryl, and
    - ••• heterocyclyl,
  - •• $C_{2-5}$ alkenyl,
  - •• $C_{1-5}$ alkoxy,
  - •• $C_{1-5}$ alkoxy substituted by halogen,
  - •• $C_{1-5}$ alkoxy substituted by carbocyclic aryl,
  - •• carbocyclic aryloxy,
  - •• mono-carbocyclic arylaminocarbonyl,
  - •• mono-carbocyclic arylaminocarbonyl substituted by halogen,
  - •• di-carbocyclic arylaminocarbonyl,
  - •• di-carbocyclic arylaminocarbonyl substituted by halogen,
  - •• carbocyclic aryl, and
  - •• heterocyclyl,
- heterocyclyl, and
- heterocyclyl substituted by substituent(s) independently selected from the group consisting of:
  - •• $C_{1-5}$ alkyl,
  - •• $C_{1-5}$ alkyl substituted by carbocyclic aryl,
  - •• $C_{1-5}$ alkoxy,
  - •• $C_{1-5}$ alkoxy substituted by carbocyclic aryl,

- •• carbocyclic aryl, and
- •• carbocyclic aryl substituted by halogen,

(ii) $C_{2-7}$ alkenyl, and
$C_{2-7}$ alkenyl substituted by substituent(s) independently selected from the group consisting of:

- carbocyclic aryl,
- carbocyclic aryl substituted by substituent(s) independently selected from the group consisting of:

  - •• halogen,
  - •• nitro, and
  - •• $C_{1-5}$ alkoxy,

(iii) $C_{2-5}$ alkynyl, and
$C_{2-5}$ alkynyl substituted by carbocyclic aryl,
(iv) $C_{3-6}$ cycloalkyl, and
$C_{3-6}$ cycloalkyl substituted by substituent(s) independently selected from the group consisting of:

- $C_{1-5}$ alkyl,
- $C_{1-5}$ alkyl substituted by oxo,
- $C_{1-5}$ alkyl substituted by carbocyclic aryl, and
- carbocyclic aryl,

(v) carbocyclyl,
(vi) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:

  - •• halogen,
  - •• oxo,
  - •• carbocyclic aryloxy,
  - •• carbocyclic aryl, and
  - •• carbocyclic aryl substituted by $C_{1-5}$ alkyl,

- $C_{1-5}$ alkoxy,
- $C_{1-5}$ alkoxy substituted by substituent(s) independently selected from the group consisting of:

  - •• halogen,
  - •• carbocyclic aryl, and
  - •• halogenated carbocyclic aryl,

- $C_{2-5}$ alkenyloxy,
- $C_{3-6}$ cycloalkoxy,
- carbocyclic aryloxy,
- carbocyclic aryloxy substituted by $C_{1-5}$ alkoxy,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,

- di-$C_{1-5}$ alkylamino,
- mono-$C_{1-5}$ alkylamino substituted by cyano,
- di-$C_{1-5}$ alkylamino substituted by cyano,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,
- mono-$C_{1-5}$ alkylaminosulfonyl,
- di-$C_{1-5}$ alkylaminosulfonyl, and
- carbocyclic aryl,

(vii) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:
    - •• halogen,
    - •• hydroxy,
    - •• $C_{1-5}$ alkylthio,
    - •• $C_{1-5}$ alkylthio substituted by carbocyclic aryl,
    - •• $C_{1-5}$ alkylthio substituted by halogenated carbocyclic aryl,
    - •• carbocyclic aryl,
    - •• carbocyclic aryl substituted by halogen, and
    - •• heterocyclyl,
- $C_{1-5}$ alkoxy,
- carbocyclic aryloxy,
- carbocyclic aryloxy substituted by $C_{1-5}$ alkyl,
- $C_{1-5}$ alkylthio,
- $C_{1-5}$ alkenylthio,
- carbocyclic arylthio,
- carbocyclic arylthio substituted by $C_{1-5}$ alkoxycarbonyl,
- $C_{1-5}$ alkylsulfonyl,
- carbocyclic arylsulfonyl,
- carbocyclic arylsulfonyl substituted by $C_{1-5}$ alkyl,
- $C_{1-5}$ alkoxycarbonyl,
- carbocyclic aryl,
- carbocyclic aryl substituted by substituent(s) independently selected from the group consisting of:
    - •• halogen,
    - •• nitro,
    - •• $C_{1-5}$ alkyl, and
    - •• $C_{1-5}$ alkyl substituted by halogen,
- heterocyclyl;

$R_2$ is methylamino or dimethylamino when Y is a single bond or $-CH_2-$;
wherein carbocyclic aryl is phenyl, naphthyl, or anthranyl;
carbocyclyl is 1,2,3,4-tetrahydronaphthyl, 1-oxo-indanyl, 9-fluorenyl, 9-oxo-9*H*-fluorenyl, bicyclo[2.2.1]heptyl, indenyl, or menthyl;

heterocyclyl is 1,2,3-triazolyl, 1*H*-indolyl, 1*H*-pyrrolyl, 2,3-dihydro-1-oxo-isoindolyl, 2,3-dihydro-benzo[1,4]dioxinyl, 2,3-dihydro-benzofuryl, 2,4-dihydro-3-oxo-pyrazolyl, 2*H*-benzopyranyl, 2-oxo-benzopyranyl, 3,4-dihydro-2*H*-benzo[b][1,4]dioxepinyl, 4-oxo-benzopyranyl, 9*H*-carbazolyl, 9*H*-xanthenyl, azetidinyl, benzo[1,3]dioxolyl, benzo[2,1,3]oxadiazolyl, benzo[1,2,5]oxadiazolyl, benzo[b]thienyl, benzofuryl, benzothiazolyl, furyl, imidazo[2,1-b]thiazolyl, imidazolyl, isoxazolyl, morpholino, pyrazolyl, pyridyl, pyrimidyl, quinolyl, quinoxalyl, thiazolyl, or thienyl; and

halogen is fluoro, chloro, bromo, or iodo;

or a pharmaceutically acceptable salt, hydrate, or solvate thereof.

**4.** The compound according to claim 3 wherein $R_1$ is selected from the group consisting of:

(i) $C_{1-7}$ alkyl, and
$C_{1-7}$ alkyl substituted by substituent(s) independently selected from the group consisting of:

- $C_{1-5}$ alkoxy,
- $C_{1-5}$ alkoxy substituted by carbocyclic aryl,
- carbocyclic aryloxy,
- carbocyclic aryloxy substituted by halogen,
- mono-$C_{1-5}$ alkylamino,
- mono-$C_{1-5}$ alkylamino substituted by substituent(s) independently selected from the group consisting of:
  - •• cyano, and
  - •• carbocyclic aryl,
- di-$C_{1-5}$ alkylamino,
- di-$C_{1-5}$ alkylamino substituted by substituent(s) independently selected from the group consisting of:
  - •• cyano, and
  - •• carbocyclic aryl,
- mono-carbocyclic arylamino,
- di-carbocyclic arylamino,
- carbocyclic arylsulfonylamino,
- carbocyclic arylsulfonylamino substituted by $C_{1-5}$ alkyl,
- carbocyclic aryl,
- carbocyclic aryl substituted by substituent(s) independently selected from the group consisting of:
  - •• halogen,
  - •• nitro,
  - •• $C_{1-5}$ alkyl,
  - •• $C_{1-5}$ alkyl substituted by substituent(s) independently selected from the group consisting of:
    - ••• oxo, and
    - ••• carbocyclic aryl,
  - •• $C_{1-5}$ alkoxy,
  - •• $C_{1-5}$ alkoxy substituted by halogen,
- heterocyclyl,
- heterocyclyl substituted by carbocyclic aryl, and
- heterocyclyl substituted by halogen,

(ii) $C_{2-7}$ alkenyl, and
$C_{2-7}$ alkenyl substituted by substituent(s) independently selected from the group consisting of:

- carbocyclic aryl, and
- carbocyclic aryl substituted by $C_{1-5}$ alkoxy,

(iii) $C_{2-5}$ alkynyl, and

$C_{2-5}$ alkynyl substituted by carbocyclic aryl,
(iv) $C_{3-6}$ cycloalkyl, and
$C_{3-6}$ cycloalkyl substituted by substituent(s) independently selected from the group consisting of:

- $C_{1-5}$ alkyl, and
- $C_{1-5}$ alkyl substituted by carbocyclic aryl,

(v) carbocyclic aryl, and
carbocyclic aryl substituted by substituent(s) independently selected from the group consisting of:

- halogen,
- hydroxy,
- cyano,
- $C_{1-5}$ alkyl,
- $C_{1-5}$ alkyl substituted by halogen,
- $C_{1-5}$ alkoxy,
- $C_{1-5}$ alkoxy substituted by substituent(s) independently selected from the group consisting of:

  - •• halogen, and
  - •• carbocyclic aryl,
  - •• carbocyclic aryl substituted by halogen,

- $C_{2-5}$ alkenyloxy,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{1-5}$ alkylthio, and
- $C_{1-5}$ alkylthio substituted by halogen,

(vi) heterocyclyl, and
heterocyclyl substituted by substituent(s) independently selected from the group consisting of:

- halogen,
- $C_{1-5}$ alkyl,
- $C_{1-5}$ alkyl substituted by substituent(s) independently selected from the group consisting of:

  - •• hydroxy, and
  - •• carbocyclic aryl,

- $C_{1-5}$ alkoxy,
- carbocyclic arylthio,
- carbocyclic arylthio substituted by $C_{1-5}$ alkoxycarbonyl,
- $C_{1-5}$ alkoxycarbonyl,
- carbocyclic aryl,
- carbocyclic aryl substituted by substituent(s) independently selected from the group consisting of:

  - •• halogen,
  - •• $C_{1-5}$ alkyl, and
  - •• $C_{1-5}$ alkyl substituted by halogen,

L is Formula (VII);
Y is a single bond or $-CH_2-$;
$R_2$ is methylamino or dimethylamino;
wherein carbocyclic aryl is phenyl or naphthyl;
heterocyclyl is 1*H*-indolyl, 1*H*-pyrrolyl, 2,3-dihydro-benzo[1,4]dioxinyl, 4-oxo-benzopyranyl, 9*H*-carbazolyl, azetidinyl, benzo[1,3]dioxolyl, benzo[b]thienyl, furyl, imidazo[2,1-b]thiazolyl, pyrazolyl, pyridyl, or thienyl; and

halogen is fluoro, chloro, bromo, or iodo;

or a pharmaceutically acceptable salt, hydrate, or solvate thereof.

**5.** The compound according to claim 4 wherein p is 0; $R_3$ and $R_4$ are hydrogen; A is a single bond or $-CH_2-$; and B is a single bond or $-CH_2-$;
or a pharmaceutically acceptable salt, hydrate, or solvate thereof.

**6.** The compound according to claim 5 wherein $R_1$ is selected from the group consisting of:

(i) $C_{1-5}$ alkyl, and
$C_{1-5}$ alkyl substituted by substituent(s) independently selected from the group consisting of:

- mono-$C_{1-5}$ alkylamino,
- mono-$C_{1-5}$ alkylamino substituted by cyano,
- di-$C_{1-5}$ alkylamino,
- di-$C_{1-5}$ alkylamino substituted by cyano,
- mono-carbocyclic arylamino,
- di-carbocyclic arylamino,
- carbocyclic aryl,
- carbocyclic aryl substituted by substituent(s) independently selected from the group consisting of:

  •• halogen, and
  •• $C_{1-5}$ alkoxy,

- heterocyclyl, and
- heterocyclyl substituted by carbocyclic aryl,

(ii) $C_{2-5}$ alkenyl, and
$C_{2-5}$ alkenyl substituted by carbocyclic aryl,
(iii) carbocyclic aryl, and
carbocyclic aryl substituted by substituent(s) independently selected from the group consisting of:

- halogen,
- hydroxy,
- $C_{1-5}$ alkoxy,
- $C_{1-5}$ alkoxy substituted by halogen, and
- $C_{2-5}$ alkenyloxy,

(iv) heterocyclyl, and
heterocyclyl substituted by substituent(s) independently selected from the group consisting of:

- halogen,
- $C_{1-5}$ alkyl,
- $C_{1-5}$ alkyl substituted by carbocyclic aryl,
- $C_{1-5}$ alkoxy, and
- $C_{1-5}$ alkoxycarbonyl;

wherein carbocyclic aryl is phenyl or naphthyl;
heterocyclyl is 1H-indolyl, azetidinyl, or benzo[1,3]dioxolyl; and
halogen is fluoro, chloro, bromo, or iodo;

or a pharmaceutically acceptable salt, hydrate, or solvate thereof.

**7.** The compound according to claim 1 selected from the group consisting of:

ethyl 4,6-dichloro-3-{[(cis-4-{[4-(dimethylamino)quinolin-2-yl]amino}-cyclohexyl)amino]methyl}-1H-indole-2-carboxylate;

3-[{2-[(*cis*-4-{[4-(dimethylamino)quinolin-2-yl]amino}cyclohexyl)-amino]ethyl}(phenyl)amino]propanenitrile;
$N^4$,$N^4$-dimethyl-$N^2$-(cis-4-{[2-(2-phenyl-1H-indol-3-yl)ethyl]amino}-cyclohexyl)quinoline-2,4-diamine;
$N^2$-[cis-4-({[1-(diphenylmethyl)azetidin-3-yl]methyl}amino)cyclohexyl]-$N^4$,$N^4$-dimethylquinoline-2,4-diamine;
$N^2$-(cis-4-{[(2,6-dimethoxybenzyl)amino]methyl}cyclohexyl)-$N^4$,$N^4$-dimethylquinoline-2,4-diamine;
$N^2$-(cis-4-{[(2-ethoxybenzyl)amino]methyl}cyclohexyl)-$N^4$,$N^4$-dimethylquinoline-2,4-diamine;
$N^2$-[cis-4-({[(4-methoxy-1-naphthyl)methyl]amino}methyl)cyclohexyl]-$N^4$,$N^4$-dimethylquinoline-2,4-diamine;
4-bromo-2-({[(cis-4-{[4-(dimethylamino)quinolin-2-yl]amino}cyclohexyl)-methyl]amino}methyl)-6-methoxyphenol;
$N^2$-[cis-4-({[(5-bromo-1H-indol-3-yl)methyl]amino}methyl)cyclohexyl]-$N^4$,$N^4$-dimethylquinoline-2,4-diamine;
$N^2$-(cis-4-{[(5-bromo-2,4-dimethoxybenzyl)amino]methyl}cyclohexyl)-$N^4$,$N^4$-dimethylquinoline-2,4-diamine;
$N^2$-(cis-4-{[(3,3-diphenylprop-2-en-1-yl)amino]methyl}cyclohexyl)-$N^4$,$N^4$-dimethylquinoline-2,4-diamine;
$N^4$,$N^4$-dimethyl-$N^2$-(cis-4-{[(2,4,6-trimethoxybenzyl)amino]methyl}-cyclohexyl)quinoline-2,4-diamine;
$N^2$-(cis-4-{[(2,5-diethoxybenzyl)amino]methyl}cyclohexyl)-$N^4$,$N^4$-dimethylquinoline-2,4-diamine;
$N^2$-(cis-4-{[(2,4-diethoxybenzyl)amino]methyl}cyclohexyl)-$N^4$,$N^4$-dimethylquinoline-2,4-diamine;
$N^2$-(cis-4-{[(3,5-dibromo-2-methoxybenzyl)amino]methyl}cyclohexyl)-$N^4$,$N^4$-dimethylquinoline-2,4-diamine;
$N^4$,$N^4$-dimethyl-$N^2$-(cis-4-{[(2,4,5-triethoxybenzyl)amino]methyl}-cyclohexyl)quinoline-2,4-diamine;
$N^4$,$N^4$-dimethyl-$N^2$-(cis-4-{[(2,4,5-trimethoxybenzyl)amino]methyl}-cyclohexyl)quinoline-2,4-diamine;
$N^2$-[cis-4-({[2-(allyloxy)benzyl]amino}methyl)cyclohexyl]-$N^4$,$N^4$-dimethylquinoline-2,4-diamine;
$N^2$-[cis-4-({[(7-methoxy-1,3-benzodioxol-5-yl)methyl]amino}methyl)-cyclohexyl]-$N^4$,$N^4$-dimethylquinoline-2,4-diamine;
$N^2$-{cis-4-[2-(4-bromo-2-trifluoromethoxy-phenyl)-ethylamino]-cyclohexyl}-$N^4$,$N^4$-dimethyl-quinoline-2,4-diamine;
$N^2$-[cis-4-(4-bromo-2-trifluoromethoxy-benzyl)amino-cyclohexyl]-$N^4$,$N^4$-dimethyl-quinoline-2,4-diamine;
$N^2$-[cis-4-(4-bromo-2-trifluoromethoxy-benzyl)amino-cyclohexyl]-$N^4$-methyl-quinoline-2,4-diamine;
$N^2$-{4-[2-(4-bromo-2-trifluoromethoxy-phenyl)-ethylamino]-cyclohexyl}-$N^4$-methyl-quinoline-2,4-diamine;
$N^4$-methyl-$N^2$-{cis-4-[(2-trifluoromethoxy-benzyl)amino-methyl]-cyclohexyl}-quinoline-2,4-diamine;
$N^2$-{cis-4-[(4-bromo-2-trifluoromethoxy-benzyl)amino-methyl]-cyclohexyl}-$N^4$-methyl-quinoline-2,4-diamine;
$N^2$-{cis-4-[(4-bromo-2-trifluoromethoxy-benzyl)amino-methyl]-cyclohexyl}-$N^4$,$N^4$-dimethyl-quinoline-2,4-diamine;
$N^4$,$N^4$-dimethyl-$N^2$-{cis-4-[(2-trifluoromethoxy-benzyl)amino-methyl]-cyclohexyl}-quinoline-2,4-diamine;
cis-N-(3,5-dimethoxybenzyl)-N'-(4-methylquinolin-2-yl)cyclohexane-1,4-diamine; and
cis-N-(3,5-dichlorobenzyl)-N'-(4-methylquinolin-2-yl)cyclohexane-1,4-diamine;
or a pharmaceutically acceptable salt, hydrate, or solvate thereof.

**8.** The compound according to claim 3 wherein $R_1$ is selected from the group consisting of:

(i) $C_{1-5}$ alkyl, and
$C_{1-5}$ alkyl substituted by substituent(s) independently selected from the group consisting of:

- hydroxy,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by substituent(s) independently selected from the group consisting of:

  - •• halogen,
  - •• nitro,
  - •• $C_{1-5}$ alkyl,
  - •• $C_{1-5}$ alkoxy, and
  - •• $C_{1-5}$ alkoxy substituted by halogen,

- heterocyclyloxy,
- heteroeyclyloxy substituted by $C_{1-5}$ alkyl,
- mono-$C_{1-5}$ alkylaminocarbonyl,
- di-$C_{1-5}$ alkylaminocarbonyl,
- mono-$C_{1-5}$ alkylamino,

- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- carbocyclic arylcarbonylamino,
- $C_{1-5}$ alkoxycarbonylamino,
- $C_{1-5}$ alkylthio,
- $C_{1-5}$ alkylthio substituted by substituent(s) independently selected from the group consisting of:

  - •• carbocyclic aryl, and
  - •• carbocyclic aryl substituted by substituent(s) independently selected from the group consisting of:

    - ••• halogen, and
    - ••• $C_{1-5}$ alkoxy,

- carbocyclic arylthio,
- heterocyclylthio,
- heterocyclylthio substituted by $C_{1-5}$ alkyl,
- $C_{3-6}$ cycloalkyl,
- $C_{3-6}$ cycloalkenyl,
- carbocyclyl,
- carbocyclyl substituted by substituent(s) independently selected from the group consisting of:

  - •• halogen,
  - •• $C_{1-5}$ alkyl,
  - •• $C_{1-5}$ alkoxy,
  - •• $C_{2-5}$ alkenyl, and
  - •• $C_{2-5}$ alkenyl substituted by substituent(s) independently selected from the group consisting of:

    - ••• carbocyclic aryl, and
    - ••• carbocyclic aryl substituted by $C_{1-5}$ alkylsulfinyl,

- carbocyclic aryl,
- carbocyclic aryl substituted by substituent(s) independently selected from the group consisting of:

  - •• halogen,
  - •• hydroxy,
  - •• nitro,
  - •• $C_{1-5}$ alkyl,
  - •• $C_{1-5}$ alkyl substituted by substituent(s) independently selected from the group consisting of:

    - ••• oxo,
    - ••• carbocyclic aryl, and
    - ••• heterocyclyl,

  - •• $C_{1-5}$ alkoxy,
  - •• $C_{1-5}$ alkoxy substituted by halogen,
  - •• $C_{1-5}$ alkoxy substituted by carbocyclic aryl,
  - •• carbocyclic aryloxy,
  - •• mono-carbocyclic arylaminocarbonyl,
  - •• mono-carbocyclic arylaminocarbonyl substituted by halogen,
  - •• di-carbocyclic arylaminocarbonyl,
  - •• di-carbocyclic arylaminocarbonyl substituted by halogen,
  - •• carbocyclic aryl, and
  - •• heterocyclyl,

- heterocyclyl, and

- heterocyclyl substituted by substituent(s) independently selected from the group consisting of:

  - $C_{1-5}$ alkyl,
  - $C_{1-5}$ alkyl substituted by carbocyclic aryl,
  - $C_{1-5}$ alkoxy,
  - $C_{1-5}$ alkoxy substituted by carbocyclic aryl,
  - carbocyclic aryl, and
  - carbocyclic aryl substituted by halogen,

(ii) $C_{2-5}$ alkenyl, and
$C_{2-5}$ alkenyl substituted by substituent(s) independently selected from the group consisting of:

- carbocyclic aryl,
- carbocyclic aryl substituted by substituent(s) independently selected from the group consisting of:

  - halogen, and
  - nitro,

(iii) $C_{3-6}$ cycloalkyl, and
$C_{3-6}$ cycloalkyl substituted by substituent(s) independently selected from the group consisting of:

- $C_{1-5}$ alkyl,
- $C_{1-5}$ alkyl substituted by substituent(s) independently selected from the group consisting of:

  - oxo, and
  - carbocyclic aryl, and

- carbocyclic aryl,

(iv) carbocyclyl,
(v) carbocyclic aryl, and
carbocyclic aryl substituted by substituent(s) independently selected from the group consisting of:

- halogen,
- hydroxy,
- cyano,
- nitro,
- carboxy,
- carbamoyl,
- $C_{1-5}$ alkyl,
- $C_{1-5}$ alkyl substituted by substituent(s) independently selected from the group consisting of:

  - halogen,
  - hydroxy,
  - oxo,
  - carbocyclic aryloxy,
  - carbocyclic aryl, and
  - carbocyclic aryl substituted by $C_{1-5}$ alkyl,

- $C_{1-5}$ alkoxy,
- $C_{1-5}$ alkoxy substituted by substituent(s) independently selected from the group consisting of:

  - halogen, and
  - carbocyclic aryl,

- carbocyclic aryloxy,
- carbocyclic aryloxy substituted by $C_{1-5}$ alkoxy,
- $C_{1-5}$ alkoxycarbonyl,

- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,
- mono-$C_{1-5}$ alkylaminosulfonyl,
- di-$C_{1-5}$ alkylaminosulfonyl, and
- carbocyclic aryl,

(vi) heterocyclyl, and
heterocyclyl substituted by substituent(s) independently selected from the group consisting of:

- halogen,
- nitro,
- hydroxy,
- amino,
- $C_{1-5}$ alkyl,
- $C_{1-5}$ alkyl substituted by substituent(s) independently selected from the group consisting of:

  - •• halogen,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by halogen, and
  - •• heterocyclyl,

- $C_{1-5}$ alkoxy,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,
- di-$C_{1-5}$ alkylamino,
- $C_{1-5}$ alkylthio,
- $C_{2-5}$ alkenylthio,
- carbocyclic arylthio,
- $C_{1-5}$ alkylsulfonyl,
- carbocyclic arylsulfonyl,
- carbocyclic arylsulfonyl substituted by $C_{1-5}$ alkyl,
- carbocyclic aryl,
- carbocyclic aryl substituted by substituent(s) independently selected from the group consisting of:

  - •• halogen,
  - •• nitro, and
  - •• $C_{1-5}$ alkyl,

- heterocyclyl;

L is Formula (VII);
Y is -C(O)-;
wherein carbocyclic aryl is phenyl, naphthyl, or anthranyl; carbocyclyl is 1,2,3,4-tetrahydronaphthyl, 1-oxo-indanyl, 9-oxo-9*H*-fluorenyl, or indenyl;
heterocyclyl is 1,2,3-triazolyl, 1*H*-indolyl, 1*H*-pyrrolyl, 2,3-dihydro-1-oxo-isoindolyl, 2,3-dihydro-benzofuryl, 2,4-dihydro-3-oxo-pyrazolyl, 2*H*-benzopyranyl, 2-oxo-benzopyranyl, 9*H*-xanthenyl, benzo[1,3]dioxolyl, benzo[2,1,3]oxadiazolyl, benzo[1,2,5]oxadiazolyl, benzo[b]thienyl, benzofuryl, benzothiazolyl, furyl, imidazolyl, isoxazolyl, morpholino, pyrazolyl, pyridyl, pyrimidyl, quinolyl, quinoxalyl, thiazolyl, or thienyl; and
halogen is fluoro, chloro, bromo, or iodo;

or a pharmaceutically acceptable salt, hydrate, or solvate thereof.

**9.** The compound according to claim 8 wherein $R_2$ is hydrogen, halogen, methyl, trifluoromethyl, methoxy, carbamoyl, amino, methylamino, or dimethylamino; p is 0; $R_3$ and $R_4$ are hydrogen; A is a single bond; B is a single bond or $-CH_2-$;
or a pharmaceutically acceptable salt, hydrate, or solvate thereof.

**10.** The compound according to claim 9 wherein $R_1$ is selected from the group consisting of:

(i) $C_{1-5}$ alkyl, and
$C_{1-5}$ alkyl substituted by substituent(s) independently selected from the group consisting of:

- oxo,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,
- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- $C_{3-6}$ cycloalkyl,
- carbocyclic aryl,
- carbocyclic aryl by substituent(s) independently selected from the group consisting of:

  - •• halogen,
  - •• $C_{1-5}$ alkyl, and
  - •• $C_{1-5}$ alkoxy,

- heterocyclyl, and
- heterocyclyl substituted by substituent(s) independently selected from the group consisting of:

  - •• $C_{1-5}$ alkyl,
  - •• $C_{1-5}$ alkoxy, and
  - •• carbocyclic aryl,

(ii) $C_{2-5}$ alkenyl, and
$C_{2-5}$ alkenyl substituted by substituent(s) independently selected from the group consisting of:

- carbocyclic aryl,
- carbocyclic aryl substituted by substituent(s) independently selected from the group consisting of:

  - •• halogen, and
  - •• nitro,

(iii) carbocyclic aryl, and
carbocyclic aryl substituted by substituent(s) independently selected from the group consisting of:

- halogen,
- hydroxy,
- cyano,
- nitro,
- carbamoyl,
- $C_{1-5}$ alkyl,
- $C_{1-5}$ alkyl substituted by halogen,
- $C_{1-5}$ alkyl substituted by hydroxy,
- $C_{1-5}$ alkoxycarbonyl,
- $C_{1-5}$ alkoxy,
- $C_{1-5}$ alkoxy substituted by substituent(s) independently selected from the group consisting of:
  - •• halogen, and
  - •• carbocyclic aryl,
- carbocyclic aryloxy, and
- carbocyelie aryloxy substituted by $C_{1-5}$ alkoxy,

(iv) heterocyclyl, and
heterocyclyl substituted by substituent(s) independently selected from the group consisting of:

- halogen,
- nitro,
- amino,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,
- di-$C_{1-5}$ alkylamino,
- carbocyclic aryl,
- carbocyclic aryl substituted by halogen,
- carbocyclic aryl substituted by nitro, and
- heterocyclyl;

wherein carbocyclic aryl is phenyl;
heterocyclyl is 1,2,3-triazolyl, 1H-indolyl, 1H-pyrrolyl, 9H-xanthenyl, benzo[2,1,3]oxadiazolyl, benzo [1,2,5]oxadiazolyl, furyl, isoxazolyl, pyridyl, quinolyl, quinoxalyl, thiazolyl, or thienyl; and
halogen is fluoro, chloro, bromo, or iodo;

or a pharmaceutically acceptable salt, hydrate, or solvate thereof.

**11.** The compound according to claim 10 wherein $R_1$ is selected from the group consisting of:

(i) $C_{1-5}$ alkyl, and
$C_{1-5}$ alkyl substituted by substituent(s) independently selected from the group consisting of:

- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- mono-$C_{1-5}$ alkylamino,

- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- carbocyclic aryl,
- carbocyclic aryl by substituent(s) independently selected from the group consisting of:
  •• halogen,
  •• $C_{1-5}$ alkyl, and
  •• $C_{1-5}$ alkoxy,
  and
- heterocyclyl,

(ii) carbocyclic aryl, and
carbocyclic aryl substituted by substituent(s) independently selected from the group consisting of:

- halogen,
- nitro,
- hydroxy,
- cyano,
- $C_{1-5}$ alkyl,
- $C_{1-5}$ alkyl substituted by halogen,
- $C_{1-5}$ alkoxycarbonyl,
- $C_{1-5}$ alkoxy,
- $C_{1-5}$ alkoxy substituted by halogen,
- carbocyclic aryloxy, and
- carbocyclic aryloxy substituted by $C_{1-5}$ alkoxy,

(iii) heterocyclyl, and
heterocyclyl substituted by substituent(s) independently selected from the group consisting of:

- halogen,
- nitro,
- $C_{1-5}$ alkyl,
- carbocyclic aryloxy,
- carbocyclic aryloxy substituted by halogen,
- carbocyclic aryloxy substituted by $C_{1-5}$ alkyl,
- carbocyclic aryloxy substituted by $C_{1-5}$ alkoxy,
- carbocyclic aryl,
- carbocyclic aryl substituted by halogen,
- carbocyclic aryl substituted by nitro, and
- heterocyclyl;

wherein carbocyclic aryl is phenyl;
heterocyclyl is 1*H*-indolyl, 1*H*-pyrrolyl, 9*H*-xanthenyl, benzo[2,1,3]oxadiazolyl, benzo[1,2,5]oxadiazolyl, furyl, isoxazolyl, pyridyl, thiazolyl, or thienyl; and
halogen is fluoro, chloro, bromo, or iodo;

or a pharmaceutically acceptable salt, hydrate, or solvate thereof.

**12.** The compound according to claim 1 selected from the group consisting of:

N-(cis-4-{[4-(dimethylamino)quinolin-2-yl]amino}cyclohexyl)-3-methoxybenzamide;
3-bromo-N-(cis-4-{[4-(dimethylamino)quinolin-2-yl]amino}cyclohexyl)-benzamide;
4-bromo-N-(cis-4-{[4-(dimethylamino)quinolin-2-yl]amino}cyclohexyl)-benzamide;
N-(cis-4-{[4-(dimethylamino)quinolin-2-yl]amino}cyclohexyl)-2,1,3-benzoxadiazole-5-carboxamide;
3-chloro-N-(cis-4-{[4-(dimethylamino)quinolin-2-yl]amino}cyclohexyl)-benzamide;
4-chloro-N-(cis-4-{[4-(dimethylamino)quinolin-2-yl]amino}cyclohexyl)-benzamide;

4-chloro-N-(cis-4-{[4-(dimethylamino)quinolin-2-yl]amino}cyclohexyl)-3-nitrobenzamide;
3-cyano-N-(cis-4-{[4-(dimethylamino)quinolin-2-yl]amino}cyclohexyl)-benzamide;
3,5-dichloro-N-(cis-4-{[4-(dimethylamino)quinolin-2-yl]amino}-cyclohexyl)benzamide;
3,4-dichloro-N-(cis-4-{[4-(dimethylamino)quinolin-2-yl]amino}-cyclohexyl)benzamide;
N-(cis-4-{[4-(dimethylamino)quinolin-2-yl]amino}cyclohexyl)-2,2-diphenylacetamide;
N-(cis-4-{(4-(dimethylamino)quinolin-2-yl]amino}cyclohexyl)-3,4-difluorobenzamide;
N-(cis-4-{[4-(dimethylamino)quinolin-2-yl]amino}cyclohexyl)-3,5-difluorobenzamide;
N-(cis-4-{[4-(dimethylamino)quinolin-2-yl]amino}cyclohexyl)-4-fluorobenzamide;
N-(cis-4-{[4-(dimethylamino)quinolin-2-yl]amino}cyclohexyl)-3-fluoro-5-(trifluoromethyl)benzamide;
N-(cis-4-{[4-(dimethylamino)quinolin-2-yl]amino}cyclohexyl)-4-methyl-3-nitrobenzamide;
N-(cis-4-{[4-(dimethylamino)quinolin-2-yl]amino}cyclohexyl)-3-nitrobenzamide;
N-(cis-4-{[4-(dimethylamino)quinolin-2-yl]amino}cyclohexyl)-2-phenoxybutanamide;
N-(cis-4-{[4-(dimethylamino)quinolin-2-yl]amino}cyclohexyl)-2-phenoxypropanamide;
N-(cis-4-{[4-(dimethylamino)quinolin-2-yl]amino}cyclohexyl)-3-methylbenzamide;
N-(cis-4-{[4-(dimethylamino)quinolin-2-yl]amino}cyclohexyl)-3-(trifluoromethoxy)benzamide;
4-bromo-N-(cis-4-{[4-(dimethylamino)quinolin-2-yl]amino}cyclohexyl)-3-methylbenzamide;
N-(cis-4-{[4-(dimethylamino)quinolin-2-yl]amino}cyclohexyl)-3-iodobenzamide;
3-chloro-N-(cis-4-{[4-(dimethylamino)quinolin-2-yl]amino}cyclohexyl)-2,4-difluorobenzamide;
N-(cis-4-{[4-(dimethylamino)quinolin-2-yl]amino}cyclohexyl)-2,5-dimethyl-3-furamide;
3-chloro-N-(cis-4-{[4-(dimethylamino)quinolin-2-yl]amino}cyclohexyl)-4-fluorobenzamide;
N-(cis-4-{[4-(dimethylamino)quinolin-2-yl]amino}cyclohexyl)-3-fluoro-4-methylbenzamide;
N-(cis-4-{[4-(dimethylamino)quinolin-2-yl]amino}cyclohexyl)-3,5-dimethoxybenzamide;
N-(cis-4-{[4-(dimethylamino)quinolin-2-yl]amino}cyclohexyl)-3,5-bis(trifluoromethyl)benzamide;
(2E)-N-(cis-4-{[4-(dimethylamino)quinolin-2-yl]amino}cyclohexyl)-3-(4-nitrophenyl)acrylamide;
N-(cis-4-{[4-(dimethylamino)quinolin-2-yl]amino}cyclohexyl)-4-fluoro-3-methylbenzamide;
2,5-dichloro-N-(cis-4-{[4-(dimethylamino)quinolin-2-yl]amino}-cyclohexyl)thiophene-3-carboxamide;
2-(4-chlorophenoxy)-N-(cis-4-{[4-(dimethylamino)quinolin-2-yl]amino}-cyclohexyl)acetamide;
3-(2-chlorophenyl)-N-(cis-4-{[4-(dimethylamino)quinolin-2-yl]amino}-cyclohexyl)-5-methylisoxazole-4-carboxamide;
1-(4-chlorophenyl)-N-(cis-4-{[4-(dimethylamino)quinolin-2-yl]amino}-cyclohexyl)cyclopentanecarboxamide;
3-(2-chloro-6-fluorophenyl)-N-(cis-4-{[4-(dimethylamino)quinolin-2-yl]amino}cyclohexyl)-5-methylisoxazole-4-carboxamide;
N-(cis-4-([4-(dimethylamino)quinolin-2-yl]amino}cyclohexyl)-3-fluorobenzamide;
N-(cis-4-{[4-(dimethylamino)quinolin-2-yl]amino}cyclohexyl)-4-fluoro-3-(trifluoromethyl)benzamide;
N-(cis-4-{[4-(dimethylamino)quinolin-2-yl]amino}cyclohexyl)-5-methyl-2-phenyl-2H-1,2,3-triazole-4-carboxamide;
N-(cis-4-{[4-(dimethylamino)quinolin-2-yl]amino}cyclohexyl)-2-(4-methoxyphenoxy)-5-nitrobenzamide;
N-(cis-4-{[4-(dimethytamino)quinoiin-2-yl]amino}cyclohexyl)-5-nitro-2-furamide;
N-(cis-4-{[4-(dimethylamino)quinolin-2-yl]amino}cyclohexyl)-2-phenoxyacetamide;
N-(cis-4-{[4-(dimethylamino)quinolin-2-yl]amino}cyclohexyl)quinoxaline-2-carboxamide;
2-(3-chlorophenoxy)-N-(cis-4-[4-(dimethylamino}quinolin-2-yl]amino}-cyclohexyl)acetamide;
3-(2,6-dichlorophenyl)-N-(cis-4-{[4-(dimethylamino)quinolin-2-yl]amino}-cyclohexyl)-5-methylisoxazole-4-carboxamide;
N-(cis-4-{[4-(dimethylamino)quinolin-2-yl]amino}cyclohexyl)-2-phenoxynicotinamide;
N-(cis-4-{[4-(dimethylamino)quinolin-2-yl]amino}cyclohexyl)-2-(4-methylphenoxy)nicotinamide;
N-(cis-4-{[4-(dimethylamino)quinolin-2-yl]amino}cyclohexyl)-2-(2-thienyl)-1,3-thiazole-4-carboxamide;
5-bromo-N-(cis-4-{[4-(dimethylamino)quinotin-2-yl]amino}cyclohexyl)-thiophene-2-carboxamide;
N-(cis-4-{[4-(dimethylamino)quinolin-2-yl]amino}cyclohexyl)-2-(2,3,6-trichlorophenyl)acetamide;
5-(4-chloro-2-nitrophenyl)-N-(cis-4-{[4-(dimethylamino)quinolin-2-yl]amino}cyclohexyl)-2-furamide;
5-chloro-N-(cis-4-{[4-(dimethylamino)quinolin-2-yl]amino}cyclohexyl)-thiophene-2-carboxamide;
N-(cis-4-{[4-(dimethylamino)quinolin-2-yl]amino}cyclohexyl)-5-iodo-2-furamide;
N-(cis-4-{[4-(dimethylamino)quinolin-2-yl]amino}cyclohexyl)-2-(2-iodophenyl)acetamide;
N-(cis-4-{[4-(dimethylamino)quinolin-2-yl]amino}cyclohexyl)-2-(5-methoxy-2-methyl-1H-indol-3-yl)acetamide;
(2E)-N-(cis-4-{[4-(dimethylamino)quinolin-2-yl]amino}cyclohexyl)-3-(3-nitrophenyl)acrylamide;
2,2-bis(4-chlorophenyl)-N-(cis-4-{[4-(dimethylamino)quinolin-2-yl]-amino}cyclohexyl)acetamide;
N-(cis-4-{[4-(dimethylamino)quinolin-2-yl]amino}cyclohexyl)-5-nitrothiophene-2-carboxamide;
N-(cis-4-{[4-(dimethylamino)quinolin-2-yl]amino}cyclohexyl)-3-methyl-4-nitrobenzamide;

N-(cis-4-{[4-(dimethylamino)quinolin-2-yl]amino}cyclohexyl)-3-methoxy-4-nitrobenzamide;
5-bromo-N-(cis-4-{[4-(dimethylamino)quinolin-2-yl]amino}cyclohexyl)-2-furamide;
4,5-dibromo-N-(cis-4-{[4-(dimethylamino)quinolin-2-yl]amino}-cyclohexyl)thiophene-2-carboxamide;
4,5-dibromo-N-(cis-4-{[4-(dimethylamino)quinolin-2-yl]amino}-cyclohexyl)-2-furamide;
N-(cis-4-{[4-(dimethylamino)quinolin-2-yl]amino}cyclohexyl)-2-(1H-indol-3-yl)acetamide;
N-(cis-4-{[4-(dimethylamino)quinolin-2-yl]amino}cyclohexyl)-2-(1H-indol-3-yl)-4-oxo-4-phenylbutanamide;
N-(cis-4-{[4-(dimethylamino)quinolin-2-yl]amino}cyclohexyl)-2-(2-phenyl-1H-indol-3-yl)acetamide;
N-(cis-4-{[4-(dimethylamino)quinolin-2-yl]amino}cyclohexyl)-2-(2,4,6-trichlorophenoxy)acetamide;
3-(benzyloxy)-N-(cis-4-{[4-(dimethylamino)quinolin-2-yl]amino}-cyclohexyl)-4-methoxybenzamide;
N-(cis-4-{[4-(dimethylamino)quinolin-2-yl]amino}cyclohexyl)-2-phenoxybenzamide;
N-(cis-4-{[4-(dimethylamino)quinolin-2-yl]amino}cyclohexyl)-2-phenylquinoline-4-carboxamide;
N-(cis-4-{[4-(dimethylamino)quinolin-2-yl]amino}cyclohexyl)-5-(3-nitrophenyl)-2-furamide;
N-(cis-4-{[4-(dimethylamino)quinolin-2-yl]amino}cyclohexyl)-5-nitrothiophene-3-carboxamide;
N-(cis-4-{[4-(dimethylamino)quinolin-2-yl]amino}cyclohexyl)-1-methyl-4-nitro-1H-pyrrole-2-carboxamide;
N-(cis-4-{[4-(dimethylamino)quinolin-2-yl]amino}cyclohexyl)-4-nitrobenzamide;
N-(cis-4-{[4-(dimethylamino)quinolin-2-yl]amino}cyclohexyl)-2-methoxy-4-nitrobenzamide;
N-(cis-4-{[4-(dimethylamino)quinolin-2-yl]amino}cyclohexyl)-3-fluoro-4-(trifluoromethyl)benzamide;
N-(cis-4-{[4-(dimethylamino)quinolin-2-yl]amino}cyclohexyl)-3,5-dimethyl-4-nitrobenzamide;
N-(cis-4-{[4-(dimethylamino)quinolin-2-yl]amino}cyclohexyl)-2-mesityl-2-oxoacetamide;
5-chloro-N-(cis-4-{[4-(dimethylamino)quinolin-2-yl]amino}cyclohexyl)-2-hydroxybenzamide;
N-[(cis-4-{[4-(dimethylamino)quinolin-2-yl]amino}cyclohexyl)methyl]-3-methoxybenzamide;
3-bromo-N-[(cis-4-{[4-(dimethylamino)quinolin-2-yl]amino}cyclohexyl)-methyl]benzamide;
4-bromo-N-[(cis-4-{[4-(dimethylamino)quinolin-2-yl]amino}cyclohexyl)-methyl]benzamide;
N-[(cis-4-{[4-(dimethylamino)quinolin-2-yl]amino}cyclohexyl)methyl]-2,1,3-benzoxadiazole-5-carboxamide;
3-chloro-N-[(cis-4-{[4-(dimethylamino)quinolin-2-yl]amino}cyclohexyl)-methyl]benzamide;
4-chloro-N-[(cis-4-{[4-(dimethylamino)quinolin-2-yl]amino}cyclohexyl)-methyl]benzamide;
4-chloro-N-[(cis-4-{[4-(dimethylamino)quinolin-2-yl]amino}cyclohexyl)-methyl]-3-nitrobenzamide;
3-cyano-N-[(cis-4-{[4-(dimethylamino)quinolin-2-yl]amino}cyclohexyl)-methyl]benzamide;
3,5-dichloro-N-[(cis-4-{[4-(dimethylamino)quinolin-2-yl]amino}-cyclohexyl)methyl]benzamide;
3,4-dichloro-N-[(cis-4-{[4-(dimethylamino)quinolin-2-yl]amino}-cyclohexyl)methyl]benzamide;
N-[(cis-4-{[4-(dimethylamino)quinolin-2-yl]amino}cyclohexyl)methyl]-2,2-diphenylacetamide;
N-[(cis-4-{[4-(dimethylamino)quinolin-2-yl]amino}cyclohexyl)methyl]-3,4-difluorobenzamide;
N-[(cis-4-{[4-(dimethylamino)quinolin-2-yl]amino}cyclohexyl)methyl]-3,5-difluorobenzamide;
N-[(cis-4-{[4-(dimethylamino)quinolin-2-yl]amino}cyclohexyl)methyl]-4-fluorobenzamide;
N-[(cis-4-{[4-(dimethylamino)quinolin-2-yl]amino}cyclohexyl)methyl]-3-fluoro-5-(trifluoromethyl)benzamide;
N-[(cis-4-[4-(dimethylamino)quinolin-2-yl]amino}cyclohexyl)methyl]-4-methyl-3-nitrobenzamide;
N-[(cis-4-{[4-(dimethylamino)quinolin-2-yl]amino}cyclohexyl)methyl]-3-nitrobenzamide;
N-[(cis-4-{[4-(dimethylamino)quinolin-2-yl]amino}cyclohexyl)methyl]-2-phenoxybutanamide;
N-[(cis-4-{[4-(dimethylamino)quinolin-2-yl]amino}cyclohexyl)methyl]-2-phenoxypropanamide;
N-[(cis-4-{[4-(dimethylamino)quinolin-2-yl]amino}cyclohexyl)methyl]-3-methylbenzamide;
N-[(cis-4-{[4-(dimethylamino)quinolin-2-yl]amino}cyclohexyl)methyl]-3-(trifluoromethoxy)benzamide;
4-bromo-N-[(cis-4-{[4-(dimethylamino)quinolin-2-yl]amino}cyclohexyl)-methyl]-3-methylbenzamide;
N-[(cis-4-{[4-(dimethylamino)quinolin-2-yl]amino}cyclohexyl)methyl]-3-iodobenzamide;
3-chloro-N-[(cis-4-{[4-(dimethylamino)quinolin-2-yl]amino}cyclohexyl)-methyl]-2,4-difluorobenzamide;
N-[(cis-4-{[4-(dimethylamino)quinolin-2-yl]amino}cyclohexyl)methyl]-2,5-dimethyl-3-furamide;
3-ch!oro-N-[(cis-4-{[4-(dimethylamino)quinolin-2-yl]amino}cyclohexyl)-methyl]-4-fluorobenzamide;
N-[(cis-4-{[4-(dimethylamino)quinolin-2-yl]amino}cyclohexyl)methyl]-3-fluoro-4-methylbenzamide;
N-[(cis-4-{[4-(dimethylamino)quinolin-2-yl]amino}cyclohexyl)methyl]-3,5-dimethoxybenzamide;
N-[(cis-4-{[4-(dimethylamino)quinolin-2-yl]amino}cyclohexyl)methyl]-3,5-bis(trifluoromethyl)benzamide;
(2E)-N-[(cis-4-{[4-(dimethylamino)quinolin-2-yl]amino}cyclohexyl)-methyl]-3-(4-nitrophenyl)acrylamide;
N-[(cis-4-{[4-(dimethylamino)quinolin-2-yl]amino}cyclohexyl)methyl]-4-fluoro-3-methylbenzamide;
2,5-dichloro-N-[(cis-4-{[4-(dimethylamino)quinolin-2-yl]amino}-cyclohexyl)methyl]thiophene-3-carboxamide;
2,6-dichloro-N-[(cis-4-{[4-(dimethylamino)quinolin-2-yl]amino}-cyclohexyl)methyl]benzamide;
N-[(cis-4-{[4-(dimethylamino)quinolin-2-yl]amino}cyclohexyl)methyl]-2,4,6-trimethylbenzamide;
2,4,6-trichloro-N-[(cis-4-{[4-(dimethylamino)quinolin-2-yl]amino}-cyclohexyl)methyl]benzamide;
(2E)-3-(2-chlorophenyl)-N-[(cis-4-{[4-(dimethylamino)quinolin-2-yl]amino}cyclohexyl)methyl]acrylamide;
5-bromo-N-[(cis-4-{[4-(dimethylamino)quinolin-2-yl]amino}cyclohexyl)-methyl]thiophene-2-carboxamide;
N-[(cis-4-{[4-(dimethylamino)quinolin-2-yl]amino}cyclohexyl)methyl]-2-(2,3,6-trichlorophenyl)acetamide;

5-(4-chloro-2-nitrophenyl)-N-[(cis-4-{[4-(dimethylamino)quinolin-2-yl]amino}cyclohexyl)methyl]-2-furamide;
5-chloro-N-[(cis-4-{[4-(dimethylamino)quinolin-2-yl]amino}cyclohexyl)-methyl]thiophene-2-carboxamide;
N-[(cis-4-{[4-(dimethylamino)quinolin-2-yl]amino}cyclohexyl)methyl)-5-iodo-2-furamide;
N-[(cis-4-{[4-(dimethylamino)quinolin-2-yl]amino}cyclohexyl)methyl]-2-(2-iodophenyl)acetamide;
(2E)-N-[(cis-4-{[4-(dimethy!amino)quinolin-2-yl]amino}cyclohexyl)-methyl] -3-(3-nitrophenyl)acrylamide;
2,2-bis(4-chlorophenyl)-N-[(cis-4-{[4-(dimethylamino)quinolin-2-yl]amino}cyclohexyl)methyl]acetamide;
N-[(cis-4-{[4-(dimethylamino)quinolin-2-yl]amino}cyclohexyl)methyl]-5-nitrothiophene-2-carboxamide;
N-[(cis-4-{[4-(dimethylamino)quinolin-2-yl]amino}cyclohexyl)methyl]-3-methyl-4-nitrobenzamide;
N-[(cis-4-{[4-(dimethylamino)quinolin-2-yl]amino}cyclohexyl)methyl]-3-methoxy-4-nitrobenzamide;
N-(cis-4-(4-methyl-quinolin-2-ylamino)-cyclohexyl]-2-phenoxy-nicotinamide;
3,4-difluoro-N-[cis-4-(4-methyl-quinolin-2-ylamino)-cyclohexyl]-benzamide;
3,4-difluoro-N-[cis-4-(quinolin-2-ylamino)-cyclohexyl]-benzamide;
2-phenoxy-N-[cis-4-(quinolin-2-ylamino)-cyclohexyl]-nicotinamide;
3-chloro-N-[cis-4-(4-methyl-quinolin-2-ylamino)-cyclohexyl]-benzamide;
N-[cis-4-(4-chloro-quinolin-2-ylamino)-cyclohexyl]-2-phenoxy-nicotinamide;
3-methyl-N-[cis-4-(quinolin-2-ylamino)-cyclohexyl]-benzamide;
3-methoxy-N-[cis-4-(quinolin-2-ylamino)-cyclohexyl]-benzamide;
3-chloro-N-[cis-4-(quinolin-2-ylamino)-cyclohexyl]-benzamide;
5-nitro-thiophene-3-carboxylic acid [cis-4-(quinolin-2-ylamino)-cyclohexyl]-amide;
5-nitro-thiophene-3-carboxylic acid [cis-4-(4-methyl-quinolin-2-ylamino)-cyclohexyl]-amide;
3-chloro-4-fluoro-N-[cis-4-(quinolin-2-ylamino)-cyclohexyl]-benzamide;
3,5-dimethoxy-N-[cis-4-(quinolin-2-ylamino)-cyclohexyl]-benzamide;
3,4-dichloro-N-[cis-4-(quinolin-2-ylamino)-cyclohexyl]-benzamide;
benzo[2,3,1]oxadiazole-5-carboxylic acid [cis-4-(quinolin-2-ylamino)-cyclohexyl]-amide;
3-methyl-N-[cis-4-(4-methyl-quinolin-2-ylamino)-cyclohexyl]-benzamide;
3-methoxy-N-[cis-4-(4-methyl-quinolin-2-ylamino)-cyclohexyl]-benzamide;
4-cyano-N-[cis-4-(4-methyl-quinolin-2-ylamino)-cyclohexyl]-benzamide;
1-methyl-4-nitro-1H-pyrrole-2-carboxylic acid [cis-4-(quinolin-2-ylamino)-cyclohexyl]-amide;
9H-xanthene-9-carboxylic acid [cis-4-(quinolin-2-ylamino)-cyclohexyl]-amide;
5-(4-chloro-phenyl)-furan-2-carboxylic acid [cis-4-(quinolin-2-ylamino)-cyclohexyl]-amide;
3-nitro-N-[cis-4-(quinolin-2-ylamino)-cyclohexyl]-benzamide;
4-fluoro-3-methyl-N-[cis-4-(quinolin-2-ylamino)-cyclohexyl]-benzamide;
3-bromo-N-[cis-4-(quinolin-2-ylamino)-cyclohexyl]-benzamide;
2-(2-bromo-phenoxy)-N-[cis-4-(quinolin-2-ylamino)-cyclohexyl]-nicotinamide;
3-cyano-N-[cis-4-(quinolin-2-ylamino)-cyclohexyl]-benzamide;
N-[cis-4-(quinolin-2-ylamino)-cyclohexyl]-trifluoromethyl-benzamide;
N-{cis-4-(4-chloro-quinolin-2-ylamino)-cyclohexyl]-3,4-difluoro-benzamide;
3,4-dichloro-N-[cis-4-(4-methyl-quinolin-2-ylamino)-cyclohexyl]-benzamide;
3-chloro-4-fluoro-N-[cis-4-(4-methyl-quinolin-2-ylamino)-cyclohexyl]-benzamide;
4-fluoro-3-methyl-N-[cis-4-(4-methyl-quinolin-2-ylamino)-cyclohexyl]-benzamide;
1-methyl-4-nitro-1H-pyrrole-2-carboxylic acid [cis-4-(4-methyl-quinolin-2-ylamino)-cyclohexyl]-amide;
9H-xanthene-9-carboxylic acid [cis-4-(4-methyl-quinolin-2-ylamino)-cyclohexyl]-amide;
5-bromo-furan-2-carboxylic acid [cis-4-(4-methyl-quinolin-2-ylamino)-cyclohexyl]-amide;
N-[cis-4-(4-methyl-quinolin-2-ylamino)-cyclohexyl]-2-*m*-tolyloxy-acetamide;
N-[cis-4-(quinolin-2-ylamino)-cyclohexyl]-2-*m*-tolyloxy-acetamide;
2,2-diphenyl-N-[cis-4-(quinolin-2-ylamino)-cyclohexyl]-acetamide;
5-bromo-furan-2-carboxylic acid [cis-4-(quinolin-2-ylamino)-cyclohexyl]-amide;
benzo[2,3,1]oxadiazole-5-carboxylic acid [cis-4-(4-methyl-quinolin-2-ylamino)-cyclohexyl]-amide;
3-bromo-N-[cis-4-(4-methyl-quinolin-2-ylamino)-cyclohexyl]-benzamide;
3-cyano-N-[cis-4-(4-methyl-quinolin-2-ylamino)-cyclohexyl]-benzamide;
N-[cis-4-(4-methyl-quinolin-2-ylamino)-cyclohexyl]-3-trifluoromethyl-benzamide;
N-[cis-4-(4-methyl-quinolin-2-ylamino)-cyclohexyl]-2,2-diphenyl-acetamide;
2-(4-fluoro-phenoxy)-N-[cis-4-(quinolin-2-ylamino)-cydohexyl]-nicotinamide;
2-(4-fluoro-phenoxy)-N-[cis-4-(4-methyl-quinolin-2-ylamino)-cyclohexyl]-nicotinamide;
2-(3,4-difluoro-phenoxy)-N-[cis-4-(quinolin-2-ylamino)-cyclohexyl]-nicotinamide;
2-(3,4-difluoro-phenoxy)-N-[cis-4-(4-methyl-quinolin-2-ylamino)-cyclohexyl]-nicotinamide;
N-[cis-4-(quinolin-2-ylamino)-cyclohexyl]-2-*p*-tolyloxy-nicotinamide;
N-[cis-4-(4-methyl-quinolin-2-ylamino)-cyclohexyl]-2-*p*-tolyloxy-nicotinamide;

2-(4-chloro-phenoxy)-N-[cis-4-(quinolin-2-ylamino)-cyclohexyl]-nicotinamide;
2-(4-chloro-phenoxy)-N-[cis-4-(4-methyl-quinolin-2-ylamino)-cyclohexyl]-nicotinamide;
2-(4-bromo-phenoxy)-N-[cis-4-(quinolin-2-ylamino)-cyclohexyl]-nicotinamide;
2-(4-bromo-phenoxy)-N-[cis-4-(4-methyl-quinolin-2-ylamino)-cyclohexyl]-nicotinamide;
2-(4-methoxy-phenoxy)-N-[cis-4-(quinolin-2-ylamino)-cyclohexyl]-nicotinamide;
2-(4-methoxy-phenoxy)-N-[cis-4-(4-methyl-quinolin-2-ylamino)-cyclohexyl]-nicotinamide;
2-(3-chloro-4-fluoro-phenoxy)-N-[cis-4-(quinolin-2-ylamino)-cyclohexyl]-nicotinamide;
2-(3-cloro-4-fluoro-phenoxy)-N-[cis-4-(4-methyl-quinolin-2-ylamino)-cyclohexyl]-nicotinamide;
N-[cis-4-(quinolin-2-ylamino)-cyclohexyl]-2-*m*-tolyloxy-nicotinamide;
N-[cis-4-(4-methyl-quinolin-2-ylamino)-cyclohexyl]-2-*m*-tolyloxy-nicotinamide;
2-(3-methoxy-phenoxy)-N-[cis-4-(4-methyl-quinolin-2-ylamino)-cyclohexyl]-acetamide;
2-(3-chloro-phenoxy)-N-[cis-4-(4-methyl-quinolin-2-ylamino)-cyclohexyl]-acetamide;
2-(3-chloro-4-fluoro-phenoxy)-N-[cis-4-(4-methyl-quinolin-2-ylamino)-cyclohexyl]-acetamide;
2-(3,4-dichloro-phenoxy)-N-[cis-4-(4-methyl-quinolin-2-ylamino)-cyclohexyl]-acetamide;
C-(methyl-phenyl-amino)-N-[cis-4-(4-methyl-quinolin-2-ylamino)-cyclohexyl]-acetamide;
2-(3,4-dichloro-phenylamino)-N-[cis-4-(4-methyl-quinolin-2-ylamino)-cyclohexyl]-acetamide;
2-(3-methoxy-phenoxy)-N-[cis-4-(quinolin-2-ylamino)-cyclohexyl]-acetamide;
2-(3-chloro-phenoxy)-N-[cis-4-(quinolin-2-ylamino)-cyclohexyl]-acetamide;
2-(3-chloro-4-fluoro-phenoxy)-N-[cis-4-(quinolin-2-ylamino)-cyclohexyl]-acetamide;
2-(3,4-dichloro-phenoxy)-N-[cis-4-(quinolin-2-ylamino)-cyclohexyl]-acetamide;
C-(methyl-phenyl-amino)-N-[cis-4-(quinolin-2-ylamino)-cyclohexyl]-acetamide;
2-(3,4-dichloro-phenylamino)-N-[cis-4-(quinolin-2-ylamino)-cyclohexyl]-acetamide;
3-hydroxy-N-[cis-4-(quinolin-2-ylamino)-cyclohexyl]-benzamide;
N-[cis-4-(quinolin-2-ylamino)-cyclohexyl]-isophthalamic acid methyl ester;
N-[cis-4-(quinolin-2-ylamino)-cyclohexyl]-3-trifluoromethoxy-benzamide;
N-[cis-4-(4-methyl-quinolin-2-ylamino)-cyclohexyl]-bis-trifluoromethyl-benzamide;
N-[cis-4-(4-methyl-quinolin-2-ylamino)-cyclohexyl]-3-trifluoromethoxy-benzamide;
N-[cis-4-(4-amino-quinolin-2-ylamino)-cyclohexyl]-3,4-difluoro-benzamide;
C-(ethyl-phenyl-amino)-N-[cis-4-(quinolin-2-ylamino)-cyclohexyl]-acetamide;
C-(ethyl-phenyl-amino)-N-[cis-4-(4-methyl-quinolin-2-ylamino)-cyclohexyl]-acetamide;
3-hydroxy-N-[cis-4-(4-methyl-quinolin-2-ylamino)-cyclohexyl]-benzamide;
2-amino-N-[cis-4-(4-methyl-quinolin-2-ylamino)-cyclohexyl]-nicotinamide;
2,3-difluoro-N-[cis-4-(4-methyl-quinolin-2-ylamino)-cyclohexyl]-benzamide;
2,4-difluoro-N-[cis-4-(4-methyl-quinolin-2-ylamino)-cyclohexyl]-benzamide;
2,5-difluoro-N-[cis-4-(4-methyl-quinolin-2-ylamino)-cyclohexyl]-benzamide;
2,6-difluoro-N-[cis-4-(4-methyl-quinolin-2-ylamino)-cyclohexyl]-benzamide;
3,5-difluoro-N-[cis-4-(4-methyl-quinolin-2-ylamino)-cyclohexyl]-benzamide;
C-[(4-chloro-phenyl)-ethyl-amino]-N-[cis-4-(4-methyl-quinolin-2-ylamino)-cyclohexyl]-acetamide;
4-chloro-3-fluoro-N-[cis-4-(4-methyl-quinolin-2-ylamino)-cyclohexyl]-benzamide;
4-fluoro-N-[cis-4-(4-methyl-quinolin-2-ylamino)-cyclohexyl]-benzamide;
3-fluoro-N-[cis-4-(4-methyl-quinolin-2-ylamino)-cyclohexyl]-benzamide;
2-fluoro-N-[cis-4-(4-methyl-quinolin-2-ylamino)-cyclohexyl]-benzamide;
4-chloro-N-[cis-4-(4-methyl-quinolin-2-ylamino)-cyclohexyl]-benzamide;
N-[cis-4-(4-methyl-quinolin-2-ylamino)-cyclohexyl]-isophthalamic acid methyl ester;
3,5-difluoro-N-[cis-4-(quinolin-2-ylamino)-cyclohexyl]-benzamide;
4-chloro-3-fluoro-N-[cis-4-(quinolin-2-ylamino)-cyclohexyl]-benzamide;
C-[(4-chloro-phenyl)-ethyl-amino]-N-[cis-4-(quinolin-2-ylamino)-cyclohexyl]-acetamide;
6-chloro-N-[cis-4-(4-methyl-quinolin-2-ylamino)-cyclohexyl]-nicotinamide;
6-dimethylamino-N-[cis-4-(4-methyl-quinolin-2-ylamino)-cyclohexyl]-nicotinamide;
3-hydroxymethyl-N-[cis-4-(4-methyl-quinolin-2-ylamino)-cyclohexyl]-benzamide;
N-[cis-4-(4-methyl-quinolin-2-ylamino)-cyclohexyl]-isophthalamide;
3-chloro-5-fluoro-N-[cis-4-(4-methyl-quinolin-2-ylamino)-cyclohexyl]-benzamide;
3,4,5-trifluoro-N-[cis-4-(4-methyl-quinolin-2-ylamino)-cyclohexyl]-benzamide;
pyridine-2-carboxylic acid [cis-4-(4-methyl-quinolin-2-ylamino)-cyclohexyl]-amide;
4-chloro-pyridine-2-carboxylic acid [cis-4-(4-methyl-quinolin-2-ylamino)-cyclohexyl]-amide;
5-bromo-N-[cis-4-(4-methyl-quinolin-2-ylamino)-cyclohexyl]-nicotinamide;
N-[cis-4-(4-methyl-quinolin-2-ylamino)-cyclohexyl]-6-trifluoromethyl-nicotinamide;
3,4-difluoro-N-[cis-4-(4-methyl-quinolin-2-ylamino)-cyclohexylmethyl]-benzamide;

N-[cis-4-(4-methyl-quinolin-2-ylamino)-cyclohexylmethyl]-2-phenoxy-nicotinamide;
N-[cis-4-(4-dimethy!amino-quinolin-2-ylamino)-cyclohexylmethyl]-3,4-diiluoro-benzamide;
3,4-difluoro-N-[cis-4-(quinolin-2-ylamino)-cyclohexylmethyl]-benzamide;
2-phenoxy-N-[cis-4-(quinolin-2-ylamino)-cyclohexylmethyl]-nicotinamide;
4-methyl-N-{cis-4-[(4-methylquinolin-2-yl)amino]cyclohexyl}benzamide;
2-(4-chlorophenoxy)-N-{cis-4-[(4-methylquinolin-2-yl)amino]cyclohexyl}acetamide;
3,4,5-trimethoxy-N-{cis-4-[(4-methylquinolin-2-yl)amino]cyclohexyl}benzamide;
2-(3,4-difluorophenyl)-N-{cis-4-[(4-methylquinolin-2-yl)amino]cyclohexyl}acetamide;
2-(2-bromo-4,5-dimethoxyphenyl)-N-{cis-4-[(4-methylquinolin-2-yl)amino]cyclohexyl}acetamide;
2,6-dimethoxy-N-{cis-4-[(4-methylquinolin-2-yl)amino]cyclohexyl}nicotinamide;
N-{cis-4-[(4-methylquinolin-2-yl)amino]cyclohexyl}-4-(trifluoromethoxy)benzamide;
5-chloro-N-[cis-4-(4-methyl-quinolin-2-ylamino)-cyclohexyl]-nicotinamide; and
5-fluoro-N-[cis-4-(4-methyl-quinolin-2-ylamino)-cyclohexyl]-nicotinamide;

or a pharmaceutically acceptable salt, hydrate, or solvate thereof.

**13.** The compound according to claim 12 selected from the group consisting of:

3-bromo-N-(cis-4-{[4-(dimethylamino)quinolin-2-yl]amino}cyclohexyl)-benzamide;
N-(cis-4-{[4-(dimethylamino)quinolin-2-yl]amino}cyclohexyl)-2,1,3-benzoxadiazole-5-carboxamide;
3-chloro-N-(cis-4-{[4-(dimethylamino)quinolin-2-yl]amino}cyclohexyl)-benzamide;
4-chloro-N-(cis-4-{[4-(dimethylamino)quinolin-2-yl]amino}cyclohexyl)-benzamide;
4-chloro-N-(cis-4-{[4-(dimethylamino)quinolin-2-yl]amino}cyclohexyl)-3-nitrobenzamide;
3,4-dichloro-N-(cis-4-{[4-(dimethylamino)quinolin-2-yl]amino}-cyclohexyl)benzamide;
N-(cis-4-{[4-(dimethylamino)quinolin-2-yl]amino}cyclohexyl)-3,4-difluorobenzamide;
N-(cis-4-{[4-(dimethylamino)quinolin-2-yl]amino}cyclohexyl)-4-fluorobenzamide;
N-(cis-4-{[4-(dimethylamino)quinolin-2-yl]amino}cyclohexyl)-3-nitrobenzamide;
N-(cis-4-{[4-(dimethylamino)quinolin-2-yl]amino}cyclohexyl)-2-phenoxybutanamide;
N-(cis-4-{[4-(dimethylamino)quinolin-2-yl]amino}cyclohexyl)-2-phenoxypropanamide;
N-(cis-4-{[4-(dimethylamino)quinolin-2-yl]amino}cyclohexyl)-3-methylbenzamide;
4-bromo-N-(cis-4-{[4-(dimethylamino)quinolin-2-yl]amino}cyclohexyl)-3-methylbenzamide;
N-(cis-4-{[4-(dimethylamino)quinolin-2-yl]amino}cyclohexyl)-2,5-dimethyl-3-furamide;
3-chloro-N-(cis-4-{[4-(dimethylamino)quinolin-2-yl]amino}cyclohexyl)-4-fluorobenzamide;
N-(cis-4-{[4-(dimethylamino)quinolin-2-yl]amino}cyclohexyl)-3,5-dimethoxybenzamide;
N-(cis-4-{[4-(dimethylamino)quinolin-2-yl]amino}cyclohexyl)-4-fluoro-3-methylbenzamide;
2-(4-chlorophenoxy)-N-(cis-4-{[4-(dimethylamino)quinolin-2-yl]amino}-cyclohexyl)acetamide;
3-(2-chlorophenyl)-N-(cis-4-{[4-(dimethylamino)quinolin-2-yl]amino}-cyclohexyl)-5-methylisoxazole-4-carboxamide;
3-(2-chloro-6-fluorophenyl)-N-(cis-4-{[4-(dimethylamino)quinolin-2-yl]amino}cyclohexyl)-5-methylisoxazole-4-carboxamide;
N-(cis-4-{[4-(dimethylamino)quinolin-2-yl]amino}cyclohexyl)-4-fluoro-3-(trifluoromethyl)benzamide;
N-(cis-4-{[4-(dimethylamino)quinolin-2-yl]amino}cyclohexyl)-2-(4-methoxyphenoxy)-5-nitrobenzamide;
N-(cis-4-{[4-(dimethylamino)quinolin-2-yl]amino}cyclohexyl)-5-nitro-2-furamide;
N-(cis-4-{[4-(dimethylamino)quinolin-2-yl]amino}cyclohexyl)-2-phenoxyacetamide;
2-(3-chlorophenoxy)-N-(cis-4-{[4-(dimethylamino)quinolin-2-yl]amino}-cyclohexyl)acetamide;
3-(2,6-dichlorophenyl)-N-(cis-4-{[4-(dimethylamino)quinolin-2-yl]amino}-cyclohexyl)-5-methylisoxazole-4-carboxamide;
N-(cis-4-{[4-(dimethylamino)quinolin-2-yl]amino}cyclohexyl)-2-phenoxynicotinamide;
N-(cis-4-{[4-(dimethylamino)quinolin-2-yl]amino}cyclohexyl)-2-(4-methylphenoxy)nicotinamide;
N-(cis-4-{[4-(dimethylamino)quinolin-2-yl]amino}cyclohexyl)-2-(2-thienyl)-1,3-thiazole-4-carboxamide;
N-(cis-4-{[4-(dimethylamino)quinolin-2-yl]amino}cyclohexyl)-2-(2,3,6-trichlorophenyl)acetamide;
N-(cis-4-{[4-(dimethylamino)quinolin-2-yl]amino}cyclohexyl)-5-iodo-2-furamide;
N-(cis-4-{[4-(dimethylamino)quinolin-2-yl]amino}cyclohexyl)-5-nitrothiophene-2-carboxamide;
N-(cis-4-{[4-(dimethylamino)quinolin-2-yl]amino}cyclohexyl)-3-methyl-4-nitrobenzamide;
N-(cis-4-{[4-(dimethylamino)quinolin-2-yl]amino}cyclohexyl)-3-methoxy-4-nitrobenzamide;
5-bromo-N-(cis-4-{[4-(dimethylamino)quinolin-2-yl]amino}cyclohexyl)-2-furamide;
4,5-dibromo-N-(cis-4-{[4-(dimethylamino)quinolin-2-yl]amino}-cyclohexyl)-2-furamide;
N-(cis-4-{[4-(dimethylamino)quinolin-2-yl]amino}cyclohexyl)-2-(1H-indol-3-yl)acetamide;

N-(cis-4-{[4-(dimethylamino)quinolin-2-yl]amino}cyclohexyl)-5-(3-nitrophenyl)-, 2-furamide;
N-(cis-4-{[4-(dimethylamino)quinolin-2-yl]amino}cyclohexyl)-5-nitrothiophene-3-carboxamide;
N-(cis-4-{[4-(dimethylamino)quinolin-2-yl]amino}cyclohexyl)-1-methyl-4-nitro-1H-pyrrole-2-carboxamide;
N-(cis-4-{[4-(dimethylamino)quinolin-2-yl]amino}cyclohexyl)-4-nitrobenzamide;
N-(cis-4-{[4-(dimethylamino)quinolin-2-yl]amino}cyclohexyl)-3-fluoro-4-(trifluoromethyl)benzamide;
3-bromo-N-[(cis-4-{[4-(dimethylamino)quinolin-2-yl]amino}cyclohexyl)-methyl]benzamide;
N-[(cis-4-{[4-(dimethylamino)quinolin-2-yl]amino}cyclohexyl)methyl]-2,1,3-benzoxadiazole-5-carboxanmide;
3-chloro-N-[(cis-{[4-(dimethylamino)quinolin-2-yl]amino}cyclohexyl)-methyl] benzamide;
4-chloro-N-[(cis-4-{[4-(dimethylamino)quinolin-2-yl]amino}cyclohexyl)-methyl]benzamide;
4-chloro-N-[(cis-4-{[4-(dimethylamino)quinolin-2-yl]amino}cyclohexyl)-methyl]-3-nitrobenzamide;
3,4-dichloro-N-[(cis-4-{[4-(dimethylamino)quinolin-2-yl]amino}-cyclohexyl)methyl]benzamide;
N-[(cis-4-{[4-(dimethylamino)quinolin-2-yl]amino}cyclohexyl)methyl]-3,4-difluorobenzamide;
N-[(cis-4-{[4-(dimethylamino)quinolin-2-yl] amino}cyclohexyl)methyl]-4-fluorobenzamide;
N-[(cis-4-{[4-(dimethylamino)quinolin-2-yl]amino}cyclohexyl)methyl]-3-nitrobenzamide;
N-[(cis-4-{[4-(dimethylamino)quinolin-2-yl]amino}cyclohexyl)methyl]-2-phenoxybutanamide;
N-[(cis-4-{[4-(dimethylamino)quinolin-2-yl]amino}cyclohexyl)methyl]-2-phenoxypropanamide;
N-[(cis-4-{[4-(dimethylamino)quinolin-2-yl]amino}cyclohexyl)methyl]-3-methylbenzamide;
4-bromo-N-[(cis-4-{[4-(dimethylamino)quinolin-2-yl]amino}cyclohexyl)-methyl]-3-methylbenzamide;
N-[(cis-4-{[4-(dimethylamino)quinolin-2-yl]amino}cyclohexyl)methyl]-2,5-dimethyl-3-furamide;
3-chloro-N-[(cis-4-{[4-(dimethylamino)quinolin-2-yl]amino}cyclohexyl)-methyl]-4-fluorobenzamide;
N-[(cis-4-{[4-(dimethylamino)quinolin-2-yl]amino}cyclohexyl)methyl]-3,5-dimethoxybenzamide;
N-[(cis-4-{[4-(dimethylamino)quinolin-2-yl]amino}cyclohexyl)methyl]-4-fluoro-3-methylbenzamide;
N-[(cis-4-{[4-(dimethylamino)quinolin-2-yl]amino}cyclohexyl)methyl]-2,4,6-trimethylbenzamide;
2,4,6-trichloro-N-[(cis-4-{[4-(dimethylamino)quinolin-2-yl]amino}-cyclohexyl)methyl]benzamide;
N-[(cis-4-{[4-(dimethylamino)quinolin-2-yl]amino}cyclohexyl)methyl]-2-(2,3,6-trichlorophenyl)acetamide;
N-[(cis-4-{[4-(dimethylamino)quinolin-2-yl]amino}cyclohexyl)methyl]-5-iodo-2-furamide;
N-[(cis-4-{[4-(dimethylamino)quinolin-2-yl]amino}cyclohexyl)methyl]-5-nitrothiophene-2-carboxamide;
N-[(cis-4-{[4-(dimethylamino)quinolin-2-yl]amino}cyclohexyl)methyl]-3-methyl-4-nitrobenzamide;
N-[(cis-4-{[4-(dimethylamino)quinolin-2-yl]amino}cyclohexyl)methyl]-3-methoxy-4-nitrobenzamide;
N-[cis-4-(4-methyl-quinolin-2-ylamino)-cyclohexyl]-2-phenoxy-nicotinamide;
3,4-difluoro-N-[cis-4-(4-methyl-quinolin-2-ylamino)-cyclohexyl]-benzamide;
3,4-difluoro-N-[cis-4-(quinolin-2-ylamino)-cyclohexyl]-benzamide;
2-phenoxy-N-[cis-4-(quinolin-2-ylamino)-cyclohexyl]-nicotinamide;
3-chloro-N-[cis-4-(4-methyl-quinolin-2-ylamino)-cyclohexyl]-benzamide;
3-methyl-N-[cis-4-(quinolin-2-ylamino)-cyclohexyl]-benzamide;
3-methoxy-N-[cis-4-(quinolin-2-ylamino)-cyclohexyl]-benzamide;
3-chloro-N-[cis-4-(quinolin-2-ylamino)-cyclohexyl]-benzamide;
5-nitro-thiophene-3-carboxylic acid [cis-4-(quinolin-2-ylamino)-cyclohexyl]-amide;
5-nitro-thiophene-3-carboxylic acid [cis-4-(4-methyl-quinolin-2-ylamino)-cyclohexyl]-amide;
3-chloro-4-fluoro-N-[cis-4-(quinolin-2-ylamino)-cyclohexyl]-benzamide;
3,5-dimethoxy-N-[cis-4-(quinolin-2-ylamino)-cyclohexyl]-benzamide;
3,4-dichloro-N-[cis-4-(quinolin-2-ylamino)-cyclohexyl]-benzamide;
benzo[2,3,1]oxadiazole-5-carboxylic acid [cis-4-(quinolin-2-ylamino)-cyclohexyl]-amide;
3-methyl-N-[cis-4-(4-methyl-quinolin-2-ylamino)-cyclohexyl]-benzamide;
3-methoxy-N-[cis-4-(4-methyl-quinolin-2-ylamino)-cyclohexyl]-benzamide;
4-cyano-N-[cis-4-(4-methyl-quinolin-2-ylamino)-cyclohexyl]-benzamide;
1-methyl-4 nitro-1H-pyrrole-2-carboxylic acid [cis-4-(quinolin-2-ylamino)-cyclohexyl]-amide;
9H-xanthene-9-carboxylic acid [cis-4-(quinolin-2-ylamino)-cyclohexyl]-amide;
3-nitro-N-[cis-4-(quinolin-2-ylamino)-cyclohexyl]-benzamide;
4-fluoro-3-methyl-N-[cis-4-(quinolin-2-ylamino)-cyclohexyl]-benzamide;
3-bromo-N-[cis-4-(quinolin-2-ylamino)-cyclohexyl]-benzamide;
2-(2-bromo-phenoxy)-N-[cis-4-(quinolin-2-ylamino)-cyclohexyl]-nicotinamide;
3-cyano-N-[cis-4-(quinolin-2-ylamino)-cyclohexyl]-benzamide;
N-[cis-4-(quinolin-2-ylamino)-cyclohexyl]-trifluoromethyl-benzamide;
N-[cis-4-(4-chloro-quinolin-2-ylamino)-cyclohexyl]-3,4-difluoro-benzamide;
3,4-dichloro-N-[cis-4-(4-methyl-quinolin-2-ylamino)-cyclohexyl]-benzamide;
3-chloro-4-fluoro-N-[cis-4-(4-methyl-quinolin-2-ylamino)-cyclohexyl]-benzamide;
4-fluoro-3-methyl-N-[cis-4-(4-methyl-quinolin-2-ylamino)-cyclohexyl]-benzamide;

1-methyl-4-nitro-1H-pyrrole-2-carboxylic acid [cis-4-(4-methyl-quinolin-2-ylamino)-cyclohexyl]-amide;
9H-xanthene-9-carboxylic acid [cis-4-(4-methyl-quinolin-2-ylamino)-cyclohexyl]-amide;
5-bromo-furan-2-carboxylic acid [cis-4-(4-methyl-quinolin-2-ylamino)-cyclohexyl]-amide;
N-[cis-4-(4-methyl-quinolin-2-ylamino)-cyclohexyl]-2-*m*-tolyloxy-acetamide;
N-[cis-4-(quinolin-2-ylamino)-cyclohexyl]-2-*m*-tolyloxy-acetamide;
2,2-diphenyl-N-[cis-4-(quinolin-2-ylamino)-cyclohexyl]-acetamide;
5-bromo-furan-2-carboxylic acid [cis-4-(quinolin-2-ylamino)-cyclohexyl]-amide;
benzo[2,3,1]oxadiazole-5-carboxylic acid [cis-4-(4-methyl-quinolin-2-ylamino)-cyclohexyl]-amide;
3-bromo-N-[cis-4-(4-methyl-quinolin-2-ylamino)-cyclohexyl]-benzamide;
3-cyano-N-[cis-4-(4-methyl-quinolin-2-ylamino)-cyclohexyl]-benzamide;
N-[cis-4-(4-methyl-quinolin-2-ylamino)-cyclohexyl]-3-trifluoromethyl-benzamide;
N-[cis-4-(4-methyl-quinolin-2-ylamino)-cyclohexyl]-2,2-diphenyl-acetamide;
2-(4-fluoro-phenoxy)-N-[cis-4-(quinolin-2-ylamino)-cyclohexyl]-nicotinamide;
2-(4-fluoro-phenoxy)-N-[cis-4-(4-methyl-quinolin-2-ylamino)-cyclohexyl]-nicotinamide;
2-(3,4-difluoro-phenoxy)-N-[cis-4-(quinolin-2-ylamino)-cyclohexyl]-nicotinamide;
2-(3,4-difluoro-phenoxy)-N-[cis-4-(4-methyl-quinolin-2-ylamino)-cyclohexyl]-nicotinamide;
N-[cis-4-(quinolin-2-ylamino)-cyclohexyl]-2 *p*-tolyloxy-nicotinamide;
N-[cis-4-(4-methyl-quinolin-2-ylamino)-cyclohexyl]-2 *p*-tolyloxy-nicotinamide;
2-(4-chloro-phenoxy)-N-[cis-4-(quinolin-2-ylamino)-cyclohexyl]-nicotinamide;
2-(4-chloro-phenoxy)-N-[cis-4-(4-methyl-quinolin-2-ylamino)-cyclohexyl]-nicotinamide;
2-(4 bromo-phenoxy)-N-[cis-4-(quinolin-2-ylamino)-cyclohexyl]-nicotinamide;
2-(4-bromo-phenoxy)-N-[cis-4-(4-methyl-quinolin-2-ylamino)-cyclohexyl]-nicotinamide;
2-(4-methoxy-phenoxy)-N-[cis-4-(quinolin-2-ylamino)-cyclohexyl]-nicotinamide;
2-(4-methoxy-phenoxy)-N-[cis-4-(4-methyl-quinolin-2-ylamino)-cyclohexyl]-nicotinamide;
2-(3-chloro-4-fluoro-phenoxy)-N-[cis-4-(4-methyl-quinolin-2-ylamino)-cyclohexyl]-nicotinamide;
N-[cis-4-(quinolin-2-ylamino)-cyclohexyl]-2-*m*-tolyloxy-nicotinamide;
N-[cis-4-(4-methyl-quinolin-2-ylamino)-cyclohexyl]-2-*m*-tolyloxy-nicotinamide;
2-(3-methoxy-phenoxy)-N-[cis-4-(4-methyl-quinolin-2-ylamino)-cyclohexyl]-acetamide;
2-(3-chloro-phenoxy)-N-[cis-4-(4-methyl-quinolin-2-ylamino)-cyclohexyl]-acetamide;
2-(3-chloro-4-fluoro-phenoxy)-N-[cis-4-(4-methyl-quinolin-2-ylamino)-cyclohexyl]-acetamide;
2-(3,4-dichloro-phenoxy)-N-[cis-4-(4-methyl-quinolin-2-ylamino)-cyclohexyl]-acetamide;
C-(methyl-phenyl-amino)-N-[cis-4-(4-methyl-quinolin-2-ylamino)-cyclohexyl]-acetamide;
2-(3-methoxy-phenoxy)-N-[cis-4-(quinolin-2-ylamino)-cyclohexyl]-acetamide;
2-(3-chloro-phenoxy)-N-[cis-4-(quinolin-2-ylamino)-cyclohexyl]-acetamide;
2-(3-chloro-4-fluoro-phenoxy)-N-[cis-4-(quinolin-2-ylamino)-cyclohexyl]-acetamide;
2-(3,4-dichloro-phenoxy)-N-[cis-4-(quinolin-2-ylamino)-cyclohexyl]-acetamide;
C-(methyl-phenyl-amino)-N-[cis-4-(quinolin-2-ylamino)-cyclohexyl]-acetamide;
N-[cis-4-(4-methyl-quinolin-2-ylamino)-cyclohexyl]-3-trifluoromethoxy-benzamide;
N-[cis-4-(4-amino-quinolin-2-ylamino)-cyclohexyl]-3,4-difluoro-benzamide;
C-(ethyl-phenyl-amino)-N-[cis-4-(quinolin-2-ylamino)-cyclohexyl]-acetamide;
C-(ethyl-phenyl-amino)-N-[cis-4-(4-methyl-quinolin-2-ylamino)-cyclohexyl]-acetamide;
3-hydroxy-N-[cis-4-(4-methyl-quinolin-2-ylamino)-cyclohexyl]-benzamide;
2,4-difluoro-N-[cis-4-(4-methyl-quinolin-2-ylamino)-cyclohexyl]-benzamide;
3,5-difluoro-N-[cis-4-(4-methyl-quinolin-2-ylamino)-cyclohexyl]-benzamide;
C-[(4-chloro-phenyl)-ethyl-amino]-N-[cis-4-(4-methyl-quinolin-2-ylamino)-cyclohexyl]-acetamide;
4-chloro-3-fluoro-N-[cis-4-(4-methyl-quinolin-2-ylamino)-cyclohexyl]-benzamide;
4-fluoro-N-[cis-4-(4-methyl-quinolin-2-ylamino)-cyclohexyl]-benzamide;
3-fluoro-N-[cis-4-(4-methyl-quinolin-2-ylamino)-cyclohexyl]-benzamide;
4-chloro-N-[cis-4-(4-methyl-quinolin-2-ylamino)-cyclohexyl]-benzamide;
N-[cis-4-(4-methyl-quinolin-2-ylamino)-cyclohexyl]-isophthalamic acid methyl ester;
3,5-difluoro-N-[cis-4-(quinolin-2-ylamino)-cyclohexyl]-benzamide;
4-chloro-3-fluoro-N-[cis-4-(quinolin-2-ylamino)-cyclohexyl]-benzamide;
C-[(4-chloro-phenyl)-ethyl-amino]-N-[cis-4-(quinolin-2-ylamino)-cyclohexyl]-acetamide;
6-chloro-N-[cis-4-(4-methyl-quinolin-2-ylamino)-cyclohexyl]-nicotinamide;
3-chloro-5-fluoro-N-[cis-4-(4-methyl-quinolin-2-ylamino)-cyclohexyl]-benzamide;
3,4,5-trifluoro-N-[cis-4-(4-methyl-quinolin-2-ylamino)-cyclohexyl]-benzamide;
5-bromo-N-[cis-4-(4-methyl-quinolin-2-ylamino)-cyclohexyl]-nicotinamide;
4-methyl-N-[cis-4-[(4-methylquinolin-2-yl)amino]cyclohexyl]benzamide;

2-(4-chlorophenoxy)-N-{cis-4-[(4-methylquinolin-2-yl)amino]cyclohexyl}-acetamide;
3,4,5-trimethoxy-N-{cis-4-[(4-methylquinolin-2-yl)amino]cyclohexyl}benzamide;
2-(3,4-difluorophenyl)-N-{cis-4-[(4-methylquinolin-2-yl)amino]cyclohexyl}-acetamide;
2-(2-bromo-4,5-dimethoxyphenyl)-N-{cis-4-[(4-methylquinolin-2-yl)amino]-cyclohexyl}acetamide;
2,6-dimethoxy-N-{cis-4-[(4-methylquinolin-2-yl)amino]cyclohexyl}nicotinamide;
N-{cis-4-[(4-methylquinolin-2-yl)amino]cyclohexyl}-4-(trifluoromethoxy)-benzamide;
5-chloro-N-[cis-4-(4-methyl-quinolin-2-ylamino)-cyclohexyl]-nicotinamide; and
5-fluoro-N-[cis-4-(4-methyl-quinolin-2-ylamino)-cyclohexyl]-nicotinamide;

or a pharmaceutically acceptable salt, hydrate, or solvate thereof.

**14.** The compound according to claim 3 wherein $R_1$ is selected from the group consisting of:

$C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:

  - •• halogen,
  - •• $C_{1-5}$ alkyl,
  - •• $C_{1-5}$ alkyl substituted by halogen,
  - •• $C_{1-5}$ alkoxy, and
  - •• $C_{1-5}$ alkoxy substituted by halogen,

L is Formula (XV);
Y is $-C(O)NR_5-$;
wherein carbocyclic aryl is phenyl; and
halogen is fluoro, chloro, or bromo;

or a pharmaceutically acceptable salt, hydrate, or solvate thereof.

**15.** The compound according to claim 14 wherein $R_1$ is selected from the group consisting of:

$C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:

  - •• halogen,
  - •• $C_{1-5}$ alkyl, and
  - •• $C_{1-5}$ alkyl substituted by halogen,

wherein carbocyclic aryl is phenyl; and
halogen is fluoro, chloro, or bromo;

or a pharmaceutically acceptable salt, hydrate, or solvate thereof.

**16.** The compound according to claim 14 or 15 wherein $R_2$ is methyl; p is 0; $R_3$ and $R_4$ are both hydrogen; A and B are both single bonds; and $R_5$ is hydrogen;
or a pharmaceutically acceptable salt, hydrate, or solvate thereof.

**17.** The compound according to claim 1 selected from the group consisting of:

cis-N-[(1R)-1-(4-bromophenyl)ethyl]-4-[(4-methylquinolin-2-yl)amino]cyclohexanecarboxamide;
cis-N-{(1S)-1-[3,5-bis(trifluoromethyl)phenyl]ethyl}-4-[(4-methylquinolin-2-yl)amino]cyclohexanecarboxamide;

cis-N-[(1R)-1-(2-fluorophenyl)ethyl]-4-[(4-methylquinolin-2-yl)amino]cyclohexanecarboxamide;
cis-N-[(1S)-1-(2-fluorophenyl)ethyl)-4-[(4-methylquinolin-2-yl)amino]cyclohexanecarboxamide;
cis-4-[(4-methylquinolin-2-yl)amino]-N-{(1S)-1-[2-(trifluoromethyl)phenyl]ethyl}cyclohexanecarboxamide;
cis-4-[(4-methylquinolin-2-yl)amino]-N-{(1S)-1-[3-(trifluoromethyl)phenyl]ethyl}cyclohexanecarboxamide;
cis-N-[(1R)-1-(4-chlorophenyl)ethyl]-4-[(4-methylquinolin-2-yl)amino]cyclohexanecarboxamide; and
cis-N-[(1S)-1-(4-chlorophenyl)ethyl]-4-[(4-methylquinolin-2-yl)amino]cyclohexanecarboxamide;
or a pharmaceutically acceptable salt, hydrate, or solvate thereof.

**18.** The compound according to claim 1 selected from the group consisting of:

cis-N-[(1R)-1-(4-bromophenyl)ethyl]-4-[(4-methylquinolin-2-yl)amino]cyclohexanecarboxamide;
cis-N-[(1S)-1-(2-fluorophenyl)ethyl]-4-[(4-methylquinolin-2-yl)amino]cyclohexanecarboxamide;
cis-4-[(4-methylquinolin-2-yl)amino]-N-{(1S)-1-[2-(trifluoromethyl)phenyl]ethyl}cyclohexanecarboxamide; and
cis-4-[(4-methylquinolin-2-yl)amino]-N-{(1S)-1-[3-(trifluoromethyl)phenyl]ethyl}cyclohexanecarboxamide;
or a pharmaceutically acceptable salt, hydrate, or solvate thereof.

**19.** The compound according to claim 3 wherein $R_1$ is selected from the group consisting of:

(i) $C_{1-5}$ alkyl, and
$C_{1-5}$ alkyl substituted by substituent(s) independently selected from the group consisting of:

- $C_{1-5}$ alkoxycarbonyl,
- $C_{1-5}$ alkylthio,
- carbocyclic aryl,
- carbocyclic aryl substituted by substituent(s) independently selected from the group consisting of:

  - •• halogen,
  - •• $C_{1-5}$ alkyl, and
  - •• $C_{2-5}$ alkenyl,

(ii) $C_{3-6}$ cycloalkyl, and
$C_{3-6}$ cycloalkyl substituted by carbocyclic aryl,
(iii) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by halogen,
- $C_{1-5}$ alkoxycarbonyl,
- $C_{1-5}$ alkoxy,
- $C_{1-5}$ alkoxy substituted by carbocyclic aryl,
- $C_{3-6}$ cycloalkoxy,
- carbocyclic aryloxy,
- $C_{1-5}$ alkylthio, and
- carbocyclic aryl,

(iv) heterocyclyl, and
heterocyclyl substituted by substituent(s) independently selected from the group consisting of:

- $C_{1-5}$ alkyl,
- $C_{1-5}$ alkyl substituted by halogen, and
- carbocyclic aryl;

L is Formula (VII);
Y is $-C(O)NR_5-$;

wherein carbocyclic aryl is phenyl or naphthyl;

heterocyclyl is 2,3-dihydro-benzo[1,4]dioxinyl, 3,4-dihydro-2*H*-benzo[b][1,4]dioxepinyl, benzo[1,3]dioxolyl, furyl, or isoxazolyl; and

halogen is fluoro, chloro, bromo, or iodo;

or a pharmaceutically acceptable salt, hydrate, or solvate thereof.

**20.** The compound according to claim 19 wherein $R_2$ is hydrogen, methyl, methylamino, or dimethylamino; p is 0; $R_3$ and $R_4$ are hydrogen; A is a single bond; B is a single bond or $-CH_2-$; $R_5$ is hydrogen; or a pharmaceutically acceptable salt, hydrate, or solvate thereof.

**21.** The compound according to claim 20 wherein $R_1$ is selected from the group consisting of:

(i) $C_{1-5}$ alkyl, and

$C_{1-5}$ alkyl substituted by substituent(s) independently selected from the group consisting of:

- $C_{1-5}$ alkoxycarbonyl,
- carbocyclic aryl, and
- carbocyclic aryl substituted by halogen,

(ii) carbocyclic aryl, and

carbocyclic aryl substituted by substituent(s) independently selected from the group consisting of:

- halogen,
- nitro,
- $C_{1-5}$ alkyl,
- $C_{1-5}$ alkyl substituted by halogen, and
- $C_{1-5}$ alkoxy,

(iii) heterocyclyl,

heterocyclyl substituted by $C_{1-5}$ alkyl, and

heterocyclyl substituted by carbocyclic aryl; wherein carbocyclic aryl is phenyl or naphthyl;

heterocyclyl is isoxazolyl; and

halogen is fluoro, chloro, bromo, or iodo;

or a pharmaceutically acceptable salt, hydrate, or solvate thereof.

**22.** The compound according to claim I selected from the group consisting of:

N-(2-chlorophenyl)-N'-(cis-4-{[4-(dimethylamino)quinolin-2-yl]amino}-cyclohexyl)urea;
N-(cis-4-{[4-(dimethylamino)quinolin-2-yl]amino}cyclohexyl)-N'-(2-ethyl-6-methylphenyl)urea;
N-(cis-4-{[4-(dimethylamino)quinolin-2-yl]amino}cyclohexyl)-N'-mesitylurea;
N-(cis-4-{[4-(dimethylamino)quinolin-2-yl]amino}cyclohexyl)-N'-(2,4,6-trichlorophenyl)urea;
N-(cis-4-{[4-(dimethylamino)quinolin-2-yl]amino}cyclohexyl)-N'-(2,4,6-tribromophenyl)urea;
N-(2,4-dibromo-6-fluorophenyl)-N'-(cis-4-{[4-(dimethylamino)quinolin-2-yl]amino}cyclohexyl)urea;
N-(2,6-diethylphenyl)-N'-(cis-4-{[4-(dimethylamino)quinolin-2-yl]amino}-cyclohexyl)urea;
N-(2-chlorobenzyl)-N'-(cis-4-{[4-(dimethylamino)quinolin-2-yl]amino}-cyclohexyl)urea;
N-(cis-4-{[4-(dimethylamino)quinolin-2-yl]amino}cyclohexyl)-N'-(2-ethyl-6-isopropylphenyl)urea;
N-(cis-4-{[4-(dimethylamino)quinolin-2-yl]amino}cyclohexyl)-N'-(2-isopropyl-6-methylphenyl)urea;
N-(2-tert-butyl-6-methylphenyl)-N'-(cis-4-{[4-(dimethylamino)quinolin-2-yl]amino}cyclohexyl)urea;
N-(cis-4-{[4-(dimethylamino)quinolin-2-yl]amino}cyclohexyl)-N'-(diphenylmethyl)urea;
N-(4-bromo-2,6-dimethylphenyl)-N'-(cis-4-{[4-(dimethylamino)quinolin-2-yl]amino}cyclohexyl)urea;
N-(cis-4-{[4-(dimethylamino)quinolin-2-yl]amino}cyclohexyl)-N'-(3-methyl-5-phenylisoxazol-4-yl)urea;
N-(cis-4-{[4-(dimethylamino)quinolin-2-yl]amino}cyclohexyl)-N'-1-naphthylurea;
N-(cis-4-{[4-(dimethylamino)quinolin-2-yl]amino}cyclohexyl)-N'-[1-(1-naphthyl)ethyl]urea;
methyl N-{[(cis-4-{[4-(dimethylamino)quinolin-2-yl]amino}cyclohexyl)-amino]carbonyl}phenylalaninate;
N-(cis-4-{[4-(dimethylamino)quinolin-2-yl]amino}cyclohexyl)-N'-(3,4,5-trimethoxyphenyl)urea;
N-(5-chloro-2,4-dimethoxyphenyl)-N'-(cis-4-{[4-(dimethylamino)quinolin-2-yl]amino}cyclohexyl)urea;

N-(4-bromo-2-methylphenyl)-N'-(cis-4-{[4-(dimethylamino)quinolin-2-yl]amino}cyclohexyl)urea;
N-[(cis-4-{[4-(dimethylamino)quinolin-2-yl]amino}cyclohexyl)methyl]-N'-(2-ethyl-6-methylphenyl)urea;
N-[(cis-4-{[4-(dimethylamino)quinolin-2-yl]amino}cyclohexyl)methyl]-N'-mesitylurea;
N-[(cis-4-{[4-(dimethylamino)quinolin-2-yl]amino}cyclohexyl)methyl]-N'-(2,4,6-trichlorophenyl)urea;
N-[(cis-4-{[4-(dimethylamino)quinolin-2-yl]amino}cyclohexyl)methyl]-N'-(2,4,6-tribromophenyl)urea;
N-(2,4-dibromo-6-fluorophenyl)-N'-[(cis-4-{[4-(dimethylamino)quinolin-2-yl]amino}cyclohexyl)methyl]urea;
N-(2,6-diethylphenyl)-N'-[(cis-4-{[4-(dimethylamino)quinolin-2-yl]amino}-cyclohexyl)methyl]urea;
N-[2-chloro-6-(trifluoromethyl)phenyl]-N'-[(cis-4-{[4-(dimethylamino)-quinolin-2-yl]amino}cyclohexyl)methyl] urea;
N-[(cis-4-{[4-(dimethylamino)quinolin-2-yl]amino}cyclohexyl)methyl]-N'-(2-ethyl-6-isopropylphenyl)urea;
N-[(cis-4-{[4-(dimethylamino)quinolin-2-yl]amino}cyclohexyl)methyl]-N'-(2-isopropyl-6-methylphenyl)urea;
N-[(cis-4-{[4-(dimethylamino)quinolin-2-yl]amino}cyclohexyl)methyl]-N'-(2-methyl-3-nitrophenyl)urea;
N-(2-tert-butyl-6-methylphenyl)-N'-[(cis-4-{[4-(dimethylamino}quinolin-2-yl]amino cyclohexyl)methyl]urea;
N-(2-tert-butylphenyl)-N'-[(cis-4-{[4-(dimethylamino)quinolin-2-yl]-amino}cyclohexyl)methyl]urea;
N-[(cis-4-{[4-(dimethylamino)quinolin-2-yl]amino}cyclohexyl)methyl]-N'-(diphenylmethyl)urea;
N-(4-bromo-2,6-dimethylphenyl)-N'-[(cis-4-{[4-(dimethylamino)quinolin-2-yl]amino}cyclohexyl)methyl]urea;
N-(2,3-dichlorophenyl)-N'-[(cis-4-{[4-(dimethylamino)quinolin-2-yl]-amino}cyclohexyl)methyl]urea;
N-(2,6-diisopropylphenyl)-N'-[(cis-4-{[4-(dimethylamino)quinolin-2-yl]-amino}cyclohexyl)methyl]urea;
1-(2,3-dichloro-phenyl)-3-[cis-4-(4-methyl-quinolin-2-ylamino)-cyclohexyl]-urea; and
1-(2,3-dichloro-phenyl)-3-[cis-4-(4-methyl-quinolin-2-ylamino)-cyclohexylmethyl]-urea;

or a pharmaceutically acceptable salt, hydrate, or solvate thereof.

**23.** The compound according to claim 3 wherein $R_1$ is selected from the group consisting of:

(i) $C_{1-5}$ alkyl, and
$C_{1-5}$ alkyl substituted by substituent(s) independently selected from the group consisting of:

- carbocyclic aryl,
- carbocyclic aryl substituted by substituent(s) independently selected from the group consisting of:
  - •• halogen, and
  - •• $C_{1-5}$ alkoxy,

(ii) carbocyclyl,
(iii) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by halogen,
- $C_{1-5}$ alkoxy carbonyl,
- $C_{1-5}$ alkoxy,
- $C_{1-5}$ alkoxy substituted by halogen,
- mono-$C_{1-5}$ alkylamino,
- di-$C_{1-5}$ alkylamino, and
- carbocyclic aryl,

(iv) heterocyclyl, and
heterocyclyl substituted by substituent(s) independently selected from the group consisting of:

- $C_{1-5}$ alkyl,
- $C_{1-5}$ alkoxy carbonyl, and
- carbocyclic aryl;

L is Formula (VII);

Y is -C(S)$NR_5$-;
wherein carbocyclic aryl is phenyl or naphthyl;
carbocyclyl is bicyclo[2.2.1]heptyl;
heterocyclyl is 2,3-dihydro-benzo[1,4]dioxinyl, benzo[1,3]dioxolyl, isoxazolyl, or thienyl; and
halogen is fluoro, chloro, bromo, or iodo;

or a pharmaceutically acceptable salt, hydrate, or solvate thereof.

**24.** The compound according to claim 23 wherein $R_2$ is methylamino or dimethylamino; p is 0; $R_3$ and $R_4$ are hydrogen; A is a single bond; B is a single bond or -$CH_2$-; $R_5$ is hydrogen;
or a pharmaceutically acceptable salt, hydrate, or solvate thereof.

**25.** The compound according to claim 24 wherein $R_1$ is selected from the group consisting of:

(i) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen,
- $C_{1-5}$ alkoxy,
- mono-$C_{1-5}$ alkylamino, and
- di-$C_{1-5}$ alkylamino,

(ii) heterocyclyl, and
heterocyclyl substituted by $C_{1-5}$ alkyl, and
heterocyclyl substituted by $C_{1-5}$ alkoxy carbonyl;
wherein carbocyclic aryl is phenyl or naphthyl;
heterocyclyl is thienyl; and
halogen is fluoro, chloro, bromo, or iodo;

or a pharmaceutically acceptable salt, hydrate, or solvate thereof.

**26.** The compound according to claim 1 selected from the group consisting of:

N-(2,4-dimethoxyphenyl)-N'-(cis-4-{[4-(dimethylamino)quinolin-2-yl]-amino}cyclohexyl)thiourea;
N-(cis-4-{[4-(dimethylamino)quinolin-2-yl]amino}cyclohexyl)-N'-(3,4,5-trimethoxyphenyl)thiourea;
N-[4-(dimethylamino)-1-naphthyl]-N'-(cis-4-{[4-(dimethylamino)quinolin-2-yl]amino}cyclohexyl)thiourea;
N-(cis-4-{[4-(dimethylamino)quinolin-2-yl]amino}cyclohexyl)-N'-(2,4,6-tribromophenyl)thiourea;
N-(cis-4-{[4-(dimethylamino)quinolin-2-yl]amino}cyclohexyl)-N'-(2,4,6-trichlorophenyl)thiourea;
N-(cis-4-{[4-(dimethylamino)quinolin-2-yl]amino}cyclohexyl)-N'-mesitylthiourea;
N-(2,6-diethylphenyl)-N'-(cis-4-{[4-(dimethylamino)quinolin-2-yl]amino}-cyclohexyl)thiourea;
N-(4-bromo-2,6-dimethylphenyl)-N'-(cis-4-{[4-(dimethylamino)quinolin-2-yl]amino}cyclohexyl)thiourea;
N-(4-bromo-2-methylphenyl)-N'-(cis-4-{[4-(dimethylamino)quinolin-2-yl]amino}cyclohexyl)thiourea;
N-[4-bromo-2-(trifluoromethyl)phenyl]-N'-(cis-4-{[4-(dimethylamino)-quinolin-2-yl]amino}cyclohexyl)thiourea;
N-(5-chloro-2,4-dimethoxyphenyl)-N'-(cis-4-{[4-(dimethylamino)quinolin-2-yl]amino}cyclohexyl)thiourea;
N-(2,4-dibromo-6-fluorophenyl)-N'-(cis-4-{[4-(dimethylamino )quinolin-2-yl]amino}cyclohexyl)thiourea;
N-(2,4-dichloro-6-methylphenyl)-N'-(cis-4-{[4-(dimethyl amino)quinolin-2-yl]amino}cyclohexyl)thiourea; and
methyl 3-({[(cis-4-{[4-(dimethylamino)quinolin-2-yl]amino}cyclohexyl)-amino]carbonothioyl}amino)-4-methylthiophene-2-carboxylate;

or a pharmaceutically acceptable salt, hydrate, or solvate thereof.

**27.** The compound according to claim 3 wherein $R_1$ is selected from the group consisting of:

(i) $C_{1-8}$ alkyl, and
$C_{1-8}$ alkyl substituted by substituent(s) independently selected from the group consisting of:

- halogen,
- $C_{1-5}$ alkoxy,
- $C_{1-5}$ alkoxy substituted by carbocyclic aryl,
- carbocyclyl,
- carbocyclic aryl,
- carbocyclic aryl substituted by substituent(s) independently selected from the group consisting of:

  - •• halogen,
  - •• nitro, and
  - •• $C_{1-5}$ alkoxy,

(ii) $C_{2-5}$ alkenyl,
(iii) carbocyclyl,
(iv) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen, and
- $C_{1-5}$ alkoxy;

L is Formula (VII);
Y is -C(O)O-;
wherein carbocyclic aryl is phenyl or naphthyl;
carbocyclyl is 9*H*-fluorenyl or menthyl; and
halogen is fluoro, chloro, bromo, or iodo;

or a pharmaceutically acceptable salt, hydrate, or solvate thereof.

**28.** The compound according to claim 27 wherein $R_2$ is methylamino or dimethylamino; p is 0; $R_3$ and $R_4$ are hydrogen; A is a single bond; B is a single bond or $-CH_2-$;
or a pharmaceutically acceptable salt, hydrate, or solvate thereof.

**29.** The compound according to claim 2 wherein Q is Formula (III);
$R_1$ is selected from the group consisting of:

(i) $C_{1-8}$ alkyl, and
$C_{1-8}$ alkyl substituted by substituent(s) independently selected from the group consisting of:

- halogen,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- heterocyclyloxy,
- heterocyclyloxy substituted by $C_{1-5}$ alkyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by cyano,
- mono-$C_{1-5}$ alkylamino substituted by carbocyclic aryl,
- di-$C_{1-5}$ alkylamino,
- di-$C_{1-5}$ alkylamino substituted by cyano,
- di-$C_{1-5}$ alkylamino substituted by carbocyclic aryl,

- mono-carbocyclic arylamino,
- mono-carbocyclic arylamino substituted by $C_{1-5}$ alkyl,
- di-carbocyclic arylamino,
- di-carbocyclic arylamino substituted by $C_{1-5}$ alkyl,
- carbocyclic arylsulfonylamino,
- carbocyclic arylsulfonylamino substituted $C_{1-5}$ alkyl,
- $C_{1-5}$ alkylthio,
- $C_{1-5}$ alkylthio substituted by substituent(s) independently selected from the group consisting of:
  - •• carbocyclic aryl,
  - •• carbocyclic aryl substituted by halogen, and
  - •• carbocyclic aryl substituted by $C_{1-5}$ alkoxy,
- carbocyclic arylthio,
- heterocyclylthio,
- heterocyclylthio substituted by $C_{1-5}$ alkyl,
- $C_{3-6}$ cycloalkyl,
- $C_{3-6}$ cycloalkenyl,
- carbocyclyl,
- carbocyclyl substituted by substituent(s) independently selected from the group consisting of:
  - •• halogen,
  - •• $C_{1-5}$ alkyl,
  - •• $C_{1-5}$ alkoxy,
  - •• $C_{1-5}$ alkenyl, and
  - •• $C_{2-5}$ alkenyl substituted by substituent(s) independently selected from the group consisting of:
    - ••• carbocyclic aryl, and
    - ••• carbocyclic aryl substituted by $C_{1-5}$ alkylsulfinyl,
- carbocyclic aryl,
- carbocyclic aryl substituted by substituent(s) independently selected from the group consisting of:
  - •• halogen,
  - •• hydroxy,
  - •• nitro,
  - •• $C_{1-5}$ alkyl,
  - •• $C_{1-5}$ alkyl substituted by substituent(s) independently selected from the group consisting of:
    - ••• oxo,
    - ••• carbocyclic aryl, and
    - ••• heterocyclyl,
  - •• $C_{2-5}$ alkenyl,
  - •• $C_{1-5}$ alkoxy,
  - •• $C_{1-5}$ alkoxy substituted by halogen,
  - •• $C_{1-5}$ alkoxy substituted by carbocyclic aryl,
  - •• carbocyclic aryloxy,
  - •• mono-carbocyclic arylaminocarbonyl,
  - •• mono-carbocyclic arylaminocarbonyl substituted by halogen,
  - •• di-carbocyclic arylaminocarbonyl,
  - •• di-carbocyclic arylaminocarbonyl substituted by halogen,
  - •• carbocyclic aryl, and
  - •• heterocyclyl,
- heterocyclyl, and
- heterocyclyl substituted by substituent(s) independently selected from the group consisting of:

- - $C_{1-5}$ alkyl,
  - $C_{1-5}$ alkoxy,
  - $C_{1-5}$ alkoxy substituted by carbocyclic aryl,
  - carbocyclic aryl, and
  - carbocyclic aryl substituted by halogen,

(ii) $C_{2-7}$ alkenyl, and
$C_{2-7}$ alkenyl substituted by substituent(s) independently selected from the group consisting of:

- carbocyclic aryl,
- carbocyclic aryl substituted by substituent(s) independently selected from the group consisting of:
  - halogen,
  - nitro, and
  - $C_{1-5}$ alkoxy,

(iii) $C_{2-5}$ alkynyl,
(iv) $C_{3-12}$ cycloalkyl, and
$C_{3-12}$ cycloalkyl substituted by substituent(s) independently selected from the group consisting of:

- $C_{1-5}$ alkyl,
- $C_{1-5}$ alkyl substituted by oxo,
- $C_{1-5}$ alkyl substituted by carbocyclic aryl, and
- carbocyclic aryl,

(v) carbocyclyl,
(vi) carbocyclic aryl, and
carbocyclic aryl substituted by substituent(s) independently selected from the group consisting of:

- halogen,
- hydroxy,
- cyano,
- nitro,
- $C_{1-10}$ alkyl,
- $C_{1-10}$ alkyl substituted by substituent(s) independently selected from the group consisting of:
  - halogen,
  - oxo,
  - carbocyclic aryloxy,
  - carbocyclic aryl, and
  - carbocyclic aryl substituted by $C_{1-5}$ alkyl,
- $C_{1-7}$ alkoxy,
- $C_{1-7}$ alkoxy substituted by substituent(s) independently selected from the group consisting of:
  - halogen,
  - carbocyelic aryl, and
  - halogenated carbocyclic aryl,
- $C_{2-5}$ alkenyloxy,
- $C_{3-6}$ cycloalkoxy,
- carbocyclic aryloxy,
- carbocyclic aryloxy substituted by nitro,
- carbocyclic aryloxy substituted by $C_{1-5}$ alkoxy,
- carboxy,
- $C_{1-5}$ alkoxycarbonyl,
- mono-$C_{1-5}$ alkylaminocarbonyl,
- di-$C_{1-5}$ alkylaminocarbonyl,

- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{2-5}$ alkynylcarbonylamino,
- $C_{2-5}$ alkynylcarbonylamino substituted by carbocyclic aryl,
- $C_{1-5}$ alkoxycarbonylamino,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- arbocyclic aryl azo,
- carbocyclic aryl azo substituted by mono-$C_{1-5}$ alkylamino,
- carbocyclic aryl azo substituted by di-$C_{1-5}$ alkylamino,
- $C_{1-5}$ alkylthio,
- $C_{1-5}$ alkylthio substituted by halogen,
- carbocyclic arylthio,
- carbocyclic arylthio substituted by nitro,
- carbocyclic arylthio substituted by cyano,
- aminosulfonyl,
- mono-$C_{1-5}$ alkylaminosulfonyl,
- di-$C_{1-5}$ alkylaminosulfonyl,
- heterocyclylsulfonyl,
- $C_{3-6}$ cycloalkyl,
- $C_{3-6}$ cycloalkyl substituted by $C_{1-5}$ alkyl,
- carbocyclic aryl,
- heterocyclyl, and
- heterocyclyl substituted by substituent(s) independently selected from the group consisting of:
    - •• $C_{1-5}$ alkyl,
    - •• carbocyclic aryl, and
    - •• halogenated carbocyclic aryl,

(vii) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:
    - •• halogen,
    - •• hydroxy,
    - •• $C_{1-5}$ alkylthio,
    - •• $C_{1-5}$ alkylthio substituted by carbocyclic aryl,
    - •• $C_{1-5}$ alkylthio substituted by halogenated carbocyclic aryl,
    - •• carbocyclic aryl,
    - •• carbocyclic aryl substituted by halogen, and
    - •• heterocyclyl,
- $C_{1-5}$ alkoxy,
- carbocyclic aryloxy,
- carbocyclic aryloxy substituted by $C_{1-5}$ alkyl,
- $C_{1-5}$ alkylthio,
- $C_{2-5}$ alkenylthio,
- carbocyclic arylthio,

- Carbocyclic arylthio substituted by $C_{1-5}$ alkoxycarbonyl,
- $C_{1-5}$ alkylsulfonyl,
- carbocyclic arylsulfonyl,
- carbocyclic arylsulfonyl substituted by $C_{1-5}$ alkyl,
- $C_{1-5}$ alkoxycarbonyl,
- $C_{1-5}$ alkoxycarbonyl substituted by carbocyclic aryl,
- carbocyclic aryl,
- carbocyclic aryl substituted by substituent(s) independently selected from the group consisting of:

    - •• halogen,
    - •• nitro,
    - •• $C_{1-5}$ alkyl, and
    - •• $C_{1-5}$ alkyl substituted by halogen,

- heterocyclyl;

wherein carbocyclic aryl is phenyl, naphthyl, or anthranyl;

carbocyclyl is 1,2,3,4-tetrahydronaphthyl, 1-oxo-indanyl, 9-fluorenyl, 9*H*-fluorenyl, 9-oxo-9*H*-fluorenyl, adamantly, bicyclo[2.2.1]heptenyl, bicyclo[2.2.1]heptyl, indanyl, indenyl, or menthyl;

heterocyclyl is 1,2,3-triazolyl, 1*H*-indolyl, 1*H*-pyrrolyl, 2,3-dihydro-1-oxo-isoindolyl, 2,3-dihydro-benzo[1,4]dioxinyl, 2,4-dihydro-3-oxo-pyrazolyl, 2*H*-benzopyranyl, 2-oxo-benzopyranyl, 3,4-dihydro-2*H*-benzo[b][1,4]dioxepinyl, 4,5,6,7-tetrahydro-benzo[b]thienyl, 4*H*-benzo[1,3]dioxinyl, 4-oxo-1,5,6,7-tetrahydro-indolyl, 4-oxo-benzopyranyl, 9*H*-carbazolyl, 9*H*-xanthenyl, azetidinyl, benzo[1,3]dioxolyl, benzo[2,1,3]oxadiazolyl, benzo[1,2,5]oxadiazolyl, benzo[2,1,3]thiadiazolyl, benzo[b]thienyl, benzofuryl, benzothiazolyl, furyl, imidazo[2,1-b]thiazolyl, isoxazolyl, morpholino, morpholinyl, oxazolyl, phenanthro[9,10-d]oxazolyl, piperidyl, pyrazolyl, pyridyl, pyrimidyl, quinolyl, quinoxalyl, tetrahydrofuryl, thiazoiyl, or thienyl; and

halogen is fluoro, chloro, bromo, or iodo;

or a pharmaceutically acceptable salt, hydrate, or solvate thereof.

**30.** The compound according to claim 29 wherein $R_1$ is selected from the group consisting of:

(i) $C_{1-7}$ alkyl, and

$C_{1-7}$ alkyl substituted by substituent(s) independently selected from the group consisting of:

- $C_{1-5}$ alkoxy,
- $C_{1-5}$ alkoxy substituted by carbocyclic aryl,
- carbocyclic aryloxy,
- mono-$C_{1-5}$ alkylamino,
- mono-$C_{1-5}$ alkylamino substituted by substituent(s) independently selected from the group consisting of:

    - •• cyano, and
    - •• carbocyclic aryl,

- di-$C_{1-5}$ alkylamino,
- di-$C_{1-5}$ alkylamino substituted by substituent(s) independently selected from the group consisting of:

    - •• cyano, and
    - •• carbocyclic aryl,

- mono-carbocyclic arylamino,
- di-carbocyclic arylamino,
- mono-carbocyclic arylamino substituted by $C_{1-5}$ alkyl,
- di-carbocyclic arylamino substituted by $C_{1-5}$ alkyl,
- carbocyclic arylsulfonylamino,
- carbocyclic arylsulfonylamino substituted by $C_{1-5}$ alkyl,
- carbocyclic aryl,
- carbocyclic aryl substituted by substituent(s) independently selected from the group consisting of:

- •• halogen,
- •• nitro,
- •• $C_{1-5}$ alkyl,
- •• $C_{1-5}$ alkyl substituted by substituent(s) independently selected from the group consisting of:
  - ••• oxo, and
  - ••• carbocyclic aryl,
- •• $C_{1-5}$ alkoxy,

- heterocyclyl, and
- heterocyclyl substituted by carbocyclic aryl,

(ii) $C_{2-7}$ alkenyl, and
$C_{2-7}$ alkenyl substituted by substituent(s) independently selected from the group consisting of:

- carbocyclic aryl, and
- carbocyclic aryl substituted by $C_{1-5}$ alkoxy,

(iii) $C_{3-6}$ cycloalkyl, and
$C_{3-6}$ cycloalkyl substituted by substituent(s) independently selected from the group consisting of:

- $C_{1-5}$ alkyl, and
- $C_{1-5}$ alkyl substituted by carbocyclic aryl,

(iv) carbocyclic aryl, and
carbocyclic aryl substituted by substituent(s) independently selected from the group consisting of:

- halogen,
- hydroxy,
- cyano,
- $C_{1-5}$ alkyl,
- $C_{1-5}$ alkyl substituted by halogen,
- $C_{1-5}$ alkoxy,
- $C_{1-5}$ alkoxy substituted by substituent(s) independently selected from the group consisting of:
  - •• halogen, and
  - •• carbocyclic aryl,
  - •• carbocyclic aryl substituted by halogen,
- $C_{2-5}$ alkenyloxy,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{1-5}$ alkylthio, and
- $C_{1-5}$ alkylthio substituted by halogen,

(v) heterocyclyl, and
heterocyclyl substituted by substituent(s) independently selected from the group consisting of:

- halogen,
- $C_{1-5}$ alkyl,
- $C_{1-5}$ alkyl substituted by substituent(s) independently selected from the group consisting of:
  - •• hydroxy,and
  - •• carbocyclic aryl,

- $C_{1-5}$ alkoxy,
- carbocyclic arylthio,
- carbocyclic arylthio substituted by $C_{1-5}$ alkoxycarbonyl,
- $C_{1-5}$ alkoxycarbonyl,
- carbocyclic aryl,
- carbocyclic aryl substituted by substituent(s) independently selected from the group consisting of:

  •• halogen,
  •• $C_{1-5}$ alkyl, and
  •• $C_{1-5}$ alkyl substituted by halogen;

L is Formula (VII);
Y is a single bond or $-CH_2-$;
wherein carbocyclic aryl is phenyl or naphthyl;
heterocyclyl is 1*H*-indolyl, 1*H*-pyrrolyl, 2,3-dihydro-benzo[1,4]dioxinyl, 4-oxo-benzopyranyl, 9*H*-carbazolyl, azetidinyl, benzo[1,3]dioxolyl, benzo[b]thienyl, furyl, imidazo[2,1-b]thiazolyl, pyrazolyl, pyridyl, or thienyl; and
halogen is fluoro, chloro, bromo, or iodo;

or a pharmaceutically acceptable salt, hydrate, or solvate thereof.

**31.** The compound according to claim 30 wherein $R_2$ is methylamino or dimethylamino; p is 0; $R_3$ and $R_4$ are hydrogen; A is a single bond; B is a single bond or $-CH_2-$;
or a pharmaceutically acceptable salt, hydrate, or solvate thereof.

**32.** The compound according to claim 31 wherein $R_1$ is selected from the group consisting of:

(i) $C_{1-5}$ alkyl, and
$C_{1-5}$ alkyl substituted by substituent(s) independently selected from the group consisting of:

- mono-$C_{1-5}$ alkylamino,
- mono-$C_{1-5}$ alkylamino substituted by cyano,
- di-$C_{1-5}$ alkylamino,
- di-$C_{1-5}$ alkylamino substituted by cyano,
- mono-carbocyclic arylamino,
- di-carbocyclic arylamino,
- mono-carbocyclic arylamino substituted by $C_{1-5}$ alkyl,
- di-carbocyclic arylamino substituted by $C_{1-5}$ alkyl,
- carbocyclic arylsulfonylamino,
- carbocyclic arylsulfonylamino substituted by $C_{1-5}$ alkyl,
- carbocyclic aryl, and
- carbocyclic aryl substituted by $C_{1-5}$ alkoxy,

(ii) $C_{2-5}$ alkenyl, and
$C_{2-5}$ alkenyl substituted by carbocyclic aryl,
(iii) carbocyclic aryl, and
carbocyclic aryl substituted by substituent(s) independently selected from the group consisting of:

- halogen,
- hydroxy,
- $C_{1-5}$ alkyl,
- $C_{1-5}$ alkoxy,
- $C_{1-5}$ alkoxy substituted by halogen,
- mono-$C_{1-5}$ alkylamino, and
- di-$C_{1-5}$ alkylamino,

(iv) heterocyclyl, and
heterocyclyl substituted by substituent(s) independently selected from the group consisting of:

- halogen,
- $C_{1-5}$ alkyl,
- $C_{1-5}$ alkyl substituted by carbocyclic aryl,
- $C_{1-5}$ alkoxy,
- $C_{1-5}$ alkoxycarbonyl,
- carbocyclic aryl,
- carbocyclic aryl substituted by substituent(s) independently selected from the group consisting of:

  - •• halogen,
  - •• $C_{1-5}$ alkyl, and
  - •• $C_{1-5}$ alkyl substituted by halogen;

wherein carbocyclic aryl is phenyl or naphthyl;
heterocyclyl is 1*H*-indolyl, 4-oxo-benzopyranyl, azetidinyl, benzo[1,3]dioxolyl, or pyrazolyl; and
halogen is fluoro, chloro, bromo, or iodo;

or a pharmaceutically acceptable salt, hydrate, or solvate thereof.

**33.** The compound according to claim 32 wherein $R_1$ is selected from the group consisting of:

(i) $C_{1-5}$ alkyl, and
$C_{1-5}$ alkyl substituted by substituent(s) independently selected from the group consisting of:

- mono-$C_{1-5}$ alkylamino,
- mono-$C_{1-5}$ alkylamino substituted by cyano,
- di-$C_{1-5}$ alkylamino,
- di-$C_{1-5}$ alkylamino substituted by cyano,
- mono-carbocyclic arylamino,
- di-carbocyclic arylamino,
- carbocyclic arylsulfonylamino,
- carbocyclic arylsulfonylamino substituted by $C_{1-5}$ alkyl, and
- carbocyclic aryl,

(ii) $C_{2-5}$ alkenyl, and
$C_{2-5}$ alkenyl substituted by carbocyclic aryl,
(iii) carbocyclic aryl, and
carbocyclic aryl substituted by substituent(s) independently selected from the group consisting of:

- halogen,
- hydroxy,
- $C_{1-5}$ alkoxy, and
- $C_{1-5}$ alkoxy substituted by halogen,

(iv) heterocyclyl, and
heterocyclyl substituted by substituent(s) independently selected from the group consisting of:

- halogen,
- $C_{1-5}$ alkyl,
- $C_{1-5}$ alkyl substituted by carbocyclic aryl,
- $C_{1-5}$ alkoxy,
- $C_{1-5}$ alkoxycarbonyl,
- carbocyclic aryl, and
- carbocyclic aryl substituted by halogen;

wherein carbocyclic aryl is phenyl;
heterocyclyl is 1*H*-indolyl, azetidinyl, or pyrazolyl; and
halogen is fluoro, chloro, bromo, or iodo;

or a pharmaceutically acceptable salt, hydrate, or solvate thereof.

**34.** The compound according to claim 1 selected from the group consisting of:

$N^2$-{cis-4-[(2,6-dimethoxybenzyl)amino]cyclohexyl}-$N^4$,$N^4$-dimethyl-5,6,7,8-tetrahydroquinazoline-2,4-diamine;
$N^2$-{cis-4-[(2-ethoxybenzyl)amino]cyclohexyl}-$N^4$,$N^4$-dimethyl-5,6,7,8-tetrahydroquinazoline-2,4-diamine;
$N^2$-{cis-4-[(1H-indol-3-ylmethyl)amino]cyclohexyl}-$N^4$,$N^4$-dimethyl-5,6,7,8-tetrahydroquinazoline-2,4-diamine;
$N^2$-{cis-4-[(2,5-dimethoxybenzyl)amino]cyclohexyl}-$N^4$,$N^4$-dimethyl-5,6,7,8-tetrahydroquinazoline-2,4-diamine;
$N^2$-(cis-4-{[(4-methoxy-1-naphthyl)methyl]amino}cyclohexyl)-$N^4$,$N^4$-dimethyl-5,6,7,8-tetrahydroquinazoline-2,4-diamine;
$N^2$-(cis-4-{[(5-methoxy-1H-indol-3-yl)methyl]amino}cyclohexyl)-$N^4$,$N^4$-dimethyl-5,6,7,8-tetrahydroquinazoline-2,4-diamine;
4-bromo-2-{[(cis-4-{[4-(dimethylamino)-5 ,6,7,8-tetrahydroquinazolin-2-yl]amino}cyclohexyl)amino]methyl}-6-methoxyphenol;
$N^2$-(cis-4-{[(5-bromo-1H-indol-3-yl)methyl]amino}cyclohexyl)-$N^4$,$N^4$-dimethyl-5,6,7,8-tetrahydroquinazoline-2,4-diamine;
4-{[(cis-4-{[4-(dimethylamino)-5,6,7,8-tetrahydroquinazolin-2-yl]amino}-cyclohexyl)amino]methyl}-2,6-dimethoxyphenol;
$N^2$-{cis-4[(3-ethoxy-4-methoxybenzyl)amino]cyclohexyl}-$N^4$,$N^4$-dimethyl-5,6,7,8-tetrahydroquinazoline-2,4-diamine;
$N^4$,$N^4$-dimethyl-$N^2$-{cis-4-[({3-[4-(trifluoromethyl)phenyl]-1H-pyrazol-4-yl}methyl)amino]cyclohexyl} - 5,6,7,8-tetrahydroquinazoline-2,4-diamine;
$N^4$,$N^4$-dimethyl-$N^2$-{cis-4-[(3,4,5-trimethoxybenzyl)amino]cyclohexyl}-5,6,7,8-tetrahydroquinazoline-2,4-diamine;
$N^4$,$N^4$-dimethyl-$N^2$-{cis-4-[(pentamethylbenzyl)amino]cyclohexyl}-5,6,7,8-tetrahydroquinazoline-2,4-diamine;
$N^2$-{cis-4-[(3,5-dimethoxybenzyl)amino]cyclohexyl}-$N^4$,$N^4$-dimethyl-5,6,7,8-tetrahydroquinazoline-2,4-diamine;
4-{[(cis-4-{[4-(dimethylamino)-5,6,7,8-tetrahydroquinazolin-2-yl]amino}-cyclohexyl)amino]methyl}-2-iodo-6-methoxyphenol;
4-{[(cis-4-{[4-(dimethyiamino)-5,6,7,8-tetrahydroquinazolin-2-yl]amino}-cyclohexyl)amino]methyl}-2,6-dimethylphenol;
3-{[(cis-4-{[4-(dimethylamino)-5,6,7,8-tetrahydroquinazolin-2-yl]amino}-cyclohexyl)amino]methyl}-6,8-dimethyl-4H-chromen-4-one;
ethyl 4,6-dichloro-3-{[(cis-4-{[4-(dimethylamino)-5,6,7,8-tetrahydroquinazolin-2-yl]amino}cyclohexyl)amino]methyl}-1H-indole-2-carboxylate;
$N^2$-[cis-4-({[3-(4-fluorophenyl)-1H-pyrazol-4-yl]methyl}amino)cyclohexyl]-$N^4$,$N^4$-dimethyl-5,6,7,8-tetrahydroquinazoline-2,4-diamine;
$N^4$,$N^4$-dimethyl-$N^2$-[4-(pentamethylphenylmethyl-amino)-cyclohexyl]-5,6,7,8-tetrahydro-quinazoline-2,4-diamine;
3-[{2-[(cis-4-{[4-(dimethylamino)-5,6,7,8-tetrahydroquinazolin-2-yl]amino}cyclohexyl)amino]ethyl}(3-methylphenyl)amino]propanenitrile;
3-[{2-[(cis-4-{[4-(dimethylamino)-5,6,7, 8-tetrahydroquinazolin-2-yl]amino}cyclohexyl)amino]ethyl}(phenyl)amino]propanenitrile;
N-{(1S)-1-benzyl-2-[(cis-4-{[4-(dimethylamino)-5,6,7,8-tetrahydroquinazolin-2-yl]amino}cyclohexyl)amino]ethyl}-4-methylbenzenesulfonamide;
$N^2$-(cis-4-{[2-(3,5-dimethoxyphenyl)ethyl]amino}cyclohexyl)-$N^4$,$N^4$-dimethyl-5,6,7,8-tetrahydroquinazoline-2,4-diamine;
$N^2$-[cis-4-({[1-(diphenylmethyl)azetidin-3-yl]methyl}amino)cyclohexyl]-$N^4$,$N^4$-dimethyl-5,6,7,8-tetrahydroquinazoline-2,4-diamine;
$N^2$-(cis-4-{[(2,6-dimethoxybenzyl)amino]methyl} cyclohexyl)-$N^4$,$N^4$-dimethyl-5,6,7,8-tetrahydroquinazoline-2,4-diamine;
$N^2$-(cis-4-{[(2-ethoxybenzyl)amino]methyl}cyclohexyl)-$N^4N^4$-dimethyl-5,6,7,8-tetrahydroquinazoline-2,4-diamine;
$N^2$-(cis-4-{[(1H-indol-3-ylmethyl)amino]methyl}cyclohexyl)-$N^4$,$N^4$-dimethyl-5,6,7,8-tetrahydroquinazoline-2,4-diamine;

$N^2$-(cis-4-{[(2,5-dimethoxybenzyl)amino]methyl}cyclohexyl)-$N^4$,$N^4$-dimethyl-5,6,7,8-tetrahydroquinazoline-2,4-diamine;
$N^2$-[cis-4-({[(4-methoxy-1-naphthyl)methyl]amino}methyl)cyclohexyl]-$N^4$,$N^4$-dimethyl-5,6,7,8-tetrahydroquinazoline-2,4-diamine;
$N^2$-[cis-4-({[(5-methoxy-1H-indol-3-yl)methyl]amino}methyl)cyclohexyl]-$N^4$,$N^4$-dimethyl-5,6,7,8-tetrahydroquinazoline-2,4-diamine;
4-bromo-2-({[(cis-4-{[4-(dimethylamino)-5,6,7,8-tetrahydroquinazolin-2-yl]amino}cyclohexyl)methyl]amino}methyl)-6-methoxyphenol;
$N^2$-[cis-4-({[(5-bromo-1H-indol-3-yl)methyl]amino}methyl)cyclohexyl]-$N^4$,$N^4$-dimethyl-5,6,7,8-tetrahydroquinazoline-2,4-diamine;
$N^2$-(cis-4-{[(3-ethoxy-4-methoxybenzyl)amino]methyl}cyclohexyl)-$N^4$,$N^4$-dimethyl-5,6,7,8-tetrahydroquinazoline-2,4-diamine;
$N^4$,$N^4$-dimethyl-$N^2$-(cis-4-{[({3-[4-(trifluoromethyl)phenyl]-1H-pyrazol-4-yl}methyl)amino]methyl}cyclohexyl)-5,6,7,8-tetrahydroquinazoline-2,4-diamine;
$N^4$,$N^4$-dimethyl-$N^2$-(cis-4-{[(3,4,5-trimethoxybenzyl)amino]-methyl}cyclohexyl)-5,6,7,8-tetrahydroquinazoline-2,4-diamine;
$N^2$-(cis-4-{[(3,5-dimethoxybenzyl)amino]methyl}cyclohexyl)-$N^4$,$N^4$-dimethyl-5,6,7,8-tetrahydroquinazoline-2,4-diamine;
4-({[(cis-4-{[4-(dimethylamino)-5,6,7,8-tetrahydroquinazolin-2-yl]amino}cyclohexyl)methyl]amino}methyl)-2-iodo-6-methoxyphenol;
4-({[(cis-4-{[4-(dimethylamino)-5,6,7,8-tetrahydroquinazolin-2-yl]amino}cyclohexyl)methyl]amino}methyl)-2,6-dimethylphenol;
3-chloro-4-({[(cis-4-{[4-(dimethylamino)-5,6,7,8-tetrahydroquinazolin-2-yl]amino}cyclohexyl)methyl]amino methyl)phenol;
$N^2$-[cis-4-({[4-(diethylamino)benzyl]amino}methyl)cyclohexyl]-$N^4$,$N^4$-dimethyl-5,6,7,8-tetrahydroquinazoline-2,4-diamine;
$N^2$-(cis-4-{[(3,3-diphenylprop-2-en-1-yl)amino]methyl}cyclohexyl)-$N^4$,$N^4$-dimethyl-5,6,7,8-tetrahydroquinazoline-2,4-diamine;
4-({[(cis-4-{[4-(dimethylamino)-5,6,7,8-tetrahydroquinazolin-2-yl]amino}cyclohexyl)methyl]amino}methyl)-2-ethoxyphenol;
$N^2$-{cis-4-[({[4-(dimethylamino)-1-naphthyl]methyl}amino)methyl]-cyclohexyl}-$N^4$ $N^4$-dimethyl-5,6,7,8-tetrahydroquinazoline-2,4-diamine;
$N^4$,$N^4$-dimethyl-$N^2$-(cis-4-{[(2,4,6-trimethoxybenzyl)amino]methyl}-cyclohexyl)-5,6,7,8-tetrahydroquinazoline-2,4-diamine;
2-bromo-4-chloro-6-({[(cis-4-{[4-(dimethylamino)-5,6,7,8-tetrahydroquinazolin-2-yl]amino}cyclohexyl)methyl]amino}methyl)phenol;
$N^2$-(cis-4-{[(2,5-diethoxybenzyl)amino]methyl}cyclohexyl)-$N^4$,$N^4$-dimethyl-5,6,7,8-tetrahydroquinazoline-2,4-diamine;
$N^2$-(cis-4-{[(2,4-diethoxybenzyl)amino]methyl}cyclohexyl)-$N^4$ $N^4$-dimethyl-5,6,7,8-tetrahydroquinazoline-2,4-diamine;
$N^2$-(cis-4-{[(3,5-dibromo-2-methoxybenzyl)amino]methyl}cyclohexyl)-$N^4$,$N^4$-dimethyl-5,6,7,8-tetrahydroquinazoline-2,4-diamine;
$N^4$,$N^4$ -dimethyl-$N^2$ -(cis-4-{[(2,4,5-triethoxybenzyl)amino]methyl}-cyclohexyl)-5,6,7,8-tetrahydroquinazoline-2,4-diamine;
$N^4$,$N^4$-dimethyl-$N^2$-(cis-4-{[2,4,5-trimethoxybenzyl)amino]methyl}-cyclohexyl)-5,6,7,8-tetrahydroquinazoline-2,4-diamine;
$N^2$-[cis-4-({[(7-methoxy-1,3-benzodioxol-5-yl)methyl]amino}methyl)-cyclohexyl]-$N^4$,$N^4$-dimethyl-5,6,7,8-tetrahydroquinazoline-2,4-diamine; 4-({[(cis-4-{[4-(dimethylamino)-5,6,7,8-tetrahydroquinazolin-2-yl]amino}cyclohexyl)methyl]amino}methyl)-2-methylphenol;
$N^2$-(cis-4-{[(4-methoxy-2,5-dimethylbenzyl)amino]methyl}cyclohexyl)-$N^4$,$N^4$-dimethyl-5,6,7,8-tetrahydroquinazoline-2,4-diamine; 4-({[(cis-4-{[4-(dimethylamino)-5,6,7,8-tetrahydroquinazolin-2-yl]amino}cyclohexyl)methyl]amino}methyl)-2-fluoro-6-methoxyphenol;
$N^4$,$N^4$-dimethyl-$N^2$-[cis-4-({[(1-phenyl-5-propyl-1H-pyrazol-4-yl)methyl]amino}methyl)cyclohexyl]-5,6,7,8-tetrahydroquinazoline-2,4-diamine;
$N^2$-{cis-4-[({[1-(4-chlorophenyl)-5-propyl-1H-pyrazol-4-yl]methyl}-amino)methyl]cyclohexyl}-$N^4$,$N^4$-dimethyl-5,6,7,8-tetrahydroquinazoline-2,4-diamine;
$N^2$-{cis-4-[2-(4-bromo-2-trifluoromethoxy-phenyl)-ethylamino]-cyclohexyl}-$N^4$,$N^4$-dimethyl-5,6,7,8-tetrahydro-quinazoline-2,4-diamine;

$N^2$-{cis-4-[2-(4-bromo-2-trifluoromethoxy-phenyl)-ethylamino]-cyclohexyl}-$N^4$-methyl-5,6,7,8-tetrahydro-quinazoline-2,4-diamine;
$N^2$-{cis-4-[(4-bromo-2-trifluoromethoxy-benzyl)amino-methyl]-cyclohexyl}-$N^4$-methyl-5,6,7,8-tetrahydro-quinazoline-2,4-diamine;
$N^2$-{cis-4-[(4-bromo-2-trifluoromethoxy-benzyl)amino-methyl]-cyclohexyl}-$N^4$,$N^4$-dimethyl-5,6,7,8-tetrahydro-quinazoline-2,4-diamine;
$N^4$,$N^4$-dimethyl-$N^2$-{cis-4-[(2-trifluoromethoxy-benzyl)amino-methyl]-cyclohexyl}-5,6,7,8-tetrahydro-quinazoline-2,4-diamine; and
$N^4$-methyl-$N^2$-{cis-4-[(2-trifluoromethoxy-benzyl)amino-methyl]-cyclohexyl}-5,6,7,8-tetrahydro-quinazoline-2,4-diamine;

or a pharmaceutically acceptable salt, hydrate, or solvate thereof.

**35.** The compound according to claim 34 selected from the group consisting of:

$N^2$-(cis-4-{[(5-methoxy-1H-indol-3-yl)methyl]amino}cyclohexyl)-$N^4$,$N^4$-dimethyl-5,6,7,8-tetrahydroquinazoline-2,4-diamine;
ethyl 4,6-dichloro-3-{[(cis-4-{[4-(dimethylamino)-5,6,7,8-tetrahydroquinazolin-2-yl]amino}cyclohexyl)amino]methyl}-1H-indole-2-carboxylate;
$N^2$-[cis-4-({[3-(4-fluorophenyl)-1H-pyrazol-4-yl]methyl}amino)cyclohexyl]-$N^4$,$N^4$-dimethyl-5,6,7,8-tetrahydro-quinazoline-2,4-diamine;
3-[{2-[(cis-4-{[4-(dimethylamino)-5,6,7,8-tetrahydroquinazolin-2-yl]amino}cyclohexyl)amino]ethyl}(phenyl)amino]propanenitrile;
N-{(1S)-1-benzyl-2-[(cis-4-{[4-(dimethylamino)-5,6,7,8-tetrahydroquinazolin-2-yl]amino}cyclohexyl)amino]ethyl}-4-methylbenzenesulfonamide;
$N^2$-[cis-4-({[1-(diphenylmethyl)azetidin-3-yl]methyl}amino)cyclohexyl]-$N^4$,$N^4$-dimethyl-5,6,7,8-tetrahydro-quinazoline-2,4-diamine;
$N^2$-(cis-4-{[(2,6-dimethoxybenzyl)amino)methyl}cyclohexyl)-$N^4$,$N^4$-dimethyl-5,6,7,8-tetrahydroquinazoline-2,4-diamine;
$N^2$-[cis-4-({[(5-methoxy-1H-indol-3-yl)methyl}amino}methyl)cyclohexyl]-$N^4$,$N^4$-dimethyl-5,6,7,8-tetrahydro-quinazoline-2,4-diamine;
$N^2$-[cis-4-({[(5-bromo-1H-indol-3-yl)methyl}amino}methyl)cyclohexyl]-$N^4$,$N^4$-dimethyl-5,6,7,8-tetrahydro-quinazoline-2,4-diamine;
$N^2$-(cis-4-{[(3-ethoxy-4-methoxybenzyl)amino]methyl}cyclohexyl)-$N^4$,$N^4$-dimethyl-5,6,7,8-tetrahydroquinazoline-2,4-diamine;
4-({[(cis-4-{[4-(dimethylamino)-5,6,7,8-tetrahydroquinazolin-2-yl]amino}cyclohexyl)methyl]amino}methyl)-2-iodo-6-methoxyphenol;
$N^2$-(cis-4-{[(3,3-diphenylprop-2-en-1-yl)amino]methyl}cyclohexyl)-$N^4$,$N^4$-dimethyl-5,6,7,8-tetrahydroquinazoline-2,4-diamine;
$N^4$,$N^4$-dimethyl-$N^2$-(cis-4-{[(2,4,6-trimethoxybenzyl)amino]methyl}-cyclohexyl)-5,6,7,8-tetrahydroquinazoline-2,4-diamine;
$N^2$-(cis-4-{[(2,5-diethoxybenzyl)amino]methyl}cyclohexyl)-$N^4$,$N^4$-dimethyl-5,6,7,8-tetrahydroquinazoline-2,4-diamine;
$N^2$-(cis-4-{[(2,4-diethoxybenzyl)amino]methyl}cyclohexyl)-$N^4$,$N^4$-dimethyl-5,6,7,8-tetrahydroquinazoline-2,4-diamine;
$N^2$-(cis-4-{[(3,5-dibromo-2-methoxybenzyl)amino]methyl}cyclohexyl)-$N^4$,$N^4$-dimethyl-5,6,7,8-tetrahydro-quinazoline-2,4-diamine;
$N^4$,$N^4$-dimethyl-$N^2$-(cis-4-{[(2,4,5-triethoxybenzyl)amino}methyl}-cyclohexyl)-5,6,7,8-tetrahydroquinazoline-2,4-diamine;
$N^4$,$N^4$-dimethyl-$N^2$-(cis-4-{[(2,4,5-trimethoxybenzyl)amino]methyl}-cyclohexyl)-5,6,7,8-tetrahydroquinazoline-2,4-diamine;
$N^4$,$N^4$-dimethyl-$N^2$-[cis-4-({[(1-phenyl-5-propyl-1H-pyrazol-4-yl)methyl]amino}methyl)cyclohexyl]-5,6,7,8-tetrahydroquinazoline-2,4-diamine;
$N^2$-{cis-4-[({[1-(4-chlorophenyl)-5-propyl-1H-pyrazol-4-yl]methyl}-amino)methyl]cyclohexyl}-$N^4$,$N^4$-dimethyl-5,6,7,8-tetrahydroquinazoline-2,4-diamine;
$N^2$-{cis-4-[2-(4-bromo-2-trifluoromethoxy-phenyl)-ethylamino]-cyclohexyl}-$N^4$,$N^4$-dimethyl-5,6,7,8-tetrahydro-quinazoline-2,4-diamine;
$N^2$-{cis-4-[(4-bromo-2-trifluoromethoxy-benzyl)amino-methyl]-cyclohexyl}-$N^4$-methyl-5,6,7,8-tetrahydro-quinazoline-2,4-diamine;
$N^2$-{cis-4-[(4-bromo-2-trofluoromethoxy-benzyl)amino-methyl]-cyclohexyl)-$N^4$,$N^4$-dimethyl-5,6,7,8-tetrahydro-quinazoline-2,4-diamine; and
$N^4$,$N^4$-dimethyl-$N^2$-{cis-4-[(2-trifluoromethoxy-benzyl)amino-methyl]-cyclohexyl}-5,6,7,8-tetrahydro-quinazoline-2,4-diamine;

or a pharmaceutically acceptable salt, hydrate, or solvate thereof.

**36.** The compound according to claim 29 wherein $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- heterocyclyloxy,
- heterocyclyloxy substituted by $C_{1-5}$ alkyl,
- mono-$C_{1-5}$ alkylaminocarbonyl,
- di-$C_{1-5}$ alkylaminocarbonyl,
- carbocyclic arylcarbonylamino,
- $C_{1-5}$ alkylthio,
- $C_{1-5}$ alkylthio substituted by substituent(s) independently selected from the group consisting of:

  •• carbocyclic aryl, and
  •• carbocyclic aryl substituted by substituent(s) independently selected from the group consisting of:

    ••• halogen, and
    ••• $C_{1-5}$ alkoxy,

- carbocyclic arylthio,
- heterocyclylthio,
- heterocyclylthio substituted by $C_{1-5}$ alkyl,
- $C_{3-6}$ cycloalkyl,
- $C_{3-6}$ cycloalkenyl,
- carbocyclyl,
- carbocyclyl substituted by substituent(s) independently selected from the group consisting of:

  •• halogen,
  •• $C_{1-5}$ alkyl,
  •• $C_{1-5}$ alkoxy,
  •• $C_{2-5}$ alkenyl, and
  •• $C_{2-5}$ alkenyl substituted by substituent(s) independently selected from the group consisting of:

    ••• carbocyclic aryl, and
    ••• carbocyclic aryl substituted by $C_{1-5}$ alkylsulfinyl,

- carbocyclic aryl,
- carbocyclic aryl substituted by substituent(s) independently selected from the group consisting of:

  •• halogen,
  •• hydroxy,
  •• nitro,
  •• $C_{1-5}$ alkyl,

- •• $C_{1-5}$ alkyl substituted by substituent(s) independently selected from the group consisting of:
  - ••• oxo,
  - ••• carbocyclic aryl, and
  - ••• heterocyclyl,
- •• $C_{1-5}$ alkoxy,
- •• $C_{1-5}$ alkoxy substituted by halogen,
- •• $C_{1-5}$ alkoxy substituted by carbocyclic aryl,
- •• carbocyclic aryloxy,
- •• mono-carbocyclic arylaminocarbonyl,
- •• mono-carbocyclic arylaminocarbonyl substituted by halogen,
- •• di-carbocyclic arylaminocarbonyl,
- •• di-carbocyclic arylaminocarbonyl substituted by halogen,
- •• carbocyclic aryl, and
- •• heterocyclyl,

- • heterocyclyl, and
- • heterocyclyl substituted by substituent(s) independently selected from the group consisting of:
  - •• $C_{1-5}$ alkyl,
  - •• $C_{1-5}$ alkoxy,
  - •• $C_{1-5}$ alkoxy substituted by carbocyclic aryl,
  - •• carbocyclic aryl, and
  - •• carbocyclic aryl substituted by halogen,

(ii) $C_{2-5}$ alkenyl, and
$C_{2-5}$ alkenyl substituted by substituent(s) independently selected from the group consisting of:

- • carbocyclic aryl,
- • carbocyclic aryl substituted by substituent(s) independently selected from the group consisting of:
  - •• halogen, and
  - •• nitro,

(iii) $C_{3-6}$ cycloalkyl, and
$C_{3-6}$ cycloalkyl substituted by substituent(s) independently selected from the group consisting of:

- • $C_{1-5}$ alkyl,
- • $C_{1-5}$ alkyl substituted by substituent(s) independently selected from the group consisting of:
  - •• oxo, and
  - •• carbocyclic aryl,
- • carbocyclic aryl,

(iv) carbocyclyl,
(v) carbocyclic aryl, and
carbocyclic aryl substituted by substituent(s) independently selected from the group consisting of:

- • halogen,
- • hydroxy,
- • cyano,
- • nitro,
- • $C_{1-5}$ alkyl,
- • $C_{1-5}$ alkyl substituted by substituent(s) independently selected from the group consisting of:
  - •• halogen,

- •• oxo,
- •• carbocyclic aryloxy,
- •• carbocyclic aryl, and
- •• carbocyclic aryl substituted by $C_{1-5}$ alkyl,

- $C_{1-5}$ alkoxy,
- $C_{1-5}$ alkoxy substituted by substituent(s) independently selected from the group consisting of:

  - •• halogen, and
  - •• carbocyclic aryl,

- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- $C_{1-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,
- mono-$C_{1-5}$ alkylaminosulfonyl,
- di-$C_{1-5}$ alkylaminosulfonyl,
- carbocyclic aryl,
- heterocyclyl,
- heterocyclyl substituted by substituent(s) independently selected from the group consisting of:

  - •• $C_{1-5}$ alkyl,
  - •• carbocyclic aryl, and
  - •• halogenated carbocyclic aryl,

(vi) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:

  - •• halogen,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by halogen, and
  - •• heterocyclyl,

- $C_{1-5}$ alkoxy,
- carbocyclic aryloxy,
- carbocyclic aryloxy substituted by $C_{1-5}$ alkyl,

- $C_{1-5}$ alkylthio,
- $C_{2-5}$ alkenylthio,
- carbocyclic arylthio,
- $C_{1-5}$ alkylsulfonyl,
- carbocyclic arylsulfonyl,
- carbocyclic arylsulfonyl substituted by $C_{1-5}$ alkyl,
- carbocyclic aryl,
- carbocyclic aryl substituted by substituent(s) independently selected from the group consisting of:

  - •• halogen,
  - •• nitro, and
  - •• $C_{1-5}$ alkyl,

- heterocyclyl; L is Formula (VII);

Y is -C(O)-;

wherein carbocyclic aryl is phenyl, naphthyl, or anthranyl;

carbocyclyl is 1,2,3,4-tetrahydronaphthyl, 1-oxo-indanyl, 9-oxo-9*H*-fluorenyl, or indenyl;

heterocyclyl is 1,2,3-triazolyl, 1*H*-indolyl, 1*H*-pyrrolyl, 2,3-dihydro-1-oxo-isoindolyl, 2,4-dihydro-3-oxo-pyrazolyl, 2*H*-benzopyranyl, 2-oxo-benzopyranyl, 4-oxo-1,5,6,7-tetrahydro-indolyl, 9*H*-xanthenyl, benzo[1,3]dioxolyl, benzo[2,1,3]oxadiazolyl, benzo[1,2,5]oxadiazolyl, benzo[b]thienyl, benzofuryl, benzothiazolyl, furyl, isoxazolyl, morpholino, pyrazolyl, pyridyl, pyrimidyl, quinolyl, quinoxalyl, thiazolyl, or thienyl; and

halogen is fluoro, chloro, bromo, or iodo;

or a pharmaceutically acceptable salt, hydrate, or solvate thereof.

**37.** The compound according to claim 36 wherein $R_2$ is methylamino or dimethylamino; p is 0; $R_3$ and $R_4$ are hydrogen; A is a single bond; B is a single bond or $-CH_2-$;
or a pharmaceutically acceptable salt, hydrate, or solvate thereof.

**38.** The compound according to claim 37 wherein $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- mono-$C_{1-5}$ alkylaminocarbonyl,
- di-$C_{1-5}$ alkylaminocarbonyl,
- carbocyclic arylcarbonylamino,
- $C_{1-5}$ alkylthio,
- $C_{1-5}$ alkylthio substituted by substituent(s) independently selected from the group consisting of:

  - •• carbocyclic aryl, and
  - •• carbocyclic aryl substituted by halogen,

- heterocyclylthio,
- heterocyclylthio substituted by $C_{1-5}$ alkyl,
- $C_{3-6}$ cycloalkyl,
- carbocyclyl,
- carbocyclyl substituted by substituent(s) independently selected from the group consisting of:

  - •• halogen,
  - •• $C_{1-5}$ alkyl,

- •• $C_{1-5}$ alkenyl, and
- •• $C_{1-5}$ alkenyl substituted by substituent(s) independently selected from the group consisting of:
  - ••• carbocyclic aryl, and
  - ••• carbocyclic aryl substituted by $C_{1-5}$ alkylsulfinyl,

- • carbocyclic aryl,
- • carbocyclic aryl substituted by substituent(s) independently selected from the group consisting of:
  - •• halogen,
  - •• hydroxy,
  - •• nitro,
  - •• $C_{1-5}$ alkyl,
  - •• $C_{1-5}$ alkyl substituted by substituent(s) independently selected from the group consisting of:
    - ••• oxo, and
    - ••• heterocyclyl,
  - •• $C_{1-5}$ alkoxy,
  - •• carbocyclic aryloxy,
  - •• carbocyclic aryl, and
  - •• heterocyclyl,

- • heterocyclyl, and
- • heterocyclyl substituted by substituent(s) independently selected from the group consisting of:
  - •• $C_{1-5}$ alkyl,
  - •• $C_{1-5}$ alkoxy, and
  - •• carbocyclic aryl,

(ii) $C_{2-5}$ alkenyl, and
$C_{2-5}$ alkenyl substituted by substituent(s) independently selected from the group consisting of:

- • carbocyclic aryl, and
- • carbocyclic aryl substituted by nitro,

(iii) $C_{3-6}$ cycloalkyl, and
$C_{3-6}$ cycloalkyl substituted by carbocyclic aryl,
(iv) carbocyclyl,
(v) carbocyclic aryl, and
carbocyclic aryl substituted by substituent(s) independently selected from the group consisting of:

- • halogen,
- • hydroxy,
- • cyano,
- • nitro,
- • $C_{1-5}$ alkyl,
- • $C_{1-5}$ alkyl substituted by substituent(s) independently selected from the group consisting of:
  - •• halogen,
  - •• oxo, and
  - •• carbocyclic aryl,
- • $C_{1-5}$ alkoxy,
- • $C_{1-5}$ alkoxy substituted by substituent(s) independently selected from the group consisting of:
  - •• halogen, and
  - •• carbocyclic aryl,
- • carbocyclic aryloxy,

- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy, and
- (carbocyclic aryl)NHC(O)NH substituted by haloganated $C_{1-5}$ alkoxy,

(vi) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:
  - •• halogen,
  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl, and
  - •• heterocyclyl,
- carbocyclic aryloxy,
- carbocyclic aryloxy substituted by $C_{1-5}$ alkyl,
- $C_{1-5}$ alkylthio,
- carbocyclic aryl,
- carbocyclic aryl substituted by substituent(s) independently selected from the group consisting of:
  - •• halogen,
  - •• nitro, and
  - •• $C_{1-5}$ alkyl,
- heterocyclyl;

wherein carbocyclic aryl is phenyl;
carbocyclyl is 1-oxo-indanyl or indenyl;
heterocyclyl is 1,2,3-triazolyl, 1*H*-indolyl, 1*H*-pyrrolyl, 2,3-dihydro-1-oxo-isoindolyl, 2-oxo-benzopyranyl, benzo[2,1,3]oxadiazolyl, benzo[1,2,5]oxadiazolyl, furyl, isoxazolyl, morpholino, pyrazolyl, pyridyl, pyrimidyl, quinolyl, quinoxalyl, thiazolyl, or thienyl;
halogen is fluoro, chloro, bromo, or iodo;

or a pharmaceutically acceptable salt, hydrate, or solvate thereof.

**39.** The compound according to claim 38 wherein $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkylcarbonyloxy,
- carbocyclic aryloxy,
- carbocyclic aryloxy substituted by halogen,

- mono-$C_{1-5}$ alkylaminocarbonyl,
- di-$C_{1-5}$ alkylaminocarbonyl,
- carbocyclic arylcarbonylamino,
- carbocyclyl,
- carbocyclyl substituted by substituent(s) independently selected from the group consisting of:

  - •• halogen,
  - •• $C_{1-5}$ alkyl,
  - •• $C_{2-5}$ alkenyl, and
  - •• $C_{2-5}$ alkenyl substituted by substituent(s) independently selected from the group consisting of:

    - ••• carbocyclic aryl, and
    - ••• carbocyclic aryl substituted by $C_{1-5}$ alkylsulfinyl,

- carbocyclic aryl,
- carbocyclic aryl substituted by substituent(s) independently selected from the group consisting of:

  - •• halogen,
  - •• hydroxy,
  - •• nitro,
  - •• $C_{1-5}$ alkyl, and
  - •• $C_{1-5}$ alkoxy,

- heterocyclyl, and
- heterocyclyl substituted by substituent(s) independently selected from the group consisting of:

  - •• $C_{1-5}$ alkyl,
  - •• $C_{1-5}$ alkoxy, and
  - •• carbocyclic aryl,

(ii) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:

  - •• halogen, and
  - •• oxo,

- $C_{1-5}$ alkoxy,
- $C_{1-5}$ alkoxy substituted by carbocyclic aryl,
- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyelic aryl)NHC(O)NH substituted by $C_{1-5}$ alkoxy, and
- (carbocyclic aryl)NHC(O)NH substituted by haloganated $C_{1-5}$ alkoxy,

(iii) heterocyclyl, and
heterocyclyl substituted by substituent(s) independently selected from the group consisting of:

- halogen,
- nitro,
- $C_{1-5}$ alkyl,
- $C_{1-5}$ alkyl substituted by halogen,
- $C_{1-5}$ alkyl substituted by heterocyclyl,
- carbocyclic aryloxy,
- carbocyclic aryloxy substituted by $C_{1-5}$ alkyl,
- $C_{1-5}$ alkylthio,
- carbocyclic aryl,
- carbocyclic aryl substituted by halogen, and
- carbocyclic aryl substituted by nitro;

wherein carbocyclic aryl is phenyl;
carbocyclyl is indenyl;
heterocyclyl is 1H-indolyl, 1H-pyrrolyl, 2-oxo-benzopyranyl, benzo[2,1,3]oxadiazolyl, benzo[1,2,5]oxadiazolyl, furyl, isoxazolyl, morpholino, pyridyl, quinoxalyl, or thienyl; and
halogen is fluoro, chloro, bromo, or iodo;

or a pharmaceutically acceptable salt, hydrate, or solvate thereof.

**40.** The compound according to claim 1 selected from the group consisting of:

N-(cis-4-{[4-(dimethylamino)-5,6,7,8-tetrahydroquinazolin-2-yl]amino}-cyclohexyl)-3-methoxybenzamide;
3-bromo-N-(cis-4-{[4-(dimethylamino)-5,6,7,8-tetrahydroquinazolin-2-yl]amino}cyclohexyl)benzamide;
4-bromo-N-(cis-4-{[4-(dimethylamino)-5,6,7,8-tetrahydroquinazolin-2-yl]amino}cyclohexyl)benzamide;
N-(cis-4-{[4-(dimethylamino)-5,6,7,8-tetrahydroquinazolin-2-yl]amino}-cyclohexyl)-2,1,3-benzoxadiazole-5-carboxamide;
3-chloro-N-(cis-4-{[4-(dimethylamino)-5,6,7,8-tetrahydroquinazolin-2-yl]amino}cyclohexyl)benzamide;
4-chloro-N-(cis-4-{[4-(dimethylamino)-5,6,7,8-tetrahydroquinazolin-2-yl]amino}cyclohexyl)benzamide;
4-chloro-N-(cis-4-{[4-(dimethylamino)-5,6,7,8-tetrahydroquinazolin-2-yl]amino}cyclohexyl)-3-nitrobenzamide;
2-(4-chloroghenyl)-N-(cis-4-{[4-(dimethylamino)-5,6,7,8-tetrahydroquinazolin-2-yl]amino}cyclohexyl)acetamide;
3-cyano-N-(cis-4-{[4-(dimethylamino)-5,6,7,8-tetrahydroquinazolin-2-yl]amino}cyclohexyl)benzamide;
3,5-dichloro-N-(cis-4-{[4-(dimethylamino)-5,6,7,8-tetrahydroquinazolin-2-yl]amino}cyelohexyl)benzamide;
3,4-dichloro-N-(cis-4-{[4-(dimethylamino)-5,6,7,8-tetrahydroquinazolin-2-yl]amino}cyclohexyl)benzamide;
N-(cis-4-{[4-(dimethylamino)-5,6,7,8-tetrahydroquinazolin-2-yl]amino}-cyclohexyl)-2,2-diphenylacetamide;
N-(cis-4-{[4-(dimethylamino)-5,6,7,8-tetrahydroquinazolin-2-yl]amino}-cyclohexyl)-3,4-difluorobenzamide;
N-(cis-4-{[4-(dimethylamino)-5,6,7,8-tetrahydroquinazolin-2-yl]amino}-cyclohexyl)-3,5-difluorobenzamide;
N-(cis-4-{[4-(dimethylamino)-5,6,7,8-tetrahydroquinazolin-2-yl]amino}-cyclohexyl)-4-fluorobenzamide;
N-(cis-4-{[4-(dimethylamino)-5,6,7,8-tetrahydroquinazolin-2-yl]amino}-cyclohexyl)-3-fluoro-5-(trifluoromethyl)benzamide;
N-(cis-4-{[4-(dimethylamino)-5,6,7,8-tetrahydroquinazolin-2-yl]amino}-cyclohexyl)hexanamide;
N-(cis-4-{[4-(dimethylamino)-5,6,7,8-tetrahydroquinazolin-2-yl]amino}-cyclohexyl)-4-methyl-3-nitrobenzamide;
N-(cis-4-{[4-(dimethylamino)-5,6,7,8-tetrahydroquinazolin-2-yl]amino}-cyclohexyl)-3-nitrobenzamide;
(2R)-N-(cis-4-{[4-(dimethylamino)-5,6,7,8-tetrahydroquinazolin-2-yl]amino}cyclohexyl)-2-phenylcyclopropanecarboxamide;
N-(cis-4-{[4-(dimethylamino)-5,6,7,8-tetrahydroquinazolin-2-yl]amino}-cyclohexyl)-2-phenoxybutanamide;
N-(cis-4-{[4-(dimethylamino)-5,6,7,8-tetrahydroquinazolin-2-yl]amino}-cyclohexyl)-2-phenoxypropanamide;
N-(cis-4-{[4-(dimethylamino)-5,6,7,8-tetrahydroquinazolin-2-yl]amino}-cyclohexyl)-3-methylbenzamide;
N-(cis-4-{[4-(dimethylamino)-5,6,7,8-tetrahydroquinazolin-2-yl]amino}-cyclohexyl)-4-methylbenzamide;
N-(cis-4-{[4-(dimethylamino)-5,6,7,8-tetrahydroquinazolin-2-yl]amino}-cyclohexyl)-3-(trifluoromethoxy)benzamide;
4-bromo-N-(cis-4-{[4-(dimethylamino)-5,6,7,8-tetrahydroquinazolin-2-yl]amino}cyclohexyl)-3-methylbenzamide;
N-(cis-4-{[4-(dimethylamino)-5,6,7,8-tetrahydroquinazolin-2-yl]amino}-cyclohexyl)-3-iodobenzamide;
2-chloro-N-(cis-4-{[4-(dimethylamino)-5,6,7,8-tetrahydroquinazolin-2-yl]amino}cyclohexyl)-4-fluorobenzamide;
N-(cis-4-{[4-(dimethylamino)-5,6,7,8-tetrahydroquinazolin-2-yl]amino}-cyclohexyl)-2-(3-methoxyphenyl) acetamide;
N-(cis-4-{[4-(dimethylamino)-5,6,7,8-tetrahydroquinazolin-2-yl]amino}-cyclohexyl)-2-(4-fluorophenyl)acetamide;
N-(cis-4-{[4-(dimethylamino)-5,6,7,8-tetrahydroquinazolin-2-yl]amino}-cyclohexyl)-2-(4-methoxyphenyl) acetamide;
N-(cis-4-{[4-(dimethylamino)-5,6,7,8-tetrahydroquinazolin-2-yl]amino}-cyclohexyl)-5-methyl-2-(trifluoromethyl)-3-furamide;
N-(cis-4-{[4-(dimethylamino)-5,6,7,8-tetrahydroquinazolin-2-yl]amino}-cyclohexyl)-2,5-dimethyl-3-furamide;
3-chloro-N-(cis-4-{[4-(dimethylamino)-5,6,7,8-tetrahydroquinazolin-2-yl]amino}cyclohexyl)-4-fluorobenzamide;
N-(cis-4-{[4-(dimethylamino)-5,6,7,8-tetrahydroquinazolin-2-yl]amino}-cyclohexyl)-3-fluoro-4-methylbenzamide;
N-(cis-4-{[4-(dimethylamino)-5,6,7,8-tetrahydroquinazolin-2-yl]amino}-cyclohexyl)-3,5-dimethoxybenzamide;
N-(cis-4-{[4-(dimethylamino)-5,6,7,8-tetrahydroquinazolin-2-yl]amino}-cyclohexyl)-3,5-bis(trifluoromethyl) benzamide;
N-(cis-4-{[4-(dimethylamino)-5,6,7,8-tetrahydroquinazolin-2-yl]amino}-cyclohexyl)-4-fluoro-3-methylbenzamide;
2,5-dichloro-N-(cis-4-{[4-(dimethylamino)-5,6,7,8-tetrahydroquinazolin-2-yl]amino}cyclohexyl)thiophene-3-carboxamide;
N-(cis-4-{[4-(dimethylamino)-5,6,7,8-tetrahydroquinazolin-2-yl]amino}-cyclohexyl)-2-(propylthio)nicotinamide;
1-benzyl-3-tert-butyl-N-(cis-4-{[4-(dimethylamino)-5,6,7,8-tetrahydroquinazolin-2-yl]amino}cyclohexyl)-1H-pyrazole-5-carboxamide;
5-bromo-N-(cis-4-{[4-(dimethylamino)-5,6,7,8-tetrahydroquinazolin-2-yl]amino}cyclohexyl)nicotinamide;
2-[(cis-4-{[4-(dimethylamino)-5,6,7,8-tetrahydroquinazolin-2-yl]amino}-cyclohexyl)amino]-2-oxo-1-phenylethyl acetate;
N-(cis-4-{[4-(dimethylamino)-5,6,7,8-tetrahydroquinazolin-2-yl]amino}-cyclohexyl)benzamide;
2-(benzyloxy)-N-(cis-4-{[4-(dimethylamino)-5,6,7,8-tetrahydroquinazolin-2-yl]amino}cyclohexyl)acetamide;
2-(4-chlorophenoxy)-N-(cis-4-{[4-(dimethylamino)-5,6,7,8-tetrahydroquinazolin-2-yl]amino}cyclohexyl)acetamide;
3-(2-chlorophenyl)-N-(cis-4-{[4-(dimethylamino)-5,6,7,8-tetrahydroquinazolin-2-yl]amino}cyclohexyl)-5-methylisoxazole-4-carboxamide;
1-(4-chlorophenyl)-N-(cis-4-{[4-(dimethylamino)-5,6,7,8-tetrahydroquinazolin-2-yl]amino}cyclohexyl)cyclopentanecarboxamide;
3-(2-chloro-6-fluorophenyl)-N-(cis-4-{[4-(dimethylamino)-5,6,7,8-tetrahydroquinazolin-2-yl]amino}cyclohexyl)-5-methylisoxazole-4-carboxamide;
N-(cis-4-{[4-(dimethylamino)-5,6,7,8-tetrahydroquinazolin-2-yl]amino}-cyclohexyl)-1,3-dimethyl-1H-pyrazole-5-carboxamide;
N-(cis-4-{[4-(dimethylamino)-5,6,7,8-tetrahydroquinazolin-2-yl]amino}-cyclohexyl)-3-fluorobenzamide;
N-(cis-4-{[4-(dimethylamino)-5,6,7,8-tetrahydroquinazolin-2-yl]amino}-cyclohexyl)-4-nuoro-3-(trifluoromethyl)benzamide;
N-(cis-4-{[4-(dimethylamino)-5,6,7,8-tetrahydroquinazolin-2-yl]amino}-cyclohexyl)-5-methyl-2-phenyl-2H-1,2,3-triazole-4-carboxamide;
N-(cis-4-{[4-(dimethylamino)-5,6,7,8-tetrahydroquinazolin-2-yl]amino}-cyclohexyl)-2-(4-methoxyphenoxy)-5-nitrobenzamide;
N-(cis-4-{[4-(dimethylamino)-5,6,7,8-tetrahydroquinazolin-2-yl]amino}-cyclohexyl)-5-nitro-2-furamide;
N-(cis-4-{[4-(dimethylamino)-5,6,7,8-tetrahydroquinazolin-2-yl]amino}-cyclohexyl)-2-phenoxyacetamide;
N-(cis-4-{[4-(dimethylamino)-5,6,7,8-tetrahydroquinazolin-2-yl]amino}-cyclohexyl)quinoxaline-2-carboxamide;
N-(cis-4-{[4-(dimethylamino)-5,6,7,8-tetrahydroquinazolin-2-yl]amino}-cyclohexyl)-3-(trifluoromethyl)benzamide;
2-(3-chlorophenoxy)-N-(cis-4-{[4-(dimethylamino)-5,6,7,8-tetrahydroquinazolin-2-yl]amino}cyclohexyl)acetamide;
3-(2,6-dichlorophenyl)-N-(cis-4-{[4-(dimethylamino)-5,6,7,8-tetrahydroquinazolin-2-yl]amino}cyclohexyl)-5-methylisoxazole-4-carboxamide;
N-(cis-4-{[4-(dimethylamino)-5,6,7,8-tetrahydroquinazolin-2-yl]amino}-cyclohexyl)-2-phenoxynicotinamide;

N-(cis-4-{[4-(dimethylamino)-5,6,7,8-tetrahydroquinazolin-2-yl]amino}-cyclohexyl)-2-(4-methylphenoxy)nicotinamide;
N-(cis-4-{[4-(dimethylamino)-5,6,7,8-tetrahydroquinazolin-2-yl]amino}-cyclohexyl)-2-(2-thienyl)-1,3-thiazole-4-carboxamide;
5-bromo-N-(cis-4-{[4-(dimethylamino)-5,6,7,8-tetrahydroquinazolin-2-yl]amino}cyclohexyl)thiophene-2-carboxamide;
N-(cis-4-{[4-(dimethylamino)-5,6,7,8-tetrahydroquinazolin-2-yl]amino}-cyclohexyl)-2-(2,3,6-trichlorophenyl) acetamide;
2-(2-chloro-4-fluorophenyl)-N-(cis-4-{[4-(dimethylamino)-5,6,7,8-tetrahydroquinazolin-2-yl]amino}cyclohexyl) acetamide;
5-(4-chloro-2-nitrophenyl)-N-(cis-4-{[4-(dimethylamino)-5,6,7,8-tetrahydroquinazolin-2-yl]amino}cyclohexyl)-2-furamide;
5-chloro-N-(cis-4-{[4-(dimethylamino)-5,6,7,8-tetrahydroquinazolin-2-yl]amino}cyclohexyl)thiophene-2-carboxamide;
N-(cis-4-{[4-(dimethylamino)-5,6,7,8-tetrahydroquinazolin-2-yl]amino}-cyclohexyl)-2,3-diphenylpropanamide;
N-(cis-4-{[4-(dimethylamino)-5,6,7,8-tetrahydroquinazolin-2-yl]amino}-cyclohexyl)-3-(2-hydroxyphenyl)propanamide;
N-(cis-4-{[4-(dimethylamino)-5,6,7,8-tetrahydroquinazolin-2-yl]amino}-cyclohexyl)-5-iodo-2-furamide;
N-(cis-4-{[4-(dimethylamino)-5,6,7,8-tetrahydroquinazolin-2-yl]amino}-cyclohexyl)-2-(2-iodophenyl)acetamide;
N-(cis-4-{[4-(dimethylamino)-5,6,7,8-tetrahydroquinazolin-2-yl]amino}-cyclohexyl)-2-(5-methoxy-2-methyl-1H-indol-3-yl)acetamide;
N-(cis-4-{[4-(dimethylamino)-5,6,7,8-tetrahydroquinazolin-2-yl]amino}-cyclohexyl)-3-oxoindane-1-carboxamide;
2-benzyl-N-(cis-4-{[4-(dimethylamino)-5,6,7,8-tetrahydroquinazolin-2-yl]amino}cyclohexyl)benzamide;
2,2-bis(4-chlorophenyl)-N-(cis-4-{[4-(dimethylamino)-5,6,7,8-tetrahydroquinazolin-2-yl]amino}cyclohexyl) acetamide;
N-(cis-4-{[4-(dimethylamino)-5,6,7,8-tetrahydroquinazolin-2-yl]amino}-cyclohexyl)-5-(4-methyl-2-nitrophenyl)-2-furamide;
N-(cis-4-{[4-(dimethylamino)-5,6,7,8-tetrahydroquinazolin-2-yl]amino}-cyclohexyl)-5-nitrothiophene-2-carboxamide;
N-(cis-4-{[4-(dimethylamino)-5,6,7,8-tetrahydroquinazolin-2-yl]amino}-cyclohexyl)-3-methyl-4-nitrobenzamide;
N-(cis-4-{[4-(dimethylamino)-5,6,7,8-tetrahydroquinazolin-2-yl]amino}-cyclohexyl)-3-methoxy-4-nitrobenzamide;
3-acetyl-N-(cis-4-{[4-(dimethylamino)-5,6,7,8-tetrahydroquinazolin-2-yl]amino}cyclohexyl)benzamide;
5-bromo-N-(cis-4-{[4-(dimethylamino)-5,6,7,8-tetrahydroquinazolin-2-yl]amino}cyclohexyl)-2-furamide;
N-(cis-4-{[4-(dimethylamino)-5,6,7,8-tetrahydroquinazolin-2-yl]amino}-cyclohexyl)-2-[(4-methylpyrimidin-2-yl)thio]acetamide;
5-(4-chlorophenyl)-N-(cis-4-{[4-(dimethylamino)-5,6,7,8-tetrahydroquinazolin-2-yl]amino}cyclohexyl)-2-furamide;
2-(3,4-dichlorophenyl)-N-(cis-4-{[4-(dimethylamino)-5,6,7,8-tetrahydroquinazolin-2-yl]amino}cyclohexyl) acetamide;
N-(cis-4-{[4-(dimethylamino)-5,6,7,8-tetrahydroquinazolin-2-yl]amino}-cyclohexyl)-2-(4-hydroxy-3,5-dimethoxyphenyl)acetamide;
4,5-dibrorno-N-(cis-4-{[4-(dimethylamino)-5,6,7,8-tetrahydroquinazolin-2-yl]amino}cyclohexyl)thiophene-2-carboxamide;
$N^2N^6$-dibenzoyl-N-(cis-4-{[4-(dimethylamino)-5,6,7,8-tetrahydroquinazolin-2-yl]amino}cyclohexyl)lysinamide;
3-(dimethylamino)-N-(cis-4-{[4-(dimethylamino)-5,6,7,8-tetrahydroquinazolin-2-yl]amino}cyclohexyl)benzamide;
4,5-dibromo-N-(cis-4-{[4-(dimethylamino)-5,6,7,8-tetrahydroquinazolin-2-yl]amino}cyclohexyl)-2-furamide;
N-(cis-4-{[4-(dimethylamino)-5,6,7,8-tetrahydroquinazolin-2-yl]amino}-cyclohexyl)-4-(4-fluorophenyl)-4-oxobutanamide;
N-(cis-4-{[4-(dimethylamino)-5,6,7,8-tetrahydroquinazolin-2-yl]amino}-cyclohexyl)-2-(2-fluorobiphenyl-4-yl) propanamide;
N-(cis-4-{[4-(dimethylamino)-5,6,7,8-tetrahydroquinazolin-2-yl]amino}-cyclohexyl)-2-[4-(1-oxo-1,3-dihydro-2H-isoindol-2-yl)phenyl]propanamide;

N-(cis-4-{[4-(dimethylamino)-5,6,7,8-tetrahydroquinazolin-2-yl]amino}-cyclohexyl)-2-(1H-indol-3-yl)acetamide;
N-(cis-4-{[4-(dimethylamino)-5,6,7,8-tetrahydroquinazolin-2-yl]amino}-cyclohexyl)-2-(7-methoxy-2-oxo-2H-chromen-4-yl)acetamide;
N-(cis-4-{[4-(dimethylamino)-5,6,7,8-tetrahydroquinazolin-2-yl]amino}-cyclohexyl)-2-(1H-indol-3-yl)-4-oxo-4-phenylbutanamide;
N-(cis-4-{[4-(dimethylamino)-5,6,7,8-tetrahydroquinazolin-2-yl]amino}-cyclohexyl)-3,5-dimethyl-2-[({[4-(trifluoromethoxy)phenyl]amino}carbonyl)amino]-benzamide;
3,5-dichloro-N-(cis-4-{[4-(dimethylamino)-5,6,7,8-tetrahydroquinazolin-2-yl]amino}cyclobexyl)-2-[(3-phenylprop-2-ynoyl)amino]benzamide;
4-(4-tert-butylphenyl)-N-(cis-4-{[4-(dimethylamino)-5,6,7,8-tetrahydroquinazolin-2-yl]amino}cyclohexyl)-2-(7-ethyl-1H-indol-3-yl)-4-oxobutanamide;
N-(cis-4-{[4-(dimethylamino)-5,6,7,8-tetrahydroquinazolin-2-yl]amino}-cyclohexyl)-2-(1-methyl-1H-indol-3-yl)-4-(4-methylphenyl)-4-oxobutanamide;
N-(cis-4-{[4-(dimethylamino)-5,6,7,8-tetrahydroquinazolin-2-yl]amino}-cyclohexyl)-2-methyl-1-(3-morpholin-4-ylpropyl)-5-phenyl-1H-pyrrole-3-carboxamide;
N-(cis-4-{[4-(dimethylamino)-5,6,7,8-tetrahydroquinazolin-2-yl]amino}-cyclohexyl)-4-(4-nitrophenyl)butanamide;
N-(cis-4-{[4-(dimethylamino)-5,6,7,8-tetrahydroquinazolin-2-yl]amino}-cyclohexyl)-2-(3-phenoxyphenyl) acetamide;
N-(cis-4-{[4-(dimethylamino)-5,6,7,8-tetrahydroquinazolin-2-yl]amino}-cyclohexyl)-2-(4-phenoxyphenyl) acetamide;
N-(cis-4-{[4-(dimethylamino)-5,6,7,8-tetrahydroquinazolin-2-yl]amino}-cyclohexyl)-2-(2-phenyl-1H-indol-3-yl) acetamide;
$N^2$-benzoyl-$N^5$-(cis-4-{[4-(dimethylamino)-5,6,7,8-tetrahydroquinazolin-2-yl]amino}cyclohexyl)-$N^1$,$N^1$-dipropylglutamamide;
N-(cis-4-{[4-(dimethylamino)-5,6,7,8-tetrahydroquinazolin-2-yl]amino}-cyclohexyl)-3-phenoxybenzamide;
N-(cis-4-{[4-(dimethylamino)-5,6,7,8-tetrahydroquinazolin-2-yl]amino}-cyclohexyl)-2-(ethylthio)-2,2-diphenylacetamide;
N'-(cis-4-{[4-(dimethylamino)-5,6,7,8-tetrahydroquinazolin-2-yl]amino}-cyclohexyl)-N,N-bis[(1S)-1-phenylethyl]phthalamide;
(2S)-N-(cis-4-{[4-(dimethylamino)-5,6,7,8-tetrahydroquinazolin-2-yl]amino}cyclohexyl}-2-(2-fluorobiphenyl-4-yl)propanamide;
2-[(4-chlorobenzyl)thio]-N-(cis-4-{[4-(dimethylamino)-5,6,7,8-tetrahydroquinazolin-2-yl]amino}cyclohexyl)-4-(4-methylphenyl)-4-oxobutanamide;
N-(cis-4-{[4-(dimethylamino)-5,6,7,8-tetrahydroquinazolin-2-yl]amino}-cyclohexyl)-2-{(1E)-5-fluoro-2-methyl-1-[4-(methylsulfinyl)benzylidene]-1H-inden-3-yl}acetamide;
N-(cis-4-{[4-(dimethylamino)-5,6,7,8-tetrahydroquinazolin-2-yl]amino}-cyclohexyl)-2-[4-(2-thienylcarbonyl) phenyl]propanamide;
3-(benzyloxy)-N-(cis-4-{[4-(dimethylamino)-5,6,7,8-tetrahydroquinazolin-2-yl]amino}cyclohexyl)-4-methoxybenzamide;
N-(cis-4-{(4-(dimethylamino)-5,6,7,8-tetrahydroquinazolin-2-yl]amino}-cyclohexyl)-2-methyl-1,5-diphenyl-1H-pyrrole-3-carboxamide;
1-{2-[(2-chloro-6-fluorobenzyl)thio]ethyl}-N-(cis-4-{[4-(dimethylamino)-5,6,7,8-tetrahydroquinazolin-2-yl]amino}cyclohexyl)-2-methyl-5-phenyl-1H-pyrrole-3-carboxamide;
N-(cis-4-{[4-(dimethylamino)-5,6,7,8-tetrahydroquinazolin-2-yl]amino}-cyclohexyl)-2-phenoxybenzamide;
N-(cis-4-{[4-(dimethylamino)-5,6,7,8-tetrahydroquinazolin-2-yl]amino}-cyclohexyl)-2-phenylquinoline-4-carboxamide;
N-(cis-4-{[4-(dimethylamino)-5,6,7,8-tetrahydroquinazolin-2-yl]amino}-cyclohexyl)-5-(3-nitrophenyl)-2-furamide;
N-(cis-4-{[4-(dimethylamino)-5,6,7,8-tetrahydroquinazolin-2-yl]amino}-cyclohexyl)-5-nitrothiophene-3-carboxamide;
N-(cis-4-{[4-(dimethylamino)-5,6,7,8-tetrahydroquinazolin-2-yl]amino}-cyclohexyl)-1-methyl-4-nitro-1H-pyrrole-2-carboxamide;
N-(cis-4-{[4-(dimethylamino)-5,6,7,8-tetrahydroquinazolin-2-yl]amino}-cyclohexyl)-2-methoxy-4-nitrobenzamide;
N-(cis-4-{[4-(dimethylamino)-5,6,7,8-tetrahydroquinazolin-2-yl]amino}-cyclohexyl)-2-methoxy-2-phenylacetamide;

5-chloro-N-(cis-4-{[4-(dimethylamino)-5,6,7,8-tetrahydroquinazolin-2-yl]amino}cyclohexyl)-2-hydroxybenzamide;
3-bromo-N-[(cis-4-{[4-(dimethylamino)-5,6,7,8-tetrahydroquinazolin-2-yl]amino}cyclohexyl)methyl]benzamide;
N-[(cis-4-{[4-(dimethylamino)-5,6,7,8-tetrahydroquinazolin-2-yl]amino}-cyclohexyl)methyl]-2-(ethylthio)nicotinamide;
N-[(cis-4-{[4-(dimethylamino)-5,6,7,8-tetrahydroquinazolin-2-yl]amino}-cyclohexyl)methyl]-2-(4-methoxyphenyl)acetamide;
N-[(cis-4-{[4-(dimethylamino)-5,6,7,8-tetrahydroquinazolin-2-yl]amino}-cyclohexyl)methyl]-5-methyl-2-(trifluoromethyl)-3-furamide;
(2E)-N-[(cis-4-{[4-(dimethylamino)-5,6,7,8-tetrahydroquinazolin-2-yl]amino}cyclohexyl)methyl]-3-(4-nitrophenyl)acrylamide;
N-[(cis-4-{[4-(dimethylamino)-5,6,7, 8-tetrahydroquinazolin-2-yl]amino}-cyclohexyl)methyl]-4-fluoro-3-methylbenzamide;
N-[(cis-4-{[4-(dimethylamino)-5,6,7,8-tetrahydroquinazolin-2-yl]amino}-cyclohexyl)methyl]-2-(propylthio) nicotinamide;
2,6-dichloro-N-[(cis-4-{[4-(dimethylamino)-5,6,7,8-tetrahydroquinazolin-2-yl]amino}cyclohexyl)methyl]benzamide;
N-[(cis-4-{[4-(dimethylamino)-5,6,7,8-tetrahydroquinazolin-2-yl]amino}-cyclohexyl)methyl]-2,4,6-trimethylbenzamide;
2-chloro-N-[(cis-4-{[4-(dimethylamino)-5,6,7,8-tetrahydroquinazolin-2-yl]amino}cyclohexyl)methyl]-6-fluorobenzamide;
2,4,6-trichloro-N-[(cis-4-{[4-(dimethylamino)-5,6,7,8-tetrahydroquinazolin-2-yl]amino}cyclohexyl)methyl]benzamide;
N-[(cis-4-{[4-(dimethylamino)-5,6,7,8-tetrahydroquinazolin-2-yl]amino}-cyclohexyl)methyl]-2-(2,3,6-trichlorophenyl)acetamide;
(2E)-N-[(cis-4-{[4-(dimethylamino)-5,6,7,8-tetrahydroquinazolin-2-yl]amino}cyclohexyl)methyl]-3-(3-nitrophenyl)acrylamide; and
N-[cis-4-(4-dimethylamino-5,6,7,8-tetrahydro-quinazolin-2-ylamino)-cyclohexylmethyl]-3,4-difluoro-benzamide;

or a pharmaceutically acceptable salt, hydrate, or solvate thereof.

**41.** The compound according to claim 40 selected from the group consisting of:

N-(cis-4-{[4-(dimethylamino)-5,6,7,8-tetrahydroquinazolin-2-yl]amino}-cyclohexyl)-3-methoxybenzamide;
3-bromo-N-(cis-4-{[4-(dimethylamino)-5,6,7,8-tetrahydroquinazolin-2-yl]amino}cyclohexyl)benzamide;
N-(cis-4-{[4-(dimethylamino)-5,6,7,8-tetrahydroquinazolin-2-yl]amino}-cyclohexyl)-2,1,3-benzoxadiazole-5-carboxamide;
3-chloro-N-(cis-4-{[4-(dimethylamino)-5,6,7,8-tetrahydroquinazolin-2-yl]amino}cyclohexyl)benzamide;
4-chloro-N-(cis-4-{[4-(dimethylamino)-5,6,7,8-tetrahydroquinazolin-2-yl]amino}cyclohexyl)benzamide;
4-chloro-N-(cis-4-{[4-(dimethylamino)-5,6,7,8-tetrahydroquinazolin-2-yl]amino}cyclohexyl)-3-nitrobenzamide;
2-(4-chlorophenyl)-N-(cis-4-{[4-(dimethylamino)-5,6,7,8-tetrahydroquinazolin-2-yl]amino}cyclohexyl)acetamide;
3-cyano-N-(cis-4-{[4-(dimethylamino)-5,6,7,8-tetrahydroquinazolin-2-yl]amino}cyclohexyl)benzamide;
3,5-dichloro-N-(cis-4-{[4-(dimethylamino)-5,6,7,8-tetrahydroquinazolin-2-yl]amino}cyclohexyl)benzamide;
3,4-dichloro-N-(cis-4-{[4-(dimethylamino)-5,6,7,8-tetrahydroquinazolin-2-yl]amino}cyclohexyl)benzamide;
N-(cis-4-{[4-(dimethylamino)-5,6,7,8-tetrahydroquinazolin-2-yl]amino}-cyclohexyl)-2,2-diphenylacetamide;
N-(cis-4-{[4-(dimethylamino)-5,6,7,8-tetrahydroquinazolin-2-yl]amino}-cyclohexyl)-3,4-difluorobenzamide;
N-(cis-4-{[4-(dimethylamino)-5,6,7,8-tetrahydroquinazolin-2-yl]amino}-cyclohexyl)-3,5-difluorobenzamide;
N-(cis4-{[4-(dimethylamino)-5,6,7,8-tetrahydroquinazolin-2-yl]amino}-cyclohexyl)-4-fluorobenzamide;
N-(cis-4-{4-(dimethylamino)-5,6,7,8-tetrahydroquinazolin-2-yl]amino}-cyclohexyl)-3-fluoro-5-(trifluoromethyl) benzamide;
N-(cis-4-{[4-(dimethylamino)-5,6,7,8-tetrahydroquinazolin-2-yl]amino}-cyclohexyl)-4-methyl-3-nitrobenzamide;
N-(cis-4-{[4-(dimethylamino)-5,6,7,8-tetrahydroquinazolin-2-yl]amino}-cyclohexyl)-3-nitrobenzamide;
N-(cis-4-{[4-(dimethylamino)-5,6,7,8-tetrahydroquinazolin-2-yl]amino}-cyclohexyl)-2-phenoxybutanamide;

N-(cis-4-{[4-(dimethylamino)-5,6,7,8-tetrahydroquinazolin-2-yl]amino}-cyclohexyl)-2-phenoxypropanamide;
N-(cis-4-{[4-(dimethylamino)-5,6,7,8-tetrahydroquinazolin-2-yl]amino}-cyclohexyl)-3-methylbenzamide;
N-(cis-4-{[4-(dimethylamino)-5,6,7,8-tetrahydroquinazolin-2-yl]amino}-cyclohexyl)-3-iodobenzamide;
N-(cis-4-{[4-(dimethylamino)-5,6,7,8-tetrahydroquinazolin-2-yl]amino}-cyclohexyl)-2-(4-fluorophenyl)acetamide;
N-(cis-4-{[4-(dimethylamino)-5,6,7,8-tetrahydroquinazolin-2-yl]amino}-cyclohexyl)-2,5-dimethyl-3-furamide;
3-chloro-N-(cis-4-{[4-(dimethylamino)-5,6,7,8-tetrahydroquinazolin-2-yl]amino}cyctohexyl)-4-fluorobenzamide;
N-(cis-4-{[4-(dimethylamino)-5,6,7,8-tetrahydroquinazolin-2-yl]amino}-cyclohexyl)-3,5-dimethoxybenzamide;
N-(cis-4-{[4-(dimethylamino)-5,6,7,8-tetrahydroquinazolin-2-yl]amino}-cyclohexyl)-3,5-bis(trifluoromethyl) benzamide;
N-(cis-4-{[4-(dimethylamino)-5,6,7,8-tetrahydroquinazolin-2-yl]amino}-cyclohexyl)-4-fluoro-3-methylbenzamide;
2,5-dichloro-N-(cis-4-{[4-(dimethylamino)-5,6,7,8-tetrahydroquinazolin-2-yl]amino}cyclohexyl)thiophene-3-carboxamide;
5-bromo-N-(cis-4-{[4-(dimethylamino)-5,6,7,8-tetrahydroquinazolin-2-yl]amino}cyclohexyl)nicotinamide;
2-[(cis-4-{[4-(dimethylamino)-5,6,7, 8-tetrahydroquinazolin-2-yl]amino}-cyclohexyl)amino]-2-oxo-1-phenylethyl acetate;
3-(2-chloro-6-fluorophenyl)-N-(cis-4-{[4-(dimethylamino)-5,6,7,8-tetrahydroquinazolin-2-yl]amino}cyclohexyl)-5-methylisoxazole-4-carboxamide;
N-(cis-4-{[4-(dimethylamino)-5,6,7,8-tetrahydroquinazolin-2-yl]amino}-cyclohexyl)-3-fluorobenzamide;
N-(cis-4-{[4-(dimethylamino)-5,6,7,8-tetrahydroquinazolin-2-yl]amino}-cyclohexyl)-4-fluoro-3-(trifluoromethyl)benzamide;
N-(cis-4-{[4-(dimethylamino)-5,6,7,8-tetrahydroquinazoiin-2-yl]amino}-cyclohexyl)-2-(4-methoxyphenoxy)-5-nitrobenzamide;
N-(cis-4-{[4-(dimethylamino)-5,6,7,8-tetrahydroquinazolin-2-yl]amino}-cyclohexyl)-5-nitro-2-furamide;
N-(cis-4-{[4-(dimethylamino)-5,6,7,8-tetrahydroquinazolin-2-yl]amino}-cyclohexyl)-2-phenoxyacetamide;
N-(cis-4-{[4-(dimethylamino)-5,6,7,8-tetrahydroquinazolin-2-yl]amino}-cyclohexyl)quinoxaline-2-carboxamide;
N-(cis-4-{[4-(dimethylamino)-5,6,7,8-tetrahydroquinazolin-2-yl]amino}-cyclohexyl)-3-(trifluoromethyl)benzamide;
2-(3-chlorophenoxy)-N-(cis-4-{[4-(dimethylamino)-5,6,7,8-tetrahydroquinazolin-2-yl]amino}cyclohexyl)acetamide;
3-(2,6-dichlorophenyl)-N-(cis-4-{[4-(dimethylamino)-5,6,7,8-tetrahydroquinazolin-2-yl]amino}cyclohexyl)-5-methylisoxazole-4-carboxamide;
N-(cis-4-{[4-(dimethylamino)-5,6,7,8-tetrahydroquinazolin-2-yl]amino}-cyclohexyl)-2-(4-methylphenoxy)nicotinamide;
2-(2-chloro-4-fluorophenyl)-N-(cis-4-{[4-(dimethylamino)-5,6,7,8-tetrahydroquinazolin-2-yl]amino}cyclohexyl) acetamide;
5-(4-chloro-2-nitrophenyl)-N-(cis-4-{[4-(dimethylamino)-5,6,7,8-tetrahydroquinazolin-2-yl]amino)}cyclohexyl)-2-furamide;
5-chloro-N-(cis-4-{[4-(dimethylamino)-5,6,7,8-tetrahydroquinazolin-2-yl]amino}cyclohexyl)thiophene-2-carboxamide;
N-(cis-4-{[4-(dimethylamino)-5,6,7,8-tetrahydroquinazolin-2-yl]amino}-cyclohexyl)-5-iodo-2-furamide;
2,2-bis(4-chlorophenyl)-N-(cis-4-{[4-(dimethylamino)-5,6,7,8-tetrahydroquinazolin-2-yl]amino}cyclohexyl) acetamide;
N-(cis-4-{[4-(dimethylamino)-5,6,7,8-tetrahydroquinazolin-2-yl]amino}-cyclohexyl)-5-nitrothiophene-2-carboxamide;
N-(cis-4-{[4-(dimethylamino)-5,6,7,8-tetrahydroquinazolin-2-yl]amino}-cyclohexyl)-3-methyl-4-nitrobenzamide;
N-(cis-4-{[4-(dimethylamino)-5,6,7,8-tetrahydroquinazolin-2-yl]amino}-cyclohexyl)-3-methoxy-4-nitrobenzamide;
3-acetyl-N-(cis-4-{[4-(dimethylamino)-5,6,7,8-tetrahydroquinazolin-2-yl]amino}cyclohexyl)benzamide;
5-bromo-N-(cis-4-{[4-(dimethylamino)-5,6,7,8-tetrahydroquinazolin-2-yl]amino}cyclohexyl)-2-furamide;
5-(4-chlorophenyl)-N-(cis-4-{[4-(dimethylamino)-5,6,7,8-tetrahydroquinazolin-2-yl]amino}cyclohexyl)-2-furamide;
2-(3,4-dichlorophenyl)-N-(cis-4-{[4-(dimethylamino)-5,6,7,8-tetrahydroquinazolin-2-yl]amino}cyclohexyl) acetamide;

N-(cis-4-{[4-(dimethylamino)-5,6,7,8-tetrahydroquinazolin-2-yl]amino}-cyclohexyl)-2-(4-hydroxy-3,5-dimethoxyphenyl)acetamide;
4,5-dibromo-N-(cis-4-{[4-(dimethylamino)-5,6,7,8-tetrahydroquinazolin-2-yl]amino}cyclohexyl)-2-furamide;
N-(cis-4-{[4-(dimethylamino)-5,6,7,8-tetrahydroquinazolin-2-yl]amino}-cyclohexyl)-2-(1H-indol-3-yl)acetamide;
N-(cis-4-{[4-(dimethylamino)-5,6,7,8-tetrahydroquinazolin-2-yl]amino}-cyclohexyl)-2-(7-methoxy-2-oxo-2H-chromen-4-yl)acetamide;
N-(cis-4-{[4-(dimethylamino)-5,6,7,8-tetrahydroquinazolin-2-yl]amino}-cyclohexyl)-3,5-dimethyl-2-[({[4-(trifluoromethoxy)phenyl]amino}carbonyl)amino]-benzamide;
3,5-dichloro-N-(cis-4-{[4-(dimethylamino)-5,6,7,8-tetrahydroquinazolin-2-yl]amino}cyclohexyl)-2-[(3-phenylprop-2-ynoyl)amino]benzamide;
4-(4-tect-butylphenyl)-N-(cis-4-{[4-(dimethylamino)-5,6,7,8-tetrahydroquinazolin-2-yl]amino}cyclohexyl)-2-(7-ethyl-1H-indol-3-yl)-4-oxobutanamide;
N-(cis-4-{[4-(dimethylamino)-5,6,7,8-tetrahydroquinazolin-2-yl]amino}-cyclohexyl)-2-methyl-1-(3-morpholin-4-ylpropyl)-5-phenyl-1H-pyrrole-3-carboxamide;
N-(cis-4-{[4-(dimethylamino)-5,6,7,8-tetrahydroquinazolin-2-yl]amino}-cyclohexyl)-4-(4-nitrophenyl)butanamide;
N-(cis-4-{[4-(dimethylamino)-5,6,7,8-tetrahydroquinazolin-2-yl]amino}-cyclohexyl)-2-(2-phenyl-1H-indol-3-yl) acetamide;
$N^2$-benzoyl-$N^5$-(cis-4-{[4-(dimethylamino)-5,6,7,8-tetrahydroquinazolin-2-yl]amino}cyclohexyl)-$N^1$,$N^1$-dipropylglutamamide;
N-(cis-4-{[4-(dimethylamino)-5,6,7,8-tetrahydroquinazolin-2-yl]amino}-cyclohexyl)-3-phenoxybenzamide;
N'-(cis-4-{[4-(dimethylamino)-5,6,7,8-tetrahydroquinazolin-2-yl]amino}-cyclohexyl)-N,N-bis[(1S)-1-phenylethyl]phthalamide;
N-(cis-4-{[4-(dimethylamino)-5,6,7,8-tetrahydroquinazolin-2-yl]amino}-cyclohexyl)-2-{(1E)-5-fluoro-2-methyl-1-[4-(methylsulfinyl)benzylidene]-1H-inden-3-yl}acetamide;
3-(benzyloxy)-N-(cis-4-{[4-(dimethylamino)-5,6,7,8-tetrahydroquinazolin-2-yl]amino}cyclohexyl)-4-methoxybenzamide;
N-(cis-4-{[4-(dimethylamino)-5,6,7,8-tetrahydroquinazolin-2-yl]amino}-cyclohexyl)-2-phenoxybenzamide;
N-(cis-4-{[4-(dimethylamino)-5,6,7,8-tetrahydroquinazolin-2-yl]amino}-cyclohexyl)-5-nitrothiophene-3-carboxamide;
N-(cis-4-{[4-(dimethylamino)-5,6,7,8-tetrahydroquinazolin-2-yl]amino}-cyclohexyl)-1-methyl-4-nitro-1H-pyrrole-2-carboxamide;
5-chloro-N-(cis-4-{[4-(dimethylamino)-5,6,7,8-tetrahydroquinazolin-2-yl]amino}cyclohexyl)-2-hydroxybenzamide;
N-[(cis-4-{[4-(dimethylamino)-5,6,7,8-tetrahydroquinazolin-2-yl]amino}-cyclohexyl)methyl]-2-(ethylthio)nicotinamide;
N-[(cis-4-{[4-(dimethylamino)-5,6,7,8-tetrahydroquinazolin-2-yl]amino}-cyclohexyl)methyl]-2-(4-methoxyphenyl)acetamide;
N-[(cis-4-{[4-(dimethylamino)-5,6,7,8-tetrahydroquinazolin-2-yl]amino}-cyclohexyl)methyl]-5-methyl-2-(trifluoromethyl)-3-furamide;
N-[(cis-4-{[4-(dimethylamino)-5,6,7,8-tetrahydroquinazolin-2-yl]amino}-cyclohexyl)methyl]-2-(propylthio) nicotinamide; and
2,4,6-trichloro-N-[(cis-4-{[4-(dimethylamino)-5,6,7,8-tetrahydroquinazolin-2-yl]amino}cyclohexyl)methyl]benzamide;

or a pharmaceutically acceptable salt, hydrate, or solvate thereof.

**42.** The compound according to claim 29 wherein $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkoxy carbonyl,
- carbocyclic aryl,
- carbocyclic aryl substituted by substituent(s) independently selected from the group consisting of:

- • halogen,
- • $C_{1-5}$ alkyl,
- • $C_{2-5}$ alkenyl, and
- • $C_{l-5}$ alkoxy,

- $C_{1-5}$ alkylthio, and
- heterocyclyl,

(ii) $C_{3-6}$ cycloalkyl, and
$C_{3-6}$ cycloalkyl substituted by carbocyclic aryl,
(iii) carbocyclyl,
(iv) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by substituent(s) independently selected from the group consisting of:

  - • halogen,
  - • oxo, and
  - • carbocyclic aryl,

- $C_{1-5}$ alkoxy carbonyl,
- $C_{1-7}$ alkoxy,
- $C_{1-7}$ alkoxy substituted by substituent(s) independently selected from the group consisting of:

  - • halogen, and
  - • carbocyclic aryl,

- $C_{3-6}$ cycloalkoxy,
- carbocyclic aryloxy,
- mono-$C_{1-5}$ alkylamino,
- di-$C_{1-5}$ alkylamino,
- $C_{1-5}$ alkylthio,
- $C_{1-5}$ alkylthio substituted by halogen, and
- carbocyclic aryl,

(v) heterocyclyl, and
heterocyclyl substituted by substituent(s) independently selected from the group consisting of:

- halogen,
- $C_{1-5}$ alkyl,
- $C_{1-5}$ alkyl substituted by halogen,
- $C_{1-5}$ alkoxy carbonyl
- $C_{1-5}$ alkoxy carbonyl substituted by carbocyclic aryl, and
- carbocyclic aryl;

L is Formula (VII);
Y is -C(O)$NR_5$-;
wherein carbocyclic aryl is phenyl or naphthyl;
carbocyclyl is indanyl, adamantly, or 9*H*-fluorenyl;
heterocyclyl is 2,3-dihydro-benzo[1,4]dioxinyl, 3,4-dihydro-2*H*-benzo[b][1,4]dioxepinyl, 4*H*-benzo(1,3] dioxinyl, benzo[1,3]dioxolyl, furyl, isoxazolyl, piperidyl, pyridyl, or thienyl;
halogen is fluoro, chloro, bromo, or iodo;

or a pharmaceutically acceptable salt, hydrate, or solvate thereof.

**43.** The compound according to claim 42 wherein $R_2$ is methylamino or dimethylamino; p is 0; $R_3$ and $R_4$ are hydrogen; A is a single bond; B is a single bond or $-CH_2-$: $R_5$ is hydrogen;
or a pharmaceutically acceptable salt, hydrate, or solvate thereof.

**44.** The compound according to claim 43 wherein $R_1$ is selected from the group consisting of:

(i) $C_{1-5}$ alkyl, and
$C_{1-5}$ alkyl substituted by substituent(s) independently selected from the group consisting of:

- $C_{1-5}$ alkoxy carbonyl,
- carbocyclic aryl, and
- carbocyclic aryl substituted by halogen,

(ii) carbocyclic aryl, and
carbocyclic aryl substituted by substituent(s) independently selected from the group consisting of:

- halogen,
- nitro,
- $C_{1-5}$ alkyl,
- $C_{1-5}$ alkyl substituted by halogen,
- $C_{1-5}$ alkoxy, and
- $C_{1-5}$ alkoxy substituted by halogen,

(iii) heterocyclyl, and
heterocyclyl substituted by $C_{1-5}$ alkyl, and
heterocyclyl substituted by carbocyclic aryl;
wherein carbocyclic aryl is phenyl or naphthyl;
heterocyclyl is isoxazolyl;
halogen is fluoro, chloro, bromo, or iodo;

or a pharmaceutically acceptable salt, hydrate, or solvate thereof.

**45.** The compound according to claim 1 selected from the group consisting of:

N-(cis-4-{[4-(dimethylamino)-5,6,7,8-tetrahydroquinazolin-2-yl]amino}-cyclohexyl)-N'-(2-ethyl-6-methylphenyl)urea;
N-(cis-4-{[4-(dimethylamino)-5,6,7,8-tetrahydroquinazolin-2-yl]amino}-cyclohexyl)-N'-(4-fluorophenyl)urea;
N-(cis-4-{[4-(dimethylamino)-5,6,7,8-tetrahydroquinazolin-2-yl]amino}-cyclohexyl)-N'-mesitylurea;
N-(cis-4-{[4-(dimethylamino)-5,6,7,8-tetrahydroquinazolin-2-yl]amino}-cyclohexyl)-N'-(2,4,6-trichlorophenyl) urea;
N-(cis-4-{[4-(dimethylamino)-5,6,7,8-tetrahydroquinazolin-2-yl]amino}-cyclohexyl)-N'-(2,4,6-tribromophenyl) urea;
N-(2,4-dibromo-6-fluorophenyl)-N'-(cis-4-{[4-(dimethylamino)-5,6,7,8-tetrahydroquinazolin-2-yl]amino}cyclohexyl)urea;
N-(2,6-diethylphenyl)-N'-(cis-4-{(4-(dimethylamino)-5,6,7,8-tetrahydroquinazolin-2-yl]amino}cyclohexyl)urea;
N-(2-chlorobenzyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(2-ethyl-6-isopropylphenyl)urea;
N-(cis-4-{[4-(dimethylamino)-5,6,7,8-tetrahydroquinazolin-2-yl]amino}-cyclohexyl)-N'-(2-ethylphenyl)urea;
N-(cis-4-{[4-(dimethylamino)-5,6,7,8-tetrahydroquinazolin-2-yl]amino}-cyclohexyl)-N'-(2-isopropyl-6-methylphenyl)urea;
N-(2-tert-butyl-6-methylphenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(diphenylmethyl)urea;
N-(4-bromo-2,6-dimethylphenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(3-methyl-5-phenylisoxazol-4-yl)urea;

N-(cis-4-{[4-(dimethylamino)-5,6,7,8-tetrahydroquinazolin-2-yl]amino}-cyclohexyl)-N'-1-naphthylurea;
N-(cis-4-{[4-(dimethylamino)-5,6,7,8-tetrahydroquinazolin-2-yl]amino}-cyclohexyl)-N'-[1-(1-naphthyl)ethyl] urea;
N-(2,4-dibromophenyl)-N'-(cis-4-{[4-(dimethylamino)-5,6,7,8-tetrahydroquinazolin-2-yl]amino}cyclohexyl) urea;
N-(2,4-dichlorobenzyl)-N'-(cis-4-{[4-(dimethylamino)-5,6,7,8-tetrahydroquinazolin-2-yl]amino}cyclohexyl) urea;
N-(2,4-dimethoxyphenyl)-N'-(cis-4-{[4-(dimethylamino)-5,6,7,8-tetrahydroquinazolin-2-yl]amino)cyclohexyl) urea;
N-(cis-4-{[4-(dimethylamino)-5,6,7,8-tetrahydroquinazolin-2-yl]amino}-cyclohexyl)-N'-(2-ethoxyphenyl)urea;
N-(cis-4-[4-(dimethylamino)-5,6,7,8-tetrahydroquinazolin-2-yl]amino}-cyclohexyl)-N'-(2-fluorobenzyl)urea;
N-(cis-4-{[4-(dimethylamino)-5,6,7,8-tetrahydroquinazolin-2-yl]amino}-cyclohexyl)-N'-(3,4,5-trimethoxyphenyl)urea;
N-(3,4-dimethoxyphenyl)-N'-(cis-4-{[4-(dimethylamino)-5,6,7,8-tetrahydroquinazolin-2-yl]amino}cyclohexyl) urea;
N-(4-chloro-2-methylphenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(4-fluorobenzyl)urea;
N-(cis-4-{[4-(dimethylamino)-5,6,7,8-tetrahydroquinazolin-2-yl]amino}-cyclohexyl)-N'-(4-methoxy-2-methylphenyl)urea;
N-(5-chloro-2,4-dimethoxyphenyl)-N'-(cis-4-{[4-(dimethylamino)-5,6,7,8-tetrahydroquinazolin-2-yl]amino}cyclohexyl)urea;
N-[1-(4-bromophenyl)ethyl]-N'-(cis-4-{[4-(dimethylamino)-5,6,7,8-tetrahydroquinazolin-2-yl]amino}cyclohexyl)urea;
N-(4-bromo-2-methylphenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}-cyclohexyl)-N'-(5-methyl-3-phenylisoxazol-4-yl)urea;
N-(2,3-dichlorophenyl)-N'-(cis-4-{[4-(dimethylamino)-5,6,7,8-tetrahydroquinazolin-2-yl]amino}cyclohexyl) urea;
N-(cis-4-{[4-(dimethylamino)-5,6,7,8-tetrahydroquinazolin-2-yl]amino}-cyclohexyl)-N'-(4-methylphenyl)urea;
N-(2,6-diisopropylphenyl)-N'-(cis-4-{[4-(dimethylamino)-5,6,7,8-tetrahydroquinazolin-2-yl]amino}cyclohexyl) urea;
N-(cis-4-{[4-(dimethylamino)-5,6,7,8-tetrahydroquinazolin-2-yl]amino}-cyclohexyl)-N'-(2,4,5-trichlorophenyl) urea;
N-(2,5-dimethoxyphenyl)-N'-(cis-4-{[4-(dimethylamino)-5,6,7,8-tetrahydroquinazolin-2-yl]amino}cyclohexyl) urea;
N-(4-bromo-2-chlorophenyl)-N'-(cis-4-{[4-(dimethylamino)-5,6,7,8-tetrahydroquinazolin-2-yl]amino}cyclohexyl)urea;
N-(cis-4-{[4-(dimethylamino)-5,6,7,8-tetrahydroquinazolin-2-yl]amino}cyclohexyl)-N'-[2-(trifluoromethoxy) phenyl]urea;
N-[(cis-4-{[4-(dimethylamino)-5,6,7,8-tetrahydroquinazolin-2-yl]amino}-cyclohexyl)methyl]-N'-(2,6-dimethylphenyl)urea;
N-(2,4-diftuorophenyl)-N'-[(cis-4-{[4-(dimethylamino)-5,6,7,8-tetrahydroquinazolin-2-yl]amino}cyclohexyl) methyl]urea;
N-[(cis-4-{[4-(dimethylamino)-5,6,7,8-tetrahydroquinazolin-2-yl]amino}-cyclohexyl)methyl]-N'-(2-ethyl-6-methylphenyl)urea;
ethyl N-({[(cis-4-{[4-(dimethylamino)-5,6,7,8-tetrahydroquinazolin-2-yl]-amino}cyclohexyl)methyl]amino}carbonyl)leucinate;
N-[(cis-4-{[4-(dimethylamino)-5,6,7,8-tetrahydroquinazolin-2-yl]amino}-cyclohexyl)methyl]-N'-(4-fluorophenyl)urea;
N-[(cis-4-{[4-(dimethylamino)-5,6,7,8-tetrahydroquinazolin-2-yl]amino}-cyclohexyl)methyl]-N'-mesitylurea;
N-[(cis-4-{[4-(dimethylamino)-5,6,7,8-tetrahydroquinazolin-2-yl]amino}-cyclohexyl)methyl]-N'-(2,4,6-trichlorophenyl)urea;
N-[(cis-4-{[4-(dimethylamino)-5,6,7,8-tetrahydroquinazolin-2-yl]amino}-cyclohexyl)methyl]-N'-(2,4,6-tribromophenyl)urea;
N-(2,6-diethylphenyl)-N'-[(cis-4-{[4-(dimethylamino)-5,6,7,8-tetrahydroquinazolin-2-yl]amino}cyclohexyl)methyl]urea;

N-[2-chloro-6-(trifluoromethyl)phenyl]-N'-[(cis-4-{[4-(dimethylamino)-5,6,7,8-tetrahydroquinazolin-2-yl]amino} cyclohexyl)methyl]urea;
N-(2-chloro-6-methylphenyl)-N'-[(cis-4-([4-(dimethylamino)-5,6,7,8-tetrahydroquinazolin-2-yl]amino}cyclohexyl)methyl]urea;
N-[(cis-4-{[4-(dimethylamino)-5,6,7,8-tetrahydroquinazolin-2-yl]amino}-cyclohexyl)methyl]-N'-(2-ethyl-6-isopropylphenyl)urea;
N-[(cis-4-{[4-(dimethylamino)-5,6,7,8-tetrahydroquinazolin-2-yl]amino}-cyclohexyl)methyl]-N'-(2-isopropyl-6-methylphenyl)urea;
N-(2-tert-butyl-6-methylphenyl)-N'-[(cis-4-{[4-(dimethylamino)-5,6,7,8-tetrahydroquinazolin-2-yl]amino}cyclohexyl)methyl]urea;
N-(2-tert-butylphenyl)-N'-[(cis-4-{[4-(dimethylamino)-5,6,7,8-tetrahydroquinazolin-2-yl]amino}cyclohexyl)methyl]urea;
N-(3-chloro-2-methylphenyl)-N'-[(cis-4-{[4-(dimethylamino)-5,6,7,8-tetrahydroquinazolin-2-yl]amino}cyclohexyl)methyl]urea;
N-(4-bromo-2,6-dimethylphenyl)-N'-[(cis-4-{[4-(dimethylamino)-5,6,7,8-tetrahydroquinazolin-2-yl]amino}cyclohexyl)methyl]urea;
N-(2,6-diisopropylphenyl)-N'-[(cis-4-([4-(dimethylamino)-5,6,7,8-tetrahydroquinazolin-2-yl]amino}cyclohexyl) methyl]urea;
N-[(cis-4-{[4-(dimethylamino)-5,6,7,8-tetrahydroquinazolin-2-yl]amino}-cyclohexyl)methyl]-N'-(2,3-dimethyl-6-nitrophenyl)urea;
N-(2,6-dibromo-4-fluorophenyl)-N'-[(cis-4-)[4-(dimethylamino)-5,6,7,8-tetrahydroquinazolin-2-yl]amino}cyclohexyl)methyl]urea;
N-(2,6-dichlorophenyl)-N'-[(cis-4-{[4-(dimethylamino)-5,6,7,8-tetrahydroquinazolin-2-yl]amino}cyclohexyl) methyl]urea; and
1-(2,3-dichloro-phenyl)-3-[cis-4-(4-dimethylamino-5,6,7,8-tetrahydro-quinazolin-2-ylamino)-cyclohexylmethyl]-urea;

or a pharmaceutically acceptable salt, hydrate, or solvate thereof.

**46.** The compound according to claim 29 wherein $R_1$ is selected from the group consisting of:

(i) $C_{1-8}$ alkyl, and
$C_{1-8}$ alkyl substituted by substituent(s) independently selected from the group consisting of:

- mono-$C_{1-5}$ alkylamino,
- di-$C_{1-5}$ alkylamino,
- $C_{3-6}$ cycloalkyl,
- $C_{3-6}$ cycloalkenyl,
- carbocyclic aryl,
- carbocyclic aryl substituted by substituent(s) independently selected from the group consisting of:
    - •• halogen,
    - •• $C_{1-5}$ alkyl, and
    - •• $C_{1-5}$ alkoxy,
- heterocyclyl,

(ii) $C_{2-5}$ alkynyl,
(iii) $C_{2-5}$ alkenyl,
(iv) $C_{3-12}$ cycloalkyl,
(v) carbocyclyl,
(vi) carbocyclic aryl, and
carbocyclic aryl substituted by substituent(s) independently selected from the group consisting of:

- halogen,
- cyano,
- nitro,
- $C_{1-10}$ alkyl,

- $C_{1-10}$ alkyl substituted by substituent(s) independently selected from the group consisting of:

  - •• halogen, and
  - •• oxo,

- carboxy,
- $C_{1-5}$ alkoxy carbonyl,
- $C_{1-5}$ alkoxy,
- $C_{1-5}$ alkoxy substituted by substituent(s) independently selected from the group consisting of:

  - •• halogen, and
  - •• carbocyclic aryl,

- carbocyclic aryloxy,
- carbocyclic aryloxy substituted by nitro,
- mono-$C_{1-5}$ alkylamino,
- di-$C_{1-5}$ alkylamino,
- $C_{1-5}$ alkoxy carbonylamino,
- carbocyclic aryl azo,
- carbocyclic aryl azo substituted by substituent(s) independently selected from the group consisting of:

  - •• mono-$C_{1-5}$ alkylamino, and
  - •• di-$C_{1-5}$ alkylamino,

- $C_{1-5}$ alkylthio,
- $C_{1-5}$ alkylthio substituted by halogen,
- carbocyclic arylthio,
- carbocyclic arylthio substituted by nitro,
- amino sulfonyl,
- heterocyclyl sulfonyl,
- $C_{3-6}$ cycloalkyl,
- $C_{3-6}$ cycloalkyl substituted by $C_{1-5}$ alkyl,
- carbocyclic aryl, and
- heterocyclyl,

(vii) heterocyclyl, and
heterocyclyl substituted by substituent(s) independently selected from the group consisting of:

- $C_{1-5}$ alkyl,
- $C_{1-5}$ alkoxy carbonyl,
- carbocyclic aryloxy,
- carbocyclic aryl, and
- heterocyclyl;

L is Formula (VII);
Y is -C(S)$NR_5$-;
wherein carbocyclic aryl is phenyl or naphthyl;
carbocyclyl is indanyl, bicyclo[2.2.1]heptyl, bicyclo[2.2.1]heptenyl, or adamantly;
heterocyclyl is 2,3-dihydro-benzo[1,4]dioxinyl, 4,5,6,7-tetrahydro-benzo[b]thieny], benzo[1,3]dioxolyl, benzo[2,1,3]thiadiazolyl, furyl, isoxazolyl, morpholinyl, oxazolyl, phenanthro[9,10-d]oxazolyl, piperidyl, pyrazolyl, pyridyl, tetrahydrofuryl, or thienyl; and
halogen is fluoro, chloro, bromo, or iodo;

or a pharmaceutically acceptable salt, hydrate, or solvate thereof.

**47.** The compound according to claim 46 wherein $R_2$ is methylamino or dimethylamino; p is 0; $R_3$ and $R_4$ are hydrogen; A is a single bond; B is a single bond or -$CH_2$-: $R_5$ is hydrogen;
or a pharmaceutically acceptable salt, hydrate, or solvate thereof.

**48.** The compound according to claim 47 wherein $R_1$ is selected from the group consisting of:

(i) $C_{1-5}$ alkyl, and
$C_{1-5}$ alkyl substituted by carbocyclic aryl,
(ii) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen,
- $C_{1-5}$ alkoxy,
- $C_{1-5}$ alkoxy substituted by halogen,
- mono-$C_{1-5}$ alkylamino, and
- di-$C_{1-5}$ alkylamino;

wherein carbocyclic aryl is phenyl or naphthyl; and
halogen is fluoro, chloro, bromo, or iodo;

or a pharmaceutically acceptable salt, hydrate, or solvate thereof.

**49.** The compound according to claim 1 selected from the group consisting of:

N-(2,4-dimethoxyphenyl)-N'-(cis-4-{[4-(dimethylamino)-5,6,7,8-tetrahydroquinazolin-2-yl]amino}cyclohexyl) thiourea;
N-(cis-4-{[4-(dimethylamino)-5,6,7,8-tetrahydroquinazolin-2-yl]amino}-cyclohexyl)-N'-(3,4,5-trimethoxyphenyl)thiourea;
N-(3,4-dimethoxyphenyl)-N'-(cis-4-{[4-(dimethylamino)-5,6,7,8-tetrahydroquinazolin-2-yl]amino}cyclohexyl) thiourea;
N-[4-(dimethylamino)-1-naphthyl]-N'-(cis-4-{[4-(dimethylamino)-5,6,7,8-tetrahydroquinazolin-2-yl]amino}cyclohexyl)thiourea;
N-(cis-4-{[4-(dimethylamino)-5,6,7,8-tetrahydroquinazolin-2-yl]amino}-cyclohexyl)-N'-(2-methoxy-5-methylphenyl)thiourea;
N-(4-bromo-2-chlorophenyl)-N'-(cis-4-{[4-(dimethylamino)-5,6,7,8-tetrahydroqninazolin-2-yl]amino}cyclohexyl)thiourea;
N-(cis-4-{[4-(dimethylaniino)-5,6,7,8-tetrahydroquinazolin-2-yl]amino}-cyclohexyl)-N'-(4-iodophenyl)thiourea;
N-(cis-4-{[4-(dimethylamino)-5,6,7,8-tetrahydroquinazolin-2-yl]amino}-cyclohexyl)-N'-(2,4,6-tribromophenyl) thiourea;
N-(cis-4-{[4-(dimethylamino)-5,6,7,8-tetrahydroquinazolin-2-yl]amino}-cyclohexyl)-N'-(2,4,6-trichlorophenyl) thiourea;
N-(cis-4-{[4-(dimethylamino)-5,6,7,8-tetrahydroquinazolin-2-yl]amino}-cyclohexyl)-N'-mesitylthiourea;
N-(cis-4-{[4-(dimethylamino)-5,6,7,8-tetrahydroquinazolin-2-yl]amino}-cyclohexyl)-N'-(2,4-dimethylphenyl) thiourea;
N-(2,6-diethylphenyl)-N'-(cis-4-{[4-(dimethylamino)-5,6,7,8-tetrahydroquinazolin-2-yl]amino}cyclohexyl) thiourea;
N-(4-bromo-2,6-dimethylphenyl)-N'-(cis-4-{[4-(dimethylamino)-5,6,7,8-tetrahydroquinazolin-2-yl]amino}cyclohexyl)thiourea;
N-(4-bromo-2-methylphenyl)-N'-(cis-4-{[4-(dimethylamino)-5,6,7,8-tetrahydroquinazolin-2-yl]amino}cyclohexyl)thiourea;
N-[4-bromo-2-(trifluoromethyl)phenyl]-N'-(cis-4-{[4-(dimethylamino)-5,6,7,8-tetrahydroquinazolin-2-yl]amino} cyclohexyl)thiourea;
N-(4-chloro-2-methylphenyl)-N'-(cis-4-{[4-(dimethylamino)-5,6,7,8-tetrahydroquinazolin-2-yl]amino}cyclohexyl)thiourea;
N-[4-chloro-2-(trifluoromethyl)phenyl]-N'-(cis-4-{[4-(dimethylamino)-5,6,7,8-tetrahydroquinazolin-2-yl]amino} cyclohexyl)thiourea;
N-(cis-4-{[4-(dimethylamino)-5,6,7,8-tetrahydroquinazolin-2-yl]amino}-cyclohexyl)-N'-(4-fluoro-2-methylphenyl)thiourea;
N-(cis-4-{[4-(dimethylamino)-5,6,7,8-tetrahydroquinazolin-2-yl]amino}-cyclohexyl)-N'-(4-methoxy-2-methylphenyl)thiourea;

N-(5-chloro-2,4-dimethoxyphenyl)-N'-(cis-4-{[4-(dimethylamino)-5,6,7,8-tetrahydroquinazolin-2-yl]amino}cyclohexyl)thiourea;
N-(2,4-dibromo-6-fluorophenyl)-N'-(cis-4-{[4-(dimethylamino)-5,6,7,8-tetrahydroquinazolin-2-yl]amino}cyclohexyl)thiourea;
N-(2,4-dichloro-6-methylphenyl)-N'-(cis-4-{[4-(dimethylamino)-5,6,7,8-tetrahydroquinazolin-2-yl]amino}cyclohexyl)thiourea;
N-(cis-4-{[4-(dimethylamino)-5,6,7,8-tetrahydroquinazolin-2-yl]amino}-cyclohexyl)-N'-(2-ethoxyphenyl)thiourea;
N-[4-bromo-2-(trifluoromethoxy)phenyl]-N'-(cis-4-{[4-(dimethylamino)-5,6,7,8-tetrahydroguinazolin-2-yl]amino}cyclohexyl)thiourea;
N-(4-chloro-2,5-dimethoxyphenyl)-N'-(cis-4-{[4-(dimethylamino)-5,6,7,8-tetrahydroquinazolin-2-yl]amino}cyclohexyl)thiourea; and
N-(cis-4-{[4-(dimethylamino)-5,6,7,8-tetrahydroquinazolin-2-yl]amino}-cyclohexyl)-N'-(2,2-diphenylethyl) thiourea;

or a pharmaceutically acceptable salt, hydrate, or solvate thereof.

**50.** The compound according to claim 29 wherein $R_1$ is selected from the group consisting of:

(i) $C_{1-8}$ alkyl, and
$C_{1-8}$ alkyl substituted by substituent(s) independently selected from the group consisting of:

- halogen,
- $C_{1-5}$ alkoxy,
- $C_{1-5}$ alkoxy substituted by carbocyclic aryl,
- carbocyclyl,
- carbocyclic aryl,
- carbocyclic aryl substituted by substituent(s) independently selected from the group consisting of:

  - •• halogen,
  - •• nitro, and
  - •• $C_{1-5}$ alkoxy,

(ii) $C_{2-5}$ alkenyl,
(iii) carbocyclyl,
(iv) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen, and
- $C_{1-5}$ alkoxy;

L is Formula (VII);
Y is -C(O)O-;
wherein carbocyclic aryl is phenyl or naphthyl;
carbocydyl is 9*H*-fluorenyl or menthyl; and
halogen is fluoro, chloro, bromo, or iodo;

or a pharmaceutically acceptable salt, hydrate, or solvate thereof.

**51.** The compound according to claim 50 wherein $R_2$ is methylamino or dimethylamino; p is 0; $R_3$ and $R_4$ are hydrogen; A is a single bond; B is a single bond or $-CH_2-$;
or a pharmaceutically acceptable salt, hydrate, or solvate thereof.

**52.** The compound according to claim 2 wherein Q is Formula (IV); p is 0;
$R_1$ is selected from the group consisting of:

(i) $C_{1-8}$ alkyl, and
$C_{1-8}$ alkyl substituted by substituent(s) independently selected from the group consisting of:

- halogen,
- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- carbocyclic aryloxy substituted by $C_{1-5}$ alkoxy,
- heterocyclyloxy,
- heterocyclyloxy substituted by $C_{1-5}$ alkyl,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylamino,
- mono-$C_{1-5}$ alkylamino substituted by cyano,
- mono-$C_{1-5}$ alkylamino substituted by carbocyclic aryl,
- di-$C_{1-5}$ alkylamino,
- di-$C_{1-5}$ alkylamino substituted by cyano,
- di-$C_{1-5}$ alkylamino substituted by carbocyclic aryl,
- mono-carbocyclic arylamino,
- mono-carbocyclic arylamino substituted by $C_{1-5}$ alkyl,
- di-carbocyclic arylamino,
- di-carbocyclic arylamino substituted by $C_{1-5}$ alkyl,
- $C_{1-5}$ alkoxycarbonylamino,
- carbocyclic arylcarbonylamino,
- $C_{1-5}$ alkylthio,
- $C_{1-5}$ alkylthio substituted by substituent(s) independently selected from the group consisting of:

  - •• carbocyclic aryl,
  - •• carbocyclic aryl substituted by halogen, and
  - •• carbocyclic aryl substituted by $C_{1-5}$ alkoxy,

- carbocyclic arylthio,
- heterocyclylthio,
- heterocyelylthio substituted by nitro,
- heterocyclylthio substituted by $C_{1-5}$ alkyl,
- $C_{3-6}$ cycloalkyl,
- $C_{3-6}$ cycloalkenyl,
- carbocyclyl,
- carbocyclyl substituted by substituent(s) independently selected from the group consisting of:

  - •• halogen,
  - •• $C_{1-5}$ alkyl,
  - •• $C_{1-5}$ alkoxy,
  - •• $C_{2-5}$ alkenyl, and
  - •• $C_{2-5}$ alkenyl substituted by substituent(s) independently selected from the group consisting of:

    - ••• carbocyclic aryl, and
    - ••• carbocyclic aryl substituted by $C_{1-5}$ alkylsulfinyl,

- carbocyclic aryl,
- carbocyclic aryl substituted by substituent(s) independently selected from the group consisting of:

  - •• halogen,

- •• hydroxy,
- •• nitro,
- •• $C_{1-5}$ alkyl,
- •• $C_{1-5}$ alkyl substituted by substituent(s) independently selected from the group consisting of:
  - ••• oxo,
  - ••• carbocyclic aryl, and
  - ••• heterocyclyl,
- •• $C_{2-5}$ alkenyl,
- •• $C_{1-5}$ alkoxy,
- •• $C_{1-5}$ alkoxy substituted by halogen,
- •• $C_{1-5}$ alkoxy substituted by carbocyclic aryl,
- •• carbocyclic aryloxy,
- •• carbocyclic aryl, and
- •• heterocyclyl,

- heterocyclyl, and
- heterocyclyl substituted by substituent(s) independently selected from the group consisting of:
  - •• $C_{1-5}$ alkyl,
  - •• $C_{1-5}$ alkyl substituted by carbocyclic aryl,
  - •• $C_{1-5}$ alkoxy,
  - •• $C_{1-5}$ alkoxy substituted by carbocyclic aryl,
  - •• carbocyclic aryl, and
  - •• carbocyclic aryl substituted by halogen,

(ii) $C_{2-7}$ alkenyl, and
$C_{2-7}$ alkenyl substituted by substituent(s) independently selected from the group consisting of:

- carbocyclic aryl,
- carbocyclic aryl substituted by substituent(s) independently selected from the group consisting of:
  - •• halogen,
  - •• nitro, and
  - •• $C_{1-5}$ alkoxy,

(iii) $C_{2-5}$ alkynyl, and
$C_{2-5}$ alkynyl substituted by carbocyclic aryl,
(iv) $C_{3-6}$ cycloalkyl, and
$C_{3-6}$ cycloalkyl substituted by substituent(s) independently selected from the group consisting of:

- $C_{1-5}$ alkyl,
- $C_{1-5}$ alkyl substituted by oxo,
- $C_{1-5}$ alkyl substituted by carbocyclic aryl, and
- carbocyclic aryl,

(v) carbocyclyl,
(vi) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:

- •• halogen,
- •• oxo,
- •• carbocyclic aryloxy,
- •• carbocyclic aryl, and
- •• carbocyclic aryl substituted by $C_{1-5}$ alkyl,

- $C_{1-5}$ alkoxy,
- $C_{1-5}$ alkoxy substituted by substituent(s) independently selected from the group consisting of:

  - •• halogen,
  - •• carbocyclic aryl, and
  - •• halogenated carbocyclic aryl,

- $C_{2-5}$ alkenyloxy,
- $C_{3-6}$ cycloalkoxy,
- carbocyclic aryloxy,
- Carbocyclic aryloxy substituted by $C_{1-5}$ alkoxy,
- $C_{1-5}$ alkoxycarbonyl,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,
- mono-$C_{1-5}$ alkylaminosulfonyl,
- di-$C_{1-5}$ alkylaminosulfonyl,
- carbocyclic aryl,
- heterocyclyl, and
- heterocyclyl substituted by substituent(s) independently selected from the group consisting of:

  - •• $C_{1-5}$ alkyl,
  - •• carbocyclic aryl, and
  - •• halogenated carbocyclic aryl,

(vii) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:

  - •• halogen,
  - •• hydroxy,
  - •• $C_{1-5}$ alkylthio,
  - •• $C_{1-5}$ alkylthio substituted by carbocyclic aryl,

- - $C_{1-5}$ alkylthio substituted by halogenated carbocyclic aryl,
  - carbocyclic aryl,
  - carbocyclic aryl substituted by halogen, and
  - heterocyclyl,

- $C_{1-5}$ alkoxy,
- carbocyclic aryloxy,
- carbocyclic aryloxy substituted by $C_{1-5}$ alkyl,
- $C_{1-5}$ alkylthio,
- $C_{2-5}$ alkenylthio,
- carbocyclic arylthio,
- carbocyclic arylthio substituted by $C_{1-5}$ alkoxycarbonyl,
- $C_{1-5}$ alkylsulfonyl,
- carbocyclic arylsulfonyl,
- carbocyclic arylsulfonyl substituted by $C_{1-5}$ alkyl,
- $C_{1-5}$ alkoxycarbonyl,
- carbocyclic aryl,
- carbocyclic aryl substituted by substituent(s) independently selected from the group consisting of:

  - halogen,
  - nitro,
  - $C_{1-5}$ alkyl, and
  - $C_{1-5}$ alkyl substituted by halogen,

- heterocyclyl;

wherein carbocyclic aryl is phenyl, naphthyl, or anthranyl;

carbocyclyl is 1,2,3,4-tetrahydronaphthyl, 1-oxo-indanyl, 9-fluorenyl, 9-oxo-9*H*-fluorenyl, bicyclo[2.2.1] heptyl, indenyl, or menthyl;

heterocyclyl is 1,2,3-triazolyl, 1*H*-indolyl, 1*H*-pyrrolyl, 2,3-dihydro-1-oxo-isoindolyl, 2,3-dihydro-benzo [1,4]dioxinyl, 2,3-dihydro-benzofuryl, 2,4-dihydro-3-oxo-pyrazolyl, 2*H*-benzopyranyl, 2-oxo-benzopyranyl, 3,4-dihydro-2*H*-benzo[b][1,4]dioxepinyl, 4-oxo-1,5,6,7-tetrahydro-indolyl, 4-oxo-benzopyranyl, 9*H*-carbazolyl, 9*H*-xanthenyl, benzo[1,3]dioxolyl, benzo[2,1,3]oxadiazolyl, benzo[1,2,5]oxadiazolyl, benzo[b]thienyl, benzofuryl, benzothiazolyl, furyl, imidazo[2,1-b]thiazolyl, imidazolyl, isoxazolyl, morpholino, pyrazolyl, pyridyl, pyrimidyl, quinolyl, quinoxalyl, thiazolyl, or thienyl; and

halogen is fluoro, chloro, bromo, or iodo;

or a pharmaceutically acceptable salt, hydrate, or solvate thereof.

**53.** The compound according to claim 52 wherein $R_1$ is selected from the group consisting of:

(i) $C_{1-7}$ alkyl, and
$C_{1-7}$ alkyl substituted by substituent(s) independently selected from the group consisting of:

- $C_{1-5}$ alkoxy,
- $C_{1-5}$ alkoxy substituted by carbocyclic aryl,
- carbocyclic aryloxy,
- carbocyclic aryloxy substituted by halogen,
- mono-$C_{1-5}$ alkylamino,
- mono-$C_{1-5}$ alkylamino substituted by substituent(s) independently selected from the group consisting of:

  - cyano, and
  - carbocyclic aryl,

- di-$C_{1-5}$ alkylamino,
- di-$C_{1-5}$ alkylamino substituted by substituent(s) independently selected from the group consisting of:

  - cyano, and

  - carbocyclic aryl,

- mono-carbocyclic arylamino,
- di-carbocyclic arylamino,
- mono-carbocyclic arylamino substituted by $C_{1-5}$ alkyl,
- di-carbocy$_d$ic arylamino substituted by $C_{1-5}$ alkyl,
- carbocyclic aryl,
- carbocyclic aryl substituted by substituent(s) independently selected from the group consisting of:

  - halogen,
  - $C_{1-5}$ alkyl, and
  - $C_{1-5}$ alkoxy,

(ii) $C_{2-7}$ alkenyl, and
$C_{2-7}$ alkenyl substituted by substituent(s) independently selected from the group consisting of:

- carbocyclic aryl, and
- carbocyclic aryl substituted by $C_{1-5}$ alkoxy,

(iii) $C_{2-5}$ alkynyl, and
$C_{2-5}$ alkynyl substituted by carbocyclic aryl,
(iv) carbocyclic aryl, and
carbocyclic aryl substituted by substituent(s) independently selected from the group consisting of:

- halogen,
- hydroxy,
- cyano,
- $C_{1-5}$ alkyl,
- $C_{1-5}$ alkyl substituted by halogen,
- $C_{1-5}$ alkoxy,
- $C_{1-5}$ alkoxy substituted by substituent(s) independently selected from the group consisting of:

  - halogen,
  - carbocyclic aryl, and
  - carbocyclic aryl substituted by halogen,

- $C_{2-5}$ alkenyloxy,
- mono-$C_{1-5}$ alkylamino,
- di-$C_{1-5}$ alkylamino,
- mono-$C_{1-5}$ alkylamino substituted by cyano,
- di-$C_{1-5}$ alkylamino substituted by cyano,
- $C_{1-5}$ alkylthio, and
- $C_{1-5}$ alkylthio substituted by halogen,

(v) heterocyclyl, and
heterocyclyl substituted by substituent(s) independently selected from the group consisting of:

- halogen,
- $C_{1-5}$ alkyl,
- $C_{1-5}$ alkyl substituted by hydroxy,
- $C_{1-5}$ alkoxy,
- carbocyclic arylthio,
- carbocyclic arylthio substituted by $C_{1-5}$ alkoxycarbonyl,
- $C_{1-5}$ alkoxycarbonyl,
- carbocyclic aryl,
- carbocyclic aryl substituted by substituent(s) independently selected from the group consisting of:

  - halogen,

- •• $C_{1-5}$ alkyl, and
- •• $C_{1-5}$ alkyl substituted by halogen;

L is Formula (VII);
Y is a single bond or $-CH_2-$;
wherein carbocyclic aryl is phenyl or naphthyl;
heterocyclyl is 1*H*-indolyl, 1*H*-pyrrolyl, 2,3-dihydro-benzo[1,4]dioxinyl, 4-oxo-benzopyranyl, 9*H*-carbazolyl, benzo[1,3]dioxolyl, benzo[b]thienyl, furyl, imidazo[2,1-b]thiazolyl, pyrazolyl, pyridyl, or thienyl; and
halogen is fluoro, chloro, bromo, or iodo;

or a pharmaceutically acceptable salt, hydrate, or solvate thereof.

**54.** The compound according to claim 53 wherein $R_2$ is methylamino, or dimethylamino; p is 0; $R_3$ and $R_4$ are hydrogen; A is a single bond; B is a single bond or $-CH_2-$;
or a pharmaceutically acceptable salt, hydrate, or solvate thereof.

**55.** The compound according to claim 54 wherein $R_1$ is selected from the group consisting of:

(i) $C_{2-5}$ alkenyl, and
$C_{2-5}$ alkenyl substituted by carbocyclic aryl,
(ii) carbocyclic aryl, and
carbocyclic aryl substituted by substituent(s) independently selected from the group consisting of:

- halogen,
- hydroxy,
- $C_{1-5}$ alkyl,
- $C_{1-5}$ alkoxy,
- $C_{1-5}$ alkoxy substituted by substituent(s) independently selected from the group consisting of:

  - •• halogen,
  - •• carbocyclic aryl, and
  - •• carbocyclic aryl substituted by halogen,

- $C_{2-5}$ alkenyloxy,
- mono-$C_{1-5}$ alkylamino,
- di-$C_{1-5}$ alkylamino,
- mono-$C_{1-5}$ alkylamino substituted by cyano, and
- di-$C_{1-5}$ alkylamino substituted by cyano,

(iii) heterocyclyl, and
heterocyclyl substituted by substituent(s) independently selected from the group consisting of:

- halogen,
- $C_{1-5}$ alkyl,
- $C_{1-5}$ alkoxy,
- $C_{1-5}$ alkoxycarbonyl,
- carbocyclic aryl,
- carbocyclic aryl substituted by substituent(s) independently selected from the group consisting of:

  - •• halogen,
  - •• $C_{1-5}$ alkyl, and
  - •• $C_{1-5}$ alkyl substituted by halogen;

wherein carbocyclic aryl is phenyl or naphthyl;
heterocyclyl is 1*H*-indolyl, 9*H*-carbazolyl, benzo[1,3]dioxolyl, pyrazolyl, or pyridyl; and
halogen is fluoro, chloro, bromo, or iodo;

or a pharmaceutically acceptable salt, hydrate, or solvate thereof.

**56.** The compound according to claim 55 wherein $R_1$ is selected from the group consisting of:

(i) $C_{2-5}$ alkenyl, and
$C_{2-5}$ alkenyl substituted by carbocyclic aryl,
(ii) carbocyclic aryl, and
carbocyclic aryl substituted by substituent(s) independently selected from the group consisting of:

- halogen,
- hydroxy,
- $C_{1-5}$ alkyl,
- $C_{1-5}$ alkoxy,
- $C_{1-5}$ alkoxy substituted by halogen,
- $C_{2-5}$ alkenyloxy,

(iii) heterocyclyl, and
heterocyclyl substituted by substituent(s) independently selected from the group consisting of:

- halogen,
- $C_{1-5}$ alkyl,
- $C_{1-5}$ alkoxy,
- $C_{1-5}$ alkoxycarbonyl,
- carbocyclic aryl,
- carbocyclic aryl substituted by $C_{1-5}$ alkyl, and
- carbocyclic aryl substituted by halogenated $C_{1-5}$ alkyl;

wherein carbocyclic aryl is phenyl or naphthyl;
heterocyclyl is 1*H*-indolyl, 9*H*-carbazolyl, benzo[1,3]dioxolyl, or pyrazolyl; and
halogen is fluoro, chloro, bromo, or iodo;

or a pharmaceutically acceptable salt, hydrate, or solvate thereof.

**57.** The compound according to claim 1 selected from the group consisting of:

$N^2$-(cis-4-{[(5-bromo-1H-indol-3-yl)methyl]amino}cyclohexyl)-$N^4$,$N^4$-dimethylpyrimidine-2,4-diamine;
$N^2$-[cis-4-({[5-(4-fluorophenyl)pyridin-3-yl]methyl}amino)cyclohexyl]-$N^4$,$N^4$-dimethylpyrimidine-2,4-diamine;
ethyl 4,6-dichloro-3-{[(cis-4-{[4-(dimethylamino)pyrimidin-2-yl]amino}-cyclohexyl)amino]methyl}-1H-indole-2-carboxylate;
$N^2$-(cis-4-{[(2,6-dimethoxybenzyl)amino]methyl}cyclohexyl)-$N^4$,$N^4$-dimethylpyrimidine-2,4-diamine;
$N^2$-(cis-4-{[(2-ethoxybenzyl)amino]methyl}cyclohexyl)-$N^4$,$N^4$-dimethylpyrimidine-2,4-diamine;
$N^2$-[cis-4-({[(4-methoxy-1-naphthyl)methyl]amino}methyl)cyclohexyl]-$N^4$,$N^4$-dimethylpyrimidine-2,4-diamine;
$N^2$-[cis-4-({[(5-methoxy-1H-indol-3-yl)methyl]amino}methyl)cyclohexyl]-$N^4$,$N^4$-dimethylpyrimidine-2,4-diamine;
$N^2$-[cis-4-({[(2-methoxy-1-naphthyl)methyl]amino}methyl)cyclohexyl]-$N^4$,$N^4$-dimethylpyrimidine-2,4-diamine;
4-bromo-2-({[(cis-4-{[4-(dimethylamino)pyrimidin-2-yl]amino}-cyclohexyl)methyl]amino}methyl)-6-methoxyphenol;
$N^2$-[cis-4-({[(5-bromo-1H-indol-3-yl)methyl]amino}methyl)cyclohexyl]-$N^4$,$N^4$-dimethylpyrimidine-2,4-diamine;
$N^2$-(cis-4-{[(2,4-dimethoxybenzyl)amino]methyl}cyclohexyl)-$N^4$,$N^4$-dimethylpyrimidine-2,4-diamine;
$N^4$,$N^4$-dimethyl-$N^2$-(cis-4-{[(2,3,4-trimethoxybenzyl)amino]methyl)-cyclohexyl)pyrimidine-2,4-diamine;
$N^2$-(cis-4-{[(3-ethoxy-4-methoxybenzyl)amino]methyl}cyclohexyl)-$N^4$,$N^4$-dimethylpyrimidine-2,4-diamine;
$N^4$,$N^4$-dimethyl-$N^2$-(cis-4-{[({3-[4-(trifluoromethyl)phenyl]-1H-pyrazol-4-yl}methyl)amino]methyl}cyclohexyl)pyrimidine-2,4-diamine;
$N^4$,$N^4$-dimethyl-$N^2$-(cis-4-{[(3,4,5-trimethoxybenzyl)amino]methyl}-cyclohexyl)pyrimidine-2,4-diamine;
4-({[(cis-4-{[4-(dimethylamino)pyrimidin-2-yl]amino}cyclohexyl)-methyl]amino}methyl)-2-iodo-6-methoxyphenol;
4-({[(cis-4-{[4-(dimethylamino)pyrimidin-2-yl]amino}cyclohexyl)methyl]-amino}methyl)-2,6-dimethylphenol;
$N^2$-(cis-4-{[(5-bromo-2,4-dimethoxybenzyl)amino]methyl}cyclohexyl)-$N^4$,$N^4$-dimethylpyrimidine-2,4-diamine;
$N^2$-(cis-4-{[(5-bromo-2-methoxybenzyl)amino]methyl}cyclohexyl)-$N^4$,$N^4$-dimethylpyrimidine-2,4-diamine;
$N^2$-[cis-4-({[4-(diethylamino)benzyl]amino}methyl)cyclohexyl]-$N^4$,$N^4$-dimethylpyrimidine-2,4-diamine;

$N^2$-[cis-4-({[(9-ethyl-9H-carbazol-3-yl)methyl]amino}methyl)cyclohexyl]-$N^4$,$N^4$-dimethylpyrimidine-2,4-diamine;
$N^2$-(cis-4-{[(4-isopropoxybenzyl)amino]methyl}cyclohexyl)-$N^4$,$N^4$-dimethylpyrimidine-2,4-diamine;
$N^2$-(cis-4-{[(3,3-diphenylprop-2-en-1-yl)amino]methyl}cyclohexyl)-$N^4$,$N^4$-dimethylpyrimidine-2,4-diamine;
4-({[cis-4-{[4-(dimethylamino)pyrimidin-2-yl]amino}cyclohexyl)methyl]-amino}methyl)-2-ethoxyphenol;
$N^2$-{cis-4-[({[4-(dimethylamino)-1-naphthyl]methyl}amino)methyl]-cyclohexyl}-$N^4$,$N^4$-dimethylpyrimidine-2,4-diamine;
$N^4$,$N^4$-dimethyl-$N^2$-(cis-4-{[(2,4,6-trimethoxybenzyl)amino]methyl}-cyclohexyl)pyrimidine-2,4-diamine;
$N^2$-(cis-4-{[(5-bromo-2-ethoxybenzyl)amino]methyl}cyclohexyl)-$N^4$,$N^4$-dimethylpyrimidine-2,4-diamine;
$N^2$-(cis-4-{[(2,4-dimethoxy-3-methylbenzyl)amino]methyl}cyclohexyl)-$N^4$,$N^4$-dimethylpyrimidine-2,4-diamine;
$N^2$-(cis-4-{[(2,5-diethoxybenzyl)amino]methyl}cyclohexyl)-$N^4$,$N^4$-dimethylpyrimidine-2,4-diamine;
$N^2$-(cis-4-{[(2,4-diethoxybenzyl)amino]methyl}cyclohexyl)-$N^4$,$N^4$-dimethylpyrimidine-2,4-diamine;
$N^2$-(cis-4-{[(3,5-dibromo-2-methoxybenzyl)amino]methyl}cyclohexyl)-$N^4$,$N^4$-dimethylpyrimidine-2,4-diamine;
$N^4$,$N^4$-dimethyl-$N^2$-(cis-4-{[(2,4,5-triethoxybenzyl)amino]methyl}-cyclohexyl)pyrimidine-2,4-diamine;
$N^4$,$N^4$-dimethyl-$N^2$-(cis-4-{[(2,4,5-trimethoxybenzyl)amino]methyl}-cyclohexyl)pyrimidine-2,4-diamine;
$N^2$-[cis-4-({[2-(allyloxy)benzyl]amino}methyl)cyclohexyl]-$N^4$,$N^4$-dimethylpyrimidine-2,4-diamine;
$N^4$,$N^4$-dimethyl-$N^2$-[cis-4-({[(1-methyl-1H-indol-3-yl)methyl]amino}-methyl)cyclohexyl]pyrimidine-2,4-diamine;
$N^2$-[cis-4-({[(7-methoxy-1,3-benzodioxol-5-yl)methyl]amino}methyl)-cyclohexyl]-$N^4$,$N^4$-dimethylpyrimidine-2,4-diamine;
$N^2$-(cis-4-{[(3-bromo-4,5-dimethoxybenzyl)amino]methyl}cyclohexyl)-$N^4$,$N^4$-dimethylpyrimidine-2,4-diamine;
$N^2$-(cis-4-{[(4-methoxy-3-methylbenzyl)amino]methyl}cyclohexyl)-$N^4$,$N^4$-dimethylpyrimidine-2,4-diamine;
$N^2$-(cis-4-{[(2-bromo-4,5-dimethoxybenzyl)amino]methyl}cyclohexyl)-$N^4$,$N^4$-dimethylpyrimidine-2,4-diamine;
$N^2$-(cis-4-{[(3,4-dimethoxybenzyl)amino]methyl}cyclohexyl)-$N^4$,$N^4$-dimethylpyrimidine-2,4-diamine;
$N^2$-(cis-4-{[(4-methoxy-2,5-dimethylbenzyl)amino]methyl}cyclohexyl)-$N^4$,$N^4$-dimethylpyrimidine-2,4-diamine;
3-[[4-({[(cis-4-{[4-(dimethylamino)pyrimidin-2-yl]amino}cyclohexyl)-methyl]amino}methyl)phenyl](methyl)amino]propanenitrile ;
$N^2$-{cis-4-[({4-[(4-bromobenzyl)oxy]benzyl}amino)methyl]cyclohexyl}-$N^4$,$N^4$-dimethylpyrimidine-2,4-diamine;
$N^2$-(cis-4-{[(3,5-dibromo-2-ethoxybenzyl)amino]methyl}cyclohexyl)-$N^4$,$N^4$-dimethylpyrimidine-2,4-diamine;
$N^2$-[4-(4-bromo-2-trifluoromethoxy-benzyl)amino-cyclohexyl]-$N^4$,$N^4$-dimethyl-pyrimidine-2,4-diamine;
$N^2$-{cis-4-[2-(4-bromo-2-trifluoromethoxy-phenyl)-ethylamino]-cyclohexyl}-$N^4$,$N^4$-dimethyl-pyrimidine-2,4-diamine ; and
$N^2$-{cis-4-[(4-bromo-2-triftuoromethoxy-benzyl)amino-methyl]-cyclohexyl}-$N^4$,$N^4$-dimethyl-pyrimidine-2,4-diamine;

or a pharmaceutically acceptable salt, hydrate, or solvate thereof.

**58.** The compound according to claim 57 selected from the group consisting of:

ethyl 4,6-dichloro-3-{[(cis-4-{[4-(dimethylamino)pyrimidin-2-yl]amino}-cyclohexyl)amino]methyl}-1H-indole-2-carboxylate;
$N^2$-[cis-4-({[(4-methoxy-1-naphthyl)methyl]amino}methyl)cyclohexyl]-$N^4$,$N^4$-dimethylpyrimidine-2,4-diamine;
$N^2$-[cis-4-({[(2-methoxy-1-naphthyl)methyl]amino}methyl)cyclohexyl]-$N^4$,$N^4$-dimethylpyrimidine-2,4-diamine;
4-bromo-2-({[(cis-4-{[4-(dimethylamino)pyrimidin-2-yl]amino}-cyclohexyl)methyl]amino}methyl)-6-methoxyphenol;
$N^2$-[cis-4-({[(5-bromo-1H-indol-3-yl)methyl]amino}methyl)cyclohexyl]-$N^4$,$N^4$-dimethylpyrimidine-2,4-diamine;
$N^4$,$N^4$-dimethyl-$N^2$-(cis-4-{[(2,3,4-trimethoxybenzyl)amino]methyl]-cyclohexyl)pyrimidine-2,4-diamine;
$N^2$-(cis-4-{[(3-ethoxy-4-methoxybenzyl)amino]methyl}cyclohexyl)-$N^4$,$N^4$-dimethylpyrimidine-2,4-diamine;
$N^4$,$N^4$-dimethyl-$N^2$-(cis-4-{[({3-[4-(trifluoromethyl)phenyl]-1H-pyrazol-4-yl)}methyl)amino]methyl}cyclohexyl) pyrimidine-2,4-diamine;
4-({[(cis-4-{[4-(dimethylamino)pyrimidin-2-yl]amino}cyclohexyl)methyl]-amino}methyl)-2-iodo-6-methoxyphenol;
4-({[(cis-4-{[4-(dimethylamino)pyrimidin-2-yl]amino}cyclohexyl)methyl]-amino}methyl)-2,6-dimethylphenol;
$N^2$-(cis-4-{[(5-bromo-2,4-dimethoxybenzyl)amino]methyl}cyclohexyl)-$N^4$,$N^4$-dimethylpyrimidine-2,4-diamine;
$N^2$-(cis-4-{[(5-bromo-2-methoxybenzyl)amino]methyl}cyclohexyl)-$N^4$,$N^4$-dimethylpyrimidine-2,4-diamine;
$N^2$-[cis-4-({[(9-ethyl-9H-carbazol-3-yl)methyl]amino}methyl)cyclohexyl]-$N^4$,$N^4$dimethylpyrimidine-2,4-diamine;
$N^2$-(cis-4-{[(3,3-diphenylprop-2-en-1-yl)amino]methyl}cyclohexyl)-$N^4$,$N^4$dimethylpyrimidine-2,4-diamine;

$N^4$,$N^4$-dimethyl-$N^2$-(cis-4-{[(2,4,6-trimethoxybenzyl)amino]methyl}cyclohexyl)pyrimidine-2,4-diamine;
$N^2$-(cis-4-{[(5-bromo-2-ethoxybenzyl)amino]methyl}cyclohexyl)-$N^4$,$N^4$dimethylpyrimidine-2,4-diamine;
$N^2$-(cis-4-{[(2,4-dimethoxy-3-methylbenzyl)amino]methyl}cyclohexyl)-$N^4$,$N^4$dimethylpyrimidine-2,4-diamine;
$N^2$-(cis-4-{[(2,5-diethoxybenzyl)amino]methyl}cyclohexyl)-$N^4$,$N^4$dimethylpyrimidine-2,4-diamine;
$N^2$-(cis-4-{[(3,5-dibromo-2-methoxybenzyl)amino]methyl}cyclohexyl)-$N^4$,$N^4$-dimethylpyrimidine-2,4-diamine;
$N^4$,$N^4$-dimethyl-$N^2$-(cis-4-{[(2,4,5-triethoxybenzyl)amino]methyl}cyclohexyl)pyrimidine-2,4-diamine;
$N^2$-[cis-4-({[2-(allyloxy)benzyl]amino}methyl)cyclohexyl]-$N^4$,$N^4$dimethylpyrimidine-2,4-diamine;
$N^2$-[cis-4-({[(7-methoxy-1,3-benzodioxol-5-yl)methyl]amino}methyl)-cyclohexyl]-$N^4$,$N^4$-dimethylpyrimidine-2,4-diamine;
$N^2$-(cis-4-{[(3-bromo-4,5-dimethoxybenzyl)amino]methyl}cyclohexyl)-$N^4$,$N^4$dimethylpyrimidine-2,4-diamine;
$N^2$-{cis-4-[2-(4-bromo-2-trifluoromethoxy-phenyl)-ethylamino]-cyclohexyl}-$N^4$,$N^4$-dimethyl-pyrimidine-2,4-diamine; and
$N^2$-{cis-4-[(4-bromo-2-trifluoromethoxy-benzyl)amino-methyl]-cyclohexyl}-$N^4$,$N^4$-dimethyl-pyrimidine-2,4-diamine;

or a pharmaceutically acceptable salt, hydrate, or solvate thereof.

**59.** The compound according to claim 52 wherein $R_1$ is selected from the group consisting of:

(i) $C_{1-5}$ alkyl, and
$C_{1-5}$ alkyl substituted by substituent(s) independently selected from the group consisting of:

- oxo,
- $C_{1-5}$ alkoxy,
- $C_{1-5}$ alkoxy substituted by carbocyclic aryl,
- $C_{1-5}$ alkylcarbonyloxy,
- carbocyclic aryloxy,
- carbocyclic aryloxy substituted by halogen,
- carbocyclic aryloxy substituted by nitro,
- carbocyclic aryloxy substituted by $C_{1-5}$ alkoxy,
- heterocyclyloxy,
- heterocyclyloxy substituted by $C_{1-5}$ alkyl,
- mono-$C_{1-5}$ alkylaminocarbonyl,
- di-$C_{1-5}$ alkylaminocarbonyl,
- mano-$C_{1-5}$ alkylamino,
- di-$C_{1-5}$ alkylamino,
- Mono-carbocyclic arylamino,
- di-carbocyclic arylamino,
- mono-carbocyclic arylamino substituted by halogen,
- di-carbocyclic arylamino substituted by halogen,
- carbocyclic arylcarbonylamino,
- $C_{1-5}$ alkoxycarbonylamino,
- $C_{1-5}$ alkylthio,
- $C_{1-5}$ alkylthio substituted by substituent(s) independently selected from the group consisting of:
    - •• carbocyclic aryl, and
    - •• carbocyclic aryl substituted by substituent(s) independently selected from the group consisting of:
        - ••• halogen, and
        - ••• $C_{1-5}$ alkoxy,
- carbocyclic arylthio,
- heterocyclylthio,
- heterocyclylthio substituted by $C_{1-5}$ alkyl,
- heterocyclylthio substituted by nitro,
- $C_{3-6}$ cycloalkyl,
- $C_{3-6}$ cycloalkenyl,
- carbocyclyl,

- carbocyclyl substituted by substituent(s) independently selected from the group consisting of:

  - •• halogen,
  - •• $C_{1-5}$ alkyl,
  - •• $C_{1-5}$ alkoxy,
  - •• $C_{2-5}$ alkenyl, and
  - •• $C_{2-5}$ alkenyl substituted by substituent(s) independently selected from the group consisting of:

    - ••• carbocyclic aryl, and
    - ••• carbocyclic aryl substituted by $C_{1-5}$ alkylsulfinyl,

- carbocyclic aryl,
- carbocyclic aryl substituted by substituent(s) independently selected from the group consisting of:

  - •• halogen,
  - •• hydroxy,
  - •• nitro,
  - •• $C_{1-5}$ alkyl,
  - •• $C_{1-5}$ alkyl substituted by substituent(s) independently selected from the group consisting of:

    - ••• oxo,
    - ••• carbocyclic aryl, and
    - ••• heterocyclyl,

  - •• $C_{1-5}$ alkoxy,
  - •• $C_{1-5}$ alkoxy substituted by halogen,
  - •• $C_{1-5}$ alkoxy substituted by carbocyclic aryl,
  - •• carbocyclic aryloxy,
  - •• carbocyclic aryl, and
  - •• heterocyclyl,

- heterocyclyl, and
- heterocyclyl substituted by substituent(s) independently selected from the group consisting of:

  - •• $C_{1-5}$ alkyl,
  - •• $C_{1-5}$ alkyl substituted by carbocyclic aryl,
  - •• $C_{1-5}$ alkoxy,
  - •• $C_{1-5}$ alkoxy substituted by carbocyclic aryl,
  - •• carbocyclic aryl, and
  - •• carbocyclic aryl substituted by halogen,

(ii) $C_{2-5}$ alkenyl, and
$C_{2-5}$ alkenyl substituted by substituent(s) independently selected from the group consisting of:

- carbocyclic aryl,
- carbocyclic aryl substituted by substituent(s) independently selected from the group consisting of:

  - •• halogen, and
  - •• nitro,

(iii) $C_{3-6}$ cycloalkyl, and
$C_{3-6}$ cycloalkyl substituted by substituent(s) independently selected from the group consisting of:

- $C_{1-5}$ alkyl,
- $C_{1-5}$ alkyl substituted by substituent(s) independently selected from the group consisting of:

  - •• oxo, and
  - •• carbocyclic aryl, and

- carbocyclic aryl,

(iv) carbocyclyl,
(v) carbocyclic aryl, and
carbocyclic aryl substituted by substituent(s) independently selected from the group consisting of:

- halogen,
- hydroxy,
- cyano,
- nitro,
- $C_{1-5}$ alkyl,
- $C_{1-5}$ alkyl substituted by substituent(s) independently selected from the group consisting of:
    - •• halogen,
    - •• oxo,
    - •• carbocyclic aryloxy,
    - •• carbocyclic aryl, and
    - •• carbocyclic aryl substituted by $C_{1-5}$ alkyl,
- $C_{1-5}$ alkoxy,
- $C_{1-5}$ alkoxy substituted by substituent(s) independently selected from the group consisting of:
    - •• halogen, and
    - •• carbocyclic aryl,
- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- amino,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- (carbocyclic aryl)NHC(O)NH,
- (carbocyclic aryl)NHC(O)NH substituted by $C_{1-5}$ alkoxy,
- (carbocyclic aryl)NHC(O)NH substituted by haloganated $C_{1-5}$ alkoxy,
- $C_{1-5}$ alkylthio,
- $C_{1-5}$ alkylthio substituted by halogen,
- carbocyclic arylthio,
- carbocyclic arylthio substituted by cyano,
- mono-$C_{1-5}$ alkylaminosulfonyl,
- di-$C_{1-5}$ alkylaminosulfonyl, and
- carbocyclic aryl,
- carbocyclic aryl substituted by halogen,
- heterocyclyl, and
- heterocyclyl substituted by substituent(s) independently selected from the group consisting of:
    - •• $C_{1-5}$ alkyl,
    - •• carbocyclic aryl, and
    - •• halogenated carbocyclic aryl,

(vi) heterocyclyl, and
heterocyclyl substituted by substituent(s) independently selected from the group consisting of:

- halogen,

- nitro,
- $C_{1-5}$ alkyl,
- $C_{1-5}$ alkyl substituted by substituent(s) independently selected from the group consisting of:

  - •halogen,
  - • $C_{1-5}$ alkylthio,
  - •$C_{1-5}$ alkylthio substituted by carbocyclic aryl,
  - •$C_{1-5}$ alkylthio substituted by halogenated carbocyclic aryl,
  - • carbocyclic aryl,
  - •carbocyclic aryl substituted by halogen, and
  - • heterocyclyl,

- $C_{1-5}$ alkoxy,
- carbocyclic aryloxy,
- carbocyclic aryloxy substituted by halogen,
- carbocyclic aryloxy substituted by $C_{1-5}$ alkyl,
- $C_{1-5}$ alkylthio,
- $C_{2-5}$ alkenylthio,
- carbocyclic arylthio,
- $C_{1-5}$ alkylsulfonyl,
- carbocyclic arylsulfonyl,
- carbocyclic arylsulfonyl substituted by $C_{1-5}$ alkyl,
- carbocyclic aryl,
- carbocyclic aryl substituted by substituent(s) independently selected from the group consisting of:

  - •• halogen,
  - •• nitro, and
  - •• $C_{1-5}$ alkyl,

- heterocyclyl;

L is Formula (VII);
Y is -C(O)-;
wherein carbocyclic aryl is phenyl, naphthyl, or anthranyl;
carbocyclyl is 1,2,3,4-tetrahydronaphthyl, 1-oxo-indanyl, 9-oxo-9*H*fluorenyl, or indenyl;
heterocyclyl is 1,2,3-triazolyl, 1*H*-indolyl, 1*H*-pyrrolyl, 2,3-dihydro-1oxo-isoindolyl, 2,3-dihydro-benzofuryl, 2,4-dihydro-3-oxo-pyrazolyl, 2*H*-benzopyranyl, 2-oxo-benzopyranyl, 4-oxo-1,5,6,7-tetrahydro-indolyl, 9*H*-xanthenyl, benzo[1,3]dioxolyl, benzo[2,1,3]oxadiazolyl, benzo[1,2,5]oxadiazolyl, benzo[b]thienyl, benzofuryl, benzothiazolyl, furyl, imidazolyl, isoxazolyl, morpholino, pyrazolyl, pyridyl, pyrimidyl, quinolyl, quinoxalyl, thiazolyl, or thienyl; and
halogen is fluoro, chloro, bromo, or iodo;

or a pharmaceutically acceptable salt, hydrate, or solvate thereof.

**60.** The compound according to claim 59 wherein $R_2$ is hydrogen, trifluoromethyl, methoxy, methylamino, dimethylamino, ethylamino, ethylmethylamino, or hydroxylethylmethylamino; p is 0; $R_3$ and $R_4$ are hydrogen; A is a single bond; B is a single bond or $-CH_2-$;
or a pharmaceutically acceptable salt, hydrate, or solvate thereof.

**61.** The compound according to claim 60 wherein $R_1$ is selected from the group consisting of:

(i) $C_{1-5}$ alkyl, and
$C_{1-5}$ alkyl substituted by substituent(s) independently selected from the group consisting of:

- oxo,
- carbocyclic aryloxy,
- carbocyclic aryloxy substituted by halogen,
- carbocyclic aryloxy substituted by $C_{1-5}$ alkoxy,

- mono-$C_{1-5}$ alkylaminocarbonyl,
- di-$C_{1-5}$ alkylaminocarbonyl,
- mono-$C_{1-5}$ alkylamino,
- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- carbocyclic arylcarbonylamino,
- $C_{1-5}$ alkylthio,
- $C_{3-6}$ cycloalkyl,
- carbocyclyl,
- carbocyclyl substituted by substituent(s) independently selected from the group consisting of:

    •• halogen,
    •• $C_{1-5}$ alkyl,
    •• $C_{2-5}$ alkenyl, and
    •• $C_{2-5}$ alkenyl substituted by substituent(s) independently selected from the group consisting of:

        ••• carbocyclic aryl, and
        ••• carbocyclic aryl substituted by $C_{1-5}$ alkylsulfinyl,

- carbocyclic aryl,
- carbocyclic aryl substituted by substituent(s) independently selected from the group consisting of:

    •• halogen,
    •• hydroxy,
    •• nitro,
    •• $C_{1-5}$ alkyl,
    •• $C_{1-5}$ alkyl substituted by substituent(s) independently selected from the group consisting of:

        ••• oxo,
        ••• carbocyclic aryl, and
        ••• heterocyclyl,

    •• $C_{1-5}$ alkoxy,
    •• $C_{1-5}$ alkoxy substituted by halogen,

- heterocyclyl, and
- heterocyclyl substituted by substituent(s) independently selected from the group consisting of:

    •• $C_{1-5}$ alkyl,
    •• carbocyclic aryl, and
    •• carbocyclic aryl substituted by halogen,

(ii) $C_{2-5}$ alkenyl, and
$C_{2-5}$ alkenyl substituted by substituent(s) independently selected from the group consisting of:

- carbocyclic aryl,
- carbocyclic aryl substituted by substituent(s) independently selected from the group consisting of:

    •• halogen, and
    •• nitro,

(iii) carbocyclyl,
(iv) carbocyclic aryl, and
carbocyclic aryl substituted by substituent(s) independently selected from the group consisting of:

- halogen,
- hydroxy,
- nitro,
- $C_{1-5}$ alkyl,
- $C_{1-5}$ alkyl substituted by substituent(s) independently selected from the group consisting of:

  - •• halogen,
  - •• oxo, and
  - •• Carbocyclic aryl,

- $C_{1-5}$ alkoxy,
- $C_{1-5}$ alkoxy substituted by substituent(s) independently selected from the group consisting of:

  - •• halogen, and
  - •• carbocyclic aryl,

- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- mono-$C_{1-5}$ alkylamino,
- di-$C_{1-5}$ alkylamino,
- $C_{2-5}$ alkynylcarbonylamino,
- $C_{2-5}$ alkynylcarbonylamino substituted by carbocyclic aryl,
- mono-$C_{1-5}$ alkylaminosulfonyl, and
- di-$C_{1-5}$ alkylaminosulfonyl,

(v) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:

  - •• $C_{1-5}$ alkylthio,
  - •• $C_{1-5}$ alkylthio substituted by carbocyclic aryl,
  - •• $C_{1-5}$ alkylthio substituted by halogenated carbocyclic aryl,
  - •• carbocyclic aryl,
  - •• carbocyclic aryl substituted by halogen, and
  - •• heterocyclyl,

- carbocyclic aryloxy,
- carbocyclic aryloxy substituted by halogen,
- carbocyclic aryloxy substituted by $C_{1-5}$ alkyl,
- $C_{1-5}$ alkylthio,
- $C_{1-5}$ alkylsulfonyl,
- carbocyclic arylsulfonyl,
- carbocyclic arylsulfonyl substituted by $C_{1-5}$ alkyl,
- carbocyclic aryl,
- carbocyclic aryl substituted by substituent(s) independently selected from the group consisting of:

  - •• halogen,
  - •• nitro, and
  - •• $C_{1-5}$ alkyl,

- heterocyclyl;

wherein carbocyclic aryl is phenyl or naphthyl;
carbocyclyl is 1-oxo-indanyl, 9-oxo-9*H*-fluorenyl, or indenyl;
heterocyclyl is 1,2,3-triazolyl, 1*H*-indolyl, 1*H*-pyrrolyl; 2,3-dihydro-benzofuryl, 2*H*-benzopyranyl, 9*H*-xanthenyl, benzo[2,1,3]oxadiazolyl, benzo[1,2,5]oxadiazolyl, benzo[b]thienyl, furyl, isoxazolyl, morpholino, pyrazolyl, pyridyl, quinolyl, quinoxalyl, thiazolyl, or thienyl; and
halogen is fluoro, chloro, bromo, or iodo;

or a pharmaceutically acceptable salt, hydrate, or solvate thereof.

**62.** The compound according to claim 61 wherein $R_1$ is selected from the group consisting of:

(i) $C_{1-5}$ alkyl, and
$C_{1-5}$ alkyl substituted by substituent(s) independently selected from the group consisting of:

- oxo,
- carbocyclic aryloxy,
- carbocyclic aryloxy substituted by halogen,
- carbocyclic aryloxy substituted by $C_{1-5}$ alkoxy,
- mono-$C_{1-5}$ alkylamino,
- di-$C_{1-5}$ alkylamino,
- mono-carbocyclic arylamino,
- di-carbocyclic arylamino,
- mono-carbocyclic arylamino substituted by halogen,
- di-carbocyclic arylamino substituted by halogen,
- $C_{1-5}$ alkylthio,
- carbocyclic aryl,
- carbocyclic aryl substituted by substituent(s) independently selected from the group consisting of:

  - •• halogen,
  - •• hydroxy,
  - •• $C_{1-5}$ alkyl,
  - •• $C_{1-5}$ alkoxy, and
  - •• $C_{1-5}$ alkoxy substituted by halogen,

- heterocyclyl, and
- heterocyclyl substituted by substituent(s) independently selected from the group consisting of:

  - •• $C_{1-5}$ alkyl,
  - •• carbocyclic aryl, and
  - •• carbocyclic aryl substituted by halogen,

(ii) $C_{2-5}$ alkenyl, and
$C_{2-5}$ alkenyl substituted by substituent(s) independently selected from the group consisting of:

- carbocyclic aryl,
- carbocyclic aryl substituted by nitro,

(iii) carbocyclyl,
(iv) carbocyclic aryl, and
carbocyclic aryl substituted by substituent(s) independently selected from the group consisting of:

- halogen,
- hydroxy,
- nitro,
- $C_{1-5}$ alkyl,
- $C_{1-5}$ alkyl substituted by halogen,

- $C_{1-5}$ alkoxy,
- $C_{1-5}$ alkoxy substituted by halogen,
- $C_{1-5}$ alkoxy substituted by carbocyclic aryl,
- carbocyclic aryloxy,
- carbocyclic aryloxy substituted by $C_{1-5}$ alkoxy,
- mono-$C_{1-5}$ alkylaminocarbonyl,
- di-$C_{1-5}$ alkylaminocarbonyl,
- mono-$C_{1-5}$ alkylaminocarbonyl substituted by carbocyclic aryl,
- di-$C_{1-5}$ alkylaminocarbonyl substituted by carbocyclic aryl,
- mono-$C_{1-5}$ alkylaminosulfonyl, and
- di-$C_{1-5}$ alkylaminosulfonyl,

(v) heterocyclyl, and
heterocyclyl substituted by substituent(s) independently selected from the group consisting of:

- halogen,
- nitro,
- $C_{1-5}$ alkyl,
- $C_{1-5}$ alkyl substituted by substituent(s) independently selected from the group consisting of:
    - •• $C_{1-5}$ alkylthio,
    - •• $C_{1-5}$ alkylthio substituted by carbocyclic aryl, and
    - •• $C_{1-5}$ alkylthio substituted by halogenated carbocyclic aryl,
- Carbocyclic aryloxy,
- carbocyclic aryloxy substituted by halogen,
- carbocyclic aryloxy substituted by $C_{1-5}$ alkyl,
- carbocyclic aryl,
- carbocyclic aryl substituted by halogen,
- carbocyclic aryl substituted by nitro, and
- heterocyclyl;

wherein carbocyclic aryl is phenyl or naphthyl;
carbocyclyl is 1-oxo-indanyl;
heterocyclyl is 1,2,3-triazolyl, 1*H*-indolyl, 1*H*-pyrrolyl, 2,3-dihydro-benzofuryl, 9*H*-xanthenyl, benzo[2,1,3]oxadiazolyl, benzo[1,2,5]oxadiazolyl, benzo[b]thienyl, furyl, isoxazolyl, pyridyl, quinoxalyl, thiazolyl, or thienyl; and
halogen is fluoro, chloro, bromo, or iodo;

or a pharmaceutically acceptable salt, hydrate, or solvate thereof.

**63.** The compound according to claim 1 selected from the group consisting of:

N-(cis-4-{[4-(dimethylamino)pyrimidin-2-yl]amino}cyclohexyl)-3-methoxybenzamide;
3-bromo-N-(cis-4-{[4-(dimethylamino)pyrimidin-2-yl]amino}cyclohexyl)-benzamide;
N-(cis-4-{[4-(dimethylamino)pyrimidin-2-yl]amino}cyclohexyl)-2,1,3-benzoxadiazole-5-carboxamide;
3-chloro-N-(cis-4-{[4-(dimethylamino)pyrimidin-2-yl]amino}cyclohexyl)-benzamide;
4-chloro-N-(cis-4-{[4-(dimethylamino)pyrimidin-2-yl]amino}cyclohexyl)-benzamide;
4-chloro-N-(cis-4-{[4-(dimethylamino)pyrimidin-2-yl]amino}cyclohexyl)-3-nitrobenzamide;
3,5-dichloro-N-(cis-4-{[4-(dimethylamino)pyrimidin-2-yl]amino}-cyclohexyl)benzamide;
3,4-dichloro-N-(cis-4-{[4-(dimethylamino)pyrimidin-2-yl]amino}-cyclohexyl)benzamide;
N-(cis-4-{[4-(dimethylamino)pyrimidin-2-yl]amino}cyclohexyl)-2,2-diphenylacetamide;
N-(cis-4-{[4-(dimethylamino)pyrimidin-2-yl]amino}cyclohexyl)-3,4-difluorobenzamide;
N-(cis-4-{[4-(dimethylamino)pyrimidin-2-yl]amino}cyclohexyl)-3,5-difluorobenzamide;
N-(cis-4-{[4-(dimethylamino)pyrimidin-2-yl]amino}cyclohexyl)-3-fluoro-5-(trifluoromethyl)benzamide;
N-(cis-4-{[4-(dimethylamino)pyrimidin-2-yl]amino}cyclohexyl)-4-methyl-3-nitrobenzamide;
N-(cis-4-{[4-(dimethylamino)pyrimidin-2-yl]amino}cyclohexyl)-3-nitrobenzamide;
N-(cis-4-{[4-(dimethylamino)pyrimidin-2-yl]amino}cyclohexyl)-2-phenoxybutanamide;

N-(cis-4-{[4-(dimethylamino)pyrimidin-2-yl]amino}cyclohexyl)-3-methylbenzamide;
N-(cis-4-{[4-(dimethylamino)pyrimidin-2-yl]amino}cyclohexyl)-3-(trifluoromethoxy)benzamide;
4-bromo-N-(cis-4-{[4-(dimethylamino)pyrimidin-2-yl]amino}cyclohexyl)-3-methylbenzamide;
N-(cis-4-{[4-(dimethylamino)pyrimidin-2-yl]amino}cyclohexyl)-3-iodobenzamide;
N-(cis-4-{[4-(dimethylamino)pyrimidin-2-yl]amino}cyclohexyl)-2,5-dimethyl-3-furamide;
3-chloro-N-(cis-4-{[4-(dimethylamino)pyrimidin-2-yl]amino}cyclohexyl)-4-fluorobenzamide;
N-(cis-4-{[4-(dimethylamino)pyrimidin-2-yl]amino}cyclohexyl)-3,5-dimethoxybenzamide;
N-(cis-4-{[4-(dimethylamino)pyrimidin-2-yl]amino}cyclohexyl)-3,5-bis(trifluoromethyl)benzamide;
N-(cis-4-{[4-(dimethylamino)pyrimidin-2-yl]amino}cyclohexyl)-4-fluoro-3-methylbenzamide;
2,5-dichloro-N-(cis-4-{[4-(dimethylamino)pyrimidin-2-yl]amino}-cyclohexyl)thiophene-3-carboxamide;
1-benzyl-3-tert-butyl-N-(cis-4-{[4-(dimethylamino)pyrimidin-2-yl]amino}-cyclohexyl)-1H-pyrazole-5-carboxamide;
N-(cis-4-{[4-(dimethylamino)pyrimidin-2-yl]amino}cyclohexyl)-2-(1-naphthyl)acetamide;
2-(4-chlorophenoxy)-N-(cis-4-{[4-(dimethylamino)pyrimidin-2-yl]amino}-cyclohexyl)acetamide;
1-(4-chlorophenyl)-N-(cis-4-{[4-(dimethylamino)pyrimidin-2-yl]amino}-cyclohexyl)cyclopentanecarboxamide;
3-(2-chloro-6-fluorophenyl)-N-(cis-4-{[4-(dimethylamino)pyrimidin-2-yl]amino}cyclohexyl)-5-methylisoxazole-4-carboxamide;
N-(cis-4-{[4-(dimethylamino)pyrimidin-2-yl]amino}cyclohexyl)-4-fluoro-3-(trifluoromethyl)benzamide;
N-(cis-4-{[4-(dimethylamino)pyrimidin-2-yl]amino}cyclohexyl)-5-methyl-2-phenyl-2H-1,2,3-triazole-4-carboxamide;
N-(cis-4-{[4-(dimethylamino)pyrimidin-2-yl]amino}cyclohexyl)-2-(4-methoxyphenoxy)-5-nitrobenzamide;
N-(cis-4-{[4-(dimethylamino)pyrimidin-2-yl]amino}cyclohexyl)-2-phenoxyacetamide;
N-(cis-4-{[4-(dimethylamino)pyrimidin-2-yl]amino}cyclohexyl)-quinoxaline-2-carboxamide;
N-(cis-4-{[4-(dimethylamino)pyrimidin-2-yl]amino}cyclohexyl)-3-(trifluoromethyl)benzamide;
N-(cis-4-{[4-(dimethylamino)pyrimidin-2-yl]amino}cyclohexyl)-2-(pentafluorophenoxy)acetamide;
2-(3-chlorophenoxy)-N-(cis-4-{[4-(dimethylamino)pyrimidin-2-yl]amino}cyclohexyl)acetamide;
3-(2,6-dichlorophenyl)-N-(cis-4-{(4-(dimethylamino)pyrimidin-2-yl]amino}cyclohexyl)-5-methylisoxazole-4-carboxamide;
N-(cis-4-{[4-(dimethylamino)pyrimidin-2-yl]amino}cyclohexyl)-2-phenoxynicotinamide;
N-(cis-4-{[4-(dimethylamino)pyrimidin-2-yl]amino}cyclohexyl)-2-(4-methylphenoxy)nicotinamide;
N-(cis-4-{[4-(dimethylamino)pyrimidin-2-yl]amino)}cyclohexyl)-4-[(dipropylamino)sulfonyl]benzamide;
2-(4-chlorophenoxy)-N-(cis-4-{[4-(dimethylamino)pyrimidin-2-yl]amino}cyclohexyl)-2-methylpropanamide;
2-(2,3-dihydro-1-benzofuran-5-yl)-N-(cis-4-{[4-(dimethylamino)pyrimidin-2-yl]amino}cyclohexyl)-1,3-thiazole-4-carboxamide;
3-tert-butyl-1-(2,4-dichlorobenzyl)-N-(cis-4-{[4-(dimethylamino)pyrimidin-2-yl]amino}cyclohexyl)-1H-pyrazole-5-carboxamide;
6-chloro-N-(cis-4-{[4-(dimethylamino)pyrimidin-2-yl]amino}cyclohexyl)-2H-chromene-3-carboxamide;
N-(cis-4-{[4-(dimethylamino)pyrimidin-2-yl]amino}cyclohexyl)-2-(2-thienyl)-1,3-thiazole-4-carboxamide;
5-(4-chloro-2-nitrophenyl)-N-(cis-4-{[4-(dimethylamino)pyrimidin-2-yl]amino}cyclohexyl)-2-furamide;
N-(cis-4-{[4-(dimethylamino)pyrimidin-2-yl]amino}cyclohexyl)-5-iodo-2-furamide;
N-(cis-4-{[4-(dimethylamino)pyrimidin-2-yl]amino}cyclohexyl)-5-(4-methyl-2-nitrophenyl)-2-furamide;
N-(cis-4-{[4-(dimethylamino)pyrimidin-2-yl]amino}cyclohexyl)-5-nitrothiophene-2-carboxamide;
N-(cis-4-{[4-(dimethylamino)pyrimidin-2-yl]amino}cyclohexyl)-3-methyl-4-nitrobenzamide;
N-(cis-4-{[4-(dimethylamino)pyrimidin-2-yl]amino}cyclohexyl)-3-methoxy-4-nitrobenzamide;
1-benzyl-N-(cis-4-{[4-(dimethylamino)pyrimidin-2-yl]amino}cyclohexyl)-1H-indole-3-carboxamide;
3-acetyl-N-(cis-4-{[4-(dimethylamino)pyrimidin-2-yl]amino}cyclohexyl)-benzamide;
5-bromo-N-(cis-4-{[4-(dimethylamino)pyrimidin-2-yl]amino}cyclohexyl)-2-furamide;
5-(4-chlorophenyl)-N-(cis-4-{[4-(dimethylamino)pyrimidin-2-yl]amino}-cyclohexyl)-2-furamide;
4,5-dibromo-N-(cis-4-{[4-(dimethylamino)pyrimidin-2-yl]amino}-cyclohexyl)thiophene-2-carboxamide;
2-(3,5-di-tert-butyl-4-hydroxyphenyl)-N-(cis-4-{[4-(dimethylamino)-pyrimidin-2-yl]amino}cyclohexyl)acetamide;
$N^2$,$N^6$-dibenzoyl-N-(cis-4-{[4-(dimethylamino)pyrimidin-2-yl]amino}-cyclohexyl)lysinamide;
3-(dimethylamino)-N-(cis-4-{[4-(dimethylamino)pyrimidin-2-yl]amino}-cyclohexyl)benzamide;
4,5-dibromo-N-(cis-4-{[4-(dimethylamino)pyrimidin-2-yl]amino}-cyclohexyl)-2-furamide;
N-(cis-4-{[4-(dimethylamino)pyrimidin-2-yl]amino}cyclohexyl)-2-(1H-indol-3-yl)acetamide;
N-(cis-4-{[4-(dimethylamino)pyrimidin-2-yl]amino}cyclohexyl)-2-(5-methyl-2-phenyl-1,3-thiazol-4-yl)acetamide;
N-(cis-4-{[4-(dimethylamino)pyrimidin-2-yl]amino}cyclohexyl)-2-(1H-indol-3-yl)-4-oxo-4-phenylbutanamide;

4-(4-bromophenyl)-N-(cis-4-{[4-(dimethylamino)pyrimidin-2-yl]amino}cyclohexyl)-2-(1H-indol-3-yl)-4-oxobutanamide;
3,5-dichloro-N-(cis-4-{[4-(dimethylamino)pyrimidin-2-yl]amino}-cyclohexyl)-2-[(3-phenylprop-2-ynoyl)amino]benzamide;
N-(cis-4-{[4-(dimethylamino)pyrimidin-2-yl]amino}cyclohexyl)-2-(1-methyl-1H-indol-3-yl)-4-(4-methylphenyl)-4-oxobutanamide;
N-(cis-4-{[4-(dimethylamino)pyrimidin-2-yl]amino}cyclohexyl)-2-methyl-1-(3-morpholin-4-ylpropyl)-5-phenyl-1H-pyrrole-3-carboxamide;
N-(cis-4-{[4-(dimethylamino)pyrimidin-2-yl]amino}cyclohexyl)-4-(4-nitrophenyl)butanamide;
N-(cis-4-{[4-(dimethylamino)pyrimidin-2-yl]amino}cyclohexyl)-2-(2-phenyl-1Hindol-3-yl)acetamide ;
$N^2$-benzoyl-$N^5$-(cis-4-{[4-(dimethylamino)pyrimidin-2-yl]amino}-cyclohexyl)-$N^1$,$N^1$-dipropylglutamamide ;
N-(cis-4-{[4-(dimethylamino)pyrimidin-2-yl]amino}cyclohexyl)-3-phenoxybenzamide;
3-benzoyl-N-(cis-4-{[4-(dimethylamino)pyrimidin-2-yl]amino}-cyclohexyl)benzamide;
N-(cis-4-{[4-(dimethylamino)pyrimidin-2-yl]amino}cyclohexyl)-2-(ethylthio)-2,2-diphenylacetamide;
N-(cis-4-{[4-(dimethylamino)pyrimidin-2-yl]amino}cyclohexyl)-N'-[(1R)-1-(1-naphthyl)ethyl]phthalamide;
(2S)-2-(3-benzoylphenyl)-N-(cis-4-{[4-(dimethylamino)pyrimidin-2-yl]amino}cyclohexyl)propanamide;
N'-(cis-4-{[4-(dimethylamino)pyrimidin-2-yl]amino)cyclohexyl)-N,N-bis[(1S)-1-phenylethyl]phthalamide;
N-(cis-4-{[4-(dimethylamino)pyrimidin-2-yl]amino}cyclohexyl)-2-{(1E)-5-fluoro-2-methyl-1-[4-(methylsulfinyl)benzylidene]-1H-inden-3-yl}acetamide;
N-(cis-4-{[4-(dimethylamino)pyrimidin-2-yl]amino}cyclohexyl)-2-[4-(2-thienylcarbonyl)phenyl]propanamide;
3-(benzyloxy)-N-(cis-4-{[4-(dimethylamino)pyrimidin-2-yl]amino}-cyclohexyl)-4-methoxybenzamide;
N-(cis-4-{[4-(dimethylamino)pyhmidin-2-yl]amino}cyclohexyl)-2-methyl-1,5-diphenyl-1H-pyrrole-3-carboxamide;
1-{2-[(2-chloro-6-fluorobenzyl)thio]ethyl}-N-(cis-4-{[4-(dimethylamino)-pyrimidin-2-yl]amino}cyclohexyl)-2-methyl-5-phenyl-1H-pyrrole-3-carboxamide;
N-(cis-4-{[4-(dimethylamino)pyrimidin-2-yl]amino}cyclohexyl)-2-phenoxybenzamide;
N-(cis-4-{[4-(dimethylamino)pyrimidin-2-yl]amino}cyclohexyl)-2-phenylquinoline-4-carboxamide;
2-[4-(4-chlorophenyl)-2-phenyl-1,3-thiazol-5-yl]-N-(cis-4-{[4-(dimethylamino)pyrimidin-2-yl]amino}cyclohexyl)acetamide;
N-(cis-4-{[4-(dimethylamino)pyrimidin-2-yl]amino]cyclohexyl}-1-[(4-methylphenyl)sulfonyl]-1H-pyrrole-3-carboxamide;
N-(cis-4-{(4-(dimethylamino)pyrimidin-2-yl]amino}cyclohexyl)-5-(3-nitrophenyl)-2-furamide;
3-chloro-N-(cis-4-{[4-(dimethylamino)pyrimidin-2-yl]amino}cyclohexyl)-4-(isopropylsulfonyl)-5-(methylthio)thiophene-2-carboxamide;
N-(cis-4-{[4-(dimethylamino)pyrimidin-2-yl]amino}cyclohexyl)-3-iodo-4-(isopropylsulfonyl)-5-(methylthio)thiophene-2-carboxamide;
N-(cis-4-{[4-(dimethylamino)pyrimidin-2-yl]amino}cyclohexyl)-5-nitrothiophene-3-carboxamide;
N-(cis-4-{[4-(dimethylamino)pyrimidin-2-yl]amino}cyclohexyl)-1-methyl-4-nitro-1H-pyrrole-2-carboxamide;
N-(cis-4-{[4-(dimethylamino)pyrimidin-2-yl]amino}cyclohexyl)-4-nitrobenzamide;
N-(cis-4-{[4-(dimethylamino)pyrimidin-2-yl]amino}cyclohexyl)-3,5-dimethyl-4-nitrobenzamide;
N-(cis-4-{[4-(dimethylamino)pyrimidin-2-yl]amino}cyclohexyl)-2-mesityl-2-oxoacetamide;
3,5-di-tert-butyl-N-(cis-4-{[4-(dimethylamino)pyrimidin-2-yl]amino}-cyclohexyl)-4-hydroxybenzamide;
4-chloro-N-[(cis-4-[4-(dimethylamino)pyrimidin-2-yl]amino}cyclohexyl)-methyl]benzamide;
(2E)-N-[(cis-4-{[4-(dimethylamino)pyrimidin-2-yl]amino}cyclohexyl)-methyl]-3-phenylacrylamide;
4-chloro-N-[(cis-4-{[4-(dimethylamino)pyrimidin-2-yl]amino}cyclohexyl)-methyl]-3-nitrobenzamide;
2-(4-chlorophenyl)-N-[(cis-4-(4-(dimethylamino)pyrimidin-2-yl]amino}-cyclohexyl)methyl]acetamide;
3,5-dichloro-N-[(cis-4-{[4-(dimethylamino)pyrimidin-2-yl]amino}-cyclohexyl)methyl]benzamide;
3,4-dichloro-N-[(cis-4-[4-(dimethylamino)pyrimidin-2-yl]amino}-cyclohexyl)methyl]benzamide;
N-[(cis-4-{[4-(dimethylamino)pyrimidin-2-yl]amino}cyclohexyl)methyl]-2,2-diphenylacetamide;
2,4-dichloro-N-[(cis-4-{[4-(dimethylamino)pyrimidin-2-yl]amino}-cyclohexyl)methyl]-5-fluorobenzamide;
N-[(cis-4-{[4-(dimethylamino)pyrimidin-2-yl]amino}cyclohexyl)methyl]-2-phenoxybutanamide;
N-[(cis-4-{[4-(dimethylamino)pyrimidin-2-yl]amino}cyclohexyl)methyl]-2-phenylbutanamide;
N-[(cis-4-{[4-(dimethylamino)pyrimidin-2-yl]amino}cyclohexyl)methyl]-2-(3-methoxyphenyl)acetamide;
N-[(cis-4-{[4-(dimethylamino)pyrimidin-2-yl]amino}cyclohexyl)methyl]-2-(4-methoxyphenyl)acetamide;
N-[(cis-4-{ [4-(dimethylamino)pyrimidin-2-yl]amino}cyclohexyl)methyl]-3,5-bis(trifluoromethyl)benzamide;
(2E)-N-[(cis-4-[4-(dimethylamino)pyrimidin-2-yl]amino}cyclohexyl)-methyl]-3-(4-nitrophenyl)acrylamide;
2-(2-bromophenyl)-N-[(cis-4-{[4-(dimethylamino)pyrimidin-2-yl]amino}-cyclohexyl)methyl]acetamide;
N-[(cis-4-{[4-(dimethylamino)pyrimidin-2-yl]amino}cyclohexyl)methyl]-2-(propylthio)nicotinamide;

N-[(cis-4-{[4-(dimethylamino)pyrimidin-2-yl]amino}cyclohexyl)methyl]-2-(1-naphthyl)acetamide;
N-[(cis-4-{[4-(dimethylamino)pyrimidin-2-yl]amino}cyclohexyl)methyl]-9-oxo-9H-fluorene-4-carboxamide;
N-[(cis-4-{[4-(dimethylamino)pyrimidin-2-yl]amino}cyclohexyl)methyl]-2,4,6-trimethylbenzamide;
2,4,6-trichloro-N-[(cis-4-{(4-(dimethylamino)pyrimidin-2-yl]amino}-cyclohexyl)methyl]benzamide;
(2E)-3-(2-chlorophenyl)-N-[(cis-4-{[4-(dimethylamino)pyrimidin-2-yl]amino}cyclohexyl)methyl]acrylamide;
N-[(cis-4-{[4-(dimethylamino)pyrimidin-2-yl]amino}cyclohexyl)methyl]-2-(2,3,6-trichlorophenyl)acetamide;
N-[(cis-4-{[4-(dimethylamino)pyrimidin-2-yl]amino}cyclohexyl)methyl]-2,3-diphenylpropanamide;
N-[(cis-4-{[4-(dimethylamino)pyrimidin-2-yl]amino}cyclohexyl)methyl]-5-iodo-2-furamide;
(2E)-N-[(cis-4-{[4-(dimethylamino)pyrimidin-2-yl]amino}cyclohexyl)-methyl]-3-(3-nitrophenyl)acrylamide;
N-[(cis-4-{[4-(dimethylamino)pyrimidin-2-yl]amino}cyclohexyl)methyl]-3-oxoindane-1-carboxamide;
2-benzyl-N-[(cis-4-{[4-(dimethylamino)pyrimidin-2-yl]amino}cyclohexyl)-methyl]benzamide;
2,2-bis(4-chlorophenyl)-N-[(cis-4-{[4-(dimethylamino)pyrimidin-2-yl]amino}cyclohexyl)methyl]acetamide;
N-[(cis-4-{[4-(dimethylamino)pyrimidin-2-yl]amino}cyclohexyl)methyl]-3-methyl-4-nitrobenzamide;
N-[(cis-4-{[4-(dimethylamino)pyrimidin-2-yl]amino}cyclohexyl)methyl]-3-methoxy-4-nitrobenzamide;
N-[(cis-4-{[4-(dimethylamino)pyrimidin-2-yl]amino}cyclohexyl)methyl]-2-[2-(trifluoromethoxy)phenyl]acetamide;
N-[(cis-4-{(4-(dimethylamino)pyrimidin-2-yl]amino}cyclohexyl)methyl]-9H-xanthene-9-carboxamide;
2-(1-benzothien-3-yl)-N-[(cis-4-{[4-(dimethylamino)pyrimidin-2-yl]amino}cyclohexyl)methyl]acetamide;
N-[cis-4-(4-dimethylamino-pyrimidin-2-ylamino)-cyclohexyl]-2-(4-fluoro-phenoxy)-nicotinamide;
N-[cis-4-(4-dimethylamino-pyrimidin-2-ylamino)-cyclohexyl]-C-(ethyl-phenyl-amino)-acetamide;
C-[cis-(4-chloro-phenyl)-ethyl-amino]-N-(4-(4-dimethylamino-pyrimidin-2-ylamino)-cyclohexyl]-acetamide;
2-(3,4-difluoro-phenyl)-N-[cis-4-(4-dimethylamino-pyrimidin-2-ylamino)-cyclohexyl]-acetamide;
4-chloro-N-[cis-4-(4-dimethylamino-pyrimidin-2-ylamino)-cyclohexyl]-3-fluoro-benzamide;
5-bromo-N-[cis-4-(4-dimethylamino-pyrimidin-2-ylamino)-cyclohexyl]-nicotinamide;
3-chloro-4-fluoro-N-[cis-4-(4-methylamino-pyrimidin-2-ylamino)-cyclohexyl]-benzamide;
N-[cis-4-(4-dimethylamino-pyrimidin-2-ylamino)-cyclohexyl]-4-fluoro-benzamide;
3-chloro-N-[cis-4-(4-dimethylamino-pyrimidin-2-ylamino)-cyclohexyl]-5-fluoro-benzamide;
N-[cis-4-(4-dimethylamino-pyrimidin-2-ylamino)-cyclohexyl]-3,4,5-trifluoro-benzamide;
N-[cis-4-(4-dimethylamino-pyrimidin-2-ylamino)-cyclohexylmethyl]-3,4-difluoro-benzamide;
2-(3,4-dichloro-phenoxy)-N-[cis-4-(4-dimethylamino-pyrimidin-2-ylamino)-cyclohexyl]-acetamide;
N-[cis-4-(4-dimethylamino-pyrimidin-2-ylamino)-cyclohexyl]-2-(3-methoxy-phenoxy)-acetamide; and
N-[cis-4-(4-dimethylamino-pyrimidin-2-ylamino)-cyclohexyl]-C-(ethyl-phenyl-amino)-acetamide;

or a pharmaceutically acceptable salt, hydrate, or solvate thereof.

**64.** The compound according to claim 63 selected from the group consisting of:

3-bromo-N-(cis-4-{[4-(dimethylamino)pyrimidin-2-yl]amino}cyclohexyl)-benzamide;
N-(cis-4-{[4-(dimethylamino)pyrimidin-2-yl]amino}cyclohexyl)-2,1,3-benzoxadiazole-5-carboxamide;
3-chloro-N-(cis-4-{[4-(dimethylamino)pyrimidin-2-yl]amino}cyclohexyl)-benzamide;
4-chloro-N-(cis-4-{[4-(dimethylamino)pyrimidin-2-yl]amino}cyclohexyl)-3-nitrobenzamide;
3,5-dichloro-N-(cis-4-{[4-(dimethylamino)pyrimidin-2-yl]amino}-cyclohexyl)benzamide;
3,4-dichloro-N-(cis-4-{[4-(dimethylamino)pyrimidin-2-yl]amino}-cyclohexyl)benzamide;
N-(cis-4-{[4-(dimethylamino)pyrimidin-2-yl]amino}cyclohexyl)-3,4-difluorobenzamide;
N-(cis-4-{[4-(dimethylamino)pyrimidin-2-yl]amino}cyclohexyl)-3-nitrobenzamide;
N-(cis-4-{[4-(dimethylamino)pyrimidin-2-yl]amino}cyclohexyl)-3-(trifluoromethoxy)benzamide;
4-bromo-N-(cis-4-{[4-(dimethylamino)pyrimidin-2-yl]amino}cyclohexyl)-3-methylbenzamide;
N-(cis-4-{[4-(dimethylamino)pyrimidin-2-yl]amino}cyclohexyl)-3-iodobenzamide;
3-chloro-N-(cis-4-{[4-(dimethylamino)pyrimidin-2-yl]amino}cyclohexyl)-4-fluorobenzamide;
N-(cis-4-{[4-(dimethylamino)pyrimidin-2-yl] amino}cyclohexyl)-3,5-dimethoxybenzamide;
N-(cis-4-{[4-(dimethylamino)pyrimidin-2-yl]amino}cyclohexyl)-3,5-bis(trifluoromethyl)benzamide;
N-(cis-4-{[4-(dimethylamino)pyrimidin-2-yl]amino}cyclohexyl)-4-fluoro-3-methylbenzamide;
2-(4-chlorophenoxy)-N-(cis-4-{[4-(dimethylamino)pyrimidin-2-yl]amino}-cyclohexyl)acetamide;
N-(cis-4-{[4-(dimethylamino)pyrimidin-2-yl]amino}cyclohexyl)-4-fluoro-3-(trifluoromethyl)benzamide;
N-(cis-4-{[4-(dimethylamino)pyrimidin-2-yl]amino}cyclohexyl)-5-methyl-2-phenyl-2H-1,2,3-triazole-4-carboxamide;
N-(cis-4-{[4-(dimethylamino)pyrimidin-2-yl]amino}cyclohexyl)-2-(4-methoxyphenoxy)-5-nitrobenzamide;
N-(cis-4-{[4-(dimethylamino)pyrimidin-2-yl]amino}cyclohexyl)-quinoxaline-2-carboxamide;

2-(3-chlorophenoxy)-N-(cis-4-{[4-(dimethylamino)pyrimidin-2-yl]amino}-cyclohexyl)acetamide;
3-(2,6-dichloroghenyl)-N-(cis-4-{[4-(dimethylamino)pyrimidin-2-yl]amino}cyclohexyl)-5-methylisoxazole-4-carboxamide;
N-(cis-4-{[4-(dimethylamino)pyrimidin-2-yl]amino}cyclohexyl)-2-(4-methylphenoxy)nicotinamide;
N-(cis-4-{[4-(dimethylamino)pyrimidin-2-yl]amino}cyclohexyl)-4-[(dipropylamino)sulfonyl]benzamide;
2-(2,3-dihydro-1-benzofuran-5-yl)-N-(cis-4-{[4-(dimethylamino)pyrimidin-2-yl]amino}cyclohexyl)-1,3-thiazole-4-carboxamide;
N-(cis-4-{[4-(dimethylamino)pyrimidin-2-yl]amino}cyclohexyl)-2-(2-thienyl)-1,3-thiazole-4-carboxamide;
5-(4-chloro-2-nitrophenyl)-N-(cis-4-{[4-(dimethylamino)pyrimidin-2-yl]amino}cyclohexyl)-2-furamide;
N-(cis-4-{[4-(dimethylamino)pyrimidin-2-yl]amino}cyclohexyl)-3-methoxy-4-nitrobenzamide;
5-bromo-N-(cis-4-{[4-(dimethylamino)pyrimidin-2-yl]amino}cyclohexyl)-2-furamide;
5-(4-chlorophenyl)-N-(cis-4-{[4-(dimethylamino)pyrimidin-2-yl]amino}-cyclohexyl)-2-furamide;
2-(3,5-di-tert-butyl-4-hydroxyphenyl)-N-(cis-4-{[4-(dimethylamino)-pyrimidin-2-yl]amino}cyclohexyl)acetamide;
4,5-dibromo-N-(cis-4-{[4-(dimethylamino)pyrimidin-2-yl]amino}-cyclohexyl)-2-furamide;
N-(cis-4-{[4-(dimethylamino)pyrimidin-2-yl]amino}cyclohexyl)-2-(1H-indol-3-yl)-4-oxo-4-phenylbutanamide;
N-(cis-4-{[4-(dimethylamino)pyrimidin-2-yl]amino}cyclohexyl)-2-(1-methyl-1H-indol-3-yl)-4-(4-methylphenyl)-4-oxobutanamide;
N-(cis-4-{[4-(dimethylamino)pyrimidin-2-yl]amino}cyclohexyl)-2-(2-phenyl-1H-indol-3-yl)acetamide;
N-(cis-4-{[4-(dimethylamino)pyrimidin-2-yl]amino}cyclohexyl)-2-(ethylthio)-2,2-diphenylacetamide;
N'-(cis-4-{[4-(dimethylamino)pyrimidin-2-yl]amino}cyclohexyl)-N,N-bis [(1S)-1-phenylethyl]phthalamide;
3-(benzyloxy)-N-(cis-4-{[4-(dimethylamino)pyrimidin-2-yl]amino}-cyclohexyl)-4-methoxybenzamide;
N-(cis-4-{[4-(dimethylamino)pyrimidin-2-yl]amino}cyclohexyl)-2-methyl-1,5-diphenyl-1H-pyrrole-3-carboxamide;
1-{2-[(2-chloro-6-fluorobenzyl)thio]ethyl}-N-(cis-4-{[4-(dimethylamino)-pyrimidin-2-yl]amino}cyclohexyl)-2-methyl-5-phenyl-1H-pyrrole-3-carboxamide;
2-[4-(4-chlorophenyl)-2-phenyl-1,3-thiazol-5-yl]-N-(cis-4-{[4-(dimethylamino)pyrimidin-2-yl]amino}cyclohexyl)acetamide;
N-(cis-4-{[4-(dimethylamino)pyrimidin-2-yl]amino}cyclohexyl)-5-nitrothiophene-3-carboxamide;
N-(cis-4-{[4-(dimethylamino)pyrimidin-2-yl]amino}cyclohexyl)-1-methyl-4-nitro-1H-pyrrole-2-carboxamide;
3,5-di-tert-butyl-N-(cis-4-{[4-(dimethylamino)pyrimidin-2-yl]amino}-cyclohexyl)-4-hydroxybenzamide;
N-[(cis-4-{[4-(dimethylamino)pyrimidin-2-yl]amino}cyclohexyl)methyl]-2,2-diphenylacetamide;
N-[(cis-4-{[4-(dimethylamino)pyrimidin-2-yl]amino}cyclohexyl)methyl]-2-phenylbutanamide;
(2E)-N-[(cis-4-{[4-(dimethylamino)pyrimidin-2-yl]amino}cyclohexyl)-methyl]-3-(4-nitrophenyl)acrylamide;
N-[(cis-4-{[4-(dimethylamino)pyrimidin-2-yl]amino}cyclohexyl)methyl]-2-(1-naphthyl)acetamide;
N-[(cis-4-{[4-(dimethylamino)pyrimidin-2-yl]amino}cyclohexyl)methyl]-2-(2,3,6-trichlorophenyl)acetamide;
(2E)-N-[(cis-4-{[4-(dimethylamino)pyrimidin-2-yl]amino}cyclohexyl)-methyl]-3-(3-nitrophenyl)acrylamide;
N-[(cis-4-{[4-(dimethylamino)pyrimidin-2-yl]amino}cyclohexyl)methyl]-3-oxoindane-1-carboxamide;
2,2-bis(4-chlorophenyl)-N-[(cis-4-{[4-(dimethylamino)pyrimidin-2-yl]amino}cyclohexyl)methyl]acetamide;
N-[(cis-4-{[4-(dimethylamino)pyrimidin-2-yl]amino}cyclohexyl)methyl]-3-methyl-4-nitrobenzamide;
N-[(cis-4-{[4-(dimethylamino)pyrimidin-2-yl]amino}cyclohexyl)methyl]-3-methoxy-4-nitrobenzamide;
N-[(cis-4-{[4-(dimethylamino)pyrimidin-2-yl]amino}cyclohexyl)methyl]-2-[2-(trifluoromethoxy)phenyl]acetamide;
N-[(cis-4-{[4-(dimethylamino)pyrimidin-2-yl]amino}cyclohexyl)methyl]-9H-xanthene-9-carboxamide;
2-(1-benzothien-3-yl)-N-[(cis-4-{[4-(dimethylamino)pyrimidin-2-yl]amino}cyclohexyl)methyl]acetamide;
N-[cis-4-(4-dimethylamino-pyrimidin-2-ylamino)-cyclohexyl]-2-(4-fluoro-phenoxy)-nicotinamide;
N-[cis-4-(4-dimethylamino-pyrimidin-2-ylamino)-cyclohexyl]-C-(ethyl-phenyl-amino)-acetamide;
C-[cis-(4-chloro-phenyl)-ethyl-amino]-N-[4-(4-dimethylamino-pyrimidin-2-ylamino)-cyclohexyl]-acetamide;
4-chloro-N-[cis-4-(4-dimethylamino-pyrimidin-2-ylamino)-cyclohexyl]-3-fluoro-benzamide;
N-[cis-4-(4-dimethylamino-pyrimidin-2-ylamino)-cyclohexyl]-3,4,5-trifluoro-benzamide;
2-(3,4-dichloro-phenoxy)-N-[cis-4-(4-dimethylamino-pyrimidin-2-ylamino)-cyclohexyl]-acetamide;
N-[cis-4-(4-dimethylamino-pyrimidin-2-ylamino)-cyclohexyl]-2-(3-methoxy-phenoxy)-acetamide; and
N-[cis-4-(4-dimethylamino-pyrimidin-2-ylamino)-cyclohexyl]-C-(ethyl-phenyl-amino)-acetamide;

or a pharmaceutically acceptable salt, hydrate, or solvate thereof.

**65.** The compound according to claim 52 wherein $R_1$ is selected from the group consisting of:

(i) $C_{1-5}$ alkyl, and
$C_{1-5}$ alkyl substituted by substituent(s) independently selected from the group consisting of:

- $C_{1-5}$ alkoxy carbonyl,
- $C_{1-5}$ alkylthio,
- carbocyclic aryl,
- carbocyclic aryl substituted by substituent(s) independently selected from the group consisting of:

  - •• halogen,
  - •• $C_{1-5}$ alkyl, and
  - •• $C_{2-5}$ alkenyl,

(ii) $C_{3-6}$ cycloalkyl,
$C_{3-6}$ cycloalkyl substituted by carbocyclic aryl,
(iii) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by halogen,
- $C_{1-5}$ alkoxy carbonyl,
- $C_{1-5}$ alkoxy,
- $C_{3-6}$ cycloalkoxy,
- carbocyclic aryloxy,
- $C_{1-5}$ alkylthio, and
- carbocyclic aryl,

(iv) heterocyclyl, and
heterocyclyl substituted by substituent(s) independently selected from the group consisting of:

- $C_{1-5}$ alkyl,
- $C_{1-5}$ alkyl substituted by halogen, and
- carbocyclic aryl;

L is Formula (VII);
Y is -C(O)$NR_5$-;
wherein carbocyclic aryl is phenyl or naphthyl;
heterocyclyl is 2,3-dihydro-benzo[1,4]dioxinyl, 3,4-dihydro-2*H*-benzo[b][1,4]dioxeginyl, benzo[1,3]dioxolyl, furyl, or isoxazolyl; and
halogen is fluoro, chloro, bromo, or iodo;

or a pharmaceutically acceptable salt, hydrate, or solvate thereof.

**66.** The compound according to claim 65 wherein $R_2$ is methylamino or dimethylamino; p is 0; $R_3$ and $R_4$ are hydrogen; A is a single bond; B is a single bond or -$CH_2$-: $R_5$ is hydrogen; or a pharmaceutically acceptable salt, hydrate, or solvate thereof.

**67.** The compound according to claim 66 wherein $R_1$ is selected from the group consisting of:

(i) $C_{1-5}$ alkyl, and
$C_{1-5}$ alkyl substituted by carbocyclic aryl,
(ii) carbocyclic aryl, and
carbocyclic aryl substituted by substituent(s) independently selected from the group consisting of:

- halogen,
- nitro,

- $C_{1-5}$ alkyl,
- $C_{1-5}$ alkyl substituted by halogen,
- $C_{1-5}$ alkoxy, and
- $C_{3-6}$ cycloalkoxy,

(iii) heterocyclyl, and
heterocyclyl substituted by $C_{1-5}$ alkyl, and
heterocyclyl substituted by carbocyclic aryl; wherein carbocyclic aryl is phenyl or naphthyl;
heterocyclyl is isoxazolyl; and
halogen is fluoro, chloro, bromo, or iodo;

or a pharmaceutically acceptable salt, hydrate, or solvate thereof.

**68.** The compound according to claim 1 selected from the group consisting of:

N-(cis-4-{[4-(dimethylamino)pyrimidin-2-yl]amino}cyclohexyl)-N'-mesitylurea;
N-(cis-4-{[4-(dimethylamino)pyrimidin-2-yl]amino}cyclohexyl)-N'-(2,4,6-trichlorophenyl)urea;
N-(cis-4-{[4-(dimethylamino)pyrimidin-2-yl]amino}cyclohexyl)-N'-(2,4,6-tribromophenyl)urea;
N-(2,4-dibromo-6-fluorophenyl)-N'-(cis-4-{[4-(dimethylamino)pyrimidin-2-yl]amino}cyclohexyl)urea;
N-(cis-4-{[4-(dimethylamino)pyrimidin-2-yl]amino}cyclohexyl)-N'-(diphenylmethyl)urea;
N-(4-bromo-2,6-dimethylphenyl)-N'-(cis-4-{[4-(dimethylamino)pyrimidin-2-yl]amino}cyclohexyl)urea;
N-(cis-4-{[4-(dimethylamino)pyrimidin-2-yl]amino}cyclohexyl)-N'-[1-(1-naphthyl)ethyl]urea;
N-(cis-4-{[4-(dimethylamino)pyrimidin-2-yl]amino}cyclohexyl)-N'-(3,4,5-trimethoxyphenyl)urea;
N-(4-chloro-2-methylphenyl)-N'-(cis-4-{[4-(dimethylamino)pyrimidin-2-yl]amino}cyclohexyl)urea;
N-(5-chloro-2,4-dimethoxyphenyl)-N'-(cis-4-{[4-(dimethylamino)-pyrimidin-2-yl]amino}cyclohexyl)urea;
N-(4-bromo-2-methylphenyl)-N'-(cis-4-{[4-(dimethylamino)pyrimidin-2-yl]amino}cyclohexyl)urea;
N-(2,6-dibromo-4-isopropylphenyl)-N'-(cis-4-{[4-(dimethylamino)-pyrimidin-2-yl]amino}cyclohexyl)urea;
N-[3-(cyclopentyloxy)-4-methoxyphenyl]-N'-(cis-4-{[4-(dimethylamino)-pyrimidin-2-yl]amino}cyclohexyl)urea;
N-[(cis-4-{[4-(dimethylamino)pyrimidin-2-yl]amino}cyclohexyl)methyl]-N'-(2,6-dimethylphenyl)urea;
N-(2,4-difluorophenyl)-N'-[(cis-4-{[4-(dimethylamino)pyrimidin-2-yl]amino}cyclohexyl)methyl]urea;
N-[(cis-4-{[4-(dimethylamino)pyrimidin-2-yl]amino}cyclohexyl)methyl]-N'-(2-ethyl-6-methylphenyl)urea;
N-[(cis-4-{[4-(dimethylamino)pyrimidin-2-yl]amino}cyclohexyl)methyl]-N'-(4-fluorophenyl)urea;
N-[(cis-4-{[4-(dimethylamino)pyrimidin-2-yl]amino}cyclohexyl)methyl]-N'-mesitylurea;
N-[(cis-4-{[4-(dimethylamino)pyrimidin-2-yl]amino}cyclohexyl)methyl]-N'-(2,4,6-trichlorophenyl)urea;
N-[(cis-4-{[4-(dimethylamino)pyrimidin-2-yl]amino}cyclohexyl)methyl]-N'-(2,4,6-tribromophenyl)urea;
N-(2,4-dibromo-6-fluorophenyl)-N'-[(cis-4-{[4-(dimethylamino)pyrimidin-2-yl]amino}cyclohexyl)methyl]urea;
N-(2,6-diethylphenyl)-N'-[(cis-4-{[4-(dimethylamino)pyrimidin-2-yl]amino}cyclohexyl)methyl]urea;
N-[2-chloro-6-(trifluoromethyl)phenyl]-N'-[(cis-4-{[4-(dimethylamino)-pyrimidin-2-yl]amino}cyclohexyl)methyl] urea;
N-(2-chloro-6-methylphenyl)-N'-[(cis-4-{[4-(dimethylamino)pyrimidin-2-yl]amino}cyclohexyl)methyl]urea;
N-[(cis-4-{[4-(dimethylamino)pyrimidin-2-yl]amino}cyclohexyl)methyl]-N'-(2-ethyl-6-isopropylphenyl)urea;
N-[(cis-4-{[4-(dimethylamino)pyrimidin-2-yl]amino}cyclohexyl)methyl]-N'-(2-isopropyl-6-methylphenyl)urea;
N-[(cis-4-{[4-(dimethylamino)pyrimidin-2-yl]amino}cyclohexyl)methyl]-N'-(2-methyl-3-nitrophenyl)urea;
N-[(cis-4-{[4-(dimethylamino)pyrimidin-2-yl]amino}cyclohexyl)methyl]-N'-(2-propylphenyl)urea;
N-(2-tert-butyl-6-methylphenyl)-N'-[(cis-4-{[4-(dimethylamino)pyrimidin-2-yl]amino}cyclohexyl)methyl]urea;
N-(2-tert-butylphenyl)-N'-[(cis-4-{[4-(dimethylamino)pyrimidin-2-yl]amino}cyclohexyl)methyl]urea;
N-(3-chloro-2-methylphenyl)-N'-[(cis-4-{[4-(dimethylamino)pyrimidin-2-yl]amino}cyclohexyl)methyl]urea;
N-(4-bromo-2,6-difluorophenyl)-N'-[(cis-4-{[4-(dimethylamino)pyrimidin-2-yl]amino}cyclohexyl)methyl]urea;
N-[4-chloro-2-(triftuoromethyl)phenyl]-N'-[(cis-4-{[4-(dimethylamino)-pyrimidin-2-yl]amino}cyclohexyl)methyl] urea;
N-[(cis-4-{[4-(dimethylamino)pyrimidin-2-yl]amino}cyclohexyl)methyl]-N'-(diphenylmethyl)urea;
N-(4-bromo-2,6-dimethylphenyl)-N'-[(cis-4-{[4-(dimethylamino)pyrimidin-2-yl]amino}cyclohexyl)methyl]urea;
N-[(cis-4-{[4-(dimethylamino)pyrimidin-2-yl]amino}cyclohexyl)methyl]-N'-(3-methyl-5-phenylisoxazol-4-yl) urea;
N-(3,5-dichlorophenyl)-N'-[(cis-4-{[4-(dimethylamino)pyrimidin-2-yl]-amino}cyclohexyl)methyl]urea;
N-(2,3-dichlorophenyl)-N'-[(cis-4-{[4-(dimethylamino)pyrimidin-2-yl]-amino}cyclohexyl)methyl]urea;
N-(2,6-diisopropylphenyl)-N'-[(cis-4-{[4-(dimethylamino)pyrimidin-2-yl]-amino}cyclohexyl)methyl]urea;
N-[(cis-4-[4-(dimethylamino)pyrimidin-2-yl]amino}cyclohexyl)methyl]-N'-(2,3-dimethyl-6-nitrophenyl)urea;

N-(2,6-dibromo-4-fluorophenyl)-N'-[(cis-4-{[4-(dimethylamino)pyrimidin-2-yl]amino}cyclohexyl)methyl]urea;
N-(2,6-dichlorophenyl)-N'-[(cis-4-{[4-(dimethylamino)pyrimidin-2-yl]-amino}cyclohexyl)methyl]urea;
N-[(cis-4-{[4-(dimethylamino)pyrimidin-2-yl]amino}cyclohexyl)methyl]-N'-(2-methoxy-5-methylphenyl)urea;
N-[(cis-4-{[4-(dimethylamino)pyrimidin-2-yl]amino}cyclohexyl)methyl]-N'-(2-methyl-6-nitrophenyl)urea;
N-(3,4-difluorophenyl)-N'-[(cis-4-{[4-(dimethylamino)pyrimidin-2-yl]-amino}cyclohexyl)methyl]urea;
N-(3,5-difluorophenyl)-N'-[(cis-4-{[4-(dimethylamino)pyrimidin-2-yl]-amino}cyclohexyl)methyl]urea; and
N-(3-chloro-4-fluorophenyl)-N'-[(cis-4-{[4-(dimethylamino)pyrimidin-2-yl]amino}cyclohexyl)methyl]urea;

or a pharmaceutically acceptable salt, hydrate, or solvate thereof.

**69.** The compound according to claim 52 wherein $R_1$ is selected from the group consisting of:

(i) $C_{1-5}$ alkyl, and
$C_{1-5}$ alkyl substituted by substituent(s) independently selected from the group consisting of:

- carbocyclic aryl,
- carbocyclic aryl substituted by substituent(s) independently selected from the group consisting of:

  - •• halogen, and
  - •• $C_{1-5}$ alkoxy,

(ii) carbocyclyl,
(iii) carbocyclic aryl, and
carbocyclic aryl substituted by substituent(s) independently selected from the group consisting of:

- halogen,
- cyano,
- nitro,
- $C_{1-5}$ alkyl,
- $C_{1-5}$ alkyl substituted by halogen,
- $C_{1-5}$ alkoxy carbonyl,
- $C_{1-5}$ alkoxy,
- $C_{1-5}$ alkoxy substituted by halogen,
- mono-$C_{1-5}$ alkylamino,
- di-$C_{1-5}$ alkylamino, and
- carbocyclic aryl,

(iv) heterocyclyl, and
heterocyclyl substituted by substituent(s) independently selected from the group consisting of:

- $C_{1-5}$ alkyl,
- $C_{1-5}$ alkoxy carbonyl, and
- carbocyclic aryl;

L is Formula (VII);
Y is -C(S)$NR_5$-;
wherein carbocyclic aryl is phenyl or naphthyl;
carbocyclyl is bicyclo[2.2.1]heptyl;
heterocyclyl is 2,3-dihydro-benzo[1,4]dioxinyl, benzo[1,3]dioxolyl, isoxazolyl, or thienyl; and
halogen is fluoro, chloro, bromo, or iodo;

or a pharmaceutically acceptable salt, hydrate, or solvate thereof.

**70.** The compound according to claim 69 wherein $R_2$ is methylamino or dimethylamino; p is 0; $R_3$ and $R_4$ are hydrogen; A is a single bond; B is a single bond or -$CH_2$-; $R_5$ is hydrogen;
or a pharmaceutically acceptable salt, hydrate, or solvate thereof.

**71.** The compound according to claim 70 wherein $R_1$ is selected from the group consisting of:

carbocyclic aryl, and
carbocyclic aryl substituted by substituent(s) independently selected from the group consisting of:

- halogen,
- cyano,
- $C_{1-5}$ alkyl,
- $C_{1-5}$ alkoxy,
- mono-$C_{1-5}$ alkylamino, and
- di-$C_{1-5}$ alkylamino;

wherein carbocyclic aryl is phenyl or naphthyl; and
halogen is fluoro, chloro, bromo, or iodo;

or a pharmaceutically acceptable salt, hydrate, or solvate thereof.

**72.** The compound according to claim 1 selected from the group consisting of:

N-(4-cyanophenyl)-N'-(cis-4-{[4-(dimethylamino)pyrimidin-2-yl]amino}-cyclohexyl)thiourea;
N-(2,4-dimethoxyphenyl)-N'-(cis-4-{[4-(dimethylamino)pyrimidin-2-yl]-amino}cyclohexyl)thiourea;
N-(cis-4-{[4-(dimethylamino)pyrimidin-2-yl]amino}cyclohexyl)-N'-(3,4,5-trimethoxyphenyl)thiourea;
N-(3,4-dimethoxyphenyl)-N'-(cis-4-{[4-(dimethylamino)pyrimidin-2-yl]-amino}cyclohexyl)thiourea;
N-[4-(dimethylamino)-1-naphthyl]-N'-(cis-4-{[4-(dimethylamino)-pyrimidin-2-yl]amino}cyclohexyl)thiourea;
N-(cis-4-{[4-(dimethylamino)pyrimidin-2-yl]amino}cyclohexyl)-N'-(2,4,6-tribromophenyl)thiourea;
N-(cis-4-{[4-(dimethylamino)pyrimidin-2-yl]amino}cyclohexyl)-N'-mesitylthiourea;
N-(4-bromo-2,6-dimethylphenyl)-N'-(cis-4-{[4-(dimethylamino)pyrimidin-2-yl]amino}cyclohexyl)thiourea;
N-(5-chloro-2,4-dimethoxyphenyl)-N'-(cis-4-{[4-(dimethylamino)-pyrimidin-2-yl]amino}cyclohexyl)thiourea;
N-(2,4-dibromo-6-fluorophenyl)-N'-(cis-4-{[4-(dimethylamino)pyrimidin-2-yl]amino}cyclohexyl)thiourea; and
N-(2,4-dichloro-6-methylphenyl)-N'-(cis-4-{[4-(dimethylamino)pyrimidin-2-yl]amino}cyclohexyl)thiourea;

or a pharmaceutically acceptable salt, hydrate, or solvate thereof.

**73.** The compound according to claim 52 wherein $R_1$ is selected from the group consisting of:

(i) $C_{1-8}$ alkyl, and
$C_{1-8}$ alkyl substituted by substituent(s) independently selected from the group consisting of:

- halogen,
- $C_{1-5}$ alkoxy,
- $C_{1-5}$ alkoxy substituted by carbocyclic aryl,
- carbocyclyl,
- carbocyclic aryl,
- Carbocyclic aryl substituted by substituent(s) independently selected from the group consisting of:
  - •• halogen,
  - •• nitro, and
  - •• $C_{1-5}$ alkoxy,

(ii) $C_{2-5}$ alkenyl,
(iii) carbocyclyl,
(iv) carbocyclic aryl, and
carbocyclic aryl substituted by substituent(s) independently selected from the group consisting of:

- halogen,
- $C_{1-5}$ alkyl,
- $C_{1-5}$ alkyl substituted by halogen, and
- $C_{1-5}$ alkoxy;

L is Formula (VII);

Y is -C(O)O-;
wherein carbocyclic aryl is phenyl or naphthyl;
carbocyclyl is 9*H*-fluorenyl or menthyl; and
halogen is fluoro, chloro, bromo, or iodo;

or a pharmaceutically acceptable salt, hydrate, or solvate thereof.

**74.** The compound according to claim 73 wherein $R_2$ is methylamino or dimethylamino; p is 0; $R_3$ and $R_4$ are hydrogen; A is a single bond; B is a single bond or $-CH_2-$:
or a pharmaceutically acceptable salt, hydrate, or solvate thereof.

**75.** The compound according to claim 2 wherein Q is Formula (IV); p is 1 or 2;
$R_1$ is selected from the group consisting of:

(i) $C_{1-16}$ alkyl, and
$C_{1-16}$ alkyl substituted by substituent(s) independently selected from the group consisting of:

- hydroxy,
- oxo,
- carbocyclic aryloxy,
- carbocyclic aryloxy substituted by substituent(s) independently selected from the group consisting of:

  •• halogen,
  •• $C_{1-5}$ alkyl,
  •• $C_{1-5}$ alkyl substituted by halogen, and
  •• $C_{1-5}$ alkoxy,

- heterocyclyloxy,
- heterocyclyloxy substituted by substituent(s) independently selected from the group consisting of:

  •• halogen,
  •• $C_{1-5}$ alkyl, and
  •• $C_{1-5}$ alkyl substituted by halogen,

- mono-carbocyclic arylamino,
- mono-carbocyclic arylamino substituted by substituent(s) independently selected from the group consisting of:

  •• halogen,
  •• $C_{1-5}$ alkoxy, and
  •• $C_{1-5}$ alkyl,

- carbocyclic arylsulfinyl,
- carbocyclic arylsulfinyl substituted by substituent(s) independently selected from the group consisting of:

  •• halogen,
  •• $C_{1-5}$ alkyl, and
  •• $C_{1-5}$ alkyl substituted by halogen,

- carbocyclic arylsulfonyl,
- carbocyclic arylsulfonyl substituted by substituent(s) independently selected from the group consisting of:

  •• halogen,
  •• $C_{1-5}$ alkyl, and
  •• $C_{1-5}$ alkyl substituted by halogen,

- carbocyclic aryl,
- carbocyclic aryl substituted by substituent(s) independently selected from the group consisting of:

- - halogen,
  - nitro,
  - $C_{1-5}$ alkylcarbonylamino,
  - $C_{3-6}$ cycloalkylcarbonylamino,
  - $C_{1-5}$ alkyl,
  - $C_{1-5}$ alkyl substituted by halogen,
  - $C_{1-5}$ alkoxy, and
  - $C_{1-5}$ alkoxy substituted by halogen, and

- heterocyclyl,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by substitutent(s) independently selected from the group consisting of:
  - $C_{1-5}$ alkoxy,
  - halogen,
  - $C_{1-5}$ alkyl, and
  - $C_{1-5}$ alkyl substituted by halogen,

(iii) carbocyclic aryl, and
carbocyclic aryl substituted by substituent(s) independently selected from the group consisting of:

- halogen,
- cyano,
- nitro,
- $C_{1-10}$ alkyl,
- $C_{1-10}$ alkyl substituted by substituent(s) independently selected from the group consisting of:
  - halogen, and
  - hydroxy,
- $C_{1-9}$ alkoxy,
- $C_{1-9}$ alkoxy substituted by substituent(s) independently selected from the group consisting of:
  - halogen, and
  - carbocyclic aryl,
- carboxy,
- $C_{1-5}$ alkoxycarbonyl,
- di-$C_{1-5}$ alkylamino,
- $C_{1-5}$ alkylcarbonylamino,
- $C_{3-6}$ cycloalkylcarbonylamino,
- $C_{1-5}$ alkylthio,
- $C_{1-5}$ alkylsulfinyl,
- $C_{1-5}$ alkylsulfonyl,
- carbocyclic aryl,

(iv) heterocyclyl, and
heterocyclyl substituted by substituent(s) independently selected from the group consisting of:

- halogen,
- hydroxy,
- amino,
- $C_{1-5}$ alkyl,
- $C_{1-5}$ alkyl substituted by halogen,

- $C_{1-5}$ alkoxy,
- carbocyclic aryloxy,
- carbocyclic aryloxy substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy,

- heterocyclyloxy,
- heterocyclyloxy substituted by halogen,
- heterocyclyl sulfonyl,
- heterocyclyl sulfonyl substituted by $C_{1-5}$ alkyl,
- mono-carbocyclic arylamino,
- mono-carbocyclic arylamino substituted by halogen,
- $C_{1-5}$ alkylthio,
- $C_{1-5}$ alkylsulfinyl,
- carbocyclic arylsulfinyl,
- carbocyclic arylsulfinyl substituted by halogen,
- carbocyclic arylsulfonyl,
- carbocyclic arylsulfonyl substituted by substituents(s) independently selected from the group consisting of:

  - •• halogen,
  - •• $C_{1-5}$ alkoxy,
  - •• $C_{1-5}$ alkyl, and
  - •• $C_{1-5}$ alkyl substituted by halogen,

$R_2$ is selected from the group consisting of:

amino, $C_{1-5}$ alkyl, $C_{1-5}$ alkoxy, $-N(R_{2a})(R_{2b})$, wherein $R_{2a}$ is hydrogen or $C_{1-5}$ alkyl and $R_{2b}$ is $C_{1-5}$ alkyl or $C_{3-6}$ cycloalkyl;

wherein carbocyclic aryl is phenyl or naphthyl;

heterocyclyl is 3,4-dihydro-1*H*-isoquinolinyl, benzo[1,3]dioxolyl, furyl, isoxazolyl, oxazolyl, pyrazolyl, pyrazinyl, pyridyl, pyrimidyl, or thienyl; and

halogen is fluoro, chloro, bromo, or iodo;

or a pharmaceutically acceptable salt, hydrate, or solvate thereof.

**76.** The compound according to claim 75 wherein $R_1$ is selected from the group consisting of:

(i) $C_{1-6}$ alkyl, and
$C_{1-6}$ alkyl substituted by substituent(s) independently selected from the group consisting of:

- oxo,
- carbocyclic aryl,
- carbocyclic aryl substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy, and
  - •• $C_{1-5}$ alkoxy substituted by halogen,

(ii) heterocyclyl, and
heterocyclyl substituted by substituent(s) independently selected from the group consisting of:

- carbocyclic arylsulfinyl, and
- carbocyclic arylsulfinyl substituted by halogen,

L is Formula (VII);
Y is a single bond or $-CH_2-$;
$R_2$ is $-N(R_{2a})(R_{2b})$, wherein $R_{2a}$ is $C_{1-5}$ alkyl and $R_{2b}$ is $C_{1-5}$ alkyl;
carbocyclic aryl is phenyl;
heterocyclyl is pyrazinyl; and
halogen is fluoro, chloro, or bromo;

or a pharmaceutically acceptable salt, hydrate, or solvate thereof.

**77.** The compound according to claim 76 wherein $R_1$ is selected from the group consisting of:

(i) $C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:

  - •• halogen, and
  - •• $C_{1-5}$ alkoxy,

(ii) heterocyclyl, and
heterocyclyl substituted by substituent(s) independently selected from the group consisting of:

- carbocyclic arylsulfinyl, and
- carbocyclic arylsulfinyl substituted by halogen,

$R_2$ is $-N(R_{2a})(R_{2b})$, wherein $R_{2a}$ is $C_{1-5}$ alkyl and $R_{2b}$ is $C_{1-5}$ alkyl;
carbocyclic aryl is phenyl;
heterocyclyl is pyrazinyl; and
halogen is fluoro or bromo;

or a pharmaceutically acceptable salt, hydrate, or solvate thereof.

**78.** The compound according to claim 77 wherein R, is selected from the group consisting of:

heterocyclyl, and
heterocyclyl substituted by substituent(s) independently selected from the group consisting of:

- carbocyclic arylsulfinyl, and
- carbocyclic arylsulfinyl substituted by halogen,

$R_2$ is $-N(R_{2a})(R_{2b})$, wherein $R_{2a}$ is $C_{1-5}$ alkyl and $R_{2b}$ is $C_{1-5}$ alkyl;
carbocyclic aryl is phenyl;
heterocyclyl is pyrazinyl; and
halogen is fluoro;

or a pharmaceutically acceptable salt, hydrate, or solvate thereof.

**79.** The compound according to any one of claims 76 to 78 wherein p is 1 and T is $C_{1-5}$ alkyl; $R_3$ and $R_4$ are both hydrogen; A and B are both single bonds:
or a pharmaceutically acceptable salt, hydrate, or solvate thereof.

**80.** The compound according to claim 1 selected from the group consisting of:

$N^2$-{cis-4-[(3,5-dimethoxybenzyl)amino]cyclohexyl}-$N^4$,$N^4$,5-trimethylpyrimidine-2,4-diamine;
$N^2$-{cis-4-[(3-bromobenzyl)amino]cyclohexyl}-$N^4$,$N^4$,5,6-tetramethylpyrimidine-2,4-diamine;
$N^2$-{cis-4-[(3,4-difluorobenzyl)amino]cyclohexyl}-$N^4$,$N^4$,5,6-tetramethylpyrimidine-2,4-diamine; and
$N^2$-[cis-4-({6-[(3,4-difluorophenyl)sulfinyl]pyrazin-2-yl}amino)cyclohexyl]-$N^4$,$N^4$,5-trimethylpyrimidine-2,4-diamine;

or a pharmaceutically acceptable salt, hydrate, or solvate thereof.

**81.** The compound according to claim 1 is:

$N^2$-{cis-4-({6-[(3,4-difluorophenyl)sulfinyl]pyrazin-2-yl}amino)cyclohexyl]-$N^4$,$N^4$,5-trimethylpyrimidine-2,4-diamine;

or a pharmaceutically acceptable salt, hydrate, or solvate thereof.

**82.** The compound according to claim 75 wherein $R_1$ is selected from the group consisting of:

(i) $C_{1-16}$ alkyl, and
$C_{1-16}$ alkyl substituted by substituent(s) independently selected from the group consisting of:

- hydroxy,
- carbocyclic aryloxy,
- carbocyclic aryloxy substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy,

- heterocyclyloxy,
- heterocyclyloxy substituted by substituent(s) independently selected from the group consisting of:

  - •• halogen,
  - •• $C_{1-5}$ alkyl, and
  - •• $C_{1-5}$ alkyl substituted by halogen,

- mono-carbocyclic arylamino,
- mono-carbocyclic arylamino substituted by substituent(s) independently selected from the group consisting of:

  - •• halogen,
  - •• $C_{1-5}$ alkoxy, and
  - •• $C_{1-5}$ alkyl,

- carbocyclic arylsulfinyl,
- carbocyclic arylsulfinyl substituted by substituent(s) independently selected from the group consisting of:

  - •• halogen,
  - •• $C_{1-5}$ alkyl, and
  - •• $C_{1-5}$ alkyl substituted by halogen,

- carbocyclic arylsulfonyl,
- carbocyclic arylsulfonyl substituted by substituent(s) independently selected from the group consisting of:

  - •• halogen,
  - •• $C_{1-5}$ alkyl, and
  - •• $C_{1-5}$ alkyl substituted by halogen,

- carbocyclic aryl,
- carbocyclic aryl substituted by substituent(s) independently selected from the group consisting of:

  - •• halogen,

- - $C_{1-5}$ alkyl,
  - $C_{1-5}$ alkyl substituted by halogen, and
  - $C_{1-5}$ alkoxy,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by substitutent(s) independently selected from the group consisting of:
  - $C_{1-5}$ alkoxy,
  - halogen,
  - $C_{1-5}$ alkyl, and
  - $C_{1-5}$ alkyl substituted by halogen,

(iii) carbocyclic aryl, and
carbocyclic aryl substituted by substituent(s) independently selected from the group consisting of:

- halogen,
- cyano,
- nitro,
- $C_{1-10}$ alkyl,
- $C_{1-10}$ alkyl substituted by substituent(s) independently selected from the group consisting of:
  - halogen, and
  - hydroxy,
- $C_{1-9}$ alkoxy,
- $C_{1-9}$ alkoxy substituted by halogen,
- carboxy,
- $C_{1-5}$ alkoxycarbonyl,
- di-$C_{1-5}$ alkylamino,
- $C_{1-5}$ alkylcarbonylamino,
- $C_{3-6}$ cycloalkylcarbonylamino,
- $C_{1-5}$ alkylsulfonyl, and
- carbocyclic aryl,

(iv) heterocyclyl, and
heterocyclyl substituted by substituent(s) independently selected from the group consisting of:

- halogen,
- hydroxy,
- amino,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by substituent(s) independently selected from the group consisting of:
  - halogen,
  - $C_{1-5}$ alkyl,
  - $C_{1-5}$ alkyl substituted by halogen, and
  - $C_{1-5}$ alkoxy,
- heterocyclyloxy,
- heteroeyclyloxy substituted by halogen,
- heterocyclyl sulfonyl,
- heterocyclyl sulfonyl substituted by $C_{1-5}$ alkyl,

- mono-carbocyclic arylamino,
- mono-carbocyclic arylamino substituted by halogen,
- $C_{1-5}$ alkylthio,
- $C_{1-5}$ alkylsulfinyl,
- carbocyclic arylsulfonyl,
- carbocyclic arylsulfonyl substituted by substituents(s) independently selected from the group consisting of:

  - •• halogen,
  - •• $C_{1-5}$ alkoxy,
  - •• $C_{1-5}$ alkyl, and
  - •• $C_{1-5}$ alkyl substituted by halogen,

L is Formula (VII);
Y is -C(O)-;
$R_2$ is selected from the group consisting of:

amino, $C_{1-5}$ alkyl, $C_{1-5}$ alkoxy, $-N(R_{2a})(R_{2b})$, wherein $R_{2a}$ is hydrogen or $C_{1-5}$ alkyl and $R_{2b}$ is $C_{1-5}$ alkyl or $C_{3-6}$ cycloalkyl;

wherein carbocyclic aryl is phenyl;
heterocyclyl is benzo[1,3]dioxolyl, furyl, isoxazolyl, oxazolyl, pyrazolyl, pyrazinyl, pyridyl, pyrimidyl, or thienyl; and
halogen is fluoro, chloro, bromo, or iodo;

or a pharmaceutically acceptable salt, hydrate, or solvate thereof.

**83.** The compound according to claim 82 wherein $R_1$ is selected from the group consisting of:

(i) $C_{1-6}$ alkyl, and
$C_{1-6}$ alkyl substituted by substituent(s) independently selected from the group consisting of:

- hydroxy,
- carbocyclic aryloxy,
- carbocyclic aryloxy substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy,

- heterocyclyloxy,
- heterocyclyloxy substituted by halogen,
- mono-carbocyclic arylamino,
- mono-carbocyclic arylamino substituted by substituent(s) independently selected from the group consisting of:

  - •• halogen,
  - •• $C_{1-5}$ alkoxy, and
  - •• $C_{1-5}$ alkyl,

- carbocyclic arylsulfinyl,
- carbocyclic arylsulfinyl substituted by substituent(s) independently selected from the group consisting of:

  - •• halogen,
  - •• $C_{1-5}$ alkyl, and
  - •• $C_{1-5}$ alkyl substituted by halogen,

- carbocyclic arylsulfonyl,

- carbocyclic arylsulfonyl substituted by substituent(s) independently selected from the group consisting of:
  - $C_{1-5}$ alkyl, and
  - $C_{1-5}$ alkyl substituted by halogen,
- carbocyclic aryl,
- carbocyclic aryl substituted by substituent(s) independently selected from the group consisting of:
  - halogen,
  - $C_{1-5}$ alkyl, and
  - $C_{1-5}$ alkyl substituted by halogen,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by substitutent(s) independently selected from the group consisting of:
  - $C_{1-5}$ alkoxy,
  - halogen,
  - $C_{1-5}$ alkyl, and
  - $C_{1-5}$ alkyl substituted by halogen,

(iii) carbocyclic aryl, and
carbocyclic aryl substituted by substituent(s) independently selected from the group consisting of:

- halogen,
- cyano,
- nitro,
- $C_{1-10}$ alkyl,
- $C_{1-10}$ alkyl substituted by substituent(s) independently selected from the group consisting of:
  - halogen, and
  - hydroxy,
- $C_{1-9}$ alkoxy,
- $C_{1-9}$ alkoxy substituted by halogen,
- carboxy,
- $C_{1-5}$ alkoxycarbonyl, and
- $C_{1-5}$ alkylsulfonyl,

(iv) heterocyclyl, and
heterocyclyl substituted by substituent(s) independently selected from the group consisting of:

- halogen,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by substituent(s) independently selected from the group consisting of:
  - halogen,
  - $C_{1-5}$ alkyl,
  - $C_{1-5}$ alkyl substituted by halogen, and
  - $C_{1-5}$ alkoxy,
- heterocyelyloxy,
- heterocyelyloxy substituted by halogen,

- heterocyclyl sulfonyl,
- heterocyclyl sulfonyl substituted by $C_{1-5}$ alkyl,
- mono-carbocyclic arylamino,
- mono-carbocyclic arylamino substituted by halogen,
- $C_{1-5}$ alkylthio,
- carbocyclic arylsulfonyl,
- carbocyclic arylsulfonyl substituted by substituents(s) independently selected from the group consisting of:

  - •• halogen,
  - •• $C_{1-5}$ alkyl, and
  - •• $C_{1-5}$ alkyl substituted by halogen,

$R_2$ is selected from the group consisting of:

$C_{1-5}$ alkoxy, $-N(R_{2a})(R_{2b})$, wherein $R_{2a}$ is hydrogen or $C_{1-5}$ alkyl and $R_{2b}$ is $C_{1-5}$ alkyl; wherein carbocyclic aryl is phenyl;
heterocyclyl is benzo[1,3]dioxolyl, furyl, isoxazolyl, oxazolyl, pyrazolyl, pyridyl, or thienyl; and
halogen is fluoro, chloro, bromo, or iodo;

or a pharmaceutically acceptable salt, hydrate, or solvate thereof.

**84.** The compound according to claim 83 wherein R, is selected from the group consisting of:

(i) $C_{1-16}$ alkyl, and
$C_{1-16}$ alkyl substituted by substituent(s) independently selected from the group consisting of:

- hydroxy,
- carbocyclic aryloxy,
- carbocyclic aryloxy substituted by substituent(s) independently selected from the group consisting of:

  - •• halogen,
  - •• $C_{1-5}$ alkyl,
  - •• $C_{1-5}$ alkyl substituted by halogen, and
  - •• $C_{1-5}$ alkoxy,

- heterocyclyloxy,
- heterocyclyloxy substituted by substituent(s) independently selected from the group consisting of:

  - •• halogen,
  - •• $C_{1-5}$ alkyl, and
  - •• $C_{1-5}$ alkyl substituted by halogen,

- mono-carbocyclic arylamino,
- mono-carbocyclic arylamino substituted by substituent(s) independently selected from the group consisting of:

  - •• halogen,
  - •• $C_{1-5}$ alkoxy, and
  - •• $C_{1-5}$ alkyl,

- carbocyclic arylsulfinyl,
- carbocyclic arylsulfinyl substituted by substituent(s) independently selected from the group consisting of:

  - •• halogen,
  - •• $C_{1-5}$ alkyl, and
  - •• $C_{1-5}$ alkyl substituted by halogen,

- carbocyclic aryl,

- carbocyclic aryl substituted by substituent(s) independently selected from the group consisting of:
  - halogen,
  - $C_{1-5}$ alkyl, and
  - $C_{1-5}$ alkyl substituted by halogen,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by substitutent(s) independently selected from the group consisting of:
  - $C_{1-5}$ alkoxy,
  - halogen,
  - $C_{1-5}$ alkyl, and
  - $C_{1-5}$ alkyl substituted by halogen,

(iii) carbocyclic aryl, and
carbocyclic aryl substituted by substituent(s) independently selected from the group consisting of:

- halogen,
- cyano,
- nitro,
- $C_{1-10}$ alkyl,
- $C_{1-10}$ alkyl substituted by halogen,
- $C_{1-9}$ alkoxy, and
- $C_{1-9}$ alkoxy substituted by halogen,

(iv) heterocyclyl, and
heterocyclyl substituted by substituent(s) independently selected from the group consisting of:

- halogen,
- $C_{1-5}$ alkyl,
- $C_{1-5}$ alkyl substituted by halogen,
- $C_{1-5}$ alkoxy,
- carbocyclic aryloxy,
- carbocyclic aryloxy substituted by substituent(s) independently selected from the group consisting of:
  - halogen,
  - $C_{1-5}$ alkyl,
  - $C_{1-5}$ alkyl substituted by halogen, and
  - $C_{1-5}$ alkoxy,
- $C_{1-5}$ alkylthio,
- carbocyclic arylsulfonyl,
- carbocyclic arylsulfonyl substituted by halogen,

$R_2$ is selected from the group consisting of:

$-N(R_{2a})(R_{2b})$, wherein $R_{2a}$ is hydrogen or $C_{1-5}$ alkyl and $R_{2b}$ is $C_{1-5}$ alkyl;
wherein carbocyclic aryl is phenyl;
heterocyclyl is benzo[1,3]dioxolyl, furyl, pyrazolyl, pyridyl, or thienyl; and
halogen is fluoro, chloro, bromo, or iodo;

or a pharmaceutically acceptable salt, hydrate, or solvate thereof.

**85.** The compound according to any one of claims 82 to 84 wherein p is 1 and T is $C_{1-5}$ alkyl; $R_3$ and $R_4$ are both hydrogen; A is a single bond and B is a single bond or $-CH_2-$;

or a pharmaceutically acceptable salt, hydrate, or solvate thereof.

**86.** The compound according to claim 1 selected from the group consisting of:

N-[(cis-4-{[4-(dimethylamino)-5-methylpyrimidin-2-yl]amino}cyclohexyl)methyl]-3,5-bis(trifluoromethyl)benzamide;
N-[(cis-4-{[4-(dimethylamino)-6-methylpyrimidin-2-yl]amino}cyclohexyl)methyl]-3,5-bis(trifluoromethyl)benzamide;
N-[(cis-4-{[4-(dimethylamino)-6-methylpyrimidin-2-yl]amino}cyclohexyl)methyl]-3,4-difluorobenzamide;
3,5-dichloro-N-[(cis-4-{[4-(dimethylamino)-6-methylpyrimidin-2-yl]amino}cyclohexyl)methyl]benzamide;
N-[(cis-4-{[4-(dimethylamino)-5-methylpyrimidin-2-yl]amino}cyclohexyl)methyl]-3,4-difluorobenzamide;
N-[(cis-4-{[4-(dimethylamino)-5-methylpyrimidin-2-yl]amino}cyclohexyl)methyl]-3,5-dimethoxybenzamide;
N-[(cis-4-{[4-(dimethylamino)-5-methylpyrimidin-2-yl]amino}cyclohexyl)methyl]-3-fluoro-4-methylbenzamide;
N-[(cis-4-{[4-(dimethylamino)-5-methylpyrimidin-2-yl]amino}cyclohexyl)methyl]-3-(trifluoromethyl)benzamide;
N-[(cis-4-{[4-(dimethylamino)-5-methylpyrimidin-2-yl]amino}cyclohexyl)methyl]-3-(trifluoromethoxy)benzamide;
4-bromo-N-[(cis-4-{[4-(dimethylamino)-5-methylpyrimidin-2-yl]amino}cyclohexyl)methyl]-3-methylbenzamide;
N-[(cis-4-{[4-(dimethylamino)-5-methylpyrimidin-2-yl]amino}cyclohexyl]methyl]-3-fluoro-4-(trifluoromethyl) benzamide;
3,5-dichloro-N-[(cis-4-3{[4-(dimethylamino)-5-methylpyrimidin-2-yl]amino}cyclohexyl)methyl]benzamide;
3,4-dichloro-N-[(cis-4-{[4-(dimethylamino)-5-methylpyrimidin-2-yl]amino}cyclohexyl)methyl]benzamide;
4-chloro-N-[(cis-4-{(4-(dimethylamino)-5-methylpyrimidin-2-yl]amino)cyclohexyl)methyl]benzamide;
4-chloro-N-[(cis-4-{[4-(dimethylamino)-6-methylpyrimidin-2-yl]amino}cyclohexyl)methyl]benzamide;
N-[cis-4-({[4-(dimethylamino)-5-methylpyrimidin-2-yl]amino}methyl)cyclohexyl]-3,5-dimethoxybenzamide;
4-bromo-N-[cis-4-({[4-(dimethylamino)-5-methylpyrimidin-2-yl]amino}methyl)cyclohexyl]benzamide;
4-bromo-N-[cis-4-({[4-(dimethylamino)-5-methylpyrimidin-2-yl]amino}methyl)cyclohexyl]-3-methylbenzamide;
3,5-dichloro-N-(cis-4-({[4-(dimethylamino)-5-methylpyrimidin-2-yl]amino}methyl)cyclohexyl]benzamide;
3,4-dichloro-N-[cis-4-({[4-(dimethylamino)-5-methylpyrimidin-2-yl]amino}methyl)cyclohexyl]benzamide;
N-[cis-4-({[4-(dimethylamino)-5-methylpyrimidin-2-yl]amino}methyl)cyclohexyl]-3,5-bis(trifluoromethyl)benzamide;
N-[cis-4-({[4-(dimethylamino)-6-methylpyrimidin-2-yl]amino}methyl)cyclohexyl]-3,4-difluorobenzamide;
4-bromo-N-[cis-4-({[4-(dimethylamino)-6-methylpyrimidin-2-yl]amino}methyl)cyclohexyl]benzamide;
4-bromo-N-[cis-4-({[4-(dimethylamino)-6-methylpyrimidin-2-yl]amino}methyl)cyclohexyl]-3-methylbenzamide;
N-(cis-4-{[4-(dimethylamino)-5-methylpyrimidin-2-yl]amino}cyclohexyl)-2-(2-fluorophenoxy)nicotinamide;
N-(cis-4-{[4-(dimethylamino)-6-methylpyrimidin-2-yl]amino}cyclohexyl)-3,4,5-trimethoxybenzamide;
N-(4-{[4-(dimethylamino)-6-methylpyrimidin-2-yl]amino}cyclohexyl)-3-nitrobenzamide;
N-(cis-4-{[4-(dimethylamino)-6-methylpyrimidin-2-yl]amino}cyclohexyl)-2,2-diphenylacetamide;
N-(cis-4-{[4-(dimethylamino)-6-methylpyrimidin-2-yl]amino}cyclohexyl)-4-methylbenzamide;
4-chloro-N-(cis-4-{[4-(dimethylamino)-6-methylpyrimidin-2-yl]amino}cyclohexyl)benzamide;
3-chloro-N-(cis-4-{[4-(dimethylamino)-6-methylpyrimidin-2-yl]amino}cyclohexyl)benzamide;
N-(cis-4-{[4-(dimethylamino)-6-methylpyrimidin-2-yl]amino}cyclohexyl)-3,4-difluorobenzamide;
N-(cis-4-{[4-(dimethylamino)-6-methylpyrimidin-2-yl]amino}cyclohexyl)-3-methylbenzamide;
N-(cis-4-{[4-(dimethylamino)-6-methylpyrimidin-2-yl]amino}cyclohexyl)-3-methoxybenzamide;
N-(cis-4-{[4-(dimethylamino)-6-methylpyrimidin-2-yl]amino}cyclohexyl)-4-fluorobenzamide;
N-(cis-4-{[4-(dimethylamino)-5-methylpyrimidin-2-yl]amino}cyclohexyl)-3-methylbenzamide;
N-(cis-4-{[4-(dimethylamino)-5-methylpyrimidin-2-yl]amino}cyclohexyl)-3-methoxybenzamide;
N-(cis-4-{[4-(dimethylamino)-5-methylpyrimidin-2-yl]amino}cyclohexyl)-4-methylbenzamide;
N-(cis-4-{[4-(dimethylamino)-5-methylpyrimidin-2-yl]amino}cyclohexyl)-3,4-difluorobenzamide;
3-chloro-N-(cis-4-{[4-(dimethylamino)-5-methylpyrimidin-2-yl]amino}cyclohexyl)benzamide;
N-(cis-4-{[4-(dimethylamino)-5-methylpyrimidin-2-yl]amino}cyclohexyl)-2-(3-methylphenoxy)nicotinamide;
2-(4-bromophenoxy)-N-(cis-4-{[4-(dimethylamino)-5-methylpyrimidin-2-yl]amino}cyclohexyl)nicotinamide;
2-(4-chlorophenoxy)-N-(cis-4-{[4-(dimethylamino)-5-methylpyrimidin-2-yl]amino}cyclohexyl)nicotinamide;
N-(cis-4-{[4-(dimethylamino)-5-methylpyrimidin-2-yl]amino}cyclohexyl)-2-(4-fluorophenoxy)nicotinamide;
N-(cis-4-{[4-(dimethylamino)-5-methylpyrimidin-2-yl]amino}cyclohexyl)-2-(3-fluorophenoxy)nicotinamide;

2-(2-bromophenoxy)-N-(cis-4-{[4-(dimethylamino)-5-methylpyrimidin-2-yl]amino}cyclohexyl)nicotinamide;
N-(cis-4-{[4-(dimethylamino)-5-methylpyrimidin-2-yl]amino}cyclohexyl)-2-(3-methoxyphenoxy)nicotinamide;
N-(cis-4-{[4-(dimethylamino)-5-methylpyrimidin-2-yl]amino}cyclohexyl)-2-(4-methoxyphenoxy)nicotinamide;
N-(cis-4-{[4-(dimethylamino)-5-methylpyrimidin-2-yl]amino}cyclohexyl)-2-(4-iodophenoxy)nicotinamide;
2-(3,4-dichlorophenoxy)-N-(cis-4-{[5-methyl-4-(methylamino)pyrimidin-2-yl]amino}cyclohexyl)acetamide;
2-(2,3-dichlorophenoxy)-N-(cis-4-{[5-methyl-4-(methylamino)pyrimidin-2-yl]amino}cyclohexyl)acetamide;
2-[(3,4-difluorophenyl)sulfonyl]-N-(cis-4-{[4-(dimethylamino)-5-methylpyrimidin-2-yl]amino}cyclohexyl)nicotinamide;
N-(cis-4-([4-(dimethylamino)-5-ethylpyrimidin-2-yl]amino}cyclohexyl)-3,4-difluorobenzamide;
N-[cis-4-{{4-[ethyl(methyl)amino]-5-methylpyrimidin-2-yl}amino)cyclohexyl]-3,4-difluorobenzamide;
N-(cis-4-{[4-(dimethylamino)-6-methylpyrimidin-2-yl]amino}cyclohexyl)-3,5-dimethoxybenzamide;
N-(cis-4-{[4-(dimethylamino)-5-methylpyrimidin-2-yl]amino}cyclohexyl)-2-(2-methoxyphenoxy)nicotinamide;
2-(2-chlorophenoxy)-N-(cis-4-{[4-(dimethylamino)-5-methylpyrimidin-2-yl]amino}cyclohexyl)nicotinamide;
2-(3-chlorophenoxy)-N-(cis-4-{[4-(dimethylamino)-5-methylpyrimidin-2-yl]amino}cydohexyl)nicotinamide;
2-(3-bromophenoxy)-N-(cis-4-{[4-(dimethylamino)-5 -methylpyrimidin-2-yl]amino}cyclohexyl)nicotinamide;
N-(cis-4-{[4-(dimethylamino)-5-methylpyrimidin-2-yl]amino}cyclohexyl)-2-[3-(trifluoromethyl)phenoxy]nicotinamide;
N-(cis-4-{[4-(dimethylamino)-5-methylpyrimidin-2-yl]amino}cyclohexyl)-2-(3-fluorophenoxy)acetamide;
N-(cis-4-{[4-(dimethylamino)-5-methylpyrimidin-2-yl]amino}cyclohexyl)-2-(3-methoxyphenoxy)acetamide;
N-(cis-4-{[4-(dimethylamino)-5-methylpyrimidin-2-yl]amino}cyclohexyl)-2-[3-(trifluoromethyl)phenoxy]acetamide;
2-(3-chlorophenoxy)-N-(cis-4-{[4-(dimethylamino)-5-methylpyrimidin-2-yl]amino}cyclohexyl)acetamide;
2-[(5-chloropyridin-3-yl)oxy]-N-(cis-4-{[4-(dimethylamino)-5-methylpyrimidin-2-yl]amino}cyclohexyl)acetamide;
N-(cis-4-{[4-(dimethylamino)-5,6-dimethylpyrimidin-2-yl]amino}cyclohexyl)-3,4-difluorobenzamide;
2-(3,4-difluorophenyl)-N-(cis-4-{[4-(dimethylamino)-5-methylpyrimidin-2-yl]amino}cyclohexyl)-2-hydroxyacetamide;
N-(cis-4-{[4-(dimethylamino)-5-methylpyrimidin-2-yl]amino}cyclohexyl)-2-hydroxy-2-(4-methoxyphenyl) acetamide;
2-(2,3-difluorophenyl)-N-(cis-4-{[4-(dimethylamino)-5-methylpyrimidin-2-yl]amino}cyclohexyl)-2-hydroxyacetamide;
N-(cis-4-{[4-(dimethylamino)-5-methylpyrimidin-2-yl]amino]}cyclohexyl)-2-hydroxy-2-[3-(trifluoromethyl}phenyl]acetamide;
N-(cis-4-{[4-(dimethylamino)-5-methylpyrimidin-2-yl]amino}cyclohexyl)-2-{[2-(trifluoromethyl)phenyl]sulfinyl} acetamide;
2-[(2-chlorophenyl)sulfinyl]-N-(cis-4-{[4-(dimethylamino)-5-methylpyrimidin-2-yl]amino}cyclohexyl)acetamide;
2-[(3-bromophenyl)sulfinyl]-N-(cis-4-{[4-(dimethylamino)-5-methylpyrimidin-2-yl]amino}cyclohexyl)acetamide;
2-[(3,4-difluorophenyl)sulfinyl]-N-(cis-4-{[4-(dimethylamino)-5-methylpyrimidin-2-yl]amino}cyclohexyl)acetamide;
N-(cis-4-{[4-(dimethylamino)-5-methylpyrimidin-2-yl]amino}cyclohexyl)-3-(trifluoromethyl)benzamide;
N-(cis-4-{[4-(dimethylamino)-5-methylpyrimidin-2-yl]amino}cyclohexyl)-3-fluorobenzamide;
3-bromo-N-(cis-4-{[4-(dimethylamino)-5-methylpyrimidin-2-yl]amino}cyclohexyl)benzamide;
N-(cis-4-{[4-(dimethylamino)-5-methylpyrimidin-2-yl]amino}cyclohexyl)-4-(trifluoromethoxy)benzamide;
N-(cis-4-{[4-(dimethylamino)-5-methylpyrimidin-2-yl]amino}cyclohexyl)-4-fluorobenzamide;
3,4-dichloro-N-(cis-4-{[4-(dimethylamino)-5-methylpyrimidin-2-yl]amino}cyclohexyl)benzamide;
N-(cis-4-{[4-(dimethylamino)-5-methylpyrimidin-2-yl]amino}cyclohexyl)-3,5-bis(trifluoromethyl)benzamide;
N-(cis-4-{[4-(dimethylamino)-5-methylpyrimidin-2-yl]amino}cyclohexyl)-3,5-dimethoxybenzamide;
N-(cis-4-{[4-(dimethylamino)-5-methylpyrimidin-2-yl]amino}cyclohexyl)-2,4-difluorobenzamide;
N-(cis-4-{[4-(dimethylamino)-5-methylpyrimidin-2-yl]amino}cyclohexyl)-2,5-difluorobenzamide;
N-(cis-4-{[4-(dimethylamino)-5-methylpyrimidin-2-yl]amino}cyclohexyl)-2,3,4-trifluorobenzamide;
4-chloro-N-(cis-4-{[4-(dimethylamino)-5-methylpyrimidin-2-yl]amino}cyclohexyl)benzamide;
3-cyano-N-(cis-4-{[4-(dimethylamino)-5-methylpyrimidin-2-yl]amino}cyclohexyl)benzamide;
4-cyano-N-(cis-4-{[4-(dimethylamino)-5-methylpyrimidin-2-yl]amino}cyclohexyl)benzamide;
2-[(3,4-dichtorophenyl)sulfinyl]-N-(cis-4-{[4-( dimethylamino)-5-methylpyrimidin-2-yl]amino}cyclohexyl)acetamide;
N-(cis-4-{[4-(dimethylamino)-5-methylpyrimidin-2-yl]amino}cyclohexyl)-2-{[3-(trifluoromethyl)phenyl]sulfinyl}acetamide;
N-(cis-4-{[4-(dimethylamino)-5-methylpyrimidin-2-yl]amino}cyclohexyl)-2-{{[3-(trifluoromethyl)phenyl]sulfonyl acetamide;
N-(cis-4-{[4-(dimethylamino)-5-methylpyrimidin-2-yl]amino}cyclohexyl)-2-(isopropylthio)nicotinamide;
2-(tert-butylthio)-N-(cis-4-{[4-(dimethylamino)-5-methylpyrimidin-2-yl]amino}cyclohexyl)nicotinamide;
N-(cis-4-{[4-(dimethylamino)-5-methylpyrimidin-2-yl]amino}cyclohexyl)-2-(propylthio)nicotinamide;
N-(cis-4-{[4-(dimethylamino)-5-methylpyrimidin-2-yl]amino}cyclohexyl)-3-(methylsulfonyl)benzamide;
N-(cis-4-{[4-(dimethylamino)-6-methylpyrimidin-2-yl]amino}cyclohexyl)-3-fluorobenzamide;
N-(cis-4-{[4-(dimethylamino)-6-methylpyrimidin-2-yl]amino}cyclohexyl)-3-(trifluoromethyl)benzamide;
3-cyano-N-(cis-4-{[4-(dimethylamino)-6-methylpyrimidin-2-yl]amino}cyclohexyl)benzamide;
4-cyano-N-(cis-4-{[4-(dimethylamino)-6-methylpyrimidin-2-yl]amino}cyclohexyl)benzamide;
N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-3-(trifluoromethyl)benzamide;
3-cyano-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-3-methylbenzamide;
3-chloro-N-(cis-4-{[4-(dimethylamino)-5,6-dimethylpyrimidin-2-yl]amino}cyclohexyl)benzamide;
3-bromo-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-3,5-dimethoxybenzamide;
N-(cis-4-{[4-(dimethylamino)-5,6-dimethylpyrimidin-2-yl]amino}cyclohexyl)-3,5-bis(trifluoromethyl)benzamide;
3,4-dichloro-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-4-(trifluoromethoxy)benzamide;
4-cyano-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-4-methylbenzamide;
N-(cis-4-{[4-(dimethylamino)-5,6-dimethylpyrimidin-2-yl]amino}cyclohexyl)-4-fluorobenzamide;
4-chloro-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-2-methoxybenzamide;
4-bromo-N-(cis-4-{[4-(dimethylamino)-5-methylpyrimidin-2-yl]amino}cyclohexyl)benzamide;
N-(cis-4-{[4-(dimethylamino)-5-methylpyrimidin-2-yl]amino}cyclohexyl)-4-(trifluoromethyl)benzamide;
N-(cis-4-{[4-(dimethylamino)-5,6-dimethylpyrimidin-2-yl]amino}cyclohexyl)-3-methoxybenzamide;
5-bromo-N-(cis-4-{[4-(dimethylamino)-5,6-dimethylpyrimidin-2-yl]amino)cyclohexyl)-2-furamide;
N-(cis-4-{[4-(dimethylamino)-5,6-dimethylpyrimidin-2-yl]amino}cyclohexyl)-5-methylisoxazole-3-carboxamide;
2-(3,5-difluorophenyl)-N-(cis-4-{[4-(dimethylamino)-5,6-dimethylpyrimidin-2-yl]amino}cyclohexyl)-2-hydroxyacetamide;
N-(cis-4-{[4-(dimethylamino)-5,6-dimethylpyrimidin-2-yl]amino}cyclohexyl)-2-methyl-1,3-oxazole-4-carboxamide;
N-(cis-4-{[4-(dimethylamino)-5,6-dimethylpyrimidin-2-yl]amino}cyclohexyl)-2,6-dimethoxynicotinamide;
4-bromo-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-4-(trifluoromethyl)benzamide;
4-bromo-N-(cis-4-{[4-(dimethylamino)-5,6-dimethylpyrimidin-2-yl]amino}cyclohexyl)-3-methylbenzamide;
N-(cis-4-{[4-(dimethylamino)-5,6-dimethylpyrimidin-2-yl]amino}cyclohexyl)-3-fluoro-4-methylbenzamide;
N-(cis-4-{[4-(dimethylamino)-5,6-dimethylpyrimidin-2-yl]amino}cyclohexyl)-4-fluoro-3-methylbenzamide;
N-(cis-4-{[4-(dimethylamino)-5,6-dimethylpyrimidin-2-yl]amino}cyclohexyl)-3-ethylbenzamide;
N-(cis-4-{[4-(dimethylamino)-5,6-dimethylpyrimidin-2-yl]amino}cyclohexyl)-3-(trifluoromethoxy)benzamide;
5-bromo-N-(cis-4-{[4-(dimethylamino)-5,6-dimethylpyrimidin-2-yl]amino}cyclohexyl)nicotinamide;
N-(cis-4-{[4-(dimethylamino)-5,6-dimethylpyrimidin-2-yl]amino}cyclohexyl)-5-methylthiophene-2-carboxamide
N-(cis-4-{[4-(dimethylamino)-5,6-dimethylpyrimidin-2-yl]amino}cyclohexyl)-6-(trifluoromethyl)nicotinamide;
N-(cis-4-{[4-(dimethylamino)-5,6-dimethylpyrimidin-2-yl]amino}cyclohexyl)-3,5-diethoxybenzamide;
N-(cis-4-{[4-(dimethylamino)-5,6-dimethylpyrimidin-2-yl]amino}cyclohexyl)-3-ethoxybenzamide;
N-(cis-4-{[4-(dimethylamino)-5,6-dimethylpyrimidin-2-yl]amino}cyclohexyl)-3-isopropoxybenzamide;
3,5-dichloro-N-(cis-4-{[4-(dimethylamino)-5-methylpyrimidin-2-yl]amino}cyclohexyl)benzamide;
4-bromo-N-(cis-4-{[4-(dimethylamino)-5-methylpyrimidin-2-yl]amino}cyclohexyl)-3-methylbenzamide;
N-(cis-4-{[4-(dimethylamino)-5-methylpyrimidin-2-yl]amino}cyclohexyl)-4-ethoxybenzamide;
N-(cis-4-{[4-(dimethylamino)-5-methylpyrimidin-2-yl]amino}cyclohexyl)-4-fluoro-3-methylbenzamide;
N-(cis-4-{[4-(dimethylamino)-5-methylpyrimidin-2-yl]amino}cyclohexyl)-3-fluoro-4-methylbenzamide;
N-(cis-4-{[4-(dimethylamino)-5-methylpyrimidin-2-yl]amino}cyclohexyl)-3-ethylbenzamide;

N-(eis-4-{[4-(dimethylamino)-6-methylpyrimidin-2-yl]amino}cyclohexyl)-3,5-bis(trifluoromethyl)benzamide;
N-(cis-4-{[4-(dimethylamino)-6-methylpyrimidin-2-yl]amino}cyclohexyl)-3-fluoro-4-(trifluoromethyl)benzamide;
N-(cis-4-{[4-(dimethylamino)-6-methylpyrimidin-2-yl]amino}cyclohexyl)-3-fluoro-5-(trifluoromethyl)benzamide;
3-chloro-N-(cis-4-{[4-(dimethylamino)-6-methylpyrimidin-2-yl]amino}cyclohexyl)-4-fluorobenzamide;
N-(cis-4-{[4-(dimethylamino)-6-methylpyrimidin-2-yl]amino}cyclohexyl)-4-fluoro-3-methylbenzamide;
N-(cis-4-{[4-(dimethylamino)-6-methylpyrimidin-2-yl]amino}cyclohexyl)-3-fluoro-4-methylbenzamide;
3,5-dichloro-N-(cis-4-{[4-(dimethylamino)-6-methylpyrimidin-2-yl]amino}cyclohexyl)benzamide;
N-(cis-4-{[4-(dimethylamino)-6-methylpyrimidin-2-yl]amino}cyclohexyl)-3-(trifluoromethoxy)benzamide;
N-(cis-4-{[4-(dimethylamino)-6-methylpyrimidin-2-yl]amino}cyclohexyl)-3,5-difluorobenzamide;
4-bromo-N-(cis-4-{[4-(dimethylamino)-6-methylpyrimidin-2-yl]amino}cyclohexyl)-3-methylbenzamide;
N-(cis-4-{[4-(dimethylamino)-6-methylpyrimidin-2-yl]amino}cyclohexyl)-3-ethylbenzamide;
N-(cis-4-{[4-(dimethylamino)-6-methylpyrimidin-2-yl]anino}cyclohexyl)-4-(trifluoromethyl)benzamide;
4-bromo-N-(cis-4-{[4-(dimethylamino)-6-methylpyrimidin-2-yl]amino}cyclohexyl)benzamide;
N-(cis-4-{[4-(dimethylamino)-6-methylpyrimidin-2-yl]amino}cyclohexyl)-4-ethylbenzamide;
N-(cis-4-{[4-(dimethytamino)-6-methylpyrimidin-2-yl]amino}cyclohexyl)-3,5-diethoxybenzamide;
N-(cis-4-{[4-(dimethylamino)-6-methylpyrimidin-2-yl]amino}cyclohexyl)-3-ethoxybenzamide;
N-(cis-4-{[4-(dimethylamino)-6-methylpyrimidin-2-yl]amino}cyclohexyl)-3-isopropoxybenzamide;
5-bromo-N-(cis-4-{[4-(dimethylamino)-6-methylpyrimidin-2-yl]amino}cyclohexyl)nicotinamide;
5-bromo-N-(cis-4-{[4-(dimethylamino)-6-methylpyrimidin-2-yl]amino}cyclohexyl)-2-furamide;
5-chloro-N-(cis-4-{[4-(dimethylamino)-6-methylpyrimidin-2-yl]amino}cyclohexyl)-2-furamide;
N-(cis-4-{[4-(dimethylamino)-5-methylpyrimidin-2-yl]amino}cyclohexyl)-3-fluoro-5-(trifluoromethyl)benzamide;
N-(cis-4-{[4-(dimethylamino)-5-methylpyrimidin-2-yl]amino}cyclohexyl)-2,2-difluoro-1,3-benzodioxole-5-carboxamide;
N-(cis-4-{[4-(dimethylamino)-5-methylpyrimidin-2-yl]amino}cyclohexyl)-3-ethoxybenzamide;
N-(cis-4-{[4-(dimethylamino)-5-methylpyrimidin-2-yl]amino}cyclohexyl)-3-isopropoxybenzamide;
5-bromo-N-(cis-4-{[4-(dimethylamino)-5-methylpyrimidin-2-yl]amino}cyclohexyl)-2-furamide;
N-(cis-4-{[4-(dimethylamino)-5-methylpyrimidin-2-yl]amino}cyclohexyl)-3,5-diethoxybenzamide;
4-chloro-N-(cis-4-{[4-(dimethylamino)-5-methylpyrimidin-2-yl]amino}cyclohexyl)-3-(trifluoromethyl}benzamide;
5-bromo-N-(cis-4-{[4-(dimethylamino)-5-methylpyrimidin-2-yl]amino}cyclohexyl)nicotinamide;
3,4-dichloro-N-(cis-4-{[4-(dimethylamino)-6-methylpyrimidin-2-yl]amino}cyclohexyl)benzamide;
3-chloro-N-(cis-4-{[4-(dimethylamino)-5-methylpyrimidin-2-yl]amino}cyclohexyl)-4-(trifluoromethoxy)benzamide;
N-(cis-4-{[4-(dimethylamino)-5-methylpyrimidin-2-yl]amino}cyclohexyl)-4-methoxy-3-(trifluoromethyl)benzamide;
N-(cis-4-{[4-(dimethylamino)-6-methylpyrimidin-2-yl]amino}cyclohexyl)-4-methoxy-3-(trifluoromethyl)benzamide;
2-(4-chlorophenyl)-N-(cis-4-{[4-(dimethylamino)-5-methylpyrimidin-2-yl]amino}cyclohexyl)-2-methylpropanamide;
1-(4-chlorophenyl)-N-(cis-4-{[4-(dimethylamino)-5-methylpyrimidin-2-yl]amino}cyclohexyl)cyclopropanecarboxamide;
1-(4-chlorophenyl)-N-(cis-4-{[4-(dimethylamino)-5-methylpyrimidin-2-yl]amino}cyclohexyl)cyclobutanecarboxamide;
1-(2,4-dichloroghenyl)-N-(cis-4-{[4-(dimethylamino)-5-methylpyrimidin-2-yl]amino}cyclohexyl)cyclopropanecarboxamide;
2-(4-chlorophenyl)-N-(cis-4-{[4-(dimethylamino)-6-methylpyrimidin-2-yl]amino}cyclohexyl)-2-methylpropanamide;
1-(4-chlorophenyl)-N-(cis-4-{[4-(dimethylamino)-6-methylpyrimidin-2-yl]amino}cyclohexyl)cyclopropanecarboxamide;
1-(4-chlorophenyl)-N-(cis-4-{[4-(dimethylamino)-6-methylpyrimidin-2-yl]amino}cyclohexyl)cyclobutanecarboxamide;
1-(2,4-dichlorophenyl)-N-(cis-4-{[4-(dimethylamino)-6-methylpyrimidin-2-yl]amino}cyclohexyl)cyclopropanecarboxamide;
2-[3,5-bis(trifluoromethyl)phenyl]-N-(cis-4-{[4-(dimethylamino)-6-methylpyrimidin-2-yl]amino}cyclohexyl) acetamide;

N-(cis-4-{[4-(dimethylamino)-5-methylpyrimidin-2-yl]amino}cyclohexyl)-4-[2,2,2-trifluoro-1-hydroxy-1-(trifluoromethyl)ethyl]benzamide;
2-(4-chlorophenyl)-N-(cis-4-{[4-(dimethylamino)-5-methylpyrimidin-2-yl]amino}cyclohexyl)acetamide;
N-(cis-4-{[4-(dimethylamino)-5-methylpyrimidin-2-yl]amino}cyclohexyl)-1-(4-methylphenyl)cyclopropanecarboxamide;
2-(4-chlorophenyl)-N-(cis-4-{[4-(dimethylamino)-5-methylpyrimidin-2-yl]amino}cyclohexyl)propanamide;
2-(4-chlorophenyl)-N-(cis-4-{[4-(dimethylamino)-5-methylpyrimidin-2-yl]amino}cyclohexyl)-2-hydroxyacetamide;
N-(cis-4-{[4-(dimethylamino)-5-methylpyrimidin-2-yl]amino}cyclohexyl)-1-(4-methoxyphenyl)cyclopropanecarboxamide;
$N^2$-(3-chlorophenyl)-N-(cis-4-{[4-(dimethylamino)-5-methylpyrimidin-2-yl]amino}cyclohexyl)-$N^2$-methylglycinamide;
$N^2$-(3,4-dichlorophenyl)-N-(cis-4-{[4-(dimethylamino)-5-methylpyrimidin-2-yl]amino}cyclohexyl)-$N^2$-methylglycinamide;
N-(cis-4-{[4-(dimethylamino)-5-methylpyrimidin-2-yl]amino}cyclohexyl)-$N^2$-methyl-$N^2$-(3-methylphenyl)glycinamide;
N-(cis-4-{[4-(dimethylamino)-5-methylpyrimidin-2-yl]amino}cyclohexyl)-$N^2$-(3-fluorophenyl)-$N^2$-methylglycinamide;
N-(cis-4-{[4-(dimethylamino)-5-methylpyrimidin-2-yl]amino}cyclohexyl)-$N^2$-(4-fluorophenyl)-$N^2$-methylglycinamide;
$N^2$-(4-chlorophenyl)-N-(cis-4-{[4-(dimethylamino)-5-methylpyrimidin-2-yl]amino}cyclohexyl)-$N^2$-methylglycinamide;
$N^2$-(3,4-difluorophenyl)-N-(cis-4-{[4-(dimethylamino)-5-methylpyrimidin-2-yl]amino}cyclohexyl)-$N^2$-methylglycinamide;
N-(cis-4-{[4-(dimethylamino)-5-methylpyrimidin-2-yl]amino}cyclohexyl)-$N^2$-(3-methoxyphenyl)-$N^2$-methylglycinamde;
N-(cis-4-{[4-(dimethylamino)-5-methylpyrimidin-2-yl]amino}cyclohexyl)-$N^2$-(4-methoxyphenyl)-$N^2$-methylglycinamide;
2-[(3,4-difluorophenyl)amino]-N-(cis-4-{[4-(dimethylamino)-5-methylpyrimidin-2-yl]amino}cyclohexyl)nicotinamide;
2-(3,4-dichlorophenoxy)-N-(cis-4-{[4-methyl-6-(methylamino)pyrimidin-2-yl]amino}cyclohexyl)acetamide;
trans-2-(4-chlorophenyl)-N-(cis-4-{[4-(dimethylamino)-5-methylpyrimidin-2-yl]amino}cyclohexyl)cyclopropanecarboxamide;
trans-2-(3-chlorophenyl)-N-(cis-4-{[4-(dimethylamino)-5-methylpyrimidin-2-yl]amino}cyclohexyl)cyclopropanecarboxamide;
trans-2-(3,4-difluorophenyl)-N-(cis-4-{[4-(dimethylamino)-5-methylpyrimidin-2-yl]amino}cyclohexyl)cyclopropanecarboxamide;
trans-2-(3,4-dichlorophenyl)-N-(cis-4-{[4-(dimethylamino)-5-methylpyrimidin-2-yl]amino}cyclohexyl)cyclopropanecarboxamide;
trans-2-[3,5-bis(trifluoromethyl)phenyl]-N-(cis-4-{[4-(dimethylamino)-5-methylpyrimidin-2-yl)amino}cyclohexyl)cyclopropanecarboxamide;
2-[(4-chlorophenyl)sulfonyl]-N-(cis-4-{[4-(dimethylamino)-5-methylpyrimidin-2-yl]amino}cyclohexyl)nicotinamide;
2-[(3-chlorophenyl)sulfonyl]-N-(cis-4-{[4-(dimethylamino)-5-methylpyrimidin-2-yl]amino}cyclohexyl)nicotinamide;
2-[(4-bromophenyl)sulfonyl]-N-(cis-4-{[4-(dimethylamino)-5-methylpyrimidin-2-yl]amino}cyclohexyl)nicotinamide;
N-(cis-4-{[4-(dimethylamino)-5-methylpyrimidin-2-yl]amino}cyclohexyl)-2-{[4-(trifluoromethyl)phenyl]sulfonyl}nicotinamide;
N-(cis-4-{[4-(dimethylamino)-5-methylpyrimidin-2-yl]amino}cyclohexyl)-2-{[1-methyl-3-(trifluoromethyl)-1H-pyrazol-5-yl]oxy}acetamide;
2-[(2-chlorophenyl)sulfonyl]-N-(cis-4-{[4-(dimethylamino)-6-methylpyrimidin-2-yl]amino}cyclohexyl)nicotinamide;
2-[(3-chlorophenyl)sulfonyl]-N-(cis-4-{[4-(dimethylamino)-6-methylpyrimidin-2-yl]amino}cyclohexyl)nicotinamide;
3,4-dichloro-N-{cis-4-[(4-methoxy-5-methylpyrimidin-2-yl)amino]cyclohexyl} benzamide;
N-[cis-4-(4-dimethylamino-5-methyl-pyrimidin-2-ylamino)-cyclohexyl]-2-phenoxy-nicotinamide;
N-[cis-4-(4-dimethylamino-6-methyl-pyrimidin-2-ylamino)-cyclohexyl]-2-phenoxy-nicotinamide;

3-chloro-N-[cis-4-(4-dimethylamino-5-methyl-pyrimidin-2-ylamino)-cyclohexyl]-4-fluoro-benzamide;
4-chloro-N-[cis-4-(4-dimethylamino-6-methyl-pyrimidin-2-ylamino)-cyclohexyl]-3-fluoro-benzamide;
3-chloro-N-[cis-4-(4-dimethylamino-6-methyl-pyrimidin-2-ylamino)-cyclohexyl]-5-fluoro-benzamide;
N-[cis-4-(4-dimethylamino-6-methyl-pyrimidin-2-ylamino)-cyclohexyl]-3,4,5-trifluoro-benzamide;
3-chloro-4-fluoro-N-[cis-4-(5-methyl-4-methylamino-pyrimidin-2-ylamino)-cyclohexyl]-benzamide;
4-chloro-N-[cis-4-(4-dimethylamino-5-methyl-pyrimidin-2-ylamino)-cyclohexyl]-3-fluoro-benzamide;
3-chloro-N-[cis-4-(4-dimethylamino-5-methyl-pyrimidin-2-ylamino)-cyclohexyl]-5-fluoro-benzamide;
N-[cis-4-(4-dimethylamino-5-methyl-pyrimidin-2-ylamino)-cyclohexyl]-3,4,5-trifluoro-benzamide;
N-[cis-4-(4-dimethylamino-5-methyl-pyrimidin-2-ylamino)-cyclohexyl]-3,5-difluoro-benzamide; and
2-(3,4-difluoro-phenyl)-N-[cis-4-(4-dimethylamino-5-methyl-pyrimidin-2-ylamino)-cyclohexyl]-acetamide;

or a pharmaceutically acceptable salt, hydrate, or solvate thereof.

**87.** The compound according to claim 1 selected from the group consisting of:

N-[(cis-4-{[4-(dimethylamino)-5-methylpyrimidin-2-yl]amino}cyclohexyl)methyl]-3,5-bis(trifluoromethyl)benzamide;
N-[(cis-4-{[4-(dimethylamino)-5-methylpyrimidin-2-yl]amino}cyclohexyl)methyl]-3,5-dimethoxybenzamide;
N-[(cis-4-{[4-(dimethylamino)-5-methylpyrimidin-2-yl]amino}cyclohexyl)methyl]-3-(trifluoromethyl)benzamide;
4-bromo-N-[(cis-4-{[4-(dimethylamino)-5-methylpyrimidin-2-yl]aminol}cyclohexyl)methyl]-3-methylbenzamide;
3,5-dichloro-N-[(cis-4-{[4-(dimethylamino)-5-methylpyrimidin-2-yl]amino}cyclohexyl)methyl]benzamide;
3,4-dichloro-N-[(cis-4-{[4-(dimethylamino)-5-methylpyrimidin-2-yl]amino}cyclohexyl)methyl]benzamide;
3,5-dichloro-N-[cis-4-({[4-(dimethylamino)-5-methylpyrimidin-2-yl]amino}methyl)cyclohexyl]benzamide;
N-(cis-4-{[4-(dimethylamino)-5-methylpyrimidin-2-yl]amino}cyclohexyl)-2-(2-fluorophenoxy)nicotinamide;
N-(cis-4-{[4-(dimethylamino)-6-methylpyrimidin-2-yl]amino}cyclohexyl)-3,4,5-trimethoxybenzamide;
N-(4-{[4-(dimethylamino)-6-methylpyrimidin-2-yl]amino}cyclohexyl)-3-nitrobenzamide;
N-(cis-4-{[4-(dimethylamino)-6-methylpyrimidin-2-yl]amino}cyclohexyl)-2,2-diphenylacetamide;
4-chloro-N-(cis-4-{[4-(dimethylamino)-6-methylpyrimidin-2-yl]amino}cyclohexyl)benzamide;
3-chloro-N-(cis-4-{[4-(dimethylamino)-6-methylpyrimidin-2-yl]amino}cyclohexyl)benzamide;
N-(cis-4-{[4-(dimethylamino)-6-methylpyrimidin-2-yl]amino}cyclohexyl)-3,4-difluorobenzamide;
N-(cis-4-{[4-(dimethylamino)-6-methylpyrimidin-2-yl]amino}cyclohexyl)-3-methylbenzamide;
N-(cis-4-{[4-(dimethylamino)-6-methylpyrimidin-2-yl]amino}cyclohexyl)-3-methoxybenzamide;
N-(cis-4-{[4-(dimethylamino)-6-methylpyrimidin-2-yl]amino}cyclohexyl)-4-fluorobenzamide;
N-(cis-4-{[4-(dimethylamino)-5-methylpyrimidin-2-yl]amino}cyclohexyl)-3-methylbenzamide;
N-(cis-4-{[4-(dimethylamino)-5-methylpyrimidin-2-yl]amino}cyclohexyl)-3-methoxybenzamide;
N-(cis-4-{[4-(dimethylamino)-5-methylpyrimidin-2-yl]amino}cyclohexyl)-4-methylbenzamide;
N-(cis-4-{[4-(dimethylamino)-5-methylpyrimidin-2-yl]amino}cyclohexyl)-3,4-difluorobenzamide;
3-chloro-N-(cis-4-{[4-(dimethylamino)-5-methylpyrimidin-2-yl]amino}cyclohexyl)benzamide;
N-(cis-4-{[4-(dimethylamino)-5-methylpyrimidin-2-yl]amino}cyclohexyl)-2-(3-methylphenoxy)nicotinamide;
2-(4-bromophenoxy)-N-(cis-4-{[4-(dimethylamino)-5-methylpyrimidin-2-yl]amino}cyclohexyl)nicotinamide;
2-(4-chlorophenoxy)-N-(cis-4-{[4-(dimethylamino)-5-methylpyrimidin-2-yl]amino}cyclohexyl)nicotinamide;
N-(cis-4-{[4-(dimethylamino)-5-methylpyrimidin-2-yl]amino}cyclohexyl)-2-(4-fluorophenoxy)nicotinamide;
N-(cis-4-{[4-(dimethylamino)-5-methylpyrimidin-2-yl]amino}cyclohexyl)-2-(3-fluorophenoxy)nicotinamide;
2-(2-bromophenoxy)-N-(cis-4-{[4-(dimethylamino)-5-methylpyrimidin-2-yl]amino}cyclohexyl)nicotinamide;
N-(cis-4-{[4-(dimethylamino)-5-methylpyrimidin-2-yl]amino}cyclohexyl)-2-(3-methoxyphenoxy)nicotinamide;
N-(cis-4-{[4-(dimethylamino)-5-methylpyrimidin-2-yl]amino}cyclohexyl)-2-(4-methoxyphenoxy)nicotinamide;
N-(cis-4-{[4-(dimethylamino)-5-methylpyrimidin-2-yl]amino}cyclohexyl)-2-(4-iodophenoxy)nicotinamide;
2-(3,4-dichlorophenoxy)-N-(cis-4-{[5-methyl-4-(methylamino)pyrimidin-2-yl]amino}cyclohexyl)acetamide;
2-(2,3-dichlorophenoxy)-N-(cis-4-{[5-methyl-4-(methylamino)pyrimidin-2-yl]amino}cyclohexyl)acetamide;
2-[(3,4-difluorophenyl)sulfonyl]-N-(cis-4-{[4-(dimethylamino)-5-methylpyrimidin-2-yl]amino}cyclohexyl)nicotinamide;
N-[cis-4-({4-[ethyl(methyl)amino]-5-methylpyrimidin-2-yl}amino)cyclohexyl]-3,4-difluorobenzamide;
N-(cis-4-{[4-(dimethylamino)-6-methylpyrimidin-2-yl]amino}cyclohexyl)-3,5-dimethoxybenzamide;
N-(cis-4-{[4-(dimethylamino)-5-methylpyrimidin-2-yl]amino}cyclohexyl)-2-(2-methoxyphenoxy)nicotinamide;
2-(2-chlorophenoxy)-N-(cis-4-{[4-(dimethylamino)-5-methylpyrimidin-2-yl]amino}cyclohexyl)nicotinamide;
2-(3-chlorophenoxy)-N-(cis-4-{[4-(dimethylamino)-5-methylpyrimidin-2-yl]amino}cyclohexyl)nicotinamide;

2-(3-bromophenoxy)-N-(cis-4-{[4-(dimethylamino)-5-methylpyrimidin-2-yl]amino}cyclohexyl)nicotinamide;
N-(cis-4-{[4-(dimethylamino)-5-methylpyrimidin-2-yl]amino}cyclohexyl)-2-[3-(trifluoromethyl)phenoxy]nicotinamide;
N-(cis-4-{[4-(dimethylamino)-5-methylpyrimidin-2-yl]amino}cyclohexyl)-2-(3-fluorophenoxy)acetamide;
N-(cis-4-{[4-(dimethylamino)-5-methylpyrimidin-2-yl]amino}cyclohexyl)-2-(3-methoxyphenoxy)acetamide;
N-(cis-4-{[4-(dimethylamino)-5-methylpyrimidin-2-yl]amino}cyclohexyl)-2-[3-(trifluoromethyl)phenoxy]acetamide;
2-(3-chlorophenoxy)-N-(cis-4-{[4-(dimethylamino)-5-methylpyrimidin-2-yl]amino}cyclohexyl)acetamide;
2-[(5-chloropyridin-3-yl)oxy]-N-(cis-4-{[4-(dimethylamino)-5-methylpyrimidin-2-yl]amino}cyclohexyl)acetamide;
N-(cis-4-{[4-(dimethylamino)-5,6-dimethylprimidin-2-yl]amino}cyclohexyl)-3,4-difluorobenzamide;
N-(cis-4-{[4-(dimethylamino)-5-methylpyrimidin-2-yl]amino}cyclohexyl)-2-hydroxy-2-(4-methoxyphenyl) acetamide;
2-(2,3-difluorophenyl)-N-(cis-4-{[4-(dimethylamino)-5-methylpyrimidin-2-yl]amino}cyclohexyl)-2-hydroxyacetamide;
N-(cis-4-{[4-(dimethylamino)-5-methylpyrimidin-2-yl]amino}cyclohexyl)-2-hydroxy-2-[3-(trifluoromethyl)phenyl]acetamide;
N-(cis-4-{[4-(dimethylamino)-5-methylpyrimidin-2-yl]amino}cyclohexyl)-2-{[2-(trifluoromethyl)phenyl]sulfinyl} acetamide;
2-[(2-chlorophenyl)sulfinyl]-N-(cis-4-{[4-(dimethylamino)-5-methylpyrimidin-2-yl]amino}cyclohexyl)acetamide;
2-[(3-bromophenyl)sulfinyl]-N-(cis-4-{[4-(dimethylamino)-5-methylpyrimidin-2-yl]amino}cyclohexyl)acetamide;
N-(cis-4{[4-(dimethylamino)-5-methylpyrimidin-2-yl]amino}cyclohexyl)-3-(trifluoromethyl)benzamide;
N-(cis-4-{[4-(dimethylamino)-5-methylpyrimidin-2-yl]amino}cyclohexyl)-3-fluorobenzamide;
3-bromo-N-(cis-4-{[4-(dimethylamino)-5-methylpyrimidin-2-yl]amino}cyclohexyl)benzamide;
N-(cis-4-{[4-(dimethylamino)-5-methylpyrimidin-2-yl]amino}cyclohexyl)-4-(trifluoromethoxy)benzamide;
N-(cis-4-{[4-(dimethylamino)-5-methylpyrimidin-2-yl]amino}cyclohexyl)-4-fluorobenzamide;
3,4-dichloro-N-(cis-4-{[4-(dimethylamino)-5-methylpyrimidin-2-yl]amino}cyclohexyl)benzamide;
N-(cis-4-{[4-(dimethylamino)-5-methylpyrimidin-2-yl]amino}cyclohexyl)-3,5-bis(trifluoromethyl)benzamide;
N-(cis-4-{[4-(dimethylamino)-5-methylpyrimidin-2-yl]amino}cyclohexyl)-3,5-dimethoxybenzamide;
N-(cis-4-{[4-(dimethylamino)-5-methylpyrimidin-2-yl]amino}cyclohexyl)-2,4-difluorobenzamide;
N-(cis-4-{[4-(dimethylamino)-5-methylpyrimidin-2-yl]amino}cyclohexyl)-2,5-difluorobenzamide;
N-(cis-4-{[4-(dimethylamino)-5-methylpyrimidin-2-yl]amino}cyclohexyl)-2,3,4-trifluorobenzamide;
4-chloro-N-(cis-4-{[4-(dimethylamino)-5-methylpyrimidin-2-yl]amino}cyclohexyl)benzamide;
3-cyano-N-(cis-4-{[4-(dimethylamino)-5-methylpyrimidin-2-yl]amino}cyclohexyl)benzamide;
4-cyano-N-(cis-4-{[4-(dimethylamino)-5-methylpyrimidin-2-yl]amino}cyclohexyl)benzamide;
N-(cis-4-3{[4-(dimethylamino)-5-methylpyrimidin-2-yl]amino}cyclohexyl)-2-(isopropylthio)nicotinamide;
N-(cis-4-{[4-(dimethylamino)-5-methytpyrimidin-2-yl]amino}cyclohexyl)-2-(propylthio)nicotinamide;
N-(cis-4-{[4-(dimethylamino)-6-methylpyrimidin-2-yl]amino}cyclohexyl)-3-(trifluoromethyl)benzamide;
3-cyano-N-(cis-4-{[4-(dimethylamino)-6-methylpyrimidin-2-yl]amino}cyclohexyl)benzamide;
4-cyano-N-(cis-4-{[4-(dimethylamino)-6-methylpyrimidin-2-yl]amino}cyclohexyl)benzamide;
N-(cis-4-{[4-(dimethylamino)-5,6-dimethylpyrimidin-2-yl]amino}cyclohexyl)-3-(trifluoromethyl)benzamide;
3-cyano-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-3-methylbenzamide;
3-chloro-N-(cis-4-{[4-(dimethylamino)-5,6-dimethylpyrimidin-2-yl]amino}cyclohexyl)benzamide;
3-bromo-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-3,5-dimethoxybenzamide;
N-(cis-4-{[4-(dimethylamino)-5,6-dimethylpyrimidin-2-yl]amino}cyclohexyl)-3,5-bis(trifluoromethyl)benzamide;
3,4-dichloro-N-(cis-4-{[4-(dimethylamino)-5,6-dimethylpyrimidin-2-yl]amino}cyclohexyl)benzamide;
4-cyano-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino}cyclohexyl)-4-methylbenzamide;
N-(cis-4-{[4-(dimethylamino)-5,6-dimethylpyrimidin-2-yl]amino}cyclohexyl)-4-fluorobenzamide;
4-chloro-N-(cis-4-{[4-(dimethylamino)-5,6-dimethylpyrimidin-2-yl]amino}cyclohexyl)benzamide;
4-bromo-N-(cis-4-{[4-(dimethylamino)-5-methylpyrimidin-2-yl]amino}cyclohexyl)benzamide;
N-(cis-4-{[4-(dimethylamino)-5-methylpyrimidin-2-yl]amino}cyclohexyl)-4-(trifluoromethyl)benzamide;
N-(cis-4-{[4-(dimethylamino)-5,6-dimethylpyrimidin-2-yl]amino}cyclohexyl)-3-methoxybenzamide;

5-bromo-N-(cis-4-{[4-(dimethylamino)-5,6-dimethylpyrimidin-2-yl]amino}cyclohexyl)-2-furamide;
N-(cis-4-{[4-(dimethylamino)-5,6-dimethylpyrimidin-2-yl]amino}cyclohexyl)-2,6-dimethoxynicotinamide;
4-bromo-N-(cis-4-{[4-(dimethylamino)-5,6-dimethylpyrimidin-2-yl]amino}cyclohexyl)benzamide;
N-(cis-4-{[4-(dimethylamino)-5,6-dimethylpyrimidin-2-yl]amino }cyclohexyl)-4-(trifluoromethyl)benzamide;
4-bromo-N-(cis-4-{(4-(dimethylamino)-5,6-dimethylpyrimidin-2-yl]amino}cyclohexyl)-3-methylbenzamide;
N-(cis-4-{[4-(dimethy!amino)-5,6-dimethylpyrimidin-2-yl]amino}cyclohexyl)-3-fluoro-4-methylbenzamide;
N-(cis-4-{[4-(dimethylamino)-5,6-dimethylpyrimidin-2-yl]amino}cyclohexyl)-4-fluoro-3-methylbenzanude;
N-(cis-4-{[4-(dimethylamino)-5,6-dimethylpyrimidin-2-yl]amino}cyclohexyl)-3-(trifluoromethoxy)benzamide;
5-bromo-N-(cis-4-{[4-(dimethylamino)-5,6-dimethylpyrimidin-2-yl]amino}cyclohexyl)nicotinamide;
N-(cis-4-{[4-(dimethylamino)-5,6-dimethylpyrimidin-2-yl]amino}cyclohexyl)-5-methylthiophene-2-carboxamide;
N-(cis-4-{[4-(dimethylamino)-5,6-dimethylpyrimidin-2-yl]amino}cyclohexyl)-3,5-diethoxybenzamide;
N-(cis-4-{[4-(dimethylamino)-5,6-dimethylpyrimidin-2-yl]amino}cyclohexyl)-3-ethoxybenzamide;
N-(cis-4-{[4-(dimethylamino)-5,6-dimethylpyrimidin-2-yl]amino}cyclohexyl)-3-isopropoxybenzamide;
3,5-dichloro-N-(cis-4-{[4-(dimethylamino)-5-methylpyrimidin-2-yl]amino}cyclohexyl)benzamide;
4-bromo-N-(cis-4-{[4-(dimethylamino)-5-methylpyrimidin-2-yl]amino}cyclohexyl)-3-methylbenzamide;
N-(cis-4-{[4-(dimethylamino)-5-methylpyrimidin-2-yl]amino}cyclohexyl)-4-fluoro-3-methylbenzamide;
N-(cis-4-{[4-(dimethylamino)-5-methylpyrimidin-2-yl]amino}cyclohexyl)-3-fluoro-4-methylbenzamide;
N-(cis-4-{[4-(dimethylamino)-5-methylpyrimidin-2-yl]amino}cyclohexyl)-3-ethylbenzamide;
N-(cis-4-{[4-(dimethylamino)-6-methylpyrimidin-2-yl]amino}cyclohexyl)-3,5-bis(trifluoromethyl)benzamide;
N-(cis-4-{[4-(dimethylamino)-6-methylpyrimidin-2-yl]amino}cyclohexyl)-3-fluoro-4-(trifluoromethyl)benzamide;
N-(cis-4-{[4-(dimethylamino)-6-methylpyrimidin-2-yl]amino}cyclohexyl)-3-fluoro-5-(trifluoromethyl)benzamide;
3-chloro-N-(cis-4-{[4-(dimethylamino)-6-methylpyrimidin-2-yl]amino}cyclohexyl)-4-fluorobenzamide;
N-(cis-4-{[4-(dimethylamino)-6-methylpyrimidin-2-yl]amino}cyclohexyl)-4-fluoro-3-methylbenzamide;
N-(cis-4-{[4-(dimethylamino)-6-methylpyrimidin-2-yl]amino}cyclohexyl)-3-fluoro-4-methylbenzamide;
3,5-dichloro-N-(cis-4-{[4-(dimethylamino)-6-methylpyrimidin-2-yl]amino}cyclohexyl)benzamide;
N-(cis-4-{[4-(dimethylamino)-6-methylpyrimidin-2-yl]amino}cyclohexyl)-3-(trifluoromethoxy)benzamide;
N-(cis-4-{[4-(dimethylamino)-6-methylpyrimidin-2-yl]amino}cyclohexyl)-3,5-difluorobenzamide;
4-bromo-N-(cis-4-{[4-(dimethylamino)-6-methylpyrimidin-2-yl]amino}cyclohexyl)-3-methylbenzamide;
N-(cis-4-{[4-(dimethylamino)-6-methylpyrimidin-2-yl]amino}cyclohexyl)-3-ethylbenzamide;
4-bromo-N-(cis-4-{[4-(dimethylamino)-6-methylpyrimidin-2-yl]amino}cyclohexyl)benzamide;
N-(cis-4-{[4-(dimethylamino)-6-methylpyrimidin-2-yl]amino}cyclohexyl)-4-ethylbenzamide;
N-(cis-4-{[4-(dimethylamino)-6-methylpyrimidin-2-yl]amino}cyclohexyl)-3,5-diethoxybenzamide;
N-(cis-4-{[4-(dimethylamino)-6-methylpyrimidin-2-yl]amino}cyclohexyl)-3-ethoxybenzamide;
N-(cis-4-{[4-(dimethylamino)-6-methylpyrimidin-2-yl]amino}cyclohexyl)-3-isopropoxybenzamide;
5-bromo-N-(cis-4-{[4-(dimethylamino)-6-methylpyrimidin-2-yl]amino}cyclohexyl)nicotinamide;
5-bromo-N-(cis-4-{[4-(dimethylamino)-6-methylpyrimidin-2-yl]amino}cyclohexyl)-2-furamide;
5-chloro-N-(cis-4-{[4-(dimethylamino)-6-methylpyrimidin-2-yl]amino}cyclohexyl)-2-furamide;
N-(cis-4-{[4-(dimethylamino)-5-methylpyrimidin-2-yl]amino}cyclohexyl)-3-fluoro-5-(trifluoromethyl)benzamide;
N-(cis-4-{[4-(dimethylamino)-5-methylpyrimidin-2-yl]amino}cylohexyl)-2,2-difluoro-1,3-benzodioxole-5-carboxamide;
N-(cis-4-{[4-(dimethylamino)-5-methylpyrimidin-2-yl]amino}cyclohexyl)-3-ethoxybenzamide;
5-bromo-N-(cis-4-{[4-(dimethylamino)-5-methylpyrimidin-2-yl]amino}cyclohexyl)-2-furamide;
N-(cis-4-{[4-(dimethylamino)-5-methylpyrimidin-2-yl]amino}cyclohexyl)-3,5-diethoxybenzamide;
4-chloro-N-(cis-4-{[4-(dimethylamino)-5-methylpyrimidin-2-yl]amino}cyclohexyl)-3-(trifluoromethyl)benzamide;
5-bromo-N-(cis-4-{[4-(dimethylamino)-5-methylpyrimidin-2-yl]amino}cyclohexyl)nicotinamide;
3,4-dichloro-N-(cis-4-{[4-(dimethylamino)-6-methylpyrimidin-2-yl]amino}cyclohexyl)benzamide;
3-chloro-N-(cis-4-{[4-(dimethylamino)-5-methylpyrimidin-2-yl]amino}cyclohexyl)-4-(trifluoromethoxy)benzamide;
N-(cis-4-{[4-(dimethylamino)-5-methylpyrimidin-2-yl]amino}cyclohexyl)-4-methoxy-3-(trifluoromethyl)benzamide;
N-(cis-4-{[4-(dimethylamino)-6-methylpyrimidin-2-yl]amino}cyclohexyl)-4-methoxy-3-(trifluoromethyl)benzamide;
2-(4-chlorophenyl)-N-(cis-4-{[4-(dimethylamino)-5-methylpyrimidin-2-yl]amino}cyclohexyl)-2-methylpropanamide
1-(4-chlorophenyl)-N-(cis-4-{[4-(dimethylamino)-5-methylpyrimidin-2-yl]amino}cyclohexyl)cyclopropanecarboxamide;
1-(4-chlorophenyl)-N-(cis-4-{[4-(dimethylamino)-5-methylpyrimidin-2-yl]amino}cyclohexyl)cyclobutanecarboxamide;
1-(2,4-dichlorophenyl)-N-(cis-4-{[4-(dimethylamino)-5-methylpyrimidin-2-yl]amino}cyclohexyl)cyclopropanecarboxamide;
2-(4-chlorophenyl)-N-(cis-4-{[4-(dimethylamino)-6-methylpyrimidin-2-yl]amino}cyclohexyl)-2-methylpropanamide
1-(4-chlorophenyl)-N-(cis-4-{[4-(dimethylamino)-6-methylpyrimidin-2-yl]amino}cyclohexyl)cyclopropanecarboxamide;
1-(4-chlorophenyl)-N-(cis-4-{[4-(dimethylamino)-6-methylpyrimidin-2-yl]amino}cyclohexyl)cyclobutanecarboxamide;
1-(2,4-dichlorophenyl)-N-(cis-4-{[4-(dimethylamino)-6-methylpyrimidin-2-yl]amino}cyclohexyl)cyclopropanecarboxamide;
2-(4-chlorophenyl)-N-(cis-4-{[4-(dimethylamino)-5-methylpyrimidin-2-yl]amino}cyclohexyl)acetamide;
N-(cis-4-{[4-(dimethylamino)-5-methylpyrimidin-2-yl]amino}cyclohexyl)-1-(4-methylphenyl)cyclopropanecarboxamide;
2-(4-chlorophenyl)-N-(cis-4-{[4-(dimethylamino)-5-methylpyrimidin-2-yl]amino}cyclohexyl)propanamide
2-(4-chlorophenyl)-N-(cis-4-{[4-(dimethylamino)-5-methylpyrimidin-2-yl]amino}cyclohexyl)-2-hydroxyacetamide;
N-(cis-4-{[4-(dimethylamino)-5-methylpyrimidin-2-yl]amino}cyclohexyl)-1-(4-methoxyphenyl)cyclopropanecarboxamide;
$N^2$-(3-chlorophenyl)-N-(cis-4-{[4-(dimethylamino)-5-methylpyrimidin-2-yl]amino}cyclohexyl)-$N^2$-methylglycinamide;
$N^2$-(3,4-dichlorophenyl)-N-(cis-4-{[4-(dimethylamino)-5-methylpyrimidin-2-yl]amino}cyclohexyl)-$N^2$-methylglycinamide;
N-(cis-4-{[4-(dimethylamino)-5-methylpyrimidin-2-yl]amino}cyclohexyl)-$N^2$-methyl-$N^2$-(3-methylphenyl)glycinamide;
N-(cis-4-{[4-(dimethylamino)-5-methylpyrimidin-2-yl]amino}cyclohexyl)-$N^2$-(3-fluorophenyl)-$N^2$-methylglycinamide;
N-(cis-4-{[4-(dimethylamino)-5-methylpyrimidin-2-yl]amino}cyclohexyl)-$N^2$-(4-fluorophenyl)-$N^2$-methylglycinamide;
$N^2$-(4-chlorophenyl)-N-(cis-4-{[4-(dimethylamino)-5-methylpyrimidin-2-yl]amino}cyclohexyl)-$N^2$-methylglycinamide;
$N^2$-(3,4-difluorophenyl)-N-(cis-4-{[4-(dimethylamino)-5-methylpyrimidin-2-yl]amino}cyclohexyl)-$N^2$-methylglycinamide;
N-(cis-4-{[4-(dimethylamino)-5-methylpyrimidin-2-yl]amino}cyclohexyl)-$N^2$-(3-methoxyphenyl)-$N^2$-methylglycinamide;
2-(3,4-dichlorophenoxy)-N-(cis-4-{[4-methyl-6-(methylamino)pyrimidin-2-yl]amino}cyclohexyl)acetamide;
trans-2-(4-chlorophenyl)-N-(cis-4-{[4-(dimethylamino)-5-methylpyrimidin-2-yl]amino}cyclohexyl)cyclopropanecarboxamide;
trans-2-(3-chlorophenyl)-N-(cis-4-{[4-(dimethylamino)-5-methylpyrimidin-2-yl]amino}cyclohexyl)cyclopropanecarboxamide;
trans-2-(3,4-difluorophenyl)-N-(cis-4-{[4-(dimethylamino)-5-methylpyrimidin-2-yl]amino}cyclohexyl)cyclopropanecarboxamide;
trans-2-(3,4-dichlorophenyl)-N-(cis-4-{[4-(dimethylamino)-5-methylpyrimidin-2-yl]amino}cyclohexyl)cyclopropanecarboxamide;
trans-2-[3,5-bis(trifluoromethyl)phenyl]-N-(cis-4-{[4-(dimethylamino)-5-methylpyrimidin-2-yl]amino}cyclohexyl)cyclopropanecarboxamide;
2-[(4-chlorophenyl)sulfonyl]-N-(cis-4-{[4-(dimethylamino)-5-methylpyrimidin-2-yl]amino}cyclohexyl)nicotinamide;
N-(cis-4-{[4-(dimethylamino)-5-methylpyrimidin-2-yl]amino}cyclohexyl)-2-{[1-methyl-3-(trifluoromethyl)-1H-pyrazol-5-yl]oxy}acetamide;
N-[cis-4-(4-dimethylamino-5-methyl-pyrimidin-2-ylamino)-cyclohexyl]-2-phenoxy-nicotinamide;
N-[cis-4-(4-dimethylamino-6-methyl-pyrimidin-2-ylamino)-cyclohexyl]-2-phenoxy-nicotinamide;
3-chloro-N-[cis-4-(4-dimethylamino-5-methyl-pyrimidin-2-ylamino)-cyclohexyl]-4-fluoro-benzamide;
4-chloro-N-[cis-4-(4-dimethylamino-6-methyl-pyrimidin-2-ylamino)-cyclohexyl]-3-fluoro-benzamide;

3-chloro-N-[cis-4-(4-dimethylamino-6-methyl-pyrimidin-2-ylamino)-cyclohexyl]-5-fluoro-benzamide;
N-[cis-4-(4-dimethylamino-6-methyl-pyrimidin-2-ylamino)-cyclohexyl]-3,4,5-trifluoro-benzamide;
3-chloro-4-fluoro-N-[cis-4-(5-methyl-4-methylamino-pyrimidin-2-ylamino)-cyclohexyl]-benzamide;
4-chloro-N-[cis-4-(4-dimethylamino-5-methyl-pyrimidin-2-ylamino)-cyclohexyl]-3-fluoro-benzamide;
3-chloro-N-[cis-4-(4-dimethylamino-5-methyl-pyrimidin-2-ylamino)-cyclohexyl]-5-fluoro-benzamide;
N-[cis-4-(4-dimethylamino-5-methyl-pyrimidin-2-ylamino)-cyclohexyl]-3,4,5-trifluoro-benzamide;
N-[cis-4-(4-dimethylamino-5-methyl-pyrimidin-2-ylamino)-cyclohexyl]-3,5-difluoro-benzamide; and
2-(3,4-difluoro-phenyl)-N-[cis-4-(4-dimethylamino-5-methyl-pyrimidin-2-ylamino)-cyclohexyl]-acetamide;

or a pharmaceutically acceptable salt, hydrate, or solvate thereof.

**88.** The compound according to claim 75 wherein $R_1$ is selected from the group consisting of:

(i) $C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:

  - •• halogen,
  - •• nitro,
  - •• $C_{1-5}$ alkylcarbonylamino,
  - •• $C_{3-6}$ cycloalkylcarbonylamino,
  - •• $C_{1-5}$ alkyl,
  - •• $C_{1-5}$ alkyl substituted by halogen,
  - •• $C_{1-5}$ alkoxy, and
  - •• $C_{1-5}$ alkoxy substituted by halogen,

(ii) $C_{3-12}$ cycloalkyl, and
$C_{3-12}$ cycloalkyl substituted by carbocyclic aryl,
(iii) carbocyclic aryl, and
carbocyclic aryl substituted by substituent(s) independently selected from the group consisting of:

- halogen,
- $C_{1-10}$ alkyl,
- $C_{1-10}$ alkyl substituted by halogen,
- $C_{1-9}$ alkoxy, and
- $C_{1-5}$ alkylthio,

(iv) heterocyclyl,

L is Formula (XV);
Y is $-C(O)NR_5-$;
$R_2$ is selected from the group consisting of:

$-N(R_{2a})(R_{2b})$, wherein $R_{2a}$ is hydrogen or $C_{1-5}$ alkyl and $R_{2b}$ is $C_{1-5}$ alkyl;
wherein carbocyclic aryl is phenyl or naphthyl;
heterocyclyl is 3,4-dihydro-1*H*-isoquinolinyl; and
halogen is fluoro, chloro, bromo, or iodo;

or a pharmaceutically acceptable salt, hydrate, or solvate thereof.

**89.** The compound according to claim 88 wherein $R_1$ is selected from the group consisting of:

(i) $C_{1-6}$ alkyl, and
$C_{1-16}$ alkyl substituted by substituent(s) independently selected from the group consisting of:

- carbocyclic aryl,

- carbocyclic aryl substituted by substituent(s) independently selected from the group consisting of:

  - •• halogen,
  - •• nitro,
  - •• $C_{1-5}$ alkyl,
  - •• $C_{1-5}$ alkyl substituted by halogen,
  - •• $C_{1-5}$ alkoxy, and
  - •• $C_{1-5}$ alkoxy substituted by halogen,

(ii) $C_{3-12}$ cycloalkyl, and
$C_{3-12}$ cycloalkyl substituted by carbocyclic aryl,
(iii) carbocyclic aryl, and
carbocyclic aryl substituted by substituent(s) independently selected from the group consisting of:

- halogen,
- $C_{1-10}$ alkyl,
- $C_{1-10}$ alkyl substituted by halogen, and
- $C_{1-9}$ alkoxy;

$R_2$ is selected from the group consisting of:

- $N(R_{2a})(R_{2b})$, wherein $R_{2a}$ is hydrogen or $C_{1-5}$ alkyl and $R_{2b}$ is $C_{1-5}$ alkyl;
wherein carbocyclic aryl is phenyl or naphthyl;
heterocyclyl is 3,4-dihydro-1H-isoquinolinyl; and
halogen is fluoro, chloro, bromo, or iodo;

or a pharmaceutically acceptable salt, hydrate, or solvate thereof.

**90.** The compound according to any one of claims 75, 88, and 89 wherein p is 1 and T is $C_{1-5}$ alkyl; $R_3$ and $R_4$ are both hydrogen; and A and B are both single bonds; $R_5$ is hydrogen: or a pharmaceutically acceptable salt, hydrate, or solvate thereof.

**91.** The compound according to claim 1 selected from the group consisting of:

cis-4-{[4-(dimethylamino)-6-methylpyrimidin-2-yl] amino}-N-(3-iodobenzyl)cyclohexanecarboxamide;
cis-N-(2,4-dichlorobenzyl)-4-{[4-(dimethylamino)-6-methylpyrimidin-2-yl]amino}cyclohexanecarboxamide;
cis-N-(2,5-dichlorobenzyl)-4-{[4-(dimethylamino)-6-methylpyrimidin-2-yl]amino}cyclohexanecarboxamide;
cis-4-{[4-(dimethylamino)-6-methylpyrimidin-2-yl]amino}-N-(4-methylbenzyl)cyclohexanecarboxamide;
cis-N-(3,5-dichlorobenzyl)-4-{[4-(dimethylamino)-6-methylpyrimidin-2-yl]amino}cyclohexanecarboxamide;
cis-N-(3,5-dimethoxybenzyl)-4-{[4-(dimethylamino)-6-methylpyrimidin-2-yl]amino}cyclohexanecarboxamide;
cis-N-(3-chlorobenzyl)-4-{[4-(dimethylamino)-6-methylpyrimidin-2-yl]amino}cyclohexanecarboxamide;
cis-4-[4-(dimethylamino)-6-methylpyrimidin-2-yl]amino}-N-[3-(trifluoromethyl)benzyl]cyclohexanecarboxamide;
cis-N-[3,5-bis(trifluoromethyl)benzyl)-4-{[4-(dimethylamino)-6-methylpyrimidin-2-yl]amino}cyclohexanecarboxamide;
cis-4-{[4-(dimethylamino)-6-methylpyrimidin-2-yl]amino}-N-(3-methoxybenzyl)cyclohexanecarboxamide;
cis-N-(4-chlorobenzyl)-4-{[4-(dimethylamino)-6-methylpyrimidin-2-yl]amino}cyclohexanecarboxamide;
cis-N-(3,4-dichlorobenzyl)-4-{[4-(dimethylamino)-6-methylpyrimidin-2-yl]amino}cyclohexanecarboxamide;
cis-N-(2,5-difluorobenzyl)-4-{[4-(dimethylamino)-6-methylpyrimidin-2-yl]amino}cyclohexanecarboxamide;
cis-N-(2,3-difluorobenzyl)-4-{[4-(dimethylamino)-6-methylpyrimidin-2-yl]amino}cyclohexanecarboxamide;
cis-N-(4-bromo-2-fluorobenzyl)-4-{[4-(dimethylamino)-6-methylpyrimidin-2-yl]amino}cyclohexanecarboxamide;
cis-N-(2,4-difluorobenzyl)-4-{[4-(dimethylamino)-6-methylpyrimidin-2-yl]amino}cyclohexanecarboxamide;
cis-4-{[4-(dimethylamino)-6-methylpyrimidin-2-yl]amino}-N-(3-methylbenzyl)cyclohexanecarboxamide;
cis-4-{[4-(dimethylamino)-6-methylpyrimidin-2-yl]amino}-N-[2-(trifluoromethoxy)benzyl]cyclohexanecarboxamide;
cis-4-{[4-(dimethylamino)-5-methylpyrimidin-2-yl]amino}-N-[(1R)-1-phenylethyl]cyclohexanecarboxamide;
cis-4-{[4-(dimethylamino)-5-methylpyrimidin-2-yl]amino}-N-[(1S)-1-(4-methylphenyl)ethyl]cyclohexanecarboxamide;
cis-4-{[4-(dimethylamino)-5-methylpyrimidin-2-yl]amino}-N-[(1R)-1-(4-fluorophenyl)ethyl]cyclohexanecarboxamide;
cis-4-{[4-(dimethylamino)-5-methylpyrimidin-2-yl]amino}-N-[(1S)-1-(4-fluorophenyl)ethyl]cyclohexanecarboxamide;
cis-4-{[4-(dimethylamino)-5-methylpyrimidin-2-yl]amino}-N-[(1R)-1-(3-methoxyphenyl)ethyl]cyclohexanecarboxamide;
cis-4-{[4-(dimethylamimo)-5-methylpyrimidin-2-yl]amino}-N-[(1S)-1-(3-methoxyphenyl)ethyl]cyclohexanecarboxamide;
cis-4-{[4-(dimethylamino)-5-methylpyrimidin-2-yl]amino}-N-[(1S)-1-(4-methoxyphenyl)ethyl]cyclohexanecarboxamide;
cis-N-[(1R)-1-(4-chlorophenyl)ethyl]-4-{[4-(dimethylamino)-5-methylpyrimidin-2-yl]amino}cyclohexanecarboxamide;
cis-N-[1-(4-bromophenyl)ethyl]-4-{[4-(dimethylamino)-5-methylpyrimidin-2-yl]amino}cyclohexanecarboxamide;
cis-4-{[4-(dimethylamino)-5-methylpyrimidin-2-yl]amino}-N-[(1R)-1-(4-nitrophenyl)ethyl]cyclohexanecarboxamide;
cis-4-{[4-(dimethylamino)-5-methylpyrimidin-2-yl]amino}-N-[(1S)-1-(4-nitrophenyl)ethyl]cyclohexanecarboxamide;
cis-4-{[4-(dimethylamino)-5-methylpyrimidin-2-yl]amino}-N-(3-fluorophenyl)cyclohexanecarboxamide;
cis-4-{[4-(dimethylamino)-5-methylpyrimidin-2-yl]amino}-N-(3-methoxyghenyl)cyclohexanecarboxamide;
cis-N-(3-chlorophenyl)-4-{[4-(dimethylamino)-5-methylpyrimidin-2-yl]amino}cyclohexanecarboxamide;
cis-4-{[4-(dimethylamino)-5-methylpyrimidin-2-yl]amino}-N-[(1S,2R)-2-phenylcyclopropyl]cyclohexanecarboxamide;
cis-4-{[4-(dimethylamino)-5-methylpyrimidin-2-yl]amino}-N-[4-(trifluoromethyl)phenyl]cyclohexanecarboxamide;
cis-4-{[4-(dimethylamino)-6-methylpyrimidin-2-yl]amino}-N-[(1R)-1-(3-methoxyphenyl)ethyl]cyclohexanecarboxamide;
cis-N-[(1S)-1-(4-chlorophenyl)ethyl]-4-{[4-(dimethylamino)-6-methylpyrimidin-2-yl]amino}cyclohexanecarboxamide;
cis-N-benzyl-4-{[4-(dimethylamino)-6-methylpyrimidin-2-yl]amino}cycloltexanecarboxamide;
cis-4-{[4-(dimethylamino)-6-methylpyrimidin-2-yl]amino}-N-(4-fluorobenzyl)cyclohexanecarboxamide;
cis-N-(3,4-difluorobenzyl)-4-{[4-(dimethylamino)-6-methylpyrimidin-2-yl]amino}cyclohexanecarboxamide;
cis-4-{[4-(dimethylamino)-6-methylpyrimidin-2-yl]amino}-N-[(1S)-1-(4-methoxyphenyl)ethyl]cyclohexanecarboxamide;
cis-4-{[4-(dimethylamino)-6-methylpyrimidin-2-yl]amino}-N-[(1S)-1-(3-methoxyphenyl)ethyl]cyclobexanecarboxamide;
cis-4-{[4-(dimethylamino)-6-methylpyrimidin-2-yl]amino}-N-[(1R)-1-(4-fluorophenyl)ethyl]cyclohexanecarboxamide;
cis-N-[(1R)-1-(4-chlorophenyl)ethyl]-4-{[4-(dimethylamino)-6-methylpyrimidin-2-yl]amino}cyclohexanecarboxamide;
cis-4-{[4-(dimethylamino)-5-methylpyrimidin-2-yl]amino}-N-[(1S)-1-(1-naphthyl)ethyl]cyclohexanecarboxamide;
cis-N-[(1R)-1-(4-bromophenyl)ethyl]-4-{[4-(dimethylamino)-5-methylpyrimidin-2-yl]amino}cyclohexanecarboxamide;
cis-N-[(1S)-1-(4-bromophenyl)ethyl]-4-{[4-(dimethylamino)-5-methylpyrimidin-2-yl]amino}cyclohexanecarboxamide;
cis-4-{[4-(dimethylamino)-5-methylpyrimidin-2-yl]amino}-N-{(1S)-1-[4-(trifluoromethoxy)phenyl]ethyl}cyclohexanecarboxamide;
cis-4-{[4-(dimethylamino)-5-methylpyrimidin-2-yl]amino}-N-[(1R)-1-(2-fluorophenyl)ethyl]cyclohexanecarboxamide;
cis-N-{(1S)-1-[3,5-bis(trifluoromethyl)phenyl]ethyl}-4-{[4-(dimethylamino)-5-methylpyrimidin-2-yl]amino}cyclohexanecarboxamide;
4-{[4-(dimethylamino)-5-methylpyrimidin-2-yl]amino}-N-{(1S)-1-[3-(trifluoromethyl)phenyl]ethyl}cyclohexanecarboxamide;
4-{[4-(dimethylamino)-5-methylpyrimidin-2-yl]amino}-N-{(1S)-1-[2-(trifluoromethyl)phenyl]ethyl}cyclohexanecarboxamide;
cis-4-{[4-(dimethylamino)-5-methylpyrimidin-2-yl]amino}-N-{(1R)-1-[4-(trifluoromethoxy)phenyl]ethyl}cyclohexanecarboxamide;
cis-N-[(1S)-1-(4-chlorophenyl)ethyl]-4-{[4-(dimethylamino)-5-methylpyrimidin-2-yl]amino}cyclohexanecarboxamide;
cis-N-[(1R)-1-(4-chlorophenyl)ethyl]-4-{[4-(dimethylamino)-5-methylpyrimidin-2-yl]amino}cyclohexanecarboxamide;
cis-N-[1-(4-chlorophenyl)-1-methylethyl]-4-{[4-(dimethylamino)-5-methylpyrimidin-2-yl]amino}cyclohexanecarboxamide; and
cis-N-{1-[3,5-bis(trifluoromethyl)phenyl]-1-methylethyl}-4-}[4-(dimethylamino)-5-methylpyrimidin-2-yl]amino}cyclohexanecarboxamide;

or a pharmaceutically acceptable salt, hydrate, or solvate thereof.

**92.** The compound according to claim 1 selected from the group consisting of:

cis-4-{[4-(dimethylamino)-6-methylpyrimidin-2-yl]amino}-N-(3-iodobenzyl)cyclohexanecarboxamide;
cis-N-(2,4-dichlorobenzyl)-4-{[4-(dimethylamino)-6-methylpyrimidin-2-yl]amino}cyclohexanecarboxamide;
cis-N-(2,5-dichlorobenzyl)-4-{[4-(dimethylamino)-6-methylpyrimidin-2-yl]amino}cyclohexanecarboxamide;
cis-4-{[4-(dimethylamino)-6-methylpyrimidin-2-yl]amino}-N-(4-methylbenzyl)cyclohexanecarboxamide;
cis-N-(3,5-dichlorobenzyl)-4-{[4-(dimethylamino)-6-methylpyrimidin-2-yl]amino}cyclohexanecarboxamide;
cis-N-(3,5-dimethoxybenzyl)-4-{[4-(dimethylamino)-6-methylpyrimidin-2-yl]amino}cyclohexanecarboxamide;
cis-N-(3-chlorobenzyl)-4-{[4-(dimethylamino)-6-methylpyrimidin-2-yl]amino}cyclohexanecarboxamide;
cis-N-[3,5-bis(trifluoromethyl)benzyl]-4-{[4-(dimethylamino)-6-methylpyrimidin-2-yl]amino}cyclohexanecarboxamide;
cis-4-{[4-(dimethylamino)-6-methylpyrimidin-2-yl]amino}-N-(3-methoxybenzyl)cyclohexanecarboxamide;
cis-N-(4-chlorobenzyl)-4-{[4-(dimethylamino)-6-methylpyrimidin-2-yl]amino}cyclohexanecarboxamide;
cis-N-(3,4-dichlorobenzyl)-4-{[4-(dimethylamino)-6-methylpyrimidin-2-yl]amino}cyclohexanecarboxamide;
cis-N-(2,5-difluorobenzyl)-4-{[4-(dimethylamino)-6-methylpyrimidin-2-yl]amino}cyclohexanecarboxamide;
cis-N-(2,3-difluorobenzyl)-4-{[4-(dimethylamino)-6-methylpyrimidin-2-yl]amino}cyclohexanecarboxamide;
cis-N-(4-bromo-2-fluorobenzyl)-4-{[4-(dimethylamino)-6-methylpyrimidin-2-yl]amino}cyclohexanecarboxamide;
cis-N-(2,4-difluorobenzyl)-4-{[4-(dimethylamino)-6-methylpyrimidin-2-yl]amino}cyclohexanecarboxamide;
cis-4-{[4-(dimethylamino)-6-methylpyrimidin-2-yl]amino}-N-(3-methylbenzyl)cyclohexanecarboxamide;
cis-4-{[4-(dimethylamino)-6-methylpyrimidin-2-yl]amino}-N-[2-(trifluoromethoxy)benzyl]cyclohexanecarboxamide;
cis-4-{[4-(dimethylamino)-5-methylpyrimidin-2-yl]amino}-N-[(1S)-1-(4-methylphenyl)ethyl]cyclohexanecarboxamide;
cis-4-{[4-(dimethylamino)-5-methylpyrimidin-2-yl]amino}-N-[(1R)-1-(4-fluorophenyl)ethyl]cyclohexanecarboxamide;
cis-4-{[4-(dimethylamino)-5-methylpyrimidin-2-yl]amino}-N-[(1R)-1-(3-methoxyphenyl)ethyl]cyclohexanecarboxamide;
cis-4-{[4-(dimethylamino)-5-methylpyrimidin-2-yl]amino}-N-[(1S)-1-(3-methoxyphenyl)ethyl]cyclohexanecarboxamide;
cis-4-{[4-(dimethylamino)-5-methylpyrimidin-2-yl]amino}-N-[(1S)-1-(4-methoxyphenyl)ethyl]cyclohexanecarboxamide;
cis-N-[(1R)-1-(4-chlorophenyl)ethyl]-4-{[4-(dimethylamino)-5-methylpyrimidin-2-yl]amino}cyclohexanecarboxamide;
cis-N-[1-(4-bromophenyl)ethyl]-4-{[4-(dimethylamino)-5-methylpyrimidin-2-yl]amino}cyclohexanecarboxamide;
cis-4-{[4-(dimethylamino)-5-methylpyrimidin-2-yl]amino}-N-[(1R)-1-(4-nitrophenyl)ethyl]cyclohexanecarboxamide;
cis-4-{[4-(dimethylamino)-5-methylpyrimidin-2-yl]amino}-N-(3-methoxyphenyl)cyclohexanecarboxamide;
cis-N-(3-chlorophenyl)-4-{[4-(dimethylamino)-5-methylpyrimidin-2-yl]amino}cyclohexanecarboxamide;
cis-4-{[4-(dimethylamino)-5-methylpyrimidin-2-yl]amino}-N-[(1S,2R)-2-phenylcyclopropyl]cyclohexanecarboxamide;
cis-4-{[4-(dimethylamino)-5-methylpyrimidin-2-yl]amino}-N-[4-(trifluoromethyl)phenyl]cyclohexanecarboxamide;
cis-N-[(1S)-1-(4-chlorophenyl)ethyl]-4-{[4-(dimethylamino)-6-methylpyrimidin-2-yl]amino}cyclohexanecarboxamide;

cis-N-(3,4-difluorobenzyl)-4-{[4-(dimethylamino)-6-methylpyrimidin-2-yl]amino}cyclohexanecarboxamide;
cis-4-{[4-(dimethylamino)-6-methylpyrimidin-2-yl]amino}-N-[(1S)-1-(4-methoxyphenyl)ethyl]cyclohexanecarboxamide;
cis-4-{[4-(dimethylamino)-6-methylpyrimidin-2-yl]amino}-N-[(1S)-1-(3-methoxyphenyl)ethyl]cyclohexanecarboxamide;
cis-N-[(1R)-1-(4-chlorophenyl)ethyl]-4-{[4-(dimethylamino)-6-methylpyrimidin-2-yl]amino}cyclohexanecarboxamide;
cis-4-{[4-(dimethylamino)-5-methylpyrimidin-2-yl]amino}-N-[(1S)-1-(1-naphthyl)ethyl]cyclohexanecarboxamide;
cis-N-[(1S)-1-(4-bromophenyl)ethyl]-4-{[4-(dimethylamino)-5-methylpyrimidin-2-yl]amino}cyclohexanecarboxamide;
cis-4-{[4-(dimethylamino)-5-methylpyrimidin-2-yl]amino}-N-{(1S)-1-[4-(trifluoromethoxy)phenyl]ethyl}cyclohexanecarboxamide;
cis-4-{[4-(dimethylamino)-5-methylpyrimidin-2-yl]amino}-N-[(1R)-1-(2-fluorophenyl)ethyl]cyclohexanecarboxamide;
cis-N-{(1S)-1-[3,5-bis(trifluoromethyl)phenyl]ethyl} -4-{[4-(dimethylamino)-5-methylpyrimidin-2-yl]amino}cyclohexanecarboxamide;
4-{[4-(dimethylamino)-5-methylpyrimidin-2-yl]amino}-N-{(1S)-1-[3-(trifluoromethyl)phenyl]ethyl}cyclohexanecarboxamide;
4-{[4-(dimethylamino)-5-methylpyrimidin-2-yl]amino}-N-{(1S)-1-[2-(trifluoromethyl)phenyl]ethyl}cyclohexanecarboxamide; and
cis-N-[(1R)-1-(4-chlorophenyl)ethyl]-4-{[4-(dimethylamino)-5-methylpyrimidin-2-yl]amino}cyclohexanecarboxamide;

or a pharmaceutically acceptable salt, hydrate, or solvate thereof.

**93.** The compound according to claim 75 wherein $R_1$ is selected from the group consisting of:

(i) $C_{1-16}$ alkyl, and
$C_{1-16}$ alkyl substituted by substituent(s) independently selected from the group consisting of:

- carbocyclic aryl,
- carbocyclic aryl substituted by substituent(s) independently selected from the group consisting of:
  - halogen,
  - $C_{1-5}$ alkyl, and
  - $C_{1-5}$ alkyl substituted by halogen,

(ii) $C_{3-12}$ cycloalkyl, and
$C_{3-12}$ cycloalkyl substituted by substituent(s) independently selected from the group consisting of:

- carbocyclic aryl, and
- carbocyclic aryl substituted by halogen,

(iii) carbocyclic aryl, and
carbocyclic aryl substituted by substituent(s) independently selected from the group consisting of:

- halogen,
- $C_{1-10}$ alkyl,
- $C_{1-10}$ alkyl substituted by halogen,
- $C_{1-9}$ alkoxy,
- $C_{1-9}$ alkoxy substituted by substituent(s) independently selected from the group consisting of:
  - halogen, and
  - carbocyclic aryl,

L is Formula (VII);
Y is $-C(O)NR_5-$;

$R_2$ is $-N(R_{2a})(R_{2b})$ wherein $R_{2a}$ is hydrogen or $C_{1-5}$ alkyl and $R_{2b}$ is $C_{1-5}$ alkyl;
wherein carbocyclic aryl is phenyl; and
halogen is fluoro, chloro, bromo, or iodo;

or a pharmaceutically acceptable salt, hydrate, or solvate thereof.

**94.** The compound according to claim 75 or 93 wherein p is 1 or 2 and each T is independently $C_{1-5}$ alkyl; $R_3$ is hydrogen; $R_4$ is hydrogen or $C_{1-5}$ alkyl; A and B are both single bonds; $R_5$ is hydrogen; or a pharmaceutically acceptable salt, hydrate, or solvate thereof.

**95.** The compound according to claim 1 selected from the group consisting of:

N-(3,4-dimethoxyphenyl)-N'-(cis-4-{[4-(dimethylamino)-5,6-dimethylpyrimidin-2-yl]amino}cyclohexyl)urea;
N-(3-chlorophenyl)-N'-(cis-4-{[4-(dimethylamino)-5-methylpyrimidin-2-yl]amino}cyclohexyl)-N-methylurea;
N-(3,4-dichlorophenyl)-N'-(cis-4-{[4-(dimethylamino)-5-methylpyrimidin-2-yl]amino}cyclohexyl)-N-methylurea;
N'-(cis-4-{[4-(dimethylamino)-5-methylpyrimidin-2-yl]amino}cyclohexyl)-N-methyl-N-(3-methylphenyl)urea;
N'-(cis-4-{[4-(dimethylamino)-5-methylpyrimidin-2-yl]amino}cyclohexyl)-N-methyl-N-(4-methylphenyl)urea;
N'-(cis-4-{[4-(dimethylamino)-5-methylpyrimidin-2-yl]amino}cyclohexyl)-N-(3-fluorophenyl)-N-methylurea;
N'-(cis-4-{[4-(dimethylamino)-5-methylpyrimidin-2-yl]amino}cyclohexyl)-N-(4-fluorophenyl)-N-methylurea;
N-(4-chlorophenyl)-N'-(cis-4-{[4-(dimethylamino)-5-methylpyrimidin-2-yl]amino}cyclohexyl)-N-methylurea;
N-(3,4-difluorophenyl)-N'-(cis-4-{[4-(dimethylamino)-5-methylpyrimidin-2-yl]amino}cyclohexyl)-N-methylurea;
N'-(cis-4-{[4-(dimethylamino)-5-methylpyrimidin-2-yl]amino}cyclohexyl)-N-(3-methoxyphenyl)-N-methylurea;
N'-(cis-4-{[4-(dimethylamino)-5-methylpyrimidin-2-yl]amino}cyclohexyl)-N-(4-methoxyphenyl)-N-methylurea;
N-{1-[3,5-bis(trifluoromethyl)phenyl]-1-methylethyl}-N'-(cis-4-{[4-(dimethylamino)-5-methylpyrimidin-2-yl]amino}cyclohexyl)urea;
N-[1-(4-chlorophenyl)-1-methylethyl]-N'-(cis-4-{[4-(dimethylamino)-5-methylpyrimidin-2-yl]amino}cyclohexyl)urea;
N-[1-(4-chlorophenyl)-1-methylethyl]-N'-(cis-4-{[4-(dimethylamino)-6-methylpyrimidin-2-yl]amino}cyclohexyl)urea;
N-[1-(4-chlorophenyl)-1-methylethyl]-N'-(cis-4-{[4-(dimethylamino)-5-methylpyrimidin-2-yl]amino}cyclohexyl)-N-methylurea;
N-[1-(4-chlorophenyl)-1-methylethyl]-N'-(cis-4-{[4-(dimethylamino)-6-methylpyrimidin-2-yl]amino}cyclohexyl)-N-methylurea;
N-[1-(4-chlorophenyl)cyclopropyl]-N'-(cis-4-{[4-(dimethylamino)-5-methylpyrimidin-2-yl]amino}cyclohexyl)-N-methylurea;
N-(cis-4-{[4-(dimethylamino)-5-methylpyrimidin-2-yl]amino}cyclohexyl)-N'-(2-methoxyphenyl)urea;
N-(cis-4-{[4-(dimethylamino)-5-methylpyrimidin-2-yl]amino}cyclohexyl)-N'-(3-methoxyphenyl)urea;
N-(3,4-dimethoxyphenyl)-N'-(cis-4-{[4-(dimethylamino)-5-methylpyrimidin-2-yl]amino}cyclohexyl)urea;
N-(cis-4-{[4-(dimethylamino)-5-methylpyrimidin-2-yl]amino}cyclohexyl)-N'-(4-fluorophenyl)urea;
N-(3,4-difluorophenyl)-N'-(cis-4-{[4-(dimethylamino)-5-methylpyrimidin-2-yl]amino}cyclohexyl)urea;
N-(cis-4-{[4-(dimethylamino)-5-methylpyrimidin-2-yl]amino}cyclohexyl)-N'-[2-(trifluoromethoxy)phenyl]urea;
N-(4-chlorophenyl)-N'-(cis-4-{[4-(dimethylamino)-5-methylpyrimidin-2-yl]amino}cyclohexyl)urea;
N-[3,5-bis(trifluoromethyl)phenyl]-N'-(cis-4-{[4-(dimethylamino)-5-methylpyrimidin-2-yl]amino}cyclohexyl)urea;
N-(4-bromophenyl)-N'-(cis-4-{[4-(dimethylamino)-5-methylpyrimidin-2-yl]amino}cyclohexyl)urea;
N-(cis-4-{[4-(dimethylamino)-5-methylpyrimidin-2-yl]amino}cyclohexyl)-N'-(2-methylphenyl)urea;
N-benzyl-N'-(cis-4-{[4-(dimethylamino)-5-methylpyrimidin-2-yl]amino}cyclohexyl)urea;
N-[2-chloro-6-(trifluoromethyl)phenyl]-N'-(cis-4-{[4-(dimethylamino)-5-methylpyrimidin-2-yl]amino}cyclohexyl)urea;
N-(cis-4-{[4-(dimethylamino)-5-methylpyrimidin-2-yl]amino}cyclohexyl)-N'-(2,4,6-trichlorophenyl)urea;
N-(2,4-dichlorophenyl)-N'-(cis-4-{[4-(dimethylamino)-5-methylpyrimidin-2-yl]amino}cyclohexyl)-N-methylurea;
N'-(cis-4-{[4-(dimethylamino)-5-methylpyrimidin-2-yl]amino}cyclohexyl)-N-methyl-N-[2-(trifluoromethoxy)phenyl]urea;
N-(4-chlorophenyl)-N'-(cis-4-{[4-(dimethylamino)-5-methylpyrimidin-2-yl]amino}cyclohexyl)-N-ethylurea;
N-[3,5-bis(trifluoromethyl)phenyl]-N'-(cis-4-{[4-(dimethylamino)-5-methylpyrimidin-2-yl]amino}cyclohexyl)-N-ethylurea;
N'-(cis-4-{[4-(dimethylamino)-5-methylpyrimidin-2-yl]amino}cyclohexyl)-N-(2-fluorophenyl)-N-methylurea;
N'-(cis-4-{[4-(dimethylamino)-5-methylpyrimidin-2-yl]amino}cyclohexyl)-N-ethyl-N-[2-(trifluoromethoxy)phenyl]urea;
N'-(cis-4-{[4-(dimethylamino)-5-methylpyrimidin-2-yl]amino}cyclohexyl)-N-ethyl-N-phenylurea;
N'-(cis-4-{[4-(dimethylamino)-5-methylpyrimidin-2-yl] amino}cyclohexyl)-N-ethyl-N-(3-methylphenyl)urea; and
1-(2,3-dichloro-phenyl)-3-[cis-4-(4-dimethylamino-5-methyl-pyrimidin-2-ylamino)-cyclohexylmethyl]-urea;

or a pharmaceutically acceptable salt, hydrate, or solvate thereof.

**96.** The compound according to claim 1 selected from the group consisting of:

N-(3,4-dimethoxyphenyl)-N'-(cis-4-{[4-(dimethylamino)-5,6-dimethylpyrimidin-2-yl]amino}cyclohexyl)urea;
N-(3-chlorophenyl)-N'-(cis-4-{[4-(dimethylamino)-5-methylpyrimidin-2-yl]amino}cyclohexyl)-N-methylurea;
N-(3,4-dichlorophenyl)-N'-(cis-4-{[4-(dimethylamino)-5-methylpyrimidin-2-yl]amino}cyclohexyl)-N-methylurea;
N'-(cis-4-{[4-(dimethylamino)-5-methylpyrimidin-2-yl]amino}cyclohexyl)-N-methyl-N-(3-methylphenyl)urea;
N'-(cis-4-{[4-(dimethylamino)-5-methylpyrimidin-2-yl]amino}cyclohexyl)-N-methyl-N-(4-methylphenyl)urea;
N'-(cis-4-{[4-(dimethylamino)-5-methylpyrimidin-2-yl]amino}cyclohexyl)-N-(3-fluorophenyl)-N-methylurea;
N'-(cis-4-{[4-(dimethylamino)-5-methylpyrimidin-2-yl]amino}cyclohexyl)-N-(4-fluorophenyl)-N-methylurea;
N-(4-chlorophenyl)-N'-(cis-4-{[4-(dimethylamino)-5-methylpyrimidin-2-yl]amino}cyclohexyl)-N-methylurea;
N-(3,4-difluorophenyl)-N'-(cis-4-{[4-(dimethylamino)-5-methylpyrimidin-2-yl]amino}cyclohexyl)-N-methylurea;
N'-(cis4-{[4-(dimethylamino)-5-methylpyrimidin-2-yl]amino}cyclohexyl)-N-(3-methoxyphenyl)-N-methylurea;
N'-(cis-4-{[4-(dimethylamino)-5-methylpyrimidin-2-yl]amino}cyclohexyl)-N-(4-methoxyphenyl)-N-methylurea;
N-[1-(4-chlorophenyl)-1-methylethyl] -N'-(cis-4-{[4-(dimethylamino)-5-methylpyrimidin-2-yl]amino}cyclohexyl)urea;
N-[1-(4-chlorophenyl)-1-methylethyl]-N'-(cis-4-{[4-(dimethylamino)-6-methylpyrimidin-2-yl]amino}cyclohexyl)urea;
N-[1-(4-chlorophenyl)-1-methylethyl]-N'-(cis-4-{[4-(dimethylamino)-5-methylpyrimidin-2-yl]amino}cyclohexyl)-N-methylurea;
N-(cis-4-{[4-(dimethylamino)-5-methylpyrimidin-2-yl]amino}cyclohexyl)-N'-(4-fluorophenyl)urea;
N-(cis-4-{[4-(dimethylamino)-5-methylpyrimidin-2-yl]amino}cyclohexyl)-N'-[2-(trifluoromethoxy)phenyl]urea;
N-(4-bromophenyl)-N'-(cis-4-{[4-(dimethylamino)-5-methylpyrimidin-2-yl]amino}cyclohexyl)urea;
N-(cis-4-{[4-(dimethylamino)-5-methylpyrimidin-2-yl]amino}cyclohexyl)-N'-(2-methylphenyl)urea;
N-(cis-4-{[4-(dimethylamino)-5-methylpyrimidin-2-yl]amino}cyclohexyl)-N'-(2,4,6-trichlorophenyl)urea;
N-(2,4-dichlorophenyl)-N'-(cis-4-{[4-(dimethylamino)-5-methylpyrimidin-2-yl]amino}cyclohexyl)-N-methylurea;
N'-(cis-4-{[4-(dimethyiamino)-5-methylpyrimidin-2-yl]amino}cyclohexyl)-N-methyl-N-[2-(trifluoromethoxy)phenyl]urea;
N-(4-chlorophenyl)-N'-(cis-4-{[4-(dimethylamino)-5-methylpyrimidin-2-yl]amino}cyclohexyl)-N-ethylurea;
N-[3,5-bis(trifluoromethyl)phenyl]-N'-(cis-4-{[4-(dimethylamino)-5-methylpyrimidin-2-yl]amino}cyclohexyl)-N-ethylurea;
N'-(cis-4-{[4-(dimethylamino)-5-methylpyrimidin-2-yl]amino}cyclohexyl)-N-ethyl-N-phenylurea;
N'-(cis-4-{[4-(dimethylamino)-5-methylpyrimidin-2-yl]amino}cyclohexyl)-N-ethyl-N-(3-methylphenyl)urea; and
1-(2,3-dichloro-phenyl)-3-[cis-4-(4-dimethylamino-5-methyl-pyrimidin-2-ylamino)-cyclohexylmethyl]-urea;

or a pharmaceutically acceptable salt, hydrate, or solvate thereof.

**97.** The compound according to claim 75 wherein $R_1$ is selected from the group consisting of:

heterocyclyl, and
heterocyclyl substituted by substituent(s) independently selected from the group consisting of:

- carbocyclic aryloxy,
- carbocyclic aryloxy substituted by substituent(s) independently selected from the group consisting of:

- •• halogen, and
- •• $C_{1-5}$ alkoxy,

L is Formula (X) or (XI);
Y is -C(O)-;
$R_2$ is $-N(R_{2a})(R_{2b})$ wherein $R_{2a}$ is $C_{1-5}$ alkyl and $R_{2b}$ is $C_{1-5}$ alkyl;
wherein carbocyclic aryl is phenyl;
heterocyclyl is pyridyl; and
halogen is fluoro, chloro, bromo, or iodo;

or a pharmaceutically acceptable salt, hydrate, or solvate thereof.

**98.** The compound according to claim 75 or 97 wherein p is 1 and T is $C_{1-5}$ alkyl; $R_3$ and $R_4$ are both hydrogen; A is a single bond and B is $-CH_2-$;
or a pharmaceutically acceptable salt, hydrate, or solvate thereof.

**99.** The compound according to claim 75 wherein $R_1$ is selected from the group consisting of:

(i) carbocyclic aryl, and
carbocyclic aryl substituted by substituent(s) independently selected from the group consisting of:

- halogen,
- $C_{1-10}$ alkyl, and
- $C_{1-10}$ alkyl substituted by halogen,

(ii) heterocyclyl,
L is Formula (VII); and
Y is $-S(O)_2-$;
$R_2$ is $-N(R_{2a})(R_{2b})$ wherein $R_{2a}$ is $C_{1-5}$ alkyl and $R_{2b}$ is $C_{1-5}$ alkyl;
wherein carbocyclic aryl is phenyl or naphthyl;
heterocyclyl is furyl; and
halogen is fluoro, chloro, bromo, or iodo;

or a pharmaceutically acceptable salt, hydrate, or solvate thereof.

**100.** The compound according to any one of claims 75 or 99 wherein p is 1 and T is $C_{1-5}$ alkyl; $R_3$ and $R_4$ are both hydrogen, and A and B are both single bonds; or a pharmaceutically acceptable salt, hydrate, or solvate thereof.

**101.** The compound according to claim 1 is:

4-chloro-N-(cis-4-{[4-(dimethylamino)-5-methylpyrimidin-2-yl]amino}cyclohexyl)benzenesulfonamide;

or a pharmaceutically acceptable salt, hydrate, or solvate thereof.

**102.** The compound according to claim 1 wherein $R_1$ is selected from hydrogen, $-CO_2{}^tBu$, or $-CO_2Bn$ (Bn is a benzyl group);
$R_2$ is selected from the group consisting of:

hydrogen, halogen, hydroxy, carboxy, carbamoyl, amino, $C_{1-5}$ alkyl, $C_{1-5}$ alkyl substituted by halogen, $C_{1-5}$ alkyl substituted by hydroxy, $C_{1-5}$ alkyl substituted by carboxy, $C_{1-5}$ alkyl substituted by carbamoyl, $C_{1-5}$ alkoxy, $C_{1-5}$ alkoxy substituted by halogen, $-N(R_{2a})(R_{2b})$;

wherein $R_{2a}$ is hydrogen or $C_{1-5}$ alkyl and $R_{2b}$ is $C_{1-5}$ alkyl, $C_{3-6}$ cycloalkyl, or $C_{1-5}$ alkyl substituted by substituent (s) independently selected from the group consisting of:

- halogen,
- hydroxy,
- carboxy,

- carbamoyl,
- $C_{1-5}$ alkoxy,
- amino, and
- $C_{3-6}$ cycloalkyl;

or $R_2$ is methylamino or dimethylamino when Q is Formula (II);

Each T is independently selected from the group consisting of halogen, hydroxy, carboxy, carbamoyl, amino, cyano, nitro, $C_{1-5}$ alkyl, $C_{1-5}$ alkyl substituted by halogen, $C_{1-5}$ alkyl substituted by hydroxy, $C_{1-5}$ alkyl substituted by carboxy, $C_{1-5}$ alkyl substituted by carbamoyl, $C_{2-5}$ alkenyl, $C_{2-5}$ alkynyl, $C_{3-6}$ cycloalkyl, $C_{1-5}$ alkoxy, $C_{1-5}$ alkoxy substituted by halogen, carbocyclic aryl, heterocyclyl, and -N($R_{2a}$)($R_{2b}$);

p is 0, 1, 2, 3, 4 or 5;

L is selected from the group consisting of Formula (VII), (X), (XI), (XV), (XVIII), or (XIX): wherein $R_3$ and $R_4$ are independently hydrogen or $C_{1-5}$ alkyl; and A and

B are independently a single bond or -$CH_2$-; and

Y is a single bond;

or a pharmaceutically acceptable salt, hydrate, or solvate thereof.

**103.** A pharmaceutical composition comprising a therapeutically effective amount of a compound according to any one of claims 1 to 102 in combination with a pharmaceutically acceptable carrier.

**104.** A method for the prophylaxis or treatment of improving memory function, sleeping and arousal, anxiety, depression, mood disorders, seizure, obesity, diabetes, appetite and eating disorders, cardiovascular disease, hypertension, dyslipidemia, myocardial infarction, binge eating disorders including bulimia, anorexia, mental disorders including manic depression, schizophrenia, delirium, dementia, stress, cognitive disorders, attention deficit disorder, substance abuse disorders and dyskinesias including Parkinson's disease, epilepsy, and addiction comprising administering to an individual suffering from said condition a therapeutically effective amount of a compound according to any one of claims 1 to 102 or a pharmaceutical composition according to claim 103.

**105.** A method for the prophylaxis or treatment of an eating disorder, obesity or an obesity related disorder comprising administering to an individual suffering from said condition a therapeutically effective amount of a compound according to any one of claims 1 to 102 or a pharmaceutical composition according to claim 103.

**106.** A method for the prophylaxis or treatment of anxiety, depression, schizophrenia, addiction, or epilepsy comprising administering to an individual suffering from said condition a therapeutically effective amount of a compound according to any one of claims 1 to 102 or a pharmaceutical composition according to claim 103.

**107.** A compound according to any one of claims 1 to 102 or a pharmaceutical composition according to claim 103 for use in a method of treatment of the human or animal body by therapy.

**108.** A compound according to any one of claims 1 to 102 or a pharmaceutical composition according to claim 103 for use in a method of prophylaxis or treatment of an eating disorder, obesity or an obesity related disorder of the human or animal body by therapy.

**109.** A compound according to any one of claims 1 to 102 or a pharmaceutical composition according to claim 103 for use in a method of prophylaxis or treatment of anxiety, depression, schizophrenia, addiction, or epilepsy of the human or animal body by therapy.

**110.** A compound according to any one of claims 1 to 102 for the manufacture of a medicament for use in the prophylaxis or treatment of an eating disorder, obesity or obesity related disorders.

**111.** A compound according to any one of claims 1 to 102 for the manufacture of a medicament for use in the prophylaxis or treatment of anxiety, depression, schizophrenia, addiction, or epilepsy.

**112.** A method of producing a pharmaceutical composition comprising admixing a compound according to any one of claims 1 to 102 and a pharmaceutically acceptable carrier.